# EUROPEAN PATENT APPLICATION

(11) **EP 1 988 097 A1**
(43) Date of publication of application: **05.11.2008**
(21) Application number: 08011769.0
(22) Date of filing: 09.05.2002
(51) Int. Cl.: C07H 21/02, C12N 15/63, C12N 15/85

(54) **Compositions and methods for the therapy and diagnosis of prostate cancer**

(30) Priority: 09.05.2001 US 852911; 29.06.2001 US 895814; 10.12.2001 US 12896
(62) Divisional of application: 02725984.5
(71) Applicant: CORIXA CORPORATION, Wilmington, DE 19808 (US)
(72) Inventor: The designation of the inventor has not yet been filed
(74) Representative: Thornton, Neil

(57) **Abstract**

Compositions and methods for the therapy and diagnosis of cancer, particularly prostate cancer, are disclosed. Illustrative compositions comprise one or more prostate-specific polypeptides, immunogenic portions thereof, polynucleotides that encode such polypeptides, antigen presenting cell that expresses such polypeptides, and T cells that are specific for cells expressing such polypeptides. The disclosed compositions are useful, for example, in the diagnosis, prevention and/or treatment of diseases, particularly prostate cancer.

SEQUENCE LISTING

<110> Corixa Corporation
Xu, Jiangchun
        Dillon, Davin C.
        Mitcham, Jennifer L.
        Harlocker, Susan L.
        Jiang, Yuqiu
        Henderson, Robert A.
        Kalos, Michael D.
        Fanger, Gary R.
        Retter, Marc W.
        Stolk, John A.
        Day, Craig H.
        Vedvick, Thomas S.
        Carter, Darrick
        Li, Samuel X.
        Wang, Aijun
        Skeiky, Yasir A. W.
        Hepler, William T.
        Hural, John
        McNeill, Patricia D.
        Houghton, Raymond L.
        Vinals y de Bassols, Carlota
        Foy, Teresa M.
        Watanabe, Yoshihiro
        Deng, Ta

<120> COMPOSITIONS AND METHODS FOR THE THERAPY AND
        DIAGNOSIS OF PROSTATE CANCER

<130> P/50732.EP02/NT

<140>   Divisional of EP 02725984.5
<141>   9 May 2002

<150>   EP 02725984.5
<151>   9 May 2002

<150>   PCT/US02/14753
<151>   9 May 2002

<150>   US 09/852,911
<151>   9 May 2001

<150>   US 09/895,814
<151>   29 June 2001

<150>   US 10/012,896
<151>   10 December 2001

<160> 1033

<170> FastSEQ for Windows Version 3.0

<210> 1
<211> 814
<212> DNA
<213> Homo sapiens

<220>
<221> misc_feature
<222> 542, 574, 588, 631, 681, 683, 698, 733, 750, 751, 757, 768,
777, 793, 802
<223> n = A,T,C or G

<400> 1
tttttttttt tttttcacag tataacagct ctttatttct gtgagttcta ctaggaaatc 60

atcaaatctg agggttgtct ggaggacttc aatacacctc cccccatagt gaatcagctt 120
ccagggggtc cagtccctct ccttacttca tccccatccc atgccaaagg aagaccctcc 180
ctccttggct cacagccttc tctaggcttc ccagtgcctc caggacagag tgggttatgt 240
tttcagctcc atccttgctg tgagtgtctg gtgcgttgtg cctccagctt ctgctcagtg 300
cttcatggac agtgtccagc acatgtcact ctccactctc tcagtgtgga tccactagtt 360
ctagagcggc cgccaccgcg gtggagctcc agcttttgtt ccctttagtg agggttaatt 420
gcgcgcttgg cgtaatcatg gtcataactg tttcctgtgt gaaattgtta tccgctcaca 480
attccacaca acatacgagc cggaagcata aagtgtaaag cctggggtgc ctaatgagtg 540
anctaactca cattaattgc gttgcgctca ctgnccgctt tccagtcngg aaaactgtcg 600
tgccagctgc attaatgaat cggccaacgc ncggggaaaa gcggtttgcg ttttgggggc 660
tcttccgctt ctcgctcact nantcctgcg ctcggtcntt cggctgcggg gaacggtatc 720
actcctcaaa ggnggtatta cggttatccn naaatcnggg gatacccngg aaaaaanttt 780
aacaaaaggg cancaaaggg cngaaacgta aaaa 814

<210> 2
<211> 816
<212> DNA
<213> Homo sapiens

<220>
<221> misc_feature
<222> 588, 600, 660, 664, 671, 698, 736, 739, 758, 784, 786, 803,
814
<223> n = A,T,C or G

<400> 2
acagaaatgt tggatggtgg agcacctttc tatacgactt acaggacagc agatggggaa 60
ttcatggctg ttggagcaat agaaccccag ttctacgagc tgctgatcaa aggacttgga 120
ctaaagtctg atgaacttcc caatcagatg agcatggatg attggccaga aatgaagaag 180
aagtttgcag atgtatttgc aaagaagacg aaggcagagt ggtgtcaaat ctttgacggc 240
acagatgcct gtgtgactcc ggttctgact tttgaggagg ttgttcatca tgatcacaac 300
aaggaacggg gctcgtttat caccagtgag gagcaggacg tgagcccccg ccctgcacct 360
ctgctgttaa acaccccagc catcccttct ttcaaaaggg atccactagt tctagaagcg 420
gccgccaccg cggtggagct ccagcttttg ttccctttag tgagggttaa ttgcgcgctt 480
ggcgtaatca tggtcatagc tgtttcctgt gtgaaattgt tatccgctca caattccccc 540
aacatacgag ccggaacata aagtgttaag cctggggtgc ctaatgantg agctaactcn 600
cattaattgc gttgcgctca ctgcccgctt tccagtcggg aaaactgtcg tgccactgcn 660
ttantgaatc ngccaccccc cgggaaaagg cggttgcntt ttgggcctct tccgctttcc 720
tcgctcattg atcctngcnc ccggtcttcg gctgcggnga acggttcact cctcaaaggc 780
ggtntnccgg ttatccccaa acnggggata cccnga 816

<210> 3
<211> 773
<212> DNA
<213> Homo sapiens

<220>
<221> misc_feature
<222> 283, 284, 436, 557, 608, 637, 647, 654, 669, 671, 672, 699,
715, 735, 737, 740, 762, 772
<223> n = A,T,C or G

<400> 3
cttttgaaag aagggatggc tggggtgttt aacagcagag gtgcagggcg ggggctcacg 60
tcctgctcct cactggtgat aaacgagccc cgttccttgt tgtgatcatg atgaacaacc 120
tcctcaaaag tcagaaccgg agtcacacag gcatctgtgc cgtcaaagat ttgacaccac 180
tctgccttcg tcttctttgc aaatacatct gcaaacttct tcttcatttc tggccaatca 240
tccatgctca tctgattggg aagttcatca gactttagtc canntccttt gatcagcagc 300
tcgtagaact ggggttctat tgctccaaca gccatgaatt ccccatctgc tgtcctgtaa 360
gtcgtataga aaggtgctcc accatccaac atgttctgtc ctcgaggggg ggcccggtac 420
ccaattcgcc ctatantgag tcgtattacg cgcgctcact ggccgtcgtt ttacaacgtc 480
gtgactggga aaaccctggg cgttaccaac ttaatcgcct tgcagcacat ccccctttcg 540
ccagctgggc gtaatancga aaaggcccgc accgatcgcc cttccaacag ttgcgcacct 600
gaatgggnaa atgggacccc cctgttaccg cgcattnaac ccccgcnggg tttngttgtt 660
acccccacnt nnaccgctta cactttgcca gcgccttanc gcccgctccc tttcnccttt 720
cttcccttcc tttcncnccn ctttcccccg gggtttcccc cntcaaaccc cna 773

<210> 4

<211> 828
<212> DNA
<213> Homo sapiens

<220>
<221> misc_feature
<222> 335, 362, 380, 383, 396, 424, 446, 452, 492, 507, 510, 559,
573, 597, 646, 663, 672, 680, 732, 737, 741, 747, 749, 768,
784, 813, 824, 826
<223> n = A,T,C or G

<400> 4
cctcctgagt cctactgacc tgtgctttct ggtgtggagt ccagggctgc taggaaaagg 60
aatgggcaga cacaggtgta tgccaatgtt tctgaaatgg gtataatttc gtcctctcct 120
tcggaacact ggctgtctct gaagacttct cgctcagttt cagtgaggac acacacaaag 180
acgtgggtga ccatgttgtt tgtggggtgc agagatggga ggggtggggc ccaccctgga 240
agagtggaca gtgacacaag gtggacactc tctacagatc actgaggata agctggagcc 300
acaatgcatg aggcacacac acagcaagga tgacnctgta aacatagccc acgctgtcct 360
gngggcactg ggaagcctan atnaggccgt gagcanaaag aaggggagga tccactagtt 420
ctanagcggc cgccaccgcg gtgganctcc ancttttgtt ccctttagtg agggttaatt 480
gcgcgcttgg cntaatcatg gtcatanctn tttcctgtgt gaaattgtta tccgctcaca 540
attccacaca acatacganc cggaaacata aantgtaaac ctggggtgcc taatgantga 600
ctaactcaca ttaattgcgt tgcgctcact gcccgctttc caatcnggaa acctgtcttg 660
ccncttgcat tnatgaatcn gccaaccccc ggggaaaagc gtttgcgttt tgggcgctct 720
tccgcttcct cnctcantta ntccctncnc tcggtcattc cggctgcngc aaaccggttc 780
accncctcca aagggggtat tccggtttcc ccnaatccgg gganancc 828

<210> 5
<211> 834
<212> DNA
<213> Homo sapiens

<220>
<221> misc_feature
<222> 693, 723, 726, 738, 743, 833
<223> n = A,T,C or G

<400> 5
tttttttttt tttttactga tagatggaat ttattaagct tttcacatgt gatagcacat 60
agttttaatt gcatccaaag tactaacaaa aactctagca atcaagaatg gcagcatgtt 120
attttataac aatcaacacc tgtggctttt aaaatttggt tttcataaga taatttatac 180
tgaagtaaat ctagccatgc ttttaaaaaa tgctttaggt cactccaagc ttggcagtta 240
acatttggca taaacaataa taaaacaatc acaatttaat aaataacaaa tacaacattg 300
taggccataa tcatatacag tataaggaaa aggtggtagt gttgagtaag cagttattag 360
aatagaatac cttggcctct atgcaaatat gtctagacac tttgattcac tcagccctga 420
cattcagttt tcaaagtagg agacaggttc tacagtatca ttttacagtt tccaacacat 480
tgaaaacaag tagaaaatga tgagttgatt tttattaatg cattacatcc tcaagagtta 540
tcaccaaccc ctcagttata aaaaattttc aagttatatt agtcatataa cttggtgtgc 600
ttattttaaa ttagtgctaa atggattaag tgaagacaac aatggtcccc taatgtgatt 660
gatattggtc atttttacca gcttctaaat ctnaactttc aggcttttga actggaacat 720
tgnatnacag tgttccanag ttncaaccta ctggaacatt acagtgtgct tgattcaaaa 780
tgttattttg ttaaaaatta aattttaacc tggtggaaaa ataatttgaa atna 834

<210> 6
<211> 818
<212> DNA
<213> Homo sapiens

<220>
<221> misc_feature
<222> 664, 667, 674, 692, 708, 728, 731, 736, 751, 763, 774, 786,
788, 799, 801, 804
<223> n = A,T,C or G

<400> 6
tttttttttt tttttttttt aagaccctca tcaatagatg gagacataca gaaatagtca 60
aaccacatct acaaaatgcc agtatcaggc ggcggcttcg aagccaaagt gatgtttgga 120
tgtaaagtga aatattagtt ggcggatgaa gcagatagtg aggaaagttg agccaataat 180

gacgtgaagt ccgtggaagc ctgtggctac aaaaaatgtt gagccgtaga tgccgtcgga 240
aatggtgaag ggagactcga agtactctga ggcttgtagg agggtaaaat agagacccag 300
taaaattgta ataagcagtg cttgaattat ttggtttcgg ttgttttcta ttagactatg 360
gtgagctcag gtgattgata ctcctgatgc gagtaatacg gatgtgttta ggagtgggac 420
ttctagggga tttagcgggg tgatgcctgt tgggggccag tgccctccta gttggggggt 480
aggggctagg ctggagtggt aaaaggctca gaaaaatcct gcgaagaaaa aaacttctga 540
ggtaataaat aggattatcc cgtatcgaag gcctttttgg acaggtggtg tgtggtggcc 600
ttggtatgtg ctttctcgtg ttacatcgcg ccatcattgg tatatggtta gtgtgttggg 660
ttantanggc ctantatgaa gaacttttgg antggaatta aatcaatngc ttggccggaa 720
gtcattanga nggctnaaaa ggccctgtta ngggtctggg ctnggtttta cccnacccat 780
ggaatncncc ccccggacna ntgnatccct attcttaa 818

<210> 7
<211> 817
<212> DNA
<213> Homo sapiens

<220>
<221> misc_feature
<222> 280, 486, 491, 505, 508, 517, 522, 534, 547, 555, 583, 590,
594, 605, 608, 609, 642, 648, 653, 654, 658, 662, 668, 670,
677, 684, 692, 705, 707, 717, 719, 720, 723, 731, 737, 743,
747, 752, 763, 765, 766, 769, 784, 786, 790, 799, 805
<223> n = A,T,C or G

<221> misc_feature
<222> 808, 815
<223> n = A,T,C or G

<400> 7
tttttttttt tttttttttt tggctctaga gggggtagag ggggtgctat agggtaaata 60
cgggccctat ttcaaagatt tttaggggaa ttaattctag gacgatgggt atgaaactgt 120
ggtttgctcc acagatttca gagcattgac cgtagtatac ccccggtcgt gtagcggtga 180
aagtggtttg gtttagacgt ccgggaattg catctgtttt taagcctaat gtggggacag 240
ctcatgagtg caagacgtct tgtgatgtaa ttattatacn aatgggggct tcaatcggga 300
gtactactcg attgtcaacg tcaaggagtc gcaggtcgcc tggttctagg aataatgggg 360
gaagtatgta ggaattgaag attaatccgc cgtagtcggt gttctcctag gttcaatacc 420
attggtggcc aattgatttg atggtaaggg gagggatcgt tgaactcgtc tgttatgtaa 480
aggatncctt ngggatggga aggcnatnaa ggactangga tnaatggcgg gcangatatt 540
tcaaacngtc tctanttcct gaaacgtctg aaatgttaat aanaattaan tttngttatt 600
gaatnttnng gaaaagggct tacaggacta gaaaccaaat angaaaanta atnntaangg 660
cnttatcntn aaaggtnata accnctccta tnatcccacc caatngnatt ccccacncnn 720
acnattggat nccccanttc canaaanggc cnccccccgg tgnannccnc cttttgttcc 780
cttnantgan ggttattcnc ccctngcntt atcancc 817

<210> 8
<211> 799
<212> DNA
<213> Homo sapiens

<220>
<221> misc_feature
<222> 252, 259, 282, 347, 358, 375, 377, 428, 459, 471, 496, 497,
522, 528, 533, 545, 550, 558, 559, 561, 565, 574, 577, 582,
592, 597, 609, 612, 622, 623, 636, 640, 641, 647, 649, 651,
658, 659, 664, 674, 677, 681, 684, 685, 690, 692, 693
<223> n = A,T,C or G

<221> misc_feature
<222> 695, 696, 698, 700, 709, 710, 712, 713, 716, 717, 719, 720,
721, 731, 734, 735, 737, 747, 748, 751, 756, 759, 764, 767,
771, 772, 774, 775, 791, 796, 797
<223> n = A,T,C or G

<400> 8
   catttccggg tttactttct aaggaaagcc gagcggaagc tgctaacgtg ggaatcggtg 60
   cataaggaga actttctgct ggcacgcgct agggacaagc gggagagcga ctccgagcgt 120
   ctgaagcgca cgtcccagaa ggtggacttg gcactgaaac agctgggaca catccgcgag 180
   tacgaacagc gcctgaaagt gctggagcgg gaggtccagc agtgtagccg cgtcctgggg 240
   tgggtggccg angcctganc cgctctgcct tgctgccccc angtgggccg ccaccccctg 300
   acctgcctgg gtccaaacac tgagccctgc tggcggactt caagganaac ccccacangg 360
   ggattttgct cctanantaa ggctcatctg ggcctcggcc cccccacctg gttggccttg 420
   tctttgangt gagccccatg tccatctggg ccactgtcng gaccaccttt ngggagtgtt 480
   ctccttacaa ccacannatg cccggctcct cccggaaacc antcccancc tgngaaggat 540
   caagncctgn atccactnnt nctanaaccg gccnccnccg cngtggaacc cnccttntgt 600
   tccttttcnt tnagggttaa tnncgccttg gccttnccan ngtcctncnc nttttccnnt 660
   gttnaaattg ttangcnccc nccnntcccn cnncnncnan cccgacccnn annttnnann 720
   ncctgggggt nccnncngat tgacccnncc nccctntant tgcnttnggg nncnntgccc 780
   ctttccctct nggganncg 799

<210> 9
<211> 801
<212> DNA
<213> Homo sapiens

<220>
<221> misc_feature
<222> 64, 216, 239, 257, 259, 273, 284, 288, 298, 300, 312, 327,
339, 364, 373, 376, 384, 392, 398, 405, 411, 436, 439, 441,
466, 470, 482, 485, 488, 495, 504, 508, 509, 513, 519, 521,
522, 525, 534, 547, 559, 578, 590, 597, 607, 615, 619
<223> n = A,T,C or G

<221> misc_feature
<222> 620, 622, 626, 632, 635, 639, 646, 647, 650, 655, 666, 668,
675, 678, 680, 686, 695, 702, 704, 706, 707, 709, 716, 717,
721, 725, 726, 727, 729, 733, 735, 737, 739, 740, 742, 753,
754, 755, 757, 767, 768, 770, 776, 784, 800
<223> n = A,T,C or G

<400> 9
acgccttgat cctcccaggc tgggactggt tctgggagga gccgggcatg ctgtggtttg 60
taangatgac actcccaaag gtggtcctga cagtggccca gatggacatg gggctcacct 120
caaggacaag gccaccaggt gcgggggccg aagcccacat gatccttact ctatgagcaa 180
aatcccctgt gggggcttct ccttgaagtc cgccancagg gctcagtctt tggacccang 240
caggtcatgg ggttgtngnc caactggggg ccncaacgca aaanggcnca gggcctcngn 300
cacccatccc angacgcggc tacactnctg gacctcccnc tccaccactt tcatgcgctg 360
ttcntacccg cgnatntgtc ccanctgttt cngtgccnac tccancttct nggacgtgcg 420
ctacatacgc ccggantcnc nctcccgctt tgtccctatc cacgtnccan caacaaattt 480
cnccntantg caccnattcc cacntttnnc agntttccnc nncgngcttc cttntaaaag 540
ggttganccc cggaaaatnc cccaaagggg gggggccngg tacccaactn cccccctnata 600
gctgaantcc ccatnaccnn gnctcnatgg anccntccnt tttaannacn ttctnaactt 660
gggaanancc ctcgnccntn cccccnttaa tcccnccttg cnangnncnt cccccnntcc 720
ncccnnntng gcntntnann cnaaaaaggc ccnnnancaa tctcctnncn cctcanttcg 780
ccanccctcg aaatcggccn c 801

<210> 10
<211> 789
<212> DNA
<213> Homo sapiens

<220>
<221> misc_feature
<222> 9, 380, 451, 565, 582, 716, 718, 758, 762, 765, 768, 771,
779, 783
<223> n = A,T,C or G

<400> 10
cagtctatnt ggccagtgtg gcagctttcc ctgtggctgc cggtgccaca tgcctgtccc 60
acagtgtggc cgtggtgaca gcttcagccg ccctcaccgg gttcaccttc tcagccctgc 120
agatcctgcc ctacacactg gcctccctct accaccggga gaagcaggtg ttcctgccca 180
aataccgagg ggacactgga ggtgctagca gtgaggacag cctgatgacc agcttcctgc 240
caggccctaa gcctggagct cccttcccta atggacacgt gggtgctgga ggcagtggcc 300
tgctcccacc tccacccgcg ctctgcgggg cctctgcctg tgatgtctcc gtacgtgtgg 360
tggtgggtga gcccaccgan gccagggtgg ttccgggccg gggcatctgc ctggacctcg 420
ccatcctgga tagtgcttcc tgctgtccca ngtggcccca tccctgttta tgggctccat 480

tgtccagctc agccagtctg tcactgccta tatggtgtct gccgcaggcc tgggtctggt 540
cccatttact ttgctacaca ggtantattt gacaagaacg anttggccaa atactcagcg 600
ttaaaaaatt ccagcaacat tgggggtgga aggcctgcct cactgggtcc aactccccgc 660
tcctgttaac cccatggggc tgccggcttg gccgccaatt tctgttgctg ccaaantnat 720
gtggctctct gctgccacct gttgctggct gaagtgcnta cngcncanct nggggggtng 780
ggngttccc 789

<210> 11
<211> 772
<212> DNA
<213> Homo sapiens

<220>
<221> misc_feature
<222> 335, 519, 539, 570, 585, 651, 674, 676, 692, 694, 698, 702,
715, 726, 734, 738, 768
<223> n = A,T,C or G

<400> 11
cccaccctac ccaaatatta gacaccaaca cagaaaagct agcaatggat tcccttctac 60
tttgttaaat aaataagtta aatatttaaa tgcctgtgtc tctgtgatgg caacagaagg 120
accaacaggc cacatcctga taaaaggtaa gaggggggtg gatcagcaaa aagacagtgc 180
tgtgggctga ggggacctgg ttcttgtgtg ttgcccctca ggactcttcc cctacaaata 240
actttcatat gttcaaatcc catggaggag tgtttcatcc tagaaactcc catgcaagag 300
ctacattaaa cgaagctgca ggttaagggg cttanagatg ggaaaccagg tgactgagtt 360
tattcagctc ccaaaaaccc ttctctaggt gtgtctcaac taggaggcta gctgttaacc 420
ctgagcctgg gtaatccacc tgcagagtcc ccgcattcca gtgcatggaa cccttctggc 480
ctccctgtat aagtccagac tgaaaccccc ttggaaggnc tccagtcagg cagccctana 540
aactggggaa aaaagaaaag gacgccccan cccccagctg tgcanctacg cacctcaaca 600
gcacagggtg gcagcaaaaa aaccacttta ctttggcaca aacaaaaact ngggggggca 660
accccggcac cccnangggg gttaacagga ancngggnaa cntggaaccc aattnaggca 720
ggcccnccac cccnaatntt gctgggaaat ttttcctccc ctaaattntt tc 772

<210> 12
<211> 751
<212> DNA
<213> Homo sapiens

<220>
<221> misc_feature
<222> 186, 370, 390, 396, 413, 440, 448, 455, 466, 468, 479, 482,
502, 508, 536, 549, 577, 620, 622, 624, 625, 634, 637, 642,
652, 654, 662, 665, 669, 680, 682, 699, 702, 703, 716, 719,
720, 723, 730, 735, 742, 746, 748, 749
<223> n = A,T,C or G

<400> 12
gccccaattc cagctgccac accacccacg gtgactgcat tagttcggat gtcatacaaa 60
agctgattga agcaaccctc tactttttgg tcgtgagcct tttgcttggt gcaggtttca 120
ttggctgtgt tggtgacgtt gtcattgcaa cagaatgggg gaaaggcact gttctctttg 180
aagtanggtg agtcctcaaa atccgtatag ttggtgaagc cacagcactt gagccctttc 240
atggtggtgt tccacacttg agtgaagtct tcctgggaac cataatcttt cttgatggca 300
ggcactacca gcaacgtcag ggaagtgctc agccattgtg gtgtacacca aggcgaccac 360
agcagctgcn acctcagcaa tgaagatgan gaggangatg aagaagaacg tcncgagggc 420
acacttgctc tcagtcttan caccatanca gcccntgaaa accaananca aagaccacna 480
cnccggctgc gatgaagaaa tnaccccncg ttgacaaact tgcatggcac tggganccac 540
agtggcccna aaaatcttca aaaaggatgc cccatcnatt gaccccccaa atgcccactg 600
ccaacagggg ctgccccacn cncnnaacga tganccnatt gnacaagatc tncntggtct 660
tnatnaacnt gaaccctgcn tngtggctcc tgttcaggnc cnnggcctga cttctnaann 720
aangaactcn gaagncccca cngganannc g 751

<210> 13
<211> 729
<212> DNA
<213> Homo sapiens

<220>
<221> misc_feature

<222> 67, 78, 96, 105, 503, 527, 582, 583, 606, 626, 704, 718, 724
<223> n = A,T,C or G

<400> 13
gagccaggcg tccctctgcc tgcccactca gtggcaacac ccgggagctg ttttgtcctt 60
tgtggancct cagcagtncc ctctttcaga actcantgcc aaganccctg aacaggagcc 120
accatgcagt gcttcagctt cattaagacc atgatgatcc tcttcaattt gctcatcttt 180
ctgtgtggtg cagccctgtt ggcagtgggc atctgggtgt caatcgatgg ggcatccttt 240
ctgaagatct tcgggccact gtcgtccagt gccatgcagt ttgtcaacgt gggctacttc 300
ctcatcgcag ccggcgttgt ggtcttagct ctaggtttcc tgggctgcta tggtgctaag 360
actgagagca agtgtgccct cgtgacgttc ttcttcatcc tcctcctcat cttcattgct 420
gaggttgcaa tgctgtggtc gccttggtgt acaccacaat ggctgagcac ttcctgacgt 480
tgctggtaat gcctgccatc aanaaaagat tatgggttcc caggaanact tcactcaagt 540
gttggaacac caccatgaaa gggctcaagt gctgtggctt cnnccaacta tacggatttt 600
gaagantcac ctacttcaaa gaaaanagtg cctttccccc atttctgttg caattgacaa 660
acgtccccaa cacagccaat tgaaaacctg cacccaaccc aaangggtcc ccaaccanaa 720
attnaaggg 729

<210> 14
<211> 816
<212> DNA
<213> Homo sapiens

<220>
<221> misc_feature
<222> 261, 303, 337, 338, 340, 349, 364, 370, 372, 385, 443, 458,
462, 467, 484, 495, 503, 506, 512, 534, 544, 556, 563, 571,
574, 597, 599, 605, 606, 608, 615, 618, 627, 635, 636, 650,
653, 654, 673, 675, 679, 686, 694, 696, 698, 700, 720
<223> n = A,T,C or G

<221> misc_feature
<222> 722, 724, 740, 741, 767, 773, 774, 777, 790, 807, 809, 815
<223> n = A,T,C or G

<400> 14
tgctcttcct caaagttgtt cttgttgcca taacaaccac cataggtaaa gcgggcgcag 60
tgttcgctga aggggttgta gtaccagcgc gggatgctct ccttgcagag tcctgtgtct 120
ggcaggtcca cgcagtgccc tttgtcactg gggaaatgga tgcgctggag ctcgtcaaag 180
ccactcgtgt atttttcaca ggcagcctcg tccgacgcgt cggggcagtt gggggtgtct 240
tcacactcca ggaaactgtc natgcagcag ccattgctgc agcggaactg ggtgggctga 300
cangtgccag agcacactgg atggcgcctt tccatgnnan gggccctgng ggaaagtccc 360
tganccccan anctgcctct caaangcccc accttgcaca ccccgacagg ctagaatgga 420
atcttcttcc cgaaaggtag ttnttcttgt tgcccaancc anccccntaa acaaactctt 480
gcanatctgc tccgnggggg tcntantacc ancgtgggaa aagaacccca ggcngcgaac 540
caancttgtt tggatncgaa gcnataatct nctnttctgc ttggtggaca gcaccantna 600
ctgtnnanct ttagnccntg gtcctcntgg gttgnncttg aacctaatcn ccnntcaact 660
gggacaaggt aantngccnt cctttnaatt cccnancntn ccccctggtt tggggttttn 720
cncnctccta ccccagaaan nccgtgttcc cccccaacta ggggccnaaa ccnnttnttc 780
cacaaccctn ccccacccac gggttcngnt ggttng 816

<210> 15
<211> 783
<212> DNA
<213> Homo sapiens

<220>
<221> misc_feature
<222> 21, 159, 278, 408, 415, 439, 441, 445, 458, 501, 541, 558,
562, 569, 591, 625, 634, 638, 648, 655, 662, 668, 677, 678,
680, 691, 694, 697, 710, 716, 724, 727, 728, 736, 738, 754,
755, 758, 766, 768, 772, 775
<223> n = A,T,C or G

<400> 15
ccaaggcctg ggcaggcata nacttgaagg tacaacccca ggaacccctg gtgctgaagg 60
atgtggaaaa cacagattgg cgcctactgc ggggtgacac ggatgtcagg gtagagagga 120
aagacccaaa ccaggtggaa ctgtggggac tcaaggaang cacctacctg ttccagctga 180

cagtgactag ctcagaccac ccagaggaca cggccaacgt cacagtcact gtgctgtcca 240
ccaagcagac agaagactac tgcctcgcat ccaacaangt gggtcgctgc cggggctctt 300
tcccacgctg gtactatgac cccacggagc agatctgcaa gagtttcgtt tatggaggct 360
gcttgggcaa caagaacaac taccttcggg aagaagagtg cattctancc tgtcngggtg 420
tgcaaggtgg gcctttgana ngcanctctg gggctcangc gactttcccc cagggcccct 480
ccatggaaag gcgccatcca ntgttctctg gcacctgtca gcccacccag ttccgctgca 540
ncaatggctg ctgcatcnac antttcctng aattgtgaca acacccccca ntgcccccaa 600
ccctcccaac aaagcttccc tgttnaaaaa tacnccantt ggcttttnac aaacncccgg 660
cncctccntt ttccccnntn aacaaagggc nctngcnttt gaactgcccn aacccnggaa 720
tctnccnngg aaaaantncc ccccctggtt cctnnaancc cctccncnaa anctnccccc 780
ccc 783

<210> 16
<211> 801
<212> DNA
<213> Homo sapiens

<220>
<221> misc_feature
<222> 412, 455, 483, 501, 550, 589, 602, 613, 616, 618, 620, 648,
651, 673, 717, 728, 731, 733, 746, 749, 790, 800
<223> n = A,T,C or G

<400> 16
   gccccaattc cagctgccac accacccacg gtgactgcat tagttcggat gtcatacaaa 60
   agctgattga agcaaccctc tactttttgg tcgtgagcct tttgcttggt gcaggtttca 120
   ttggctgtgt tggtgacgtt gtcattgcaa cagaatgggg gaaaggcact gttctctttg 180
   aagtagggtg agtcctcaaa atccgtatag ttggtgaagc cacagcactt gagccctttc 240
   atggtggtgt tccacacttg agtgaagtct tcctgggaac cataatcttt cttgatggca 300
   ggcactacca gcaacgtcag gaagtgctca gccattgtgg tgtacaccaa ggcgaccaca 360
   gcagctgcaa cctcagcaat gaagatgagg aggaggatga agaagaacgt cncgagggca 420
   cacttgctct ccgtcttagc accatagcag cccangaaac caagagcaaa gaccacaacg 480
   ccngctgcga atgaaagaaa ntacccacgt tgacaaactg catggccact ggacgacagt 540
   tggcccgaan atcttcagaa aagggatgcc ccatcgattg aacacccana tgcccactgc 600
   cnacagggct gcnccncncn gaaagaatga gccattgaag aaggatcntc ntggtcttaa 660
   tgaactgaaa ccntgcatgg tggcccctgt tcagggctct tggcagtgaa ttctganaaa 720
   aaggaacngc ntnagccccc ccaaangana aaacaccccc gggtgttgcc ctgaattggc 780
   ggccaaggan ccctgccccn g 801

<210> 17
<211> 740
<212> DNA
<213> Homo sapiens

<220>
<221> misc_feature
<222> 494, 508, 543, 545, 555, 580, 614, 617, 637, 639, 648, 654,
673, 680, 683, 690, 702, 703, 704, 713, 716, 729, 731, 732,
740
<223> n = A,T,C or G

<400> 17
gtgagagcca ggcgtccctc tgcctgccca ctcagtggca acacccggga gctgttttgt 60
cctttgtgga gcctcagcag ttccctcttt cagaactcac tgccaagagc cctgaacagg 120
agccaccatg cagtgcttca gcttcattaa gaccatgatg atcctcttca atttgctcat 180
ctttctgtgt ggtgcagccc tgttggcagt gggcatctgg gtgtcaatcg atggggcatc 240
ctttctgaag atcttcgggc cactgtcgtc cagtgccatg cagtttgtca acgtgggcta 300
cttcctcatc gcagccggcg ttgtggtctt tgctcttggt ttcctgggct gctatggtgc 360
taagacggag agcaagtgtg ccctcgtgac gttcttcttc atcctcctcc tcatcttcat 420
tgctgaagtt gcagctgctg tggtcgcctt ggtgtacacc acaatggctg aaccattcct 480
gacgttgctg gtantgcctg ccatcaanaa agattatggg ttcccaggaa aaattcactc 540
aantntggaa caccnccatg aaaagggctc caatttctgn tggcttcccc aactataccg 600
gaattttgaa agantcnccc tacttccaaa aaaaaanant tgcctttncc cccnttctgt 660
tgcaatgaaa acntcccaan acngccaatn aaaacctgcc cnnncaaaaa ggntcncaaa 720
caaaaaaant nnaagggttn 740

<210> 18
<211> 802

<212> DNA
<213> Homo sapiens

<220>
<221> misc_feature
<222> 22, 24, 343, 516, 530, 534, 535, 556, 559, 569, 592, 603,
611, 629, 640, 644, 653, 654, 667, 677, 678, 679, 695, 698,
727, 728, 748, 750, 753, 762, 765, 768, 773, 776, 779, 782,
786, 789, 794, 801
<223> n = A,T,C or G

<400> 18
ccgctggttg cgctggtcca gngnagccac gaagcacgtc agcatacaca gcctcaatca 60
caaggtcttc cagctgccgc acattacgca gggcaagagc ctccagcaac actgcatatg 120
ggatacactt tactttagca gccagggtga caactgagag gtgtcgaagc ttattcttct 180
gagcctctgt tagtggagga agattccggg cttcagctaa gtagtcagcg tatgtcccat 240
aagcaaacac tgtgagcagc cggaaggtag aggcaaagtc actctcagcc agctctctaa 300
cattgggcat gtccagcagt tctccaaaca cgtagacacc agnggcctcc agcacctgat 360
ggatgagtgt ggccagcgct gccccccttgg ccgacttggc taggagcaga aattgctcct 420
ggttctgccc tgtcaccttc acttccgcac tcatcactgc actgagtgtg ggggacttgg 480
gctcaggatg tccagagacg tggttccgcc ccctcnctta atgacaccgn ccanncaacc 540
gtcggctccc gccgantgng ttcgtcgtnc ctgggtcagg gtctgctggc cnctacttgc 600
aancttcgtc nggcccatgg aattcaccnc accggaactn gtangatcca ctnnttctat 660
aaccggncgc caccgcnnnt ggaactccac tcttnttncc tttacttgag ggttaaggtc 720
acccttnncg ttaccttggt ccaaaccntn ccntgtgtcg anatngtnaa tcnggnccna 780
tnccanccnc atangaagcc ng 802

<210> 19
<211> 731
<212> DNA
<213> Homo sapiens

<220>
<221> misc_feature
<222> 2, 15, 25, 28, 37, 43, 46, 49, 54, 56, 76, 79, 82, 91, 112,
119, 122, 138, 141, 143, 146, 174, 193, 198, 199, 207, 208,
220, 221, 243, 249, 252, 260, 269, 270, 293, 296, 297, 298,
310, 315, 321, 325, 332, 334, 360, 363, 366, 374, 404
<223> n = A,T,C or G

<221> misc_feature
<222> 405, 412, 415, 458, 469, 494, 503, 508, 511, 518, 524, 528,
556, 589, 595, 597, 602, 603, 605, 606, 619, 641, 643, 648,
650, 655, 660, 666, 682, 684, 686, 689, 690, 691, 692, 695,
699, 706, 707, 708, 714, 721, 722, 728
<223> n = A,T,C or G

<400> 19
cnaagcttcc aggtnacggg ccgcnaancc tgacccnagg tancanaang cagncngcgg 60
gagcccaccg tcacgnggng gngtctttat nggagggggc ggagccacat cnctggacnt 120
cntgacccca actccccncc ncncantgca gtgatgagtg cagaactgaa ggtnacgtgg 180
caggaaccaa gancaaannc tgctccnntc caagtcggcn nagggggcgg ggctggccac 240
gcncatccnt cnagtgctgn aaagccccnn cctgtctact tgtttggaga acngcnnnga 300
catgcccagn gttanataac nggcngagag tnantttgcc tctcccttcc ggctgcgcan 360
cgngtntgct tagnggacat aacctgacta cttaactgaa cccnngaatc tnccncccct 420
ccactaagct cagaacaaaa aacttcgaca ccactcantt gtcacctgnc tgctcaagta 480
aagtgtaccc catncccaat gtntgctnga ngctctgncc tgcnttangt tcggtcctgg 540
gaagacctat caattnaagc tatgtttctg actgcctctt gctccctgna acaancnacc 600
cnncnntcca agggggggnc ggcccccaat ccccccaacc ntnaattnan tttanccccn 660
cccccnggcc cggcctttta cnancntcnn nnacngggna aaaccnnngc tttncccaac 720
nnaatccncc t 731

<210> 20
<211> 754
<212> DNA
<213> Homo sapiens

<220>

<221> misc_feature
<222> 38, 43, 71, 79, 80, 83, 92, 110, 114, 115, 122, 123, 135,
138, 139, 144, 147, 148, 149, 152, 155, 161, 163, 171, 178,
182, 184, 188, 192, 203, 206, 208, 219, 232, 245, 249, 251,
263, 269, 286, 288, 292, 297, 301, 302, 315, 336, 342
<223> n = A,T,C or G

<221> misc_feature
<222> 351, 358, 363, 364, 366, 376, 378, 388, 389, 405, 413, 423,
427, 443, 444, 445, 446, 452, 462, 465, 466, 475, 485, 489,
494, 496, 497, 527, 537, 546, 551, 552, 554, 561, 573, 585,
587, 616, 625, 628, 643, 648, 656, 658, 665, 668, 669
<223> n = A,T,C or G

<221> misc_feature
<222> 677, 680, 685, 690, 695, 698, 700, 709, 715, 726, 730, 735,
740, 749, 750
<223> n = A,T,C or G

<400> 20
tttttttttt tttttttttt taaaaacccc ctccattnaa tgnaaacttc cgaaattgtc 60
caacccccctc ntccaaatnn ccntttccgg gngggggttc caaacccaan ttannttttgg 120
annttaaatt aaatnttnnt tggnggnnna anccnaatgt nangaaagtt naacccanta 180
tnancttnaa tncctggaaa ccngtngntt ccaaaaatnt ttaaccctta antccctccg 240
aaatngttna nggaaaaccc aanttctcnt aaggttgttt gaaggntnaa tnaaaanccc 300
nnccaattgt ttttngccac gcctgaatta attggnttcc gntgttttcc nttaaaanaa 360
ggnnanccccc ggttantnaa tccccccnnc cccaattata ccganttttt ttngaattgg 420
gancccncgg gaattaacgg ggnnnntccc tnttgggggg cnggnncccc ccccntcggg 480
ggttngggnc aggncnnaat tgtttaaggg tccgaaaaat ccctccnaga aaaaaanctc 540
ccaggntgag nntngggttt nccccccccc canggcccct ctcgnanagt tggggtttgg 600
ggggcctggg attttntttc ccctnttncc tccccccccc ccngggganag aggttngngt 660
tttgntcnnc ggccccnccn aaganctttn ccganttnan ttaaatccnt gcctnggcga 720
agtccnttgn agggntaaan ggccccctnn cggg 754

<210> 21
<211> 755
<212> DNA
<213> Homo sapiens

<220>
<221> misc_feature
<222> 5, 17, 21, 22, 29, 34, 39, 41, 42, 43, 45, 49, 56, 61, 62,
65, 68, 69, 74, 76, 77, 80, 81, 87, 92, 95, 105, 107, 109,
112, 117, 121, 122, 125, 128, 136, 137, 144, 147, 150, 151,
158, 161, 168, 171, 177, 180, 191, 193, 196, 201, 202
<223> n = A,T,C or G

<221> misc_feature
<222> 203, 208, 211, 212, 213, 221, 224, 226, 232, 240, 241, 242,
244, 245, 248, 260, 262, 273, 275, 283, 299, 308, 313, 316,
321, 322, 326, 328, 338, 344, 345, 352, 354, 356, 367, 370,
371, 377, 384, 426, 430, 444, 453, 458, 464, 485, 491
<223> n = A,T,C or G

<221> misc_feature
<222> 493, 496, 498, 517, 523, 533, 536, 546, 555, 567, 568, 580,
581, 594, 596, 607, 624, 630, 634, 635, 656, 667, 669, 672,
677, 685, 689, 736
<223> n = A,T,C or G

<400> 21
atcancccat gaccccnaac nngggaccnc tcanccggnc nnncnaccnc cggccnatca 60
nngtnagnnc actncnnttn natcacnccc cnccnactac gcccncnanc cnacgcncta 120
nncanatncc actganngcg cgangtngan ngagaaanct nataccanag ncaccanacn 180
ccagctgtcc nanaangcct nnnatacngg nnnatccaat ntgnancctc cnaagtattn 240
nncnncanat gatttcctn anccgattac ccntnccccc tanccccctcc cccccaacna 300
cgaaggcnct ggnccnaagg nngcgncncc ccgctagntc cccnncaagt cncncnccta 360
aactcanccn nattacncgc ttcntgagta tcactccccg aatctcaccc tactcaactc 420

aaaaanatcn gatacaaaat aatncaagcc tgnttatnac actntgactg ggtctctatt 480
ttagnggtcc ntnaancntc ctaatacttc cagtctncct tcnccaattt ccnaanggct 540
ctttcngaca gcatnttttg gttcccnntt gggttcttan ngaattgccc ttcntngaac 600
gggctcntct tttccttcgg ttancctggn ttcnnccggc cagttattat ttcccntttt 660
aaattcntnc cntttanttt tggcnttcna aacccccggc cttgaaaacg gccccctggt 720
aaaaggttgt tttganaaaa tttttgtttt gttcc 755

<210> 22
<211> 849
<212> DNA
<213> Homo sapiens

<220>
<221> misc_feature
<222> 17, 22, 49, 56, 66, 70, 74, 84, 93, 94, 96, 108, 120, 127,
128, 129, 136, 146, 147, 151, 152, 163, 166, 173, 174, 175,
177, 180, 189, 191, 219, 221, 223, 240, 242, 243, 250, 255,
256, 259, 261, 270, 271, 276, 277, 279, 285, 299, 315
<223> n = A,T,C or G

<221> misc_feature
<222> 318, 320, 322, 326, 333, 337, 351, 352, 362, 366, 371, 374,
376, 389, 391, 392, 404, 406, 407, 416, 419, 421, 422, 428,
429, 430, 435, 449, 452, 457, 459, 464, 472, 487, 507, 511,
512, 526, 528, 531, 542, 544, 545, 548, 549, 555, 571
<223> n = A,T,C or G

<221> misc_feature
<222> 575, 578, 581, 585, 586, 587, 601, 607, 609, 611, 613, 616,
618, 620, 625, 628, 637, 638, 661, 665, 670, 673, 674, 678,
679, 681, 685, 687, 688, 693, 700, 704, 706, 715, 717, 723,
725, 728, 734, 736, 737, 745, 748, 749, 752, 773, 778
<223> n = A,T,C or G

<221> misc_feature
<222> 781, 787, 800, 806, 812, 814, 815, 820, 830, 831, 832, 841,
842, 845
<223> n = A,T,C or G

<400> 22
tttttttttt tttttangtg tngtcgtgca ggtagaggct tactacaant gtgaanacgt 60
acgctnggan taangcgacc cganttctag ganncnccct aaaatcanac tgtgaagatn 120
atcctgnnna cggaanggtc accggnngat nntgctaggg tgnccnctcc cannncnttn 180
cataactcng nggccctgcc caccaccttc ggcggcccng ngnccgggcc cgggtcattn 240
gnnttaaccn cactnngcna ncggtttccn nccccnncng acccnggcga tccggggtnc 300
tctgtcttcc cctgnagncn anaaantggg ccncggnccc ctttacccct nnacaagcca 360
cngccntcta nccncngccc cccctccant nngggggact gccnanngct ccgttnctng 420
nnaccccnnn gggtncctcg gttgtcgant cnaccgnang ccanggattc cnaaggaagg 480
tgcgttnttg gcccctaccc ttcgctncgg nncacccttc ccgacnanga nccgctcccg 540
cncnncgnng cctcncctcg caacacccgc nctcntcngt ncggnnnccc ccccacccgc 600
nccctcncnc ngncgnancn ctccnccncc gtctcannca ccaccccgcc ccgccaggcc 660
ntcanccacn ggnngacnng nagcncnntc gcnccgcgcn gcgncnccct cgccncngaa 720
ctncntcngg ccantnncgc tcaanccnna cnaaacgccg ctgcgcggcc cgnagcgncc 780
ncctccncga gtcctcccgn cttccnaccc angnnttccn cgaggacacn nnaccccgcc 840
nncangcgg 849

<210> 23
<211> 872
<212> DNA
<213> Homo sapiens

<220>
<221> misc_feature
<222> 22, 39, 67, 69, 77, 81, 88, 90, 94, 99, 113, 126, 128, 130,
134, 141, 152, 157, 160, 168, 169, 178, 181, 195, 213, 218,
228, 230, 236, 239, 243, 246, 254, 260, 261, 269, 270, 277,
280, 283, 291, 309, 310, 311, 312, 319, 322, 323, 335
<223> n = A,T,C or G

<221> misc_feature
<222> 341, 346, 349, 361, 363, 364, 367, 371, 377, 378, 388, 398,
400, 408, 416, 418, 420, 439, 440, 443, 445, 446, 451, 454,
456, 469, 470, 472, 473, 475, 485, 486, 487, 496, 510, 511,
513, 514, 529, 534, 543, 548, 553, 554, 555, 559, 572
<223> n = A,T,C or G

<221> misc_feature
<222> 577, 588, 603, 608, 613, 621, 628, 631, 635, 645, 646, 647,
653, 655, 656, 662, 664, 666, 668, 670, 675, 678, 688, 693,
694, 697, 698, 701, 711, 714, 717, 725, 727, 733, 736, 740,
742, 757, 758, 764, 768, 777, 781, 791, 793, 796, 798
<223> n = A,T,C or G

<221> misc_feature
<222> 809, 813, 818, 828, 832, 842, 846, 847, 853, 855, 860, 862
<223> n = A,T,C or G

<400> 23
gcgcaaacta tacttcgctc gnactcgtgc gcctcgctnc tcttttcctc cgcaaccatg 60
tctgacnanc ccgattnggc ngatatcnan aagntcganc agtccaaact gantaacaca 120
cacacncnan aganaaatcc nctgccttcc anagtanacn attgaacnng agaaccangc 180
nggcgaatcg taatnaggcg tgcgccgcca atntgtcncc gtttattntn ccagcntcnc 240
ctnccnaccc tacntcttcn nagctgtcnn acccctngtn cgnacccccc naggtcggga 300
tcgggtttnn nntgaccgng cnncccctcc ccccntccat nacganccnc ccgcaccacc 360
nanngcncgc nccccgnnct cttcgccncc ctgtcctntn cccctgtngc ctggcncngn 420
accgcattga ccctcgccnn ctncnngaaa ncgnanacgt ccgggttgnn annancgctg 480
tgggnnngcg tctgcnccgc gttccttccn ncnncttcca ccatcttcnt tacngggtct 540
ccncgccntc tcnnncacnc cctgggacgc tntcctntgc cccccttnac tccccccctt 600
cgncgtgncc cgnccccacc ntcatttnca nacgntcttc acaannncct ggntnnctcc 660
cnancngncn gtcanccnag ggaagggngg ggnnccnntg nttgacgttg nggngangtc 720
cgaanantcc tcnccntcan cnctacccct cgggcgnnct ctcngttncc aacttancaa 780
ntctcccccg ngngcncntc tcagcctcnc ccnccccnct ctctgcantg tnctctgctc 840
tnaccnntac gantnttcgn cnccctcttt cc 872

<210> 24
<211> 815
<212> DNA
<213> Homo sapiens

<220>
<221> misc_feature
<222> 26, 39, 46, 58, 61, 65, 87, 90, 93, 104, 107, 116, 117, 123,
125, 138, 142, 143, 157, 160, 163, 172, 173, 177, 180, 188,
190, 193, 196, 200, 205, 211, 214, 215, 216, 218, 219, 220,
221, 226, 227, 236, 238, 243, 254, 265, 267, 272
<223> n = A,T,C or G

<221> misc_feature
<222> 273, 275, 281, 300, 303, 305, 314, 316, 326, 328, 329, 332,
337, 338, 374, 375, 384, 397, 398, 402, 405, 406, 411, 413,
414, 415, 418, 420, 434, 437, 449, 463, 472, 475, 477, 487,
489, 492, 503, 513, 517, 526, 539, 557, 559, 562, 567
<223> n = A,T,C or G

<221> misc_feature
<222> 582, 584, 587, 596, 599, 604, 613, 614, 624, 633, 643, 655,
657, 671, 672, 675, 688, 692, 694, 695, 699, 702, 704, 710,
711, 715, 726, 730, 733, 738, 739, 742, 744, 745, 748, 753,
757, 759, 777, 781, 784, 787, 789, 793, 802, 807, 813
<223> n = A,T,C or G

<400> 24
gcatgcaagc ttgagtattc tatagngtca cctaaatanc ttggcntaat catggtcnta 60
nctgncttcc tgtgtcaaat gtatacnaan tanatatgaa tctnatntga caaganngta 120
tcntncatta gtaacaantg tnntgtccat cctgtcngan canattccca tnnattncgn 180
cgcattcncn gcncantatn taatngggaa ntcnnntnnn ncaccnncat ctatcntncc 240

gcnccctgac tggnagagat ggatnanttc tnntntgacc nacatgttca tcttggattn 300
aananccccc cgcngnccac cggttngnng cnagccnntc ccaagacctc ctgtggaggt 360
aacctgcgtc aganncatca aacntgggaa acccgcnncc angtnnaagt ngnnncanan 420
gatcccgtcc aggnttnacc atcccttcnc agcgccccct ttngtgcctt anagngnagc 480
gtgtccnanc cnctcaacat ganacgcgcc agnccanccg caattnggca caatgtcgnc 540
gaacccccta gggggantna tncaaanccc caggattgtc cncncangaa atcccncanc 600
cccnccctac ccnnctttgg gacngtgacc aantcccgga gtnccagtcc ggccngnctc 660
ccccaccggt nnccntgggg gggtgaanct cngnntcanc cngncgaggn ntcgnaagga 720
accggncctn ggncgaanng ancnntcnga agngccncnt cgtataaccc cccctcncca 780
nccnacngnt agntcccccc cngggtncgg aangg 815

<210> 25
<211> 775
<212> DNA
<213> Homo sapiens

<220>
<221> misc_feature
<222> 174, 195, 258, 370, 372, 373, 433, 442, 451, 472, 495, 497,
503, 505, 507, 529, 533, 536, 552, 556, 560, 561, 572, 573,
598, 622, 627, 644, 653, 657, 663, 667, 672, 673, 679, 681,
682, 684, 687, 702, 718, 719, 721, 726, 727, 732, 740
<223> n = A,T,C or G

<221> misc_feature
<222> 744, 748, 751, 755, 756, 760
<223> n = A,T,C or G

<400> 25
ccgagatgtc tcgctccgtg gccttagctg tgctcgcgct actctctctt tctggcctgg 60
aggctatcca gcgtactcca aagattcagg tttactcacg tcatccagca gagaatggaa 120
agtcaaattt cctgaattgc tatgtgtctg ggtttcatcc atccgacatt gaanttgact 180
tactgaagaa tggananaga attgaaaaag tggagcattc agacttgtct ttcagcaagg 240
actggtcttt ctatctcntg tactacactg aattcacccc cactgaaaaa gatgagtatg 300
cctgccgtgt gaaccatgtg actttgtcac agcccaagat agttaagtgg gatcgagaca 360
tgtaagcagn cnncatggaa gtttgaagat gccgcatttg gattggatga attccaaatt 420
ctgcttgctt gcnttttaat antgatatgc ntatacaccc taccctttat gnccccaaat 480
tgtaggggtt acatnantgt tcncntngga catgatcttc ctttataant ccnccnttcg 540
aattgcccgt cncccngttn ngaatgtttc cnnaaccacg gttggctccc ccaggtcncc 600
tcttacggaa gggcctgggc cnctttncaa ggttggggga accnaaaatt tcncttntgc 660
ccncccncca cnntcttgng nncncanttt ggaacccttc cnattcccct tggcctcnna 720
nccttnncta anaaaacttn aaancgtngc naaanntttn acttcccccc ttacc 775

<210> 26
<211> 820
<212> DNA
<213> Homo sapiens

<220>
<221> misc_feature
<222> 2, 7, 38, 65, 71, 78, 187, 209, 220, 232, 241, 276, 289,
291, 294, 301, 333, 338, 340, 373, 379, 384, 385, 386, 393,
407, 414, 423, 424, 452, 456, 469, 494, 501, 511, 524, 539,
540, 552, 564, 565, 591, 592, 598, 608, 611, 617, 619
<223> n = A,T,C or G

<221> misc_feature
<222> 627, 633, 634, 635, 645, 656, 657, 660, 663, 672, 673, 679,
682, 685, 689, 691, 694, 697, 701, 705, 709, 714, 724, 725,
731, 748, 754, 757, 758, 775, 788, 792, 798, 800, 808, 809,
810, 817
<223> n = A,T,C or G

<400> 26
anattantac agtgtaatct tttcccagag gtgtgtanag ggaacggggc ctagaggcat 60
cccanagata ncttatanca acagtgcttt gaccaagagc tgctgggcac atttcctgca 120
gaaaaggtgg cggtccccat cactcctcct ctcccatagc catcccagag gggtgagtag 180
ccatcangcc ttcggtggga gggagtcang gaaacaacan accacagagc anacagacca 240

ntgatgacca tgggcgggag cgagcctctt ccctgnaccg gggtggcana nganagccta 300
nctgaggggt cacactataa acgttaacga ccnagatnan cacctgcttc aagtgcaccc 360
ttcctacctg acnaccagng accnnnaact gcngcctggg gacagcnctg ggancagcta 420
acnnagcact cacctgcccc cccatggccg tncgcntccc tggtcctgnc aagggaagct 480
ccctgttgga attncgggga naccaaggga ncccccctcct ccanctgtga aggaaaaann 540
gatggaattt tnccccttccg gccnntcccc tcttccttta cacgcccccct nntactcntc 600
tccctctntt ntcctgncnc acttttnacc ccnnnatttc ccttnattga tcggannctn 660
ganattccac tnncgcctnc cntcnatcng naanacnaaa nactntctna cccnggggat 720
gggnncctcg ntcatcctct ctttttcnct accnccnntt ctttgcctct ccttngatca 780
tccaaccntc gntggccntn ccccccccnnn tcctttnccc 820

<210> 27
<211> 818
<212> DNA
<213> Homo sapiens

<220>
<221> misc_feature
<222> 267, 271, 276, 314, 328, 352, 355, 377, 382, 383, 399, 400,
407, 414, 418, 424, 428, 430, 434, 439, 445, 446, 460, 481,
492, 496, 497, 498, 499, 504, 509, 512, 515, 523, 526, 533,
539, 553, 558, 564, 565, 566, 574, 576, 577, 580, 588
<223> n = A,T,C or G

<221> misc_feature
<222> 589, 593, 594, 597, 599, 604, 606, 607, 612, 616, 618, 619,
622, 632, 633, 635, 636, 640, 641, 642, 646, 647, 651, 653,
659, 660, 662, 663, 670, 671, 674, 678, 681, 683, 686, 688,
690, 692, 693, 694, 695, 696, 698, 700, 701, 703, 705
<223> n = A,T,C or G

<221> misc_feature
<222> 706, 708, 710, 719, 722, 723, 724, 727, 729, 732, 733, 736,
737, 738, 739, 741, 743, 745, 746, 751, 752, 753, 754, 756,
757, 758, 759, 760, 761, 764, 765, 766, 768, 769, 770, 774,
776, 778, 784, 785, 791, 793, 806, 807, 817
<223> n = A,T,C or G

<400> 27
tctgggtgat ggcctcttcc tcctcaggga cctctgactg ctctgggcca aagaatctct 60
tgtttcttct ccgagcccca ggcagcggtg attcagccct gcccaacctg attctgatga 120
ctgcggatgc tgtgacggac ccaaggggca aatagggtcc cagggtccag ggaggggcgc 180
ctgctgagca cttccgcccc tcaccctgcc cagcccctgc catgagctct gggctgggtc 240
tccgcctcca gggttctgct cttccangca ngccancaag tggcgctggg ccacactggc 300
ttcttcctgc cccntccctg gctctgantc tctgtcttcc tgtcctgtgc angcnccttg 360
gatctcagtt tccctcnctc anngaactct gtttctgann tcttcantta actntgantt 420
tatnaccnan tggnctgtnc tgtcnnactt taatgggccn gaccggctaa tccctccctc 480
nctcccttcc anttcnnnna accngcttnc cntcntctcc ccntancccg ccngggaanc 540
ctcctttgcc ctnaccangg gccnnnaccg cccntnnctn ggggggcnng gtnnctncnc 600
ctgntnnccc cnctcncnnt tncctcgtcc cnncnncgcn nngcannttc ncngtcccnn 660
tnnctcttcn ngtntcgnaa ngntcncntn tnnnnngncn ngntnntncn tccctctcnc 720
cnnntgnang tnnttnnnnc ncngnncccc nnnncnnnnn nggnnntnnn tctncncngc 780
cccnnccccc ngnattaagg cctccnntct ccggccnc 818

<210> 28
<211> 731
<212> DNA
<213> Homo sapiens

<220>
<221> misc_feature
<222> 24, 43, 48, 72, 77, 78, 92, 101, 109, 110, 113, 125, 143,
144, 150, 154, 181, 184, 193, 205, 208, 213, 214, 216, 224,
238, 244, 261, 264, 270, 272, 277, 294, 308, 311, 320, 322,
336, 337, 345, 350, 362, 363, 364, 367, 378, 388, 389
<223> n = A,T,C or G

<221> misc_feature

<222> 403, 404, 406, 408, 414, 418, 420, 421, 422, 423, 427, 429,
436, 437, 438, 439, 444, 449, 450, 453, 459, 479, 480, 487,
491, 502, 516, 519, 526, 527, 534, 541, 547, 556, 557, 559,
562, 566, 567, 568, 570, 573, 579, 587, 591, 594, 602
<223> n = A,T,C or G

<221> misc_feature
<222> 614, 618, 625, 629, 630, 632, 636, 645, 646, 649, 655, 657,
665, 689, 694, 709, 710, 714, 721, 722, 723, 728, 729
<223> n = A,T,C or G

<400> 28
aggaagggcg gagggatatt gtangggatt gagggatagg agnataangg gggaggtgtg 60
tcccaacatg anggtgnngt tctcttttga angagggttg ngtttttann ccnggtgggt 120
gattnaaccc cattgtatgg agnnaaaggn tttnagggat ttttcggctc ttatcagtat 180
ntanattcct gtnaatcgga aaatnatntt tcnncnggaa aatnttgctc ccatccgnaa 240
attnctcccg ggtagtgcat nttngggggn cngccangtt tcccaggctg ctanaatcgt 300
actaaagntt naagtgggan tncaaatgaa aacctnncac agagnatccn tacccgactg 360
tnnnttncct tcgccctntg actctgcnng agcccaatac ccnngngnat gtcncccngn 420
nnngcgncnc tgaaannnnc tcgnggctnn gancatcang gggtttcgca tcaaaagcnn 480
cgtttcncat naaggcactt tngcctcatc caaccnctng ccctcnncca tttngccgtc 540
nggttcncct acgctnntng cncctnnntn ganattttnc ccgcctnggg naancctcct 600
gnaatgggta gggncttntc ttttnaccnn gnggtntact aatcnnctnc acgcntnctt 660
tctcnacccc cccccttttt caatcccanc ggcnaatggg gtctccccnn cgangggggg 720
nnncccannc c 731

<210> 29
<211> 822
<212> DNA
<213> Homo sapiens

<220>
<221> misc_feature
<222> 37, 39, 49, 52, 54, 67, 76, 83, 86, 88, 97, 101, 103, 104,
111, 116, 118, 119, 123, 125, 129, 136, 137, 142, 143, 144,
164, 177, 180, 182, 183, 187, 191, 200, 202, 204, 210, 218,
222, 224, 225, 227, 234, 243, 248, 250, 256, 258, 261
<223> n = A,T,C or G

<221> misc_feature
<222> 264, 281, 291, 292, 293, 298, 300, 306, 308, 312, 313, 314,
329, 331, 339, 343, 345, 376, 382, 383, 384, 392, 394, 401,
404, 407, 421, 437, 444, 451, 454, 467, 468, 491, 501, 510,
528, 530, 533, 540, 554, 557, 561, 573, 583, 585, 587
<223> n = A,T,C or G

<221> misc_feature
<222> 588, 599, 604, 610, 611, 620, 621, 624, 626, 634, 640, 647,
648, 663, 683, 692, 693, 694, 705, 716, 719, 723, 732, 734,
742, 744, 751, 754, 762, 764, 769, 772, 774, 775, 776, 783,
797, 800, 805, 811, 815
<223> n = A,T,C or G

<400> 29
actagtccag tgtggtggaa ttccattgtg ttggggncnc ttctatgant antnttagat 60
cgctcanacc tcacancctc ccnacnangc ctataangaa nannaataga nctgtncnnt 120
atntntacnc tcatanncct cnnnacccac tccctcttaa cccntactgt gcctatngcn 180
tnnctantct ntgccgcctn cnanccaccn gtgggccnac cncnngnatt ctcnatctcc 240
tcnccatntn gcctananta ngtncatacc ctatacctac nccaatgcta nnnctaancn 300
tccatnantt annntaacta ccactgacnt ngactttcnc atnanctcct aatttgaatc 360
tactctgact cccacngcct annnattagc ancntccccc nacnatntct caaccaaatc 420
ntcaacaacc tatctanctg ttcnccaacc nttncctccg atccccnnac aacccccctc 480
ccaaataccc nccacctgac ncctaacccn caccatcccg gcaagccnan ggncatttan 540
ccactggaat cacnatngga naaaaaaaac ccnaactctc tancncnnat ctccctaana 600
aatnctcctn naatttactn ncantnccat caancccacn tgaaacnnaa cccctgtttt 660
tanatccctt ctttcgaaaa ccnacccttt annncccaac ctttngggcc cccccnctnc 720
ccnaatgaag gncncccaat cnangaaacg nccntgaaaa ancnaggcna anannntccg 780
canatcctat cccttanttn ggggnccctt ncccngggcc cc 822

<210> 30
<211> 787
<212> DNA
<213> Homo sapiens

<220>
<221> misc_feature
<222> 179, 238, 275, 277, 314, 378, 384, 390, 398, 425, 444, 461,
467, 472, 493, 501, 503, 509, 511, 513, 520, 530, 555, 560,
561, 562, 567, 572, 610, 614, 626, 635, 638, 639, 657, 674,
679, 685, 693, 705, 710, 715, 729, 731, 735, 744, 748
<223> n = A,T,C or G

<221> misc_feature
<222> 751, 752, 753, 759, 761, 769, 773, 774, 775, 777
<223> n = A,T,C or G

<400> 30
cggccgcctg ctctggcaca tgcctcctga atggcatcaa aagtgatgga ctgcccattg 60
ctagagaaga ccttctctcc tactgtcatt atggagccct gcagactgag ggctcccctt 120
gtctgcagga tttgatgtct gaagtcgtgg agtgtggctt ggagctcctc atctacatna 180
gctggaagcc ctggagggcc tctctcgcca gcctccccct tctctccacg ctctccangg 240
acaccagggg ctccaggcag cccattattc ccagnangac atggtgtttc tccacgcgga 300
cccatggggc ctgnaaggcc agggtctcct ttgacaccat ctctcccgtc ctgcctggca 360
ggccgtggga tccactantt ctanaacggn cgccaccncg gtgggagctc cagcttttgt 420
tcccnttaat gaaggttaat tgcncgcttg gcgtaatcat nggtcanaac tntttcctgt 480
gtgaaattgt ttntcccctc ncnattccnc ncnacatacn aacccggaan cataaagtgt 540
taaagcctgg gggtngcctn nngaatnaac tnaactcaat taattgcgtt ggctcatggc 600
ccgctttccn ttcnggaaaa ctgtcntccc ctgcnttnnt gaatcggcca ccccccnggg 660
aaaagcggtt tgcnttttng ggggntcctt ccncttcccc cctcnctaan ccctncgcct 720
cggtcgttnc nggtngcggg gaangggnat nnnctcccnc naagggggng agnnngntat 780
ccccaaa 787

<210> 31
<211> 799
<212> DNA
<213> Homo sapiens

<220>
<221> misc_feature
<222> 224, 250, 252, 262, 267, 321, 324, 331, 333, 334, 340, 362,
366, 398, 403, 444, 447, 450, 470, 473, 477, 494, 513, 517,
529, 532, 541, 543, 547, 550, 557, 583, 587, 598, 600, 601,
606, 607, 609, 613, 627, 634, 650, 667, 676, 683, 684
<223> n = A,T,C or G

<221> misc_feature
<222> 685, 688, 690, 692, 695, 697, 706, 714, 717, 719, 723, 724,
725, 728, 746, 751, 753, 759, 765, 766, 767, 768, 769, 770,
771, 775, 776, 778, 793, 794, 797, 798
<223> n = A,T,C or G

<400> 31
tttttttttt tttttttggc gatgctactg tttaattgca ggaggtgggg gtgtgtgtac 60
catgtaccag ggctattaga agcaagaagg aaggagggag ggcagagcgc cctgctgagc 120
aacaaaggac tcctgcagcc ttctctgtct gtctcttggc gcaggcacat ggggaggcct 180
cccgcagggt gggggccacc agtccagggg tgggagcact acanggggtg ggagtgggtg 240
gtggctggtn cnaatggcct gncacanatc cctacgattc ttgacacctg gatttcacca 300
ggggaccttc tgttctccca nggnaacttc ntnnatctcn aaagaacaca actgtttctt 360
cngcanttct ggctgttcat ggaaagcaca ggtgtccnat ttnggctggg acttggtaca 420
tatggttccg gcccacctct cccntcnaan aagtaattca cccccccccn ccntctnttg 480
cctgggccct taantaccca caccggaact canttantta ttcatcttng gntgggcttg 540
ntnatcnccn cctgaangcg ccaagttgaa aggccacgcc gtncccnctc cccatagnan 600
nttttnncnt canctaatgc ccccccnggc aacnatccaa tccccccccn tgggggcccc 660
agcccanggc ccccgnctcg ggnnnccngn cncgnantcc ccaggntctc ccantcngnc 720
ccnnngcncc cccgcacgca gaacanaagg ntngagccnc cgcannnnnn nggtnncnac 780
ctcgcccccc ccnncgnng 799

<210> 32
<211> 789
<212> DNA
<213> Homo sapiens

<220>
<221> misc_feature
<222> 65, 68, 92, 103, 171, 195, 219, 238, 240, 260, 288, 301,
317, 324, 327, 336, 345, 358, 395, 417, 419, 421, 424, 436,
449, 468, 470, 483, 493, 494, 507, 510, 512, 528, 531, 536,
554, 560, 568, 579, 628, 631, 656, 657, 664, 665, 672
<223> n = A,T,C or G

<221> misc_feature
<222> 676, 703, 704, 711, 718, 721, 726, 727, 728, 741, 742, 744,
745, 749, 753, 757, 759, 773, 787
<223> n = A,T,C or G

<400> 32
tttttttttt tttttttttt tttttttttt tttttttttt tttttttttt tttttttttt 60
ttttnccnag ggcaggttta ttgacaacct cncgggacac aancaggctg gggacaggac 120
ggcaacaggc tccggcggcg gcggcggcgg ccctacctgc ggtaccaaat ntgcagcctc 180
cgctcccgct tgatnttcct ctgcagctgc aggatgccnt aaaacagggc ctcggccntn 240
ggtgggcacc ctgggatttn aatttccacg ggcacaatgc ggtcgcancc cctcaccacc 300
nattaggaat agtggtntta cccnccnccg ttggcncact ccccntggaa accacttntc 360
gcggctccgg catctggtct taaaccttgc aaacnctggg gccctctttt tggttantnt 420
nccngccaca atcatnactc agactggcnc gggctggccc caaaaaancn ccccaaaacc 480
ggnccatgtc ttnncggggt tgctgcnatn tncatcacct cccgggcnca ncaggncaac 540
ccaaaagttc ttgnggcccn caaaaaanct ccggggggnc ccagtttcaa caaagtcatc 600
ccccttggcc cccaaatcct ccccccgntt nctgggtttg ggaacccacg cctctnnctt 660
tggnnggcaa gntggntccc ccttcgggcc cccggtgggc ccnnctctaa ngaaaacncc 720
ntcctnnnca ccatcccccc nngnnacgnc tancaangna tccctttttt tanaaacggg 780
ccccccncg 789

<210> 33
<211> 793
<212> DNA
<213> Homo sapiens

<220>
<221> misc_feature
<222> 84, 179, 305, 328, 423, 500, 552, 563, 580, 584, 594, 601,
671, 682, 705, 714, 724, 749, 779, 789
<223> n = A,T,C or G

<400> 33
gacagaacat gttggatggt ggagcacctt tctatacgac ttacaggaca gcagatgggg 60
aattcatggc tgttggagca atanaacccc agttctacga gctgctgatc aaaggacttg 120
gactaaagtc tgatgaactt cccaatcaga tgagcatgga tgattggcca gaaatgaana 180
agaagtttgc agatgtattt gcaaagaaga cgaaggcaga gtggtgtcaa atctttgacg 240
gcacagatgc ctgtgtgact ccggttctga cttttgagga ggttgttcat catgatcaca 300
acaangaacg ggctcgtttt atcaccantg aggagcagga cgtgagcccc cgccctgcac 360
ctctgctgtt aaacacccca gccatccctt ctttcaaaag ggatccacta cttctagagc 420
ggncgccacc gcggtggagc tccagctttt gttcccttta gtgagggtta attgcgcgct 480
tggcgtaatc atggtcatan ctgtttcctg tgtgaaattg ttatccgctc acaattccac 540
acaacatacg anccggaagc atnaaatttt aaagcctggn ggtngcctaa tgantgaact 600
nactcacatt aattggcttt gcgctcactg cccgctttcc agtccggaaa acctgtcctt 660
gccagctgcc nttaatgaat cnggccaccc cccggggaaa aggcngtttg cttnttgggg 720
cgcncttccc gctttctcgc ttcctgaant ccttcccccc ggtctttcgg cttgcggcna 780
acggtatcna cct 793

<210> 34
<211> 756
<212> DNA
<213> Homo sapiens

<220>

<221> misc_feature
<222> 62, 76, 210, 270, 354, 356, 366, 372, 388, 397, 413, 445,
456, 463, 473, 549, 569, 573, 576, 586, 603, 605, 611, 639,
656, 673, 703, 706, 710, 725, 749, 755
<223> n = A,T,C or G

<400> 34
gccgcgaccg gcatgtacga gcaactcaag ggcgagtgga accgtaaaag ccccaatctt 60
ancaagtgcg gggaanagct gggtcgactc aagctagttc ttctggagct caacttcttg 120
ccaaccacag ggaccaagct gaccaaacag cagctaattc tggcccgtga catactggag 180
atcggggccc aatggagcat cctacgcaan gacatcccct ccttcgagcg ctacatggcc 240
cagctcaaat gctactactt tgattacaan gagcagctcc ccgagtcagc ctatatgcac 300
cagctcttgg gcctcaacct cctcttcctg ctgtcccaga accgggtggc tgantnccac 360
acgganttgg ancggctgcc tgcccaanga catacanacc aatgtctaca tcnaccacca 420
gtgtcctgga gcaatactga tgganggcag ctaccncaaa gtnttcctgg ccnagggtaa 480
catcccccgc cgagagctac accttcttca ttgacatcct gctcgacact atcagggatg 540
aaaatcgcng ggttgctcca gaaaggctnc aanaanatcc ttttcnctga aggcccccgg 600
atncnctagt nctagaatcg gcccgccatc gcggtgganc ctccaacctt tcgttnccct 660
ttactgaggg ttnattgccg cccttggcgt tatcatggtc acnccngttn cctgtgttga 720
aattnttaac cccccacaat tccacgccna cattng 756

<210> 35
<211> 834
<212> DNA
<213> Homo sapiens

<220>
<221> misc_feature
<222> 12, 16, 22, 57, 96, 132, 150, 157, 188, 217, 219, 236, 244,
249, 252, 306, 307, 310, 312, 315, 316, 318, 323, 338, 346,
424, 440, 456, 481, 482, 486, 493, 499, 504, 508, 509, 525,
526, 535, 558, 561, 587, 605, 625, 626, 639, 653, 656
<223> n = A,T,C or G

<221> misc_feature
<222> 659, 663, 665, 682, 690, 694, 696, 697, 700, 721, 724, 742,
747, 751, 758, 767, 784, 792, 805, 807, 810, 812, 819, 827,
829
<223> n = A,T,C or G

<400> 35
ggggatctct anatcnacct gnatgcatgg ttgtcggtgt ggtcgctgtc gatgaanatg 60
aacaggatct tgcccttgaa gctctcggct gctgtnttta agttgctcag tctgccgtca 120
tagtcagaca cnctcttggg caaaaaacan caggatntga gtcttgattt cacctccaat 180
aatcttcngg gctgtctgct cggtgaactc gatgacnang ggcagctggt tgtgtntgat 240
aaantccanc angttctcct tggtgacctc cccttcaaag ttgttccggc cttcatcaaa 300
cttctnnaan angannancc canctttgtc gagctggnat ttgganaaca cgtcactgtt 360
ggaaactgat cccaaatggt atgtcatcca tcgcctctgc tgcctgcaaa aaacttgctt 420
ggcncaaatc cgactccccn tccttgaaag aagccnatca cacccccctc cctggactcc 480
nncaangact ctnccgctnc cccntccnng cagggttggt ggcannccgg gcccntgcgc 540
ttcttcagcc agttcacnat nttcatcagc ccctctgcca gctgttntat tccttggggg 600
ggaanccgtc tctcccttcc tgaannaact ttgaccgtng gaatagccgc gcntcnccnt 660
acntnctggg ccgggttcaa antccctccn ttgncnntcn cctcggccca ttctggattt 720
nccnaacttt ttccttcccc cnccccncgg ngtttggntt tttcatnggg ccccaactct 780
gctnttggcc antcccctgg gggcntntan cnccccctnt ggtcccntng ggcc 834

<210> 36
<211> 814
<212> DNA
<213> Homo sapiens

<220>
<221> misc_feature
<222> 4, 13, 35, 49, 59, 60, 66, 102, 121, 211, 223, 263, 309,
333, 383, 401, 414, 422, 426, 441, 461, 487, 491, 495, 512,
514, 520, 528, 529, 553, 563, 566, 587, 616, 639, 653, 664,
673, 678, 680, 683, 690, 694, 701, 712, 727, 732, 750
<223> n = A,T,C or G

<221> misc_feature
<222> 751, 762, 770, 774, 777, 780, 783, 800, 801, 812, 814
<223> n = A,T,C or G

<400> 36
cggncgcttt ccngccgcgc cccgtttcca tgacnaaggc tcccttcang ttaaatacnn 60
cctagnaaac attaatgggt tgctctacta atacatcata cnaaccagta agcctgccca 120
naacgccaac tcaggccatt cctaccaaag gaagaaaggc tggtctctcc accccctgta 180
ggaaaggcct gccttgtaag acaccacaat ncggctgaat ctnaagtctt gtgttttact 240
aatggaaaaa aaaaataaac aanaggtttt gttctcatgg ctgcccaccg cagcctggca 300
ctaaaacanc ccagcgctca cttctgcttg ganaaatatt ctttgctctt ttggacatca 360
ggcttgatgg tatcactgcc acntttccac ccagctgggc ncccttcccc catntttgtc 420
antganctgg aaggcctgaa ncttagtctc caaaagtctc ngcccacaag accggccacc 480
aggggangtc ntttncagtg gatctgccaa anantacccn tatcatcnnt gaataaaaag 540
gcccctgaac ganatgcttc cancancctt taagacccat aatcctngaa ccatggtgcc 600
cttccggtct gatccnaaag gaatgttcct gggtcccant ccctcctttg ttncttacgt 660
tgtnttggac ccntgctngn atnacccaan tganatcccc ngaagcaccc tncccctggc 720
atttganttt cntaaattct ctgccctacn nctgaaagca cnattccctn ggcnccnaan 780
ggngaactca agaaggtctn ngaaaaacca cncn 814

<210> 37
<211> 760
<212> DNA
<213> Homo sapiens

<220>
<221> misc_feature
<222> 183, 186, 207, 225, 270, 273, 330, 362, 366, 368, 461, 468,
479, 508, 509, 510, 516, 528, 538, 540, 543, 546, 561, 566,
596, 617, 626, 632, 635, 675, 698, 706, 708, 716, 717, 728,
742, 753, 756
<223> n = A,T,C or G

<400> 37
gcatgctgct cttcctcaaa gttgttcttg ttgccataac aaccaccata ggtaaagcgg 60
gcgcagtgtt cgctgaaggg gttgtagtac cagcgcggga tgctctcctt gcagagtcct 120
gtgtctggca ggtccacgca atgccctttg tcactgggga aatggatgcg ctggagctcg 180
tcnaanccac tcgtgtattt ttcacangca gcctcctccg aagcntccgg gcagttgggg 240
gtgtcgtcac actccactaa actgtcgatn cancagccca ttgctgcagc ggaactgggt 300
gggctgacag gtgccagaac acactggatn ggcctttcca tggaagggcc tgggggaaat 360
cncctnancc caaactgcct ctcaaaggcc accttgcaca ccccgacagg ctagaaatgc 420
actcttcttc ccaaaggtag ttgttcttgt tgcccaagca ncctccanca aaccaaaanc 480
ttgcaaaatc tgctccgtgg gggtcatnnn taccanggtt ggggaaanaa acccggcngn 540
ganccncctt gtttgaatgc naaggnaata atcctcctgt cttgcttggg tggaanagca 600
caattgaact gttaacnttg ggccgngttc cnctngggtg gtctgaaact aatcaccgtc 660
actggaaaaa ggtangtgcc ttccttgaat tcccaaantt cccctngntt tgggtnnttt 720
ctcctctncc ctaaaaatcg tnttcccccc ccntanggcg 760

<210> 38
<211> 724
<212> DNA
<213> Homo sapiens

<220>
<221> misc_feature
<222> 66, 85, 86, 93, 94, 136, 149, 152, 156, 163, 166, 175, 196,
206, 210, 218, 222, 261, 265, 280, 290, 301, 317, 319, 322,
327, 332, 333, 335, 338, 341, 347, 349, 365, 370, 372, 374,
386, 394, 395, 396, 403, 440, 441, 449, 465, 478, 484
<223> n = A,T,C or G

<221> misc_feature
<222> 500, 501, 516, 517, 519, 521, 531, 539, 555, 556, 563, 568,
597, 604, 613, 618, 622, 630, 633, 636, 647, 654, 661, 663,
664, 684, 686, 700, 718
<223> n = A,T,C or G

<400> 38
tttttttttt tttttttttt tttttttttt tttttaaaaa ccccctccat tgaatgaaaa 60
cttccnaaat tgtccaaccc cctcnnccaa atnnccattt ccgggggggg gttccaaacc 120
caaattaatt ttgganttta aattaaatnt tnattngggg aanaanccaa atgtnaagaa 180
aatttaaccc attatnaact taaatncctn gaaacccntg gnttccaaaa atttttaacc 240
cttaaatccc tccgaaattg ntaanggaaa accaaattcn cctaaggctn tttgaaggtt 300
ngatttaaac ccccttnant tnttttnacc cnngnctnaa ntatttngnt tccggtgttt 360
tcctnttaan cntnggtaac tcccgntaat gaannnccct aanccaatta aaccgaattt 420
tttttgaatt ggaaattccn ngggaattna ccggggtttt tcccntttgg gggccatncc 480
cccnctttcg gggtttgggn ntaggttgaa tttttnnang ncccaaaaaa ncccccaana 540
aaaaaactcc caagnnttaa ttngaatntc ccccttccca ggccttttgg gaaaggnggg 600
tttntggggg ccngggantt cnttcccccn ttnccncccc ccccccnggt aaanggttat 660
ngnntttggt ttttgggccc cttnanggac cttccggatn gaaattaaat ccccgggncg 720
gccg 724

<210> 39
<211> 751
<212> DNA
<213> Homo sapiens

<220>
<221> misc_feature
<222> 226, 261, 321, 345, 348, 359, 380, 390, 406, 417, 428, 429,
432, 444, 447, 451, 461, 467, 469, 476, 495, 502, 513, 518,
519, 520, 533, 538, 540, 548, 550, 555, 558, 559, 569, 573,
575, 576, 582, 598, 600, 608, 612, 613, 615, 616, 618
<223> n = A,T,C or G

<221> misc_feature
<222> 619, 621, 625, 645, 646, 649, 662, 663, 669, 675, 676, 678,
680, 682, 695, 696, 705, 708, 709, 712, 713, 720, 721, 722,
723, 724, 726, 732, 735, 740, 741, 746, 750
<223> n = A,T,C or G

<400> 39
tttttttttt tttttctttg ctcacattta atttttattt tgattttttt taatgctgca 60
caacacaata tttatttcat ttgtttcttt tatttcattt tatttgtttg ctgctgctgt 120
tttatttatt tttactgaaa gtgagaggga acttttgtgg ccttttttcc tttttctgta 180
ggccgcctta agctttctaa atttggaaca tctaagcaag ctgaanggaa aagggggttt 240
cgcaaaatca ctcgggggaa nggaaaggtt gctttgttaa tcatgcccta tggtgggtga 300
ttaactgctt gtacaattac ntttcacttt taattaattg tgctnaangc tttaattana 360
cttgggggtt ccctccccan accaaccccn ctgacaaaaa gtgccngccc tcaaatnatg 420
tcccggcnnt cnttgaaaca cacngcngaa ngttctcatt ntccccncnc caggtnaaaa 480
tgaagggtta ccatntttaa cnccacctcc acntggcnnn gcctgaatcc tcnaaaancn 540
ccctcaancn aattnctnng ccccggtcnc gcntnngtcc cncccgggct ccgggaantn 600
cacccccnga anncnntnnc naacnaaatt ccgaaaatat tcccnntcnc tcaattcccc 660
cnnagactnt cctcnncnan cncaattttc ttttnntcac gaacncgnnc cnnaaaatgn 720
nnnncncctc cnctngtccn naatcnccan c 751

<210> 40
<211> 753
<212> DNA
<213> Homo sapiens

<220>
<221> misc_feature
<222> 260, 290, 310, 359, 388, 401, 404, 412, 422, 435, 436, 451,
472, 485, 497, 508, 510, 518, 521, 528, 544, 550, 558, 572,
575, 586, 604, 615, 620, 629, 631, 639, 642, 643, 649, 657,
658, 660, 666, 671, 682, 688, 690, 695, 699, 722, 732
<223> n = A,T,C or G

<221> misc_feature
<222> 738, 740, 750, 752
<223> n = A,T,C or G

<400> 40
gtggtatttt ctgtaagatc aggtgttcct ccctcgtagg tttagaggaa acaccctcat 60

agatgaaaac cccccgaga cagcagcact gcaactgcca agcagccggg gtaggagggg 120
cgccctatgc acagctgggc ccttgagaca gcagggcttc gatgtcaggc tcgatgtcaa 180
tggtctggaa gcggcggctg tacctgcgta ggggcacacc gtcagggccc accaggaact 240
tctcaaagtt ccaggcaacn tcgttgcgac acaccggaga ccaggtgatn agcttggggt 300
cggtcataan cgcggtggcg tcgtcgctgg gagctggcag ggcctcccgc aggaaggcna 360
ataaaaggtg cgcccccgca ccgttcanct cgcacttctc naanaccatg angttgggct 420
cnaacccacc accannccgg acttccttga nggaattccc aaatctcttc gntcttgggc 480
ttctnctgat gccctanctg gttgcccngn atgccaanca nccccaancc ccggggtcct 540
aaancacccn cctcctcntt tcatctgggt tnttntcccc ggaccntggt tcctctcaag 600
gganccccata tctcnaccan tactcaccnt ncccccccnt gnnacccanc cttctanngn 660
ttcccncccg ncctctggcc cntcaaanan gcttncacna cctgggtctg ccttcccccc 720
tnccctatct gnaccccncn tttgtctcan tnt 753

<210> 41
<211> 341
<212> DNA
<213> Homo sapiens

<400> 41
actatatcca tcacaacaga catgcttcat cccatagact tcttgacata gcttcaaatg 60
agtgaaccca tccttgattt atatacatat atgttctcag tattttggga gcctttccac 120
ttctttaaac cttgttcatt atgaacactg aaaataggaa tttgtgaaga gttaaaaagt 180
tatagcttgt ttacgtagta agtttttgaa gtctacattc aatccagaca cttagttgag 240
tgttaaactg tgatttttaa aaaatatcat ttgagaatat tctttcagag gtattttcat 300
ttttactttt tgattaattg tgttttatat attagggtag t 341

<210> 42
<211> 101
<212> DNA
<213> Homo sapiens

<400> 42
acttactgaa tttagttctg tgctcttcct tatttagtgt tgtatcataa atactttgat 60
gtttcaaaca ttctaaataa ataattttca gtggcttcat a 101

<210> 43
<211> 305
<212> DNA
<213> Homo sapiens

<400> 43
acatctttgt tacagtctaa gatgtgttct taaatcacca ttccttcctg gtcctcaccc 60
tccagggtgg tctcacactg taattagagc tattgaggag tctttacagc aaattaagat 120
tcagatgcct tgctaagtct agagttctag agttatgttt cagaaagtct aagaaaccca 180
cctcttgaga ggtcagtaaa gaggacttaa tatttcatat ctacaaaatg accacaggat 240
tggatacaga acgagagtta tcctggataa ctcagagctg agtacctgcc cgggggccgc 300
tcgaa 305

<210> 44
<211> 852
<212> DNA
<213> Homo sapiens

<220>
<221> misc_feature
<222> 574, 704, 782
<223> n = A,T,C or G

<400> 44
acataaatat cagagaaaag tagtctttga aatatttacg tccaggagtt ctttgtttct 60
gattatttgg tgtgtgtttt ggtttgtgtc caaagtattg gcagcttcag ttttcatttt 120
ctctccatcc tcgggcattc ttcccaaatt tatataccag tcttcgtcca tccacacgct 180
ccagaatttc tcttttgtag taatatctca tagctcggct gagcttttca taggtcatgc 240
tgctgttgtt cttcttttta ccccatagct gagccactgc ctctgatttc aagaacctga 300
agacgccctc agatcggtct tcccatttta ttaatcctgg gttcttgtct gggttcaaga 360
ggatgtcgcg gatgaattcc cataagtgag tccctctcgg gttgtgcttt ttggtgtggc 420
acttggcagg ggggtcttgc tcctttttca tatcaggtga ctctgcaaca ggaaggtgac 480
tggtggttgt catggagatc tgagcccggc agaaagtttt gctgtccaac aaatctactg 540

tgctaccata gttggtgtca tataaatagt tctngtcttt ccaggtgttc atgatggaag 600
gctcagtttg ttcagtcttg acaatgacat tgtgtgtgga ctggaacagg tcactactgc 660
actggccgtt ccacttcaga tgctgcaagt tgctgtagag gagntgcccc gccgtccctg 720
ccgcccgggt gaactcctgc aaactcatgc tgcaaaggtg ctcgccgttg atgtcgaact 780
cntggaaagg gatacaattg gcatccagct ggttggtgtc caggaggtga tggagccact 840
cccacacctg gt 852

<210> 45
<211> 234
<212> DNA
<213> Homo sapiens

<400> 45
acaacagacc cttgctcgct aacgacctca tgctcatcaa gttggacgaa tccgtgtccg 60
agtctgacac catccggagc atcagcattg cttcgcagtg ccctaccgcg ggaactcttt 120
gcctcgtttc tggctggggt ctgctggcga acggcagaat gcctaccgtg ctgcagtgcg 180
tgaacgtgtc ggtggtgtct gaggaggtct gcagtaagct ctatgacccg ctgt 234

<210> 46
<211> 590
<212> DNA
<213> Homo sapiens

<220>
<221> misc_feature
<222> 144, 184, 254, 258, 274, 310, 323, 351, 358, 380, 408
<223> n = A,T,C or G

<400> 46
actttttatt taaatgttta taaggcagat ctatgagaat gatagaaaac atggtgtgta 60
atttgatagc aatattttgg agattacaga gttttagtaa ttaccaatta cacagttaaa 120
aagaagataa tatattccaa gcanatacaa aatatctaat gaaagatcaa ggcaggaaaa 180
tgantataac taattgacaa tggaaaatca attttaatgt gaattgcaca ttatccttta 240
aaagctttca aaanaaanaa ttattgcagt ctanttaatt caaacagtgt taaatggtat 300
caggataaan aactgaaggg canaaagaat taattttcac ttcatgtaac ncacccanat 360
ttacaatggc ttaaatgcan ggaaaaagca gtggaagtag ggaagtantc aaggtctttc 420
tggtctctaa tctgccttac tctttgggtg tggctttgat cctctggaga cagctgccag 480
ggctcctgtt atatccacaa tcccagcagc aagatgaagg gatgaaaaag gacacatgct 540
gccttccttt gaggagactt catctcactg gccaacactc agtcacatgt 590

<210> 47
<211> 774
<212> DNA
<213> Homo sapiens

<220>
<221> misc_feature
<222> 29, 78, 156, 680, 716, 735
<223> n = A,T,C or G

<400> 47
acaagggggc ataatgaagg agtggggana gattttaaag aaggaaaaaa aacgaggccc 60
tgaacagaat tttcctgnac aacggggctt caaaataatt ttcttgggga ggttcaagac 120
gcttcactgc ttgaaactta aatggatgtg ggacanaatt ttctgtaatg accctgaggg 180
cattacagac gggactctgg gaggaaggat aaacagaaag gggacaaagg ctaatcccaa 240
aacatcaaag aaaggaaggt ggcgtcatac ctcccagcct acacagttct ccagggctct 300
cctcatccct ggaggacgac agtggaggaa caactgacca tgtccccagg ctcctgtgtg 360
ctggctcctg gtcttcagcc cccagctctg gaagcccacc ctctgctgat cctgcgtggc 420
ccacactcct tgaacacaca tccccaggtt atattcctgg acatggctga acctcctatt 480
cctacttccg agatgccttg ctccctgcag cctgtcaaaa tcccactcac cctccaaacc 540
acggcatggg aagcctttct gacttgcctg attactccag catcttggaa caatccctga 600
ttccccactc cttagaggca agatagggtg gttaagagta gggctggacc acttggagcc 660
aggctgctgg cttcaaattn tggctcattt acgagctatg ggaccttggg caagtnatct 720
tcacttctat gggcntcatt ttgttctacc tgcaaaatgg gggataataa tagt 774

<210> 48
<211> 124
<212> DNA

<213> Homo sapiens

<220>
<221> misc_feature
<222> 3, 67, 72
<223> n = A,T,C or G

<400> 48
canaaattga aattttataa aaaggcattt ttctcttata tccataaaat gatataattt 60
ttgcaantat anaaatgtgt cataaattat aatgttcctt aattacagct caacgcaact 120
tggt 124

<210> 49
<211> 147
<212> DNA
<213> Homo sapiens

<220>
<221> misc_feature
<222> 89, 95, 144
<223> n = A,T,C or G

<400> 49
gccgatgcta ctattttatt gcaggaggtg ggggtgtttt tattattctc tcaacagctt 60
tgtggctaca ggtggtgtct gactgcatna aaaanttttt tacgggtgat tgcaaaaatt 120
ttagggcacc catatcccaa gcantgt 147

<210> 50
<211> 107
<212> DNA
<213> Homo sapiens

<400> 50
acattaaatt aataaaagga ctgttggggt tctgctaaaa cacatggctt gatatattgc 60
atggtttgag gttaggagga gttaggcata tgttttggga gaggggt 107

<210> 51
<211> 204
<212> DNA
<213> Homo sapiens

<400> 51
gtcctaggaa gtctagggga cacacgactc tggggtcacg gggccgacac acttgcacgg 60
cgggaaggaa aggcagagaa gtgacaccgt cagggggaaa tgacagaaag gaaaatcaag 120
gccttgcaag gtcagaaagg ggactcaggg cttccaccac agccctgccc cacttggcca 180
cctccctttt gggaccagca atgt 204

<210> 52
<211> 491
<212> DNA
<213> Homo sapiens

<220>
<221> misc_feature
<222> 226, 244, 269, 283, 288, 382
<223> n = A,T,C or G

<400> 52
acaaagataa catttatctt ataacaaaaa tttgatagtt ttaaaggtta gtattgtgta 60
gggtattttc caaaagacta aagagataac tcaggtaaaa agttagaaat gtataaaaca 120
ccatcagaca ggtttttaaa aaacaacata ttacaaaatt agacaatcat ccttaaaaaa 180
aaaacttctt gtatcaattt cttttgttca aaatgactga cttaantatt tttaaatatt 240
tcanaaacac ttcctcaaaa attttcaana tggtagcttt canatgtncc ctcagtccca 300
atgttgctca gataaataaa tctcgtgaga acttaccacc caccacaagc tttctggggc 360
atgcaacagt gtcttttctt tnctttttct tttttttttt ttacaggcac agaaactcat 420
caattttatt tggataacaa agggtctcca aattatattg aaaaataaat ccaagttaat 480
atcactcttg t 491

<210> 53
<211> 484
<212> DNA
<213> Homo sapiens

<220>
<221> misc_feature
<222> 45, 50, 252, 272, 299, 309, 326, 376, 384, 418, 423, 429,
446, 471, 483
<223> n = A,T,C or G

<400> 53
acataattta gcagggctaa ttaccataag atgctattta ttaanaggtn tatgatctga 60
gtattaacag ttgctgaagt ttggtatttt tatgcagcat tttctttttg ctttgataac 120
actacagaac ccttaaggac actgaaaatt agtaagtaaa gttcagaaac attagctgct 180
caatcaaatc tctacataac actatagtaa ttaaaacgtt aaaaaaaagt gttgaaatct 240
gcactagtat anaccgctcc tgtcaggata anactgcttt ggaacagaaa gggaaaaanc 300
agctttgant ttctttgtgc tgatangagg aaaggctgaa ttaccttgtt gcctctccct 360
aatgattggc aggtcnggta aatnccaaaa catattccaa ctcaacactt cttttccncg 420
tancttgant ctgtgtattc caggancagg cggatggaat gggccagccc ncggatgttc 480
cant 484

<210> 54
<211> 151
<212> DNA
<213> Homo sapiens

<400> 54
actaaacctc gtgcttgtga actccataca gaaaacggtg ccatccctga acacggctgg 60
ccactgggta tactgctgac aaccgcaaca acaaaaacac aaatccttgg cactggctag 120
tctatgtcct ctcaagtgcc tttttgtttg t 151

<210> 55
<211> 91
<212> DNA
<213> Homo sapiens

<400> 55
acctggcttg tctccgggtg gttcccggcg ccccccacgg tccccagaac ggacactttc 60
gccctccagt ggatactcga gccaaagtgg t 91

<210> 56
<211> 133
<212> DNA
<213> Homo sapiens

<400> 56
ggcggatgtg cgttggttat atacaaatat gtcattttat gtaagggact tgagtatact 60
tggatttttg gtatctgtgg gttgggggga cggtccagga accaataccc catggatacc 120
aagggacaac tgt 133

<210> 57
<211> 147
<212> DNA
<213> Homo sapiens

<220>
<221> misc_feature
<222> 50, 53, 114, 119, 126, 137
<223> n = A,T,C or G

<400> 57
actctggaga acctgagccg ctgctccgcc tctgggatga ggtgatgcan gcngtggcgc 60
gactgggagc tgagcccttc cctttgcgcc tgcctcagag gattgttgcc gacntgcana 120
tctcantggg ctggatncat gcagggt 147

<210> 58
<211> 198

<212> DNA
<213> Homo sapiens

<220>
<221> misc_feature
<222> 17, 28
<223> n = A,T,C or G

<400> 58
acagggatat aggtttnaag ttattgtnat tgtaaaatac attgaatttt ctgtatactc 60
tgattacata catttatcct ttaaaaaaga tgtaaatctt aatttttatg ccatctatta 120
atttaccaat gagttacctt gtaaatgaga agtcatgata gcactgaatt ttaactagtt 180
ttgacttcta agtttggt 198

<210> 59
<211> 330
<212> DNA
<213> Homo sapiens

<400> 59 .
acaacaaatg ggttgtgagg aagtcttatc agcaaaactg gtgatggcta ctgaaaagat 60
ccattgaaaa ttatcattaa tgattttaaa tgacaagtta tcaaaaactc actcaatttt 120
cacctgtgct agcttgctaa aatgggagtt aactctagag caaatatagt atcttctgaa 180
tacagtcaat aaatgacaaa gccagggcct acaggtggtt tccagacttt ccagacccag 240
cagaaggaat ctattttatc acatggatct ccgtctgtgc tcaaaatacc taatgatatt 300
tttcgtcttt attggacttc tttgaagagt 330

<210> 60
<211> 175
<212> DNA
<213> Homo sapiens

<400> 60
accgtgggtg ccttctacat tcctgacggc tccttcacca acatctggtt ctacttcggc 60
gtcgtgggct ccttcctctt catcctcatc cagctggtgc tgctcatcga ctttgcgcac 120
tcctggaacc agcggtggct gggcaaggcc gaggagtgcg attcccgtgc ctggt 175

<210> 61
<211> 154
<212> DNA
<213> Homo sapiens

<400> 61
accccacttt tcctcctgtg agcagtctgg acttctcact gctacatgat gagggtgagt 60
ggttgttgct cttcaacagt atcctcccct ttccggatct gctgagccgg acagcagtgc 120
tggactgcac agccccgggg ctccacattg ctgt 154

<210> 62
<211> 30
<212> DNA
<213> Homo sapiens

<400> 62
cgctcgagcc ctatagtgag tcgtattaga 30

<210> 63
<211> 89
<212> DNA
<213> Homo sapiens

<400> 63
acaagtcatt tcagcaccct ttgctcttca aaactgacca tcttttatat ttaatgcttc 60
ctgtatgaat aaaaatggtt atgtcaagt 89

<210> 64
<211> 97
<212> DNA
<213> Homo sapiens

<400> 64
accggagtaa ctgagtcggg acgctgaatc tgaatccacc aataaataaa ggttctgcag 60
aatcagtgca tccaggattg gtccttggat ctggggt 97

<210> 65
<211> 377
<212> DNA
<213> Homo sapiens

<220>
<221> misc_feature
<222> 8, 11, 104, 141, 191, 204, 320, 346
<223> n = A,T,C or G

<400> 65
acaacaanaa ntcccttctt taggccactg atggaaacct ggaaccccct tttgatggca 60
gcatggcgtc ctaggccttg acacagcggc tggggtttgg gctntcccaa accgcacacc 120
ccaaccctgg tctacccaca nttctggcta tgggctgtct ctgccactga acatcagggt 180
tcggtcataa natgaaatcc caanggggac agaggtcagt agaggaagct caatgagaaa 240
ggtgctgttt gctcagccag aaaacagctg cctggcattc gccgctgaac tatgaacccg 300
tgggggtgaa ctacccccan gaggaatcat gcctgggcga tgcaanggtg ccaacaggag 360
gggcgggagg agcatgt 377

<210> 66
<211> 305
<212> DNA
<213> Homo sapiens

<400> 66
acgcctttcc ctcagaattc agggaagaga ctgtcgcctg ccttcctccg ttgttgcgtg 60
agaacccgtg tgccccttcc caccatatcc accctcgctc catctttgaa ctcaaacacg 120
aggaactaac tgcaccctgg tcctctcccc agtccccagt tcaccctcca tccctcacct 180
tcctccactc taagggatat caacactgcc cagcacaggg gccctgaatt tatgtggttt 240
ttatatattt tttaataaga tgcactttat gtcatttttt aataaagtct gaagaattac 300
tgttt 305

<210> 67
<211> 385
<212> DNA
<213> Homo sapiens

<400> 67
actacacaca ctccacttgc ccttgtgaga cactttgtcc cagcacttta ggaatgctga 60
ggtcggacca gccacatctc atgtgcaaga ttgcccagca gacatcaggt ctgagagttc 120
cccttttaaa aaaggggact tgcttaaaaa agaagtctag ccacgattgt gtagagcagc 180
tgtgctgtgc tggagattca cttttgagag agttctcctc tgagacctga tctttagagg 240
ctgggcagtc ttgcacatga gatggggctg gtctgatctc agcactcctt agtctgcttg 300
cctctcccag ggccccagcc tggccacacc tgcttacagg gcactctcag atgcccatac 360
catagtttct gtgctagtgg accgt 385

<210> 68
<211> 73
<212> DNA
<213> Homo sapiens

<400> 68
acttaaccag atatattttt accccagatg gggatattct ttgtaaaaaa tgaaaataaa 60
gtttttttaa tgg 73

<210> 69
<211> 536
<212> DNA
<213> Homo sapiens

<220>
<221> misc_feature
<222> 484, 520

<223> n = A,T,C or G

<400> 69
actagtccag tgtggtggaa ttccattgtg ttgggggctc tcaccctcct ctcctgcagc 60
tccagctttg tgctctgcct ctgaggagac catggcccag catctgagta ccctgctgct 120
cctgctggcc accctagctg tggccctggc ctggagcccc aaggaggagg ataggataat 180
cccgggtggc atctataacg cagacctcaa tgatgagtgg gtacagcgtg cccttcactt 240
cgccatcagc gagtataaca aggccaccaa agatgactac tacagacgtc cgctgcgggt 300
actaagagcc aggcaacaga ccgttggggg ggtgaattac ttcttcgacg tagaggtggg 360
ccgaaccata tgtaccaagt cccagcccaa cttggacacc tgtgccttcc atgaacagcc 420
agaactgcag aagaaacagt tgtgctcttt cgagatctac gaagttccct ggggagaaca 480
gaangtccct gggtgaaatc caggtgtcaa gaaatcctan ggatctgttg ccaggc 536

<210> 70
<211> 477
<212> DNA
<213> Homo sapiens

<400> 70
   atgaccccta acaggggccc tctcagccct cctaatgacc tccggcctag ccatgtgatt 60
   tcacttccac tccataacgc tcctcatact aggcctacta accaacacac taaccatata 120
   ccaatgatgg cgcgatgtaa cacgagaaag cacataccaa ggccaccaca caccacctgt 180
   ccaaaaaggc cttcgatacg ggataatcct atttattacc tcagaagttt ttttcttcgc 240
   agggattttt ctgagccttt taccactcca gcctagcccc taccccccaa ctaggagggc 300
   actggccccc aacaggcatc accccgctaa atcccctaga agtcccactc ctaaacacat 360
   ccgtattact cgcatcagga gtatcaatca cctgagctca ccatagtcta atagaaaaca 420
   accgaaacca aattattcaa agcactgctt attacaattt tactgggtct ctatttt 477

<210> 71
<211> 533
<212> DNA
<213> Homo sapiens

<220>
<221> misc_feature
<222> 439
<223> n = A,T,C or G

<400> 71
   agagctatag gtacagtgtg atctcagctt tgcaaacaca ttttctacat agatagtact 60
   aggtattaat agatatgtaa agaaagaaat cacaccatta ataatggtaa gattggttta 120
   tgtgatttta gtggtatttt tggcaccctt atatatgttt tccaaacttt cagcagtgat 180
   attatttcca taacttaaaa agtgagtttg aaaaagaaaa tctccagcaa gcatctcatt 240
   taaataaagg tttgtcatct ttaaaaatac agcaatatgt gactttttaa aaaagctgtc 300
   aaataggtgt gaccctacta ataattatta gaaatacatt taaaaacatc gagtacctca 360
   agtcagtttg ccttgaaaaa tatcaaatat aactcttaga gaaatgtaca taaaagaatg 420
   cttcgtaatt ttggagtang aggttccctc ctcaattttg tatttttaaa aagtacatgg 480
   taaaaaaaaa aattcacaac agtatataag gctgtaaaat gaagaattct gcc 533

<210> 72
<211> 511
<212> DNA
<213> Homo sapiens

<220>
<221> misc_feature
<222> 34, 42, 145, 190, 205, 237, 279, 358, 439, 441, 464, 479,
501, 505
<223> n = A,T,C or G

<400> 72
tattacggaa aaacacacca cataattcaa ctancaaaga anactgcttc agggcgtgta 60
aaatgaaagg cttccaggca gttatctgat taaagaacac taaaagaggg acaaggctaa 120
aagccgcagg atgtctacac tatancaggc gctatttggg ttggctggag gagctgtgga 180
aaacatggan agattggtgc tgganatcgc cgtggctatt cctcattgtt attacanagt 240
gaggttctct gtgtgcccac tggtttgaaa accgttctnc aataatgata gaatagtaca 300
cacatgagaa ctgaaatggc ccaaacccag aaagaaagcc caactagatc ctcagaanac 360
gcttctaggg acaataaccg atgaagaaaa gatggcctcc ttgtgccccc gtctgttatg 420

atttctctcc attgcagcna naaacccgtt cttctaagca aacncaggtg atgatggcna 480
aaatacaccc cctcttgaag naccnggagg a 511

<210> 73
<211> 499
<212> DNA
<213> Homo sapiens

<220>
<221> misc_feature
<222> 362, 412, 445, 493
<223> n = A,T,C or G

<400> 73
cagtgccagc actggtgcca gtaccagtac caataacagt gccagtgcca gtgccagcac 60
cagtggtggc ttcagtgctg gtgccagcct gaccgccact ctcacatttg ggctcttcgc 120
tggccttggt ggagctggtg ccagcaccag tggcagctct ggtgcctgtg gtttctccta 180
caagtgagat tttagatatt gttaatcctg ccagtctttc tcttcaagcc agggtgcatc 240
ctcagaaacc tactcaacac agcactctag gcagccacta tcaatcaatt gaagttgaca 300
ctctgcatta aatctatttg ccatttctga aaaaaaaaaa aaaaaaaggg cggccgctcg 360
antctagagg gcccgtttaa acccgctgat cagcctcgac tgtgccttct anttgccagc 420
catctgttgt ttgcccctcc cccgntgcct tccttgaccc tggaaagtgc cactcccact 480
gtcctttcct aantaaaat 499

<210> 74
<211> 537
<212> DNA
<213> Homo sapiens

<220>
<221> misc_feature
<222> 317, 430
<223> n = A,T,C or G

<400> 74
tttcatagga gaacacactg aggagatact tgaagaattt ggattcagcc gcgaagagat 60
ttatcagctt aactcagata aaatcattga aagtaataag gtaaaagcta gtctctaact 120
tccaggccca cggctcaagt gaatttgaat actgcattta cagtgtagag taacacataa 180
cattgtatgc atggaaacat ggaggaacag tattacagtg tcctaccact ctaatcaaga 240
aaagaattac agactctgat tctacagtga tgattgaatt ctaaaaatgg taatcattag 300
ggcttttgat ttataanact ttgggtactt atactaaatt atggtagtta tactgccttc 360
cagtttgctt gatatatttg ttgatattaa gattcttgac ttatattttg aatgggttct 420
actgaaaaan gaatgatata ttcttgaaga catcgatata catttattta cactcttgat 480
tctacaatgt agaaaatgaa ggaaatgccc caaattgtat ggtgataaaa gtcccgt 537

<210> 75
<211> 467
<212> DNA
<213> Homo sapiens

<220>
<221> misc_feature
<222> 5, 158, 278, 328, 337, 409, 450, 467
<223> n = A,T,C or G

<400> 75
caaanacaat tgttcaaaag atgcaaatga tacactactg ctgcagctca caaacacctc 60
tgcatattac acgtacctcc tcctgctcct caagtagtgt ggtctatttt gccatcatca 120
cctgctgtct gcttagaaga acggctttct gctgcaangg agagaaatca taacagacgg 180
tggcacaagg aggccatctt ttcctcatcg gttattgtcc ctagaagcgt cttctgagga 240
tctagttggg ctttctttct gggtttgggc catttcantt ctcatgtgtg tactattcta 300
tcattattgt ataacggttt tcaaaccngt gggcacncag agaacctcac tctgtaataa 360
caatgaggaa tagccacggt gatctccagc accaaatctc tccatgttnt tccagagctc 420
ctccagccaa cccaaatagc cgctgctatn gtgtagaaca tccctgn 467

<210> 76
<211> 400
<212> DNA

<213> Homo sapiens

<220>
<221> misc_feature
<222> 359, 363, 375, 390
<223> n = A,T,C or G

<400> 76
aagctgacag cattcgggcc gagatgtctc gctccgtggc cttagctgtg ctcgcgctac 60
tctctctttc tggcctggag gctatccagc gtactccaaa gattcaggtt tactcacgtc 120
atccagcaga gaatggaaag tcaaatttcc tgaattgcta tgtgtctggg tttcatccat 180
ccgacattga agttgactta ctgaagaatg gagagagaat tgaaaaagtg gagcattcag 240
acttgtcttt cagcaaggac tggtctttct atctcttgta ctacactgaa ttcaccccca 300
ctgaaaaaga tgagtatgcc tgccgtgtga accatgtgac tttgtcacag cccaagatng 360
ttnagtggga tcganacatg taagcagcan catgggaggt 400

<210> 77
<211> 248
<212> DNA
<213> Homo sapiens

<400> 77
ctggagtgcc ttggtgtttc aagcccctgc aggaagcaga atgcaccttc tgaggcacct 60
ccagctgccc cggcggggga tgcgaggctc ggagcaccct tgcccggctg tgattgctgc 120
caggcactgt tcatctcagc ttttctgtcc ctttgctccc ggcaagcgct tctgctgaaa 180
gttcatatct ggagcctgat gtcttaacga ataaaggtcc catgctccac ccgaaaaaaa 240
aaaaaaaa 248

<210> 78
<211> 201
<212> DNA
<213> Homo sapiens

<400> 78
actagtccag tgtggtggaa ttccattgtg ttgggcccaa cacaatggct acctttaaca 60
tcacccagac cccgccctgc ccgtgcccca cgctgctgct aacgacagta tgatgcttac 120
tctgctactc ggaaactatt tttatgtaat taatgtatgc tttcttgttt ataaatgcct 180
gatttaaaaa aaaaaaaaaa a 201

<210> 79
<211> 552
<212> DNA
<213> Homo sapiens

<220>
<221> misc_feature
<222> 398, 402, 452, 462, 474, 476, 482, 510, 514, 523
<223> n = A,T,C or G

<400> 79
tccttttgtt aggtttttga gacaacccta gacctaaact gtgtcacaga cttctgaatg 60
tttaggcagt gctagtaatt tcctcgtaat gattctgtta ttactttcct attctttatt 120
cctctttctt ctgaagatta atgaagttga aaattgaggt ggataaatac aaaaaggtag 180
tgtgatagta taagtatcta agtgcagatg aaagtgtgtt atatatatcc attcaaaatt 240
atgcaagtta gtaattactc agggttaact aaattacttt aatatgctgt tgaacctact 300
ctgttccttg gctagaaaaa attataaaca ggactttgtt agtttgggaa gccaaattga 360
taatattcta tgttctaaaa gttgggctat acataaanta tnaagaaata tggaatttta 420
ttcccaggaa tatggggttc atttatgaat antacccggg anagaagttt tgantnaaac 480
cngttttggt taatacgtta atatgtcctn aatnaacaag gcntgactta tttccaaaaa 540
aaaaaaaaaa aa 552

<210> 80
<211> 476
<212> DNA
<213> Homo sapiens

<220>
<221> misc_feature

<222> 356, 358, 413, 449
<223> n = A,T,C or G

<400> 80
acagggattt gagatgctaa ggccccagag atcgtttgat ccaaccctct tattttcaga 60
ggggaaaatg gggcctagaa gttacagagc atctagctgg tgcgctggca cccctggcct 120
cacacagact cccgagtagc tgggactaca ggcacacagt cactgaagca ggccctgttt 180
gcaattcacg ttgccacctc caacttaaac attcttcata tgtgatgtcc ttagtcacta 240
aggttaaact ttcccaccca gaaaaggcaa cttagataaa atcttagagt actttcatac 300
tcttctaagt cctcttccag cctcactttg agtcctcctt gggggttgat aggaantntc 360
tcttggcttt ctcaataaaa tctctatcca tctcatgttt aatttggtac gcntaaaaat 420
gctgaaaaaa ttaaaatgtt ctggtttcnc tttaaaaaaa aaaaaaaaaa aaaaaa 476

<210> 81
<211> 232
<212> DNA
<213> Homo sapiens

<220>
<221> misc_feature
<222> 17, 20, 27
<223> n = A,T,C or G

<400> 81
tttttttttg tatgccntcn ctgtggngtt attgttgctg ccaccctgga ggagcccagt 60
ttcttctgta tctttctttt ctgggggatc ttcctggctc tgcccctcca ttcccagcct 120
ctcatcccca tcttgcactt ttgctagggt tggaggcgct ttcctggtag cccctcagag 180
actcagtcag cgggaataag tcctaggggt gggggtgtgg gcaagccggc ct 232

<210> 82
<211> 383
<212> DNA
<213> Homo sapiens

<220>
<221> misc_feature
<222> 309
<223> n = A,T,C or G

<400> 82
aggcgggagc agaagctaaa gccaaagccc aagaagagtg gcagtgccag cactggtgcc 60
agtaccagta ccaataacat gccagtgcca gtgccagcac cagtggtggc ttcagtgctg 120
gtgccagcct gaccgccact ctcacatttg ggctcttcgc tggccttggt ggagctggtg 180
ccagcaccag tggcagctct ggtgcctgtg gtttctccta caagtgagat tttagatatt 240
gttaatcctg ccagtctttc tcttcaagcc agggtgcatc ctcagaaacc tactcaacac 300
agcactctng gcagccacta tcaatcaatt gaagttgaca ctctgcatta aatctatttg 360
ccatttcaaa aaaaaaaaaa aaa 383

<210> 83
<211> 494
<212> DNA
<213> Homo sapiens

<220>
<221> misc_feature
<222> 337, 398, 415, 425, 441, 451
<223> n = A,T,C or G

<400> 83
accgaattgg gaccgctggc ttataagcga tcatgtcctc cagtattacc tcaacgagca 60
gggagatcga gtctatacgc tgaagaaatt tgacccgatg ggacaacaga cctgctcagc 120
ccatcctgct cggttctccc cagatgacaa atactctcga caccgaatca ccatcaagaa 180
acgcttcaag gtgctcatga cccagcaacc gcgccctgtc ctctgagggt ccttaaactg 240
atgtcttttc tgccacctgt tacccctcgg agactccgta accaaactct tcggactgtg 300
agccctgatg cctttttgcc agccatactc tttggcntcc agtctctcgt ggcgattgat 360
tatgcttgtg tgaggcaatc atggtggcat cacccatnaa gggaacacat ttganttttt 420
tttcncatat tttaaattac naccagaata nttcagaata aatgaattga aaaactctta 480
aaaaaaaaaa aaaa 494

<210> 84
<211> 380
<212> DNA
<213> Homo sapiens

<220>
<221> misc_feature
<222> 28, 342, 367
<223> n = A,T,C or G

<400> 84
gctggtagcc tatggcgtgg ccacggangg gctcctgagg cacgggacag tgacttccca 60
agtatcctgc gccgcgtctt ctaccgtccc tacctgcaga tcttcgggca gattccccag 120
gaggacatgg acgtggccct catggagcac agcaactgct cgtcggagcc cggcttctgg 180
gcacaccctc ctggggccca ggcgggcacc tgcgtctccc agtatgccaa ctggctggtg 240
gtgctgctcc tcgtcatctt cctgctcgtg gccaacatcc tgctggtcac ttgctcattg 300
ccatgttcag ttacacattc ggcaaagtac agggcaacag cnatctctac tgggaaggcc 360
agcgttnccg cctcatccgg 380

<210> 85
<211> 481
<212> DNA
<213> Homo sapiens

<220>
<221> misc_feature
<222> 62, 146, 149, 311, 352, 381, 397, 416, 417, 444, 459, 467,
468, 473, 475
<223> n = A,T,C or G

<400> 85
gagttagctc ctccacaacc ttgatgaggt cgtctgcagt ggcctctcgc ttcataccgc 60
tnccatcgtc atactgtagg tttgccacca cctcctgcat cttggggcgg ctaatatcca 120
ggaaactctc aatcaagtca ccgtcnatna aacctgtggc tggttctgtc ttccgctcgg 180
tgtgaaagga tctccagaag gagtgctcga tcttccccac acttttgatg actttattga 240
gtcgattctg catgtccagc aggaggttgt accagctctc tgacagtgag gtcaccagcc 300
ctatcatgcc nttgaacgtg ccgaagaaca ccgagccttg tgtggggggt gnagtctcac 360
ccagattctg cattaccaga nagccgtggc aaaaganatt gacaactcgc ccaggnngaa 420
aaagaacacc tcctggaagt gctngccgct cctcgtccnt tggtggnngc gcntnccttt 480
t 481

<210> 86
<211> 472
<212> DNA
<213> Homo sapiens

<220>
<221> misc_feature
<222> 40, 78, 273, 365, 377, 425, 428
<223> n = A,T,C or G

<400> 86
aacatcttcc tgtataatgc tgtgtaatat cgatccgatn ttgtctgctg agaattcatt 60
acttggaaaa gcaacttnaa gcctggacac tggtattaaa attcacaata tgcaacactt 120
taaacagtgt gtcaatctgc tcccttactt tgtcatcacc agtctgggaa taagggtatg 180
ccctattcac acctgttaaa agggcgctaa gcatttttga ttcaacatct ttttttttga 240
cacaagtccg aaaaaagcaa aagtaaacag ttnttaattt gttagccaat tcactttctt 300
catgggacag agccatttga tttaaaaagc aaattgcata atattgagct ttgggagctg 360
atatntgagc ggaagantag cctttctact tcaccagaca caactccttt catattggga 420
tgttnacnaa agttatgtct cttacagatg ggatgctttt gtggcaattc tg 472

<210> 87
<211> 413
<212> DNA
<213> Homo sapiens

<220>

<221> misc_feature
<222> 17, 219, 238, 270, 279, 318, 329, 340, 355, 374, 386, 388,
389, 398
<223> n = A,T,C or G

<400> 87
agaaaccagt atctctnaaa acaacctctc ataccttgtg gacctaattt tgtgtgcgtg 60
tgtgtgtgcg cgcatattat atagacaggc acatcttttt tacttttgta aaagcttatg 120
cctctttggt atctatatct gtgaaagttt taatgatctg ccataatgtc ttggggacct 180
ttgtcttctg tgtaaatggt actagagaaa acacctatnt tatgagtcaa tctagttngt 240
tttattcgac atgaaggaaa tttccagatn acaacactna caaactctcc cttgactagg 300
ggggacaaag aaaagcanaa ctgaacatna gaaacaattn cctggtgaga aattncataa 360
acagaaattg ggtngtatat tgaaananng catcattnaa acgttttttt ttt 413

<210> 88
<211> 448
<212> DNA
<213> Homo sapiens

<220>
<221> misc_feature
<222> 70, 246, 248, 266, 276, 280, 306, 319, 327, 358, 359, 373,
382, 391, 424, 427, 433
<223> n = A,T,C or G

<400> 88
cgcagcgggt cctctctatc tagctccagc ctctcgcctg ccccactccc cgcgtcccgc 60
gtcctagccn accatggccg ggcccctgcg cgccccgctg ctcctgctgg ccatcctggc 120
cgtggccctg gccgtgagcc ccgcggccgg ctccagtccc ggcaagccgc cgcgcctggt 180
gggaggccca tggaccccgc gtggaagaag aaggtgtgcg gcgtgcactg gactttgccg 240
tcggcnanta caacaaaccc gcaacnactt ttaccnagcn cgcgctgcag gttgtgccgc 300
cccaancaaa ttgttactng gggtaantaa ttcttggaag ttgaacctgg gccaaacnng 360
tttaccagaa ccnagccaat tngaacaatt ncccctccat aacagcccct tttaaaaagg 420
gaancantcc tgntcttttc caaatttt 448

<210> 89
<211> 463
<212> DNA
<213> Homo sapiens

<220>
<221> misc_feature
<222> 195, 196, 214, 244, 250, 263, 265, 279, 316, 317, 330, 334,
354, 360, 376, 377, 386, 397, 409, 412, 419, 420, 426, 427,
429, 432, 438, 440, 451, 455, 459
<223> n = A,T,C or G

<400> 89
gaattttgtg cactggccac tgtgatggaa ccattgggcc aggatgcttt gagtttatca 60
gtagtgattc tgccaaagtt ggtgttgtaa catgagtatg taaaatgtca aaaaattagc 120
agaggtctag gtctgcatat cagcagacag tttgtccgtg tattttgtag ccttgaagtt 180
ctcagtgaca agttnnttct gatgcgaagt tctnattcca gtgttttagt cctttgcatc 240
tttnatgttn agacttgcct ctntnaaatt gcttttgtnt tctgcaggta ctatctgtgg 300
tttaacaaaa tagaannact tctctgcttn gaanatttga atatcttaca tctnaaaatn 360
aattctctcc ccatannaaa acccangccc ttggganaat ttgaaaaang gntccttcnn 420
aattcnnana anttcagntn tcatacaaca naacngganc ccc 463

<210> 90
<211> 400
<212> DNA
<213> Homo sapiens

<220>
<221> misc_feature
<222> 16, 19, 37, 83, 85, 292, 340
<223> n = A,T,C or G

<400> 90

agggattgaa ggtctnttnt actgtcggac tgttcancca ccaactctac aagttgctgt 60
cttccactca ctgtctgtaa gcntnttaac ccagactgta tcttcataaa tagaacaaat 120
tcttcaccag tcacatcttc taggaccttt ttggattcag ttagtataag ctcttccact 180
tcctttgtta agacttcatc tggtaaagtc ttaagttttg tagaaaggaa tttaattgct 240
cgttctctaa caatgtcctc tccttgaagt atttggctga acaacccacc tnaagtccct 300
ttgtgcatcc attttaaata tacttaatag ggcattggtn cactaggtta aattctgcaa 360
gagtcatctg tctgcaaaag ttgcgttagt atatctgcca 400

<210> 91
<211> 480
<212> DNA
<213> Homo sapiens

<220>
<221> misc_feature
<222> 44, 56, 93, 171, 172, 201, 421, 465
<223> n = A,T,C or G

<400> 91
gagctcggat ccaataatct ttgtctgagg gcagcacaca tatncagtgc catggnaact 60
ggtctacccc acatgggagc agcatgccgt agntatataa ggtcattccc tgagtcagac 120
atgcctcttt gactaccgtg tgccagtgct ggtgattctc acacacctcc nnccgctctt 180
tgtggaaaaa ctggcacttg nctggaacta gcaagacatc acttacaaat tcacccacga 240
gacacttgaa aggtgtaaca aagcgactct tgcattgctt tttgtccctc cggcaccagt 300
tgtcaatact aacccgctgg tttgcctcca tcacatttgt gatctgtagc tctggataca 360
tctcctgaca gtactgaaga acttcttctt ttgtttcaaa agcaactctt ggtgcctgtt 420
ngatcaggtt cccatttccc agtccgaatg ttcacatggc atatnttact tcccacaaaa 480

<210> 92
<211> 477
<212> DNA
<213> Homo sapiens

<220>
<221> misc_feature
<222> 11, 184, 283, 346, 407, 430, 459
<223> n = A,T,C or G

<400> 92
atacagccca natcccacca cgaagatgcg cttgttgact gagaacctga tgcggtcact 60
ggtcccgctg tagccccagc gactctccac ctgctggaag cggttgatgc tgcactcctt 120
cccacgcagg cagcagcggg gccggtcaat gaactccact cgtggcttgg ggttgacggt 180
taantgcagg aagaggctga ccacctcgcg gtccaccagg atgcccgact gtgcgggacc 240
tgcagcgaaa ctcctcgatg gtcatgagcg ggaagcgaat gangcccagg gccttgccca 300
gaaccttccg cctgttctct ggcgtcacct gcagctgctg ccgctnacac tcggcctcgg 360
accagcggac aaacggcgtt gaacagccgc acctcacgga tgcccantgt gtcgcgctcc 420
aggaacggcn ccagcgtgtc caggtcaatg tcggtgaanc ctccgcgggt aatggcg 477

<210> 93
<211> 377
<212> DNA
<213> Homo sapiens

<220>
<221> misc_feature
<222> 139, 180, 269, 285, 298, 335, 352
<223> n = A,T,C or G

<400> 93
gaacggctgg accttgcctc gcattgtgct gctggcagga ataccttggc aagcagctcc 60
agtccgagca gccccagacc gctgccgccc gaagctaagc ctgcctctgg ccttcccctc 120
cgcctcaatg cagaaccant agtgggagca ctgtgtttag agttaagagt gaacactgtn 180
tgattttact tgggaatttc ctctgttata tagcttttcc caatgctaat ttccaaacaa 240
caacaacaaa ataacatgtt tgcctgttna gttgtataaa agtangtgat tctgtatnta 300
aagaaaatat tactgttaca tatactgctt gcaanttctg tatttattgg tnctctggaa 360
ataaatatat tattaaa 377

<210> 94
<211> 495
<212> DNA
<213> Homo sapiens

<220>
<221> misc_feature
<222> 53, 69, 249, 259, 271, 351, 374, 377, 398, 412, 429, 435,
436, 460, 464, 465, 477, 479, 489
<223> n = A,T,C or G

<400> 94
ccctttgagg ggttagggtc cagttcccag tggaagaaac aggccaggag aantgcgtgc 60
cgagctgang cagatttccc acagtgaccc cagagccctg ggctatagtc tctgacccct 120
ccaaggaaag accaccttct ggggacatgg gctggagggc aggacctaga ggcaccaagg 180
gaaggcccca ttccggggct gttccccgag gaggaaggga aggggctctg tgtgcccccc 240
acgaggaana ggccctgant cctgggatca nacacccctt cacgtgtatc cccacacaaa 300
tgcaagctca ccaaggtccc ctctcagtcc cttccctaca ccctgaacgg ncactggccc 360
acacccaccc agancancca cccgccatgg ggaatgtnct caaggaatcg cngggcaacg 420
tggactctng tcccnnaagg gggcagaatc tccaatagan gganngaacc cttgctnana 480
aaaaaaaana aaaaa 495

<210> 95
<211> 472
<212> DNA
<213> Homo sapiens

<220>
<221> misc_feature
<222> 177, 280, 414, 425, 430
<223> n = A,T,C or G

<400> 95
ggttacttgg tttcattgcc accacttagt ggatgtcatt tagaaccatt ttgtctgctc 60
cctctggaag ccttgcgcag agcggacttt gtaattgttg gagaataact gctgaatttt 120
tagctgtttt gagttgattc gcaccactgc accacaactc aatatgaaaa ctatttnact 180
tatttattat cttgtgaaaa gtatacaatg aaaattttgt tcatactgta tttatcaagt 240
atgatgaaaa gcaatagata tatattcttt tattatgttn aattatgatt gccattatta 300
atcggcaaaa tgtggagtgt atgttctttt cacagtaata tatgcctttt gtaacttcac 360
ttggttattt tattgtaaat gaattacaaa attcttaatt taagaaaatg gtangttata 420
tttanttcan taatttcttt ccttgtttac gttaattttg aaaagaatgc at 472

<210> 96
<211> 476
<212> DNA
<213> Homo sapiens

<220>
<221> misc_feature
<222> 22, 222, 226, 237, 255, 291, 383, 410, 441, 443, 450
<223> n = A,T,C or G

<400> 96
ctgaagcatt tcttcaaact tntctacttt tgtcattgat acctgtagta agttgacaat 60
gtggtgaaat ttcaaaatta tatgtaactt ctactagttt tactttctcc cccaagtctt 120
ttttaactca tgatttttac acacacaatc cagaacttat tatatagcct ctaagtcttt 180
attcttcaca gtagatgatg aaagagtcct ccagtgtctt gngcanaatg ttctagntat 240
agctggatac atacngtggg agttctataa actcatacct cagtgggact naaccaaaat 300
tgtgttagtc tcaattccta ccacactgag ggagcctccc aaatcactat attcttatct 360
gcaggtactc ctccagaaaa acngacaggg caggcttgca tgaaaaagtn acatctgcgt 420
tacaaagtct atcttcctca nangtctgtn aaggaacaat ttaatcttct agcttt 476

<210> 97
<211> 479
<212> DNA
<213> Homo sapiens

<220>

<221> misc_feature
<222> 213, 226, 270, 291, 309, 361, 363, 364, 371, 395, 399, 400,
424, 440, 444, 449, 452, 464, 474
<223> n = A,T,C or G

<400> 97
actctttcta atgctgatat gatcttgagt ataagaatgc atatgtcact agaatggata 60
aaataatgct gcaaacttaa tgttcttatg caaaatggaa cgctaatgaa acacagctta 120
caatcgcaaa tcaaaactca caagtgctca tctgttgtag atttagtgta ataagactta 180
gattgtgctc cttcggatat gattgtttct canatcttgg gcaatnttcc ttagtcaaat 240
caggctacta gaattctgtt attggatatn tgagagcatg aaatttttaa naatacactt 300
gtgattatna aattaatcac aaatttcact tatacctgct atcagcagct agaaaaacat 360
ntnnttttta natcaaagta ttttgtgttt ggaantgtnn aaatgaaatc tgaatgtggg 420
ttcnatctta ttttttcccn gacnactant tnctttttta gggnctattc tganccatc 479

<210> 98
<211> 461
<212> DNA
<213> Homo sapiens

<400> 98
agtgacttgt cctccaacaa aacccccttga tcaagtttgt ggcactgaca atcagaccta 60
tgctagttcc tgtcatctat tcgctactaa atgcagactg gaggggacca aaaaggggca 120
tcaactccag ctggattatt ttggagcctg caaatctatt cctacttgta cggactttga 180
agtgattcag tttcctctac ggatgagaga ctggctcaag aatatcctca tgcagcttta 240
tgaagccact ctgaacacgc tggttatcta gatgagaaca gagaaataaa gtcagaaaat 300
ttacctggag aaaagaggct ttggctgggg accatcccat tgaaccttct cttaaggact 360
ttaagaaaaa ctaccacatg ttgtgtatcc tggtgccggc cgtttatgaa ctgaccaccc 420
tttggaataa tcttgacgct cctgaacttg ctcctctgcg a 461

<210> 99
<211> 171
<212> DNA
<213> Homo sapiens

<400> 99
gtggccgcgc gcaggtgttt cctcgtaccg cagggccccc tcccttcccc aggcgtccct 60
cggcgcctct gcgggcccga ggaggagcgg ctggcgggtg gggggagtgt gacccaccct 120
cggtgagaaa agccttctct agcgatctga gaggcgtgcc ttgggggtac c 171

<210> 100
<211> 269
<212> DNA
<213> Homo sapiens

<400> 100
cggccgcaag tgcaactcca gctggggccg tgcggacgaa gattctgcca gcagttggtc 60
cgactgcgac gacggcggcg gcgacagtcg caggtgcagc gcgggcgcct ggggtcttgc 120
aaggctgagc tgacgccgca gaggtcgtgt cacgtcccac gaccttgacg ccgtcgggga 180
cagccggaac agagcccggt gaagcgggag gcctcgggga gcccctcggg aagggcggcc 240
cgagagatac gcaggtgcag gtggccgcc 269

<210> 101
<211> 405
<212> DNA
<213> Homo sapiens

<400> 101
tttttttttt ttttggaatc tactgcgagc acagcaggtc agcaacaagt ttattttgca 60
gctagcaagg taacagggta gggcatggtt acatgttcag gtcaacttcc tttgtcgtgg 120
ttgattggtt tgtctttatg gggggcgggt ggggtagggg aaacgaagca aataacatgg 180
agtgggtgca ccctccctgt agaacctggt tacaaagctt ggggcagttc acctggtctg 240
tgaccgtcat tttcttgaca tcaatgttat tagaagtcag gatatctttt agagagtcca 300
ctgttctgga gggagattag ggtttcttgc caaatccaac aaaatccact gaaaaagttg 360
gatgatcagt acgaataccg aggcatattc tcatatcggt ggcca 405

<210> 102
<211> 470

<212> DNA
<213> Homo sapiens

<400> 102
tttttttttt tttttttttt tttttttttt tttttttttt tttttttttt tttttttttt 60
ggcacttaat ccatttttat ttcaaaatgt ctacaaattt aatcccatta tacggtattt 120
tcaaaatcta aattattcaa attagccaaa tccttaccaa ataataccca aaaatcaaaa 180
atatacttct ttcagcaaac ttgttacata aattaaaaaa atatatacgg ctggtgtttt 240
caaagtacaa ttatcttaac actgcaaaca ttttaaggaa ctaaaataaa aaaaaacact 300
ccgcaaaggt taaagggaac aacaaattct tttacaacac cattataaaa atcatatctc 360
aaatcttagg ggaatatata cttcacacgg gatcttaact tttactcact ttgtttattt 420
ttttaaacca ttgtttgggc ccaacacaat ggaatccccc ctggactagt 470

<210> 103
<211> 581
<212> DNA
<213> Homo sapiens

<400> 103
tttttttttt ttttttttga cccccctctt ataaaaaaca agttaccatt ttattttact 60
tacacatatt tattttataa ttggtattag atattcaaaa ggcagctttt aaaatcaaac 120
taaatggaaa ctgccttaga tacataattc ttaggaatta gcttaaaatc tgcctaaagt 180
gaaaatcttc tctagctctt ttgactgtaa atttttgact cttgtaaaac atccaaattc 240
atttttcttg tctttaaaat tatctaatct ttccattttt tccctattcc aagtcaattt 300
gcttctctag cctcatttcc tagctcttat ctactattag taagtggctt ttttcctaaa 360
agggaaaaca ggaagagaaa tggcacacaa aacaaacatt ttatattcat atttctacct 420
acgttaataa aatagcattt tgtgaagcca gctcaaaaga aggcttagat ccttttatgt 480
ccattttagt cactaaacga tatcaaagtg ccagaatgca aaaggtttgt gaacatttat 540
tcaaaagcta atataagata tttcacatac tcatctttct g 581

<210> 104
<211> 578
<212> DNA
<213> Homo sapiens

<400> 104
tttttttttt tttttttttt tttttctctt cttttttttt gaaatgagga tcgagttttt 60
cactctctag atagggcatg aagaaaactc atctttccag ctttaaaata acaatcaaat 120
ctcttatgct atatcatatt ttaagttaaa ctaatgagtc actggcttat cttctcctga 180
aggaaatctg ttcattcttc tcattcatat agttatatca agtactacct tgcatattga 240
gaggtttttc ttctctattt acacatatat ttccatgtga atttgtatca aacctttatt 300
ttcatgcaaa ctagaaaata atgtttcttt tgcataagag aagagaacaa tatagcatta 360
caaaactgct caaattgttt gttaagttat ccattataat tagttggcag gagctaatac 420
aaatcacatt tacgacagca ataataaaac tgaagtacca gttaaatatc caaaataatt 480
aaaggaacat ttttagcctg ggtataatta gctaattcac tttacaagca tttattagaa 540
tgaattcaca tgttattatt cctagcccaa cacaatgg 578

<210> 105
<211> 538
<212> DNA
<213> Homo sapiens

<400> 105
tttttttttt tttttcagta ataatcagaa caatatttat ttttatattt aaaattcata 60
gaaaagtgcc ttacatttaa taaaagtttg tttctcaaag tgatcagagg aattagatat 120
gtcttgaaca ccaatattaa tttgaggaaa atacaccaaa atacattaag taaattattt 180
aagatcatag agcttgtaag tgaaaagata aaatttgacc tcagaaactc tgagcattaa 240
aaatccacta ttagcaaata aattactatg gacttcttgc tttaattttg tgatgaatat 300
ggggtgtcac tggtaaacca acacattctg aaggatacat tacttagtga tagattctta 360
tgtactttgc taatacgtgg atatgagttg acaagtttct ctttcttcaa tcttttaagg 420
ggcgagaaat gaggaagaaa agaaaaggat tacgcatact gttctttcta tggaaggatt 480
agatatgttt cctttgccaa tattaaaaaa ataataatgt ttactactag tgaaaccc 538

<210> 106
<211> 473
<212> DNA
<213> Homo sapiens

<400> 106
tttttttttt ttttttagtc aagtttctat ttttattata attaaagtct tggtcatttc 60
atttattagc tctgcaactt acatatttaa attaaagaaa cgttttagac aactgtacaa 120
tttataaatg taaggtgcca ttattgagta atatattcct ccaagagtgg atgtgtccct 180
tctcccacca actaatgaac agcaacatta gtttaatttt attagtagat atacactgct 240
gcaaacgcta attctcttct ccatccccat gtgatattgt gtatatgtgt gagttggtag 300
aatgcatcac aatctacaat caacagcaag atgaagctag gctgggcttt cggtgaaaat 360
agactgtgtc tgtctgaatc aaatgatctg acctatcctc ggtggcaaga actcttcgaa 420
ccgcttcctc aaaggcgctg ccacatttgt ggctctttgc acttgtttca aaa 473

<210> 107
<211> 1621
<212> DNA
<213> Homo sapiens

<400> 107
cgccatggca ctgcagggca tctcggtcat ggagctgtcc ggcctggccc cgggcccgtt 60
ctgtgctatg gtcctggctg acttcggggc gcgtgtggta cgcgtggacc ggcccggctc 120
ccgctacgac gtgagccgct tgggccgggg caagcgctcg ctagtgctgg acctgaagca 180
gccgcgggga gccgccgtgc tgcggcgtct gtgcaagcgg tcggatgtgc tgctggagcc 240
cttccgccgc ggtgtcatgg agaaactcca gctgggccca gagattctgc agcgggaaaa 300
tccaaggctt atttatgcca ggctgagtgg atttggccag tcaggaagct tctgccggtt 360
agctggccac gatatcaact atttggcttt gtcaggtgtt ctctcaaaaa ttggcagaag 420
tggtgagaat ccgtatgccc cgctgaatct cctggctgac tttgctggtg gtggccttat 480
gtgtgcactg ggcattataa tggctctttt tgaccgcaca cgcactgaca agggtcaggt 540
cattgatgca aatatggtgg aaggaacagc atatttaagt tcttttctgt ggaaaactca 600
gaaatcgagt ctgtgggaag cacctcgagg acagaacatg ttggatggtg gagcaccttt 660
ctatacgact tacaggacag cagatgggga attcatggct gttggagcaa tagaacccca 720
gttctacgag ctgctgatca aaggacttgg actaaagtct gatgaacttc ccaatcagat 780
gagcatggat gattggccag aaatgaagaa gaagtttgca gatgtatttg caaagaagac 840
gaaggcagag tggtgtcaaa tctttgacgg cacagatgcc tgtgtgactc cggttctgac 900
ttttgaggag gttgttcatc atgatcacaa caaggaacgg ggctcgttta tcaccagtga 960
ggagcaggac gtgagccccc gccctgcacc tctgctgtta aacaccccag ccatcccttc 1020
tttcaaaagg gatcctttca taggagaaca cactgaggag atacttgaag aatttggatt 1080
cagccgcgaa gagatttatc agcttaactc agataaaatc attgaaagta ataaggtaaa 1140
agctagtctc taacttccag gcccacggct caagtgaatt tgaatactgc atttacagtg 1200
tagagtaaca cataacattg tatgcatgga aacatggagg aacagtatta cagtgtccta 1260
ccactctaat caagaaaaga attacagact ctgattctac agtgatgatt gaattctaaa 1320
aatggttatc attagggctt ttgatttata aaactttggg tacttatact aaattatggt 1380
agttattctg ccttccagtt tgcttgatat atttgttgat attaagattc ttgacttata 1440
ttttgaatgg gttctagtga aaaaggaatg atatattctt gaagacatcg atatacattt 1500
atttacactc ttgattctac aatgtagaaa atgaggaaat gccacaaatt gtatggtgat 1560
aaaagtcacg tgaaacaaaa aaaaaaaaaa aaaaaaaaaa aaaaaaaaaa aaaaaaaaaa 1620
a 1621

<210> 108
<211> 382
<212> PRT
<213> Homo sapiens

<400> 108

<210> 109
<211> 1524
<212> DNA
<213> Homo sapiens

<400> 109
ggcacgaggc tgcgccaggg cctgagcgga ggcgggggca gcctcgccag cgggggcccc 60
gggcctggcc atgcctcact gagccagcgc ctgcgcctct acctcgccga cagctggaac 120
cagtgcgacc tagtggctct cacctgcttc ctcctgggcg tgggctgccg gctgaccccg 180
ggtttgtacc acctgggccg cactgtcctc tgcatcgact tcatggtttt cacggtgcgg 240
ctgcttcaca tcttcacggt caacaaacag ctggggccca agatcgtcat cgtgagcaag 300
atgatgaagg acgtgttctt cttcctcttc ttcctcggcg tgtggctggt agcctatggc 360
gtggccacgg aggggctcct gaggccacgg gacagtgact tcccaagtat cctgcgccgc 420
gtcttctacc gtccctacct gcagatcttc gggcagattc cccaggagga catggacgtg 480
gccctcatgg agcacagcaa ctgctcgtcg gagcccggct tctgggcaca ccctcctggg 540
gcccaggcgg gcacctgcgt ctcccagtat gccaactggc tggtggtgct gctcctcgtc 600
atcttcctgc tcgtggccaa catcctgctg gtcaacttgc tcattgccat gttcagttac 660
acattcggca aagtacaggg caacagcgat ctctactgga aggcgcagcg ttaccgcctc 720
atccgggaat tccactctcg gcccgcgctg gccccgccct ttatcgtcat ctcccacttg 780
cgcctcctgc tcaggcaatt gtgcaggcga ccccggagcc cccagccgtc ctccccggcc 840
ctcgagcatt tccgggttta cctttctaag gaagccgagc ggaagctgct aacgtgggaa 900
tcggtgcata aggagaactt tctgctggca cgcgctaggg acaagcggga gagcgactcc 960
gagcgtctga agcgcacgtc ccagaaggtg gacttggcac tgaaacagct gggacacatc 1020
cgcgagtacg aacagcgcct gaaagtgctg gagcgggagg tccagcagtg tagccgcgtc 1080
ctggggtggg tggccgaggc cctgagccgc tctgccttgc tgcccccagg tgggccgcca 1140
ccccctgacc tgcctgggtc caaagactga gccctgctgg cggacttcaa ggagaagccc 1200
ccacagggga ttttgctcct agagtaaggc tcatctgggc ctcggccccc gcacctggtg 1260
gccttgtcct tgaggtgagc cccatgtcca tctgggccac tgtcaggacc acctttggga 1320
gtgtcatcct tacaaaccac agcatgcccg gctcctccca gaaccagtcc cagcctggga 1380
ggatcaaggc ctggatcccg ggccgttatc catctggagg ctgcagggtc cttggggtaa 1440
cagggaccac agacccctca ccactcacag attcctcaca ctggggaaat aaagccattt 1500
cagaggaaaa aaaaaaaaaa aaaa                                        1524

<210> 110
<211> 3410

<212> DNA
<213> Homo sapiens

<400> 110
gggaaccagc ctgcacgcgc tggctccggg tgacagccgc gcgcctcggc caggatctga 60
gtgatgagac gtgtccccac tgaggtgccc cacagcagca ggtgttgagc atgggctgag 120
aagctggacc ggcaccaaag ggctggcaga aatgggcgcc tggctgattc ctaggcagtt 180
ggcggcagca aggaggagag gccgcagctt ctggagcaga gccgagacga agcagttctg 240
gagtgcctga acggccccct gagccctacc cgcctggccc actatggtcc agaggctgtg 300
ggtgagccgc ctgctgcggc accggaaagc ccagctcttg ctggtcaacc tgctaacctt 360
tggcctggag gtgtgtttgg ccgcaggcat cacctatgtg ccgcctctgc tgctggaagt 420
gggggtagag gagaagttca tgaccatggt gctgggcatt ggtccagtgc tgggcctggt 480
ctgtgtcccg ctcctaggct cagccagtga ccactggcgt ggacgctatg gccgccgccg 540
gcccttcatc tgggcactgt ccttgggcat cctgctgagc ctctttctca tcccaagggc 600
cggctggcta gcagggctgc tgtgcccgga tcccaggccc ctggagctgg cactgctcat 660
cctgggcgtg gggctgctgg acttctgtgg ccaggtgtgc ttcactccac tggaggccct 720
gctctctgac ctcttccggg acccggacca ctgtcgccag gcctactctg tctatgcctt 780
catgatcagt cttgggggct gcctgggcta cctcctgcct gccattgact gggacaccag 840
tgccctggcc ccctacctgg gcacccagga ggagtgcctc tttggcctgc tcaccctcat 900
cttcctcacc tgcgtagcag ccacactgct ggtggctgag gaggcagcgc tgggccccac 960
cgagccagca gaagggctgt cggccccctc cttgtcgccc cactgctgtc catgccgggc 1020
ccgcttggct ttccggaacc tgggcgccct gcttccccgg ctgcaccagc tgtgctgccg 1080
catgccccgc accctgcgcc ggctcttcgt ggctgagctg tgcagctgga tggcactcat 1140
gaccttcacg ctgttttaca cggatttcgt gggcgagggg ctgtaccagg gcgtgcccag 1200
agctgagccg ggcaccgagg cccggagaca ctatgatgaa ggcgttcgga tgggcagcct 1260
ggggctgttc ctgcagtgcg ccatctccct ggtcttctct ctggtcatgg accggctggt 1320
gcagcgattc ggcactcgag cagtctattt ggccagtgtg gcagctttcc ctgtggctgc 1380
cggtgccaca tgcctgtccc acagtgtggc cgtggtgaca gcttcagccg ccctcaccgg 1440
gttcaccttc tcagccctgc agatcctgcc ctacacactg gcctccctct accaccggga 1500
gaagcaggtg ttcctgccca aataccgagg ggacactgga ggtgctagca gtgaggacag 1560
cctgatgacc agcttcctgc caggccctaa gcctggagct cccttcccta atggacacgt 1620
gggtgctgga ggcagtggcc tgctcccacc tccacccgcg ctctgcgggg cctctgcctg 1680
tgatgtctcc gtacgtgtgg tggtgggtga gcccaccgag gccagggtgg ttccgggccg 1740
gggcatctgc ctggacctcg ccatcctgga tagtgccttc ctgctgtccc aggtggcccc 1800
atccctgttt atgggctcca ttgtccagct cagccagtct gtcactgcct atatggtgtc 1860
tgccgcaggc ctgggtctgg tcgccattta ctttgctaca caggtagtat ttgacaagag 1920
cgacttggcc aaatactcag cgtagaaaac ttccagcaca ttggggtgga gggcctgcct 1980
cactgggtcc cagctccccg ctcctgttag ccccatgggg ctgccgggct ggccgccagt 2040
ttctgttgct gccaaagtaa tgtggctctc tgctgccacc ctgtgctgct gaggtgcgta 2100
gctgcacagc tgggggctgg ggcgtccctc tcctctctcc ccagtctcta gggctgcctg 2160
actggaggcc ttccaagggg gtttcagtct ggacttatac agggaggcca gaagggctcc 2220
atgcactgga atgcggggac tctgcaggtg gattacccag gctcagggtt aacagctagc 2280
ctcctagttg agacacacct agagaagggt ttttgggagc tgaataaact cagtcacctg 2340
gtttcccatc tctaagcccc ttaacctgca gcttcgttta atgtagctct tgcatgggag 2400
tttctaggat gaaacactcc tccatgggat ttgaacatat gacttatttg taggggaaga 2460
gtcctgaggg gcaacacaca agaaccaggt cccctcagcc cacagcactg tctttttgct 2520
gatccacccc cctcttacct tttatcagga tgtggcctgt tggtccttct gttgccatca 2580
cagagacaca ggcatttaaa tatttaactt atttatttaa caaagtagaa gggaatccat 2640
tgctagcttt tctgtgttgg tgtctaatat ttgggtaggg tgggggatcc ccaacaatca 2700
ggtcccctga gatagctggt cattgggctg atcattgcca gaatcttctt ctcctggggt 2760
ctggcccccc aaaatgccta acccaggacc ttggaaattc tactcatccc aaatgataat 2820
tccaaatgct gttacccaag gttagggtgt tgaaggaagg tagagggtgg ggcttcaggt 2880
ctcaacggct tccctaacca cccctcttct cttggcccag cctggttccc cccacttcca 2940
ctcccctcta ctctctctag gactgggctg atgaaggcac tgcccaaaat ttcccctacc 3000
cccaactttc ccctaccccc aactttcccc accagctcca caaccctgtt tggagctact 3060
gcaggaccag aagcacaaag tgcggtttcc caagcctttg tccatctcag cccccagagt 3120
atatctgtgc ttggggaatc tcacacagaa actcaggagc accccctgcc tgagctaagg 3180
gaggtcttat ctctcagggg gggtttaagt gccgtttgca ataatgtcgt cttatttatt 3240
tagcggggtg aatattttat actgtaagtg agcaatcaga gtataatgtt tatggtgaca 3300
aaattaaagg ctttcttata tgtttaaaaa aaaaaaaaaa aaaaaaaaaa aaaaaaaaaa 3360
aaaaaaaara aaaaaaaaaa aaaaaaaaaa aaaaaaataa aaaaaaaaaa 3410

<210> 111
<211> 1289
<212> DNA
<213> Homo sapiens

<400> 111

agccaggcgt ccctctgcct gcccactcag tggcaacacc cgggagctgt tttgtccttt 60
gtggagcctc agcagttccc tctttcagaa ctcactgcca agagccctga acaggagcca 120
ccatgcagtg cttcagcttc attaagacca tgatgatcct cttcaatttg ctcatctttc 180
tgtgtggtgc agccctgttg gcagtgggca tctgggtgtc aatcgatggg gcatcctttc 240
tgaagatctt cgggccactg tcgtccagtg ccatgcagtt tgtcaacgtg ggctacttcc 300
tcatcgcagc cggcgttgtg gtctttgctc ttggtttcct gggctgctat ggtgctaaga 360
ctgagagcaa gtgtgccctc gtgacgttct tcttcatcct cctcctcatc ttcattgctg 420
aggttgcagc tgctgtggtc gccttggtgt acaccacaat ggctgagcac ttcctgacgt 480
tgctggtagt gcctgccatc aagaaagatt atggttccca ggaagacttc actcaagtgt 540
ggaacaccac catgaaaggg ctcaagtgct gtggcttcac caactatacg gattttgagg 600
actcacccta cttcaaagag aacagtgcct ttcccccatt ctgttgcaat gacaacgtca 660
ccaacacagc caatgaaacc tgcaccaagc aaaaggctca cgaccaaaaa gtagagggtt 720
gcttcaatca gcttttgtat gacatccgaa ctaatgcagt caccgtgggt ggtgtggcag 780
ctggaattgg gggcctcgag ctggctgcca tgattgtgtc catgtatctg tactgcaatc 840
tacaataagt ccacttctgc ctctgccact actgctgcca catgggaact gtgaagaggc 900
accctggcaa gcagcagtga ttgggggagg ggacaggatc taacaatgtc acttgggcca 960
gaatggacct gccctttctg ctccagactt ggggctagat agggaccact ccttttagcg 1020
atgcctgact ttccttccat tggtgggtgg atgggtgggg ggcattccag agcctctaag 1080
gtagccagtt ctgttgccca ttcccccagt ctattaaacc cttgatatgc cccctaggcc 1140
tagtggtgat cccagtgctc tactggggga tgagagaaag gcattttata gcctgggcat 1200
aagtgaaatc agcagagcct ctgggtggat gtgtagaagg cacttcaaaa tgcataaacc 1260
tgttacaatg ttaaaaaaaa aaaaaaaaa 1289

<210> 112
<211> 315
<212> PRT
<213> Homo sapiens

<400> 112
Met Val Phe Thr Val Arg Leu Leu His Ile Phe Thr Val Asn Lys Gln
1               5                   10                  15
Leu Gly Pro Lys Ile Val Ile Val Ser Lys Met Met Lys Asp Val Phe
            20                  25                  30
Phe Phe Leu Phe Phe Leu Gly Val Trp Leu Val Ala Tyr Gly Val Ala
        35                  40                  45
Thr Glu Gly Leu Leu Arg Pro Arg Asp Ser Asp Phe Pro Ser Ile Leu
    50                  55                  60
Arg Arg Val Phe Tyr Arg Pro Tyr Leu Gln Ile Phe Gly Gln Ile Pro
65                  70                  75                  80
Gln Glu Asp Met Asp Val Ala Leu Met Glu His Ser Asn Cys Ser Ser
                85                  90                  95
Glu Pro Gly Phe Trp Ala His Pro Pro Gly Ala Gln Ala Gly Thr Cys
            100                 105                 110
Val Ser Gln Tyr Ala Asn Trp Leu Val Val Leu Leu Leu Val Ile Phe
        115                 120                 125
Leu Leu Val Ala Asn Ile Leu Leu Val Asn Leu Leu Ile Ala Met Phe
    130                 135                 140
Ser Tyr Thr Phe Gly Lys Val Gln Gly Asn Ser Asp Leu Tyr Trp Lys
145                 150                 155                 160
Ala Gln Arg Tyr Arg Leu Ile Arg Glu Phe His Ser Arg Pro Ala Leu
                165                 170                 175
Ala Pro Pro Phe Ile Val Ile Ser His Leu Arg Leu Leu Leu Arg Gln
            180                 185                 190
Leu Cys Arg Arg Pro Arg Ser Pro Gln Pro Ser Ser Pro Ala Leu Glu
        195                 200                 205
His Phe Arg Val Tyr Leu Ser Lys Glu Ala Glu Arg Lys Leu Leu Thr
    210                 215                 220
Trp Glu Ser Val His Lys Glu Asn Phe Leu Leu Ala Arg Ala Arg Asp
225                 230                 235                 240
Lys Arg Glu Ser Asp Ser Glu Arg Leu Lys Arg Thr Ser Gln Lys Val
                245                 250                 255
Asp Leu Ala Leu Lys Gln Leu Gly His Ile Arg Glu Tyr Glu Gln Arg
            260                 265                 270
Leu Lys Val Leu Glu Arg Glu Val Gln Gln Cys Ser Arg Val Leu Gly
        275                 280                 285
Trp Val Ala Glu Ala Leu Ser Arg Ser Ala Leu Leu Pro Pro Gly Gly
    290                 295                 300
Pro Pro Pro Pro Asp Leu Pro Gly Ser Lys Asp

305                 310                 315

<210> 113
<211> 553
<212> PRT
<213> Homo sapiens

<400> 113
Met Val Gln Arg Leu Trp Val Ser Arg Leu Leu Arg His Arg Lys Ala
1               5                   10                  15
Gln Leu Leu Leu Val Asn Leu Leu Thr Phe Gly Leu Glu Val Cys Leu
            20                  25                  30
Ala Ala Gly Ile Thr Tyr Val Pro Pro Leu Leu Leu Glu Val Gly Val
        35                  40                  45
Glu Glu Lys Phe Met Thr Met Val Leu Gly Ile Gly Pro Val Leu Gly
    50                  55                  60
Leu Val Cys Val Pro Leu Leu Gly Ser Ala Ser Asp His Trp Arg Gly
65                  70                  75                  80
Arg Tyr Gly Arg Arg Arg Pro Phe Ile Trp Ala Leu Ser Leu Gly Ile
                85                  90                  95
Leu Leu Ser Leu Phe Leu Ile Pro Arg Ala Gly Trp Leu Ala Gly Leu
            100                 105                 110
Leu Cys Pro Asp Pro Arg Pro Leu Glu Leu Ala Leu Leu Ile Leu Gly
        115                 120                 125
Val Gly Leu Leu Asp Phe Cys Gly Gln Val Cys Phe Thr Pro Leu Glu
    130                 135                 140
Ala Leu Leu Ser Asp Leu Phe Arg Asp Pro Asp His Cys Arg Gln Ala
145                 150                 155                 160
Tyr Ser Val Tyr Ala Phe Met Ile Ser Leu Gly Gly Cys Leu Gly Tyr
                165                 170                 175
Leu Leu Pro Ala Ile Asp Trp Asp Thr Ser Ala Leu Ala Pro Tyr Leu
            180                 185                 190
Gly Thr Gln Glu Glu Cys Leu Phe Gly Leu Leu Thr Leu Ile Phe Leu
        195                 200                 205
Thr Cys Val Ala Ala Thr Leu Leu Val Ala Glu Glu Ala Ala Leu Gly
    210                 215                 220
Pro Thr Glu Pro Ala Glu Gly Leu Ser Ala Pro Ser Leu Ser Pro His
225                 230                 235                 240
Cys Cys Pro Cys Arg Ala Arg Leu Ala Phe Arg Asn Leu Gly Ala Leu
                245                 250                 255
Leu Pro Arg Leu His Gln Leu Cys Cys Arg Met Pro Arg Thr Leu Arg
            260                 265                 270
Arg Leu Phe Val Ala Glu Leu Cys Ser Trp Met Ala Leu Met Thr Phe
        275                 280                 285
Thr Leu Phe Tyr Thr Asp Phe Val Gly Glu Gly Leu Tyr Gln Gly Val
    290                 295                 300
Pro Arg Ala Glu Pro Gly Thr Glu Ala Arg Arg His Tyr Asp Glu Gly
305                 310                 315                 320
Val Arg Met Gly Ser Leu Gly Leu Phe Leu Gln Cys Ala Ile Ser Leu
                325                 330                 335
Val Phe Ser Leu Val Met Asp Arg Leu Val Gln Arg Phe Gly Thr Arg
            340                 345                 350
Ala Val Tyr Leu Ala Ser Val Ala Ala Phe Pro Val Ala Ala Gly Ala
        355                 360                 365
Thr Cys Leu Ser His Ser Val Ala Val Val Thr Ala Ser Ala Ala Leu
    370                 375                 380
Thr Gly Phe Thr Phe Ser Ala Leu Gln Ile Leu Pro Tyr Thr Leu Ala
385                 390                 395                 400
Ser Leu Tyr His Arg Glu Lys Gln Val Phe Leu Pro Lys Tyr Arg Gly
                405                 410                 415
Asp Thr Gly Gly Ala Ser Ser Glu Asp Ser Leu Met Thr Ser Phe Leu
            420                 425                 430
Pro Gly Pro Lys Pro Gly Ala Pro Phe Pro Asn Gly His Val Gly Ala
        435                 440                 445
Gly Gly Ser Gly Leu Leu Pro Pro Pro Pro Ala Leu Cys Gly Ala Ser
    450                 455                 460
Ala Cys Asp Val Ser Val Arg Val Val Val Gly Glu Pro Thr Glu Ala

465                 470                 475                 480
Arg Val Val Pro Gly Arg Gly Ile Cys Leu Asp Leu Ala Ile Leu Asp
                485                 490                 495
Ser Ala Phe Leu Leu Ser Gln Val Ala Pro Ser Leu Phe Met Gly Ser
            500                 505                 510
Ile Val Gln Leu Ser Gln Ser Val Thr Ala Tyr Met Val Ser Ala Ala
        515                 520                 525
Gly Leu Gly Leu Val Ala Ile Tyr Phe Ala Thr Gln Val Val Phe Asp
    530                 535                 540
Lys Ser Asp Leu Ala Lys Tyr Ser Ala
545                 550

<210> 114
<211> 241
<212> PRT
<213> Homo sapiens

<400> 114
Met Gln Cys Phe Ser Phe Ile Lys Thr Met Met Ile Leu Phe Asn Leu
 1               5                  10                  15
Leu Ile Phe Leu Cys Gly Ala Ala Leu Leu Ala Val Gly Ile Trp Val
            20                  25                  30
Ser Ile Asp Gly Ala Ser Phe Leu Lys Ile Phe Gly Pro Leu Ser Ser
        35                  40                  45
Ser Ala Met Gln Phe Val Asn Val Gly Tyr Phe Leu Ile Ala Ala Gly
    50                  55                  60
Val Val Val Phe Ala Leu Gly Phe Leu Gly Cys Tyr Gly Ala Lys Thr
65                  70                  75                  80
Glu Ser Lys Cys Ala Leu Val Thr Phe Phe Phe Ile Leu Leu Leu Ile
                85                  90                  95
Phe Ile Ala Glu Val Ala Ala Ala Val Val Ala Leu Val Tyr Thr Thr
            100                 105                 110
Met Ala Glu His Phe Leu Thr Leu Leu Val Val Pro Ala Ile Lys Lys
        115                 120                 125
Asp Tyr Gly Ser Gln Glu Asp Phe Thr Gln Val Trp Asn Thr Thr Met
    130                 135                 140
Lys Gly Leu Lys Cys Cys Gly Phe Thr Asn Tyr Thr Asp Phe Glu Asp
145                 150                 155                 160
Ser Pro Tyr Phe Lys Glu Asn Ser Ala Phe Pro Pro Phe Cys Cys Asn
                165                 170                 175
Asp Asn Val Thr Asn Thr Ala Asn Glu Thr Cys Thr Lys Gln Lys Ala
            180                 185                 190
His Asp Gln Lys Val Glu Gly Cys Phe Asn Gln Leu Leu Tyr Asp Ile
        195                 200                 205
Arg Thr Asn Ala Val Thr Val Gly Gly Val Ala Ala Gly Ile Gly Gly
    210                 215                 220
Leu Glu Leu Ala Ala Met Ile Val Ser Met Tyr Leu Tyr Cys Asn Leu
225                 230                 235                 240
Gln

<210> 115
<211> 366
<212> DNA
<213> Homo sapiens

<400> 115
gctctttctc tcccctcctc tgaatttaat tctttcaact tgcaatttgc aaggattaca 60
catttcactg tgatgtatat tgtgttgcaa aaaaaaaaaa gtgtctttgt ttaaaattac 120
ttggtttgtg aatccatctt gctttttccc cattggaact agtcattaac ccatctctga 180
actggtagaa aaacatctga agagctagtc tatcagcatc tgacaggtga attggatggt 240
tctcagaacc atttcaccca gacagcctgt ttctatcctg tttaataaat tagtttgggt 300
tctctacatg cataacaaac cctgctccaa tctgtcacat aaaagtctgt gacttgaagt 360
ttagtc                                                            366

<210> 116

<211> 282
<212> DNA
<213> Homo sapiens

<220>
<221> misc_feature
<222> 73, 83, 248, 259
<223> n = A,T,C or G

<400> 116
acaaagatga accatttcct atattatagc aaaattaaaa tctacccgta ttctaatatt 60
gagaaatgag atnaaacaca atnttataaa gtctacttag agaagatcaa gtgacctcaa 120
agactttact attttcatat tttaagacac atgatttatc ctattttagt aacctggttc 180
atacgttaaa caaaggataa tgtgaacagc agagaggatt tgttggcaga aaatctatgt 240
tcaatctnga actatctana tcacagacat ttctattcct tt 282

<210> 117
<211> 305
<212> DNA
<213> Homo sapiens

<220>
<221> misc_feature
<222> 46, 96, 276
<223> n = A,T,C or G

<400> 117
acacatgtcg cttcactgcc ttcttagatg cttctggtca acatanagga acagggacca 60
tatttatcct ccctcctgaa acaattgcaa aataanacaa aatatatgaa acaattgcaa 120
aataaggcaa aatatatgaa acaacaggtc tcgagatatt ggaaatcagt caatgaagga 180
tactgatccc tgatcactgt cctaatgcag gatgtgggaa acagatgagg tcacctctgt 240
gactgcccca gcttactgcc tgtagagagt ttctangctg cagttcagac agggagaaat 300
tgggt 305

<210> 118
<211> 71
<212> DNA
<213> Homo sapiens

<220>
<221> misc_feature
<222> 11, 63
<223> n = A,T,C or G

<400> 118
accaaggtgt ntgaatctct gacgtgggga tctctgattc ccgcacaatc tgagtggaaa 60
aantcctggg t 71

<210> 119
<211> 212
<212> DNA
<213> Homo sapiens

<220>
<221> misc_feature
<222> 38, 92, 108, 169, 179, 187, 194
<223> n = A,T,C or G

<400> 119
actccggttg gtgtcagcag cacgtggcat tgaacatngc aatgtggagc ccaaaccaca 60
gaaaatgggg tgaaattggc caactttcta tnaacttatg ttggcaantt tgccaccaac 120
agtaagctgg cccttctaat aaaagaaaat tgaaaggttt ctcactaanc ggaattaant 180
aatggantca aganactccc aggcctcagc gt 212

<210> 120
<211> 90
<212> DNA
<213> Homo sapiens

<220>
<221> misc_feature
<222> 11
<223> n = A,T,C or G

<400> 120
actcgttgca natcaggggc cccccagagt caccgttgca ggagtccttc tggtcttgcc 60
ctccgccggc gcagaacatg ctggggtggt 90

<210> 121
<211> 218
<212> DNA
<213> Homo sapiens

<220>
<221> misc_feature
<222> 5, 12, 21, 43, 125, 128, 137, 177, 187, 204
<223> n = A,T,C or G

<400> 121
tgtancgtga anacgacaga nagggttgtc aaaaatggag aanccttgaa gtcattttga 60
gaataagatt tgctaaaaga tttggggcta aaacatggtt attgggagac atttctgaag 120
atatncangt aaattangga atgaattcat ggttcttttg ggaattcctt tacgatngcc 180
agcatanact tcatgtgggg atancagcta cccttgta 218

<210> 122
<211> 171
<212> DNA
<213> Homo sapiens

<400> 122
taggggtgta tgcaactgta aggacaaaaa ttgagactca actggcttaa ccaataaagg 60
catttgttag ctcatggaac aggaagtcgg atggtggggc atcttcagtg ctgcatgagt 120
caccaccccg gcggggtcat ctgtgccaca ggtccctgtt gacagtgcgg t 171

<210> 123
<211> 76
<212> DNA
<213> Homo sapiens

<220>
<221> misc_feature
<222> 15, 68
<223> n = A,T,C or G

<400> 123
tgtagcgtga agacnacaga atggtgtgtg ctgtgctatc caggaacaca tttattatca 60
ttatcaanta ttgtgt 76

<210> 124
<211> 131
<212> DNA
<213> Homo sapiens

<400> 124
acctttcccc aaggccaatg tcctgtgtgc taactggccg gctgcaggac agctgcaatt 60
caatgtgctg ggtcatatgg aggggaggag actctaaaat agccaatttt attctcttgg 120
ttaagatttg t 131

<210> 125
<211> 432
<212> DNA
<213> Homo sapiens

<400> 125
actttatcta ctggctatga aatagatggt ggaaaattgc gttaccaact ataccactgg 60
cttgaaaaag aggtgatagc tcttcagagg acttgtgact tttgctcaga tgctgaagaa 120

ctacagtctg catttggcag aaatgaagat gaatttggat taaatgagga tgctgaagat 180
ttgcctcacc aaacaaaagt gaaacaactg agagaaaatt ttcaggaaaa aagacagtgg 240
ctcttgaagt atcagtcact tttgagaatg tttcttagtt actgcatact tcatggatcc 300
catggtgggg gtcttgcatc tgtaagaatg gaattgattt tgcttttgca agaatctcag 360
caggaaacat cagaaccact attttctagc cctctgtcag agcaaacctc agtgcctctc 420
ctctttgctt gt 432

<210> 126
<211> 112
<212> DNA
<213> Homo sapiens

<400> 126
acacaacttg aatagtaaaa tagaaactga gctgaaattt ctaattcact ttctaaccat 60
agtaagaatg atatttcccc ccagggatca ccaaatattt ataaaaattt gt 112

<210> 127
<211> 54
<212> DNA
<213> Homo sapiens

<400> 127
accacgaaac cacaaacaag atggaagcat caatccactt gccaagcaca gcag 54

<210> 128
<211> 323
<212> DNA
<213> Homo sapiens

<400> 128
acctcattag taattgtttt gttgtttcat ttttttctaa tgtctcccct ctaccagctc 60
acctgagata acagaatgaa aatggaagga cagccagatt tctcctttgc tctctgctca 120
ttctctctga agtctaggtt acccattttg gggacccatt ataggcaata aacacagttc 180
ccaaagcatt tggacagttt cttgttgtgt tttagaatgg ttttcctttt tcttagcctt 240
ttcctgcaaa aggctcactc agtcccttgc ttgctcagtg gactgggctc cccagggcct 300
aggctgcctt cttttccatg tcc 323

<210> 129
<211> 192
<212> DNA
<213> Homo sapiens

<220>
<221> misc_feature
<222> 83, 141
<223> n = A,T,C or G

<400> 129
acatacatgt gtgtatattt ttaaatatca cttttgtatc actctgactt tttagcatac 60
tgaaaacaca ctaacataat ttntgtgaac catgatcaga tacaacccaa atcattcatc 120
tagcacattc atctgtgata naaagatagg tgagtttcat ttccttcacg ttggccaatg 180
gataaacaaa gt 192

<210> 130
<211> 362
<212> DNA
<213> Homo sapiens

<220>
<221> misc_feature
<222> 37, 43, 225
<223> n = A,T,C or G

<400> 130
ccctttttta tggaatgagt agactgtatg tttgaanatt tanccacaac ctctttgaca 60
tataatgacg caacaaaaag gtgctgttta gtcctatggt tcagtttatg cccctgacaa 120
gtttccattg tgttttgccg atcttctggc taatcgtggt atcctccatg ttattagtaa 180
ttctgtattc catttgttaa acgcctggta gatgtaacct gctangaggc taactttata 240

cttatttaaa agctcttatt ttgtggtcat taaaatggca atttatgtgc agcactttat 300
tgcagcagga agcacgtgtg ggttggttgt aaagctcttt gctaatctta aaaagtaatg 360
gg 362

<210> 131
<211> 332
<212> DNA
<213> Homo sapiens

<220>
<221> misc_feature
<222> 64, 280, 284, 304
<223> n = A,T,C or G

<400> 131
ctttttgaaa gatcgtgtcc actcctgtgg acatcttgtt ttaatggagt ttcccatgca 60
gtangactgg tatggttgca gctgtccaga taaaaacatt tgaagagctc caaaatgaga 120
gttctcccag gttcgccctg ctgctccaag tctcagcagc agcctctttt aggaggcatc 180
ttctgaacta gattaaggca gcttgtaaat ctgatgtgat ttggtttatt atccaactaa 240
cttccatctg ttatcactgg agaaagccca gactccccan gacnggtacg gattgtgggc 300
atanaaggat tgggtgaagc tggcgttgtg gt 332

<210> 132
<211> 322
<212> DNA
<213> Homo sapiens

<220>
<221> misc_feature
<222> 200, 284
<223> n = A,T,C or G

<400> 132
acttttgcca ttttgtatat ataaacaatc ttgggacatt ctcctgaaaa ctaggtgtcc 60
agtggctaag agaactcgat ttcaagcaat tctgaaagga aaaccagcat gacacagaat 120
ctcaaattcc caaacagggg ctctgtggga aaaatgaggg aggacctttg tatctcgggt 180
tttagcaagt taaaatgaan atgacaggaa aggcttattt atcaacaaag agaagagttg 240
ggatgcttct aaaaaaaact ttggtagaga aaataggaat gctnaatcct agggaagcct 300
gtaacaatct acaattggtc ca 322

<210> 133
<211> 278
<212> DNA
<213> Homo sapiens

<220>
<221> misc_feature
<222> 46, 163, 224
<223> n = A,T,C or G

<400> 133
acaagccttc acaagtttaa ctaaattggg attaatcttt ctgtanttat ctgcataatt 60
cttgtttttc tttccatctg gctcctgggt tgacaatttg tggaaacaac tctattgcta 120
ctatttaaaa aaaatcacaa atctttccct ttaagctatg ttnaattcaa actattcctg 180
ctattcctgt tttgtcaaag aaattatatt tttcaaaata tgtntatttg tttgatgggt 240
cccacgaaac actaataaaa accacagaga ccagcctg 278

<210> 134
<211> 121
<212> DNA
<213> Homo sapiens

<220>
<221> misc_feature
<222> 6
<223> n = A,T,C or G

<400> 134

gtttanaaaa cttgtttagc tccatagagg aaagaatgtt aaactttgta ttttaaaaca 60
tgattctctg aggttaaact tggttttcaa atgttatttt tacttgtatt ttgcttttgg 120
t 121

<210> 135
<211> 350
<212> DNA
<213> Homo sapiens

<220>
<221> misc_feature
<222> 6, 64, 162, 170, 220, 226
<223> n = A,T,C or G

<400> 135
acttanaacc atgcctagca catcagaatc cctcaaagaa catcagtata atcctatacc 60
atancaagtg gtgactggtt aagcgtgcga caaaggtcag ctggcacatt acttgtgtgc 120
aaacttgata cttttgttct aagtaggaac tagtatacag tncctaggan tggtactcca 180
gggtgccccc caactcctgc agccgctcct ctgtgccagn ccctgnaagg aactttcgct 240
ccacctcaat caagccctgg gccatgctac ctgcaattgg ctgaacaaac gtttgctgag 300
ttcccaagga tgcaaagcct ggtgctcaac tcctggggcg tcaactcagt 350

<210> 136
<211> 399
<212> DNA
<213> Homo sapiens

<220>
<221> misc_feature
<222> 81, 143, 235, 269, 278, 369, 385
<223> n = A,T,C or G

<400> 136
tgtaccgtga agacgacaga agttgcatgg cagggacagg gcagggccga ggccagggtt 60
gctgtgattg tatccgaata ntcctcgtga gaaaagataa tgagatgacg tgagcagcct 120
gcagacttgt gtctgccttc aanaagccag acaggaaggc cctgcctgcc ttggctctga 180
cctggcggcc agccagccag ccacaggtgg gcttcttcct tttgtggtga caacnccaag 240
aaaactgcag aggcccaggg tcaggtgtna gtgggtangt gaccataaaa caccaggtgc 300
tcccaggaac ccgggcaaag gccatcccca cctacagcca gcatgcccac tggcgtgatg 360
ggtgcagang gatgaagcag ccagntgttc tgctgtggt 399

<210> 137
<211> 165
<212> DNA
<213> Homo sapiens

<220>
<221> misc_feature
<222> 12, 32, 40, 50, 91
<223> n = A,T,C or G

<400> 137
actggtgtgg tngggggtga tgctggtggt anaagttgan gtgacttcan gatggtgtgt 60
ggaggaagtg tgtgaacgta gggatgtaga ngttttggcc gtgctaaatg agcttcggga 120
ttggctggtc ccactggtgg tcactgtcat tggtggggtt cctgt 165

<210> 138
<211> 338
<212> DNA
<213> Homo sapiens

<220>
<221> misc_feature
<222> 235, 237, 243, 295
<223> n = A,T,C or G

<400> 138
actcactgga atgccacatt cacaacagaa tcagaggtct gtgaaaacat taatggctcc 60

ttaacttctc cagtaagaat cagggacttg aaatggaaac gttaacagcc acatgcccaa 120
tgctgggcag tctcccatgc cttccacagt gaaagggctt gagaaaaatc acatccaatg 180
tcatgtgttt ccagccacac caaaaggtgc ttggggtgga gggctggggg catananggt 240
cangcctcag gaagcctcaa gttccattca gctttgccac tgtacattcc ccatntttaa 300
aaaaactgat gccttttttt tttttttttg taaaattc 338

<210> 139
<211> 382
<212> DNA
<213> Homo sapiens

<400> 139
gggaatcttg gtttttggca tctggtttgc ctatagccga ggccactttg acagaacaaa 60
gaaagggact tcgagtaaga aggtgattta cagccagcct agtgcccgaa gtgaaggaga 120
attcaaacag acctcgtcat tcctggtgtg agcctggtcg gctcaccgcc tatcatctgc 180
atttgcctta ctcaggtgct accggactct ggcccctgat gtctgtagtt tcacaggatg 240
ccttatttgt cttctacacc ccacagggcc ccctacttct tcggatgtgt ttttaataat 300
gtcagctatg tgccccatcc tccttcatgc cctccctccc tttcctacca ctgctgagtg 360
gcctggaact tgtttaaagt gt 382

<210> 140
<211> 200
<212> DNA
<213> Homo sapiens

<220>
<221> misc_feature
<222> 7, 25, 45, 50, 58, 76, 112, 156
<223> n = A,T,C or G

<400> 140
accaaanctt ctttctgttg tgttngattt tactataggg gtttngcttn ttctaaanat 60
acttttcatt taacancttt tgttaagtgt caggctgcac tttgctccat anaattattg 120
ttttcacatt tcaacttgta tgtgtttgtc tcttanagca ttggtgaaat cacatatttt 180
atattcagca taaaggagaa 200

<210> 141
<211> 335
<212> DNA
<213> Homo sapiens

<220>
<221> misc_feature
<222> 224, 262, 275
<223> n = A,T,C or G

<400> 141
actttatttt caaaacactc atatgttgca aaaaacacat agaaaaataa agtttggtgg 60
gggtgctgac taaacttcaa gtcacagact tttatgtgac agattggagc agggtttgtt 120
atgcatgtag agaacccaaa ctaatttatt aaacaggata gaaacaggct gtctgggtga 180
aatggttctg agaaccatcc aattcacctg tcagatgctg atanactagc tcttcagatg 240
tttttctacc agttcagaga tnggttaatg actanttcca atggggaaaa agcaagatgg 300
attcacaaac caagtaattt taaacaaaga cactt 335

<210> 142
<211> 459
<212> DNA
<213> Homo sapiens

<220>
<221> misc_feature
<222> 219, 389, 397, 426, 458
<223> n = A,T,C or G

<400> 142
accaggttaa tattgccaca tatatccttt ccaattgcgg gctaaacaga cgtgtattta 60
gggttgttta aagacaaccc agcttaatat caagagaaat tgtgaccttt catggagtat 120
ctgatggaga aaacactgag ttttgacaaa tcttatttta ttcagatagc agtctgatca 180

cacatggtcc aacaacactc aaataataaa tcaaatatna tcagatgtta aagattggtc 240
ttcaaacatc atagccaatg atgccccgct tgcctataat ctctccgaca taaaaccaca 300
tcaacacctc agtggccacc aaaccattca gcacagcttc cttaactgtg agctgtttga 360
agctaccagt ctgagcacta ttgactatnt ttttcangct ctgaatagct ctagggatct 420
cagcangggt gggaggaacc agctcaacct tggcgtant 459

<210> 143
<211> 140
<212> DNA
<213> Homo sapiens

<400> 143
acatttcctt ccaccaagtc aggactcctg gcttctgtgg gagttcttat cacctgaggg 60
aaatccaaac agtctctcct agaaaggaat agtgtcacca accccaccca tctccctgag 120
accatccgac ttccctgtgt 140

<210> 144
<211> 164
<212> DNA
<213> Homo sapiens

<220>
<221> misc_feature
<222> 90, 99
<223> n = A,T,C or G

<400> 144
acttcagtaa caacatacaa taacaacatt aagtgtatat tgccatcttt gtcattttct 60
atctatacca ctctcccttc tgaaaacaan aatcactanc caatcactta tacaaatttg 120
aggcaattaa tccatatttg ttttcaataa ggaaaaaaag atgt 164

<210> 145
<211> 303
<212> DNA
<213> Homo sapiens

<220>
<221> misc_feature
<222> 44, 250
<223> n = A,T,C or G

<400> 145
acgtagacca tccaactttg tatttgtaat ggcaaacatc cagnagcaat tcctaaacaa 60
actggagggt atttataccc aattatccca ttcattaaca tgccctcctc ctcaggctat 120
gcaggacagc tatcataagt cggcccaggc atccagatac taccatttgt ataaacttca 180
gtaggggagt ccatccaagt gacaggtcta atcaaaggag gaaatggaac ataagcccag 240
tagtaaaatn ttgcttagct gaaacagcca caaaagactt accgccgtgg tgattaccat 300
caa 303

<210> 146
<211> 327
<212> DNA
<213> Homo sapiens

<220>
<221> misc_feature
<222> 39
<223> n = A,T,C or G

<400> 146
actgcagctc aattagaagt ggtctctgac tttcatcanc ttctccctgg gctccatgac 60
actggcctgg agtgactcat tgctctggtt ggttgagaga gctcctttgc caacaggcct 120
ccaagtcagg gctgggattt gtttcctttc cacattctag caacaatatg ctggccactt 180
cctgaacagg gagggtggga ggagccagca tggaacaagc tgccactttc taaagtagcc 240
agacttgccc ctgggcctgt cacacctact gatgaccttc tgtgcctgca ggatggaatg 300
taggggtgag ctgtgtgact ctatggt 327

<210> 147

<211> 173
<212> DNA
<213> Homo sapiens

<220>
<221> misc_feature
<222> 29
<223> n = A,T,C or G

<400> 147
acattgtttt tttgagataa agcattgana gagctctcct taacgtgaca caatggaagg 60
actggaacac atacccacat ctttgttctg agggataatt ttctgataaa gtcttgctgt 120
atattcaagc acatatgtta tatattattc agttccatgt ttatagccta gtt 173

<210> 148
<211> 477
<212> DNA
<213> Homo sapiens

<220>
<221> misc_feature
<222> 205, 241, 245, 309, 405
<223> n = A,T,C or G

<400> 148
acaaccactt tatctcatcg aatttttaac ccaaactcac tcactgtgcc tttctatcct 60
atgggatata ttatttgatg ctccatttca tcacacatat atgaataata cactcatact 120
gccctactac ctgctgcaat aatcacattc ccttcctgtc ctgaccctga agccattggg 180
gtggtcctag tggccatcag tccangcctg caccttgagc ccttgagctc cattgctcac 240
nccancccac ctcaccgacc ccatcctctt acacagctac ctccttgctc tctaacccca 300
tagattatnt ccaaattcag tcaattaagt tactattaac actctacccg acatgtccag 360
caccactggt aagccttctc cagccaacac acacacacac acacncacac acacacatat 420
ccaggcacag gctacctcat cttcacaatc acccctttaa ttaccatgct atggtgg 477

<210> 149
<211> 207
<212> DNA
<213> Homo sapiens

<400> 149
acagttgtat tataatatca agaaataaac ttgcaatgag agcatttaag agggaagaac 60
taacgtattt tagagagcca aggaaggttt ctgtggggag tgggatgtaa ggtggggcct 120
gatgataaat aagagtcagc caggtaagtg ggtggtgtgg tatgggcaca gtgaagaaca 180
tttcaggcag agggaacagc agtgaaa 207

<210> 150
<211> 111
<212> DNA
<213> Homo sapiens

<220>
<221> misc_feature
<222> 97
<223> n = A,T,C or G

<400> 150
accttgattt cattgctgct ctgatggaaa cccaactatc taatttagct aaaacatggg 60
cacttaaatg tggtcagtgt ttggacttgt taactantgg catctttggg t 111

<210> 151
<211> 196
<212> DNA
<213> Homo sapiens

<400> 151
agcgcggcag gtcatattga acattccaga tacctatcat tactcgatgc tgttgataac 60
agcaagatgg ctttgaactc agggtcacca ccagctattg gaccttacta tgaaaaccat 120
ggataccaac cggaaaaccc ctatcccgca cagcccactg tggtccccac tgtctacgag 180

gtgcatccgg ctcagt 196

<210> 152
<211> 132
<212> DNA
<213> Homo sapiens

<400> 152
acagcacttt cacatgtaag aagggagaaa ttcctaaatg taggagaaag ataacagaac 60
cttccccttt tcatctagtg gtggaaacct gatgctttat gttgacagga atagaaccag 120
gagggagttt gt 132

<210> 153
<211> 285
<212> DNA
<213> Homo sapiens

<220>
<221> misc_feature
<222> 5, 11, 14, 278
<223> n = A,T,C or G

<400> 153
acaanaccca nganaggcca ctggccgtgg tgtcatggcc tccaaacatg aaagtgtcag 60
cttctgctct tatgtcctca tctgacaact ctttaccatt tttatcctcg ctcagcagga 120
gcacatcaat aaagtccaaa gtcttggact tggccttggc ttggaggaag tcatcaacac 180
cctggctagt gagggtgcgg cgccgctcct ggatgacggc atctgtgaag tcgtgcacca 240
gtctgcaggc cctgtggaag cgccgtccac acggagtnag gaatt 285

<210> 154
<211> 333
<212> DNA
<213> Homo sapiens

<400> 154
accacagtcc tgttgggcca gggcttcatg accctttctg tgaaaagcca tattatcacc 60
accccaaatt tttccttaaa tatctttaac tgaaggggtc agcctcttga ctgcaaagac 120
cctaagccgg ttacacagct aactcccact ggccctgatt tgtgaaattg ctgctgcctg 180
attggcacag gagtcgaagg tgttcagctc ccctcctccg tggaacgaga ctctgatttg 240
agtttcacaa attctcgggc cacctcgtca ttgctcctct gaaataaaat ccggagaatg 300
gtcaggcctg tctcatccat atggatcttc cgg 333

<210> 155
<211> 308
<212> DNA
<213> Homo sapiens

<220>
<221> misc_feature
<222> 255, 285
<223> n = A,T,C or G

<400> 155
actggaaata ataaaaccca catcacagtg ttgtgtcaaa gatcatcagg gcatggatgg 60
gaaagtgctt tgggaactgt aaagtgccta acacatgatc gatgattttt gttataatat 120
ttgaatcacg gtgcatacaa actctcctgc ctgctcctcc tgggccccag ccccagcccc 180
atcacagctc actgctctgt tcatccaggc ccagcatgta gtggctgatt cttcttggct 240
gcttttagcc tccanaagtt tctctgaagc caaccaaacc tctangtgta aggcatgctg 300
gccctggt 308

<210> 156
<211> 295
<212> DNA
<213> Homo sapiens

<400> 156
accttgctcg gtgcttggaa catattagga actcaaaata tgagatgata acagtgccta 60
ttattgatta ctgagagaac tgttagacat ttagttgaag attttctaca caggaactga 120

gaataggaga ttatgtttgg ccctcatatt ctctcctatc ctccttgcct cattctatgt 180
ctaatatatt ctcaatcaaa taaggttagc ataatcagga aatcgaccaa ataccaatat 240
aaaaccagat gtctatcctt aagattttca aatagaaaac aaattaacag actat 295

<210> 157
<211> 126
<212> DNA
<213> Homo sapiens

<400> 157
acaagtttaa atagtgctgt cactgtgcat gtgctgaaat gtgaaatcca ccacatttct 60
gaagagcaaa acaaattctg tcatgtaatc tctatcttgg gtcgtgggta tatctgtccc 120
cttagt 126

<210> 158
<211> 442
<212> DNA
<213> Homo sapiens

<220>
<221> misc_feature
<222> 63, 241, 361, 397
<223> n = A,T,C or G

<400> 158
acccactggt cttggaaaca cccatcctta atacgatgat ttttctgtcg tgtgaaaatg 60
aanccagcag gctgccccta gtcagtcctt ccttccagag aaaaagagat ttgagaaagt 120
gcctgggtaa ttcaccatta atttcctccc ccaaactctc tgagtcttcc cttaatattt 180
ctggtggttc tgaccaaagc aggtcatggt ttgttgagca tttgggatcc cagtgaagta 240
natgtttgta gccttgcata cttagccctt cccacgcaca aacggagtgg cagagtggtg 300
ccaaccctgt tttcccagtc cacgtagaca gattcacagt gcggaattct ggaagctgga 360
nacagacggg ctctttgcag agccgggact ctgagangga catgagggcc tctgcctctg 420
tgttcattct ctgatgtcct gt 442

<210> 159
<211> 498
<212> DNA
<213> Homo sapiens

<220>
<221> misc_feature
<222> 176, 194, 257, 263, 302, 305, 341, 348, 389, 420, 429, 454,
471
<223> n = A,T,C or G

<400> 159
acttccaggt aacgttgttg tttccgttga gcctgaactg atgggtgacg ttgtaggttc 60
tccaacaaga actgaggttg cagagcgggt agggaagagt gctgttccag ttgcacctgg 120
gctgctgtgg actgttgttg attcctcact acggcccaag gttgtggaac tggcanaaag 180
gtgtgttgtt gganttgagc tcgggcggct gtggtaggtt gtgggctctt caacaggggc 240
tgctgtggtg ccgggangtg aangtgttgt gtcacttgag cttggccagc tctggaaagt 300
antanattct tcctgaaggc cagcgcttgt ggagctggca ngggtcantg ttgtgtgtaa 360
cgaaccagtg ctgctgtggg tgggtgtana tcctccacaa agcctgaagt tatggtgtcn 420
tcaggtaana atgtggtttc agtgtccctg ggcngctgtg gaaggttgta nattgtcacc 480
aagggaataa gctgtggt 498

<210> 160
<211> 380
<212> DNA
<213> Homo sapiens

<220>
<221> misc_feature
<222> 145
<223> n = A,T,C or G

<400> 160
acctgcatcc agcttccctg ccaaactcac aaggagacat caacctctag acagggaaac 60

agcttcagga tacttccagg agacagagcc accagcagca aaacaaatat tcccatgcct 120
ggagcatggc atagaggaag ctganaaatg tggggtctga ggaagccatt tgagtctggc 180
cactagacat ctcatcagcc acttgtgtga agagatgccc catgacccca gatgcctctc 240
ccacccttac ctccatctca cacacttgag ctttccactc tgtataattc taacatcctg 300
gagaaaaatg gcagtttgac cgaacctgtt cacaacggta gaggctgatt tctaacgaaa 360
cttgtagaat gaagcctgga 380

<210> 161
<211> 114
<212> DNA
<213> Homo sapiens

<400> 161
actccacatc ccctctgagc aggcggttgt cgttcaaggt gtatttggcc ttgcctgtca 60
cactgtccac tggcccctta tccacttggt gcttaatccc tcgaaagagc atgt 114

<210> 162
<211> 177
<212> DNA
<213> Homo sapiens

<400> 162
actttctgaa tcgaatcaaa tgatacttag tgtagtttta atatcctcat atatatcaaa 60
gttttactac tctgataatt ttgtaaacca ggtaaccaga acatccagtc atacagcttt 120
tggtgatata taacttggca ataacccagt ctggtgatac ataaaactac tcactgt 177

<210> 163
<211> 137
<212> DNA
<213> Homo sapiens

<220>
<221> misc_feature
<222> 63
<223> n = A,T,C or G

<400> 163
catttataca gacaggcgtg aagacattca cgacaaaaac gcgaaattct atcccgtgac 60
canagaaggc agctacggct actcctacat cctggcgtgg gtggccttcg cctgcacctt 120
catcagcggc atgatgt 137

<210> 164
<211> 469
<212> DNA
<213> Homo sapiens

<220>
<221> misc_feature
<222> 453
<223> n = A,T,C or G

<400> 164
cttatcacaa tgaatgttct cctgggcagc gttgtgatct ttgccacctt cgtgacttta 60
tgcaatgcat catgctattt catacctaat gagggagttc caggagattc aaccaggaaa 120
tgcatggatc tcaaaggaaa caaacaccca ataaactcgg agtggcagac tgacaactgt 180
gagacatgca cttgctacga aacagaaatt tcatgttgca cccttgtttc tacacctgtg 240
ggttatgaca aagacaactg ccaaagaatc ttcaagaagg aggactgcaa gtatatcgtg 300
gtggagaaga aggacccaaa aaagacctgt tctgtcagtg aatggataat ctaatgtgct 360
tctagtaggc acagggctcc caggccaggc ctcattctcc tctggcctct aatagtcaat 420
gattgtgtag ccatgcctat cagtaaaaag atntttgagc aaacacttt 469

<210> 165
<211> 195
<212> DNA
<213> Homo sapiens

<220>
<221> misc_feature

<222> 14
<223> n = A,T,C or G

<400> 165
acagtttttt atanatatcg acattgccgg cacttgtgtt cagtttcata aagctggtgg 60
atccgctgtc atccactatt ccttggctag agtaaaaatt attcttatag cccatgtccc 120
tgcaggccgc ccgcccgtag ttctcgttcc agtcgtcttg gcacacaggg tgccaggact 180
tcctctgaga tgagt 195

<210> 166
<211> 383
<212> DNA
<213> Homo sapiens

<220>
<221> misc_feature
<222> 258, 283, 303, 323, 361
<223> n = A,T,C or G

<400> 166
acatcttagt agtgtggcac atcagggggc catcagggtc acagtcactc atagcctcgc 60
cgaggtcgga gtccacacca ccggtgtagg tgtgctcaat cttgggcttg gcgcccacct 120
ttggagaagg gatatgctgc acacacatgt ccacaaagcc tgtgaactcg ccaaagaatt 180
tttgcagacc agcctgagca aggggcggat gttcagcttc agctcctcct tcgtcaggtg 240
gatgccaacc tcgtctangg tccgtgggaa gctggtgtcc acntcaccta caacctgggc 300
gangatctta taaagaggct ccnagataaa ctccacgaaa cttctctggg agctgctagt 360
nggggccttt ttggtgaact ttc 383

<210> 167
<211> 247
<212> DNA
<213> Homo sapiens

<220>
<221> misc_feature
<222> 29, 58, 125, 157, 172, 190, 224, 244
<223> n = A,T,C or G

<400> 167
acagagccag accttggcca taaatgaanc agagattaag actaaacccc aagtcganat 60
tggagcagaa actggagcaa gaagtgggcc tggggctgaa gtagagacca aggccactgc 120
tatanccata cacagagcca actctcaggc caaggcnatg gttggggcag anccagagac 180
tcaatctgan tccaaagtgg tggctggaac actggtcatg acanaggcag tgactctgac 240
tgangtc 247

<210> 168
<211> 273
<212> DNA
<213> Homo sapiens

<220>
<221> misc_feature
<222> 34, 70, 84, 99, 138
<223> n = A,T,C or G

<400> 168
acttctaagt tttctagaag tggaaggatt gtantcatcc tgaaaatggg tttacttcaa 60
aatccctcan ccttgttctt cacnactgtc tatactgana gtgtcatgtt tccacaaagg 120
gctgacacct gagcctgnat tttcactcat ccctgagaag ccctttccag tagggtgggc 180
aattcccaac ttccttgcca caagcttccc aggctttctc ccctggaaaa ctccagcttg 240
agtcccagat acactcatgg gctgccctgg gca 273

<210> 169
<211> 431
<212> DNA
<213> Homo sapiens

<220>

<221> misc_feature
<222> 199, 335
<223> n = A,T,C or G

<400> 169
acagccttgg cttccccaaa ctccacagtc tcagtgcaga aagatcatct tccagcagtc 60
agctcagacc agggtcaaag gatgtgacat caacagtttc tggtttcaga acaggttcta 120
ctactgtcaa atgacccccc atacttcctc aaaggctgtg gtaagttttg cacaggtgag 180
ggcagcagaa agggggtant tactgatgga caccatcttc tctgtatact ccacactgac 240
cttgccatgg gcaaaggccc ctaccacaaa aacaatagga tcactgctgg gcaccagctc 300
acgcacatca ctgacaaccg ggatggaaaa agaantgcca actttcatac atccaactgg 360
aaagtgatct gatactggat tcttaattac cttcaaaagc ttctgggggc catcagctgc 420
tcgaacactg a 431

<210> 170
<211> 266
<212> DNA
<213> Homo sapiens

<220>
<221> misc_feature
<222> 89, 98, 103, 203, 218, 227, 232
<223> n = A,T,C or G

<400> 170
acctgtgggc tgggctgtta tgcctgtgcc ggctgctgaa agggagttca gaggtggagc 60
tcaaggagct ctgcaggcat tttgccaanc ctctccanag canagggagc aacctacact 120
ccccgctaga aagacaccag attggagtcc tgggaggggg agttggggtg ggcatttgat 180
gtatacttgt cacctgaatg aangagccag agaggaanga gacgaanatg anattggcct 240
tcaaagctag gggtctggca ggtgga 266

<210> 171
<211> 1248
<212> DNA
<213> Homo sapiens

<220>
<221> misc_feature
<222> 1028, 1067, 1103, 1168, 1209
<223> n = A,T,C or G

<400> 171
   ggcagccaaa tcataaacgg cgaggactgc agcccgcact cgcagccctg gcaggcggca 60
   ctggtcatgg aaaacgaatt gttctgctcg ggcgtcctgg tgcatccgca gtgggtgctg 120
   tcagccgcac actgtttcca gaagtgagtg cagagctcct acaccatcgg gctgggcctg 180
   cacagtcttg aggccgacca agagccaggg agccagatgg tggaggccag cctctccgta 240
   cggcacccag agtacaacag acccttgctc gctaacgacc tcatgctcat caagttggac 300
   gaatccgtgt ccgagtctga caccatccgg agcatcagca ttgcttcgca gtgccctacc 360
   gcggggaact cttgcctcgt ttctggctgg ggtctgctgg cgaacggcag aatgcctacc 420
   gtgctgcagt gcgtgaacgt gtcggtggtg tctgaggagg tctgcagtaa gctctatgac 480
   ccgctgtacc accccagcat gttctgcgcc ggcggagggc aagaccagaa ggactcctgc 540
   aacggtgact ctgggggccc cctgatctgc aacgggtact tgcagggcct tgtgtctttc 600
   ggaaaagccc cgtgtggcca agttggcgtg ccaggtgtct acaccaacct ctgcaaattc 660
   actgagtgga tagagaaaac cgtccaggcc agttaactct ggggactggg aacccatgaa 720
   attgaccccc aaatacatcc tgcggaagga attcaggaat atctgttccc agcccctcct 780
   ccctcaggcc caggagtcca ggcccccagc ccctcctccc tcaaaccaag ggtacagatc 840
   cccagcccct cctccctcag acccaggagt ccagaccccc cagcccctcc tccctcagac 900
   ccaggagtcc agcccctcct ccctcagacc caggagtcca gaccccccag cccctcctcc 960
   ctcagaccca ggggtccagg cccccaaccc ctcctccctc agactcagag gtccaagccc 1020
   ccaacccntc attccccaga cccagaggtc caggtcccag cccctcntcc ctcagaccca 1080
   gcggtccaat gccacctaga ctntccctgt acacagtgcc cccttgtggc acgttgaccc 1140
   aaccttacca gttggttttt catttttngt ccctttcccc tagatccaga aataaagttt 1200
   aagagaagng caaaaaaaaa aaaaaaaaaa aaaaaaaaaa aaaaaaaa 1248

<210> 172
<211> 159
<212> PRT
<213> Homo sapiens

<220>
<221> VARIANT
<222> 103, 105
<223> Xaa = Any Amino Acid

<400> 172
Met Val Glu Ala Ser Leu Ser Val Arg His Pro Glu Tyr Asn Arg Pro
1               5                   10                  15
Leu Leu Ala Asn Asp Leu Met Leu Ile Lys Leu Asp Glu Ser Val Ser
            20                  25                  30
Glu Ser Asp Thr Ile Arg Ser Ile Ser Ile Ala Ser Gln Cys Pro Thr
        35                  40                  45
Ala Gly Asn Ser Cys Leu Val Ser Gly Trp Gly Leu Leu Ala Asn Gly
    50                  55                  60
Arg Met Pro Thr Val Leu Gln Cys Val Asn Val Ser Val Val Ser Glu
65                  70                  75                  80
Glu Val Cys Ser Lys Leu Tyr Asp Pro Leu Tyr His Pro Ser Met Phe
                85                  90                  95
Cys Ala Gly Gly Gly Gln Xaa Gln Xaa Asp Ser Cys Asn Gly Asp Ser
            100                 105                 110
Gly Gly Pro Leu Ile Cys Asn Gly Tyr Leu Gln Gly Leu Val Ser Phe
        115                 120                 125
Gly Lys Ala Pro Cys Gly Gln Val Gly Val Pro Gly Val Tyr Thr Asn
    130                 135                 140
Leu Cys Lys Phe Thr Glu Trp Ile Glu Lys Thr Val Gln Ala Ser
145                 150                 155


<210> 173
<211> 1265
<212> DNA
<213> Homo sapiens

<220>
<221> misc_feature
<222> 967, 1094, 1095, 1115, 1126, 1154, 1174, 1176, 1212, 1251,
1253
<223> n = A,T,C or G

<400> 173
ggcagcccgc actcgcagcc ctggcaggcg gcactggtca tggaaaacga attgttctgc 60
tcgggcgtcc tggtgcatcc gcagtgggtg ctgtcagccg cacactgttt ccagaactcc 120
tacaccatcg ggctgggcct gcacagtctt gaggccgacc aagagccagg gagccagatg 180
gtggaggcca gcctctccgt acggcaccca gagtacaaca gacccttgct cgctaacgac 240
ctcatgctca tcaagttgga cgaatccgtg tccgagtctg acaccatccg gagcatcagc 300
attgcttcgc agtgccctac cgcggggaac tcttgcctcg tttctggctg gggtctgctg 360
gcgaacggtg agctcacggg tgtgtgtctg ccctcttcaa ggaggtcctc tgcccagtcg 420
cgggggctga cccagagctc tgcgtcccag gcagaatgcc taccgtgctg cagtgcgtga 480
acgtgtcggt ggtgtctgag gaggtctgca gtaagctcta tgacccgctg taccacccca 540
gcatgttctg cgccggcgga gggcaagacc agaaggactc ctgcaacggt gactctgggg 600
ggcccctgat ctgcaacggg tacttgcagg gccttgtgtc tttcggaaaa gccccgtgtg 660
gccaagttgg cgtgccaggt gtctacacca acctctgcaa attcactgag tggatagaga 720
aaaccgtcca ggccagttaa ctctggggac tgggaaccca tgaaattgac ccccaaatac 780
atcctgcgga aggaattcag gaatatctgt tcccagcccc tcctccctca ggcccaggag 840
tccaggcccc cagcccctcc tccctcaaac caagggtaca gatccccagc ccctcctccc 900
tcagacccag gagtccagac cccccagccc ctcctccctc agacccagga gtccagcccc 960
tcctccntca gacccaggag tccagacccc ccagcccctc ctccctcaga cccaggggtt 1020
gaggccccca acccctcctc cttcagagtc agaggtccaa gcccccaacc cctcgttccc 1080
cagacccaga ggtnnaggtc ccagcccctc ttccntcaga cccagnggtc caatgccacc 1140
tagattttcc ctgnacacag tgcccccttg tggnangttg acccaacctt accagttggt 1200
ttttcatttt tngtcccttt cccctagatc cagaaataaa gtttaagaga ngngcaaaaa 1260
aaaaa                                                             1265

<210> 174
<211> 1459
<212> DNA
<213> Homo sapiens

<220>
<221> misc_feature
<222> 421, 432, 462
<223> n = A,T,C or G

<400> 174
ggtcagccgc acactgtttc cagaagtgag tgcagagctc ctacaccatc gggctgggcc 60
tgcacagtct tgaggccgac caagagccag ggagccagat ggtggaggcc agcctctccg 120
tacggcaccc agagtacaac agacccttgc tcgctaacga cctcatgctc atcaagttgg 180
acgaatccgt gtccgagtct gacaccatcc ggagcatcag cattgcttcg cagtgcccta 240
ccgcggggaa ctcttgcctc gtttctggct ggggtctgct ggcgaacggt gagctcacgg 300
gtgtgtgtct gccctcttca aggaggtcct ctgcccagtc gcgggggctg acccagagct 360
ctgcgtccca ggcagaatgc ctaccgtgct gcagtgcgtg aacgtgtcgg tggtgtctga 420
ngaggtctgc antaagctct atgacccgct gtaccacccc ancatgttct gcgccggcgg 480
agggcaagac cagaaggact cctgcaacgt gagagagggg aaaggggagg gcaggcgact 540
cagggaaggg tggagaaggg ggagacagag acacacaggg ccgcatggcg agatgcagag 600
atggagagac acacagggag acagtgacaa ctagagagag aaactgagag aaacagagaa 660
ataaacacag gaataaagag aagcaaagga agagagaaac agaaacagac atggggaggc 720
agaaacacac acacatagaa atgcagttga ccttccaaca gcatggggcc tgagggcggt 780
gacctccacc caatagaaaa tcctcttata acttttgact ccccaaaaac ctgactagaa 840
atagcctact gttgacgggg agccttacca ataacataaa tagtcgattt atgcatacgt 900
tttatgcatt catgatatac ctttgttgga attttttgat atttctaagc tacacagttc 960
gtctgtgaat ttttttaaat tgttgcaact ctcctaaaat ttttctgatg tgtttattga 1020
aaaaatccaa gtataagtgg acttgtgcat tcaaaccagg gttgttcaag ggtcaactgt 1080
gtacccagag ggaaacagtg acacagattc atagaggtga aacacgaaga gaaacaggaa 1140
aaatcaagac tctacaaaga ggctgggcag ggtggctcat gcctgtaatc ccagcacttt 1200
gggaggcgag gcaggcagat cacttgaggt aaggagttca agaccagcct ggccaaaatg 1260
gtgaaatcct gtctgtacta aaaatacaaa agttagctgg atatggtggc aggcgcctgt 1320
aatcccagct acttgggagg ctgaggcagg agaattgctt gaatatggga ggcagaggtt 1380
gaagtgagtt gagatcacac cactatactc cagctggggc aacagagtaa gactctgtct 1440
caaaaaaaaa aaaaaaaaa 1459

<210> 175
<211> 1167
<212> DNA
<213> Homo sapiens

<220>
<221> misc_feature
<222> 330, 408, 637, 697, 839, 843, 872, 903, 906, 943, 947, 999,
1050, 1052, 1081, 1148
<223> n = A,T,C or G

<400> 175
   gcgcagccct ggcaggcggc actggtcatg gaaaacgaat tgttctgctc gggcgtcctg 60
   gtgcatccgc agtgggtgct gtcagccgca cactgtttcc agaactccta caccatcggg 120
   ctgggcctgc acagtcttga ggccgaccaa gagccaggga gccagatggt ggaggccagc 180
   ctctccgtac ggcacccaga gtacaacaga ctcttgctcg ctaacgacct catgctcatc 240
   aagttggacg aatccgtgtc cgagtctgac accatccgga gcatcagcat tgcttcgcag 300
   tgccctaccg cggggaactc ttgcctcgtn tctggctggg gtctgctggc gaacggcaga 360
   atgcctaccg tgctgcactg cgtgaacgtg tcggtggtgt ctgaggangt ctgcagtaag 420
   ctctatgacc cgctgtacca ccccagcatg ttctgcgccg gcggagggca agaccagaag 480
   gactcctgca acggtgactc tgggggcccc ctgatctgca acgggtactt gcagggcctt 540
   gtgtctttcg gaaaagcccc gtgtggccaa cttggcgtgc caggtgtcta caccaacctc 600
   tgcaaattca ctgagtggat agagaaaacc gtccagncca gttaactctg gggactggga 660
   acccatgaaa ttgaccccca aatacatcct gcggaangaa ttcaggaata tctgttccca 720
   gcccctcctc cctcaggccc aggagtccag gcccccagcc cctcctccct caaaccaagg 780
   gtacagatcc ccagcccctc ctccctcaga cccaggagtc cagacccccc agcccctcnt 840
   ccntcagacc caggagtcca gcccctcctc cntcagacgc aggagtccag accccccagc 900
   ccntcntccg tcagacccag gggtgcaggc ccccaacccc tcntccntca gagtcagagg 960
   tccaagcccc caacccctcg ttccccagac ccagaggtnc aggtcccagc ccctcctccc 1020
   tcagacccag cggtccaatg ccacctagan tntccctgta cacagtgccc ccttgtggca 1080
   ngttgaccca accttaccag ttggtttttc attttttgtc cctttcccct agatccagaa 1140
   ataaagtnta agagaagcgc aaaaaaa 1167

<210> 176
<211> 205

<212> PRT
<213> Homo sapines

<220>
<221> VARIANT
<222> 127, 204
<223> Xaa = Any Amino Acid

<400> 176
Met Glu Asn Glu Leu Phe Cys Ser Gly Val Leu Val His Pro Gln Trp
 1               5                  10                  15
Val Leu Ser Ala Ala His Cys Phe Gln Asn Ser Tyr Thr Ile Gly Leu
            20                  25                  30
Gly Leu His Ser Leu Glu Ala Asp Gln Glu Pro Gly Ser Gln Met Val
        35                  40                  45
Glu Ala Ser Leu Ser Val Arg His Pro Glu Tyr Asn Arg Leu Leu Leu
    50                  55                  60
Ala Asn Asp Leu Met Leu Ile Lys Leu Asp Glu Ser Val Ser Glu Ser
65                  70                  75                  80
Asp Thr Ile Arg Ser Ile Ser Ile Ala Ser Gln Cys Pro Thr Ala Gly
                85                  90                  95
Asn Ser Cys Leu Val Ser Gly Trp Gly Leu Leu Ala Asn Gly Arg Met
            100                 105                 110
Pro Thr Val Leu His Cys Val Asn Val Ser Val Val Ser Glu Xaa Val
        115                 120                 125
Cys Ser Lys Leu Tyr Asp Pro Leu Tyr His Pro Ser Met Phe Cys Ala
    130                 135                 140
Gly Gly Gly Gln Asp Gln Lys Asp Ser Cys Asn Gly Asp Ser Gly Gly
145                 150                 155                 160
Pro Leu Ile Cys Asn Gly Tyr Leu Gln Gly Leu Val Ser Phe Gly Lys
                165                 170                 175
Ala Pro Cys Gly Gln Leu Gly Val Pro Gly Val Tyr Thr Asn Leu Cys
            180                 185                 190
Lys Phe Thr Glu Trp Ile Glu Lys Thr Val Gln Xaa Ser
        195                 200                 205

<210> 177
<211> 1119
<212> DNA
<213> Homo sapiens

<400> 177
gcgcactcgc agccctggca ggcggcactg gtcatggaaa acgaattgtt ctgctcgggc 60
gtcctggtgc atccgcagtg ggtgctgtca gccgcacact gtttccagaa ctcctacacc 120
atcgggctgg gcctgcacag tcttgaggcc gaccaagagc cagggagcca gatggtggag 180
gccagcctct ccgtacggca cccagagtac aacagaccct tgctcgctaa cgacctcatg 240
ctcatcaagt tggacgaatc cgtgtccgag tctgacacca tccggagcat cagcattgct 300
tcgcagtgcc ctaccgcggg gaactcttgc ctcgtttctg gctggggtct gctggcgaac 360
gatgctgtga ttgccatcca gtcccagact gtgggaggct gggagtgtga gaagctttcc 420
caaccctggc agggttgtac catttcggca acttccagtg caaggacgtc ctgctgcatc 480
ctcactgggt gctcactact gctcactgca tcacccggaa cactgtgatc aactagccag 540
caccatagtt ctccgaagtc agactatcat gattactgtg ttgactgtgc tgtctattgt 600
actaaccatg ccgatgttta ggtgaaatta gcgtcacttg gcctcaacca tcttggtatc 660
cagttatcct cactgaattg agatttcctg cttcagtgtc agccattccc acataatttc 720
tgacctacag aggtgaggga tcatatagct cttcaaggat gctggtactc ccctcacaaa 780
ttcatttctc ctgttgtagt gaaaggtgcg ccctctggag cctcccaggg tgggtgtgca 840
ggtcacaatg atgaatgtat gatcgtgttc ccattaccca aagcctttaa atccctcatg 900
ctcagtacac cagggcaggt ctagcatttc ttcatttagt gtatgctgtc cattcatgca 960
accacctcag gactcctgga ttctctgcct agttgagctc ctgcatgctg cctccttggg 1020
gaggtgaggg agagggccca tggttcaatg ggatctgtgc agttgtaaca cattaggtgc 1080
ttaataaaca gaagctgtga tgttaaaaaa aaaaaaaaa                        1119

<210> 178
<211> 164
<212> PRT
<213> Homo sapiens

<220>
<221> VARIANT
<222> 118
<223> Xaa = Any Amino Acid

<400> 178
Met Glu Asn Glu Leu Phe Cys Ser Gly Val Leu Val His Pro Gln Trp
 1               5                  10                  15
Val Leu Ser Ala Ala His Cys Phe Gln Asn Ser Tyr Thr Ile Gly Leu
            20                  25                  30
Gly Leu His Ser Leu Glu Ala Asp Gln Glu Pro Gly Ser Gln Met Val
        35                  40                  45
Glu Ala Ser Leu Ser Val Arg His Pro Glu Tyr Asn Arg Pro Leu Leu
    50                  55                  60
Ala Asn Asp Leu Met Leu Ile Lys Leu Asp Glu Ser Val Ser Glu Ser
65                  70                  75                  80
Asp Thr Ile Arg Ser Ile Ser Ile Ala Ser Gln Cys Pro Thr Ala Gly
                85                  90                  95
Asn Ser Cys Leu Val Ser Gly Trp Gly Leu Leu Ala Asn Asp Ala Val
            100                 105                 110
Ile Ala Ile Gln Ser Xaa Thr Val Gly Gly Trp Glu Cys Glu Lys Leu
        115                 120                 125
Ser Gln Pro Trp Gln Gly Cys Thr Ile Ser Ala Thr Ser Ser Ala Arg
    130                 135                 140
Thr Ser Cys Cys Ile Leu Thr Gly Cys Ser Leu Leu Leu Thr Ala Ser
145                 150                 155                 160
Pro Gly Thr Leu

<210> 179
<211> 250
<212> DNA
<213> Homo sapiens

<400> 179
ctggagtgcc ttggtgtttc aagcccctgc aggaagcaga atgcaccttc tgaggcacct 60
ccagctgccc ccggccgggg gatgcgaggc tcggagcacc cttgcccggc tgtgattgct 120
gccaggcact gttcatctca gcttttctgt ccctttgctc ccggcaagcg cttctgctga 180
aagttcatat ctggagcctg atgtcttaac gaataaaggt cccatgctcc acccgaaaaa 240
aaaaaaaaaa                                                         250

<210> 180
<211> 202
<212> DNA
<213> Homo sapiens

<400> 180
actagtccag tgtggtggaa ttccattgtg ttgggcccaa cacaatggct acctttaaca 60
tcacccagac cccgcccctg cccgtgcccc acgctgctgc taacgacagt atgatgctta 120
ctctgctact cggaaactat ttttatgtaa ttaatgtatg ctttcttgtt tataaatgcc 180
tgatttaaaa aaaaaaaaaa aa                                           202

<210> 181
<211> 558
<212> DNA
<213> Homo sapiens

<220>
<221> misc_feature
<222> 11, 40, 58, 115
<223> n = A,T,C or G

<400> 181
tccytttgkt naggtttkkg agacamccck agacctwaan ctgtgtcaca gacttcyngg 60
aatgtttagg cagtgctagt aatttcytcg taatgattct gttattactt tcctnattct 120
ttattcctct ttcttctgaa gattaatgaa gttgaaaatt gaggtggata aatacaaaaa 180
ggtagtgtga tagtataagt atctaagtgc agatgaaagt gtgttatata tatccattca 240

aaattatgca agttagtaat tactcagggt taactaaatt actttaatat gctgttgaac 300
ctactctgtt ccttggctag aaaaaattat aaacaggact ttgttagttt gggaagccaa 360
attgataata ttctatgttc taaaagttgg gctatacata aattattaag aaatatggaw 420
ttttattccc aggaatatgg kgttcatttt atgaatatta cscrggatag awgtwtgagt 480
aaaaycagtt ttggtwaata ygtwaatatg tcmtaaataa acaakgcttt gacttatttc 540
caaaaaaaaa aaaaaaaa 558

<210> 182
<211> 479
<212> DNA
<213> Homo sapiens

<220>
<221> misc_feature
<222> 359, 361
<223> n = A,T,C or G

<400> 182
acagggwttk grggatgcta agsccccrga rwtygtttga tccaaccctg gcttwttttc 60
agaggggaaa atggggccta gaagttacag mscatytagy tggtgcgmtg gcacccctgg 120
cstcacacag astcccgagt agctgggact acaggcacac agtcactgaa gcaggccctg 180
ttwgcaattc acgttgccac ctccaactta aacattcttc atatgtgatg tccttagtca 240
ctaaggttaa actttcccac ccagaaaagg caacttagat aaaatcttag agtactttca 300
tactmttcta agtcctcttc cagcctcact kkgagtcctm cytgggggtt gataggaant 360
ntctcttggc tttctcaata aartctctat ycatctcatg tttaatttgg tacgcatara 420
awtgstgara aaattaaaat gttctggtty mactttaaaa araaaaaaaa aaaaaaaaa 479

<210> 183
<211> 384
<212> DNA
<213> Homo sapiens

<400> 183
aggcgggagc agaagctaaa gccaaagccc aagaagagtg gcagtgccag cactggtgcc 60
agtaccagta ccaataacag tgccagtgcc agtgccagca ccagtggtgg cttcagtgct 120
ggtgccagcc tgaccgccac tctcacattt gggctcttcg ctggccttgg tggagctggt 180
gccagcacca gtggcagctc tggtgcctgt ggtttctcct acaagtgaga ttttagatat 240
tgttaatcct gccagtcttt ctcttcaagc cagggtgcat cctcagaaac ctactcaaca 300
cagcactcta ggcagccact atcaatcaat tgaagttgac actctgcatt aratctattt 360
gccatttcaa aaaaaaaaaa aaaa 384

<210> 184
<211> 496
<212> DNA
<213> Homo sapiens

<220>
<221> misc_feature
<222> 39
<223> n = A,T,C or G

<400> 184
accgaattgg gaccgctggc ttataagcga tcatgtyynt ccrgtatkac ctcaacgagc 60
agggagatcg agtctatacg ctgaagaaat ttgacccgat gggacaacag acctgctcag 120
cccatcctgc tcggttctcc ccagatgaca aatactctsg acaccgaatc accatcaaga 180
aacgcttcaa ggtgctcatg acccagcaac cgcgccctgt cctctgaggg tcccttaaac 240
tgatgtcttt tctgccacct gttacccctc ggagactccg taaccaaact cttcggactg 300
tgagccctga tgcctttttg ccagccatac tctttggcat ccagtctctc gtggcgattg 360
attatgcttg tgtgaggcaa tcatggtggc atcacccata aagggaacac atttgacttt 420
tttttctcat attttaaatt actacmagaw tattwmagaw waaatgawtt gaaaaactst 480
taaaaaaaaa aaaaaa 496

<210> 185
<211> 384
<212> DNA
<213> Homo sapiens

<400> 185

gctggtagcc tatggcgkgg cccacggagg ggctcctgag gccacggrac agtgacttcc 60
caagtatcyt gcgcsgcgtc ttctaccgtc cctacctgca gatcttcggg cagattcccc 120
aggaggacat ggacgtggcc ctcatggagc acagcaactg ytcgtcggag cccggcttct 180
gggcacaccc tcctggggcc caggcgggca cctgcgtctc ccagtatgcc aactggctgg 240
tggtgctgct cctcgtcatc ttcctgctcg tggccaacat cctgctggtc aacttgctca 300
ttgccatgtt cagttacaca ttcggcaaag tacagggcaa cagcgatctc tactgggaag 360
gcgcagcgtt accgcctcat ccgg 384

<210> 186
<211> 577
<212> DNA
<213> Homo sapiens

<220>
<221> misc_feature
<222> 62, 113, 450, 528, 546, 559
<223> n = A,T,C or G

<400> 186
   gagttagctc ctccacaacc ttgatgaggt cgtctgcagt ggcctctcgc ttcataccgc 60
   tnccatcgtc atactgtagg tttgccacca cytcctggca tcttggggcg gcntaatatt 120
   ccaggaaact ctcaatcaag tcaccgtcga tgaaacctgt gggctggttc tgtcttccgc 180
   tcggtgtgaa aggatctccc agaaggagtg ctcgatcttc cccacacttt tgatgacttt 240
   attgagtcga ttctgcatgt ccagcaggag gttgtaccag ctctctgaca gtgaggtcac 300
   cagccctatc atgccgttga mcgtgccgaa garcaccgag ccttgtgtgg gggkkgaagt 360
   ctcacccaga ttctgcatta ccagagagcc gtggcaaaag acattgacaa actcgcccag 420
   gtggaaaaag amcamctcct ggargtgctn gccgctcctc gtcmgttggt ggcagcgctw 480
   tccttttgac acacaaacaa gttaaaggca ttttcagccc ccagaaantt gtcatcatcc 540
   aagatntcgc acagcactna tccagttggg attaaat 577

<210> 187
<211> 534
<212> DNA
<213> Homo sapiens

<220>
<221> misc_feature
<222> 40, 148, 428, 431
<223> n = A,T,C or G

<400> 187
aacatcttcc tgtataatgc tgtgtaatat cgatccgatn ttgtctgstg agaatycatw 60
actkggaaaa gmaacattaa agcctggaca ctggtattaa aattcacaat atgcaacact 120
ttaaacagtg tgtcaatctg ctcccyynac tttgtcatca ccagtctggg aakaagggta 180
tgccctattc acacctgtta aaagggcgct aagcattttt gattcaacat cttttttttt 240
gacacaagtc cgaaaaaagc aaaagtaaac agttatyaat ttgttagcca attcactttc 300
ttcatgggac agagccatyt gatttaaaaa gcaaattgca taatattgag cttygggagc 360
tgatatttga gcggaagagt agcctttcta cttcaccaga cacaactccc tttcatattg 420
ggatgttnac naaagtwatg tctctwacag atgggatgct tttgtggcaa ttctgttctg 480
aggatctccc agtttattta ccacttgcac aagaaggcgt tttcttcctc aggc 534

<210> 188
<211> 761
<212> DNA
<213> Homo sapiens

<220>
<221> misc_feature
<222> 17, 219, 238, 270, 279, 386, 436, 474, 502, 670, 687, 706,
717, 745, 747, 754
<223> n = A,T,C or G

<400> 188
agaaaccagt atctctnaaa acaacctctc ataccttgtg gacctaattt tgtgtgcgtg 60
tgtgtgtgcg cgcatattat atagacaggc acatcttttt tacttttgta aaagcttatg 120
cctctttggt atctatatct gtgaaagttt taatgatctg ccataatgtc ttggggacct 180
ttgtcttctg tgtaaatggt actagagaaa acacctatnt tatgagtcaa tctagttngt 240
tttattcgac atgaaggaaa tttccagatn acaacactna caaactctcc ctkgackarg 300

ggggacaaag aaaagcaaaa ctgamcataa raaacaatwa cctggtgaga arttgcataa 360
acagaaatwr ggtagtatat tgaarnacag catcattaaa rmgttwtktt wttctccctt 420
gcaaaaaaca tgtacngact tcccgttgag taatgccaag ttgttttttt tatnataaaa 480
cttgcccttc attacatgtt tnaaagtggt gtggtgggcc aaaatattga aatgatggaa 540
ctgactgata aagctgtaca aataagcagt gtgcctaaca agcaacacag taatgttgac 600
atgcttaatt cacaaatgct aatttcatta taaatgtttg ctaaaataca ctttgaacta 660
tttttctgtn ttcccagagc tgagatntta gattttatgt agtatnaagt gaaaaantac 720
gaaaataata acattgaaga aaaananaaa aaanaaaaaa a 761

<210> 189
<211> 482
<212> DNA
<213> Homo sapiens

<220>
<221> misc_feature
<222> 20, 40, 73, 226, 288, 330, 371, 377, 381, 387, 396, 399,
404, 416, 436, 447, 457, 458, 463, 474, 477
<223> n = A,T,C or G

<400> 189
tttttttttt tttgccgatn ctactatttt attgcaggan gtgggggtgt atgcaccgca 60
caccggggct atnagaagca agaaggaagg agggagggca cagccccttg ctgagcaaca 120
aagccgcctg ctgccttctc tgtctgtctc ctggtgcagg cacatgggga gaccttcccc 180
aaggcagggg ccaccagtcc aggggtggga atacaggggg tgggangtgt gcataagaag 240
tgataggcac aggccacccg gtacagaccc ctcggctcct gacaggtnga tttcgaccag 300
gtcattgtgc cctgcccagg cacagcgtan atctggaaaa gacagaatgc tttccttttc 360
aaatttggct ngtcatngaa ngggcanttt tccaanttng gctnggtctt ggtacncttg 420
gttcggccca gctccncgtc caaaaantat tcacccnnct ccnaattgct tgcnggnccc 480
cc 482

<210> 190
<211> 471
<212> DNA
<213> Homo sapiens

<220>
<221> misc_feature
<222> 107, 115, 284, 299, 338, 353, 355, 368, 390, 398, 402, 406,
408, 418, 430, 432, 447, 455, 458, 459
<223> n = A,T,C or G

<400> 190
tttttttttt ttttaaaaca gtttttcaca acaaaattta ttagaagaat agtggttttg 60
aaaactctcg catccagtga gaactaccat acaccacatt acagctngga atgtnctcca 120
aatgtctggt caaatgatac aatggaacca ttcaatctta cacatgcacg aaagaacaag 180
cgcttttgac atacaatgca caaaaaaaaa aggggggggg gaccacatgg attaaaattt 240
taagtactca tcacatacat taagacacag ttctagtcca gtcnaaaatc agaactgcnt 300
tgaaaaattt catgtatgca atccaaccaa agaacttnat tggtgatcat gantnctcta 360
ctacatcnac cttgatcatt gccaggaacn aaaagttnaa ancacncngt acaaaaanaa 420
tctgtaattn anttcaacct ccgtacngaa aaatnttnnt tatacactcc c 471

<210> 191
<211> 402
<212> DNA
<213> Homo sapiens

<220>
<221> misc_feature
<222> 342
<223> n = A,T,C or G

<400> 191
gagggattga aggtctgttc tastgtcggm ctgttcagcc accaactcta acaagttgct 60
gtcttccact cactgtctgt aagcttttta acccagacwg tatcttcata aatagaacaa 120
attcttcacc agtcacatct tctaggacct ttttggattc agttagtata agctcttcca 180
cttcctttgt taagacttca tctggtaaag tcttaagttt tgtagaaagg aattyaattg 240
ctcgttctct aacaatgtcc tctccttgaa gtatttggct gaacaaccca cctaaagtcc 300

ctttgtgcat ccattttaaa tatacttaat agggcattgk tncactaggt taaattctgc 360
aagagtcatc tgtctgcaaa agttgcgtta gtatatctgc ca 402

<210> 192
<211> 601
<212> DNA
<213> Homo sapiens

<220>
<221> misc_feature
<222> 44, 56, 93
<223> n = A,T,C or G

<400> 192
gagctcggat ccaataatct ttgtctgagg gcagcacaca tatncagtgc catggnaact 60
ggtctacccc acatgggagc agcatgccgt agntatataa ggtcattccc tgagtcagac 120
atgcytyttt gaytaccgtg tgccaagtgc tggtgattct yaacacacyt ccatcccgyt 180
cttttgtgga aaaactggca cttktctgga actagcarga catcacttac aaattcaccc 240
acgagacact tgaaaggtgt aacaaagcga ytcttgcatt gctttttgtc cctccggcac 300
cagttgtcaa tactaacccg ctggtttgcc tccatcacat ttgtgatctg tagctctgga 360
tacatctcct gacagtactg aagaacttct tcttttgttt caaaagcarc tcttggtgcc 420
tgttggatca ggttcccatt tcccagtcyg aatgttcaca tggcatattt wacttcccac 480
aaaacattgc gatttgaggc tcagcaacag caaatcctgt tccggcattg gctgcaagag 540
cctcgatgta gccggccagc gccaaggcag gcgccgtgag ccccaccagc agcagaagca 600
g 601

<210> 193
<211> 608
<212> DNA
<213> Homo sapiens

<220>
<221> misc_feature
<222> 11
<223> n = A,T,C or G

<400> 193
atacagccca natcccacca cgaagatgcg cttgttgact gagaacctga tgcggtcact 60
ggtcccgctg tagccccagc gactctccac ctgctggaag cggttgatgc tgcactcytt 120
cccaacgcag gcagmagcgg gsccggtcaa tgaactccay tcgtggcttg gggtkgacgg 180
tkaagtgcag gaagaggctg accacctcgc ggtccaccag gatgcccgac tgtgcgggac 240
ctgcagcgaa actcctcgat ggtcatgagc gggaagcgaa tgaggcccag ggccttgccc 300
agaaccttcc gcctgttctc tggcgtcacc tgcagctgct gccgctgaca ctcggcctcg 360
gaccagcgga caaacggcrt tgaacagccg cacctcacgg atgcccagtg tgtcgcgctc 420
caggammgsc accagcgtgt ccaggtcaat gtcggtgaag ccctccgcgg gtratggcgt 480
ctgcagtgtt tttgtcgatg ttctccaggc acaggctggc cagctgcggt tcatcgaaga 540
gtcgcgcctg cgtgagcagc atgaaggcgt tgtcggctcg cagttcttct tcaggaactc 600
cacgcaat 608

<210> 194
<211> 392
<212> DNA
<213> Homo sapiens

<220>
<221> misc_feature
<222> 354, 389
<223> n = A,T,C or G

<400> 194
gaacggctgg accttgcctc gcattgtgct tgctggcagg gaataccttg gcaagcagyt 60
ccagtccgag cagccccaga ccgctgccgc ccgaagctaa gcctgcctct ggccttcccc 120
tccgcctcaa tgcagaacca gtagtgggag cactgtgttt agagttaaga gtgaacactg 180
tttgatttta cttgggaatt tcctctgtta tatagctttt cccaatgcta atttccaaac 240
aacaacaaca aaataacatg tttgcctgtt aagttgtata aaagtaggtg attctgtatt 300
taaagaaaat attactgtta catatactgc ttgcaatttc tgtatttatt gktnctstgg 360
aaataaatat agttattaaa ggttgtcant cc 392

<210> 195
<211> 502
<212> DNA
<213> Homo sapiens

<220>
<221> misc_feature
<222> 125, 418, 436, 467, 484, 486, 496
<223> n = A,T,C or G

<400> 195
ccsttkgagg ggtkaggkyc cagttyccga gtggaagaaa caggccagga gaagtgcgtg 60
ccgagctgag gcagatgttc ccacagtgac ccccagagcc stgggstata gtytctgacc 120
cctcncaagg aaagaccacs ttctggggac atgggctgga gggcaggacc tagaggcacc 180
aagggaaggc cccattccgg ggstgttccc cgaggaggaa gggaaggggc tctgtgtgcc 240
ccccasgagg aagaggccct gagtcctggg atcagacacc ccttcacgtg tatccccaca 300
caaatgcaag ctcaccaagg tcccctctca gtccccttcc stacaccctg amcggccact 360
gscscacacc cacccagagc acgccacccg ccatggggar tgtgctcaag gartcgcngg 420
gcarcgtgga catctngtcc cagaaggggg cagaatctcc aatagangga ctgarcmstt 480
gctnanaaaa aaaaanaaaa aa 502

<210> 196
<211> 665
<212> DNA
<213> Homo sapiens

<220>
<221> misc_feature
<222> 572, 630, 660
<223> n = A,T,C or G

<400> 196
ggttacttgg tttcattgcc accacttagt ggatgtcatt tagaaccatt ttgtctgctc 60
cctctggaag ccttgcgcag agcggacttt gtaattgttg gagaataact gctgaatttt 120
wagctgtttk gagttgatts gcaccactgc acccacaact tcaatatgaa aacyawttga 180
actwatttat tatcttgtga aaagtataac aatgaaaatt ttgttcatac tgtattkatc 240
aagtatgatg aaaagcaawa gatatatatt cttttattat gttaaattat gattgccatt 300
attaatcggc aaaatgtgga gtgtatgttc ttttcacagt aatatatgcc ttttgtaact 360
tcacttggtt attttattgt aaatgartta caaaattctt aatttaagar aatggtatgt 420
watatttatt tcattaattt ctttcctkgt ttacgtwaat tttgaaaaga wtgcatgatt 480
tcttgacaga aatcgatctt gatgctgtgg aagtagtttg acccacatcc ctatgagttt 540
ttcttagaat gtataaaggt tgtagcccat cnaacttcaa agaaaaaaat gaccacatac 600
tttgcaatca ggctgaaatg tggcatgctn ttctaattcc aactttataa actagcaaan 660
aagtg 665

<210> 197
<211> 492
<212> DNA
<213> Homo sapiens

<220>
<221> misc_feature
<222> 5, 111, 144, 148, 169, 174, 191, 203, 285, 377, 477, 482,
484
<223> n = A,T,C or G

<400> 197
ttttnttttt ttttttttgc aggaaggatt ccatttattg tggatgcatt ttcacaatat 60
atgtttattg gagcgatcca ttatcagtga aaagtatcaa gtgtttataa natttttagg 120
aaggcagatt cacagaacat gctngtcngc ttgcagtttt acctcgtana gatnacagag 180
aattatagtc naaccagtaa acnaggaatt tacttttcaa aagattaaat ccaaactgaa 240
caaaattcta ccctgaaact tactccatcc aaatattgga ataanagtca gcagtgatac 300
attctcttct gaactttaga ttttctagaa aaatatgtaa tagtgatcag gaagagctct 360
tgttcaaaag tacaacnaag caatgttccc ttaccatagg ccttaattca aactttgatc 420
catttcactc ccatcacggg agtcaatgct acctgggaca cttgtatttt gttcatnctg 480
ancntggctt aa 492

<210> 198

<211> 478
<212> DNA
<213> Homo sapiens

<220>
<221> misc_feature
<222> 4, 10, 17, 24, 25, 28, 49, 58, 64, 70, 80, 85, 87, 100, 154,
157, 169, 174, 213, 236, 241, 255, 290, 295, 418, 441, 444,
460, 475
<223> n = A,T,C or G

<400> 198
tttnttttgn atttcantct gtannaanta ttttcattat gtttattana aaaatatnaa 60
tgtntccacn acaaatcatn ttacntnagt aagaggccan ctacattgta caacatacac 120
tgagtatatt ttgaaaagga caagtttaaa gtanacncat attgccganc atancacatt 180
tatacatggc ttgattgata tttagcacag canaaactga gtgagttacc agaaanaaat 240
natatatgtc aatcngattt aagatacaaa acagatccta tggtacatan catcntgtag 300
gagttgtggc tttatgttta ctgaaagtca atgcagttcc tgtacaaaga gatggccgta 360
agcattctag tacctctact ccatggttaa gaatcgtaca cttatgttta catatgtnca 420
gggtaagaat tgtgttaagt naanttatgg agaggtccan gagaaaaatt tgatncaa 478

<210> 199
<211> 482
<212> DNA
<213> Homo sapiens

<220>
<221> misc_feature
<222> 248, 271, 281, 297, 314, 328, 362, 375, 390, 394, 408, 418,
424, 426, 430, 431, 439, 447, 449, 457, 460
<223> n = A,T,C or G

<400> 199
agtgacttgt cctccaacaa aaccccttga tcaagtttgt ggcactgaca atcagaccta 60
tgctagttcc tgtcatctat tcgctactaa atgcagactg gaggggacca aaaaggggca 120
tcaactccag ctggattatt ttggagcctg caaatctatt cctacttgta cggactttga 180
agtgattcag tttcctctac ggatgagaga ctggctcaag aatatcctca tgcagcttta 240
tgaagccnac tctgaacacg ctggttatct nagatgagaa ncagagaaat aaagtcnaga 300
aaatttacct ggangaaaag aggctttngg ctggggacca tcccattgaa ccttctctta 360
anggacttta agaanaaact accacatgtn tgtngtatcc tggtgccngg ccgtttantg 420
aacntngacn ncacccttnt ggaatanant cttgacngcn tcctgaactt gctcctctgc 480
ga 482

<210> 200
<211> 270
<212> DNA
<213> Homo sapiens

<220>
<221> misc_feature
<222> 204
<223> n = A,T,C or G

<400> 200
cggccgcaag tgcaactcca gctggggccg tgcggacgaa gattctgcca gcagttggtc 60
cgactgcgac gacggcggcg gcgacagtcg caggtgcagc gcgggcgcct ggggtcttgc 120
aaggctgagc tgacgccgca gaggtcgtgt cacgtcccac gaccttgacg ccgtcgggga 180
cagccggaac agagcccggt gaangcggga ggcctcgggg agcccctcgg gaagggcggc 240
ccgagagata cgcaggtgca ggtggccgcc 270

<210> 201
<211> 419
<212> DNA
<213> Homo sapiens

<220>
<221> misc_feature
<222> 164, 172, 175, 217, 309, 339, 346, 357, 375, 378, 383, 393,

400, 409
<223> n = A,T,C or G

<400> 201
tttttttttt ttttggaatc tactgcgagc acagcaggtc agcaacaagt ttattttgca 60
gctagcaagg taacagggta gggcatggtt acatgttcag gtcaacttcc tttgtcgtgg 120
ttgattggtt tgtctttatg ggggcggggt ggggtagggg aaancgaagc anaantaaca 180
tggagtgggt gcaccctccc tgtagaacct ggttacnaaa gcttggggca gttcacctgg 240
tctgtgaccg tcattttctt gacatcaatg ttattagaag tcaggatatc ttttagagag 300
tccactgtnt ctggagggag attagggttt cttgccaana tccaancaaa atccacntga 360
aaaagttgga tgatncangt acngaatacc ganggcatan ttctcatant cggtggcca 419

<210> 202
<211> 509
<212> DNA
<213> Homo sapiens

<220>
<221> misc_feature
<222> 4, 99, 103, 107, 109, 119, 123, 129, 141, 142, 153, 155,
163, 171, 177, 178, 184, 186, 205, 207, 223, 293, 297, 298,
325, 355, 366, 368, 369, 371, 419, 438, 458, 468, 487, 491,
494, 497, 499
<223> n = A,T,C or G

<400> 202
tttntttttt tttttttttt tttttttttt tttttttttt tttttttttt tttttttttt 60
tggcacttaa tccattttta tttcaaaatg tctacaaant ttnaatncnc cattatacng 120
gtnattttnc aaaatctaaa nnttattcaa atntnagcca aantccttac ncaaatnnaa 180
tacncncaaa aatcaaaaat atacntntct ttcagcaaac ttngttacat aaattaaaaa 240
aatatatacg gctggtgttt tcaaagtaca attatcttaa cactgcaaac atntttnnaa 300
ggaactaaaa taaaaaaaaa cactnccgca aaggttaaag ggaacaacaa attcntttta 360
caacancnnc nattataaaa atcatatctc aaatcttagg ggaatatata cttcacacng 420
ggatcttaac ttttactnca ctttgtttat ttttttanaa ccattgtntt gggcccaaca 480
caatggnaat nccnccncnc tggactagt 509

<210> 203
<211> 583
<212> DNA
<213> Homo sapiens

<220>
<221> misc_feature
<222> 379, 506
<223> n = A,T,C or G

<400> 203
tttttttttt ttttttttga cccccctctt ataaaaaaca agttaccatt ttattttact 60
tacacatatt tattttataa ttggtattag atattcaaaa ggcagctttt aaaatcaaac 120
taaatggaaa ctgccttaga tacataattc ttaggaatta gcttaaaatc tgcctaaagt 180
gaaaatcttc tctagctctt ttgactgtaa atttttgact cttgtaaaac atccaaattc 240
atttttcttg tctttaaaat tatctaatct ttccattttt tccctattcc aagtcaattt 300
gcttctctag cctcatttcc tagctcttat ctactattag taagtggctt ttttcctaaa 360
agggaaaaca ggaagagana atggcacaca aaacaaacat tttatattca tatttctacc 420
tacgttaata aaatagcatt ttgtgaagcc agctcaaaag aaggcttaga tccttttatg 480
tccattttag tcactaaacg atatcnaaag tgccagaatg caaaaggttt gtgaacattt 540
attcaaaagc taatataaga tatttcacat actcatcttt ctg 583

<210> 204
<211> 589
<212> DNA
<213> Homo sapiens

<220>
<221> misc_feature
<222> 9, 27, 45, 328, 358, 392, 412, 441, 445, 546, 573
<223> n = A,T,C or G

<400> 204
ttttttttnt tttttttttt ttttttnctc ttcttttttt ttganaatga ggatcgagtt 60
tttcactctc tagatagggc atgaagaaaa ctcatctttc cagctttaaa ataacaatca 120
aatcttttat gctatatcat attttaagtt aaactaatga gtcactggct tatcttctcc 180
tgaaggaaat ctgttcattc ttctcattca tatagttata tcaagtacta ccttgcatat 240
tgagaggttt ttcttctcta tttacacata tatttccatg tgaatttgta tcaaaccttt 300
attttcatgc aaactagaaa ataatgtntt cttttgcata agagaagaga acaatatnag 360
cattacaaaa ctgctcaaat tgtttgttaa gnttatccat tataattagt tnggcaggag 420
ctaatacaaa tcacatttac ngacnagcaa taataaaact gaagtaccag ttaaatatcc 480
aaaataatta aaggaacatt tttagcctgg gtataattag ctaattcact ttacaagcat 540
ttattnagaa tgaattcaca tgttattatt ccntagccca acacaatgg 589

<210> 205
<211> 545
<212> DNA
<213> Homo sapiens

<220>
<221> misc_feature
<222> 6, 122, 375, 378, 428, 431, 478
<223> n = A,T,C or G

<400> 205
tttttntttt ttttttcagt aataatcaga acaatattta tttttatatt taaaattcat 60
agaaaagtgc cttacattta ataaaagttt gtttctcaaa gtgatcagag gaattagata 120
tngtcttgaa caccaatatt aatttgagga aaatacacca aaatacatta agtaaattat 180
ttaagatcat agagcttgta agtgaaaaga taaaatttga cctcagaaac tctgagcatt 240
aaaaatccac tattagcaaa taaattacta tggacttctt gctttaattt tgtgatgaat 300
atggggtgtc actggtaaac caacacattc tgaaggatac attacttagt gatagattct 360
tatgtacttt gctanatnac gtggatatga gttgacaagt ttctctttct tcaatctttt 420
aaggggcnga ngaaatgagg aagaaaagaa aaggattacg catactgttc tttctatngg 480
aaggattaga tatgtttcct ttgccaatat taaaaaaata ataatgttta ctactagtga 540
aaccc 545

<210> 206
<211> 487
<212> DNA
<213> Homo sapiens

<220>
<221> misc_feature
<222> 29, 106, 119, 154, 204, 236, 279, 306, 318, 325, 353, 448,
466, 470
<223> n = A,T,C or G

<400> 206
tttttttttt ttttttagtc aagtttctna tttttattat aattaaagtc ttggtcattt 60
catttattag ctctgcaact tacatattta aattaaagaa acgttnttag acaactgtna 120
caatttataa atgtaaggtg ccattattga gtanatatat tcctccaaga gtggatgtgt 180
cccttctccc accaactaat gaancagcaa cattagttta attttattag tagatnatac 240
actgctgcaa acgctaattc tcttctccat ccccatgtng atattgtgta tatgtgtgag 300
ttggtnagaa tgcatcanca atctnacaat caacagcaag atgaagctag gcntgggctt 360
tcggtgaaaa tagactgtgt ctgtctgaat caaatgatct gacctatcct cggtggcaag 420
aactcttcga accgcttcct caaaggcngc tgccacattt gtggcntctn ttgcacttgt 480
ttcaaaa 487

<210> 207
<211> 332
<212> DNA
<213> Homo sapiens

<220>
<221> misc_feature
<222> 28, 156, 163, 179, 229
<223> n = A,T,C or G

<400> 207
tgaattggct aaaagactgc atttttanaa ctagcaactc ttatttcttt cctttaaaaa 60

tacatagcat taaatcccaa atcctattta aagacctgac agcttgagaa ggtcactact 120
gcatttatag gaccttctgg tggttctgct gttacntttg aantctgaca atccttgana 180
atctttgcat gcagaggagg taaaaggtat tggattttca cagaggaana acacagcgca 240
gaaatgaagg ggccaggctt actgagcttg tccactggag ggctcatggg tgggacatgg 300
aaaagaaggc agcctaggcc ctggggagcc ca 332

<210> 208
<211> 524
<212> DNA
<213> Homo sapiens

<220>
<221> misc_feature
<222> 256
<223> n = A,T,C or G

<400> 208
agggcgtggt gcggagggcg ttactgtttt gtctcagtaa caataaatac aaaaagactg 60
gttgtgttcc ggccccatcc aaccacgaag ttgatttctc ttgtgtgcag agtgactgat 120
tttaaaggac atggagcttg tcacaatgtc acaatgtcac agtgtgaagg gcacactcac 180
tcccgcgtga ttcacattta gcaaccaaca atagctcatg agtccatact tgtaaatact 240
tttggcagaa tacttnttga aacttgcaga tgataactaa gatccaagat atttcccaaa 300
gtaaatagaa gtgggtcata atattaatta cctgttcaca tcagcttcca tttacaagtc 360
atgagcccag acactgacat caaactaagc ccacttagac tcctcaccac cagtctgtcc 420
tgtcatcaga caggaggctg tcaccttgac caaattctca ccagtcaatc atctatccaa 480
aaaccattac ctgatccact tccggtaatg caccaccttg gtga 524

<210> 209
<211> 159
<212> DNA
<213> Homo sapiens

<400> 209
gggtgaggaa atccagagtt gccatggaga aaattccagt gtcagcattc ttgctccttg 60
tggccctctc ctacactctg gccagagata ccacagtcaa acctggagcc aaaaaggaca 120
caaaggactc tcgacccaaa ctgccccaga ccctctcca 159

<210> 210
<211> 256
<212> DNA
<213> Homo sapiens

<220>
<221> misc_feature
<222> 96, 134, 143, 146, 191, 200, 214
<223> n = A,T,C or G

<400> 210
actccctggc agacaaaggc agaggagaga gctctgttag ttctgtgttg ttgaactgcc 60
actgaatttc tttccacttg gactattaca tgccanttga gggactaatg gaaaaacgta 120
tggggagatt ttanccaatt tangtntgta aatggggaga ctggggcagg cgggagagat 180
ttgcagggtg naaatgggan ggctggtttg ttanatgaac agggacatag gaggtaggca 240
ccaggatgct aaatca 256

<210> 211
<211> 264
<212> DNA
<213> Homo sapiens

<220>
<221> misc_feature
<222> 195, 224
<223> n = A,T,C or G

<400> 211
acattgtttt tttgagataa agcattgaga gagctctcct taacgtgaca caatggaagg 60
actggaacac atacccacat ctttgttctg agggataatt ttctgataaa gtcttgctgt 120
atattcaagc acatatgtta tatattattc agttccatgt ttatagccta gttaaggaga 180

ggggagatac attcngaaag aggactgaaa gaaatactca agtnggaaaa cagaaaaaga 240
aaaaaaggag caaatgagaa gcct 264

<210> 212
<211> 328
<212> DNA
<213> Homo sapiens

<220>
<221> misc_feature
<222> 43, 183, 248, 266
<223> n = A,T,C or G

<400> 212
acccaaaaat ccaatgctga atatttggct tcattattcc canattcttt gattgtcaaa 60
ggatttaatg ttgtctcagc ttgggcactt cagttaggac ctaaggatgc cagccggcag 120
gtttatatat gcagcaacaa tattcaagcg cgacaacagg ttattgaact tgcccgccag 180
ttnaatttca ttcccattga cttgggatcc ttatcatcag ccagagagat tgaaaattta 240
cccctacnac tctttactct ctgganaggg ccagtggtgg tagctataag cttggccaca 300
tttttttttc ctttattcct ttgtcaga 328

<210> 213
<211> 250
<212> DNA
<213> Homo sapiens

<220>
<221> misc_feature
<222> 25, 136, 209, 219, 236
<223> n = A,T,C or G

<400> 213
acttatgagc agagcgacat atccnagtgt agactgaata aaactgaatt ctctccagtt 60
taaagcattg ctcactgaag ggatagaagt gactgccagg agggaaagta agccaaggct 120
cattatgcca aagganatat acatttcaat tctccaaact tcttcctcat tccaagagtt 180
ttcaatattt gcatgaacct gctgataanc catgttaana aacaaatatc tctctnacct 240
tctcatcggt 250

<210> 214
<211> 444
<212> DNA
<213> Homo sapiens

<220>
<221> misc_feature
<222> 223, 350, 387
<223> n = A,T,C or G

<400> 214
acccagaatc caatgctgaa tatttggctt cattattccc agattctttg attgtcaaag 60
gatttaatgt tgtctcagct tgggcacttc agttaggacc taaggatgcc agccggcagg 120
tttatatatg cagcaacaat attcaagcgc gacaacaggt tattgaactt gcccgccagt 180
tgaatttcat tcccattgac ttgggatcct tatcatcagc canagagatt gaaaatttac 240
ccctacgact ctttactctc tggagagggc cagtggtggt agctataagc ttggccacat 300
ttttttttcc tttattcctt tgtcagagat gcgattcatc catatgctan aaaccaacag 360
agtgactttt acaaaattcc tataganatt gtgaataaaa ccttacctat agttgccatt 420
actttgctct ccctaatata cctc 444

<210> 215
<211> 366
<212> DNA
<213> Homo sapiens

<220>
<221> misc_feature
<222> 32, 136, 286
<223> n = A,T,C or G

<400> 215
acttatgagc agagcgacat atccaagtgt anactgaata aaactgaatt ctctccagtt 60
taaagcattg ctcactgaag ggatagaagt gactgccagg agggaaagta agccaaggct 120
cattatgcca aagganatat acatttcaat tctccaaact tcttcctcat tccaagagtt 180
ttcaatattt gcatgaacct gctgataagc catgttgaga aacaaatatc tctctgacct 240
tctcatcggt aagcagaggc tgtaggcaac atggaccata gcgaanaaaa aacttagtaa 300
tccaagctgt tttctacact gtaaccaggt ttccaaccaa ggtggaaatc tcctatactt 360
ggtgcc 366

<210> 216
<211> 260
<212> DNA
<213> Homo sapiens

<220>
<221> misc_feature
<222> 25, 96, 97, 106, 109, 132, 133, 163, 195, 201, 221
<223> n = A,T,C or G

<400> 216
ctgtataaac agaactccac tgcangaggg agggccgggc caggagaatc tccgcttgtc 60
caagacaggg gcctaaggag ggtctccaca ctgctnntaa gggctnttnc atttttttat 120
taataaaaag tnnaaaaggc ctcttctcaa cttttttccc ttnggctgga aaatttaaaa 180
atcaaaaatt tcctnaagtt ntcaagctat catatatact ntatcctgaa aaagcaacat 240
aattcttcct tccctccttt 260

<210> 217
<211> 262
<212> DNA
<213> Homo sapiens

<220>
<221> misc_feature
<222> 20, 41
<223> n = A,T,C or G

<400> 217
acctacgtgg gtaagtttan aaatgttata atttcaggaa naggaacgca tataattgta 60
tcttgcctat aattttctat tttaataagg aaatagcaaa ttggggtggg gggaatgtag 120
ggcattctac agtttgagca aaatgcaatt aaatgtggaa ggacagcact gaaaaatttt 180
atgaataatc tgtatgatta tatgtctcta gagtagattt ataattagcc acttacccta 240
atatccttca tgcttgtaaa gt 262

<210> 218
<211> 205
<212> DNA
<213> Homo sapiens

<220>
<221> misc_feature
<222> 24, 33, 154, 157, 159, 161, 182
<223> n = A,T,C or G

<400> 218
accaaggtgg tgcattaccg gaantggatc aangacacca tcgtggccaa cccctgagca 60
cccctatcaa ctcccttttg tagtaaactt ggaaccttgg aaatgaccag gccaagactc 120
aggcctcccc agttctactg acctttgtcc ttangtntna ngtccagggt tgctaggaaa 180
anaaatcagc agacacaggt gtaaa 205

<210> 219
<211> 114
<212> DNA
<213> Homo sapiens

<400> 219
tactgttttg tctcagtaac aataaataca aaaagactgg ttgtgttccg gccccatcca 60
accacgaagt tgatttctct tgtgtgcaga gtgactgatt ttaaaggaca tgga 114

<210> 220
<211> 93
<212> DNA
<213> Homo sapiens

<400> 220
actagccagc acaaaaggca gggtagcctg aattgctttc tgctctttac atttctttta 60
aaataagcat ttagtgctca gtccctactg agt 93

<210> 221
<211> 167
<212> DNA
<213> Homo sapiens

<220>
<221> misc_feature
<222> 5, 116, 149
<223> n = A,T,C or G

<400> 221
actangtgca ggtgcgcaca aatatttgtc gatattccct tcatcttgga ttccatgagg 60
tcttttgccc agcctgtggc tctactgtag taagtttctg ctgatgagga gccagnatgc 120
cccccactac cttccctgac gctccccana aatcacccaa cctctgt 167

<210> 222
<211> 351
<212> DNA
<213> Homo sapiens

<400> 222
agggcgtggt gcggagggcg gtactgacct cattagtagg aggatgcatt ctggcacccc 60
gttcttcacc tgtcccccaa tccttaaaag gccatactgc ataaagtcaa caacagataa 120
atgtttgctg aattaaagga tggatgaaaa aaattaataa tgaatttttg cataatccaa 180
ttttctcttt tatatttcta gaagaagttt ctttgagcct attagatccc gggaatcttt 240
taggtgagca tgattagaga gcttgtaggt tgcttttaca tatatctggc atatttgagt 300
ctcgtatcaa aacaatagat tggtaaaggt ggtattattg tattgataag t 351

<210> 223
<211> 383
<212> DNA
<213> Homo sapiens

<220>
<221> misc_feature
<222> 296
<223> n = A,T,C or G

<400> 223
aaaacaaaca aacaaaaaaa acaattcttc attcagaaaa attatcttag ggactgatat 60
tggtaattat ggtcaattta atwrtrttkt ggggcatttc cttacattgt cttgacaaga 120
ttaaaatgtc tgtgccaaaa ttttgtattt tatttggaga cttcttatca aaagtaatgc 180
tgccaaagga agtctaagga attagtagtg ttcccmtcac ttgtttggag tgtgctattc 240
taaaagattt tgatttcctg gaatgacaat tatattttaa ctttggtggg ggaaanagtt 300
ataggaccac agtcttcact tctgatactt gtaaattaat cttttattgc acttgttttg 360
accattaagc tatatgttta aaa 383

<210> 224
<211> 320
<212> DNA
<213> Homo sapiens

<400> 224
cccctgaagg cttcttgtta gaaaatagta cagttacaac caataggaac aacaaaaaga 60
aaaagtttgt gacattgtag tagggagtgt gtacccctta ctccccatca aaaaaaaaat 120
ggatacatgg ttaaaggata raagggcaat attttatcat atgttctaaa agagaaggaa 180
gagaaaatac tactttctcr aaatggaagc ccttaaaggt gctttgatac tgaaggacac 240
aaatgtggcc gtccatcctc ctttaragtt gcatgacttg gacacggtaa ctgttgcagt 300
tttaractcm gcattgtgac 320

<210> 225
<211> 1214
<212> DNA
<213> Homo sapiens

<400> 225
gaggactgca gcccgcactc gcagccctgg caggcggcac tggtcatgga aaacgaattg 60
ttctgctcgg gcgtcctggt gcatccgcag tgggtgctgt cagccgcaca ctgtttccag 120
aactcctaca ccatcgggct gggcctgcac agtcttgagg ccgaccaaga gccagggagc 180
cagatggtgg aggccagcct ctccgtacgg cacccagagt acaacagacc cttgctcgct 240
aacgacctca tgctcatcaa gttggacgaa tccgtgtccg agtctgacac catccggagc 300
atcagcattg cttcgcagtg ccctaccgcg gggaactctt gcctcgtttc tggctggggt 360
ctgctggcga acggcagaat gcctaccgtg ctgcagtgcg tgaacgtgtc ggtggtgtct 420
gaggaggtct gcagtaagct ctatgacccg ctgtaccacc ccagcatgtt ctgcgccggc 480
ggagggcaag accagaagga ctcctgcaac ggtgactctg gggggcccct gatctgcaac 540
gggtacttgc agggccttgt gtctttcgga aaagccccgt gtggccaagt tggcgtgcca 600
ggtgtctaca ccaacctctg caaattcact gagtggatag agaaaaccgt ccaggccagt 660
taactctggg gactgggaac ccatgaaatt gacccccaaa tacatcctgc ggaaggaatt 720
caggaatatc tgttcccagc ccctcctccc tcaggcccag gagtccaggc ccccagcccc 780
tcctccctca aaccaagggt acagatcccc agcccctcct ccctcagacc caggagtcca 840
gaccccccag cccctcctcc ctcagaccca ggagtccagc ccctcctccc tcagacccag 900
gagtccagac cccccagccc ctcctccctc agacccaggg gtccaggccc ccaacccctc 960
ctccctcaga ctcagaggtc caagccccca acccctcctt ccccagaccc agaggtccag 1020
gtcccagccc ctcctccctc agacccagcg gtccaatgcc acctagactc tccctgtaca 1080
cagtgccccc ttgtggcacg ttgacccaac cttaccagtt ggtttttcat tttttgtccc 1140
tttcccctag atccagaaat aaagtctaag agaagcgcaa aaaaaaaaaa aaaaaaaaaa 1200
aaaaaaaaaa aaaa 1214

<210> 226
<211> 119
<212> DNA
<213> Homo sapiens

<400> 226
acccagtatg tgcagggaga cggaacccca tgtgacagcc cactccacca gggttcccaa 60
agaacctggc ccagtcataa tcattcatcc tgacagtggc aataatcacg ataaccagt 119

<210> 227
<211> 818
<212> DNA
<213> Homo sapiens

<400> 227
acaattcata gggacgacca atgaggacag ggaatgaacc cggctctccc ccagccctga 60
tttttgctac atatggggtc ccttttcatt ctttgcaaaa acactgggtt ttctgagaac 120
acggacggtt cttagcacaa tttgtgaaat ctgtgtaraa ccgggctttg caggggagat 180
aattttcctc ctctggagga aaggtggtga ttgacaggca gggagacagt gacaaggcta 240
gagaaagcca cgctcggcct tctctgaacc aggatggaac ggcagacccc tgaaaacgaa 300
gcttgtcccc ttccaatcag ccacttctga gaacccccat ctaacttcct actggaaaag 360
agggcctcct caggagcagt ccaagagttt tcaaagataa cgtgacaact accatctaga 420
ggaaagggtg caccctcagc agagaagccg agagcttaac tctggtcgtt tccagagaca 480
acctgctggc tgtcttggga tgcgcccagc ctttgagagg ccactacccc atgaacttct 540
gccatccact ggacatgaag ctgaggacac tgggcttcaa cactgagttg tcatgagagg 600
gacaggctct gccctcaagc cggctgaggg cagcaaccac tctcctcccc tttctcacgc 660
aaagccattc ccacaaatcc agaccatacc atgaagcaac gagacccaaa cagtttggct 720
caagaggata tgaggactgt ctcagcctgg ctttgggctg acaccatgca cacacacaag 780
gtccacttct aggttttcag cctagatggg agtcgtgt 818

<210> 228
<211> 744
<212> DNA
<213> Homo sapiens

<400> 228
actggagaca ctgttgaact tgatcaagac ccagaccacc ccaggtctcc ttcgtgggat 60
gtcatgacgt ttgacatacc tttggaacga gcctcctcct tggaagatgg aagaccgtgt 120
tcgtggccga cctggcctct cctggcctgt ttcttaagat gcggagtcac atttcaatgg 180

taggaaaagt ggcttcgtaa aatagaagag cagtcactgt ggaactacca aatggcgaga 240
tgctcggtgc acattggggt gctttgggat aaaagattta tgagccaact attctctggc 300
accagattct aggccagttt gttccactga agcttttccc acagcagtcc acctctgcag 360
gctggcagct gaatggcttg ccggtggctc tgtggcaaga tcacactgag atcgatgggt 420
gagaaggcta ggatgcttgt ctagtgttct tagctgtcac gttggctcct tccaggttgg 480
ccagacggtg ttggccactc ccttctaaaa cacaggcgcc ctcctggtga cagtgacccg 540
ccgtggtatg ccttggccca ttccagcagt cccagttatg catttcaagt ttggggtttg 600
ttcttttcgt taatgttcct ctgtgttgtc agctgtcttc atttcctggg ctaagcagca 660
ttgggagatg tggaccagag atccactcct taagaaccag tggcgaaaga cactttcttt 720
cttcactctg aagtagctgg tggt 744

<210> 229
<211> 300
<212> DNA
<213> Homo sapiens

<400> 229
cgagtctggg ttttgtctat aaagtttgat ccctcctttt ctcatccaaa tcatgtgaac 60
cattacacat cgaaataaaa gaaaggtggc agacttgccc aacgccaggc tgacatgtgc 120
tgcagggttg ttgtttttta attattattg ttagaaacgt cacccacagt ccctgttaat 180
ttgtatgtga cagccaactc tgagaaggtc ctatttttcc acctgcagag gatccagtct 240
cactaggctc ctccttgccc tcacactgga gtctccgcca gtgtgggtgc ccactgacat 300

<210> 230
<211> 301
<212> DNA
<213> Homo sapiens

<400> 230
cagcagaaca aatacaaata tgaagagtgc aaagatctca taaaatctat gctgaggaat 60
gagcgacagt tcaaggagga gaagcttgca gagcagctca agcaagctga ggagctcagg 120
caatataaag tcctggttca cactcaggaa cgagagctga cccagttaag ggagaagttg 180
cgggaaggga gagatgcctc cctctcattg aatgagcatc tccaggccct cctcactccg 240
gatgaaccgg acaagtccca ggggcaggac ctccaagaaa cagacctcgg ccgcgaccac 300
g 301

<210> 231
<211> 301
<212> DNA
<213> Homo sapiens

<400> 231
gcaagcacgc tggcaaatct ctgtcaggtc agctccagag aagccattag tcattttagc 60
caggaactcc aagtccacat ccttggcaac tggggacttg cgcaggttag ccttgaggat 120
ggcaacacgg gacttctcat caggaagtgg gatgtagatg agctgatcaa gacggccagg 180
tctgaggatg gcaggatcaa tgatgtcagg ccggttggta ccgccaatga tgaacacatt 240
tttttttgtg gacatgccat ccatttctgt caggatctgg ttgatgactc ggtcagcagc 300
c 301

<210> 232
<211> 301
<212> DNA
<213> Homo sapiens

<400> 232
agtaggtatt tcgtgagaag ttcaacacca aaactggaac atagttctcc ttcaagtgtt 60
ggcgacagcg gggcttcctg attctggaat ataactttgt gtaaattaac agccacctat 120
agaagagtcc atctgctgtg aaggagagac agagaactct gggttccgtc gtcctgtcca 180
cgtgctgtac caagtgctgg tgccagcctg ttacctgttc tcactgaaaa tctggctaat 240
gctcttgtgt atcacttctg attctgacaa tcaatcaatc aatggcctag agcactgact 300
g 301

<210> 233
<211> 301
<212> DNA
<213> Homo sapiens

<400> 233
atgactgact tcccagtaag gctctctaag gggtaagtag gaggatccac aggatttgag 60
atgctaaggc cccagagatc gtttgatcca accctcttat tttcagaggg gaaaatgggg 120
cctagaagtt acagagcatc tagctggtgc gctggcaccc ctggcctcac acagactccc 180
gagtagctgg gactacaggc acacagtcac tgaagcaggc cctgttagca attctatgcg 240
tacaaattaa catgagatga gtagagactt tattgagaaa gcaagagaaa atcctatcaa 300
c 301

<210> 234
<211> 301
<212> DNA
<213> Homo sapiens

<400> 234
aggtcctaca catcgagact catccatgat tgatatgaat ttaaaaatta caagcaaaga 60
cattttattc atcatgatgc tttcttttgt ttcttctttt cgttttcttc tttttctttt 120
tcaatttcag caacatactt ctcaatttct tcaggattta aaatcttgag ggattgatct 180
cgcctcatga cagcaagttc aatgtttttg ccacctgact gaaccacttc caggagtgcc 240
ttgatcacca gcttaatggt cagatcatct gcttcaatgg cttcgtcagt atagttcttc 300
t 301

<210> 235
<211> 283
<212> DNA
<213> Homo sapiens

<400> 235
tggggctgtg catcaggcgg gtttgagaaa tattcaattc tcagcagaag ccagaatttg 60
aattccctca tcttttaggg aatcatttac caggtttgga gaggattcag acagctcagg 120
tgctttcact aatgtctctg aacttctgtc cctctttgtt catggatagt ccaataaata 180
atgttatctt tgaactgatg ctcataggag agaatataag aactctgagt gatatcaaca 240
ttagggattc aaagaaatat tagatttaag ctcacactgg tca 283

<210> 236
<211> 301
<212> DNA
<213> Homo sapiens

<400> 236
aggtcctcca ccaactgcct gaagcacggt taaaattggg aagaagtata gtgcagcata 60
aatactttta aatcgatcag atttccctaa cccacatgca atcttcttca ccagaagagg 120
tcggagcagc atcattaata ccaagcagaa tgcgtaatag ataaatacaa tggtatatag 180
tgggtagacg gcttcatgag tacagtgtac tgtggtatcg taatctggac ttgggttgta 240
aagcatcgtg taccagtcag aaagcatcaa tactcgacat gaacgaatat aaagaacacc 300
a 301

<210> 237
<211> 301
<212> DNA
<213> Homo sapiens

<400> 237
cagtggtagt ggtggtggac gtggcgttgg tcgtggtgcc ttttttggtg cccgtcacaa 60
actcaatttt tgttcgctcc tttttggcct tttccaattt gtccatctca attttctggg 120
ccttggctaa tgcctcatag taggagtcct cagaccagcc atggggatca aacatatcct 180
ttgggtagtt ggtgccaagc tcgtcaatgg cacagaatgg atcagcttct cgtaaatcta 240
gggttccgaa attctttctt cctttggata atgtagttca tatccattcc ctcctttatc 300
t 301

<210> 238
<211> 301
<212> DNA
<213> Homo sapiens

<400> 238
gggcaggttt tttttttttt ttttttgatg gtgcagaccc ttgctttatt tgtctgactt 60
gttcacagtt cagccccctg ctcagaaaac caacgggcca gctaaggaga ggaggaggca 120
ccttgagact tccggagtcg aggctctcca gggttcccca gcccatcaat cattttctgc 180

accccctgcc tgggaagcag ctccctgggg ggtgggaatg ggtgactaga agggatttca 240
gtgtgggacc cagggtctgt tcttcacagt aggaggtgga agggatgact aatttcttta 300
t 301

<210> 239
<211> 239
<212> DNA
<213> Homo sapiens

<400> 239
ataagcagct agggaattct ttatttagta atgtcctaac ataaaagttc acataactgc 60
ttctgtcaaa ccatgatact gagctttgtg acaacccaga aataactaag agaaggcaaa 120
cataatacct tagagatcaa gaaacattta cacagttcaa ctgtttaaaa atagctcaac 180
attcagccag tgagtagagt gtgaatgcca gcatacacag tatacaggtc cttcaggga 239

<210> 240
<211> 300
<212> DNA
<213> Homo sapiens

<400> 240
ggtcctaatg aagcagcagc ttccacattt taacgcaggt ttacggtgat actgtccttt 60
gggatctgcc ctccagtgga accttttaag gaagaagtgg gcccaagcta agttccacat 120
gctgggtgag ccagatgact tctgttccct ggtcactttc ttcaatgggg cgaatggggg 180
ctgccaggtt tttaaaatca tgcttcatct tgaagcacac ggtcacttca ccctcctcac 240
gctgtgggtg tactttgatg aaaataccca ctttgttggc ctttctgaag ctataatgtc 300

<210> 241
<211> 301
<212> DNA
<213> Homo sapiens

<400> 241
gaggtctggt gctgaggtct ctgggctagg aagaggagtt ctgtggagct ggaagccaga 60
cctctttgga ggaaactcca gcagctatgt tggtgtctct gagggaatgc aacaaggctg 120
ctcctccatg tattggaaaa ctgcaaactg gactcaactg gaaggaagtg ctgctgccag 180
tgtgaagaac cagcctgagg tgacagaaac ggaagcaaac aggaacagcc agtcttttct 240
tcctcctcct gtcatacggt ctctctcaag catcctttgt tgtcaggggc ctaaaaggga 300
g 301

<210> 242
<211> 301
<212> DNA
<213> Homo sapiens

<400> 242
ccgaggtcct gggatgcaac caatcactct gtttcacgtg acttttatca ccatacaatt 60
tgtggcattt cctcattttc tacattgtag aatcaagagt gtaaataaat gtatatcgat 120
gtcttcaaga atatatcatt cctttttcac tagaacccat tcaaaatata agtcaagaat 180
cttaatatca acaaatatat caagcaaact ggaaggcaga ataactacca taatttagta 240
taagtaccca aagttttata aatcaaaagc cctaatgata accattttta gaattcaatc 300
a 301

<210> 243
<211> 301
<212> DNA
<213> Homo sapiens

<400> 243
aggtaagtcc cagtttgaag ctcaaaagat ctggtatgag cataggctca tcgacgacat 60
ggtggcccaa gctatgaaat cagagggagg cttcatctgg gcctgtaaaa actatgatgg 120
tgacgtgcag tcggactctg tggcccaagg gtatggctct ctcggcatga tgaccagcgt 180
gctggtttgt ccagatggca agacagtaga agcagaggct gcccacggga ctgtaacccg 240
tcactaccgc atgttccaga aaggacagga gacgtccacc aatcccattg cttccatttt 300
t 301

<210> 244

<211> 300
<212> DNA
<213> Homo sapiens

<400> 244
gctggtttgc aagaatgaaa tgaatgattc tacagctagg acttaacctt gaaatggaaa 60
gtcatgcaat cccatttgca ggatctgtct gtgcacatgc ctctgtagag agcagcattc 120
ccagggacct tggaaacagt tgacactgta aggtgcttgc tccccaagac acatcctaaa 180
aggtgttgta atggtgaaaa cgtcttcctt ctttattgcc ccttcttatt tatgtgaaca 240
actgtttgtc ttttgtgtat cttttttaaa ctgtaaagtt caattgtgaa aatgaatatc 300

<210> 245
<211> 301
<212> DNA
<213> Homo sapiens

<400> 245
gtctgagtat ttaaaatgtt attgaaatta tccccaacca atgttagaaa agaaagaggt 60
tatatactta gataaaaaat gaggtgaatt actatccatt gaaatcatgc tcttagaatt 120
aaggccagga gatattgtca ttaatgtara cttcaggaca ctagagtata gcagccctat 180
gttttcaaag agcagagatg caattaaata ttgtttagca tcaaaaaggc cactcaatac 240
agctaataaa atgaaagacc taatttctaa agcaattctt tataatttac aaagttttaa 300
g 301

<210> 246
<211> 301
<212> DNA
<213> Homo sapiens

<400> 246
ggtctgtcct acaatgcctg cttcttgaaa gaagtcggca ctttctagaa tagctaaata 60
acctgggctt attttaaaga actatttgta gctcagattg gttttcctat ggctaaaata 120
agtgcttctt gtgaaaatta aataaaacag ttaattcaaa gccttgatat atgttaccac 180
taacaatcat actaaatata ttttgaagta caaagtttga catgctctaa agtgacaacc 240
caaatgtgtc ttacaaaaca cgttcctaac aaggtatgct ttacactacc aatgcagaaa 300
c 301

<210> 247
<211> 301
<212> DNA
<213> Homo sapiens

<400> 247
aggtcctttg gcagggctca tggatcagag ctcaaactgg agggaaaggc atttcgggta 60
gcctaagagg gcgactggcg gcagcacaac caaggaaggc aaggttgttt cccccacgct 120
gtgtcctgtg ttcaggtgcg acacacaatc ctcatgggaa caggatcacc catgcgctgc 180
ccttgatgat caaggttggg gcttaagtgg attaagggag gcaagttctg ggttccttgc 240
cttttcaaac catgaagtca ggctctgtat ccctcctttt cctaactgat attctaacta 300
a 301

<210> 248
<211> 301
<212> DNA
<213> Homo sapiens

<400> 248
aggtccttgg agatgccatt tcagccgaag gactcttctw ttcggaagta caccctcact 60
attaggaaga ttcttagggg taatttttct gaggaaggag aactagccaa cttaagaatt 120
acaggaagaa agtggtttgg aagacagcca aagaaataaa agcagattaa attgtatcag 180
gtacattcca gcctgttggc aactccataa aaacatttca gattttaatc ccgaatttag 240
ctaatgagac tggatttttg ttttttatgt tgtgtgtcgc agagctaaaa actcagttcc 300
c 301

<210> 249
<211> 301
<212> DNA
<213> Homo sapiens

<400> 249
gtccagagga agcacctggt gctgaactag gcttgccctg ctgtgaactt gcacttggag 60
ccctgacgct gctgttctcc ccgaaaaacc cgaccgacct ccgcgatctc cgtcccgccc 120
ccagggagac acagcagtga ctcagagctg gtcgcacact gtgcctccct cctcaccgcc 180
catcgtaatg aattattttg aaaattaatt ccaccatcct ttcagattct ggatggaaag 240
actgaatctt tgactcagaa ttgtttgctg aaaagaatga tgtgactttc ttagtcattt 300
a 301

<210> 250
<211> 301
<212> DNA
<213> Homo sapiens

<400> 250
ggtctgtgac aaggacttgc aggctgtggg aggcaagtga cccttaacac tacacttctc 60
cttatcttta ttggcttgat aaacataatt atttctaaca ctagcttatt tccagttgcc 120
cataagcaca tcagtacttt tctctggctg gaatagtaaa ctaaagtatg gtacatctac 180
ctaaaagact actatgtgga ataatacata ctaatgaagt attacatgat ttaaagacta 240
caataaaacc aaacatgctt ataacattaa gaaaaacaat aaagatacat gattgaaacc 300
a 301

<210> 251
<211> 301
<212> DNA
<213> Homo sapiens

<400> 251
gccgaggtcc tacatttggc ccagtttccc cctgcatcct ctccagggcc cctgcctcat 60
agacaacctc atagagcata ggagaactgg ttgccctggg ggcaggggga ctgtctggat 120
ggcaggggtc ctcaaaaatg ccactgtcac tgccaggaaa tgcttctgag cagtacacct 180
cattgggatc aatgaaaagc ttcaagaaat cttcaggctc actctcttga aggcccggaa 240
cctctggagg ggggcagtgg aatcccagct ccaggacgga tcctgtcgaa aagatatcct 300
c 301

<210> 252
<211> 301
<212> DNA
<213> Homo sapiens

<400> 252
gcaaccaatc actctgtttc acgtgacttt tatcaccata caatttgtgg catttcctca 60
ttttctacat tgtagaatca agagtgtaaa taaatgtata tcgatgtctt caagaatata 120
tcattccttt ttcactagga acccattcaa aatataagtc aagaatctta atatcaacaa 180
atatatcaag caaactggaa ggcagaataa ctaccataat ttagtataag tacccaaagt 240
tttataaatc aaaagcccta atgataacca tttttagaat tcaatcatca ctgtagaatc 300
a 301

<210> 253
<211> 301
<212> DNA
<213> Homo sapiens

<400> 253
ttccctaaga agatgttatt ttgttgggtt ttgttccccc tccatctcga ttctcgtacc 60
caactaaaaa aaaaaaataa agaaaaaatg tgctgcgttc tgaaaaataa ctccttagct 120
tggtctgatt gttttcagac cttaaaatat aaacttgttt cacaagcttt aatccatgtg 180
gatttttttt cttagagaac cacaaaacat aaaaggagca agtcggactg aatacctgtt 240
tccatagtgc ccacagggta ttcctcacat tttctccata ggaaaatgct ttttcccaag 300
g 301

<210> 254
<211> 301
<212> DNA
<213> Homo sapiens

<400> 254
cgctgcgcct ttcccttggg ggaggggcaa ggccagaggg ggtccaagtg cagcacgagg 60

aacttgacca attcccttga agcgggtggg ttaaaccctg taaatgggaa caaaatcccc 120
ccaaatctct tcatcttacc ctggtggact cctgactgta gaattttttg gttgaaacaa 180
gaaaaaaata aagctttgga cttttcaagg ttgcttaaca ggtactgaaa gactggcctc 240
acttaaactg agccaggaaa agctgcagat ttattaatgg gtgtgttagt gtgcagtgcc 300
t 301

<210> 255
<211> 302
<212> DNA
<213> Homo sapiens

<400> 255
agcttttttt tttttttttt tttttttttt ttcattaaaa aatagtgctc tttattataa 60
attactgaaa tgtttctttt ctgaatataa atataaatat gtgcaaagtt tgacttggat 120
tgggattttg ttgagttctt caagcatctc ctaataccct caagggcctg agtagggggg 180
aggaaaaagg actggaggtg gaatctttat aaaaaacaag agtgattgag gcagattgta 240
aacattatta aaaaacaaga aacaaacaaa aaaatagaga aaaaaaccac cccaacacac 300
aa 302

<210> 256
<211> 301
<212> DNA
<213> Homo sapiens

<220>
<221> misc_feature
<222> 290
<223> n = A,T,C or G

<400> 256
gttccagaaa acattgaagg tggcttccca aagtctaact agggataccc cctctagcct 60
aggaccctcc tccccacacc tcaatccacc aaaccatcca taatgcaccc agataggccc 120
acccccaaaa gcctggacac cttgagcaca cagttatgac caggacagac tcatctctat 180
aggcaaatag ctgctggcaa actggcatta cctggtttgt ggggatgggg gggcaagtgt 240
gtggcctctc ggcctggtta gcaagaacat tcagggtagg cctaagttan tcgtgttagt 300
t 301

<210> 257
<211> 301
<212> DNA
<213> Homo sapiens

<400> 257
gttgtggagg aactctggct tgctcattaa gtcctactga ttttcactat cccctgaatt 60
tccccactta tttttgtctt tcactatcgc aggccttaga agaggtctac ctgcctccag 120
tcttacctag tccagtctac cccctggagt tagaatggcc atcctgaagt gaaaagtaat 180
gtcacattac tcccttcagt gatttcttgt agaagtgcca atccctgaat gccaccaaga 240
tcttaatctt cacatcttta atcttatctc tttgactcct ctttacaccg gagaaggctc 300
c 301

<210> 258
<211> 301
<212> DNA
<213> Homo sapiens

<220>
<221> misc_feature
<222> 209
<223> n = A,T,C or G

<400> 258
cagcagtagt agatgccgta tgccagcacg cccagcactc ccaggatcag caccagcacc 60
aggggcccag ccaccaggcg cagaagcaag ataaacagta ggctcaagac cagagccacc 120
cccagggcaa caagaatcca ataccaggac tgggcaaaat cttcaaagat cttaacactg 180
atgtctcggg cattgaggct gtcaataana cgctgatccc ctgctgtatg gtggtgtcat 240
tggtgatccc tgggagcgcc ggtggagtaa cgttggtcca tggaaagcag cgcccacaac 300
t 301

<210> 259
<211> 301
<212> DNA
<213> Homo sapiens

<220>
<221> misc_feature
<222> 27
<223> n = A,T,C or G

<400> 259
tcatatatgc aaacaaatgc agactangcc tcaggcagag actaaaggac atctcttggg 60
gtgtcctgaa gtgatttgga cccctgaggg cagacaccta agtaggaatc ccagtgggaa 120
gcaaagccat aaggaagccc aggattcctt gtgatcagga agtgggccag gaaggtctgt 180
tccagctcac atctcatctg catgcagcac ggaccggatg cgcccactgg gtcttggctt 240
ccctcccatc ttctcaagca gtgtccttgt tgagccattt gcatccttgg ctccaggtgg 300
c 301

<210> 260
<211> 301
<212> DNA
<213> Homo sapiens

<400> 260
ttttttttct ccctaaggaa aaagaaggaa caagtctcat aaaaccaaat aagcaatggt 60
aaggtgtctt aacttgaaaa agattaggag tcactggttt acaagttata attgaatgaa 120
agaactgtaa cagccacagt tggccatttc atgccaatgg cagcaaacaa caggattaac 180
tagggcaaaa taaataagtg tgtggaagcc ctgataagtg cttaataaac agactgattc 240
actgagacat cagtacctgc ccgggcggcc gctcgagccg aattctgcag atatccatca 300
c 301

<210> 261
<211> 301
<212> DNA
<213> Homo sapiens

<400> 261
aaatattcga gcaaatcctg taactaatgt gtctccataa aaggctttga actcagtgaa 60
tctgcttcca tccacgattc tagcaatgac ctctcggaca tcaaagctcc tcttaaggtt 120
agcaccaact attccataca attcatcagc aggaaataaa ggctcttcag aaggttcaat 180
ggtgacatcc aatttcttct gataatttag attcctcaca accttcctag ttaagtgaag 240
ggcatgatga tcatccaaag cccagtggtc acttactcca gactttctgc aatgaagatc 300
a 301

<210> 262
<211> 301
<212> DNA
<213> Homo sapiens

<400> 262
gaggagagcc tgttacagca tttgtaagca cagaatactc caggagtatt tgtaattgtc 60
tgtgagcttc ttgccgcaag tctctcagaa atttaaaaag atgcaaatcc ctgagtcacc 120
cctagacttc ctaaaccaga tcctctgggg ctggaacctg gcactctgca tttgtaatga 180
gggctttctg gtgcacacct aattttgtgc atctttgccc taaatcctgg attagtgccc 240
catcattacc cccacattat aatgggatag attcagagca gatactctcc agcaaagaat 300
c 301

<210> 263
<211> 301
<212> DNA
<213> Homo sapiens

<220>
<221> misc_feature
<222> 222
<223> n = A,T,C or G

<400> 263

tttagcttgt ggtaaatgac tcacaaaact gattttaaaa tcaagttaat gtgaattttg 60
aaaattacta cttaatccta attcacaata acaatggcat taaggtttga cttgagttgg 120
ttcttagtat tatttatggt aaataggctc ttaccacttg caaataactg gccacatcat 180
taatgactga cttcccagta aggctctcta aggggtaagt angaggatcc acaggatttg 240
agatgctaag gccccagaga tcgtttgatc caaccctctt attttcagag gggaaaatgg 300
g 301

<210> 264
<211> 301
<212> DNA
<213> Homo sapiens

<400> 264
aaagacgtta aaccactcta ctaccacttg tggaactctc aaagggtaaa tgacaaascc 60
aatgaatgac tctaaaaaca atatttacat ttaatggttt gtagacaata aaaaaacaag 120
gtggatagat ctagaattgt aacattttaa gaaaaccata scatttgaca gatgagaaag 180
ctcaattata gatgcaaagt tataactaaa ctactatagt agtaaagaaa tacatttcac 240
acccttcata taaattcact atcttggctt gaggcactcc ataaaatgta tcacgtgcat 300
a 301

<210> 265
<211> 301
<212> DNA
<213> Homo sapiens

<400> 265
tgcccaagtt atgtgtaagt gtatccgcac ccagaggtaa aactacactg tcatctttgt 60
cttcttgtga cgcagtattt cttctctggg gagaagccgg gaagtcttct cctggctcta 120
catattcttg gaagtctcta atcaactttt gttccatttg tttcatttct tcaggaggga 180
ttttcagttt gtcaacatgt tctctaacaa cacttgccca tttctgtaaa gaatccaaag 240
cagtccaagg ctttgacatg tcaacaacca gcataactag agtatccttc agagatacgg 300
c 301

<210> 266
<211> 301
<212> DNA
<213> Homo sapiens

<400> 266
taccgtctgc ccttcctccc atccaggcca tctgcgaatc tacatgggtc ctcctattcg 60
acaccagatc actctttcct ctacccacag gcttgctatg agcaagagac acaacctcct 120
ctcttctgtg ttccagcttc ttttcctgtt cttcccaccc cttaagttct attcctgggg 180
atagagacac caatacccat aacctctctc ctaagcctcc ttataaccca gggtgcacag 240
cacagactcc tgacaactgg taaggccaat gaactgggag ctcacagctg gctgtgcctg 300
a 301

<210> 267
<211> 301
<212> DNA
<213> Homo sapiens

<400> 267
aaagagcaca ggccagctca gcctgccctg gccatctaga ctcagcctgg ctccatgggg 60
gttctcagtg ctgagtccat ccaggaaaag ctcacctaga ccttctgagg ctgaatcttc 120
atcctcacag gcagcttctg agagcctgat attcctagcc ttgatggtct ggagtaaagc 180
ctcattctga ttcctctcct tcttttcttt caagttggct ttcctcacat ccctctgttc 240
aattcgcttc agcttgtctg ctttagccct catttccaga agcttcttct ctttggcatc 300
t 301

<210> 268
<211> 301
<212> DNA
<213> Homo sapiens

<400> 268
aatgtctcac tcaactactt cccagcctac cgtggcctaa ttctgggagt tttcttctta 60
gatcttggga gagctggttc ttctaaggag aaggaggaag gacagatgta actttggatc 120
tcgaagagga agtctaatgg aagtaattag tcaacggtcc ttgtttagac tcttggaata 180

tgctgggtgg ctcagtgagc ccttttggag aaagcaagta ttattcttaa ggagtaacca 240
cttcccattg ttctactttc taccatcatc aattgtatat tatgtattct ttggagaact 300
a 301

<210> 269
<211> 301
<212> DNA
<213> Homo sapiens

<400> 269
taacaatata cactagctat ctttttaact gtccatcatt agcaccaatg aagattcaat 60
aaaattacct ttattcacac atctcaaaac aattctgcaa attcttagtg aagtttaact 120
atagtcacag accttaaata ttcacattgt tttctatgtc tactgaaaat aagttcacta 180
cttttctgga tattctttac aaaatcttat taaaattcct ggtattatca cccccaatta 240
tacagtagca caaccacctt atgtagtttt tacatgatag ctctgtagaa gtttcacatc 300
t 301

<210> 270
<211> 301
<212> DNA
<213> Homo sapiens

<400> 270
cattgaagag cttttgcgaa acatcagaac acaagtgctt ataaaattaa ttaagcctta 60
cacaagaata catattcctt ttatttctaa ggagttaaac atagatgtag ctgatgtgga 120
gagcttgctg gtgcagtgca tattggataa cactattcat ggccgaattg atcaagtcaa 180
ccaactcctt gaactggatc atcagaagaa gggtggtgca cgatatactg cactagataa 240
tggaccaacc aactaaattc tctcaccagg ctgtatcagt aaactggctt aacagaaaac 300
a 301

<210> 271
<211> 301
<212> DNA
<213> Homo sapiens

<220>
<221> misc_feature
<222> 237, 257
<223> n = A,T,C or G

<400> 271
aaaaggttct cataagatta acaatttaaa taaatatttg atagaacatt ctttctcatt 60
tttatagctc atctttaggg ttgatattca gttcatgctt cccttgctgt tcttgatcca 120
gaattgcaat cacttcatca gcctgtattc gctccaattc tctataaagt gggtccaagg 180
tgaaccacag agccacagca cacctctttc ccttggtgac tgccttcacc ccatganggt 240
tctctcctcc agatganaac tgatcatgcg cccacatttt gggttttata gaagcagtca 300
c 301

<210> 272
<211> 301
<212> DNA
<213> Homo sapiens

<400> 272
taaattgcta agccacagat aacaccaatc aaatggaaca aatcactgtc ttcaaatgtc 60
ttatcagaaa accaaatgag cctggaatct tcataatacc taaacatgcc gtatttagga 120
tccaataatt ccctcatgat gagcaagaaa aattctttgc gcacccctcc tgcatccaca 180
gcatcttctc caacaaatat aaccttgagt ggcttcttgt aatctatgtt ctttgttttc 240
ctaaggactt ccattgcatc tcctacaata ttttctctac gcaccactag aattaagcag 300
g 301

<210> 273
<211> 301
<212> DNA
<213> Homo sapiens

<220>
<221> misc_feature

<222> 30, 33, 66, 212, 214, 248, 256, 285, 288, 297, 299
<223> n = A,T,C or G

<400> 273
acatgtgtgt atgtgtatct ttgggaaaan aanaagacat cttgtttayt atttttttgg 60
agagangctg ggacatggat aatcacwtaa tttgctayta tyactttaat ctgactygaa 120
gaaccgtcta aaaataaaat ttaccatgtc dtatattcct tatagtatgc ttatttcacc 180
ttytttctgt ccagagagag tatcagtgac ananatttma gggtgaamac atgmattggt 240
gggacttnty tttacngagm accctgcccg sgcgccctcg makcngantt ccgcsananc 300
t 301

<210> 274
<211> 301
<212> DNA
<213> Homo sapiens

<220>
<221> misc_feature
<222> 86, 294
<223> n = A,T,C or G

<400> 274
cttatatact ctttctcaga ggcaaaagag gagatgggta atgtagacaa ttctttgagg 60
aacagtaaat gattattaga gagaangaat ggaccaagga gacagaaatt aacttgtaaa 120
tgattctctt tggaatctga atgagatcaa gaggccagct ttagcttgtg gaaaagtcca 180
tctaggtatg gttgcattct cgtcttcttt tctgcagtag ataatgaggt aaccgaaggc 240
aattgtgctt cttttgataa gaagctttct tggtcatatc aggaaattcc aganaaagtc 300
c 301

<210> 275
<211> 301
<212> DNA
<213> Homo sapiens

<220>
<221> misc_feature
<222> 262, 270, 286
<223> n = A,T,C or G

<400> 275
tcggtgtcag cagcacgtgg cattgaacat tgcaatgtgg agcccaaacc acagaaaatg 60
gggtgaaatt ggccaacttt ctattaactt atgttggcaa ttttgccacc aacagtaagc 120
tggcccttct aataaaagaa aattgaaagg tttctcacta aacggaatta agtagtggag 180
tcaagagact cccaggcctc agcgtacctg cccgggcggc cgctcgaagc cgaattctgc 240
agatatccat cacactggcg gncgctcgan catgcatcta gaaggnccaa ttcgccctat 300
a 301

<210> 276
<211> 301
<212> DNA
<213> Homo sapiens

<400> 276
tgtacacata ctcaataaat aaatgactgc attgtggtat tattactata ctgattatat 60
ttatcatgtg acttctaatt agaaaatgta tccaaaagca aaacagcaga tatacaaaat 120
taaagagaca gaagatagac attaacagat aaggcaactt atacattgag aatccaaatc 180
caatacattt aaacatttgg gaaatgaggg ggacaaatgg aagccagatc aaatttgtgt 240
aaaactattc agtatgtttc ccttgcttca tgtctgagaa ggctctcctt caatggggat 300
g 301

<210> 277
<211> 301
<212> DNA
<213> Homo sapiens

<220>
<221> misc_feature
<222> 222, 245, 286

<223> n = A,T,C or G

<400> 277
tttgttgatg tcagtatttt attacttgcg ttatgagtgc tcacctggga aattctaaag 60
atacagagga cttggaggaa gcagagcaac tgaatttaat ttaaaagaag gaaaacattg 120
gaatcatggc actcctgata ctttcccaaa tcaacactct caatgcccca ccctcgtcct 180
caccatagtg gggagactaa agtggccacg gatttgcctt angtgtgcag tgcgttctga 240
gttcnctgtc gattacatct gaccagtctc ctttttccga agtccntccg ttcaatcttg 300
c 301

<210> 278
<211> 301
<212> DNA
<213> Homo sapiens

<220>
<221> misc_feature
<222> 266
<223> n = A,T,C or G

<400> 278
taccactaca ctccagcctg ggcaacagag caagacctgt ctcaaagcat aaaatggaat 60
aacatatcaa atgaaacagg gaaaatgaag ctgacaattt atggaagcca gggcttgtca 120
cagtctctac tgttattatg cattacctgg gaatttatat aagcccttaa taataatgcc 180
aatgaacatc tcatgtgtgc tcacaatgtt ctggcactat tataagtgct tcacaggttt 240
tatgtgttct tcgtaacttt atggantagg tactcggccg cgaacacgct aagccgaatt 300
c 301

<210> 279
<211> 301
<212> DNA
<213> Homo sapiens

<220>
<221> misc_feature
<222> 215, 218
<223> n = A,T,C or G

<400> 279
aaagcaggaa tgacaaagct tgcttttctg gtatgttcta ggtgtattgt gacttttact 60
gttatattaa ttgccaatat aagtaaatat agattatata tgtatagtgt ttcacaaagc 120
ttagaccttt accttccagc caccccacag tgcttgatat ttcagagtca gtcattggtt 180
atacatgtgt agttccaaag cacataagct agaanaanaa atatttctag ggagcactac 240
catctgtttt cacatgaaat gccacacaca tagaactcca acatcaattt cattgcacag 300
a 301

<210> 280
<211> 301
<212> DNA
<213> Homo sapiens

<400> 280
ggtactggag ttttcctccc ctgtgaaaac gtaactactg ttgggagtga attgaggatg 60
tagaaaggtg gtggaaccaa attgtggtca atggaaatag gagaatatgg ttctcactct 120
tgagaaaaaa acctaagatt agcccaggta gttgcctgta acttcagttt ttctgcctgg 180
gtttgatata gtttagggtt ggggttagat taagatctaa attacatcag gacaaagaga 240
cagactatta actccacagt taattaagga ggtatgttcc atgtttattt gttaaagcag 300
t 301

<210> 281
<211> 301
<212> DNA
<213> Homo sapiens

<400> 281
aggtacaaga aggggaatgg gaaagagctg ctgctgtggc attgttcaac ttggatattc 60
gccgagcaat ccaaatcctg aatgaagggg catcttctga aaaaggagat ctgaatctca 120
atgtggtagc aatggcttta tcgggttata cggatgagaa gaactccctt tggagagaaa 180

tgtgtagcac actgcgatta cagctaaata acccgtattt gtgtgtcatg tttgcatttc 240
tgacaagtga aacaggatct tacgatggag ttttgtatga aaacaaagtt gcagtacctc 300
g 301

<210> 282
<211> 301
<212> DNA
<213> Homo sapiens

<400> 282
caggtactac agaattaaaa tactgacaag caagtagttt cttggcgtgc acgaattgca 60
tccagaaccc aaaaattaag aaattcaaaa agacattttg tgggcacctg ctagcacaga 120
agcgcagaag caaagcccag gcagaaccat gctaacctta cagctcagcc tgcacagaag 180
cgcagaagca aagcccaggc agaaccatgc taaccttaca gctcagcctg cacagaagcg 240
cagaagcaaa gcccaggcag aacatgctaa ccttacagct cagcctgcac agaagcacag 300
a 301

<210> 283
<211> 301
<212> DNA
<213> Homo sapiens

<400> 283
atctgtatac ggcagacaaa ctttatarag tgtagagagg tgagcgaaag gatgcaaaag 60
cactttgagg gctttataat aatatgctgc ttgaaaaaaa aaatgtgtag ttgatactca 120
gtgcatctcc agacatagta aggggttgct ctgaccaatc aggtgatcat tttttctatc 180
acttcccagg ttttatgcaa aaattttgtt aaattctata atggtgatat gcatctttta 240
ggaaacatat acatttttaa aaatctattt tatgtaagaa ctgacagacg aatttgcttt 300
g 301

<210> 284
<211> 301
<212> DNA
<213> Homo sapiens

<400> 284
caggtacaaa acgctattaa gtggcttaga atttgaacat ttgtggtctt tatttacttt 60
gcttcgtgtg tgggcaaagc aacatcttcc ctaaatatat attaccaaga aaagcaagaa 120
gcagattagg tttttgacaa aacaaacagg ccaaaagggg gctgacctgg agcagagcat 180
ggtgagaggc aaggcatgag agggcaagtt tgttgtggac agatctgtgc ctactttatt 240
actggagtaa aagaaaacaa agttcattga tgtcgaagga tatatacagt gttagaaatt 300
a 301

<210> 285
<211> 301
<212> DNA
<213> Homo sapiens

<220>
<221> misc_feature
<222> 167
<223> n = A,T,C or G

<400> 285
acatcaccat gatcggatcc cccacccatt atacgttgta tgtttacata aatactcttc 60
aatgatcatt agtgttttaa aaaaaatact gaaaactcct tctgcatccc aatctctaac 120
caggaaagca aatgctattt acagacctgc aagccctccc tcaaacnaaa ctatttctgg 180
attaaatatg tctgacttct tttgaggtca cacgactagg caaatgctat ttacgatctg 240
caaaagctgt ttgaagagtc aaagccccca tgtgaacacg atttctggac cctgtaacag 300
t 301

<210> 286
<211> 301
<212> DNA
<213> Homo sapiens

<400> 286
taccactgca ttccagcctg ggtgacagag tgagactccg tctccaaaaa aaactttgct 60

tgtatattat ttttgcctta cagtggatca ttctagtagg aaaggacagt aagatttttt 120
atcaaaatgt gtcatgccag taagagatgt tatattcttt tctcatttct tccccaccca 180
aaaataagct accatatagc ttataagtct caaatttttg ccttttacta aaatgtgatt 240
gtttctgttc attgtgtatg cttcatcacc tatattaggc aaattccatt ttttcccttg 300
t 301

<210> 287
<211> 301
<212> DNA
<213> Homo sapiens

<400> 287
tacagatctg ggaactaaat attaaaaatg agtgtggctg gatatatgga gaatgttggg 60
cccagaagga acgtagagat cagatattac aacagctttg ttttgagggt tagaaatatg 120
aaatgatttg gttatgaacg cacagtttag gcagcagggc cagaatcctg accctctgcc 180
ccgtggttat ctcctcccca gcttggctgc ctcatgttat cacagtattc cattttgttt 240
gttgcatgtc ttgtgaagcc atcaagattt tctcgtctgt tttcctctca ttggtaatgc 300
t 301

<210> 288
<211> 301
<212> DNA
<213> Homo sapiens

<400> 288
gtacacctaa ctgcaaggac agctgaggaa tgtaatgggc agccgctttt aaagaagtag 60
agtcaatagg aagacaaatt ccagttccag ctcagtctgg gtatctgcaa agctgcaaaa 120
gatctttaaa gacaatttca agagaatatt tccttaaagt tggcaatttg gagatcatac 180
aaaagcatct gcttttgtga tttaatttag ctcatctggc cactggaaga atccaaacag 240
tctgccttaa ttttggatga atgcatgatg gaaattcaat aatttagaaa gttaaaaaaa 300
a 301

<210> 289
<211> 301
<212> DNA
<213> Homo sapiens

<220>
<221> misc_feature
<222> 230, 297
<223> n = A,T,C or G

<400> 289
ggtacactgt ttccatgtta tgtttctaca cattgctacc tcagtgctcc tggaaactta 60
gcttttgatg tctccaagta gtccaccttc atttaactct ttgaaactgt atcatctttg 120
ccaagtaaga gtggtggcct atttcagctg ctttgacaaa atgactggct cctgacttaa 180
cgttctataa atgaatgtgc tgaagcaaag tgcccatggt ggcggcgaan aagagaaaga 240
tgtgttttgt tttggactct ctgtggtccc ttccaatgct gtgggtttcc aaccagngga 300
a 301

<210> 290
<211> 301
<212> DNA
<213> Homo sapiens

<220>
<221> misc_feature
<222> 162
<223> n = A,T,C or G

<400> 290
acactgagct cttcttgata aatatacaga atgcttggca tatacaagat tctatactac 60
tgactgatct gttcatttct ctcacagctc ttacccccaa aagcttttcc accctaagtg 120
ttctgacctc cttttctaat cacagtaggg atagaggcag anccacctac aatgaacatg 180
gagttctatc aagaggcaga aacagcacag aatcccagtt ttaccattcg ctagcagtgc 240
tgccttgaac aaaaacattt ctccatgtct cattttcttc atgcctcaag taacagtgag 300
a 301

<210> 291
<211> 301
<212> DNA
<213> Homo sapiens

<400> 291
caggtaccaa tttcttctat cctagaaaca tttcatttta tgttgttgaa acataacaac 60
tatatcagct agattttttt tctatgcttt acctgctatg gaaaatttga cacattctgc 120
tttactcttt tgtttatagg tgaatcacaa aatgtatttt tatgtattct gtagttcaat 180
agccatggct gtttacttca tttaatttat ttagcataaa gacattatga aaaggcctaa 240
acatgagctt cacttcccca ctaactaatt agcatctgtt atttcttaac cgtaatgcct 300
a 301

<210> 292
<211> 301
<212> DNA
<213> Homo sapiens

<220>
<221> misc_feature
<222> 131, 175, 206
<223> n = A,T,C or G

<400> 292
accttttagt agtaatgtct aataataaat aagaaatcaa ttttataagg tccatatagc 60
tgtattaaat aatttttaag tttaaaagat aaaataccat cattttaaat gttggtattc 120
aaaaccaaag natataaccg aaaggaaaaa cagatgagac ataaaatgat ttgcnagatg 180
ggaaatatag tasttyatga atgttnatta aattccagtt ataatagtgg ctacacactc 240
tcactacaca cacagacccc acagtcctat atgccacaaa cacatttcca taacttgaaa 300
a 301

<210> 293
<211> 301
<212> DNA
<213> Homo sapiens

<400> 293
ggtaccaagt gctggtgcca gcctgttacc tgttctcact gaaaagtctg gctaatgctc 60
ttgtgtagtc acttctgatt ctgacaatca atcaatcaat ggcctagagc actgactgtt 120
aacacaaacg tcactagcaa agtagcaaca gctttaagtc taaatacaaa gctgttctgt 180
gtgagaattt tttaaaaggc tacttgtata ataacccttg tcatttttaa tgtacctcgg 240
ccgcgaccac gctaagccga attctgcaga tatccatcac actggcggcc gctcgagcat 300
g 301

<210> 294
<211> 301
<212> DNA
<213> Homo sapiens

<220>
<221> misc_feature
<222> 139
<223> n = A,T,C or G

<400> 294
tgacccataa caatatacac tagctatctt tttaactgtc catcattagc accaatgaag 60
attcaataaa attaccttta ttcacacatc tcaaaacaat tctgcaaatt cttagtgaag 120
tttaactata gtcacaganc ttaaatattc acattgtttt ctatgtctac tgaaaataag 180
ttcactactt ttctgggata ttctttacaa aatcttatta aaattcctgg tattatcacc 240
cccaattata cagtagcaca accaccttat gtagttttta catgatagct ctgtagaggt 300
t 301

<210> 295
<211> 305
<212> DNA
<213> Homo sapiens

<400> 295

gtactctttc tctcccctcc tctgaattta attctttcaa cttgcaattt gcaaggatta 60
cacatttcac tgtgatgtat attgtgttgc aaaaaaaaaa gtgtctttgt ttaaaattac 120
ttggtttgtg aatccatctt gctttttccc cattggaact agtcattaac ccatctctga 180
actggtagaa aaacrtctga agagctagtc tatcagcatc tgacaggtga attggatggt 240
tctcagaacc atttcaccca gacagcctgt ttctatcctg tttaataaat tagtttgggt 300
tctct 305

<210> 296
<211> 301
<212> DNA
<213> Homo sapiens

<400> 296
aggtactatg ggaagctgct aaaataatat ttgatagtaa aagtatgtaa tgtgctatct 60
cacctagtag taaactaaaa ataaactgaa actttatgga atctgaagtt attttccttg 120
attaaataga attaataaac caatatgagg aaacatgaaa ccatgcaatc tactatcaac 180
tttgaaaaag tgattgaacg aaccacttag ctttcagatg atgaacactg ataagtcatt 240
tgtcattact ataaatttta aaatctgtta ataagatggc ctatagggag gaaaaagggg 300
c 301

<210> 297
<211> 300
<212> DNA
<213> Homo sapiens

<220>
<221> misc_feature
<222> 128, 157
<223> n = A,T,C or G

<400> 297
actgagtttt aactggacgc caagcaggca aggctggaag gttttgctct ctttgtgcta 60
aaggttttga aaaccttgaa ggagaatcat tttgacaaga agtacttaag agtctagaga 120
acaaagangt gaaccagctg aaagctctcg ggggaanctt acatgtgttg ttaggcctgt 180
tccatcattg ggagtgcact ggccatccct caaaatttgt ctgggctggc ctgagtggtc 240
accgcacctc ggccgcgacc acgctaagcc gaattctgca gatatccatc acactggcgg 300

<210> 298
<211> 301
<212> DNA
<213> Homo sapiens

<220>
<221> misc_feature
<222> 227
<223> n = A,T,C or G

<400> 298
tatggggttt gtcacccaaa agctgatgct gagaaaggcc tccctggggc ccctcccgcg 60
ggcatctgag agacctggtg ttccagtgtt tctggaaatg ggtcccagtg ccgccggctg 120
tgaagctctc agatcaatca cgggaagggc ctggcggtgg tggccacctg gaaccaccct 180
gtcctgtctg tttacatttc actaycaggt tttctctggg cattacnatt tgttccccta 240
caacagtgac ctgtgcattc tgctgtggcc tgctgtgtct gcaggtggct ctcagcgagg 300
t 301

<210> 299
<211> 301
<212> DNA
<213> Homo sapiens

<400> 299
gttttgagac ggagtttcac tcttgttgcc cagactggac tgcaatggca gggtctctgc 60
tcactgcacc ctctgcctcc caggttcgag caattctcct gcctcagcct cccaggtagc 120
tgggattgca ggctcacgcc accataccca gctaattttt ttgtattttt agtagagacg 180
gagtttcgcc atgttggcca gctggtctca aactcctgac ctcaagcgac ctgcctgcct 240
cggcctccca aagtgctgga attataggca tgagtcaaca cgcccagcct aaagatattt 300
t 301

<210> 300
<211> 301
<212> DNA
<213> Homo sapiens

<400> 300
attcagtttt atttgctgcc ccagtatctg taaccaggag tgccacaaaa tcttgccaga 60
tatgtcccac acccactggg aaaggctccc acctggctac ttcctctatc agctgggtca 120
gctgcattcc acaaggttct cagcctaatg agtttcacta cctgccagtc tcaaaactta 180
gtaaagcaag accatgacat tcccccacgg aaatcagagt ttgccccacc gtcttgttac 240
tataaagcct gcctctaaca gtccttgctt cttcacacca atcccgagcg catcccccat 300
g 301

<210> 301
<211> 301
<212> DNA
<213> Homo sapiens

<400> 301
ttaaattttt gagaggataa aaaggacaaa taatctagaa atgtgtcttc ttcagtctgc 60
agaggacccc aggtctccaa gcaaccacat ggtcaagggc atgaataatt aaaagttggt 120
gggaactcac aaagaccctc agagctgaga cacccacaac agtgggagct cacaaagacc 180
ctcagagctg agacacccac aacagtggga gctcacaaag accctcagag ctgagacacc 240
cacaacagca cctcgttcag ctgccacatg tgtgaataag gatgcaatgt ccagaagtgt 300
t 301

<210> 302
<211> 301
<212> DNA
<213> Homo sapiens

<400> 302
aggtacacat ttagcttgtg gtaaatgact cacaaaactg attttaaaat caagttaatg 60
tgaattttga aaattactac ttaatcctaa ttcacaataa caatggcatt aaggtttgac 120
ttgagttggt tcttagtatt atttatggta aataggctct taccacttgc aaataactgg 180
ccacatcatt aatgactgac ttcccagtaa ggctctctaa ggggtaagta ggaggatcca 240
caggatttga gatgctaagg ccccagagat cgtttgatcc aaccctctta ttttcagagg 300
g 301

<210> 303
<211> 301
<212> DNA
<213> Homo sapiens

<400> 303
aggtaccaac tgtggaaata ggtagaggat catttttttct ttccatatca actaagttgt 60
atattgtttt ttgacagttt aacacatctt cttctgtcag agattctttc acaatagcac 120
tggctaatgg aactaccgct tgcatgttaa aaatggtggt ttgtgaaatg atcataggcc 180
agtaacgggt atgtttttct aactgatctt ttgctcgttc caaaggacc tcaagacttc 240
catcgatttt atatctgggg tctagaaaag gagttaatct gttttccctc ataaattcac 300
c 301

<210> 304
<211> 301
<212> DNA
<213> Homo sapiens

<400> 304
acatggatgt tattttgcag actgtcaacc tgaatttgta tttgcttgac attgcctaat 60
tattagtttc agtttcagct tacccacttt ttgtctgcaa catgcaraas agacagtgcc 120
ctttttagtg tatcatatca ggaatcatct cacattggtt tgtgccatta ctggtgcagt 180
gactttcagc cacttgggta aggtggagtt ggccatatgt ctccactgca aaattactga 240
ttttcctttt gtaattaata agtgtgtgtg tgaagattct ttgagatgag gtatatatct 300
c 301

<210> 305
<211> 301

<212> DNA
<213> Homo sapiens

<220>
<221> misc_feature
<222> 3, 266
<223> n = A,T,C or G

<400> 305
gangtacagc gtggtcaagg taacaagaag aaaaaaatgt gagtggcatc ctgggatgag 60
cagggggaca gacctggaca gacacgttgt catttgctgc tgtgggtagg aaaatgggcg 120
taaaggagga gaaacagata caaaatctcc aactcagtat taaggtattc tcatgcctag 180
aatattggta gaaacaagaa tacattcata tggcaaataa ctaaccatgg tggaacaaaa 240
ttctgggatt taagttggat accaangaaa ttgtattaaa agagctgttc atggaataag 300
a 301

<210> 306
<211> 8
<212> PRT
<213> Homo sapiens

<400> 306
Val Leu Gly Trp Val Ala Glu Leu
 1               5

<210> 307
<211> 637
<212> DNA
<213> Homo sapiens

<400> 307
acagggratg aagggaaagg gagaggatga ggaagccccc ctggggattt ggtttggtcc 60
ttgtgatcag gtggtctatg gggcttatcc ctacaaagaa gaatccagaa ataggggcac 120
attgaggaat gatacttgag cccaaagagc attcaatcat tgttttattt gccttmtttt 180
cacaccattg gtgagggagg gattaccacc ctggggttat gaagatggtt gaacacccca 240
cacatagcac cggagatatg agatcaacag tttcttagcc atagagattc acagcccaga 300
gcaggaggac gcttgcacac catgcaggat gacatggggg atgcgctcgg gattggtgtg 360
aagaagcaag gactgttaga ggcaggcttt atagtaacaa gacggtgggg caaactctga 420
tttccgtggg ggaatgtcat ggtcttgctt tactaagttt tgagactggc aggtagtgaa 480
actcattagg ctgagaacct tgtggaatgc acttgaccca sctgatagag gaagtagcca 540
ggtgggagcc tttcccagtg ggtgtgggac atatctggca agattttgtg gcactcctgg 600
ttacagatac tggggcagca aataaaactg aatcttg 637

<210> 308
<211> 647
<212> DNA
<213> Homo sapiens

<220>
<221> misc_feature
<222> 123
<223> n = A,T,C or G

<400> 308
acgattttca ttatcatgta aatcgggtca ctcaaggggc caaccacagc tgggagccac 60
tgctcagggg aaggttcata tgggactttc tactgcccaa ggttctatac aggatataaa 120
ggngcctcac agtatagatc tggtagcaaa gaagaagaaa caaacactga tctctttctg 180
ccacccctct gaccctttgg aactcctctg accctttaga acaagcctac ctaatatctg 240
ctagagaaaa gaccaacaac ggcctcaaag gatctcttac catgaaggtc tcagctaatt 300
cttggctaag atgtgggttc cacattaggt tctgaatatg gggggaaggg tcaatttgct 360
cattttgtgt gtggataaag tcaggatgcc caggggccag agcagggggc tgcttgcttt 420
gggaacaatg gctgagcata taaccatagg ttatggggaa caaaacaaca tcaaagtcac 480
tgtatcaatt gccatgaaga cttgagggac ctgaatctac cgattcatct taaggcagca 540
ggaccagttt gagtggcaac aatgcagcag cagaatcaat ggaaacaaca gaatgattgc 600
aatgtccttt tttttctcct gcttctgact tgataaaagg ggaccgt 647

<210> 309

<211> 460
<212> DNA
<213> Homo sapiens

<400> 309
actttatagt ttaggctgga cattggaaaa aaaaaaaagc cagaacaaca tgtgatagat 60
aatatgattg gctgcacact tccagactga tgaatgatga acgtgatgga ctattgtatg 120
gagcacatct tcagcaagag ggggaaatac tcatcatttt tggccagcag ttgtttgatc 180
accaaacatc atgccagaat actcagcaaa ccttcttagc tcttgagaag tcaaagtccg 240
ggggaattta ttcctggcaa ttttaattgg actccttatg tgagagcagc ggctacccag 300
ctggggtggt ggagcgaacc cgtcactagt ggacatgcag tggcagagct cctggtaacc 360
acctagagga atacacaggc acatgtgtga tgccaagcgt gacacctgta gcactcaaat 420
ttgtcttgtt tttgtctttc ggtgtgtaag attcttaagt 460

<210> 310
<211> 539
<212> DNA
<213> Homo sapiens

<400> 310
acgggactta tcaaataaag ataggaaaag aagaaaactc aaatattata ggcagaaatg 60
ctaaaggttt taaaatatgt caggattgga agaaggcatg gataaagaac aaagttcagt 120
taggaaagag aaacacagaa ggaagagaca caataaaagt cattatgtat tctgtgagaa 180
gtcagacagt aagatttgtg ggaaatgggt tggtttgttg tatggtatgt attttagcaa 240
taatctttat ggcagagaaa gctaaaatcc tttagcttgc gtgaatgatc acttgctgaa 300
ttcctcaagg taggcatgat gaaggagggt ttagaggaga cacagacaca atgaactgac 360
ctagatagaa agccttagta tactcagcta ggaatagtga ttctgagggc acactgtgac 420
atgattatgt cattacatgt atggtagtga tggggatgat aggaaggaag aacttatggc 480
atattttcac ccccacaaaa gtcagttaaa tattgggaca ctaaccatcc aggtcaaga 539

<210> 311
<211> 526
<212> DNA
<213> Homo sapiens

<220>
<221> misc_feature
<222> 499
<223> n = A,T,C or G

<400> 311
caaatttgag ccaatgacat agaattttac aaatcaagaa gcttattctg gggccatttc 60
ttttgacgtt ttctctaaac tactaaagag gcattaatga tccataaatt atattatcta 120
catttacagc atttaaaatg tgttcagcat gaaatattag ctacagggga agctaaataa 180
attaaacatg gaataaagat ttgtccttaa atataatcta caagaagact ttgatatttg 240
tttttcacaa gtgaagcatt cttataaagt gtcataacct ttttggggaa actatgggaa 300
aaaatgggga aactctgaag ggttttaagt atcttacctg aagctacaga ctccataacc 360
tctctttaca gggagctcct gcagccccta cagaaatgag tggctgagat tcttgattgc 420
acagcaagag cttctcatct aaaccctttc cctttttagt atctgtgtat caagtataaa 480
agttctataa actgtagtnt acttatttta atccccaaag cacagt 526

<210> 312
<211> 500
<212> DNA
<213> Homo sapiens

<220>
<221> misc_feature
<222> 388, 401
<223> n = A,T,C or G

<400> 312
cctctctctc cccaccccct gactctagag aactgggttt tctcccagta ctccagcaat 60
tcatttctga aagcagttga gccactttat tccaaagtac actgcagatg ttcaaactct 120
ccatttctct ttcccttcca cctgccagtt ttgctgactc tcaacttgtc atgagtgtaa 180
gcattaagga cattatgctt cttcgattct gaagacaggc cctgctcatg gatgactctg 240
gcttcttagg aaaatatttt tcttccaaaa tcagtaggaa atctaaactt atcccctctt 300
tgcagatgtc tagcagcttc agacatttgg ttaagaaccc atgggaaaaa aaaaaatcct 360

tgctaatgtg gtttcctttg taaaccanga ttcttatttg nctggtatag aatatcagct 420
ctgaacgtgt ggtaaagatt tttgtgtttg aatataggag aaatcagttt gctgaaaagt 480
tagtcttaat tatctattgg 500

<210> 313
<211> 718
<212> DNA
<213> Homo sapiens

<220>
<221> misc_feature
<222> 318, 643, 666, 683, 693, 696, 703, 704, 708, 715
<223> n = A,T,C or G

<400> 313
ggagatttgt gtggtttgca gccgagggag accaggaaga tctgcatggt gggaaggacc 60
tgatgataca gaggtgagaa ataagaaagg ctgctgactt taccatctga ggccacacat 120
ctgctgaaat ggagataatt aacatcacta gaaacagcaa gatgacaata taatgtctaa 180
gtagtgacat gtttttgcac atttccagcc cttttaaata tccacacaca caggaagcac 240
aaaaggaagc acagagatcc ctgggagaaa tgcccggccg ccatcttggg tcatcgatga 300
gcctcgccct gtgcctgntc ccgcttgtga gggaaggaca ttagaaaatg aattgatgtg 360
ttccttaaag gatggcagga aaacagatcc tgttgtggat atttatttga acgggattac 420
agatttgaaa tgaagtcaca aagtgagcat taccaatgag aggaaaacag acgagaaaat 480
cttgatggtt cacaagacat gcaacaaaca aaatggaata ctgtgatgac acgagcagcc 540
aactggggag gagataccac ggggcagagg tcaggattct ggccctgctg cctaactgtg 600
cgttatacca atcatttcta tttctaccct caaacaagct gtngaatatc tgacttacgg 660
ttcttntggc ccacattttc atnatccacc ccntcntttt aannttantc caaantgt 718

<210> 314
<211> 358
<212> DNA
<213> Homo sapiens

<400> 314
gtttatttac attacagaaa aaacatcaag acaatgtata ctatttcaaa tatatccata 60
cataatcaaa tatagctgta gtacatgttt tcattggtgt agattaccac aaatgcaagg 120
caacatgtgt agatctcttg tcttattctt ttgtctataa tactgtattg tgtagtccaa 180
gctctcggta gtccagccac tgtgaaacat gctcccttta gattaacctc gtggacgctc 240
ttgttgtatt gctgaactgt agtgccctgt attttgcttc tgtctgtgaa ttctgttgct 300
tctggggcat ttccttgtga tgcagaggac caccacacag atgacagcaa tctgaatt 358

<210> 315
<211> 341
<212> DNA
<213> Homo sapiens

<400> 315
taccacctcc ccgctggcac tgatgagccg catcaccatg gtcaccagca ccatgaaggc 60
ataggtgatg atgaggacat ggaatgggcc cccaaggatg gtctgtccaa agaagcgagt 120
gacccccatt ctgaagatgt ctggaacctc taccagcagg atgatgatag ccccaatgac 180
agtcaccagc tccccgacca gccggatatc gtccttaggg gtcatgtagg cttcctgaag 240
tagcttctgc tgtaagaggg tgttgtcccg ggggctcgtg cggttattgg tcctgggctt 300
gagggggcgg tagatgcagc acatggtgaa gcagatgatg t 341

<210> 316
<211> 151
<212> DNA
<213> Homo sapiens

<400> 316
agactgggca agactcttac gccccacact gcaatttggt cttgttgccg tatccattta 60
tgtgggcctt tctcgagttt ctgattataa acaccactgg agcgatgtgt tgactggact 120
cattcaggga gctctggttg caatattagt t 151

<210> 317
<211> 151
<212> DNA
<213> Homo sapiens

<400> 317
agaactagtg gatcctaatg aaatacctga aacatatatt ggcatttatc aatggctcaa 60
atcttcattt atctctggcc ttaaccctgg ctcctgaggc tgcggccagc agatcccagg 120
ccagggctct gttcttgcca cacctgcttg a 151

<210> 318
<211> 151
<212> DNA
<213> Homo sapiens

<400> 318
actggtggga ggcgctgttt agttggctgt tttcagaggg gtctttcgga gggacctcct 60
gctgcaggct ggagtgtctt tattcctggc gggagaccgc acattccact gctgaggctg 120
tgggggcggt ttatcaggca gtgataaaca t 151

<210> 319
<211> 151
<212> DNA
<213> Homo sapiens

<400> 319
aactagtgga tccagagcta taggtacagt gtgatctcag ctttgcaaac acattttcta 60
catagatagt actaggtatt aatagatatg taaagaaaga aatcacacca ttaataatgg 120
taagattggg tttatgtgat tttagtgggt a 151

<210> 320
<211> 150
<212> DNA
<213> Homo sapiens

<400> 320
aactagtgga tccactagtc cagtgtggtg gaattccatt gtgttggggt tctagatcgc 60
gagcggctgc cctttttttt tttttttttg ggggggaatt tttttttttt aatagttatt 120
gagtgttcta cagcttacag taaataccat 150

<210> 321
<211> 151
<212> DNA
<213> Homo sapiens

<400> 321
agcaactttg tttttcatcc aggttatttt aggcttagga tttcctctca cactgcagtt 60
tagggtggca ttgtaaccag ctatggcata ggtgttaacc aaaggctgag taaacatggg 120
tgcctctgag aaatcaaagt cttcatacac t 151

<210> 322
<211> 151
<212> DNA
<213> Homo sapiens

<220>
<221> misc_feature
<222> 141
<223> n = A,T,C or G

<400> 322
atccagcatc ttctcctgtt tcttgccttc ctttttcttc ttcttasatt ctgcttgagg 60
tttgggcttg gtcagtttgc cacagggctt ggagatggtg acagtcttct ggcattcggc 120
attgtgcagg gctcgcttca nacttccagt t 151

<210> 323
<211> 151
<212> DNA
<213> Homo sapiens

<220>
<221> misc_feature

<222> 61, 69, 142
<223> n = A,T,C or G

<400> 323
tgaggacttg tkttcttttt ctttattttt aatcctctta ckttgtaaat atattgccta 60
nagactcant tactacccag tttgtggttt twtgggagaa atgtaactgg acagttagct 120
gttcaatyaa aaagacactt ancccatgtg g 151

<210> 324
<211> 461
<212> DNA
<213> Homo sapiens

<220>
<221> misc_feature
<222> 457
<223> n = A,T,C or G

<400> 324
acctgtgtgg aatttcagct ttcctcatgc aaaaggattt tgtatccccg gcctacttga 60
agaagtggtc agctaaagga atccaggttg ttggttggac tgttaatacc tttgatgaaa 120
agagttacta cgaatcccat cttggttcca gctatatcac tgacagcatg gtagaagact 180
gcgaacctca cttctagact ttcacggtgg gacgaaacgg gttcagaaac tgccaggggc 240
ctcatacagg gatatcaaaa taccctttgt gctacccagg ccctggggaa tcaggtgact 300
cacacaaatg caatagttgg tcactgcatt tttacctgaa ccaaagctaa acccggtgtt 360
gccaccatgc accatggcat gccagagttc aacactgttg ctcttgaaaa ttgggtctga 420
aaaaacgcac aagagcccct gccctgccct agctgangca c 461

<210> 325
<211> 400
<212> DNA
<213> Homo sapiens

<400> 325
acactgtttc catgttatgt ttctacacat tgctacctca gtgctcctgg aaacttagct 60
tttgatgtct ccaagtagtc caccttcatt taactctttg aaactgtatc atctttgcca 120
agtaagagtg gtggcctatt tcagctgctt tgacaaaatg actggctcct gacttaacgt 180
tctataaatg aatgtgctga agcaaagtgc ccatggtggc ggcgaagaag agaaagatgt 240
gttttgtttt ggactctctg tggtcccttc caatgctgtg ggtttccaac caggggaagg 300
gtccctttgg cattgccaag tgccataacc atgagcacta cgctaccatg gttctgcctc 360
ctggccaagc aggctggttt gcaagaatga aatgaatgat 400

<210> 326
<211> 1215
<212> DNA
<213> Homo sapiens

<400> 326
   ggaggactgc agcccgcact cgcagccctg gcaggcggca ctggtcatgg aaaacgaatt 60
   gttctgctcg ggcgtcctgg tgcatccgca gtgggtgctg tcagccgcac actgtttcca 120
   gaactcctac accatcgggc tgggcctgca cagtcttgag gccgaccaag agccagggag 180
   ccagatggtg gaggccagcc tctccgtacg gcacccagag tacaacagac ccttgctcgc 240
   taacgacctc atgctcatca agttggacga atccgtgtcc gagtctgaca ccatccggag 300
   catcagcatt gcttcgcagt gccctaccgc ggggaactct tgcctcgttt ctggctgggg 360
   tctgctggcg aacggcagaa tgcctaccgt gctgcagtgc gtgaacgtgt cggtggtgtc 420
   tgaggaggtc tgcagtaagc tctatgaccc gctgtaccac cccagcatgt tctgcgccgg 480
   cggagggcaa gaccagaagg actcctgcaa cggtgactct gggggccccc tgatctgcaa 540
   cgggtacttg cagggccttg tgtctttcgg aaaagccccg tgtggccaag ttggcgtgcc 600
   aggtgtctac accaacctct gcaaattcac tgagtggata gagaaaaccg tccaggccag 660
   ttaactctgg ggactgggaa cccatgaaat tgacccccaa atacatcctg cggaaggaat 720
   tcaggaatat ctgttcccag cccctcctcc ctcaggccca ggagtccagg cccccagccc 780
   ctcctccctc aaaccaaggg tacagatccc cagcccctcc tccctcagac ccaggagtcc 840
   agacccccca gcccctcctc cctcagaccc aggagtccag cccctcctcc ctcagaccca 900
   ggagtccaga ccccccagcc cctcctccct cagacccagg ggtccaggcc cccaacccct 960
   cctccctcag actcagaggt ccaagccccc aacccctcct tccccagacc cagaggtcca 1020
   ggtcccagcc cctcctccct cagacccagc ggtccaatgc cacctagact ctccctgtac 1080
   acagtgcccc cttgtggcac gttgacccaa ccttaccagt tggtttttca ttttttgtcc 1140
   ctttccccta gatccagaaa taaagtctaa gagaagcgca aaaaaaaaaa aaaaaaaaaa 1200
   aaaaaaaaaa aaaaa 1215

<210> 327
<211> 220
<212> PRT
<213> Homo sapiens

<400> 327
Glu Asp Cys Ser Pro His Ser Gln Pro Trp Gln Ala Ala Leu Val Met
1               5                   10                  15
Glu Asn Glu Leu Phe Cys Ser Gly Val Leu Val His Pro Gln Trp Val
            20                  25                  30
Leu Ser Ala Ala His Cys Phe Gln Asn Ser Tyr Thr Ile Gly Leu Gly
        35                  40                  45
Leu His Ser Leu Glu Ala Asp Gln Glu Pro Gly Ser Gln Met Val Glu
    50                  55                  60
Ala Ser Leu Ser Val Arg His Pro Glu Tyr Asn Arg Pro Leu Leu Ala
65                  70                  75                  80
Asn Asp Leu Met Leu Ile Lys Leu Asp Glu Ser Val Ser Glu Ser Asp
                85                  90                  95
Thr Ile Arg Ser Ile Ser Ile Ala Ser Gln Cys Pro Thr Ala Gly Asn
            100                 105                 110
Ser Cys Leu Val Ser Gly Trp Gly Leu Leu Ala Asn Gly Arg Met Pro
        115                 120                 125
Thr Val Leu Gln Cys Val Asn Val Ser Val Val Ser Glu Glu Val Cys
    130                 135                 140
Ser Lys Leu Tyr Asp Pro Leu Tyr His Pro Ser Met Phe Cys Ala Gly
145                 150                 155                 160
Gly Gly Gln Asp Gln Lys Asp Ser Cys Asn Gly Asp Ser Gly Gly Pro
                165                 170                 175
Leu Ile Cys Asn Gly Tyr Leu Gln Gly Leu Val Ser Phe Gly Lys Ala
            180                 185                 190
Pro Cys Gly Gln Val Gly Val Pro Gly Val Tyr Thr Asn Leu Cys Lys
        195                 200                 205
Phe Thr Glu Trp Ile Glu Lys Thr Val Gln Ala Ser
    210                 215                 220

<210> 328
<211> 234
<212> DNA
<213> Homo sapiens

<400> 328
cgctcgtctc tggtagctgc agccaaatca taaacggcga ggactgcagc ccgcactcgc 60
agccctggca ggcggcactg gtcatggaaa acgaattgtt ctgctcgggc gtcctggtgc 120
atccgcagtg ggtgctgtca gccacacact gtttccagaa ctcctacacc atcgggctgg 180
gcctgcacag tcttgaggcc gaccaagagc cagggagcca gatggtggag gcca 234

<210> 329
<211> 77
<212> PRT
<213> Homo sapiens

<400> 329
Leu Val Ser Gly Ser Cys Ser Gln Ile Ile Asn Gly Glu Asp Cys Ser
1               5                   10                  15
Pro His Ser Gln Pro Trp Gln Ala Ala Leu Val Met Glu Asn Glu Leu
            20                  25                  30
Phe Cys Ser Gly Val Leu Val His Pro Gln Trp Val Leu Ser Ala Thr
        35                  40                  45
His Cys Phe Gln Asn Ser Tyr Thr Ile Gly Leu Gly Leu His Ser Leu
    50                  55                  60
Glu Ala Asp Gln Glu Pro Gly Ser Gln Met Val Glu Ala
65                  70                  75

<210> 330

<211> 70
<212> DNA
<213> Homo sapiens

<400> 330
cccaacacaa tggcccgatc ccatccctga ctccgccctc aggatcgctc gtctctggta 60
gctgcagcca 70

<210> 331
<211> 22
<212> PRT
<213> Homo sapiens

<400> 331
Gln His Asn Gly Pro Ile Pro Ser Leu Thr Pro Pro Ser Gly Ser Leu
1               5                   10                  15
Val Ser Gly Ser Cys Ser
            20

<210> 332
<211> 2507
<212> DNA
<213> Homo sapiens

<400> 332
tggtgccgct gcagccggca gagatggttg agctcatgtt cccgctgttg ctcctccttc 60
tgcccttcct tctgtatatg gctgcgcccc aaatcaggaa aatgctgtcc agtggggtgt 120
gtacatcaac tgttcagctt cctgggaaag tagttgtggt cacaggagct aatacaggta 180
tcgggaagga gacagccaaa gagctggctc agagaggagc tcgagtatat ttagcttgcc 240
gggatgtgga aaagggggaa ttggtggcca aagagatcca gaccacgaca gggaaccagc 300
aggtgttggt gcggaaactg gacctgtctg atactaagtc tattcgagct tttgctaagg 360
gcttcttagc tgaggaaaag cacctccacg ttttgatcaa caatgcagga gtgatgatgt 420
gtccgtactc gaagacagca gatggctttg agatgcacat aggagtcaac cacttgggtc 480
acttcctcct aacccatctg ctgctagaga aactaaagga atcagcccca tcaaggatag 540
taaatgtgtc ttccctcgca catcacctgg gaaggatcca cttccataac ctgcagggcg 600
agaaattcta caatgcaggc ctggcctact gtcacagcaa gctagccaac atcctcttca 660
cccaggaact ggcccggaga ctaaaaggct ctggcgttac gacgtattct gtacaccctg 720
gcacagtcca atctgaactg gttcggcact catctttcat gagatggatg tggtggcttt 780
tctccttttt catcaagact cctcagcagg gagcccagac cagcctgcac tgtgccttaa 840
cagaaggtct tgagattcta agtgggaatc atttcagtga ctgtcatgtg gcatgggtct 900
ctgcccaagc tcgtaatgag actatagcaa ggcggctgtg ggacgtcagt tgtgacctgc 960
tgggcctccc aatagactaa caggcagtgc cagttggacc caagagaaga ctgcagcaga 1020
ctacacagta cttcttgtca aaatgattct ccttcaaggt tttcaaaacc tttagcacaa 1080
agagagcaaa accttccagc cttgcctgct tggtgtccag ttaaaactca gtgtactgcc 1140
agattcgtct aaatgtctgt catgtccaga tttactttgc ttctgttact gccagagtta 1200
ctagagatat cataatagga taagaagacc ctcatatgac ctgcacagct cattttcctt 1260
ctgaaagaaa ctactaccta ggagaatcta agctatagca gggatgattt atgcaaattt 1320
gaactagctt ctttgttcac aattcagttc ctcccaacca accagtcttc acttcaagag 1380
ggccacactg caacctcagc ttaacatgaa taacaaagac tggctcagga gcagggcttg 1440
cccaggcatg gtggatcacc ggaggtcagt agttcaagac cagcctggcc aacatggtga 1500
aaccccacct ctactaaaaa ttgtgtatat ctttgtgtgt cttcctgttt atgtgtgcca 1560
agggagtatt ttcacaaagt tcaaaacagc cacaataatc agagatggag caaaccagtg 1620
ccatccagtc tttatgcaaa tgaaatgctg caaagggaag cagattctgt atatgttggt 1680
aactacccac caagagcaca tgggtagcag ggaagaagta aaaaaagaga aggagaatac 1740
tggaagataa tgcacaaaat gaagggacta gttaaggatt aactagccct ttaaggatta 1800
actagttaag gattaatagc aaaagayatt aaatatgcta acatagctat ggaggaattg 1860
agggcaagca cccaggactg atgaggtctt aacaaaaacc agtgtggcaa aaaaaaaaaa 1920
aaaaaaaaaa aaaaatccta aaaacaaaca aacaaaaaaa acaattcttc attcagaaaa 1980
attatcttag ggactgatat tggtaattat ggtcaattta ataatatttt ggggcatttc 2040
cttacattgt cttgacaaga ttaaaatgtc tgtgccaaaa ttttgtattt tatttggaga 2100
cttcttatca aaagtaatgc tgccaaagga agtctaagga attagtagtg ttcccatcac 2160
ttgtttggag tgtgctattc taaaagattt tgatttcctg gaatgacaat tatattttaa 2220
ctttggtggg ggaaagagtt ataggaccac agtcttcact tctgatactt gtaaattaat 2280
cttttattgc acttgttttg accattaagc tatatgttta gaaatggtca ttttacggaa 2340
aaattagaaa aattctgata atagtgcaga ataaatgaat taatgtttta cttaatttat 2400
attgaactgt caatgacaaa taaaaattct ttttgattat tttttgtttt catttaccag 2460
aataaaaacg taagaattaa aagtttgatt acaaaaaaaa aaaaaaa 2507

<210> 333
<211> 3030
<212> DNA
<213> Homo sapiens

<400> 333
gcaggcgact tgcgagctgg gagcgattta aaacgctttg gattccccg gcctgggtgg 60
ggagagcgag ctgggtgccc cctagattcc ccgcccccgc acctcatgag ccgaccctcg 120
gctccatgga gcccggcaat tatgccacct tggatggagc caaggatatc gaaggcttgc 180
tgggagcggg aggggggcgg aatctggtcg cccactcccc tctgaccagc cacccagcgg 240
cgcctacgct gatgcctgct gtcaactatg cccccttgga tctgccaggc tcggcggagc 300
cgccaaagca atgccaccca tgccctgggg tgccccaggg gacgtcccca gctcccgtgc 360
cttatggtta ctttggaggc gggtactact cctgccgagt gtcccggagc tcgctgaaac 420
cctgtgccca ggcagccacc ctggccgcgt accccgcgga gactcccacg gccggggaag 480
agtaccccag ycgccccact gagtttgcct tctatccggg atatccggga acctaccagc 540
ctatggccag ttacctggac gtgtctgtgg tgcagactct gggtgctcct ggagaaccgc 600
gacatgactc cctgttgcct gtggacagtt accagtcttg ggctctcgct ggtggctgga 660
acagccagat gtgttgccag ggagaacaga acccaccagg tcccttttgg aaggcagcat 720
ttgcagactc cagcgggcag caccctcctg acgcctgcgc ctttcgtcgc ggccgcaaga 780
aacgcattcc gtacagcaag gggcagttgc gggagctgga gcgggagtat gcggctaaca 840
agttcatcac caaggacaag aggcgcaaga tctcggcagc caccagcctc tcggagcgcc 900
agattaccat ctggtttcag aaccgccggg tcaaagagaa gaaggttctc gccaaggtga 960
agaacagcgc taccccttaa gagatctcct tgcctgggtg ggaggagcga aagtgggggt 1020
gtcctgggga gaccaggaac ctgccaagcc caggctgggg ccaaggactc tgctgagagg 1080
cccctagaga caacaccctt cccaggccac tggctgctgg actgttcctc aggagcggcc 1140
tgggtaccca gtatgtgcag ggagacggaa ccccatgtga cagcccactc caccagggtt 1200
cccaaagaac ctggcccagt cataatcatt catcctgaca gtggcaataa tcacgataac 1260
cagtactagc tgccatgatc gttagcctca tattttctat ctagagctct gtagagcact 1320
ttagaaaccg ctttcatgaa ttgagctaat tatgaataaa tttggaaggc gatccctttg 1380
cagggaagct ttctctcaga ccccccttcca ttacacctct caccctggta acagcaggaa 1440
gactgaggag aggggaacgg gcagattcgt tgtgtggctg tgatgtccgt ttagcatttt 1500
tctcagctga cagctgggta ggtggacaat tgtagaggct gtctcttcct ccctccttgt 1560
ccaccccata gggtgtaccc actggtcttg gaagcaccca tccttaatac gatgattttt 1620
ctgtcgtgtg aaaatgaagc cagcaggctg cccctagtca gtccttcctt ccagagaaaa 1680
agagatttga gaaagtgcct gggtaattca ccattaattt cctcccccaa actctctgag 1740
tcttccctta atatttctgg tggttctgac caaagcaggt catggtttgt tgagcatttg 1800
ggatcccagt gaagtagatg tttgtagcct tgcatactta gcccttccca ggcacaaacg 1860
gagtggcaga gtggtgccaa ccctgttttc ccagtccacg tagacagatt cacagtgcgg 1920
aattctggaa gctggagaca gacgggctct ttgcagagcc gggactctga gagggacatg 1980
agggcctctg cctctgtgtt cattctctga tgtcctgtac ctgggctcag tgcccggtgg 2040
gactcatctc ctggccgcgc agcaaagcca gcgggttcgt gctggtcctt cctgcacctt 2100
aggctggggg tggggggcct gccggcgcat tctccacgat tgagcgcaca ggcctgaagt 2160
ctggacaacc cgcagaaccg aagctccgag cagcgggtcg gtggcgagta gtggggtcgg 2220
tggcgagcag ttggtggtgg gccgcggccg ccactacctc gaggacattt ccctcccgga 2280
gccagctctc ctagaaaccc cgcggcggcc gccgcagcca agtgtttatg gcccgcggtc 2340
gggtgggatc ctagccctgt ctcctctcct gggaaggagt gagggtggga cgtgacttag 2400
acacctacaa atctatttac caaagaggag cccgggactg agggaaaagg ccaaagagtg 2460
tgagtgcatg cggactgggg gttcagggga agaggacgag gaggaggaag atgaggtcga 2520
tttcctgatt taaaaaatcg tccaagcccc gtggtccagc ttaaggtcct cggttacatg 2580
cgccgctcag agcaggtcac tttctgcctt ccacgtcctc cttcaaggaa gccccatgtg 2640
ggtagctttc aatatcgcag gttcttactc ctctgcctct ataagctcaa acccaccaac 2700
gatcgggcaa gtaaaccccc tccctcgccg acttcggaac tggcgagagt tcagcgcaga 2760
tgggcctgtg ggagggggc aagatagatg aggggggagcg gcatggtgcg gggtgacccc 2820
ttggagagag gaaaaaggcc acaagagggg ctgccaccgc cactaacgga gatggccctg 2880
gtagagacct ttgggggtct ggaacctctg gactccccat gctctaactc ccacactctg 2940
ctatcagaaa cttaaacttg aggattttct ctgtttttca ctcgcaataa aytcagagca 3000
aacaaaaaaa aaaaaaaaaa aaaactcgag 3030

<210> 334
<211> 2417
<212> DNA
<213> Homo sapiens

<400> 334
   ggcggccgct ctagagctag tgggatcccc cgggctgcac gaattcggca cgagtgagtt 60
   ggagttttac ctgtattgtt ttaatttcaa caagcctgag gactagccac aaatgtaccc 120
   agtttacaaa tgaggaaaca ggtgcaaaaa ggttgttacc tgtcaaaggt cgtatgtggc 180
   agagccaaga tttgagccca gttatgtctg atgaacttag cctatgctct ttaaacttct 240
   gaatgctgac cattgaggat atctaaactt agatcaattg cattttccct ccaagactat 300
   ttacttatca atacaataat accaccttta ccaatctatt gttttgatac gagactcaaa 360
   tatgccagat atatgtaaaa gcaacctaca agctctctaa tcatgctcac ctaaaagatt 420
   cccgggatct aataggctca aagaaacttc ttctagaaat ataaaagaga aaattggatt 480
   atgcaaaaat tcattattaa tttttttcat ccatccttta attcagcaaa catttatctg 540
   ttgttgactt tatgcagtat ggccttttaa ggattggggg acaggtgaag aacggggtgc 600
   cagaatgcat cctcctacta atgaggtcag tacacatttg cattttaaaa tgccctgtcc 660
   agctgggcat ggtggatcat gcctgtaatc tcaacattgg aaggccaagg caggaggatt 720
   gcttcagccc aggagttcaa gaccagcctg ggcaacatag aaagacccca tctctcaatc 780
   aatcaatcaa tgccctgtct ttgaaaataa aactctttaa gaaaggttta atgggcaggg 840
   tgtggtagct catgcctata atacagcact ttgggaggct gaggcaggag gatcacttta 900
   gcccagaagt tcaagaccag cctgggcaac aagtgacacc tcatctcaat tttttaataa 960
   aatgaataca tacataagga aagataaaaa gaaaagttta atgaaagaat acagtataaa 1020
   acaaatctct tggacctaaa agtatttttg ttcaagccaa atattgtgaa tcacctctct 1080
   gtgttgagga tacagaatat ctaagcccag gaaactgagc agaaagttca tgtactaact 1140
   aatcaacccg aggcaaggca aaaatgagac taactaatca atccgaggca aggggcaaat 1200
   tagacggaac ctgactctgg tctattaagc gacaactttc cctctgttgt atttttcttt 1260
   tattcaatgt aaaaggataa aaactctcta aaactaaaaa caatgtttgt caggagttac 1320
   aaaccatgac caactaatta tggggaatca taaaatatga ctgtatgaga tcttgatggt 1380
   ttacaaagtg tacccactgt taatcacttt aaacattaat gaacttaaaa atgaatttac 1440
   ggagattgga atgtttcttt cctgttgtat tagttggctc aggctgccat aacaaaatac 1500
   cacagactgg gaggcttaag taacagaaat tcatttctca cagttctggg ggctggaagt 1560
   ccacgatcaa ggtgcaggaa aggcaggctt cattctgagg cccctctctt ggctcacatg 1620
   tggccaccct cccactgcgt gctcacatga cctctttgtg ctcctggaaa gagggtgtgg 1680
   gggacagagg gaaagagaag gagagggaac tctctggtgt ctcgtctttc aaggacccta 1740
   acctgggcca ctttggccca ggcactgtgg ggtggggggt tgtggctgct ctgctctgag 1800
   tggccaagat aaagcaacag aaaaatgtcc aaagctgtgc agcaaagaca agccaccgaa 1860
   cagggatctg ctcatcagtg tggggacctc caagtcggcc accctggagg caagccccca 1920
   cagagcccat gcaaggtggc agcagcagaa gaagggaatt gtccctgtcc ttggcacatt 1980
   cctcaccgac ctggtgatgc tggacactgc gatgaatggt aatgtggatg agaatatgat 2040
   ggactcccag aaaaggagac ccagctgctc aggtggctgc aaatcattac agccttcatc 2100
   ctggggagga actgggggcc tggttctggg tcagagagca gcccagtgag ggtgagagct 2160
   acagcctgtc ctgccagctg gatccccagt cccggtcaac cagtaatcaa ggctgagcag 2220
   atcaggcttc ccggagctgg tcttgggaag ccagccctgg ggtgagttgg ctcctgctgt 2280
   ggtactgaga caatattgtc ataaattcaa tgcgcccttg tatccctttt tcttttttat 2340
   ctgtctacat ctataatcac tatgcatact agtctttgtt agtgtttcta ttcmacttaa 2400
   tagagatatg ttatact 2417

<210> 335
<211> 2984
<212> DNA
<213> Homo sapiens

<400> 335
   atccctcctt ccccactctc ctttccagaa ggcacttggg gtcttatctg ttggactctg 60
   aaaacacttc aggcgccctt ccaaggcttc cccaaacccc taagcagccg cagaagcgct 120
   cccgagctgc cttctcccac actcaggtga tcgagttgga gaggaagttc agccatcaga 180
   agtacctgtc ggcccctgaa cgggcccacc tggccaagaa cctcaagctc acggagaccc 240
   aagtgaagat atggttccag aacagacgct ataagactaa gcgaaagcag ctctcctcgg 300
   agctgggaga cttggagaag cactcctctt tgccggccct gaaagaggag gccttctccc 360
   gggcctccct ggtctccgtg tataacagct atccttacta cccatacctg tactgcgtgg 420
   gcagctggag cccagctttt tggtaatgcc agctcaggtg acaaccatta tgatcaaaaa 480
   ctgccttccc cagggtgtct ctatgaaaag cacaaggggc caaggtcagg gagcaagagg 540
   tgtgcacacc aaagctattg gagatttgcg tggaaatctc asattcttca ctggtgagac 600
   aatgaaacaa cagagacagt gaaagtttta atacctaagt cattccccca gtgcatactg 660
   taggtcattt tttttgcttc tggctacctg tttgaagggg agagagggaa aatcaagtgg 720
   tattttccag cactttgtat gattttggat gagctgtaca cccaaggatt ctgttctgca 780
   actccatcct cctgtgtcac tgaatatcaa ctctgaaaga gcaaacctaa caggagaaag 840
   gacaaccagg atgaggatgt caccaactga attaaactta agtccagaag cctcctgttg 900
   gccttggaat atggccaagg ctctctctgt ccctgtaaaa gagaggggca aatagagagt 960
   ctccaagaga acgccctcat gctcagcaca tatttgcatg ggagggggag atgggtggga 1020
   ggagatgaaa atatcagctt ttcttattcc tttttattcc ttttaaaatg gtatgccaac 1080
   ttaagtattt acagggtggc ccaaatagaa caagatgcac tcgctgtgat tttaagacaa 1140
   gctgtataaa cagaactcca ctgcaagagg gggggccggg ccaggagaat ctccgcttgt 1200
   ccaagacagg ggcctaagga gggtctccac actgctgcta ggggctgttg cattttttta 1260
   ttagtagaaa gtggaaaggc ctcttctcaa cttttttccc ttgggctgga gaatttagaa 1320
   tcagaagttt cctggagttt tcaggctatc atatatactg tatcctgaaa ggcaacataa 1380
   ttcttccttc cctcctttta aaattttgtg ttcctttttg cagcaattac tcactaaagg 1440
   gcttcatttt agtccagatt tttagtctgg ctgcacctaa cttatgcctc gcttatttag 1500
   cccgagatct ggtctttttt tttttttttt tttttccgtc tccccaaagc tttatctgtc 1560
   ttgacttttt aaaaaagttt gggggcagat tctgaattgg ctaaaagaca tgcattttta 1620
   aaactagcaa ctcttatttc tttcctttaa aaatacatag cattaaatcc caaatcctat 1680
   ttaaagacct gacagcttga gaaggtcact actgcattta taggaccttc tggtggttct 1740
   gctgttacgt ttgaagtctg acaatccttg agaatctttg catgcagagg aggtaagagg 1800
   tattggattt tcacagagga agaacacagc gcagaatgaa gggccaggct tactgagctg 1860
   tccagtggag ggctcatggg tgggacatgg aaaagaaggc agcctaggcc ctggggagcc 1920
   cagtccactg agcaagcaag ggactgagtg agccttttgc aggaaaaggc taagaaaaag 1980
   gaaaaccatt ctaaaacaca acaagaaact gtccaaatgc tttgggaact gtgtttattg 2040
   cctataatgg gtccccaaaa tgggtaacct agacttcaga gagaatgagc agagagcaaa 2100
   ggagaaatct ggctgtcctt ccattttcat tctgttatct caggtgagct ggtagagggg 2160
   agacattaga aaaaaatgaa acaacaaaac aattactaat gaggtacgct gaggcctggg 2220
   agtctcttga ctccactact taattccgtt tagtgagaaa cctttcaatt ttcttttatt 2280
   agaagggcca gcttactgtt ggtggcaaaa ttgccaacat aagttaatag aaagttggcc 2340
   aatttcaccc cattttctgt ggtttgggct ccacattgca atgttcaatg ccacgtgctg 2400
   ctgacaccga ccggagtact agccagcaca aaaggcaggg tagcctgaat tgctttctgc 2460
   tctttacatt tcttttaaaa taagcattta gtgctcagtc cctactgagt actctttctc 2520
   tcccctcctc tgaatttaat tctttcaact tgcaatttgc aaggattaca catttcactg 2580
   tgatgtatat tgtgttgcaa aaaaaaaaaa aagtgtcttt gtttaaaatt acttggtttg 2640
   tgaatccatc ttgctttttc cccattggaa ctagtcatta acccatctct gaactggtag 2700
   aaaaacatct gaagagctag tctatcagca tctgacaggt gaattggatg gttctcagaa 2760
   ccatttcacc cagacagcct gtttctatcc tgtttaataa attagtttgg gttctctaca 2820
   tgcataacaa accctgctcc aatctgtcac ataaaagtct gtgacttgaa gtttagtcag 2880
   cacccccacc aaactttatt tttctatgtg ttttttgcaa catatgagtg ttttgaaaat 2940
   aaagtaccca tgtctttatt agaaaaaaaa aaaaaaaaaa aaaa 2984

<210> 336
<211> 147
<212> PRT
<213> Homo sapiens

<400> 336
Pro Ser Phe Pro Thr Leu Leu Ser Arg Arg His Leu Gly Ser Tyr Leu
1               5                   10                  15
Leu Asp Ser Glu Asn Thr Ser Gly Ala Leu Pro Arg Leu Pro Gln Thr
            20                  25                  30
Pro Lys Gln Pro Gln Lys Arg Ser Arg Ala Ala Phe Ser His Thr Gln
        35                  40                  45
Val Ile Glu Leu Glu Arg Lys Phe Ser His Gln Lys Tyr Leu Ser Ala
    50                  55                  60
Pro Glu Arg Ala His Leu Ala Lys Asn Leu Lys Leu Thr Glu Thr Gln
65                  70                  75                  80
Val Lys Ile Trp Phe Gln Asn Arg Arg Tyr Lys Thr Lys Arg Lys Gln
                85                  90                  95
Leu Ser Ser Glu Leu Gly Asp Leu Glu Lys His Ser Ser Leu Pro Ala
            100                 105                 110
Leu Lys Glu Glu Ala Phe Ser Arg Ala Ser Leu Val Ser Val Tyr Asn
        115                 120                 125
Ser Tyr Pro Tyr Tyr Pro Tyr Leu Tyr Cys Val Gly Ser Trp Ser Pro
    130                 135                 140
Ala Phe Trp
145

<210> 337
<211> 9
<212> PRT
<213> Homo sapiens

<400> 337
Ala Leu Thr Gly Phe Thr Phe Ser Ala
1               5

<210> 338
<211> 9

<212> PRT
<213> Homo sapiens

<400> 338
Leu Leu Ala Asn Asp Leu Met Leu Ile
1               5


<210> 339
<211> 318
<212> PRT
<213> Homo sapiens

<400> 339
Met Val Glu Leu Met Phe Pro Leu Leu Leu Leu Leu Leu Pro Phe Leu
1               5                   10                  15
Leu Tyr Met Ala Ala Pro Gln Ile Arg Lys Met Leu Ser Ser Gly Val
            20                  25                  30
Cys Thr Ser Thr Val Gln Leu Pro Gly Lys Val Val Val Val Thr Gly
        35                  40                  45
Ala Asn Thr Gly Ile Gly Lys Glu Thr Ala Lys Glu Leu Ala Gln Arg
    50                  55                  60
Gly Ala Arg Val Tyr Leu Ala Cys Arg Asp Val Glu Lys Gly Glu Leu
65                  70                  75                  80
Val Ala Lys Glu Ile Gln Thr Thr Thr Gly Asn Gln Gln Val Leu Val
                85                  90                  95
Arg Lys Leu Asp Leu Ser Asp Thr Lys Ser Ile Arg Ala Phe Ala Lys
            100                 105                 110
Gly Phe Leu Ala Glu Glu Lys His Leu His Val Leu Ile Asn Asn Ala
        115                 120                 125
Gly Val Met Met Cys Pro Tyr Ser Lys Thr Ala Asp Gly Phe Glu Met
    130                 135                 140
His Ile Gly Val Asn His Leu Gly His Phe Leu Leu Thr His Leu Leu
145                 150                 155                 160
Leu Glu Lys Leu Lys Glu Ser Ala Pro Ser Arg Ile Val Asn Val Ser
                165                 170                 175
Ser Leu Ala His His Leu Gly Arg Ile His Phe His Asn Leu Gln Gly
            180                 185                 190
Glu Lys Phe Tyr Asn Ala Gly Leu Ala Tyr Cys His Ser Lys Leu Ala
        195                 200                 205
Asn Ile Leu Phe Thr Gln Glu Leu Ala Arg Arg Leu Lys Gly Ser Gly
    210                 215                 220
Val Thr Thr Tyr Ser Val His Pro Gly Thr Val Gln Ser Glu Leu Val
225                 230                 235                 240
Arg His Ser Ser Phe Met Arg Trp Met Trp Trp Leu Phe Ser Phe Phe
                245                 250                 255
Ile Lys Thr Pro Gln Gln Gly Ala Gln Thr Ser Leu His Cys Ala Leu
            260                 265                 270
Thr Glu Gly Leu Glu Ile Leu Ser Gly Asn His Phe Ser Asp Cys His
        275                 280                 285
Val Ala Trp Val Ser Ala Gln Ala Arg Asn Glu Thr Ile Ala Arg Arg
    290                 295                 300
Leu Trp Asp Val Ser Cys Asp Leu Leu Gly Leu Pro Ile Asp
305                 310                 315


<210> 340
<211> 483
<212> DNA
<213> Homo sapiens

<400> 340
gccgaggtct gccttcacac ggaggacacg agactgcttc ctcaagggct cctgcctgcc 60
tggacactgg tgggaggcgc tgtttagttg gctgttttca gaggggtctt tcggagggac 120
ctcctgctgc aggctggagt gtctttattc ctggcgggag accgcacatt ccactgctga 180
ggttgtgggg gcggtttatc aggcagtgat aaacataaga tgtcatttcc ttgactccgg 240
ccttcaattt tctctttggc tgacgacgga gtccgtggtg tcccgatgta actgacccct 300
gctccaaacg tgacatcact gatgctcttc tcgggggtgc tgatggcccg cttggtcacg 360

tgctcaatct cgccattcga ctcttgctcc aaactgtatg aagacacctg actgcacgtt 420
ttttctgggc ttccagaatt taaagtgaaa ggcagcactc ctaagctccg actccgatgc 480
ctg 483

<210> 341
<211> 344
<212> DNA
<213> Homo sapiens

<400> 341
ctgctgctga gtcacagatt tcattataaa tagcctccct aaggaaaata cactgaatgc 60
tatttttact aaccattcta tttttataga aatagctgag agtttctaaa ccaactctct 120
gctgccttac aagtattaaa tattttactt ctttccataa agagtagctc aaaatatgca 180
attaatttaa taatttctga tgatggtttt atctgcagta atatgtatat catctattag 240
aatttactta atgaaaaact gaagagaaca aaatttgtaa ccactagcac ttaagtactc 300
ctgattctta acattgtctt taatgaccac aagacaacca acag 344

<210> 342
<211> 592
<212> DNA
<213> Homo sapiens

<400> 342
acagcaaaaa agaaactgag aagcccaaty tgctttcttg ttaacatcca cttatccaac 60
caatgtggaa acttcttata cttggttcca ttatgaagtt ggacaattgc tgctatcaca 120
cctggcaggt aaaccaatgc caagagagtg atggaaacca ttggcaagac tttgttgatg 180
accaggattg gaattttata aaaatattgt tgatgggaag ttgctaaagg gtgaattact 240
tccctcagaa gagtgtaaag aaaagtcaga gatgctataa tagcagctat tttaattggc 300
aagtgccact gtggaaagag ttcctgtgtg tgctgaagtt ctgaagggca gtcaaattca 360
tcagcatggg ctgtttggtg caaatgcaaa agcacaggtc tttttagcat gctggtctct 420
cccgtgtcct tatgcaaata atcgtcttct tctaaatttc tcctaggctt cattttccaa 480
agttcttctt ggtttgtgat gtcttttctg ctttccatta attctataaa atagtatggc 540
ttcagccacc cactcttcgc cttagcttga ccgtgagtct cggctgccgc tg 592

<210> 343
<211> 382
<212> DNA
<213> Homo sapiens

<400> 343
ttcttgacct cctcctcctt caagctcaaa caccacctcc cttattcagg accggcactt 60
cttaatgttt gtggctttct ctccagcctc tcttaggagg ggtaatggtg gagttggcat 120
cttgtaactc tcctttctcc tttcttcccc tttctctgcc cgcctttccc atcctgctgt 180
agacttcttg attgtcagtc tgtgtcacat ccagtgattg ttttggtttc tgttcccttt 240
ctgactgccc aaggggctca gaaccccagc aatcccttcc tttcactacc ttcttttttg 300
ggggtagttg gaagggactg aaattgtggg gggaaggtag gaggcacatc aataaagagg 360
aaaccaccaa gctgaaaaaa aa 382

<210> 344
<211> 536
<212> DNA
<213> Homo sapiens

<400> 344
ctgggcctga agctgtaggg taaatcagag gcaggcttct gagtgatgag agtcctgaga 60
caataggcca cataaacttg gctggatgga acctcacaat aaggtggtca cctcttgttt 120
gtttaggggg atgccaagga taaggccagc tcagttatat gaagagaagc agaacaaaca 180
agtctttcag agaaatggat gcaatcagag tgggatcccg gtcacatcaa ggtcacactc 240
caccttcatg tgcctgaatg gttgccaggt cagaaaaatc caccccttac gagtgcggct 300
tcgaccctat atcccccgcc cgcgtccctt tctccataaa attcttctta gtagctatta 360
ccttcttatt atttgatcta gaaattgccc tccttttacc cctaccatga gccctacaaa 420
caactaacct gccactaata gttatgtcat ccctcttatt aatcatcatc ctagccctaa 480
gtctggccta tgagtgacta caaaaaggat tagactgagc cgaataacaa aaaaaa 536

<210> 345
<211> 251
<212> DNA
<213> Homo sapiens

<400> 345
   accttttgag gtctctctca ccacctccac agccaccgtc accgtgggat gtgctggatg 60
   tgaatgaagc ccccatcttt gtgcctcctg aaaagagagt ggaagtgtcc gaggactttg 120
   gcgtgggcca ggaaatcaca tcctacactg cccaggagcc agacacattt atggaacaga 180
   aaataacata tcggatttgg agagacactg ccaactggct ggagattaat ccggacactg 240
   gtgccatttc c 251

<210> 346
<211> 282
<212> DNA
<213> Homo sapiens

<220>
<221> misc_feature
<222> 274
<223> n = A,T,C or G

<400> 346
cgcgtctctg acactgtgat catgacaggg gttcaaacag aaagtgcctg ggccctcctt 60
ctaagtcttg ttaccaaaaa aaggaaaaag aaaagatctt ctcagttaca aattctggga 120
agggagacta tacctggctc ttgccctaag tgagaggtct tccctcccgc accaaaaaat 180
agaaaggctt tctatttcac tggcccaggt agggggaagg agagtaactt tgagtctgtg 240
ggtctcattt cccaaggtgc cttcaatgct catnaaaacc aa 282

<210> 347
<211> 201
<212> DNA
<213> Homo sapiens

<220>
<221> misc_feature
<222> 79, 81, 87, 101
<223> n = A,T,C or G

<400> 347
   acacacataa tattataaaa tgccatctaa ttggaaggag ctttctatca ttgcaagtca 60
   taaatataac ttttaaaana ntactancag cttttaccta ngctcctaaa tgcttgtaaa 120
   tctgagactg actggaccca cccagaccca gggcaaagat acatgttacc atatcatctt 180
   tataaagaat ttttttttgt c 201

<210> 348
<211> 251
<212> DNA
<213> Homo sapiens

<400> 348
ctgttaatca caacatttgt gcatcacttg tgccaagtga gaaaatgttc taaaatcaca 60
agagagaaca gtgccagaat gaaactgacc ctaagtccca ggtgcccctg ggcaggcaga 120
aggagacact cccagcatgg aggagggttt atcttttcat cctaggtcag gtctacaatg 180
ggggaaggtt ttattataga actcccaaca gcccacctca ctcctgccac ccacccgatg 240
gccctgcctc c 251

<210> 349
<211> 251
<212> DNA
<213> Homo sapiens

<400> 349
taaaaatcaa gccatttaat tgtatctttg aaggtaaaca atatatggga gctggatcac 60
aacccctgag gatgccagag ctatgggtcc agaacatggt gtggtattat caacagagtt 120
cagaagggtc tgaactctac gtgttaccag agaacataat gcaattcatg cattccactt 180
agcaattttg taaaatacca gaaacagacc ccaagagtct ttcaagatga ggaaaattca 240
actcctggtt t 251

<210> 350
<211> 908
<212> DNA

<213> Homo sapiens

<400> 350
ctggacactt tgcgagggct tttgctggct gctgctgctg cccgtcatgc tactcatcgt 60
agcccgcccg gtgaagctcg ctgctttccc tacctcctta agtgactgcc aaacgcccac 120
cggctggaat tgctctggtt atgatgacag agaaaatgat ctcttcctct gtgacaccaa 180
cacctgtaaa tttgatgggg aatgtttaag aattggagac actgtgactt gcgtctgtca 240
gttcaagtgc aacaatgact atgtgcctgt gtgtggctcc aatggggaga gctaccagaa 300
tgagtgttac ctgcgacagg ctgcatgcaa acagcagagt gagatacttg tggtgtcaga 360
aggatcatgt gccacagtcc atgaaggctc tggagaaact agtcaaaagg agacatccac 420
ctgtgatatt tgccagtttg gtgcagaatg tgacgaagat gccgaggatg tctggtgtgt 480
gtgtaatatt gactgttctc aaaccaactt caatcccctc tgcgcttctg atgggaaatc 540
ttatgataat gcatgccaaa tcaaagaagc atcgtgtcag aaacaggaga aaattgaagt 600
catgtctttg ggtcgatgtc aagataacac aactacaact actaagtctg aagatgggca 660
ttatgcaaga acagattatg cagagaatgc taacaaatta gaagaaagtg ccagagaaca 720
ccacatacct tgtccggaac attacaatgg cttctgcatg catgggaagt gtgagcattc 780
tatcaatatg caggagccat cttgcaggtg tgatgctggt tatactggac aacactgtga 840
aaaaaaggac tacagtgttc tatacgttgt tcccggtcct gtacgatttc agtatgtctt 900
aatcgcag 908

<210> 351
<211> 472
<212> DNA
<213> Homo sapiens

<400> 351
ccagttattt gcaagtggta agagcctatt taccataaat aatactaaga accaactcaa 60
gtcaaacctt aatgccattg ttattgtgaa ttaggattaa gtagtaattt tcaaaattca 120
cattaacttg attttaaaat cagwtttgyg agtcatttac cacaagctaa atgtgtacac 180
tatgataaaa acaaccattg tattcctgtt tttctaaaca gtcctaattt ctaacactgt 240
atatatcctt cgacatcaat gaactttgtt ttcttttact ccagtaataa agtaggcaca 300
gatctgtcca caacaaactt gccctctcat gccttgcctc tcaccatgct ctgctccagg 360
tcagccccct tttggcctgt ttgttttgtc aaaaacctaa tctgcttctt gcttttcttg 420
gtaatatata tttagggaag atgttgcttt gcccacacac gaagcaaagt aa 472

<210> 352
<211> 251
<212> DNA
<213> Homo sapiens

<400> 352
ctcaaagcta atctctcggg aatcaaacca gaaaagggca aggatcttag gcatggtgga 60
tgtggataag gccaggtcaa tggctgcaag catgcagaga aagaggtaca tcggagcgtg 120
caggctgcgt tccgtcctta cgatgaagac cacgatgcag tttccaaaca ttgccactac 180
atacatggaa aggaggggga agccaaccca gaaatgggct ttctctaatc ctgggatacc 240
aataagcaca a 251

<210> 353
<211> 436
<212> DNA
<213> Homo sapiens

<400> 353
tttttttttt tttttttttt ttttttacaa caatgcagtc atttatttat tgagtatgtg 60
cacattatgg tattattact atactgatta tatttatcat gtgacttcta attaraaaat 120
gtatccaaaa gcaaaacagc agatatacaa aattaaagag acagaagata gacattaaca 180
gataaggcaa cttatacatt gacaatccaa atccaataca tttaaacatt tgggaaatga 240
gggggacaaa tggaagccar atcaaatttg tgtaaaacta ttcagtatgt ttcccttgct 300
tcatgtctga raaggctctc ccttcaatgg ggatgacaaa ctccaaatgc cacacaaatg 360
ttaacagaat actagattca cactggaacg ggggtaaaga agaaattatt ttctataaaa 420
gggctcctaa tgtagt 436

<210> 354
<211> 854
<212> DNA
<213> Homo sapiens

<400> 354
   ccttttctag ttcaccagtt ttctgcaagg atgctggtta gggagtgtct gcaggaggag 60
   caagtctgaa accaaatcta ggaaacatag gaaacgagcc aggcacaggg ctggtgggcc 120
   atcagggacc accctttggg ttgatatttt gcttaatctg catcttttga gtaagatcat 180
   ctggcagtag aagctgttct ccaggtacat ttctctagct catgtacaaa aacatcctga 240
   aggactttgt caggtgcctt gctaaaagcc agatgcgttc ggcacttcct tggtctgagg 300
   ttaattgcac acctacaggc actgggctca tgctttcaag tattttgtcc tcactttagg 360
   gtgagtgaaa gatccccatt ataggagcac ttgggagaga tcatataaaa gctgactctt 420
   gagtacatgc agtaatgggg tagatgtgtg tggtgtgtct tcattcctgc aagggtgctt 480
   gttagggagt gtttccagga ggaacaagtc tgaaaccaat catgaaataa atggtaggtg 540
   tgaactggaa aactaattca aaagagagat cgtgatatca gtgtggttga tacaccttgg 600
   caatatggaa ggctctaatt tgcccatatt tgaaataata attcagcttt ttgtaataca 660
   aaataacaaa ggattgagaa tcatggtgtc taatgtataa aagacccagg aaacataaat 720
   atatcaactg cataaatgta aaatgcatgt gacccaagaa ggccccaaag tggcagacaa 780
   cattgtaccc attttccctt ccaaaatgtg agcggcgggc ctgctgcttt caaggctgtc 840
   acacgggatg tcag 854

<210> 355
<211> 676
<212> DNA
<213> Homo sapiens

<400> 355
gaaattaagt atgagctaaa ttccctgtta aaacctctag ggtgacagaa tctcttcaac 60
caggtcaaag ctgatctttc tggaatgtca ccaaccaagg gcctatattt atcaaaagcc 120
atccacaagt catacctgga tgtcagcgaa gagggcacgg aggcagcagc agccactggg 180
gacagcatcg ctgtaaaaag cctaccaatg agagctcagt tcaaggcgaa ccaccccttc 240
ctgttcttta taaggcacac tcataccaac acgatcctat tctgtggcaa gcttgcctct 300
ccctaatcag atggggttga gtaaggctca gagttgcaga tgaggtgcag agacaatcct 360
gtgactttcc cacggccaaa aagctgttca cacctcacgc acctctgtgc ctcagtttgc 420
tcatctgcaa aataggtcta ggatttcttc caaccatttc atgagttgtg aagctaaggc 480
tttgttaatc atggaaaaag gtagacttat gcagaaagcc tttctggctt tcttatctgt 540
ggtgtctcat ttgagtgctg tccagtgaca tgatcaagtc aatgagtaaa attttaaggg 600
attagatttt cttgacttgt atgtatctgt gagatcttga ataagtgacc tgacatctct 660
gcttaaagaa aaccag 676

<210> 356
<211> 574
<212> DNA
<213> Homo sapiens

<400> 356
tttttttttt tttttcagga aaacattctc ttactttatt tgcatctcag caaaggttct 60
catgtggcac ctgactggca tcaaaccaaa gttcgtaggc caacaaagat gggccactca 120
caagcttccc atttgtagat ctcagtgcct atgagtatct gacacctgtt cctctcttca 180
gtctcttagg gaggcttaaa tctgtctcag gtgtgctaag agtgccagcc caaggkggtc 240
aaaagtccac aaaactgcag tctttgctgg gatagtaagc caagcagtgc ctggacagca 300
gagttctttt cttgggcaac agataaccag acaggactct aatcgtgctc ttattcaaca 360
ttcttctgtc tctgcctaga ctggaataaa aagccaatct ctctcgtggc acagggaagg 420
agatacaagc tcgtttacat gtgatagatc taacaaaggc atctaccgaa gtctggtctg 480
gatagacggc acagggagct cttaggtcag cgctgctggt tggaggacat tcctgagtcc 540
agctttgcag cctttgtgca acagtacttt ccca 574

<210> 357
<211> 393
<212> DNA
<213> Homo sapiens

<400> 357
tttttttttt tttttttttt tttttttttt tacagaatat aratgcttta tcactgkact 60
taatatggkg kcttgttcac tatacttaaa aatgcaccac tcataaatat ttaattcagc 120
aagccacaac caaracttga ttttatcaac aaaaacccct aaatataaac ggsaaaaaag 180
atagatataa ttattccagt ttttttaaaa cttaaaarat attccattgc cgaattaara 240
araarataag tgttatatgg aaagaagggc attcaagcac actaaaraaa cctgaggkaa 300
gcataatctg tacaaaatta aactgtcctt tttggcattt taacaaattt gcaacgktct 360
tttttttctt tttctgtttt tttttttttt tac 393

<210> 358
<211> 630

<212> DNA
<213> Homo sapiens

<400> 358
acagggtaaa caggaggatc cttgctctca cggagcttac attctagcag gaggacaata 60
ttaatgttta taggaaaatg atgagtttat gacaaaggaa gtagatagtg ttttacaaga 120
gcatagagta gggaagctaa tccagcacag ggaggtcaca gagacatccc taaggaagtg 180
gagtttaaac tgagagaagc aagtgcttaa actgaaggat gtgttgaaga agaagggaga 240
gtagaacaat ttgggcagag ggaaccttat agaccctaag gtgggaaggt tcaaagaact 300
gaaagagagc tagaacagct ggagccgttc tccggtgtaa agaggagtca aagagataag 360
attaaagatg tgaagattaa gatcttggtg gcattcaggg attggcactt ctacaagaaa 420
tcactgaagg gagtaatgtg acattacttt tcacttcagg atggccattc taactccagg 480
gggtagactg gactaggtaa gactggaggc aggtagacct cttctaaggc ctgcgatagt 540
gaaagacaaa aataagtggg gaaattcagg ggatagtgaa aatcagtagg acttaatgag 600
caagccagag gttcctccac aacaaccagt 630

<210> 359
<211> 620
<212> DNA
<213> Homo sapiens

<400> 359
acagcattcc aaaatataca tctagagact aarrgtaaat gctctatagt gaagaagtaa 60
taattaaaaa atgctactaa tatagaaaat ttataatcag aaaaataaat attcagggag 120
ctcaccagaa gaataaagtg ctctgccagt tattaaagga ttactgctgg tgaattaaat 180
atggcattcc ccaagggaaa tagagagatt cttctggatt atgttcaata tttatttcac 240
aggattaact gttttaggaa cagatataaa gcttcgccac ggaagagatg gacaaagcac 300
aaagacaaca tgatacctta ggaagcaaca ctaccctttc aggcataaaa tttggagaaa 360
tgcaacatta tgcttcatga ataatatgta gaaagaaggt ctgatgaaaa tgacatcctt 420
aatgtaagat aactttataa gaattctggg tcaaataaaa ttctttgaag aaaacatcca 480
aatgtcattg acttatcaaa tactatcttg gcatataacc tatgaaggca aaactaaaca 540
aacaaaaagc tcacaccaaa caaaaccatc aacttatttt gtattctata acatacgaga 600
ctgtaaagat gtgacagtgt 620

<210> 360
<211> 431
<212> DNA
<213> Homo sapiens

<400> 360
aaaaaaaaaa agccagaaca acatgtgata gataatatga ttggctgcac acttccagac 60
tgatgaatga tgaacgtgat ggactattgt atggagcaca tcttcagcaa gagggggaaa 120
tactcatcat ttttggccag cagttgtttg atcaccaaac atcatgccag aatactcagc 180
aaaccttctt agctcttgag aagtcaaagt ccgggggaat ttattcctgg caattttaat 240
tggactcctt atgtgagagc agcggctacc cagctggggt ggtggagcga acccgtcact 300
agtggacatg cagtggcaga gctcctggta accacctaga ggaatacaca ggcacatgtg 360
tgatgccaag cgtgacacct gtagcactca aatttgtctt gtttttgtct ttcggtgtgt 420
agattcttag t 431

<210> 361
<211> 351
<212> DNA
<213> Homo sapiens

<400> 361
acactgattt ccgatcaaaa gaatcatcat ctttaccttg acttttcagg gaattactga 60
actttcttct cagaagatag ggcacagcca ttgccttggc ctcacttgaa gggtctgcat 120
ttgggtcctc tggtctcttg ccaagtttcc cagccactcg agggagaaat atcgggaggt 180
ttgacttcct ccggggcttt cccgagggct tcaccgtgag ccctgcggcc ctcagggctg 240
caatcctgga ttcaatgtct gaaacctcgc tctctgcctg ctggacttct gaggccgtca 300
ctgccactct gtcctccagc tctgacagct cctcatctgt ggtcctgttg t 351

<210> 362
<211> 463
<212> DNA
<213> Homo sapiens

<400> 362

acttcatcag gccataatgg gtgcctcccg tgagaatcca agcacctttg gactgcgcga 60
tgtagatgag ccggctgaag atcttgcgca tgcgcggctt cagggcgaag ttcttggcgc 120
ccccggtcac agaaatgacc aggttgggtg ttttcaggtg ccagtgctgg gtcagcagct 180
cgtaaaggat ttccgcgtcc gtgtcgcagg acagacgtat atacttccct ttcttcccca 240
gtgtctcaaa ctgaatatcc ccaaaggcgt cggtaggaaa ttccttggtg tgtttcttgt 300
agttccattt ctcactttgg ttgatctggg tgccttccat gtgctggctc tgggcatagc 360
cacacttgca cacattctcc ctgataagca cgatggtgtg gacaggaagg aaggatttca 420
ttgagcctgc ttatggaaac tggtattgtt agcttaaata gac 463

<210> 363
<211> 653
<212> DNA
<213> Homo sapiens

<220>
<221> misc_feature
<222> 11, 16, 28, 68, 225, 311, 323, 352, 481, 554
<223> n = A,T,C or G

<400> 363
acccccgagt ncctgnctgg catactgnga acgaccaacg acacacccaa gctcggcctc 60
ctcttggnga ttctgggtga catcttcatg aatggcaacc gtgccagwga ggctgtcctc 120
tgggaggcac tacgcaagat gggactgcgt cctggggtga gacatcctct ccttggagat 180
ctaacgaaac ttctcaccta tgagttgtaa agcagaaata cctgnactac agacgagtgc 240
ccaacagcaa ccccccggaa gtatgagttc ctctrgggcc tccgttccta ccatgagasc 300
tagcaagatg naagtgttga gantcattgc agaggttcag aaaagagacc cntcgtgact 360
ggtctgcaca gttcatggag gctgcagatg aggccttgga tgctctggat gctgctgcag 420
ctgaggccga agcccgggct gaagcaagaa cccgcatggg aattggagat gaggctgtgt 480
ntgggccctg gagctgggat gacattgagt ttgagctgct gacctgggat gaggaaggag 540
attttggaga tccntggtcc agaattccat ttaccttctg ggccagatac caccagaatg 600
cccgctccag attccctcag acctttgccg gtcccattat tggtcstggt ggt 653

<210> 364
<211> 401
<212> DNA
<213> Homo sapiens

<400> 364
actagaggaa agacgttaaa ccactctact accacttgtg gaactctcaa agggtaaatg 60
acaaagccaa tgaatgactc taaaaacaat atttacattt aatggtttgt agacaataaa 120
aaaacaaggt ggatagatct agaattgtaa cattttaaga aaaccatagc atttgacaga 180
tgagaaagct caattataga tgcaaagtta taactaaact actatagtag taaagaaata 240
catttcacac ccttcatata aattcactat cttggcttga ggcactccat aaaatgtatc 300
acgtgcatag taaatcttta tatttgctat ggcgttgcac tagaggactt ggactgcaac 360
aagtggatgc gcggaaaatg aaatcttctt caatagccca g 401

<210> 365
<211> 356
<212> DNA
<213> Homo sapiens

<400> 365
ccagtgtcat atttgggctt aaaatttcaa gaagggcact tcaaatggct ttgcatttgc 60
atgtttcagt gctagagcgt aggaatagac cctggcgtcc actgtgagat gttcttcagc 120
taccagagca tcaagtctct gcagcaggtc attcttgggt aaagaaatga cttccacaaa 180
ctctccatcc cctggctttg gcttcggcct tgcgttttcg gcatcatctc cgttaatggt 240
gactgtcacg atgtgtatag tacagtttga caagcctggg tccatacaga ccgctggaga 300
acattcggca atgtcccctt tgtagccagt ttcttcttcg agctcccgga gagcag 356

<210> 366
<211> 1851
<212> DNA
<213> Homo sapiens

<400> 366
   tcatcaccat tgccagcagc ggcaccgtta gtcaggtttt ctgggaatcc cacatgagta 60
   cttccgtgtt cttcattctt cttcaatagc cataaatctt ctagctctgg ctggctgttt 120
   tcacttcctt taagcctttg tgactcttcc tctgatgtca gctttaagtc ttgttctgga 180
   ttgctgtttt cagaagagat ttttaacatc tgtttttctt tgtagtcaga aagtaactgg 240
   caaattacat gatgatgact agaaacagca tactctctgg ccgtctttcc agatcttgag 300
   aagatacatc aacattttgc tcaagtagag ggctgactat acttgctgat ccacaacata 360
   cagcaagtat gagagcagtt cttccatatc tatccagcgc atttaaattc gcttttttct 420
   tgattaaaaa tttcaccact tgctgttttt gctcatgtat accaagtagc agtggtgtga 480
   ggccatgctt gttttttgat tcgatatcag caccgtataa gagcagtgct ttggccatta 540
   atttatcttc attgtagaca gcatagtgta gagtggtatt tccatactca tctggaatat 600
   ttggatcagt gccatgttcc agcaacatta acgcacattc atcttcctgg cattgtacgg 660
   cctttgtcag agctgtcctc tttttgttgt caaggacatt aagttgacat cgtctgtcca 720
   gcacgagttt tactacttct gaattcccat tggcagaggc cagatgtaga gcagtcctct 780
   tttgcttgtc cctcttgttc acatccgtgt ccctgagcat gacgatgaga tcctttctgg 840
   ggactttacc ccaccaggca gctctgtgga gcttgtccag atcttctcca tggacgtggt 900
   acctgggatc catgaaggcg ctgtcatcgt agtctcccca agcgaccacg ttgctcttgc 960
   cgctcccctg cagcagggga agcagtggca gcaccacttg cacctcttgc tcccaagcgt 1020
   cttcacagag gagtcgttgt ggtctccaga agtgcccacg ttgctcttgc cgctccccct 1080
   gtccatccag ggaggaagaa atgcaggaaa tgaaagatgc atgcacgatg gtatactcct 1140
   cagccatcaa acttctggac agcaggtcac ttccagcaag gtggagaaag ctgtccaccc 1200
   acagaggatg agatccagaa accacaatat ccattcacaa acaaacactt ttcagccaga 1260
   cacaggtact gaaatcatgt catctgcggc aacatggtgg aacctaccca atcacacatc 1320
   aagagatgaa gacactgcag tatatctgca caacgtaata ctcttcatcc ataacaaaat 1380
   aatataattt tcctctggag ccatatggat gaactatgaa ggaagaactc cccgaagaag 1440
   ccagtcgcag agaagccaca ctgaagctct gtcctcagcc atcagcgcca cggacaggar 1500
   tgtgtttctt ccccagtgat gcagcctcaa gttatcccga agctgccgca gcacacggtg 1560
   gctcctgaga aacaccccag ctcttccggt ctaacacagg caagtcaata aatgtgataa 1620
   tcacataaac agaattaaaa gcaaagtcac ataagcatct caacagacac agaaaaggca 1680
   tttgacaaaa tccagcatcc ttgtatttat tgttgcagtt ctcagaggaa atgcttctaa 1740
   cttttcccca tttagtatta tgttggctgt gggcttgtca taggtggttt ttattacttt 1800
   aaggtatgtc ccttctatgc ctgttttgct gagggtttta attctcgtgc c 1851

<210> 367
<211> 668
<212> DNA
<213> Homo sapiens

<400> 367
cttgagcttc caaataygga agactggccc ttacacasgt caatgttaaa atgaatgcat 60
ttcagtattt tgaagataaa attrgtagat ctataccttg ttttttgatt cgatatcagc 120
accrtataag agcagtgctt tggccattaa tttatctttc attrtagaca gcrtagtgya 180
gagtggtatt tccatactca tctggaatat ttggatcagt gccatgttcc agcaacatta 240
acgcacattc atcttcctgg cattgtacgg cctgtcagta ttagacccaa aaacaaatta 300
catatcttag gaattcaaaa taacattcca cagctttcac caactagtta tatttaaagg 360
agaaaactca tttttatgcc atgtattgaa atcaaaccca cctcatgctg atatagttgg 420
ctactgcata cctttatcag agctgtcctc tttttgttgt caaggacatt aagttgacat 480
cgtctgtcca gcaggagttt tactacttct gaattcccat tggcagaggc cagatgtaga 540
gcagtcctat gagagtgaga agacttttta ggaaattgta gtgcactagc tacagccata 600
gcaatgattc atgtaactgc aaacactgaa tagcctgcta ttactctgcc ttcaaaaaaa 660
aaaaaaaa 668

<210> 368
<211> 1512
<212> DNA
<213> Homo sapiens

<400> 368
gggtcgccca gggggsgcgt gggctttcct cgggtgggtg tgggttttcc ctgggtgggg 60
tgggctgggc trgaatcccc tgctggggtt ggcaggtttt ggctgggatt gacttttytc 120
ttcaaacaga ttggaaaccc ggagttacct gctagttggt gaaactggtt ggtagacgcg 180
atctgttggc tactactggc ttctcctggc tgttaaaagc agatggtggt tgaggttgat 240
tccatgccgg ctgcttcttc tgtgaagaag ccatttggtc tcaggagcaa gatgggcaag 300
tggtgctgcc gttgcttccc ctgctgcagg gagagcggca agagcaacgt gggcacttct 360
ggagaccacg acgactctgc tatgaagaca ctcaggagca agatgggcaa gtggtgccgc 420
cactgcttcc cctgctgcag ggggagtggc aagagcaacg tgggcgcttc tggagaccac 480
gacgaytctg ctatgaagac actcaggaac aagatgggca agtggtgctg ccactgcttc 540
ccctgctgca gggggagcrg caagagcaag gtgggcgctt ggggagacta cgatgacagt 600
gccttcatgg agcccaggta ccacgtccgt ggagaagatc tggacaagct ccacagagct 660
gcctggtggg gtaaagtccc cagaaaggat ctcatcgtca tgctcaggga cactgacgtg 720
aacaagaagg acaagcaaaa gaggactgct ctacatctgg cctctgccaa tgggaattca 780
gaagtagtaa aactcstgct ggacagacga tgtcaactta atgtccttga caacaaaaag 840

aggacagctc tgayaaaggc cgtacaatgc caggaagatg aatgtgcgtt aatgttgctg 900
gaacatggca ctgatccaaa tattccagat gagtatggaa ataccactct rcactaygct 960
rtctayaatg aagataaatt aatggccaaa gcactgctct tatayggtgc tgatatcgaa 1020
tcaaaaaaca aggtatagat ctactaattt tatcttcaaa atactgaaat gcattcattt 1080
taacattgac gtgtgtaagg gccagtcttc cgtatttgga agctcaagca taacttgaat 1140
gaaaatattt tgaaatgacc taattatctm agactttatt ttaaatattg ttattttcaa 1200
agaagcatta gagggtacag tttttttttt ttaaatgcac ttctggtaaa tacttttgtt 1260
gaaaacactg aatttgtaaa aggtaatact tactattttt caatttttcc ctcctaggat 1320
tttttcccc taatgaatgt aagatggcaa aatttgccct gaaataggtt ttacatgaaa 1380
actccaagaa aagttaaaca tgtttcagtg aatagagatc ctgctccttt ggcaagttcc 1440
taaaaaacag taatagatac gaggtgatgc gcctgtcagt ggcaaggttt aagatatttc 1500
tgatctcgtg cc 1512

<210> 369
<211> 1853
<212> DNA
<213> Homo sapiens

<400> 369
gggtcgccca gggggsgcgt gggctttcct cgggtgggtg tgggttttcc ctgggtgggg 60
tgggctgggc trgaatcccc tgctggggtt ggcaggtttt ggctgggatt gacttttytc 120
ttcaaacaga ttggaaaccc ggagttacct gctagttggt gaaactggtt ggtagacgcg 180
atctgttggc tactactggc ttctcctggc tgttaaaagc agatggtggt tgaggttgat 240
tccatgccgg ctgcttcttc tgtgaagaag ccatttggtc tcaggagcaa gatgggcaag 300
tggtgctgcc gttgcttccc ctgctgcagg gagagcggca agagcaacgt gggcacttct 360
ggagaccacg acgactctgc tatgaagaca ctcaggagca agatgggcaa gtggtgccgc 420
cactgcttcc cctgctgcag ggggagtggc aagagcaacg tgggcgcttc tggagaccac 480
gacgaytctg ctatgaagac actcaggaac aagatgggca agtggtgctg ccactgcttc 540
ccctgctgca gggggagcrg caagagcaag gtgggcgctt ggggagacta cgatgacagy 600
gccttcatgg akcccaggta ccacgtccrt ggagaagatc tggacaagct ccacagagct 660
gcctggtggg gtaaagtccc cagaaaggat ctcatcgtca tgctcaggga cackgaygtg 720
aacaagargg acaagcaaaa gaggactgct ctacatctgg cctctgccaa tgggaattca 780
gaagtagtaa aactcstgct ggacagacga tgtcaactta atgtccttga caacaaaaag 840
aggacagctc tgayaaaggc cgtacaatgc caggaagatg aatgtgcgtt aatgttgctg 900
gaacatggca ctgatccaaa tattccagat gagtatggaa ataccactct rcactaygct 960
rtctayaatg aagataaatt aatggccaaa gcactgctct tatayggtgc tgatatcgaa 1020
tcaaaaaaca agcatggcct cacaccactg ytacttggtr tacatgagca aaaacagcaa 1080
gtsgtgaaat ttttaatyaa gaaaaaagcg aatttaaaat gcrctggata gatatggaag 1140
ractgctctc atacttgctg tatgttgtgg atcagcaagt atagtcagcc ytctacttga 1200
gcaaaatrtt gatgtatctt ctcaagatct ggaaagacgg ccagagagta tgctgtttct 1260
agtcatcatc atgtaatttg ccagttactt tctgactaca aagaaaaaca gatgttaaaa 1320
atctcttctg aaaacagcaa tccagaacaa gacttaaagc tgacatcaga ggaagagtca 1380
caaaggctta aaggaagtga aaacagccag ccagaggcat ggaaactttt aaatttaaac 1440
ttttggttta atgttttttt tttttgcctt aataatatta gatagtccca aatgaaatwa 1500
cctatgagac taggctttga gaatcaatag attctttttt taagaatctt ttggctagga 1560
gcggtgtctc acgcctgtaa ttccagcacc ttgagaggct gaggtgggca gatcacgaga 1620
tcaggagatc gagaccatcc tggctaacac ggtgaaaccc catctctact aaaaatacaa 1680
aaacttagct gggtgtggtg gcgggtgcct gtagtcccag ctactcagga rgctgaggca 1740
ggagaatggc atgaacccgg gaggtggagg ttgcagtgag ccgagatccg ccactacact 1800
ccagcctggg tgacagagca agactctgtc tcaaaaaaaa aaaaaaaaaa aaa 1853

<210> 370
<211> 2184
<212> DNA
<213> Homo sapiens

<400> 370
ggcacgagaa ttaaaaccct cagcaaaaca ggcatagaag ggacatacct taaagtaata 60
aaaaccacct atgacaagcc cacagccaac ataatactaa atggggaaaa gttagaagca 120
tttcctctga gaactgcaac aataaataca aggatgctgg attttgtcaa atgccttttc 180
tgtgtctgtt gagatgctta tgtgactttg cttttaattc tgtttatgtg attatcacat 240
ttattgactt gcctgtgtta gaccggaaga gctggggtgt ttctcaggag ccaccgtgtg 300
ctgcggcagc ttcgggataa cttgaggctg catcactggg gaagaaacac aytcctgtcc 360
gtggcgctga tggctgagga cagagcttca gtgtggcttc tctgcgactg gcttcttcgg 420
ggagttcttc cttcatagtt catccatatg gctccagagg aaaattatat tattttgtta 480
tggatgaaga gtattacgtt gtgcagatat actgcagtgt cttcatctct tgatgtgtga 540
ttgggtaggt tccaccatgt tgccgcagat gacatgattt cagtacctgt gtctggctga 600
aaagtgtttg tttgtgaatg gatattgtgg tttctggatc tcatcctctg tgggtggaca 660

gctttctcca ccttgctgga agtgacctgc tgtccagaag tttgatggct gaggagtata 720
ccatcgtgca tgcatctttc atttcctgca tttcttcctc cctggatgga caggggggagc 780
ggcaagagca acgtgggcac ttctggagac cacaacgact cctctgtgaa gacgcttggg 840
agcaagaggt gcaagtggtg ctgccactgc ttcccctgct gcaggggagc ggcaagagca 900
acgtggtcgc ttggggagac tacgatgaca gcgccttcat ggatcccagg taccacgtcc 960
atggagaaga tctggacaag ctccacagag ctgcctggtg gggtaaagtc cccagaaagg 1020
atctcatcgt catgctcagg gacacggatg tgaacaagag ggacaagcaa aagaggactg 1080
ctctacatct ggcctctgcc aatgggaatt cagaagtagt aaaactcgtg ctggacagac 1140
gatgtcaact taatgtcctt gacaacaaaa agaggacagc tctgacaaag gccgtacaat 1200
gccaggaaga tgaatgtgcg ttaatgttgc tggaacatgg cactgatcca aatattccag 1260
atgagtatgg aaataccact ctacactatg ctgtctacaa tgaagataaa ttaatggcca 1320
aagcactgct cttatacggt gctgatatcg aatcaaaaaa caagcatggc ctcacaccac 1380
tgctacttgg tatacatgag caaaaacagc aagtggtgaa atttttaatc aagaaaaaag 1440
cgaatttaaa tgcgctggat agatatggaa gaactgctct catacttgct gtatgttgtg 1500
gatcagcaag tatagtcagc cctctacttg agcaaaatgt tgatgtatct tctcaagatc 1560
tggaaagacg gccagagagt atgctgtttc tagtcatcat catgtaattt gccagttact 1620
ttctgactac aaagaaaaac agatgttaaa aatctcttct gaaaacagca atccagaaca 1680
agacttaaag ctgacatcag aggaagagtc acaaaggctt aaaggaagtg aaaacagcca 1740
gccagaggca tggaaacttt taaatttaaa cttttggttt aatgtttttt tttttgcct 1800
taataatatt agatagtccc aaatgaaatw acctatgaga ctaggctttg agaatcaata 1860
gattcttttt ttaagaatct tttggctagg agcggtgtct cacgcctgta attccagcac 1920
cttgagaggc tgaggtgggc agatcacgag atcaggagat cgagaccatc ctggctaaca 1980
cggtgaaacc ccatctctac taaaaataca aaaacttagc tgggtgtggt ggcgggtgcc 2040
tgtagtccca gctactcagg argctgaggc aggagaatgg catgaacccg ggaggtggag 2100
gttgcagtga gccgagatcc gccactacac tccagcctgg gtgacagagc aagactctgt 2160
ctcaaaaaaa aaaaaaaaaa aaaa 2184

<210> 371
<211> 1855
<212> DNA
<213> Homo sapiens

<220>
<221> misc_feature
<222> 606
<223> n = A,T,C or G

<400> 371
tgcacgcatc ggccagtgtc tgtgccacgt acactgacgc cccctgagat gtgcacgccg 60
cacgcgcacg ttgcacgcgc ggcagcggct tggctggctt gtaacggctt gcacgcgcac 120
gccgcccccg cataaccgtc agactggcct gtaacggctt gcaggcgcac gccgcacgcg 180
cgtaacggct tggctgccct gtaacggctt gcacgtgcat gctgcacgcg cgttaacggc 240
ttggctggca tgtagccgct tggcttggct ttgcattytt tgctkggctk ggcgttgkty 300
tcttggattg acgcttcctc cttggatkga cgtttcctcc ttggatkgac gtttcytyty 360
tcgcgttcct ttgctggact tgacctttty tctgctgggt ttggcattcc tttggggtgg 420
gctgggtgtt ttctccgggg gggktkgccc ttcctggggt gggcgtgggk cgcccccagg 480
gggcgtgggc tttccccggg tgggtgtggg ttttcctggg gtggggtggg ctgtgctggg 540
atccccctgc tggggttggc agggattgac ttttttcttc aaacagattg gaaacccgga 600
gtaacntgct agttggtgaa actggttggt agacgcgatc tgctggtact actgtttctc 660
ctggctgtta aaagcagatg gtggctgagg ttgattcaat gccggctgct tcttctgtga 720
agaagccatt tggtctcagg agcaagatgg gcaagtggtg cgccactgct tcccctgctg 780
caggggggagc ggcaagagca acgtgggcac ttctggagac cacaacgact cctctgtgaa 840
gacgcttggg agcaagaggt gcaagtggtg ctgcccactg cttcccctgc tgcaggggag 900
cggcaagagc aacgtggkcg cttggggaga ctacgatgac agcgccttca tggakcccag 960
gtaccacgtc crtggagaag atctggacaa gctccacaga gctgcctggt ggggtaaagt 1020
ccccagaaag gatctcatcg tcatgctcag ggacactgay gtgaacaaga rggacaagca 1080
aaagaggact gctctacatc tggcctctgc caatgggaat tcagaagtag taaaactcgt 1140
gctggacaga cgatgtcaac ttaatgtcct tgacaacaaa aagaggacag ctctgacaaa 1200
ggccgtacaa tgccaggaag atgaatgtgc gttaatgttg ctggaacatg gcactgatcc 1260
aaatattcca gatgagtatg gaaataccac tctacactat gctgtctaca atgaagataa 1320
attaatggcc aaagcactgc tcttatacgg tgctgatatc gaatcaaaaa acaaggtata 1380
gatctactaa ttttatcttc aaaatactga aatgcattca ttttaacatt gacgtgtgta 1440
agggccagtc ttccgtattt ggaagctcaa gcataacttg aatgaaaata ttttgaaatg 1500
acctaattat ctaagacttt attttaaata ttgttatttt caaagaagca ttagagggta 1560
cagttttttt tttttaaatg cacttctggt aaatactttt gttgaaaaca ctgaatttgt 1620
aaaaggtaat acttactatt tttcaatttt tccctcctag gatttttttc ccctaatgaa 1680
tgtaagatgg caaaatttgc cctgaaatag gttttacatg aaaactccaa gaaaagttaa 1740
acatgtttca gtgaatagag atcctgctcc tttggcaagt tcctaaaaaa cagtaataga 1800

tacgaggtga tgcgcctgtc agtggcaagg tttaagatat ttctgatctc gtgcc 1855

<210> 372
<211> 1059
<212> DNA
<213> Homo sapiens

<400> 372
gcaacgtggg cacttctgga gaccacaacg actcctctgt gaagacgctt gggagcaaga 60
ggtgcaagtg gtgctgccca ctgcttcccc tgctgcaggg gagcggcaag agcaacgtgg 120
gcgcttgrgg agactmcgat gacagygcct tcatggagcc caggtaccac gtccgtggag 180
aagatctgga caagctccac agagctgccc tggtggggta aagtccccag aaaggatctc 240
atcgtcatgc tcagggacac tgaygtgaac aagarggaca agcaaaagag gactgctcta 300
catctggcct ctgccaatgg gaattcagaa gtagtaaaac tcstgctgga cagacgatgt 360
caacttaatg tccttgacaa caaaaagagg acagctctga yaaaggccgt acaatgccag 420
gaagatgaat gtgcgttaat gttgctggaa catggcactg atccaaatat tccagatgag 480
tatggaaata ccactctrca ctaygctrtc tayaatgaag ataaattaat ggccaaagca 540
ctgctcttat ayggtgctga tatcgaatca aaaaacaagg tatagatcta ctaattttat 600
cttcaaaata ctgaaatgca ttcattttaa cattgacgtg tgtaagggcc agtcttccgt 660
atttggaagc tcaagcataa cttgaatgaa aatattttga aatgacctaa ttatctaaga 720
ctttatttta aatattgtta ttttcaaaga agcattagag ggtacagttt ttttttttta 780
aatgcacttc tggtaaatac ttttgttgaa aacactgaat ttgtaaaagg taatacttac 840
tatttttcaa tttttccctc ctaggatttt tttcccctaa tgaatgtaag atggcaaaat 900
ttgccctgaa ataggtttta catgaaaact ccaagaaaag ttaaacatgt ttcagtgaat 960
agagatcctg ctcctttggc aagttcctaa aaaacagtaa tagatacgag gtgatgcgcc 1020
tgtcagtggc aaggtttaag atatttctga tctcgtgcc 1059

<210> 373
<211> 1155
<212> DNA
<213> Homo sapiens

<400> 373
atggtggttg aggttgattc catgccggct gcctcttctg tgaagaagcc atttggtctc 60
aggagcaaga tgggcaagtg gtgctgccgt tgcttcccct gctgcaggga gagcggcaag 120
agcaacgtgg gcacttctgg agaccacgac gactctgcta tgaagacact caggagcaag 180
atgggcaagt ggtgccgcca ctgcttcccc tgctgcaggg ggagtggcaa gagcaacgtg 240
ggcgcttctg gagaccacga cgactctgct atgaagacac tcaggaacaa gatgggcaag 300
tggtgctgcc actgcttccc ctgctgcagg gggagcggca agagcaaggt gggcgcttgg 360
ggagactacg atgacagtgc cttcatggag cccaggtacc acgtccgtgg agaagatctg 420
gacaagctcc acagagctgc ctggtggggt aaagtcccca gaaaggatct catcgtcatg 480
ctcagggaca ctgacgtgaa caagaaggac aagcaaaaga ggactgctct acatctggcc 540
tctgccaatg ggaattcaga agtagtaaaa ctcctgctgg acagacgatg tcaacttaat 600
gtccttgaca acaaaaagag gacagctctg ataaaggccg tacaatgcca ggaagatgaa 660
tgtgcgttaa tgttgctgga acatggcact gatccaaata ttccagatga gtatggaaat 720
accactctgc actacgctat ctataatgaa gataaattaa tggccaaagc actgctctta 780
tatggtgctg atatcgaatc aaaaaacaag catggcctca caccactgtt acttggtgta 840
catgagcaaa aacagcaagt cgtgaaattt ttaatcaaga aaaaagcgaa tttaaatgca 900
ctggatagat atggaaggac tgctctcata cttgctgtat gttgtggatc agcaagtata 960
gtcagccttc tacttgagca aaatattgat gtatcttctc aagatctatc tggacagacg 1020
gccagagagt atgctgtttc tagtcatcat catgtaattt gccagttact ttctgactac 1080
aaagaaaaac agatgctaaa aatctcttct gaaaacagca atccagaaaa tgtctcaaga 1140
accagaaata aataa 1155

<210> 374
<211> 2000
<212> DNA
<213> Homo sapiens

<400> 374
atggtggttg aggttgattc catgccggct gcctcttctg tgaagaagcc atttggtctc 60
aggagcaaga tgggcaagtg gtgctgccgt tgcttcccct gctgcaggga gagcggcaag 120
agcaacgtgg gcacttctgg agaccacgac gactctgcta tgaagacact caggagcaag 180
atgggcaagt ggtgccgcca ctgcttcccc tgctgcaggg ggagtggcaa gagcaacgtg 240
ggcgcttctg gagaccacga cgactctgct atgaagacac tcaggaacaa gatgggcaag 300
tggtgctgcc actgcttccc ctgctgcagg gggagcggca agagcaaggt gggcgcttgg 360
ggagactacg atgacagtgc cttcatggag cccaggtacc acgtccgtgg agaagatctg 420
gacaagctcc acagagctgc ctggtggggt aaagtcccca gaaaggatct catcgtcatg 480

ctcagggaca ctgacgtgaa caagaaggac aagcaaaaga ggactgctct acatctggcc 540
tctgccaatg ggaattcaga agtagtaaaa ctcctgctgg acagacgatg tcaacttaat 600
gtccttgaca acaaaaagag gacagctctg ataaaggccg tacaatgcca ggaagatgaa 660
tgtgcgttaa tgttgctgga acatggcact gatccaaata ttccagatga gtatggaaat 720
accactctgc actacgctat ctataatgaa gataaattaa tggccaaagc actgctctta 780
tatggtgctg atatcgaatc aaaaaacaag catggcctca caccactgtt acttggtgta 840
catgagcaaa aacagcaagt cgtgaaattt ttaatcaaga aaaaagcgaa tttaaatgca 900
ctggatagat atggaaggac tgctctcata cttgctgtat gttgtggatc agcaagtata 960
gtcagccttc tacttgagca aaatattgat gtatcttctc aagatctatc tggacagacg 1020
gccagagagt atgctgtttc tagtcatcat catgtaattt gccagttact ttctgactac 1080
aaagaaaaac agatgctaaa aatctcttct gaaaacagca atccagaaca agacttaaag 1140
ctgacatcag aggaagagtc acaaaggttc aaaggcagtg aaaatagcca gccagagaaa 1200
atgtctcaag aaccagaaat aaataaggat ggtgatagag aggttgaaga agaaatgaag 1260
aagcatgaaa gtaataatgt gggattacta gaaaacctga ctaatggtgt cactgctggc 1320
aatggtgata atggattaat tcctcaaagg aagagcagaa cacctgaaaa tcagcaattt 1380
cctgacaacg aaagtgaaga gtatcacaga atttgcgaat tagtttctga ctacaaagaa 1440
aaacagatgc caaaatactc ttctgaaaac agcaacccag aacaagactt aaagctgaca 1500
tcagaggaag agtcacaaag gcttgagggc agtgaaaatg gccagccaga gctagaaaat 1560
tttatggcta tcgaagaaat gaagaagcac ggaagtactc atgtcggatt cccagaaaac 1620
ctgactaatg gtgccactgc tggcaatggt gatgatggat taattcctcc aaggaagagc 1680
agaacacctg aaagccagca atttcctgac actgagaatg aagagtatca cagtgacgaa 1740
caaaatgata ctcagaagca attttgtgaa gaacagaaca ctggaatatt acacgatgag 1800
attctgattc atgaagaaaa gcagatagaa gtggttgaaa aaatgaattc tgagctttct 1860
cttagttgta agaaagaaaa agacatcttg catgaaaata gtacgttgcg ggaagaaatt 1920
gccatgctaa gactggagct agacacaatg aaacatcaga gccagctaaa aaaaaaaaaa 1980
aaaaaaaaaa aaaaaaaaaa 2000

<210> 375
<211> 2040
<212> DNA
<213> Homo sapiens

<400> 375
atggtggttg aggttgattc catgccggct gcctcttctg tgaagaagcc atttggtctc 60
aggagcaaga tgggcaagtg gtgctgccgt tgcttcccct gctgcaggga gagcggcaag 120
agcaacgtgg gcacttctgg agaccacgac gactctgcta tgaagacact caggagcaag 180
atgggcaagt ggtgccgcca ctgcttcccc tgctgcaggg ggagtggcaa gagcaacgtg 240
ggcgcttctg gagaccacga cgactctgct atgaagacac tcaggaacaa gatgggcaag 300
tggtgctgcc actgcttccc ctgctgcagg gggagcggca agagcaaggt gggcgcttgg 360
ggagactacg atgacagtgc cttcatggag cccaggtacc acgtccgtgg agaagatctg 420
gacaagctcc acagagctgc ctggtggggt aaagtcccca gaaaggatct catcgtcatg 480
ctcagggaca ctgacgtgaa caagaaggac aagcaaaaga ggactgctct acatctggcc 540
tctgccaatg ggaattcaga agtagtaaaa ctcctgctgg acagacgatg tcaacttaat 600
gtccttgaca acaaaaagag gacagctctg ataaaggccg tacaatgcca ggaagatgaa 660
tgtgcgttaa tgttgctgga acatggcact gatccaaata ttccagatga gtatggaaat 720
accactctgc actacgctat ctataatgaa gataaattaa tggccaaagc actgctctta 780
tatggtgctg atatcgaatc aaaaaacaag catggcctca caccactgtt acttggtgta 840
catgagcaaa aacagcaagt cgtgaaattt ttaatcaaga aaaaagcgaa tttaaatgca 900
ctggatagat atggaaggac tgctctcata cttgctgtat gttgtggatc agcaagtata 960
gtcagccttc tacttgagca aaatattgat gtatcttctc aagatctatc tggacagacg 1020
gccagagagt atgctgtttc tagtcatcat catgtaattt gccagttact ttctgactac 1080
aaagaaaaac agatgctaaa aatctcttct gaaaacagca atccagaaca agacttaaag 1140
ctgacatcag aggaagagtc acaaaggttc aaaggcagtg aaaatagcca gccagagaaa 1200
atgtctcaag aaccagaaat aaataaggat ggtgatagag aggttgaaga agaaatgaag 1260
aagcatgaaa gtaataatgt gggattacta gaaaacctga ctaatggtgt cactgctggc 1320
aatggtgata atggattaat tcctcaaagg aagagcagaa cacctgaaaa tcagcaattt 1380
cctgacaacg aaagtgaaga gtatcacaga atttgcgaat tagtttctga ctacaaagaa 1440
aaacagatgc caaaatactc ttctgaaaac agcaacccag aacaagactt aaagctgaca 1500
tcagaggaag agtcacaaag gcttgagggc agtgaaaatg gccagccaga gaaaagatct 1560
caagaaccag aaataaataa ggatggtgat agagagctag aaaattttat ggctatcgaa 1620
gaaatgaaga agcacggaag tactcatgtc ggattcccag aaaacctgac taatggtgcc 1680
actgctggca atggtgatga tggattaatt cctccaagga agagcagaac acctgaaagc 1740
cagcaatttc ctgacactga gaatgaagag tatcacagtg acgaacaaaa tgatactcag 1800
aagcaatttt gtgaagaaca gaacactgga atattacacg atgagattct gattcatgaa 1860
gaaaagcaga tagaagtggt tgaaaaaatg aattctgagc tttctcttag ttgtaagaaa 1920
gaaaaagaca tcttgcatga aaatagtacg ttgcgggaag aaattgccat gctaagactg 1980
gagctagaca caatgaaaca tcagagccag ctaaaaaaaa aaaaaaaaaa aaaaaaaaaa 2040

<210> 376
<211> 329
<212> PRT
<213> Homo sapiens

<400> 376
Met Asp Ile Val Val Ser Gly Ser His Pro Leu Trp Val Asp Ser Phe
1               5                   10                  15
Leu His Leu Ala Gly Ser Asp Leu Leu Ser Arg Ser Leu Met Ala Glu
            20                  25                  30
Glu Tyr Thr Ile Val His Ala Ser Phe Ile Ser Cys Ile Ser Ser Ser
        35                  40                  45
Leu Asp Gly Gln Gly Glu Arg Gln Glu Gln Arg Gly His Phe Trp Arg
    50                  55                  60
Pro Gln Arg Leu Leu Cys Glu Asp Ala Trp Glu Gln Glu Val Gln Val
65                  70                  75                  80
Val Leu Pro Leu Leu Pro Leu Leu Gln Gly Ser Gly Lys Ser Asn Val
                85                  90                  95
Val Ala Trp Gly Asp Tyr Asp Asp Ser Ala Phe Met Asp Pro Arg Tyr
            100                 105                 110
His Val His Gly Glu Asp Leu Asp Lys Leu His Arg Ala Ala Trp Trp
        115                 120                 125
Gly Lys Val Pro Arg Lys Asp Leu Ile Val Met Leu Arg Asp Thr Asp
    130                 135                 140
Val Asn Lys Arg Asp Lys Gln Lys Arg Thr Ala Leu His Leu Ala Ser
145                 150                 155                 160
Ala Asn Gly Asn Ser Glu Val Val Lys Leu Val Leu Asp Arg Arg Cys
                165                 170                 175
Gln Leu Asn Val Leu Asp Asn Lys Lys Arg Thr Ala Leu Thr Lys Ala
            180                 185                 190
Val Gln Cys Gln Glu Asp Glu Cys Ala Leu Met Leu Leu Glu His Gly
        195                 200                 205
Thr Asp Pro Asn Ile Pro Asp Glu Tyr Gly Asn Thr Thr Leu His Tyr
    210                 215                 220
Ala Val Tyr Asn Glu Asp Lys Leu Met Ala Lys Ala Leu Leu Leu Tyr
225                 230                 235                 240
Gly Ala Asp Ile Glu Ser Lys Asn Lys His Gly Leu Thr Pro Leu Leu
                245                 250                 255
Leu Gly Ile His Glu Gln Lys Gln Gln Val Val Lys Phe Leu Ile Lys
            260                 265                 270
Lys Lys Ala Asn Leu Asn Ala Leu Asp Arg Tyr Gly Arg Thr Ala Leu
        275                 280                 285
Ile Leu Ala Val Cys Cys Gly Ser Ala Ser Ile Val Ser Pro Leu Leu
    290                 295                 300
Glu Gln Asn Val Asp Val Ser Ser Gln Asp Leu Glu Arg Arg Pro Glu
305                 310                 315                 320
Ser Met Leu Phe Leu Val Ile Ile Met
                325

<210> 377
<211> 148
<212> PRT
<213> Homo sapiens

<220>
<221> VARIANT
<222> 3, 46, 69, 88, 124
<223> Xaa = Any Amino Acid

<400> 377
Met Thr Xaa Pro Ser Trp Ser Pro Gly Thr Thr Ser Val Glu Lys Ile
1               5                   10                  15
Trp Thr Ser Ser Thr Glu Leu Pro Trp Trp Gly Lys Val Pro Arg Lys
            20                  25                  30
Asp Leu Ile Val Met Leu Arg Asp Thr Asp Val Asn Lys Xaa Asp Lys
        35                  40                  45

Gln Lys Arg Thr Ala Leu His Leu Ala Ser Ala Asn Gly Asn Ser Glu
    50                  55                  60
Val Val Lys Leu Xaa Leu Asp Arg Arg Cys Gln Leu Asn Val Leu Asp
65                  70                  75                  80
Asn Lys Lys Arg Thr Ala Leu Xaa Lys Ala Val Gln Cys Gln Glu Asp
                85                  90                  95
Glu Cys Ala Leu Met Leu Leu Glu His Gly Thr Asp Pro Asn Ile Pro
            100                 105                 110
Asp Glu Tyr Gly Asn Thr Thr Leu His Tyr Ala Xaa Tyr Asn Glu Asp
        115                 120                 125
Lys Leu Met Ala Lys Ala Leu Leu Leu Tyr Gly Ala Asp Ile Glu Ser
    130                 135                 140
Lys Asn Lys Val
145

<210> 378
<211> 1719
<212> PRT
<213> Homo sapiens

<400> 378


<210> 379
<211> 656
<212> PRT
<213> Homo sapiens

<400> 379
Met Val Val Glu Val Asp Ser Met Pro Ala Ala Ser Ser Val Lys Lys
1               5                   10                  15
Pro Phe Gly Leu Arg Ser Lys Met Gly Lys Trp Cys Cys Arg Cys Phe
            20                  25                  30
Pro Cys Cys Arg Glu Ser Gly Lys Ser Asn Val Gly Thr Ser Gly Asp
        35                  40                  45
His Asp Asp Ser Ala Met Lys Thr Leu Arg Ser Lys Met Gly Lys Trp
    50                  55                  60
Cys Arg His Cys Phe Pro Cys Cys Arg Gly Ser Gly Lys Ser Asn Val
65                  70                  75                  80
Gly Ala Ser Gly Asp His Asp Asp Ser Ala Met Lys Thr Leu Arg Asn
                85                  90                  95
Lys Met Gly Lys Trp Cys Cys His Cys Phe Pro Cys Cys Arg Gly Ser
            100                 105                 110
Gly Lys Ser Lys Val Gly Ala Trp Gly Asp Tyr Asp Asp Ser Ala Phe
        115                 120                 125
Met Glu Pro Arg Tyr His Val Arg Gly Glu Asp Leu Asp Lys Leu His
    130                 135                 140
Arg Ala Ala Trp Trp Gly Lys Val Pro Arg Lys Asp Leu Ile Val Met
145                 150                 155                 160
Leu Arg Asp Thr Asp Val Asn Lys Lys Asp Lys Gln Lys Arg Thr Ala
                165                 170                 175
Leu His Leu Ala Ser Ala Asn Gly Asn Ser Glu Val Val Lys Leu Leu
            180                 185                 190
Leu Asp Arg Arg Cys Gln Leu Asn Val Leu Asp Asn Lys Lys Arg Thr
        195                 200                 205

Ala Leu Ile Lys Ala Val Gln Cys Gln Glu Asp Glu Cys Ala Leu Met
    210                 215                 220
Leu Leu Glu His Gly Thr Asp Pro Asn Ile Pro Asp Glu Tyr Gly Asn
225                 230                 235                 240
Thr Thr Leu His Tyr Ala Ile Tyr Asn Glu Asp Lys Leu Met Ala Lys
                245                 250                 255
Ala Leu Leu Leu Tyr Gly Ala Asp Ile Glu Ser Lys Asn Lys His Gly
            260                 265                 270
Leu Thr Pro Leu Leu Leu Gly Val His Glu Gln Lys Gln Gln Val Val
        275                 280                 285
Lys Phe Leu Ile Lys Lys Lys Ala Asn Leu Asn Ala Leu Asp Arg Tyr
    290                 295                 300
Gly Arg Thr Ala Leu Ile Leu Ala Val Cys Cys Gly Ser Ala Ser Ile
305                 310                 315                 320
Val Ser Leu Leu Leu Glu Gln Asn Ile Asp Val Ser Ser Gln Asp Leu
                325                 330                 335
Ser Gly Gln Thr Ala Arg Glu Tyr Ala Val Ser Ser His His His Val
            340                 345                 350
Ile Cys Gln Leu Leu Ser Asp Tyr Lys Glu Lys Gln Met Leu Lys Ile
        355                 360                 365
Ser Ser Glu Asn Ser Asn Pro Glu Gln Asp Leu Lys Leu Thr Ser Glu
    370                 375                 380
Glu Glu Ser Gln Arg Phe Lys Gly Ser Glu Asn Ser Gln Pro Glu Lys
385                 390                 395                 400
Met Ser Gln Glu Pro Glu Ile Asn Lys Asp Gly Asp Arg Glu Val Glu
                405                 410                 415
Glu Glu Met Lys Lys His Glu Ser Asn Asn Val Gly Leu Leu Glu Asn
            420                 425                 430
Leu Thr Asn Gly Val Thr Ala Gly Asn Gly Asp Asn Gly Leu Ile Pro
        435                 440                 445
Gln Arg Lys Ser Arg Thr Pro Glu Asn Gln Gln Phe Pro Asp Asn Glu
    450                 455                 460
Ser Glu Glu Tyr His Arg Ile Cys Glu Leu Val Ser Asp Tyr Lys Glu
465                 470                 475                 480
Lys Gln Met Pro Lys Tyr Ser Ser Glu Asn Ser Asn Pro Glu Gln Asp
                485                 490                 495
Leu Lys Leu Thr Ser Glu Glu Glu Ser Gln Arg Leu Glu Gly Ser Glu
            500                 505                 510
Asn Gly Gln Pro Glu Leu Glu Asn Phe Met Ala Ile Glu Glu Met Lys
        515                 520                 525
Lys His Gly Ser Thr His Val Gly Phe Pro Glu Asn Leu Thr Asn Gly
    530                 535                 540
Ala Thr Ala Gly Asn Gly Asp Asp Gly Leu Ile Pro Pro Arg Lys Ser
545                 550                 555                 560
Arg Thr Pro Glu Ser Gln Gln Phe Pro Asp Thr Glu Asn Glu Glu Tyr
                565                 570                 575
His Ser Asp Glu Gln Asn Asp Thr Gln Lys Gln Phe Cys Glu Glu Gln
            580                 585                 590
Asn Thr Gly Ile Leu His Asp Glu Ile Leu Ile His Glu Glu Lys Gln
        595                 600                 605
Ile Glu Val Val Glu Lys Met Asn Ser Glu Leu Ser Leu Ser Cys Lys
    610                 615                 620
Lys Glu Lys Asp Ile Leu His Glu Asn Ser Thr Leu Arg Glu Glu Ile
625                 630                 635                 640
Ala Met Leu Arg Leu Glu Leu Asp Thr Met Lys His Gln Ser Gln Leu
                645                 650                 655

<210> 380
<211> 671
<212> PRT
<213> Homo sapiens

<400> 380
Met Val Val Glu Val Asp Ser Met Pro Ala Ala Ser Ser Val Lys Lys
1               5                   10                  15
Pro Phe Gly Leu Arg Ser Lys Met Gly Lys Trp Cys Cys Arg Cys Phe
            20                  25                  30

Pro Cys Cys Arg Glu Ser Gly Lys Ser Asn Val Gly Thr Ser Gly Asp
        35                  40                  45
His Asp Asp Ser Ala Met Lys Thr Leu Arg Ser Lys Met Gly Lys Trp
    50                  55                  60
Cys Arg His Cys Phe Pro Cys Cys Arg Gly Ser Gly Lys Ser Asn Val
65                  70                  75                  80
Gly Ala Ser Gly Asp His Asp Asp Ser Ala Met Lys Thr Leu Arg Asn
                85                  90                  95
Lys Met Gly Lys Trp Cys Cys His Cys Phe Pro Cys Cys Arg Gly Ser
            100                 105                 110
Gly Lys Ser Lys Val Gly Ala Trp Gly Asp Tyr Asp Asp Ser Ala Phe
        115                 120                 125
Met Glu Pro Arg Tyr His Val Arg Gly Glu Asp Leu Asp Lys Leu His
    130                 135                 140
Arg Ala Ala Trp Trp Gly Lys Val Pro Arg Lys Asp Leu Ile Val Met
145                 150                 155                 160
Leu Arg Asp Thr Asp Val Asn Lys Lys Asp Lys Gln Lys Arg Thr Ala
                165                 170                 175
Leu His Leu Ala Ser Ala Asn Gly Asn Ser Glu Val Val Lys Leu Leu
            180                 185                 190
Leu Asp Arg Arg Cys Gln Leu Asn Val Leu Asp Asn Lys Lys Arg Thr
        195                 200                 205
Ala Leu Ile Lys Ala Val Gln Cys Gln Glu Asp Glu Cys Ala Leu Met
    210                 215                 220
Leu Leu Glu His Gly Thr Asp Pro Asn Ile Pro Asp Glu Tyr Gly Asn
225                 230                 235                 240
Thr Thr Leu His Tyr Ala Ile Tyr Asn Glu Asp Lys Leu Met Ala Lys
                245                 250                 255
Ala Leu Leu Leu Tyr Gly Ala Asp Ile Glu Ser Lys Asn Lys His Gly
            260                 265                 270
Leu Thr Pro Leu Leu Leu Gly Val His Glu Gln Lys Gln Gln Val Val
        275                 280                 285
Lys Phe Leu Ile Lys Lys Lys Ala Asn Leu Asn Ala Leu Asp Arg Tyr
    290                 295                 300
Gly Arg Thr Ala Leu Ile Leu Ala Val Cys Cys Gly Ser Ala Ser Ile
305                 310                 315                 320
Val Ser Leu Leu Leu Glu Gln Asn Ile Asp Val Ser Ser Gln Asp Leu
                325                 330                 335
Ser Gly Gln Thr Ala Arg Glu Tyr Ala Val Ser Ser His His His Val
            340                 345                 350
Ile Cys Gln Leu Leu Ser Asp Tyr Lys Glu Lys Gln Met Leu Lys Ile
        355                 360                 365
Ser Ser Glu Asn Ser Asn Pro Glu Gln Asp Leu Lys Leu Thr Ser Glu
    370                 375                 380
Glu Glu Ser Gln Arg Phe Lys Gly Ser Glu Asn Ser Gln Pro Glu Lys
385                 390                 395                 400
Met Ser Gln Glu Pro Glu Ile Asn Lys Asp Gly Asp Arg Glu Val Glu
                405                 410                 415
Glu Glu Met Lys Lys His Glu Ser Asn Asn Val Gly Leu Leu Glu Asn
            420                 425                 430
Leu Thr Asn Gly Val Thr Ala Gly Asn Gly Asp Asn Gly Leu Ile Pro
        435                 440                 445
Gln Arg Lys Ser Arg Thr Pro Glu Asn Gln Gln Phe Pro Asp Asn Glu
    450                 455                 460
Ser Glu Glu Tyr His Arg Ile Cys Glu Leu Val Ser Asp Tyr Lys Glu
465                 470                 475                 480
Lys Gln Met Pro Lys Tyr Ser Ser Glu Asn Ser Asn Pro Glu Gln Asp
                485                 490                 495
Leu Lys Leu Thr Ser Glu Glu Glu Ser Gln Arg Leu Glu Gly Ser Glu
            500                 505                 510
Asn Gly Gln Pro Glu Lys Arg Ser Gln Glu Pro Glu Ile Asn Lys Asp
        515                 520                 525
Gly Asp Arg Glu Leu Glu Asn Phe Met Ala Ile Glu Glu Met Lys Lys
    530                 535                 540
His Gly Ser Thr His Val Gly Phe Pro Glu Asn Leu Thr Asn Gly Ala
545                 550                 555                 560
Thr Ala Gly Asn Gly Asp Asp Gly Leu Ile Pro Pro Arg Lys Ser Arg
                565                 570                 575

Thr Pro Glu Ser Gln Gln Phe Pro Asp Thr Glu Asn Glu Glu Tyr His
            580                 585                 590
Ser Asp Glu Gln Asn Asp Thr Gln Lys Gln Phe Cys Glu Glu Gln Asn
        595                 600                 605
Thr Gly Ile Leu His Asp Glu Ile Leu Ile His Glu Glu Lys Gln Ile
    610                 615                 620
Glu Val Val Glu Lys Met Asn Ser Glu Leu Ser Leu Ser Cys Lys Lys
625                 630                 635                 640
Glu Lys Asp Ile Leu His Glu Asn Ser Thr Leu Arg Glu Glu Ile Ala
                645                 650                 655
Met Leu Arg Leu Glu Leu Asp Thr Met Lys His Gln Ser Gln Leu
            660                 665                 670

<210> 381
<211> 251
<212> DNA
<213> Homo sapiens

<400> 381
ggagaagcgt ctgctggggc aggaaggggt ttccctgccc tctcacctgt ccctcaccaa 60
ggtaacatgc ttcccctaag ggtatcccaa cccaggggcc tcaccatgac ctctgagggg 120
ccaatatccc aggagaagca ttggggagtt gggggcaggt gaaggaccca ggactcacac 180
atcctgggcc tccaaggcag aggagagggt cctcaagaag gtcaggagga aaatccgtaa 240
caagcagtca g                                                      251

<210> 382
<211> 3279
<212> DNA
<213> Homo sapiens

<400> 382
cttcctgcag cccccatgct ggtgaggggc acgggcagga acagtggacc caacatggaa 60
atgctggagg gtgtcaggaa gtgatcgggc tctggggcag ggaggagggg tggggagtgt 120
cactgggagg ggacatcctg cagaaggtag gagtgagcaa acacccgctg caggggaggg 180
gagagccctg cggcacctgg gggagcagag ggagcagcac ctgcccaggc ctgggaggag 240
gggcctggag ggcgtgagga ggagcgaggg ggctgcatgg ctggagtgag ggatcagggg 300
cagggcgcga gatggcctca cacagggaag agagggcccc tcctgcaggg cctcacctgg 360
gccacaggag gacactgctt ttcctctgag gagtcaggag ctgtggatgg tgctggacag 420
aagaaggaca gggcctggct caggtgtcca gaggctgtcg ctggcttccc tttgggatca 480
gactgcaggg agggagggcg gcagggttgt ggggggagtg acgatgagga tgacctgggg 540
gtggctccag gccttgcccc tgcctgggcc ctcacccagc ctccctcaca gtctcctggc 600
cctcagtctc tcccctccac tccatcctcc atctggcctc agtgggtcat tctgatcact 660
gaactgacca tacccagccc tgcccacggc cctccatggc tccccaatgc cctggagagg 720
ggacatctag tcagagagta gtcctgaaga ggtggcctct gcgatgtgcc tgtgggggca 780
gcatcctgca gatggtcccg gccctcatcc tgctgacctg tctgcaggga ctgtcctcct 840
ggaccttgcc ccttgtgcag gagctggacc ctgaagtccc ctccccatag gccaagactg 900
gagccttgtt ccctctgttg gactccctgc ccatattctt gtgggagtgg gttctggaga 960
catttctgtc tgttcctgag agctgggaat tgctctcagt catctgcctg cgcggttctg 1020
agagatggag ttgcctaggc agttattggg gccaatcttt ctcactgtgt ctctcctcct 1080
ttacccttag ggtgattctg ggggtccact tgtctgtaat ggtgtgcttc aaggtatcac 1140
atcatggggc cctgagccat gtgccctgcc tgaaaagcct gctgtgtaca ccaaggtggt 1200
gcattaccgg aagtggatca aggacaccat cgcagccaac ccctgagtgc ccctgtccca 1260
cccctacctc tagtaaattt aagtccacct cacgttctgg catcacttgg cctttctgga 1320
tgctggacac ctgaagcttg gaactcacct ggccgaagct cgagcctcct gagtcctact 1380
gacctgtgct ttctggtgtg gagtccaggg ctgctaggaa aaggaatggg cagacacagg 1440
tgtatgccaa tgtttctgaa atgggtataa tttcgtcctc tccttcggaa cactggctgt 1500
ctctgaagac ttctcgctca gtttcagtga ggacacacac aaagacgtgg gtgaccatgt 1560
tgtttgtggg gtgcagagat gggaggggtg gggcccaccc tggaagagtg gacagtgaca 1620
caaggtggac actctctaca gatcactgag gataagctgg agccacaatg catgaggcac 1680
acacacagca aggttgacgc tgtaaacata gcccacgctg tcctgggggc actgggaagc 1740
ctagataagg ccgtgagcag aaagaagggg aggatcctcc tatgttgttg aaggagggac 1800
tagggggaga aactgaaagc tgattaatta caggaggttt gttcaggtcc cccaaaccac 1860
cgtcagattt gatgatttcc tagcaggact tacagaaata aagagctatc atgctgtggt 1920
ttattatggt ttgttacatt gataggatac atactgaaat cagcaaacaa aacagatgta 1980
tagattagag tgtggagaaa acagaggaaa acttgcagtt acgaagactg gcaacttggc 2040
tttactaagt tttcagactg gcaggaagtc aaacctatta ggctgaggac cttgtggagt 2100
gtagctgatc cagctgatag aggaactagc caggtggggg cctttccctt tggatggggg 2160

gcatatccga cagttattct ctccaagtgg agacttacgg acagcatata attctccctg 2220
caaggatgta tgataatatg tacaaagtaa ttccaactga ggaagctcac ctgatcctta 2280
gtgtccaggg tttttactgg gggtctgtag gacgagtatg gagtacttga ataattgacc 2340
tgaagtcctc agacctgagg ttccctagag ttcaaacaga tacagcatgg tccagagtcc 2400
cagatgtaca aaaacaggga ttcatcacaa atcccatctt tagcatgaag ggtctggcat 2460
ggcccaaggc cccaagtata tcaaggcact tgggcagaac atgccaagga atcaaatgtc 2520
atctcccagg agttattcaa gggtgagccc tttacttggg atgtacaggc tttgagcagt 2580
gcagggctgc tgagtcaacc ttttattgta caggggatga gggaaaggga gaggatgagg 2640
aagcccccct ggggatttgg tttggtcttg tgatcaggtg gtctatgggg ctatccctac 2700
aaagaagaat ccagaaatag gggcacattg aggaatgata ctgagcccaa agagcattca 2760
atcattgttt tatttgcctt cttttcacac cattggtgag ggagggatta ccaccctggg 2820
gttatgaaga tggttgaaca ccccacacat agcaccggag atatgagatc aacagtttct 2880
tagccataga gattcacagc ccagagcagg aggacgctgc acaccatgca ggatgacatg 2940
ggggatgcgc tcgggattgg tgtgaagaag caaggactgt tagaggcagg ctttatagta 3000
acaagacggt ggggcaaact ctgatttccg tgggggaatg tcatggtctt gctttactaa 3060
gttttgagac tggcaggtag tgaaactcat taggctgaga accttgtgga atgcagctga 3120
cccagctgat agaggaagta gccaggtggg agcctttccc agtgggtgtg ggacatatct 3180
ggcaagattt tgtggcactc ctggttacag atactggggc agcaaataaa actgaatctt 3240
gttttcagac cttaaaaaaa aaaaaaaaaa aaaagtttt 3279

<210> 383
<211> 154
<212> PRT
<213> Homo sapiens

<400> 383

<210> 384
<211> 557
<212> DNA
<213> Homo sapiens

<400> 384
   ggatcctcta gagcggccgc ctactactac taaattcgcg gccgcgtcga cgaagaagag 60
   aaagatgtgt tttgttttgg actctctgtg gtcccttcca atgctgtggg tttccaacca 120
   ggggaagggt cccttttgca ttgccaagtg ccataaccat gagcactact ctaccatggt 180
   tctgcctcct ggccaagcag gctggtttgc aagaatgaaa tgaatgattc tacagctagg 240
   acttaacctt gaaatggaaa gtcttgcaat cccatttgca ggatccgtct gtgcacatgc 300
   ctctgtagag agcagcattc ccagggacct tggaaacagt tggcactgta aggtgcttgc 360
   tccccaagac acatcctaaa aggtgttgta atggtgaaaa cgtcttcctt ctttattgcc 420
   ccttcttatt tatgtgaaca actgtttgtc tttttttgta tcttttttaa actgtaaagt 480
   tcaattgtga aaatgaatat catgcaaata aattatgcga tttttttttc aaagtaaaaa 540
   aaaaaaaaaa aaaaaaa 557

<210> 385
<211> 337
<212> DNA

<213> Homo sapiens

<400> 385
ttcccaggtg atgtgcgagg gaagacacat ttactatcct tgatggggct gattccttta 60
gtttctctag cagcagatgg gttaggagga agtgacccaa gtggttgact cctatgtgca 120
tctcaaagcc atctgctgtc ttcgagtacg gacacatcat cactcctgca ttgttgatca 180
aaacgtggag gtgcttttcc tcagctaaga agcccttagc aaaagctcga atagacttag 240
tatcagacag gtccagtttc cgcaccaaca cctgctggtt ccctgtcgtg gtctggatct 300
ctttggccac caattccccc ttttccacat cccggca 337

<210> 386
<211> 300
<212> DNA
<213> Homo sapiens

<400> 386
gggcccgcta ccggcccagg ccccgcctcg cgagtcctcc tccccgggtg cctgcccgca 60
gcccgctcgg cccagagggt gggcgcgggg ctgcctctac cggctggcgg ctgtaactca 120
gcgaccttgg cccgaaggct ctagcaagga cccaccgacc ccagccgcgg cggcggcggc 180
gcggactttg cccggtgtgt ggggcggagc ggactgcgtg tccgcggacg ggcagcgaag 240
atgttagcct tcgctgccag gaccgtggac cgatcccagg gctgtggtgt aacctcagcc 300

<210> 387
<211> 537
<212> DNA
<213> Homo sapiens

<400> 387
gggccgagtc gggcaccaag ggactctttg caggcttcct tcctcggatc atcaaggctg 60
ccccctcctg tgccatcatg atcagcacct atgagttcgg caaaagcttc ttccagaggc 120
tgaaccagga ccggcttctg ggcggctgaa aggggcaagg aggcaaggac cccgtctctc 180
ccacggatgg ggagagggca ggaggagacc cagccaagtg ccttttcctc agcactgagg 240
gagggggctt gtttcccttc cctcccggcg acaagctcca gggcagggct gtccctctgg 300
gcggcccagc acttcctcag acacaacttc ttcctgctgc tccagtcgtg gggatcatca 360
cttacccacc ccccaagttc aagaccaaat cttccagctg cccccttcgt gtttccctgt 420
gtttgctgta gctgggcatg tctccaggaa ccaagaagcc ctcagcctgg tgtagtctcc 480
ctgacccttg ttaattcctt aagtctaaag atgatgaact tcaaaaaaaa aaaaaaa 537

<210> 388
<211> 520
<212> DNA
<213> Homo sapiens

<400> 388
aggataattt ttaaaccaat caaatgaaaa aaacaaacaa acaaaaaagg aaatgtcatg 60
tgaggttaaa ccagtttgca ttcccctaat gtggaaaaag taagaggact actcagcact 120
gtttgaagat tgcctcttct acagcttctg agaattgtgt tatttcactt gccaagtgaa 180
ggaccccctc cccaacatgc cccagcccac ccctaagcat ggtcccttgt caccaggcaa 240
ccaggaaact gctacttgtg gacctcacca gagaccagga gggtttggtt agctcacagg 300
acttccccca ccccagaaga ttagcatccc atactagact catactcaac tcaactaggc 360
tcatactcaa ttgatggtta ttagacaatt ccatttcttt ctggttatta taaacagaaa 420
atctttcctc ttctcattac cagtaaaggc tcttggtatc tttctgttgg aatgatttct 480
atgaacttgt cttattttaa tggtgggttt tttttctggt 520

<210> 389
<211> 365
<212> DNA
<213> Homo sapiens

<400> 389
cgttgcccca gtttgacaga aggaaaggcg gagcttattc aaagtctaga gggagtggag 60
gagttaaggc tggatttcag atctgcctgg ttccagccgc agtgtgccct ctgctccccc 120
aacgactttc caaataatct caccagcgcc ttccagctca ggcgtcctag aagcgtcttg 180
aagcctatgg ccagctgtct ttgtgttccc tctcacccgc ctgtcctcac agctgagact 240
cccaggaaac cttcagacta ccttcctctg ccttcagcaa ggggcgttgc ccacattctc 300
tgagggtcag tggaagaacc tagactccca ttgctagagg tagaaagggg aagggtgctg 360
gggag 365

<210> 390
<211> 221
<212> DNA
<213> Homo sapiens

<220>
<221> misc_feature
<222> 68, 127, 136, 138, 141, 153, 155, 161
<223> n = A,T,C or G

<400> 390
tgcctctcca tcctggcccc gacttctctg tcaggaaagt ggggatggac cccatctgca 60
tacacggntt ctcatgggtg tggaacatct ctgcttgcgg tttcaggaag gcctctggct 120
gctctangag tctgancnga ntcgttgccc cantntgaca naaggaaagg cggagcttat 180
tcaaagtcta gagggagtgg aggagttaag gctggatttc a 221

<210> 391
<211> 325
<212> DNA
<213> Homo sapiens

<220>
<221> misc_feature
<222> 47, 55, 108, 152, 153, 166, 175, 181, 184, 187, 191, 196,
210, 231
<223> n = A,T,C or G

<400> 391
tggagcaggt cccgaggcct ccctagagcc tggggccgac tctgtgncga tgcangcttt 60
ctctcgcgcc cagcctggag ctgctcctgg catctaccaa caatcagncg aggcgagcag 120
tagccagggc actgctgcca acagccagtc cnnataccat catgtnaccc ggtgngctct 180
naanttngat ntccanagcc ctacccatcn tagttctgct ctcccaccgg ntaccagccc 240
cactgcccag gaatcctaca gccagtaccc tgtcccgacg tctctaccta ccagtacgat 300
gagacctccg gctactacta tgacc 325

<210> 392
<211> 277
<212> DNA
<213> Homo sapiens

<220>
<221> misc_feature
<222> 45, 71, 75, 78, 80, 109, 110, 116, 118, 122, 128, 135, 140,
146, 150, 151, 160, 167, 168
<223> n = A,T,C or G

<400> 392
atattgttta actccttcct ttatatcttt taacattttc atggngaaag gttcacatct 60
agtctcactt nggcnagngn ctcctacttg agtctcttcc ccggcctgnn ccagtngnaa 120
antaccanga accgncatgn cttaanaacn ncctggtttn tgggttnntc aatgactgca 180
tgcagtgcac caccctgtcc actacgtgat gctgtaggat taaagtctca cagtgggcgg 240
ctgaggatac agcgccgcgt cctgtgttgc tggggaa 277

<210> 393
<211> 566
<212> DNA
<213> Homo sapiens

<400> 393
actagtccag tgtggtggaa ttcgcggccg cgtcgacgga caggtcagct gtctggctca 60
gtgatctaca ttctgaagtt gtctgaaaat gtcttcatga ttaaattcag cctaaacgtt 120
ttgccgggaa cactgcagag acaatgctgt gagtttccaa ccttagccca tctgcgggca 180
gagaaggtct agtttgtcca tcagcattat catgatatca ggactggtta cttggttaag 240
gaggggtcta ggagatctgt cccttttaga gacaccttac ttataatgaa gtatttggga 300
gggtggtttt caaaagtaga aatgtcctgt attccgatga tcatcctgta aacattttat 360
catttattaa tcatccctgc ctgtgtctat tattatattc atatctctac gctggaaact 420
ttctgcctca atgtttactg tgcctttgtt tttgctagtt tgtgttgttg aaaaaaaaaa 480

cattctctgc ctgagtttta atttttgtcc aaagttattt taatctatac aattaaaagc 540
ttttgcctat caaaaaaaaa aaaaaa 566

<210> 394
<211> 384
<212> DNA
<213> Homo sapiens

<220>
<221> misc_feature
<222> 69, 119
<223> n = A,T,C or G

<400> 394
gaacatacat gtcccggcac ctgagctgca gtctgacatc atcgccatca cgggcctcgc 60
tgcaaattng gaccgggcca aggctggact gctggagcgt gtgaaggagc tacaggccna 120
gcaggaggac cgggctttaa ggagttttaa gctgagtgtc actgtagacc ccaaatacca 180
tcccaagatt atcgggagaa agggggcagt aattacccaa atccggttgg agcatgacgt 240
gaacatccag tttcctgata aggacgatgg gaaccagccc caggaccaaa ttaccatcac 300
agggtacgaa aagaacacag aagctgccag ggatgctata ctgagaattg tgggtgaact 360
tgagcagatg gtttctgagg acgt 384

<210> 395
<211> 399
<212> DNA
<213> Homo sapiens

<400> 395
ggcaaaactg tgtgacctca ataagacctc gcagatccaa ggtcaagtat cagaagtgac 60
tctgaccttg gactccaaga cctacatcaa cagcctggct atattagatg atgagccagt 120
tatcagaggt ttcatcattg cggaaattgt ggagtctaag gaaatcatgg cctctgaagt 180
attcacgtct ttccagtacc ctgagttctc tatagagttg cctaacacag gcagaattgg 240
ccagctactt gtctgcaatt gtatcttcaa gaataccctg gccatccctt tgactgacgt 300
caagttctct ttggaaagcc tgggcatctc ctcactacag acctctgacc atgggacggt 360
gcagcctggt gagaccatcc aatcccaaat aaaatgcac 399

<210> 396
<211> 403
<212> DNA
<213> Homo sapiens

<220>
<221> misc_feature
<222> 8
<223> n = A,T,C or G

<400> 396
tggagttntc agtgcaaaca agccataaag cttcagtagc aaattactgt ctcacagaaa 60
gacattttca acttctgctc cagctgctga taaaacaaat catgtgttta gcttgactcc 120
agacaaggac aacctgttcc ttcataactc tctagagaaa aaaaggagtt gttagtagat 180
actaaaaaaa gtggatgaat aatctggata tttttcctaa aaagattcct tgaaacacat 240
taggaaaatg gagggcctta tgatcagaat gctagaatta gtccattgtg ctgaagcagg 300
gtttagggga gggagtgagg gataaaagaa ggaaaaaaag aagagtgaga aaacctattt 360
atcaaagcag gtgctatcac tcaatgttag gccctgctct ttt 403

<210> 397
<211> 100
<212> DNA
<213> Homo sapiens

<220>
<221> misc_feature
<222> 7, 41, 44, 83
<223> n = A,T,C or G

<400> 397
actagtncag tgtggtggaa ttcgcggccg cgtcgaccta naanccatct ctatagcaaa 60
tccatccccg ctcctggttg gtnacagaat gactgacaaa 100

<210> 398
<211> 278
<212> DNA
<213> Homo sapiens

<220>
<221> misc_feature
<222> 258
<223> n = A,T,C or G

<400> 398
gcggccgcgt cgacagcagt tccgccagcg ctcgcccctg ggtggggatg tgctgcacgc 60
ccacctggac atctggaagt cagcggcctg gatgaaagag cggacttcac ctggggcgat 120
tcactactgt gcctcgacca gtgaggagag ctggaccgac agcgaggtgg actcatcatg 180
ctccgggcag cccatccacc tgtggcagtt cctcaaggag ttgctactca agccccacag 240
ctatggccgc ttcattangt ggctcaacaa ggagaagg 278

<210> 399
<211> 298
<212> DNA
<213> Homo sapiens

<220>
<221> misc_feature
<222> 19, 32, 46, 55, 60, 69, 229, 236, 280
<223> n = A,T,C or G

<400> 399
acggaggtgg aggaagcgnc cctgggatcg anaggatggg tcctgncatt gaccncctcn 60
ggggtgccng catggagcgc atgggcgcgg gcctgggcca cggcatggat cgcgtgggct 120
ccgagatcga gcgcatgggc ctggtcatgg accgcatggg ctccgtggag cgcatgggct 180
ccggcattga gcgcatgggc ccgctgggcc tcgaccacat ggcctccanc attgancgca 240
tgggccagac catggagcgc attggctctg gcgtggagcn catgggtgcc ggcatggg 298

<210> 400
<211> 548
<212> DNA
<213> Homo sapiens

<400> 400
acatcaacta cttcctcatt ttaaggtatg gcagttccct tcatcccctt ttcctgcctt 60
gtacatgtac atgtatgaaa tttccttctc ttaccgaact ctctccacac atcacaaggt 120
caaagaacca cacgcttaga agggtaagag ggcaccctat gaaatgaaat ggtgatttct 180
tgagtctctt ttttccacgt ttaaggggcc atggcaggac ttagagttgc gagttaagac 240
tgcagagggc tagagaatta tttcatacag gctttgaggc cacccatgtc acttatcccg 300
tataccctct caccatcccc ttgtctactc tgatgccccc aagatgcaac tgggcagcta 360
gttggcccca taattctggg cctttgttgt ttgttttaat tacttgggca tcccaggaag 420
ctttccagtg atctcctacc atgggccccc ctcctgggat caagcccctc ccaggccctg 480
tccccagccc ctcctgcccc agcccacccg cttgccttgg tgctcagccc tcccattggg 540
agcaggtt 548

<210> 401
<211> 355
<212> DNA
<213> Homo sapiens

<220>
<221> misc_feature
<222> 230, 277, 350
<223> n = A,T,C or G

<400> 401
actgtttcca tgttatgttt ctacacattg ctacctcagt gctcctggaa acttagcttt 60
tgatgtctcc aagtagtcca ccttcattta actctttgaa actgtatcat ctttgccaag 120
taagagtggt ggcctatttc agctgctttg acaaaatgac tggctcctga cttaacgttc 180
tataaatgaa tgtgctgaag caaagtgccc atggtggcgg cgaagaagan aaagatgtgt 240
tttgttttgg actctctgtg gtcccttcca atgctgnggg tttccaacca ggggaagggt 300

cccttttgca ttgccaagtg ccataaccat gagcactact ctaccatggn tctgc 355

<210> 402
<211> 407
<212> DNA
<213> Homo sapiens

<220>
<221> misc_feature
<222> 362
<223> n = A,T,C or G

<400> 402
atggggcaag ctggataaag aaccaagacc cactggagta tgctgtcttc aagaaaccca 60
tctcacatgc ggtggcatac ataggctcaa aataaaggaa tggagaaaaa tatttcaagc 120
aaatggaaaa cagaaaaaag caggtgttgc actcctactt tctgacaaaa cagactatgc 180
gaataaagat aaaaaagaga aggacattac aaaggtggtc ctgacctttg ataaatctca 240
ttgcttgata ccaacctggg ctgttttaat tgcccaaacc aaaaggataa tttgctgagg 300
ttgtggagct tctcccctgc agagagtccc tgatctccca aaatttggtt gagatgtaag 360
gntgattttg ctgacaactc cttttctgaa gttttactca tttccaa 407

<210> 403
<211> 303
<212> DNA
<213> Homo sapiens

<220>
<221> misc_feature
<222> 15
<223> n = A,T,C or G

<400> 403
cagtatttat agccnaactg aaaagctagt agcaggcaag tctcaaatcc aggcaccaaa 60
tcctaagcaa gagccatggc atggtgaaaa tgcaaaagga gagtctggcc aatctacaaa 120
tagagaacaa gacctactca gtcatgaaca aaaaggcaga caccaacatg gatctcatgg 180
gggattggat attgtaatta tagagcagga agatgacagt gatcgtcatt tggcacaaca 240
tcttaacaac gaccgaaacc cattatttac ataaacctcc attcggtaac catgttgaaa 300
gga 303

<210> 404
<211> 225
<212> DNA
<213> Homo sapiens

<400> 404
aagtgtaact tttaaaaatt tagtggattt tgaaaattct tagaggaaag taaaggaaaa 60
attgttaatg cactcattta cctttacatg gtgaaagttc tctcttgatc ctacaaacag 120
acattttcca ctcgtgtttc catagttgtt aagtgtatca gatgtgttgg gcatgtgaat 180
ctccaagtgc ctgtgtaata aataaagtat ctttatttca ttcat 225

<210> 405
<211> 334
<212> DNA
<213> Homo sapiens

<220>
<221> misc_feature
<222> 318, 319
<223> n = A,T,C or G

<400> 405
gagctgttat actgtgagtt ctactaggaa atcatcaaat ctgagggttg tctggaggac 60
ttcaatacac ctccccccat agtgaatcag cttccagggg gtccagtccc tctccttact 120
tcatccccat cccatgccaa aggaagaccc tccctccttg gctcacagcc ttctctaggc 180
ttcccagtgc ctccaggaca gagtgggtta tgttttcagc tccatccttg ctgtgagtgt 240
ctggtgcggt tgtgcctcca gcttctgctc agtgcttcat ggacagtgtc cagcccatgt 300
cactctccac tctctcanng tggatcccac ccct 334

<210> 406
<211> 216
<212> DNA
<213> Homo sapiens

<220>
<221> misc_feature
<222> 25, 28, 33, 34, 59, 123, 136, 194, 215
<223> n = A,T,C or G

<400> 406
tttcatacct aatgagggag ttganatnac atnnaaccag gaaatgcatg gatctcaang 60
gaaacaaaca cccaataaac tcggagtggc agactgacaa ctgtgagaca tgcacttgct 120
acnaaacaca aatttnatgt tgcacccttg tttctacacc tgtgggttat gacaaagaca 180
actgccaaag aatnttcaag aaggaggact gccant 216

<210> 407
<211> 413
<212> DNA
<213> Homo sapiens

<400> 407
gctgacttgc tagtatcatc tgcattcatt gaagcacaag aacttcatgc cttgactcat 60
gtaaatgcaa taggattaaa aaataaattt gatatcacat ggaaacagac aaaaaatatt 120
gtacaacatt gcacccagtg tcagattcta cacctggcca ctcaggaagc aagagttaat 180
cccagaggtc tatgtcctaa tgtgttatgg caaatggatg tcatgcacgt accttcattt 240
ggaaaattgt catttgtcca tgtgacagtt gatacttatt cacatttcat atgggcaacc 300
tgccagacag gagaaagtct tcccatgtta aaagacattt attatcttgt tttcctgtca 360
tgggagttcc agaaaaagtt aaaacagaca atgggccagg ttctgtagta aag 413

<210> 408
<211> 183
<212> DNA
<213> Homo sapiens

<220>
<221> misc_feature
<222> 7, 25, 35, 54, 55, 62, 90, 91, 140, 147, 181
<223> n = A,T,C or G

<400> 408
ggagctngcc ctcaattcct ccatntctat gttancatat ttaatgtctt ttgnnattaa 60
tncttaacta gttaatcctt aaagggctan ntaatcctta actagtccct ccattgtgag 120
cattatcctt ccagtattcn ccttctnttt tatttactcc ttcctggcta cccatgtact 180
ntt 183

<210> 409
<211> 250
<212> DNA
<213> Homo sapiens

<220>
<221> misc_feature
<222> 225, 245
<223> n = A,T,C or G

<400> 409
cccacgcatg ataagctctt tatttctgta agtcctgcta ggaaatcatc aaatctgacg 60
gtggtttggg ggacctgaac aaacctcctg taattaatca gctttcagtt tctcccccta 120
gtccctcctt caacaacata ggaggatcct ccccttcttt ctgctcacgg ccttatctag 180
gcttcccagt gcccccagga cagcgtgggc tatgtttaca gcgcntcctt gctggggggg 240
ggccntatgc 250

<210> 410
<211> 306
<212> DNA
<213> Homo sapiens

<220>
<221> misc_feature
<222> 241, 260, 289, 303
<223> n = A,T,C or G

<400> 410
ggctggtttg caagaatgaa atgaatgatt ctacagctag gacttaacct tgaaatggaa 60
agtcttgcaa tcccatttgc aggatccgtc tgtgcacatg cctctgtaga gagcagcatt 120
cccagggacc ttggaaacag ttggcactgt aaggtgcttg ctccccaaga cacatcctaa 180
aaggtgttgt aatggtgaaa accgcttcct tctttattgc cccttcttat ttatgtgaac 240
nactggttgg ctttttttgn atctttttta aactggaaag ttcaattgng aaaatgaata 300
tcntgc 306

<210> 411
<211> 261
<212> DNA
<213> Homo sapiens

<220>
<221> misc_feature
<222> 10, 18, 253
<223> n = A,T,C or G

<400> 411
agagatattn cttaggtnaa agttcataga gttcccatga actatatgac tggccacaca 60
ggatcttttg tatttaagga ttctgagatt ttgcttgagc aggattagat aaggctgttc 120
tttaaatgtc tgaaatggaa cagatttcaa aaaaaaaccc cacaatctag ggtgggaaca 180
aggaaggaaa gatgtgaata ggctgatggg caaaaaacca atttacccat cagttccagc 240
cttctctcaa ggngaggcaa a 261

<210> 412
<211> 241
<212> DNA
<213> Homo sapiens

<220>
<221> misc_feature
<222> 223
<223> n = A,T,C or G

<400> 412
gttcaatgtt acctgacatt tctacaacac cccactcacc gatgtattcg ttgcccagtg 60
ggaacatacc agcctgaatt tggaaaaaat aattgtgttt cttgcccagg aaatactacg 120
actgactttg atggctccac aaacataacc cagtgtaaaa acagaagatg tggaggggag 180
ctgggagatt tcactgggta cattgaattc ccaaactacc cangcaatta cccagccaac 240
a 241

<210> 413
<211> 231
<212> DNA
<213> Homo sapiens

<220>
<221> misc_feature
<222> 196, 220
<223> n = A,T,C or G

<400> 413
aactcttaca atccaagtga ctcatctgtg tgcttgaatc ctttccactg tctcatctcc 60
ctcatccaag tttctagtac cttctctttg ttgtgaagga taatcaaact gaacaacaaa 120
aagtttactc tcctcatttg gaacctaaaa actctcttct tcctgggtct gagggctcca 180
agaatccttg aatcanttct cagatcattg gggacaccan atcaggaacc t 231

<210> 414
<211> 234
<212> DNA
<213> Homo sapiens

<400> 414
actgtccatg aagcactgag cagaagctgg aggcacaacg caccagacac tcacagcaag 60
gatggagctg aaaacataac ccactctgtc ctggaggcac tgggaagcct agagaaggct 120
gtgagccaag gagggagggt cttcctttgg catgggatgg ggatgaagta aggagaggga 180
ctggaccccc tggaagctga ttcactatgg ggggaggtgt attgaagtcc tcca 234

<210> 415
<211> 217
<212> DNA
<213> Homo sapiens

<220>
<221> misc_feature
<222> 101, 182
<223> n = A,T,C or G

<400> 415
gcataggatt aagactgagt atcttttcta cattctttta actttctaag gggcacttct 60
caaaacacag accaggtagc aaatctccac tgctctaagg ntctcaccac cactttctca 120
cacctagcaa tagtagaatt cagtcctact tctgaggcca gaagaatggt tcagaaaaat 180
antggattat aaaaaataac aattaagaaa aataatc 217

<210> 416
<211> 213
<212> DNA
<213> Homo sapiens

<220>
<221> misc_feature
<222> 9, 17, 45
<223> n = A,T,C or G

<400> 416
atgcatatnt aaagganact gcctcgcttt tagaagacat ctggnctgct ctctgcatga 60
ggcacagcag taaagctctt tgattcccag aatcaagaac tctccccttc agactattac 120
cgaatgcaag gtggttaatt gaaggccact aattgatgct caaatagaag gatattgact 180
atattggaac agatggagtc tctactacaa aag 213

<210> 417
<211> 303
<212> DNA
<213> Homo sapiens

<220>
<221> misc_feature
<222> 1, 244, 271, 277, 288, 290
<223> n = A,T,C or G

<400> 417
nagtcttcag gcccatcagg gaagttcaca ctggagagaa gtcatacata tgtactgtat 60
gtgggaaagg ctttactctg agttcaaatc ttcaagccca tcagagagtc cacactggag 120
agaagccata caaatgcaat gagtgtggga agagcttcag gagggattcc cattatcaag 180
ttcatctagt ggtccacaca ggagagaaac cctataaatg tgagatatgt gggaagggct 240
tcantcaaag ttcgtatctt caaatccatc ngaaggncca cagtatanan aaacctttta 300
agt 303

<210> 418
<211> 328
<212> DNA
<213> Homo sapiens

<220>
<221> misc_feature
<222> 32, 237, 245, 310
<223> n = A,T,C or G

<400> 418
tttttggcgg tggtggggca gggacgggac angagtctca ctctgttgcc caggctggag 60

tgcacaggca tgatctcggc tcactacaac ccctgcctcc catgtccaag cgattcttgt 120
gcctcagcct tccctgtagc tagaattaca ggcacatgcc accacaccca gctagttttt 180
gtatttttag tagagacagg gtttcaccat gttggccagg ctggtctcaa actcctnacc 240
tcagnggtca ggctggtctc aaactcctga cctcaagtga tctgcccacc tcagcctccc 300
aaagtgctan gattacaggc cgtgagcc 328

<210> 419
<211> 389
<212> DNA
<213> Homo sapiens

<220>
<221> misc_feature
<222> 373
<223> n = A,T,C or G

<400> 419
cctcctcaag acggcctgtg gtccgcctcc cggcaaccaa gaagcctgca gtgccatatg 60
acccctgagc catggactgg agcctgaaag gcagcgtaca ccctgctcct gatcttgctg 120
cttgtttcct ctctgtggct ccattcatag cacagttgtt gcactgaggc ttgtgcaggc 180
cgagcaaggc caagctggct caaagagcaa ccagtcaact ctgccacggt gtgccaggca 240
ccggttctcc agccaccaac ctcactcgct cccgcaaatg gcacatcagt tcttctaccc 300
taaaggtagg accaaagggc atctgctttt ctgaagtcct ctgctctatc agccatcacg 360
tggcagccac tcnggctgtg tcgacgcgg 389

<210> 420
<211> 408
<212> DNA
<213> Homo sapiens

<400> 420
gttcctccta actcctgcca gaaacagctc tcctcaacat gagagctgca cccctcctcc 60
tggccagggc agcaagcctt agccttggct tcttgtttct gctttttttc tggctagacc 120
gaagtgtact agccaaggag ttgaagtttg tgactttggt gtttcggcat ggagaccgaa 180
gtcccattga cacctttccc actgacccca taaaggaatc ctcatggcca caaggatttg 240
gccaactcac ccagctgggc atggagcagc attatgaact tggagagtat ataagaaaga 300
gatatagaaa attcttgaat gagtcctata aacatgaaca ggtttatatt cgaagcacag 360
acgttgaccg gactttgatg aagtgctatg acaaacctgg caagcccg 408

<210> 421
<211> 352
<212> DNA
<213> Homo sapiens

<220>
<221> misc_feature
<222> 23, 101, 166, 294
<223> n = A,T,C or G

<400> 421
gctcaaaaat ctttttactg atnggcatgg ctacacaatc attgactatt acggaggcca 60
gaggagaatg aggcctggcc tgggagccct gtgcctacta naagcacatt agattatcca 120
ttcactgaca gaacaggtct tttttgggtc cttcttctcc accacnatat acttgcagtc 180
ctccttcttg aagattcttt ggcagttgtc tttgtcataa cccacaggtg tagaaacaag 240
ggtgcaacat gaaatttctg tttcgtagca agtgcatgtc tcacaagttg gcangtctgc 300
cactccgagt ttattgggtg tttgtttcct ttgagatcca tgcatttcct gg 352

<210> 422
<211> 337
<212> DNA
<213> Homo sapiens

<400> 422
atgccaccat gctggcaatg cagcgggcgg tcgaaggcct gcatatccag cccaagctgg 60
cgatgatcga cggcaaccgt tgcccgaagt tgccgatgcc agccgaagcg gtggtcaagg 120
gcgatagcaa ggtgccggcg atcgcggcgg cgtcaatcct ggccaaggtc agccgtgatc 180
gtgaaatggc agctgtcgaa ttgatctacc cgggttatgg catcggcggg cataagggct 240
atccgacacc ggtgcacctg gaagccttgc agcggctggg gccgacgccg attcaccgac 300

gcttcttccg ccggtacggc tggcctatga aaattat 337

<210> 423
<211> 310
<212> DNA
<213> Homo sapiens

<220>
<221> misc_feature
<222> 100, 105, 232
<223> n = A,T,C or G

<400> 423
gctcaaaaat ctttttactg atatggcatg gctacacaat cattgactat tagaggccag 60
aggagaatga ggcctggcct gggagccctg tgcctactan aagcncatta gattatccat 120
tcactgacag aacaggtctt ttttgggtcc ttcttctcca ccacgatata cttgcagtcc 180
tccttcttga agattctttg gcagttgtct ttgtcataac ccacaggtgt anaaacaagg 240
gtgcaacatg aaatttctgt ttcgtagcaa gtgcatgtct cacagttgtc aagtctgccc 300
tccgagttta 310

<210> 424
<211> 370
<212> DNA
<213> Homo sapiens

<220>
<221> misc_feature
<222> 336
<223> n = A,T,C or G

<400> 424
gctcaaaaat ctttttactg ataggcatgg ctacacaatc attgactatt agaggccaga 60
ggagaatgag gcctggcctg ggagccctgt gcctactaga agcacattag attatccatt 120
cactgacaga acaggtcttt tttgggtcct tcttctccac cacgatatac ttgcagtcct 180
ccttcttgaa gattctttgg cagttgtctt tgtcataacc cacaggtgta gaaacatcct 240
ggttgaatct cctggaactc cctcattagg tatgaaatag catgatgcat tgcataaagt 300
cacgaaggtg gcaaagatca caacgctgcc cagganaaca ttcattgtga taagcaggac 360
tccgtcgacg 370

<210> 425
<211> 216
<212> DNA
<213> Homo sapiens

<220>
<221> misc_feature
<222> 10, 11, 52, 68, 80, 84, 86, 98, 104, 114, 122, 135, 154,
185, 187, 205, 208
<223> n = A,T,C or G

<400> 425
aattgctatn ntttattttg ccactcaaaa taattaccaa aaaaaaaaaa tnttaaatga 60
taacaacnca acatcaaggn aaananaaca ggaatggntg actntgcata aatnggccga 120
anattatcca ttatnttaag ggttgacttc aggntacagc acacagacaa acatgcccag 180
gaggntntca ggaccgctcg atgtnttntg aggagg 216

<210> 426
<211> 596
<212> DNA
<213> Homo sapiens

<400> 426
cttccagtga ggataaccct gttgccccgg gccgaggttc tccattaggc tctgattgat 60
tggcagtcag tgatggaagg gtgttctgat cattccgact gccccaaggg tcgctggcca 120
gctctctgtt ttgctgagtt ggcagtagga cctaatttgt taattaagag tagatggtga 180
gctgtccttg tattttgatt aacctaatgg ccttcccagc acgactcgga ttcagctgga 240
gacatcacgg caacttttaa tgaaatgatt tgaagggcca ttaagaggca cttcccgtta 300
ttaggcagtt catctgcact gataacttct tggcagctga gctggtcgga gctgtggccc 360

aaacgcacac ttggcttttg gttttgagat acaactctta atcttttagt catgcttgag 420
ggtggatggc cttttcagct ttaacccaat ttgcactgcc ttggaagtgt agccaggaga 480
atacactcat atactcgtgg gcttagaggc cacagcagat gtcattggtc tactgcctga 540
gtcccgctgg tcccatccca ggaccttcca tcggcgagta cctgggagcc cgtgct 596

<210> 427
<211> 107
<212> DNA
<213> Homo sapiens

<220>
<221> misc_feature
<222> 36, 47, 55, 77, 106
<223> n = A,T,C or G

<400> 427
gaagaattca agttaggttt attcaaaggg cttacngaga atcctanacc caggncccag 60
cccgggagca gccttanaga gctcctgttt gactgcccgg ctcagng 107

<210> 428
<211> 38
<212> DNA
<213> Homo sapiens

<220>
<221> misc_feature
<222> 9, 12, 15
<223> n = A,T,C or G

<400> 428
gaacttccna anaangactt tattcactat tttacatt 38

<210> 429
<211> 544
<212> DNA
<213> Homo sapiens

<400> 429
   ctttgctgga cggaataaaa gtggacgcaa gcatgacctc ctgatgaggg cgctgcattt 60
   attgaagagc ggctgcagcc ctgcggttca gattaaaatc cgagaattgt atagacgccg 120
   atatccacga actcttgaag gactttctga tttatccaca atcaaatcat cggttttcag 180
   tttggatggt ggctcatcac ctgtagaacc tgacttggcc gtggctggaa tccactcgtt 240
   gccttccact tcagttacac ctcactcacc atcctctcct gttggttctg tgctgcttca 300
   agatactaag cccacatttg agatgcagca gccatctccc ccaattcctc ctgtccatcc 360
   tgatgtgcag ttaaaaaatc tgccctttta tgatgtcctt gatgttctca tcaagcccac 420
   gagtttagtt caaagcagta ttcagcgatt tcaagagaag ttttttattt ttgctttgac 480
   acctcaacaa gttagagaga tatgcatatc cagggatttt ttgccaggtg gtaggagaga 540
   ttat 544

<210> 430
<211> 507
<212> DNA
<213> Homo sapiens

<220>
<221> misc_feature
<222> 7, 153, 180, 262, 298
<223> n = A,T,C or G

<400> 430
cttatcncaa tggggctccc aaacttggct gtgcagtgga aactccgggg gaattttgaa 60
gaacactgac acccatcttc caccccgaca ctctgattta attgggctgc agtgagaaca 120
gagcatcaat ttaaaaagct gcccagaatg ttntcctggg cagcgttgtg atctttgccn 180
ccttcgtgac tttatgcaat gcatcatgct atttcatacc taatgaggga gttccaggag 240
attcaaccag gatgtttcta cncctgtggg ttatgacaaa gacaactgcc aaagaatntt 300
caagaaggag gactgcaagt atatcgtggt ggagaagaag gacccaaaaa agacctgttc 360
tgtcagtgaa tggataatct aatgtgcttc tagtaggcac agggctccca ggccaggcct 420
cattctcctc tggcctctaa tagtcaatga ttgtgtagcc atgcctatca gtaaaaagat 480

ttttgagcaa aaaaaaaaa aaaaaaa 507

<210> 431
<211> 392
<212> DNA
<213> Homo sapiens

<220>
<221> misc_feature
<222> 110, 268
<223> n = A,T,C or G

<400> 431
gaaaattcag aatggataaa aacaaatgaa gtacaaaata tttcagattt acatagcgat 60
aaacaagaaa gcacttatca ggaggactta caaatggaag tacactctan aaccatcatc 120
tatcatggct aaatgtgaga ttagcacagc tgtattattt gtacattgca aacacctaga 180
aagagatggg aaacaaaatc ccaggagttt tgtgtgtgga gtcctgggtt ttccaacaga 240
catcattcca gcattctgag attagggnga ttggggatca ttctggagtt ggaatgttca 300
acaaaagtga tgttgttagg taaaatgtac aacttctgga tctatgcaga cattgaaggt 360
gcaatgagtc tggcttttac tctgctgttt ct 392

<210> 432
<211> 387
<212> DNA
<213> Homo sapiens

<220>
<221> misc_feature
<222> 8, 48, 121, 139, 153, 187, 190, 197, 214, 238, 269, 311
<223> n = A,T,C or G

<400> 432
ggtatccnta cataatcaaa tatagctgta gtacatgttt tcattggngt agattaccac 60
aaatgcaagg caacatgtgt agatctcttg tcttattctt ttgtctataa tactgtattg 120
ngtagtccaa gctctcggna gtccagccac tgngaaacat gctcccttta gattaacctc 180
gtggacnctn ttgttgnatt gtctgaactg tagngccctg tattttgctt ctgtctgnga 240
attctgttgc ttctggggca tttccttgng atgcagagga ccaccacaca gatgacagca 300
atctgaattg ntccaatcac agctgcgatt aagacatact gaaatcgtac aggaccggga 360
acaacgtata gaacactgga gtccttt 387

<210> 433
<211> 281
<212> DNA
<213> Homo sapiens

<220>
<221> misc_feature
<222> 12, 17, 30, 126, 200, 204, 216, 221, 229, 242, 243, 251
<223> n = A,T,C or G

<400> 433
ttcaactagc anagaanact gcttcagggn gtgtaaaatg aaaggcttcc acgcagttat 60
ctgattaaag aacactaaga gagggacaag gctagaagcc gcaggatgtc tacactatag 120
caggcnctat ttgggttggc tggaggagct gtggaaaaca tggagagatt ggcgctggag 180
atcgccgtgg ctattcctcn ttgntattac accagngagg ntctctgtnt gcccactggt 240
tnnaaaaccg ntatacaata atgatagaat aggacacaca t 281

<210> 434
<211> 484
<212> DNA
<213> Homo sapiens

<400> 434
ttttaaaata agcatttagt gctcagtccc tactgagtac tctttctctc ccctcctctg 60
aatttaattc tttcaacttg caatttgcaa ggattacaca tttcactgtg atgtatattg 120
tgttgcaaaa aaaaaaaagt gtctttgttt aaaattactt ggtttgtgaa tccatcttgc 180
tttttcccca ttggaactag tcattaaccc atctctgaac tggtagaaaa acatctgaag 240
agctagtcta tcagcatctg acaggtgaat tggatggttc tcagaaccat ttcacccaga 300

cagcctgttt ctatcctgtt taataaatta gtttgggttc tctacatgca taacaaaccc 360
tgctccaatc tgtcacataa aagtctgtga cttgaagttt agtcagcacc cccaccaaac 420
tttatttttc tatgtgtttt ttgcaacata tgagtgtttt gaaaataaag tacccatgtc 480
ttta 484

<210> 435
<211> 424
<212> DNA
<213> Homo sapiens

<400> 435
gcgccgctca gagcaggtca ctttctgcct tccacgtcct ccttcaagga agccccatgt 60
gggtagcttt caatatcgca ggttcttact cctctgcctc tataagctca aacccaccaa 120
cgatcgggca agtaaacccc ctccctcgcc gacttcggaa ctggcgagag ttcagcgcag 180
atgggcctgt ggggaggggg caagatagat gagggggagc ggcatggtgc ggggtgaccc 240
cttggagaga ggaaaaaggc cacaagaggg gctgccaccg ccactaacgg agatggccct 300
ggtagagacc tttgggggtc tggaacctct ggactcccca tgctctaact cccacactct 360
gctatcagaa acttaaactt gaggattttc tctgtttttc actcgcaata aattcagagc 420
aaac 424

<210> 436
<211> 667
<212> DNA
<213> Homo sapiens

<220>
<221> misc_feature
<222> 11
<223> n = A,T,C or G

<400> 436
accttgggaa nactctcaca atataaaggg tcgtagactt tactccaaat tccaaaaagg 60
tcctggccat gtaatcctga aagttttccc aaggtagcta taaaatcctt ataagggtgc 120
agcctcttct ggaattcctc tgatttcaaa gtctcactct caagttcttg aaaacgaggg 180
cagttcctga aaggcaggta tagcaactga tcttcagaaa gaggaactgt gtgcaccggg 240
atgggctgcc agagtaggat aggattccag atgctgacac cttctggggg aaacagggct 300
gccaggtttg tcatagcact catcaaagtc cggtcaacgt ctgtgcttcg aatataaacc 360
tgttcatgtt tataggactc attcaagaat tttctatatc tctttcttat atactctcca 420
agttcataat gctgctccat gcccagctgg gtgagttggc caaatccttg tggccatgag 480
gattccttta tggggtcagt gggaaaggtg tcaatgggac ttcggtctcc atgccgaaac 540
accaaagtca caaacttcaa ctccttggct agtacacttc ggtctagcca gaaaaaaagc 600
agaaacaaga agccaaggct aaggcttgct gccctgccag gaggaggggt gcagctctca 660
tgttgag 667

<210> 437
<211> 693
<212> DNA
<213> Homo sapiens

<400> 437
ctacgtctca accctcattt ttaggtaagg aatcttaagt ccaaagatat taagtgactc 60
acacagccag gtaaggaaag ctggattggc acactaggac tctaccatac cgggttttgt 120
taaagctcag gttaggaggc tgataagctt ggaaggaact tcagacagct ttttcagatc 180
ataaaagata attcttagcc catgttcttc tccagagcag acctgaaatg acagcacagc 240
aggtactcct ctattttcac ccctcttgct tctactctct ggcagtcaga cctgtgggag 300
gccatgggag aaagcagctc tctggatgtt tgtacagatc atggactatt ctctgtggac 360
catttctcca ggttacccta ggtgtcacta ttggggggac agccagcatc tttagctttc 420
atttgagttt ctgtctgtct tcagtagagg aaacttttgc tcttcacact tcacatctga 480
acacctaact gctgttgctc ctgaggtggt gaaagacaga tatagagctt acagtattta 540
tcctatttct aggcactgag ggctgtgggg taccttgtgg tgccaaaaca gatcctgttt 600
taaggacatg ttgcttcaga gatgtctgta actatctggg ggctctgttg gctctttacc 660
ctgcatcatg tgctctcttg gctgaaaatg acc 693

<210> 438
<211> 360
<212> DNA
<213> Homo sapiens

<400> 438
ctgcttatca caatgaatgt tctcctgggc agcgttgtga tctttgccac cttcgtgact 60
ttatgcaatg catcatgcta tttcatacct aatgagggag ttccaggaga ttcaaccagg 120
atgtttctac acctgtgggt tatgacaaag acaactgcca aagaatcttc aagaaggagg 180
actgcaagta tatctggtgg agaagaagga cccaaaaaag acctgttctg tcagtgaatg 240
gataatctaa tgtgcttcta gtaggcacag ggctcccagg ccaggcctca ttctcctctg 300
gcctctaata gtcaataatt gtgtagccat gcctatcagt aaaaagattt ttgagcaaac 360

<210> 439
<211> 431
<212> DNA
<213> Homo sapiens

<220>
<221> misc_feature
<222> 7, 8
<223> n = A,T,C or G

<400> 439
gttcctnnta actcctgcca gaaacagctc tcctcaacat gagagctgca cccctcctcc 60
tggccagggc agcaagcctt agccttggct tcttgtttct gctttttttc tggctagacc 120
gaagtgtact agccaaggag ttgaagtttg tgactttggt gtttcggcat ggagaccgaa 180
gtcccattga cacctttccc actgacccca taaaggaatc ctcatggcca caaggatttg 240
gccaactcac ccagctgggc atggagcagc attatgaact tggagagtat ataagaaaga 300
gatatagaaa attcttgaat gagtcctata aacatgaaca ggtttatatt cgaagcacag 360
acgttgaccg gactttgatg agtgctatga caaacctggc agcccgtcga cgcggccgcg 420
aatttagtag t 431

<210> 440
<211> 523
<212> DNA
<213> Homo sapiens

<400> 440
agagataaag cttaggtcaa agttcataga gttcccatga actatatgac tggccacaca 60
ggatcttttg tatttaagga ttctgagatt ttgcttgagc aggattagat aaggctgttc 120
tttaaatgtc tgaaatggaa cagatttcaa aaaaaaaccc cacaatctag ggtgggaaca 180
aggaaggaaa gatgtgaata ggctgatggg caaaaaacca atttacccat cagttccagc 240
cttctctcaa ggagaggcaa agaaaggaga tacagtggag acatctggaa agttttctcc 300
actggaaaac tgctactatc tgtttttata tttctgttaa aatatatgag gctacagaac 360
taaaaattaa aacctctttg tgtcccttgg tcctggaaca tttatgttcc ttttaaagaa 420
acaaaaatca aactttacag aaagatttga tgtatgtaat acatatagca gctcttgaag 480
tatatatatc atagcaaata agtcatctga tgagaacaag cta 523

<210> 441
<211> 430
<212> DNA
<213> Homo sapiens

<400> 441
gttcctccta actcctgcca gaaacagctc tcctcaacat gagagctgca cccctcctcc 60
tggccagggc agcaagcctt agccttggct tcttgtttct gctttttttc tggctagacc 120
gaagtgtact agccaaggag ttgaagtttg tgactttggt gtttcggcat ggagaccgaa 180
gtcccattga cacctttccc actgacccca taaaggaatc ctcatggcca caaggatttg 240
gccaactcac ccagctgggc atggagcagc attatgaact tggagagtat ataagaaaga 300
gatatagaaa attcttgaat gagtcctata aacatgaaca ggtttatatt cgaagcacag 360
acgttgaccg gactttgatg agtgctatga caaacctggc agcccgtcga cgcggccgcg 420
aatttagtag 430

<210> 442
<211> 362
<212> DNA
<213> Homo sapiens

<400> 442
ctaaggaatt agtagtgttc ccatcacttg tttggagtgt gctattctaa aagattttga 60
tttcctggaa tgacaattat attttaactt tggtggggga aagagttata ggaccacagt 120

cttcacttct gatacttgta aattaatctt ttattgcact tgttttgacc attaagctat 180
atgtttagaa atggtcattt tacggaaaaa ttagaaaaat tctgataata gtgcagaata 240
aatgaattaa tgttttactt aatttatatt gaactgtcaa tgacaaataa aaattctttt 300
tgattatttt ttgttttcat ttaccagaat aaaaactaag aattaaaagt ttgattacag 360
tc 362

<210> 443
<211> 624
<212> DNA
<213> Homo sapiens

<220>
<221> misc_feature
<222> 541
<223> n = A,T,C or G

<400> 443
tttttttttt gcaacacaat atacatcaca gtgaaatgtg taatccttgc aaattgcaag 60
ttgaaagaat taaattcaga ggaggggaga gaaagagtac tcagtaggga ctgagcacta 120
aatgcttatt ttaaaagaaa tgtaaagagc agaaagcaat tcaggctacc ctgccttttg 180
tgctggctag tactccggtc ggtgtcagca gcacgtggca ttgaacattg caatgtggag 240
cccaaaccac agaaaatggg gtgaaattgg ccaactttct attaacttgg cttcctgttt 300
tataaaatat tgtgaataat atcacctact tcaaagggca gttatgaggc ttaaatgaac 360
taacgcctac aaaacactta aacatagata acataggtgc aagtactatg tatctggtac 420
atggtaaaca tccttattat taaagtcaac gctaaaatga atgtgtgtgc atatgctaat 480
agtacagaga gagggcactt aaaccaacta agggcctgga gggaaggttt cctggaaaga 540
ngatgcttgt gctgggtcca aatcttggtc tactatgacc ttggccaaat tatttaaact 600
ttgtccctat ctgctaaaca gatc 624

<210> 444
<211> 425
<212> DNA
<213> Homo sapiens

<220>
<221> misc_feature
<222> 11, 12
<223> n = A,T,C or G

<400> 444
gcacatcatt nntcttgcat tctttgagaa taagaagatc agtaaatagt tcagaagtgg 60
gaagctttgt ccaggcctgt gtgtgaaccc aatgttttgc ttagaaatag aacaagtaag 120
ttcattgcta tagcataaca caaaatttgc ataagtggtg gtcagcaaat ccttgaatgc 180
tgcttaatgt gagaggttgg taaaatcctt tgtgcaacac tctaactccc tgaatgtttt 240
gctgtgctgg gacctgtgca tgccagacaa ggccaagctg gctgaaagag caaccagcca 300
cctctgcaat ctgccacctc ctgctggcag gatttgtttt tgcatcctgt gaagagccaa 360
ggaggcacca gggcataagt gagtagactt atggtcgacg cggccgcgaa tttagtagta 420
gtaga 425

<210> 445
<211> 414
<212> DNA
<213> Homo sapiens

<220>
<221> misc_feature
<222> 11
<223> n = A,T,C or G

<400> 445
catgtttatg nttttggatt actttgggca cctagtgttt ctaaatcgtc tatcattctt 60
ttctgttttt caaaagcaga gatggccaga gtctcaacaa actgtatctt caagtctttg 120
tgaaattctt tgcatgtggc agattattgg atgtagtttc ctttaactag catataaatc 180
tggtgtgttt cagataaatg aacagcaaaa tgtggtggaa ttaccatttg gaacattgtg 240
aatgaaaaat tgtgtctcta gattatgtaa caaataacta tttcctaacc attgatcttt 300
ggatttttat aatcctactc acaaatgact aggcttctcc tcttgtattt tgaagcagtg 360
tgggtgctgg attgataaaa aaaaaaaaag tcgacgcggc cgcgaattta gtag 414

<210> 446
<211> 631
<212> DNA
<213> Homo sapiens

<220>
<221> misc_feature
<222> 12
<223> n = A,T,C or G

<400> 446
acaaattaga anaaagtgcc agagaacacc acataccttg tccggaacat tacaatggct 60
tctgcatgca tgggaagtgt gagcattcta tcaatatgca ggagccatct tgcaggtgtg 120
atgctggtta tactggacaa cactgtgaaa aaaaggacta cagtgttcta tacgttgttc 180
ccggtcctgt acgatttcag tatgtcttaa tcgcagctgt gattggaaca attcagattg 240
ctgtcatctg tgtggtggtc ctctgcatca caagggccaa actttaggta atagcattgg 300
actgagattt gtaaactttc caaccttcca ggaaatgccc cagaagcaac agaattcaca 360
gacagaagca aaatacaggg cactacagtt cagacaatac aacaagagcg tccacgaggt 420
taatctaaag ggagcatgtt tcacagtggc tggactaccg agagcttgga ctacacaata 480
cagtattata gacaaaagaa taagacaaga gatctacaca tgttgccttg catttgtggt 540
aatctacacc aatgaaaaca tgtactacag ctatatttga ttatgtatgg atatatttga 600
aatagtatac attgtcttga tgttttttct g 631

<210> 447
<211> 585
<212> DNA
<213> Homo sapiens

<220>
<221> misc_feature
<222> 12, 14
<223> n = A,T,C or G

<400> 447
ccttgggaaa antntcacaa tataaagggt cgtagacttt actccaaatt ccaaaaaggt 60
cctggccatg taatcctgaa agttttccca aggtagctat aaaatcctta taagggtgca 120
gcctcttctg gaattcctct gatttcaaag tctcactctc aagttcttga aaacgagggc 180
agttcctgaa aggcaggtat agcaactgat cttcagaaag aggaactgtg tgcaccggga 240
tgggctgcca gagtaggata ggattccaga tgctgacacc ttctggggga aacagggctg 300
ccaggtttgt catagcactc atcaaagtcc ggtcaacgtc tgtgcttcga atataaacct 360
gttcatgttt ataggactca ttcaagaatt ttctatatct ctttcttata tactctccaa 420
gttcataatg ctgctccatg cccagctggg tgagttggcc aaatccttgt ggccatgagg 480
attcctttat ggggtcagtg ggaaaggtgt caatgggact tcggtctcca tgccgaaaca 540
ccaaagtcac aaacttcaac tccttggcta gtacacttcg gtcta 585

<210> 448
<211> 93
<212> DNA
<213> Homo sapiens

<220>
<221> misc_feature
<222> 20, 21, 22, 34, 50, 56, 85
<223> n = A,T,C or G

<400> 448
tgctcgtggg tcattctgan nnccgaactg accntgccag ccctgccgan gggccnccat 60
ggctccctag tgccctggag agganggggc tag 93

<210> 449
<211> 706
<212> DNA
<213> Homo sapiens

<220>
<221> misc_feature
<222> 14, 45, 46, 67, 171, 347, 390, 494, 528, 569, 650, 655
<223> n = A,T,C or G

<400> 449
   ccaagttcat gctntgtgct ggacgctgga cagggggcaa aagcnnttgc tcgtgggtca 60
   ttctgancac cgaactgacc atgccagccc tgccgatggt cctccatggc tccctagtgc 120
   cctggagagg aggtgtctag tcagagagta gtcctggaag gtggcctctg ngaggagcca 180
   cggggacagc atcctgcaga tggtcgggcg cgtcccattc gccattcagg ctgcgcaact 240
   gttgggaagg gcgatcggtg cgggcctctt cgctattacg ccagctggcg aaagggggat 300
   gtgctgcaag gcgattaagt tgggtaacgc cagggttttc ccagtcncga cgttgtaaaa 360
   cgacggccag tgaattgaat ttaggtgacn ctatagaaga gctatgacgt cgcatgcacg 420
   cgtacgtaag cttggatcct ctagagcggc cgcctactac tactaaattc gcggccgcgt 480
   cgacgtggga tccncactga gagagtggag agtgacatgt gctggacnct gtccatgaag 540
   cactgagcag aagctggagg cacaacgcnc cagacactca cagctactca ggaggctgag 600
   aacaggttga acctgggagg tggaggttgc aatgagctga gatcaggccn ctgcncccca 660
   gcatggatga cagagtgaaa ctccatctta aaaaaaaaaa aaaaaa 706

<210> 450
<211> 493
<212> DNA
<213> Homo sapiens

<400> 450
gagacggagt gtcactctgt tgcccaggct ggagtgcagc aagacactgt ctaagaaaaa 60
acagttttaa aaggtaaaac aacataaaaa gaaatatcct atagtggaaa taagagagtc 120
aaatgaggct gagaacttta caaagggatc ttacagacat gtcgccaata tcactgcatg 180
agcctaagta taagaacaac ctttggggag aaaccatcat ttgacagtga ggtacaattc 240
caagtcaggt agtgaaatgg gtggaattaa actcaaatta atcctgccag ctgaaacgca 300
agagacactg tcagagagtt aaaaagtgag ttctatccat gaggtgattc cacagtcttc 360
tcaagtcaac acatctgtga actcacagac caagttctta aaccactgtt caaactctgc 420
tacacatcag aatcacctgg agagctttac aaactcccat tgccgagggt cgacgcggcc 480
gcgaatttag tag 493

<210> 451
<211> 501
<212> DNA
<213> Homo sapiens

<220>
<221> misc_feature
<222> 142, 185, 289, 323, 364, 445, 450
<223> n = A,T,C or G

<400> 451
gggcgcgtcc cattcgccat tcaggctgcg caactgttgg gaagggcgat cggtgcgggc 60
ctcttcgcta ttacgccagc tggcgaaagg gggatgtgct gcaaggcgat taagttgggt 120
aacgccaggg ttttcccagt cncgacgttg taaaacgacg gccagtgaat tgaatttagg 180
tgacnctata gaagagctat gacgtcgcat gcacgcgtac gtaagcttgg atcctctaga 240
gcggccgcct actactacta aattcgcggc cgcgtcgacg tgggatccnc actgagagag 300
tggagagtga catgtgctgg acnctgtcca tgaagcactg agcagaagct ggaggcacaa 360
cgcnccagac actcacagct actcaggagg ctgagaacag gttgaacctg ggaggtggag 420
gttgcaatga gctgagatca ggccnctgcn ccccagcatg gatgacagag tgaaactcca 480
tcttaaaaaa aaaaaaaaaa a 501

<210> 452
<211> 51
<212> DNA
<213> Homo sapiens

<220>
<221> misc_feature
<222> 14, 22, 37, 38
<223> n = A,T,C or G

<400> 452
   agacggtttc accnttacaa cnccttttag gatgggnntt ggggagcaag c 51

<210> 453
<211> 317
<212> DNA

<213> Homo sapiens

<220>
<221> misc_feature
<222> 129, 232
<223> n = A,T,C or G

<400> 453
tacatcttgc tttttcccca ttggaactag tcattaaccc atctctgaac tggtagaaaa 60
acatctgaag agctagtcta tcagcatctg gcaagtgaat tggatggttc tcagaaccat 120
ttcacccana cagcctgttt ctatcctgtt taataaatta gtttgggttc tctacatgca 180
taacaaaccc tgctccaatc tgtcacataa aagtctgtga cttgaagttt antcagcacc 240
cccaccaaac tttatttttc tatgtgtttt ttgcaacata tgagtgtttt gaaaataagg 300
tacccatgtc tttatta 317

<210> 454
<211> 231
<212> DNA
<213> Homo sapiens

<400> 454
ttcgaggtac aatcaactct cagagtgtag tttccttcta tagatgagtc agcattaata 60
taagccacgc cacgctcttg aaggagtctt gaattctcct ctgctcactc agtagaacca 120
agaagaccaa attcttctgc atcccagctt gcaaacaaaa ttgttcttct aggtctccac 180
ccttcctttt tcagtgttcc aaagctcctc acaatttcat gaacaacagc t 231

<210> 455
<211> 231
<212> DNA
<213> Homo sapiens

<400> 455
taccaaagag ggcataataa tcagtctcac agtagggttc accatcctcc aagtgaaaaa 60
cattgttccg aatgggcttt ccacaggcta cacacacaaa acaggaaaca tgccaagttt 120
gtttcaacgc attgatgact tctccaagga tcttcctttg gcatcgacca cattcagggg 180
caaagaattt ctcatagcac agctcacaat acagggctcc tttctcctct a 231

<210> 456
<211> 231
<212> DNA
<213> Homo sapiens

<400> 456
ttggcaggta cccttacaaa gaagacacca taccttatgc gttattaggt ggaataatca 60
ttccattcag tattatcgtt attattcttg gagaaaccct gtctgtttac tgtaaccttt 120
tgcactcaaa ttcctttatc aggaataact acatagccac tatttacaaa gccattggaa 180
cctttttatt tggtgcagct gctagtcagt ccctgactga cattgccaag t 231

<210> 457
<211> 231
<212> DNA
<213> Homo sapiens

<220>
<221> misc_feature
<222> 29, 30, 35
<223> n = A,T,C or G

<400> 457
cgaggtaccc aggggtctga aaatctctnn tttantagtc gatagcaaaa ttgttcatca 60
gcattcctta atatgatctt gctataatta gatttttctc cattagagtt catacagttt 120
tatttgattt tattagcaat ctctttcaga agacccttga gatcattaag ctttgtatcc 180
agttgtctaa atcgatgcct catttcctct gaggtgtcgc tggcttttgt g 231

<210> 458
<211> 231
<212> DNA
<213> Homo sapiens

<400> 458
aggtctggtt ccccccactt ccactcccct ctactctctc taggactggg ctgggccaag 60
agaagagggg tggttaggga agccgttgag acctgaagcc ccaccctcta ccttccttca 120
acaccctaac cttgggtaac agcatttgga attatcattt gggatgagta gaatttccaa 180
ggtcctgggt taggcatttt ggggggccag accccaggag aagaagattc t 231

<210> 459
<211> 231
<212> DNA
<213> Homo sapiens

<400> 459
ggtaccgagg ctcgctgaca cagagaaacc ccaacgcgag gaaaggaatg gccagccaca 60
ccttcgcgaa acctgtggtg gcccaccagt cctaacggga caggacagag agacagagca 120
gccctgcact gttttccctc caccacagcc atcctgtccc tcattggctc tgtgctttcc 180
actatacaca gtcaccgtcc caatgagaaa caagaaggag caccctccac a 231

<210> 460
<211> 231
<212> DNA
<213> Homo sapiens

<400> 460
gcaggtataa catgctgcaa caacagatgt gactaggaac ggccggtgac atggggaggg 60
cctatcaccc tattcttggg ggctgcttct tcacagtgat catgaagcct agcagcaaat 120
cccacctccc cacacgcaca cggccagcct ggagcccaca gaagggtcct cctgcagcca 180
gtggagcttg gtccagcctc cagtccaccc ctaccaggct taaggataga a 231

<210> 461
<211> 231
<212> DNA
<213> Homo sapiens

<400> 461
cgaggtttga gaagctctaa tgtgcagggg agccgagaag caggcggcct agggagggtc 60
gcgtgtgctc cagaagagtg tgtgcatgcc agaggggaaa caggcgcctg tgtgtcctgg 120
gtggggttca gtgaggagtg ggaaattggt tcagcagaac caagccgttg ggtgaataag 180
agggggattc catggcactg atagagccct atagtttcag agctgggaat t 231

<210> 462
<211> 231
<212> DNA
<213> Homo sapiens

<400> 462
aggtaccctc attgtagcca tgggaaaatt gatgttcagt ggggatcagt gaattaaatg 60
gggtcatgca agtataaaaa ttaaaaaaaa aagacttcat gcccaatctc atatgatgtg 120
gaagaactgt tagagagacc aacagggtag tgggttagag atttccagag tcttacattt 180
tctagaggag gtatttaatt tcttctcact catccagtgt tgtatttagg a 231

<210> 463
<211> 231
<212> DNA
<213> Homo sapiens

<400> 463
tactccagcc tggtgacaga gcgagaccct atcaccgccc cccaccccac caaaaaaaaa 60
actgagtaga caggtgtcct cttggcatgg taagtcttaa gtcccctccc agatctgtga 120
catttgacag gtgtcttttc ctctggacct cggtgtcccc atctgagtga gaaaaggcag 180
tggggaggtg gatcttccag tcgaagcggt atagaagccc gtgtgaaaag c 231

<210> 464
<211> 231
<212> DNA
<213> Homo sapiens

<400> 464

gtactctaag attttatcta agttgccttt tctgggtggg aaagtttaac cttagtgact 60
aaggacatca catatgaaga atgtttaagt tggaggtggc aacgtgaatt gcaaacaggg 120
cctgcttcag tgactgtgtg cctgtagtcc cagctactcg ggagtctgtg tgaggccagg 180
ggtgccagcg caccagctag atgctctgta acttctaggc cccattttcc c 231

<210> 465
<211> 231
<212> DNA
<213> Homo sapiens

<400> 465
catgttgttg tagctgtggt aatgctggct gcatctcaga cagggttaac ttcagctcct 60
gtggcaaatt agcaacaaat tctgacatca tatttatggt ttctgtatct ttgttgatga 120
aggatggcac aatttttgct tgtgttcata atatactcag attagttcag ctccatcaga 180
taaactggag acatgcagga cattagggta gtgttgtagc tctggtaatg a 231

<210> 466
<211> 231
<212> DNA
<213> Homo sapiens

<400> 466
   caggtacctc tttccattgg atactgtgct agcaagcatg ctctccgggg tttttttaat 60
   ggccttcgaa cagaacttgc cacataccca ggtataatag tttctaacat ttgcccagga 120
   cctgtgcaat caaatattgt ggagaattcc ctagctggag aagtcacaaa gactataggc 180
   aataatggag accagtccca caagatgaca accagtcgtt gtgtgcggct g 231

<210> 467
<211> 311
<212> DNA
<213> Homo sapiens

<400> 467
gtacaccctg gcacagtcca atctgaactg gttcggcact catctttcat gagatggatg 60
tggtggcttt tctccttttt catcaagact cctcagcagg gagcccagac cagcctgcac 120
tgtgccttaa cagaaggtct tgagattcta agtgggaatc atttcagtga ctgtcatgtg 180
gcatgggtct ctgcccaagc tcgtaatgag actatagcaa ggcggctgtg ggacgtcagt 240
tgtgacctgc tgggcctccc aatagactaa caggcagtgc cagttggacc caagagaaga 300
ctgcagcaga c 311

<210> 468
<211> 3112
<212> DNA
<213> Homo sapiens

<400> 468
   cattgtgttg ggagaaaaac agaggggaga tttgtgtggc tgcagccgag ggagaccagg 60
   aagatctgca tggtgggaag gacctgatga tacagagttt gataggagac aattaaaggc 120
   tggaaggcac tggatgcctg atgatgaagt ggactttcaa actggggcac tactgaaacg 180
   atgggatggc cagagacaca ggagatgagt tggagcaagc tcaataacaa agtggttcaa 240
   cgaggacttg gaattgcatg gagctggagc tgaagtttag cccaattgtt tactagttga 300
   gtgaatgtgg atgattggat gatcatttct catctctgag cctcaggttc cccatccata 360
   aaatgggata cacagtatga tctataaagt gggatatagt atgatctact tcactgggtt 420
   atttgaagga tgaattgaga taatttattt caggtgccta gaacaatgcc cagattagta 480
   catttggtgg aactgagaaa tggcataaca ccaaatttaa tatatgtcag atgttactat 540
   gattatcatt caatctcata gttttgtcat ggcccaattt atcctcactt gtgcctcaac 600
   aaattgaact gttaacaaag gaatctctgg tcctgggtaa tggctgagca ccactgagca 660
   tttccattcc agttggcttc ttgggtttgc tagctgcatc actagtcatc ttaaataaat 720
   gaagttttaa catttctcca gtgatttttt tatctcacct ttgaagatac tatgttatgt 780
   gattaaataa agaacttgag aagaacaggt ttcattaaac ataaaatcaa tgtagacgca 840
   aattttctgg atgggcaata cttatgttca caggaaatgc tttaaaatat gcagaagata 900
   attaaatggc aatggacaaa gtgaaaaact tagacttttt tttttttttt ggaagtatct 960
   ggatgttcct tagtcactta aaggagaact gaaaaatagc agtgagttcc acataatcca 1020
   acctgtgaga ttaaggctct ttgtggggaa ggacaaagat ctgtaaattt acagtttcct 1080
   tccaaagcca acgtcgaatt ttgaaacata tcaaagctct tcttcaagac aaataatcta 1140
   tagtacatct ttcttatggg atgcacttat gaaaaatggt ggctgtcaac atctagtcac 1200
   tttagctctc aaaatggttc attttaagag aaagttttag aatctcatat ttattcctgt 1260
   ggaaggacag cattgtggct tggactttat aaggtcttta ttcaactaaa taggtgagaa 1320
   ataagaaagg ctgctgactt taccatctga ggccacacat ctgctgaaat ggagataatt 1380
   aacatcacta gaaacagcaa gatgacaata taatgtctaa gtagtgacat gtttttgcac 1440
   atttccagcc cctttaaata tccacacaca caggaagcac aaaaggaagc acagagatcc 1500
   ctgggagaaa tgcccggccg ccatcttggg tcatcgatga gcctcgccct gtgcctggtc 1560
   ccgcttgtga gggaaggaca ttagaaaatg aattgatgtg ttccttaaag gatgggcagg 1620
   aaaacagatc ctgttgtgga tatttatttg aacgggatta cagatttgaa atgaagtcac 1680
   aaagtgagca ttaccaatga gaggaaaaca gacgagaaaa tcttgatggc ttcacaagac 1740
   atgcaacaaa caaaatggaa tactgtgatg acatgaggca gccaagctgg ggaggagata 1800
   accacggggc agagggtcag gattctggcc ctgctgccta aactgtgcgt tcataaccaa 1860
   atcatttcat atttctaacc ctcaaaacaa agctgttgta atatctgatc tctacggttc 1920
   cttctgggcc caacattctc catatatcca gccacactca tttttaatat ttagttccca 1980
   gatctgtact gtgacctttc tacactgtag aataacatta ctcattttgt tcaaagaccc 2040
   ttcgtgttgc tgcctaatat gtagctgact gtttttccta aggagtgttc tggcccaggg 2100
   gatctgtgaa caggctggga agcatctcaa gatctttcca gggttatact tactagcaca 2160
   cagcatgatc attacggagt gaattatcta atcaacatca tcctcagtgt ctttgcccat 2220
   actgaaattc atttcccact tttgtgccca ttctcaagac ctcaaaatgt cattccatta 2280
   atatcacagg attaactttt tttttaaccc tggaagaatt caatgttaca tgcagctatg 2340
   ggaatttaat tacatatttt gttttccagt gcaaagatga ctaagtcctt tatccctccc 2400
   ctttgtttga ttttttttcc agtataaagt taaaatgctt agccttgtac tgaggctgta 2460
   tacagccaca gcctctcccc atccctccag ccttatctgt catcaccatc aacccctccc 2520
   atgcacctaa acaaaatcta acttgtaatt ccttgaacat gtcaggcata cattattcct 2580
   tctgcctgag aagctcttcc ttgtctctta aatctagaat gatgtaaagt tttgaataag 2640
   ttgactatct tacttcatgc aaagaaggga cacatatgag attcatcatc acatgagaca 2700
   gcaaatacta aaagtgtaat ttgattataa gagtttagat aaatatatga aatgcaagag 2760
   ccacagaggg aatgtttatg gggcacgttt gtaagcctgg gatgtgaagc aaaggcaggg 2820
   aacctcatag tatcttatat aatatacttc atttctctat ctctatcaca atatccaaca 2880
   agcttttcac agaattcatg cagtgcaaat ccccaaaggt aacctttatc catttcatgg 2940
   tgagtgcgct ttagaatttt ggcaaatcat actggtcact tatctcaact ttgagatgtg 3000
   tttgtccttg tagttaattg aaagaaatag ggcactcttg tgagccactt tagggttcac 3060
   tcctggcaat aaagaattta caaagagcaa aaaaaaaaaa aaaaaaaaaa aa 3112

<210> 469
<211> 2229
<212> DNA
<213> Homo sapiens

<400> 469
agctctttgt aaattcttta ttgccaggag tgaaccctaa agtggctcac aagagtgccc 60
tatttctttc aattaactac aaggacaaac acatctcaaa gttgagataa gtgaccagta 120
tgatttgcca aaattctaaa gcgcactcac catgaaatgg ataaaggtta cctttgggga 180
tttgcactgc atgaattctg tgaaaagctt gttggatatt gtgatagaga tagagaaatg 240
aagtatatta tataagatac tatgaggttc cctgcctttg cttcacatcc caggcttaca 300
aacgtgcccc ataaacattc cctctgtggc tcttgcattt catatattta tctaaactct 360
tataatcaaa tacactttta gtatttgctg tctcatgtga tgatgaatct catatgtgtc 420
ccttctttgc atgaagtaag atagtcaact tattcaaaac tttacatcat tctagattta 480
agagacaagg aagagcttct caggcagaag gaataatgta tgcctgacat gttcaaggaa 540
ttacaagtta gattttgttt aggtgcatgg gaggggttga tggtgatgac agataaggct 600
ggagggatgg ggagaggctg tggctgtata cagcctcagt acaaggctaa gcattttaac 660
tttatactgg aaaaaaaatc aaacaaaggg gagggataaa ggacttagtc atctttgcac 720
tggaaaacaa aatatgtaat taaattccca tagctgcatg taacattgaa ttcttccagg 780
ttaaaaaaaa agttaatcct gtgatattaa tggaatgaca ttttgaggtc ttgagaatgg 840
gcacaaaagt gggaaatgaa tttcagtatg ggcaaagaca ctgaggatga tgttgattag 900
ataattcact ccgtaatgat catgctgtgt gctagtaagt ataaccctgg aaagatcttg 960
agatgcttcc cagcctgttc acagatcccc tgggccagaa cactccttag gaaaaacagt 1020
cagctacata ttaggcagca acacgaaggg tctttgaaca aaatgagtaa tgttattcta 1080
cagtgtagaa aggtcacagt acagatctgg gaactaaata ttaaaaatga gtgtggctgg 1140
atatatggag aatgttgggc ccagaaggaa ccgtagagat cagatattac aacagctttg 1200
ttttgagggt tagaaatatg aaatgatttg gttatgaacg cacagtttag gcagcagggc 1260
cagaatcctg accctctgcc ccgtggttat ctcctcccca gcttggctgc ctcatgtcat 1320
cacagtattc cattttgttt gttgcatgtc ttgtgaagcc atcaagattt tctcgtctgt 1380
tttcctctca ttggtaatgc tcactttgtg acttcatttc aaatctgtaa tcccgttcaa 1440
ataaatatcc acaacaggat ctgttttcct gcccatcctt taaggaacac atcaattcat 1500
tttctaatgt ccttccctca caagcgggac caggcacagg gcgaggctca tcgatgaccc 1560
aagatggcgg ccgggcattt ctcccaggga tctctgtgct tccttttgtg cttcctgtgt 1620
gtgtggatat ttaaaggggc tggaaatgtg caaaaacatg tcactactta gacattatat 1680
tgtcatcttg ctgtttctag tgatgttaat tatctccatt tcagcagatg tgtggcctca 1740
gatggtaaag tcagcagcct ttcttatttc tcacctggaa atacatacga ccatttgagg 1800
agacaaatgg caaggtgtca gcataccctg aacttgagtt gagagctaca cacaatatta 1860

ttggtttccg agcatcacaa acaccctctc tgtttcttca ctgggcacag aattttaata 1920
cttatttcag tgggctgttg gcaggaacaa atgaagcaat ctacataaag tcactagtgc 1980
agtgcctgac acacaccatt ctcttgaggt cccctctaga gatcccacag gtcatatgac 2040
ttcttgggga gcagtggctc acacctgtaa tcccagcact ttgggaggct gaggcaggtg 2100
ggtcacctga ggtcaggagt tcaagaccag cctggccaat atggtgaaac cccatctcta 2160
ctaaaaatac aaaaattagc tgggcgtgct ggtgcatgcc tgtaatccca gccccaacac 2220
aatggaatt 2229

<210> 470
<211> 2426
<212> DNA
<213> Homo sapiens

<400> 470
gtaaattctt tattgccagg agtgaaccct aaagtggctc acaagagtgc cctatttctt 60
tcaattaact acaaggacaa acacatctca aagttgagat aagtgaccag tatgatttgc 120
caaaattcta aagcgcactc accatgaaat ggataaaggt tacctttggg gatttgcact 180
gcatgaattc tgtgaaaagc ttgttggata ttgtgataga gatagagaaa tgaagtatat 240
tatataagat actatgaggt tccctgcctt tgcttcacat cccaggctta caaacgtgcc 300
ccataaacat tccctctgtg gctcttgcat ttcatatatt tatctaaact cttataatca 360
aattacactt ttagtatttg ctgtctcatg tgatgatgaa tctcatatgt gtcccttctt 420
tgcatgaagt aagatagtca acttattcaa aactttacat cattctagat ttaagagaca 480
aggaagagct tctcaggcag aaggaataat gtatgcctga catgttcaag gaattacaag 540
ttagattttg tttaggtgca tgggaggggt tgatggtgat gacagataag gctggaggga 600
tggggagagg ctgtggctgt atacagcctc agtacaaggc taagcatttt aactttatac 660
tggaaaaaaa atcaaacaaa ggggagggat aaaggactta gtcatctttg cactggaaaa 720
caaaatatgt aattaaattc ccatagctgc atgtaacatt gaattcttcc aggttaaaaa 780
aaaaagttaa tcctgtgata ttaatggaat gacattttga ggtcttgaga atgggcacaa 840
aagtgggaaa tgaatttcag tatgggcaaa gacactgagg atgatgttga ttagataatt 900
cactccgtaa tgatcatgct gtgtgctagt aagtataacc ctggaaagat cttgagatgc 960
ttcccagcct gttcacagat cccctgggcc agaacactcc ttaggaaaaa cagtcagcta 1020
catattaggc agcaacacga agggtctttg aacaaaatga gtaatgttat tctacagtgt 1080
agaaaggtca cagtacagat ctgggaacta aatattaaaa atgagtgtgg ctggatatat 1140
ggagaatgtt gggcccagaa ggaaccgtag agatcagata ttacaacagc tttgttttga 1200
gggttagaaa tatgaaatga tttggttatg aacgcacagt ttaggcagca gggccagaat 1260
cctgaccctc tgccccgtgg ttatctcctc cccagcttgg ctgcctcatg tcatcacagt 1320
attccatttt gtttgttgca tgtcttgtga agccatcaag attttctcgt ctgttttcct 1380
ctcattggta atgctcactt tgtgacttca tttcaaatct gtaatcccgt tcaaataaat 1440
atccacaaca ggatctgttt tcctgcccat cctttaagga acacatcaat tcattttcta 1500
atgtccttcc ctcacaagcg ggaccaggca cagggcgagg ctcatcgatg acccaagatg 1560
gcggccgggc atttctccca gggatctctg tgcttccttt tgtgcttcct gtgtgtgtgg 1620
atatttaaag gggctggaaa tgtgcaaaaa catgtcacta cttagacatt atattgtcat 1680
cttgctgttt ctagtgatgt taattatctc catttcagca gatgtgtggc ctcagatggt 1740
aaagtcagca gcctttctta tttctcacct ggaaatacat acgaccattt gaggagacaa 1800
atggcaaggt gtcagcatac cctgaacttg agttgagagc tacacacaat attattggtt 1860
tccgagcatc acaaacaccc tctctgtttc ttcactgggc acagaatttt aatacttatt 1920
tcagtgggct gttggcagga acaaatgaag caatctacat aaagtcacta gtgcagtgcc 1980
tgacacacac cattctcttg aggtcccctc tagagatccc acaggtcata tgacttcttg 2040
gggagcagtg gctcacacct gtaatcccag cactttggga ggctgaggca ggtgggtcac 2100
ctgaggtcag gagttcaaga ccagcctggc caatatggtg aaaccccatc tctactaaaa 2160
atacaaaaat tagctgggcg tgctggtgca tgcctgtaat cccagctact tgggaggctg 2220
aggcaggaga attgctggaa catgggaggc ggaggttgca gtgagctgta attgtgccat 2280
tgcactcgaa cctgggcgac agagtggaac tctgtttcca aaaaacaaac aaacaaaaaa 2340
ggcatagtca gatacaacgt gggtgggatg tgtaaataga agcaggatat aaagggcatg 2400
gggtgacggt tttgcccaac acaatg 2426

<210> 471
<211> 812
<212> DNA
<213> Homo sapiens

<400> 471
gaacaaaatg agtaatgtta ttctacagtg tagaaaggtc acagtacaga tctgggaact 60
aaatattaaa aatgagtgtg gctggatata tggagaatgt tgggcccaga aggaaccgta 120
gagatcagat attacaacag ctttgttttg agggttagaa atatgaaatg atttggttat 180
gaacgcacag tttaggcagc agggccagaa tcctgaccct ctgccccgtg gttatctcct 240
ccccagcttg gctgcctcat gtcatcacag tattccattt tgtttgttgc atgtcttgtg 300
aagccatcaa gattttctcg tctgttttcc tctcattggt aatgctcact ttgtgacttc 360

atttcaaatc tgtaatcccg ttcaaataaa tatccacaac aggatctgtt ttcctgccca 420
tcctttaagg aacacatcaa ttcattttct aatgtccttc cctcacaagc gggaccaggc 480
acagggcgag gctcatcgat gacccaagat ggcggccggg catttctccc agggatctct 540
gtgcttcctt ttgtgcttcc tgtgtgtgtg gatatttaaa ggggctggaa atgtgcaaaa 600
acatgtcact acttagacat tatattgtca tcttgctgtt tctagtgatg ttaattatct 660
ccatttcagc agatgtgtgg cctcagatgg taaagtcagc agcctttctt atttctcacc 720
tctgtatcat caggtccttc ccaccatgca gatcttcctg gtctccctcg gctgcagcca 780
cacaaatctc ccctctgttt ttctgatgcc ag 812

<210> 472
<211> 515
<212> DNA
<213> Homo sapiens

<220>
<221> misc_feature
<222> 491, 504, 510
<223> n = A,T,C or G

<400> 472
acggagactt attttctgat attgtctgca tatgtatgtt tttaagagtc tggaaatagt 60
cttatgactt tcctatcatg cttattaata aataatacag cccagagaag atgaaaatgg 120
gttccagaat tattggtcct tgcagcccgg tgaatctcag caagaggaac caccaactga 180
caatcaggat attgaacctg gacaagagag agaaggaaca cctccgatcg aagaacgtaa 240
agtagaaggt gattgccagg aaatggatct ggaaaagact cggagtgagc gtggagatgg 300
ctctgatgta aaagagaaga ctccacctaa tcctaagcat gctaagacta aagaagcagg 360
agatgggcag ccataagtta aaaagaagac aagctgaagc tacacacatg gctgatgtca 420
cattgaaaat gtgactgaaa atttgaaaat tctctcaata aagtttgagt tttctctgaa 480
gaaaaaaaaa naaaaaaaaa aaanaaaaan aaaaa 515

<210> 473
<211> 5829
<212> DNA
<213> Homo sapiens

<400> 473
   cgcatgccgg ggaagcccaa gctggctcga agagccacca gccacctgtg caagggtggg 60
   cctggaccag ttggaccagc caccaagctc acctactcaa ggaagcaggg atggccaggt 120
   tgcaacagcc tgagtggctg ccacctgata gctgatggag cagaggcctg aggaaaatca 180
   gatggcacat ttagctcttt aatggatctt aagttaattt ttctataaag cacatggcac 240
   cagtccatgc ctcagagctc gtatggcact gcggaccaca gcaggccgag ttcccaggat 300
   tgccatccag gggggccttc tgtagccctg gccagacctt gcagaggtgg ctgggtgctc 360
   tttgagcgag ctcggcctcc ctggcatgca caggccccag gtactgacac gctgctctga 420
   gtgagcttgt cctgccttgg ctgccaccta actgctgatg gagcagcggc cttaggaaaa 480
   gcaaatggcg ctgtagccca actttagggt agaagaagat gtaccatgtc cggccgctag 540
   ttggtgactg gtgcacctgc tcctggcgta cccttgcaga ggtgggtggt tgctctttgg 600
   ccagcttggc cttgcctggc atgcacaagc ctcagtgcaa caactgtcct acaaatggag 660
   acacagagag gaaacaagca gcgggctcag gagcagggtg tgtgctgcct ttggggctcc 720
   agtccatgcc tcgggtcgta tggtactgca ggcttcttgg ttgccaagag gcggaccaca 780
   ggccttcttg aggaggactt tacgttcaag tgcagaaagc agccaaaatt accatccatg 840
   agactaagcc ttctgtggcc ctggcgagac ttaaaatttg tgccaaggca ggacaagctc 900
   actcggagca gcgtgtcagt agctggggcc tatgcatgcc gggcagggcc gggctggctg 960
   aaggagcaac cagccacctc tgcaagggtg cgcctagtgc aggcggagca tccaccacct 1020
   cacccgctcg aggaagtggg gatggccagg ttcccacagc ctgagtgtct gccaccttat 1080
   tgctgatgga gcagaggcct taagaaaagc agatggcact gtggccctac ctttagggtg 1140
   gaagaagtga tgtacatgtc cggacgctaa ttggtgactg gtacaccggc tcctgctaca 1200
   cctttgcaga ggtggctggt tgctctttga gccagcttgt ccttgcccgg catgcacaag 1260
   tttcagtgca acaactttgc cacaaatgga gccatataga ggaaacaaga agcaggttca 1320
   ggagaagggt gtaccctgcc tttggggctc cagtccatgc ctcaggtgtc acatggcact 1380
   gcgggcttct tggttgccag gaggcggacc acaggccatc ttggggagga ctttgtgttc 1440
   aagtgcagaa agcagccagg attgccatcc agggggacct tctatagccc tggccaaacc 1500
   ttgcaggggt gtctggttgc tctttgagcc ggcttggcct ccctggcatg cacgggcccc 1560
   aggtgctggc acgctgctcc gagtgtgctt gtcctgcctt ggctgccacc tctgcggggg 1620
   tgcgtctgga gggggtggac cggccaccaa ccttacccag tcaaggaagt ggatggccat 1680
   gttcccacag cctgagtggc tgccacctga tggctgatgg agcaaaggcc ttaggaaaag 1740
   cagatggccc ttggccctac ctttttgtta gaagaactga tgttccatgt cctgcagcga 1800
   gtgaggttgg tggctgtgcc cccagctcct ggcgcgccct cgcagaggtg actggttgct 1860
   ctttgggccc tcttggcctt gcccagcatg cacaagcctc agtgctacta ctgtgctaca 1920
   aatggagcca tataggggaa acgagcagcc atctcaggag caaggtgtat gctgcctttg 1980
   ggggctccag tccttgcctc aagggtctta tgtcactgtg ggcttcttgg ttgtcaagag 2040
   gcagaccata ggccgtcttg agagggactt tatgttcaag tgcagaaagc agccaggatt 2100
   gccaccctcg ggactctgcc ttctgtggcc ctggccaaac ttagaatttg gccgtagaca 2160
   ggacaggctc acttggagta gcgtgtccgt agctggggtc tgtgcatgcc gggcaaggcc 2220
   gggctggctc ggggagcaac cagccacctc tgcgggggtg cgcctggagc aggtggagca 2280
   gccaccagct cacccactcc aggaagccgg ggtagccagg ttcccaaggc ctgagtgggt 2340
   gccacctaat ggctgaagaa acagaggcct tgggaaaacc agatggcact gtggccctac 2400
   ctttatggta gaagagctga tttagcctga ctggcagcgt gtggggttgg tggctggtct 2460
   gcctgctgct ggcgcatccg tgcaaggatg gctggttgcc ctttgagcca gcttgccctt 2520
   gcccggcatg cgcaagcctc agtgcaacaa ctgtgctgca aatggggcca tatagaggaa 2580
   aggagcagct ggctctggag catggtgtgc actccctttg ggccttcagt ccatgtctca 2640
   tgggtcgtat gacactgcgg gcttgttggt tgccaagagg cagaccacag gtcatcttga 2700
   ggaggacttt atgttccagt ccagaaagca gccagtggta ccacccaggg gacttgtgct 2760
   tctgtgccca ggccagacgt agaatttgac aaagtcagga cggtctcagt cagagcggcg 2820
   tgtcggtccc cggggcctgt gcatgccggg cagggccggg ctggcttggg gagcaagcag 2880
   ccacctctgt taagggtgtg cctggagcag gtggagcagc caccaacctc acgcactgaa 2940
   agaagcaggg atggccaggt tccaacatcc tgagtggctg ccacctgatg gctgatggag 3000
   cagaggcctg aggaaaagca gatggcactg ctttgtagtg ctgttctttg tctctcttga 3060
   tctttttcag ttaatgtctg ttttatcaga gactaggatt gcaaaccctg ctcttttttg 3120
   ctttccattt gcttggtaaa tattcctcca tccctttatt ttaagcctat gtgtgtcttt 3180
   gcacatgaga tgggtctcct gaatacagga caacaatggg tctttactct ttatccaact 3240
   tgccagtctg tgtcttttaa ctggggcatt tagcccattt acatttaagt ttagtattgt 3300
   tacatgtgaa atttatcctg tcatgatgtt gctagctttt tatttttccc attagtttgc 3360
   agtttcttta tagtgtcaat ggtctttaca attcgatatg tttttgtagt ggctggtact 3420
   ggtttttcct ttctacgttt agtgtctcct tcaggagctc ttgtaacaca agaatgtgga 3480
   tttatttctt gtaaggtaaa tatgtggatt tatttcttgg gactgtattc tatggccttt 3540
   accccaagaa tcattacttt ttaaaatgca attcaaatta gcataaaaca tttacagcct 3600
   atggaaaggc ttgtggcatt agaatcctta tttataggat tattttgtgt ttttttgaga 3660
   tatggtcttt gtcatcgagg cagaagtgcc gtggtttgat cataattcac cacagccctg 3720
   aactcttgag tccaagccat ccttttgcct taatctccca accagttgga tctgcaggca 3780
   taaggcatca tgcgtggcta attttttcac gttttttttt tttttttgtc gagattatgg 3840
   tgtcactgtg ttgctctggc tgatctcaaa tgtttgacct caagggatct ttctgccacg 3900
   gcctcctaaa gtgctaggat tatatgcatg atacaccatg cctattgtag agtattacat 3960
   tattttcaaa gtcttattgt aagagccatt tattgccttt ggcctaaata actcaatata 4020
   atatctctga aacttttttt tgacaaattt tggggcgtga tgatgagaga agggggtttg 4080
   aaactttcta ataagagtta acttagagcc atttaagaaa ggaaaaaaca caaattatca 4140
   gaaaaacaac agtaagatca agtgcaaaag ttctgtggca aagatgatga gagtaaagaa 4200
   tatatgtttg tgactcatgg tggcttttac tttgttcttg aatttctgag tacgggttaa 4260
   catttaaaga atctacatta tagataacat tttattgcaa gtaaatgtat ttcaaaattt 4320
   gttattggtt ttgtatgaga ttattctcag cctacttcat tatcaagcta tattatttta 4380
   ttaatgtagt tcgatgatct tacagcaaag ctgaaagctg tatcttcaaa atatgtctat 4440
   ttgactaaaa agttattcaa caggagttat tatctataaa aaaaatacaa caggaatata 4500
   aaaaacttga ggataaaaag atgttggaaa aagtaatatt aaatcttaaa aaacatatgg 4560
   aaactacaca atggtgaaga cacattggtg aagtacaaaa atataaattg gatctagaag 4620
   aaagggcaat gcaggcaata gaaaaattag tagaaatccc tttaaaggtt agtttgtaaa 4680
   atcaggtaag tttatttata atttgctttc atttatttca ctgcaaatta tattttggat 4740
   atgtatatat attgtgcttc ctctgcctgt cttacagcaa tttgccttgc agagttctag 4800
   gaaaaaggtg gcatgtgttt ttactttcaa aatatttaaa tttccatcat tataacaaaa 4860
   tcaatttttc agagtaatga ttctcactgt ggagtcattt gattattaag acccgttggc 4920
   ataagattac atcctctgac tataaaaatc ctggaagaaa acctaggaaa tattcgtctg 4980
   gacattgcac ttggcaatga atttatgggt aaccactgat ccacttccag tcactatcca 5040
   tgagttttta tttccagata catgaaatca tatgagttga aactttcttt tgattgagca 5100
   gtttggaaac cgtctttttg tagaatctgc aagtggatat ttggaaccct ttgaggccta 5160
   tgctgaaaaa agaaatatct tcactacatg atgaccacca gcagcagctg gggaaaccag 5220
   caccctgtgg aattccatac ggtgcataga atacatcctc ccttcagtcg gcttgggtca 5280
   acttaggtca tgggccacct ggctgatagc agtttccaca gaaatgcttc aagatgaaag 5340
   tggatgaccg ggccaccctc caccactgcc ctgtaagacc atgggacaca caggccacca 5400
   gttcttttca tgtggtcatc ccctgttaga tgggagaaaa tacacctgcc tcatttttgt 5460
   accttctgtg tgaacattcc acggcagact gtcgctaaat gtggatgaag aattgaatga 5520
   atgaatgaat atgagagaaa atgaataaat ggttcagatc ctgggctgga aggctgtgta 5580
   tgaggatggt gggtagagga gggtctgttt ttcttgcctt taagtcacta attgtcactt 5640
   tggggcagga gcacaggctt tgaatgcaga ccgactggac tttaattctg gctttactag 5700
   ttgtgattgt gtgaccttgt gaaagttact taaaccctct gtgcctgttt ctttatctgt 5760
   aaaatggaga taataagatg tcaaaggact gtggtaagaa ttaaatgctt taaaaaaaaa 5820
   aaaaaaaaa 5829

<210> 474
<211> 1594
<212> DNA
<213> Homo sapiens

<400> 474
atttatggat cattaatgcc tctttagtag tttagagaaa acgtcaaaag aaatggcccc 60
agaataagct tcttgatttg taaaattcta tgtcattggc tcaaatttgt atagtatctc 120
aaaatataaa tatatagaca tctcagataa tatatttgaa atagcaaatt cctgttagaa 180
aataatagta cttaactaga tgagaataac aggtcgccat tatttgaatt gtctcctatt 240
cgtttttcat ttgttgtgtt actcatgttt tacttatgag ggatatatat aacttccact 300
gttttcagaa ttattgtatg cagtcagtat gagaatgcaa tttaagtttc cttgatgctt 360
tttcacactt ctattactag aaataagaat acagtaatat tggcaaagaa aattgaccag 420
ttcaataaaa ttttttagta aatctgattg aaaataaaca ttgcttatgg ctttcttaca 480
tcaatattgt tatgtcctag acaccttatc tgaaattacg gcttcaaaat tctaattatg 540
tgcaaatgtg taaaatatca atactttatg ttcaagctgg ggcctcttca ggcgtcctgg 600
gctgagagag aaagatgcta gctccgcaag ccggagaggg aacaccgcca cattgttaca 660
cggacacacc gccacgtgga cacatgacca gactcacatg tacagacaca cggagacatt 720
accacatgga gacaccgtca cacagtcaca cggacacact ggcatagtca catggacgga 780
cacacagaca tatggagaaa tcacatggac acaccaccac actatcacag ggacacagac 840
acacggagac atcaccacat ggacacactg tcacactacc acagggacac gagacatcac 900
actgtcacat ggacacacca tcacacacat gaacacaccg acacactgcc atatggacac 960
tggcacacac actgccacac tgtcacatgg acacacctcc acaccatcac accaccacac 1020
acactgcctg tggacacaag gacacacaga cactgtcaca cagatacaca aaacactgtc 1080
acacggagac atcaccatgc agatacacca ccactctggt gccgtctgaa ttaccctgct 1140
gggggacag cagtggcata ctcatgccta agtgactggc tttcacccca gtagtgattg 1200
ccctccatca acactgccca ccccaggttg gggctacccc agcccatctt tacaaaacag 1260
ggcaaggtga actaatggag tgggtggagg agttggaaga aatcccagcg tcagtcaccg 1320
ggatagaatt cccaaggaac cctcttttg gaggatggtt tccatttctg gaggcgatct 1380
gccgacaggg tgaatgcctt cttgcttgtc ttctggggaa tcagagagag tccgttttgt 1440
ggtgggaaga gtgtggctgt gtactttgaa ctcctgtaaa ttctctgact catgtccaca 1500
aaaccaacag ttttgtgaat gtgtctggag gcaagggaag ggccactcag gatctatgtt 1560
gaagggaaga ggcctggggc tggagtattc gctt 1594

<210> 475
<211> 2414
<212> DNA
<213> Homo sapiens

<220>
<221> misc_feature
<222> 33
<223> n = A,T,C or G

<400> 475
cccaacacaa tggctttata agaatgcttc acntgtgaaa aacaaatatc aaagtcttct 60
tgtagattat ttttaaggac aaatctttat tccatgttta atttatttag ctttccctgt 120
agctaatatt tcatgctgaa cacattttaa atgctgtaaa tgtagataat gtaatttatg 180
tatcattaat gcctctttag tagtttagag aaaacgtcaa aagaaatggc cccagaataa 240
gcttcttgat ttgtaaaatt ctatgtcatt ggctcaaatt tgtatagtat ctcaaaatat 300
aaatatatag acatctcaga taatatattt gaaatagcaa attcctgtta gaaaataata 360
gtacttaact agatgagaat aacaggtcgc cattatttga attgtctcct attcgttttt 420
catttgttgt gttactcatg ttttacttat gggggatat atataacttc cgctgttttc 480
agaagtattg tatgcagtca gtatgagaat gcaatttaag tttccttgat gctttttcac 540
acttctatta ctagaaataa gaatacagta atattggcaa agaaaattga ccagttcaat 600
aaaatttttt agtaaatctg attgaaaata aacattgctt atggctttct tacatcaata 660
ttgttatgtc ctagacacct tatctgaaat tacggcttca aaattctaat tatgtgcaaa 720
tgtgtaaaat atcaatactt tatgttcaag ctggggcctc ttcaggcgtc ctgggctgag 780
agagaaagat gctagctccg caagccgggg agggaacacc gccacattgt tacatggaca 840
caccgccacg tggacacatg accagactca catgtacaga cacacggaga cattaccaca 900
tggagacacc gtcacacagt cacacgagca cactggcata gtcacatgga cggacacaca 960
gacatatgga gaaatcacac tgacacacca ccacactatc acagggacac agacacacgg 1020
agacatcacc acatggacac actgtcacac taccacaggg acacgagaca tcacactgtc 1080
acatggacac accatcacac acatgaacac accgacacac tgccatatgg acactgccac 1140
acacactgcc acactgtcac atggacacac ctccatacca tcacaccacc acacacactg 1200
ccatgtggac acaaggacac acagacactg tcacacagat acacaaaaca ctgtcacacg 1260
gagacatcac catgcagata caccaccaca tggacatagc accagacact ctgccacaca 1320

gatacaccac cacacagaaa tgcggacaca ctgccacaca gacaccacca catcgttgcc 1380
acactttcat gtgtcagctg gcggtgtggg ccccacgact ctgggctcta atcgagaaat 1440
tacttggaca tatagtgaag gcaaaatttt tttttatttt ctgggtaacc aagcgcgact 1500
ctgtctcaaa aaaagaaaaa aaaagcaata tactgtgtaa tcgttgacag cataattcac 1560
tattatgtag atcggagagc agaggattct gaatgcatga acatatcatt aacatttcaa 1620
tacattactc ataattactg atgaactaaa gagaaaccaa gaaattatgg tgatagttat 1680
attgacctgg agaaatgtag acacaaaaga accgtaagat gagaaatgtg ttaacacagt 1740
ctataagggc atgcaagaat aaaaataggg gagaaaacag gagagttttt caagagcttt 1800
ctggtcatgt aagtcaactt gtatcggtta atttttaaaa ggtttattta catgcaataa 1860
actgcacata cttcaattgt acattttggt aattcttggc atttgtagct ctataaaacc 1920
agcaacatat taaaatagca aacatatcca ttacctttac caccaaagtt ttcttgtgtt 1980
ttttctactc actttttcct gcctatcccc ccatctcttc cacaggtaac cactgatcca 2040
cttccagtca ctatccatga gtttttattt ccaaatacat gaaatcatat gaatttctgg 2100
tttttcctgt tggagcccaa ggagcaaggg cagaatgagg aacatgatgt ttcttwccga 2160
cagttactca tgacgtctcc atccaggact gaggggggca tccttctcca tctaggactg 2220
ggggcatcct tctccatcca gtattggggg tcatccttct ccatccagta ttgggggtca 2280
tcctcctcca tccaggacct gaggggtgtc cttttctgcg cttccttgga tggcagtctt 2340
tcccttcatg tttatagtra cttaccatta aatcactgtg ccgttttttc ctaaaataaa 2400
aaaaaaaaaa aaaa 2414

<210> 476
<211> 3434
<212> DNA
<213> Homo sapiens

<400> 476
   ctgtgctgca aatggggcca tatagaggaa aggagcagct ggctctggag catggtgtgc 60
   actccctttg ggccttcagt ccatgtctca tgggtcgtat gacactgcgg gcttgttggt 120
   tgccaagagg cagaccacag gtcatcttga ggaggacttt atgttccagt ccagaaagca 180
   gccagtggta ccacccaggg gacttgtgct tctgtggccc aggccagacg tagaatttga 240
   caaagtcagg acggtctcag tcagagcagc atgtcggtcc ccggggcctg tgcatgccgg 300
   gcagggccag gctggcttaa ggagcaagca gccacctctg ttaggggtgt gcctggagca 360
   ggtggagcag ccaccaacct cacgcactga aagaagcagg gatggccagg ttccaacatc 420
   ctgagtggct gccacctgat ggctgatgga gcagaggcct gaggaaaagc agatggcact 480
   gctttgtagt gctgttcttt gtctctcttg atctttttca gttaatgtct gttttatcag 540
   agactaggat tgcaaaccct gctctttttt gctttccatt tgcttggtaa atattcctcc 600
   atccctttat tttaagccta tgtgtgtctt tgcacatgag atgggtctcc tgaatacagg 660
   acaacaatgg gtctttactc tttatccaac ttgccagtct gtgtctttta actggggcat 720
   ttagcccatt tacatttaag tttagtattt gttacatgtg aaatttatcc tgtcatgatg 780
   ttgctagctt ttttattttc ccattagttt gcagtttctt tatagtgtca atggtcttta 840
   caattcgata tgtttttgta gtggctggta ctggtttttc ctttctacgt ttagtgtctc 900
   cttcaggagc tcttgtaaca caagaatgtg gatttatttc ttgtaaggta aatatgtgga 960
   tttattctgg gactgtattc tatggccttt accccaagaa tcattacttt ttaaaatgca 1020
   attcaaatta gcataaaaca tttacagcct atggaaaggc ttgtggcatt agaatcctta 1080
   tttataggat tattttgtgt ttttttgaga tatggtcttt gtcatcgagg cagaagtgcc 1140
   gtggtttgat cataattcac cacagccctg aactcttgag tccaagccat ccttttgcct 1200
   taatctccca accagttgga tctacaagca taaggcatca tgcgtggcta attttttcac 1260
   gttttttttt tttttgtcga gattatggta tcactgtgtt gctctggctg atctcaaatg 1320
   tttgacctca agggatcttt ctgccacagc ctcctaaagt gctaggatta tatgcatgat 1380
   acaccatgcc tattgtagag tattacatta ttttcaaagt cttattgtaa gagccattta 1440
   ttgcctttgg cctaaataac tcaatataat atctctgaaa cttttttttg acaaattttg 1500
   gggcgtgatg atgagagaag ggggtttgaa actttctaat aagagttaac ttagagccat 1560
   ttaagaaagg aaaaaacaca aattatcaga aaaacaacag taagatcaag tgcaaaagtt 1620
   ctgtggcaaa gatgatgaga gtaaagaata tatgtttgtg actcatggtg gcttttactt 1680
   tgttcttgaa tttctgagta cgggttaaca tttaaagaat ctacattata gataacattt 1740
   tattgcaagt aaatgtattt caaaatttgt tattggtttt gtatgagatt attctcagcc 1800
   tacttcatta tcaagctata ttattttatt aatgtagttc gatgatctta cagcaaagct 1860
   gaaagctgta tcttcaaaat atgtctattt gactaaaaag ttattcaaca ggagttatta 1920
   tctataaaaa aatacaacag gaatataaaa aacttgagga taaaaagatg ttggaaaaag 1980
   taatattaaa tcttaaaaaa catatggaaa ctacacaatg gtgaagacac attggtgaag 2040
   tacaaaaata taaattggat ctagaagaaa gggcaatgca ggcaatagaa aaattagtag 2100
   aaatcccttt aaaggttagt ttgtaaaatc aggtaagttt atttataatt tgctttcatt 2160
   tatttcactg caaattatat tttggatatg tatatatatt gtgcttcctc tgcctgtctt 2220
   acagcaattt gccttgcaga gttctaggaa aaaggtggca tgtgttttta ctttcaaaat 2280
   atttaaattt ccatcattat aacaaaatca atttttcaga gtaatgattc tcactgtgga 2340
   gtcatttgat tattaagacc cgttggcata agattacatc ctctgactat aaaaatcctg 2400
   gaagaaaacc taggaaatat tcgtctggac attgcacttg gcaatgaatt tatgggcgct 2460
   ttggaatcct gcagatataa taatgataat taaacaaaac actcagagaa actgccaacc 2520
   ctaggatgaa gtatattgtt actgtgcttt gggattaaaa taagtaacta cagtttatag 2580
   aacttttata ctgatacaca gacactaaaa agggaaaggg tttagatgag aagctctgct 2640
   atgcaatcaa gaatctcagc cactcatttc tgtaggggct gcaggagctc cctgtaaaga 2700
   gaggttatgg agtctgtagc ttcaggtaag atacttaaaa cccttcagag tttctccatt 2760
   ttttcccata gtttccccaa aaaggttatg acactttata agaatgcttc acttgtgaaa 2820
   aacaaatatc aaagtcttct tgtagattat ttttaaggac aaatctttat tccatgttta 2880
   atttatttag ctttccctgt agctaatatt tcatgctgaa cacattttaa atgctgtaaa 2940
   tgtagataat gtaatttatg tatcattaat gcctctttag tagtttagag aaaacgtcaa 3000
   aagaaatggc cccagaataa gcttcttgat ttgtaaaatt ctatgtcatt ggctcaaatt 3060
   tgtatagtat ctcaaaatat aaatatatag acatctcaga taatatattt gaaatagcaa 3120
   attcctgtta gaaaataata gtacttaact agatgagaat aacaggtcgc cattatttga 3180
   attgtctcct attcgttttt catttgttgt gttactcatg ttttacttat ggggggatat 3240
   atataacttc cgctgttttc agaagtattg tatgcagtca gtatgagaat gcaatttaag 3300
   tttccttgat gctttttcac acttctatta ctagaaataa gaatacagta atattggcaa 3360
   agaaaattga ccagttcaat aaaatttttt agtaaatctg attgaaaata aaaaaaaaaa 3420
   aaaaaaaaaa aaaa 3434

<210> 477
<211> 140
<212> PRT
<213> Homo sapiens

<400> 477
Met Asp Gly His Thr Asp Ile Trp Arg Asn His Met Asp Thr Pro Pro
1               5                   10                  15
His Tyr His Arg Asp Thr Asp Thr Arg Arg His His His Met Asp Thr
            20                  25                  30
Leu Ser His Tyr His Arg Asp Thr Arg His His Thr Val Thr Trp Thr
        35                  40                  45
His His His Thr His Glu His Thr Asp Thr Leu Pro Tyr Gly His Trp
    50                  55                  60
His Thr His Cys His Thr Val Thr Trp Thr His Leu His Thr Ile Thr
65                  70                  75                  80
Pro Pro His Thr Leu Pro Val Asp Thr Arg Thr His Arg His Cys His
                85                  90                  95
Thr Asp Thr Gln Asn Thr Val Thr Arg Arg His His His Ala Asp Thr
            100                 105                 110
Pro Pro Leu Trp Cys Arg Leu Asn Tyr Pro Ala Gly Gly Thr Ala Val
        115                 120                 125
Ala Tyr Ser Cys Leu Ser Asp Trp Leu Ser Pro Gln
    130                 135                 140

<210> 478
<211> 143
<212> PRT
<213> Homo sapiens

<400> 478
Met Tyr Arg His Thr Glu Thr Leu Pro His Gly Asp Thr Val Thr Gln
1               5                   10                  15
Ser His Gly His Thr Gly Ile Val Thr Trp Thr Asp Thr Gln Thr Tyr
            20                  25                  30
Gly Glu Ile Thr Trp Thr His His His Thr Ile Thr Gly Thr Gln Thr
        35                  40                  45
His Gly Asp Ile Thr Thr Trp Thr His Cys His Thr Thr Thr Gly Thr
    50                  55                  60
Arg Asp Ile Thr Leu Ser His Gly His Thr Ile Thr His Met Asn Thr
65                  70                  75                  80
Pro Thr His Cys His Met Asp Thr Gly Thr His Thr Ala Thr Leu Ser
                85                  90                  95
His Gly His Thr Ser Thr Pro Ser His His His Thr His Cys Leu Trp
            100                 105                 110
Thr Gln Gly His Thr Asp Thr Val Thr Gln Ile His Lys Thr Leu Ser
        115                 120                 125
His Gly Asp Ile Thr Met Gln Ile His His His Ser Gly Ala Val
    130                 135                 140

<210> 479
<211> 222
<212> PRT
<213> Homo sapiens

<400> 479
Met Tyr Arg His Thr Glu Thr Leu Pro His Gly Asp Thr Val Thr Gln
1               5                   10                  15
Ser His Glu His Thr Gly Ile Val Thr Trp Thr Asp Thr Gln Thr Tyr
            20                  25                  30
Gly Glu Ile Thr Leu Thr His His His Thr Ile Thr Gly Thr Gln Thr
        35                  40                  45
His Gly Asp Ile Thr Thr Trp Thr His Cys His Thr Thr Thr Gly Thr
    50                  55                  60
Arg Asp Ile Thr Leu Ser His Gly His Thr Ile Thr His Met Asn Thr
65                  70                  75                  80
Pro Thr His Cys His Met Asp Thr Ala Thr His Thr Ala Thr Leu Ser
                85                  90                  95
His Gly His Thr Ser Ile Pro Ser His His His Thr His Cys His Val
            100                 105                 110
Asp Thr Arg Thr His Arg His Cys His Thr Asp Thr Gln Asn Thr Val
        115                 120                 125
Thr Arg Arg His His His Ala Asp Thr Pro Pro His Gly His Ser Thr
    130                 135                 140
Arg His Ser Ala Thr Gln Ile His His His Thr Glu Met Arg Thr His
145                 150                 155                 160
Cys His Thr Asp Thr Thr Thr Ser Leu Pro His Phe His Val Ser Ala
                165                 170                 175
Gly Gly Val Gly Pro Thr Thr Leu Gly Ser Asn Arg Glu Ile Thr Trp
            180                 185                 190
Thr Tyr Ser Glu Gly Lys Ile Phe Phe Tyr Phe Leu Gly Asn Gln Ala
        195                 200                 205
Arg Leu Cys Leu Lys Lys Arg Lys Lys Lys Gln Tyr Thr Val
    210                 215                 220


<210> 480
<211> 144
<212> PRT
<213> Homo sapiens

<400> 480


<210> 481
<211> 167
<212> PRT
<213> Homo sapiens

<400> 481
Met His Gly Pro Gln Val Leu Ala Arg Cys Ser Glu Cys Ala Cys Pro
1               5                   10                  15
Ala Leu Ala Ala Thr Ser Ala Gly Val Arg Leu Glu Gly Val Asp Arg
            20                  25                  30
Pro Pro Thr Leu Pro Ser Gln Gly Ser Gly Trp Pro Cys Ser His Ser
        35                  40                  45
Leu Ser Gly Cys His Leu Met Ala Asp Gly Ala Lys Ala Leu Gly Lys
    50                  55                  60
Ala Asp Gly Pro Trp Pro Tyr Leu Phe Val Arg Arg Thr Asp Val Pro
65                  70                  75                  80
Cys Pro Ala Ala Ser Glu Val Gly Gly Cys Ala Pro Ser Ser Trp Arg
                85                  90                  95
Ala Leu Ala Glu Val Thr Gly Cys Ser Leu Gly Pro Leu Gly Leu Ala
            100                 105                 110
Gln His Ala Gln Ala Ser Val Leu Leu Leu Cys Tyr Lys Trp Ser His
        115                 120                 125
Ile Gly Glu Thr Ser Ser His Leu Arg Ser Lys Val Tyr Ala Ala Phe
    130                 135                 140
Gly Gly Ser Ser Pro Cys Leu Lys Gly Leu Met Ser Leu Trp Ala Ser
145                 150                 155                 160
Trp Leu Ser Arg Gly Arg Pro
                165

<210> 482
<211> 143
<212> PRT
<213> Homo sapiens

<400> 482

<210> 483
<211> 143
<212> PRT
<213> Homo sapiens

<400> 483
Met Glu Thr Gln Arg Gly Asn Lys Gln Arg Ala Gln Glu Gln Gly Val
1               5                   10                  15
Cys Cys Leu Trp Gly Ser Ser Pro Cys Leu Gly Ser Tyr Gly Thr Ala
            20                  25                  30
Gly Phe Leu Val Ala Lys Arg Arg Thr Thr Gly Leu Leu Glu Glu Asp
        35                  40                  45
Phe Thr Phe Lys Cys Arg Lys Gln Pro Lys Leu Pro Ser Met Arg Leu
    50                  55                  60
Ser Leu Leu Trp Pro Trp Arg Asp Leu Lys Phe Val Pro Arg Gln Asp
65                  70                  75                  80
Lys Leu Thr Arg Ser Ser Val Ser Val Ala Gly Ala Tyr Ala Cys Arg

85                  90                  95
Ala Gly Pro Gly Trp Leu Lys Glu Gln Pro Ala Thr Ser Ala Arg Val
            100                 105                 110
Arg Leu Val Gln Ala Glu His Pro Pro Pro His Pro Leu Glu Glu Val
        115                 120                 125
Gly Met Ala Arg Phe Pro Gln Pro Glu Cys Leu Pro Pro Tyr Cys
    130                 135                 140

<210> 484
<211> 30
<212> PRT
<213> Homo sapiens

<400> 484
Thr Ala Ala Ser Asp Asn Phe Gln Leu Ser Gln Gly Gly Gln Gly Phe
1               5                   10                  15
Ala Ile Pro Ile Gly Gln Ala Met Ala Ile Ala Gly Gln Ile
            20                  25                  30

<210> 485
<211> 31
<212> DNA
<213> Artificial Sequence

<220>
<223> Made in a lab

<400> 485
gggaagctta tcacctatgt gccgcctctg c                                31

<210> 486
<211> 27
<212> DNA
<213> Artificial Sequence

<220>
<223> Made in a lab

<400> 486
gcgaattctc acgctgagta tttggcc                                     27

<210> 487
<211> 36
<212> DNA
<213> Artificial Sequence

<220>
<223> Made in a lab

<400> 487
cccgaattct tagctgccca tccgaacgcc ttcatc                           36

<210> 488
<211> 33
<212> DNA
<213> Artificial Sequence

<220>
<223> Made in a lab

<400> 488
gggaagcttc ttccccggct gcaccagctg tgc                              33

<210> 489
<211> 19
<212> PRT

<213> Artificial Sequence

<220>
<223> Made in a lab

<400> 489
Met Asp Arg Leu Val Gln Arg Phe Gly Thr Arg Ala Val Tyr Leu Ala
1               5                   10                  15
Ser Val Ala

<210> 490
<211> 20
<212> PRT
<213> Artificial Sequence

<220>
<223> Made in a lab

<400> 490
Tyr Leu Ala Ser Val Ala Ala Phe Pro Val Ala Ala Gly Ala Thr Cys
1               5                   10                  15
Leu Ser His Ser
            20

<210> 491
<211> 20
<212> PRT
<213> Artificial Sequence

<220>
<223> Made in a lab

<400> 491
Thr Cys Leu Ser His Ser Val Ala Val Val Thr Ala Ser Ala Ala Leu
1               5                   10                  15
Thr Gly Phe Thr
            20

<210> 492
<211> 20
<212> PRT
<213> Artificial Sequence

<220>
<223> Made in a lab

<400> 492
Ala Leu Thr Gly Phe Thr Phe Ser Ala Leu Gln Ile Leu Pro Tyr Thr
1               5                   10                  15
Leu Ala Ser Leu
            20

<210> 493
<211> 20
<212> PRT
<213> Artificial Sequence

<220>
<223> Made in a lab

<400> 493
Tyr Thr Leu Ala Ser Leu Tyr His Arg Glu Lys Gln Val Phe Leu Pro
1               5                   10                  15

Lys Tyr Arg Gly
            20


<210> 494
<211> 20
<212> PRT
<213> Artificial Sequence

<220>
<223> Made in a lab

<400> 494
Leu Pro Lys Tyr Arg Gly Asp Thr Gly Gly Ala Ser Ser Glu Asp Ser
 1               5                   10                  15
Leu Met Ile Ser
            20


<210> 495
<211> 20
<212> PRT
<213> Artificial Sequence

<220>
<223> Made in a lab

<400> 495
Asp Ser Leu Met Thr Ser Phe Leu Pro Gly Pro Lys Pro Gly Ala Pro
 1               5                   10                  15
Phe Pro Asn Gly
            20


<210> 496
<211> 21
<212> PRT
<213> Artificial Sequence

<220>
<223> Made in a lab

<400> 496
Ala Pro Phe Pro Asn Gly His Val Gly Ala Gly Gly Ser Gly Leu Leu
 1               5                   10                  15
Pro Pro Pro Pro Ala
            20


<210> 497
<211> 20
<212> PRT
<213> Artificial Sequence

<220>
<223> Made in a lab

<400> 497
Leu Leu Pro Pro Pro Pro Ala Leu Cys Gly Ala Ser Ala Cys Asp Val
 1               5                   10                  15
Ser Val Arg Val
            20


<210> 498
<211> 20
<212> PRT
<213> Artificial Sequence

<220>
<223> Made in a lab

<400> 498
Asp Val Ser Val Arg Val Val Val Gly Glu Pro Thr Glu Ala Arg Val
1               5                   10                  15
Val Pro Gly Arg
            20


<210> 499
<211> 20
<212> PRT
<213> Artificial Sequence

<220>
<223> Made in a lab

<400> 499
Arg Val Val Pro Gly Arg Gly Ile Cys Leu Asp Leu Ala Ile Leu Asp
1               5                   10                  15
Ser Ala Phe Leu
            20


<210> 500
<211> 20
<212> PRT
<213> Artificial Sequence

<220>
<223> Made in a lab

<400> 500
Leu Asp Ser Ala Phe Leu Leu Ser Gln Val Ala Pro Ser Leu Phe Met
1               5                   10                  15
Gly Ser Ile Val
            20


<210> 501
<211> 20
<212> PRT
<213> Artificial Sequence

<220>
<223> Made in a lab

<400> 501
Phe Met Gly Ser Ile Val Gln Leu Ser Gln Ser Val Thr Ala Tyr Met
1               5                   10                  15
Val Ser Ala Ala
            20


<210> 502
<211> 414
<212> DNA
<213> Homo sapiens

<220>
<221> misc_feature
<222> 116, 139, 226, 253, 256, 270, 397
<223> n = A,T,C or G

<400> 502
caccatggag acaggcctgc gctggctttt cctggtcgct gtgctcaaag gtgtccaatg 60

tcagtcggtg gaggagtccg ggggtcgcct ggtcacgcct gggacacctt tgacantcac 120
ctgtagagtt tttggaatng acctcagtag caatgcaatg agctgggtcc gccaggctcc 180
agggaagggg ctggaatgga tcggagccat tgataattgt ccacantacg cgacctgggc 240
gaaaggccga ttnatnattt ccaaaacctn gaccacggtg gatttgaaaa tgaccagtcc 300
gacaaccgag gacacggcca cctatttttg tggcagaatg aatactggta atagtggttg 360
gaagaatatt tggggcccag gcaccctggt caccgtntcc tcagggcaac ctaa 414

<210> 503
<211> 379
<212> DNA
<213> Homo sapiens

<220>
<221> misc_feature
<222> 3, 102, 111, 167, 244, 302, 362
<223> n = A,T,C or G

<400> 503
atncgatggt gcttggtcaa aggtgtccag tgtcagtcgg tggaggagtc cgggggtcgc 60
ctggtcacgc ctgggacacc cctgacactc acctgcaccg tntctggatt ngacatcagt 120
agctatggag tgagctgggt ccgccaggct ccagggaagg ggctggnata catcggatca 180
ttagtagtag tggtacattt tacgcgagct gggcgaaagg ccgattcacc atttccaaaa 240
cctngaccac ggtggatttg aaaatcacca gtttgacaac cgaggacacg gccacctatt 300
tntgtgccag aggggggttt aattataaag acatttgggg cccaggcacc ctggtcaccg 360
tntccttagg gcaacctaa 379

<210> 504
<211> 19
<212> PRT
<213> Artificial Sequence

<220>
<223> Made in a lab

<400> 504
Gly Phe Thr Asn Tyr Thr Asp Phe Glu Asp Ser Pro Tyr Phe Lys Glu
1               5                   10                  15
Asn Ser Ala

<210> 505
<211> 20
<212> PRT
<213> Artificial Sequence

<220>
<223> Made in a lab

<400> 505
Lys Glu Asn Ser Ala Phe Pro Pro Phe Cys Cys Asn Asp Asn Val Thr
1               5                   10                  15
Asn Thr Ala Asn
            20

<210> 506
<211> 407
<212> DNA
<213> Homo sapiens

<400> 506
atggagacag gcctgcgctg gcttctcctg gtcgctgcgc tcaaaggtgt ccagtgtcag 60
tcgctggagg agtccggggg tcgcctggtc acgcctggga cacccctgac actcacctgc 120
accgtctctg gattctccct cagtagcaat gcaatgatct gggtccgcca ggctccaggg 180
aaggggctgg aatacatcgg atacattagt tatggtggta gcgcatacta cgcgagctgg 240
gtgaaaggcc gattcaccat ctccaaaacc tcgaccacgg tggatctgag aatgaccagt 300
ctgacaaccg aggacacggc cacctatttc tgtgccagaa atagtgattt tagtggtatg 360

ttgtggggcc caggcaccct ggtcaccgtc tcctcagggc aacctaa                407

<210> 507
<211> 422
<212> DNA
<213> Homo sapiens

<400> 507
atggagacag gcctgcgctg gcttctcctg gtcgctgtgc tcaaaggtgt ccagtgtcag 60
tcggtggagg agtccggggg tcgcctggtc acgcctggga cacccctgac actcacctgt 120
acagtctctg gattctccct cagcaactac gacctgaact gggtccgcca ggctccaggg 180
aaggggctgg aatggatcgg gatcattaat tatgttggta ggacggacta cgcgaactgg 240
gcaaaaggcc ggttcaccat ctccaaaacc tcgaccaccg tggatctcaa gatcgccagt 300
ccgacaaccg aggacacggc cacctatttc tgtgccagag ggtggaagtg cgatgagtct 360
ggtccgtgct tgcgcatctg gggcccaggc accctggtca ccgtctcctt agggcaacct 420
aa                                                                 422

<210> 508
<211> 411
<212> DNA
<213> Homo sapiens

<220>
<221> misc_feature
<222> 285, 294
<223> n = A,T,C or G

<400> 508
atggagacag gcctcgctgg cttctcctgg tcgctgtgct caaaggtgtc cagtgtcagt 60
cggtggagga gtccgggggt cgcctggtca cgcctgggac acccctgaca ctcacctgca 120
cagtctctgg aatcgacctc agtagctact gcatgagctg ggtccgccag gctccaggga 180
aggggctgga atggatcgga atcattggta ctcctggtga cacatactac gcgaggtggg 240
cgaaaggccg attcaccatc tccaaaacct cgaccacggt gcatntgaaa atcnccagtc 300
cgacaaccga ggacacggcc acctatttct gtgccagaga tcttcgggat ggtagtagta 360
ctggttatta taaaatctgg ggcccaggca ccctggtcac cgtctccttg g          411

<210> 509
<211> 15
<212> PRT
<213> Artificial Sequence

<220>
<223> Made in a lab

<400> 509
Leu Cys Lys Phe Thr Glu Trp Ile Glu Lys Thr Val Gln Ala Ser
 1               5                   10                  15

<210> 510
<211> 15
<212> PRT
<213> Artificial Sequence

<220>
<223> Made in a lab

<400> 510
Pro Glu Tyr Asn Arg Pro Leu Leu Ala Asn Asp Leu Met Leu Ile
 1               5                   10                  15

<210> 511
<211> 15
<212> PRT
<213> Artificial Sequence

<220>

<223> Made in a lab

<400> 511
Tyr His Pro Ser Met Phe Cys Ala Gly Gly Gly Gln Asp Gln Lys
1               5                   10                  15


<210> 512
<211> 15
<212> PRT
<213> Artificial Sequence

<220>
<223> Made in a lab

<400> 512
Asp Ser Gly Gly Pro Leu Ile Cys Asn Gly Tyr Leu Gln Gly Leu
1               5                   10                  15


<210> 513
<211> 15
<212> PRT
<213> Artificial Sequence

<220>
<223> Made in a lab

<400> 513
Ala Pro Cys Gly Gln Val Gly Val Pro Asx Val Tyr Thr Asn Leu
1               5                   10                  15


<210> 514
<211> 15
<212> PRT
<213> Artificial Sequence

<220>
<223> Made in a lab

<400> 514
Leu Cys Lys Phe Thr Glu Trp Ile Glu Lys Thr Val Gln Ala Ser
1               5                   10                  15


<210> 515
<211> 15
<212> PRT
<213> Artificial Sequence

<220>
<223> Made in a lab

<400> 515
Met Val Glu Ala Ser Leu Ser Val Arg His Pro Glu Tyr Asn Arg
1               5                   10                  15


<210> 516
<211> 15
<212> PRT
<213> Artificial Sequence

<220>
<223> Made in a lab

<400> 516

Val Ser Glu Ser Asp Thr Ile Arg Ser Ile Ser Ile Ala Ser Gln
1               5                   10                  15

<210> 517
<211> 15
<212> PRT
<213> Artificial Sequence

<220>
<223> Made in a lab

<400> 517
Glu Val Cys Ser Lys Leu Tyr Asp Pro Leu Tyr His Pro Ser Met
1               5                   10                  15

<210> 518
<211> 15
<212> PRT
<213> Artificial Sequence

<220>
<223> Made in a lab

<400> 518
Arg Ala Glu Pro Gly Thr Glu Ala Arg Arg His Tyr Asp Glu Gly
1               5                   10                  15

<210> 519
<211> 17
<212> PRT
<213> Artificial Sequence

<220>
<223> Made in a lab

<400> 519
Arg Ala Glu Pro Gly Thr Glu Ala Arg Arg Asn Tyr Asp Glu Gly Cys
1               5                   10                  15
Gly

<210> 520
<211> 25
<212> PRT
<213> Artificial Sequence

<220>
<223> Made in a lab

<400> 520
Val Gly Glu Gly Leu Tyr Gln Gly Val Pro Arg Ala Glu Pro Gly Thr
1               5                   10                  15
Glu Ala Arg Arg His Tyr Asp Glu Gly
            20                  25

<210> 521
<211> 21
<212> PRT
<213> Artificial Sequence

<220>
<223> Made in a lab

<400> 521
Ala Pro Phe Pro Asn Gly His Val Gly Ala Gly Gly Ser Gly Leu Leu
1               5                   10                  15
Pro Pro Pro Pro Ala
                20

<210> 522
<211> 20
<212> PRT
<213> Artificial Sequence

<220>
<223> Made in a lab

<400> 522
Leu Leu Val Val Pro Ala Ile Lys Lys Asp Tyr Gly Ser Gln Glu Asp
1               5                   10                  15
Phe Thr Gln Val
                20

<210> 523
<211> 254
<212> PRT
<213> Artificial Sequence

<220>
<223> Made in a lab

<221> VARIANT
<222> 198, 200
<223> Xaa = Any Amino Acid

<400> 523
Met Ala Thr Ala Gly Asn Pro Trp Gly Trp Phe Leu Gly Tyr Leu Ile
1               5                   10                  15
Leu Gly Val Ala Gly Ser Leu Val Ser Gly Ser Cys Ser Gln Ile Ile
            20                  25                  30
Asn Gly Glu Asp Cys Ser Pro His Ser Gln Pro Trp Gln Ala Ala Leu
        35                  40                  45
Val Met Glu Asn Glu Leu Phe Cys Ser Gly Val Leu Val His Pro Gln
    50                  55                  60
Trp Val Leu Ser Ala Thr His Cys Phe Gln Asn Ser Tyr Thr Ile Gly
65                  70                  75                  80
Leu Gly Leu His Ser Leu Glu Ala Asp Gln Glu Pro Gly Ser Gln Met
                85                  90                  95
Val Glu Ala Ser Leu Ser Val Arg His Pro Glu Tyr Asn Arg Pro Leu
            100                 105                 110
Leu Ala Asn Asp Leu Met Leu Ile Lys Leu Asp Glu Ser Val Ser Glu
        115                 120                 125
Ser Asp Thr Ile Arg Ser Ile Ser Ile Ala Ser Gln Cys Pro Thr Ala
    130                 135                 140
Gly Asn Ser Cys Leu Val Ser Gly Trp Gly Leu Leu Ala Asn Gly Arg
145                 150                 155                 160
Met Pro Thr Val Leu Gln Cys Val Asn Val Ser Val Val Ser Glu Glu
                165                 170                 175
Val Cys Ser Lys Leu Tyr Asp Pro Leu Tyr His Pro Ser Met Phe Cys
            180                 185                 190
Ala Gly Gly Gly Gln Xaa Gln Xaa Asp Ser Cys Asn Gly Asp Ser Gly
        195                 200                 205
Gly Pro Leu Ile Cys Asn Gly Tyr Leu Gln Gly Leu Val Ser Phe Gly
    210                 215                 220
Lys Ala Pro Cys Gly Gln Val Gly Val Pro Gly Val Tyr Thr Asn Leu
225                 230                 235                 240
Cys Lys Phe Thr Glu Trp Ile Glu Lys Thr Val Gln Ala Ser
                245                 250

<210> 524
<211> 765
<212> DNA
<213> Homo sapiens

<400> 524
atggccacag caggaaatcc ctggggctgg ttcctggggt acctcatcct tggtgtcgca 60
ggatcgctcg tctctggtag ctgcagccaa atcataaacg gcgaggactg cagcccgcac 120
tcgcagccct ggcaggcggc actggtcatg gaaaacgaat tgttctgctc gggcgtcctg 180
gtgcatccgc agtgggtgct gtcagccgca cactgtttcc agaactccta caccatcggg 240
ctgggcctgc acagtcttga ggccgaccaa gagccaggga gccagatggt ggaggccagc 300
ctctccgtac ggcacccaga gtacaacaga cccttgctcg ctaacgacct catgctcatc 360
aagttggacg aatccgtgtc cgagtctgac accatccgga gcatcagcat tgcttcgcag 420
tgccctaccg cggggaactc ttgcctcgtt tctggctggg gtctgctggc gaacggcaga 480
atgcctaccg tgctgcagtg cgtgaacgtg tcggtggtgt ctgaggaggt ctgcagtaag 540
ctctatgacc cgctgtacca ccccagcatg ttctgcgccg gcggagggca agaccagaag 600
gactcctgca acggtgactc tggggggccc ctgatctgca acgggtactt gcagggcctt 660
gtgtctttcg gaaaagcccc gtgtggccaa gttggcgtgc caggtgtcta caccaacctc 720
tgcaaattca ctgagtggat agagaaaacc gtccaggcca gttaa 765

<210> 525
<211> 254
<212> PRT
<213> Homo sapiens

<400> 525
Met Ala Thr Ala Gly Asn Pro Trp Gly Trp Phe Leu Gly Tyr Leu Ile
1               5                   10                  15
Leu Gly Val Ala Gly Ser Leu Val Ser Gly Ser Cys Ser Gln Ile Ile
            20                  25                  30
Asn Gly Glu Asp Cys Ser Pro His Ser Gln Pro Trp Gln Ala Ala Leu
        35                  40                  45
Val Met Glu Asn Glu Leu Phe Cys Ser Gly Val Leu Val His Pro Gln
    50                  55                  60
Trp Val Leu Ser Ala Ala His Cys Phe Gln Asn Ser Tyr Thr Ile Gly
65                  70                  75                  80
Leu Gly Leu His Ser Leu Glu Ala Asp Gln Glu Pro Gly Ser Gln Met
                85                  90                  95
Val Glu Ala Ser Leu Ser Val Arg His Pro Glu Tyr Asn Arg Pro Leu
            100                 105                 110
Leu Ala Asn Asp Leu Met Leu Ile Lys Leu Asp Glu Ser Val Ser Glu
        115                 120                 125
Ser Asp Thr Ile Arg Ser Ile Ser Ile Ala Ser Gln Cys Pro Thr Ala
    130                 135                 140
Gly Asn Ser Cys Leu Val Ser Gly Trp Gly Leu Leu Ala Asn Gly Arg
145                 150                 155                 160
Met Pro Thr Val Leu Gln Cys Val Asn Val Ser Val Val Ser Glu Glu
                165                 170                 175
Val Cys Ser Lys Leu Tyr Asp Pro Leu Tyr His Pro Ser Met Phe Cys
            180                 185                 190
Ala Gly Gly Gly Gln Asp Gln Lys Asp Ser Cys Asn Gly Asp Ser Gly
        195                 200                 205
Gly Pro Leu Ile Cys Asn Gly Tyr Leu Gln Gly Leu Val Ser Phe Gly
    210                 215                 220
Lys Ala Pro Cys Gly Gln Val Gly Val Pro Gly Val Tyr Thr Asn Leu
225                 230                 235                 240
Cys Lys Phe Thr Glu Trp Ile Glu Lys Thr Val Gln Ala Ser
                245                 250

<210> 526
<211> 963
<212> DNA
<213> Homo sapiens

<400> 526
   atgagttcct gcaacttcac acatgccacc tttgtgctta ttggtatccc aggattagag 60
   aaagcccatt tctgggttgg cttccccctc ctttccatgt atgtagtggc aatgtttgga 120
   aactgcatcg tggtcttcat cgtaaggacg gaacgcagcc tgcacgctcc gatgtacctc 180
   tttctctgca tgcttgcagc cattgacctg gccttatcca catccaccat gcctaagatc 240
   cttgcccttt tctggtttga ttcccgagag attagctttg aggcctgtct tacccagatg 300
   ttctttattc atgccctctc agccattgaa tccaccatcc tgctggccat ggcctttgac 360
   cgttatgtgg ccatctgcca cccactgcgc catgctgcag tgctcaacaa tacagtaaca 420
   gcccagattg gcatcgtggc tgtggtccgc ggatccctct tttttttccc actgcctctg 480
   ctgatcaagc ggctggcctt ctgccactcc aatgtcctct cgcactccta ttgtgtccac 540
   caggatgtaa tgaagttggc ctatgcagac actttgccca atgtggtata tggtcttact 600
   gccattctgc tggtcatggg cgtggacgta atgttcatct ccttgtccta ttttctgata 660
   atacgaacgg ttctgcaact gccttccaag tcagagcggg ccaaggcctt tggaacctgt 720
   gtgtcacaca ttggtgtggt actcgccttc tatgtgccac ttattggcct ctcagttgta 780
   caccgctttg gaaacagcct tcatcccatt gtgcgtgttg tcatgggtga catctacctg 840
   ctgctgcctc ctgtcatcaa tcccatcatc tatggtgcca aaaccaaaca gatcagaaca 900
   cgggtgctgg ctatgttcaa gatcagctgt gacaaggact tgcaggctgt gggaggcaag 960
   tga 963

<210> 527
<211> 320
<212> PRT
<213> Homo sapiens

<400> 527
Met Ser Ser Cys Asn Phe Thr His Ala Thr Phe Val Leu Ile Gly Ile
1               5                   10                  15
Pro Gly Leu Glu Lys Ala His Phe Trp Val Gly Phe Pro Leu Leu Ser
            20                  25                  30
Met Tyr Val Val Ala Met Phe Gly Asn Cys Ile Val Val Phe Ile Val
        35                  40                  45
Arg Thr Glu Arg Ser Leu His Ala Pro Met Tyr Leu Phe Leu Cys Met
    50                  55                  60
Leu Ala Ala Ile Asp Leu Ala Leu Ser Thr Ser Thr Met Pro Lys Ile
65                  70                  75                  80
Leu Ala Leu Phe Trp Phe Asp Ser Arg Glu Ile Ser Phe Glu Ala Cys
                85                  90                  95
Leu Thr Gln Met Phe Phe Ile His Ala Leu Ser Ala Ile Glu Ser Thr
            100                 105                 110
Ile Leu Leu Ala Met Ala Phe Asp Arg Tyr Val Ala Ile Cys His Pro
        115                 120                 125
Leu Arg His Ala Ala Val Leu Asn Asn Thr Val Thr Ala Gln Ile Gly
    130                 135                 140
Ile Val Ala Val Val Arg Gly Ser Leu Phe Phe Phe Pro Leu Pro Leu
145                 150                 155                 160
Leu Ile Lys Arg Leu Ala Phe Cys His Ser Asn Val Leu Ser His Ser
                165                 170                 175
Tyr Cys Val His Gln Asp Val Met Lys Leu Ala Tyr Ala Asp Thr Leu
            180                 185                 190
Pro Asn Val Val Tyr Gly Leu Thr Ala Ile Leu Leu Val Met Gly Val
        195                 200                 205
Asp Val Met Phe Ile Ser Leu Ser Tyr Phe Leu Ile Ile Arg Thr Val
    210                 215                 220
Leu Gln Leu Pro Ser Lys Ser Glu Arg Ala Lys Ala Phe Gly Thr Cys
225                 230                 235                 240
Val Ser His Ile Gly Val Val Leu Ala Phe Tyr Val Pro Leu Ile Gly
                245                 250                 255
Leu Ser Val Val His Arg Phe Gly Asn Ser Leu His Pro Ile Val Arg
            260                 265                 270
Val Val Met Gly Asp Ile Tyr Leu Leu Leu Pro Pro Val Ile Asn Pro
        275                 280                 285
Ile Ile Tyr Gly Ala Lys Thr Lys Gln Ile Arg Thr Arg Val Leu Ala
    290                 295                 300
Met Phe Lys Ile Ser Cys Asp Lys Asp Leu Gln Ala Val Gly Gly Lys
305                 310                 315                 320

<210> 528
<211> 20
<212> DNA

<213> Homo sapiens

<400> 528
actatggtcc agaggctgtg                                                    20

<210> 529
<211> 20
<212> DNA
<213> Homo sapiens

<400> 529
atcacctatg tgccgcctct                                                    20

<210> 530
<211> 1852
<212> DNA
<213> Homo sapiens

<400> 530
ggcacgagaa ttaaaaccct cagcaaaaca ggcatagaag ggacatacct taaagtaata 60
aaaaccacct atgacaagcc cacagccaac ataatactaa atggggaaaa gttagaagca 120
tttcctctga gaactgcaac aataaataca aggatgctgg attttgtcaa atgccttttc 180
tgtgtctgtt gagatgctta tgtgactttg cttttaattc tgtttatgtg attatcacat 240
ttattgactt gcctgtgtta gaccggaaga gctggggtgt ttctcaggag ccaccgtgtg 300
ctgcggcagc ttcgggataa cttgaggctg catcactggg gaagaaacac aytcctgtcc 360
gtggcgctga tggctgagga cagagcttca gtgtggcttc tctgcgactg gcttcttcgg 420
ggagttcttc cttcatagtt catccatatg gctccagagg aaaattatat tattttgtta 480
tggatgaaga gtattacgtt gtgcagatat actgcagtgt cttcatctct tgatgtgtga 540
ttgggtaggt tccaccatgt tgccgcagat gacatgattt cagtacctgt gtctggctga 600
aaagtgtttg tttgtgaatg gatattgtgg tttctggatc tcatcctctg tgggtggaca 660
gctttctcca ccttgctgga agtgacctgc tgtccagaag tttgatggct gaggagtata 720
ccatcgtgca tgcatctttc atttcctgca tttcttcctc cctggatgga cagggggagc 780
ggcaagagca acgtgggcac ttctggagac cacaacgact cctctgtgaa gacgcttggg 840
agcaagaggt gcaagtggtg ctgccactgc ttcccctgct gcagggggag cggcaagagc 900
aacgtggtcg cttggggaga ctacgatgac agcgccttca tggatcccag gtaccacgtc 960
catggagaag atctggacaa gctccacaga gctgcctggt ggggtaaagt ccccagaaag 1020
gatctcatcg tcatgctcag ggacacggat gtgaacaaga gggacaagca aaagaggact 1080
gctctacatc tggcctctgc caatgggaat tcagaagtag taaaactcgt gctggacaga 1140
cgatgtcaac ttaatgtcct tgacaacaaa aagaggacag ctctgacaaa ggccgtacaa 1200
tgccaggaag atgaatgtgc gttaatgttg ctggaacatg gcactgatcc aaatattcca 1260
gatgagtatg gaaataccac tctacactat gctgtctaca atgaagataa attaatggcc 1320
aaagcactgc tcttatacgg tgctgatatc gaatcaaaaa acaagcatgg cctcacacca 1380
ctgctacttg gtatacatga gcaaaaacag caagtggtga aatttttaat caagaaaaaa 1440
gcgaatttaa atgcgctgga tagatatgga agaactgctc tcatacttgc tgtatgttgt 1500
ggatcagcaa gtatagtcag ccctctactt gagcaaaatg ttgatgtatc ttctcaagat 1560
ctggaaagac ggccagagag tatgctgttt ctagtcatca tcatgtaatt tgccagttac 1620
tttctgacta caaagaaaaa cagatgttaa aaatctcttc tgaaaacagc aatccagaac 1680
aagacttaaa gctgacatca gaggaagagt cacaaaggct taaaggaagt gaaaacagcc 1740
agccagagct agaagattta tggctattga agaagaatga agaacacgga agtactcatg 1800
tgggattccc agaaaacctg actaacggtg ccgctgctgg caatggtgat ga        1852

<210> 531
<211> 879
<212> DNA
<213> Homo sapiens

<400> 531
atgcatcttt catttcctgc atttcttcct ccctggatgg acagggggag cggcaagagc 60
aacgtgggca cttctggaga ccacaacgac tcctctgtga agacgcttgg gagcaagagg 120
tgcaagtggt gctgccactg cttcccctgc tgcaggggga gcggcaagag caacgtggtc 180
gcttggggag actacgatga cagcgccttc atggatccca ggtaccacgt ccatggagaa 240
gatctggaca agctccacag agctgcctgg tggggtaaag tccccagaaa ggatctcatc 300
gtcatgctca gggacacgga tgtgaacaag agggacaagc aaaagaggac tgctctacat 360
ctggcctctg ccaatgggaa ttcagaagta gtaaaactcg tgctggacag acgatgtcaa 420
cttaatgtcc ttgacaacaa aaagaggaca gctctgacaa aggccgtaca atgccaggaa 480
gatgaatgtg cgttaatgtt gctggaacat ggcactgatc caaatattcc agatgagtat 540
ggaaatacca ctctacacta tgctgtctac aatgaagata aattaatggc caaagcactg 600
ctcttatacg gtgctgatat cgaatcaaaa aacaagcatg gcctcacacc actgctactt 660

ggtatacatg agcaaaaaca gcaagtggtg aaatttttaa tcaagaaaaa agcgaattta 720
aatgcgctgg atagatatgg aagaactgct ctcatacttg ctgtatgttg tggatcagca 780
agtatagtca gccctctact tgagcaaaat gttgatgtat cttctcaaga tctggaaaga 840
cggccagaga gtatgctgtt tctagtcatc atcatgtaa 879

<210> 532
<211> 292
<212> PRT
<213> Homo sapiens

<400> 532
Met His Leu Ser Phe Pro Ala Phe Leu Pro Pro Trp Met Asp Arg Gly
1               5                   10                  15
Ser Gly Lys Ser Asn Val Gly Thr Ser Gly Asp His Asn Asp Ser Ser
            20                  25                  30
Val Lys Thr Leu Gly Ser Lys Arg Cys Lys Trp Cys Cys His Cys Phe
        35                  40                  45
Pro Cys Cys Arg Gly Ser Gly Lys Ser Asn Val Val Ala Trp Gly Asp
    50                  55                  60
Tyr Asp Asp Ser Ala Phe Met Asp Pro Arg Tyr His Val His Gly Glu
65                  70                  75                  80
Asp Leu Asp Lys Leu His Arg Ala Ala Trp Trp Gly Lys Val Pro Arg
                85                  90                  95
Lys Asp Leu Ile Val Met Leu Arg Asp Thr Asp Val Asn Lys Arg Asp
            100                 105                 110
Lys Gln Lys Arg Thr Ala Leu His Leu Ala Ser Ala Asn Gly Asn Ser
        115                 120                 125
Glu Val Val Lys Leu Val Leu Asp Arg Arg Cys Gln Leu Asn Val Leu
    130                 135                 140
Asp Asn Lys Lys Arg Thr Ala Leu Thr Lys Ala Val Gln Cys Gln Glu
145                 150                 155                 160
Asp Glu Cys Ala Leu Met Leu Leu Glu His Gly Thr Asp Pro Asn Ile
                165                 170                 175
Pro Asp Glu Tyr Gly Asn Thr Thr Leu His Tyr Ala Val Tyr Asn Glu
            180                 185                 190
Asp Lys Leu Met Ala Lys Ala Leu Leu Leu Tyr Gly Ala Asp Ile Glu
        195                 200                 205
Ser Lys Asn Lys His Gly Leu Thr Pro Leu Leu Leu Gly Ile His Glu
    210                 215                 220
Gln Lys Gln Gln Val Val Lys Phe Leu Ile Lys Lys Lys Ala Asn Leu
225                 230                 235                 240
Asn Ala Leu Asp Arg Tyr Gly Arg Thr Ala Leu Ile Leu Ala Val Cys
                245                 250                 255
Cys Gly Ser Ala Ser Ile Val Ser Pro Leu Leu Glu Gln Asn Val Asp
            260                 265                 270
Val Ser Ser Gln Asp Leu Glu Arg Arg Pro Glu Ser Met Leu Phe Leu
        275                 280                 285
Val Ile Ile Met
    290

<210> 533
<211> 801
<212> DNA
<213> Homo sapiens

<400> 533
atgtacaagc ttcagtgcaa caactgtgct acaaatggag ccacagagag gaaacaagca 60
gcaggctcag gagcagggta tgcgctgcct tcggctctcc aatccatgcc tcagggctcc 120
tatgccactg cacgattctt ggttgccaag aggccaacca caggccatct tgagaaggag 180
tttatgttcc actgcagaaa gcagccagga tcaccatcca ggggacttgg tcttctgtgg 240
ccctggccag acatagaatt tgtgccaagg caggacaagc tcactcagag cagcgtgtta 300
gtacctcaaa tctgtgcgtg ccagacaagg ccaaactggc tcaatgagca accagccacc 360
tctgcagggg tgcgtctgga ggaggtggac cagccaccaa ccttacccag tcaaggaagt 420
ggatggccat gttcccacag cctgagtggc tgccacctga tggctgatat agcaaaggcc 480
ttaggaaaag cagatggccc ttggccctac ctttttgtta gaagaactga tgttccatgt 540
cctgcagcga gtgaggttgg tggctgtgcc cccagctcct ggcacaccct cgcagaggtg 600
actggttgct ctttgagccc tcttagcctt gcccagcatg cacaagcctc agtgctacta 660

ctgtgctaca aatggagcca tataggggaa acgagcagcc atctcaggag caaggtgtat 720
gctgcctttg ggggctccag tccttgcctc aagggtctta tgtcactgtg ggcttcttgg 780
ttgccaagag gcagaccata g 801

<210> 534
<211> 266
<212> PRT
<213> Homo sapiens

<400> 534

<210> 535
<211> 6082
<212> DNA
<213> Homo sapiens

<400> 535
cctccactat tacagcttat aggaaattac aatccacttt acaggcctca aaggttcatt 60
ctggccgagc ggacaggcgt ggcggccgga gccccagcat ccctgcttga ggtccaggag 120
cggagcccgc ggccactgcc gcctgatcag cgcgaccccg gcccgcgccc gccccgcccg 180
gcaagatgct gcccgtgtac caggaggtga agcccaaccc gctgcaggac gcgaacctct 240
gctcacgcgt gttcttctgg tggctcaatc ccttgtttaa aattggccat aaacggagat 300
tagaggaaga tgatatgtat tcagtgctgc cagaagaccg ctcacagcac cttggagagg 360
agttgcaagg gttctgggat aaagaagttt taagagctga gaatgacgca cagaagcctt 420
ctttaacaag agcaatcata aagtgttact ggaaatctta tttagttttg ggaattttta 480
cgttaattga ggaaagtgcc aaagtaatcc agcccatatt tttgggaaaa attattaatt 540
attttgaaaa ttatgatccc atggattctg tggctttgaa cacagcgtac gcctatgcca 600
cggtgctgac tttttgcacg ctcattttgg ctatactgca tcacttatat ttttatcacg 660
ttcagtgtgc tgggatgagg ttacgagtag ccatgtgcca tatgatttat cggaaggcac 720
ttcgtcttag taacatggcc atggggaaga caaccacagg ccagatagtc aatctgctgt 780
ccaatgatgt gaacaagttt gatcaggtga cagtgttctt acacttcctg tgggcaggac 840
cactgcaggc gatcgcagtg actgccctac tctggatgga gataggaata tcgtgccttg 900
ctgggatggc agttctaatc attctcctgc ccttgcaaag ctgttttggg aagttgttct 960

catcactgag gagtaaaact gcaactttca cggatgccag gatcaggacc atgaatgaag 1020
ttataactgg tataaggata ataaaaatgt acgcctggga aaagtcattt tcaaatctta 1080
ttaccaattt gagaaagaag gagatttcca agattctgag aagttcctgc ctcaggggga 1140
tgaatttggc ttcgtttttc agtgcaagca aaatcatcgt gtttgtgacc ttcaccacct 1200
acgtgctcct cggcagtgtg atcacagcca gccgcgtgtt cgtggcagtg acgctgtatg 1260
gggctgtgcg gctgacggtt accctcttct tcccctcagc cattgagagg gtgtcagagg 1320
caatcgtcag catccgaaga atccagacct tttgctact tgatgagata tcacagcgca 1380
accgtcagct gccgtcagat ggtaaaaaga tggtgcatgt gcaggatttt actgcttttt 1440
gggataaggc atcagagacc ccaactctac aaggcctttc ctttactgtc agacctggcg 1500
aattgttagc tgtggtcggc cccgtgggag cagggaagtc atcactgtta agtgccgtgc 1560
tcggggaatt ggccccaagt cacgggctgg tcagcgtgca tggaagaatt gcctatgtgt 1620
ctcagcagcc ctgggtgttc tcgggaactc tgaggagtaa tattttattt gggaagaaat 1680
acgaaaagga acgatatgaa aaagtcataa aggcttgtgc tctgaaaaag gatttacagc 1740
tgttggagga tggtgatctg actgtgatag gagatcgggg aaccacgctg agtggagggc 1800
agaaagcacg ggtaaacctt gcaagagcag tgtatcaaga tgctgacatc tatctcctgg 1860
acgatcctct cagtgcagta gatgcggaag ttagcagaca cttgttcgaa ctgtgtattt 1920
gtcaaatttt gcatgagaag atcacaattt tagtgactca tcagttgcag tacctcaaag 1980
ctgcaagtca gattctgata ttgaaagatg gtaaaatggt gcagaagggg acttacactg 2040
agttcctaaa atctggtata gattttggct cccttttaaa gaaggataat gaggaaagtg 2100
aacaacctcc agttccagga actcccacac taaggaatcg taccttctca gagtcttcgg 2160
tttggtctca acaatcttct agaccctcct tgaaagatgg tgctctggag agccaagata 2220
cagagaatgt cccagttaca ctatcagagg agaaccgttc tgaaggaaaa gttggttttc 2280
aggcctataa gaattacttc agagctggtg ctcactggat tgtcttcatt ttccttattc 2340
tcctaaacac tgcagctcag gttgcctatg tgcttcaaga ttggtggctt tcatactggg 2400
caaacaaaca aagtatgcta aatgtcactg taaatggagg aggaaatgta accgagaagc 2460
tagatcttaa ctggtactta ggaatttatt caggtttaac tgtagctacc gttctttttg 2520
gcatagcaag atctctattg gtattctacg tccttgttaa ctcttcacaa actttgcaca 2580
acaaaatgtt tgagtcaatt ctgaaagctc cggtattatt ctttgataga aatccaatag 2640
gaagaatttt aaatcgtttc tccaaagaca ttggacactt ggatgatttg ctgccgctga 2700
cgtttttaga tttcatccag acattgctac aagtggttgg tgtggtctct gtggctgtgg 2760
ccgtgattcc ttggatcgca ataccttgg ttccccttgg aatcattttc atttttcttc 2820
ggcgatattt tttggaaacg tcaagagatg tgaagcgcct ggaatctaca actcggagtc 2880
cagtgttttc ccacttgtca tcttctctcc aggggctctg gaccatccgg gcatacaaag 2940
cagaagagag gtgtcaggaa ctgtttgatg cacaccagga tttacattca gaggcttggt 3000
tcttgttttt gacaacgtcc cgctggttcg ccgtccgtct ggatgccatc tgtgccatgt 3060
ttgtcatcat cgttgccttt gggtccctga ttctggcaaa aactctggat gccgggcagg 3120
ttggtttggc actgtcctat gccctcacgc tcatggggat gtttcagtgg tgtgttcgac 3180
aaagtgctga agttgagaat atgatgatct cagtagaaag ggtcattgaa tacacagacc 3240
ttgaaaaaga agcaccttgg gaatatcaga aacgcccacc accagcctgg ccccatgaag 3300
gagtgataat ctttgacaat gtgaacttca tgtacagtcc aggtgggcct ctggtactga 3360
agcatctgac agcactcatt aaatcacaag aaaaggttgg cattgtggga agaaccggag 3420
ctggaaaaag ttccctcatc tcagcccttt ttagattgtc agaacccgaa ggtaaaattt 3480
ggattgataa gatcttgaca actgaaattg gacttcacga tttaaggaag aaaatgtcaa 3540
tcatacctca ggaacctgtt ttgttcactg gaacaatgag gaaaaacctg gatcccttta 3600
atgagcacac ggatgaggaa ctgtggaatg ccttacaaga ggtacaactt aaagaaacca 3660
ttgaagatct tcctggtaaa atggatactg aattagcaga atcaggatcc aattttagtg 3720
ttggacaaag acaactggtg tgccttgcca gggcaattct caggaaaaat cagatattga 3780
ttattgatga agcgacggca aatgtggatc caagaactga tgagttaata caaaaaaaat 3840
ccgggagaaa tttgcccact gcaccgtgct aaccattgca cacagattga acaccattat 3900
tgacagcgac aagataatgg ttttagattc aggaagactg aaagaatatg atgagccgta 3960
tgttttgctg caaaataaag agagcctatt ttacaagatg gtgcaacaac tgggcaaggc 4020
agaagccgct gccctcactg aaacagcaaa acaggtatac ttcaaaagaa attatccaca 4080
tattggtcac actgaccaca tggttacaaa cacttccaat ggacagccct cgaccttaac 4140
tattttcgag acagcactgt gaatccaacc aaaatgtcaa gtccgttccg aaggcatttg 4200
ccactagttt ttggactatg taaaccacat tgtacttttt tttactttgg caacaaatat 4260
ttatacatac aagatgctag ttcatttgaa tatttctccc aacttatcca aggatctcca 4320
gctctaacaa aatggtttat ttttatttaa atgtcaatag ttgtttttta aaatccaaat 4380
cagaggtgca ggccaccagt taaatgccgt ctatcaggtt ttgtgcctta agagactaca 4440
gagtcaaagc tcatttttaa aggagtagga cagagttgtc acaggttttt gttgttgttt 4500
ttattgcccc caaaattaca tgttaatttc catttatatc agggattcta tttacttgaa 4560
gactgtgaag ttgccatttt gtctcattgt tttctttgac ataactagga tccattattt 4620
cccctgaagg cttcttgtta gaaaatagta cagttacaac caataggaac aacaaaaaga 4680
aaaagtttgt gacattgtag tagggagtgt gtacccctta ctccccatca aaaaaaaaaa 4740
tggatacatg gttaaaggat agaagggcaa tattttatca tatgttctaa aagagaagga 4800
agagaaaata ctactttctc aaaatggaag cccttaaagg tgctttgata ctgaaggaca 4860
caaatgtgac cgtccatcct cctttagagt tgcatgactt ggacacggta actgttgcag 4920
ttttagactc agcattgtga cacttcccaa gaaggccaaa cctctaaccg acattcctga 4980
aatacgtggc attattcttt tttggatttc tcatttatgg aaggctaacc ctctgttgac 5040

tgtaagcctt ttggtttggg ctgtattgaa atcctttcta aattgcatga ataggctctg 5100
ctaacgtgat gagacaaact gaaaattatt gcaagcattg actataatta tgcagtacgt 5160
tctcaggatg catccagggg ttcattttca tgagcctgtc caggttagtt tactcctgac 5220
cactaatagc attgtcattt gggctttctg ttgaatgaat caacaaacca caatacttcc 5280
tgggaccttt tgtactttat ttgaactatg agtctttaat ttttcctgat gatggtggct 5340
gtaatatgtt gagttcagtt tactaaaggt tttactatta tggtttgaag tggagtctca 5400
tgacctctca gaataaggtg tcacctccct gaaattgcat atatgtatat agacatgcac 5460
acgtgtgcat ttgtttgtat acatatattt gtccttcgta tagcaagttt tttgctcatc 5520
agcagagagc aacagatgtt ttattgagtg aagccttaaa aagcacacac cacacacagc 5580
taactgccaa aatacattga ccgtagtagc tgttcaactc ctagtactta gaaatacacg 5640
tatggttaat gttcagtcca acaaaccaca cacagtaaat gtttattaat agtcatggtt 5700
cgtattttag gtgactgaaa ttgcaacagt gatcataatg aggtttgtta aaatgatagc 5760
tatattcaaa atgtctatat gtttatttgg acttttgagg ttaaagacag tcatataaac 5820
gtcctgtttc tgttttaatg ttatcataga attttttaat gaaactaaat tcaattgaaa 5880
taaatgatag ttttcatctc caaaaaaaaa aaaaaaaagg gcggccgctc gagtctagag 5940
ggcccgttta aacccgctga tcagcctcga ctgtgccttc tagttgccag ccatctgttg 6000
tttgcccctc ccccgtgcct tccttgaccc tggaaggtgc cactcccact gtcctttcct 6060
aataaaatga ggaaattgca tc 6082

<210> 536
<211> 6140
<212> DNA
<213> Homo sapiens

<220>
<221> misc_feature
<222> 4535
<223> n = A,T,C or G

<400> 536
cagtggcgca gtctcagctc actgcagcct ccacctcctg tgttcaagca gtcctcctgc 60
ctcagccacc agactagcag gtctcccccg cctctttctt ggaaggacac ttgccattgg 120
atttaggacc cacttggata atccaggatg atgtcttcac tccaacatcc tcagtttaat 180
tccatgtgca aatacccttt tcccaaataa cattcaattc tttaccagga aaggtggctc 240
aatcccttgt ttaaaattgg ccataaacgg agattagagg aagatgatat gtattcagtg 300
ctgccagaag accgctcaca gcaccttgga gaggagttgc aagggttctg ggataaagaa 360
gttttaagag ctgagaatga cgcacagaag ccttctttaa caagagcaat cataaagtgt 420
tactggaaat cttatttagt tttgggaatt tttacgttaa ttgaggaaag tgccaaagta 480
atccagccca tatttttggg aaaaattatt aattattttg aaaattatga tcccatggat 540
tctgtggctt tgaacacagc gtacgcctat gccacggtgc tgactttttg cacgctcatt 600
ttggctatac tgcatcactt atatttttat cacgttcagt gtgctgggat gaggttacga 660
gtagccatgt gccatatgat ttatcggaag gcacttcgtc ttagtaacat ggccatgggg 720
aagacaacca caggccagat agtcaatctg ctgtccaatg atgtgaacaa gtttgatcag 780
gtgacagtgt tcttacactt cctgtgggca ggaccactgc aggcgatcgc agtgactgcc 840
ctactctgga tggagatagg aatatcgtgc cttgctggga tggcagttct aatcattctc 900
ctgcccttgc aaagctgttt tgggaagttg ttctcatcac tgaggagtaa aactgcaact 960
ttcacggatg ccaggatcag gaccatgaat gaagttataa ctggtataag gataataaaa 1020
atgtacgcct gggaaaagtc attttcaaat cttattacca atttgagaaa gaaggagatt 1080
tccaagattc tgagaagttc ctgcctcagg gggatgaatt tggcttcgtt tttcagtgca 1140
agcaaaatca tcgtgtttgt gaccttcacc acctacgtgc tcctcggcag tgtgatcaca 1200
gccagccgcg tgttcgtggc agtgacgctg tatggggctg tgcggctgac ggttaccctc 1260
ttcttcccct cagccattga gagggtgtca gaggcaatcg tcagcatccg aagaatccag 1320
acctttttgc tacttgatga gatatcacag cgcaaccgtc agctgccgtc agatggtaaa 1380
aagatggtgc atgtgcagga ttttactgct ttttgggata aggcatcaga gaccccaact 1440
ctacaaggcc tttcctttac tgtcagacct ggcgaattgt tagctgtggt cggccccgtg 1500
ggagcaggga agtcatcact gttaagtgcc gtgctcgggg aattggcccc aagtcacggg 1560
ctggtcagcg tgcatggaag aattgcctat gtgtctcagc agccctgggt gttctcggga 1620
actctgagga gtaatatttt atttgggaag aaatacgaaa aggaacgata tgaaaaagtc 1680
ataaaggctt gtgctctgaa aaaggattta cagctgttgg aggatggtga tctgactgtg 1740
ataggagatc ggggaaccac gctgagtgga gggcagaaag cacgggtaaa ccttgcaaga 1800
gcagtgtatc aagatgctga catctatctc ctggacgatc ctctcagtgc agtagatgcg 1860
gaagttagca gacacttgtt cgaactgtgt atttgtcaaa ttttgcatga gaagatcaca 1920
attttagtga ctcatcagtt gcagtacctc aaagctgcaa gtcagattct gatattgaaa 1980
gatggtaaaa tggtgcagaa ggggacttac actgagttcc taaaatctgg tatagatttt 2040
ggctcccttt taaagaagga taatgaggaa agtgaacaac ctccagttcc aggaactccc 2100
acactaagga atcgtacctt ctcagagtct tcggtttggt ctcaacaatc ttctagaccc 2160

tccttgaaag atggtgctct ggagagccaa gatacagaga atgtcccagt tacactatca 2220
gaggagaacc gttctgaagg aaaagttggt tttcaggcct ataagaatta cttcagagct 2280
ggtgctcact ggattgtctt cattttcctt attctcctaa acactgcagc tcaggttgcc 2340
tatgtgcttc aagattggtg gctttcatac tgggcaaaca aacaaagtat gctaaatgtc 2400
actgtaaatg gaggaggaaa tgtaaccgag aagctagatc ttaactggta cttaggaatt 2460
tattcaggtt taactgtagc taccgttctt tttggcatag caagatctct attggtattc 2520
tacgtccttg ttaactcttc acaaactttg cacaacaaaa tgtttgagtc aattctgaaa 2580
gctccggtat tattctttga tagaaatcca ataggaagaa ttttaaatcg tttctccaaa 2640
gacattggac acttggatga tttgctgccg ctgacgtttt tagatttcat ccagacattg 2700
ctacaagtgg ttggtgtggt ctctgtggct gtggccgtga ttccttggat cgcaataccc 2760
ttggttcccc ttggaatcat tttcattttt cttcggcgat attttttgga aacgtcaaga 2820
gatgtgaagc gcctggaatc tacaactcgg agtccagtgt tttcccactt gtcatcttct 2880
ctccaggggc tctggaccat ccgggcatac aaagcagaag agaggtgtca ggaactgttt 2940
gatgcacacc aggatttaca ttcagaggct tggttcttgt ttttgacaac gtcccgctgg 3000
ttcgccgtcc gtctggatgc catctgtgcc atgtttgtca tcatcgttgc ctttgggtcc 3060
ctgattctgg caaaaactct ggatgccggg caggttggtt tggcactgtc ctatgccctc 3120
acgctcatgg ggatgtttca gtggtgtgtt cgacaaagtg ctgaagttga gaatatgatg 3180
atctcagtag aaagggtcat tgaatacaca gaccttgaaa aagaagcacc ttgggaatat 3240
cagaaacgcc caccaccagc ctggccccat gaaggagtga taatctttga caatgtgaac 3300
ttcatgtaca gtccaggtgg gcctctggta ctgaagcatc tgacagcact cattaaatca 3360
caagaaaagg ttggcattgt gggaagaacc ggagctggaa aaagttccct catctcagcc 3420
ctttttagat tgtcagaacc cgaaggtaaa atttggattg ataagatctt gacaactgaa 3480
attggacttc acgatttaag gaagaaaatg tcaatcatac ctcaggaacc tgttttgttc 3540
actggaacaa tgaggaaaaa cctggatccc tttaatgagc acacggatga ggaactgtgg 3600
aatgccttac aagaggtaca acttaaagaa accattgaag atcttcctgg taaaatggat 3660
actgaattag cagaatcagg atccaatttt agtgttggac aaagacaact ggtgtgcctt 3720
gccagggcaa ttctcaggaa aaatcagata ttgattattg atgaagcgac ggcaaatgtg 3780
gatccaagaa ctgatgagtt aatacaaaaa aaaatccggg agaaatttgc ccactgcacc 3840
gtgctaacca ttgcacacag attgaacacc attattgaca gcgacaagat aatggtttta 3900
gattcaggaa gactgaaaga atatgatgag ccgtatgttt tgctgcaaaa taaagagagc 3960
ctattttaca agatggtgca acaactgggc aaggcagaag ccgctgccct cactgaaaca 4020
gcaaaacaga gatggggttt caccatgttg gccaggctgg tctcaaactc ctgacctcaa 4080
gtgatccacc tgccttggcc tcccaaactg ctgagattac aggtgtgagc caccacgccc 4140
agcctgagta tacttcaaaa gaaattatcc acatattggt cacactgacc acatggttac 4200
aaacacttcc aatggacagc cctcgacctt aactattttc gagacagcac tgtgaatcca 4260
accaaaatgt caagtccgtt ccgaaggcat ttgccactag tttttggact atgtaaacca 4320
cattgtactt ttttttactt tggcaacaaa tatttataca tacaagatgc tagttcattt 4380
gaatatttct cccaacttat ccaaggatct ccagctctaa caaaatggtt tatttttatt 4440
taaatgtcaa tagtkgkttt ttaaaatcca aatcagaggt gcaggccacc agttaaatgc 4500
cgtctatcag gttttgtgcc ttaagagact acagnagtca gaagctcatt tttaaaggag 4560
taggacagag ttgtcacagg tttttgttgg tgtttktatt gcccccaaaa ttacatgtta 4620
atttccattt atatcagggg attctattta cttgaagact gtgaagttgc cattttgtct 4680
cattgttttc tttgacatam ctaggatcca ttatttcccc tgaaggcttc ttgkagaaaa 4740
tagtacagtt acaaccaata ggaactamca aaaagaaaaa gtttgtgaca ttgtagtagg 4800
gagtgtgtac cccttactcc ccatcaaaaa aaaaaatgga tacatggtta aaggatagaa 4860
gggcaatatt ttatcatatg ttctaaaaga gaaggaagag aaaatactac tttctcaaaa 4920
tggaagccct taaaggtgct ttgatactga aggacacaaa tgtgaccgtc catcctcctt 4980
tagagttgca tgacttggac acggtaactg ttgcagtttt agactcagca ttgtgacact 5040
tcccaagaag gccaaacctc taaccgacat tcctgaaata cgtggcatta ttcttttttg 5100
gatttctcat ttaggaaggc taaccctctg ttgamtgtam kccttttggt ttgggctgta 5160
ttgaaatcct ttctaaattg catgaatagg ctctgctaac cgtgatgaga caaactgaaa 5220
attattgcaa gcattgacta taattatgca gtacgttctc aggatgcatc caggggttca 5280
ttttcatgag cctgtccagg ttagtttact cctgaccact aatagcattg tcatttgggc 5340
tttctgttga atgaatcaac aaaccacaat acttcctggg accttttgta ctttatttga 5400
actatgagtc tttaattttt cctgatgatg gtggctgtaa tatgttgagt tcagtttact 5460
aaaggtttta ctattatggt ttgaagggag tctcatgacc tctcagaaaa ggtgcacctc 5520
cctgaaattg catatatgta tatagacatg cacacgtgtg catttgtttg tatacatata 5580
tttgtccttc gtatagcaag ttttttgctc atcagcagag agcaacagat gttttattga 5640
gtgaagcctt aaaaagcaca caccacacac agctaactgc caaaatacat tgaccgtagt 5700
agctgttcaa ctcctagtac ttagaaatac acgtatggtt aatgttcagt ccaacaaacc 5760
acacacagta aatgtttatt aatagtcatg gttcgtattt taggtgactg aaattgcaac 5820
agtgatcata atgaggtttg ttaaaatgat agctatattc aaaatgtcta tatgtttatt 5880
tggacttttg aggttaaaga cagtcatata aacgtcctgt ttctgtttta atgttatcat 5940
agaatttttt aatgaaacta aattcaattg aaataaatga tagttttcat ctccaaaaaa 6000
aaaaaaaaag ggcggcccgc tcgagtctag agggcccggt ttaaacccgc tgatcagcct 6060
cgactgtgcc ttctagttgc cagccatctg ttgtttggcc ctcccccgtg ccttccttga 6120
ccctggaagg ggccactccc 6140

<210> 537
<211> 1228
<212> PRT
<213> Homo sapiens

<400> 537
Met Leu Pro Val Tyr Gln Glu Val Lys Pro Asn Pro Leu Gln Asp Ala
1               5                   10                  15
Asn Leu Cys Ser Arg Val Phe Phe Trp Trp Leu Asn Pro Leu Phe Lys
            20                  25                  30
Ile Gly His Lys Arg Arg Leu Glu Glu Asp Asp Met Tyr Ser Val Leu
        35                  40                  45
Pro Glu Asp Arg Ser Gln His Leu Gly Glu Glu Leu Gln Gly Phe Trp
    50                  55                  60
Asp Lys Glu Val Leu Arg Ala Glu Asn Asp Ala Gln Lys Pro Ser Leu
65                  70                  75                  80
Thr Arg Ala Ile Ile Lys Cys Tyr Trp Lys Ser Tyr Leu Val Leu Gly
                85                  90                  95
Ile Phe Thr Leu Ile Glu Glu Ser Ala Lys Val Ile Gln Pro Ile Phe
            100                 105                 110
Leu Gly Lys Ile Ile Asn Tyr Phe Glu Asn Tyr Asp Pro Met Asp Ser
        115                 120                 125
Val Ala Leu Asn Thr Ala Tyr Ala Tyr Ala Thr Val Leu Thr Phe Cys
    130                 135                 140
Thr Leu Ile Leu Ala Ile Leu His His Leu Tyr Phe Tyr His Val Gln
145                 150                 155                 160
Cys Ala Gly Met Arg Leu Arg Val Ala Met Cys His Met Ile Tyr Arg
                165                 170                 175
Lys Ala Leu Arg Leu Ser Asn Met Ala Met Gly Lys Thr Thr Thr Gly
            180                 185                 190
Gln Ile Val Asn Leu Leu Ser Asn Asp Val Asn Lys Phe Asp Gln Val
        195                 200                 205
Thr Val Phe Leu His Phe Leu Trp Ala Gly Pro Leu Gln Ala Ile Ala
    210                 215                 220
Val Thr Ala Leu Leu Trp Met Glu Ile Gly Ile Ser Cys Leu Ala Gly
225                 230                 235                 240
Met Ala Val Leu Ile Ile Leu Leu Pro Leu Gln Ser Cys Phe Gly Lys
                245                 250                 255
Leu Phe Ser Ser Leu Arg Ser Lys Thr Ala Thr Phe Thr Asp Ala Arg
            260                 265                 270
Ile Arg Thr Met Asn Glu Val Ile Thr Gly Ile Arg Ile Ile Lys Met
        275                 280                 285
Tyr Ala Trp Glu Lys Ser Phe Ser Asn Leu Ile Thr Asn Leu Arg Lys
    290                 295                 300
Lys Glu Ile Ser Lys Ile Leu Arg Ser Ser Cys Leu Arg Gly Met Asn
305                 310                 315                 320
Leu Ala Ser Phe Phe Ser Ala Ser Lys Ile Ile Val Phe Val Thr Phe
                325                 330                 335
Thr Thr Tyr Val Leu Leu Gly Ser Val Ile Thr Ala Ser Arg Val Phe
            340                 345                 350
Val Ala Val Thr Leu Tyr Gly Ala Val Arg Leu Thr Val Thr Leu Phe
        355                 360                 365
Phe Pro Ser Ala Ile Glu Arg Val Ser Glu Ala Ile Val Ser Ile Arg
    370                 375                 380
Arg Ile Gln Thr Phe Leu Leu Leu Asp Glu Ile Ser Gln Arg Asn Arg
385                 390                 395                 400
Gln Leu Pro Ser Asp Gly Lys Lys Met Val His Val Gln Asp Phe Thr
                405                 410                 415
Ala Phe Trp Asp Lys Ala Ser Glu Thr Pro Thr Leu Gln Gly Leu Ser
            420                 425                 430
Phe Thr Val Arg Pro Gly Glu Leu Leu Ala Val Val Gly Pro Val Gly
        435                 440                 445
Ala Gly Lys Ser Ser Leu Leu Ser Ala Val Leu Gly Glu Leu Ala Pro
    450                 455                 460
Ser His Gly Leu Val Ser Val His Gly Arg Ile Ala Tyr Val Ser Gln
465                 470                 475                 480

Gln Pro Trp Val Phe Ser Gly Thr Leu Arg Ser Asn Ile Leu Phe Gly
                485                 490                 495
Lys Lys Tyr Glu Lys Glu Arg Tyr Glu Lys Val Ile Lys Ala Cys Ala
            500                 505                 510
Leu Lys Lys Asp Leu Gln Leu Leu Glu Asp Gly Asp Leu Thr Val Ile
        515                 520                 525
Gly Asp Arg Gly Thr Thr Leu Ser Gly Gly Gln Lys Ala Arg Val Asn
    530                 535                 540
Leu Ala Arg Ala Val Tyr Gln Asp Ala Asp Ile Tyr Leu Leu Asp Asp
545                 550                 555                 560
Pro Leu Ser Ala Val Asp Ala Glu Val Ser Arg His Leu Phe Glu Leu
                565                 570                 575
Cys Ile Cys Gln Ile Leu His Glu Lys Ile Thr Ile Leu Val Thr His
            580                 585                 590
Gln Leu Gln Tyr Leu Lys Ala Ala Ser Gln Ile Leu Ile Leu Lys Asp
        595                 600                 605
Gly Lys Met Val Gln Lys Gly Thr Tyr Thr Glu Phe Leu Lys Ser Gly
    610                 615                 620
Ile Asp Phe Gly Ser Leu Leu Lys Lys Asp Asn Glu Glu Ser Glu Gln
625                 630                 635                 640
Pro Pro Val Pro Gly Thr Pro Thr Leu Arg Asn Arg Thr Phe Ser Glu
                645                 650                 655
Ser Ser Val Trp Ser Gln Gln Ser Ser Arg Pro Ser Leu Lys Asp Gly
            660                 665                 670
Ala Leu Glu Ser Gln Asp Thr Glu Asn Val Pro Val Thr Leu Ser Glu
        675                 680                 685
Glu Asn Arg Ser Glu Gly Lys Val Gly Phe Gln Ala Tyr Lys Asn Tyr
    690                 695                 700
Phe Arg Ala Gly Ala His Trp Ile Val Phe Ile Phe Leu Ile Leu Leu
705                 710                 715                 720
Asn Thr Ala Ala Gln Val Ala Tyr Val Leu Gln Asp Trp Trp Leu Ser
                725                 730                 735
Tyr Trp Ala Asn Lys Gln Ser Met Leu Asn Val Thr Val Asn Gly Gly
            740                 745                 750
Gly Asn Val Thr Glu Lys Leu Asp Leu Asn Trp Tyr Leu Gly Ile Tyr
        755                 760                 765
Ser Gly Leu Thr Val Ala Thr Val Leu Phe Gly Ile Ala Arg Ser Leu
    770                 775                 780
Leu Val Phe Tyr Val Leu Val Asn Ser Ser Gln Thr Leu His Asn Lys
785                 790                 795                 800
Met Phe Glu Ser Ile Leu Lys Ala Pro Val Leu Phe Phe Asp Arg Asn
                805                 810                 815
Pro Ile Gly Arg Ile Leu Asn Arg Phe Ser Lys Asp Ile Gly His Leu
            820                 825                 830
Asp Asp Leu Leu Pro Leu Thr Phe Leu Asp Phe Ile Gln Thr Leu Leu
        835                 840                 845
Gln Val Val Gly Val Val Ser Val Ala Val Ala Val Ile Pro Trp Ile
    850                 855                 860
Ala Ile Pro Leu Val Pro Leu Gly Ile Ile Phe Ile Phe Leu Arg Arg
865                 870                 875                 880
Tyr Phe Leu Glu Thr Ser Arg Asp Val Lys Arg Leu Glu Ser Thr Thr
                885                 890                 895
Arg Ser Pro Val Phe Ser His Leu Ser Ser Ser Leu Gln Gly Leu Trp
            900                 905                 910
Thr Ile Arg Ala Tyr Lys Ala Glu Glu Arg Cys Gln Glu Leu Phe Asp
        915                 920                 925
Ala His Gln Asp Leu His Ser Glu Ala Trp Phe Leu Phe Leu Thr Thr
    930                 935                 940
Ser Arg Trp Phe Ala Val Arg Leu Asp Ala Ile Cys Ala Met Phe Val
945                 950                 955                 960
Ile Ile Val Ala Phe Gly Ser Leu Ile Leu Ala Lys Thr Leu Asp Ala
                965                 970                 975
Gly Gln Val Gly Leu Ala Leu Ser Tyr Ala Leu Thr Leu Met Gly Met
            980                 985                 990
Phe Gln Trp Cys Val Arg Gln Ser Ala Glu Val Glu Asn Met Met Ile
        995                 1000                1005
Ser Val Glu Arg Val Ile Glu Tyr Thr Asp Leu Glu Lys Glu Ala Pro
    1010                1015                1020

Trp Glu Tyr Gln Lys Arg Pro Pro Pro Ala Trp Pro His Glu Gly Val
1025                1030                1035                1040
Ile Ile Phe Asp Asn Val Asn Phe Met Tyr Ser Pro Gly Gly Pro Leu
                1045                1050                1055
Val Leu Lys His Leu Thr Ala Leu Ile Lys Ser Gln Glu Lys Val Gly
            1060                1065                1070
Ile Val Gly Arg Thr Gly Ala Gly Lys Ser Ser Leu Ile Ser Ala Leu
        1075                1080                1085
Phe Arg Leu Ser Glu Pro Glu Gly Lys Ile Trp Ile Asp Lys Ile Leu
    1090                1095                1100
Thr Thr Glu Ile Gly Leu His Asp Leu Arg Lys Lys Met Ser Ile Ile
1105                1110                1115                1120
Pro Gln Glu Pro Val Leu Phe Thr Gly Thr Met Arg Lys Asn Leu Asp
                1125                1130                1135
Pro Phe Asn Glu His Thr Asp Glu Glu Leu Trp Asn Ala Leu Gln Glu
            1140                1145                1150
Val Gln Leu Lys Glu Thr Ile Glu Asp Leu Pro Gly Lys Met Asp Thr
        1155                1160                1165
Glu Leu Ala Glu Ser Gly Ser Asn Phe Ser Val Gly Gln Arg Gln Leu
    1170                1175                1180
Val Cys Leu Ala Arg Ala Ile Leu Arg Lys Asn Gln Ile Leu Ile Ile
1185                1190                1195                1200
Asp Glu Ala Thr Ala Asn Val Asp Pro Arg Thr Asp Glu Leu Ile Gln
                1205                1210                1215
Lys Lys Ser Gly Arg Asn Leu Pro Thr Ala Pro Cys
            1220                1225

<210> 538
<211> 1261
<212> PRT
<213> Homo sapiens

<400> 538
Met Tyr Ser Val Leu Pro Glu Asp Arg Ser Gln His Leu Gly Glu Glu
 1               5                  10                  15
Leu Gln Gly Phe Trp Asp Lys Glu Val Leu Arg Ala Glu Asn Asp Ala
            20                  25                  30
Gln Lys Pro Ser Leu Thr Arg Ala Ile Ile Lys Cys Tyr Trp Lys Ser
        35                  40                  45
Tyr Leu Val Leu Gly Ile Phe Thr Leu Ile Glu Glu Ser Ala Lys Val
    50                  55                  60
Ile Gln Pro Ile Phe Leu Gly Lys Ile Ile Asn Tyr Phe Glu Asn Tyr
65                  70                  75                  80
Asp Pro Met Asp Ser Val Ala Leu Asn Thr Ala Tyr Ala Tyr Ala Thr
                85                  90                  95
Val Leu Thr Phe Cys Thr Leu Ile Leu Ala Ile Leu His His Leu Tyr
            100                 105                 110
Phe Tyr His Val Gln Cys Ala Gly Met Arg Leu Arg Val Ala Met Cys
        115                 120                 125
His Met Ile Tyr Arg Lys Ala Leu Arg Leu Ser Asn Met Ala Met Gly
    130                 135                 140
Lys Thr Thr Thr Gly Gln Ile Val Asn Leu Leu Ser Asn Asp Val Asn
145                 150                 155                 160
Lys Phe Asp Gln Val Thr Val Phe Leu His Phe Leu Trp Ala Gly Pro
                165                 170                 175
Leu Gln Ala Ile Ala Val Thr Ala Leu Leu Trp Met Glu Ile Gly Ile
            180                 185                 190
Ser Cys Leu Ala Gly Met Ala Val Leu Ile Ile Leu Leu Pro Leu Gln
        195                 200                 205
Ser Cys Phe Gly Lys Leu Phe Ser Ser Leu Arg Ser Lys Thr Ala Thr
    210                 215                 220
Phe Thr Asp Ala Arg Ile Arg Thr Met Asn Glu Val Ile Thr Gly Ile
225                 230                 235                 240
Arg Ile Ile Lys Met Tyr Ala Trp Glu Lys Ser Phe Ser Asn Leu Ile
                245                 250                 255
Thr Asn Leu Arg Lys Lys Glu Ile Ser Lys Ile Leu Arg Ser Ser Cys
            260                 265                 270

Leu Arg Gly Met Asn Leu Ala Ser Phe Phe Ser Ala Ser Lys Ile Ile
            275                 280                 285
Val Phe Val Thr Phe Thr Thr Tyr Val Leu Leu Gly Ser Val Ile Thr
    290                 295                 300
Ala Ser Arg Val Phe Val Ala Val Thr Leu Tyr Gly Ala Val Arg Leu
305                 310                 315                 320
Thr Val Thr Leu Phe Phe Pro Ser Ala Ile Glu Arg Val Ser Glu Ala
                325                 330                 335
Ile Val Ser Ile Arg Arg Ile Gln Thr Phe Leu Leu Leu Asp Glu Ile
            340                 345                 350
Ser Gln Arg Asn Arg Gln Leu Pro Ser Asp Gly Lys Lys Met Val His
        355                 360                 365
Val Gln Asp Phe Thr Ala Phe Trp Asp Lys Ala Ser Glu Thr Pro Thr
    370                 375                 380
Leu Gln Gly Leu Ser Phe Thr Val Arg Pro Gly Glu Leu Leu Ala Val
385                 390                 395                 400
Val Gly Pro Val Gly Ala Gly Lys Ser Ser Leu Leu Ser Ala Val Leu
                405                 410                 415
Gly Glu Leu Ala Pro Ser His Gly Leu Val Ser Val His Gly Arg Ile
            420                 425                 430
Ala Tyr Val Ser Gln Gln Pro Trp Val Phe Ser Gly Thr Leu Arg Ser
        435                 440                 445
Asn Ile Leu Phe Gly Lys Lys Tyr Glu Lys Glu Arg Tyr Glu Lys Val
    450                 455                 460
Ile Lys Ala Cys Ala Leu Lys Lys Asp Leu Gln Leu Leu Glu Asp Gly
465                 470                 475                 480
Asp Leu Thr Val Ile Gly Asp Arg Gly Thr Thr Leu Ser Gly Gly Gln
                485                 490                 495
Lys Ala Arg Val Asn Leu Ala Arg Ala Val Tyr Gln Asp Ala Asp Ile
            500                 505                 510
Tyr Leu Leu Asp Asp Pro Leu Ser Ala Val Asp Ala Glu Val Ser Arg
        515                 520                 525
His Leu Phe Glu Leu Cys Ile Cys Gln Ile Leu His Glu Lys Ile Thr
    530                 535                 540
Ile Leu Val Thr His Gln Leu Gln Tyr Leu Lys Ala Ala Ser Gln Ile
545                 550                 555                 560
Leu Ile Leu Lys Asp Gly Lys Met Val Gln Lys Gly Thr Tyr Thr Glu
                565                 570                 575
Phe Leu Lys Ser Gly Ile Asp Phe Gly Ser Leu Leu Lys Lys Asp Asn
            580                 585                 590
Glu Glu Ser Glu Gln Pro Pro Val Pro Gly Thr Pro Thr Leu Arg Asn
        595                 600                 605
Arg Thr Phe Ser Glu Ser Ser Val Trp Ser Gln Gln Ser Ser Arg Pro
    610                 615                 620
Ser Leu Lys Asp Gly Ala Leu Glu Ser Gln Asp Thr Glu Asn Val Pro
625                 630                 635                 640
Val Thr Leu Ser Glu Glu Asn Arg Ser Glu Gly Lys Val Gly Phe Gln
                645                 650                 655
Ala Tyr Lys Asn Tyr Phe Arg Ala Gly Ala His Trp Ile Val Phe Ile
            660                 665                 670
Phe Leu Ile Leu Leu Asn Thr Ala Ala Gln Val Ala Tyr Val Leu Gln
        675                 680                 685
Asp Trp Trp Leu Ser Tyr Trp Ala Asn Lys Gln Ser Met Leu Asn Val
    690                 695                 700
Thr Val Asn Gly Gly Gly Asn Val Thr Glu Lys Leu Asp Leu Asn Trp
705                 710                 715                 720
Tyr Leu Gly Ile Tyr Ser Gly Leu Thr Val Ala Thr Val Leu Phe Gly
                725                 730                 735
Ile Ala Arg Ser Leu Leu Val Phe Tyr Val Leu Val Asn Ser Ser Gln
            740                 745                 750
Thr Leu His Asn Lys Met Phe Glu Ser Ile Leu Lys Ala Pro Val Leu
        755                 760                 765
Phe Phe Asp Arg Asn Pro Ile Gly Arg Ile Leu Asn Arg Phe Ser Lys
    770                 775                 780
Asp Ile Gly His Leu Asp Asp Leu Leu Pro Leu Thr Phe Leu Asp Phe
785                 790                 795                 800
Ile Gln Thr Leu Leu Gln Val Val Gly Val Val Ser Val Ala Val Ala
                805                 810                 815

Val Ile Pro Trp Ile Ala Ile Pro Leu Val Pro Leu Gly Ile Ile Phe
                820                 825                 830
Ile Phe Leu Arg Arg Tyr Phe Leu Glu Thr Ser Arg Asp Val Lys Arg
        835                 840                 845
Leu Glu Ser Thr Thr Arg Ser Pro Val Phe Ser His Leu Ser Ser Ser
    850                 855                 860
Leu Gln Gly Leu Trp Thr Ile Arg Ala Tyr Lys Ala Glu Glu Arg Cys
865                 870                 875                 880
Gln Glu Leu Phe Asp Ala His Gln Asp Leu His Ser Glu Ala Trp Phe
                885                 890                 895
Leu Phe Leu Thr Thr Ser Arg Trp Phe Ala Val Arg Leu Asp Ala Ile
                900                 905                 910
Cys Ala Met Phe Val Ile Ile Val Ala Phe Gly Ser Leu Ile Leu Ala
        915                 920                 925
Lys Thr Leu Asp Ala Gly Gln Val Gly Leu Ala Leu Ser Tyr Ala Leu
    930                 935                 940
Thr Leu Met Gly Met Phe Gln Trp Cys Val Arg Gln Ser Ala Glu Val
945                 950                 955                 960
Glu Asn Met Met Ile Ser Val Glu Arg Val Ile Glu Tyr Thr Asp Leu
                965                 970                 975
Glu Lys Glu Ala Pro Trp Glu Tyr Gln Lys Arg Pro Pro Pro Ala Trp
                980                 985                 990
Pro His Glu Gly Val Ile Ile Phe Asp Asn Val Asn Phe Met Tyr Ser
        995                 1000                1005
Pro Gly Gly Pro Leu Val Leu Lys His Leu Thr Ala Leu Ile Lys Ser
    1010                1015                1020
Gln Glu Lys Val Gly Ile Val Gly Arg Thr Gly Ala Gly Lys Ser Ser
1025                1030                1035                1040
Leu Ile Ser Ala Leu Phe Arg Leu Ser Glu Pro Glu Gly Lys Ile Trp
                1045                1050                1055
Ile Asp Lys Ile Leu Thr Thr Glu Ile Gly Leu His Asp Leu Arg Lys
                1060                1065                1070
Lys Met Ser Ile Ile Pro Gln Glu Pro Val Leu Phe Thr Gly Thr Met
        1075                1080                1085
Arg Lys Asn Leu Asp Pro Phe Asn Glu His Thr Asp Glu Glu Leu Trp
    1090                1095                1100
Asn Ala Leu Gln Glu Val Gln Leu Lys Glu Thr Ile Glu Asp Leu Pro
1105                1110                1115                1120
Gly Lys Met Asp Thr Glu Leu Ala Glu Ser Gly Ser Asn Phe Ser Val
                1125                1130                1135
Gly Gln Arg Gln Leu Val Cys Leu Ala Arg Ala Ile Leu Arg Lys Asn
                1140                1145                1150
Gln Ile Leu Ile Ile Asp Glu Ala Thr Ala Asn Val Asp Pro Arg Thr
        1155                1160                1165
Asp Glu Leu Ile Gln Lys Lys Ile Arg Glu Lys Phe Ala His Cys Thr
    1170                1175                1180
Val Leu Thr Ile Ala His Arg Leu Asn Thr Ile Ile Asp Ser Asp Lys
1185                1190                1195                1200
Ile Met Val Leu Asp Ser Gly Arg Leu Lys Glu Tyr Asp Glu Pro Tyr
                1205                1210                1215
Val Leu Leu Gln Asn Lys Glu Ser Leu Phe Tyr Lys Met Val Gln Gln
                1220                1225                1230
Leu Gly Lys Ala Glu Ala Ala Ala Leu Thr Glu Thr Ala Lys Gln Arg
        1235                1240                1245
Trp Gly Phe Thr Met Leu Ala Arg Leu Val Ser Asn Ser
    1250                1255                1260


<210> 539
<211> 10
<212> PRT
<213> Artificial Sequence

<220>
<223> Made in a lab

<400> 539
Cys Leu Ser His Ser Val Ala Val Val Thr

1                   5                   10


<210> 540
<211> 9
<212> PRT
<213> Artificial Sequence

<220>
<223> Made in a lab

<400> 540
Ala Val Val Thr Ala Ser Ala Ala Leu
1               5


<210> 541
<211> 14
<212> PRT
<213> Homo sapiens

<400> 541
Leu Ala Gly Leu Leu Cys Pro Asp Pro Arg Pro Leu Glu Leu
1               5                   10


<210> 542
<211> 15
<212> PRT
<213> Homo sapiens

<400> 542
Thr Gln Val Val Phe Asp Lys Ser Asp Leu Ala Lys Tyr Ser Ala
1               5                   10                  15


<210> 543
<211> 12
<212> PRT
<213> Homo sapiens

<400> 543
Phe Met Gly Ser Ile Val Gln Leu Ser Gln Ser Val
1               5                   10


<210> 544
<211> 18
<212> PRT
<213> Homo sapiens

<400> 544
Thr Tyr Val Pro Pro Leu Leu Leu Glu Val Gly Val Glu Glu Lys Phe
1               5                   10                  15
Met Thr


<210> 545
<211> 18
<212> PRT
<213> Homo sapiens

<400> 545
Met Asp Arg Leu Val Gln Arg Phe Gly Thr Arg Ala Val Tyr Leu Ala
1               5                   10                  15
Ser Val

<210> 546
<211> 29
<212> PRT
<213> Homo sapiens

<400> 546
Phe Val Gly Glu Gly Leu Tyr Gln Gly Val Pro Arg Ala Glu Pro Gly
1               5                   10                  15
Thr Glu Ala Arg Arg His Tyr Asp Glu Gly Val Arg Met
            20                  25


<210> 547
<211> 58
<212> PRT
<213> Homo sapiens

<400> 547
Val Ala Glu Glu Ala Ala Leu Gly Pro Thr Glu Pro Ala Glu Gly Leu
1               5                   10                  15
Ser Ala Pro Ser Leu Ser Pro His Cys Cys Pro Cys Arg Ala Arg Leu
            20                  25                  30
Ala Phe Arg Asn Leu Gly Ala Leu Leu Pro Arg Leu His Gln Leu Cys
        35                  40                  45
Cys Arg Met Pro Arg Thr Leu Arg Arg Leu
    50                  55


<210> 548
<211> 18
<212> PRT
<213> Homo sapiens

<400> 548
Ile Asp Trp Asp Thr Ser Ala Leu Ala Pro Tyr Leu Gly Thr Gln Glu
1               5                   10                  15
Glu Cys


<210> 549
<211> 18
<212> PRT
<213> Homo sapiens

<400> 549
Leu Glu Ala Leu Leu Ser Asp Leu Phe Arg Asp Pro Asp His Cys Arg
1               5                   10                  15
Gln Ala


<210> 550
<211> 14
<212> PRT
<213> Homo sapiens

<400> 550
Ser Asp His Trp Arg Gly Arg Tyr Gly Arg Arg Arg Pro Phe
1               5                   10


<210> 551
<211> 11
<212> PRT
<213> Homo sapiens

<400> 551
Phe Asp Lys Ser Asp Leu Ala Lys Tyr Ser Ala
1               5                   10

<210> 552
<211> 2577
<212> DNA
<213> Homo sapiens

<400> 552
agcatatgta acatgacctg tgcttcagtg ttcttttgtg atcaaaaatt ccttactttt 60
agtttttat ctatggtaga accacccaga gcaggggtcc tcaactccca ggccacagac 120
tcataccagt ccacggacta ttatgaacca caccacacag gaggaggtga gcactaggca 180
agccaaggaa gcttcacctg tacttacagc cacacgccat ggctcatatt acagcctgaa 240
ctctgcctcc actcagatca gtgataacat tagaaactca ttggagcacg aaccctgttg 300
tgaactgcct atccgaagga tctaggttgt gtgcttcgta tgagaatcta atgccagatg 360
atctatcatt gtctcacttt gcccccagat aagaccatct agttgcagaa aaataagctc 420
agagcttcca ctgattctac attatggata tgtgccgccg aagcaagcac aaagccctac 480
ttttacacat gcctagtgat gcttcatgga caaggcttgg ctctgttgag tccaactaac 540
ctacctgaga ttctgagatt tctcttcaat ggcttcctgt gagctagagt ttgaaaatat 600
cttaaaatct tgagctagag atggaagtag cttggacgat tttcattatc atgtaaatcg 660
ggtcactcaa ggggccaacc acagctggga gccactgctc aggggaaggt tcatatggga 720
ctttctactg cccaaggttc tatacaggat ataaaggtgc ctcacagtat agatctggta 780
gcaaagaaga agaaacaaac actgatctct ttctgccacc cctctgaccc tttggaactc 840
ctctgaccct ttagaacaag cctacctaat atctgctaga gaaaagacca acaacggcct 900
caaaggatct cttaccatga aggtctcagc taattcttgg ctaagatgtg ggttccacat 960
taggttctga atatgggggg aagggtcaat ttgctcattt tgtgtgtgga taaagtcagg 1020
atgcccaggg gccagagcag ggggctgctg ctttgggaac aatggctgag catataacca 1080
taggtatggg aacaaaaaac atcaaagtca ctgtatcaat tgccatgaag actcgaggga 1140
cctgaatcta ccgattcatc ttaaggcagc aggaccagtt tgagtggcaa caatgcagca 1200
gcagaatcaa tggaaacaac agaatgattg caatgtcctt ttttttctcc tccttctgac 1260
ttgataaaag ggaccgtctt ccttggattt agtgaacccc tttggttcct gaaaaattca 1320
aggagtatct aggacatagt ccccagaaga cagtacaaga ctttctgata aactggacat 1380
ttcaagrccc aaataactaa tcagaaaaat caaagatgtg atactatttt ttatcccatg 1440
cataggtgct acacttggat caaatgaaca atgttgggat ctytatggat aaaggtctta 1500
aaagtcctga gataaagaat cctgcaccca ctggtacttc taacttgtct tgttttttgt 1560
ctgwtttctg gctgatgcag gggactaact cactgccacg cgaaaactac ctgaactgaa 1620
ctatgacatc tcacctgata tgtaagatgt aactgttata attattttaa acctcaattt 1680
agcattaact agccttttaa tgtaaacact tacacattat gaygactaga aacagcatac 1740
tctctggccg tctgtccaga tagatcttga gaagatacat caatgttttg ctcaagtaga 1800
aggctgacta tacttgccga tccacaacat acagcaagta tgagagcagt tctaaaatga 1860
cagagatagg aacagtaata aagttattkt aaaagctaat ttgatatact ttaccaattt 1920
aacatcttgc ctgtccgtgc agaatcaaac atttacatgc actaaaagac ataagcatct 1980
tcagtgctca agtgttcatc tttgtaaaat accaccaagg ttaaaaggaa gggacaaaaa 2040
aaaaaaaccc tcttatctca gtggggtatt gcatagcaga agctactaat ttgaagtcct 2100
ttgatggaca agaaacaata ttagggccac ttatctgaaa tgaacaaaga tttaagtgaa 2160
gatttcatca cagcttccct agactgatat gctgtaatag aaaatcagct agggggtaaa 2220
ataaataaga gctctctgca tgctgaaagc aagtaagatt aataataatg gtaagaatag 2280
tagtcacagg agtttcagtt aatgatgcca ataagcatgt gctaggcact gaattaaatg 2340
ccacatatat ctttcttatg cgcagcaaac tttgaaggat atattctcct acttttcata 2400
tatgacaaca tatttggtgg taaataacgt tcccaaggtc acacacctag caagtaagaa 2460
agttaggaat taaacccagt attgtgtgaa tctaaagcct aacttttttc tctttatcac 2520
ccacctacgg cttgtcttca ttaaaggaaa agtgtatcca cttaaaaaaa aaaaaaa 2577

<210> 553
<211> 58
<212> PRT
<213> Homo sapiens

<400> 553
Ser Ile Cys Asn Met Thr Cys Ala Ser Val Phe Phe Cys Asp Gln Lys
1               5                   10                  15
Phe Leu Thr Phe Ser Phe Leu Ser Met Val Glu Pro Pro Arg Ala Gly
            20                  25                  30
Val Leu Asn Ser Gln Ala Thr Asp Ser Tyr Gln Ser Thr Asp Tyr Tyr
        35                  40                  45

Glu Pro His His Thr Gly Gly Gly Glu His
    50                  55

<210> 554
<211> 59
<212> PRT
<213> Homo sapiens

<400> 554
Leu Gln Lys Asn Lys Leu Arg Ala Ser Thr Asp Ser Thr Leu Trp Ile
1               5                   10                  15
Cys Ala Ala Glu Ala Ser Thr Lys Pro Tyr Phe Tyr Thr Cys Leu Val
            20                  25                  30
Met Leu His Gly Gln Gly Leu Ala Leu Leu Ser Pro Thr Asn Leu Pro
        35                  40                  45
Glu Ile Leu Arg Phe Leu Phe Asn Gly Phe Leu
    50                  55

<210> 555
<211> 71
<212> PRT
<213> Homo sapiens

<400> 555
Leu Gly Arg Phe Ser Leu Ser Cys Lys Ser Gly His Ser Arg Gly Gln
1               5                   10                  15
Pro Gln Leu Gly Ala Thr Ala Gln Gly Lys Val His Met Gly Leu Ser
            20                  25                  30
Thr Ala Gln Gly Ser Ile Gln Asp Ile Lys Val Pro His Ser Ile Asp
        35                  40                  45
Leu Val Ala Lys Lys Lys Lys Gln Thr Leu Ile Ser Phe Cys His Pro
    50                  55                  60
Ser Asp Pro Leu Glu Leu Leu
65                  70

<210> 556
<211> 81
<212> PRT
<213> Homo sapiens

<400> 556
Asn His Pro Glu Gln Gly Ser Ser Thr Pro Arg Pro Gln Thr His Thr
1               5                   10                  15
Ser Pro Arg Thr Ile Met Asn His Thr Thr Gln Glu Glu Val Ser Thr
            20                  25                  30
Arg Gln Ala Lys Glu Ala Ser Pro Val Leu Thr Ala Thr Arg His Gly
        35                  40                  45
Ser Tyr Tyr Ser Leu Asn Ser Ala Ser Thr Gln Ile Ser Asp Asn Ile
    50                  55                  60
Arg Asn Ser Leu Glu His Glu Pro Cys Cys Glu Leu Pro Ile Arg Arg
65                  70                  75                  80
Ile

<210> 557
<211> 54
<212> PRT
<213> Homo sapiens

<400> 557
Ser Leu Ser Ala Thr Pro Leu Thr Leu Trp Asn Ser Ser Asp Pro Leu
1               5                   10                  15
Glu Gln Ala Tyr Leu Ile Ser Ala Arg Glu Lys Thr Asn Asn Gly Leu
            20                  25                  30

Lys Gly Ser Leu Thr Met Lys Val Ser Ala Asn Ser Trp Leu Arg Cys
        35                  40                  45
Gly Phe His Ile Arg Phe
    50

<210> 558
<211> 77
<212> PRT
<213> Homo sapiens

<220>
<221> VARIANT
<222> 12
<223> Xaa = Any Amino Acid

<400> 558
Asn Asp Arg Asp Arg Asn Ser Asn Lys Val Ile Xaa Lys Ala Asn Leu
 1               5                  10                  15
Ile Tyr Phe Thr Asn Leu Thr Ser Cys Leu Ser Val Gln Asn Gln Thr
            20                  25                  30
Phe Thr Cys Thr Lys Arg His Lys His Leu Gln Cys Ser Ser Val His
        35                  40                  45
Leu Cys Lys Ile Pro Pro Arg Leu Lys Gly Arg Asp Lys Lys Lys Lys
    50                  55                  60
Pro Ser Tyr Leu Ser Gly Val Leu His Ser Arg Ser Tyr
65                  70                  75

<210> 559
<211> 50
<212> PRT
<213> Homo sapiens

<400> 559
Thr Leu Pro Pro Leu Arg Ser Val Ile Thr Leu Glu Thr His Trp Ser
 1               5                  10                  15
Thr Asn Pro Val Val Asn Cys Leu Ser Glu Gly Ser Arg Leu Cys Ala
            20                  25                  30
Ser Tyr Glu Asn Leu Met Pro Asp Asp Leu Ser Leu Ser His Phe Ala
        35                  40                  45
Pro Arg
    50

<210> 560
<211> 56
<212> PRT
<213> Homo sapiens

<400> 560
Ile Gly Ser Leu Lys Gly Pro Thr Thr Ala Gly Ser His Cys Ser Gly
 1               5                  10                  15
Glu Gly Ser Tyr Gly Thr Phe Tyr Cys Pro Arg Phe Tyr Thr Gly Tyr
            20                  25                  30
Lys Gly Ala Ser Gln Tyr Arg Ser Gly Ser Lys Glu Glu Glu Thr Asn
        35                  40                  45
Thr Asp Leu Phe Leu Pro Pro Leu
    50                  55

<210> 561
<211> 57
<212> PRT
<213> Homo sapiens

<220>
<221> VARIANT

<222> 13, 40
<223> Xaa = Any Amino Acid

<400> 561
Val Leu His Leu Asp Gln Met Asn Asn Val Gly Ile Xaa Met Asp Lys
 1               5                  10                  15
Gly Leu Lys Ser Pro Glu Ile Lys Asn Pro Ala Pro Thr Gly Thr Ser
            20                  25                  30
Asn Leu Ser Cys Phe Leu Ser Xaa Phe Trp Leu Met Gln Gly Thr Asn
        35                  40                  45
Ser Leu Pro Arg Glu Asn Tyr Leu Asn
    50                  55


<210> 562
<211> 59
<212> PRT
<213> Homo sapiens

<220>
<221> VARIANT
<222> 5, 37
<223> Xaa = Any Amino Acid

<400> 562
Asp Leu Tyr Pro Xaa Arg Ser Gln His Cys Ser Phe Asp Pro Ser Val
 1               5                  10                  15
Ala Pro Met His Gly Ile Lys Asn Ser Ile Thr Ser Leu Ile Phe Leu
            20                  25                  30
Ile Ser Tyr Leu Xaa Leu Glu Met Ser Ser Leu Ser Glu Ser Leu Val
        35                  40                  45
Leu Ser Ser Gly Asp Tyr Val Leu Asp Thr Pro
    50                  55


<210> 563
<211> 79
<212> PRT
<213> Homo sapiens

<400> 563
Cys Phe Leu Phe Pro Tyr Leu Trp Leu Tyr Ala Gln Pro Leu Phe Pro
 1               5                  10                  15
Lys Gln Gln Pro Pro Ala Leu Ala Pro Gly His Pro Asp Phe Ile His
            20                  25                  30
Thr Gln Asn Glu Gln Ile Asp Pro Ser Pro His Ile Gln Asn Leu Met
        35                  40                  45
Trp Asn Pro His Leu Ser Gln Glu Leu Ala Glu Thr Phe Met Val Arg
    50                  55                  60
Asp Pro Leu Arg Pro Leu Leu Val Phe Ser Leu Ala Asp Ile Arg
65                  70                  75


<210> 564
<211> 64
<212> PRT
<213> Homo sapiens

<400> 564
Ala Cys Ser Lys Gly Ser Glu Glu Phe Gln Arg Val Arg Gly Val Ala
 1               5                  10                  15
Glu Arg Asp Gln Cys Leu Phe Leu Leu Leu Cys Tyr Gln Ile Tyr Thr
            20                  25                  30
Val Arg His Leu Tyr Ile Leu Tyr Arg Thr Leu Gly Ser Arg Lys Ser
        35                  40                  45
His Met Asn Leu Pro Leu Ser Ser Gly Ser Gln Leu Trp Leu Ala Pro
    50                  55                  60

<210> 565
<211> 57
<212> PRT
<213> Homo sapiens

<220>
<221> VARIANT
<222> 54
<223> Xaa = Any Amino Acid

<400> 565
Leu Tyr Tyr Cys Ser Tyr Leu Cys His Phe Arg Thr Ala Leu Ile Leu
 1               5                  10                  15
Ala Val Cys Cys Gly Ser Ala Ser Ile Val Ser Leu Leu Leu Glu Gln
            20                  25                  30
Asn Ile Asp Val Ser Ser Gln Asp Leu Ser Gly Gln Thr Ala Arg Glu
        35                  40                  45
Tyr Ala Val Ser Ser Xaa His Asn Val
    50                  55


<210> 566
<211> 55
<212> PRT
<213> Homo sapiens

<400> 566
Ile Leu Leu Glu Phe Phe Arg Asn Gln Arg Gly Ser Leu Asn Pro Arg
 1               5                  10                  15
Lys Thr Val Pro Phe Ile Lys Ser Glu Gly Gly Glu Lys Lys Gly His
            20                  25                  30
Cys Asn His Ser Val Val Ser Ile Asp Ser Ala Ala Ala Leu Leu Pro
        35                  40                  45
Leu Lys Leu Val Leu Leu Pro
    50                  55


<210> 567
<211> 51
<212> PRT
<213> Homo sapiens

<400> 567
Tyr Ser Asp Phe Asp Val Phe Cys Ser His Thr Tyr Gly Tyr Met Leu
 1               5                  10                  15
Ser His Cys Ser Gln Ser Ser Ser Pro Leu Leu Trp Pro Leu Gly Ile
            20                  25                  30
Leu Thr Leu Ser Thr His Lys Met Ser Lys Leu Thr Leu Pro Pro Ile
        35                  40                  45
Phe Arg Thr
    50


<210> 568
<211> 75
<212> PRT
<213> Homo sapiens

<400> 568
Lys Val Gly Glu Tyr Ile Leu Gln Ser Leu Leu Arg Ile Arg Lys Ile
 1               5                  10                  15
Tyr Val Ala Phe Asn Ser Val Pro Ser Thr Cys Leu Leu Ala Ser Leu
            20                  25                  30
Thr Glu Thr Pro Val Thr Thr Ile Leu Thr Ile Ile Ile Asn Leu Thr
        35                  40                  45
Cys Phe Gln His Ala Glu Ser Ser Tyr Leu Phe Tyr Pro Leu Ala Asp
    50                  55                  60

Phe Leu Leu Gln His Ile Ser Leu Gly Lys Leu
65                  70                  75

<210> 569
<211> 4809
<212> DNA
<213> Homo sapiens

<400> 569
   gcatccagag tggtggactg gttacaggct atgaacctac actgatgcgg caccaccacc 60
   cagagtccac rggttatgtt ggttcacatt tactcttgct gtggtatggt ctataggttt 120
   ggacagatgt ccgataatcc tttttacatt ttggcatcct tgggtagctc gtcttgtagg 180
   aatggacttg cttcaaagtg gaggcaggca gatccttcag acgggtatat ggagccctgt 240
   tttcagttgc ttttctaatt ctctcttatc gtttacctca aaatcttcct gaggtctcgc 300
   ttccttttaa aatccttgtc tactttgcag catcactctg acactcccat tgattcctca 360
   gcacctactg actacacggt taggagtgca agggtagaat tcatgtttta ttcatctttg 420
   ggtctgtagc acccagcaaa gtgctcagta aatgcgcagt aattgatttg acctctgaac 480
   aaatacacac tgtactaaga atctacacac cgaaagacaa aaacaagaca aatttgagtg 540
   ctacaggtgt cacgcttggc atcacacatg tgcctgtgta ttcctctagg tggttaccag 600
   gagctctgcc actgcatgtc cactagtgac gggttcgctc caccacccca gctgggtagc 660
   cgctgctctc acataagggg tccaattaaa attgccagga ataaattccc ccggactttg 720
   acttctcaag agctaagaag gtttgctgag tattctggca tgatgtttgg tgatcaaaca 780
   actgctggcc aaaaatgatg agtatttccc cctcttgctg aagatgtgct ccatacaata 840
   gtccatcaca ttcatcattc atcagtctgg aagtgtgcag aacaacatgt aatagataat 900
   atgattggct gcacacttcc agactgatga atgatgaatg tgatggacta ttgtatggag 960
   cacatcttca gcaagagggg gaaatactca tcattttatc tattacatgt tgttctggtt 1020
   tttttttttt tccaatgtcc agcctaaact ataaagtact ttgagaacgc acagtgagcc 1080
   ataagcttgc caataaagag tcctctgtgg tatggaactg gcttatttca tacacaatct 1140
   gcaaacaatg agggcactat tggaaacata ctgtgctgca cagagcattt acaccgctta 1200
   tctttaatct tccccagcaa tccttgcttt gtgcgcattt atgatccttg ctctcagaag 1260
   tccacatact tttccccaac cgtaacaaat tatttaactc atctaatgta tgtatgtccg 1320
   cgcagtctga aaacagtaat tgtccttggg aagaagtgag tttaagagag ctctagggca 1380
   ctcatcacaa ctccagccct gccctccatg tggtagcagc tctttggact ggggctaagt 1440
   gcttattctt gtgcttcatt cctggtaagc tcaatttctt taccttagga taactttgct 1500
   ggaaaagggc tcagattcag ccgaccattg tggcctctgt ggctgtcaca gcttgtccct 1560
   gacatgctat gatgttgggt ccccttctca tccccttggg atttcttctg ctggcccaca 1620
   gccagaacaa ctaggccttt tactccacca tccctttgtt ttcttttgtt tcgttggtaa 1680
   aaatcaatcc ttctaccatc catgcatagc aatttctaaa aactgaattt caagagcagt 1740
   atctgaagaa acaaacatga tttggtcctt ttagtaaaca gaataaattt taataaatca 1800
   actttgaaat agttgtaaga gttaagaaaa agcacaaaac tgagatcatc agagcagctt 1860
   ggcctcaaag gacaggcagc aggattctac agggtttgag ccttcctaag tgaagctgtt 1920
   tcctgcaggc tccctgctcc aagctcctag ctaacagccc cttctcccac gattggcaac 1980
   aaagagcaaa aataactttg tacttgatgc tgagtcagtg taaaaagcca taaaaaattc 2040
   cctctaaatg tcaaaatgtt tgcctccttt gaggcttctc tcctcctact gggtctggat 2100
   aaattagcac tgggcttata ttgagtcaca gatctgggcc ctgccacaga gagcttcctc 2160
   ctagtgtgtg atgctttttc tccaaactat tgatacaaaa tgcactggaa tagaaatcaa 2220
   cagaaactgg tcaaaggtgt ggcatacaca ttctcatgta gatgtaaagc tgtgcttaga 2280
   attcctttgt ggagtctggt ttggtcttgg ttttcttggt gtttgattca tttttttacg 2340
   taaattacaa aaaccctcca catttcttca tggattgtat tagtccatgt tctccagaga 2400
   agcagaacga gttggatgta tgttttggaa gagattatga ggaaccggct catgtgatga 2460
   aggaggttga gaggtcctgt gctctgccat ctgcaagctg aagacctgga aagctgaggg 2520
   tgtggctcca gtctgagtct gaaggcccaa gaaccagggg aaccaacggt gtagattcca 2580
   ggttgaaggc aggagaagat ggatgtccca gctcagcagg caggcaggaa gcaaatgggg 2640
   taaattcctc cttcctccac cttttgttcc attcaggcct tcaacagatt ggatgagcgc 2700
   ccccccaccc ccacactagg gagggccatc tgctttactg agtcggctga gtcaagtgcc 2760
   agcctcatcc caaaacactc tccagacaca cgcagaaatg tttcatctgg gcaccctgtg 2820
   gccagtcatg ctgacacaca gaactaacca tgacatggat tcttcttaaa gcagtgatag 2880
   gagcgaacag aaacattttc ataattttca attattttta atgaaaacta tatctgatgg 2940
   aattgtttaa acctagtctg gccacacatt atttcctggg accgcccctc cttcaatccc 3000
   ttggacactg atgactttat gcccagatta cactggaggc ctgtgctgat tttctaacac 3060
   atacctgcaa ctgagctggc aaaaagaaaa ctaggcaagt atgacagata catgatgcac 3120
   aggctaagtg caaaggaaag aaaaacacca actgcaggga tgagggactc acccctttag 3180
   aagtttctac ttgagcagct agaagactac aatgccactc atcaaaacag tgactcaggg 3240
   ggagtatttg ggataaagga ggaatctgat gttggaggtc aaatttgaag tgtctttaag 3300
   acctacaggt aacgagacag ctggacaaac acatggaact caggacaaag gctctaagga 3360
   cagcacagca gctgacatcc tgtgtgacag ccttgaaagc agcaggcccg ccgctcacat 3420
   tttggaaggg aaaatgggta caatgttgtc tgccactttg ggccttcttt gggtcacatg 3480
   cattttacat ttatgcagtt gatatattta tgtttcctgg gtcttttata cattagacac 3540
   catgattctc aatcctttgt tattttgtat tacaaaaagc tgaattatta tttcaaatat 3600
   gggcaaatta gagccttcca tattgccaag gtgtatcaac cacactgata ycaygatctc 3660
   tcttttgaat tagttttcca gttcacacct accatttatt tcatgattgg tttcagactt 3720
   gttcctcctg gaaacactcc ctaacaagca cccttgcagg aatgaagaca caccacacac 3780
   atctacccca ttactgcatg tactcaagag tcagctttta tatgatctct cccaagtgct 3840
   cctataatgg ggatctttca ctcaccctaa agtgaggaca aaatacttga aagcatgagc 3900
   ccagtgcctg taggtgtgca attaacctca gaccaaggaa gtgccgaacg catctggctt 3960
   ttagcaaggc acctgacaaa gtccttcagg atgtttttgt acatgagcta gagaaatgta 4020
   cctggagaac agcttctact gccagatgat cttactcaaa agatgcagat taagcaaaat 4080
   atcaacccaa agggtggtcc ctgatggccc accagcccct gtgcctggct cgtttcctat 4140
   gtttcctaga tttggtttca gacttgctcc tcctgcagac actccctaac cagcatcctt 4200
   gcagaaaact ggtgaactag aaaaggcctg tgtgggtcac gtggccaccc aacaccacag 4260
   cagtgtctaa ggtatgcgtg ggagcctgca cagcaggagc ggggtcttct ggagacccgc 4320
   atgagatgca aagggcagtg gacaaggagc caagggaggt ggctctagtc acgctggtat 4380
   ggtgccagct tgaggatgct gggcaagtcc cgagccgtct gccttcctag taccacagtt 4440
   accactgtct gttacctcgc gagttcaagt gcttcacgtg agacagctac gagacaggcc 4500
   cctggaaact ggaaaatgcy aagtaaatgt catgcacaat tgttgttcac attttatctc 4560
   aatcactttt accaaatcag gctaaaccct gggtattcat aacgtcttgg gctgtacaaa 4620
   ttgttccttg aaatgactca gagacatttt ctgaattggc ttccatcagc caagcatttc 4680
   ttcagaactg gaaaaatgct ttaaatttgg ctttgtcatg attattaaaa cactctgtac 4740
   attttttatt attgaaatta acacattgcc tactttttaa aaattggaaa aagaaaaaaa 4800
   aaaaaaaaa 4809

<210> 570
<211> 951
<212> DNA
<213> Homo sapiens

<400> 570
   aaaattgaat attgagatac cattctttag tgttaccttt tttacccaca tgtgtttctg 60
   aaaatattgg aattttattc atcttaaaaa ttggacccgg ccttatttac catctttaat 120
   ccattttagt actatgggtg agtacatgga attgaagtct ggcttaaatc ttcagaaagt 180
   tatatatcta ttttatttta tttttttgag acagagtctc gctgtgtcac ccaggctgga 240
   gtgcggtgcc acaatcttgg ctcactgcaa cctctgagtc ccaggttcaa gcgatactca 300
   tgcctcggcc tcctgagtag ctgggactac aggcgtgcac caccacatct ggctaatctt 360
   tttttgtatt tttagtagag acggggtttc actgtggtct ccatctcctg acctcgtgat 420
   ccgcctgcct cccaaagtgc tgggattaca ggcatgagcc accgcacaca gctgggactg 480
   ggtaatttat aaagaaaaga ggtttaatga ctcacagttc cgcatggctg gagaggcctc 540
   aggaaactta caatcatggt ggaaggcgaa ggggaagcaa ggcacgtctt acatggtggc 600
   aggagagaac gagtgagggg ggagactgcc acaaactttt tttttttgag acaagagtct 660
   ggccctgttg cccaggctgg agtgcagtgg catgatctca gctcactgca acctctgcct 720
   cacaggttca agcaattctc atgcctcagc ctcccgcata gctgggacca caggtatgca 780
   ccaccacacc tagctaattt ttgtagtttt agtagagatg gggtctcact atgttgctca 840
   ggctggtcta aaactcctgg gctccagcaa tccgcctgcc ttggcctccc aaagtgctgg 900
   ggttacaggc ataagccacc acatccagcc tgccacatac ttttaaacta t 951

<210> 571
<211> 819
<212> DNA
<213> Homo sapiens

<400> 571
cagcttaaaa atggtttctt gaaatcagtg attagcattc actcaccagt acccctacta 60
aggggtaggc actggtttgt actcctggga atacaggagt acaccagaat ttatttctgc 120
ttattgcttt tgttgcaaat gccgtggctt catctgagga attctagaat tcagagggtg 180
tagccctcca ctctgctgtc ttgctatctg ctctcattgc atccgtttaa cctgcattct 240
gaaagatgtt tctcaggttt ttccttgacg atttcttct tttctgattc tgacaatgtt 300
ttaaatcatt gtactgtggt tatcatttct ctgcatttat tttacccatc ttcctttgta 360
acttgtccta ttgtctttta atttctgcct gttctttatg gctttcaact tcataaataa 420
catgttttct caaatctctt tgtgaattcc agagagggcc aggcacggtg gctcacatct 480
gtaatcccag cactttgggg aggctgagac gggtggatca cttgaggtca ggagtttgag 540
accagcctgg ccaacatggt gaaatcccgt ttcactaaaa atacaaaaat tacccaggca 600
tggtggcggg cgcctgtaat cccaggtact cgggaggctg agggaggaga atcgcttgaa 660
cctgggaggc tgagggagga gaatcgcttg aacccgggag gcagaggttg cagtgaaccg 720
agatcatgtt gctgcactcc agcctggtca acagagcaag actctgcctc aaaaacaaac 780
aaataaacaa acaaacaaac aaaacagaga gattttgct 819

<210> 572
<211> 203
<212> DNA
<213> Homo sapiens

<400> 572
tatagaatac tcaagctatg catcaagctt ggtaccgagc tcggatccac tatttacggc 60
cgccagtgtg ctggaattcg cccttagctc ggatccacta gtccagtgtg gtggaattcc 120
attgtgttgg gcccaacaca atggagccac cacatccagc ctgccacata cttttaaact 180
atcaggtctc atgagaactc atg 203

<210> 573
<211> 132
<212> PRT
<213> Homo sapiens

<400> 573
Met Val Glu Gly Glu Gly Glu Ala Arg His Val Leu His Gly Gly Arg
1               5                   10                  15
Arg Glu Arg Val Arg Gly Glu Thr Ala Thr Asn Phe Phe Phe Leu Arg
            20                  25                  30
Gln Glu Ser Gly Pro Val Ala Gln Ala Gly Val Gln Trp His Asp Leu
        35                  40                  45
Ser Ser Leu Gln Pro Leu Pro His Arg Phe Lys Gln Phe Ser Cys Leu
    50                  55                  60
Ser Leu Pro His Ser Trp Asp His Arg Tyr Ala Pro Pro His Leu Ala
65                  70                  75                  80
Asn Phe Cys Ser Phe Ser Arg Asp Gly Val Ser Leu Cys Cys Ser Gly
                85                  90                  95
Trp Ser Lys Thr Pro Gly Leu Gln Gln Ser Ala Cys Leu Gly Leu Pro
            100                 105                 110
Lys Cys Trp Gly Tyr Arg His Lys Pro Pro His Pro Ala Cys His Ile
        115                 120                 125
Leu Leu Asn Tyr
    130

<210> 574
<211> 62
<212> PRT
<213> Homo sapiens

<400> 574
Met Thr His Ser Ser Ala Trp Leu Glu Arg Pro Gln Glu Thr Tyr Asn
1               5                   10                  15
His Gly Gly Arg Arg Arg Gly Ser Lys Ala Arg Leu Thr Trp Trp Gln
            20                  25                  30
Glu Arg Thr Ser Glu Gly Gly Asp Cys His Lys Leu Phe Phe Phe Glu
        35                  40                  45
Thr Arg Val Trp Pro Cys Cys Pro Gly Trp Ser Ala Val Ala
    50                  55                  60

<210> 575
<211> 76
<212> PRT
<213> Homo sapiens

<400> 575
Met Val Lys Ser Arg Phe Thr Lys Asn Thr Lys Ile Thr Gln Ala Trp
1               5                   10                  15
Trp Arg Ala Pro Val Ile Pro Gly Thr Arg Glu Ala Glu Gly Gly Glu
            20                  25                  30
Ser Leu Glu Pro Gly Arg Leu Arg Glu Glu Asn Arg Leu Asn Pro Gly
        35                  40                  45
Gly Arg Gly Cys Ser Glu Pro Arg Ser Cys Cys Cys Thr Pro Ala Trp
    50                  55                  60
Ser Thr Glu Gln Asp Ser Ala Ser Lys Thr Asn Lys

65                  70                  75

<210> 576
<211> 68
<212> PRT
<213> Homo sapiens

<220>
<221> VARIANT
<222> 42
<223> Xaa = Any Amino Acid

<400> 576
Met Leu Gly Lys Ser Arg Ala Val Cys Leu Pro Ser Thr Thr Val Thr
 1               5                  10                  15
Thr Val Cys Tyr Leu Ala Ser Ser Ser Ala Ser Arg Glu Thr Ala Thr
            20                  25                  30
Arg Gln Ala Pro Gly Asn Trp Lys Met Xaa Ser Lys Cys His Ala Gln
        35                  40                  45
Leu Leu Phe Thr Phe Tyr Leu Asn His Phe Tyr Gln Ile Arg Leu Asn
    50                  55                  60
Pro Gly Tyr Ser
65

<210> 577
<211> 57
<212> PRT
<213> Homo sapiens

<400> 577
Met Tyr Leu Glu Asn Ser Phe Tyr Cys Gln Met Ile Leu Leu Lys Arg
 1               5                  10                  15
Cys Arg Leu Ser Lys Ile Ser Thr Gln Arg Val Val Pro Asp Gly Pro
            20                  25                  30
Pro Ala Pro Val Pro Gly Ser Phe Pro Met Phe Pro Arg Phe Gly Phe
        35                  40                  45
Arg Leu Ala Pro Pro Ala Asp Thr Pro
    50                  55

<210> 578
<211> 51
<212> PRT
<213> Homo sapiens

<400> 578
Met Gln Leu Ile Tyr Leu Cys Phe Leu Gly Leu Leu Tyr Ile Arg His
 1               5                  10                  15
His Asp Ser Gln Ser Phe Val Ile Leu Tyr Tyr Lys Lys Leu Asn Tyr
            20                  25                  30
Tyr Phe Lys Tyr Gly Gln Ile Arg Ala Phe His Ile Ala Lys Val Tyr
        35                  40                  45
Gln Pro His
    50

<210> 579
<211> 56
<212> PRT
<213> Homo sapiens

<400> 579
Met His Phe Thr Phe Met Gln Leu Ile Tyr Leu Cys Phe Leu Gly Leu
 1               5                  10                  15
Leu Tyr Ile Arg His His Asp Ser Gln Ser Phe Val Ile Leu Tyr Tyr
            20                  25                  30

Lys Lys Leu Asn Tyr Tyr Phe Lys Tyr Gly Gln Ile Arg Ala Phe His
        35                  40                  45
Ile Ala Lys Val Tyr Gln Pro His
    50                  55

<210> 580
<211> 67
<212> PRT
<213> Homo sapiens

<400> 580
Met Glu Leu Arg Thr Lys Ala Leu Arg Thr Ala Gln Gln Leu Thr Ser
 1               5                  10                  15
Cys Val Thr Ala Leu Lys Ala Ala Gly Pro Pro Leu Thr Phe Trp Lys
            20                  25                  30
Gly Lys Trp Val Gln Cys Cys Leu Pro Leu Trp Gly Leu Leu Gly Ser
        35                  40                  45
His Ala Phe Tyr Ile Tyr Ala Val Asp Ile Phe Met Phe Pro Gly Ser
    50                  55                  60
Phe Ile His
65

<210> 581
<211> 77
<212> PRT
<213> Homo sapiens

<400> 581

<210> 582
<211> 51
<212> PRT
<213> Homo sapiens

<400> 582
Met Leu Phe Leu Gln Thr Ile Asp Thr Lys Cys Thr Gly Ile Glu Ile
 1               5                  10                  15
Asn Arg Asn Trp Ser Lys Val Trp His Thr His Ser His Val Asp Val
            20                  25                  30
Lys Leu Cys Leu Glu Phe Leu Cys Gly Val Trp Phe Gly Leu Gly Phe
        35                  40                  45
Leu Gly Val
    50

<210> 583
<211> 60
<212> PRT
<213> Homo sapiens

<400> 583


<210> 584
<211> 76
<212> PRT
<213> Homo sapiens

<400> 584
Met Cys Leu Cys Ile Pro Leu Gly Gly Tyr Gln Glu Leu Cys His Cys
 1               5                  10                  15
Met Ser Thr Ser Asp Gly Phe Ala Pro Pro Pro Gln Leu Gly Ser Arg
            20                  25                  30
Cys Ser His Ile Arg Gly Pro Ile Lys Ile Ala Arg Asn Lys Phe Pro
        35                  40                  45
Arg Thr Leu Thr Ser Gln Glu Leu Arg Arg Phe Ala Glu Tyr Ser Gly
    50                  55                  60
Met Met Phe Gly Asp Gln Thr Thr Ala Gly Gln Lys
65                  70                  75


<210> 585
<211> 50
<212> PRT
<213> Homo sapiens

<400> 585
Met Val Tyr Arg Phe Gly Gln Met Ser Asp Asn Pro Phe Tyr Ile Leu
 1               5                  10                  15
Ala Ser Leu Gly Ser Ser Ser Cys Arg Asn Gly Leu Ala Ser Lys Trp
            20                  25                  30
Arg Gln Ala Asp Pro Ser Asp Gly Tyr Met Glu Pro Cys Phe Gln Leu
        35                  40                  45
Leu Phe
    50


<210> 586
<211> 60
<212> PRT
<213> Homo sapiens

<400> 586
Met Leu Val His Ile Tyr Ser Cys Cys Gly Met Val Tyr Arg Phe Gly
 1               5                  10                  15
Gln Met Ser Asp Asn Pro Phe Tyr Ile Leu Ala Ser Leu Gly Ser Ser
            20                  25                  30
Ser Cys Arg Asn Gly Leu Ala Ser Lys Trp Arg Gln Ala Asp Pro Ser
        35                  40                  45
Asp Gly Tyr Met Glu Pro Cys Phe Gln Leu Leu Phe
    50                  55                  60


<210> 587
<211> 1408
<212> DNA
<213> Homo sapiens

<400> 587
ctggacactt tgcgagggct tttgctggct gctgctgctg cccgtcatgc tactcatcgt 60
agcccgcccg gtgaagctcg ctgctttccc tacctcctta agtgactgcc aaacgcccac 120
cggctggaat tgctctggtt atgatgacag agaaaatgat ctcttcctct gtgacaccaa 180
cacctgtaaa tttgatgggg aatgtttaag aattggagac actgtgactt gcgtctgtca 240
gttcaagtgc aacaatgact atgtgcctgt gtgtggctcc aatggggaga gctaccagaa 300
tgagtgttac ctgcgacagg ctgcatgcaa acagcagagt gagatacttg tggtgtcaga 360

aggatcatgt gccacagatg caggatcagg atctggagat ggagtccatg aaggctctgg 420
agaaactagt caaaaggaga catccacctg tgatatttgc cagtttggtg cagaatgtga 480
cgaagatgcc gaggatgtct ggtgtgtgtg taatattgac tgttctcaaa ccaacttcaa 540
tcccctctgc gcttctgatg ggaaatctta tgataatgca tgccaaatca aagaagcatc 600
gtgtcagaaa caggagaaaa ttgaagtcat gtctttgggt cgatgtcaag ataacacaac 660
tacaactact aagtctgaag atgggcatta tgcaagaaca gattatgcag agaatgctaa 720
caaattagaa gaaagtgcca gagaacacca cataccttgt ccggaacatt acaatggctt 780
ctgcatgcat gggaagtgtg agcattctat caatatgcag gagccatctt gcaggtgtga 840
tgctggttat actggacaac actgtgaaaa aaaggactac agtgttctat acgttgttcc 900
cggtcctgta cgatttcagt atgtcttaat cgcagctgtg attggaacaa ttcagattgc 960
tgtcatctgt gtggtggtcc tctgcatcac aaggaaatgc cccagaagca acagaattca 1020
cagacagaag caaaatacag ggcactacag ttcagacaat acaacaagag cgtccacgag 1080
gttaatctaa agggagcatg tttcacagtg gctggactac cgagagcttg gactacacaa 1140
tacagtatta tagacaaaag aataagacaa gagatctaca catgttgcct tgcatttgtg 1200
gtaatctaca ccaatgaaaa catgtactac agctatattt gattatgtat ggatatattt 1260
gaaatagtat acattgtctt gatgtttttt ctgtaatgta aataaactat ttatatcaca 1320
caatawagtt ttttctttcc catgtatttg ttatatataa taaatactca gtgatgagaa 1380
aaaaaaaaaa aaaaaaaaaa rwmgaccc 1408

<210> 588
<211> 81
<212> PRT
<213> Homo sapiens

<400> 588

<210> 589
<211> 157
<212> PRT
<213> Homo sapiens

<400> 589
Met Thr Met Cys Leu Cys Val Ala Pro Met Gly Arg Ala Thr Arg Met
1               5                   10                  15
Ser Val Thr Cys Asp Arg Leu His Ala Asn Ser Arg Val Arg Tyr Leu
            20                  25                  30
Trp Cys Gln Lys Asp His Val Pro Gln Met Gln Asp Gln Asp Leu Glu
        35                  40                  45
Met Glu Ser Met Lys Ala Leu Glu Lys Leu Val Lys Arg Arg His Pro
    50                  55                  60
Pro Val Ile Phe Ala Ser Leu Val Gln Asn Val Thr Lys Met Pro Arg
65                  70                  75                  80
Met Ser Gly Val Cys Val Ile Leu Thr Val Leu Lys Pro Thr Ser Ile
                85                  90                  95
Pro Ser Ala Leu Leu Met Gly Asn Leu Met Ile Met His Ala Lys Ser
            100                 105                 110
Lys Lys His Arg Val Arg Asn Arg Arg Lys Leu Lys Ser Cys Leu Trp
        115                 120                 125
Val Asp Val Lys Ile Thr Gln Leu Gln Leu Leu Ser Leu Lys Met Gly
    130                 135                 140
Ile Met Gln Glu Gln Ile Met Gln Arg Met Leu Thr Asn
145                 150                 155

<210> 590

<211> 347
<212> PRT
<213> Homo sapiens

<400> 590
Met Leu Leu Ile Val Ala Arg Pro Val Lys Leu Ala Ala Phe Pro Thr
1               5                   10                  15
Ser Leu Ser Asp Cys Gln Thr Pro Thr Gly Trp Asn Cys Ser Gly Tyr
            20                  25                  30
Asp Asp Arg Glu Asn Asp Leu Phe Leu Cys Asp Thr Asn Thr Cys Lys
        35                  40                  45
Phe Asp Gly Glu Cys Leu Arg Ile Gly Asp Thr Val Thr Cys Val Cys
    50                  55                  60
Gln Phe Lys Cys Asn Asn Asp Tyr Val Pro Val Cys Gly Ser Asn Gly
65                  70                  75                  80
Glu Ser Tyr Gln Asn Glu Cys Tyr Leu Arg Gln Ala Ala Cys Lys Gln
                85                  90                  95
Gln Ser Glu Ile Leu Val Val Ser Glu Gly Ser Cys Ala Thr Asp Ala
            100                 105                 110
Gly Ser Gly Ser Gly Asp Gly Val His Glu Gly Ser Gly Glu Thr Ser
        115                 120                 125
Gln Lys Glu Thr Ser Thr Cys Asp Ile Cys Gln Phe Gly Ala Glu Cys
    130                 135                 140
Asp Glu Asp Ala Glu Asp Val Trp Cys Val Cys Asn Ile Asp Cys Ser
145                 150                 155                 160
Gln Thr Asn Phe Asn Pro Leu Cys Ala Ser Asp Gly Lys Ser Tyr Asp
                165                 170                 175
Asn Ala Cys Gln Ile Lys Glu Ala Ser Cys Gln Lys Gln Glu Lys Ile
            180                 185                 190
Glu Val Met Ser Leu Gly Arg Cys Gln Asp Asn Thr Thr Thr Thr Thr
        195                 200                 205
Lys Ser Glu Asp Gly His Tyr Ala Arg Thr Asp Tyr Ala Glu Asn Ala
    210                 215                 220
Asn Lys Leu Glu Glu Ser Ala Arg Glu His His Ile Pro Cys Pro Glu
225                 230                 235                 240
His Tyr Asn Gly Phe Cys Met His Gly Lys Cys Glu His Ser Ile Asn
                245                 250                 255
Met Gln Glu Pro Ser Cys Arg Cys Asp Ala Gly Tyr Thr Gly Gln His
            260                 265                 270
Cys Glu Lys Lys Asp Tyr Ser Val Leu Tyr Val Val Pro Gly Pro Val
        275                 280                 285
Arg Phe Gln Tyr Val Leu Ile Ala Ala Val Ile Gly Thr Ile Gln Ile
    290                 295                 300
Ala Val Ile Cys Val Val Val Leu Cys Ile Thr Arg Lys Cys Pro Arg
305                 310                 315                 320
Ser Asn Arg Ile His Arg Gln Lys Gln Asn Thr Gly His Tyr Ser Ser
                325                 330                 335
Asp Asn Thr Thr Arg Ala Ser Thr Arg Leu Ile
            340                 345


<210> 591
<211> 565
<212> DNA
<213> Homo sapiens

<400> 591
actaaagcaa atgaacaagc tgacttgcta gtatcatctg cattcattga agcacaagaa 60
cttcatgcct tgactcatgt aaatgcaata ggattaaaaa ataaatttga tatcacatgg 120
aaacagacaa aaaatattgt acaacattgc acccagtgtc agattctaca cctggccact 180
caggaagcaa gagttaatcc cagaggtcta tgtcctaatg tgttatggca aatggatgtc 240
atgcacgtac cttcatttgg aaaattgtca tttgtccatg tgacagttga tacttattca 300
catttcatat gggcaacctg ccagacagga gaaagtactt cccatgttaa aagacattta 360
ttatcttgtt ttcctgtcat gggagttcca gaaaaagtta aaacagacaa tgggccaggt 420
tactgtagta aagcatttca aaaattctta aatcagtgga aaattacaca tacaatagga 480
attctctata attcccaagg acaggccata attgaaggaa ctaatagaac actcaaagct 540
caattggtta aacaaaaaaa aaaaa                                       565

<210> 592
<211> 188
<212> PRT
<213> Homo sapiens

<400> 592
Thr Lys Ala Asn Glu Gln Ala Asp Leu Leu Val Ser Ser Ala Phe Ile
1               5                   10                  15
Glu Ala Gln Glu Leu His Ala Leu Thr His Val Asn Ala Ile Gly Leu
            20                  25                  30
Lys Asn Lys Phe Asp Ile Thr Trp Lys Gln Thr Lys Asn Ile Val Gln
        35                  40                  45
His Cys Thr Gln Cys Gln Ile Leu His Leu Ala Thr Gln Glu Ala Arg
    50                  55                  60
Val Asn Pro Arg Gly Leu Cys Pro Asn Val Leu Trp Gln Met Asp Val
65                  70                  75                  80
Met His Val Pro Ser Phe Gly Lys Leu Ser Phe Val His Val Thr Val
                85                  90                  95
Asp Thr Tyr Ser His Phe Ile Trp Ala Thr Cys Gln Thr Gly Glu Ser
            100                 105                 110
Thr Ser His Val Lys Arg His Leu Leu Ser Cys Phe Pro Val Met Gly
        115                 120                 125
Val Pro Glu Lys Val Lys Thr Asp Asn Gly Pro Gly Tyr Cys Ser Lys
    130                 135                 140
Ala Phe Gln Lys Phe Leu Asn Gln Trp Lys Ile Thr His Thr Ile Gly
145                 150                 155                 160
Ile Leu Tyr Asn Ser Gln Gly Gln Ala Ile Ile Glu Gly Thr Asn Arg
                165                 170                 175
Thr Leu Lys Ala Gln Leu Val Lys Gln Lys Lys Lys
            180                 185


<210> 593
<211> 271
<212> DNA
<213> Homo sapiens

<220>
<221> misc_feature
<222> 12, 19, 23, 26, 41, 59, 66, 71, 103, 145, 147, 151, 158,
166, 171, 181, 186, 189, 219, 228, 230, 242, 246, 249, 257,
265
<223> n = A,T,C or G

<400> 593
actttatgtt cnagtgcana aanccncctg gattgccacc ntactctcag ggctgtgant 60
tgtgcnccca nagcaacctg ggcacgcggg gacagggggg ccnacaattg agggagcggt 120
gtccctagct ggggtctata catgncnggg naagggcngc tgagtnccat nagcaaagga 180
nctagnatnt gcgggggtgc ggcctgggcc taccctttna agcatccntn gatccactcc 240
angaanccng gggtagncag gtttnccaac a                                271

<210> 594
<211> 376
<212> DNA
<213> Homo sapiens

<220>
<221> misc_feature
<222> 11, 33, 54, 69, 70, 90, 91, 102, 113, 116, 118, 131, 148,
172, 200, 206, 209, 220, 224, 230, 248, 251, 269, 285, 287,
290, 297, 307, 325, 347, 351, 353, 357, 376
<223> n = A,T,C or G

<400> 594
cctttggggg nggggggaac ctttaccatt gtnccccttt atttcatttg gttngggttc 60
gcgccctcnn gggccaacaa agttatcgtn nttgaagaga anattttttt ggnttngncc 120
cgattaagcg ncaaatgtgt agcaaaangc cgtgccactt gtggcgtagc tncgtcgggt 180
cgattcgacg acaaggcgtn gcgcgntanc gttagtctcn aatngacccn gtggcatgag 240

cccacgangg nttcgtgtcg tcacatggnc tctagacata acgcncnccn ttttttncag 300
agggggntgc cgcccttagg gaggnagggg tggggacact agccaancca nantctnacc 360
ccattgaaga aaaggn 376

<210> 595
<211> 242
<212> DNA
<213> Homo sapiens

<220>
<221> misc_feature
<222> 3, 10, 15, 20, 34, 35, 45, 49, 63, 65, 79, 113, 116, 127,
133, 152, 156, 160, 167, 187, 191, 196, 204, 206, 231, 233,
235
<223> n = A,T,C or G

<400> 595
agnctgctgn tcgtnccctn tatgtggctt catnntgagg acaanagtng cactgaggct 60
tgngnatgcc aggcaaggnc aagctggctc aaaaagcatc cacccacctc tgnaangggt 120
atgccangag cangtgcacc agtcccaact angagncccn ggcatgntac atcttcttcc 180
acccctnaaa ntttgngcta caangnccat ttttcttttt ctcttaaggg ncncntggct 240
tc 242

<210> 596
<211> 535
<212> DNA
<213> Homo sapiens

<220>
<221> misc_feature
<222> 21
<223> n = A,T,C or G

<400> 596
accagttgga tactgctaaa nagatattta tgcagcctca tatgttaagt cgtatatttt 60
gaaagctttt taaatttttt ctttaagaag attttagatg cttatcactg agtaccagag 120
ggatgtaggc tgatgccctt atcaacaaag tcagggactg tggcacacaa ggattgacta 180
ctgcagacac ggccacaatg ctacctctag agggcctgaa tccccctgcc ctctctggtg 240
gggagaaggg ctggcagagc cattagcatg ggctccggcc aatcctggcc actttgacac 300
tcctggtgct gacccagggt cctggaggaa gggatgaggt gggcagtaga gatgctcagg 360
gcagtggccc ctttccatcc acactggaac tatttcagta ttttaccacc aattcagcca 420
ttcccttgtg cgctggctga acatcagccc tgctccaggt ctcagtttcc cctttgtaaa 480
gggaaagctc tggattcagg gagtgatgaa gaggtcatca tggtcttgag aattc 535

<210> 597
<211> 257
<212> DNA
<213> Homo sapiens

<220>
<221> misc_feature
<222> 5, 16, 29, 35, 46, 62, 64, 69, 86, 87, 101, 116, 120, 124,
135, 136, 140, 171, 182, 196, 235, 236, 241, 246, 250
<223> n = A,T,C or G

<400> 597
tttcnatacc caaaantacc ccatattang accanacatt tgtctnggaa aaattaccat 60
tntntaacnt ttgggccacc tgagannaaa tgggtgtaat ncatgataag atggancagn 120
attnctctta agatnngatn agaccccgtt tttcacggaa catatccaag nacccaatag 180
gnaacaagcc acgggnggag tcacaaacat atattcttta ctctcataat ccgtnncaca 240
naactnttgn acttgac 257

<210> 598
<211> 222
<212> DNA
<213> Homo sapiens

<220>

<221> misc_feature
<222> 1, 2, 6, 14, 21, 22, 101, 108, 120, 121, 124, 134, 135, 139,
144, 145, 158, 163, 196, 216
<223> n = A,T,C or G

<400> 598
nntggntacc gtcnaaactt nncttggtac ccgagctcgg atccactagt ccagtgtggt 60
ggaattccat tgtgttgggc tataagctgt aatagtggag ncgtgctngg ttcattgcan 120
nagnccctcc gcanncacnc ttgnnacaac ctgtgagnag gcnataaatt attcacataa 180
tcatcactgc atgaanctga ctcaaacgca tccacntaca cc 222

<210> 599
<211> 238
<212> DNA
<213> Homo sapiens

<220>
<221> misc_feature
<222> 12, 19, 25, 26, 33, 37, 47, 49, 51, 60, 64, 75, 120, 128,
136, 138, 140, 182, 224
<223> n = A,T,C or G

<400> 599
gcatgacatc ancgatgtnt ttggnnacct ganattngct aaaactngng natgccgggn 60
atgnaggttt ggtantgatc tatgcactca catctcatgg ggacgtttca tgtggagtgn 120
tcgacaangt tgctgnancn gagaagtgat gatctcagtt gaaagggtca tgtgaataca 180
cnttacactt gaaaaagaag cacattggga atatcacgaa acgnccacca acatcctg 238

<210> 600
<211> 232
<212> DNA
<213> Homo sapiens

<220>
<221> misc_feature
<222> 167
<223> n = A,T,C or G

<400> 600
cgaactattt agactaccta ggaaaattat tttagtatca gaagaatatc aggggtgtag 60
tactcatcag agctaaatga gagcgcttta aaaatgttag tttgtcttcc gccatttcta 120
cagaaagctg caatttcagg ttttcaacct aataggtgat atttaanaaa aaaaaaaagc 180
aatcgcaaat agccccactg cttttacaaa tcattttttc cccaacacaa tg 232

<210> 601
<211> 547
<212> DNA
<213> Homo sapiens

<220>
<221> misc_feature
<222> 183, 282, 301, 308, 325, 333, 348, 368, 373, 421, 422, 431,
436, 491, 518, 523
<223> n = A,T,C or G

<400> 601
cattgtgttg gggaaaaaat gatttgtata agcagtgggg ctatttgcga ttgctttttt 60
tttttcttaa atatcaccta ttaggttgaa aacctgaaat tgcagctttc tgtagaaatg 120
gcggaagaca aactaacatt tttaaagcgc tctcatttag ctctgatgag tactacaccc 180
ctnatattct tctgatacta aaataatttt cctagtgtag tctaaacttt tttaaaaaga 240
catgtaatcc gcggagttag taactcaaaa cgagtgcatc tnggaagtat cgcagccgtt 300
nctggatnaa attcccagct tgctngcttg ctnagccggg gggcggtnaa aaaaacatct 360
gcagcccngg ggnaaaaacc ttcgcattgt tcttacgtgt ttacgttatt ttatttccct 420
nnagcaaggc nggganttgg ggactcgaaa tggtacagtt gggctgggga tcgcccttgt 480
tacataaaag ncgtccagaa gagggacggt tacaggcngg ganctccaaa ggtcagtccc 540
tgccatt 547

<210> 602

<211> 826
<212> DNA
<213> Homo sapiens

<220>
<221> misc_feature
<222> 8, 9, 359, 431, 447, 470, 628, 691, 693, 699, 710, 737, 761,
781, 790, 792, 800
<223> n = A,T,C or G

<400> 602
cggggggnnt tacgtctctc tggacgcttt tattgtacca gggcgatccc agcccaactg 60
taccattcga gtccctactc ctgccttgct ctagggaaat aaaataacgt aaacacgtaa 120
gaacaatgcg aaagcgtttt cttccctagg ctgcagattg tcttcttcac cgcccctgct 180
tagctagcta gctagctggg aatttaatcc agaaacggct tgcgatacct cctagatgca 240
ctcgttttga gttacaaact ccgcggatta catgtctttt taaaaaagtt tagactacac 300
tagggaaaat tattttagta tcagaagaat atcagggggt gtagtactca tcagagctna 360
atgagagcgc tttaaaaatg ttagtttgtc ttccgccatt tctacagaaa gctgcaattt 420
caggttttca ncctaatagg tgatatntaa gaaaaaaaaa acaatcgcan atagcccact 480
gcttttacaa atcatttttc tcttctaggt atagcctgtc aggtggccta atgtattttt 540
gacatctcta ggaattttaa tagaccagaa atgggtgcca gagatatgcc tgcactaatc 600
ttaagtgggg atttatgtat ttctcaanca agtgattaaa gcaaaactag gcacgaatga 660
aatcaagatc tttaggccag aaatcatgaa nanttttana attattttan gaatctgtgg 720
cttctcttct taaaatngaa aaaaaaattg tttaaaccca naaggtctga atacccaagc 780
nccctgaacn anagaacaan gccggagcac cccctcccaa atcccc 826

<210> 603
<211> 817
<212> DNA
<213> Homo sapiens

<220>
<221> misc_feature
<222> 1, 2, 4, 17, 28, 613, 617, 688, 697, 743, 751, 756, 762,
777, 799
<223> n = A,T,C or G

<400> 603
nnangacttt tgtggtntta tacaattntt ttttctattt ctatgaagag aaagccacag 60
agtcctaaaa taattctaaa actcatcatg actttcttgc ctaaaagatc ttgatttcaa 120
tcgtgcctag ttttgcttta atcacttgct tgagaaatac ataaatcccc acttaagatt 180
agtgcaggca tatctctggc acccatttct ggttctatta aaattcctag agatgtcaaa 240
aattacatta ggccacctga caggctatac ctagaagaga aaaaatgatt tgtaaaagca 300
gtggggctat ttgcgattgc tttttttttt tcttaaatat cacctattag gttgaaaacc 360
tgaaattgca gctttctgta gaaatggcgg aagacaaact aacattttta aagcgctctc 420
atttagctct gatgagtact acacccctga tattcttctg atactaaaat aattttccta 480
gtgtagtcta aactttttta aaaagacatg taatccgcgg agtttgtaac tcaaaacgag 540
tgcatctagg aggtatcgca agccgtttct ggattaaatt cccagctagc ttgcttgctt 600
agcaggggcg ggnaaanaag acatctgcag cctagggaag aaaacctttc gcattgttct 660
tacgtgttta cgttatttta tttcctanaa caaggcngaa ttgggactcg aatggttcag 720
ttggggtggg ggatcccctg gtncataaaa ngtcanaaag anggtacagg cggaacncca 780
agggtcgtcc tgcatttana ctcggaattt tggtgcc 817

<210> 604
<211> 694
<212> DNA
<213> Homo sapiens

<220>
<221> misc_feature
<222> 18, 33, 71, 202, 318, 330, 353, 432, 467, 476, 492, 509,
519, 521, 533, 543, 571, 608, 609, 663, 667, 689, 691
<223> n = A,T,C or G

<400> 604
cttttcaaat catttttnct cttctaggta tancctgtca ggtggcctaa tgtaattttt 60
gacatctcta ngaattttaa tagaaccaga aatgggtgcc agagatatgc ctgcactaat 120
cttaagtggg gatttatgta tttctcaagc aagtgattaa agcaaaacta ggcacgattg 180

aaatcaagat cttttaggca anaaagtcat gatgagtttt agaattattt taggactctg 240
tggctttctc ttcatagaaa tagaaaaaaa aattgtataa aaccacaaaa ggtcctgaat 300
agccaaagca acactganca aaaagaacan agcagggaag caacacacta ccngaattca 360
aattatacta ccagggtgta gtaaccaaaa cagcattcta ttggcataaa atagacacca 420
agaccaatgg ancagaataa agaaccccac aaataaatcc atatatntac cgccanctga 480
ttatcaataa cnaacaccaa gaacatatnt taagggacnt nctattcaat aantagtgct 540
ggnaaaaact gggaaatcca tatgcagaaa naatgaaact agacccctat ccctcaccat 600
acgcaaannt caacttcgga atgggattac aaaacttaag acattccaac ccaagaaact 660
atnaaancta ctattaagaa aacagatcnc nccc 694

<210> 605
<211> 678
<212> DNA
<213> Homo sapiens

<220>
<221> misc_feature
<222> 34, 69, 388, 422, 527, 582, 648, 672, 677
<223> n = A,T,C or G

<400> 605
taaaaatcta gactacacta ggaaattatt ttantatcag aagaatatca ggggtgtagt 60
actcatcana gctaaatgag agcgctttaa aaatgttagt ttgtcttccg ccatttctac 120
agaaagctgc aatttcaggt tttcaaccta ataggtgata tttaagaaaa aaaaaaagca 180
atcgcaaata gccccactgc ttttacaaat cattttttct cttctaggta tagcctgtca 240
ggtggcctaa tgtaattttt gacatctcta ggaattttaa tagaaccaga aatgggtgcc 300
agagatatgc ctgcactaat cttaagtggg gatttatgta tttctcaagc aagtgattaa 360
agcaaaacta ggcacgattg aaatcaanat cttttaggca agaaagtcat gatgagtttt 420
anaattattt taggactctg tggctttctc ttcatagaaa tagaaaaaaa aaattgtata 480
aaaaccacaa aaggtcctga atagcccaaa gcaacactga acaaaangaa caaagcagga 540
agcaacacac taccggaatt caattatact accaaggtgt antaaccaaa acagcattct 600
attgggcata aaatagacca aagaccagtg ggaaacagaa taaagaancc caaaataaat 660
cctatattta cngcccnc 678

<210> 606
<211> 263
<212> DNA
<213> Homo sapiens

<220>
<221> misc_feature
<222> 9, 13, 75, 99, 113, 126, 129, 132, 158, 160, 165, 173, 179,
198, 200, 212, 215, 222, 223, 237, 240, 241, 255
<223> n = A,T,C or G

<400> 606
gtggggtcng cancagccaa ctcagcttcc tttcgggctt tgttagcaga cggatcatcc 60
tctagtccac tgtgntcaaa ttccattgtg tgggggccnc tcgcctcggc canagatctg 120
agtgancana cntgtcccca ctgaggtgcc ccacagcngn ttgtnttcag cangggctna 180
caactcgacc ggcagcgnan ggctggcaga antgngcgcc tnnctcattc ctacgcngtn 240
ngccgcagga aggangacag gcc 263

<210> 607
<211> 22
<212> DNA
<213> Artificial Sequence

<220>
<223> Primer

<400> 607
ccatgtgggt cccggttgtc tt 22

<210> 608
<211> 22
<212> DNA
<213> Artificial Sequence

<220>
<223> Primer

<400> 608
gataggggtg ctcaggggtt gg                                                22

<210> 609
<211> 40
<212> DNA
<213> Artificial Sequence

<220>
<223> Primer

<400> 609
gctggacagg gggcaaaagc tggggcagtg aaccatgtgc                             40

<210> 610
<211> 27
<212> DNA
<213> Artificial Sequence

<220>
<223> Primer

<400> 610
ccttgtccag atagcccagt agctgac                                           27

<210> 611
<211> 46
<212> DNA
<213> Artificial Sequence

<220>
<223> Primer

<400> 611
gatagagaaa accgtccagg ccagtattgt gggaggctgg gagtgc                      46

<210> 612
<211> 40
<212> DNA
<213> Artificial Sequence

<220>
<223> Primer

<400> 612
gcacatggtt cactgcccca gcttttgccc cctgtccagc                             40

<210> 613
<211> 38
<212> DNA
<213> Artificial Sequence

<220>
<223> Primer

<400> 613
gccgctcgag ttagaattcg ggttggccca cgatggtg                               38

<210> 614
<211> 53
<212> DNA
<213> Artificial Sequence

<220>
<223> Primer

<400> 614
cggcgggcat atgcatcacc atcaccatca catcataaac ggcgaggact gca        53

<210> 615
<211> 46
<212> DNA
<213> Artificial Sequence

<220>
<223> Primer

<400> 615
gcactcccag cctcccacaa tactggcctg gacggttttc tctatc                46

<210> 616
<211> 1350
<212> DNA
<213> Homo sapiens

<400> 616
atgcatcacc atcaccatca catcataaac ggcgaggact gcagcccgca ctcgcagccc 60
tggcaggcgg cactggtcat ggaaaacgaa ttgttctgct cgggcgtcct ggtgcatccg 120
cagtgggtgc tgtcagccgc acactgtttc cagaactcct acaccatcgg gctgggcctg 180
cacagtcttg aggccgacca agagccaggg agccagatgg tggaggccag cctctccgta 240
cggcacccag agtacaacag acccttgctc gctaacgacc tcatgctcat caagttggac 300
gaatccgtgt ccgagtctga caccatccgg agcatcagca ttgcttcgca gtgccctacc 360
gcggggaact cttgcctcgt ttctggctgg ggtctgctgg cgaacggcag aatgcctacc 420
gtgctgcagt gcgtgaacgt gtcggtggtg tctgaggagg tctgcagtaa gctctatgac 480
ccgctgtacc accccagcat gttctgcgcc ggcggagggc aagaccagaa ggactcctgc 540
aacggtgact ctggggggcc cctgatctgc aacgggtact tgcagggcct tgtgtctttc 600
ggaaaagccc cgtgtggcca agttggcgtg ccaggtgtct acaccaacct ctgcaaattc 660
actgagtgga tagagaaaac cgtccaggcc agtattgtgg gaggctggga gtgcgagaag 720
cattcccaac cctggcaggt gcttgtggcc tctcgtggca gggcagtctg cggcggtgtt 780
ctggtgcacc cccagtgggt cctcacagct gcccactgca tcaggaacaa aagcgtgatc 840
ttgctgggtc ggcacagcct gtttcatcct gaagacacag gccaggtatt tcaggtcagc 900
cacagcttcc cacacccgct ctacgatatg agcctcctga agaatcgatt cctcaggcca 960
ggtgatgact ccagccacga cctcatgctg ctccgcctgt cagagcctgc cgagctcacg 1020
gatgctgtga aggtcatgga cctgcccacc caggagccag cactggggac cacctgctac 1080
gcctcaggct ggggcagcat tgaaccagag gagttcttga ccccaaagaa acttcagtgt 1140
gtggacctcc atgttatttc caatgacgtg tgtgcgcaag ttcaccctca gaaggtgacc 1200
aagttcatgc tgtgtgctgg acgctggaca gggggcaaaa gctggggcag tgaaccatgt 1260
gccctgcccg aaaggccttc cctgtacacc aaggtggtgc attaccggaa gtggatcaag 1320
gacaccatcg tggccaaccc cgaattctaa                                  1350

<210> 617
<211> 449
<212> PRT
<213> Homo sapiens

<400> 617

<210> 618
<211> 385
<212> DNA
<213> Homo sapiens

<220>
<221> misc_feature
<222> 26, 51, 91, 156, 161, 184, 202, 214, 220, 254, 286, 289,
291, 304, 312, 321, 340, 344, 348, 357, 371, 377, 384
<223> n = A,T,C or G

<400> 618
ctgtgctgag aaccaaaagc tatgancact gcttttccaa atgtccataa naccaacatt 60
tttatcacta ccaccatcac ctgggagctc nttagaaagc tagtctcccg ggcaccaccc 120
tggcctactg aacctaatgt gcatttaaca agattnacgt ngaaatctgc aaagcacagg 180
ggcngataac agtaccacct gntctggttc ctanccccan gacccttaca gtctaactgg 240
gacacaaggg cttnaaatca aattgcctat cattaagata tacaanganc ntgagaaact 300
gctncactta tntattaagg ngctctaaga cttagaaacn aaangcantg ctgagangat 360
tcaaatatga ngggggncac tttnc 385

<210> 619
<211> 869
<212> DNA
<213> Homo sapiens

<220>
<221> misc_feature

<222> 685, 689, 692, 717, 719, 750, 754, 762, 801, 803, 810, 817,
844, 845, 847, 856, 859, 862
<223> n = A,T,C or G

<400> 619
gatatcccgg gaattcgcgg ccgcgtcgac ctctacttgt ttagacataa atgcagtcta 60
gcattaaaga tcctttaaaa aaatgttttc ccaatggtta aaagacaagc tcaaataaat 120
gaactctcat acatatgcca aaattgatga gtagataaat atttcagtag gtagttacta 180
gctttctgtg tatgagtaaa catatgggag aaatttaaaa cactaaagta gactcaatga 240
aagcatagta tcctatgtat tcgtttttca gaaatgtcta atgaaggaag gaaacaatga 300
atgaatgccc ttattcctct tagagtgctg ggacatggtt ttgcctgaaa acttcatgtg 360
aattttatat tttgctacac attacaccca tcttagactt atacgtataa gacataaggc 420
atatcttatg tcttacatgt ataataatct aagcagaaca aaaaataacg aaatattttc 480
ttccccaaat ttttgagaca gatggatttt ccggaaagat gtgtttagct tttaatcctg 540
tggttttgtg taccacctgg cacactagag tgttgctcta attcagtgag ttgtaactct 600
gggtgaacag tggaaatact agggtacatt ttaaaaatgc taatgctcgg gcctcgctga 660
agaccaaatt aattggaatc tctgngggng gnattgatct ttttataatc tttctanang 720
attctaatgg gcttccaggg atgaaaaccn ctgntggagc tnggaacctt cctttagttt 780
ggagaaaccc cgatgagggt ntnttaggcn ccgcctnttt ttggcctggg cttcccccct 840
tatnntnttt tggaanggnc cnaattttt 869

<210> 620
<211> 339
<212> DNA
<213> Homo sapiens

<220>
<221> misc_feature
<222> 2, 12, 60, 126, 245, 285, 299, 308, 328
<223> n = A,T,C or G

<400> 620
gngcgggcct cnccgtgctt gctctcgctg ccgacgctct ttttccacca gctgtaggan 60
aagcccgaag accactggtc ccccgggtag cccaagtacc actggtcctc ctggctcctg 120
acgctncggg tcttcctcgt ggcgtagact gccagcttcg gagacccctc agcccctccc 180
cgcttttctc caccccagga ggccatcagt agcgagctac tgcctcggcc acaacctccc 240
agcangatag cccgcggttt ccaatctgcg aaaggaggac cgccnagccc gaaatgccna 300
gcccagcnat cactgccacg ccgagccnag cgctcgtgc 339

<210> 621
<211> 267
<212> DNA
<213> Homo sapiens

<220>
<221> misc_feature
<222> 5, 16, 177, 213, 227, 232, 251, 256
<223> n = A,T,C or G

<400> 621
ggggngcatg gtcccnggta gccaagtaca tggtcctcct ggctcctgac gctacgggtc 60
ttcctcgtgg cgtagactgc cagcttcgga gacccctcag cccctccccg cttttctcca 120
ccccaggagg ccatcagtag cgagctactg cctcggccac aacctcccag caggatngcc 180
cgcggtttcc aatctgcgaa aggaggaccg ccnagccaga aatgccnagc cnagcgatca 240
ctgccacgcc nagccnagcg ctcgtgc 267

<210> 622
<211> 847
<212> DNA
<213> Homo sapiens

<220>
<221> misc_feature
<222> 5, 7, 30, 656, 673, 678, 746, 750, 760, 791, 825, 826, 836
<223> n = A,T,C or G

<400> 622
cttangntgt cgactgacgt catgcatgan ttaaagcaga ggtttggtga aatttatgaa 60

aaatacaaaa ttccggcttg tcctgaggaa gagccactac ttgataactc tacaagagga 120
acagatgtga aggatattcc ctttaatttg acaaataaca tacctggttg tgaggaagaa 180
gatgcatctg aaatatctgt ctcagtggta ttcgagacat ttcctgaaca aaaagaaccc 240
agtctcaaaa atatcatcca tccatactat catccgtact ctgggtccca ggaacatgtt 300
tgccagtcat cttctaagct tcatttacat gaaaataaat tagactgcga caatgataac 360
aaactaggca ttggacatat ttttagtaca gataacaact ttcataatga tgcaagcact 420
aagaaagcaa ggaacccaga agtggttacg gttgaaatga aagaagacca agagtttgat 480
ttgcaaatga caaaaaatat gaaccaaaat agtgacagtg gcagtacaaa taactataaa 540
agcctgaaac ctaaattaga aaatctgagt tctttaccac cagattctga cagaacatca 600
ggaagtatat ctacatgaag aattacagca agacatgcca aaagtttaag aatgangtca 660
acacattaga aanaagantt ctgggctttg aagaaagaaa atgttccact tcataaagaa 720
ggttgaaaga agaatgggag agcccngaan tttttgcccn gaaattttcg ggaaccctac 780
tggatgggtc nactggttgg ccatgaatga ataatggact aatcnnccaa ttcctnggga 840
agggaat 847

<210> 623
<211> 681
<212> DNA
<213> Homo sapiens

<220>
<221> misc_feature
<222> 64, 70, 115, 121, 137, 160, 166, 188, 210, 299, 382, 406,
481, 527, 544, 570, 622, 629, 639, 665, 670, 681
<223> n = A,T,C or G

<400> 623
aaaactgtac tcgcgcgctg catgtcgaca ctagtggatc caaagaatcg gcacgagcga 60
aaangctcan gcagcccggc tggccgccgc cgctcctccc cccaggaaag ccaangtgga 120
ngctgatgtg gctgcangag ctcgtttcac agcccctcan gtgganctgg ttgggccgcg 180
gctgccangg gcggaagtgg gtgtccccan gtctcagccc caaggctgcc cctcacaaag 240
cactggtggt ttgcctccac tgccaccttg ggctccgaac ccgctcccct gctgtggang 300
cccaccgtgg gaatccaggt ccccaggtgg actgcctgcc ttgccctcac tgcccactct 360
gcccacactt ccctgcctag anaccgggaa ggggctgtgt cggtantggt gcccacctgg 420
atgtggcagc accgactgtg gggtggaccc tggccttgcc gggtgcaaaa gtgggggccc 480
ngggaaaagc acctgaagtg gccctgaaaa atcccccctt aattttnccc caatttgggg 540
ctcnaacaaa aggaaattgc tgaagccaan ggtaccaagg tcacccctaa ggccagggtg 600
aaaaggtccc aaaattccaa tncccaccnt ttgggcttnc ctcttggaac cccggccccc 660
tctcntgaan ttttaaaaaa n 681

<210> 624
<211> 661
<212> DNA
<213> Homo sapiens

<220>
<221> misc_feature
<222> 174, 217, 231, 302, 309, 326, 397, 430, 459, 460, 488, 507,
530, 532, 535, 559, 563, 571, 585, 599, 634, 646, 647
<223> n = A,T,C or G

<400> 624
attggtctta ctgtaccacc gggtggaaat cgatggccgc ggcgtctaaa tatccgattt 60
tttttttttt tcctcttctg actgtccatg gacaaatgaa actaacttaa tctaactaaa 120
aaacacaact atattttgaa gattttctat ctgcactcaa ggacactttc cacncggttg 180
ttgttacctt ttggtcttgt ctctgaacat gaaattnatc tcaagggatt ngatttctgg 240
acctcctatt cctgctatgg gtttgatatt tcttgggctc cagggccact gttgcattgg 300
gntgacagnt acctcctagc ccatancctc ctatcttggg aaacaaacct aacaactacg 360
tgtaccttcc atagatctct gattgagtct cagtatncgc ttgctcatgg gcgattcact 420
tgaatccgtn attggtgcca acaatcctga ctcatgggnn aatggatcct atcacgttcc 480
cctgattngc aacccctgta tacatanatc taatcgcata gaatctagcn tnggntatgc 540
gcggctacgc tatcagggnt tgntaactat ngcatggcta cgaancctga tcatgatcna 600
gggtcatgga ctcttatcag gggggttggg ccgngcttct ttttcnnacc ttggtaaaac 660
c 661

<210> 625
<211> 181
<212> DNA

<213> Homo sapiens

<400> 625
gcaacaatca gatcatgtta aagtaaatct ccattgccct ggatcacttc aggatttaat 60
tgtccaagga gagcagggtt ctcctgtgaa aaaaaggtgg ggaaatgttt gagagtaaaa 120
aatacaaaat tcaaccggtc gaaaatacac cactccattc agtgctctac ccccataagc 180
c 181

<210> 626
<211> 181
<212> DNA
<213> Homo sapiens

<400> 626
gcaacaatca gatcatgtta aagtaaatct ccattgccct ggatcacttc aggatttaat 60
tgtccaagga gagcagggtt ctcctgtgaa aaaaaggtgg ggaaatgttt gagagtaaaa 120
aatacaaaat tcaaccggtc gaaaatacac cactccattc agtgctctac ccccataagc 180
c 181

<210> 627
<211> 813
<212> DNA
<213> Homo sapiens

<220>
<221> misc_feature
<222> 571, 642, 704, 713, 726, 728, 734, 751, 774, 779, 784, 790,
791, 803, 812
<223> n = A,T,C or G

<400> 627
accaagctgg agctcgcgcg cctgcaggtc gacactagtg gatccaaagt gaacgtgaag 60
gtgagcagag gagaacttgc gatggcaaag ttaaaaacaa gaggagatga tggtcttggt 120
gtggcacagg atgttaaaaa aattctcctg tccttaagga gttactgcta tttgagtaat 180
gtgccacttc cctacatagc cttctatgca gaaatgctat atttccactt cacaacccag 240
aacgtgcatt ttattttaca tttagaggag gaacaaacaa ccagaaggca aaaactggtg 300
cattattttt tgcaattctc ttggaaagag ttcgttttta acttctgctc agacagcaca 360
caactactgg gaatatattt taatttcaaa tctgatgtgt gacatctggt aactcattta 420
ttgctaatga agttttcaca ggaagcagca gtcaccagta gctcatctta tttttcagtt 480
ggcaaagtgt tgtttacctt ttattggcct gcatcggtgt ctcttatcac aggatattta 540
attagaaaac gcaagtagcc taacatagaa nagaaatgga gtggtagata atagtagata 600
gaatggctaa atatttttat tacagtgatg taatatcact gnaatttatg gttaaaaatt 660
atgtaatact caaaaggaat tctcagactg gcgaaacagc tggncaacag ctntcacagg 720
gctttnanct cctnttgagc tttccccctg ntggacttta gtcttccttt tacncccgna 780
gttnccattn nttaccaatt gtnccgggaa ana 813

<210> 628
<211> 646
<212> DNA
<213> Homo sapiens

<220>
<221> misc_feature
<222> 7, 10, 19
<223> n = A,T,C or G

<400> 628
tttgggnggn ggtgtctcnt ttgggtggac tttttgggtc gtagggcccc aaggccgtta 60
atcccgtaat aacggaagac gaagaagagt cagaagagtg cttctataag gatcgggacg 120
agactacctt agaggaataa aggaaaaaag cagaggagga agagtggtag aaggagtcag 180
aagaaaccca cacgtcgttc tgaacctgga gccttatcaa aaaggtctag ataaacgata 240
gcgatctcga tatcgagctc aagaggtagg tttagagact tctcgtcctc gagagcgaaa 300
tggaagatct cgacgacgat aagaagttaa agtgtagagg gtgcttgagg agcgcgtgga 360
aggattctgc ggagggaccc atcgacgtag agacttgaag gcctactaag gtccacaaga 420
agcccggctc tttctccgaa tggtcggagc gtacagtatg cgacgtcgat cggcagacaa 480
gctggcggta gactcgaagt gttcgggcga atcgacttat aatagtcgcg cgctagtaac 540
gtaggaacac gaagagtagt cgaaagaaaa cgtttagtga gggaaaagat tagggaaaaa 600
ggagaggctt aataactaag acacttggag cctaggccaa cgcgaa 646

<210> 629
<211> 617
<212> DNA
<213> Homo sapiens

<220>
<221> misc_feature
<222> 7, 18, 27, 545, 600, 613
<223> n = A,T,C or G

<400> 629
gcccccnccc ccctcctngg gcttatnggg acagacccac gtagtactct aaatcttctc 60
ctacgccgga caacggaccc tataccaatt cgaatcttgg acactccgac cgccggattc 120
tcttcccctt tcggcttccc ctttctgtcg gtacccctcc ctagtcgtct cctacacctt 180
cgtaccgtcg atatatagtc gccgcggact agcctattta ggtgtcctag actcgttatt 240
gatccactca ttagtctagt actatgcgtc acgtatctta gttgcctaag agggagatta 300
aatcctccac aagttccgac gaattcctgg actctcgtac tagcaaactt tcttatgagg 360
cttccttgta tatcttctgg atgtttctcg tgtcccggtc ctccgctact actagagctc 420
cttgccctat ctctagaagt agaggactct cgggttcgtt ctccaaatct agcgctagag 480
ctatcgctac ccgctcgatt cccccagcgg aatcttgaaa cctgaggtag tacacaaacc 540
ctccncatct tccctcggtt gctccttctt ctcatccccc cttcccgcct tctcgggaan 600
gaatctactt tancttc 617

<210> 630
<211> 644
<212> DNA
<213> Homo sapiens

<220>
<221> misc_feature
<222> 2, 3, 9, 18, 26, 27, 28, 436, 520, 538, 583, 588, 633
<223> n = A,T,C or G

<400> 630
cnntcggcnt gggttttntt ctgagnnncc cccccccccc cccccccaaa cttacaccca 60
ccaaacactt tccgccccct acctaggaga cattagaagg gtttaggctt cggcgtatag 120
taaagtcctc tacctcggaa gtagagaatt cggtatttaa attcagggtt agaggctcgc 180
tcgttagatt tatagtttag gtttagaatc ggaaaccttc gatcttcctt agaagggtaa 240
taagtgaggc cctaaatccg tctaaccaag gcgttaaggt ccgtacctaa acctagtctt 300
atcttctatc aggcgcacca atataggtag gttctacttt cgtataggcc ttaaggaata 360
gttcggtagt tatcgaaggc actcctctct aggctaggct tttctcagtc ttagtactcc 420
gggaccgtcg tcgcanaaat atcgatggac ggtaggtatc tccgcgttac gcgtcgggct 480
agggatatag agcgaattat cggcgagagg cggtcgctan gaatcggtat caatatgntg 540
ttctttaccc tacggatatc ggcagaaaac ataaaacctt ctnaccangg ataagggatt 600
atcggacccc taaaataaca gtaacattta gantactagt accc 644

<210> 631
<211> 526
<212> DNA
<213> Homo sapiens

<220>
<221> misc_feature
<222> 3, 77, 104, 114, 126, 134, 156, 161, 163, 170, 182, 190,
195, 205, 212, 225, 235, 242, 253, 258, 281, 284, 290, 291,
300, 301, 343, 364, 381, 391, 393, 405, 410, 419, 422, 439,
441, 459, 473, 481, 484, 501
<223> n = A,T,C or G

<400> 631
ccntcggctt gggttttttt ctgagccccc cccccccccc cccccccccc cccccccggc 60
cccatagccc caccggnccc acccaaattt taacaaaata aatntaccta tcgntcacct 120
atcccncgta tcgngtaggt cggtaccggt accggngatc ncnacgattn ttcgggtcgt 180
cncccttaan acggncccgt agccnccgga anaaatacta cgagngactc taatntagca 240
anacccgccg tcnattanta gcatccttag tcttccaatg ncgnggattn ngaatccttn 300
naagttatcg ggtagaacgg gtcccggtcc cccgccctct ttncaattaa cgccgggtac 360
aaantcggtt tctaaattcc ncacgaattt ngncggcaac attcncgggn ccttattanc 420

cntttccaac cccgatacnc nagctcgatc gggctttanc gaatccgggg tcncccccga 480
ngantccggg tcctttgagt ngctctagga cggttacgac ggagga 526

<210> 632
<211> 647
<212> DNA
<213> Homo sapiens

<220>
<221> misc_feature
<222> 7, 14, 539, 543, 591, 592, 603
<223> n = A,T,C or G

<400> 632
tttgggnggc gggngctcat ttgggtggac tttttgggtc gtaggaacct ggtatgaggg 60
gtgttttgag tttcttcttc gtcgtctctg ggaggttcgg tttcgattga gattcgggtt 120
cgtctttatc ttacgaggca ccctgatatt gttgcgcttt ggtttggttg tggagagttt 180
tgtcctactc tagcgggtca tgcggatgat atgtagcctg cgtggcctga tagtgatgtt 240
gtgagcttga gaggggagtt gtgggtgttg cgggcggagt aggaggggtt ggagcaccgg 300
gattgggaga tatagaatca taagtgttag gtataggtcg attgagcgag ttcgtggaat 360
tcgtgtggtc atcataatta gagtgaggat gggctctata tttcttagag gacgcacggt 420
cgtgattcgg ggtttgatgg gtgttcttct tgtgggcacg attagcttgt tcatgatggt 480
aaggaccata ctgtttcgaa tgaggattcg tgtcttcgga ttgttgtgga tattgtggnc 540
tanactattt agtgtaagcc ggaggtggtt tgccgtggtg gagtatccga nnttcattcg 600
ganggtatgc gtgcggagcg gtccttgtag acattccgga aaaatgg 647

<210> 633
<211> 630
<212> DNA
<213> Homo sapiens

<220>
<221> misc_feature
<222> 568, 579
<223> n = A,T,C or G

<400> 633
tccttcggct tgggtttttt tctgaccccc cccccccccc ccccctcgga aggcctctag 60
gctcccaccc gtctctctaa tcctcaggaa ccgatccacc caaccaactt actaatgtcc 120
tacagtaaac acccgagaat ataaacccac acctaggcct ccaatcctac cagggaagca 180
agaagccgta gtctagcgta ttacgaaccc gagatagaga cggagatact tagttttatt 240
ctctcggaat aggaaagacg actggggagg gaatataggc tagcgcgggg ataggggcta 300
tggcggatat gggggcgggt cgctctctta ttcttctata ccacgtcaat aggaatgtag 360
atatacctag atgttcccgt agaaagagac gttagaggtc tccgaagcta taaaggagag 420
gcgcgaagaa acttcgtact ctagctttat ataggtagtc gctctagtcc cataagcgac 480
gagagatcta ctagatttcg gtatcgccgt cgtatgtatt cgaaatagtc ttcttcccct 540
tttcgatctc ctctctatac tacatggnga ttatagtcnt aagatagtca ggatattagg 600
atattagtta tatgacgttc gacgggacgg 630

<210> 634
<211> 647
<212> DNA
<213> Homo sapiens

<220>
<221> misc_feature
<222> 3, 53, 593, 612
<223> n = A,T,C or G

<400> 634
ccntcggctt gggttttttt ctgacccccc cccccccccc cctccactaa gancttaacc 60
caaccctata gtttactcgt ataggggaat cgaggagaaa taggaacgaa gagcgggtga 120
taaagagaaa gtactttcct ttatatgtta agagcttagc gtaatgactt tcgttatatg 180
gctagttgat tttatccggc gttatagggc ttagttctgg ttatctcggg tctaattccc 240
ttagtatgct cgggagttta acgaggtcac gggatagcgc gtaccctttc taaggttctt 300
ggaaagctat tcgttattta tcgcgattct cgaggtcgaa aggatcaagg atcttccctt 360
ttactaccct agtcgggtta gcggtcggtc aaaactagtg tagtaccttt acctcctcga 420
aagttatagt cgaaacaacg tattagtcga aattatagcg gatagatcga gacggttctt 480

tctcgggttc tcagccggta atccctctat ttgggggtct tctccctctt cccctttgtc 540
ttccgcctta gcttccaagg ttcctcggaa gcgaggggtt ctacttaagt cgntagcgtt 600
ccttataaac cncctacagg cagacccccct tgtaaacggc tcggggt 647

<210> 635
<211> 645
<212> DNA
<213> Homo sapiens

<220>
<221> misc_feature
<222> 595
<223> n = A,T,C or G

<400> 635
ccttcggctt gggttttttt ctgagccccc cccccccccc cccgaaactc gccttaccct 60
agatacccaa agaatagttc cactcaactt cgtctaagta aaactctaga acttccaaac 120
ataaaagact tcgcgcggtt agctacacag cctacgggaa tctcacgaat cccgattcaa 180
gtcccactct cgaccacacc ccggtatcgt cgttttccca taccaatgtc gaaaaataaa 240
ataaaatcca gtcaagcccc acggtaagcg ggggtagggc taggcgaaga ggcaggaacc 300
gttcgaggcc gggggctttc aaaatacaaa acaactactt aaagtttacc ccttctaaag 360
tcgggggcaa cggttaaagc acgcctctaa agtactactc gtttcgagaa ggggtagtca 420
tctcccgcat agagactctc gcgtatatca actcgcatcg cttctagcat tccgacggtc 480
gcccgcggct acatatcttg cggattagct ccgagggact atagggttaa ttagtctagt 540
aaattctctt agaggatagt cggggtcgta gttaggcagt acgaggggac atggnctgcg 600
tcgtgctcta ccttgacagc atactcttat aaacatcttt ttcct 645

<210> 636
<211> 643
<212> DNA
<213> Homo sapiens

<220>
<221> misc_feature
<222> 137, 199, 232, 245, 247, 282, 298, 322, 360, 373, 396, 417,
425, 437, 476, 496, 542, 593, 595, 606, 607, 640, 642
<223> n = A,T,C or G

<400> 636
ccttcggctt gggttttttt ctgacccccc cccccccccc cctagcggaa aacaatcccc 60
accgagattt tattaatcgt aaaactcgcc ttcggtacca agtcttcctc cttcccgtaa 120
cctggctccc tcctagnggc tttacgaacg tccctcctct tcttacggct cggaagtggt 180
tacggttaaa tccggaggng gggctaacga atccaaggct aactcctctt anagtttgtt 240
gtccncncgt ttagtaagga tccgtggagg gcgagtattt gncccccggc ctttattnta 300
tagttcccta gtacgataaa gntaccggct atcctattac agcggataaa agttatttan 360
agggccgacg tcnccgctag acaggctaca gctagnggag gtaccgcctc cgactantcc 420
gttgnttccg acaaggnagt ttcggttaac tccacaaact cctccgccga ctctanggtg 480
gggacggcag ttcccncgtt tagtgtgcgt tatagagaag ggcatttgag ttggacgtta 540
cnttttaaca taggttattc cgtttaggtt cttgcgggcc cgtgggggta gtncnccggc 600
gcgttnntat cggcgatttt ccgcagtttc cgtttccggn tnt 643

<210> 637
<211> 631
<212> DNA
<213> Homo sapiens

<220>
<221> misc_feature
<222> 6, 48, 622
<223> n = A,T,C or G

<400> 637
gggttntctc atttgggtgg actttttggg tcgtaggaac cggtatgnag gagtaggagt 60
cgctgggaag actagaagtt agctacggac gattagtgtg attccactct taataacgag 120
taatcgttta cgtcgggttg gtgtttcggg gttttggaga gtaagcgtag ttgtggagtt 180
tcgcatatag gtccccttac ttcggcgatc tcgtcttctg tcggttaggt tattattgtt 240
catccttcgc attagtagta gggttggtcg gataaatcga tagctattct ttagaattcg 300
tagtcggaga attcgtgtac gaagtccttt aagttcttta agttcgcgag taagacgtgt 360

acggttattt tgtcgtcgac gtaggtgtcg tttacgggag tttcgtttta ggggtttacg 420
tagaacgtta ttaagcacgg taatacgata gaggattacg cgacgtattc gtcttagaac 480
gtcgattttt cgaaggcgca tttgttatcg aaggggagtc cttggagaat cgagatattc 540
caagaatatt acggagatta cagatcggaa ggctcccgag atcggacgta ttaccggtct 600
cgcccgaaac gagtaggtat cntccggata a 631

<210> 638
<211> 606
<212> DNA
<213> Homo sapiens

<220>
<221> misc_feature
<222> 21, 100, 554, 567, 587, 602, 603
<223> n = A,T,C or G

<400> 638
cccccccccc ctcaaccatc nattccccac ctcaacgcga attacggttt cgaaagtcga 60
caataagtcc ggtcgagtag agggaatcag gggctggtan aaaggaccac gggcggaaaa 120
taccggtctc cttccgggga gcgacgtcgg ggaaagggaa gagagcggtc tagttcgtag 180
gcaaacaggt cagaaaagtt aaggttaaag gtcggagggg agaggatagc tagtacgctt 240
agttcggggc tcgggcgcag ggccactttc ctctttcgcg ttcctttact ctgcttacga 300
gttcaggctc cggagttccg cgccggaggt cgtcgcgacg ctaggaatgg ggactcgctc 360
agtccccggt tatccttcgg gattctatgt tttcgccgat agacggagac cgggtagtag 420
ggttccgtcg taccgccact cgtcgccttg atccggcccg ctccgcttaa gggcgatgaa 480
agattaggta ttagggctct acgggacgag gcatagggcg ggagaagggg ggaggggtcg 540
ggggtcgaag ggantaagaa atcgcantcg cgcggggtcg gtagganccg aaatttttct 600
cnncgt 606

<210> 639
<211> 592
<212> DNA
<213> Homo sapiens

<220>
<221> misc_feature
<222> 4, 89, 528, 547, 591
<223> n = A,T,C or G

<400> 639
tccntcggct tgggtttttt tctgagcccc cccccccccc cccccgggaa cgagaaaaca 60
atcccaccct accgcgggga gtgggttgna cgcttagttc tagaatcctc ggaatcgtcc 120
tccggcgttg gtagttccgg cgattccgag tatgccgaag tgtatcgctc cgtctagagg 180
ttggtatctg tttatcgcga tgacgctatt gactcggatg ctttcgaagt aggggatag 240
gcgcatagat acgcctccgc ggtgtcctct gaagtggccg catccgtgga cgcagcgtag 300
acagctctgg tggacgataa cggcttctcg tactcctact ccggctatta tgttagagag 360
gacttgtttc tgaacggata taccattagc gaaggggtac cctccgctaa cgcaggcgtt 420
tctaacagtt cttccgggcg ctccgaattt agattgacgc ctccgcagca ttgtgggatc 480
ctcttccgtt agccctcttt ataggatttc tcctccgccc cgaaagangg ctggtcgtcc 540
ccggcangta tgtctagctc gaacgctttg ttactccttt gttttcgaaa na 592

<210> 640
<211> 637
<212> DNA
<213> Homo sapiens

<220>
<221> misc_feature
<222> 15, 60
<223> n = A,T,C or G

<400> 640
ctttgtggcg gtggntgtct catttgggtg gactttttgg gtcgtaggct tatccgggtn 60
gggctcccga agtagcttag gatcgccggc tagttccggt cccgcccgtc gaaagcgcgg 120
ttcggcgggc ggccccgcgt tcgttcgcgg gctttaccct catagagtgc caggtctcgg 180
ttcttacggg ttcgtcggcg atagatttta cggcgagagg tcggtatctt cgccgcttta 240
cgttcggtcg gcatctacgc ctagttcaca ggtagtttat gcgccggagc gcgtgacgga 300
gaggttatac gggacgcgga agaaccgcct ccaaatgact agtacaggct cgttcgggcg 360

tagatctcct cgctcggtcg gcggttctta cttctagggc cgctctacgg tttaaggcgg 420
tcgttagatc ttagaaacta tactcaagtt tcagtcggaa gaaaggaagt agagagaagg 480
gtaaacgatt acctccggtt ctagcccttt ttactcgcat aacgggagaa cggggtccgg 540
ctctcagata cgcctcgcga gacgtcgcga ttcaacttta acctccgcta gggcatccgt 600
atacggttaa cgcggtaaaa gcgacctcgg aaacctc 637

<210> 641
<211> 649
<212> DNA
<213> Homo sapiens

<220>
<221> misc_feature
<222> 3, 49, 649
<223> n = A,T,C or G

<400> 641
ctntgtggcg gtggttgtct cagtttgggt ggatttttgg gtcgtaggna acctggtatg 60
aggtctagtt tcttcaacga ttcttggttc agttacgcga ccctatcctt atcttacaat 120
gtcttctaca tcaggttcat caattaatat atcaattaca cattaacgac ggtgtgacgc 180
aatatgagaa agtatacatt aaggttatta tatattattc gcttaaaaag gttcctgaca 240
tgggacaact tcacccacca ttctagaagc cccccctcct gtaggacccc ctcgagttcc 300
ccattatctt agttcagttt tcatttttta accaggaggg tatcggtttt taataggtac 360
tattttgtca aacttttcag aagctttatc ttcaaatata cttgcaccat ctgtactagg 420
agcactaact attcgagtct attacagctc aacagaaaat aattgaaatt aaacaaccta 480
agtatcgtcc accataaccc catcgggctc tcaccccatt tcttcataag ttctagagca 540
tcctgagctc tttcctatta cccttgatgg tactcatggt ctaatacccc ccgcagttat 600
aggtccttat ggatcctatg ctaccaccgg tctaatccct tctatcacn 649

<210> 642
<211> 645
<212> DNA
<213> Homo sapiens

<220>
<221> misc_feature
<222> 637
<223> n = A,T,C or G

<400> 642
tccttcggct tgggtttttt ttcgtcgcgg gttactatta tcgattgtta cttgtaaagg 60
cgatactccc accgctcacg atattagacc tgctcctcta gaagcgaacg gcgataggtc 120
tactcggccg gcgaagacgg cgaacgggta ggaggagcca tatgcaaccc taacggagat 180
tataagtact gggaaaaata ctagtattaa ggtagcgggt taagataggt ggagagacac 240
tattcacgag cataagcact tagaaggtct tctcgaggag aggtaggcta cggactacgt 300
tccttcttcc tctagcctcg agagggagta tagatgattc gcaaaagaga atccctccta 360
tacgctggca taactagacg acgcgtcgtc gggaaatctc gccaacccta ttgcgacctc 420
caaaaggaag attgtcgttt catagaacgc taatactccg ggtcttcccg aatcatagcc 480
gcatatcggt aagaagacgg taaaatcgcg cgattctaac aagattctgt agacttaagg 540
ctaagcacta gaagcgatct cgattccgga tcttaagatc atactaatag ttcggtcaca 600
ccagacgacg attagccact agaagcccta ctccgtngaa accgg 645

<210> 643
<211> 586
<212> DNA
<213> Homo sapiens

<220>
<221> misc_feature
<222> 97, 98, 100, 132, 464, 528, 531, 571, 582
<223> n = A,T,C or G

<400> 643
ctttgtggcg gcggtgtctc atttgggtgg atttttgggt cgtaggaacc tggtatgcag 60
ggtccgcccg gaattaaaag cgggatcccc aaaacgnngn ttcgcaagaa gagaagaatc 120
atagcgatag anctttcata gtacaaaggt aactaagagg aaaataatgc agattcagaa 180
ctagttgcca aattagaact cgattaggcc aaggatccga gcctggcgct atcacttcgg 240
gacttaagct acggtagagc agtcggtcct gaagcatagc tcccgtagga cgtaggaaac 300

tagtccggca cggaggacat actctcgagt ctcggaacgt ctatttagaa tataaacgca 360
ttaacctcag aaggcgccga cgcggttact ctctagggaa ctatttcatt ccttccggag 420
ctcccctatt tttccaacac atataccggc aaaggaaaat cttntgtcct cggtctaaag 480
agagggaaaa aaaacgatat ctaggttcgg gtttatccat ttaaaaanat ngacgcgact 540
actccctttc aaagggagtt tccccctagg nagagttcaa cngaag 586

<210> 644
<211> 646
<212> DNA
<213> Homo sapiens

<220>
<221> misc_feature
<222> 59, 643
<223> n = A,T,C or G

<400> 644
ctttgtggcg gtggttgtct catttgggtg gcatttttgg gtcgtaggaa cctggtatng 60
agggctattt gacttgtttc tcaaatccca tggtatggtg ggtggcgtgc ggggtggcgg 120
tcggttcggc gggggtgggg gtcgtcctcc aaaggagttg ctagagggct tttagtggtt 180
ttagggcggg aaggggttag agcggagaga cgtcgtcgtg gaagcttctg gcggagcgcg 240
agaaggtagt tagcgccggt tcggaagatt ctcagaattc gagaagaggt agtggggcgc 300
ggagagagag tttctaagtc taaacgtaga ggtcgtccta gtcgggccgg gagtagcttt 360
taagctagag gtcgaggtcc tcgtttaggc tccgggctct tcgggcagta tcctctttct 420
cgaggaacgg agcgaccgac gtcgtagccg gacccgtcta tccgtacgtt tagagatacg 480
ctcacctcca cgggcgtata tgcccgtata cgtataaacg cgtaatatac tcgcgcgtaa 540
aacacgtata cactatatac acgcatcgta cggaccgtat agcgttatac gcgcgcgtat 600
attaatttac acttatatac gcgttaacac gatatatcac acnccg 646

<210> 645
<211> 654
<212> DNA
<213> Homo sapiens

<220>
<221> misc_feature
<222> 1, 4, 484, 503, 551, 566, 582, 591, 607, 615, 618, 623, 629,
635, 654
<223> n = A,T,C or G

<400> 645
nccntcggct tgggtttttt tctgaccccc cccccccccc cccccggtcg acaacgtgcc 60
caccgttgcc atcccagcat agctggttcg ttctgtttta ttcttagtag tttagttcgc 120
ctatagtccc tcgtctatcg tctatcattt aaggaggcgg ggctcgctct ttagggcggg 180
tatcttaggt attcttctgg tttcggctgc cgtctcggag tctggtcctt ttgctttcct 240
ttcttggtcg aacttcgtgt ttgatcgcgt tgtttctttg gggtcgtcat acctaagggc 300
cacttcgcca acaaacaagt ttgtgtagtc gtttctatta gggttcgctg gccggcgctc 360
ttactggttg gcgattttta acgcgtttgg ttttaatttg cttcctcccc tagggctcgc 420
tcggtcttct ctctgttcgc tgctctcgtc cggcctttgg tgcggggata gctccggcta 480
ttancgtgcc gtgtccgtgt ggnttttgtc caatgtgaag gcctaggggt gcgggcttct 540
ttggccatgg nttcccctct tgtgancctt aggggtaacg antcgtaatt naaggtcggg 600
ggttggnata cgttntangg gangcctgng tccgntattc cttgttttgg cctn 654

<210> 646
<211> 645
<212> DNA
<213> Homo sapiens

<220>
<221> misc_feature
<222> 601, 606, 608, 612, 618, 629
<223> n = A,T,C or G

<400> 646
tccttcggct tgggtttttt tctgagcccc cccccccccc cccccacgcc aagtacacag 60
acccaccaaa aacaacgtca acacaacttc gggtatacgg accttaagag agaccccgta 120
gtagacccta ccacagccat ccaatagtca aacaacaagg gcgcacccaa tccatccata 180
gagctatcaa acaacggagg ggaaaggaaa gagcagggtc aacttagcag agatcgaagt 240

cggcactaat tcctttcaag tactcgctcg gcttgtagtt cggggtaaag tccgctctca 300
aagggccaac gaggttttaa agcgaccccc gtatcgagtc ttcttcgtat tcattaaggc 360
gttaaaggta cgagacctag aagagagtag aattagccca ccaaatcgcc taaaccggca 420
aaaacgacca aaagtcaaag acccttacaa atatcacctt aaaacgccaa ccccaaaaac 480
gcgatcagta acgcacgtac ctttcccacg cttttctttc tttcactctc caaaacaaac 540
ccgaatattt agcgcaaaaa atatccgagg gagaattaga agctattacc cgaaaaaaaa 600
ncgganangg antaaatngt ggggaatana cgtttggttt ttctg 645

<210> 647
<211> 753
<212> DNA
<213> Homo sapiens

<220>
<221> misc_feature
<222> 569, 624, 697, 700, 703, 712, 734, 740, 744, 749
<223> n = A,T,C or G

<400> 647
accttacctg gtaccgggcc ccccctcgag tttttttttt tccaaataca actcagattg 60
tatacgaaaa gctgataata cattgacttt tgctgtttaa atcccttgag cctttgataa 120
tgattttttt tgtgttaaca attgtagtat ataaaatcgg attcaccatc cttctgatgc 180
catattgatt agtttgattt tatggtgatg ggatcattgt gtgttaactg tattaagaag 240
aaatggattt gattgacttt gcatccattt ttatctgtgt tactttcatg ttttatttaa 300
aagcatttct ggaccagaat aagttaagtg gtataatttg ctttttacac gtttatataa 360
ttgaagttag caatgtggca aaatctctaa tggaaataaa atgcttcaga atgatgacat 420
aaatctgagc tatttcttgc ctggagaaca agtgttattc ataataattt aatagcttct 480
gaggtgtttt gttcatgtga tgaaggctta tccaccttgt atcaattcat gggctctgct 540
ttgtttaatg tagtcaggtt gttaatacna gacttaagag tcatcctact gtgataagtg 600
gtgagtgaag attacatgtc ttangaaaat tatactggga atatctctga cattaatggg 660
tttaaatgtt ttaaggctag gggatgatgc aatgganaan atncttccaa angtttctgg 720
ttgtttatat ttgnggaagn catnaagana ccg 753

<210> 648
<211> 383
<212> DNA
<213> Homo sapiens

<220>
<221> misc_feature
<222> 29, 64
<223> n = A,T,C or G

<400> 648
gatatcccgg ggaaatgcgg aggcctttng gcttacgtgt ttaccgcgta gggcaaagcc 60
ttgncaaatt cccggccagc ggagcggcga gggtggggac tcacgggaag ttaaacagcc 120
tcgtcggcgt cctcgaggct ccaaaaccag gctctaggcg gggacgactg cagccgttat 180
ggaggccacc gcggctacgg ccgcggctga ggcctcccca ggtggagcgg tggcctggag 240
gggaatcttg atcctgggcc agccacctgt caagaggagg cggagcgtca tgcctctgga 300
agactggatg aatattctcc aggagcctga cgaaggcgaa gaagtctttg cagaggaaat 360
tgaatgctgt ctgatgctac aat 383

<210> 649
<211> 349
<212> DNA
<213> Homo sapiens

<220>
<221> misc_feature
<222> 8, 12, 33, 37, 117, 122, 154, 161, 257, 283, 285, 300, 303,
308, 316, 317, 323, 336, 339, 346
<223> n = A,T,C or G

<400> 649
cgattgtnta cnagtcttag agtaagctta agntcgntac cgagctcgga tccactagtc 60
cagtgtggtg ggaattccat tgtgttgggt cactagtaaa tggatttagc tagacanagg 120
anatttaccc tattccattt agcacagtga gganaggcta nacagctagg atgcaataaa 180
aaaaatttta atgagaaatg tgtgtggtag attaattcta ttaatctcaa gttatagatt 240

aaaaaattta agtaccncat aaatgccatt tgcctttgct aangntacat ttttatgaan 300
aangaccntg catacnnaat ganatactgg actttnggna cttgangga 349

<210> 650
<211> 306
<212> DNA
<213> Homo sapiens

<220>
<221> misc_feature
<222> 235, 255, 257, 264, 280, 288, 289, 298
<223> n = A,T,C or G

<400> 650
cattgtgttg ggagcatcct tccatcagct cccatgagaa attctctgtt gggtttaagc 60
aatccccaaa tatatcatat tgacatgaat atatcatctc ctcaatgtcc agcattagca 120
gacaagatga gtgctgaaga tgatataact cctacctctt atgtaggcta gaggtaaagt 180
ctggctctgc tgactgtggg gacataccga aaaggaatgt gggttaatat cagangacct 240
ccctgcagat ccganantca gggnctggac tttctgggan aggaagcnna aagttatntc 300
tgaacc 306

<210> 651
<211> 769
<212> DNA
<213> Homo sapiens

<220>
<221> misc_feature
<222> 613, 615, 629, 665, 666, 684, 698, 711, 735, 752, 754
<223> n = A,T,C or G

<400> 651
cattgtgttg ggcagggtca tttctaaggc atgggctgga agcttttatt taaaacttta 60
catgtcttag aagcactctg gttgttgcta ggcagacaat tttacatctc ttgctatacc 120
agttgcatga agttcatcat gcatattggc tgtggaaaac cttaacagca tcatgtcata 180
aggtttcagt aaggtttaaa tgaaatcatg tattaagcac ttagtatagt gcaccttaaa 240
tgttagcttc aaaacaatga caacctaact aatgttgaaa gaagcttgtg tttgtaaatt 300
atgtcttatt gaaagatgtc atcaaatcct gttatttcta atcccttaaa gtctctcaat 360
gtatttcttt ttgccatatc caatgacagg accttagttt aagccagtgg ttctctcaac 420
ttctaatcca gagatacctg ggtgtcccca agaccttttc agagcatcct tgatgtcaaa 480
accattttca taataatatt aaaatattat ttgctcattg tactcttatt ctctcccaaa 540
tattcagcga gttttccaga agctatataa catgtggtaa catcttatca ctctgacgat 600
taatagaata tgngnttttg gattcttgng tttaaaattt tctcactttg gggttctaat 660
atggnnacga ttaatagata tggnctccat gaccagangg ctttaaagca ntcaataatt 720
tttaagagac taagnactat cctttaaaga tngngaactc catcttaat 769

<210> 652
<211> 267
<212> DNA
<213> Homo sapiens

<220>
<221> misc_feature
<222> 1, 2, 4, 20, 32, 33, 55, 64, 72, 74, 77, 89, 94, 103, 140,
146, 149, 164, 170, 185, 190, 194, 196, 220, 233, 240, 244,
247, 255
<223> n = A,T,C or G

<400> 652
nnangccctt taaccattgn ggcctccacg cnntggcggc cgctctacaa ctagnggatc 60
cgcnactcta gnanaangat tggctcttnt gggntgggcc ggncgggctg gggcgttaag 120
cggggctggg cgcgcgccgn ggttgnacna ggcgccgccg cccncacacn cccggagcac 180
cctcnttgcn gccntncccc gctcaccccg cgcgcgccgn tccgcttttt ccncacccan 240
agcnctnttt atctntgtct cctccgg 267

<210> 653
<211> 501
<212> DNA

<213> Homo sapiens

<220>
<221> misc_feature
<222> 4, 7, 46, 63, 65, 74, 79, 87, 88, 98, 113, 116, 118, 124,
133, 164, 171, 173, 182, 193, 194, 197, 205, 229, 232, 241,
245, 257, 275, 290, 305, 313, 316, 324, 325, 327, 342, 349,
350, 370, 385, 394, 401, 410, 414, 416, 425, 439, 466
<223> n = A,T,C or G

<221> misc_feature
<222> 471, 477, 481, 484, 494
<223> n = A,T,C or G

<400> 653
cccnttnacc cattgctgga ctccaccgcg gtggcggccg ctctanaact agtgggatcc 60
ttncnatgag atgngcgang gaggacnnat ttgctatnct ggatggggct gantcntnta 120
gctnctctag cancagatgg gttatcgagg aagatgactc caangggcta nantcctatg 180
cncatcctaa aanncanctg ctgtnttcag agtacgcgac acatcatcnc tnatgcattg 240
ntgancaaga cgggcangtg cttatcctca gcgangatgc ccttaaccan gagctcgaat 300
ggacntatca ccntanaggt acanntnccg caccacacac cngcttgcnn cctgacgctg 360
gactggatcn cttaggccac caatnccccg tttnccacat ncctgggacn ctananatac 420
tcganggggg gcccggtanc caattcgccc taatactgag ccttgntacg nacgctnact 480
nggngtccta ttanaacgtt g 501

<210> 654
<211> 710
<212> DNA
<213> Homo sapiens

<220>
<221> misc_feature
<222> 4, 10, 12, 79, 88, 142, 186, 204, 253, 299, 316, 346, 349,
379, 414, 425, 433, 449, 451, 484, 489, 494, 511, 531, 544,
549, 588, 591, 597, 601, 606, 615, 616, 623, 628, 631, 645,
646, 650, 668, 669, 672, 686, 689, 695, 696
<223> n = A,T,C or G

<400> 654
gcgnctttan cncatgctgg gctccacgcg gtggcggccg ctctacacta gtggatccca 60
acactgagtc caccacagna aaactcanca ccaggcagac cccacaactg cagaatccag 120
gctgcaattc acagactaat cntctagacc cacctcagta ccagatggta ccacacagct 180
caaggnttta ggtttgcgtg gtanactcaa tctctatctt tcaccactgc cagcctgact 240
tcagagatcc tgngctctgg acagtcctca gtggcaggca actctcagga gcctcaggnt 300
tttggcacat cccagnacca gccagctgcc acaggccctg accttntanc aacactgccc 360
atgtattcca gacttctanc ataccacagt gccatgctga ttgcatctat agangctcag 420
gtgcncctca aanctgtgcc tgctgcagna ngccccacgt ctctggcatg ccccaatgcc 480
atgngtggna acanttgact tctgggcatg ntggaattcc ctaccactga ncctgaccat 540
aggnggganc ccattttttt cgaggggggg gcccggcccc caattccncc ntatagngag 600
ncgtanttac gcgcnnctta ctnggccngt ngtttaacaa cgtcnntgan ctggggaaaa 660
cccctggnng cnacccaaat taaacngcnt tgcannacat ccccctttcg 710

<210> 655
<211> 202
<212> DNA
<213> Homo sapiens

<220>
<221> misc_feature
<222> 8, 17, 33, 40, 50, 63, 93, 98, 103, 110, 114, 118, 124, 138
<223> n = A,T,C or G

<400> 655
cccctttncc ctttcanccc ccccgttttg gcngccgccn acacctactn catccaccca 60
cantcgacca cccgagcttt tttccgatcc cancatcnat gcngattttn tctntgcntg 120
ctgngcctgc acctttgnta ggtcaagcct ggcccatctt cgacaacttc ctcatcacca 180
acgatgaggc atactctgac ga 202

<210> 656
<211> 308
<212> DNA
<213> Homo sapiens

<220>
<221> misc_feature
<222> 5, 30, 55, 97, 107, 110, 140, 142, 151, 156, 158, 162, 166,
169, 172, 180, 196, 199, 206, 215, 219, 223, 226, 239, 243,
244, 252, 281, 282, 289, 297, 299, 301, 308
<223> n = A,T,C or G

<400> 656
gctgntgaaa gaccacaccg aaaaactctn ctttccgact tccacatgat gatcngcatg 60
tggtggtgag agacttatca tgacgacatc gcttccnacc atcgcanccn ctgcccaagc 120
ccattcatgg aggcctgggn anttctgtga ntgacntnga cnctanacnc tnccactgtn 180
tgctatccag acttgnttng aatatnttat tggcnaaana canttncgga atgctgtgnt 240
tgnncattga angatctgat cactatgaga gggtgaggac nncctgctng ctggcantnt 300
ntaacccn 308

<210> 657
<211> 696
<212> DNA
<213> Homo sapiens

<220>
<221> misc_feature
<222> 4, 15, 19, 20, 424, 441, 448, 457, 475, 519, 530, 531, 549,
602, 604, 624, 627, 642, 646, 655, 668, 673, 682, 685
<223> n = A,T,C or G

<400> 657
accntttcca caatnctgnn ctccccgcgg tggcggccgc gtcgaccagc aacctcagct 60
gtgggtcttg ttacagtaat gagttactgt aaggaaagtg tgacatttcg agcaatttga 120
tttgtttaaa aactagagca gtttcagggt tttccttgta aatctgtctt atgtgtcttc 180
aatgttcttt cttgaggagt agagaaagga attgttagga atgatgcata aaccatggct 240
tattttatct cgctgccacc cataatcaga gcagattctt gggactatga ccctcatgga 300
gacatgacaa ttgtgtgtgt ggtgggtggg agaaaagagc tgggaatttt tagggtctag 360
agggtccaat caggactatt ttatggagct ctgctcacca actttaagtg agcaccaggg 420
gtgngaaagc gaatcttggg ntcaaaanaa caatggnaag gggtaagttg gtatnctgaa 480
ctggccactt cggactctta tttaactggg tattctcant taaggaggcn ngggtggtct 540
tggcttgtna aggaaagcct gtgcaatgga atgactttaa aaccccccat taaaaaaaaa 600
angntataaa tcttgggtct taanaangaa gcctgggttc tnttanccca ttttnccccc 660
gggaaggnaa atnttcttag gnaanggaag ggaagg 696

<210> 658
<211> 698
<212> DNA
<213> Homo sapiens

<220>
<221> misc_feature
<222> 553, 580, 620, 653, 662, 670, 676, 684, 690
<223> n = A,T,C or G

<400> 658
ctggactccc cgcggtggcg gccgctctag aactagtgga tccgtgttgg ctcaattctc 60
aaggctgttg ctgtgcggcc tgttccccac acgtgctgct cagctcaggc aagcaccgag 120
cttgtgttgt ttcatgctca gcgtggaggc ccctcctcca ggtcgctgct ctgtggggtt 180
cccatacact caggctccta ggaggagtcc atttagaaag ccagggtttt tctcagagtc 240
ttagttcctt gtgctgtcat ccatttcaca cgacttgggc cctgctcggg gcaacacagc 300
aagagaaaag acagggaaaa taagagaggg accttgcaca cacacgctct ggaccacaga 360
gccctgtgcc cagctcctct gtcaatacag gtggaatctc gtgcaggatc gcaggggtct 420
gtgatgccac caaagagcag gccgggacag ggttaggaga gaaaggagag ggaagtgggg 480
gtttctccta cgcactctta tttgcagagg gaaaggcggg tttgtattgg ggttgtcggt 540
ctttgcaccc acngcacagt tgtgagacac ccccatcctn agatcaaagc cccacataca 600
gcttggggaa aaacaaaacn aaacaaaaca aaaacagtaa acctccatgc canttgttgg 660
gnaagttttn aatttncttc cccnacccan cttgcttc 698

<210> 659
<211> 750
<212> DNA
<213> Homo sapiens

<220>
<221> misc_feature
<222> 1, 5, 10, 111, 135, 176, 222, 282, 438, 505, 546, 552, 554,
576, 580, 587, 588, 620, 640, 665, 681, 682, 702
<223> n = A,T,C or G

<400> 659
ncaanctggn ctccaccgcg gtggcggccg ctctagacta gtggatcctc ctcatgggcc 60
tggatatctc tgaacatatg atgaacattg cttatgaaaa attatttgta ngaaaattgt 120
gaggcctaag aatgntattt tcttttagtg atggtctttg tttgcttctg taaggnactt 180
gtgggcactc gtaagcttgg atctctttaa tctaatacca gntttgagat tttcttggcc 240
ccatagatga attaaaactg gcgtacttct tgtttacaag anggataagt ctcctagggt 300
aagtcttttg gggtcccaag tcaaaaagat gagggattta ccagttctct aaccttggta 360
gccccagact ccaaactttg ccttctagtc ccaagaggct atcaaaaagc aaaggccatc 420
ttccaccttc ttttccanaa cagcacacat tccagacagt acttgaaagc aggaacctcc 480
ttatccctta aaaacctctt ggaancatct tccctctctt gcttctacta tgcttggccc 540
acctancatt cncntttttc tggaaaccgg aaaaancttn tgacttnngt tggctacatt 600
cagcttggcc ccctacaatn tggtttccat ctgccctaan gaaattttaa agggcacttt 660
ttttntggcc cctgactttc nntttttagg gctttccccc angctttgcc cctttggtta 720
aaggggttat tttccttccc cttttggaag 750

<210> 660
<211> 849
<212> DNA
<213> Homo sapiens

<220>
<221> misc_feature
<222> 64, 66, 683, 733, 737, 774, 778, 794, 809, 812, 815, 823,
836
<223> n = A,T,C or G

<400> 660
tcggatccac tagtccagtg tggtggaatt cgcggcccgc gtcgacgggc agtagtggta 60
tgcntntcta aatgttataa ttatttcaga attactctgc cagaaagtta tgatcataca 120
tagaagagtt tgtagctaac tttgaaagta gtggaaagtg gttttcatgt attgtttggg 180
ttaatttaat tttgattata tttggttttt agttcaggta atttttttgt tgaaaacttc 240
aaatgacaat ttcttcatgg ttactaaaga tcactcatgt ggagtagttt cagatttttt 300
tctgaataca tgtattactt ttagagatgt aaagatgtga aattactaag agagaaaccc 360
atgtgatttg tttagtggat caaaagtcgg tagctccttt gatcctaagt gccactgata 420
gttaaataga tactgaagct atgggcaggc tggattgata agaaaaaagg agacagagaa 480
atgggaaatt gggaaagaac tgtgcaaata ggaaaaggag agagcaacag aacagaatta 540
gtaccacagt gccgaagtgc cacctcaggt acttccatct cccatctcct gaagaattca 600
gtaacagttt gcaaatggtc aacacaatca tttagtgatc ctggttgata ttttcaatac 660
tttctgggga tttcttggct ggnttcaaaa gatgatgctg atagttttat tgcccctgaa 720
ggtattctga agnttancat aatttattgg tcagtaaaat atttgaataa aagngganga 780
aggaaaatct ggcntcttat tttgggatnt cngcnggggg aangaggata taattnaccc 840
cggccttgg 849

<210> 661
<211> 653
<212> DNA
<213> Homo sapiens

<220>
<221> misc_feature
<222> 507, 556, 560, 589, 602, 611, 619, 622, 628, 633, 634, 642,
646
<223> n = A,T,C or G

<400> 661
aacttaagct tggtaccgag ctcggatccc tagtccagtg tggtggaatt cgcggccgcg 60

tcgacctcca ttcgtttctt gtcctttttt ttcatttttt ctcatgttct attcacttta 120
ggtttctaag ataaatatta taaaataatt tttacttata aattattcac tgataccctg 180
tctttaacat gtgaaatgaa ttcaaaagga atcttaatga gaaataatat actcatgatg 240
tttaatagat ttgatttcga aataataagc cctctgaagt cctaagttaa aaataaagca 300
acttgtttga taatttttca tcaagaatgt atctgagtct ctgagtaatt attagtagga 360
atattccatt atcacaatta cacagtataa gctatttagt ctaactttac caaaaaaggg 420
agctacttca acactgtgtg agacttttaa tgggtttgca ttgggtatgc actattagca 480
agataaccta ttttacagca gtgtttntta acctttccca tttatttgaa aggcagctaa 540
gatatagtag ttaatntaan gggctgatgc atttatatta catgtagana atgggagata 600
cnaaagggag nggggggana tnttttgnat tcnnaagctt cnttgncaat taa 653

<210> 662
<211> 646
<212> DNA
<213> Homo sapiens

<220>
<221> misc_feature
<222> 86, 123, 312, 489, 500, 501, 504, 513, 533, 541, 552, 559,
564, 599, 618, 619, 631, 634, 635
<223> n = A,T,C or G

<400> 662
aaacttaagc ttggtacccg agctcggatc cctagtccag tgtggtggaa ttcgcggccg 60
cgtcgaccca gggacaggca gccagngctg gggtcaccag ggtcccctct tgggccctcc 120
aanagcaaca gtactggcaa cagctgggat ttgctgagca cagactctgc agcaggctcg 180
gttgagctct ctgtgcctgt tccttcatac catcctcacg cccatccatg agatgggtcc 240
agctgttttc agatgagaaa atggcacagg aagctggtaa gtgacagtca gaaatgaatg 300
ctggcagctt antccttgga cccaccgcag tgcaggacct tgctcaacag ggatcaccct 360
tgtccgccac ctgttcatga ggccacccag ggtttgtgtg gtcatttgtc tcctttcatc 420
tgcttgcctt caaccagctg ggtcattagg gctggggaac ccagacccca cacagtcctt 480
ctcccagang ccagacacan nctncgccac agnaaggact tcagtccccg aancaaatgt 540
ncctgggcgt anaaactgna gggnccccaa tccctggtgg ggtactgctt tgcactggng 600
gaattcaccc ctcattgnna acctttccct nttnncaccc ctaaac 646

<210> 663
<211> 650
<212> DNA
<213> Homo sapiens

<220>
<221> misc_feature
<222> 79, 121, 462, 505, 508, 514, 530, 532, 570, 579, 609
<223> n = A,T,C or G

<400> 663
aacttaagct tggtacccga gctcggatcc ctagtccagt gtggtggaat tcgcggccgc 60
gtcgacgtcg acgcggcgng ccgtttcgac gcagttgata catattatta tatactacat 120
nggttttcta gaattaaaaa attaatgtgt agtgccagcc ctagatgtaa gttacatata 180
tcaactctat ccaattttgt cagccataaa acttaccttt ttcacatact tctaactcta 240
acaatgtgag aaatgtagat cattgcaatt atacccacaa ggcagatggc tacatgcaga 300
atggatagca gaatctagct acttacgcta gccacatggt agacgttttt tcctttgttt 360
ttgcaaaatt gcaatataag ttgcatatcg ttagagtgaa aagatgtaaa gaacccatag 420
aagccagtga tgaaggacat ttatattttc acctttacaa angaccttaa aattgcctat 480
gtggagcaga aactggagga gggcnaancc atcngtaaaa aaaattttgn tnctatttgg 540
atttgggcac cattattacc tccccaggtn cctttttgnt ttaacctttc ttttaaaaaa 600
aataattcnt aatttttggg caaaaaaaaa caaggttttt atttaaattt 650

<210> 664
<211> 678
<212> DNA
<213> Homo sapiens

<220>
<221> misc_feature
<222> 34, 69, 388, 422, 527, 582, 648, 672, 677
<223> n = A,T,C or G

<400> 664
taaaaatcta gactacacta ggaaattatt ttantatcag aagaatatca ggggtgtagt 60
actcatcana gctaaatgag agcgctttaa aaatgttagt ttgtcttccg ccatttctac 120
agaaagctgc aatttcaggt tttcaaccta ataggtgata tttaagaaaa aaaaaaagca 180
atcgcaaata gccccactgc ttttacaaat cattttttct cttctaggta tagcctgtca 240
ggtggcctaa tgtaattttt gacatctcta ggaattttaa tagaaccaga aatgggtgcc 300
agagatatgc ctgcactaat cttaagtggg gatttatgta tttctcaagc aagtgattaa 360
agcaaaacta ggcacgattg aaatcaanat cttttaggca agaaagtcat gatgagtttt 420
anaattattt taggactctg tggctttctc ttcatagaaa tagaaaaaaa aaattgtata 480
aaaaccacaa aaggtcctga atagcccaaa gcaacactga acaaaangaa caaagcagga 540
agcaacacac taccggaatt caattatact accaaggtgt antaaccaaa acagcattct 600
attgggcata aaatagacca aagaccagtg ggaaacagaa taaagaancc caaaataaat 660
cctatattta cngcccnc 678

<210> 665
<211> 694
<212> DNA
<213> Homo sapiens

<220>
<221> misc_feature
<222> 18, 33, 71, 202, 318, 330, 353, 432, 467, 476, 492, 509,
519, 521, 533, 543, 571, 608, 609, 663, 667, 689, 691
<223> n = A,T,C or G

<400> 665
cttttcaaat catttttnct cttctaggta tancctgtca ggtggcctaa tgtaattttt 60
gacatctcta ngaattttaa tagaaccaga aatgggtgcc agagatatgc ctgcactaat 120
cttaagtggg gatttatgta tttctcaagc aagtgattaa agcaaaacta ggcacgattg 180
aaatcaagat cttttaggca anaaagtcat gatgagtttt agaattattt taggactctg 240
tggctttctc ttcatagaaa tagaaaaaaa aattgtataa aaccacaaaa ggtcctgaat 300
agccaaagca acactganca aaaagaacan agcagggaag caacacacta ccngaattca 360
aattatacta ccagggtgta gtaaccaaaa cagcattcta ttggcataaa atagacacca 420
agaccaatgg ancagaataa agaaccccac aaataaatcc atatatntac cgccanctga 480
ttatcaataa cnaacaccaa gaacatatnt taagggacnt nctattcaat aantagtgct 540
ggnaaaaact gggaaatcca tatgcagaaa naatgaaact agacccctat ccctcaccat 600
acgcaaannt caacttcgga atgggattac aaaacttaag acattccaac ccaagaaact 660
atnaaancta ctattaagaa aacagatcnc nccc 694

<210> 666
<211> 705
<212> DNA
<213> Homo sapiens

<220>
<221> misc_feature
<222> 22, 425, 609, 621, 679, 695, 704
<223> n = A,T,C or G

<400> 666
tttaaaaatt tagatacact angaaaatta ttttagtatc agaagaatat cagggggtgt 60
agtactcatc agagctaaat gagagcgctt taaaaatgtt agtttgtctt ccgccatttc 120
tacagaaagc tgcaatttca ggttttcaac ctaataggtg atatttaaga aaaaaaaaaa 180
gcaatcgcaa atagccccac tgcttttaca aatcattttt tctcttctag gtatagcctg 240
tcaggtggcc taatgtaatt tttgacatct ctaggaattt taatagaacc agaaatgggt 300
gccagagata tgcctgcact aatcttaagt ggggatttat gtatttctca agcaagtgat 360
taaagcaaaa ctaggcacga ttgaaatcaa gatcttttag gcaagaaagt catgatgagt 420
tttanaatta ttttaggact ctgtggcttt ctcttcatag aaatagaaaa aaaaattgta 480
taaaaccaca aaaggtcctg aatagcccaa gcaacactga acaaaaagaa caaagcagga 540
agcaacacac taccagaatt caaattatac taccaaggtg tagtaaccaa aacagcattc 600
tattgggcnt aaaatagacc naagaccaat ggaacagaat aaagaaccca aaataaatcc 660
atatttttac agccagctna ttatcaataa aaacnccaag aacnt 705

<210> 667
<211> 817
<212> DNA
<213> Homo sapiens

<220>
<221> misc_feature
<222> 1, 2, 4, 17, 28, 613, 617, 688, 697, 743, 751, 756, 762,
777, 799
<223> n = A,T,C or G

<400> 667
nnangacttt tgtggtntta tacaattntt ttttctattt ctatgaagag aaagccacag 60
agtcctaaaa taattctaaa actcatcatg actttcttgc ctaaaagatc ttgatttcaa 120
tcgtgcctag ttttgcttta atcacttgct tgagaaatac ataaatcccc acttaagatt 180
agtgcaggca tatctctggc acccatttct ggttctatta aaattcctag agatgtcaaa 240
aattacatta ggccacctga caggctatac ctagaagaga aaaaatgatt tgtaaaagca 300
gtggggctat ttgcgattgc tttttttttt tcttaaatat cacctattag gttgaaaacc 360
tgaaattgca gctttctgta gaaatggcgg aagacaaact aacattttta aagcgctctc 420
atttagctct gatgagtact acacccctga tattcttctg atactaaaat aattttccta 480
gtgtagtcta aactttttta aaaagacatg taatccgcgg agtttgtaac tcaaaacgag 540
tgcatctagg aggtatcgca agccgtttct ggattaaatt cccagctagc ttgcttgctt 600
agcaggggcg ggnaaanaag acatctgcag cctagggaag aaaacctttc gcattgttct 660
tacgtgttta cgttatttta tttcctanaa caaggcngaa ttgggactcg aatggttcag 720
ttggggtggg ggatcccctg gtncataaaa ngtcanaaag anggtacagg cggaacncca 780
agggtcgtcc tgcatttana ctcggaattt tggtgcc 817

<210> 668
<211> 826
<212> DNA
<213> Homo sapiens

<220>
<221> misc_feature
<222> 8, 9, 359, 431, 447, 470, 628, 691, 693, 699, 710, 737, 761,
781, 790, 792, 800
<223> n = A,T,C or G

<400> 668
cggggggnnt tacgtctctc tggacgcttt tattgtacca gggcgatccc agcccaactg 60
taccattcga gtccctactc ctgccttgct ctagggaaat aaaataacgt aaacacgtaa 120
gaacaatgcg aaagcgtttt cttccctagg ctgcagattg tcttcttcac cgcccctgct 180
tagctagcta gctagctggg aatttaatcc agaaacggct tgcgatacct cctagatgca 240
ctcgttttga gttacaaact ccgcggatta catgtctttt taaaaaagtt tagactacac 300
tagggaaaat tattttagta tcagaagaat atcagggggt gtagtactca tcagagctna 360
atgagagcgc tttaaaaatg ttagtttgtc ttccgccatt tctacagaaa gctgcaattt 420
caggttttca ncctaatagg tgatatntaa gaaaaaaaaa acaatcgcan atagcccact 480
gcttttacaa atcatttttc tcttctaggt atagcctgtc aggtggccta atgtattttt 540
gacatctcta ggaattttaa tagaccagaa atgggtgcca gagatatgcc tgcactaatc 600
ttaagtgggg atttatgtat ttctcaanca agtgattaaa gcaaaactag gcacgaatga 660
aatcaagatc tttaggccag aaatcatgaa nanttttana attattttan gaatctgtgg 720
cttctcttct taaaatngaa aaaaaaattg tttaaaccca naaggtctga atacccaagc 780
nccctgaacn anagaacaan gccggagcac cccctcccaa atcccc 826

<210> 669
<211> 547
<212> DNA
<213> Homo sapiens

<220>
<221> misc_feature
<222> 183, 282, 301, 308, 325, 333, 348, 368, 373, 421, 422, 431,
436, 491, 518, 523
<223> n = A,T,C or G

<400> 669
cattgtgttg ggaaaaaaat gatttgtata agcagtgggg ctatttgcga ttgctttttt 60
tttttcttaa atatcaccta ttaggttgaa aacctgaaat tgcagctttc tgtagaaatg 120
gcggaagaca aactaacatt tttaaagcgc tctcatttag ctctgatgag tactacaccc 180
ctnatattct tctgatacta aaataatttt cctagtgtag tctaaacttt tttaaaaaga 240
catgtaatcc gcggagttag taactcaaaa cgagtgcatc tnggaagtat cgcagccgtt 300
nctggatnaa attcccagct tgctngcttg ctnagccggg gggcggtnaa aaaaacatct 360
gcagcccngg ggnaaaaacc ttcgcattgt tcttacgtgt ttacgttatt ttatttccct 420

nnagcaaggc nggganttgg ggactcgaaa tggtacagtt gggctgggga tcgcccttgt 480
tacataaaag ncgtccagaa gagggacggt tacaggcngg ganctccaaa ggtcagtccc 540
tgccatt 547

<210> 670
<211> 232
<212> DNA
<213> Homo sapiens

<220>
<221> misc_feature
<222> 167
<223> n = A,T,C or G

<400> 670
cgaactattt agactaccta ggaaaattat tttagtatca gaagaatatc aggggtgtag 60
tactcatcag agctaaatga gagcgcttta aaaatgttag tttgtcttcc gccatttcta 120
cagaaagctg caatttcagg ttttcaacct aataggtgat atttaanaaa aaaaaaaagc 180
aatcgcaaat agccccactg cttttacaaa tcattttttc cccaacacaa tg 232

<210> 671
<211> 214
<212> DNA
<213> Homo sapiens

<220>
<221> misc_feature
<222> 11, 24, 26, 34, 35, 42, 49, 54, 72, 77, 91, 93, 94, 120,
122, 135, 138, 145, 149, 155, 171, 175, 181, 201
<223> n = A,T,C or G

<400> 671
ctccccttcc ntccttcgct actncncatt ttcnnaaatt tntttcgcnt atgnggaaaa 60
acacccacat tnttcanctc gcacagaaca ngnnggggtg tgtaaaatga agggcttccn 120
cnctttctct tattnaanaa cactnaaana gggangggct aaaacccgcg ngatntctac 180
nctatcgcgg gcgcttttgg ngttggctag aaga 214

<210> 672
<211> 328
<212> DNA
<213> Homo sapiens

<220>
<221> misc_feature
<222> 1, 4, 11, 42, 64, 65, 69, 98, 106, 113, 131, 192, 207, 209,
265, 277, 293, 295, 301, 303, 313
<223> n = A,T,C or G

<400> 672
ngancagcgg ngtttaaacg ggcctctaga ctcgaggaga cncctgttgg atggtggatc 60
acanntcgnt actactatac aggacagagt atcggganct cttggntgtt ggngcctgcc 120
aaccactgct nctgttaact gcgtatctga agggactcgg actggcttca gaagaactac 180
cggctcgaat gnaccatgga tgattcncnc tagttgaaaa aaaactcagg cacatgtatt 240
gccactgatg actagcgcca gactnctctc ggctctntaa cgagcccaca tgncngtgtg 300
ncncccgtgc tgnctccaga agaggttc 328

<210> 673
<211> 223
<212> DNA
<213> Homo sapiens

<220>
<221> misc_feature
<222> 55, 56, 97, 105, 112, 116, 118, 131, 160, 163, 173, 174,
184, 185, 195, 201, 202, 211, 219
<223> n = A,T,C or G

<400> 673

gggggcaaag ctggctagcg tttaaactta agcttggtac cgagctcgga tcccnnagac 60
attgtgcatg aaaatgcaaa ttgagtgtgg tctatantgc catcntcacc tnctgncngc 120
tcaaaacaac ngctttctgc tgcaatgggt agggctcctn acncacggtc gcnnacggag 180
gccnncttat cctcntcggt nnggatccct ngaagcatnt tct 223

<210> 674
<211> 256
<212> DNA
<213> Homo sapiens

<220>
<221> misc_feature
<222> 2, 9, 11, 30, 37, 40, 45, 47, 68, 88, 100, 110, 111, 117,
140, 150, 155, 180, 198, 199, 219, 226, 228
<223> n = A,T,C or G

<400> 674
gnggggtcnt ngatgagcgc gcgtaatacn atcactntcn ggcgngntgg gtaccgggcc 60
ccccctcnaa gcggccgccc tttttttntt ttttttcatn acatgataan ntctttnttc 120
taaacagacc acaccactan agttcctttn ctttngtacg gaattgagtt aaagtagagn 180
atacaatgca gggcttcnnc tctatttcac attccaggnt ggttcngnat ggatcggccc 240
tgcctctccg atgggt 256

<210> 675
<211> 439
<212> DNA
<213> Homo sapiens

<220>
<221> misc_feature
<222> 1, 2, 63, 77, 88, 99, 136, 139, 165, 209, 238, 246, 253,
258, 276, 280, 296, 303, 305, 321, 335, 366, 374, 378, 383,
398, 400, 413, 429, 433
<223> n = A,T,C or G

<400> 675
nnactagtcc agtgtggtgg aattccattg tgttgggctt gtatgggttt ttttgtctag 60
ttntttggga aatgttngtg ttactatntt ttggatatna tatatgatat gtatggccct 120
tctatgggct cctcanacng aactcaacca ttttccacaa aaccnattcc tcctttccct 180
tcatgactga gtggtgttgg tactatccng gaaactggga cattgtcctt cacatctntc 240
ccttanctgc ctngtccnat tgatgtcttt gagctntgan atgtctttgt taactntctc 300
ctncntctgt actgccggca naattaagca ccatntgtca caaaaagtat tgcgttacct 360
tcacgnatct gttngttncc atncttgctg cttctccngn ggaaaatagg ctnttctggc 420
aaccgaacng aanaaatac 439

<210> 676
<211> 587
<212> DNA
<213> Homo sapiens

<220>
<221> misc_feature
<222> 1, 4, 10, 29, 37, 59, 72, 75, 80, 145, 154, 163, 166, 172,
187, 195, 200, 213, 216, 239, 261, 283, 297, 305, 342, 395,
408, 433, 474, 491, 500, 531, 535, 537, 541, 550, 559, 577
<223> n = A,T,C or G

<400> 676
ngggnggcctn attaagcgcg cgtaatacna ctcactntgg ggcgaattgg gtaccgggnc 60
cccctcaagt tnatntgccn aacctctctt ttggaataac aaaaggttta acacatatgt 120
cctcataggg acgcgctttc acacnttcct gacngcttca tanacntcat tnctatttct 180
cctcagnaca agttnaggcn gaaggtgagg canacnttat aatttccatt tcacaaatnc 240
ggaaagtgag gctcaaaggg nttaaaaaat aacctgatac aantcataga gccggtntct 300
ggaanaagca ggagcaaagt ccaggcatcc tgatccaagc tnggtccact gccttccact 360
ctggagaggc ttcatctccg acaaaggaag ggacntgagt ggctgganaa tctcatggga 420
taaagacctc agnatttcat gctcctggaa atcccatggg ttgaacaaca ggtntttggc 480
ccgtggttct ntccctttgn ccatctttta accttggggt aaatgatggc ntctntnagc 540
nttttttttn aaagagatng aaattgaatg attattngct cattggg 587

<210> 677
<211> 444
<212> DNA
<213> Homo sapiens

<220>
<221> misc_feature
<222> 10, 48, 108, 114, 118, 131, 159, 197, 205, 210, 216, 219,
228, 231, 251, 259, 261, 266, 269, 281, 293, 324, 325, 332,
335, 352, 361, 369, 372, 384, 394, 400, 415, 443, 444
<223> n = A,T,C or G

<400> 677
gtggggcatn attaagcgcg cgtaatacga ctcactatag gggcgaantg ggtaccgggc 60
ccccctcgaa gcggccgccc tttttttttt tttttactgt ccaaactntc tatngatnta 120
gttgaactgt ncaacgattt catgaaattc tatacacana gccttcaggt ccagagagta 180
aaacaaattt aaatttnttc accanattgn agcagncana agcatccnat natatccgac 240
tacaatgaat natatgctna nggtanctna tttacccact ntggggtctt tanggtctgt 300
cacaaactat tttcgtaaac atcnntttaa anttnggtga atggacctaa tnccagataa 360
ntctatttna tntaccctag catncctgtg gctnactttn cgggctgtgt tggcntactt 420
ttaggagaaa attggtataa atnn 444

<210> 678
<211> 670
<212> DNA
<213> Homo sapiens

<220>
<221> misc_feature
<222> 59, 68, 78, 109, 123, 135, 158, 174, 175, 177, 195, 198,
241, 298, 303, 318, 331, 359, 377, 412, 434, 437, 449, 453,
466, 498, 516, 532, 533, 535, 545, 562, 596, 600, 601, 614,
616, 659
<223> n = A,T,C or G

<400> 678
actagtccag tgtggtggaa ttccattgtg ttgggagcag tttaaaaaaa aaaaagacna 60
aatatacnac tcttgatnaa acataaaggt acagtggtct atgaggaana gaaaaggtac 120
ctnaggatgc aaaantacct accacatggg aaccgttngt ccacactcat tccnnanaaa 180
accgagtcct ctcanttnca cacgtgtacg tttcagttgg gaagtgcttg ccattactcc 240
naagcctaga accttcacgt cctgaaggtt ctggaaggtt tttcagattg cttaaganac 300
gcngcccttc catattcntc tccactaccc nggggaacgg aacaaatgga gctgcgacng 360
ggaagcgtcc cttcccntcc gaacgctttc tttcaaacct gcctgccttc cnggcgaatg 420
gaccggaagg tttnctngct tcctttcanc ccnaattact tcctgngttg aaaattggcc 480
tgttggtttg caaatgcngg aatttgttta ctttcntcat gtcctgtgtt gnncnaaccg 540
gctcncttgt tgcctcccct tngaaaggtt ttcatcaggc cccgcccttt ctcttntaan 600
ngtcctaatc cggncnggac cactcgggga aaattttttc ttttcgaaaa gccgccccnt 660
ccgtccggct 670

<210> 679
<211> 449
<212> DNA
<213> Homo sapiens

<220>
<221> misc_feature
<222> 51, 70, 75, 80, 85, 103, 106, 107, 119, 124, 144, 154, 157,
163, 168, 175, 187, 198, 205, 222, 234, 237, 241, 264, 277,
283, 307, 316, 317, 318, 327, 333, 336, 351, 358, 368, 376,
380, 395, 410, 415, 424, 427, 439, 447
<223> n = A,T,C or G

<400> 679
actagtccag tgtggtggaa ttccattgtg ttgggagtag gtctactaca ncctacttcc 60
cctatcatan aagancttan caacnttcat gatccccccc tcntanncct tttcctcanc 120
tgcntcctag tcctgtttgt cctnttccta acantcntaa ganagatnac taatnctact 180
atctctnacc tccggaanct acaanacgtc tggaactatt cngaccccat gcanccncat 240

nctccatcgt cctcccagcc cctncccttc ctttacntta ctnaacgaag gtcgacgatc 300
cctcccntac ctcccnnncc attgggnccc aanggnactg gacctcacga ntacaccnac 360
tacggggnga ctaagnctgn aactccttac atatntcccc gttacccccn gaacncagcg 420
aacngcnaca ccttggacnt caagaanta 449

<210> 680
<211> 670
<212> DNA
<213> Homo sapiens

<220>
<221> misc_feature
<222> 5, 41, 51, 75, 131, 150, 152, 196, 202, 207, 208, 213, 231,
241, 279, 323, 401, 432, 451, 502, 520, 522, 532, 564, 574,
575, 587, 590, 596, 606, 612, 615, 617, 626, 639, 643, 657,
660, 668
<223> n = A,T,C or G

<400> 680
   tttcngtgtg gtggaattcg cggccgcgtc gacgagaaga nggaggagga naaggagaag 60
   gagaagaagg agaanaagga ggagaaggag aagaaggaga agaaatcatc atcatcatca 120
   tccactgtct ngcaactatt taagtttgcn antcccttga aaacaggtac ttttgtttca 180
   atgtttggga ccactnctga cnatgannag aanaccaata aatgcttgat naatgaaaaa 240
   nccacttttt acctgttaga accctgaggc taagagaant gatgtgactc gacttagtta 300
   ccacaaacta tgatcctagc atnaattggg gcatctcaac acctcaactc cctgtgcaag 360
   aacagatttt caatgtctac tgatgatttt aaatggatta nttcctctct ttacttctta 420
   agggcatgaa gntttatgaa acaaaactat ncagttccag acgcttaacc cacatagtgt 480
   taatagtcac cttcaacaca cnactaaacc cccaaaaaan gntttttacg gngtttcgac 540
   agttttcttt tctttttgac ttgnttaaca cccnngacaa ctttgtnctn tttccntgaa 600
   tcacancttt cnaanancca atggtncggt tttttctcnt tcngggccct tcccttnttn 660
   aaaaccanat 670

<210> 681
<211> 494
<212> DNA
<213> Homo sapiens

<220>
<221> misc_feature
<222> 83, 110, 114, 137, 184, 225, 248, 251, 319, 352, 400, 402,
414, 432, 435
<223> n = A,T,C or G

<400> 681
tcatggtgtc cacagtctga tgtgagcgca ttaaatttaa ggatctccgc ccttctcctt 60
aaaactcagg acttggcaat gancctagga agcgcccctc ccctccccan ccanatccaa 120
gccccggacc gctgcgnctc cagctgcgcc tagtgaaacc gccgaattcg aattcacact 180
cggngggccg gcgaaggtgt gcgcgcccgc gggagcgccg gggcnagccc gagggactgc 240
aagccaanaa nggaggcatg ggtggcgggg ggcgccgtct gatccaggaa ggagcggagg 300
cgccgatcac acactcttna gacgccctgc ccgcgcctgg ccagcgcgca gnctgcagga 360
cgcgcggagc aggaactcgc tggagtttgc caagccccan gnctctggaa agtntgtagc 420
tccctttcgg ancgnctctt ctggcccttt gggacgggtg tgtcattggg cgggggtctg 480
tataaggggg ggac 494

<210> 682
<211> 263
<212> DNA
<213> Homo sapiens

<220>
<221> misc_feature
<222> 15, 18, 22, 35, 71, 165, 187, 195, 200, 224, 233, 241, 245,
249
<223> n = A,T,C or G

<400> 682
tgatcattca agcgntgngc gnataacgat tgctnagccc aacctttcat agggtcgttc 60
ctttgggaat nggatgtcta ttgaatggca gggatagggg cactcggcat tcgcctctgg 120

tacagttttg catatatatc ctcatcgcga gcgagcgtag gggancgtta agtttgggga 180
aatgccnccg catgnccctn ccggagctta aacccccaac aatncccatt ttnaaaaaag 240
ntttnttant taaaaaaaaa aac 263

<210> 683
<211> 255
<212> DNA
<213> Homo sapiens

<220>
<221> misc_feature
<222> 21, 23, 35, 46, 54, 56, 71, 82, 84, 87, 95, 118, 120, 127,
146, 155, 164, 168, 174, 192, 198, 207, 213, 220, 223, 241
<223> n = A,T,C or G

<400> 683
cttgcccggc atgcacagac ntntttacgg acacnctact ccaagngagc ctgnanctgt 60
ctacggtcaa nctctaaggt tngncantgc cacanatggc atagtcccga gggcggtnan 120
tctggantgc tctctgcact tgaacntaaa gcgcntttca aganaggnct aatngcctgc 180
ctcttgacaa cnaacaancc cacaccnacc tangaccctn tangcaagga ctggattctg 240
naaatgcaat acaca 255

<210> 684
<211> 922
<212> DNA
<213> Homo sapiens

<220>
<221> misc_feature
<222> 448, 473, 590, 596, 623, 674, 679, 695, 715, 736, 740, 741,
773, 785, 787, 821, 824, 827, 832, 841, 844, 872, 891, 894,
898, 901, 913, 918
<223> n = A,T,C or G

<400> 684
   acccttcatt tcatgtgctt ctattttcct acatctttta catgactaag ggattaatga 60
   aatcacctct tcataatcat gaccataatt tcatccaaca agtactcaag tttggtgtta 120
   gcactttatt aatgcttacg aattctctct ctctccctct ttctcttttc cttagtcctt 180
   gcacaataag gatttttgaa tgtataatat catcttaggt aagctttcat atggttttgg 240
   catatgaagc ttatgactgt cataagccat accaagcctg tggagtatgg catgattttc 300
   attacataat ccaatgaaaa tagacttatt ttaaatccct aactttgtag ttttaatttg 360
   tatttcacta tcttgaaatt aacagctagt acttatccat cacagcagtc tcctactgac 420
   atgaagcaag ttgttgaatg cagtaganca tgaatgaaag catttaatgt tanacaaaaa 480
   tgggtgatac ccaagcattc tgaattattt gcatcaagga atgggacatg tacattagtg 540
   gcatcatttc taccaatatg tgacttgaat tgttttttta aaaaaaggan aatgantttc 600
   tcaatttgct ttaaaaaatt ttnaaaaagt tcaatggcat gctgctttgt ctggacttaa 660
   tttattaaca attnttaanc cttccttaag gacanaattt tggtgttcag gatcnccctg 720
   aagggtctta tttttnatan nattccaaac ccaaaaggtg gtttaaaatg ggngggttcc 780
   ccccncnaaa atttggaccg gcttttttat atttaaaaaa nttnccnttt gngtttgaaa 840
   nctnaatacc aattaagggg gaattttacc tnccagtggg aaaaaaaaac nctngccntt 900
   naaaaaattc ccnggagnca at 922

<210> 685
<211> 531
<212> DNA
<213> Homo sapiens

<220>
<221> misc_feature
<222> 208, 218, 224, 226, 350, 376, 392, 406, 419, 449, 486, 488,
504, 516
<223> n = A,T,C or G

<400> 685
tgaggctctg taaaactgtt cctctgctag gcatacttca tattctctat attaaactca 60
tctttaattg gcatggaaga ttcattgttc caaatctcag atgaagatcc tatattggat 120
gcaattaagc ctggcagcgc cctcaaaaga cagtcttgtc actgctagcc acagccagga 180
cacagtaaca gttccttcta gtgacccnag accataanaa atananatct aaagaattct 240

gactccaaag gcattagccc attcctggta ttgccaatta tgatagaaaa aattgccaag 300
ctcctgggac atggaaatac actcagtaca tttgagaact ggagaactan tttccaaaat 360
agtatgaaga catganggtg attgtagata tntgagtttg gagaanttga gggaaatcng 420
attacacatg tttactacaa gagatgttna taagtaaaga aggcctgata tacaatctaa 480
cagacnantg agataaatct taantcacaa ctgacntccc ttttggggcg g 531

<210> 686
<211> 336
<212> DNA
<213> Homo sapiens

<220>
<221> misc_feature
<222> 3, 5, 9, 93, 105, 127, 139, 189, 213, 242, 259, 267, 298
<223> n = A,T,C or G

<400> 686
ggngncctna tgagcgcgcg taatacgatc atatagggcg aattgggtac cgggcccccc 60
tcaagaacac tacaagctat gtcctcttct canagagccc tgaantttta acatattgaa 120
agctctnatc ttgccaaana actccactta acttcaaaac acaccctcca cacacatcat 180
gatcaactna gatcttactg aaccagaatc ctnaatggca tacttcagga acaggggtcc 240
anagaagcag ttctcaaant gcagctnaaa aagaaactga aaacccaatt catgcaanac 300
ctagggctta tttgagagca ttttccagtg cagatt 336

<210> 687
<211> 271
<212> DNA
<213> Homo sapiens

<220>
<221> misc_feature
<222> 188, 228
<223> n = A,T,C or G

<400> 687
aatctgcact ggaaaatgct ctaaaataag ccctaggtct tgcatgaatt gggttttcag 60
tttcttttta agctgcactt tgagaactgc ttctctggac ccctgttcct gaagtatgcc 120
atttaggatt ctggttcagt aagatctcag ttaatcatga tgtgtgtgga gggtgtgttt 180
tgaagttnag tggagttctt tggcaagatc agagctttca atatgttnaa acttcagggc 240
tctctgagaa gaggacatag cttgtagtgt t 271

<210> 688
<211> 740
<212> DNA
<213> Homo sapiens

<220>
<221> misc_feature
<222> 16, 17, 21, 31, 40, 54, 82, 175, 270, 274, 301, 328, 378,
459, 547, 554, 584, 587, 606, 635, 639, 644, 660, 662, 710,
723, 728, 731, 734, 740
<223> n = A,T,C or G

<400> 688
tgatgaagcg cgcgtnntac nactcactat nggggcgaan tatgggtacc gggnccccct 60
cgaagcggcc gccctttttt tntttttttg tgagagttta aataaaatat ttgagtttaa 120
tttaaagttt gagtttaatt aaaatatatg gcatatccca agttgggctt tgcanaaaga 180
acacttctca ggaactgtta gttggtgtac caggaactca gaagggtcct gttattaaat 240
atatttggaa aatgcatgga ttctctgaan atcnctctgc atgtgagcaa cacttacatc 300
ncaaaccaaa attggcattg catacatnaa ccaatatttc ccaaacattt ctggttatgg 360
cccaccccct ttgtgtanta cttattgctg ttttttggaa ccctggggaa attacttaaa 420
atattcagct ggaaattaca ggcgttactt ttaaggganc aagaattaca gtgactccca 480
aaattgcaag tgttgattac tatttaagaa cccaagaatt tgaaagaaat tttgaaaagt 540
gaaaacngga aatnttaaat gacttctcaa attttgaaaa ctcnggnaaa catctccact 600
ttggtnccct tcctttaaaa attggctaaa aattntttnt tatncccacc ccattggaan 660
tncccccccc ctggaacaat tggattcccc tatttcctaa aaaacggccn ccccccccgg 720
ggngaacncc nacnttttgn 740

<210> 689
<211> 635
<212> DNA
<213> Homo sapiens

<220>
<221> misc_feature
<222> 109, 227, 369, 376, 407, 454, 462, 560, 618, 625
<223> n = A,T,C or G

<400> 689
actagtccag tgtggtggaa ttccattgtg ttgggattac atatactttt agcaattttt 60
aaagaagtgt acaaagttga gatgtttcct gagctctcat atatctgana atgtcatttt 120
acatctccgt cttcacctct caaaacttct ttcaattctt tggctcttaa tagtaatcaa 180
cacttgcact ctggagtcac tgtaattctt gctcctttac agctacncct gttatttcca 240
gctgaatatt tttagttatt tcccagggtt ccaaaaaaca gcaataagta ctacacaaag 300
ggggtgggcc ataaccagaa atgtttggga aatactggct catgtatgca atgccaaatc 360
tggtttgcna ttgtantgtt gctcacatgc agagtgaatc ttcaaanaat ccatgcattt 420
tccaaatata tttaataaca gggaaccttc tganttcctg gntacaccaa ctaacagttc 480
ctgaaaaatg ttctttctgc aaaacccaac ttggggatat gccatatatt ttaattaaac 540
tcaaacttta aattaaactn caattatttt attttaaact cctcaaaaaa aaaaaaaaaa 600
agggggggcc cttccaangg ggggnccggt tcccc 635

<210> 690
<211> 3923
<212> DNA
<213> Homo sapiens

<400> 690
acagaagaaa tagcaagtgc cgagaagctg gcatcagaaa aacagagggg agatttgtgt 60
ggctgcagcc gagggagacc aggaagatct gcatggtggg aaggacctga tgatacagag 120
gaattacaac acatatactt agtgtttcaa tgaacaccaa gataaataag tgaagagcta 180
gtccgctgtg agtctcctca gtgacacagg gctggatcac catcgacggc actttctgag 240
tactcagtgc agcaaagaaa gactacagac atctcaatgg caggggtgag aaataagaaa 300
ggctgctgac tttaccatct gaggccacac atctgctgaa atggagataa ttaacatcac 360
tagaaacagc aagatgacaa tataatgtct aagtagtgac atgtttttgc acatttccag 420
cccctttaaa tatccacaca cacaggaagc acaaaaggaa gcacagagat ccctgggaga 480
aatgcccggc cgccatcttg ggtcatcgat gagcctcgcc ctgtgcctgg tcccgcttgt 540
gagggaagga cattagaaaa tgaattgatg tgttccttaa aggatgggca ggaaaacaga 600
tcctgttgtg gatatttatt tgaacgggat tacagatttg aaatgaagtc acaaagtgag 660
cattaccaat gagaggaaaa cagacgagaa aatcttgatg gcttcacaag acatgcaaca 720
aacaaaatgg aatactgtga tgacatgagg cagccaagct ggggaggaga taaccacggg 780
gcagagggtc aggattctgg ccctgctgcc taaactgtgc gttcataacc aaatcatttc 840
atatttctaa ccctcaaaac aaagctgttg taatatctga tctctacggt tccttctggg 900
cccaacattc tccatatatc cagccacact catttttaat atttagttcc cagatctgta 960
ctgtgacctt tctacactgt agaataacat tactcatttt gttcaaagac ccttcgtgtt 1020
gctgcctaat atgtagctga ctgtttttcc taaggagtgt tctggcccag gggatctgtg 1080
aacaggctgg gaagcatctc aagatctttc cagggttata cttactagca cacagcatga 1140
tcattacgga gtgaattatc taatcaacat catcctcagt gtctttgccc atactgaaat 1200
tcatttccca cttttgtgcc cattctcaag acctcaaaat gtcattccat taatatcaca 1260
ggattaactt ttttttttaa cctggaagaa ttcaatgtta catgcagcta tgggaattta 1320
attacatatt ttgttttcca gtgcaaagat gactaagtcc tttatccctc ccctttgttt 1380
gatttttttt ccagtataaa gttaaaatgc ttagccttgt actgaggctg tatacagcac 1440
agcctctccc catccctcca gccttatctg tcatcaccat caacccctcc cataccacct 1500
aaacaaaatc taacttgtaa ttccttgaac atgtcaggac atacattatt ccttctgcct 1560
gagaagctct tccttgtctc ttaaatctag aatgatgtaa agttttgaat aagttgacta 1620
tcttacttca tgcaaagaag ggacacatat gagattcatc atcacatgag acagcaaata 1680
ctaaaagtgt aatttgatta taagagttta gataaatata tgaaatgcaa gagccacaga 1740
gggaatgttt atggggcacg tttgtaagcc tgggatgtga agcaaaggca gggaacctca 1800
tagtatctta tataatatac ttcatttctc tatctctatc acaatatcca acaagctttt 1860
cacagaattc atgcagtgca aatccccaaa ggtaaccttt atccatttca tggtgagtgc 1920
gctttagaat tttggcaaat catactggtc acttatctca actttgagat gtgtttgtcc 1980
ttgtagttaa ttgaaagaaa tagggcactc ttgtgagcca ctttagggtt cactcctggc 2040
aataaagaat ttacaaagag ctactcagga ccagttgtta agagctctgt gtgtgtgtgt 2100
gtgtgtgtgt gagtgtacat gccaaagtgt gcctctctct cttgacccat tatttcagac 2160
ttaaaacaag catgttttca aatggcacta tgagctgcca atgatgtatc accaccatat 2220
ctcattattc tccagtaaat gtgataataa tgtcatctgt taacataaaa aaagtttgac 2280
ttcacaaaag cagctggaaa tggacaacca caatatgcat aaatctaact cctaccatca 2340

gctacacact gcttgacata tattgttaga agcacctcgc atttgtgggt tctcttaagc 2400
aaaatacttg cattaggtct cagctggggc tgtgcatcag gcggtttgag aaatattcaa 2460
ttctcagcag aagccagaat ttgaattccc tcatctttta ggaatcattt accaggtttg 2520
gagaggattc agacagctca ggtgctttca ctaatgtctc tgaacttctg tccctctttg 2580
tgttcatgga tagtccaata aataatgtta tctttgaact gatgctcata ggagagaata 2640
taagaactct gagtgatatc aacattaggg attcaaagaa atattagatt taagctcaca 2700
ctggtcaaaa ggaaccaaga tacaaagaac tctgagctgt catcgtcccc atctctgtga 2760
gccacaacca acagcaggac ccaacgcatg tctgagatcc ttaaatcaag gaaaccagtg 2820
tcatgagttg aattctccta ttatggatgc tagcttctgg ccatctctgg ctctcctctt 2880
gacacatatt agcttctagc ctttgcttcc acgactttta tcttttctcc aacacatcgc 2940
ttaccaatcc tctctctgct ctgttgcttt ggacttcccc acaagaattt caacgactct 3000
caagtctttt cttccatccc caccactaac ctgaatgcct agacccttat ttttattaat 3060
ttccaataga tgctgcctat gggctatatt gctttagatg aacattagat atttaaagct 3120
caagaggttc aaaatccaac tcattatctt ctctttcttt cacctccctg ctcctctccc 3180
tatattactg attgcactga acagcatggt ccccaatgta gccatgcaaa tgagaaaccc 3240
agtggctcct tgtggtacat gcatgcaaga ctgctgaagc cagaaggatg actgattacg 3300
cctcatgggt ggaggggacc actcctgggc cttcgtgatt gtcaggagca agacctgaga 3360
tgctccctgc cttcagtgtc ctctgcatct cccctttcta atgaagatcc atagaatttg 3420
ctacatttga gaattccaat taggaactca catgttttat ctgccctatc aattttttaa 3480
acttgctgaa aattaagttt tttcaaaatc tgtccttgta aattactttt tcttacagtg 3540
tcttggcata ctatatcaac tttgattctt tgttacaact tttcttactc ttttatcacc 3600
aaagtggctt ttattctctt tattattatt attttctttt actactatat tacgttgtta 3660
ttattttgtt ctctatagta tcaatttatt tgatttagtt tcaatttatt tttattgctg 3720
acttttaaaa taagtgattc gggggtgggg agaacagggg agggagagca ttaggacaaa 3780
tacctaatgc atgtgggact taaaacctag atgatgggtt gataggtgca gcaaaccact 3840
atggcacacg tatacctgtg taacaaacct acacattctg cacatgtatc ccagaacgta 3900
aagtaaaatt taaaaaaaag tga 3923

<210> 691
<211> 882
<212> DNA
<213> Homo sapiens

<220>
<221> misc_feature
<222> 511, 547, 575, 596, 607, 616, 631, 638, 640, 649, 655, 695,
714, 732, 764, 795, 810, 846, 856, 866
<223> n = A,T,C or G

<400> 691
ttactcacta tagggctcga gcggccgctg aattctgctg cagtgagctg tgattatgtc 60
cctgcactcc agcctggatg acagaacacg atcatttctc taaagacaaa caaaaaacat 120
aaaataaaac tagtataagg atagaagccc agggttgatt taagtctgcg gaaatcataa 180
accataggtc agacttctca ttgatgaggt acttgtgggt tagaatacaa ttaggtatat 240
ttggtctaga aaccaggatg gaattagaga ataaaagact gagcaatagc atgttatagt 300
attagaaata ctatagaaat aggaaaagcc ctgattatga ctttggagtt ctgatccaac 360
atctgggatt atttagatat tttaaaggaa aacgatgact tttagctctc aggatgttag 420
tttcctcaac cataaaatga agagcctcga aaagatttcg tttaccagat tatttctgaa 480
gtcaattcca gttctaaaat tccatcactg ngcactaagg caaattgaat tgaataaagt 540
attgggnatg cataaaatac tctattttta aaaangaata gtaattatcc attggnaaca 600
gacgcantca tccagncatc tcctaccctg ncccatgncn tatgtagana tgtanctcta 660
atcccttaac aaaccgattt tgcaaaggag cttanccttg gggtacttgg tcanggcaac 720
tggtctactt tnaagactca tcttcactta ctgggcacca aatncctacc attgcatcaa 780
actggggttc ccatncaagg caaaccctgn gaaatcttta atcccgaaat tggcgcccaa 840
ttttgngggg tttccnaaaa gaatcntccc ccccgagggg cc 882

<210> 692
<211> 235
<212> DNA
<213> Homo sapiens

<220>
<221> misc_feature
<222> 8, 13, 15, 23, 25, 30, 33, 40, 44, 87, 95, 104, 107, 115,
128, 155, 158, 169, 173, 179, 181, 201, 218, 228
<223> n = A,T,C or G

<400> 692

ccgcactngt aangnccgcc agngngctgn aantccgctn agcncggatc cactagtcca 60
ttgatggtaa aagggtagct tactggnatg tccgnctgct ccanganata atacncagga 120
cttctcanag cacttaatat gttaatataa aactncgnga aaaaagatnt tcnatgaanc 180
nttcctctta ggaggtcagg ngagaatagt gttaatgnca ttaagganag aacga 235

<210> 693
<211> 383
<212> DNA
<213> Homo sapiens

<220>
<221> misc_feature
<222> 1, 256, 264, 284, 310, 338, 348, 352
<223> n = A,T,C or G

<400> 693
nttatgtaag aaatgtcata tatcttttat tttctttaaa tcaaaataaa tatgactttg 60
agcatcccat cccatgcccc atcctatcag aatggtagga acatcaacac aaataattag 120
taatgcaccg catctacatt cccatgctct ctttacttct tcagcattgc ctaaaggcat 180
aatacacctt taattaatta attcagcctc ctaatgcaca ttaacaaagc ccctgctaga 240
ctctgtccat aatggnaaac ctgnatgatc cttgatatta acantttaag gaatgctcat 300
ggattggttn cagacttaaa aaattgaggg ggctgaanaa aatctaangg anaaatcatg 360
gaagcatttg cacatattac ata 383

<210> 694
<211> 204
<212> DNA
<213> Homo sapiens

<400> 694
tctcttggct ggtcagcctg aagggtggta atgactcacc aacgctacta atccttcttc 60
actgtccctt atttttttcc ctcccaggct cataactcga ggttaaactc tcttttatac 120
aagaaccctg tctgatgaag catcatttca gaattttaag tcaacttaca aatgtggtat 180
tattcacatc tgagtacaaa ttta 204

<210> 695
<211> 670
<212> DNA
<213> Homo sapiens

<220>
<221> misc_feature
<222> 347, 436, 457, 468, 485, 503, 601, 610, 616, 633, 643, 646,
651, 653, 660, 668, 669
<223> n = A,T,C or G

<400> 695
gcaccagccc aggtgctgtt tcttcacttg agctccatga ccctccctgt gtggtgggtt 60
gaacggtgac ctccaaaaga tatgtccacc tggaacctca gaataagatc ttatttggaa 120
tagtctttgt agatgtcagt aaggtaaaga tttggagatg agaccctcct ggattagggt 180
aggccctagg tccactggca ggtgtgcttc tcagggtctg aaaggggaag acagggccac 240
ccagaggagg agacggaggc agagacaggg ccacccagag gaggagacgg aggcagagac 300
agggccaccc agaggaggag acggaggcag agacaggggc cacccanagg aggagacgga 360
ggcagagaca gggccaccca gaggaggaga cggaggcaga gacagggcca cccaaaggag 420
gagacggagg cagaanacag gcccccccaa agaaganacc ggaggcanaa aacagggcca 480
cccanaggag gagacggagg canaaacagg gccaccccaa aggaggagac ggaggcaaaa 540
cagggccacc caaaaggagg aagccggaag gaaaaaacag ggccccccca aaggaggaag 600
ncggagggcn aaaaanaggg ccccccccaa agngagaaaa ccnggnaggc nanaaaaccn 660
ggggcccnnc 670

<210> 696
<211> 317
<212> DNA
<213> Homo sapiens

<220>
<221> misc_feature
<222> 10, 40

<223> n = A,T,C or G

<400> 696
tgacccgttn tttctgcaaa ggagagtggg gaaggagggn tgggaagaca aaagttacat 60
gttagcaggg aagagaacag aattttatcc acccttatct ctttagtgag tgaacaaaca 120
gcccactgtc atcgtggata catttcactt ttttcacatg actaaggagc tctccggagt 180
gaagagtgag taaatatgtt tattacgcat tcatttgcta agaatcatca agaacccaaa 240
gttagagacg tttcgtggtt gaactttctc cctactgtct agtagaatta tatggggatt 300
ctggatctgc tggtgcc 317

<210> 697
<211> 246
<212> DNA
<213> Homo sapiens

<220>
<221> misc_feature
<222> 3, 19, 24, 28, 37, 43, 55, 69, 70, 72, 103, 132, 139, 141,
169, 174, 183, 192, 196, 197, 207, 211, 218, 221, 232, 235
<223> n = A,T,C or G

<400> 697
ctncagctct aatcgactnc tatnaggnat gatggcncgt gcngcgcgta cgtantgctt 60
ggatcctcnn anagcggacg cctactacta ctaaattcgc ggncgcgttg actttttttg 120
tttttttcct tnacagagnt nttttgtgc ccttggttct tatgctcana ctcngcaaaa 180
aanatcaaaa gntacnnatg aaaaacntat nccatctnca naaaggaggt gnagntatta 240
ctttct 246

<210> 698
<211> 3674
<212> DNA
<213> Homo sapiens

<400> 698
agaaagtttc cttttttttt tttaatggtg aaaagatata cacatattta gaattagcca 60
gctgggctca gtttagatta ttccaatttt gttggcaaca tccagagcat cgtaatcagg 120
agccagtgaa acatattcct tcttctctcc atcaggccaa atcacggtgt tgaccttggc 180
cacatcaatg tcttagaact tcttcacagc ctgtttgatc tggtgcttgt tggctttaac 240
atccacaatg aacacaagtg tgttgttgtc ttctatcttc ttcgtggtga ctcagtggtc 300
agcggaaact tgatgatagc gtagtggtca agcttgtatc tcctgggagc gctcttccaa 360
agatatttgg gctgcctcgg gagttgcagc gtcttgggcc gccggaaggt gggtgacgta 420
cggatcttct ttttttgtgt ggctgtggac acctttcaac actgtcttct tggcctttaa 480
atccttcgct ttggtttcgg ctataggagg ggcaggagct tccttcttca ctttcggcgc 540
catcttgtga aaagggaaag tttcctttct aataccattt tcacttctcc cgaattttgt 600
ggatcgtttc ttggtatcta ccccagattt caggagtgtt ggctggatct tagggattgt 660
gaagtcttca tttccctgtg gtgagatctg aggcatgatt ttaaacagtg tgagggaagg 720
agatctccag gcactttaat agaatggaga agcaggatgg gatttgagag gaaatctgat 780
tttgaaaaaa ggagaactag agttgagttc gtaattaact agcaccttaa aggtcattca 840
gcatgcccat ctgcacagtg ggtgtaatca ccctacagaa caaaaacaaa aaggcaatgg 900
agaggaagct gtaaagcact gtacatgttt aactcattgt tatgtaagct agccgaaggc 960
ttcacagact tgaattcatc tcccaagttc tcttcctgta ctggaaactc tgccttaggt 1020
tgcttaaaac ttgagaaaca gaatattgct tcccctgcct gccttcttga gtacacttgc 1080
ctacacaaag atgcacatcc ttgtttgtgt gtgtgtgtcc atttgctgtg acattcttgt 1140
gaaagtcaaa gtttcccagc tgttgacata cacaagtttg tttggtgcaa cctgtcagat 1200
gcatccctta gacaggccct ttgatactct gggaaagaca ttggacttac agtcggaacg 1260
aaaagaaaga aatgtgatat gtatagcgtg cagtgagttg gagttttacc tgtattgttt 1320
taatttcaac aagcctgagg actagccaca aatgtaccca gtttacaaat gaggaaacag 1380
gtgcaaaaag gttgttacct gtcaaaggtc gtatgtggca gagccaagat ttgagcccag 1440
ttatgtctga tgaacttagc ctatgctctt taaacttctg aatgctgacc attgaggata 1500
tctaaactta gatcaattgc attttccctc caagactatt tacttatcaa tacaataata 1560
ccacctttac caatctattg ttttgatacg agactcaaat atgccagata tatgtaaaag 1620
caacctacaa gctctctaat catgctcacc taaaagattc ccgggatcta ataggctcaa 1680
agaaacttct tctagaaata taaaagagaa aattggatta tgcaaaaatt cattattaat 1740
ttttttcatc catcctttaa ttcagcaaac atttatctgt tgttgacttt atgcagtatg 1800
gccttttaag gattggggga caggtgaaga acggggtgcc agaatgcatc ctcctactaa 1860
tgaggtcagt acacatttgc attttaaaat gccctgtcca gctgggcatg gtggatcatg 1920
cctgtaatct caacattgga aggccaaggc aggaggattg cttcagccca ggagttcaag 1980
accagcctgg gcaacataga aagaccccat ctctcaatca atcaatcaat gccctgtctt 2040

tgaaaataaa actctttaag aaaggtttaa tgggcagggt gtggtagctc atgcctataa 2100
tacagcactt tgggaggctg aggcaggagg atcactttag cccagaagtt caagaccagc 2160
ctgggcaaca agtgacacct catctcaatt ttttaataaa atgaatacat acataaggaa 2220
agataaaaag aaaagtttaa tgaaagaata cagtataaaa caaatctctt ggacctaaaa 2280
gtatttttgt tcaagccaaa tattgtgaat cacctctctg tgttgaggat acagaatatc 2340
taagcccagg aaactgagca gaaagttcat gtactaacta atcaacccga ggcaaggcaa 2400
aaatgagact aactaatcaa tccgaggcaa ggggcaaatt agacggaacc tgactctggt 2460
ctattaagcg acaactttcc ctctgttgta tttttctttt attcaatgta aaaggataaa 2520
aactctctaa aactaaaaac aatgtttgtc aggagttaca aaccatgacc aactaattat 2580
ggggaatcat aaaatatgac tgtatgagat cttgatggtt tacaaagtgt acccactgtt 2640
aatcacttta aacattaatg aacttaaaaa tgaatttacg gagattggaa tgtttctttc 2700
ctgttgtatt agttggctca ggctgccata acaaaatacc acagactggg aggcttaagt 2760
aacagaaatt catttctcac agttctgggg gctggaagtc cacgatcaag gtgcaggaaa 2820
ggcaggcttc attctgaggc ccctctcttg gctcacatgt ggccaccctc ccactgcgtg 2880
ctcacatgac ctctttgtgc tcctggaaag agggtgtggg ggacagaggg aaagagaagg 2940
agagggaact ctctggtgtc tcgtctttca aggaccctaa cctgggccac tttggcccag 3000
gcactgtggg gtggggggtt gtggctgctc tgctctgagt ggccaagata aagcaacaga 3060
aaaatgtcca aagctgtgca gcaaagacaa gccaccgaac agggatctgc tcatcagtgt 3120
ggggacctcc aagtcggcca ccctggaggc aagcccccam agagcccatg caaggtggca 3180
gcagcagaag aagggaattg tccctgtcct tggcacattc ctcaccgacc tggtgatgct 3240
ggacactgcg atgaatggta atgtggatga gaatatgatg gactcccaga aaaggagacc 3300
cagctgctca ggtggctgca aatcattaca gccttcatcc tggggaggaa ctgggggcct 3360
ggttctgggt cagagagcag cccagtgagg gtgagagcta cagcctgtcc tgccagctgg 3420
atccccagtc ccggtcaacc agtaatcaag gctgagcaga tcaggcttcc cggagctggt 3480
cttgggaagc cagccctggg gtgagttggc tcctgctgtg gtactgagac aatattgtca 3540
taaattcaat gcgcccttgt atcccttttt ctttttatc tgtctacatc tataatcact 3600
atgcatacta gtctttgtta gtgtttctat tcmacttaat agagatatgt tatacttaaa 3660
aaaaaaaaaa aaaa 3674

<210> 699
<211> 2051
<212> DNA
<213> Homo sapiens

<220>
<221> misc_feature
<222> 329
<223> n = A,T,C or G

<400> 699
ggaccagggg ctgaagtgaa cccccagcac agcacagctg ctctataaaa acgtggccag 60
actttttttt ttgaagcaag tccctgttct tgttcgtcct gactagtccc atcagggccc 120
tggatcccaa gactcagcat ccaaggtccc ctccaggaat cctggcagct cagcatactt 180
tatcctgttt catctgagag caaaaatgta aaattggatg cacagaaaag tgactcaaag 240
tgcttaatga ctagaagaaa tctaggagca gcaagaagag caggacaaac aggccaggcg 300
gtgtcaggag cccaggtctc cagctggang gaacgtcaac cctgcagtgg gagcaggggc 360
cctttgcaca tcctaggcac agatggtaat gtagacacca caggtaagct gggcttggta 420
cctacccctc cccggattca gaaagaaacc aaacaaggag ctttgtgtgg aatgaaacct 480
cctttcctcc cagaagcact gctgactgtt tggtggttgc catttgtggc agtgagccct 540
tgtttgttct gaggttgggc tggtttctcc tcttggccct gccctacaga tcataaagga 600
gaacagcaag acgtccccag caaacatcca cagatggcct tggaaataag tcaccttcct 660
caccctgcag gaatgccagt gaacatattg ctgacatctt ggagctcagt acctcatagt 720
gtaacggcgt cagtagatct gcctgtgctg ggacttcctg tactacccat tcctgagggg 780
cgatgcttct gcagggcctg tgacttggtg cacaacttca gacaccatca tcttgcagca 840
gcaccgcacc ctcactagcc agggtgttga tgacttcctc aaggccaagg ccacattcaa 900
ggcttcggac ttcattgatg cgcttgtgct gagcaaggtg gcttctccgg gatcttaatt 960
caggaggtag aatggagctt gagatcaagt gtctgatcaa gcctcagtgt atgggcgctg 1020
ttcatcctct ggtgctgaag cagccaagag acccaagtct gcctggctgc ctcttaggat 1080
atgacagcag agccagtggc ctctactaga tcctgtacaa cctcacaaaa cacccagaca 1140
tcgggagtgc tgccagcctg tgatgcaaga gtcctaatcc tgaagacatt gaatgacctg 1200
tcgttgtgct gtttttacca aaaaggatca tgaggatcag agaggaaaag tcacttgccc 1260
aaagtcacac agctgaacag tggtggagtt caactttgac cgtgggctgt ctggccccca 1320
aggtgtatgc ttgcttctct cccaagagac tcctttctta tcaggctcaa atgaatgaaa 1380
ggaggatgtt aaagacaacg ccattattga cgagatcact cccaagcgga ttggagattg 1440
tcccaatatt tagacctata gcaaggcctt gggagaaatg gtggtgcagc aggagagcag 1500
gaacctaacc attgccatcc taaggccctc cattgtgtgg agcaacgtgg caccagcttt 1560
tcctgggttg ggttgataat ctaaatggat gtagccgact cattattgcg gtatgtatag 1620
ggatgaagaa gtaactgtaa tgtagtggag gaatagtaag aaaattctta gtgctggctt 1680

agcttaattg atccaaaaac ataaatgcta ctttactatc aattgaagca tattatttca 1740
attattctgg ttataatatg gaggcaggat gaaattgttt ttattctttt agaatttttt 1800
tttatcagga aaacagaggt aaagtgctat caattactat ttaagagttc tattttgaaa 1860
agtgagaatt aaggattttt cttttctttt taaaaaaaac ttttttaaaa attaaaaata 1920
aaagaagcaa aagtcttagg aaaatgaagc aagtagccct gccactctat gtacagtaat 1980
aacaatatct gtcccagtta ttatgtacaa tattataaaa aatgtcgcag acagtaaaaa 2040
aaaaaaaaaa a 2051

<210> 700
<211> 2841
<212> DNA
<213> Homo sapiens

<220>
<221> misc_feature
<222> 1447, 2244
<223> n = A,T,C or G

<400> 700
   gcagagcaca gcatagctgc tttaccaaat catggccaga ctgcttctgt aagcaggccc 60
   ctgatcctgt tccacctcac tggacaggac ctcccaactg gggcctccag ctacccccac 120
   cagcatccct tggccaatgg aaatttgaaa tgttcctggg acagagctcc tggagagagg 180
   ggcaggccac cacctttgct gtttgggtga ctagccgttc tggcctgcag gctttggaga 240
   gcccaagctg acaaggggta gaagaggtgc ctcagcacag cacagccacg ctacgaaaac 300
   atggccagac tcttgtttaa gtcagtcccc gaacacattt ctagtcagtg ggtgaagtct 360
   ttcaaccagg gtctctggct accttgactg ctgttctctg gccgacagag gtctcaggcc 420
   tccctgagtc agagctcccg gggggaggac cagattgtca tctttgctgt ttgggtgacc 480
   cagccatttc agccttaggg cttcagagtg tctgaggtag ccaggggctg aagtgaaccc 540
   ccagcacagc acagctgctg tataaaaacg tggccagact ttttctttaa gcaagtccct 600
   gttcttattc ctcctgacta ggtaagactt ctcaacttgc ctccagccac atcttattgg 660
   tgtgttcaga ttggcaacag gtttgtacct cagtggtaca gagctcccag aggaaggggt 720
   aggctatcat cttccctgga aaatacgagt caattaggga cttgagggga cccccagcat 780
   tccacagcag cccttcagaa aagtggccag actctgtact tgatgggcag atcctcctgg 840
   cctgtgtctc tagccagccc accactggag ctatcaagcc agtagcaact cagcagttcc 900
   ttggacagag cttccaggag caaatgaaat cctttctgcc actgcctttg cagtgaactg 960
   cccttgctat cctcagaaga tatatcacgg gagcaaagac cctaagtgcc atatcaacac 1020
   ctccaataag ctgcagttga cccaaagaac aagccaatcc atctcccaca ggttccacac 1080
   acactccact actcatcacc agacagggaa ccctggcttg ggcccacagc acagaccctc 1140
   catcctgggc cgattacact gagtgattgc taactcacat gtctctggga tggagcaccc 1200
   aggagacaag caaagtggtg gagcagcaag tcaggtgatg tggagcccag agggcaggga 1260
   gagctatctc tctgggctcc acttgccctt gtgagacact ttgtcccagc actccttagt 1320
   ctgcttgcct ctcccagggc cccagcctgg ccacacctgc ttacagggca ctctcagatg 1380
   cccataccat agtttctgtg ctagtggacc gtaccatatc agtggagagc tgcagcaagg 1440
   tggcccntac ggccacgcac cagcctgcac attacctctc catactgcag ccctttatat 1500
   ggaaacttcc tacatcactt tgctgtgtgt gtttacacag gtggattttg ctttacttgc 1560
   actgacagca cacaggaggg cagcacacac cccaacccac atcaactgcc attaaagaaa 1620
   agaaatttca gcccataatt tcatgtccag caaaattagg catcataagt gaaggagaaa 1680
   taagatcctt ttcagacaag caaatgctga gggaattcaa tatcaccaga tctaccttac 1740
   aagagctcct gaaggaagca ctaaatatgg aaagaaaaaa ccatcaccag ccactacaaa 1800
   aatgcagtga agaacgcagt gaattacgca gtccagtgat gctaaaaacc aaccacatac 1860
   gttaagtctg caaaataacc agctgacagc atgacgacag gataaatcca cacataccat 1920
   tactaacctt aaatgaaaat gggctaaatg ctcccattga aagacatggg gcaagctgga 1980
   taaagaacca agacccactg gagtatgctg tcttcaagaa acccatctca catgcggtgg 2040
   catacatagg ctcaaaataa aggaatggag aaaaatattt caagcaaatg gaaaacagaa 2100
   aaaagcaggt gttgcactcc tactttctga caaaacagac tatgcgaata aagataaaaa 2160
   agagaaggac attacaaagg tggtcctgac ctttgatata tctcattgct tgataccaac 2220
   ctgggctgtt ttaattgccc aaanccaata ggataatttg ctgaggttgt ggagcttctc 2280
   ccctgcagag agtccctgat ctcccaaaat ttggttgaga tgtaaggttg attttgctgt 2340
   acaactcctt ttctgaagtt ttactcattt ccaaaaagga aggcaagttt tcctgcttcc 2400
   atgacgatgg agagcaggca tctcctttcc tgagtttcag cttgcttctg acagggaagg 2460
   tgagtgtaag ttttttccag cttctaagat ggcagagaac gatcaccagc ctgagcctta 2520
   tttccaggta agtagctgaa ttagagtttt gtcttaaaat ttttccttaa tgattaaaat 2580
   gtaagattac ccaccagctg cttttaattt ctcccttagc attagaacac tcagtaatca 2640
   tatgaattgt gcatttgttt gttttgctta actctttctg tttgtttatg tttggggttt 2700
   tattgttgtt gtttcacttt tctcccatct cttcctgact tggtcaaatc caaaggaatg 2760
   ttcgaaattg tggggagcaa ggcatctgaa atggctaaaa ctcctgtggc tgcaaaaaat 2820
   agaaataaaa aaaaaaaaaa a 2841

<210> 701
<211> 3228
<212> DNA
<213> Homo sapiens

<220>
<221> misc_feature
<222> 66, 2343, 2387, 3065
<223> n = A,T,C or G

<400> 701
   tccgccccat tgacgcaaat ggcggtaggc gtgtacggtg ggaggtctat ataagcagag 60
   ctctcnggct aactagagaa cccactgctt actggcttat cgaaattaat acgactcact 120
   atagggagac ccaagctggc tagcgtttaa acttaagctt ggtaccgagc tcggatccac 180
   tagtccagtg tggtggaatt ccattgtgtt gggcaggaaa caagcaaagt ggtggagcag 240
   caagtcaggt gatgtggagc ccagaggtca gggatggctg tctctctagg gtccacttgc 300
   ccttgtgaga cactttatcc cagcacttta ggaatactga ggtcatacca gccacatctt 360
   atatgcaaga ttgcccagca gagatcaggt ccgagagttc cctttttaaa aaaaggagac 420
   ttgcttaata aaagaagtct agccacgttt gtgtagagcg gctgtgctgt gctgggggtt 480
   cacttttgag agagttctcc tctgagacct gatctctgga ggctgggcaa tcttgcactt 540
   gagatggggc tggtctgatc tcagcactcc ttagtctgct cgcctctccc atggccccag 600
   cctggccaca cctgcttacg gggcactctt agatgcccac accataactt ccatgctagt 660
   ggactgtacc atatcagtgg agagctgcag caaggtggcc cctagagcca cgcaccagcc 720
   tgcacattgc ctctccatac ggcagccctt tatttggaaa cttcctaaat cactttgctg 780
   tgtgtgttta cacgggtgtg ttttgcttta cttgccctga gagcacacgg gagtgcagca 840
   cacaccccaa cccacatcaa ctgccattaa agaaaagaaa tttcagccca gaatttcatg 900
   tccagcaaaa ttaagcatca taagtgaagg agaaataaga tccttttcag acaagcaagt 960
   gctgagggaa tttggtatca ccagatctac cttacgagag ctcctgaagg aagcactaaa 1020
   tatggaaaga aaagatcatc acctgctact acaaaaacac actgaagtac acagtccaat 1080
   gatgctaaaa agcaagcaca tatgtaagtc tgcaaaataa ccagctgaca gcatgacgac 1140
   aggataaaat ccacacatac cattactaac cttaaatgta aatgggctaa atgctcccat 1200
   tgaaagacac ggggcaagct gggtaaagaa ccaagaccca ctggagtatg ccgtcttcaa 1260
   gcaacccatc tcacgtgcag tgccatacat aggctcaaaa taaaggaatg gagaaaaata 1320
   tttcaagcaa atggaaaaca gaaaaaaggt gttgcactcc cagtttctga caaaacagac 1380
   tctaccaata aagataaaaa aagagaagga cattacaaag gtggtcctga cctttgataa 1440
   atctcattat tgcttgatac caacctgggc tatttgtatt gcccaaacga ataggataat 1500
   ttgctgaggt tgtggagctt ctccccttca cagagtccct gatctccgaa aatttggttg 1560
   agatgtaagg ttgattttgc tgtacaactc cttttttgaa gttttactca tttccaacaa 1620
   ggaaggcaag ttttcctgct tccattgaca aaggagagca ggcacctcct ttcctgagtt 1680
   tcagcttgct tctgacaggg aaggagcttt gagatttgaa tactggcctg ctgggttttg 1740
   gacgtgcatt gggcctgtgg tcccatttgt gttatttttc tgggaaattt cttccctttg 1800
   gagtgagaaa gcttacccaa tgcctgtacc atcatcgtac cttaaaagaa ctccatttta 1860
   agttcaggga ctccttggca gaagagaccg tagccttgta tcagatcata aaggagaaga 1920
   gcaagaggtc cccggcaaac atccacagat ggccttggaa ataagtcacc ttgctcaccc 1980
   tgcaggaatg ccagtgaact tattgctgac atcttggagc tcagtaccct catagtgtaa 2040
   cggcgtcagc agatctgcct gtgctgggac ttcctgtact acccattcct gaggggcgat 2100
   gcttctgcag ggcctgtgac ttggtgcaca acttcagaca ccatcatctt gcagcagcac 2160
   cgcaccctca ctagccaggg tgttgatgac ttcctcaagg ccaaggccac attcaaggct 2220
   tcggacttca ttgatgcgct tgtgctgagc aaggtggctt ctccgggatc ttaattcagg 2280
   aggtagaatg gagcttgaga tcaagtgtct gatcaagcct cagtgtatgg gcgctgttca 2340
   tcntctggtg ctgaagcagc caagagaccc aagtctgcct ggctgcntct taggatatga 2400
   cagcagagcc agtggcctct actagatcct gtacaacctc acaaaacacc cagacatcgg 2460
   gagtgctgcc agcctgtgat gcaagagtcc taatcctgaa gacattgaat gacctgtcat 2520
   tctgctgttt ttaccaaaaa ggatcatgag gatcagagag gaaaagtcac ttgcccaaag 2580
   tcacacagct gaacagtggt ggagttcaac tttgaccgtg ggctgtctga ccccaaggtg 2640
   tatgcttgct tctctcccaa gagacaactt tcttatcagg ctcaaatgaa tgaaaggagg 2700
   atgttaaagg taggatctct gaagcctgtg ccagtggaac cgcagctcat ggctggcacc 2760
   tgtgttctca ttcttacctc attaagagta aagtttattg agtttattga atttaagtat 2820
   ctttagtgag atcatatatt attagtaaga actgggacca aacagatttt ctgactctaa 2880
   aagagagatt ttcacagaaa cagatatata cctgtaagta tacagacacg catacacaca 2940
   tttctttact gctcataaaa attagtcctt attagaatgt gggatgtata aatgtaagag 3000
   aattttcatg ttaaaattga cagatacatt tttaaattgt cctaaaataa atttaattat 3060
   ttttntttta gaattttcca ttattaatgt tatttttatg agaaactata taactttatt 3120
   gataatacat acaataaccc tttgtttttc aaattgaaaa tacagtgtat tttgcaaata 3180
   actaagtcct aattttgtat taaaatttta aattttcaaa aaaaaaaa 3228

<210> 702
<211> 4894

<212> DNA
<213> Homo sapiens

<400> 702
ctgcacgcgc tggctccggg tgacagccgc gcgcctcggc caggatctga gtgatgagac 60
gtgtccccac tgaggtgccc cacagcagca ggtgttgagc atgggctgag aagctggacc 120
ggcaccaaag ggctggcaga aatgggcgcc tggctgattc ctaggcagtt ggcggcagca 180
aggaggagag gccgcagctt ctggagcaga gccgagacga agcagttctg gagtgcctga 240
acggccccct gagccctacc cgcctggccc actatggtcc agaggctgtg ggtgagccgc 300
ctgctgcggc accggaaagc ccagctcttg ctggtcaacc tgctaacctt tggcctggag 360
gtgtgtttgg ccgcaggcat cacctatgtg ccgcctctgc tgctggaagt gggggtagag 420
gagaagttca tgaccatggt gctgggtgag tcactacatc ctccttcctt cctgttccag 480
atacatgcca cctggcatgt gggacaggag tacctctgcc ctgggagctg cttggaggga 540
gaggtggtct gctgggaagg cattgctggg caggagggtg accctgggct gagggggcac 600
accaagagaa agaagagaat accaaggaca taccccagtc acctctggat ccctggtcct 660
gcacagagcc tggctcatag gagacactgg agaaatgctc ctaacctttg gctagccctt 720
ttataattta tagcgattat ctcatttaat gcttacaacc accatttgag gtgatccatt 780
ttacagagaa ggaagcagag gcttttaaga ggttaggtaa gtcttagcca aagccaaata 840
gcagctgaac agtagagctg ggactccatc aaggtctccc agccggagct tgctcctacc 900
cctaggacaa ggggtggact cctgactctg cagataaatt ctacaaaagc cacagaaggc 960
aagtagtaac cattgtgtga caacccctca cccccaggaa gaggggcccc tgtgaggatt 1020
gcaggctctg gagtcacact gcttgttgaa acgctgcctc ttaccctccc taggtctgcg 1080
cctttgaata agtatcactt cttagttgct ccatgcctca gtttgtccat ctgaaaatgg 1140
gggcatctgt aatgcctgtg ttatgaggag taaattacag catccctgtg aagacgtagc 1200
acagtgtcga gtacggaatg ttatttccat ccttctcacg gagcttggtt ccccttcccc 1260
ttgcccttta cttgtcccag ccattgactc atactacttc ccttcttgca ggcattggtc 1320
cagtgctggg cctggtctgt gtcccgctcc taggctcagc cagtgaccac tggcgtggac 1380
gctatggccg ccgccggccc ttcatctggg cactgtcctt gggcatcctg ctgagcctct 1440
ttctcatccc aagggccggc tggctagcag ggctgctgtg cccggatccc aggcccctgg 1500
agctggcact gctcatcctg ggcgtggggc tgctggactt ctgtggccag gtgtgcttca 1560
ctccactgga ggccctgctc tctgacctct tccgggaccc ggaccactgt cgccaggcct 1620
actctgtcta tgccttcatg atcagtcttg gggggctgcct gggctacctc ctgcctgcca 1680
ttgactggga caccagtgcc ctggccccct acctgggcac ccaggaggag tgcctctttg 1740
gcctgctcac cctcatcttc ctcacctgcg tagcagccac actgctggtg gctgaggagg 1800
cagcgctggg ccccaccgag ccagcagaag ggctgtcggc cccctccttg tcgccccact 1860
gctgtccatg ccgggcccgc ttggctttcc ggaacctggg cgccctgctt ccccggctgc 1920
accagctgtg ctgccgcatg ccccgcaccc tgcgccggct cttcgtggct gagctgtgca 1980
gctggatggc actcatgacc ttcacgctgt tttacacgga tttcgtgggc gaggggctgt 2040
accagggcgt gcccagagct gagccgggca ccgaggcccg gagacactat gatgaaggta 2100
aggccttggc agccagcaga ggctggtgtg ggagccgccc accagagacg acactcgggg 2160
ctgtgtctgg gctggtgcct ctccatcctg gccccgactt ctctgtcagg aaagtgggga 2220
tggaccccat ctgcatacac ggcttctcat gggtgtggaa catctctgct tgcggtttca 2280
ggaaggcctc tggctgctct aggagtctga tcagagtcgt tgccccagtt tgacagaagg 2340
aaaggcggag cttattcaaa gtctagaggg agtggaggag ttaaggctgg atttcagatc 2400
tgcctggttc cagccgcagt gtgccctctg ctcccccaac gactttccaa ataatctcac 2460
cagcgccttc cagctcaggc gtcctagaag cgtcttgaag cctatggcca gctgtctttg 2520
tgttccctct cacccgcctg tcctcacagc tgagactccc aggaaacctt cagactacct 2580
tcctctgcct tcagcaaggg gcgttgccca cattctctga gggtcagtgg aagaacctag 2640
actcccattg ctagaggtag aaaggggaag ggtgctgggg agcagggctg gtccacagca 2700
ggtctcgtgc agcaggtacc tgtggttccg ccttctcatc tccctgagac tgctccgacc 2760
cttccctccc aggctctgtc tgatggcccc tctccctctg caggcgttcg gatgggcagc 2820
ctggggctgt tcctgcagtg cgccatctcc ctggtcttct ctctggtcat ggaccggctg 2880
gtgcagcgat tcggcactcg agcagtctat ttggccagtg tggcagcttt ccctgtggct 2940
gccggtgcca catgcctgtc ccacagtgtg gccgtggtga cagcttcagc cgccctcacc 3000
gggttcacct tctcagccct gcagatcctg ccctacacac tggcctccct ctaccaccgg 3060
gagaagcagg tgttcctgcc caaataccga ggggacactg gaggtgctag cagtgaggac 3120
agcctgatga ccagcttcct gccaggccct aagcctggag ctcccttccc taatggacac 3180
gtgggtgytg gaggcagtgg cctgctccca cctccacccg cgctctgcgg ggcctctgcc 3240
tgtgatgtct ccgtacgtgt ggtggtgggt gagcccaccg aggccagggt ggttccgggc 3300
cggggcatct gcctggacct cgccatcctg gatagtgcct tcctgctgtc ccaggtggcc 3360
ccatccctgt ttatgggctc cattgtccag ctcagccagt ctgtcactgc ctatatggtg 3420
tctgccgcag gcctgggtct ggtcgccatt tactttgcta cacaggtagt atttgacaag 3480
agcgacttgg ccaaatactc agcgtagaaa acttccagca cattggggtg gagggcctgc 3540
ctcactgggt cccagctccc tgctcctgtt agccccatgg ggctgccggg ctggccgcca 3600
gtttctgttg ctgccaaagt aatgtggctc tctgctgcca ccctgtgctg ctgaggtgcg 3660
tagctgcaca gctgggggct ggggcgtccc tctcctctct ccccagtctc tagggctgcc 3720
tgactggagg ccttccaagg gggtttcagt ctggacttat acagggaggc cagaagggct 3780
ccatgcactg gaatgcgggg actctgcagg tggattaccc aggctcaggg ttaacagcta 3840

gcctcctagt tgagacacac ctagagaagg gtttttggga gctgaataaa ctcagtcacc 3900
tggtttccca tctctaagcc ccttaacctg cagcttcgtt taatgtagct cttgcatggg 3960
agtttctagg atgaaacact ccaccatggg atttgaacat atgaaagtta tttgtagggg 4020
aagagtcctg aggggcaaca cacaagaacc aggtcccctc agcccacagc actgtctttt 4080
tgctgatcca cccccctctt accttttatc aggatgtggc ctgttggtcc ttctgttgcc 4140
atcacagaga cacaggcatt taaatattta acttatttat ttaacaaagt agaagggaat 4200
ccattgctag cttttctgtg ttggtgtcta atatttgggt agggtggggg atccccaaca 4260
atcaggtccc ctgagatagc tggtcattgg gctgatcatt gccagaatct tcttctcctg 4320
gggtctggcc ccccaaaatg cctaacccag gaccttggaa attctactca tcccaaatga 4380
taattccaaa tgctgttacc caaggttagg gtgttgaagg aaggtagagg gtggggcttc 4440
aggtctcaac ggcttcccta accacccctc ttctcttggc ccagcctggt tccccccact 4500
tccactcccc tctactctct ctaggactgg gctgatgaag gcactgccca aaatttcccc 4560
tacccccaac tttcccctac cccaactttt ccccaccagc tccacaaccc tgtttggagc 4620
tactgcagga ccagaagcac aaagtgcggt ttcccaagcc tttgtccatc tcagccccca 4680
gagtatatct gtgcttgggg aatctcacac agaaactcag gagcaccccc tgcctgagct 4740
aagggaggtc ttatctctca gggggggttt aagtgccgtt tgcaataatg tcgtcttatt 4800
tatttagcgg ggtgaatatt ttatactgta agtgagcaat cagagtataa tgtttatggt 4860
gacaaaatta aaggctttct tatatgttta aaaa 4894

<210> 703
<211> 2904
<212> DNA
<213> Homo sapiens

<400> 703
gtctatgcct tcatgatcag tcttgggggc tgcctgggct acctcctgcc tgccattgac 60
tgggacacca gtgccctggc cccctacctg ggcacccagg aggagtgcct ctttggcctg 120
ctcaccctca tcttcctcac ctgcgtagca gccacactgc tggtggctga ggaggcagcg 180
ctgggcccca ccgagccagc agaagggctg tcggccccct ccttgtcgcc ccactgctgt 240
ccatgccggg cccgcttggc tttccggaac ctgggcgccc tgcttccccg gctgcaccag 300
ctgtgctgcc gcatgccccg caccctgcgc cggctcttcg tggctgagct gtgcagctgg 360
atggcactca tgaccttcac gctgttttac acggatttcg tgggcgaggg gctgtaccag 420
ggcgtgccca gagctgagcc gggcaccgag gcccggagac actatgatga aggaaggcct 480
ctggctgctc taggagtctg atcagagtcg ttgccccagt ttgacagaag gaaaggcgga 540
gcttattcaa agtctagagg gagtggagga gttaaggctg gatttcagat ctgcctggtt 600
ccagccgcag tgtgccctct gctcccccaa cgactttcca aataatctca ccagcgcctt 660
ccagctcagg cgtcctagaa gcgtcttgaa gcctatggcc agctgtcttt gtgttccctc 720
tcacccgcct gtcctcacag ctgagactcc caggaaacct tcagactacc ttcctctgcc 780
ttcagcaagg ggcgttgccc acattctctg agggcgttcg gatgggcagc ctggggctgt 840
tcctgcagtg cgccatctcc ctggtcttct ctctggtcat ggaccggctg gtgcagcgat 900
tcggcactcg agcagtctat ttggccagtg tggcagcttt ccctgtggct gccggtgcca 960
catgcctgtc ccacagtgtg gccgtggtga cagcttcagc cgccctcacc gggttcacct 1020
tctcagccct gcagatcctg ccctacacac tggcctccct ctaccaccgg gagaagcagg 1080
tgttcctgcc caaataccga ggggacactg gaggtgctag cagtgaggac agcctgatga 1140
ccagcttcct gccaggccct aagcctggag ctcccttccc taatggacac gtgggtgctg 1200
gaggcagtgg cctgctccca cctccacccg cgctctgcgg ggcctctgcc tgtgatgtct 1260
ccgtacgtgt ggtggtgggt gagcccaccg aggccagggt ggttccgggc cggggcatct 1320
gcctggacct cgccatcctg gatagtgcct tcctgctgtc ccaggtggcc ccatccctgt 1380
ttatgggctc cattgtccag ctcagccagt ctgtcactgc ctatatggtg tctgccgcag 1440
gcctgggtct ggtcgccatt tactttgcta cacaggtagt atttgacaag agcgacttgg 1500
ccaaatactc agcgtagaaa acttccagca cattggggtg gagggcctgc ctcactgggt 1560
cccagctccc cgctcctgtt agccccatgg ggctgccggg ctggccgcca gtttctgttg 1620
ctgccaaagt aatgtggctc tctgctgcca ccctgtgctg ctgaggtgcg tagctgcaca 1680
gctgggggct ggggcgtccc tctcctctct ccccagtctc tagggctgcc tgactggagg 1740
ccttccaagg gggtttcagt ctggacttat acagggaggc cagaagggct ccatgcactg 1800
gaatgcgggg actctgcagg tggattaccc aggctcaggg ttaacagcta gcctcctagt 1860
tgagacacac ctagagaagg gtttttggga gctgaataaa ctcagtcacc tggtttccca 1920
tctctaagcc ccttaacctg cagcttcgtt taatgtagct cttgcatggg agtttctagg 1980
atgaaacact cctccatggg atttgaacat atgaaagtta tttgtagggg aagagtcctg 2040
aggggcaaca cacaagaacc aggtcccctc agcccacagc actgtctttt tgctgatcca 2100
cccccctctt accttttatc aggatgtggc ctgttggtcc ttctgttgcc atcacagaga 2160
cacaggcatt taaatattta acttatttat ttaacaaagt agaagggaat ccattgctag 2220
cttttctgtg ttggtgtcta atatttgggt agggtggggg atccccaaca atcaggtccc 2280
ctgagatagc tggtcattgg gctgatcatt gccagaatct tcttctcctg gggtctggcc 2340
ccccaaaatg cctaacccag gaccttggaa attctactca tcccaaatga taattccaaa 2400
tgctgttacc caaggttagg gtgttgaagg aaggtagagg gtggggcttc aggtctcaac 2460
ggcttcccta accacccctc ttctcttggc ccagcctggt tccccccact tccactcccc 2520
tctactctct ctaggactgg gctgatgaag gcactgccca aaatttcccc tacccccaac 2580

tttcccctac ccccaacttt ccccaccagc tccacaaccc tgtttggagc tactgcagga 2640
ccagaagcac aaagtgcggt ttcccaagcc tttgtccatc tcagccccca gagtatatct 2700
gtgcttgggg aatctcacac agaaactcag gagcaccccc tgcctgagct aagggaggtc 2760
ttatctctca ggggggtttt aagtgccgtt tgcaataatg tcgtcttatt tatttagcgg 2820
ggtgaatatt ttatactgta agtgagcaat cagagtataa tgtttatggt gacaaaatta 2880
aaggctttct tatatgttta aaaa 2904

<210> 704
<211> 4034
<212> DNA
<213> Homo sapiens

<400> 704
aaccagcctg cacgcgctgg ctccgggtga cagccgcgcg cctcggccag gatctgagtg 60
atgagacgtg tccccactga ggtgccccac agcagcaggt gttgagcatg ggctgagaag 120
ctggaccggc accaaagggc tggcagaaat gggcgcctgg ctgattccta ggcagttggc 180
ggcagcaagg aggagaggcc gcagcttctg gagcagagcc gagacgaagc agttctggag 240
tgcctgaacg gccccctgag ccctacccgc ctggcccact atggtccaga ggctgtgggt 300
gagccgcctg ctgcggcacc ggaaagccca gctcttgctg gtcaacctgc taacctttgg 360
cctggaggtg tgtttggccg caggcatcac ctatgtgccg cctctgctgc tggaagtggg 420
ggtagaggag aagttcatga ccatggtgct gggcattggt ccagtgctgg gcctggtctg 480
tgtcccgctc ctaggctcag ccagtgacca ctggcgtgga cgctatggcc gccgccggcc 540
cttcatctgg gcactgtcct tgggcatcct gctgagcctc tttctcatcc caagggccgg 600
ctggctagca gggctgctgt gcccggatcc caggcccctg gagctggcac tgctcatcct 660
gggcgtgggg ctgctggact tctgtggcca ggtgtgcttc actccactgg aggccctgct 720
ctctgacctc ttccgggacc cggaccactg tcgccaggcc tactctgtct atgccttcat 780
gatcagtctt gggggctgcc tgggctacct cctgcctgcc attgactggg acaccagtgc 840
cctggccccc tacctgggca cccaggagga gtgcctcttt ggcctgctca ccctcatctt 900
cctcacctgc gtagcagcca cactgctggt ggctgaggag gcagcgctgg gccccaccga 960
gccagcagaa gggctgtcgg ccccctcctt gtcgccccac tgctgtccat gccgggcccg 1020
cttggctttc cggaacctgg gcgccctgct tccccggctg caccagctgt gctgccgcat 1080
gccccgcacc ctgcgccggc tcttcgtggc tgagctgtgc agctggatgg cactcatgac 1140
cttcacgctg ttttacacgg atttcgtggg cgaggggctg taccagggcg tgcccagagc 1200
tgagccgggc accgaggccc ggagacacta tgatgaaggt aaggccttgg cagccagcag 1260
aggctggtgt gggagccgcc caccagagac gacactcggg gctgtgtctg ggctggtgcc 1320
tctccatcct ggccccgact tctctgtcag gaaagtgggg atggacccca tctgcataca 1380
cggcttctca tgggtgtgga acatctctgc ttgcggtttc aggaaggcct ctggctgctc 1440
taggagtctg atcagagtcg ttgccccagt ttgacagaag gaaaggcgga gcttattcaa 1500
agtctagagg gagtggagga gttaaggctg gatttcagat ctgcctggtt ccagccgcag 1560
tgtgccctct gctcccccaa cgactttcca aataatctca ccagcgcctt ccagctcagg 1620
cgtcctagaa gcgtcttgaa gcctatggcc agctgtcttt gtgttccctc tcacccgcct 1680
gtcctcacag ctgagactcc caggaaacct tcagactacc ttcctctgcc ttcagcaagg 1740
ggcgttgccc acattctctg agggtcagtg gaagaaccta gactcccatt gctagaggta 1800
gaaaggggaa gggtgctggg gagcagggct ggtccacagc aggtctcgtg cagcaggtac 1860
ctgtggttcc gccttctcat ctccctgaga ctgctccgac ccttccctcc caggctctgt 1920
ctgatggccc ctctccctct gcaggcgttc ggatgggcag cctggggctg ttcctgcagt 1980
gcgccatctc cctggtcttc tctctggtca tggaccggct ggtgcagcga ttcggcactc 2040
gagcagtcta tttggccagt gtggcagctt tccctgtggc tgccggtgcc acatgcctgt 2100
cccacagtgt ggccgtggtg acagcttcag ccgccctcac cgggttcacc ttctcagccc 2160
tgcagatcct gccctacaca ctggcctccc tctaccaccg ggagaagcag gtgttcctgc 2220
ccaaataccg aggggacact ggaggtgcta gcagtgagga cagcctgatg accagcttcc 2280
tgccaggccc taagcctgga gctcccttcc ctaatggaca cgtgggtgct ggaggcagtg 2340
gcctgctccc acctccaccc gcgctctgcg gggcctctgc ctgtgatgtc tccgtacgtg 2400
tggtggtggg tgagcccacc gaggccaggg tggttccggg ccggggcatc tgcctggacc 2460
tcgccatcct ggatagtgcc ttcctgctgt cccaggtggc cccatccctg tttatgggct 2520
ccattgtcca gctcagccag tctgtcactg cctatatggt gtctgccgca ggcctgggtc 2580
tggtcgccat ttactttgct acacaggtag tatttgacaa gagcgacttg gccaaatact 2640
cagcgtagaa aacttccagc acattggggt ggagggcctg cctcactggg tcccagctcc 2700
ccgctcctgt tagccccatg ggctgccgg gctggccgcc agtttctgtt gctgccaaag 2760
taatgtggct ctctgctgcc accctgtgct gctgaggtgc gtagctgcac agctgggggc 2820
tggggcgtcc ctctcctctc tccccagtct ctagggctgc ctgactggag gccttccaag 2880
ggggtttcag tctggactta tacagggagg ccagaagggc tccatgcact ggaatgcggg 2940
gactctgcag gtggattacc caggctcagg gttaacagct agcctcctag ttgagacaca 3000
cctagagaag ggtttttggg agctgaataa actcagtcac ctggtttccc atctctaagc 3060
cccttaacct gcagcttcgt ttaatgtagc tcttgcatgg gagtttctag gatgaaacac 3120
tcctccatgg gatttgaaca tatgaaagtt atttgtaggg gaagagtcct gaggggcaac 3180
acacaagaac caggtcccct cagcccacag cactgtcttt ttgctgatcc acccccctct 3240
tacctttat caggatgtgc ctgttggtcc ttctgttgcc atcacagaga cacaggcatt 3300

taaatattta acttatttat ttaacaaagt agaagggaat ccattgctag cttttctgtg 3360
ttggtgtcta atatttgggt agggtggggg atccccaaca atcaggtccc ctgagatagc 3420
tggtcattgg gctgatcatt gccagaatct tcttctcctg gggtctggcc ccccaaaatg 3480
cctaacccag gaccttggaa attctactca tcccaaatga taattccaaa tgctgttacc 3540
caaggttagg gtgttgaagg aaggtagagg gtggggcttc aggtctcaac ggcttcccta 3600
accacccctc ttctcttggc ccagcctggt tccccccact tccactcccc tctactctct 3660
ctaggactgg gctgatgaag gcactgccca aaatttcccc taccccccaac tttcccctac 3720
ccccaacttt ccccaccagc tccacaaccc tgtttggagc tactgcagga ccagaagcac 3780
aaagtgcggt ttcccaagcc tttgtccatc tcagccccca gagtatatct gtgcttgggg 3840
aatctcacac agaaactcag gagcaccccc tgcctgagct aagggaggtc ttatctctca 3900
gggggggttt aagtgccgtt tgcaataatg tcgtcttatt tatttagcgg ggtgaatatt 3960
ttatactgta agtgagcaat cagagtataa tgtttatggt gacaaaatta aaggctttct 4020
tatatgttta aaaa 4034

<210> 705
<211> 6976
<212> DNA
<213> Homo sapiens

<400> 705
   gaagctggac cggcaccaaa gggctggcag aaatgggcgc ctggctgatt cctaggcagt 60
   tggcggcagc aaggaggaga ggccgcagct tctggagcag agccgagacg aagcagttct 120
   ggagtgcctg aacggccccc tgagccctac ccgcctggcc cactatggtc cagaggctgt 180
   gggtgagccg cctgctgcgg caccggaaag cccagctctt gctggtcaac ctgctaacct 240
   ttggcctgga ggtgtgtttg gccgcaggca tcacctatgt gccgcctctg ctgctggaag 300
   tgggggtaga ggagaagttc atgaccatgg tgctgggtga gtcactacat cctccttcct 360
   tcctgttcca gatacatgcc acctggcatg tgggacagga gtacctctgc cctgggagct 420
   gcttggaggg agaggtggtc tgctgggaag gcattgctgg gcaggagggt gaccctgggc 480
   tgagggggca caccaagaga aagaagagaa taccaaggac ataccccagt cacctctgga 540
   tccctggtcc tgcacagagc ctggctcata ggagacactg gagaaatgct cctaaccttt 600
   ggctagccct tttataattt atagcgatta tctcatttaa tgcttacaac caccatttga 660
   ggtgatccat tttacagaga aggaagcaga ggcttttaag aggttaggta agtcttagcc 720
   aaagccaaat agcagctgaa cagtagagct gggactccat caaggtctcc cagccggagc 780
   ttgctcctac ccctaggaca aggggtggac tcctgactct gcagataaat tctacaaaag 840
   ccacagaagg caagtagtaa ccattgtgtg acaacccctc acccccagga agaggggccc 900
   ctgtgaggat tgcaggctct ggagtcacac tgcttgttga aacgctgcct cttaccctcc 960
   ctaggtctgc gcctttgaat aagtatcact tmttagttgc tccatgcctc agtttgtcca 1020
   tctgaaaatg ggggcatctg taatgcctgt gttatgagga gtaaattaca gcatccctgt 1080
   gaagacgtag cacagtgtcg agtacggaat gttatttcca tccttctcac ggagcttggt 1140
   tccccttccc cttgcccttt acttgtccca gccattgact catactactt cccttcttgc 1200
   aggcattggt ccagtgctgg gcctggtctg tgtcccgctc ctaggctcag ccagtgacca 1260
   ctggcgtgga cgctatggcc gccgccggcc cttcatctgg gcactgtcct tgggcatcct 1320
   gctgagcctc tttctcatcc caagggccgg ctggctagca gggctgctgt gcccggatcc 1380
   caggcccctg gagctggcac tgctcatcct gggcgtgggg ctgctggact tctgtggcca 1440
   ggtgtgcttc actccactgg aggccctgct ctctgacctc ttccgggacc cggaccactg 1500
   tcgccaggcc tactctgtct atgccttcat gatcagtctt gggggctgcc tgggctacct 1560
   cctgcctgcc attgactggg acaccagtgc cctggccccc tacctgggca cccaggagga 1620
   gtgcctcttt ggcctgctca ccctcatctt cctcacctgc gtagcagcca cactgctggt 1680
   ggctgaggag gcagcgctgg gccccaccga gccagcagaa gggctgtcgg ccccctcctt 1740
   gtcgccccac tgctgtccat gccgggcccg cttggctttc cggaacctgg gcgccctgct 1800
   tccccggctg caccagctgt gctgccgcat gccccgcacc ctgcgccggc tcttcgtggc 1860
   tgagctgtgc agctggatgg cactcatgac cttcacgctg ttttacacgg atttcgtggg 1920
   cgaggggctg taccagggcg tgcccagagc tgagccgggc accgaggccc ggagacacta 1980
   tgatgaaggt aaggccttgg cagccagcag aggctggtgt gggagccgcc caccagagac 2040
   gacactcggg gctgtgtctg ggctggtgcc tctccatcct ggccccgact tctctgtcag 2100
   gaaagtgggg atggacccca tctgcataca cggcttctca tgggtgtgga acatctctgc 2160
   ttgcggtttc aggaaggcct ctggctgctc taggagtctg atcagagtcg ttgccccagt 2220
   ttgacagaag gaaaggcgga gcttattcaa agtctagagg gagtggagga gttaaggctg 2280
   gatttcagat ctgcctggtt ccagccgcag tgtgccctct gctcccccaa cgactttcca 2340
   aataatctca ccagcgcctt ccagctcagg cgtcctagaa gcgtcttgaa gcctatggcc 2400
   agctgtcttt gtgttccctc tcacccgcct gtcctcacag ctgagactcc caggaaacct 2460
   tcagactacc ttcctctgcc ttcagcaagg ggcgttgccc acattctctg agggtcagtg 2520
   gaagaaccta gactcccatt gctagaggta gaaaggggaa gggtgctggg gagcagggct 2580
   ggtccacagc aggtctcgtg cagcaggtac ctgtggttcc gccttctcat ctccctgaga 2640
   ctgctccgac ccttccctcc caggctctgt ctgatggccc ctctccctct gcaggcgttc 2700
   ggatgggcag cctggggctg ttcctgcagt gcgccatctc cctggtcttc tctctggtca 2760
   tggaccggct ggtgcagcga ttcggcactc gagcagtcta tttggccagt gtggcagctt 2820
   tccctgtggc tgccggtgcc acatgcctgt cccacagtgt ggccgtggtg acagcttcag 2880
   ccgccctcac cgggttcacc ttctcagccc tgcagatcct gccctacaca ctggcctccc 2940
   tctaccaccg ggagaagcag gtactcattg gccagtgggt ggagtcaggg tgggaggggt 3000
   ggtctgggtt tttgggaggc caactagctc agaacctggt atctggcaag caactttgga 3060
   gaatgcttct ttgaatcaga gaagaagctt atcctagccc cagggccaga ggcttgggct 3120
   gcagaacagt gtagattaga ttctgggaat gacttcctgg ggtcaggact gtgtagcact 3180
   tgaatggatg attgcaggaa atgcaaaata cgatagtggg aatcccgaag ggtcaggcca 3240
   gcaggagccc taggcttcta ggctggttgt tctatggaga ggcagggcgc tgaatcagat 3300
   gacccctggg ccattcagcc tcagcagacg ggagtgggaa tggtccagcc ttagcaacac 3360
   ctttcttcag ggagcagcaa cctgacttag cctgtatcct actctggtct ctgagatggg 3420
   gcaggctcct tcctaccccc tttctttctg gcttattttt cttttctgtc taattccctt 3480
   ttcttttcct gcatccctcc tttgcctcct tccctttctc cttccccttc cccttcccct 3540
   gtggcagata tctgagcttg acacctgacc cactcacttg ggcactgtgt aagttgtggg 3600
   gacctccttc ttggttggcc ctacactaac cagcccctcc aggggcccct ttccttggga 3660
   agccacctaa cccaggtagt gtggtcatcc ttgtcccctc cactgacctc actgagctac 3720
   aaacctgggt gctggactct gccttgaggg gcatgaagtt ggggtgtccc aagggaggag 3780
   gagatgcagg actgctctca tagagctctc agactgtagg gaagacctgc ccctgcgtct 3840
   cgtagcactt gaggagagga gtaggtaagt tcgtagctga gaggctggtt aactgagtag 3900
   gtagctgcag gggtgagagg tatggagggg aggggctaag gttttggttg ggggagcctg 3960
   gtccctgaga cccctgttag cccactgata accttcttca gccttcactc ttctgcttgc 4020
   ctgggctggg ggcagggggc tggcatcagc ggccaggcct gagtatgtgc tgtcgtgcca 4080
   gggaacgttc tggggctagc catcttctcc agatggagga gcatgtctgt cctcggacca 4140
   ctccagactc caacctcagc ggacattcct ggggtggcag gcagggagga gaagtcctgg 4200
   gaggcccctt cctaacagca gctgatggca gacttggcac tgcacgctgt ctgcctgttc 4260
   ctttgcccac ttgttgagct gcatggtgag ccgtgggctt ccctggtgtc aggtttgagc 4320
   tctgccatgg ctcccacctc gcaaatgcag ccaactcaac tcttctggca tggggacaat 4380
   gttggataag acctggcctt gtccttaaat aggaggctct gggccatcaa gggcaggggt 4440
   tggggggatg gtggtcgacc agtcactctg atctaagtca gacagcagga aggaagtgag 4500
   aagccttcaa cattagcaca gctggggctg ggggaggtgg gaagagggac attcctcctg 4560
   cttggggtct actggattct ccctgcccca aggctgggga caagggagct catggcaggg 4620
   cagctaccct agtggcatct gggaccccag agaggcagag cttctctgca ccgggcaatg 4680
   aggatttcca gatgtcggag tggagggcag gcaggaagga aggttaggag agcctgcgtg 4740
   gggtttgggc catcaggggc cctgccttgg cttttgttcc tctgttctgt gcatctctta 4800
   ccaccgtctt cattccccct gtgtcttttc cttaccttgg agctctgttc tctctgatct 4860
   gtgatattga gtttgtctgc ctcttacctg ttctaagagg ctagaggaga cctagacttc 4920
   tgggttcaca tttgtccccg ccctaccccg ttacccttct cccactcctg aggaagggtc 4980
   ctggttagac ttggaccaag tagggtctcc atcttctctc ctgctcctga ttctcatgaa 5040
   gtcccattgc ccctgggatg gaggcaaggg tctgttctca cagctggggt ggtgccagtg 5100
   ctgggtacac acctgtcctc ttcccctttt cttcacccct ctgccttagg tgttcctgcc 5160
   caaataccga ggggacactg gaggtgctag cagtgaggac agcctgatga ccagcttcct 5220
   gccaggccct aagcctggag ctcccttccc taatggacac gtgggtgctg gaggcagtgg 5280
   cctgctccca cctccacccg cgctctgcgg ggcctctgcc tgtgatgtct ccgtacgtgt 5340
   ggtggtgggt gagcccaccg aggccagggt ggttccgggc cggggcatct gcctggacct 5400
   cgccatcctg gatagtgcct tcctgctgtc ccaggtggcc ccatccctgt ttatgggctc 5460
   cattgtccag ctcagccagt ctgtcactgc ctatatggtg tctgccgcag gcctgggtct 5520
   ggtcgccatt tactttgcta cacaggtagt atttgacaag agcgacttgg ccaaatactc 5580
   agcgtagaaa acttccagca cattggggtg gagggcctgc ctcactgggt cccagctccc 5640
   cgctcctgtt agccccatgg ggctgccggg ctggccgcca gtttctgttg ctgccaaagt 5700
   aatgtggctc tctgctgcca ccctgtgctg ctgaggtgcg tagctgcaca gctgggggct 5760
   ggggcgtccc tctcctctct ccccagtctc tagggctgcc tgactggagg ccttccaagg 5820
   gggtttcagt ctggacttat acagggaggc cagaagggct ccatgcactg gaatgcgggg 5880
   actctgcagg tggattaccc aggctcaggg ttaacagcta gcctcctagt tgagacacac 5940
   ctagagaagg gtttttggga gctgaataaa ctcagtcacc tggtttccca tctctaagcc 6000
   ccttaacctg cagcttcgtt taatgtagct cttgcatggg agtttctagg atgaaacact 6060
   cctccatggg atttgaacat atgaaagtta tttgtagggg aagagtcctg aggggcaaca 6120
   cacaagaacc aggtcccctc agcccacagc actgtctttt tgctgatcca cccccctctt 6180
   accttttatc aggatgtggc ctgttggtcc ttctgttgcc atcacagaga cacaggcatt 6240
   taaatattta acttatttat ttaacaaagt agaagggaat ccattgctag cttttctgtg 6300
   ttggtgtcta atatttgggt agggtggggg atccccaaca atcaggtccc ctgagatagc 6360
   tggtcattgg gctgatcatt gccagaatct tcttctcctg gggtctggcc ccccaaaatg 6420
   cctaacccag gaccttggaa attctactca tcccaaatga taattccaaa tgctgttacc 6480
   caaggttagg gtgttgaagg aaggtagagg gtggggcttc aggtctcaac ggcttcccta 6540
   accacccctc ttctcttggc ccagcctggt tccccccact tccactcccc tctactctct 6600
   ctaggactgg gctgatgaag gcactgccca aaatttcccc tacccccaac tttcccctac 6660
   ccccaacttt ccccaccagc tccacaaccc tgtttggagc tactgcagga ccagaagcac 6720
   aaagtgcggt ttcccaagcc tttgtccatc tcagccccca gagtatatct gtgcttgggg 6780
   aatctcacac agaaactcag gagcaccccc tgcctgagct aagggaggtc ttatctctca 6840
   gggggggttt aagtgccgtt tgcaataatg tcgtcttatt tatttagcgg ggtgaatatt 6900
   ttatactgta agtgagcaat cagagtataa tgtttatggt gacaaaatta aaggctttct 6960
   tatatgttta aaaaaa                                                    6976

<210> 706
<211> 123
<212> PRT
<213> Homo sapiens

<400> 706

<210> 707
<211> 150
<212> PRT
<213> Homo sapiens

<400> 707
Met Val Gln Arg Leu Trp Val Ser Arg Leu Leu Arg His Arg Lys Ala
1               5                   10                  15
Gln Leu Leu Leu Val Asn Leu Leu Thr Phe Gly Leu Glu Val Cys Leu
            20                  25                  30
Ala Ala Gly Ile Thr Tyr Val Pro Pro Leu Leu Leu Glu Val Gly Val
        35                  40                  45
Glu Glu Lys Phe Met Thr Met Val Leu Gly Glu Ser Leu His Pro Pro
    50                  55                  60
Ser Phe Leu Phe Gln Ile His Ala Thr Trp His Val Gly Gln Glu Tyr
65                  70                  75                  80
Leu Cys Pro Gly Ser Cys Leu Glu Gly Glu Val Val Cys Trp Glu Gly
                85                  90                  95
Ile Ala Gly Gln Glu Gly Asp Pro Gly Leu Arg Gly His Thr Lys Arg
            100                 105                 110
Lys Lys Arg Ile Pro Arg Thr Tyr Pro Ser His Leu Trp Ile Pro Gly
        115                 120                 125
Pro Ala Gln Ser Leu Ala His Arg Arg His Trp Arg Asn Ala Pro Asn
    130                 135                 140
Leu Trp Leu Ala Leu Leu
145                 150

<210> 708
<211> 371
<212> PRT
<213> Homo sapiens

<400> 708

<210> 709
<211> 141
<212> DNA
<213> Homo sapiens

<220>
<221> misc_feature
<222> 40, 41, 67, 73, 97, 104, 111, 125, 128
<223> n = A,T,C or G

<400> 709
tacggcgtgg tgcggagggc ggtaccccac aaataacacn nacaccccat cctatctgtg 60
tccacanata aantgactca ttcctctcct cgcatanccc actntcccct ngcgataccg 120
taacnaancc cttcccctt t                                            141

<210> 710
<211> 196
<212> DNA
<213> Homo sapiens

<220>
<221> misc_feature
<222> 2, 10, 12, 24, 65, 87, 91, 93, 106, 126, 131, 140, 153, 156,
160, 170, 185

<223> n = A,T,C or G

<400> 710
cnatccttcn cntacaccca tgangtccat gtcgcacgtc cacctcccct caaaacttgg 60
gtccncatcc acccgtcact ctccccntaa ncnataaccc cttttngcga atagacccca 120
ccttancaat nggtttttcn ttttttgtcc ctnggnccgn gcgattcaan aaattgaagg 180
cccanaaaaa cccccct 196

<210> 711
<211> 177
<212> DNA
<213> Homo sapiens

<220>
<221> misc_feature
<222> 1, 5, 8, 13, 27, 44, 57, 63, 97, 107, 110, 124, 142, 145,
150, 153, 165, 166
<223> n = A,T,C or G

<400> 711
ntacntcnct ccnaatgaaa ttcgaanctc ggttacccgg gggnattccg attaggngcg 60
tantctcgga tgtgcagtca caagtctttt gctaatnctt ataattntcn ctaccctttc 120
ttcnacaata ctgctatcct anttnttctn tcncctctct cccannttac taaccac 177

<210> 712
<211> 185
<212> DNA
<213> Homo sapiens

<220>
<221> misc_feature
<222> 6, 11, 12, 23, 31, 56, 83, 99, 101, 108, 135, 137, 157, 159,
163, 178
<223> n = A,T,C or G

<400> 712
aaacgnacca nngccaacga tangtgttgg ngttggttgc ggttgttcct cttatntgca 60
ctggttgtcc gtgtcgcacg ganggccacg tccctctgnc ntgagtanca catagcatcc 120
acgtttagtc gactntnccg ggcggccgct ctacccntnt atngattctt attaaaantc 180
ggatc 185

<210> 713
<211> 172
<212> DNA
<213> Homo sapiens

<220>
<221> misc_feature
<222> 1, 2, 13, 18, 32, 39, 48, 51, 54, 72, 77, 85, 86, 98, 101,
124, 132, 149, 154, 159, 164, 165
<223> n = A,T,C or G

<400> 713
nntggtcgcc tgngcgtnta ctctaaagga tntactatnc atatggantc naanacgact 60
cactacacgg cnctctncgg agccnnggtc agtgcctnct nggagacctt ctctggggca 120
ggangagcac tnggtatgtt cacgtatcnc ttcntaaana tacnnccctc cg 172

<210> 714
<211> 112
<212> DNA
<213> Homo sapiens

<220>
<221> misc_feature
<222> 1, 18, 28, 44, 54, 69, 75, 94
<223> n = A,T,C or G

<400> 714

nttgcgtgcc tggacgtnta ctctgcanga tctactactc atgngaattc taantacgga 60
ctcactatnc ggcancgcag gcgcagcagg gaangggtca cctcccagtc tc 112

<210> 715
<211> 326
<212> DNA
<213> Homo sapiens

<220>
<221> misc_feature
<222> 8, 16, 19, 25, 36, 56, 60, 64, 92, 111, 126, 141, 146, 151,
154, 159, 165, 167, 182, 183, 192, 203, 206, 211, 214, 222,
232, 249, 253, 261, 273, 285, 288, 289, 297, 302, 316, 318,
319
<223> n = A,T,C or G

<400> 715
   tactctanag gatctncgng tcatntggat tctatntcga ctcactctag ggctcnagcn 60
   gtcngccggg caagttattc ggatcgtcgg gntccgagct tcgcaattaa ntgtgccatc 120
   gttctncaac gttcctgact nggaancccc ngcngttcng atccncnggt acctagctcc 180
   anntcccccg tnctccttct ggngtntcat naangaggac cnccctcgat cncccttcct 240
   taatctgcnc acnctgaacg nccaatggac atngtgcgtt taatntanna ggcccgnttc 300
   gngtgccctt cccgtnannt cagctc 326

<210> 716
<211> 122
<212> DNA
<213> Homo sapiens

<220>
<221> misc_feature
<222> 1, 2, 14, 19, 37, 65, 66, 81, 86, 88, 97, 101, 108, 113, 120
<223> n = A,T,C or G

<400> 716
nntgcgtcgc ctgngcgtnt actctagatg atctgantag tcatatggat tctaatacga 60
ctcannatag ggctctagcg nggatncnga ttcgtcntcc ngattcantg acnccggtan 120
ca 122

<210> 717
<211> 203
<212> DNA
<213> Homo sapiens

<220>
<221> misc_feature
<222> 2, 72, 82, 96, 106, 121, 123, 131, 179, 185
<223> n = A,T,C or G

<400> 717
cntgcatgcc tgcaggtcga ctctagagga tctactagtc atatggatcg agcggccgcc 60
cgggcaggtg tnaatgataa anatgcatca tactanccta cagaanggag agataatgtt 120
ngntggacca ngttggtttt cttgcgtgtg tgtggcagta gtaagttatt agtttttana 180
atcantaccg ccctccgcac cac 203

<210> 718
<211> 168
<212> DNA
<213> Homo sapiens

<220>
<221> misc_feature
<222> 8, 13, 24, 48, 63, 64, 77, 82, 106, 109, 112, 122, 133, 137,
142, 149, 158
<223> n = A,T,C or G

<400> 718
ggcagganga tcncttgagc cccngaggtc gaggctacag tgagccanga gtgcactact 60

gtnncgccct ccgcatncac gngtggtccg atccccgggt accganctng anttcactgg 120
anttcttttt aancgtnttg antggtacna ccctcgantc cctggctg 168

<210> 719
<211> 210
<212> DNA
<213> Homo sapiens

<220>
<221> misc_feature
<222> 3, 9, 25, 30, 39, 50, 53, 59, 72, 75, 76, 84, 87, 99, 100,
103, 109, 115, 124, 127, 132, 143, 160, 161, 167, 171, 173,
178, 182, 184, 199, 204
<223> n = A,T,C or G

<400> 719
cancgtcgnc ataacacgta ttttntgatn aagattctna ctgacccatn aantctacnt 60
ctcaagctct tncanngtcc agtnaangga atgtgtatnn gtngggatnc cacanaaaaa 120
aganatntcg gncgcttcat tantcatcct tcttacccan ntctctngat ncncagtntg 180
ancntgaacg cacactacng gatntctcca 210

<210> 720
<211> 131
<212> DNA
<213> Homo sapiens

<220>
<221> misc_feature
<222> 63, 65, 119
<223> n = A,T,C or G

<400> 720
tccatcctaa tacgactcac tatagggctg ccaacctgcc atccactact gaggaagacc 60
cgnanactta ggggctcact gcgagccacc ggccacaggt cgtatagggc aaagcacgng 120
gaagcacccc t 131

<210> 721
<211> 121
<212> DNA
<213> Homo sapiens

<220>
<221> misc_feature
<222> 31, 35, 37, 61, 70, 73, 91, 92, 96, 101, 108, 116
<223> n = A,T,C or G

<400> 721
tccatcctaa tacgactcac tatagggccg ntgantnctg gcgaaaggct tacaattaag 60
naggaaaaan ganccaacaa ctaaaaaaaa nncggncgtg ncagcttnga tgactngtcc 120
a 121

<210> 722
<211> 246
<212> DNA
<213> Homo sapiens

<220>
<221> misc_feature
<222> 2, 38, 58, 62, 66, 77, 94, 97, 100, 125, 133, 137, 142, 164,
186, 198, 206, 232, 237
<223> n = A,T,C or G

<400> 722
anctggagtc gcgcgctgca gtcacattgt ggatccanaa aatcggcaca agctctcntg 60
gnttcntcga tatgaanaac actaatccca tgtngtntgn gtctccgtga ttcatccctc 120
gcacnggtcc ccntccnaac cnttgcatag gtgttatgtt gtantctccc cagtgcacaa 180
agattnacac tctctcantg tctganatat gcacgagttc attgtcctgt cnccgtnaac 240
atcaag 246

<210> 723
<211> 160
<212> DNA
<213> Homo sapiens

<220>
<221> misc_feature
<222> 17, 26, 28, 30, 44, 51, 57, 58, 79, 87, 89, 96, 105, 111,
122, 126, 128, 136, 142, 145
<223> n = A,T,C or G

<400> 723
cctccggaaa atccaantag agtaantncn ctctaatccg gggnaattgg nggggtnnat 60
acgtcctcct ccccccagnt aggattnana aaaggnctcc cagancaaaa nctccaaagt 120
gnatcnanta gccgtncccg anatncaacg cccctacgtc 160

<210> 724
<211> 156
<212> DNA
<213> Homo sapiens

<220>
<221> misc_feature
<222> 2, 4, 7, 33, 109, 110, 115, 138, 139, 148
<223> n = A,T,C or G

<400> 724
tnanccnata tacaccaaat tctgattcta aantcccacc caagggaaaa aagttgagaa 60
gagcctttcc acttttctac taataaaaaa atgcaccagc ccctaccann agtgnggaaa 120
acctccttag gcccttgnnt ggaacaancg aaaatc 156

<210> 725
<211> 347
<212> DNA
<213> Homo sapiens

<220>
<221> misc_feature
<222> 4, 9, 13, 106, 131, 163, 172, 269, 282, 291, 296, 319, 324,
325, 332, 336
<223> n = A,T,C or G

<400> 725
aganggttnt atncatgctg tactcgcgcg cctgcagtcg acactagtgg atccaaagaa 60
ttcggcacga gagacggtgc gcgatggacc gagggcccca gccggngagg cgccgccgcc 120
gagcccgcgg ncagacgccc catcagtagc gtccgcaccg ggnagccgcg gntctcgccc 180
gagccgtggg cgcgcccgag gggcgggctc gcctcccgcc gtccctcgca gctctgccgg 240
gcccgagccc gcgccgtcgc cgccgccgnc ttgccgctcg gnccgcgcgg nccggnaaac 300
gcggtcgagg tctggatgng gcanngcccg cncctntcgc tgagcct 347

<210> 726
<211> 162
<212> DNA
<213> Homo sapiens

<220>
<221> misc_feature
<222> 21, 28, 53, 72, 76, 85, 88, 104, 112, 115, 121, 123, 126,
131, 134, 136, 144, 155
<223> n = A,T,C or G

<400> 726
ttgggtgggt tgggtggggg naaatttncc catttgggtg ggtttggggg ggnaaatact 60
tcccgccttt tnggtnccca aaganacnaa gggggagtcc cttnatagag gnagngcgat 120
ncntcncaac nacntngact ttgnccatgg ggagnaaggt gg 162

<210> 727

<211> 120
<212> DNA
<213> Homo sapiens

<220>
<221> misc_feature
<222> 33, 54, 67, 72, 75, 77, 101, 106, 113, 117
<223> n = A,T,C or G

<400> 727
gtgtgggtgg ggaattccat tgtggttggg ggnaaatctc cgcttgtcca aagnacaggg 60
ggggtcnctt anagngnagg gggttcctcc ccaccacttg ncttgnccat tgngagnaag 120


<210> 728
<211> 130
<212> DNA
<213> Homo sapiens

<220>
<221> misc_feature
<222> 17, 91, 98, 102, 104, 116
<223> n = A,T,C or G

<400> 728
gacccactgc agcgttnaac ttagcttgga ccgagctcgg atccctagtc cgtgtggtgg 60
aattccatgt gtcgagagag gggcaaatac nctccaanac ancnccctca tgctcnacac 120
atattcgcat 130

<210> 729
<211> 182
<212> DNA
<213> Homo sapiens

<220>
<221> misc_feature
<222> 2, 10, 27, 45, 46, 54, 83, 104, 106, 110, 122, 123, 132,
135, 141, 142, 150, 158, 162, 166, 177
<223> n = A,T,C or G

<400> 729
cngactgctn gcgtttaaac ttaagcnagg taccgaacgg ggatnnacga ctantgatcg 60
gctggctgct tccagtcgat tanatttgtg aaaaagctga accncngccn gttaaggggg 120
annatgcaaa anatncatcc nnctgccccn taaactgntc tntccnaggg aaaaaangga 180
ag 182

<210> 730
<211> 678
<212> DNA
<213> Homo sapiens

<220>
<221> misc_feature
<222> 6, 23, 58, 108, 561, 595, 652, 669, 674
<223> n = A,T,C or G

<400> 730
cactcncact ccggacctag gcncttcacc actgctctct tcctcctcct cctcctcntc 60
ctcggggctg ggggaccttc cccagtgacc atctcacttt ggctgaancc cactcggggc 120
agcctgagtt tggggctctt ggccttctca ccctcctcgg ccccctcctt ggcccgcacc 180
aggccaaacc ggggcagccg taccttgagc ttgtgtccgg cctctccctc cccctctgcc 240
acctggtact cggcatggtt gcccccggga tggcgagagc tccacgtcgg gcagtgagaa 300
gcagaaagta cgctcggccc ctgggggctg ctcctcagca ccctcgcccc ccaccctagc 360
tctggccccc agtgtgggca acttcagcct cagcccaccc tcgcctgtgg ccgcctcgcc 420
cgcctgtgcc tctcggctta gccccacgtc caactcaagc tggggcactg tcacggtggg 480
catcttaaag acaccctcac ccaccagcag ctcaccacct gcaacctggg ctccaggcaa 540
aaaaagggtc acctggggca nctgaaccct gtacctgctg tgccctctgc tgaanggaat 600
gttatctgaa cctgctgccc tgggggtact gccttcccaa aaccgggtca antccacctg 660

ttggaaggna aatncccc 678

<210> 731
<211> 135
<212> DNA
<213> Homo sapiens

<220>
<221> misc_feature
<222> 54, 114, 115
<223> n = A,T,C or G

<400> 731
gagatccgac gtcaccccct tccggcggcc caagacgctg caactcccga ggcngcccaa 60
atatctttgg aagagcgctc ccagcccaac acaatggaat tccaccacac tggnntagtg 120
gatccgagct aagcc 135

<210> 732
<211> 660
<212> DNA
<213> Homo sapiens

<220>
<221> misc_feature
<222> 16, 69, 71, 557, 621, 653
<223> n = A,T,C or G

<400> 732
gcttggtacc gagctnggat ccctagtaac ggccgccagt gtgctggaat tcggctttct 60
tcaatcagnt nacgagctgc atggtctgct aacattgtca taattgctgg catagattac 120
tgaaaataaa gaaaaaaaat tgaagctgcc tatcaagttt tggtattatc aaaaacttcc 180
tacaagttat tttacttcaa ccatgttatt acaaatattt taatgaatac tttagagact 240
ttaattacaa aaaactgaga tagtaaaagc aagtaataaa agctgaaatt acttagctat 300
ttgataatta cataaattat tatggtccat tcaacttttc tagtgtttag tttatacacc 360
aggaagactt tcctattcta ctaacattta taaagtatgc taacctatta tttaaacgca 420
tccactatta ggattttatg gcctaaaacg tgatacagtt cagtatcttg atgtcaaaac 480
tttttaagca agtagggatt aagttcaagt gaatgtgatt ttctttcttc ccagtagggt 540
cttctgaata actcagnaaa gctcacttcc attatcttac tttataaaaa aatgctataa 600
gacagaatgg gccgacgtgg nggctccacc tgtatccacc tttggaggcg agnggcgaat 660

<210> 733
<211> 836
<212> DNA
<213> Homo sapiens

<220>
<221> misc_feature
<222> 728, 743, 746, 766, 819, 822, 833
<223> n = A,T,C or G

<400> 733
aattaatgac tttttttccg ccctgccaag ctagtttgtc taaatataat gtaaagaaat 60
tagctactca ttttctggtc cacgaaggtt cctaaaatgg gaagaagtgg agatctgacc 120
ttgttagttc taaatacact aaactgggag tgccatggat ggctttcagg atgtcctgaa 180
tcctctataa ttgtatacaa aatcgtgagt ttttaaaaac tgggttagag ctattggttc 240
ctcagagtct caggcatctt agacccccaa aaaggttaag gactactgac ttaaccaatt 300
aggtttgagt ggcattggct ttgaagaaaa gcagaggaaa gatatatttt ataattctgg 360
gcaacaaaaa agtggatgtg tgccagcatc ttagagtaga atcctcttaa aaggatagca 420
ctgcatatga actagtaggt tttaaccagt gcatatttag gcgaagtagc tcatttttct 480
gttagaattc ttttttattt gggaatgggc aagcttttac agcttttacc ttgccaatga 540
atacctggaa tttaaaaaat cttgttaggc atattgccca taaagttttt tttcctagat 600
catatattca gtaaatatgt ttgtagcttt atttcaatcc cccaattcat tgagggttga 660
aacaatttga atggtttgag tgtagaagct aagttatttc tgtagaggct aagggcattt 720
ataccaanat atgttagact tgnggntcct gttaaccatg ctgtanacaa taggaattac 780
tgtatatcca cattttaatt ttaacatctt ctgctttgnt gntggtttga gangga 836

<210> 734

<211> 694
<212> DNA
<213> Homo sapiens

<220>
<221> misc_feature
<222> 1, 5, 12, 23
<223> n = A,T,C or G

<400> 734
nagtnctatt tncactaaac tgngagtgcc ttggatggct ttcaggatgt cctgaatcct 60
ctataattgt atacaaaatc gtgagttttt aaaaactggg ttagagctat tggttcctca 120
gagtctcagg catcttagac ccccaaaaag gttaaggact actgacttaa ccaattaggt 180
ttgagtggca ttggctttga agaaaagcag aggaaagata tattttataa ttctgggcaa 240
caaaaaagtg gatgtgtgcc agcatcttag agtagaatcc tcttaaaagg atagcactgc 300
atatgaacta gtaggtttta accagtgcat atttaggcga agtagctcat ttttctgtta 360
gaattctttt ttatttggga atgggcaagc ttttacagct tttaccttgc caatgaatac 420
ctggaattta aaaaatcttg ttaggcatat tgcccataaa gttttttttc ctagatcata 480
tattcagtaa atatgtttgt agctttattt caatccccca attcattgag ggttgaaaca 540
atttgaatgg tttgagtgta gaagctaagt tatttctgta gaggctaagg gcatttatac 600
caagatatgt tagacttgtg gttcctgtta accattgctg tagacaatag gaattactgt 660
atatccacat tttaattttt aacatcattc tgtc 694

<210> 735
<211> 126
<212> DNA
<213> Homo sapiens

<220>
<221> misc_feature
<222> 1, 3, 11, 111
<223> n = A,T,C or G

<400> 735
ncnttgaaac nggttgacca gacttcaggc ctgtgcgctc aatcgtggag aatctcgtgc 60
cgaattcggc acgagtctct ctctctctct ctctctctct ctctctctct ntctctctct 120
ctctct 126

<210> 736
<211> 165
<212> DNA
<213> Homo sapiens

<220>
<221> misc_feature
<222> 21
<223> n = A,T,C or G

<400> 736
cagaagcctt taaaccggtt ngaccagact tcaggcctgt gcgctcaatc gtggagaatc 60
tcgtgccgaa ttcggcacga gtctctctct ctctctctct ctctctctct ctctctctct 120
ctctctctct ctctctctct ctctctctct ctctctctct ctctc 165

<210> 737
<211> 125
<212> DNA
<213> Homo sapiens

<220>
<221> misc_feature
<222> 3, 116
<223> n = A,T,C or G

<400> 737
ggnagcccct ttaaccgttt gtccagactt caggcctgtg cgctcaatcg tggagaatct 60
cgtgccgaat tcggcacgag tctctctctc tctctctctc tctctctctc tctctntctc 120
tctct 125

<210> 738
<211> 137
<212> DNA
<213> Homo sapiens

<220>
<221> misc_feature
<222> 5, 7, 13
<223> n = A,T,C or G

<400> 738
ggagncnctt gancaggatg accgacttca ggcctgtgcg ctcaatcgtg gagaatctcg 60
tgccgaattc ggcacgagtc tctctctctc tctctctctc tctctctctc tctctctctc 120
tctctctctc tctctct 137

<210> 739
<211> 970
<212> DNA
<213> Homo sapiens

<220>
<221> misc_feature
<222> 90, 101, 303, 479, 497, 632, 641, 698, 730, 765, 770, 778,
801, 833, 848, 850, 872, 877, 891, 904, 921, 929, 931, 937,
960, 965
<223> n = A,T,C or G

<400> 739
aggcctattt aggtgacact atagaacaag tttgtacaaa aaagcaggct ggtaccggtc 60
cggaattcgc ggccgcgtcg acggcccttn gtgccactag ntctttcatt cttccccccc 120
atcaatcagt gaacttttta gcctactcaa agctttgctc caatgcatag gatttatgat 180
tgtggggatt tccagataat ataaatattc aacatgaata ttttaaatta aggcatgaga 240
catttttcct aactgagcat agccatgaac ctctcacgtc tgttcctctg tgtcagtttg 300
tancactgaa tacagcagcc ctcctaaaag tccaggcagt gcacaggtct tgacatgatg 360
aagtgacgtg ttgctatggt gattttgcag ctggccaaat agtcactggt tgattttacc 420
cagcaggaga tttttgcaaa aatttcctgg gtgagagtga aatcaaactc ctattttgnt 480
tctcctctgc aagctgnagt taagatggat taatgagtac ttttagatta attaactctg 540
aagagaaaat gggagaaaag tgaggaaggt tgttggcaga agtcattgct ggaatccttc 600
tgaagggagt actgacttca cttgcaaaga cnagagacta naagacaatg aagttaaact 660
tggcctgtct ctcatatgat agatgctgag agtcaggntc agggaaattt aattctgtca 720
tacgcatatn ggattatgtg gtcatggatt tgttggcact aaccngcctn taatcagnat 780
aagaaaagtg ttttggtaga naaagaaaat tatggcccag aaaaacctgg aanacttgga 840
aaaaatgntn gggggccttg ggtggtggtc tnaaaanacc ccctggggat ntttaaacca 900
aaantgaaga agggaaaaat ntttccccnt ntttttnttt tttgccccct tgggattggn 960
ttttntttcc 970

<210> 740
<211> 739
<212> DNA
<213> Homo sapiens

<220>
<221> misc_feature
<222> 2, 7, 87, 262, 466, 473, 530, 610, 640, 669, 690, 732
<223> n = A,T,C or G

<400> 740
gntgtcnaaa aagcaggctg gtaccggtcc ggaattcgcg gccgcgtcga cggcccttgg 60
tgccactagt tctttcattc ttccccncca tcaatcagtg aactttttag cctactcaaa 120
gctttgctcc aatgcatagg atttatgatt gtggggattt ccagataata taaatattca 180
acatgaatat tttaaattaa ggcatgagac atttttccta actgagcata gccatgaacc 240
tctcacgtct gttcctctgt gncagtttgt agcactgaat acagcagccc tcctaaaagt 300
ccaggcagtg cacaggtctt gacatgatga agtgacgtgt tgctatggtg attttgcagc 360
tggccaaata gtcactggtt gattttaccc agcaggagat ttttgcaaaa atttcctggg 420
tgagagtgaa atcaaactcc tattttgttt ctcctctgca agctgnagtt aanatggatt 480
aatgagtact tttagattaa ttaactctga agagaaaatg ggagaaaagn gaggaaggtt 540
gttggcagaa gtcattgctg gaatccttct gaagggagta ctgacttcac ttgcaaagac 600
aagagactan aagacaatga agttaaactt ggcctgtctn tcatatgata gatgcttgag 660

agtacaggnt cagggaaatt ttaattctgn catacgcata ttggattatg tgggtcatgg 720
ctttgtttgg cncctaacc 739

<210> 741
<211> 1171
<212> DNA
<213> Homo sapiens

<220>
<221> misc_feature
<222> 7, 86, 87, 590, 604, 620, 624, 628, 646, 657, 668, 677, 696,
697, 704, 724, 743, 764, 771, 778, 781, 804, 830, 845, 849,
854, 874, 876, 922, 934, 948, 949, 959, 971, 972, 976, 987,
990, 993, 997, 1032, 1034, 1035, 1044, 1062, 1075
<223> n = A,T,C or G

<221> misc_feature
<222> 1109, 1114, 1118, 1124, 1135, 1136, 1155, 1159, 1164
<223> n = A,T,C or G

<400> 741
gccttgnggt gacactatag aacatgtttg tacaaaaaag caggctggta ccggtccgga 60
attcgcggcc gcgtcgacgg cccttnntgc cactagttct ttcattcttc ccccccatca 120
atcagtgaac tttttagcct actcaaagct ttgctccaat gcataggatt tatgattgtg 180
gggatttcca gataatataa atattcaaca tgaatatttt aaattaaggc atgagacatt 240
tttcctaact gagcatagcc atgaacctct cacgtctgtt cctctgtgtc agtttgtagc 300
actgaataca gcagccctcc taaaagtcca ggcagtgcac aggtcttgac atgatgaagt 360
gacgtgttgc tatggtgatt ttgcagctgg ccaaatagtc actggttgat tttacccagc 420
aggagatttt tgcaaaaatt tcctgggtga gagtgaaatc aaactcctat tttgtttctc 480
ctctgcaagc tgtagttaag aagggattaa tggagtactt tttaagaatt aaattaacct 540
cttgaaagaa gaaaaaatgg gggaagaaaa aaagtggaag ggaaaagggn ttggttttgg 600
gccnaaaaaa aagttccaan tttnggcntt ggggaaaaat tccccntttt ccttggnaaa 660
aggggggnaa ggttaancct tgggaacctt tttccnncct tttnggccca aaaggggaac 720
ccanggggaa agaaccttta ggnaaaggaa acccatttgg gaangggttt naaaaccntt 780
ngggcccccg ggccctcctc caanaaggga aaaaaaaagg cctggaaaan gtaccagggt 840
ttcangggna aaanttaaaa ttcttggcca atancnccat aattgggaat tatggggggg 900
ccatgggctt ttggtttggg cncttaaccc cgcnttttaa attcaaanna aaaaaaagng 960
gtttggaaaa nnaaanaaaa aaaattnaan ggncccnaaa aaaaaccctg gaaaaccttt 1020
ggaaaaaaat tngnnggggg gccntttggt tgggggggtt tnaaaaaacc ccctnggggg 1080
ttttttaagc ccaaaagggg gggaggggna aaanggtncc cttntttttt ttttnngccc 1140
cccttgggga atggnttant tcanggggcc c 1171

<210> 742
<211> 739
<212> DNA
<213> Homo sapiens

<220>
<221> misc_feature
<222> 2, 7, 87, 262, 466, 473, 530, 610, 640, 669, 690, 732
<223> n = A,T,C or G

<400> 742
   gntgtcnaaa aagcaggctg gtaccggtcc ggaattcgcg gccgcgtcga cggcccttgg 60
   tgccactagt tctttcattc ttccccncca tcaatcagtg aactttttag cctactcaaa 120
   gctttgctcc aatgcatagg atttatgatt gtggggattt ccagataata taaatattca 180
   acatgaatat tttaaattaa ggcatgagac atttttccta actgagcata gccatgaacc 240
   tctcacgtct gttcctctgt gncagtttgt agcactgaat acagcagccc tcctaaaagt 300
   ccaggcagtg cacaggtctt gacatgatga agtgacgtgt tgctatggtg attttgcagc 360
   tggccaaata gtcactggtt gattttaccc agcaggagat ttttgcaaaa atttcctggg 420
   tgagagtgaa atcaaactcc tattttgttt ctcctctgca agctgnagtt aanatggatt 480
   aatgagtact ttttagatta ttaactctga agagaaaatg ggagaaaagn gaggaaggtt 540
   gttggcagaa gtcattgctg gaatccttct gaagggagta ctgacttcac ttgcaaagac 600
   aagagactan aagacaatga agttaaactt ggcctgtctn tcatatgata gatgcttgag 660
   agtacaggnt cagggaaatt ttaattctgn catacgcata ttggattatg tgggtcatgg 720
   ctttgtttgg cncctaacc 739

<210> 743

<211> 610
<212> DNA
<213> Homo sapiens

<220>
<221> misc_feature
<222> 487, 536, 568, 570, 594, 605
<223> n = A,T,C or G

<400> 743
ctgtccttat ttctttagca aaaatttccc aagagaagaa ttgctgggat aatgcacatt 60
taaatttttg atagacattc ccaaatatta tacctgtttt tgagaccttt aattcctgtt 120
gtcaaattgc cctatatatg gagtaataaa cacgatttaa agaaatgagg actaaaaaaa 180
gattatatat aacccaacat aaaggcaacc tcttaggcgt tgacagaaac tgacaacttt 240
ttatctgtgg gtgcgatcca ttataagtaa cctgagcacc ttattttttc tttttaaact 300
ctaggtagga tacccgaggt ccacaaattt ttcataagaa atattttttc tctgccctat 360
gagattttaa aaaatattat actgcttcaa ttgcatcaaa agaaatggac cctaatatct 420
atgatgaagg atttggagtt agaagacctg agtttcaatt ttggcatggc tgtttgtcta 480
gctctgngat cttggacagg tcaattgact tggcttaatc ttctcatcca tttagnggag 540
acagcaccac tattcacagg actattgncn gaattaccag acaatagcat aggngaaaat 600
ataangcctt 610

<210> 744
<211> 127
<212> DNA
<213> Homo sapiens

<220>
<221> misc_feature
<222> 3, 38, 80, 115, 117
<223> n = A,T,C or G

<400> 744
ttnacctccc tggaccgggc cccccttccc cgggcggntc ccccgggctg caggaattct 60
gcacgaggga gagagagttn gagagagaga gagagagaga gagagagaga gagananaga 120
gagagag 127

<210> 745
<211> 458
<212> DNA
<213> Homo sapiens

<220>
<221> misc_feature
<222> 365, 367, 369, 385, 427, 428, 442, 452
<223> n = A,T,C or G

<400> 745
gatatcccgg gattcgcggc cgcgtcgacg tggcctctag tttgtcctgg tccaaagcag 60
ggaagctggg ctacgtcctg cccaggtcag ccttaggtta agggctgcct gggggaggga 120
acttcctggg ccttcgggtc tctgtgcact ggggtggctc ctgtggccca gaatgccctg 180
gagaagggtc ctactggaag cgaaggtgca gggcagcagg gcctgaggcg caggagctgg 240
tggaggctcc cagcacaggt cgccgcccca gtcacatcac tgctgatggt ggggggactt 300
ggggagtttc ccccgagaat gggaggtctc acagtccccg tgctgcaatg ctgtcggtgc 360
actgngncng caatgtgctc atggncactt gctttttctc tgtggccccg gccgatttat 420
ccagcanngc acccctcttc tnctctccgg anaaagcc 458

<210> 746
<211> 893
<212> DNA
<213> Homo sapiens

<220>
<221> misc_feature
<222> 123, 124, 181, 456, 586, 626, 698, 705, 743, 751, 764, 774,
790, 806, 814, 815, 824, 836, 837, 841, 846, 851, 854, 859,
861, 876, 882
<223> n = A,T,C or G

<400> 746
aagcaggctg gtaccggtcc ggaattcgcg gccgcgtcga cgtggggagt tagctctctg 60
gaccccgtca tagagtaagt catcgataga gcatttgctt gatggggact tccagaaggc 120
canngaaagt cctgccgact tcctggggaa gcccatccgc acgtggggtg agggtcccca 180
natggaagca gctgtgtatg cagggagggg gcagaggctg ctgccaatgg gcatgtccct 240
tacctgaaag ggccacctct ccaggtgaca tgtcctgggg gagccggggc cgtctgctcc 300
ggccagaggc gctcagctca ggccacacca ggcagggcac ctcccaacct ggacaggtgg 360
ggaccaaggt ggccttggac aaaactctct gtgtttgcca agcacccaat cggacacaga 420
gagtcaacca caccccagtc acatggtgtc cacacngcag gggtcaagga ggcccggccc 480
ctccccctca gacgtccctg ggcctctggg agtcagcaag gacgaggacg gcattgccct 540
tcgagacagg aagggagtga cctcctcccg gcggcatcca ggctcngctt ctccggagag 600
gagagggggc tacttgctgg ataaancggc cggggccaca gagaaaaagc aaggtgacca 660
tgagcacctt gcaaacacag tgcacccacc agcatttnag caccngggac tgtgaagacc 720
tcccatttct tcggggggaa acncgcccaa ngttcccccc accntcacta gtgnattgtg 780
acctgggggn cgggccgacc cctgtngctt gggnnagccc tccncccagg tttctnnggc 840
ngcccnttaa nggnccctng nttggcccct tggccncctt tncgcttttc cca 893

<210> 747
<211> 738
<212> DNA
<213> Homo sapiens

<220>
<221> misc_feature
<222> 28, 45, 82, 206, 297, 308, 366, 404, 423, 484, 568, 588,
650, 653, 655, 661, 691, 708, 717, 726
<223> n = A,T,C or G

<400> 747
   gatatcccgg gaattcgcgg ccgcgtcnac gaagcacaga cctgngccct gctctcatgg 60
   ggcagactgc catttgtcat tnattactga aggaaaggga tcctcagttt gcttgtggac 120
   atttcaaatt tgaggtgaga gttggataag taagaataaa gctgctcttc aaagagatga 180
   atatagaaaa agaaacaaga tacagncttg gcagtaaggc tgggaggaag gggaaaaggt 240
   aataaagaat gaaagagtga gaaatgtgag caggagctga acacagaaaa gttcagngac 300
   agaagcanaa ggagggaaga agggaggagg gtccctttca cagaggctca cgaggatgct 360
   ttatgngtgc catgcagtcc atgttcagga tgtctgcttc ttanctctct acttttctaa 420
   tanaaatttg gatacttact gatcctacat atgtaacagg gagagaaggt gaatttcaaa 480
   gcantaaatt gaaaaattgt tcacaatttc attttttaaa aaaagggagc taacagaaga 540
   agaggttaat gtggtaatta taggatgnct cttgcgacac atgaatgnat ctggtatcat 600
   ctgagtggga ggggagctgt cttcctgacc caaaaggatc ctttcgttan ccngnactta 660
   ngtcccaaaa cctcaccacc ttggagaaat natttccttt tgggggtntc attaaancct 720
   tttggncccc gcaaaagc 738

<210> 748
<211> 647
<212> DNA
<213> Homo sapiens

<220>
<221> misc_feature
<222> 3, 59, 607
<223> n = A,T,C or G

<400> 748
ctntgtggcg gtggctgtct catttgggtg gactttttgg gtcgtaggaa cctggtatng 60
aggtcgagag taagacgggc tattagtagt cgcatcggag ttatttgtga aaacctggtt 120
agggcctctg tctccgctgc gctcgcctaa attggtatgg ctcgacttgg aaacacggtt 180
ctaacacgcg ttgttagcgc ccttgctagc atgtgaagga cactggccct accaagaaag 240
attcgagtcg ctccttccgg tatcgttcac ggaggcgata tttactcttc ttactacggt 300
tacttcgaga ttgtctgtga agtttaagac tactaaaaag agtattaagc ctatcgggaa 360
ttagctagat cgacacgcta aaaccaaggg caatcggcgg aaatatagag gcaccaataa 420
tagggcctac agaaggcccg agggttagac tcacgtttaa taccggccac gggagaaata 480
aaaagataaa gtatacatcg tttagcggtc ctcggaagcc ttcggcttta atgccaagga 540
gtcggaagca tcgtcggcga gtaataaact ccatcgcgcc gagactatct acgacgccct 600
ccttaanatc cgtaaattac tcccggaaag agtatttagg cggctct 647

<210> 749

<211> 642
<212> DNA
<213> Homo sapiens

<220>
<221> misc_feature
<222> 3, 15, 629
<223> n = A,T,C or G

<400> 749
ctntgtggcg gtggntgtct catttgggtg gactttttgg gtcgtaggaa cctggtatgc 60
aggtccgcgg agcgtgggct ctcgtcgtgg atgttggggg ttggtgtggt gccggttgtt 120
tttggttctg ttgagcgtag tgtgtttgaa ggttagcgtt cgtgtcttgc ttgtggtttg 180
gtgtttaggg cgggtgggga ggttgttgtg tagctgttgt atgtcatatt gttggtgttg 240
ctgccctgtg ctgtttgtcc ttggttattg tggttgttac cccgcctgtg tggaagtgtt 300
gtggcagggc gggaatttaa gtgggagagt tgtgggaccc gtggttgttg ttacgttgct 360
gcttttgtcg tgggcggtgg cggcgcgtct gataattaga attggatacg gagtgtataa 420
tacttctagt aaatggggac ctagtgcttg acttcccgga atagggatct atgcgaagtc 480
cttaggatag tctttgataa gtttaacgcc cacgacccta aaattataca cgattagacg 540
cataacgact cctccaggaa agataaagaa tctcacatat agaacgggac cccatacacg 600
tcggatagga aacaagagaa ctaattttng ttaaaaagac tt 642

<210> 750
<211> 639
<212> DNA
<213> Homo sapiens

<220>
<221> misc_feature
<222> 57
<223> n = A,T,C or G

<400> 750
tttgtggcgg tggtgtctca tttgggtgga tttttgggtc gtaggtaacc tggtatngag 60
gtatagatgc cgattggtcc cgacgagcgt cacgataaat tcggtagttt cgcccttttt 120
agaaggcgct agtactcgga acttcacttc atctcggtag tttactttgg cgtatatagc 180
cttctccctc gaagactagc cgtcacattc gttccctagg aatcgtttct gcccctaaga 240
atccgagagc gagatcccga aactagagga accttagaag agtcgtattt ccacaaggac 300
cccacagtca ttccgggaaa atccctagga ccatacggtt aggattcccc cggaacccgg 360
agcaaagctc atgatttccc acaccgcgag agcgcctata accctatccc atttcttcgg 420
gttatcgagg atattacgat caagccgaga gaaccgctag aaccgctttc ttcgctttct 480
cacggaacct ataagtagaa agagaaactc aggtcttaag ggggcgcttc ggctaacgaa 540
acttctactt acgaagagag tatctagaca ttaagtcata aaaatccact acgcacctcg 600
tgtacgatat catcgggagc ggttcataga cggtgtccg 639

<210> 751
<211> 637
<212> DNA
<213> Homo sapiens

<220>
<221> misc_feature
<222> 13, 48, 59, 97, 113, 123, 147, 171, 183, 186, 202, 216, 225,
237, 258, 261, 269, 274, 283, 297, 334, 344, 348, 353, 358,
360, 366, 379, 383, 389, 394, 400, 425, 444, 452, 527, 530,
556, 564, 581, 612, 636, 637
<223> n = A,T,C or G

<400> 751
cttttgtggc ggnggtgtct catttgggtg gatttttggg tcgtaggnaa cctggtatng 60
aggcagctct gagccccccc cccccccccc ccccccnccc ccccccccta ggnggttggg 120
aanacggtgg atacctaaat cgagtgngtt cattaaaagt agttgattac nccctaaaat 180
aanaanaggg cttcgtcggg anaaatcggt aagganaagt ctttntggca tcataanaat 240
actggctcgg gtcctaanat ntttaaggng gtcnccgagg gtnttcatac cgataanaaa 300
cgttttccta tcggcaacgg gcttacctga gggnggactt ctcncggngc ggngattnan 360
acgaanacgt agaggattnc cgntacttnt tganatcacn cgtatcatac ttgtaagcat 420
aattntcctg aaaagtgtta taanaatacg cncgcatatt cgctttttcg tcctagggat 480
gcttaaatgg cgatactgct atagcgggtg agcgttggtt ctcgagnaan aaagcgtgtc 540

ctaatgcgtc taaggnttta aggncgttgg tttaaaaata nccttagaaa cctcgaggcg 600
gatactggtt tntttttaac gaaacaaagc accccnn 637

<210> 752
<211> 644
<212> DNA
<213> Homo sapiens

<220>
<221> misc_feature
<222> 2, 579
<223> n = A,T,C or G

<400> 752
tntgtggcgg tggtgctcat ttgggtggat ttttgggtcg taggaacctg gtatgaggtc 60
ttgcgagttg ttggtgtgtc ctgtcgttcg gtggttccct tttgagttga gtttgtcctt 120
tgaggttgtt agctgctgtt cgtttgtgtt cgtgtagtgc tttgggttga gagggttatg 180
gtggtggtta cggtgtattg tcgcccgtgg tcgcggggtt ggggtggtcg tcggttttgt 240
ggttcatagt agtcttctgc gttcggtggt gcgggtttgg gtgagtagtt tcgttcttgg 300
atgtcccatt gacccgccat aatctaagta agggttagta gaaacctctc cccgatagac 360
acaaccgtcg tccactaaag acctcgcctc tgatttttaa aaggacccga aaaacatccc 420
ttcaacggaa aaaacggaaa aaaagtcagc gaattcaaag aagccacggg agagaaaaaa 480
gaactaaagt tagtccgtca ttatatgtct cctcggagga ggaagcggcg gtggcggaaa 540
atgaggcggt aagaaagacg acctctatcg gcggcttang ccctaaaagg gcgatacctt 600
acgggatgat aaggacccta ggacgcctcc ttctcggatc gtcc 644

<210> 753
<211> 635
<212> DNA
<213> Homo sapiens

<220>
<221> misc_feature
<222> 216, 336, 428, 493, 499, 513, 552, 579, 585, 588, 591, 596,
621, 633
<223> n = A,T,C or G

<400> 753
ctttgtggcg gtggtgctca tttgggtgga tttttgggtc gtaggaacct ggtatgaggg 60
aatcagctcg accccccccc ccccccccct ccgaagcaga gcccaaccca aagtccaccg 120
actacccgag taaactctcg gagggtagaa taagaaggag taggtcctag ccaatagaag 180
tagttccgag ccgttaggac agcggacgga acattnaaga aagagcctat attagggagg 240
aagtaacgtt cctctttcgg agctctttaa ggggtagtcc cagaacaagg gaagaggacc 300
cgtcggctat tgcccgtcga tacgggctct cacggngagc ctaggttcga ggatagggcc 360
gctcgtaaaa ttatacggtt tccgagaaac gcttccgtag accgggtcct aaatcgtccg 420
gagtattngg agagggatcc ttcggaccct agggacagag agaggagaac ggaggttaca 480
ggaggagaac gtntcctcnc tagttttctt tangtcgaaa aatttcttac cgatagggtt 540
cctagggtcg gngaatttac ggttcgaaaa acggtagtnc ctaanggntg ntattngggg 600
tagtatcggg tcgtttacaa ntcgtccgtc ttntg 635

<210> 754
<211> 721
<212> DNA
<213> Homo sapiens

<220>
<221> misc_feature
<222> 9, 13, 46, 63, 105, 132, 300, 551, 576, 590, 614, 621, 630,
639, 643, 644, 650, 673, 676, 691, 698
<223> n = A,T,C or G

<400> 754
accggattng ttnctgagcg cgtgactgct aataaaaaag atggantgcc atcttttttt 60
ttnccttgct ttatatatcc agcagcaaaa caaaattgtt ctgcngggct ataaaatttg 120
gcttgtgagt cntgtacaca actcaggagt gtgacacagc taccagcttt cctcctaact 180
ctcaagggaa gaaaattcaa gttctgtcta ggctcactct gtaaagtggg aaacttgctg 240
gttttgtagg ctttttttcc ccttctttcc ctctctcagc ttctccctgc ttctcagaan 300
atggagttgt gatgcctgca acttaccaaa tttatctatg aatcagattc cagtgggaga 360

cccctaaagc agagggagaa taaggagttc tccccatgat ggaaaatatc caaagacaag 420
gtttcatgga gcaaagaatt ctggctagat ttggtttgta agtggatccc tccccactgc 480
gtgtacactt tatctgtctc tttgcttctt ccccaccctc tttcccagct ctctctctgt 540
ctctctcttg ntcccctgac ccttttttct tcccantgca tacttttttn tttccctttt 600
ttaatcttct atantcttaa ncctaccaan gggccctcnt gannaatttn tcacccctga 660
ataggggatt ctntangccc tgagaatttc nttatcanaa aaatattttt ttaaagcatt 720
a 721

<210> 755
<211> 721
<212> DNA
<213> Homo sapiens

<220>
<221> misc_feature
<222> 9, 13, 46, 63, 105, 132, 300, 551, 576, 590, 614, 621, 630,
639, 643, 644, 650, 673, 676, 691, 698
<223> n = A,T,C or G

<400> 755
accggattng ttnctgagcg cgtgactgct aataaaaaag atggantgcc atcttttttt 60
ttnccttgct ttatatatcc agcagcaaaa caaaattgtt ctgcngggct ataaaatttg 120
gcttgtgagt cntgtacaca actcaggagt gtgacacagc taccagcttt cctcctaact 180
ctcaagggaa gaaaattcaa gttctgtcta ggctcactct gtaaagtggg aaacttgctg 240
gttttgtagg ctttttttcc ccttctttcc ctctctcagc ttctccctgc ttctcagaan 300
atggagttgt gatgcctgca acttaccaaa tttatctatg aatcagattc cagtgggaga 360
cccctaaagc agagggagaa taaggagttc tccccatgat ggaaaatatc caaagacaag 420
gtttcatgga gcaaagaatt ctggctagat ttggtttgta agtggatccc tccccactgc 480
gtgtacactt tatctgtctc tttgcttctt ccccaccctc tttcccagct ctctctctgt 540
ctctctcttg ntcccctgac ccttttttct tcccantgca tacttttttn tttccctttt 600
ttaatcttct atantcttaa ncctaccaan gggccctcnt gannaatttn tcacccctga 660
ataggggatt ctntangccc tgagaatttc nttatcanaa aaatattttt ttaaagcatt 720
a 721

<210> 756
<211> 873
<212> DNA
<213> Homo sapiens

<220>
<221> misc_feature
<222> 590, 615, 708, 715, 728, 737, 768, 787, 802, 840, 846, 858,
868, 870
<223> n = A,T,C or G

<400> 756
ggaagaatac agtaagtttg caaattaaaa tttctctatt tttctgttat ttattcattt 60
ggaaactgtc agcctgtctc tttcactttg ggcaagtgaa agcaaagacg tccagtccta 120
tcagcaatta ggctgaaagt caacgccaag ctggcgggca agggctggtc tgagtagagg 180
ttccctaggc aggcaagaga gagactccca ctcgatactc ccagctcggc aactgcctga 240
atgccaatga gcactcatta taacccgccc tattttatag gatttaattt tacacttcag 300
gcttaatcag tctgaaagtt aaactgacag tgttaagtta cggaatcaat gacatttagg 360
ctttatgact ttgtagctga atatctatgg gctatatttc cattctaaca gtgatatcct 420
gttccagaat ctcattcttt ggtgatggca ctttctagtg gagcagtcat ggtaacagtc 480
cacacccatt accatgtggg tgctttacag catactgacg gaaggactga ggagccaccg 540
gagcaggagt tcctctcagg gaggacgctg acacttccac agctgcctan gtatgggcac 600
ctgatgccaa cgaanaaccc aaagcgctct cccttccaga tggaagctgc cccacactgg 660
gctgacagca tctggagctg ctctggctca aatcccggaa tcgcacanct cctancgggg 720
gcgtttanag atcctcnggg ccagctaccg accacttttg acaagggnct taggagcgat 780
aactagnctg gcgcgttaca cncggatgga acgtcttgga cttgagacct cttgggggan 840
atggcncccc caaataantt gggaaaantn ggg 873

<210> 757
<211> 782
<212> DNA
<213> Homo sapiens

<220>

<221> misc_feature
<222> 285, 304, 327, 349, 364, 400, 410, 412, 416, 485, 495, 521,
548, 569, 579, 616, 625, 650, 676, 682, 692, 701, 705, 718,
725, 733, 749, 750, 758, 763
<223> n = A,T,C or G

<400> 757
ggcccctcga gggatactct agagcggccg ccgactagtg agctcgtcga cgatatcccg 60
ggatttgaga ccaggagaca gctccagatg ctgtcagccc agtgctgggg gcaggcttcc 120
atctgtgaag tggagaggcg ctttgggctt cttcgttggc atcaggtgcc catacctagg 180
gcagctgtgg aagtgtcagc gtcctccctg agaggaactc ctgctccggt ggctcctcag 240
tccttccgtc agtatgctgt aaagcaccca catggtaatg ggtgnggact ggtaccatga 300
ctgntccctt aaaaggtggc cttcccnaag aaaggagaat tcttggacna gggatttcac 360
ttgnttagaa atgggaaaaa ttacccatta gaattttcgn ttccaaggcn tnaagnccta 420
aaaggccttt gattcccgaa ccttaaccct gggcagttaa cctttcaaac gggataaacc 480
ctgangggga aaatnaaatc ctttaaaaaa gggggggttt naaggagggc tctttggctt 540
tcaggcantt gccaacctgg gaaattcana ggggaagtnt ttttttttgc ctgcctaggg 600
aacctttact taaacnaacc cttgnccccc catttggggt tgactttcan cctaattgct 660
gaaaggaccg ggccgntttt gntttccttt gncccaaagg naaanaaacg ggtgccantt 720
cccangggat tanttcccga aaatttggnn aatttttntt tgnaactttt tgggtttttt 780
cc 782

<210> 758
<211> 647
<212> DNA
<213> Homo sapiens

<220>
<221> misc_feature
<222> 1, 58, 575
<223> n = A,T,C or G

<400> 758
ntttgtggcg gtggtgtctc atttgggtgg actttttggg tcgtaggaac ctggtatnga 60
gggaagagcg ccgtcggtcc gagtacagta tggagtagta tagtcttcgc gccttctcgg 120
gcggcggggc tattctctcc aaaggcagag gtccctagtc gacctcgctc ccctaggtta 180
ggaacagccg tcgaatattt taggttcgtc gaggctttct tccgagctct acgcctaagt 240
agctccgcga gcaaagtatc ggtcattttc ccctatccat cactccccta agtacgcctc 300
attattccgg aaggcaagag gccagcattc ctccttagag tagagggtag gtacctccgt 360
cgcgtgccgc gaaagggcag agcttcgtgt cttccctccg cagcagctta acggtctacg 420
taggcgttct cgatcttttc acgggaatcg gggtccggga gggcggcgga aaacgtcgac 480
gtctcggtca ccgtcaccgc cccgaacaac tagcggcttt ccgctttcaa ctgaggaacc 540
ccgcacccct cattagcgct tacgaaatcg gggangtgat tgcgccaatt cgttagcctt 600
cgataattat tctctattag cggtcctatc tcgcgctttc gatttat 647

<210> 759
<211> 657
<212> DNA
<213> Homo sapiens

<220>
<221> misc_feature
<222> 58, 513, 533, 589, 644, 652
<223> n = A,T,C or G

<400> 759
ctttgtggcg gtggtgtctc atttgggtgg actttttggg tcgtaggaac ctggtatnga 60
gggctctata gaaagcctct tgtctttaga tacgggcttt ctggtccttc gttctggaag 120
tgtagtagta ggtactgcgg gaaggcgaag agtcctttca aggacgattt acttaagttg 180
gcttattcta tagttccttc gggacataag gtcggtacga tctatactgc gtgggaagct 240
gataggttgg gacttaaggc gaataagaag gaggcggcgg aggtcgcgat taccgcagag 300
atattattta cggcggccgc gggtaccgcg ggtcatgcgg aaattttctg aggttcttgg 360
attcctaaga tcgctcccgt cgagtatact agcgacgaac gtaagagtgc cctcacaaga 420
accggtacaa actcaagaag aagttcccat taagcatcgt aagaaacggt aggacgagga 480
cggtaagaag taatcggaga aaggatccta gtngttacga agaagcatcg ttnagctact 540
ttgcgctacc gtttatattt agacgtgttc cgtccttctc cgtgtttana aaaaaggttt 600
attccgacgg gagacttagg cgaatggagg gttccgcggt tganaatcgg ancgggg 657

<210> 760
<211> 644
<212> DNA
<213> Homo sapiens

<220>
<221> misc_feature
<222> 59, 644
<223> n = A,T,C or G

<400> 760
ctttgtggcg gtggtgtctc atttgggtgg actttttggg tcgtaggaac ctggtatgna 60
ggaaaagaag taagcctcga agcctatctc cgaccgtatt tatttcgcag aagacggaac 120
tacggacgtc gttaaccccg agtagccccc gtaagaaagg actaaagcga atggaaaagt 180
cgggaattcc ggcggagggg cggcgattac tgaaaggagt aagagtaaga ctattgcgat 240
acttgaggcg ttccctctta aaaggcaccc gaaacactct attaaaaaac acccgaagaa 300
gaacaactca tgcgatcggc cgtgtgcagc cgtcaatagt aaagagagcc atgaaccatg 360
ccatccttag accaattagg atgaagaaga ggaggaagat gaggaccaaa ccctacccac 420
tcggaaaacc ccgcacgagc ctccgaacaa aatccgggaa ttaaaacggc ggcccacttc 480
cgcactctcg tagcgcggac cgaatagaaa accggaaact acagctaaag ggtcctttcc 540
ggcctgttat ctacccaccc gcaatccgat cctccccccc cctcgtccaa aaaccctaac 600
ctctgcggca acattagagc agaaggagag ggcgatccct tgan 644

<210> 761
<211> 647
<212> DNA
<213> Homo sapiens

<220>
<221> misc_feature
<222> 58, 550, 643
<223> n = A,T,C or G

<400> 761
ctttgtggcg gtggtgtctc atttgggtgg actttttggg tcgtaggaac ctggtatnga 60
ggcgggtact ctctgggata atcggtataa gtgttgtaaa attgggggta agagaaagtt 120
tcattataag aagtggaagc acgagccggg gtgtttagtc gttaatatta agaccggttt 180
ttgttgtact tatatagctt gcgcgtgggg aggcaataag aaacattgcg tttcgaggcc 240
ggatgcgggg aaccctcttc ggggtctaga gcgccgcatc tgcaaaataa ggactactga 300
cgccgctcat aacgtactca acaatgagtc ggcctgcatt aagatttcgg cgaagaaccg 360
tactgcgtct actgatagta tattgcattg atagcggcat gagctttatc acgtgtcgtt 420
ttcgggttgt aagaagggag ttaagtcgat cttcgaggaa gaagagaccc caaataaaaa 480
atgactcaaa aaaacctaga agaaacacga cgaaaggaaa aagaacgtta aaactagtag 540
ctcttcggan gagtagcctt agtagggtaa gtcctccgtg cgtactgtcc taaggtttgg 600
atagcgcggt tgaatagacg gtcacgcgtc agaaggtaaa aanccgg 647

<210> 762
<211> 628
<212> DNA
<213> Homo sapiens

<220>
<221> misc_feature
<222> 578, 585, 622
<223> n = A,T,C or G

<400> 762
cattgtgttg gggtcactga gcccactttt ttccagattt tttgtaaaat tgtttcgcat 60
tgtgttccct ttattcgctt gtattaatat ttgcgtagtg gattaaacaa atacttggtg 120
ttgactgtca gtcttagagg actgactaga agtagttttc atttggggct caggaaatac 180
ctactttata tttctagcta attaggaaag tcatttttca gttaggttgg tgttttggtt 240
caggcactcg ctagctagat gacctaacat gctacttaat ttctgagtgt ttgtgtccat 300
ccctgtagga ttgttgcggg gttaaatgaa attgtgtata tttgtaaagc atttacctca 360
gtgcccagac tgtgacagag tagattatta ggcttgctct tatttctgtg attaaattta 420
gtgtcagatt agcaacctat agctacttct aaagctgctg ctgctttctt tgtttagggt 480
taggaagaaa catgctggac agtttgccaa atgagagtta catgatgtgg cttgtgggaa 540
cattctaact tggaacttgc ccatttccag gactttgngg ttcanagatt tttggggata 600
gatgtaaggg ttaaaaaaaa cngaaaac 628

<210> 763
<211> 147
<212> DNA
<213> Homo sapiens

<220>
<221> misc_feature
<222> 110, 144
<223> n = A,T,C or G

<400> 763
cattgtgttg gggcagagat aaataattcc tctgaaaagt gttttattgg aatttcaaat 60
gaaaagctaa ctggataact tacagcatgt ttctgccaat aatctcttan aacaggcctc 120
tttttttat gcacaccacc ttcnggc 147

<210> 764
<211> 146
<212> DNA
<213> Homo sapiens

<220>
<221> misc_feature
<222> 93, 121, 122, 123, 135, 136
<223> n = A,T,C or G

<400> 764
cattgtgttg ggtatgtttt ttgaaggcag gtggacagga tttgctgatg ggtaaatggc 60
agagttaggg ggactgttag aacagagaaa ganatcatgg ggttgggttt gagtctgatg 120
nnnaactggt gccgnntgct cagtat 146

<210> 765
<211> 129
<212> DNA
<213> Homo sapiens

<220>
<221> misc_feature
<222> 2, 4, 12, 14, 22, 29, 71, 99, 106, 111, 121
<223> n = A,T,C or G

<400> 765
tncncgattc gntnctagcg tntacactna tgtcttggta ccgagctcgg atccactagt 60
ccagtgtggg nggaattcca ttgtgttggg gcaggaggng ctttgngtac ngtgcggctg 120
nagaggcgg 129

<210> 766
<211> 175
<212> DNA
<213> Homo sapiens

<220>
<221> misc_feature
<222> 73, 88, 131, 152, 155, 163, 167
<223> n = A,T,C or G

<400> 766
cattgtgttg ggcctagtcc gaatactttt agtaacttca gacagatctc ctcatctctt 60
tctggggctt ggnttttctc ctttgtanaa tgatgccttt ctgtggtttt gtcatttcta 120
acattctgtg ngtgatgagg tgtatattcg anganctcta tcnccanagt actct 175

<210> 767
<211> 602
<212> DNA
<213> Homo sapiens

<220>
<221> misc_feature

<222> 1, 2, 3, 11, 16
<223> n = A,T,C or G

<400> 767
nnntttaaaa nctgtnctcc ccgcggtggc ggccgctcta gaactagtgg atcctttcca 60
cctggtttgt tttcagtgtt taatcctatt agtatcagca ggatataggt caggatatca 120
ggtgcagaac ctgtggaatc agccaatttg gcttgctcat ttactttaat aaggtcccat 180
aatgagtgag agtacaaagt tcaagccctg ttgagggtct gcattaaact ctcagaagta 240
tttagagtgt gccaggagcc gcgaaggtct ggttcgggtg gtggcgggaa ctgtattaga 300
gtgctaggca cggcgcgaca aagtctgtcc aacccaaaac ggtgctgagg cgttgggtgt 360
gagctccagt actcagaaaa gcatctcagc aggtactcaa cagatcctca ggggcttggg 420
ggcccagcac tggcagtgag ggcatgaaag acataaaagg gcactacctg tgggtatttt 480
ctgttctcca aggaggaagt agcaaaaatt aggacgctgg aatatcctat gttgtagcaa 540
tcccagaaca actgatgctc aacaaatacc acacaaaaca aattttttaa aatttaatct 600
ta 602

<210> 768
<211> 671
<212> DNA
<213> Homo sapiens

<220>
<221> misc_feature
<222> 68, 348, 384, 385, 398, 421, 430, 435, 500, 508, 530, 541,
550, 578, 597, 611, 641, 646, 649, 651, 663, 669, 671
<223> n = A,T,C or G

<400> 768
   tccaccgcgg tggcggccgc tctagactag tggatccact agtccagtgt gggtgggaat 60
   tcgcggcncg cgtcgacaaa aatactgcta aagtaatatt tttatagatg actatttgcc 120
   ttggggccag gaaaagcagc tggagttatt cacttagtac catttttaca tactaacttt 180
   gccttttcca tgcttgcttg atgcggcttg cagcactgaa gaacagtttc aattgctagc 240
   caaccagaga gcatgatcaa accaaacaag ttccctgttt caggaaaaac aggttttagg 300
   taactgaagg gttaccagtt actgattcca caatcttctc tgtaaaanat ttctgcctat 360
   tatgcagact gggcggcttt aaanntggta aaactatnaa atacccatac aatattttaa 420
   nggggccccn ttatnaagct tttcaggcct tcccctttcc atagcattgg tgggatacaa 480
   gaaaccttta aacagcaacn agctatcnag gcccaaaagg aaagtaattn tgatttttta 540
   nagattccgn aacgaaaaaa tggctgggtt caaatacnac cttcttttta aaatggnttc 600
   cttattaaac nttttttttt tttaatttta ccccatggtc ntgatnttng ngcttccgcc 660
   canaaaatng n 671

<210> 769
<211> 877
<212> DNA
<213> Homo sapiens

<220>
<221> misc_feature
<222> 58, 59, 61, 99, 199, 682, 695, 740, 746, 751, 762, 765, 773,
774, 788, 792, 798, 811, 824, 831, 845, 858, 874
<223> n = A,T,C or G

<400> 769
aaagctggag ctccccgcgg tggcggccgc tctagaacta gtggatccac tagtccanng 60
ngggggaatt cgcggccgcg tcgacctcta tacctttgnt catgcagctt cctctgactg 120
ggtttgttct tcacttggct aacccctctt ttacttaagc acaccttgaa cattccctcc 180
ttccccattt ccccgcagng cccctaatgg acatacttct gaataacaca ggtggtattc 240
cttccttgtt ggaacctcct ggaggaagag acagatgatt aacaaatcct tccatcaacc 300
cctttgacca tgacatcaac agtgctccaa attatggggt accgtattag cctatgtcta 360
tcttgatcag aatccttacc tcggtgtatt gaaattatct atttcgtgcc tgcctcttta 420
aagtcagggt ttgccttatc tattgtctaa caccatgcag taggtaacat gcagtaggaa 480
acatggcatt aaattatttg ggttcaaatc ccagttatgg tgtgtaaatg cctaccaggc 540
cgtgaggcac ctgctaagca ggttgcacgc atcatttgaa ttcacaccac ccttttgcaa 600
tagaacagat aggcaacaga ggctcatttg ggctaaagga tttgatggag gggaagtgcc 660
aggattccca ccaaggcctc anggcccagg tccanggacc atgtctgttg tgacaactgg 720
agtgcatttc atatcccctn ctctgngggg naaggtccct cncgnggaga acnnttaaaa 780
caatcatntc tngggggntt aatgcttctt nccccagtgt ggtncactgc ngccacgagt 840
cccanccact agtcccangt ctgtcatgaa ccanccc 877

<210> 770
<211> 874
<212> DNA
<213> Homo sapiens

<220>
<221> misc_feature
<222> 5, 113, 114, 115, 709, 721, 727, 779, 797, 798, 812, 815,
833, 834, 839, 861, 862, 873
<223> n = A,T,C or G

<400> 770
ctggnctccc cgcggtggcg gccgctctag aactagtgga tccactagtc cagtgtggtg 60
gaattcgcgg ccgcgtcgac cttttcaaag gttaacttat ttaattatca cannngcaac 120
ccgatgagta ggtaacagta ttttactgat aggtaatcta aagaaggagg ctaaataaat 180
tgcccaattt cgaacagtga gaggaagaat taggattgaa acacatatag tggcttcaga 240
atctgtaacc ctcacgatgc cactactact tctttcagaa taccctttgc ctatctattc 300
tgttcctatg tcatcaaatt atacttactt taaaaagtat ttgtctttat tatttttaaa 360
aaaacacagg gaagtatttc tgatcagggg cagtattggt tctgaaagac aagccagtgt 420
ttttgagggt ttctcccttg ccagtttttc tatgctgggt tattcaagtc ctaagaattg 480
tgtagctatt acagaaccgc tttagcaaat gtgttccatt aatcaaggtg atttataaca 540
aaatttcatc caagtttgga gtgctctgaa aacatagcca aaatgttcgc agggtctacc 600
cctctcgtgt gtcccttttt tttagctatt tcagaagcac actggtgcaa tattttacga 660
aatgagtttc ttccccttac ctctgcatcc tctaagaaaa aatcattgnt gttttatgaa 720
natgaanatc ctgctatttc atatcttgat tggagctgct taattaaatg accattttna 780
aatttgtttt gattccnngc aaaaaaagtt tnttnttgga tgtagggggc tcnnaaagnc 840
caaaaccccc caaaattttt nnttgggaac ccna 874

<210> 771
<211> 156
<212> DNA
<213> Homo sapiens

<220>
<221> misc_feature
<222> 8, 13, 120, 121, 133
<223> n = A,T,C or G

<400> 771
ttaaaaanct ggnctccccg cggtggcggc cgctctagaa ctagtggatc cactagtcca 60
gtgtggtgga attcgcggcc gcgtcgaccg cgagcggtcg cccttttttt tttttttttn 120
ngtttttttg aanaattcat tgggtattta ttattc 156

<210> 772
<211> 586
<212> DNA
<213> Homo sapiens

<220>
<221> misc_feature
<222> 1, 5, 10, 106, 108, 347, 397, 415, 439, 453, 454, 459, 485,
487, 508, 510, 522, 545, 550, 552, 568, 573, 579, 584
<223> n = A,T,C or G

<400> 772
ncaanctggn ctccaccgcg gtggcggccg ctctagacta gtggatccac tagtccagtg 60
tggtggaatt cgcggccgcg tcgatcacaa agtgctcaca agtccngnat ttattttatc 120
tccagatatg aaacttaccc ccagctatgg tcttctattt gttatttaat ttctaggcca 180
atttttttcca cttgaatgtc agtattttaa ttcaaagtca ccttgtccaa ataccaagtc 240
atcaacttac cctcaaatta tatcctcatt cagaaaatct acatctatta atggtagcta 300
ttttatccct gccccctgct ttttcttttt atatttaatt aatttgntca tccagcaaat 360
gcttattgag caggtattgt aggctaaaca attctanact ttaaggggac acagnttgca 420
aaacaaaatc ctgccttgna tggatactta tgnnatggng ggatacagac aatcaacata 480
atgangngca tcatatataa tggttagnan aatgataagg gntttgggga aaaaaatgca 540
cccanccaan anggattggg aagtggangg ganggtcang ggangg 586

<210> 773

<211> 2983
<212> DNA
<213> Homo sapiens

<400> 773
agagatagag tcttccctgg cattgcagga gagaatctga agggatgatg gatgcatcaa 60
aagagctgca agttctccac attgacttct tgaatcagga caacgccgtt tctcaccaca 120
catgggagtt ccaaacgagc agtcctgtgt tccggcgagg acaggtgttt cacctgcggc 180
tggtgctgaa ccagccccta caatcctacc accaactgaa actggaattc agcacagggc 240
cgaatcctag catcgccaaa cacaccctgg tggtgctcga cccgaggacg ccctcagacc 300
actacaactg gcaggcaacc cttcaaaatg agtctggcaa agaggtcaca gtggctgtca 360
ccagttcccc caatgccatc ctgggcaagt accaactaaa cgtgaaaact ggaaaccaca 420
tccttaagtc tgaagaaaac atcctatacc ttctcttcaa cccatggtgt aaagaggaca 480
tggttttcat gcctgatgag gacgagcgca aagagtacat cctcaatgac acgggctgcc 540
attacgtggg ggctgccaga agtatcaaat gcaaaccctg gaactttggt cagtttgaga 600
aaaatgtcct ggactgctgc atttccctgc tgactgagag ctccctcaag cccacagata 660
ggagggaccc cgtgctggtg tgcagggcca tgtgtgctat gatgagcttt gagaaaggcc 720
agggcgtgct cattgggaat tggactgggg actatgaagg tggcacagcc ccatacaagt 780
ggacaggcag tgccccgatc ctgcagcagt actacaacac gaagcaggct gtgtgctttg 840
gccagtgctg ggtgtttgct gggatcctga ctacagtgct gagagcgttg ggcatcccag 900
cacgcagtgt gacaggcttc gattcagctc acgacacaga aaggaacctc acggtggaca 960
cctatgtgaa tgagaatggc aagaaaatca ccagtatgac ccacgactct gtctggaatt 1020
tccatgtgtg gacggatgcc tggatgaagc gaccggatct gcccaagggc tacgacggct 1080
ggcaggctgt ggacgcaacg ccgcaggagc gaagccaggg tgtcttctgc tgtgggccat 1140
caccactgac cgccatccgc aaaggtgaca tctttattgt ctatgacacc agattcgtct 1200
tctcagaagt gaatggtgac aggctcatct ggttggtgaa gatggtgaat gggcaggagg 1260
agttacacgt aatttcaatg gagaccacaa gcatcgggaa aaacatcagc accaaggcag 1320
tgggccaaga caggcggaga gatatcacct atgagtacaa gtatccagaa ggctcctctg 1380
aggagaggca ggtcatggat catgccttcc tccttctcag ttctgagagg gagcacagac 1440
gacctgtaaa agagaacttt cttcacatgt cggtacaatc agatgatgtg ctgctgggaa 1500
actctgttaa tttcaccgtg attcttaaaa ggaagaccgc tgccctacag aatgtcaaca 1560
tcttgggctc ctttgaacta cagttgtaca ctggcaagaa gatggcaaaa ctgtgtgacc 1620
tcaataagac ctcgcagatc caaggtcaag tatcagaagt gactctgacc ttggactcca 1680
agacctacat caacagcctg gctatattag atgatgagcc agttatcaga ggtttcatca 1740
ttgcggaaat tgtggagtct aaggaaatca tggcctctga agtattcacg tctttccagt 1800
accctgagtt ctctatagag ttgcctaaca caggcagaat tggccagcta cttgtctgca 1860
attgtatctt caagaatacc ctggccatcc ctttgactga cgtcaagttc tctttggaaa 1920
gcctgggcat ctcctcacta cagacctctg accatgggac ggtgcagcct ggtgagacca 1980
tccaatccca aataaaatgc accccaataa aaactggacc caagaaattt atcgtcaagt 2040
taagttccaa acaagtgaaa gagattaatg ctcagaagat tgttctcatc accaagtagc 2100
cttgtctgat gctgtggagc cttagttgag atttcagcat ttcctacctt gtgcttagct 2160
ttcagattat ggatgattaa atttgatgac ttatatgagg gcagattcaa gagccagcag 2220
gtcaaaaagg ccaacacaac cataagcagc cagacccaca aggccaggtc ctgtgctatc 2280
acagggtcac ctcttttaca gttagaaaca ccagccgagg ccacagaatc ccatcccttt 2340
cctgagtcat ggcctcaaaa atcagggcca ccattgtctc aattcaaatc catagatttc 2400
gaagccacag agtctctccc tggagcagca gactatgggc agcccagtgc tgccacctgc 2460
tgacgaccct tgagaagctg ccatatcttc aggccatggg ttcaccagcc ctgaaggcac 2520
ctgtcaactg gagtgctctc tcagcactgg gatgggcctg atagaagtgc attctcctcc 2580
tattgcctcc attctcctct ctctatccct gaaatccagg aagtccctct cctggtgctc 2640
caagcagttt gaagcccaat ctgcaaggac atttctcaag ggccatgtgg ttttgcagac 2700
aaccctgtcc tcaggcctga actcaccata gagacccatg tcagcaaacg gtgaccagca 2760
aatcctcttc ccttattcta aagctgcccc ttgggagact ccagggagaa ggcattgctt 2820
cctccctggt gtgaactctt tctttggtat tccatccact atcctggcaa ctcaaggctg 2880
cttctgttaa ctgaagcctg ctccttcttg ttctgccctc cagagatttg ctcaaatgat 2940
caataagctt taaattaaac tctacttcaa gaaaaaaaaa ccg 2983

<210> 774
<211> 3064
<212> DNA
<213> Homo sapiens

<400> 774
aattctaaaa atgcttttgc aagcttgcat gcctgcaggt gcagcggccg ccagtgtgat 60
ggatatctgc agaattcggc ttgcgctcag ctggaattcc gcagagatag agtcttccct 120
ggcattgcag gagagaatct gaagggatga tggatgcatc aaaagagctg caagttctcc 180
acattgactt cttgaatcag gacaacgccg tttctcacca cacatgggag ttccaaacga 240
gcagtcctgt gttccggcga ggacaggtgt ttcacctgcg gctggtgctg aaccagcccc 300
tacaatccta ccaccaactg aaactggaat tcagcacagg gccgaatcct agcatcgcca 360

aacacaccct ggtggtgctc gacccgagga cgccctcaga ccactacaac tggcaggcaa 420
cccttcaaaa tgagtctggc aaagaggtca cagtggctgt caccagttcc cccaatgcca 480
tcctgggcaa gtaccaacta aacgtgaaaa ctggaaacca catccttaag tctgaagaaa 540
acatcctata ccttctcttc aacccatggt gtaaagagga catggttttc atgcctgatg 600
aggacgagcg caaagagtac atcctcaatg acacgggctg ccattacgtg ggggctgcca 660
gaagtatcaa atgcaaaccc tggaactttg gtcagtttga gaaaaatgtc ctggactgct 720
gcatttccct gctgactgag agctccctca agcccacaga taggagggac cccgtgctgg 780
tgtgcagggc catgtgtgct atgatgagct ttgagaaagg ccagggcgtg ctcattggga 840
attggactgg ggactacgaa ggtggcacag ccccatacaa gtggacaggc agtgccccga 900
tcctgcagca gtactacaac acgaagcagg ctgtgtgctt tggccagtgc tgggtgtttg 960
ctgggatcct gactacagtg ctgagagcgt tgggcatccc agcacgcagt gtgacaggct 1020
tcgattcagc tcacgacaca gaaaggaacc tcacggtgga cacctatgtg aatgagaatg 1080
gcgagaaaat caccagtatg acccacgact ctgtctggaa tttccatgtg tggacggatg 1140
cctggatgaa gcgaccctac gacggctggc aggctgtgga cgcaacgccg caggagcgaa 1200
gccagggtgt cttctgctgt gggccatcac cactgaccgc catccgcaaa ggtgacatct 1260
ttattgtcta tgacaccaga ttcgtcttct cagaagtgaa tggtgacagg ctcatctggt 1320
tggtgaagat ggtgaatggg caggaggagt tacacgtaat ttcaatggag accacaagca 1380
tcgggaaaaa catcagcacc aaggcagtgg gccaagacag gcggagagat atcacctatg 1440
agtacaagta tccagaaggc tcctctgagg agaggcaggt catggatcat gccttcctcc 1500
ttctcagttc tgagagggag cacagacagc ctgtaaaaga gaactttctt cacatgtcgg 1560
tacaatcaga tgatgtgctg ctgggaaact ctgttaattt caccgtgatt cttaaaagga 1620
agaccgctgc cctacagaat gtcaacatct tgggctcctt tgaactacag ttgtacactg 1680
gcaagaagat ggcaaaactg tgtgacctca ataagacctc gcagatccaa ggtcaagtat 1740
cagaagtgac tctgaccttg gactccaaga cctacatcaa cagcctggct atattagatg 1800
atgagccagt tatcagaggt ttcatcattg cggaaattgt ggagtctaag gaaatcatgg 1860
cctctgaagt attcacgtca aaccagtacc ctgagttctc tatagagttg cctaacacag 1920
gcagaattgg ccagctactt gtctgcaatt gtatcttcaa gaataccctg gccatccctt 1980
tgactgacgt caagttctct ttggaaagcc tgggcatctc ctcactacag acctctgacc 2040
atgggacggt gcagcctggt gagaccatcc aatcccaaat aaaatgcacc ccaataaaaa 2100
ctggacccaa gaaatttatc gtcaagttaa gttccaaaca agtgaaagag attaatgctc 2160
agaagattgt tctcatcacc aagtagcctt gtctgatgct gtggagcctt agttgagatt 2220
tcagcatttc ctaccttgtg cttagctttc agattatgga tgattaaatt tgatgactta 2280
tatgagggca gattcaagag ccagcaggtc aaaaaggcca acacaaccat aagcagccag 2340
acccacaagg ccaggtcctg tgctatcaca gggtcacctc ttttacagtt agaaacacca 2400
gccgaggcca cagaatccca tccctttcct gagtcatggc ctcaaaaatc agggccacca 2460
ttgtctcaat tcaaatccat agatttcgaa gccacagagc tcttccctgg agcagcagac 2520
tatgggcagc ccagtgctgc cacctgctga cgacccttga gaagctgcca tatcttcagg 2580
ccatgggttc accagccctg aaggcacctg tcaactggag tgctctctca gcactgggat 2640
gggcctgata gaagtgcatt ctcctcctat tgcctccatt ctcctctctc tatccctgaa 2700
atccaggaag tccctctcct ggtgctccaa gcagtttgaa gcccaatctg caaggacatt 2760
tctcaagggc catgtggttt tgcagacaac cctgtcctca ggcctgaact caccatagag 2820
acccatgtca gcaaacggtg accagcaaat cctcttccct tattctaaag ctgccccttg 2880
ggagactcca gggagaaggc attgcttcct ccctggtgtg aactctttct ttggtattcc 2940
atccactatc ctggcaactc aaggctgctt ctgttaactg aagcctgctc cttcttgttc 3000
tgccctccag agatttgctc aaatgatcaa taagctttaa attaaaccgg aatccgcgga 3060
attc 3064

<210> 775
<211> 684
<212> PRT
<213> Homo sapiens

<400> 775
Met Met Asp Ala Ser Lys Glu Leu Gln Val Leu His Ile Asp Phe Leu
1               5                   10                  15
Asn Gln Asp Asn Ala Val Ser His His Thr Trp Glu Phe Gln Thr Ser
            20                  25                  30
Ser Pro Val Phe Arg Arg Gly Gln Val Phe His Leu Arg Leu Val Leu
        35                  40                  45
Asn Gln Pro Leu Gln Ser Tyr His Gln Leu Lys Leu Glu Phe Ser Thr
    50                  55                  60
Gly Pro Asn Pro Ser Ile Ala Lys His Thr Leu Val Val Leu Asp Pro
65                  70                  75                  80
Arg Thr Pro Ser Asp His Tyr Asn Trp Gln Ala Thr Leu Gln Asn Glu
                85                  90                  95
Ser Gly Lys Glu Val Thr Val Ala Val Thr Ser Ser Pro Asn Ala Ile
            100                 105                 110
Leu Gly Lys Tyr Gln Leu Asn Val Lys Thr Gly Asn His Ile Leu Lys

115                 120                 125
Ser Glu Glu Asn Ile Leu Tyr Leu Leu Phe Asn Pro Trp Cys Lys Glu
    130                 135                 140
Asp Met Val Phe Met Pro Asp Glu Asp Glu Arg Lys Glu Tyr Ile Leu
145                 150                 155                 160
Asn Asp Thr Gly Cys His Tyr Val Gly Ala Ala Arg Ser Ile Lys Cys
                165                 170                 175
Lys Pro Trp Asn Phe Gly Gln Phe Glu Lys Asn Val Leu Asp Cys Cys
            180                 185                 190
Ile Ser Leu Leu Thr Glu Ser Ser Leu Lys Pro Thr Asp Arg Arg Asp
        195                 200                 205
Pro Val Leu Val Cys Arg Ala Met Cys Ala Met Met Ser Phe Glu Lys
    210                 215                 220
Gly Gln Gly Val Leu Ile Gly Asn Trp Thr Gly Asp Tyr Glu Gly Gly
225                 230                 235                 240
Thr Ala Pro Tyr Lys Trp Thr Gly Ser Ala Pro Ile Leu Gln Gln Tyr
                245                 250                 255
Tyr Asn Thr Lys Gln Ala Val Cys Phe Gly Gln Cys Trp Val Phe Ala
            260                 265                 270
Gly Ile Leu Thr Thr Val Leu Arg Ala Leu Gly Ile Pro Ala Arg Ser
        275                 280                 285
Val Thr Gly Phe Asp Ser Ala His Asp Thr Glu Arg Asn Leu Thr Val
    290                 295                 300
Asp Thr Tyr Val Asn Glu Asn Gly Lys Lys Ile Thr Ser Met Thr His
305                 310                 315                 320
Asp Ser Val Trp Asn Phe His Val Trp Thr Asp Ala Trp Met Lys Arg
                325                 330                 335
Pro Asp Leu Pro Lys Gly Tyr Asp Gly Trp Gln Ala Val Asp Ala Thr
            340                 345                 350
Pro Gln Glu Arg Ser Gln Gly Val Phe Cys Cys Gly Pro Ser Pro Leu
        355                 360                 365
Thr Ala Ile Arg Lys Gly Asp Ile Phe Ile Val Tyr Asp Thr Arg Phe
    370                 375                 380
Val Phe Ser Glu Val Asn Gly Asp Arg Leu Ile Trp Leu Val Lys Met
385                 390                 395                 400
Val Asn Gly Gln Glu Glu Leu His Val Ile Ser Met Glu Thr Thr Ser
                405                 410                 415
Ile Gly Lys Asn Ile Ser Thr Lys Ala Val Gly Gln Asp Arg Arg Arg
            420                 425                 430
Asp Ile Thr Tyr Glu Tyr Lys Tyr Pro Glu Gly Ser Ser Glu Glu Arg
        435                 440                 445
Gln Val Met Asp His Ala Phe Leu Leu Leu Ser Ser Glu Arg Glu His
    450                 455                 460
Arg Arg Pro Val Lys Glu Asn Phe Leu His Met Ser Val Gln Ser Asp
465                 470                 475                 480
Asp Val Leu Leu Gly Asn Ser Val Asn Phe Thr Val Ile Leu Lys Arg
                485                 490                 495
Lys Thr Ala Ala Leu Gln Asn Val Asn Ile Leu Gly Ser Phe Glu Leu
            500                 505                 510
Gln Leu Tyr Thr Gly Lys Lys Met Ala Lys Leu Cys Asp Leu Asn Lys
        515                 520                 525
Thr Ser Gln Ile Gln Gly Gln Val Ser Glu Val Thr Leu Thr Leu Asp
    530                 535                 540
Ser Lys Thr Tyr Ile Asn Ser Leu Ala Ile Leu Asp Asp Glu Pro Val
545                 550                 555                 560
Ile Arg Gly Phe Ile Ile Ala Glu Ile Val Glu Ser Lys Glu Ile Met
                565                 570                 575
Ala Ser Glu Val Phe Thr Ser Phe Gln Tyr Pro Glu Phe Ser Ile Glu
            580                 585                 590
Leu Pro Asn Thr Gly Arg Ile Gly Gln Leu Leu Val Cys Asn Cys Ile
        595                 600                 605
Phe Lys Asn Thr Leu Ala Ile Pro Leu Thr Asp Val Lys Phe Ser Leu
    610                 615                 620
Glu Ser Leu Gly Ile Ser Ser Leu Gln Thr Ser Asp His Gly Thr Val
625                 630                 635                 640
Gln Pro Gly Glu Thr Ile Gln Ser Gln Ile Lys Cys Thr Pro Ile Lys
                645                 650                 655
Thr Gly Pro Lys Lys Phe Ile Val Lys Leu Ser Ser Lys Gln Val Lys

                660                 665                 670
Glu Ile Asn Ala Gln Lys Ile Val Leu Ile Thr Lys
        675                 680


<210> 776
<211> 679
<212> PRT
<213> Homo sapiens

<400> 776
Met Met Asp Ala Ser Lys Glu Leu Gln Val Leu His Ile Asp Phe Leu
 1               5                  10                  15
Asn Gln Asp Asn Ala Val Ser His His Thr Trp Glu Phe Gln Thr Ser
                20                  25                  30
Ser Pro Val Phe Arg Arg Gly Gln Val Phe His Leu Arg Leu Val Leu
        35                  40                  45
Asn Gln Pro Leu Gln Ser Tyr His Gln Leu Lys Leu Glu Phe Ser Thr
    50                  55                  60
Gly Pro Asn Pro Ser Ile Ala Lys His Thr Leu Val Val Leu Asp Pro
65                  70                  75                  80
Arg Thr Pro Ser Asp His Tyr Asn Trp Gln Ala Thr Leu Gln Asn Glu
                85                  90                  95
Ser Gly Lys Glu Val Thr Val Ala Val Thr Ser Ser Pro Asn Ala Ile
                100                 105                 110
Leu Gly Lys Tyr Gln Leu Asn Val Lys Thr Gly Asn His Ile Leu Lys
        115                 120                 125
Ser Glu Glu Asn Ile Leu Tyr Leu Leu Phe Asn Pro Trp Cys Lys Glu
    130                 135                 140
Asp Met Val Phe Met Pro Asp Glu Asp Glu Arg Lys Glu Tyr Ile Leu
145                 150                 155                 160
Asn Asp Thr Gly Cys His Tyr Val Gly Ala Ala Arg Ser Ile Lys Cys
                165                 170                 175
Lys Pro Trp Asn Phe Gly Gln Phe Glu Lys Asn Val Leu Asp Cys Cys
                180                 185                 190
Ile Ser Leu Leu Thr Glu Ser Ser Leu Lys Pro Thr Asp Arg Arg Asp
        195                 200                 205
Pro Val Leu Val Cys Arg Ala Met Cys Ala Met Met Ser Phe Glu Lys
    210                 215                 220
Gly Gln Gly Val Leu Ile Gly Asn Trp Thr Gly Asp Tyr Glu Gly Gly
225                 230                 235                 240
Thr Ala Pro Tyr Lys Trp Thr Gly Ser Ala Pro Ile Leu Gln Gln Tyr
                245                 250                 255
Tyr Asn Thr Lys Gln Ala Val Cys Phe Gly Gln Cys Trp Val Phe Ala
                260                 265                 270
Gly Ile Leu Thr Thr Val Leu Arg Ala Leu Gly Ile Pro Ala Arg Ser
        275                 280                 285
Val Thr Gly Phe Asp Ser Ala His Asp Thr Glu Arg Asn Leu Thr Val
    290                 295                 300
Asp Thr Tyr Val Asn Glu Asn Gly Glu Lys Ile Thr Ser Met Thr His
305                 310                 315                 320
Asp Ser Val Trp Asn Phe His Val Trp Thr Asp Ala Trp Met Lys Arg
                325                 330                 335
Pro Tyr Asp Gly Trp Gln Ala Val Asp Ala Thr Pro Gln Glu Arg Ser
                340                 345                 350
Gln Gly Val Phe Cys Cys Gly Pro Ser Pro Leu Thr Ala Ile Arg Lys
        355                 360                 365
Gly Asp Ile Phe Ile Val Tyr Asp Thr Arg Phe Val Phe Ser Glu Val
    370                 375                 380
Asn Gly Asp Arg Leu Ile Trp Leu Val Lys Met Val Asn Gly Gln Glu
385                 390                 395                 400
Glu Leu His Val Ile Ser Met Glu Thr Thr Ser Ile Gly Lys Asn Ile
                405                 410                 415
Ser Thr Lys Ala Val Gly Gln Asp Arg Arg Arg Asp Ile Thr Tyr Glu
                420                 425                 430
Tyr Lys Tyr Pro Glu Gly Ser Ser Glu Glu Arg Gln Val Met Asp His
        435                 440                 445
Ala Phe Leu Leu Leu Ser Ser Glu Arg Glu His Arg Gln Pro Val Lys

450                 455                 460
Glu Asn Phe Leu His Met Ser Val Gln Ser Asp Asp Val Leu Leu Gly
465                 470                 475                 480
Asn Ser Val Asn Phe Thr Val Ile Leu Lys Arg Lys Thr Ala Ala Leu
                485                 490                 495
Gln Asn Val Asn Ile Leu Gly Ser Phe Glu Leu Gln Leu Tyr Thr Gly
            500                 505                 510
Lys Lys Met Ala Lys Leu Cys Asp Leu Asn Lys Thr Ser Gln Ile Gln
        515                 520                 525
Gly Gln Val Ser Glu Val Thr Leu Thr Leu Asp Ser Lys Thr Tyr Ile
    530                 535                 540
Asn Ser Leu Ala Ile Leu Asp Asp Glu Pro Val Ile Arg Gly Phe Ile
545                 550                 555                 560
Ile Ala Glu Ile Val Glu Ser Lys Glu Ile Met Ala Ser Glu Val Phe
                565                 570                 575
Thr Ser Asn Gln Tyr Pro Glu Phe Ser Ile Glu Leu Pro Asn Thr Gly
            580                 585                 590
Arg Ile Gly Gln Leu Leu Val Cys Asn Cys Ile Phe Lys Asn Thr Leu
        595                 600                 605
Ala Ile Pro Leu Thr Asp Val Lys Phe Ser Leu Glu Ser Leu Gly Ile
    610                 615                 620
Ser Ser Leu Gln Thr Ser Asp His Gly Thr Val Gln Pro Gly Glu Thr
625                 630                 635                 640
Ile Gln Ser Gln Ile Lys Cys Thr Pro Ile Lys Thr Gly Pro Lys Lys
                645                 650                 655
Phe Ile Val Lys Leu Ser Ser Lys Gln Val Lys Glu Ile Asn Ala Gln
            660                 665                 670
Lys Ile Val Leu Ile Thr Lys
        675

<210> 777
<211> 5668
<212> DNA
<213> Homo sapiens

<400> 777
gtcacttagg aaaaggtgtc ctttcgggca gccgggctca gcatgaggaa cagaaggaat 60
gacactctgg acagcacccg gaccctgtac tccagcgcgt ctcggagcac agacttgtct 120
tacagtgaaa gcgacttggt gaattttatt caagcaaatt ttaagaaacg agaatgtgtc 180
ttctttacca aagattccaa ggccacggag aatgtgtgca agtgtggcta tgcccagagc 240
cagcacatgg aaggcaccca gatcaaccaa agtgagaaat ggaactacaa gaaacacacc 300
aaggaatttc ctaccgacgc ctttggggat attcagtttg agacactggg gaagaaaggg 360
aagtatatac gtctgtcctg cgacacggac gcggaaatcc tttacgagct gctgacccag 420
cactggcacc tgaaaacacc caacctggtc atttctgtga ccgggggcgc caagaacttc 480
gccctgaagc cgcgcatgcg caagatcttc agccggctca tctacatcgc gcagtccaaa 540
ggtgcttgga ttctcacggg aggcacccat tatggcctga cgaagtacat cggggaggtg 600
gtgagagata acaccatcag caggagttca gaggagaata ttgtggccat tggcatagca 660
gcttggggca tggtctccaa ccgggacacc ctcatcagga attgcgatgc tgagggctat 720
tttttagccc agtaccttat ggatgacttc acaagggatc cactgtatat cctggacaac 780
aaccacacac atttgctgct cgtggacaat ggctgtcatg gacatcccac tgtcgaagca 840
aagctccgga atcagctaga gaagcatatc tctgagcgca ctattcaaga ttccaactat 900
ggtggcaaga tccccattgt gtgttttgcc caaggaggtg gaaaagagac tttgaaagcc 960
atcaatacct ccatcaaaaa taaaattcct tgtgtggtgg tggaaggctc gggccggatc 1020
gctgatgtga tcgctagcct ggtggaggtg gaggatgccc cgacatcttc tgccgtcaag 1080
gagaagctgg tgcgcttttt accccgcacg gtgtcccggc tgtctgagga ggagactgag 1140
agttggatca aatggctcaa agaaattctc gaatgttctc acctattaac agttattaaa 1200
atggaagaag ctggggatga aattgtgagc aatgccatct cctacgctct atacaaagcc 1260
ttcagcacca gtgagcaaga caaggataac tggaatgggc agctgaagct tctgctggag 1320
tggaaccagc tggacttagc caatgatgag attttcacca atgaccgccg atgggagtct 1380
gctgaccttc aagaagtcat gtttacggct ctcataaagg acagacccaa gtttgtccgc 1440
ctctttctgg agaatggctt gaacctacgg aagtttctca cccatgatgt cctcactgaa 1500
ctcttctcca accacttcag cacgcttgtg taccggaatc tgcagatcgc caagaattcc 1560
tataatgatg ccctcctcac gtttgtctgg aaactggttg cgaacttccg aagaggcttc 1620
cggaaggaag acagaaatgg ccgggacgag atggacatag aactccacga cgtgtctcct 1680
attactcggc acccccctgca agctctcttc atctgggcca ttcttcagaa taagaaggaa 1740
ctctccaaag tcatttggga gcagaccagg ggctgcactc tggcagccct gggagccagc 1800
aagcttctga agactctggc caaagtgaag aacgacatca atgctgctgg ggagtccgag 1860

gagctggcta atgagtacga gacccgggct gttgagctgt tcactgagtg ttacagcagc 1920
gatgaagact tggcagaaca gctgctggtc tattcctgtg aagcttgggg tggaagcaac 1980
tgtctggagc tggcggtgga ggccacagac cagcatttca ccgcccagcc tggggtccag 2040
aattttcttt ctaagcaatg gtatggagag atttcccgag acaccaagaa ctggaagatt 2100
atcctgtgtc tgtttattat acccttggtg ggctgtggct ttgtatcatt taggaagaaa 2160
cctgtcgaca agcacaagaa gctgctttgg tactatgtgg cgttcttcac ctcccccttc 2220
gtggtcttct cctggaatgt ggtcttctac atcgccttcc tcctgctgtt tgcctacgtg 2280
ctgctcatgg atttccattc ggtgccacac ccccccgagc tggtcctgta ctcgctggtc 2340
tttgtcctct tctgtgatga agtgagacag tggtacgtaa atggggtgaa ttattttact 2400
gacctgtgga atgtgatgga cacgctgggg cttttttact tcatagcagg aattgtattt 2460
cggctccact cttctaataa aagctctttg tattctggac gagtcatttt ctgtctggac 2520
tacattattt tcactctaag attgatccac atttttactg taagcagaaa cttaggaccc 2580
aagattataa tgctgcagag gatgctgatc gatgtgttct tcttcctgtt cctctttgcg 2640
gtgtggatgg tggcctttgg cgtggccagg caagggatcc ttaggcagaa tgagcagcgc 2700
tggaggtgga tattccgttc ggtcatctac gagccctacc tggccatgtt cggccaggtg 2760
cccagtgacg tggatggtac cacgtatgac tttgcccact gcaccttcac tgggaatgag 2820
tccaagccac tgtgtgtgga gctggatgag cacaacctgc cccggttccc cgagtggatc 2880
accatccccc tggtgtgcat ctacatgtta tccaccaaca tcctgctggt caacctgctg 2940
gtcgccatgt ttggctacac ggtgggcacc gtccaggaga acaatgacca ggtctggaag 3000
ttccagaggt acttcctggt gcaggagtac tgcagccgcc tcaatatccc cttccccttc 3060
atcgtcttcg cttacttcta catggtggtg aagaagtgct tcaagtgttg ctgcaaggag 3120
aaaaacatgg agtcttctgt ctgctgtttc aaaaatgaag acaatgagac tctggcatgg 3180
gagggtgtca tgaaggaaaa ctaccttgtc aagatcaaca caaaagccaa cgacacctca 3240
gaggaaatga ggcatcgatt tagacaactg gatacaaagc ttaatgatct caagggtctt 3300
ctgaaagaga ttgctaataa aatcaaataa aactgtatga aactctaatg gagaaaaatc 3360
taattatagc aagatcatat taaggaatgc tgatgaacaa ttttgctatc gactactaaa 3420
tgagagattt tcagacccct gggtacatgg tggatgattt taaatcaccc tagtgtgctg 3480
agaccttgag aataaagtgt gtgattggtt tcatacttga agacggatat aaaggaagaa 3540
tatttccttt atgtgtttct ccagaatggt gcctgtttct ctctgtgtct caatgcctgg 3600
gactggaggt tgatagttta agtgtgttct taccgcctcc tttttccttt aatcttattt 3660
ttgatgaaca catatatagg agaacatcta tcctatgaat aagaacctgg tcatgcttta 3720
ctcctgtatt gttattttgt tcatttccaa ttgattctct acttttccct tttttgtatt 3780
atgtgactaa ttagttggca tattgttaaa agtctctcaa attaggccag attctaaaac 3840
atgctgcagc aagaggaccc cgctctcttc aggaaaagtg ttttcatttc tcaggatgct 3900
tcttacctgt cagaggaggt gacaaggcag tctcttgctc tcttggactc accaggctcc 3960
tattgaagga accaccccca ttcctaaata tgtgaaaagt cgcccaaaat gcaaccttga 4020
aaggcactac tgactttgtt cttattggat actcctctta tttattattt ttccattaaa 4080
aataatagct ggctattata gaaaatttag accatacaga gatgtagaaa gaacataaat 4140
tgtccccatt accttaaggt aatcactgct aacaatttct ggatggtttt tcaagtctat 4200
tttttttcta tgtatgtctc aattctcttt caaaatttta cagaatgtta tcatactaca 4260
tatatacttt ttatgtaagc tttttcactt agtattttat caaatatgtt tttattatat 4320
tcatagcctt cttaaacatt atatcaataa ttgcataata ggcaacctct agcgattacc 4380
ataattttgc tcattgaagg ctatctccag ttgatcattg ggatgagcat ctttgtgcat 4440
gaatcctatt gctgtatttg ggaaaatttt ccaaggttag attccaataa atatctattt 4500
attattaaat attaaaatat cgatttatta ttaaaaccat ttataaggct ttttcataaa 4560
tgtatagcaa ataggaatta ttaacttgag cataagatat gagatacatg aacctgaact 4620
attaaaataa aatattatat ttaaccctag tttaagaaga agtcaatatg cttatttaaa 4680
tattatggat ggtgggcaga tcacttgagg tcaggagttc gagaccagcc tggccaacat 4740
ggcaaaacca catctctact aaaaataaaa aaattagctg ggtgtggtgg tgcactcctg 4800
taatcccagc tactcagaag gctgaggtac aagaattgct ggaacctggg aggcggaggt 4860
tgcagtgaac caagattgca ccactgcact ccagccgggg tgacagagtg agactccgac 4920
tgaaaataaa taaataaata aataaataaa taaataaata aatattatgg atggtgaagg 4980
gaatggtata gaattggaga gattatctta ctgaacacct gtagtcccag ctttctctgg 5040
aagtggtggt atttgagcag gatgtgcaca aggcaattga aatgcccata attagtttct 5100
cagctttgaa tacactataa actcagtggc tgaaggagga aattttagaa ggaagctact 5160
aaaagatcta atttgaaaaa ctacaaaagc attaactaaa aaagtttatt ttccttttgt 5220
ctgggcagta gtgaaaataa ctactcacaa cattcactat gtttgcaagg aattaacaca 5280
aataaaagat gcctttttac ttaaacgcca agacagaaaa cttgcccaat actgagaagc 5340
aacttgcatt agagagggaa ctgttaaatg ttttcaaccc agttcatctg gtggatgttt 5400
ttgcaggtta ctctgagaat tttgcttatg aaaaatcatt atttttagtg tagttcacaa 5460
taatgtattg aacatacttc taatcaaagg tgctatgtcc ttgtgtatgg tactaaatgt 5520
gtcctgtgta cttttgcaca actgagaatc ctgcggcttg gtttaatgag tgtgttcatg 5580
aaataaataa tggaggaatt gtcaaaaaaa aaaaaaaaaa aaaaaaaaaa aaaaaaaaaa 5640
aaaaaaaaaa aaaaaaaaaa aaaaaaaa 5668

<210> 778

<211> 1095
<212> PRT
<213> Homo sapiens

<400> 778
Met Arg Asn Arg Arg Asn Asp Thr Leu Asp Ser Thr Arg Thr Leu Tyr
1               5                   10                  15
Ser Ser Ala Ser Arg Ser Thr Asp Leu Ser Tyr Ser Glu Ser Asp Leu
            20                  25                  30
Val Asn Phe Ile Gln Ala Asn Phe Lys Lys Arg Glu Cys Val Phe Phe
        35                  40                  45
Thr Lys Asp Ser Lys Ala Thr Glu Asn Val Cys Lys Cys Gly Tyr Ala
    50                  55                  60
Gln Ser Gln His Met Glu Gly Thr Gln Ile Asn Gln Ser Glu Lys Trp
65                  70                  75                  80
Asn Tyr Lys Lys His Thr Lys Glu Phe Pro Thr Asp Ala Phe Gly Asp
                85                  90                  95
Ile Gln Phe Glu Thr Leu Gly Lys Lys Gly Lys Tyr Ile Arg Leu Ser
            100                 105                 110
Cys Asp Thr Asp Ala Glu Ile Leu Tyr Glu Leu Leu Thr Gln His Trp
        115                 120                 125
His Leu Lys Thr Pro Asn Leu Val Ile Ser Val Thr Gly Gly Ala Lys
    130                 135                 140
Asn Phe Ala Leu Lys Pro Arg Met Arg Lys Ile Phe Ser Arg Leu Ile
145                 150                 155                 160
Tyr Ile Ala Gln Ser Lys Gly Ala Trp Ile Leu Thr Gly Gly Thr His
                165                 170                 175
Tyr Gly Leu Thr Lys Tyr Ile Gly Glu Val Val Arg Asp Asn Thr Ile
            180                 185                 190
Ser Arg Ser Ser Glu Glu Asn Ile Val Ala Ile Gly Ile Ala Ala Trp
        195                 200                 205
Gly Met Val Ser Asn Arg Asp Thr Leu Ile Arg Asn Cys Asp Ala Glu
    210                 215                 220
Gly Tyr Phe Leu Ala Gln Tyr Leu Met Asp Asp Phe Thr Arg Asp Pro
225                 230                 235                 240
Leu Tyr Ile Leu Asp Asn Asn His Thr His Leu Leu Leu Val Asp Asn
                245                 250                 255
Gly Cys His Gly His Pro Thr Val Glu Ala Lys Leu Arg Asn Gln Leu
            260                 265                 270
Glu Lys His Ile Ser Glu Arg Thr Ile Gln Asp Ser Asn Tyr Gly Gly
        275                 280                 285
Lys Ile Pro Ile Val Cys Phe Ala Gln Gly Gly Gly Lys Glu Thr Leu
    290                 295                 300
Lys Ala Ile Asn Thr Ser Ile Lys Asn Lys Ile Pro Cys Val Val Val
305                 310                 315                 320
Glu Gly Ser Gly Arg Ile Ala Asp Val Ile Ala Ser Leu Val Glu Val
                325                 330                 335
Glu Asp Ala Pro Thr Ser Ser Ala Val Lys Glu Lys Leu Val Arg Phe
            340                 345                 350
Leu Pro Arg Thr Val Ser Arg Leu Ser Glu Glu Glu Thr Glu Ser Trp
        355                 360                 365
Ile Lys Trp Leu Lys Glu Ile Leu Glu Cys Ser His Leu Leu Thr Val
    370                 375                 380
Ile Lys Met Glu Glu Ala Gly Asp Glu Ile Val Ser Asn Ala Ile Ser
385                 390                 395                 400
Tyr Ala Leu Tyr Lys Ala Phe Ser Thr Ser Glu Gln Asp Lys Asp Asn
                405                 410                 415
Trp Asn Gly Gln Leu Lys Leu Leu Leu Glu Trp Asn Gln Leu Asp Leu
            420                 425                 430
Ala Asn Asp Glu Ile Phe Thr Asn Asp Arg Arg Trp Glu Ser Ala Asp
        435                 440                 445
Leu Gln Glu Val Met Phe Thr Ala Leu Ile Lys Asp Arg Pro Lys Phe
    450                 455                 460
Val Arg Leu Phe Leu Glu Asn Gly Leu Asn Leu Arg Lys Phe Leu Thr
465                 470                 475                 480
His Asp Val Leu Thr Glu Leu Phe Ser Asn His Phe Ser Thr Leu Val
                485                 490                 495
Tyr Arg Asn Leu Gln Ile Ala Lys Asn Ser Tyr Asn Asp Ala Leu Leu

                    500                 505                 510
Thr Phe Val Trp Lys Leu Val Ala Asn Phe Arg Arg Gly Phe Arg Lys
        515                 520                 525
Glu Asp Arg Asn Gly Arg Asp Glu Met Asp Ile Glu Leu His Asp Val
    530                 535                 540
Ser Pro Ile Thr Arg His Pro Leu Gln Ala Leu Phe Ile Trp Ala Ile
545                 550                 555                 560
Leu Gln Asn Lys Lys Glu Leu Ser Lys Val Ile Trp Glu Gln Thr Arg
                565                 570                 575
Gly Cys Thr Leu Ala Ala Leu Gly Ala Ser Lys Leu Leu Lys Thr Leu
            580                 585                 590
Ala Lys Val Lys Asn Asp Ile Asn Ala Ala Gly Glu Ser Glu Glu Leu
        595                 600                 605
Ala Asn Glu Tyr Glu Thr Arg Ala Val Glu Leu Phe Thr Glu Cys Tyr
    610                 615                 620
Ser Ser Asp Glu Asp Leu Ala Glu Gln Leu Leu Val Tyr Ser Cys Glu
625                 630                 635                 640
Ala Trp Gly Gly Ser Asn Cys Leu Glu Leu Ala Val Glu Ala Thr Asp
                645                 650                 655
Gln His Phe Thr Ala Gln Pro Gly Val Gln Asn Phe Leu Ser Lys Gln
            660                 665                 670
Trp Tyr Gly Glu Ile Ser Arg Asp Thr Lys Asn Trp Lys Ile Ile Leu
        675                 680                 685
Cys Leu Phe Ile Ile Pro Leu Val Gly Cys Gly Phe Val Ser Phe Arg
    690                 695                 700
Lys Lys Pro Val Asp Lys His Lys Lys Leu Leu Trp Tyr Tyr Val Ala
705                 710                 715                 720
Phe Phe Thr Ser Pro Phe Val Val Phe Ser Trp Asn Val Val Phe Tyr
                725                 730                 735
Ile Ala Phe Leu Leu Leu Phe Ala Tyr Val Leu Leu Met Asp Phe His
            740                 745                 750
Ser Val Pro His Pro Pro Glu Leu Val Leu Tyr Ser Leu Val Phe Val
        755                 760                 765
Leu Phe Cys Asp Glu Val Arg Gln Trp Tyr Val Asn Gly Val Asn Tyr
    770                 775                 780
Phe Thr Asp Leu Trp Asn Val Met Asp Thr Leu Gly Leu Phe Tyr Phe
785                 790                 795                 800
Ile Ala Gly Ile Val Phe Arg Leu His Ser Ser Asn Lys Ser Ser Leu
                805                 810                 815
Tyr Ser Gly Arg Val Ile Phe Cys Leu Asp Tyr Ile Ile Phe Thr Leu
            820                 825                 830
Arg Leu Ile His Ile Phe Thr Val Ser Arg Asn Leu Gly Pro Lys Ile
        835                 840                 845
Ile Met Leu Gln Arg Met Leu Ile Asp Val Phe Phe Phe Leu Phe Leu
    850                 855                 860
Phe Ala Val Trp Met Val Ala Phe Gly Val Ala Arg Gln Gly Ile Leu
865                 870                 875                 880
Arg Gln Asn Glu Gln Arg Trp Arg Trp Ile Phe Arg Ser Val Ile Tyr
                885                 890                 895
Glu Pro Tyr Leu Ala Met Phe Gly Gln Val Pro Ser Asp Val Asp Gly
            900                 905                 910
Thr Thr Tyr Asp Phe Ala His Cys Thr Phe Thr Gly Asn Glu Ser Lys
        915                 920                 925
Pro Leu Cys Val Glu Leu Asp Glu His Asn Leu Pro Arg Phe Pro Glu
    930                 935                 940
Trp Ile Thr Ile Pro Leu Val Cys Ile Tyr Met Leu Ser Thr Asn Ile
945                 950                 955                 960
Leu Leu Val Asn Leu Leu Val Ala Met Phe Gly Tyr Thr Val Gly Thr
                965                 970                 975
Val Gln Glu Asn Asn Asp Gln Val Trp Lys Phe Gln Arg Tyr Phe Leu
            980                 985                 990
Val Gln Glu Tyr Cys Ser Arg Leu Asn Ile Pro Phe Pro Phe Ile Val
        995                 1000                1005
Phe Ala Tyr Phe Tyr Met Val Val Lys Lys Cys Phe Lys Cys Cys Cys
    1010                1015                1020
Lys Glu Lys Asn Met Glu Ser Ser Val Cys Cys Phe Lys Asn Glu Asp
1025                1030                1035                1040
Asn Glu Thr Leu Ala Trp Glu Gly Val Met Lys Glu Asn Tyr Leu Val

                1045                    1050                    1055
Lys Ile Asn Thr Lys Ala Asn Asp Thr Ser Glu Glu Met Arg His Arg
            1060                    1065                    1070
Phe Arg Gln Leu Asp Thr Lys Leu Asn Asp Leu Lys Gly Leu Leu Lys
        1075                    1080                    1085
Glu Ile Ala Asn Lys Ile Lys
    1090                    1095

<210> 779
<211> 3639
<212> DNA
<213> Homo sapiens

<400> 779
gattacgcaa gctatttagg tgacactata gaatwctcag cttgcatcaa gcttggtacc 60
gagctcggat ccctagtaac ggccgccagt gtgctggaat tcgcccttgc agccgggctc 120
agcatgagga acagaaggaa tgacactctg gacagcaccc ggaccctgta ctccagcgcg 180
tctcggagca cagacttgtc ttacagtgaa agcgacttgg tgaattttat tcaagcaaat 240
tttaagaaac gagaatgtgt cttctttacc aaagattcca aggccacgga gaatgtgtgc 300
aagtgtggct atgcccagag ccagcacatg gaaggcaccc agatcaacca aagtgagaaa 360
tggaactaca agaaacacac caaggaattt cctaccgacg cctttgggga tattcagttt 420
gagacactgg ggaagaaagg gaagtatata cgtctgtcct gcgacacgga cgcggaaatc 480
ctttacgagc tgctgaccca gcactggcac ctgaaaacac ccaacctggt catttctgtg 540
accgggggcg ccaagaactt cgccctgaag ccgcgcatgc gcaagatctt cagccggctc 600
atctacatcg cgcagtccaa aggtgcttgg attctcacgg gaggcaccca ttatggcctg 660
atgaagtaca tcggggaggt ggtgagagat aacaccatca gcaggagttc agaggagaat 720
attgtggcca ttggcatagc agcttggggc atggtctcca accgggacac cctcatcagg 780
aattgcgatg ctgagggcta tttttagcc cagtacctta tggatgactt cacaagagat 840
ccactgtata tcctggacaa caaccacaca catttgctgc tcgtggacaa tggctgtcat 900
ggacatccca ctgtcgaagc aaagctccgg aatcagctag agaagtatat ctctgagcgc 960
actattcaag attccaacta tggtggcaag atccccattg tgtgttttgc ccaaggaggt 1020
ggaaaagaga ctttgaaagc catcaatacc tccatcaaaa ataaaattcc ttgtgtggtg 1080
gtggaaggct cgggccagat cgctgatgtg atcgctagcc tggtggaggt ggaggatgcc 1140
ctgacatctt ctgccgtcaa ggagaagctg gtgcgctttt taccccgcac ggtgtcccgg 1200
ctgcctgagg aggagactga gagttggatc aaatggctca aagaaattct cgaatgttct 1260
cacctattaa cagttattaa aatggaagaa gctggggatg aaattgtgag caatgccatc 1320
tcctacgctc tatacaaagc cttcagcacc agtgagcaag acaaggataa ctggaatggg 1380
cagctgaagc ttctgctgga gtggaaccag ctggacttag ccaatgatga gattttcacc 1440
aatgaccgcc gatgggagtc tgctgacctt caagaagtca tgtttacggc tctcataaag 1500
gacagaccca agtttgtccg cctctttctg gagaatggct tgaacctacg gaagtttctc 1560
acccatgatg tcctcactga actcttctcc aaccacttca gcacgcttgt gtaccggaat 1620
ctgcagatcg ccaagaattc ctataatgat gccctcctca cgtttgtctg gaaactggtt 1680
gcgaacttcc gaagaggctt ccggaaggaa gacagaaatg gccgggacga gatggacata 1740
gaactccacg acgtgtctcc tattactcgg caccccctgc aagctctctt catctgggcc 1800
attcttcaga ataagaagga actctccaaa gtcatttggg agcagaccag ggctgcact 1860
ctggcagccc tgggagccag caagcttctg aagactctgg ccaaagtgaa gaacgacatc 1920
aatgctgctg gggagtccga ggagctggct aatgagtacg agacccgggc tgttgagctg 1980
ttcactgagt gttacagcag cgatgaagac ttggcagaac agctgctggt ctattcctgt 2040
gaagcttggg gtggaagcaa ctgtctggag ctggcggtgg aggccacaga ccagcatttc 2100
atcgcccagc ctggggtcca gaattttctt tctaagcaat ggtatggaga gatttcccga 2160
gacaccaaga actggaagat tatcctgtgt ctgtttatta taccccttggt gggctgtggc 2220
tttgtatcat ttaggaagaa acctgtcgac aagcacaaga agctgctttg gtactatgtg 2280
gcgttcttca cctccccctt cgtggtcttc tcctggaatg tggtcttcta catcgccttc 2340
ctcctgctgt ttgcctacgt gctgctcatg gatttccatt cggtgccaca cccccccgag 2400
ctggtcctgt actcgctggt ctttgtcctc ttctgtgatg aagtgagaca gtggtacgta 2460
aatggggtga attattttac tgacctgtgg aatgtgatgg acacgctggg gcttttttac 2520
ttcatagcag gaattgtatt tcggctccac tcttctaata aaagctcttt gtattctgga 2580
cgagtcattt tctgtctgga ctacattatt ttcactctaa gattgatcca catttttact 2640
gtaagcagaa acttaggacc caagattata atgctgcaga ggatgctgat cgatgtgttc 2700
ttcttcctgt tcctctttgc ggwgtggatg gtggcctttg gcgtggccag gcaagggatc 2760
cttaggcaga atgagcagcg ctggaggtgg atattccgtt cggtcatcta cgagccctac 2820
ctggccatgt tcggccaggt gcccagtgac gtggatggta ccacgtatga ctttgcccac 2880
tgcaccttca ctgggaatga gtccaagcca ctgtgtgtgg agctggatga gcacaacctg 2940
ccccggttcc ccgagtggat caccatcccc ctggtgtgca tctacatgtt atccaccaac 3000
atcctgctgg tcaacctgct ggtcgccatg tttggctaca cggtgggcac cgtccaggag 3060
aacaatgacc aggtctggaa gttccagagg tacttcctgg tgcaggagta ctgcagccgc 3120
ctcaatatcc ccttcccctt catcgtcttc gcttacttct acatggtggt gaagaagtgc 3180

ttcaagtgtt gctgcaagga gaaaaacatg gagtcttctg tctgctgttt caaaaatgaa 3240
gacaatgaga ctctggcatg ggagggtgtc atgaaggaaa actaccttgt caagatcaac 3300
acaaaagcca acgacacctc agaggaaatg aggcatcgat ttagacaact ggatacaaag 3360
cttaatgatc tcaagggtct tctgaaagag attgctaata aaatcaaata aaactgtatg 3420
aactctaatg gagaaaaatc taattatagc aagatcatat taaggaatgc tgatgaacaa 3480
ttttgctatc gactactaaa tgagagattt tcagacccct gggtacatgg tggatgattt 3540
taaatcaccc tagtgtgctg agaccttgag aataaagtgt gaagggcgaa ttctgcagat 3600
atccatcaca ctggcggccg ctcgagcatg catctagag 3639

<210> 780
<211> 1095
<212> PRT
<213> Homo sapiens

<220>
<221> VARIANT
<222> 867
<223> Xaa = Any Amino Acid

<400> 780
Met Arg Asn Arg Arg Asn Asp Thr Leu Asp Ser Thr Arg Thr Leu Tyr
1               5                   10                  15
Ser Ser Ala Ser Arg Ser Thr Asp Leu Ser Tyr Ser Glu Ser Asp Leu
            20                  25                  30
Val Asn Phe Ile Gln Ala Asn Phe Lys Lys Arg Glu Cys Val Phe Phe
        35                  40                  45
Thr Lys Asp Ser Lys Ala Thr Glu Asn Val Cys Lys Cys Gly Tyr Ala
    50                  55                  60
Gln Ser Gln His Met Glu Gly Thr Gln Ile Asn Gln Ser Glu Lys Trp
65                  70                  75                  80
Asn Tyr Lys Lys His Thr Lys Glu Phe Pro Thr Asp Ala Phe Gly Asp
                85                  90                  95
Ile Gln Phe Glu Thr Leu Gly Lys Lys Gly Lys Tyr Ile Arg Leu Ser
            100                 105                 110
Cys Asp Thr Asp Ala Glu Ile Leu Tyr Glu Leu Leu Thr Gln His Trp
        115                 120                 125
His Leu Lys Thr Pro Asn Leu Val Ile Ser Val Thr Gly Gly Ala Lys
    130                 135                 140
Asn Phe Ala Leu Lys Pro Arg Met Arg Lys Ile Phe Ser Arg Leu Ile
145                 150                 155                 160
Tyr Ile Ala Gln Ser Lys Gly Ala Trp Ile Leu Thr Gly Gly Thr His
                165                 170                 175
Tyr Gly Leu Met Lys Tyr Ile Gly Glu Val Val Arg Asp Asn Thr Ile
            180                 185                 190
Ser Arg Ser Ser Glu Glu Asn Ile Val Ala Ile Gly Ile Ala Ala Trp
        195                 200                 205
Gly Met Val Ser Asn Arg Asp Thr Leu Ile Arg Asn Cys Asp Ala Glu
    210                 215                 220
Gly Tyr Phe Leu Ala Gln Tyr Leu Met Asp Asp Phe Thr Arg Asp Pro
225                 230                 235                 240
Leu Tyr Ile Leu Asp Asn Asn His Thr His Leu Leu Leu Val Asp Asn
                245                 250                 255
Gly Cys His Gly His Pro Thr Val Glu Ala Lys Leu Arg Asn Gln Leu
            260                 265                 270
Glu Lys Tyr Ile Ser Glu Arg Thr Ile Gln Asp Ser Asn Tyr Gly Gly
        275                 280                 285
Lys Ile Pro Ile Val Cys Phe Ala Gln Gly Gly Gly Lys Glu Thr Leu
    290                 295                 300
Lys Ala Ile Asn Thr Ser Ile Lys Asn Lys Ile Pro Cys Val Val Val
305                 310                 315                 320
Glu Gly Ser Gly Gln Ile Ala Asp Val Ile Ala Ser Leu Val Glu Val
                325                 330                 335
Glu Asp Ala Leu Thr Ser Ser Ala Val Lys Glu Lys Leu Val Arg Phe
            340                 345                 350
Leu Pro Arg Thr Val Ser Arg Leu Pro Glu Glu Glu Thr Glu Ser Trp
        355                 360                 365
Ile Lys Trp Leu Lys Glu Ile Leu Glu Cys Ser His Leu Leu Thr Val
    370                 375                 380

Ile Lys Met Glu Glu Ala Gly Asp Glu Ile Val Ser Asn Ala Ile Ser
385                 390                 395                 400
Tyr Ala Leu Tyr Lys Ala Phe Ser Thr Ser Glu Gln Asp Lys Asp Asn
                405                 410                 415
Trp Asn Gly Gln Leu Lys Leu Leu Leu Glu Trp Asn Gln Leu Asp Leu
            420                 425                 430
Ala Asn Asp Glu Ile Phe Thr Asn Asp Arg Arg Trp Glu Ser Ala Asp
        435                 440                 445
Leu Gln Glu Val Met Phe Thr Ala Leu Ile Lys Asp Arg Pro Lys Phe
    450                 455                 460
Val Arg Leu Phe Leu Glu Asn Gly Leu Asn Leu Arg Lys Phe Leu Thr
465                 470                 475                 480
His Asp Val Leu Thr Glu Leu Phe Ser Asn His Phe Ser Thr Leu Val
                485                 490                 495
Tyr Arg Asn Leu Gln Ile Ala Lys Asn Ser Tyr Asn Asp Ala Leu Leu
            500                 505                 510
Thr Phe Val Trp Lys Leu Val Ala Asn Phe Arg Arg Gly Phe Arg Lys
        515                 520                 525
Glu Asp Arg Asn Gly Arg Asp Glu Met Asp Ile Glu Leu His Asp Val
    530                 535                 540
Ser Pro Ile Thr Arg His Pro Leu Gln Ala Leu Phe Ile Trp Ala Ile
545                 550                 555                 560
Leu Gln Asn Lys Lys Glu Leu Ser Lys Val Ile Trp Glu Gln Thr Arg
                565                 570                 575
Gly Cys Thr Leu Ala Ala Leu Gly Ala Ser Lys Leu Leu Lys Thr Leu
            580                 585                 590
Ala Lys Val Lys Asn Asp Ile Asn Ala Ala Gly Glu Ser Glu Glu Leu
        595                 600                 605
Ala Asn Glu Tyr Glu Thr Arg Ala Val Glu Leu Phe Thr Glu Cys Tyr
    610                 615                 620
Ser Ser Asp Glu Asp Leu Ala Glu Gln Leu Leu Val Tyr Ser Cys Glu
625                 630                 635                 640
Ala Trp Gly Gly Ser Asn Cys Leu Glu Leu Ala Val Glu Ala Thr Asp
                645                 650                 655
Gln His Phe Ile Ala Gln Pro Gly Val Gln Asn Phe Leu Ser Lys Gln
            660                 665                 670
Trp Tyr Gly Glu Ile Ser Arg Asp Thr Lys Asn Trp Lys Ile Ile Leu
        675                 680                 685
Cys Leu Phe Ile Ile Pro Leu Val Gly Cys Gly Phe Val Ser Phe Arg
    690                 695                 700
Lys Lys Pro Val Asp Lys His Lys Lys Leu Leu Trp Tyr Tyr Val Ala
705                 710                 715                 720
Phe Phe Thr Ser Pro Phe Val Val Phe Ser Trp Asn Val Val Phe Tyr
                725                 730                 735
Ile Ala Phe Leu Leu Leu Phe Ala Tyr Val Leu Leu Met Asp Phe His
            740                 745                 750
Ser Val Pro His Pro Pro Glu Leu Val Leu Tyr Ser Leu Val Phe Val
        755                 760                 765
Leu Phe Cys Asp Glu Val Arg Gln Trp Tyr Val Asn Gly Val Asn Tyr
    770                 775                 780
Phe Thr Asp Leu Trp Asn Val Met Asp Thr Leu Gly Leu Phe Tyr Phe
785                 790                 795                 800
Ile Ala Gly Ile Val Phe Arg Leu His Ser Ser Asn Lys Ser Ser Leu
                805                 810                 815
Tyr Ser Gly Arg Val Ile Phe Cys Leu Asp Tyr Ile Ile Phe Thr Leu
            820                 825                 830
Arg Leu Ile His Ile Phe Thr Val Ser Arg Asn Leu Gly Pro Lys Ile
        835                 840                 845
Ile Met Leu Gln Arg Met Leu Ile Asp Val Phe Phe Phe Leu Phe Leu
    850                 855                 860
Phe Ala Xaa Trp Met Val Ala Phe Gly Val Ala Arg Gln Gly Ile Leu
865                 870                 875                 880
Arg Gln Asn Glu Gln Arg Trp Arg Trp Ile Phe Arg Ser Val Ile Tyr
                885                 890                 895
Glu Pro Tyr Leu Ala Met Phe Gly Gln Val Pro Ser Asp Val Asp Gly
            900                 905                 910
Thr Thr Tyr Asp Phe Ala His Cys Thr Phe Thr Gly Asn Glu Ser Lys
        915                 920                 925

Pro Leu Cys Val Glu Leu Asp Glu His Asn Leu Pro Arg Phe Pro Glu
    930                 935                 940
Trp Ile Thr Ile Pro Leu Val Cys Ile Tyr Met Leu Ser Thr Asn Ile
945                 950                 955                 960
Leu Leu Val Asn Leu Leu Val Ala Met Phe Gly Tyr Thr Val Gly Thr
                965                 970                 975
Val Gln Glu Asn Asn Asp Gln Val Trp Lys Phe Gln Arg Tyr Phe Leu
            980                 985                 990
Val Gln Glu Tyr Cys Ser Arg Leu Asn Ile Pro Phe Pro Phe Ile Val
        995                 1000                1005
Phe Ala Tyr Phe Tyr Met Val Val Lys Lys Cys Phe Lys Cys Cys Cys
    1010                1015                1020
Lys Glu Lys Asn Met Glu Ser Ser Val Cys Cys Phe Lys Asn Glu Asp
1025                1030                1035                1040
Asn Glu Thr Leu Ala Trp Glu Gly Val Met Lys Glu Asn Tyr Leu Val
                1045                1050                1055
Lys Ile Asn Thr Lys Ala Asn Asp Thr Ser Glu Glu Met Arg His Arg
            1060                1065                1070
Phe Arg Gln Leu Asp Thr Lys Leu Asn Asp Leu Lys Gly Leu Leu Lys
        1075                1080                1085
Glu Ile Ala Asn Lys Ile Lys
    1090                1095


<210> 781
<211> 15
<212> PRT
<213> Homo sapiens

<400> 781
Arg Met Pro Thr Val Leu Gln Cys Val Asn Val Ser Val Val Ser
1               5                   10                  15


<210> 782
<211> 45
<212> DNA
<213> Homo sapiens

<400> 782
agaatgccta ccgtgctgca gtgcgtgaac gtgtcggtgg tgtct                45

<210> 783
<211> 45
<212> DNA
<213> Homo sapiens

<400> 783
gagccaggga gccagatggt ggaggccagc ctctccgtac ggcac                45

<210> 784
<211> 45
<212> DNA
<213> Homo sapiens

<400> 784
gaggccgacc aagagccagg gagccagatg gtggaggcca gcctc                45

<210> 785
<211> 45
<212> DNA
<213> Homo sapiens

<400> 785
ggcctgcaca gtcttgaggc cgaccaagag ccagggagcc agatg                45

<210> 786
<211> 45

<212> DNA
<213> Homo sapiens

<400> 786
tacaccatcg ggctgggcct gcacagtctt gaggccgacc aagag                45

<210> 787
<211> 42
<212> DNA
<213> Homo sapiens

<400> 787
ttccagaact cctacaccat cgggctgggc ctgcacagtc tt                   42

<210> 788
<211> 45
<212> DNA
<213> Homo sapiens

<400> 788
ctgtcagccg cacactgttt ccagaactcc tacaccatcg ggctg                45

<210> 789
<211> 45
<212> DNA
<213> Homo sapiens

<400> 789
catccgcagt gggtgctgtc agccgcacac tgtttccaga actcc                45

<210> 790
<211> 45
<212> DNA
<213> Homo sapiens

<400> 790
tcgggcgtcc tggtgcatcc gcagtgggtg ctgtcagccg cacac                45

<210> 791
<211> 45
<212> DNA
<213> Homo sapiens

<400> 791
aacgaattgt tctgctcggg cgtcctggtg catccgcagt gggtg                45

<210> 792
<211> 45
<212> DNA
<213> Homo sapiens

<400> 792
gcactggtca tggaaaacga attgttctgc tcgggcgtcc tggtg                45

<210> 793
<211> 51
<212> DNA
<213> Homo sapiens

<400> 793
tcgcagccct ggcaggcggc actggtcatg gaaaacgaat tgttctgctc g         51

<210> 794
<211> 45
<212> DNA
<213> Homo sapiens

<400> 794

atcagcattg cttcgcagtg ccctaccgcg gggaactctt gcctc 45

<210> 795
<211> 45
<212> DNA
<213> Homo sapiens

<400> 795
tccgtgtccg agtctgacac catccggagc atcagcattg cttcg 45

<210> 796
<211> 45
<212> DNA
<213> Homo sapiens

<400> 796
atcaagttgg acgaatccgt gtccgagtct gacaccatcc ggagc 45

<210> 797
<211> 45
<212> DNA
<213> Homo sapiens

<400> 797
aacgacctca tgctcatcaa gttggacgaa tccgtgtccg agtct 45

<210> 798
<211> 45
<212> DNA
<213> Homo sapiens

<400> 798
agacccttgc tcgctaacga cctcatgctc atcaagttgg acgaa 45

<210> 799
<211> 15
<212> PRT
<213> Homo sapiens

<400> 799
Glu Pro Gly Ser Gln Met Val Glu Ala Ser Leu Ser Val Arg His
1               5                   10                  15

<210> 800
<211> 15
<212> PRT
<213> Homo sapiens

<400> 800
Glu Ala Asp Gln Glu Pro Gly Ser Gln Met Val Glu Ala Ser Leu
1               5                   10                  15

<210> 801
<211> 15
<212> PRT
<213> Homo sapiens

<400> 801
Gly Leu His Ser Leu Glu Ala Asp Gln Glu Pro Gly Ser Gln Met
1               5                   10                  15

<210> 802
<211> 15
<212> PRT
<213> Homo sapiens

<400> 802
Tyr Thr Ile Gly Leu Gly Leu His Ser Leu Glu Ala Asp Gln Glu
1               5                   10                  15

<210> 803
<211> 14
<212> PRT
<213> Homo sapiens

<400> 803
Phe Gln Asn Ser Tyr Thr Ile Gly Leu Gly Leu His Ser Leu
1               5                   10

<210> 804
<211> 15
<212> PRT
<213> Homo sapiens

<400> 804
Leu Ser Ala Ala His Cys Phe Gln Asn Ser Tyr Thr Ile Gly Leu
1               5                   10                  15

<210> 805
<211> 15
<212> PRT
<213> Homo sapiens

<400> 805
His Pro Gln Trp Val Leu Ser Ala Ala His Cys Phe Gln Asn Ser
1               5                   10                  15

<210> 806
<211> 15
<212> PRT
<213> Homo sapiens

<400> 806
Ser Gly Val Leu Val His Pro Gln Trp Val Leu Ser Ala Ala His
1               5                   10                  15

<210> 807
<211> 15
<212> PRT
<213> Homo sapiens

<400> 807
Asn Glu Leu Phe Cys Ser Gly Val Leu Val His Pro Gln Trp Val
1               5                   10                  15

<210> 808
<211> 15
<212> PRT
<213> Homo sapiens

<400> 808
Ala Leu Val Met Glu Asn Glu Leu Phe Cys Ser Gly Val Leu Val
1               5                   10                  15

<210> 809
<211> 17

<212> PRT
<213> Homo sapiens

<400> 809
Ser Gln Pro Trp Gln Ala Ala Leu Val Met Glu Asn Glu Leu Phe Cys
1               5                   10                  15
Ser

<210> 810
<211> 15
<212> PRT
<213> Homo sapiens

<400> 810
Ile Ser Ile Ala Ser Gln Cys Pro Thr Ala Gly Asn Ser Cys Leu
1               5                   10                  15

<210> 811
<211> 15
<212> PRT
<213> Homo sapiens

<400> 811
Ser Val Ser Glu Ser Asp Thr Ile Arg Ser Ile Ser Ile Ala Ser
1               5                   10                  15

<210> 812
<211> 15
<212> PRT
<213> Homo sapiens

<400> 812
Ile Lys Leu Asp Glu Ser Val Ser Glu Ser Asp Thr Ile Arg Ser
1               5                   10                  15

<210> 813
<211> 15
<212> PRT
<213> Homo sapiens

<400> 813
Asn Asp Leu Met Leu Ile Lys Leu Asp Glu Ser Val Ser Glu Ser
1               5                   10                  15

<210> 814
<211> 15
<212> PRT
<213> Homo sapiens

<400> 814
Arg Pro Leu Leu Ala Asn Asp Leu Met Leu Ile Lys Leu Asp Glu
1               5                   10                  15

<210> 815
<211> 35
<212> DNA
<213> Artificial Sequence

<220>
<223> PCR Primer

<400> 815
ggaccagcat atgaggaaca gaaggaatga cactc                                35

<210> 816
<211> 29
<212> DNA
<213> Artificial Sequence

<220>
<223> PCR Primer

<400> 816
ccgctcgagt ccaccccaag cttcacagg                                       29

<210> 817
<211> 1959
<212> DNA
<213> Homo sapiens

<400> 817
atgaggaaca gaaggaatga cactctggac agcacccgga ccctgtactc cagcgcgtct 60
cggagcacag acttgtctta cagtgaaagc gacttggtga attttattca agcaaatttt 120
aagaaacgag aatgtgtctt ctttaccaaa gattccaagg ccacggagaa tgtgtgcaag 180
tgtggctatg cccagagcca gcacatggaa ggcacccaga tcaaccaaag tgagaaatgg 240
aactacaaga aacacaccaa ggaatttcct accgacgcct ttggggatat tcagtttgag 300
acactgggga agaaagggaa gtatatacgt ctgtcctgcg acacggacgc ggaaatcctt 360
tacgagctgc tgacccagca ctggcacctg aaaacaccca acctggtcat ttctgtgacc 420
gggggcgcca agaacttcgc cctgaagccg cgcatgcgca agatcttcag ccggctcatc 480
tacatcgcgc agtccaaagg tgcttggatt ctcacgggag gcacccatta tggcctgatg 540
aagtacatcg gggaggtggt gagagataac accatcagca ggagttcaga ggagaatatt 600
gtggccattg gcatagcagc ttggggcatg gtctccaacc gggacaccct catcaggaat 660
tgcgatgctg agggctattt tttagcccag taccttatgg atgacttcac aagagatcca 720
ctgtatatcc tggacaacaa ccacacacat ttgctgctcg tggacaatgg ctgtcatgga 780
catcccactg tcgaagcaaa gctccggaat cagctagaga agtatatctc tgagcgcact 840
attcaagatt ccaactatgg tggcaagatc cccattgtgt gttttgccca aggaggtgga 900
aaagagactt tgaaagccat caatacctcc atcaaaaata aaattccttg tgtggtggtg 960
gaaggctcgg gccagatcgc tgatgtgatc gctagcctgg tggaggtgga ggatgccctg 1020
acatcttctg ccgtcaagga gaagctggtg cgctttttac cccgcacggt gtcccggctg 1080
cctgaggagg agactgagag ttggatcaaa tggctcaaag aaattctcga atgttctcac 1140
ctattaacag ttattaaaat ggaagaagct ggggatgaaa ttgtgagcaa tgccatctcc 1200
tacgctctat acaaagcctt cagcaccagt gagcaagaca aggataactg gaatgggcag 1260
ctgaagcttc tgctggagtg gaaccagctg gacttagcca atgatgagat tttcaccaat 1320
gaccgccgat gggagtctgc tgaccttcaa gaagtcatgt ttacggctct cataaaggac 1380
agacccaagt ttgtccgcct ctttctggag aatggcttga acctacggaa gtttctcacc 1440
catgatgtcc tcactgaact cttctccaac cacttcagca cgcttgtgta ccggaatctg 1500
cagatcgcca agaattccta taatgatgcc ctcctcacgt ttgtctggaa actggttgcg 1560
aacttccgaa gaggcttccg gaaggaagac agaaatggcc gggacgagat ggacatagaa 1620
ctccacgacg tgtctcctat tactcggcac cccctgcaag ctctcttcat ctgggccatt 1680
cttcagaata agaaggaact ctccaaagtc atttgggagc agaccagggg ctgcactctg 1740
gcagccctgg gagccagcaa gcttctgaag actctggcca aagtgaagaa cgacatcaat 1800
gctgctgggg agtccgagga gctggctaat gagtacgaga cccgggctgt tgagctgttc 1860
actgagtgtt acagcagcga tgaagacttg gcagaacagc tgctggtcta ttcctgtgaa 1920
gcttggggtg gactcgagca ccaccaccac caccactga                            1959

<210> 818
<211> 652
<212> PRT
<213> Homo sapiens

<400> 818
Met Arg Asn Arg Arg Asn Asp Thr Leu Asp Ser Thr Arg Thr Leu Tyr
1               5                   10                  15
Ser Ser Ala Ser Arg Ser Thr Asp Leu Ser Tyr Ser Glu Ser Asp Leu
            20                  25                  30
Val Asn Phe Ile Gln Ala Asn Phe Lys Lys Arg Glu Cys Val Phe Phe
        35                  40                  45
Thr Lys Asp Ser Lys Ala Thr Glu Asn Val Cys Lys Cys Gly Tyr Ala
    50                  55                  60

Gln Ser Gln His Met Glu Gly Thr Gln Ile Asn Gln Ser Glu Lys Trp
65                  70                  75                  80
Asn Tyr Lys Lys His Thr Lys Glu Phe Pro Thr Asp Ala Phe Gly Asp
                85                  90                  95
Ile Gln Phe Glu Thr Leu Gly Lys Lys Gly Lys Tyr Ile Arg Leu Ser
            100                 105                 110
Cys Asp Thr Asp Ala Glu Ile Leu Tyr Glu Leu Leu Thr Gln His Trp
        115                 120                 125
His Leu Lys Thr Pro Asn Leu Val Ile Ser Val Thr Gly Gly Ala Lys
    130                 135                 140
Asn Phe Ala Leu Lys Pro Arg Met Arg Lys Ile Phe Ser Arg Leu Ile
145                 150                 155                 160
Tyr Ile Ala Gln Ser Lys Gly Ala Trp Ile Leu Thr Gly Gly Thr His
                165                 170                 175
Tyr Gly Leu Met Lys Tyr Ile Gly Glu Val Val Arg Asp Asn Thr Ile
            180                 185                 190
Ser Arg Ser Ser Glu Glu Asn Ile Val Ala Ile Gly Ile Ala Ala Trp
        195                 200                 205
Gly Met Val Ser Asn Arg Asp Thr Leu Ile Arg Asn Cys Asp Ala Glu
    210                 215                 220
Gly Tyr Phe Leu Ala Gln Tyr Leu Met Asp Asp Phe Thr Arg Asp Pro
225                 230                 235                 240
Leu Tyr Ile Leu Asp Asn Asn His Thr His Leu Leu Leu Val Asp Asn
                245                 250                 255
Gly Cys His Gly His Pro Thr Val Glu Ala Lys Leu Arg Asn Gln Leu
            260                 265                 270
Glu Lys Tyr Ile Ser Glu Arg Thr Ile Gln Asp Ser Asn Tyr Gly Gly
        275                 280                 285
Lys Ile Pro Ile Val Cys Phe Ala Gln Gly Gly Gly Lys Glu Thr Leu
    290                 295                 300
Lys Ala Ile Asn Thr Ser Ile Lys Asn Lys Ile Pro Cys Val Val Val
305                 310                 315                 320
Glu Gly Ser Gly Gln Ile Ala Asp Val Ile Ala Ser Leu Val Glu Val
                325                 330                 335
Glu Asp Ala Leu Thr Ser Ser Ala Val Lys Glu Lys Leu Val Arg Phe
            340                 345                 350
Leu Pro Arg Thr Val Ser Arg Leu Pro Glu Glu Glu Thr Glu Ser Trp
        355                 360                 365
Ile Lys Trp Leu Lys Glu Ile Leu Glu Cys Ser His Leu Leu Thr Val
    370                 375                 380
Ile Lys Met Glu Glu Ala Gly Asp Glu Ile Val Ser Asn Ala Ile Ser
385                 390                 395                 400
Tyr Ala Leu Tyr Lys Ala Phe Ser Thr Ser Glu Gln Asp Lys Asp Asn
                405                 410                 415
Trp Asn Gly Gln Leu Lys Leu Leu Leu Glu Trp Asn Gln Leu Asp Leu
            420                 425                 430
Ala Asn Asp Glu Ile Phe Thr Asn Asp Arg Arg Trp Glu Ser Ala Asp
        435                 440                 445
Leu Gln Glu Val Met Phe Thr Ala Leu Ile Lys Asp Arg Pro Lys Phe
    450                 455                 460
Val Arg Leu Phe Leu Glu Asn Gly Leu Asn Leu Arg Lys Phe Leu Thr
465                 470                 475                 480
His Asp Val Leu Thr Glu Leu Phe Ser Asn His Phe Ser Thr Leu Val
                485                 490                 495
Tyr Arg Asn Leu Gln Ile Ala Lys Asn Ser Tyr Asn Asp Ala Leu Leu
            500                 505                 510
Thr Phe Val Trp Lys Leu Val Ala Asn Phe Arg Arg Gly Phe Arg Lys
        515                 520                 525
Glu Asp Arg Asn Gly Arg Asp Glu Met Asp Ile Glu Leu His Asp Val
    530                 535                 540
Ser Pro Ile Thr Arg His Pro Leu Gln Ala Leu Phe Ile Trp Ala Ile
545                 550                 555                 560
Leu Gln Asn Lys Lys Glu Leu Ser Lys Val Ile Trp Glu Gln Thr Arg
                565                 570                 575
Gly Cys Thr Leu Ala Ala Leu Gly Ala Ser Lys Leu Leu Lys Thr Leu
            580                 585                 590
Ala Lys Val Lys Asn Asp Ile Asn Ala Ala Gly Glu Ser Glu Glu Leu
        595                 600                 605

Ala Asn Glu Tyr Glu Thr Arg Ala Val Glu Leu Phe Thr Glu Cys Tyr
    610                 615                 620
Ser Ser Asp Glu Asp Leu Ala Glu Gln Leu Leu Val Tyr Ser Cys Glu
625                 630                 635                 640
Ala Trp Gly Gly Leu Glu His His His His His His
                645                 650

<210> 819
<211> 132
<212> PRT
<213> Homo sapiens

<400> 819
Thr Ala Ala Ser Asp Asn Phe Gln Leu Ser Gln Gly Gly Gln Gly Phe
 1               5                  10                  15
Ala Ile Pro Ile Gly Gln Ala Met Ala Ile Ala Gly Gln Ile Arg Ser
            20                  25                  30
Gly Gly Gly Ser Pro Thr Val His Ile Gly Pro Thr Ala Phe Leu Gly
        35                  40                  45
Leu Gly Val Val Asp Asn Asn Gly Asn Gly Ala Arg Val Gln Arg Val
    50                  55                  60
Val Gly Ser Ala Pro Ala Ala Ser Leu Gly Ile Ser Thr Gly Asp Val
65                  70                  75                  80
Ile Thr Ala Val Asp Gly Ala Pro Ile Asn Ser Ala Thr Ala Met Ala
                85                  90                  95
Asp Ala Leu Asn Gly His His Pro Gly Asp Val Ile Ser Val Asn Trp
            100                 105                 110
Gln Thr Lys Ser Gly Gly Thr Arg Thr Gly Asn Val Thr Leu Ala Glu
        115                 120                 125
Gly Pro Pro Ala
    130

<210> 820
<211> 36
<212> DNA
<213> Artificial Sequence

<220>
<223> PCR Primer

<400> 820
ggggaattca tgatccggga gaaatttgcc cactgc                                36

<210> 821
<211> 33
<212> DNA
<213> Artificial Sequence

<220>
<223> PCR Primer

<400> 821
gggctcgagt caggagtttg agaccagcct ggc                                   33

<210> 822
<211> 675
<212> DNA
<213> Homo sapiens

<400> 822
atgcatcacc atcaccatca cacggccgcg tccgataact tccagctgtc ccagggtggg 60
cagggattcg ccattccgat cgggcaggcg atggcgatcg cgggccagat caagcttccc 120
accgttcata tcgggcctac cgccttcctc ggcttgggtg ttgtcgacaa caacggcaac 180
ggcgcacgag tccaacgcgt ggtcgggagc gctccggcgg caagtctcgg catctccacc 240
ggcgacgtga tcaccgcggt cgacggcgct ccgatcaact cggccaccgc gatggcggac 300
gcgcttaacg ggcatcatcc cggtgacgtc atctcggtga cctggcaaac caagtcgggc 360

ggcacgcgta cagggaacgt gacattggcc gagggacccc cggccgaatt catgatccgg 420
gagaaatttg cccactgcac cgtgctaacc attgcacaca gattgaacac cattattgac 480
agcgacaaga taatggtttt agattcagga agactgaaag aatatgatga gccgtatgtt 540
ttgctgcaaa ataaagagag cctattttac aagatggtgc aacaactggg caaggcagaa 600
gccgctgccc tcactgaaac agcaaaacag agatggggtt tcaccatgtt ggccaggctg 660
gtctcaaact cctga 675

<210> 823
<211> 291
<212> DNA
<213> Homo sapiens

<400> 823
atggggatcc gggagaaatt tgcccactgc accgtgctaa ccattgcaca cagattgaac 60
accattattg acagcgacaa gataatggtt ttagattcag gaagactgaa agaatatgat 120
gagccgtatg ttttgctgca aaataaagag agcctatttt acaagatggt gcaacaactg 180
ggcaaggcag aagccgctgc cctcactgaa acagcaaaac agagatgggg tttcaccatg 240
ttggccaggc tggtctcaaa ctccctcgag caccaccacc accaccactg a 291

<210> 824
<211> 1074
<212> DNA
<213> Homo sapiens

<400> 824
atgtcagcca ttgagagggt gtcagaggca atcgtcagca tccgaagaat ccagaccttt 60
ttgctacttg atgagatatc acagcgcaac cgtcagctgc cgtcagatgg taaaaagatg 120
gtgcatgtgc aggattttac tgctttttgg gataaggcat cagagacccc aactctacaa 180
ggcctttcct ttactgtcag acctggcgaa ttgttagctg tggtcggccc cgtgggagca 240
gggaagtcat cactgttaag tgccgtgctc ggggaattgg ccccaagtca cgggctggtc 300
agcgtgcatg gaagaattgc ctatgtgtct cagcagccct gggtgttctc gggaactctg 360
aggagtaata ttttatttgg gaagaaatac gaaaaggaac gatatgaaaa agtcataaag 420
gcttgtgctc tgaaaaagga tttacagctg ttggaggatg gtgatctgac tgtgatagga 480
gatcggggaa ccacgctgag tggagggcag aaagcacggg taaaccttgc aagagcagtg 540
tatcaagatg ctgacatcta tctcctggac gatcctctca gtgcagtaga tgcggaagtt 600
agcagacact tgttcgaact gtgtatttgt caaattttgc atgagaagat cacaatttta 660
gtgactcatc agttgcagta cctcaaagct gcaagtcaga ttctgatatt gaaagatggt 720
aaaatggtgc agaaggggac ttacactgag ttcctaaaat ctggtataga ttttggctcc 780
cttttaaaga aggataatga ggaaagtgaa caacctccag ttccaggaac tcccacacta 840
aggaatcgta ccttctcaga gtcttcggtt tggtctcaac aatcttctag accctccttg 900
aaagatggtg ctctggagag ccaagataca gagaatgtcc cagttacact atcagaggag 960
aaccgttctg aaggaaaagt tggttttcag gcctataaga attacttcag agctggtgct 1020
cactggattg tcttcatttt ccttattctc gagcaccacc accaccacca ctga 1074

<210> 825
<211> 224
<212> PRT
<213> Homo sapiens

<400> 825
Met His His His His His His Thr Ala Ala Ser Asp Asn Phe Gln Leu
1               5                   10                  15
Ser Gln Gly Gly Gln Gly Phe Ala Ile Pro Ile Gly Gln Ala Met Ala
            20                  25                  30
Ile Ala Gly Gln Ile Lys Leu Pro Thr Val His Ile Gly Pro Thr Ala
        35                  40                  45
Phe Leu Gly Leu Gly Val Val Asp Asn Asn Gly Asn Gly Ala Arg Val
    50                  55                  60
Gln Arg Val Val Gly Ser Ala Pro Ala Ala Ser Leu Gly Ile Ser Thr
65                  70                  75                  80
Gly Asp Val Ile Thr Ala Val Asp Gly Ala Pro Ile Asn Ser Ala Thr
                85                  90                  95
Ala Met Ala Asp Ala Leu Asn Gly His His Pro Gly Asp Val Ile Ser
            100                 105                 110
Val Thr Trp Gln Thr Lys Ser Gly Gly Thr Arg Thr Gly Asn Val Thr
        115                 120                 125
Leu Ala Glu Gly Pro Pro Ala Glu Phe Met Ile Arg Glu Lys Phe Ala
    130                 135                 140

His Cys Thr Val Leu Thr Ile Ala His Arg Leu Asn Thr Ile Ile Asp
145                 150                 155                 160
Ser Asp Lys Ile Met Val Leu Asp Ser Gly Arg Leu Lys Glu Tyr Asp
                165                 170                 175
Glu Pro Tyr Val Leu Leu Gln Asn Lys Glu Ser Leu Phe Tyr Lys Met
            180                 185                 190
Val Gln Gln Leu Gly Lys Ala Glu Ala Ala Ala Leu Thr Glu Thr Ala
        195                 200                 205
Lys Gln Arg Trp Gly Phe Thr Met Leu Ala Arg Leu Val Ser Asn Ser
    210                 215                 220

<210> 826
<211> 357
<212> PRT
<213> Homo sapiens

<400> 826
Met Ser Ala Ile Glu Arg Val Ser Glu Ala Ile Val Ser Ile Arg Arg
 1               5                  10                  15
Ile Gln Thr Phe Leu Leu Leu Asp Glu Ile Ser Gln Arg Asn Arg Gln
            20                  25                  30
Leu Pro Ser Asp Gly Lys Lys Met Val His Val Gln Asp Phe Thr Ala
        35                  40                  45
Phe Trp Asp Lys Ala Ser Glu Thr Pro Thr Leu Gln Gly Leu Ser Phe
    50                  55                  60
Thr Val Arg Pro Gly Glu Leu Leu Ala Val Val Gly Pro Val Gly Ala
65                  70                  75                  80
Gly Lys Ser Ser Leu Leu Ser Ala Val Leu Gly Glu Leu Ala Pro Ser
                85                  90                  95
His Gly Leu Val Ser Val His Gly Arg Ile Ala Tyr Val Ser Gln Gln
            100                 105                 110
Pro Trp Val Phe Ser Gly Thr Leu Arg Ser Asn Ile Leu Phe Gly Lys
        115                 120                 125
Lys Tyr Glu Lys Glu Arg Tyr Glu Lys Val Ile Lys Ala Cys Ala Leu
    130                 135                 140
Lys Lys Asp Leu Gln Leu Leu Glu Asp Gly Asp Leu Thr Val Ile Gly
145                 150                 155                 160
Asp Arg Gly Thr Thr Leu Ser Gly Gly Gln Lys Ala Arg Val Asn Leu
                165                 170                 175
Ala Arg Ala Val Tyr Gln Asp Ala Asp Ile Tyr Leu Leu Asp Asp Pro
            180                 185                 190
Leu Ser Ala Val Asp Ala Glu Val Ser Arg His Leu Phe Glu Leu Cys
        195                 200                 205
Ile Cys Gln Ile Leu His Glu Lys Ile Thr Ile Leu Val Thr His Gln
    210                 215                 220
Leu Gln Tyr Leu Lys Ala Ala Ser Gln Ile Leu Ile Leu Lys Asp Gly
225                 230                 235                 240
Lys Met Val Gln Lys Gly Thr Tyr Thr Glu Phe Leu Lys Ser Gly Ile
                245                 250                 255
Asp Phe Gly Ser Leu Leu Lys Lys Asp Asn Glu Glu Ser Glu Gln Pro
            260                 265                 270
Pro Val Pro Gly Thr Pro Thr Leu Arg Asn Arg Thr Phe Ser Glu Ser
        275                 280                 285
Ser Val Trp Ser Gln Gln Ser Ser Arg Pro Ser Leu Lys Asp Gly Ala
    290                 295                 300
Leu Glu Ser Gln Asp Thr Glu Asn Val Pro Val Thr Leu Ser Glu Glu
305                 310                 315                 320
Asn Arg Ser Glu Gly Lys Val Gly Phe Gln Ala Tyr Lys Asn Tyr Phe
                325                 330                 335
Arg Ala Gly Ala His Trp Ile Val Phe Ile Phe Leu Ile Leu Glu His
            340                 345                 350
His His His His His
            355

<210> 827
<211> 96

<212> PRT
<213> Homo sapiens

<400> 827
Met Gly Ile Arg Glu Lys Phe Ala His Cys Thr Val Leu Thr Ile Ala
1               5                   10                  15
His Arg Leu Asn Thr Ile Ile Asp Ser Asp Lys Ile Met Val Leu Asp
            20                  25                  30
Ser Gly Arg Leu Lys Glu Tyr Asp Glu Pro Tyr Val Leu Leu Gln Asn
        35                  40                  45
Lys Glu Ser Leu Phe Tyr Lys Met Val Gln Gln Leu Gly Lys Ala Glu
    50                  55                  60
Ala Ala Ala Leu Thr Glu Thr Ala Lys Gln Arg Trp Gly Phe Thr Met
65                  70                  75                  80
Leu Ala Arg Leu Val Ser Asn Ser Leu Glu His His His His His His
                85                  90                  95


<210> 828
<211> 35
<212> DNA
<213> Artificial Sequence

<220>
<223> PCR Primer

<400> 828
cgcccatggg gatccgggag aaatttgccc actgc                                35

<210> 829
<211> 35
<212> DNA
<213> Artificial Sequence

<220>
<223> PCR Primer

<400> 829
cgcctcgagg gagtttgaga ccagcctggc caaca                                35

<210> 830
<211> 38
<212> DNA
<213> Artificial Sequence

<220>
<223> PCR Primer

<400> 830
gcatggacca tatgtcagcc attgagaggg tgtcagag                             38

<210> 831
<211> 34
<212> DNA
<213> Artificial Sequence

<220>
<223> PCR Primer

<400> 831
ccgctcgaga ataaggaaaa tgaagacaat ccag                                 34

<210> 832
<211> 27
<212> DNA
<213> Artificial Sequence

<220>

<223> PCR Primer

<400> 832
gttgaattca tgcacgggcc ccaggtg                                          27

<210> 833
<211> 30
<212> DNA
<213> Artificial Sequence

<220>
<223> PCR Primer

<400> 833
cccctcgagt cactatggtc tgcctcttga                                       30

<210> 834
<211> 915
<212> DNA
<213> Homo sapiens

<400> 834
atgcatcacc atcaccatca cacggccgcg tccgataact tccagctgtc ccagggtggg 60
cagggattcg ccattccgat cgggcaggcg atggcgatcg cgggccagat caagcttccc 120
accgttcata tcgggcctac cgccttcctc ggcttgggtg ttgtcgacaa caacggcaac 180
ggcgcacgag tccaacgcgt ggtcgggagc gctccggcgg caagtctcgg catctccacc 240
ggcgacgtga tcaccgcggt cgacggcgct ccgatcaact cggccaccgc gatggcggac 300
gcgcttaacg ggcatcatcc cggtgacgtc atctcggtga cctggcaaac caagtcgggc 360
ggcacgcgta cagggaacgt gacattggcc gagggacccc cggccgaatt catgcacggg 420
ccccaggtgc tggcacgctg ctccgagtgt gcttgtcctg ccttggctgc cacctctgcg 480
ggggtgcgtc tggagggggt ggaccggcca ccaaccttac ccagtcaagg aagtggatgg 540
ccatgttccc acagcctgag tggctgccac ctgatggctg atggagcaaa ggccttagga 600
aaagcagatg gcccttggcc ctaccttttt gttagaagaa ctgatgttcc atgtcctgca 660
gcgagtgagg ttggtggctg tgcccccagc tcctggcgcg ccctcgcaga ggtgactggt 720
tgctctttgg gccctcttgg ccttgcccag catgcacaag cctcagtgct actactgtgc 780
tacaaatgga gccatatagg ggaaacgagc agccatctca ggagcaaggt gtatgctgcc 840
tttgggggct ccagtccttg cctcaagggt cttatgtcac tgtgggcttc ttggttgtca 900
agaggcagac catag                                                       915

<210> 835
<211> 304
<212> PRT
<213> Homo sapiens

<400> 835

<210> 836
<211> 24
<212> DNA
<213> Artificial Sequence

<220>
<223> PCR Primer

<400> 836
cgaagtcacg tggaggccag cctc                                              24

<210> 837
<211> 29
<212> DNA
<213> Artificial Sequence

<220>
<223> PCR Primer

<400> 837
cctgaccgaa ttcattaact ggcctggac                                         29

<210> 838
<211> 166
<212> PRT
<213> Homo sapiens

<220>
<221> VARIANT
<222> 110, 112
<223> Xaa = Any Amino Acid

<400> 838
Met Gly His His His His His His Val Glu Ala Ser Leu Ser Val Arg
 1               5                  10                  15
His Pro Glu Tyr Asn Arg Pro Leu Leu Ala Asn Asp Leu Met Leu Ile
                20                  25                  30
Lys Leu Asp Glu Ser Val Ser Glu Ser Asp Thr Ile Arg Ser Ile Ser
        35                  40                  45
Ile Ala Ser Gln Cys Pro Thr Ala Gly Asn Ser Cys Leu Val Ser Gly
    50                  55                  60
Trp Gly Leu Leu Ala Asn Gly Arg Met Pro Thr Val Leu Gln Cys Val
65                  70                  75                  80
Asn Val Ser Val Val Ser Glu Glu Val Cys Ser Lys Leu Tyr Asp Pro
                85                  90                  95
Leu Tyr His Pro Ser Met Phe Cys Ala Gly Gly Gly Gln Xaa Gln Xaa
                100                 105                 110
Asp Ser Cys Asn Gly Asp Ser Gly Gly Pro Leu Ile Cys Asn Gly Tyr
        115                 120                 125
Leu Gln Gly Leu Val Ser Phe Gly Lys Ala Pro Cys Gly Gln Val Gly
    130                 135                 140

Val Pro Gly Val Tyr Thr Asn Leu Cys Lys Phe Thr Glu Trp Ile Glu
145                 150                 155                 160
Lys Thr Val Gln Ala Ser
                165

<210> 839
<211> 504
<212> DNA
<213> Homo sapiens

<220>
<221> misc_feature
<222> 328, 336
<223> n = A,T,C or G

<400> 839
atgggccatc atcatcatca tcacgtggag gccagcctct ccgtacggca cccagagtac 60
aacagaccct tgctcgctaa cgacctcatg ctcatcaagt tggacgaatc cgtgtccgag 120
tctgacacca tccggagcat cagcattgct tcgcagtgcc ctaccgcggg gaactcttgc 180
ctcgtttctg gctggggtct gctggcgaac ggcagaatgc ctaccgtgct gcagtgcgtg 240
aacgtgtcgg tggtgtctga ggaggtctgc agtaagctct atgacccgct gtaccacccc 300
agcatgttct gcgccggcgg agggcaanac cagaangact cctgcaacgg tgactctggg 360
gggcccctga tctgcaacgg gtacttgcag ggccttgtgt ctttcggaaa agccccgtgt 420
ggccaagttg gcgtgccagg tgtctacacc aacctctgca aattcactga gtggatagag 480
aaaaccgtcc aggccagtta atga                                         504

<210> 840
<211> 21
<212> DNA
<213> Artificial Sequence

<220>
<223> PCR Primer

<400> 840
ctcagggttc cggagccgcg g                                            21

<210> 841
<211> 35
<212> DNA
<213> Artificial Sequence

<220>
<223> PCR Primer

<400> 841
ctatagaatt cattaccaaa aagctgggct ccagc                             35

<210> 842
<211> 241
<212> PRT
<213> Homo sapiens

<400> 842
Met Gln His His His His His His Leu Arg Val Pro Glu Pro Arg Pro
 1               5                  10                  15
Gly Glu Ala Lys Ala Glu Gly Ala Ala Pro Pro Thr Pro Ser Lys Pro
            20                  25                  30
Leu Thr Ser Phe Leu Ile Gln Asp Ile Leu Arg Asp Gly Ala Gln Arg
        35                  40                  45
Gln Gly Gly Arg Thr Ser Ser Gln Arg Gln Arg Asp Pro Glu Pro Glu
    50                  55                  60
Pro Glu Pro Glu Pro Glu Gly Gly Arg Ser Arg Ala Gly Ala Gln Asn
65                  70                  75                  80
Asp Gln Leu Ser Thr Gly Pro Arg Ala Ala Pro Glu Glu Ala Glu Thr
                85                  90                  95
Leu Ala Glu Thr Glu Pro Glu Arg His Leu Gly Ser Tyr Leu Leu Asp

100                 105                 110
Ser Glu Asn Thr Ser Gly Ala Leu Pro Arg Leu Pro Gln Thr Pro Lys
        115                 120                 125
Gln Pro Gln Lys Arg Ser Arg Ala Ala Phe Ser His Thr Gln Val Ile
    130                 135                 140
Glu Leu Glu Arg Lys Phe Ser His Gln Lys Tyr Leu Ser Ala Pro Glu
145                 150                 155                 160
Arg Ala His Leu Ala Lys Asn Leu Lys Leu Thr Glu Thr Gln Val Lys
                165                 170                 175
Ile Trp Phe Gln Asn Arg Arg Tyr Lys Thr Lys Arg Lys Gln Leu Ser
            180                 185                 190
Ser Glu Leu Gly Asp Leu Glu Lys His Ser Ser Leu Pro Ala Leu Lys
        195                 200                 205
Glu Glu Ala Phe Ser Arg Ala Ser Leu Val Ser Val Tyr Asn Ser Tyr
    210                 215                 220
Pro Tyr Tyr Pro Tyr Leu Tyr Cys Val Gly Ser Trp Ser Pro Ala Phe
225                 230                 235                 240
Trp

<210> 843
<211> 729
<212> DNA
<213> Homo sapiens

<400> 843
atgcagcatc accaccatca ccacctcagg gttccggagc cgcggcccgg ggaggcgaaa 60
gcggaggggg ccgcgccgcc gaccccgtcc aagccgctca cgtccttcct catccaggac 120
atcctgcggg acggcgcgca gcggcaaggc ggccgcacga gcagccagag acagcgcgac 180
ccggagccgg agccagagcc agagccagag ggaggacgca gccgcgccgg ggcgcagaac 240
gaccagctga gcaccgggcc ccgcgccgcg ccggatgagg ccgagacgct ggcagagacc 300
gagccagaaa ggcacttggg gtcttatctg ttggactctg aaaacacttc aggcgccctt 360
ccaaggcttc cccaaacccc taagcagccg cagaagcgct cccgagctgc cttctcccac 420
actcaggtga tcgagttgga gaggaagttc agccatcaga agtacctgtc ggcccctgaa 480
cgggcccacc tggccaagaa cctcaagctc acggagaccc aagtgaagat atggttccag 540
aacagacgct ataagactaa gcgaaagcag ctctcctcgg agctgggaga cttggagaag 600
cactcctttt tgccggccct gaaagaggag gccttctccc gggcctccct ggtctccgtg 660
tataacagct atccttacta cccatacctg cactgcgtgg gcagctggag cccagctttt 720
tggtaatga                                                          729

<210> 844
<211> 27
<212> DNA
<213> Artificial Sequence

<220>
<223> PCR Primer

<400> 844
ctactaagcg ctggagtgag ggatcag                                      27

<210> 845
<211> 33
<212> DNA
<213> Artificial Sequence

<220>
<223> PCR Primer

<400> 845
catcgagaat tcactactct ctgactagat gtc                               33

<210> 846
<211> 161
<212> PRT
<213> Homo sapiens

<400> 846

<210> 847
<211> 489
<212> DNA
<213> Homo sapiens

<400> 847
atgcagcatc accaccatca ccacgctgga gtgagggatc aggggcaggg cgcgagatgg 60
cctcacacag ggaagagagg gcccctcctg cagggcctca cctgggccac aggaggacac 120
tgcttttcct ctgaggagtc aggagctgtg gatggtgctg gacagaagaa ggacagggcc 180
tggctcaggt gtccagaggc tgtcgctggc ttccctttgg gatcagactg cagggaggga 240
gggcggcagg gttgtggggg gagtgacgat gaggatgacc tgggggtggc tccaggcctt 300
gcccctgcct gggccctcac ccagcctccc tcacagtctc ctggccctca gtctctcccc 360
tccactccat cctccatctg gcctcagtgg gtcattctga tcactgaact gaccataccc 420
agccctgccc acggccctcc atggctcccc aatgccctgg agaggggaca tctagtcaga 480
gagtagtga                                                          489

<210> 848
<211> 132
<212> PRT
<213> Homo sapiens

<400> 848
Thr Ala Ala Ser Asp Asn Phe Gln Leu Ser Gln Gly Gly Gln Gly Phe
1               5                   10                  15
Ala Ile Pro Ile Gly Gln Ala Met Ala Ile Ala Gly Gln Ile Arg Ser
            20                  25                  30
Gly Gly Gly Ser Pro Thr Val His Ile Gly Pro Thr Ala Phe Leu Gly
        35                  40                  45
Leu Gly Val Val Asp Asn Asn Gly Asn Gly Ala Arg Val Gln Arg Val
    50                  55                  60
Val Gly Ser Ala Pro Ala Ala Ser Leu Gly Ile Ser Thr Gly Asp Val
65                  70                  75                  80
Ile Thr Ala Val Asp Gly Ala Pro Ile Asn Ser Ala Thr Ala Met Ala
                85                  90                  95
Asp Ala Leu Asn Gly His His Pro Gly Asp Val Ile Ser Val Asn Trp
            100                 105                 110
Gln Thr Lys Ser Gly Gly Thr Arg Thr Gly Asn Val Thr Leu Ala Glu
        115                 120                 125
Gly Pro Pro Ala
    130

<210> 849

<211> 31
<212> DNA
<213> Artificial Sequence

<220>
<223> PCR Primer

<400> 849
ggggaattca tcacctatgt gccgcctctg c                                    31

<210> 850
<211> 40
<212> DNA
<213> Artificial Sequence

<220>
<223> PCR Primer

<400> 850
gggctcgagt cactcgccca cgaaatccgt gtaaaacagc                           40

<210> 851
<211> 1203
<212> DNA
<213> Homo sapiens

<400> 851
atgcatcacc atcaccatca cacggccgcg tccgataact tccagctgtc ccagggtggg 60
cagggattcg ccattccgat cgggcaggcg atggcgatcg cgggccagat caagcttccc 120
accgttcata tcgggcctac cgccttcctc ggcttgggtg ttgtcgacaa caacggcaac 180
ggcgcacgag tccaacgcgt ggtcgggagc gctccggcgg caagtctcgg catctccacc 240
ggcgacgtga tcaccgcggt cgacggcgct ccgatcaact cggccaccgc gatggcggac 300
gcgcttaacg ggcatcatcc cggtgacgtc atctcggtga cctggcaaac caagtcgggc 360
ggcacgcgta cagggaacgt gacattggcc gagggacccc cggccgaatt catcacctat 420
gtgccgcctc tgctgctgga agtgggggta gaggagaagt tcatgaccat ggtgctgggc 480
attggtccag tgctgggcct ggtctgtgtc ccgctcctag gctcagccag tgaccactgg 540
cgtggacgct atggccgccg ccggcccttc atctgggcac tgtccttggg catcctgctg 600
agcctctttc tcatcccaag ggccggctgg ctagcagggc tgctgtgccc ggatcccagg 660
cccctggagc tggcactgct catcctgggc gtggggctgc tggacttctg tggccaggtg 720
tgcttcactc cactggaggc cctgctctct gacctcttcc gggacccgga ccactgtcgc 780
caggcctact ctgtctatgc cttcatgatc agtcttgggg gctgcctggg ctacctcctg 840
cctgccattg actgggacac cagtgccctg gccccctacc tgggcaccca ggaggagtgc 900
ctctttggcc tgctcaccct catcttcctc acctgcgtag cagccacact gctggtggct 960
gaggaggcag cgctgggccc caccgagcca gcagaagggc tgtcggcccc ctccttgtcg 1020
ccccactgct gtccatgccg ggcccgcttg gctttccgga acctgggcgc cctgcttccc 1080
cggctgcacc agctgtgctg ccgcatgccc cgcaccctgc gccggctctt cgtggctgag 1140
ctgtgcagct ggatggcact catgaccttc acgctgtttt acacggattt cgtgggcgag 1200
tga                                                                   1203

<210> 852
<211> 400
<212> PRT
<213> Homo sapiens

<400> 852


<210> 853
<211> 20
<212> PRT
<213> Homo sapiens

<400> 853
Leu Leu Pro Pro Pro Pro Ala Leu Cys Gly Ala Ser Ala Cys Asp Val
1               5                   10                  15
Ser Val Arg Val
            20


<210> 854
<211> 60
<212> DNA
<213> Homo sapiens

<400> 854
ctgctcccac ctccacccgc gctctgcggg gcctctgcct gtgatgtctc cgtacgtgtg 60


<210> 855
<211> 10
<212> PRT
<213> Homo sapiens

<400> 855
Ala Ser Ala Cys Asp Val Ser Val Arg Val
1               5                   10

<210> 856
<211> 30
<212> DNA
<213> Homo sapiens

<400> 856
gcctctgcct gtgatgtctc cgtacgtgtg                                        30

<210> 857
<211> 9
<212> PRT
<213> Homo sapiens

<400> 857
Ala Ser Ala Cys Asp Val Ser Val Arg
1               5

<210> 858
<211> 9
<212> PRT
<213> Homo sapiens

<400> 858
Ser Ala Cys Asp Val Ser Val Arg Val
1               5

<210> 859
<211> 27
<212> DNA
<213> Homo sapiens

<400> 859
tctgcctgtg atgtctccgt acgtgtg                                           27

<210> 860
<211> 19
<212> PRT
<213> Homo sapiens

<400> 860
Gly Ile Gly Pro Val Leu Gly Leu Val Cys Val Pro Leu Leu Gly Ser
1               5                   10                  15
Ala Ser Asp

<210> 861
<211> 19
<212> PRT
<213> Homo sapiens

<400> 861
Val Pro Pro Leu Leu Leu Glu Val Gly Val Glu Glu Lys Phe Met Thr
1               5                   10                  15
Met Val Leu

<210> 862
<211> 19
<212> PRT
<213> Homo sapiens

<400> 862

Met Val Gln Arg Leu Trp Val Ser Arg Leu Leu Arg His Arg Lys Ala
1               5                   10                  15
Gln Leu Leu

<210> 863
<211> 57
<212> DNA
<213> Homo sapiens

<220>
<221> misc_feature
<222> 3, 9, 12, 15, 18, 21, 24, 27, 33, 36, 39, 42, 45, 48, 51, 54
<223> n = A,T,C or G

<400> 863
ggnathggnc cngtnytngg nytngtntgy gtnccnytny tnggnwsngc nwsngay     57

<210> 864
<211> 57
<212> DNA
<213> Homo sapiens

<220>
<221> misc_feature
<222> 3, 6, 9, 12, 15, 18, 24, 27, 30, 48, 54, 57
<223> n = A,T,C or G

<400> 864
gtnccnccny tnytnytnga rgtnggngtn gargaraart tyatgacnat ggtnytn     57

<210> 865
<211> 57
<212> DNA
<213> Homo sapiens

<220>
<221> misc_feature
<222> 6, 12, 15, 21, 24, 27, 30, 33, 36, 42, 48, 54, 57
<223> n = A,T,C or G

<400> 865
atggtncarm gnytntgggt nwsnmgnytn ytnmgncaym gnaargcnca rytnytn     57

<210> 866
<211> 9
<212> PRT
<213> Homo sapiens

<400> 866
Val Leu Gln Cys Val Asn Val Ser Val
1               5

<210> 867
<211> 9
<212> PRT
<213> Homo sapiens

<400> 867
Arg Met Pro Thr Val Leu Gln Cys Val
1               5

<210> 868
<211> 9
<212> PRT

<213> Homo sapiens

<400> 868
Asn Leu Cys Lys Phe Thr Glu Trp Ile
1               5


<210> 869
<211> 9
<212> PRT
<213> Homo sapiens

<400> 869
Met Leu Ile Lys Leu Asp Glu Ser Val
1               5


<210> 870
<211> 9
<212> PRT
<213> Homo sapiens

<400> 870
Leu Leu Ala Asn Asp Leu Met Leu Ile
1               5


<210> 871
<211> 10
<212> PRT
<213> Homo sapiens

<400> 871
Leu Leu Ala Asn Gly Arg Met Pro Thr Val
1               5                   10


<210> 872
<211> 10
<212> PRT
<213> Homo sapiens

<400> 872
Leu Met Leu Ile Lys Leu Asp Glu Ser Val
1               5                   10


<210> 873
<211> 10
<212> PRT
<213> Homo sapiens

<400> 873
Val Leu Gln Cys Val Asn Val Ser Val Val
1               5                   10


<210> 874
<211> 10
<212> PRT
<213> Homo sapiens

<400> 874
Gly Leu Leu Ala Asn Gly Arg Met Pro Thr
1               5                   10


<210> 875

<211> 10
<212> PRT
<213> Homo sapiens

<400> 875
Thr Val Leu Gln Cys Val Asn Val Ser Val
1               5                   10


<210> 876
<211> 9
<212> PRT
<213> Homo sapiens

<400> 876
Gly Val Leu Val His Pro Gln Trp Val
1               5


<210> 877
<211> 9
<212> PRT
<213> Homo sapiens

<400> 877
Val Leu Val His Pro Gln Trp Val Leu
1               5


<210> 878
<211> 1195
<212> DNA
<213> Homo sapiens

<400> 878
ccgagactca cggtcaagct aaggcgaaga gtgggtggct gaagccatac tattttatag 60
aattaatgga aagcagaaaa gacatcacaa accaagaaga actttggaaa atgaagccta 120
ggagaaattt agaagaagac gattatttgc ataaggacac gggagagacc agcatgctaa 180
aaagacctgt gcttttgcat ttgcaccaaa cagcccatgc tgatgaattt gactgccctt 240
cagaacttca gcacacacag gaactctttc cacagtggca cttgccaatt aaaatagctg 300
ctattatagc atctctgact tttctttaca ctcttctgag ggaagtaatt caccctttag 360
caacttccca tcaacaatat ttttataaaa ttccaatcct ggtcatcaac aaagtcttgc 420
caatggtttc catcactctc ttggcattgg tttacctgcc aggtgtgata gcagcaattg 480
tccaacttca taatggaacc aagtataaga agtttccaca ttggttggat aagtggatgt 540
taacaagaaa gcagtttggg cttctcagtt tcttttttgc tgtactgcat gcaatttata 600
gtctgtctta cccaatgagg cgatcctaca gatacaagtt gctaaactgg gcatatcaac 660
aggtccaaca aaataaagaa gatgcctgga ttgagcatga tgtttggaga atggagattt 720
atgtgtctct gggaattgtg ggattggcaa tactggctct gttggctgtg acatctattc 780
catctgtgag tgactctttg acatggagag aatttcacta tattcagagc aagctaggaa 840
ttgtttccct tctactgggc acaatacacg cattgatttt tgcctggaat aagtggatag 900
atataaaaca atttgtatgg tatacacctc caacttttat gatagctgtt ttccttccaa 960
ttgttgtcct gatatttaaa agcatactat tcctgccatg cttgaggaag aagatactga 1020
agattagaca tggttgggaa gacgtcacca aaattaacaa aactgagata tgttcccagt 1080
tgtagaatta ctgtttacac acatttttgt tcaatattga tatattttat caccaacatt 1140
tcaagtttgt atttgttaat aaaatgatta ttcaaggaaa aaaaaaaaaa aaaaa 1195

<210> 879
<211> 339
<212> PRT
<213> Homo sapiens

<400> 879
Met Glu Ser Arg Lys Asp Ile Thr Asn Gln Glu Glu Leu Trp Lys Met
1               5                   10                  15
Lys Pro Arg Arg Asn Leu Glu Glu Asp Asp Tyr Leu His Lys Asp Thr
            20                  25                  30
Gly Glu Thr Ser Met Leu Lys Arg Pro Val Leu Leu His Leu His Gln
        35                  40                  45

Thr Ala His Ala Asp Glu Phe Asp Cys Pro Ser Glu Leu Gln His Thr
    50                  55                  60
Gln Glu Leu Phe Pro Gln Trp His Leu Pro Ile Lys Ile Ala Ala Ile
65                  70                  75                  80
Ile Ala Ser Leu Thr Phe Leu Tyr Thr Leu Leu Arg Glu Val Ile His
                85                  90                  95
Pro Leu Ala Thr Ser His Gln Gln Tyr Phe Tyr Lys Ile Pro Ile Leu
            100                 105                 110
Val Ile Asn Lys Val Leu Pro Met Val Ser Ile Thr Leu Leu Ala Leu
        115                 120                 125
Val Tyr Leu Pro Gly Val Ile Ala Ala Ile Val Gln Leu His Asn Gly
    130                 135                 140
Thr Lys Tyr Lys Lys Phe Pro His Trp Leu Asp Lys Trp Met Leu Thr
145                 150                 155                 160
Arg Lys Gln Phe Gly Leu Leu Ser Phe Phe Phe Ala Val Leu His Ala
                165                 170                 175
Ile Tyr Ser Leu Ser Tyr Pro Met Arg Arg Ser Tyr Arg Tyr Lys Leu
            180                 185                 190
Leu Asn Trp Ala Tyr Gln Gln Val Gln Gln Asn Lys Glu Asp Ala Trp
        195                 200                 205
Ile Glu His Asp Val Trp Arg Met Glu Ile Tyr Val Ser Leu Gly Ile
    210                 215                 220
Val Gly Leu Ala Ile Leu Ala Leu Leu Ala Val Thr Ser Ile Pro Ser
225                 230                 235                 240
Val Ser Asp Ser Leu Thr Trp Arg Glu Phe His Tyr Ile Gln Ser Lys
                245                 250                 255
Leu Gly Ile Val Ser Leu Leu Leu Gly Thr Ile His Ala Leu Ile Phe
            260                 265                 270
Ala Trp Asn Lys Trp Ile Asp Ile Lys Gln Phe Val Trp Tyr Thr Pro
        275                 280                 285
Pro Thr Phe Met Ile Ala Val Phe Leu Pro Ile Val Val Leu Ile Phe
    290                 295                 300
Lys Ser Ile Leu Phe Leu Pro Cys Leu Arg Lys Lys Ile Leu Lys Ile
305                 310                 315                 320
Arg His Gly Trp Glu Asp Val Thr Lys Ile Asn Lys Thr Glu Ile Cys
                325                 330                 335
Ser Gln Leu

<210> 880
<211> 2172
<212> DNA
<213> Homo sapiens

<400> 880
   aaaattgaat attgagatac cattctttag tgttaccttt tttacccaca tgtgtttctg 60
   aaaatattgg aattttattc atcttaaaaa ttggacccgg ccttatttac catctttaat 120
   ccattttagt actatgggtg agtacatgga attgaagtct ggcttaaatc ttcagaaagt 180
   tatatatcta ttttatttta tttttttgag acagagtctc gctgtgtcac ccaggctgga 240
   gtgcggtgcc acaatcttgg ctcactgcaa cctctgagtc ccaggttcaa gcgatactca 300
   tgcctcggcc tcctgagtag ctgggactac aggcgtgcac caccacatct ggctaatctt 360
   tttttgtatt tttagtagag acggggtttc actgtggtct ccatctcctg acctcgtgat 420
   ccgcctgcct cccaaagtgc tgggattaca ggcatgagcc accgcacaca gctgggactg 480
   ggtaatttat aaagaaaaga ggtttaatga ctcacagttc cgcatggctg gagaggcctc 540
   aggaaactta caatcatggt ggaaggcgaa ggggaagcaa ggcacgtctt acatggtggc 600
   aggagagaac gagtgagggg ggagactgcc acaaactttt tttttttgag acaagagtct 660
   ggccctgttg cccaggctgg agtgcagtgg catgatctca gctcactgca acctctgcct 720
   cacaggttca agcaattctc atgcctcagc ctcccgcata gctgggacca caggtatgca 780
   ccaccacacc tagctaattt ttgtagtttt agtagagatg gggtctcact atgttgctca 840
   ggctggtcta aaactcctgg gctccagcaa tccgcctgcc ttggcctccc aaagtgctgg 900
   ggttacaggc ataagccacc acatccagcc tgccacatac ttttaaacta tcaggtctca 960
   tgagaactca tgcactatca caagaatagc atggggaaaa tcccccccat aatccaatca 1020
   cctcccacca ggtctcctcc gacacgtggg attgggtggg gacacagagc caaaccgtat 1080
   cagatgctgc aggggctggg gacactgaga ccactcagac ctggtgtctc tgtcactctt 1140
   ctgggctctg tctgtctcca ggacctccct ccccttccat ggtatagaag gaaagtgctg 1200
   taaggtgcaa attgcacagg aactccttaa gacatacatc atccactcag cagttttagg 1260
   ttcgcagcaa aatggagtgg aaggaacaga aatttcctgt gcacccctcc ccgctgtctc 1320
   cgccatatcg gcatcctgca tccagagtgg tggactggtt acaggctatg aacctacact 1380
   gatgcggcac caccacccag agtccacggg ttatgttggt tcacatttac tcttgctgtg 1440
   gtatggtcta taggtttgga cagatgtccg ataatccttt ttacattttg gcatccttgg 1500
   gtagctcgtc ttgtaggaat ggacttgctt caaagtggag gcaggcagat ccttcagacg 1560
   ggtatatgga gccctgtttt cagttgcttt tctaattctc tcttatcgtt tacctcaaaa 1620
   tcttcctgag gtctcgcttc cttttaaaat ccttgtctac tttgcagcat cactctgaca 1680
   ctccattgat tcctcagcac ctactgacta cacggttagg agtgcaaggg tagaattcat 1740
   gttttattca tctttgggtc tgtagcaccc agcaaagtgc tcagtaaatg cgcagtaatt 1800
   gatttgacct ctgaacaaat acacactgta ctaagaatct acacaccgaa agacaaaaac 1860
   aagacaaatt tgagtgctac aggtgtcacg cttggcatca cacatgtgcc tgtgtattcc 1920
   tctaggtggt taccaggagc tctgccactg catgtccact agtgacgggt tcgctccacc 1980
   accccagctg ggtagccgct gctctcacat aaggggtcca attaaaattg ccaggaataa 2040
   attcccccgg actttgactt ctcaagagct aagaaggttt gctgagtatt ctggcatgat 2100
   gtttggtgat caaacaactg ctggccaaaa atgatgagta tttccccctc ttgctgaaga 2160
   tgtgctccat ac 2172

<210> 881
<211> 2455
<212> DNA
<213> Homo sapiens

<400> 881
cagcttaaaa atggtttctt gaaatcagtg attagcattc actcaccagt acccctacta 60
aggggtaggc actggtttgt actcctggga atacaggagt acaccagaat ttatttctgc 120
ttattgcttt tgttgcaaat gccgtggctt catctgagga attctagaat tcagagggtg 180
tagccctcca ctctgctgtc ttgctatctg ctctcattgc atccgtttaa cctgcattct 240
gaaagatgtt tctcaggttt ttccttgacg attttcttct tttctgattc tgacaatgtt 300
ttaaatcatt gtactgtggt tatcatttct ctgcatttat tttacccatc ttcctttgta 360
acttgtccta ttgtctttta atttctgcct gttctttatg gctttcaact tcataaataa 420
catgttttct caaatctctt tgtgaattcc agagagggcc aggcacggtg gctcacatct 480
gtaatcccag cactttgggg aggctgagac gggtggatca cttgaggtca ggagtttgag 540
accagcctgg ccaacatggt gaaatcccgt ttcactaaaa atacaaaaat tacccaggca 600
tggtggcggg cgcctgtaat cccaggtact cgggaggctg agggaggaga atcgcttgaa 660
cctgggaggc tgagggagga gaatcgcttg aacccgggag gcagaggttg cagtgaaccg 720
agatcatgtt gctgcactcc agcctggtca acagagcaag actctgcctc aaaaacaaac 780
aaataaacaa acaaacaaac aaaacagaga gattttgctg caatgtacaa ggagcaattt 840
gctcctttaa aaaaataatt tttggccagg cacagtggct cacacctgta atcccagcac 900
tttgggaagc caaggtgggt ggatcatttg aggtcaggag tttgagatca gcctggccaa 960
catggtgaaa cactatctct attaaaaata caaaaatgtg ctcagtgtgg tggtgcacat 1020
ctgtaatctc agcctcccgc atagctggga ccacaggtat gcaccaccac acctagctaa 1080
tttttgtagt tttagtagag atggggtctc actatgttgc tcaggctggt ctaaaactcc 1140
tgggctccag caatccgcct gccttggcct cccaaagtgc tggggttaca ggcataagcc 1200
accacatcca gcctgccaca tacttttaaa ctatcaggtc tcatgagaac tcatgcacta 1260
tcacaagaat agcatgggga aaatcccccc cataatccaa tcacctccca ccaggtctcc 1320
tccgacacgt gggattgggt ggggacacag agccaaaccg tatcagatgc tgcaggggct 1380
ggggacactg agaccactca gacctggtgt ctctgtcact cttctgggct ctgtctgtct 1440
ccaggacctc cctccccttc catggtatag aaggaaagtg ctgtaaggtg caaattgcac 1500
aggaactcct taagacatac atcatccact cagcagtttt aggttcgcag caaaatggag 1560
tggaaggaac agaaatttcc tgtgcacccc tccccgctgt ctccgccata tcggcatcct 1620
gcatccagag tggtggactg gttacaggct atgaacctac actgatgcgg caccaccacc 1680
cagagtccac aggttatgtt ggttcacatt tactcttgct gtggtatggt ctataggttt 1740
ggacagatgt ccgataatcc tttttacatt ttggcatcct tgggtagctc gtcttgtagg 1800
aatggacttg cttcaaagtg gaggcaggca gatccttcag acgggtatat ggagccctgt 1860
tttcagttgc ttttctaatt ctctcttatc gtttacctca aaatcttcct gaggtctcgc 1920
ttccttttaa aatccttgtc tactttgcag catcactctg acactccatt gattcctcag 1980
cacctactga ctacacggtt aggagtgcaa gggtagaatt catgttttat tcatctttgg 2040
gtctgtagca cccagcaaag tgctcagtaa atgcgcagta attgatttga cctctgaaca 2100
aatacacact gtactaagaa tctacacacc gaaagacaaa aacaagacaa atttgagtgc 2160
tacaggtgtc acgcttggca tcacacatgt gcctgtgtat tcctctaggt ggttaccagg 2220
agctctgcca ctgcatgtcc actagtgacg ggttcgctcc accaccccag ctgggtagcc 2280
gctgctctca cataaggggt ccaattaaaa ttgccaggaa taaattcccc cggactttga 2340
cttctcaaga gctaagaagg tttgctgagt attctggcat gatgtttggt gatcaaacaa 2400
ctgctggcca aaaatgatga gtatttcccc ctcttgctga agatgtgctc catac 2455

<210> 882
<211> 2455
<212> DNA
<213> Homo sapiens

<400> 882
cagcttaaaa atggtttctt gaaatcagtg attagcattc actcaccagt acccctacta 60
aggggtaggc actggtttgt actcctggga atacaggagt acaccagaat ttatttctgc 120
ttattgcttt tgttgcaaat gccgtggctt catctgagga attctagaat tcagagggtg 180
tagccctcca ctctgctgtc ttgctatctg ctctcattgc atccgtttaa cctgcattct 240
gaaagatgtt tctcaggttt ttccttgacg attttcttct tttctgattc tgacaatgtt 300
ttaaatcatt gtactgtggt tatcatttct ctgcatttat tttacccatc ttcctttgta 360
acttgtccta ttgtctttta atttctgcct gttctttatg gctttcaact tcataaataa 420
catgttttct caaatctctt tgtgaattcc agagagggcc aggcacggtg gctcacatct 480
gtaatcccag cactttgggg aggctgagac gggtggatca cttgaggtca ggagtttgag 540
accagcctgg ccaacatggt gaaatcccgt ttcactaaaa atacaaaaat tacccaggca 600
tggtggcggg cgcctgtaat cccaggtact cgggaggctg agggaggaga atcgcttgaa 660
cctgggaggc tgagggagga gaatcgcttg aacccgggag gcagaggttg cagtgaaccg 720
agatcatgtt gctgcactcc agcctggtca acagagcaag actctgcctc aaaaacaaac 780
aaataaacaa acaaacaaac aaaacagaga gattttgctg caatgtacaa ggagcaattt 840
gctcctttaa aaaaataatt tttggccagg cacagtggct cacacctgta atcccagcac 900
tttgggaagc caaggtgggt ggatcatttg aggtcaggag tttgagatca gcctggccaa 960
catggtgaaa cactatctct attaaaaata caaaaatgtg ctcagtgtgg tggtgcacat 1020
ctgtaatctc agcctcccgc atagctggga ccacaggtat gcaccaccac acctagctaa 1080
tttttgtagt tttagtagag atggggtctc actatgttgc tcaggctggt ctaaaactcc 1140
tgggctccag caatccgcct gccttggcct cccaaagtgc tggggttaca ggcataagcc 1200
accacatcca gcctgccaca tacttttaaa ctatcaggtc tcatgagaac tcatgcacta 1260
tcacaagaat agcatgggga aaatcccccc cataatccaa tcacctccca ccaggtctcc 1320
tccgacacgt gggattgggt ggggacacag agccaaaccg tatcagatgc tgcaggggct 1380
ggggacactg agaccactca gacctggtgt ctctgtcact cttctgggct ctgtctgtct 1440
ccaggacctc cctccccttc catggtatag aaggaaagtg ctgtaaggtg caaattgcac 1500
aggaactcct taagacatac atcatccact cagcagtttt aggttcgcag caaaatggag 1560
tggaaggaac agaaatttcc tgtgcacccc tccccgctgt ctccgccata tcggcatcct 1620
gcatccagag tggtggactg gttacaggct atgaacctac actgatgcgg caccaccacc 1680
cagagtccac aggttatgtt ggttcacatt tactcttgct gtggtatggt ctataggttt 1740
ggacagatgt ccgataatcc tttttacatt ttggcatcct tgggtagctc gtcttgtagg 1800
aatggacttg cttcaaagtg gaggcaggca gatccttcag acgggtatat ggagccctgt 1860
tttcagttgc ttttctaatt ctctcttatc gtttacctca aaatcttcct gaggtctcgc 1920
ttccttttaa aatccttgtc tactttgcag catcactctg acactccatt gattcctcag 1980
cacctactga ctacacggtt aggagtgcaa gggtagaatt catgttttat tcatctttgg 2040
gtctgtagca cccagcaaag tgctcagtaa atgcgcagta attgatttga cctctgaaca 2100
aatacacact gtactaagaa tctacacacc gaaagacaaa aacaagacaa atttgagtgc 2160
tacaggtgtc acgcttggca tcacacatgt gcctgtgtat tcctctaggt ggttaccagg 2220
agctctgcca ctgcatgtcc actagtgacg ggttcgctcc accaccccag ctgggtagcc 2280
gctgctctca cataaggggt ccaattaaaa ttgccaggaa taaattcccc cggactttga 2340
cttctcaaga gctaagaagg tttgctgagt attctggcat gatgtttggt gatcaaacaa 2400
ctgctggcca aaaatgatga gtatttcccc ctcttgctga agatgtgctc catac 2455

<210> 883
<211> 62
<212> PRT
<213> Homo sapiens

<400> 883
Met Thr His Ser Ser Ala Trp Leu Glu Arg Pro Gln Glu Thr Tyr Asn
1               5                   10                  15
His Gly Gly Arg Arg Arg Gly Ser Lys Ala Arg Leu Thr Trp Trp Gln
            20                  25                  30
Glu Arg Thr Ser Glu Gly Gly Asp Cys His Lys Leu Phe Phe Phe Glu
        35                  40                  45
Thr Arg Val Trp Pro Cys Cys Pro Gly Trp Ser Ala Val Ala
    50                  55                  60

<210> 884
<211> 135
<212> PRT
<213> Homo sapiens

<400> 884

<210> 885
<211> 77
<212> PRT
<213> Homo sapiens

<400> 885
Met His Tyr His Lys Asn Ser Met Gly Lys Ile Pro Pro Ile Ile Gln
 1               5                  10                  15
Ser Pro Pro Thr Arg Ser Pro Pro Thr Arg Gly Ile Gly Trp Gly His
            20                  25                  30
Arg Ala Lys Pro Tyr Gln Met Leu Gln Gly Leu Gly Thr Leu Arg Pro
        35                  40                  45
Leu Arg Pro Gly Val Ser Val Thr Leu Leu Gly Ser Val Cys Leu Gln
    50                  55                  60
Asp Leu Pro Pro Leu Pro Trp Tyr Arg Arg Lys Val Leu
65                  70                  75

<210> 886
<211> 60
<212> PRT
<213> Homo sapiens

<400> 886
Met Leu Val His Ile Tyr Ser Cys Cys Gly Met Val Tyr Arg Phe Gly
 1               5                  10                  15
Gln Met Ser Asp Asn Pro Phe Tyr Ile Leu Ala Ser Leu Gly Ser Ser
            20                  25                  30
Ser Cys Arg Asn Gly Leu Ala Ser Lys Trp Arg Gln Ala Asp Pro Ser
        35                  40                  45
Asp Gly Tyr Met Glu Pro Cys Phe Gln Leu Leu Phe
    50                  55                  60

<210> 887
<211> 76
<212> PRT
<213> Homo sapiens

<400> 887
Met Cys Leu Cys Ile Pro Leu Gly Gly Tyr Gln Glu Leu Cys His Cys
 1               5                  10                  15
Met Ser Thr Ser Asp Gly Phe Ala Pro Pro Pro Gln Leu Gly Ser Arg
            20                  25                  30
Cys Ser His Ile Arg Gly Pro Ile Lys Ile Ala Arg Asn Lys Phe Pro
        35                  40                  45
Arg Thr Leu Thr Ser Gln Glu Leu Arg Arg Phe Ala Glu Tyr Ser Gly
    50                  55                  60
Met Met Phe Gly Asp Gln Thr Thr Ala Gly Gln Lys
65                  70                  75

<210> 888
<211> 76
<212> PRT
<213> Homo sapiens

<400> 888
Met Val Lys Ser Arg Phe Thr Lys Asn Thr Lys Ile Thr Gln Ala Trp
1               5                   10                  15
Trp Arg Ala Pro Val Ile Pro Gly Thr Arg Glu Ala Glu Gly Gly Glu
            20                  25                  30
Ser Leu Glu Pro Gly Arg Leu Arg Glu Glu Asn Arg Leu Asn Pro Gly
        35                  40                  45
Gly Arg Gly Cys Ser Glu Pro Arg Ser Cys Cys Cys Thr Pro Ala Trp
    50                  55                  60
Ser Thr Glu Gln Asp Ser Ala Ser Lys Thr Asn Lys
65                  70                  75


<210> 889
<211> 80
<212> PRT
<213> Homo sapiens

<400> 889


<210> 890
<211> 72
<212> PRT
<213> Homo sapiens

<400> 890
Met His Tyr His Lys Asn Ser Met Gly Lys Ile Pro Pro His Asn Pro
1               5                   10                  15
Ile Thr Ser His Gln Val Ser Ser Asp Thr Trp Asp Trp Val Gly Thr
            20                  25                  30
Gln Ser Gln Thr Val Ser Asp Ala Ala Gly Ala Gly Asp Thr Glu Thr
        35                  40                  45
Thr Gln Thr Trp Cys Leu Cys His Ser Ser Gly Leu Cys Leu Ser Pro
    50                  55                  60
Gly Pro Pro Ser Pro Ser Met Val
65                  70


<210> 891
<211> 77
<212> PRT
<213> Homo sapiens

<400> 891
Met His Tyr His Lys Asn Ser Met Gly Lys Ile Pro Pro Ile Ile Gln
1               5                   10                  15
Ser Pro Pro Thr Arg Ser Pro Pro Thr Arg Gly Ile Gly Trp Gly His
            20                  25                  30
Arg Ala Lys Pro Tyr Gln Met Leu Gln Gly Leu Gly Thr Leu Arg Pro
        35                  40                  45

Leu Arg Pro Gly Val Ser Val Thr Leu Leu Gly Ser Val Cys Leu Gln
    50                  55                  60
Asp Leu Pro Pro Leu Pro Trp Tyr Arg Arg Lys Val Leu
65                  70                  75

<210> 892
<211> 60
<212> PRT
<213> Homo sapiens

<400> 892
Met Leu Val His Ile Tyr Ser Cys Cys Gly Met Val Tyr Arg Phe Gly
 1               5                  10                  15
Gln Met Ser Asp Asn Pro Phe Tyr Ile Leu Ala Ser Leu Gly Ser Ser
            20                  25                  30
Ser Cys Arg Asn Gly Leu Ala Ser Lys Trp Arg Gln Ala Asp Pro Ser
        35                  40                  45
Asp Gly Tyr Met Glu Pro Cys Phe Gln Leu Leu Phe
    50                  55                  60

<210> 893
<211> 76
<212> PRT
<213> Homo sapiens

<400> 893
Met Cys Leu Cys Ile Pro Leu Gly Gly Tyr Gln Glu Leu Cys His Cys
 1               5                  10                  15
Met Ser Thr Ser Asp Gly Phe Ala Pro Pro Pro Gln Leu Gly Ser Arg
            20                  25                  30
Cys Ser His Ile Arg Gly Pro Ile Lys Ile Ala Arg Asn Lys Phe Pro
        35                  40                  45
Arg Thr Leu Thr Ser Gln Glu Leu Arg Arg Phe Ala Glu Tyr Ser Gly
    50                  55                  60
Met Met Phe Gly Asp Gln Thr Thr Ala Gly Gln Lys
65                  70                  75

<210> 894
<211> 2479
<212> DNA
<213> Homo sapiens

<400> 894
gtcatattga acattccaga tacctatcat tactcgatgc tgttgataac agcaagatgg 60
ctttgaactc agggtcacca ccagctattg gaccttacta tgaaaaccat ggataccaac 120
cggaaaaccc ctatcccgca cagcccactg tggtccccac tgtctacgag gtgcatccgg 180
ctcagtacta cccgtccccc gtgccccagt acgccccgag ggtcctgacg caggcttcca 240
accccgtcgt ctgcacgcag cccaaatccc catccgggac agtgtgcacc tcaaagacta 300
agaaagcact gtgcatcacc ttgaccctgg ggaccttcct cgtgggagct gcgctggccg 360
ctggcctact ctggaagttc atgggcagca agtgctccaa ctctgggata gagtgcgact 420
cctcaggtac ctgcatcaac ccctctaact ggtgtgatgg cgtgtcacac tgccccggcg 480
gggaggacga gaatcggtgt gttcgcctct acggaccaaa cttcatcctt cagatgtact 540
catctcagag gaagtcctgg caccctgtgt gccaagacga ctggaacgag aactacgggc 600
gggcggcctg cagggacatg ggctataaga ataattttta ctctagccaa ggaatagtgg 660
atgacagcgg atccaccagc tttatgaaac tgaacacaag tgccggcaat gtcgatatct 720
ataaaaaact gtaccacagt gatgcctgtt cttcaaaagc agtggtttct ttacgctgtt 780
tagcctgcgg ggtcaacttg aactcaagcc gccagagcag gatcgtgggc ggtgagagcg 840
cgctcccggg ggcctggccc tggcaggtca gcctgcacgt ccagaacgtc cacgtgtgcg 900
gaggctccat catcaccccc gagtggatcg tgacagccgc ccactgcgtg gaaaaacctc 960
ttaacaatcc atggcattgg acggcatttg cggggatttt gagacaatct ttcatgttct 1020
atggagccgg ataccaagta caaaaagtga tttctcatcc aaattatgac tccaagacca 1080
agaacaatga cattgcgctg atgaagctgc agaagcctct gactttcaac gacctagtga 1140
aaccagtgtg tctgcccaac ccaggcatga tgctgcagcc agaacagctc tgctggattt 1200
ccgggtgggg ggccaccgag gagaaaggga agacctcaga agtgctgaac gctgccaagg 1260
tgcttctcat tgagacacag agatgcaaca gcagatatgt ctatgacaac ctgatcacac 1320

cagccatgat ctgtgccggc ttcctgcagg ggaacgtcga ttcttgccag ggtgacagtg 1380
gagggcctct ggtcacttcg aacaacaata tctggtggct gataggggat acaagctggg 1440
gttctggctg tgccaaagct tacagaccag gagtgtacgg gaatgtgatg gtattcacgg 1500
actggattta tcgacaaatg aaggcaaacg gctaatccac atggtcttcg tccttgacgt 1560
cgttttacaa gaaaacaatg gggctggttt tgcttccccg tgcatgattt actcttagag 1620
atgattcaga ggtcacttca tttttattaa acagtgaact tgtctggctt tggcactctc 1680
tgccatactg tgcaggctgc agtggctccc ctgcccagcc tgctctccct aaccccttgt 1740
ccgcaagggg tgatggccgg ctggttgtgg gcactggcgg tcaattgtgg aaggaagagg 1800
gttggaggct gcccccattg agatcttcct gctgagtcct ttccaggggc caattttgga 1860
tgagcatgga gctgtcactt ctcagctgct ggatgacttg agatgaaaaa ggagagacat 1920
ggaaagggag acagccaggt ggcacctgca gcggctgccc tctggggcca cttggtagtg 1980
tccccagcct acttcacaag gggattttgc tgatgggttc ttagagcctt agcagccctg 2040
gatggtggcc agaaataaag ggaccagccc ttcatgggtg gtgacgtggt agtcacttgt 2100
aaggggaaca gaaacatttt tgttcttatg gggtgagaat atagacagtg cccttggtgc 2160
gagggaagca attgaaaagg aacttgccct gagcactcct ggtgcaggtc tccacctgca 2220
cattgggtgg ggctcctggg agggagactc agccttcctc ctcatcctcc ctgaccctgc 2280
tcctagcacc ctggagagtg aatgcccctt ggtccctggc agggcgccaa gtttggcacc 2340
atgtcggcct cttcaggcct gatagtcatt ggaaattgag gtccatgggg gaaatcaagg 2400
atgctcagtt taaggtacac tgtttccatg ttatgtttct acacattgat ggtggtgacc 2460
ctgagttcaa agccatctt 2479

<210> 895
<211> 492
<212> PRT
<213> Homo sapiens

<400> 895
Met Ala Leu Asn Ser Gly Ser Pro Pro Ala Ile Gly Pro Tyr Tyr Glu
1               5                   10                  15
Asn His Gly Tyr Gln Pro Glu Asn Pro Tyr Pro Ala Gln Pro Thr Val
            20                  25                  30
Val Pro Thr Val Tyr Glu Val His Pro Ala Gln Tyr Tyr Pro Ser Pro
        35                  40                  45
Val Pro Gln Tyr Ala Pro Arg Val Leu Thr Gln Ala Ser Asn Pro Val
    50                  55                  60
Val Cys Thr Gln Pro Lys Ser Pro Ser Gly Thr Val Cys Thr Ser Lys
65                  70                  75                  80
Thr Lys Lys Ala Leu Cys Ile Thr Leu Thr Leu Gly Thr Phe Leu Val
                85                  90                  95
Gly Ala Ala Leu Ala Ala Gly Leu Leu Trp Lys Phe Met Gly Ser Lys
            100                 105                 110
Cys Ser Asn Ser Gly Ile Glu Cys Asp Ser Ser Gly Thr Cys Ile Asn
        115                 120                 125
Pro Ser Asn Trp Cys Asp Gly Val Ser His Cys Pro Gly Gly Glu Asp
    130                 135                 140
Glu Asn Arg Cys Val Arg Leu Tyr Gly Pro Asn Phe Ile Leu Gln Met
145                 150                 155                 160
Tyr Ser Ser Gln Arg Lys Ser Trp His Pro Val Cys Gln Asp Asp Trp
                165                 170                 175
Asn Glu Asn Tyr Gly Arg Ala Ala Cys Arg Asp Met Gly Tyr Lys Asn
            180                 185                 190
Asn Phe Tyr Ser Ser Gln Gly Ile Val Asp Asp Ser Gly Ser Thr Ser
        195                 200                 205
Phe Met Lys Leu Asn Thr Ser Ala Gly Asn Val Asp Ile Tyr Lys Lys
    210                 215                 220
Leu Tyr His Ser Asp Ala Cys Ser Ser Lys Ala Val Val Ser Leu Arg
225                 230                 235                 240
Cys Leu Ala Cys Gly Val Asn Leu Asn Ser Ser Arg Gln Ser Arg Ile
                245                 250                 255
Val Gly Gly Glu Ser Ala Leu Pro Gly Ala Trp Pro Trp Gln Val Ser
            260                 265                 270
Leu His Val Gln Asn Val His Val Cys Gly Gly Ser Ile Ile Thr Pro
        275                 280                 285
Glu Trp Ile Val Thr Ala Ala His Cys Val Glu Lys Pro Leu Asn Asn
    290                 295                 300
Pro Trp His Trp Thr Ala Phe Ala Gly Ile Leu Arg Gln Ser Phe Met
305                 310                 315                 320
Phe Tyr Gly Ala Gly Tyr Gln Val Gln Lys Val Ile Ser His Pro Asn

                325                 330                 335
Tyr Asp Ser Lys Thr Lys Asn Asn Asp Ile Ala Leu Met Lys Leu Gln
            340                 345                 350
Lys Pro Leu Thr Phe Asn Asp Leu Val Lys Pro Val Cys Leu Pro Asn
        355                 360                 365
Pro Gly Met Met Leu Gln Pro Glu Gln Leu Cys Trp Ile Ser Gly Trp
    370                 375                 380
Gly Ala Thr Glu Glu Lys Gly Lys Thr Ser Glu Val Leu Asn Ala Ala
385                 390                 395                 400
Lys Val Leu Leu Ile Glu Thr Gln Arg Cys Asn Ser Arg Tyr Val Tyr
                405                 410                 415
Asp Asn Leu Ile Thr Pro Ala Met Ile Cys Ala Gly Phe Leu Gln Gly
            420                 425                 430
Asn Val Asp Ser Cys Gln Gly Asp Ser Gly Gly Pro Leu Val Thr Ser
        435                 440                 445
Asn Asn Asn Ile Trp Trp Leu Ile Gly Asp Thr Ser Trp Gly Ser Gly
    450                 455                 460
Cys Ala Lys Ala Tyr Arg Pro Gly Val Tyr Gly Asn Val Met Val Phe
465                 470                 475                 480
Thr Asp Trp Ile Tyr Arg Gln Met Lys Ala Asn Gly
                485                 490

<210> 896
<211> 683
<212> DNA
<213> Homo sapiens

<400> 896
gtcatattga acattccaga tacctatcat tactcgatgc tgttgataac agcaagatgg 60
ctttgaactc agggtcacca ccagctattg gaccttacta tgaaaaccat ggataccaac 120
cggaaaaccc ctatcccgca cagcccactg tggtccccac tgtctacgag gtgcatccgg 180
ctcagtacta cccgtccccc gtgccccagt acgccccgag ggtcctgacg caggcttcca 240
accccgtcgt ctgcacgcag cccaaatccc catccgggac agtgtgcacc tcaaagacta 300
agaaagcact gtgcatcacc ttgaccctgg ggaccttcct cgtgggagct gcgctggccg 360
ctggcctact ctggaagttc atgggcagca agtgctccaa ctctgggata gagtgcgact 420
cctcaggtac ctgcatcaac ccctctaact ggtgtgatgg cgtgtcacac tgccccggcg 480
gggaggacga gaatcggtgt gttcgcctct acggaccaaa cttcatcctt cagatgtact 540
catctcagag gaagtcctgg caccctgtgt gccaagacga ctggaacgag aactacgggc 600
gggcggcctg cagggacatg ggctataaga ataattttta ctctagccaa ggaatagtgg 660
atgacagcgg atccaccagc ttt 683

<210> 897
<211> 209
<212> PRT
<213> Homo sapiens

<400> 897
Met Ala Leu Asn Ser Gly Ser Pro Pro Ala Ile Gly Pro Tyr Tyr Glu
1               5                   10                  15
Asn His Gly Tyr Gln Pro Glu Asn Pro Tyr Pro Ala Gln Pro Thr Val
            20                  25                  30
Val Pro Thr Val Tyr Glu Val His Pro Ala Gln Tyr Tyr Pro Ser Pro
        35                  40                  45
Val Pro Gln Tyr Ala Pro Arg Val Leu Thr Gln Ala Ser Asn Pro Val
    50                  55                  60
Val Cys Thr Gln Pro Lys Ser Pro Ser Gly Thr Val Cys Thr Ser Lys
65                  70                  75                  80
Thr Lys Lys Ala Leu Cys Ile Thr Leu Thr Leu Gly Thr Phe Leu Val
                85                  90                  95
Gly Ala Ala Leu Ala Ala Gly Leu Leu Trp Lys Phe Met Gly Ser Lys
            100                 105                 110
Cys Ser Asn Ser Gly Ile Glu Cys Asp Ser Ser Gly Thr Cys Ile Asn
        115                 120                 125
Pro Ser Asn Trp Cys Asp Gly Val Ser His Cys Pro Gly Gly Glu Asp
    130                 135                 140
Glu Asn Arg Cys Val Arg Leu Tyr Gly Pro Asn Phe Ile Leu Gln Met
145                 150                 155                 160

Tyr Ser Ser Gln Arg Lys Ser Trp His Pro Val Cys Gln Asp Asp Trp
                165                 170                 175
Asn Glu Asn Tyr Gly Arg Ala Ala Cys Arg Asp Met Gly Tyr Lys Asn
            180                 185                 190
Asn Phe Tyr Ser Ser Gln Gly Ile Val Asp Asp Ser Gly Ser Thr Ser
        195                 200                 205
Phe

<210> 898
<211> 27
<212> PRT
<213> Homo sapiens

<400> 898
Val Gly Glu Gly Leu Tyr Gln Gly Val Pro Arg Ala Glu Pro Gly Thr
 1               5                  10                  15
Glu Ala Arg Arg His Tyr Asp Glu Gly Val Arg
            20                  25

<210> 899
<211> 35
<212> DNA
<213> Artificial Sequence

<220>
<223> PCR Primer

<400> 899
ggatccgccg ccaccatgtc actttctagc ctgct                                35

<210> 900
<211> 27
<212> DNA
<213> Artificial Sequence

<220>
<223> PCR Primer

<400> 900
gtcgactcag ctggaccaca gccgcag                                         27

<210> 901
<211> 34
<212> DNA
<213> Artificial Sequence

<220>
<223> PCR Primer

<400> 901
ggatccgccg ccaccatggg ctgcaggctg ctct                                 34

<210> 902
<211> 27
<212> DNA
<213> Artificial Sequence

<220>
<223> PCR Primer

<400> 902
gtcgactcag aaatcctttc tcttgac                                         27

<210> 903
<211> 936

<212> DNA
<213> Homo sapiens

<220>
<221> misc_feature
<222> 16
<223> n = A,T,C or G

<400> 903
atgggctgca ggctgntctg ctgtgcggtt ctctgtctcc tgggagcggt ccccatggaa 60
acgggagtta cgcagacacc aagacacctg gtcatgggaa tgacaaataa gaagtctttg 120
aaatgtgaac aacatctggg tcataacgct atgtattggt acaagcaaag tgctaagaag 180
ccactggagc tcatgtttgt ctacagtctt gaagaacggg ttgaaaacaa cagtgtgcca 240
agtcgcttct cacctgaatg ccccaacagc tctcacttat tccttcacct acacaccctg 300
cagccagaag actcggccct gtatctctgc gccagcagcc aagaccggac aagcagctcc 360
tacgagcagt acttcgggcc gggcaccagg ctcacggtca cagaggacct gaaaaacgtg 420
ttcccacccg aggtcgctgt gtttgagcca tcagaagcag agatctccca cacccaaaag 480
gccacactgg tgtgcctggc cacaggcttc taccccgacc acgtggagct gagctggtgg 540
gtgaatggga aggaggtgca cagtggggtc agcacagacc cgcagcccct caaggagcag 600
cccgccctca atgactccag atactgcctg agcagccgcc tgagggtctc ggccaccttc 660
tggcagaacc cccgcaacca cttccgctgt caagtccagt tctacgggct ctcggagaat 720
gacgagtgga cccaggatag ggccaaacct gtcacccaga tcgtcagcgc cgaggcctgg 780
ggtagagcag actgtggctt cacctccgag tcttaccagc aaggggtcct gtctgccacc 840
atcctctatg agatcttgct agggaaggcc accttgtatg ccgtgctggt cagtgccctc 900
gtgctgatgg ccatggtcaa gagaaaggat ttctga 936

<210> 904
<211> 834
<212> DNA
<213> Homo sapiens

<220>
<221> misc_feature
<222> 21
<223> n = A,T,C or G

<400> 904
atgtcacttt ctagcctgct naaggtggtc acagcttcac tgtggctagg acctggcatt 60
gcccagaaga taactcaaac ccaaccagga atgttcgtgc aggaaaagga ggctgtgact 120
ctggactgca catatgacac cagtgatcaa agttatggtc tcttctggta caagcagccc 180
agcagtgggg aaatgatttt tcttatttat caggggtctt atgacgagca aaatgcaaca 240
gaaggtcgct actcattgaa tttccagaag gcaagaaaat ccgccaacct tgtcatctcc 300
gcttcacaac tgggggactc agcaatgtat ttctgtgcaa tgagagaggg cgcgggagga 360
ggaaacaaac tcacctttgg gacaggcact cagctaaaag tggaactcaa tatccagaac 420
cctgaccctg ccgtgtacca gctgagagac tctaaatcca gtgacaagtc tgtctgccta 480
ttcaccgatt ttgattctca aacaaatgtg tcacaaagta aggattctga tgtgtatatc 540
acagacaaaa ctgtgctaga catgaggtct atggacttca agagcaacag tgctgtggcc 600
tggagcaaca aatctgactt tgcatgtgca aacgccttca acaacagcat tattccagaa 660
gacaccttct tccccagccc agaaagttcc tgtgatgtca agctggtcga gaaaagcttt 720
gaaacagata cgaacctaaa ctttcaaaac ctgtcagtga ttgggttccg aatcctcctc 780
ctgaaagtgg ccgggtttaa tctgctcatg acgctgcggc tgtggtccag ctga 834

<210> 905
<211> 311
<212> PRT
<213> Homo sapiens

<220>
<221> VARIANT
<222> 6
<223> Xaa = Any Amino Acid

<400> 905
Met Gly Cys Arg Leu Xaa Cys Cys Ala Val Leu Cys Leu Leu Gly Ala
1               5                   10                  15
Val Pro Met Glu Thr Gly Val Thr Gln Thr Pro Arg His Leu Val Met
            20                  25                  30
Gly Met Thr Asn Lys Lys Ser Leu Lys Cys Glu Gln His Leu Gly His

35                  40                  45
Asn Ala Met Tyr Trp Tyr Lys Gln Ser Ala Lys Lys Pro Leu Glu Leu
    50                  55                  60
Met Phe Val Tyr Ser Leu Glu Glu Arg Val Glu Asn Asn Ser Val Pro
65                  70                  75                  80
Ser Arg Phe Ser Pro Glu Cys Pro Asn Ser Ser His Leu Phe Leu His
                85                  90                  95
Leu His Thr Leu Gln Pro Glu Asp Ser Ala Leu Tyr Leu Cys Ala Ser
            100                 105                 110
Ser Gln Asp Arg Thr Ser Ser Ser Tyr Glu Gln Tyr Phe Gly Pro Gly
        115                 120                 125
Thr Arg Leu Thr Val Thr Glu Asp Leu Lys Asn Val Phe Pro Pro Glu
    130                 135                 140
Val Ala Val Phe Glu Pro Ser Glu Ala Glu Ile Ser His Thr Gln Lys
145                 150                 155                 160
Ala Thr Leu Val Cys Leu Ala Thr Gly Phe Tyr Pro Asp His Val Glu
                165                 170                 175
Leu Ser Trp Trp Val Asn Gly Lys Glu Val His Ser Gly Val Ser Thr
            180                 185                 190
Asp Pro Gln Pro Leu Lys Glu Gln Pro Ala Leu Asn Asp Ser Arg Tyr
        195                 200                 205
Cys Leu Ser Ser Arg Leu Arg Val Ser Ala Thr Phe Trp Gln Asn Pro
    210                 215                 220
Arg Asn His Phe Arg Cys Gln Val Gln Phe Tyr Gly Leu Ser Glu Asn
225                 230                 235                 240
Asp Glu Trp Thr Gln Asp Arg Ala Lys Pro Val Thr Gln Ile Val Ser
                245                 250                 255
Ala Glu Ala Trp Gly Arg Ala Asp Cys Gly Phe Thr Ser Glu Ser Tyr
            260                 265                 270
Gln Gln Gly Val Leu Ser Ala Thr Ile Leu Tyr Glu Ile Leu Leu Gly
        275                 280                 285
Lys Ala Thr Leu Tyr Ala Val Leu Val Ser Ala Leu Val Leu Met Ala
    290                 295                 300
Met Val Lys Arg Lys Asp Phe
305                 310


<210> 906
<211> 277
<212> PRT
<213> Homo sapiens

<400> 906
Met Ser Leu Ser Ser Leu Leu Lys Val Val Thr Ala Ser Leu Trp Leu
 1               5                  10                  15
Gly Pro Gly Ile Ala Gln Lys Ile Thr Gln Thr Gln Pro Gly Met Phe
            20                  25                  30
Val Gln Glu Lys Glu Ala Val Thr Leu Asp Cys Thr Tyr Asp Thr Ser
        35                  40                  45
Asp Gln Ser Tyr Gly Leu Phe Trp Tyr Lys Gln Pro Ser Ser Gly Glu
    50                  55                  60
Met Ile Phe Leu Ile Tyr Gln Gly Ser Tyr Asp Glu Gln Asn Ala Thr
65                  70                  75                  80
Glu Gly Arg Tyr Ser Leu Asn Phe Gln Lys Ala Arg Lys Ser Ala Asn
                85                  90                  95
Leu Val Ile Ser Ala Ser Gln Leu Gly Asp Ser Ala Met Tyr Phe Cys
            100                 105                 110
Ala Met Arg Glu Gly Ala Gly Gly Gly Asn Lys Leu Thr Phe Gly Thr
        115                 120                 125
Gly Thr Gln Leu Lys Val Glu Leu Asn Ile Gln Asn Pro Asp Pro Ala
    130                 135                 140
Val Tyr Gln Leu Arg Asp Ser Lys Ser Ser Asp Lys Ser Val Cys Leu
145                 150                 155                 160
Phe Thr Asp Phe Asp Ser Gln Thr Asn Val Ser Gln Ser Lys Asp Ser
                165                 170                 175
Asp Val Tyr Ile Thr Asp Lys Thr Val Leu Asp Met Arg Ser Met Asp
            180                 185                 190
Phe Lys Ser Asn Ser Ala Val Ala Trp Ser Asn Lys Ser Asp Phe Ala

195                 200                 205
Cys Ala Asn Ala Phe Asn Asn Ser Ile Ile Pro Glu Asp Thr Phe Phe
    210                 215                 220
Pro Ser Pro Glu Ser Ser Cys Asp Val Lys Leu Val Glu Lys Ser Phe
225                 230                 235                 240
Glu Thr Asp Thr Asn Leu Asn Phe Gln Asn Leu Ser Val Ile Gly Phe
                245                 250                 255
Arg Ile Leu Leu Leu Lys Val Ala Gly Phe Asn Leu Leu Met Thr Leu
            260                 265                 270
Arg Leu Trp Ser Ser
        275

<210> 907
<211> 1536
<212> DNA
<213> Homo sapiens

<400> 907
   atgtacaacc tgttgctgtc ctacgacaga catggggacc acctgcagcc cctggacctc 60
   gtgcccaatc accagggtct cacccctttc aagctggctg gagtggaggg taacactgtg 120
   atgtttcagc acctgatgca gaagcggaag cacacccagt ggacgtatgg accactgacc 180
   tcgactctct atgacctcac agagatcgac tcctcagggg atgagcagtc cctgctggaa 240
   cttatcatca ccaccaagaa gcgggaggct cgccagatcc tggaccagac gccggtgaag 300
   gagctggtga gcctcaagtg gaagcggtac gggcggccgt acttctgcat gctgggtgcc 360
   atatatctgc tgtacatcat ctgcttcacc atgtgctgca tctaccgccc cctcaagccc 420
   aggaccaata accgcacgag cccccgggac aacaccctct tacagcagaa gctacttcag 480
   gaagcctaca tgacccctaa ggacgatatc cggctggtcg gggagctggt gactgtcatt 540
   ggggctatca tcatcctgct ggtagaggtt ccagacatct tcagaatggg ggtcactcgc 600
   ttctttggac agaccatcct tgggggccca ttccatgtcc tcatcatcac ctatgccttc 660
   atggtgctgg tgaccatggt gatgcggctc atcagtgcca gcggggaggt ggtacccatg 720
   tcctttgcac tcgtgctggg ctggtgcaac gtcatgtact tcgcccgagg attccagatg 780
   ctaggcccct tcaccatcat gattcagaag atgattttgg gcgacctgat gcgattctgc 840
   tggctgatgg ctgtggtcat cctgggcttt gcttcagcct tctatatcat cttccagaca 900
   gaggaccccg aggagctagg ccacttctac gactacccca tggccctgtt cagcaccttc 960
   gagctgttcc ttaccatcat cgatggccca gccaactaca acgtggacct gcccttcatg 1020
   tacagcatca cctatgctgc ctttgccatc atcgccacac tgctcatgct caacctcctc 1080
   attgccatga tgggcgacac tcactggcga gtggcccatg agcgggatga gctgtggagg 1140
   gcccagattg tggccaccac ggtgatgctg gagcggaagc tgcctcgctg cctgtggcct 1200
   cgctccggga tctgcggacg ggagtatggc ctgggagacc gctggttcct gcgggtggaa 1260
   gacaggcaag atctcaaccg gcagcggatc caacgctacg cacaggcctt ccacacccgg 1320
   ggctctgagg atttggacaa agactcagtg gaaaaactag agctgggctg tcccttcagc 1380
   ccccacctgt cccttcctat gccctcagtg tctcgaagta cctcccgcag cagtgccaat 1440
   tgggaaaggc ttcggcaagg gaccctgagg agagacctgc gtgggataat caacaggggt 1500
   ctggaggacg gggagagctg ggaatatcag atctga 1536

<210> 908
<211> 1533
<212> DNA
<213> Homo sapiens

<400> 908
atgtacaacc tgttgctgtc ctacgacaga catggggacc acctgcagcc cctggacctc 60
gtgcccaatc accagggtct cacccctttc aagctggctg gagtggaggg taacactgtg 120
atgtttcagc acctgatgca gaagcggaag cacacccagt ggacgtatgg accactgacc 180
tcgactctct atgacctcac agagatcgac tcctcagggg atgagcagtc cctgctggaa 240
cttatcatca ccaccaagaa gcgggaggct cgccagatcc tggaccagac gccggtgaag 300
gagctggtga gcctcaagtg gaagcggtac gggcggccgt acttctgcat gctgggtgcc 360
atatatctgc tgtacatcat ctgcttcacc atgtgctgca tctaccgccc cctcaagccc 420
aggaccaata accgcacgag cccccgggac aacaccctct tacagcagaa gctacttcag 480
gaagcctaca tgacccctaa ggacgatatc cggctggtcg gggagctggt gactgtcatt 540
ggggctatca tcatcctgct ggtagaggtt ccagacatct tcagaatggg ggtcactcgc 600
ttctttggac agaccatcct tgggggccca ttccatgtcc tcatcatcac ctatgccttc 660
atggtgctgg tgaccatggt gatgcggctc atcagtgcca gcggggaggt ggtacccatg 720
tcctttgcac tcgtgctggg ctggtgcaac gtcatgtact tcgcccgagg attccagatg 780
ctaggcccct tcaccatcat gattcagaag atgattttgg gcgacctgat gcgattctgc 840
tggctgatgg ctgtggtcat cctgggcttt gcttcagcct tctatatcat cttccagaca 900
gaggaccccg aggagctagg ccacttctac gactacccca tggccctgtt cagcaccttc 960

gagctgttcc ttaccatcat cgatggccca gccaactaca acgtggacct gcccttcatg 1020
tacagcatca cctatgctgc ctttgccatc atcgccacac tgctcatgct caacctcctc 1080
attgccatga tgggcgacac tcactggcga gtggcccatg agcgggatga gctgtggagg 1140
gcccagattg tggccaccac ggtgatgctg gagcggaagc tgcctcgctg cctgtggcct 1200
cgctccggga tctgcggacg ggagtatggc ctgggagacc gctggttcct gcgggtggaa 1260
gacaggcaag atctcaaccg gcagcggatc caacgctacg cacaggcctt ccacacccgg 1320
ggctctgagg atttggacaa agactcagtg gaaaaactag agctgggctg tcccttcagc 1380
ccccacctgt cccttcctat gccctcagtg tctcgaagta cctcccgcag cagtgccaat 1440
tgggaaaggc ttcggcaagg gaccctgagg agagacctgc gtgggataat caacaggggt 1500
ctggaggacg gggagagctg ggaatatcag atc 1533

<210> 909
<211> 511
<212> PRT
<213> Homo sapiens

<400> 909
Met Tyr Asn Leu Leu Leu Ser Tyr Asp Arg His Gly Asp His Leu Gln
1               5                   10                  15
Pro Leu Asp Leu Val Pro Asn His Gln Gly Leu Thr Pro Phe Lys Leu
            20                  25                  30
Ala Gly Val Glu Gly Asn Thr Val Met Phe Gln His Leu Met Gln Lys
        35                  40                  45
Arg Lys His Thr Gln Trp Thr Tyr Gly Pro Leu Thr Ser Thr Leu Tyr
    50                  55                  60
Asp Leu Thr Glu Ile Asp Ser Ser Gly Asp Glu Gln Ser Leu Leu Glu
65                  70                  75                  80
Leu Ile Ile Thr Thr Lys Lys Arg Glu Ala Arg Gln Ile Leu Asp Gln
                85                  90                  95
Thr Pro Val Lys Glu Leu Val Ser Leu Lys Trp Lys Arg Tyr Gly Arg
            100                 105                 110
Pro Tyr Phe Cys Met Leu Gly Ala Ile Tyr Leu Leu Tyr Ile Ile Cys
        115                 120                 125
Phe Thr Met Cys Cys Ile Tyr Arg Pro Leu Lys Pro Arg Thr Asn Asn
    130                 135                 140
Arg Thr Ser Pro Arg Asp Asn Thr Leu Leu Gln Gln Lys Leu Leu Gln
145                 150                 155                 160
Glu Ala Tyr Met Thr Pro Lys Asp Asp Ile Arg Leu Val Gly Glu Leu
                165                 170                 175
Val Thr Val Ile Gly Ala Ile Ile Ile Leu Leu Val Glu Val Pro Asp
            180                 185                 190
Ile Phe Arg Met Gly Val Thr Arg Phe Phe Gly Gln Thr Ile Leu Gly
        195                 200                 205
Gly Pro Phe His Val Leu Ile Ile Thr Tyr Ala Phe Met Val Leu Val
    210                 215                 220
Thr Met Val Met Arg Leu Ile Ser Ala Ser Gly Glu Val Val Pro Met
225                 230                 235                 240
Ser Phe Ala Leu Val Leu Gly Trp Cys Asn Val Met Tyr Phe Ala Arg
                245                 250                 255
Gly Phe Gln Met Leu Gly Pro Phe Thr Ile Met Ile Gln Lys Met Ile
            260                 265                 270
Phe Gly Asp Leu Met Arg Phe Cys Trp Leu Met Ala Val Val Ile Leu
        275                 280                 285
Gly Phe Ala Ser Ala Phe Tyr Ile Ile Phe Gln Thr Glu Asp Pro Glu
    290                 295                 300
Glu Leu Gly His Phe Tyr Asp Tyr Pro Met Ala Leu Phe Ser Thr Phe
305                 310                 315                 320
Glu Leu Phe Leu Thr Ile Ile Asp Gly Pro Ala Asn Tyr Asn Val Asp
                325                 330                 335
Leu Pro Phe Met Tyr Ser Ile Thr Tyr Ala Ala Phe Ala Ile Ile Ala
            340                 345                 350
Thr Leu Leu Met Leu Asn Leu Leu Ile Ala Met Met Gly Asp Thr His
        355                 360                 365
Trp Arg Val Ala His Glu Arg Asp Glu Leu Trp Arg Ala Gln Ile Val
    370                 375                 380
Ala Thr Thr Val Met Leu Glu Arg Lys Leu Pro Arg Cys Leu Trp Pro
385                 390                 395                 400
Arg Ser Gly Ile Cys Gly Arg Glu Tyr Gly Leu Gly Asp Arg Trp Phe

                405                 410                 415
Leu Arg Val Glu Asp Arg Gln Asp Leu Asn Arg Gln Arg Ile Gln Arg
            420                 425                 430
Tyr Ala Gln Ala Phe His Thr Arg Gly Ser Glu Asp Leu Asp Lys Asp
        435                 440                 445
Ser Val Glu Lys Leu Glu Leu Gly Cys Pro Phe Ser Pro His Leu Ser
    450                 455                 460
Leu Pro Met Pro Ser Val Ser Arg Ser Thr Ser Arg Ser Ser Ala Asn
465                 470                 475                 480
Trp Glu Arg Leu Arg Gln Gly Thr Leu Arg Arg Asp Leu Arg Gly Ile
                485                 490                 495
Ile Asn Arg Gly Leu Glu Asp Gly Glu Ser Trp Glu Tyr Gln Ile
            500                 505                 510

<210> 910
<211> 134
<212> PRT
<213> Homo sapiens

<400> 910
Met Tyr Asn Leu Leu Leu Ser Tyr Asp Arg His Gly Asp His Leu Gln
1               5                   10                  15
Pro Leu Asp Leu Val Pro Asn His Gln Gly Leu Thr Pro Phe Lys Leu
            20                  25                  30
Ala Gly Val Glu Gly Asn Thr Val Met Phe Gln His Leu Met Gln Lys
        35                  40                  45
Arg Lys His Thr Gln Trp Thr Tyr Gly Pro Leu Thr Ser Thr Leu Tyr
    50                  55                  60
Asp Leu Thr Glu Ile Asp Ser Ser Gly Asp Glu Gln Ser Leu Leu Glu
65                  70                  75                  80
Leu Ile Ile Thr Thr Lys Lys Arg Glu Ala Arg Gln Ile Leu Asp Gln
                85                  90                  95
Thr Pro Val Lys Glu Leu Val Ser Leu Lys Trp Lys Arg Tyr Gly Arg
            100                 105                 110
Pro Tyr Phe Cys Met Leu Gly Ala Ile Tyr Leu Leu Tyr Ile Ile Cys
        115                 120                 125
Phe Thr Met Cys Cys Ile
    130

<210> 911
<211> 55
<212> PRT
<213> Homo sapiens

<400> 911
Ala Tyr Arg Pro Leu Lys Pro Arg Thr Asn Asn Arg Thr Ser Pro Arg
1               5                   10                  15
Asp Asn Thr Leu Leu Gln Gln Lys Leu Leu Gln Glu Ala Tyr Met Thr
            20                  25                  30
Pro Lys Asp Asp Ile Arg Leu Val Gly Glu Leu Val Thr Val Ile Gly
        35                  40                  45
Ala Ile Ile Ile Leu Leu Val
    50                  55

<210> 912
<211> 39
<212> PRT
<213> Homo sapiens

<400> 912
Glu Val Pro Asp Ile Phe Arg Met Gly Val Thr Arg Phe Phe Gly Gln
1               5                   10                  15
Thr Ile Leu Gly Gly Pro Phe His Val Leu Ile Ile Thr Tyr Ala Phe
            20                  25                  30
Met Val Leu Val Thr Met Val

        35

<210> 913
<211> 19
<212> PRT
<213> Homo sapiens

<400> 913
Met Arg Leu Ile Ser Ala Ser Gly Glu Val Val Pro Met Ser Phe Ala
1               5                   10                  15
Leu Val Leu

<210> 914
<211> 52
<212> PRT
<213> Homo sapiens

<400> 914
Gly Trp Cys Asn Val Met Tyr Phe Ala Arg Gly Phe Gln Met Leu Gly
1               5                   10                  15
Pro Phe Thr Ile Met Ile Gln Lys Met Ile Phe Gly Asp Leu Met Arg
            20                  25                  30
Phe Cys Trp Leu Met Ala Val Val Ile Leu Gly Phe Ala Ser Ala Phe
        35                  40                  45
Tyr Ile Ile Phe
    50

<210> 915
<211> 213
<212> PRT
<213> Homo sapiens

<400> 915
Gln Thr Glu Asp Pro Glu Glu Leu Gly His Phe Tyr Asp Tyr Pro Met
1               5                   10                  15
Ala Leu Phe Ser Thr Phe Glu Leu Phe Leu Thr Ile Ile Asp Gly Pro
            20                  25                  30
Ala Asn Tyr Asn Val Asp Leu Pro Phe Met Tyr Ser Ile Thr Tyr Ala
        35                  40                  45
Ala Phe Ala Ile Ile Ala Thr Leu Leu Met Leu Asn Leu Leu Ile Ala
    50                  55                  60
Met Met Gly Asp Thr His Trp Arg Val Ala His Glu Arg Asp Glu Leu
65                  70                  75                  80
Trp Arg Ala Gln Ile Val Ala Thr Thr Val Met Leu Glu Arg Lys Leu
                85                  90                  95
Pro Arg Cys Leu Trp Pro Arg Ser Gly Ile Cys Gly Arg Glu Tyr Gly
            100                 105                 110
Leu Gly Asp Arg Trp Phe Leu Arg Val Glu Asp Arg Gln Asp Leu Asn
        115                 120                 125
Arg Gln Arg Ile Gln Arg Tyr Ala Gln Ala Phe His Thr Arg Gly Ser
    130                 135                 140
Glu Asp Leu Asp Lys Asp Ser Val Glu Lys Leu Glu Leu Gly Cys Pro
145                 150                 155                 160
Phe Ser Pro His Leu Ser Leu Pro Met Pro Ser Val Ser Arg Ser Thr
                165                 170                 175
Ser Arg Ser Ser Ala Asn Trp Glu Arg Leu Arg Gln Gly Thr Leu Arg
            180                 185                 190
Arg Asp Leu Arg Gly Ile Ile Asn Arg Gly Leu Glu Asp Gly Glu Ser
        195                 200                 205
Trp Glu Tyr Gln Ile
    210

<210> 916

<211> 1302
<212> DNA
<213> Homo sapiens

<400> 916
tggacaaagg gggtcacaca ttccttccat acggttgagc ctctacctgc ctggtgctgg 60
tcacagttca gcttcttcat gatggtggat cccaatggca atgaatccag tgctacatac 120
ttcatcctaa taggcctccc tggtttagaa gaggctcagt tctggttggc cttcccattg 180
tgctccctct accttattgc tgtgctaggt aacttgacaa tcatctacat tgtgcggact 240
gagcacagcc tgcatgagcc catgtatata tttctttgca tgctttcagg cattgacatc 300
ctcatctcca cctcatccat gcccaaaatg ctggccatct tctggttcaa ttccactacc 360
atccagtttg atgcttgtct gctacagatg tttgccatcc actccttatc tggcatggaa 420
tccacagtgc tgctggccat ggcttttgac cgctatgtgg ccatctgtca cccactgcgc 480
catgccacag tacttacgtt gcctcgtgtc accaaaattg gtgtggctgc tgtggtgcgg 540
ggggctgcac tgatggcacc ccttcctgtc ttcatcaagc agctgccctt ctgccgctcc 600
aatatccttt cccattccta ctgcctacac caagatgtca tgaagctggc ctgtgatgat 660
atccgggtca atgtcgtcta tggccttatc gtcatcatct ccgccattgg cctggactca 720
cttctcatct ccttctcata tctgcttatt cttaagactg tgttgggctt gacacgtgaa 780
gcccaggcca aggcatttgg cacttgcgtc tctcatgtgt gtgctgtgtt catattctat 840
gtacctttca ttggattgtc catggtgcat cgctttagca agcggcgtga ctctccgctg 900
cccgtcatct tggccaatat ctatctgctg gttcctcctg tgctcaaccc aattgtctat 960
ggagtgaaga caaaggagat tcgacagcgc atccttcgac ttttccatgt ggccacacac 1020
gcttcagagc cctaggtgtc agtgatcaaa cttcttttcc attcagagtc ctctgattca 1080
gattttaatg ttaacatttt ggaagacagt attcagaaaa aaaatttcct taataaaaat 1140
acaactcaga tccttcaaat atgaaactgg ttggggaatc tccatttttt caatattatt 1200
ttcttctttg ttttcttgct acatataatt attaataccc tgactaggtt gtggtttgag 1260
ggttattact tttcatttta ccatgcagtc caaatctaaa ct 1302

<210> 917
<211> 2061
<212> DNA
<213> Homo sapiens

<400> 917
acgattcgac agcgcatcct tcgacttttc catgtggcca cacacgcttc agagccctag 60
gtgtcagtga tcaaacttct tttccattca gagtcctctg attcagattt taatgttaac 120
attttggaag acagtattca gaaaaaaaat ttccttaata aaaatacaac tcagatcctt 180
caaatatgaa actggttggg gaatctccat tttttcaata ttattttctt ctttgttttc 240
ttgctacata taattattaa taccctgact aggttgtggt ttgagggtta ttacttttca 300
ttttaccatg cagtccaaat ctaaactgct tctactgatg gtttacagca ttctgagata 360
agaatggtac atctagagaa catttgccaa aggcctaagc acggcaaagg aaaataaaca 420
cagaatataa taaaatgaga taatctagct taaaactata acttcctctt cagaactccc 480
aaccacattg gatctcagaa aaatgctgtc ttcaaaatga cttctacaga gaagaaataa 540
tttttcctct ggacactagc acttaagggg aagattggaa gtaaagcctt gaaaagagta 600
catttaccta cgttaatgaa agttgacaca ctgttctgag agttttcaca gcatatggac 660
cctgtttttc ctatttaatt ttcttatcaa ccctttaatt aggcaaagat attattagta 720
ccctcattgt agccatggga aaattgatgt tcagtgggga tcagtgaatt aaatggggtc 780
atacaagtat aaaaattaaa aaaaaaggac ttcatgccca atctcatatg atgtggaaga 840
actgttagag agaccaacag ggtagtgggt tagagatttc cagagtctta cattttctag 900
aggaggtatt taatttcttc tcactcatcc agtgttgtat ttaggaattt cctggcaaca 960
gaactcatgg ctttaatccc actagctatt gcttattgtc ctggtccaat tgccaattac 1020
ctgtgtcttg gaagaagtga tttctaggtt caccattatg gaagattctt attcagaaag 1080
tctgcatagg gcttatagca agttatttat ttttaaaagt tccataggtg attctgatag 1140
gcagtgaggt tagggagcca ccagttatga tgggaagtat ggaatggcag gtcttgaaga 1200
taacattggc cttttgagtg tgactcgtag ctggaaagtg agggaatctt caggaccatg 1260
ctttatttgg ggctttgtgc agtatggaac agggactttg agaccaggaa agcaatctga 1320
cttaggcatg ggaatcaggc atttttgctt ctgaggggct attaccaagg gttaataggt 1380
ttcatcttca acaggatatg acaacagtgt taaccaagaa actcaaatta caaatactaa 1440
aacatgtgat catatatgtg gtaagtttca ttttcttttt caatcctcag gttccctgat 1500
atggattcct ataacatgct ttcatcccct tttgtaatgg atatcatatt tggaaatgcc 1560
tatttaatac ttgtatttgc tgctggactg taagcccatg agggcactgt ttattattga 1620
atgtcatctc tgttcatcat tgactgctct ttgctcatca ttgaatcccc cagcaaagtg 1680
cctagaacat aatagtgctt atgcttgaca ccggttattt ttcatcaaac ctgattcctt 1740
ctgtcctgaa cacatagcca ggcaattttc cagccttctt tgagttgggt attattaaat 1800
tctggccatt acttccaatg tgagtggaag tgacatgtgc aatttctata cctggctcat 1860
aaaaccctcc catgtgcagc ctttcatgtt gacattaaat gtgacttggg aagctatgtg 1920
ttacacagag taaatcacca gaagcctgga tttctgaaaa aactgtgcag agccaaacct 1980
ctgtcatttg caactcccac ttgtatttgt acgaggcagt tggataagtg aaaaataaag 2040

tactattgtg tcaagtctct g                                              2061

<210> 918
<211> 957
<212> DNA
<213> Homo sapiens

<400> 918
atgatggtgg atcccaatgg caatgaatcc agtgctacat acttcatcct aataggcctc 60
cctggtttag aagaggctca gttctggttg gccttcccat tgtgctccct ctaccttatt 120
gctgtgctag gtaacttgac aatcatctac attgtgcgga ctgagcacag cctgcatgag 180
cccatgtata tatttctttg catgctttca ggcattgaca tcctcatctc cacctcatcc 240
atgcccaaaa tgctggccat cttctggttc aattccacta ccatccagtt tgatgcttgt 300
ctgctacaga tgtttgccat ccactcctta tctggcatgg aatccacagt gctgctggcc 360
atggcttttg accgctatgt ggccatctgt cacccactgc gccatgccac agtacttacg 420
ttgcctcgtg tcaccaaaat tggtgtggct gctgtggtgc ggggggctgc actgatggca 480
ccccttcctg tcttcatcaa gcagctgccc ttctgccgct ccaatatcct ttcccattcc 540
tactgcctac accaagatgt catgaagctg gcctgtgatg atatccgggt caatgtcgtc 600
tatggcctta tcgtcatcat ctccgccatt ggcctggact cacttctcat ctccttctca 660
tatctgctta ttcttaagac tgtgttgggc ttgacacgtg aagcccaggc caaggcattt 720
ggcacttgcg tctctcatgt gtgtgctgtg ttcatattct atgtaccttt cattggattg 780
tccatggtgc atcgctttag caagcggcgt gactctccgc tgcccgtcat cttggccaat 840
atctatctgc tggttcctcc tgtgctcaac ccaattgtct atggagtgaa gacaaaggag 900
attcgacagc gcatccttcg acttttccat gtggccacac acgcttcaga gccctag    957

<210> 919
<211> 954
<212> DNA
<213> Homo sapiens

<400> 919
atgatggtgg atcccaatgg caatgaatcc agtgctacat acttcatcct aataggcctc 60
cctggtttag aagaggctca gttctggttg gccttcccat tgtgctccct ctaccttatt 120
gctgtgctag gtaacttgac aatcatctac attgtgcgga ctgagcacag cctgcatgag 180
cccatgtata tatttctttg catgctttca ggcattgaca tcctcatctc cacctcatcc 240
atgcccaaaa tgctggccat cttctggttc aattccacta ccatccagtt tgatgcttgt 300
ctgctacaga tgtttgccat ccactcctta tctggcatgg aatccacagt gctgctggcc 360
atggcttttg accgctatgt ggccatctgt cacccactgc gccatgccac agtacttacg 420
ttgcctcgtg tcaccaaaat tggtgtggct gctgtggtgc ggggggctgc actgatggca 480
ccccttcctg tcttcatcaa gcagctgccc ttctgccgct ccaatatcct ttcccattcc 540
tactgcctac accaagatgt catgaagctg gcctgtgatg atatccgggt caatgtcgtc 600
tatggcctta tcgtcatcat ctccgccatt ggcctggact cacttctcat ctccttctca 660
tatctgctta ttcttaagac tgtgttgggc ttgacacgtg aagcccaggc caaggcattt 720
ggcacttgcg tctctcatgt gtgtgctgtg ttcatattct atgtaccttt cattggattg 780
tccatggtgc atcgctttag caagcggcgt gactctccgc tgcccgtcat cttggccaat 840
atctatctgc tggttcctcc tgtgctcaac ccaattgtct atggagtgaa gacaaaggag 900
attcgacagc gcatccttcg acttttccat gtggccacac acgcttcaga gccc       954

<210> 920
<211> 318
<212> PRT
<213> Homo sapiens

<400> 920
Met Met Val Asp Pro Asn Gly Asn Glu Ser Ser Ala Thr Tyr Phe Ile
1               5                   10                  15
Leu Ile Gly Leu Pro Gly Leu Glu Glu Ala Gln Phe Trp Leu Ala Phe
            20                  25                  30
Pro Leu Cys Ser Leu Tyr Leu Ile Ala Val Leu Gly Asn Leu Thr Ile
        35                  40                  45
Ile Tyr Ile Val Arg Thr Glu His Ser Leu His Glu Pro Met Tyr Ile
    50                  55                  60
Phe Leu Cys Met Leu Ser Gly Ile Asp Ile Leu Ile Ser Thr Ser Ser
65                  70                  75                  80
Met Pro Lys Met Leu Ala Ile Phe Trp Phe Asn Ser Thr Thr Ile Gln
                85                  90                  95
Phe Asp Ala Cys Leu Leu Gln Met Phe Ala Ile His Ser Leu Ser Gly
            100                 105                 110

Met Glu Ser Thr Val Leu Leu Ala Met Ala Phe Asp Arg Tyr Val Ala
            115                 120                 125
Ile Cys His Pro Leu Arg His Ala Thr Val Leu Thr Leu Pro Arg Val
    130                 135                 140
Thr Lys Ile Gly Val Ala Ala Val Val Arg Gly Ala Ala Leu Met Ala
145                 150                 155                 160
Pro Leu Pro Val Phe Ile Lys Gln Leu Pro Phe Cys Arg Ser Asn Ile
                165                 170                 175
Leu Ser His Ser Tyr Cys Leu His Gln Asp Val Met Lys Leu Ala Cys
            180                 185                 190
Asp Asp Ile Arg Val Asn Val Val Tyr Gly Leu Ile Val Ile Ile Ser
        195                 200                 205
Ala Ile Gly Leu Asp Ser Leu Leu Ile Ser Phe Ser Tyr Leu Leu Ile
    210                 215                 220
Leu Lys Thr Val Leu Gly Leu Thr Arg Glu Ala Gln Ala Lys Ala Phe
225                 230                 235                 240
Gly Thr Cys Val Ser His Val Cys Ala Val Phe Ile Phe Tyr Val Pro
                245                 250                 255
Phe Ile Gly Leu Ser Met Val His Arg Phe Ser Lys Arg Arg Asp Ser
            260                 265                 270
Pro Leu Pro Val Ile Leu Ala Asn Ile Tyr Leu Leu Val Pro Pro Val
        275                 280                 285
Leu Asn Pro Ile Val Tyr Gly Val Lys Thr Lys Glu Ile Arg Gln Arg
    290                 295                 300
Ile Leu Arg Leu Phe His Val Ala Thr His Ala Ser Glu Pro
305                 310                 315


<210> 921
<211> 28
<212> PRT
<213> Homo sapiens

<400> 921
Met Met Val Asp Pro Asn Gly Asn Glu Ser Ser Ala Thr Tyr Phe Ile
 1               5                  10                  15
Leu Ile Gly Leu Pro Gly Leu Glu Glu Ala Gln Phe
            20                  25


<210> 922
<211> 9
<212> PRT
<213> Homo sapiens

<400> 922
Arg Thr Glu His Ser Leu His Glu Pro
 1               5


<210> 923
<211> 21
<212> PRT
<213> Homo sapiens

<400> 923
Lys Met Leu Ala Ile Phe Trp Phe Asn Ser Thr Thr Ile Gln Phe Asp
 1               5                  10                  15
Ala Cys Leu Leu Gln
            20


<210> 924
<211> 20
<212> PRT
<213> Homo sapiens

<400> 924

Asp Arg Tyr Val Ala Ile Cys His Pro Leu Arg His Ala Thr Val Leu
1               5                   10                  15
Thr Leu Pro Arg
            20

<210> 925
<211> 37
<212> PRT
<213> Homo sapiens

<400> 925
Phe Ile Lys Gln Leu Pro Phe Cys Arg Ser Asn Ile Leu Ser His Ser
1               5                   10                  15
Tyr Cys Leu His Gln Asp Val Met Lys Leu Ala Cys Asp Asp Ile Arg
            20                  25                  30
Val Asn Val Val Tyr
        35

<210> 926
<211> 13
<212> PRT
<213> Homo sapiens

<400> 926
Lys Thr Val Leu Gly Leu Thr Arg Glu Ala Gln Ala Lys
1               5                   10

<210> 927
<211> 10
<212> PRT
<213> Homo sapiens

<400> 927
Val His Arg Phe Ser Lys Arg Arg Asp Ser
1               5                   10

<210> 928
<211> 22
<212> PRT
<213> Homo sapiens

<400> 928
Lys Thr Lys Glu Ile Arg Gln Arg Ile Leu Arg Leu Phe His Val Ala
1               5                   10                  15
Thr His Ala Ser Glu Pro
            20

<210> 929
<211> 3245
<212> DNA
<213> Homo sapiens

<400> 929
gtcgacccac gcgtccgcgc gagctaagca ggaggcggag gcggaggcgg agggcgaggg 60
gcggggagcg ccgcctggag cgcggcaggt catattgaac attccagata cctatcatta 120
ctcgatgctg ttgataacag caagatggct ttgaactcag ggtcaccacc agctattgga 180
ccttactatg aaaaccatgg ataccaaccg gaaaacccct atcccgcaca gcccactgtg 240
gtccccactg tctacgaggt gcatccggct cagtactacc cgtcccccgt gccccagtac 300
gccccgaggg tcctgacgca ggcttccaac cccgtcgtct gcacgcagcc caaatcccca 360
tccgggacag tgtgcacctc aaagactaag aaagcactgt gcatcacctt gaccctgggg 420
accttcctcg tgggagctgc gctggccgct ggcctactct ggaagttcat gggcagcaag 480
tgctccaact ctgggataga gtgcgactcc tcaggtacct gcatcaaccc ctctaactgg 540
tgtgatggcg tgtcacactg ccccggcggg gaggacgaga atcggtgtgt tcgcctctac 600

ggatcaaact tcatccttca ggtgtactca tctcagagga agtcctggca ccctgtgtgc 660
caagacgact ggaacgagaa ctacgggcgg gcggcctgca gggacatggg ctataagaat 720
aatttttact ctagccaagg aatagtggat gacagcggat ccaccagctt tatgaaactg 780
aacacaagtg ccggcaatgt cgatatctat aaaaaactgt accacagtga tgcctgttct 840
tcaaaagcag tggtttcttt acgctgtata gcctgcgggg tcaacttgaa ctcaagccgc 900
cagagcagga ttgtgggcgg cgagagcgcg ctcccggggg cctggccctg gcaggtcagc 960
ctgcacgtcc agaacgtcca cgtgtgcgga ggctccatca tcacccccga gtggatcgtg 1020
acagccgccc actgcgtgga aaaacctctt aacaatccat ggcattggac ggcatttgcg 1080
gggattttga gacaatcttt catgttctat ggagccggat accaagtaga aaaagtgatt 1140
tctcatccaa attatgactc caagaccaag aacaatgaca ttgcgctgat gaagctgcag 1200
aagcctctga ctttcaacga cctagtgaaa ccagtgtgtc tgcccaaccc aggcatgatg 1260
ctgcagccag aacagctctg ctggatttcc gggtgggggg ccaccgagga gaaagggaag 1320
acctcagaag tgctgaacgc tgccaaggtg cttctcattg agacacagag atgcaacagc 1380
agatatgtct atgacaacct gatcacacca gccatgatct gtgccggctt cctgcagggg 1440
aacgtcgatt cttgccaggg tgacagtgga gggcctctgg tcacttcgaa gaacaatatc 1500
tggtggctga taggggatac aagctggggt tctggctgtg ccaaagctta cagaccagga 1560
gtgtacggga atgtgatggt attcacggac tggatttatc gacaaatgag ggcagacggc 1620
taatccacat ggtcttcgtc cttgacgtcg ttttacaaga aaacaatggg gctggttttg 1680
cttccccgtg catgatttac tcttagagat gattcagagg tcacttcatt tttattaaac 1740
agtgaacttg tctggctttg gcactctctg ccattctgtg caggctgcag tggctcccct 1800
gcccagcctg ctctccctaa ccccttgtcc gcaaggggtg atggccggct ggttgtgggc 1860
actggcggtc aagtgtggag gagaggggtg gaggctgccc cattgagatc ttcctgctga 1920
gtcctttcca ggggccaatt ttggatgagc atggagctgt cacctctcag ctgctggatg 1980
acttgagatg aaaaaggaga gacatggaaa gggagacagc caggtggcac ctgcagcggc 2040
tgccctctgg ggccacttgg tagtgtcccc agcctacctc tccacaaggg gattttgctg 2100
atgggttctt agagccttag cagccctgga tggtggccag aaataaaggg accagccctt 2160
catgggtggt gacgtggtag tcacttgtaa ggggaacaga aacatttttg ttcttatggg 2220
gtgagaatat agacagtgcc cttggtgcga gggaagcaat tgaaaaggaa cttgccctga 2280
gcactcctgg tgcaggtctc cacctgcaca ttgggtgggg ctcctgggag ggagactcag 2340
ccttcctcct catcctccct gaccctgctc ctagcaccct ggagagtgca catgcccctt 2400
ggtcctggca gggcgccaag tctggcacca tgttggcctc ttcaggcctg ctagtcactg 2460
gaaattgagg tccatggggg aaatcaagga tgctcagttt aaggtacact gtttccatgt 2520
tatgtttcta cacattgcta cctcagtgct cctggaaact tagcttttga tgtctccaag 2580
tagtccacct tcatttaact ctttgaaact gtatcatctt tgccaagtaa gagtggtggc 2640
ctatttcagc tgctttgaca aaatgactgg ctcctgactt aacgttctat aaatgaatgt 2700
gctgaagcaa agtgcccatg gtggcggcga agaagagaaa gatgtgtttt gttttggact 2760
ctctgtggtc ccttccaatg ctgtgggttt ccaaccaggg gaagggtccc ttttgcattg 2820
ccaagtgcca taaccatgag cactactcta ccatggttct gcctcctggc caagcaggct 2880
ggtttgcaag aatgaaatga atgattctac agctaggact taaccttgaa atggaaagtc 2940
ttgcaatccc atttgcagga tccgtctgtg cacatgcctc tgtagagagc agcattccca 3000
gggaccttgg aaacagttgg cactgtaagg tgcttgctcc ccaagacaca tcctaaaagg 3060
tgttgtaatg gtgaaaacgt cttccttctt tattgcccct tcttatttat gtgaacaact 3120
gtttgtcttt ttttgtatct tttttaaact gtaaagttca attgtgaaaa tgaatatcat 3180
gcaaataaat tatgcgattt ttttttcaaa gtaaaaaaaa aaaaaaaaaa aaaaagggcg 3240
gccgc 3245

<210> 930
<211> 1479
<212> DNA
<213> Homo sapiens

<400> 930
atggctttga actcagggtc accaccagct attggacctt actatgaaaa ccatggatac 60
caaccggaaa acccctatcc cgcacagccc actgtggtcc ccactgtcta cgaggtgcat 120
ccggctcagt actacccgtc ccccgtgccc cagtacgccc cgagggtcct gacgcaggct 180
tccaaccccg tcgtctgcac gcagcccaaa tccccatccg ggacagtgtg cacctcaaag 240
actaagaaag cactgtgcat caccttgacc ctggggacct tcctcgtggg agctgcgctg 300
gccgctggcc tactctggaa gttcatgggc agcaagtgct ccaactctgg gatagagtgc 360
gactcctcag gtacctgcat caacccctct aactggtgtg atggcgtgtc acactgcccc 420
ggcggggagg acgagaatcg gtgtgttcgc ctctacggat caaacttcat ccttcaggtg 480
tactcatctc agaggaagtc ctggcaccct gtgtgccaag acgactggaa cgagaactac 540
gggcgggcgg cctgcaggga catgggctat aagaataatt tttactctag ccaaggaata 600
gtggatgaca gcggatccac cagctttatg aaactgaaca caagtgccgg caatgtcgat 660
atctataaaa aactgtacca cagtgatgcc tgttcttcaa aagcagtggt ttctttacgc 720
tgtatagcct gcggggtcaa cttgaactca agccgccaga gcaggattgt gggcggcgag 780
agcgcgctcc cgggggcctg gccctggcag gtcagcctgc acgtccagaa cgtccacgtg 840
tgcggaggct ccatcatcac ccccgagtgg atcgtgacag ccgcccactg cgtggaaaaa 900
cctcttaaca atccatggca ttggacggca tttgcgggga ttttgagaca atctttcatg 960

ttctatggag ccggatacca agtagaaaaa gtgatttctc atccaaatta tgactccaag 1020
accaagaaca atgacattgc gctgatgaag ctgcagaagc ctctgacttt caacgaccta 1080
gtgaaaccag tgtgtctgcc caacccaggc atgatgctgc agccagaaca gctctgctgg 1140
atttccgggt ggggggccac cgaggagaaa gggaagacct cagaagtgct gaacgctgcc 1200
aaggtgcttc tcattgagac acagagatgc aacagcagat atgtctatga caacctgatc 1260
acaccagcca tgatctgtgc cggcttcctg caggggaacg tcgattcttg ccagggtgac 1320
agtggagggc ctctggtcac ttcgaagaac aatatctggt ggctgatagg ggatacaagc 1380
tggggttctg gctgtgccaa agcttacaga ccaggagtgt acgggaatgt gatggtattc 1440
acggactgga tttatcgaca aatgagggca gacggctaa 1479

<210> 931
<211> 1476
<212> DNA
<213> Homo sapiens

<400> 931
atggctttga actcagggtc accaccagct attggacctt actatgaaaa ccatggatac 60
caaccggaaa acccctatcc cgcacagccc actgtggtcc ccactgtcta cgaggtgcat 120
ccggctcagt actacccgtc ccccgtgccc cagtacgccc cgagggtcct gacgcaggct 180
tccaaccccg tcgtctgcac gcagcccaaa tccccatccg ggacagtgtg cacctcaaag 240
actaagaaag cactgtgcat caccttgacc ctggggacct tcctcgtggg agctgcgctg 300
gccgctggcc tactctggaa gttcatgggc agcaagtgct ccaactctgg gatagagtgc 360
gactcctcag gtacctgcat caacccctct aactggtgtg atggcgtgtc acactgcccc 420
ggcggggagg acgagaatcg gtgtgttcgc ctctacggat caaacttcat ccttcaggtg 480
tactcatctc agaggaagtc ctggcaccct gtgtgccaag acgactggaa cgagaactac 540
gggcgggcgg cctgcaggga catgggctat aagaataatt tttactctag ccaaggaata 600
gtggatgaca gcggatccac cagctttatg aaactgaaca caagtgccgg caatgtcgat 660
atctataaaa aactgtacca cagtgatgcc tgttcttcaa aagcagtggt ttctttacgc 720
tgtatagcct gcggggtcaa cttgaactca agccgccaga gcaggattgt gggcggcgag 780
agcgcgctcc cgggggcctg gccctggcag gtcagcctgc acgtccagaa cgtccacgtg 840
tgcggaggct ccatcatcac ccccgagtgg atcgtgacag ccgcccactg cgtggaaaaa 900
cctcttaaca atccatggca ttggacggca tttgcgggga ttttgagaca atctttcatg 960
ttctatggag ccggatacca agtagaaaaa gtgatttctc atccaaatta tgactccaag 1020
accaagaaca atgacattgc gctgatgaag ctgcagaagc ctctgacttt caacgaccta 1080
gtgaaaccag tgtgtctgcc caacccaggc atgatgctgc agccagaaca gctctgctgg 1140
atttccgggt ggggggccac cgaggagaaa gggaagacct cagaagtgct gaacgctgcc 1200
aaggtgcttc tcattgagac acagagatgc aacagcagat atgtctatga caacctgatc 1260
acaccagcca tgatctgtgc cggcttcctg caggggaacg tcgattcttg ccagggtgac 1320
agtggagggc ctctggtcac ttcgaagaac aatatctggt ggctgatagg ggatacaagc 1380
tggggttctg gctgtgccaa agcttacaga ccaggagtgt acgggaatgt gatggtattc 1440
acggactgga tttatcgaca aatgagggca gacggc 1476

<210> 932
<211> 492
<212> PRT
<213> Homo sapiens

<400> 932
Met Ala Leu Asn Ser Gly Ser Pro Pro Ala Ile Gly Pro Tyr Tyr Glu
1               5                   10                  15
Asn His Gly Tyr Gln Pro Glu Asn Pro Tyr Pro Ala Gln Pro Thr Val
            20                  25                  30
Val Pro Thr Val Tyr Glu Val His Pro Ala Gln Tyr Tyr Pro Ser Pro
        35                  40                  45
Val Pro Gln Tyr Ala Pro Arg Val Leu Thr Gln Ala Ser Asn Pro Val
    50                  55                  60
Val Cys Thr Gln Pro Lys Ser Pro Ser Gly Thr Val Cys Thr Ser Lys
65                  70                  75                  80
Thr Lys Lys Ala Leu Cys Ile Thr Leu Thr Leu Gly Thr Phe Leu Val
                85                  90                  95
Gly Ala Ala Leu Ala Ala Gly Leu Leu Trp Lys Phe Met Gly Ser Lys
            100                 105                 110
Cys Ser Asn Ser Gly Ile Glu Cys Asp Ser Ser Gly Thr Cys Ile Asn
        115                 120                 125
Pro Ser Asn Trp Cys Asp Gly Val Ser His Cys Pro Gly Gly Glu Asp
    130                 135                 140
Glu Asn Arg Cys Val Arg Leu Tyr Gly Ser Asn Phe Ile Leu Gln Val
145                 150                 155                 160

Tyr Ser Ser Gln Arg Lys Ser Trp His Pro Val Cys Gln Asp Asp Trp
                165                 170                 175
Asn Glu Asn Tyr Gly Arg Ala Ala Cys Arg Asp Met Gly Tyr Lys Asn
            180                 185                 190
Asn Phe Tyr Ser Ser Gln Gly Ile Val Asp Asp Ser Gly Ser Thr Ser
        195                 200                 205
Phe Met Lys Leu Asn Thr Ser Ala Gly Asn Val Asp Ile Tyr Lys Lys
    210                 215                 220
Leu Tyr His Ser Asp Ala Cys Ser Ser Lys Ala Val Val Ser Leu Arg
225                 230                 235                 240
Cys Ile Ala Cys Gly Val Asn Leu Asn Ser Ser Arg Gln Ser Arg Ile
                245                 250                 255
Val Gly Gly Glu Ser Ala Leu Pro Gly Ala Trp Pro Trp Gln Val Ser
            260                 265                 270
Leu His Val Gln Asn Val His Val Cys Gly Gly Ser Ile Ile Thr Pro
        275                 280                 285
Glu Trp Ile Val Thr Ala Ala His Cys Val Glu Lys Pro Leu Asn Asn
    290                 295                 300
Pro Trp His Trp Thr Ala Phe Ala Gly Ile Leu Arg Gln Ser Phe Met
305                 310                 315                 320
Phe Tyr Gly Ala Gly Tyr Gln Val Glu Lys Val Ile Ser His Pro Asn
                325                 330                 335
Tyr Asp Ser Lys Thr Lys Asn Asn Asp Ile Ala Leu Met Lys Leu Gln
            340                 345                 350
Lys Pro Leu Thr Phe Asn Asp Leu Val Lys Pro Val Cys Leu Pro Asn
        355                 360                 365
Pro Gly Met Met Leu Gln Pro Glu Gln Leu Cys Trp Ile Ser Gly Trp
    370                 375                 380
Gly Ala Thr Glu Glu Lys Gly Lys Thr Ser Glu Val Leu Asn Ala Ala
385                 390                 395                 400
Lys Val Leu Leu Ile Glu Thr Gln Arg Cys Asn Ser Arg Tyr Val Tyr
                405                 410                 415
Asp Asn Leu Ile Thr Pro Ala Met Ile Cys Ala Gly Phe Leu Gln Gly
            420                 425                 430
Asn Val Asp Ser Cys Gln Gly Asp Ser Gly Gly Pro Leu Val Thr Ser
        435                 440                 445
Lys Asn Asn Ile Trp Trp Leu Ile Gly Asp Thr Ser Trp Gly Ser Gly
    450                 455                 460
Cys Ala Lys Ala Tyr Arg Pro Gly Val Tyr Gly Asn Val Met Val Phe
465                 470                 475                 480
Thr Asp Trp Ile Tyr Arg Gln Met Arg Ala Asp Gly
                485                 490

<210> 933
<211> 100
<212> PRT
<213> Homo sapiens

<400> 933
Met Ala Leu Asn Ser Gly Ser Pro Pro Ala Ile Gly Pro Tyr Tyr Glu
 1               5                  10                  15
Asn His Gly Tyr Gln Pro Glu Asn Pro Tyr Pro Ala Gln Pro Thr Val
            20                  25                  30
Val Pro Thr Val Tyr Glu Val His Pro Ala Gln Tyr Tyr Pro Ser Pro
        35                  40                  45
Val Pro Gln Tyr Ala Pro Arg Val Leu Thr Gln Ala Ser Asn Pro Val
    50                  55                  60
Val Cys Thr Gln Pro Lys Ser Pro Ser Gly Thr Val Cys Thr Ser Lys
65                  70                  75                  80
Thr Lys Lys Ala Leu Cys Ile Thr Leu Thr Leu Gly Thr Phe Leu Val
                85                  90                  95
Gly Ala Ala Leu
            100

<210> 934
<211> 393

<212> PRT
<213> Homo sapiens

<400> 934
Leu Ala Ala Gly Leu Leu Trp Lys Phe Met Gly Ser Lys Cys Ser Asn
1               5                   10                  15
Ser Gly Ile Glu Cys Asp Ser Ser Gly Thr Cys Ile Asn Pro Ser Asn
            20                  25                  30
Trp Cys Asp Gly Val Ser His Cys Pro Gly Gly Glu Asp Glu Asn Arg
        35                  40                  45
Cys Val Arg Leu Tyr Gly Ser Asn Phe Ile Leu Gln Val Tyr Ser Ser
    50                  55                  60
Gln Arg Lys Ser Trp His Pro Val Cys Gln Asp Asp Trp Asn Glu Asn
65                  70                  75                  80
Tyr Gly Arg Ala Ala Cys Arg Asp Met Gly Tyr Lys Asn Asn Phe Tyr
                85                  90                  95
Ser Ser Gln Gly Ile Val Asp Asp Ser Gly Ser Thr Ser Phe Met Lys
            100                 105                 110
Leu Asn Thr Ser Ala Gly Asn Val Asp Ile Tyr Lys Lys Leu Tyr His
        115                 120                 125
Ser Asp Ala Cys Ser Ser Lys Ala Val Val Ser Leu Arg Cys Ile Ala
    130                 135                 140
Cys Gly Val Asn Leu Asn Ser Ser Arg Gln Ser Arg Ile Val Gly Gly
145                 150                 155                 160
Glu Ser Ala Leu Pro Gly Ala Trp Pro Trp Gln Val Ser Leu His Val
                165                 170                 175
Gln Asn Val His Val Cys Gly Gly Ser Ile Ile Thr Pro Glu Trp Ile
            180                 185                 190
Val Thr Ala Ala His Cys Val Glu Lys Pro Leu Asn Asn Pro Trp His
        195                 200                 205
Trp Thr Ala Phe Ala Gly Ile Leu Arg Gln Ser Phe Met Phe Tyr Gly
    210                 215                 220
Ala Gly Tyr Gln Val Glu Lys Val Ile Ser His Pro Asn Tyr Asp Ser
225                 230                 235                 240
Lys Thr Lys Asn Asn Asp Ile Ala Leu Met Lys Leu Gln Lys Pro Leu
                245                 250                 255
Thr Phe Asn Asp Leu Val Lys Pro Val Cys Leu Pro Asn Pro Gly Met
            260                 265                 270
Met Leu Gln Pro Glu Gln Leu Cys Trp Ile Ser Gly Trp Gly Ala Thr
        275                 280                 285
Glu Glu Lys Gly Lys Thr Ser Glu Val Leu Asn Ala Ala Lys Val Leu
    290                 295                 300
Leu Ile Glu Thr Gln Arg Cys Asn Ser Arg Tyr Val Tyr Asp Asn Leu
305                 310                 315                 320
Ile Thr Pro Ala Met Ile Cys Ala Gly Phe Leu Gln Gly Asn Val Asp
                325                 330                 335
Ser Cys Gln Gly Asp Ser Gly Gly Pro Leu Val Thr Ser Lys Asn Asn
            340                 345                 350
Ile Trp Trp Leu Ile Gly Asp Thr Ser Trp Gly Ser Gly Cys Ala Lys
        355                 360                 365
Ala Tyr Arg Pro Gly Val Tyr Gly Asn Val Met Val Phe Thr Asp Trp
    370                 375                 380
Ile Tyr Arg Gln Met Arg Ala Asp Gly
385                 390


<210> 935
<211> 22
<212> DNA
<213> Artificial Sequence

<220>
<223> PCR Primer

<400> 935
gtgctgtggg agtccccgcg gc                                              22

<210> 936

<211> 36
<212> DNA
<213> Artificial Sequence

<220>
<223> PCR Primer

<400> 936
cgtgaactcg agtcattaga ttaacctcgt ggacgc                                36

<210> 937
<211> 22
<212> DNA
<213> Artificial Sequence

<220>
<223> PCR Primer

<400> 937
gtgctgtggg agtccccgcg gc                                               22

<210> 938
<211> 1158
<212> DNA
<213> Homo sapiens

<400> 938
catatgcagc atcaccacca tcaccacgtg ctgtgggagt ccccgcggca gtgcagcagc 60
tggacacttt gcgagggctt ttgctggctg ctgctgctgc ccgtcatgct actcatcgta 120
gcccgcccgg tgaagctcgc tgctttccct acctccttaa gtgactgcca aacgcccacc 180
ggctggaatt gctctggtta tgatgacaga gaaaatgatc tcttcctctg tgacaccaac 240
acctgtaaat ttgatgggga atgtttaaga attggagaca ctgtgacttg cgtctgtcag 300
ttcaagtgca acaatgacta tgtgcctgtg tgtggctcca atggggagag ctaccagaat 360
gagtgttacc tgcgacaggc tgcatgcaaa cagcagagtg agatacttgt ggtgtcagaa 420
ggatcatgtg ccacagatgc aggatcagga tctggagatg gagtccatga aggctctgga 480
gaaactagtc aaaaggagac atccacctgt gatatttgcc agtttggtgc agaatgtgac 540
gaagatgccg aggatgtctg gtgtgtgtgt aatattgact gttctcaaac caacttcaat 600
cccctctgcg cttctgatgg gaaatcttat gataatgcat gccaaatcaa agaagcatcg 660
tgtcagaaac aggagaaaat tgaagtcatg tctttgggtc gatgtcaaga taacacaact 720
acaactacta agtctgaaga tgggcattat gcaagaacag attatgcaga gaatgctaac 780
aaattagaag aaagtgccag agaacaccac ataccttgtc cggaacatta caatggcttc 840
tgcatgcatg ggaagtgtga gcattctatc aatatgcagg agccatcttg caggtgtgat 900
gctggttata ctggacaaca ctgtgaaaaa aaggactaca gtgttctata cgttgttccc 960
ggtcctgtac gatttcagta tgtcttaatc gcagctgtga ttggaacaat tcagattgct 1020
gtcatctgtg tggtggtcct ctgcatcaca aggaaatgcc ccagaagcaa cagaattcac 1080
agacagaagc aaaatacagg gcactacagt tcagacaata caacaagagc gtccacgagg 1140
ttaatctaat gactcgag                                                    1158

<210> 939
<211> 1020
<212> DNA
<213> Homo sapiens

<400> 939
atgcagcatc accaccatca ccacgactgc caaacgccca ccggctggaa ttgctctggt 60
tatgatgaca gagaaaatga tctcttcctc tgtgacacca acacctgtaa atttgatggg 120
gaatgtttaa gaattggaga cactgtgact tgcgtctgtc agttcaagtg caacaatgac 180
tatgtgcctg tgtgtggctc caatggggag agctaccaga atgagtgtta cctgcgacag 240
gctgcatgca aacagcagag tgagatactt gtggtgtcag aaggatcatg tgccacagat 300
gcaggatcag gatctggaga tggagtccat gaaggctctg gagaaactag tcaaaaggag 360
acatccacct gtgatatttg ccagtttggt gcagaatgtg acgaagatgc cgaggatgtc 420
tggtgtgtgt gtaatattga ctgttctcaa accaacttca atcccctctg cgcttctgat 480
gggaaatctt atgataatgc atgccaaatc aaagaagcat cgtgtcagaa acaggagaaa 540
attgaagtca tgtctttggg tcgatgtcaa gataacacaa ctacaactac taagtctgaa 600
gatgggcatt atgcaagaac agattatgca gagaatgcta acaaattaga agaaagtgcc 660
agagaacacc acataccttg tccggaacat tacaatggct tctgcatgca tgggaagtgt 720
gagcattcta tcaatatgca ggagccatct tgcaggtgtg atgctggtta tactggacaa 780
cactgtgaaa aaaaggacta cagtgttcta tacgttgttc ccggtcctgt acgatttcag 840

tatgtcttaa tcgcagctgt gattggaaca attcagattg ctgtcatctg tgtggtggtc 900
ctctgcatca caaggaaatg ccccagaagc aacagaattc acagacagaa gcaaaataca 960
gggcactaca gttcagacaa tacaacaaga gcgtccacga ggttaatcta atgactcgag 1020

<210> 940
<211> 336
<212> PRT
<213> Homo sapiens

<400> 940
Met Gln His His His His His His Asp Cys Gln Thr Pro Thr Gly Trp
1               5                   10                  15
Asn Cys Ser Gly Tyr Asp Asp Arg Glu Asn Asp Leu Phe Leu Cys Asp
            20                  25                  30
Thr Asn Thr Cys Lys Phe Asp Gly Glu Cys Leu Arg Ile Gly Asp Thr
        35                  40                  45
Val Thr Cys Val Cys Gln Phe Lys Cys Asn Asn Asp Tyr Val Pro Val
    50                  55                  60
Cys Gly Ser Asn Gly Glu Ser Tyr Gln Asn Glu Cys Tyr Leu Arg Gln
65                  70                  75                  80
Ala Ala Cys Lys Gln Gln Ser Glu Ile Leu Val Val Ser Glu Gly Ser
                85                  90                  95
Cys Ala Thr Asp Ala Gly Ser Gly Ser Gly Asp Gly Val His Glu Gly
            100                 105                 110
Ser Gly Glu Thr Ser Gln Lys Glu Thr Ser Thr Cys Asp Ile Cys Gln
        115                 120                 125
Phe Gly Ala Glu Cys Asp Glu Asp Ala Glu Asp Val Trp Cys Val Cys
    130                 135                 140
Asn Ile Asp Cys Ser Gln Thr Asn Phe Asn Pro Leu Cys Ala Ser Asp
145                 150                 155                 160
Gly Lys Ser Tyr Asp Asn Ala Cys Gln Ile Lys Glu Ala Ser Cys Gln
                165                 170                 175
Lys Gln Glu Lys Ile Glu Val Met Ser Leu Gly Arg Cys Gln Asp Asn
            180                 185                 190
Thr Thr Thr Thr Thr Lys Ser Glu Asp Gly His Tyr Ala Arg Thr Asp
        195                 200                 205
Tyr Ala Glu Asn Ala Asn Lys Leu Glu Glu Ser Ala Arg Glu His His
    210                 215                 220
Ile Pro Cys Pro Glu His Tyr Asn Gly Phe Cys Met His Gly Lys Cys
225                 230                 235                 240
Glu His Ser Ile Asn Met Gln Glu Pro Ser Cys Arg Cys Asp Ala Gly
                245                 250                 255
Tyr Thr Gly Gln His Cys Glu Lys Lys Asp Tyr Ser Val Leu Tyr Val
            260                 265                 270
Val Pro Gly Pro Val Arg Phe Gln Tyr Val Leu Ile Ala Ala Val Ile
        275                 280                 285
Gly Thr Ile Gln Ile Ala Val Ile Cys Val Val Val Leu Cys Ile Thr
    290                 295                 300
Arg Lys Cys Pro Arg Ser Asn Arg Ile His Arg Gln Lys Gln Asn Thr
305                 310                 315                 320
Gly His Tyr Ser Ser Asp Asn Thr Thr Arg Ala Ser Thr Arg Leu Ile
                325                 330                 335

<210> 941
<211> 381
<212> PRT
<213> Homo sapiens

<400> 941
Met Gln His His His His His His Val Leu Trp Glu Ser Pro Arg Gln
1               5                   10                  15
Cys Ser Ser Trp Thr Leu Cys Glu Gly Phe Cys Trp Leu Leu Leu Leu
            20                  25                  30
Pro Val Met Leu Leu Ile Val Ala Arg Pro Val Lys Leu Ala Ala Phe
        35                  40                  45
Pro Thr Ser Leu Ser Asp Cys Gln Thr Pro Thr Gly Trp Asn Cys Ser

50                  55                  60
Gly Tyr Asp Asp Arg Glu Asn Asp Leu Phe Leu Cys Asp Thr Asn Thr
65                  70                  75                  80
Cys Lys Phe Asp Gly Glu Cys Leu Arg Ile Gly Asp Thr Val Thr Cys
                85                  90                  95
Val Cys Gln Phe Lys Cys Asn Asn Asp Tyr Val Pro Val Cys Gly Ser
            100                 105                 110
Asn Gly Glu Ser Tyr Gln Asn Glu Cys Tyr Leu Arg Gln Ala Ala Cys
        115                 120                 125
Lys Gln Gln Ser Glu Ile Leu Val Val Ser Glu Gly Ser Cys Ala Thr
    130                 135                 140
Asp Ala Gly Ser Gly Ser Gly Asp Gly Val His Glu Gly Ser Gly Glu
145                 150                 155                 160
Thr Ser Gln Lys Glu Thr Ser Thr Cys Asp Ile Cys Gln Phe Gly Ala
                165                 170                 175
Glu Cys Asp Glu Asp Ala Glu Asp Val Trp Cys Val Cys Asn Ile Asp
            180                 185                 190
Cys Ser Gln Thr Asn Phe Asn Pro Leu Cys Ala Ser Asp Gly Lys Ser
        195                 200                 205
Tyr Asp Asn Ala Cys Gln Ile Lys Glu Ala Ser Cys Gln Lys Gln Glu
    210                 215                 220
Lys Ile Glu Val Met Ser Leu Gly Arg Cys Gln Asp Asn Thr Thr Thr
225                 230                 235                 240
Thr Thr Lys Ser Glu Asp Gly His Tyr Ala Arg Thr Asp Tyr Ala Glu
                245                 250                 255
Asn Ala Asn Lys Leu Glu Glu Ser Ala Arg Glu His His Ile Pro Cys
            260                 265                 270
Pro Glu His Tyr Asn Gly Phe Cys Met His Gly Lys Cys Glu His Ser
        275                 280                 285
Ile Asn Met Gln Glu Pro Ser Cys Arg Cys Asp Ala Gly Tyr Thr Gly
    290                 295                 300
Gln His Cys Glu Lys Lys Asp Tyr Ser Val Leu Tyr Val Val Pro Gly
305                 310                 315                 320
Pro Val Arg Phe Gln Tyr Val Leu Ile Ala Ala Val Ile Gly Thr Ile
                325                 330                 335
Gln Ile Ala Val Ile Cys Val Val Val Leu Cys Ile Thr Arg Lys Cys
            340                 345                 350
Pro Arg Ser Asn Arg Ile His Arg Gln Lys Gln Asn Thr Gly His Tyr
        355                 360                 365
Ser Ser Asp Asn Thr Thr Arg Ala Ser Thr Arg Leu Ile
    370                 375                 380

<210> 942
<211> 45
<212> DNA
<213> Homo sapiens

<400> 942
ctgctggcga acggcagaat gcctaccgtg ctgcagtgcg tgaac                45

<210> 943
<211> 15
<212> PRT
<213> Homo sapiens

<400> 943
Leu Leu Ala Asn Gly Arg Met Pro Thr Val Leu Gln Cys Val Asn
1               5                   10                  15

<210> 944
<211> 1883
<212> DNA
<213> Homo sapiens

<400> 944

tactttccaa ccagcaggtg aagagctgaa tcactaaggg cacaaatgaa aagggagggg 60
caaaatatga aggctttata tttcttctga gggacgcagc tccagtctaa cttgccactc 120
tgtgaacttc actactggaa agcaacaaag gcagtcggca taaaaatggg ttctctcagc 180
acagctaacg ttgaattttg ccttgatgtg ttcaaagagc tgaacagtaa caacatagga 240
gataacatct tcttttcttc gctgagtctg ctttatgctc taagcatggt cctccttggt 300
gccaggggag agactgcaga gcaattggag aaggtgcttc attttagtca tactgtagac 360
tcattaaaac cagggttcaa ggactcacct aagtgcagcc aagctggaag aattcattcc 420
gagtttggtg tcgaattctc tcaaatcaac cagccagact ctaactgtac cctcagcatt 480
gccaacaggc tctacgggac aaagacgatg gcatttcatc agcaatattt aagctgttct 540
gagaaatggt atcaagccag gttgcaaact gtggattttg aacagtctac agaagaaacg 600
aggaaaatga ttaatgcttg ggttgaaaat aaaactaatg gaaaagtcgc aaatctcttt 660
ggaaagagca caattgaccc ttcatctgta atggtcctgg tgaataccat atatttcaaa 720
ggacaaaggc aaaataaatt tcaaggtaaa aatgtaactg tggaaatgat gtatcaaatt 780
ggaacattta aactggcctt tgtaaaggag ccgcagatgc aagttcttga gctgccctac 840
gttaacaaca aattaagcat gattattctg cttccagtag gcatagctaa tctgaaacag 900
atagaaaagc agctgaattc ggggacgttt catgagtgga caagctcttc taacatgatg 960
gaaagagaag ttgaagtaca cctccccaga ttcaaacttg aaattaagta tgagctaaat 1020
tccctgttaa aacctctagg ggtgacagat ctcttcaacc aggtcaaagc tgatctttct 1080
ggaatgtcac caaccaaggg cctatattta tcaaaagcca tccacaagtc atacctggat 1140
gtcagcgaag agggcacgga ggcagcagca gccactgggg acagcatcgc tgtaaaaagc 1200
ctaccaatga gagctcagtt caaggcgaac caccccttcc tgttctttat aaggcacact 1260
cataccaaca cgatcctatt ctgtggcaag cttgcctctc cctaatcaga tggggttgag 1320
taaggctcag agttgcagat gaggtgcaga gacaatcctg tgactttccc acggccaaaa 1380
agctgttcac acctcacaca cctctgtgcc tcagtttgct catctgcaaa ataggtctag 1440
gatttcttcc aaccatttca tgagttgtga agctaaggct ttgttaatca tggaaaaagg 1500
tagacttatg cagaaagcct ttctggcttt cttatctgtg gtgtctcatt tgagtgctgt 1560
ccagtgacat gatcaagtca atgagtaaaa ttttaaggga ttagattttc ttgacttgta 1620
tgtatctgtg agatcttgaa taagtgacct gacatctctg cttaaagaaa accagctgaa 1680
gggcttcaac tttgcttgga tttttaaata ttttccttgc atatgtaaat agaatgtggt 1740
gagttttagt tcaaaattct ctgttgagaa taataaatgc atgaaatacc ttaaagctct 1800
gtgaagactt gtaacatggc agcaatcaaa tggcttataa aaggatactt tgaatgtgga 1860
taaattgaaa aaaaaaaaaa aaa 1883

<210> 945
<211> 2471
<212> DNA
<213> Homo sapiens

<400> 945
tggaaagcaa caaaggcagt cggcataaaa atgggttctc tcagcacagc taacgttgaa 60
ttttgccttg atgtgttcaa agagctgaac agtaacaaca taggagataa catcttcttt 120
tcttcgctga gtctgcttta tgctctaagc atggtcctcc ttggtgccag gggagagact 180
gcagagcaat tggagaaggt gcttcatttt agtcatactg tagactcatt aaaaccaggg 240
ttcaaggact cacctaagtg cagccaagct ggaagaattc attccgagtt tggtgtctaa 300
ttctctcaaa tcaaccagcc agactctaac tgtaccctca gcattgccaa caggctctac 360
gggacaaaga cgatggcatt tcatcagcaa tatttaagct gttctgagaa atggtatcaa 420
gccaggttgc aaactgtgga ttttgaacag tctacagaag aaacgaggaa aacgattaat 480
gcttgggttg aaaataaaac taatggaaaa gtcgcaaatc tctttggaaa gagcacaatt 540
gacccttcat ctgtaatggt cctggtgaat gccatatatt tcaaaggaca atggcaaaat 600
aaatttcaag taagagagac agttaaaagt ccttttcagc taagtgaggt aagtatttta 660
ttttcagact catgacaaat gttggaggat acaataatca tttaaggaca atttagaaag 720
atgtagtgat ttagtgaaaa tattggtcta ggtttctgtt ggttcttttt attgtatttt 780
ctacagattt tcatttttcc tttattaagt gacaataact tttatcacag agcacctaac 840
tggagacttg ggatactaag attctctggc agaggagaaa acccattctt ttcttaacgc 900
tctctcggct tattctttcc atatatactg tggttatgtt ctccagcagc acactaaggc 960
tatctgtgtt attttttttt ctagagagtt tcttgaggaa agattaaaaa gaaaatgatt 1020
taatgatata tcctttttct tcccactaaa atcatgttag ctaaaaatcc tgactttgtt 1080
tttggtagac caagctatct ctatatatta ttagaacaat tcagtctcta accatgactt 1140
attttaaatt aaaaaaatgg ataacctttg caaagggaag taataaggaa tctactttgt 1200
agtttctatt ttacccatgc ctttagagtg ttcatgcaga tatccgtgtt acgcaaggta 1260
atcagtacac acacatgtgc actcacatat gtaagtatac tataatcttt ttcttcttaa 1320
cagggtaaaa atgtaactgt ggaaatgatg tatcaaattg gaacatttaa actggccttt 1380
gtaaaggagc cgcagatgca agttcttgag ctgccctacg ttaacaacaa attaagcatg 1440
attattctgc ttccagtagg catagctaat ctgaaacaga tagaaaagca gctgaattcg 1500
gggacgtttc atgagtggac aagctcttct aacatgatgg aaagagaagt tgaagtacac 1560
ctcccccgat tcaaacttga aactaagtat gagctaaatt ccctgttaaa atctctaggg 1620
gtgacagatc tcttcaacca ggtcaaagct gatctttctg gaatgtcacc aaccaagggc 1680
ctatatttat caaaagccat ccacaagtca tacctggatg tcagcgaaga gggcacggag 1740

gcagcagcag ccactgggga cagcatcgct gtaaaaagcc taccaatgag agctcagttc 1800
aaggcgaacc accccttcct tttctttata aggcacactc ataccaacac gatcctattc 1860
tgtggcaagc ttgcctctcc ctaatcagat ggggttgagc aaggctcaga gttgcagatg 1920
aggtgcagag acaatcctgt gactttccca cggccaaaaa gctgttcaca cctcacacac 1980
ctctgtgcct cagtttgctc atctgcaaaa taggtctagg atttcttcca accatttcat 2040
gagttgtgaa gctaaggctt tgttaatcat ggaaaaaggt agatttatgc agaaagcctt 2100
tctggctttc ttatctgtgg tgtctcattt gagtgctgtc cagtgacatg atcaagtcaa 2160
tgagtaaaat tttaagggat tagattttct tgacttgtat gtatctgtga gatcttgaat 2220
aagtgacctg acatctctgc ttaaagaaaa ccagctgaag ggcttcaact ttgcttggat 2280
ttttaaatat tttccttgca tatgtaaata gaatgtggtg agttttagtt caaaattctc 2340
tgttgagaat aataaatgca tgaaatacct taaagctctg tgaagacttg taacatggca 2400
gcaatcaaat ggcttataaa aggatacttt gaatgtggat aaattgaatt caaaaaaaaa 2460
aaaaaaaaaa a 2471

<210> 946
<211> 379
<212> PRT
<213> Homo sapiens

<400> 946
Met Gly Ser Leu Ser Thr Ala Asn Val Glu Phe Cys Leu Asp Val Phe
1               5                   10                  15

Lys Glu Leu Asn Ser Asn Asn Ile Gly Asp Asn Ile Phe Phe Ser Ser
            20                  25                  30

Leu Ser Leu Leu Tyr Ala Leu Ser Met Val Leu Leu Gly Ala Arg Gly
        35                  40                  45

Glu Thr Ala Glu Gln Leu Glu Lys Val Leu His Phe Ser His Thr Val
    50                  55                  60

Asp Ser Leu Lys Pro Gly Phe Lys Asp Ser Pro Lys Cys Ser Gln Ala
65                  70                  75                  80

Gly Arg Ile His Ser Glu Phe Gly Val Glu Phe Ser Gln Ile Asn Gln
                85                  90                  95

Pro Asp Ser Asn Cys Thr Leu Ser Ile Ala Asn Arg Leu Tyr Gly Thr
            100                 105                 110

Lys Thr Met Ala Phe His Gln Gln Tyr Leu Ser Cys Ser Glu Lys Trp
        115                 120                 125

Tyr Gln Ala Arg Leu Gln Thr Val Asp Phe Glu Gln Ser Thr Glu Glu
    130                 135                 140

Thr Arg Lys Met Ile Asn Ala Trp Val Glu Asn Lys Thr Asn Gly Lys
145                 150                 155                 160

Val Ala Asn Leu Phe Gly Lys Ser Thr Ile Asp Pro Ser Ser Val Met
                165                 170                 175

Val Leu Val Asn Thr Ile Tyr Phe Lys Gly Gln Arg Gln Asn Lys Phe
            180                 185                 190

Gln Gly Lys Asn Val Thr Val Glu Met Met Tyr Gln Ile Gly Thr Phe
        195                 200                 205

Lys Leu Ala Phe Val Lys Glu Pro Gln Met Gln Val Leu Glu Leu Pro
    210                 215                 220

Tyr Val Asn Asn Lys Leu Ser Met Ile Ile Leu Leu Pro Val Gly Ile
225                 230                 235                 240

Ala Asn Leu Lys Gln Ile Glu Lys Gln Leu Asn Ser Gly Thr Phe His
                245                 250                 255

Glu Trp Thr Ser Ser Ser Asn Met Met Glu Arg Glu Val Glu Val His
            260                 265                 270

Leu Pro Arg Phe Lys Leu Glu Ile Lys Tyr Glu Leu Asn Ser Leu Leu
        275                 280                 285

Lys Pro Leu Gly Val Thr Asp Leu Phe Asn Gln Val Lys Ala Asp Leu
    290                 295                 300

Ser Gly Met Ser Pro Thr Lys Gly Leu Tyr Leu Ser Lys Ala Ile His
305                 310                 315                 320

Lys Ser Tyr Leu Asp Val Ser Glu Glu Gly Thr Glu Ala Ala Ala Ala
                325                 330                 335

Thr Gly Asp Ser Ile Ala Val Lys Ser Leu Pro Met Arg Ala Gln Phe
            340                 345                 350

Lys Ala Asn His Pro Phe Leu Phe Phe Ile Arg His Thr His Thr Asn
        355                 360                 365

Thr Ile Leu Phe Cys Gly Lys Leu Ala Ser Pro
    370                 375


<210> 947
<211> 617
<212> PRT
<213> Homo sapiens

<220>
<221> VARIANT
<222> 90, 215, 225, 232, 233, 289, 309, 318, 322, 331, 332, 367,
369, 374, 378, 425, 430
<223> Xaa = Any Amino Acid

<400> 947
Met Gly Ser Leu Ser Thr Ala Asn Val Glu Phe Cys Leu Asp Val Phe
 1               5                  10                  15
Lys Glu Leu Asn Ser Asn Asn Ile Gly Asp Asn Ile Phe Phe Ser Ser
            20                  25                  30
Leu Ser Leu Leu Tyr Ala Leu Ser Met Val Leu Leu Gly Ala Arg Gly
        35                  40                  45
Glu Thr Ala Glu Gln Leu Glu Lys Val Leu His Phe Ser His Thr Val
    50                  55                  60
Asp Ser Leu Lys Pro Gly Phe Lys Asp Ser Pro Lys Cys Ser Gln Ala
65                  70                  75                  80
Gly Arg Ile His Ser Glu Phe Gly Val Xaa Phe Ser Gln Ile Asn Gln
                85                  90                  95
Pro Asp Ser Asn Cys Thr Leu Ser Ile Ala Asn Arg Leu Tyr Gly Thr
            100                 105                 110
Lys Thr Met Ala Phe His Gln Gln Tyr Leu Ser Cys Ser Glu Lys Trp
        115                 120                 125
Tyr Gln Ala Arg Leu Gln Thr Val Asp Phe Glu Gln Ser Thr Glu Glu
    130                 135                 140
Thr Arg Lys Thr Ile Asn Ala Trp Val Glu Asn Lys Thr Asn Gly Lys
145                 150                 155                 160
Val Ala Asn Leu Phe Gly Lys Ser Thr Ile Asp Pro Ser Ser Val Met
                165                 170                 175
Val Leu Val Asn Ala Ile Tyr Phe Lys Gly Gln Trp Gln Asn Lys Phe
            180                 185                 190
Gln Val Arg Glu Thr Val Lys Ser Pro Phe Gln Leu Ser Glu Val Ser
        195                 200                 205
Ile Leu Phe Ser Asp Ser Xaa Gln Met Leu Glu Asp Thr Ile Ile Ile
    210                 215                 220
Xaa Gly Gln Phe Arg Lys Met Xaa Xaa Phe Ser Glu Asn Ile Gly Leu
225                 230                 235                 240
Gly Phe Cys Trp Phe Phe Leu Leu Tyr Phe Leu Gln Ile Phe Ile Phe

                    245                 250                 255
Pro Leu Leu Ser Asp Asn Asn Phe Tyr His Arg Ala Pro Asn Trp Arg
                260                 265                 270
Leu Gly Ile Leu Arg Phe Ser Gly Arg Gly Glu Asn Pro Phe Phe Ser
        275                 280                 285
Xaa Arg Ser Leu Gly Leu Phe Phe Pro Tyr Ile Leu Trp Leu Cys Ser
    290                 295                 300
Pro Ala Ala His Xaa Gly Tyr Leu Cys Tyr Phe Phe Phe Xaa Arg Val
305                 310                 315                 320
Ser Xaa Gly Lys Ile Lys Lys Lys Met Ile Xaa Xaa Tyr Ile Leu Phe
                325                 330                 335
Leu Pro Thr Lys Ile Met Leu Ala Lys Asn Pro Asp Phe Val Phe Gly
            340                 345                 350
Arg Pro Ser Tyr Leu Tyr Ile Leu Leu Glu Gln Phe Ser Leu Xaa Pro
        355                 360                 365
Xaa Leu Ile Leu Asn Xaa Lys Asn Gly Xaa Pro Leu Gln Arg Glu Val
    370                 375                 380
Ile Arg Asn Leu Leu Cys Ser Phe Tyr Phe Thr His Ala Phe Arg Val
385                 390                 395                 400
Phe Met Gln Ile Ser Val Leu Arg Lys Val Ile Ser Thr His Thr Cys
                405                 410                 415
Ala Leu Thr Tyr Val Ser Ile Leu Xaa Ser Phe Ser Ser Xaa Gln Gly
            420                 425                 430
Lys Asn Val Thr Val Glu Met Met Tyr Gln Ile Gly Thr Phe Lys Leu
        435                 440                 445
Ala Phe Val Lys Glu Pro Gln Met Gln Val Leu Glu Leu Pro Tyr Val
    450                 455                 460
Asn Asn Lys Leu Ser Met Ile Ile Leu Leu Pro Val Gly Ile Ala Asn
465                 470                 475                 480
Leu Lys Gln Ile Glu Lys Gln Leu Asn Ser Gly Thr Phe His Glu Trp
                485                 490                 495
Thr Ser Ser Ser Asn Met Met Glu Arg Glu Val Glu Val His Leu Pro
            500                 505                 510
Arg Phe Lys Leu Glu Thr Lys Tyr Glu Leu Asn Ser Leu Leu Lys Ser
        515                 520                 525
Leu Gly Val Thr Asp Leu Phe Asn Gln Val Lys Ala Asp Leu Ser Gly
    530                 535                 540
Met Ser Pro Thr Lys Gly Leu Tyr Leu Ser Lys Ala Ile His Lys Ser
545                 550                 555                 560
Tyr Leu Asp Val Ser Glu Glu Gly Thr Glu Ala Ala Ala Ala Thr Gly
                565                 570                 575
Asp Ser Ile Ala Val Lys Ser Leu Pro Met Arg Ala Gln Phe Lys Ala
            580                 585                 590
Asn His Pro Phe Leu Phe Phe Ile Arg His Thr His Thr Asn Thr Ile
        595                 600                 605
Leu Phe Cys Gly Lys Leu Ala Ser Pro
    610                 615

<210> 948
<211> 729
<212> DNA
<213> Homo sapiens

<400> 948
atgcagcatc accaccatca ccacctcagg gttccggagc cgcggcccgg ggaggcgaaa 60
gcggaggggg ccgcgccgcc gaccccgtcc aagccgctca cgtccttcct catccaggac 120
atcctgcggg acggcgcgca gcggcaaggc ggccgcacga gcagccagag acagcgcgac 180
ccggagccgg agccagagcc agagccagag ggaggacgca gccgcgccgg ggcgcagaac 240
gaccagctga gcaccgggcc ccgcgccgcg ccggaggagg ccgagacgct ggcagagacc 300
gagccagaaa ggcacttggg gtcttatctg ttggactctg aaaacacttc aggcgccctt 360
ccaaggcttc cccaaacccc taagcagccg cagaagcgct cccgagctgc cttctcccac 420
actcaggtga tcgagttgga gaggaagttc agccatcaga agtacctgtc ggcccctgaa 480
cgggcccacc tggccaagaa cctcaagctc acggagaccc aagtgaagat atggttccag 540
aacagacgct ataagactaa gcgaaagcag ctctcctcgg agctgggaga cttggagaag 600
cactcctctt tgccggccct gaaagaggag gccttctccc gggcctccct ggtctccgtg 660
tataacagct atccttacta cccatacctg tactgcgtgg gcagctggag cccagctttt 720

tggtaatga                                                          729

<210> 949
<211> 16
<212> PRT
<213> Homo sapiens

<400> 949
Arg Glu Ile Ser Phe Glu Ala Cys Leu Thr Gln Met Phe Phe Ile His
1               5                   10                  15

<210> 950
<211> 18
<212> PRT
<213> Homo sapiens

<400> 950
Asp Arg Tyr Val Ala Ile Cys His Pro Leu Arg His Ala Ala Val Leu
1               5                   10                  15
Asn Asn

<210> 951
<211> 13
<212> PRT
<213> Homo sapiens

<400> 951
Lys Arg Leu Ala Phe Cys His Ser Asn Val Leu Ser His
1               5                   10

<210> 952
<211> 15
<212> PRT
<213> Homo sapiens

<400> 952
His Gln Asp Val Met Lys Leu Ala Tyr Ala Asp Thr Leu Pro Asn
1               5                   10                  15

<210> 953
<211> 14
<212> PRT
<213> Homo sapiens

<400> 953
Arg Thr Val Leu Gln Leu Pro Ser Lys Ser Glu Arg Ala Lys
1               5                   10

<210> 954
<211> 12
<212> PRT
<213> Homo sapiens

<400> 954
His Arg Phe Gly Asn Ser Leu His Pro Ile Val Arg
1               5                   10

<210> 955
<211> 15
<212> PRT
<213> Homo sapiens

<400> 955
Asn Pro Ile Ile Tyr Gly Ala Lys Thr Lys Gln Ile Arg Thr Arg
1               5                   10                  15

<210> 956
<211> 14
<212> PRT
<213> Homo sapiens

<400> 956
Lys Ile Ser Cys Asp Lys Asp Leu Gln Ala Val Gly Gly Lys
1               5                   10

<210> 957
<211> 15
<212> PRT
<213> Homo sapiens

<400> 957
Tyr Ser Glu Gly Lys Ile Phe Phe Tyr Phe Leu Gly Asn Gln Ala
1               5                   10                  15

<210> 958
<211> 15
<212> PRT
<213> Homo sapiens

<400> 958
Val Thr Gln Ile His Lys Thr Leu Ser His Gly Asp Ile Thr Met
1               5                   10                  15

<210> 959
<211> 15
<212> PRT
<213> Homo sapiens

<400> 959
Ile Thr Thr Trp Thr His Cys His Thr Thr Thr Gly Thr Arg Asp
1               5                   10                  15

<210> 960
<211> 15
<212> PRT
<213> Homo sapiens

<400> 960
Leu His Thr Ile Thr Pro Pro His Thr Leu Pro Val Asp Thr Arg
1               5                   10                  15

<210> 961
<211> 15
<212> PRT
<213> Homo sapiens

<400> 961
Gly Met Ala Arg Phe Pro Gln Pro Glu Cys Leu Pro Pro Tyr Cys
1               5                   10                  15

<210> 962
<211> 15
<212> PRT
<213> Homo sapiens

<400> 962
Met Ala Leu Arg Ala Ser Trp Leu Pro Gly Gly Gly Pro Gln Ala
1               5                   10                  15

<210> 963
<211> 15
<212> PRT
<213> Homo sapiens

<400> 963
Ala Tyr Ala Cys Arg Ala Gly Pro Gly Trp Leu Lys Glu Gln Pro
1               5                   10                  15

<210> 964
<211> 15
<212> PRT
<213> Homo sapiens

<400> 964
Gln Ser Leu Pro Gln Gly Ser Tyr Val Thr Val Gly Phe Leu Val
1               5                   10                  15

<210> 965
<211> 15
<212> PRT
<213> Homo sapiens

<400> 965
Pro Gln Ile Cys Ala Cys Gln Thr Arg Pro Asn Trp Leu Asn Glu
1               5                   10                  15

<210> 966
<211> 15
<212> PRT
<213> Homo sapiens

<400> 966
His Leu Arg Ser Lys Val Tyr Ala Ala Phe Gly Gly Ser Ser Pro
1               5                   10                  15

<210> 967
<211> 1292
<212> DNA
<213> Homo sapiens

<400> 967
tgtggtcatt gaggacaagt caacgagaga ttcccgagta cgtctacagt gagccttgtg 60
ggtgaaggta ctctacagtg tggtcattga ggacaagttg acgagagagt cccaagtacg 120
tccacggtca gccttgcggt aagcttgtgt gcttagagga acccagggta acgatggggc 180
aaactgaaag taaatatgcc tcttatctca gctttattaa aattctttta agaagagggg 240
gagttagagc ttctacagaa aatctaatta cgctatttca aacaatagaa caattctgcc 300
catggtttcc agaacaggga actttagatc taaaagattg ggaaaaaatt ggcaaagaat 360
taaaacaagc aaatagggaa ggtaaaatca tcccacttac agtatggaat gattgggcca 420
ttattaaagc aactttagaa ccatttcaaa caggagaaga tattgtttca gtttctgatg 480
cccctaaaag ctgtgtaaca gattgtgaag aagaggcagg gacagaatcc cagcaaggaa 540
cggaaagttc acattgtaaa tatgtagcag agtctgtaat ggctcagtca acgcaaaatg 600
ttgactacag tcaattacag gagataatat accctgaatc atcaaaattg ggggaaggag 660
gtccagaatc attggggcca tcagagccta aaccacgatc gccatcaact cctcctcccg 720
tggttcagat gcctgtaaca ttacaacctc aaacgcaggt tagacaagca caaaccccaa 780
gagaaaatca agtagaaagg gacagagtct ctatcccggc aatgccaact cagatacagt 840
atccacaata tcagccggta gaaaataaga cccaaccgct ggtagtttat caataccggc 900
tgccaaccga gcttcagtat cggcctcctt cagaggttca atacagacct caagcggtgt 960
gtcctgtgcc aaatagcacg gcaccatacc agcaacccac agcgatggcg tctaattcac 1020
cagcaacaca ggacgcggcg ctgtatcctc agccgcccac tgtgagactt aatcctacag 1080
catcacgtag tggacagggt ggtgcactgc atgcagtcat tgatgaagcc agaaaacagg 1140
gcgatcttga ggcatggcgg ttcctggtaa ttttacaact ggtacaggcc ggggaagaga 1200
ctcaagtagg agcgcctgcc cgagctgaga ctagatgtga acctttcacc atgaaaatgt 1260
taaaagatat aaaggaagga gttaaacaat at                                1292

<210> 968
<211> 373
<212> PRT
<213> Homo sapiens

<400> 968
Met Gly Gln Thr Glu Ser Lys Tyr Ala Ser Tyr Leu Ser Phe Ile Lys
                5                   10                  15

Ile Leu Leu Arg Arg Gly Gly Val Arg Ala Ser Thr Glu Asn Leu Ile
            20                  25                  30

Thr Leu Phe Gln Thr Ile Glu Gln Phe Cys Pro Trp Phe Pro Glu Gln
        35                  40                  45

Gly Thr Leu Asp Leu Lys Asp Trp Glu Lys Ile Gly Lys Glu Leu Lys
    50                  55                  60

Gln Ala Asn Arg Glu Gly Lys Ile Ile Pro Leu Thr Val Trp Asn Asp
65                  70                  75                  80

Trp Ala Ile Ile Lys Ala Thr Leu Glu Pro Phe Gln Thr Gly Glu Asp
                85                  90                  95

Ile Val Ser Val Ser Asp Ala Pro Lys Ser Cys Val Thr Asp Cys Glu
            100                 105                 110

Glu Glu Ala Gly Thr Glu Ser Gln Gln Gly Thr Glu Ser Ser His Cys
        115                 120                 125

Lys Tyr Val Ala Glu Ser Val Met Ala Gln Ser Thr Gln Asn Val Asp
    130                 135                 140

Tyr Ser Gln Leu Gln Glu Ile Ile Tyr Pro Glu Ser Ser Lys Leu Gly
145                 150                 155                 160

Glu Gly Gly Pro Glu Ser Leu Gly Pro Ser Glu Pro Lys Pro Arg Ser
                165                 170                 175

Pro Ser Thr Pro Pro Pro Val Val Gln Met Pro Val Thr Leu Gln Pro
            180                 185                 190

Gln Thr Gln Val Arg Gln Ala Gln Thr Pro Arg Glu Asn Gln Val Glu
        195                 200                 205

Arg Asp Arg Val Ser Ile Pro Ala Met Pro Thr Gln Ile Gln Tyr Pro
    210                 215                 220

Gln Tyr Gln Pro Val Glu Asn Lys Thr Gln Pro Leu Val Val Tyr Gln
225                 230                 235                 240

Tyr Arg Leu Pro Thr Glu Leu Gln Tyr Arg Pro Pro Ser Glu Val Gln
                245                 250                 255

Tyr Arg Pro Gln Ala Val Cys Pro Val Pro Asn Ser Thr Ala Pro Tyr
            260                 265                 270

Gln Gln Pro Thr Ala Met Ala Ser Asn Ser Pro Ala Thr Gln Asp Ala
        275                 280                 285

Ala Leu Tyr Pro Gln Pro Pro Thr Val Arg Leu Asn Pro Thr Ala Ser
    290                 295                 300

Arg Ser Gly Gln Gly Gly Ala Leu His Ala Val Ile Asp Glu Ala Arg
305                 310                 315                 320

Lys Gln Gly Asp Leu Glu Ala Trp Arg Phe Leu Val Ile Leu Gln Leu
                325                 330                 335

Val Gln Ala Gly Glu Glu Thr Gln Val Gly Ala Pro Ala Arg Ala Glu
            340                 345                 350

Thr Arg Cys Glu Pro Phe Thr Met Lys Met Leu Lys Asp Ile Lys Glu

355                 360                 365

Gly Val Lys Gln Tyr
    370



<210> 969
<211> 50
<212> DNA
<213> Homo sapiens

<400> 969
gcctgtgatg tctccgtacg tgtggtggtg ggtgagccca ccgaggccag          50

<210> 970
<211> 31
<212> DNA
<213> Homo sapiens

<400> 970
tctgcctgtg atgtctccgt acgtgtggtg g                              31

<210> 971
<211> 45
<212> DNA
<213> Homo sapiens

<400> 971
ggggcctctg cctgtgatgt ctccgtacgt gtggtggtgg gtgag               45

<210> 972
<211> 60
<212> DNA
<213> Homo sapiens

<400> 972
ttttacacgg atttcgtggg cgaggggctg taccagggcg tgcccagagc tgagccgggc 60

<210> 973
<211> 30
<212> DNA
<213> Homo sapiens

<400> 973
acggatttcg tgggcgaggg gctgtaccag                                30

<210> 974
<211> 24
<212> DNA
<213> Homo sapiens

<400> 974
acggatttcg tgggcgaggg gctg                                      24

<210> 975
<211> 32
<212> DNA
<213> Homo sapiens

<400> 975
ttttacacgg atttcgtggg cgaggggctg ta                             32

<210> 976
<211> 30
<212> DNA
<213> Homo sapiens

<400> 976
tacacggatt tcgtgggcga ggggctgtac                                        30

<210> 977
<211> 16
<212> PRT
<213> Homo sapiens

<400> 977
Ala Cys Asp Val Ser Val Arg Val Val Val Gly Glu Pro Thr Glu Ala
                5                   10                  15

<210> 978
<211> 11
<212> PRT
<213> Homo sapiens

<400> 978
Ser Ala Cys Asp Val Ser Val Arg Val Val Val
                5                   10

<210> 979
<211> 15
<212> PRT
<213> Homo sapiens

<400> 979
Gly Ala Ser Ala Cys Asp Val Ser Val Arg Val Val Val Gly Glu
                5                   10                  15

<210> 980
<211> 10
<212> PRT
<213> Homo sapiens

<400> 980
Thr Asp Phe Val Gly Glu Gly Leu Tyr Gln
                5                   10

<210> 981
<211> 8
<212> PRT
<213> Homo sapiens

<400> 981
Thr Asp Phe Val Gly Glu Gly Leu
                5

<210> 982
<211> 10
<212> PRT
<213> Homo sapiens

<400> 982
Tyr Thr Asp Phe Val Gly Glu Gly Leu Tyr
                5                   10

<210> 983
<211> 20
<212> PRT
<213> Homo sapiens

<400> 983
Phe Tyr Thr Asp Phe Val Gly Glu Gly Leu Tyr Gln Gly Val Pro Arg
                5                   10                  15

Ala Glu Pro Gly
            20


<210> 984
<211> 10
<212> PRT
<213> Homo sapiens

<400> 984
Phe Tyr Thr Asp Phe Val Gly Glu Gly Leu
                5                   10


<210> 985
<211> 19
<212> PRT
<213> Homo sapiens

<400> 985
Gly Leu Ser Ala Pro Ser Leu Ser His Cys Cys Pro Cys Arg Ala Arg
                5                   10                  15

Leu Ala Phe


<210> 986
<211> 21
<212> PRT
<213> Homo sapiens

<400> 986
Ala Arg Leu Ala Phe Arg Asn Leu Gly Ala Leu Leu Pro Arg Leu His
                5                   10                  15

Gln Leu Cys Cys Arg
            20


<210> 987
<211> 30
<212> PRT
<213> Homo sapiens

<400> 987
Leu Ala Ile Leu Asp Ser Ala Phe Leu Leu Ser Gln Val Ala Pro Ser
                5                   10                  15

Leu Phe Met Gly Ser Ile Val Gln Leu Ser Gln Ser Val Thr
            20                  25                  30


<210> 988
<211> 15
<212> PRT
<213> Homo sapiens

<400> 988
Arg Pro Leu Leu Ala Asn Asp Leu Met Leu Ile Lys Leu Asp Glu
                5                   10                  15


<210> 989

<211> 15
<212> PRT
<213> Homo sapiens

<400> 989
Ser Gln Cys Pro Thr Ala Gly Asn Ser Cys Leu Val Ser Gly Trp
                5                   10                  15


<210> 990
<211> 15
<212> PRT
<213> Homo sapiens

<400> 990
Met Phe Cys Ala Gly Gly Gly Gln Asp Gln Lys Asp Ser Cys Asn
                5                   10                  15


<210> 991
<211> 45
<212> DNA
<213> Homo sapiens

<400> 991
gggaactctt gcctcgtttc tggctggggt ctgctggcga acggc                 45


<210> 992
<211> 15
<212> PRT
<213> Homo sapiens

<400> 992
Gly Asn Ser Cys Leu Val Ser Gly Trp Gly Leu Leu Ala Asn Gly
 1               5                   10                  15


<210> 993
<211> 848
<212> DNA
<213> Homo sapiens

<220>
<221> misc_feature
<222> 33,843
<223> n = A,T,C or G

<400> 993
tacttatgag cagagcgaca tatccaagtg takactgaat aaaactgaat tctctccagt 60
ttaaagcatt gctcactgaa gggatagaag tgactgccag gagggaaagt aagccaaggc 120
tcattatgcc aaaggagata tacatttcaa ttctccaaac ttcttcctca ttccaagagt 180
tttcaatatt tgcatgaacc tgctgataag ccatgttgag aaacaaatat ctctctgacc 240
ttctcatcgg taagcagagg ctgtaggcaa catggaccat agcgaagaaa aaacttagta 300
atccaagctg ttttctacac tgtaaccagg tttccaacca aggtggaaat ctcctatact 360
tggtgccgta ataaagttga taagcagctg ccagaagacc tgcgaggtat actagggaga 420
gcaaagtaat ggcaactata ggtaaggttt tattcacaat ctctatagga attttgtaaa 480
agtcactctg ttggtttcta gcatatggat gaatcacatc tctgacaaag gaataaagga 540
aaaaaaaatg tggccaagct tatagctacc accactggcc ctctccagag agtaaagagt 600
cgtaggggta aattttcaat ctctctggct gatgataagg atcccaagtc aatgggaatg 660
aaattcaact ggcgggcaag ttcaataacc tgttgtcgcg cttgaatatt gttgctgcat 720
atataaacct gccggctggc atccttaggt cctaactgaa gtgcccaagc tgagacaaca 780
ttaaatcctt tgacaatcaa agaatctggg aataatgaag ccaaatattc agcattggat 840
ttytgggt                                                          848

<210> 994
<211> 762
<212> DNA

<213> Homo sapiens

<400> 994
gtacttttta tttaaatgtt tataaggcag atctatgaga atgatagaaa acatggtgtg 60
taatttgata gcaatatttt ggagattaca gagttttagt aattaccaat tacacagtta 120
aaaagaagat aatatattcc aagcagatac aaaatatcta atgaaagatc aaggcaggaa 180
aatgagtata actaattgac aatggaaaat caattttaat gtgaattgca cattatcctt 240
taaaagcttt caaaagaaag aattattgca gtctagttaa ttcagacagt gttaaatggt 300
atcaggataa agaactgaag ggcagaaaga attaattttc acttcatgta acacacccag 360
atttacaatg gcttaaatgc aggaaaaagc agtggaagta gggaagtagt caaggtcttt 420
ctggtctcta atctgcctta ctctttgggt gtggctttga tcctctggag acagctgcca 480
gggctcctgt tatatccaca atcccagcag caagatgaag ggatgaaaaa ggacacatgc 540
tgccttcctt tgaggagact tcatctcact ggccaacact cagtcacatg tacacctaac 600
tgcaaggaca gctgaggaat gtaatgggca gccgctttta aagaagtaga gtcaatagga 660
agacaaattc cagttccagc tcagtctggg tatctgcaaa gctgcaaaag atctttaaag 720
acaatttcaa gagaatattt ccttaaagtt ggcaatttgg ag 762

<210> 995
<211> 1403
<212> DNA
<213> Homo sapiens

<400> 995
tgactcttta ctctctggag agggccagtg gtggtagcta taggcttggc cacatttttt 60
ttcctttatt cctttgtcag agatgtgatt catccatatg ctagaaacca acagagtgac 120
ttttacaaaa ttcctataga gattgtgaat aaaaccttac ctatagttgc cattactttg 180
ctctccctag tatacctcgc aggtcttctg gcagctgctt atcaacttta ttacggcacc 240
aagtatagga gatttccacc ttggttggaa acctggttac agtgtagaaa acagcttgga 300
ttactaagtt ttttcttcgc tatggtccat gttgcctaca gcctctgctt accgatgaga 360
aggtcagaga gatatttgtt tctcaacatg gcttatcagc aggttcatgc aaatattgaa 420
aactcttgga atgaggaaga agtttggaga attgaaatgt atatctcctt tggcataatg 480
agccttggct tactttccct cctggcagtc acttctatcc cttcagtgag caatgcttta 540
aactggagag aattcagttt tattcagtct acacttggat atgtcgctct gctcataagt 600
actttccatg ttttaattta tggatggaaa cgagcttttg aggaagagta ctacagattt 660
tatacaccac caaactttgt tcttgctctt gttttgccct caattgtaat tctggatctt 720
ttgcagcttt gcagataccc agactgagct ggaactggaa tttgtcttcc tattgactct 780
acttctttaa aagcggctgt ccattacatt cctcagctgt ccttgcagtt aggtgtacat 840
gtgactgagt gttggccagt gagatgaagt ctcctcaaag gaaggcagca tgtgtccttt 900
ttcatccctt catcttgctg ctgggattgt ggatataaca ggagccctgg cagctgtctc 960
cagaggatca aagccacacc caaagagtaa ggcagattag agaccagaaa gaccttgact 1020
acttccctac ttccactgct ttttcctgca tttaagccat tgtaaatctg ggtgtgttac 1080
atgaagtgaa aattaattct ttctgccctt cagttcttta tcctgatacc atttaacact 1140
gtctgaatta actagactgc aataattctt tcttttgaaa gcttttaaag gataatgtgc 1200
aattcacatt aaaattgatt ttccattgtc aattagttat actcattttc ctgccttgat 1260
ctttcattag atattttgta tctgcttgga atatattatc ttctttttaa ctgtgtaatt 1320
ggtaattact aaaactctgt aatctccaaa atattgctat caaattacac accatgtttt 1380
ctatcattct catagatctg ccc 1403

<210> 996
<211> 511
<212> DNA
<213> Homo sapiens

<220>
<221> misc_feature
<222> 69
<223> n = A,T,C or G

<400> 996
tggctttaac aacagaaatt tattttgtta cagttctgga ggctagaagt tgaagactaa 60
ggtgttggma ggattggttt catctcgagg catctctcct tggcatgccg tcttctccct 120
tttacttcac gtggtcttcc ctctgtgcat gcctgattcc taatctcctc ttcttatgag 180
gacaccagtc atactggatt agggcccaac tgtatgacct cactttacct ctttaaagat 240
cctatctcca aatacattct gaggtagtgt gggagttagg atttcaatgt atcaattttg 300
aggaaacaca gttcagccca taacagatcc cagtatcttt tgaattataa cttcaattct 360
tataaacagc agattcaaga ataaagtatg tttaatcaag gcacaaatgg tattctcgcc 420
tacaggacac tgatatttca cacagtagtg ctgagttact attttgattt aacagtgtat 480
gactgtacat ttgatcaatg tgaatctgcc c 511

<210> 997
<211> 1152
<212> DNA
<213> Homo sapiens

<220>
<221> misc_feature
<222> 613
<223> n = A,T,C or G

<400> 997
acctataaat atattttatt catactttta aatattttac aattcaaata aaaaccttat 60
atgtagacaa tctgggctaa atttccatgt atgttttgaa aaataatgtt agcatgaata 120
gattcatatt taaatatgat tttaaatact cttaatagag gagacataag aaatatttac 180
ataaaagcta agtagcatga tacagctcat ggttattttc ctcataggaa aacaattact 240
tgattttttt tttttgcata ggattaagac tgagtatctt ttctacattc ttttaacttt 300
ctaaggggca cttctcaaaa cacagaccag gtagcaaatc tccactgctc taaggttctc 360
accaccactt tctcacacct agcaatagta gaattcagtc ctacttctga ggccagaaga 420
atggttcaga aaaataatga ttataaaaaa taacaattaa gaaaaataat cacatagagg 480
taaaataaag caactaaaca tcatagtttt gcatttttag aaaagcattt aaacatttag 540
aattatagta aatatattac atttattcta aaacttcaaa attattctgt ttttgtagta 600
ctgaaaagaa gayagtgcca tttgaaacaa cagatgcatc ttttatacat tttcacaagt 660
ttggtttttc atatttttaa aggccccatt tatctgtaac agtggtattt ttatttagag 720
tatcggctac ttaatatata catgcaacaa tatatgcttt aatagtcatt taacttttag 780
gaatatttca tcacattaag tggttaagca tagtgttaaa agagtggaat ttaaggaata 840
agaaaatatt gaaaatacgc tgttattttc atttgttcac tataatagaa tgtttttgcc 900
cataaaagtt atcattgccc aactgaattc ctaccaagaa ctaacaagtg attctcaagt 960
ggggagaaat attattaaaa taaagaaaat agagggctca gttagaaagt acaagataat 1020
aggccgggcg cggtagctca cacctgtaat cccagcactt tggaggccaa ggcgggcgga 1080
tcacgaggtc aggagatcga gaccatcctg gctaacacgg tgaaactccg tctctactaa 1140
aaatacaaaa ac 1152

<210> 998
<211> 1184
<212> DNA
<213> Homo sapiens

<220>
<221> misc_feature
<222> 186,191,192,197,198,199,201,209,222,223,
243,246,248,253,254,265,266,269,277,285,
286,300,307,315,316,319,321,348,352,355,
357,361,363,365,369,371,373,374,375,376,
377,379,380,381,383,384,387,388,397,398,
401,402,403,405,407,953,1034,1045,1080,1085,
1092,1109
<223> n = A,T,C or G

<400> 998
ctaatggttc ctttaaaata tatttaaaaa taaccagcaa tagtgggctg gtcccggtgg 60
ctcacgcctg taatcccagc acttttgaga gactgaggca ggcggatcac gaggtcagaa 120
gatagagacc accctggcta acatggtgaa accctgtctc tactaaaaat acaaaaaatt 180
agccgsgtcg wsgtggyrkg stcctgtart cccagctact crrgaggctg aggcaggaga 240
atsgcwtraa ccyrggagac ggagsytgma gtgagcwgag atcgyrccac tgcactccak 300
cctgggygac agagwsaamt ycatctcaaa aaattaaagt aataatarta awaakasata 360
wayaytttrg sakkmswaks mtskcakmtc atcagarstc wksarawtat ttgtatgtac 420
tgcagctcaa ttagaagtgg tctctgactt tcatcagctt ctccctgggc tccatgacac 480
tggcctggag tgactcattg ctctggttgg ttgagagagc tcctttgcca acaggcctcc 540
aagtcagggc tgggatttgt ttcctttcca cattctagca acaatatgct ggccacttcc 600
tgaacaggga gggtgggagg agccagcatg gaacaagctg ccactttcta gagtagccag 660
acttgcccct gggcctgtca gcacctactg atgaccttct gtgcctgcag gatggaatgt 720
aggggtgagc tgtgtgactc tatggtacag actggtccag gttcctggag gtcacactct 780
ggggctgcca gccaaagggt gcccttaagt tggagtgtgg gtgacttcct gaacagagct 840
ggcactgggg agggggtgct cggggtcctt tctcttctaa caggccccag ttttgtctca 900
atgacagtgt ttctacacct gtgggttatg acaaagacaa ctgccaaaga atyttcaaga 960
aggaggactg caagtatatc gtggtggaga agaaggaccc aaaaaagacc tgttctgtca 1020

gtgaatggat aatytaatgt gcttytagta ggcacagggc tcccaggcca ggcctcatty 1080
tcctytggcc tytaatagtc aatgattgkg tagccatgcc tatcagtaaa aagatttttg 1140
agcaaaaaaa aaaaaaaaaa aaaaaaaaaa aaaaaaaaaa aaaa 1184

<210> 999
<211> 956
<212> DNA
<213> Homo sapiens

<400> 999
tctccagaac tgtgagcaaa taaatttctt tagtcaaagc cacccagttg cagtgttttg 60
ttgtggtggc cagagcaaac agataaagta gtaagtccct ccagcaccct aggaggttct 120
gcttgttgcc attccccaaa ggccatccta gccatgatga gggcaggtgc ggttgtttgg 180
gaggagacca ctgtggaaca aagagaaaca aggcttgctt gatgcagcag ggagtagact 240
acagattcaa gaagggctct agaaaggtcc agcaattcag cttgtcaggg aaccccgagc 300
tgtatgtagt tcttgctctc ctgtgccctg caggatggct tgacccattt ctgttctcat 360
gtcagaccct aagggcaggg tcatctgggc cacagaccca ggcccggtgg gcctggtggg 420
ctgacctgtg ggctattctt atgccttcag gaaacccacc tttaccacct ggctgttgtg 480
actggcatcg ctgaggctct gtttctttgt gtataaaatc gaataaggac acatcacgtg 540
ctagcaatcc accagcatag ggcgaaggct cagcaaagag aagttctctc ttcctctgct 600
cttttaggtc agcagcaaat gcagatcggg gtggggacaa ggtaaacaca taacttgggt 660
ggagatgtga ttgattaata actcataaat catctgaaac catactttcc ttttgatagt 720
caaccctctg taaacactca atgtgttctc accttgtatc tattcccaat gatgtcgaat 780
gcgtggttgc cctctccagt ataaaagttt gatgcagctt ttcctggatg tacctgtcta 840
taaggagtcc tgcttatcac aatggtaggt aactggtttt atatataaag gctagaaaag 900
caaaagagaa tatgtattcc tattttaata tgcgtgtgct tctaccacat agcatt 956

<210> 1000
<211> 234
<212> DNA
<213> Homo sapiens

<400> 1000
cactcagtca aggattatcc tttgaattaa ttcattctcc accctgacct ttcttcacat 60
tacagaatgt tctcctgggc agcgttgtga tctttgccac cttcgtgact ttatgcaatg 120
catcatgcta tttcatacct aatgagggag ttccaggaga ttcaaccagg agtaagtctt 180
ggcttttcaa tgtttattat gttattgcag cctggtagat ggacctgtct gcag 234

<210> 1001
<211> 260
<212> DNA
<213> Homo sapiens

<400> 1001
taggggcagg actcaatgtc ctgataggcc ttgttctcat tgcatgactt gcactgatga 60
tgttgtgtgt attattttag aatgcatgga tctcaaagga aacaaacacc caataaactc 120
ggagtggcag actgacaact gtgagacatg cacttgctac gaaacagaaa tttcatgttg 180
caccctgtaa gtctcaagcc agtgctggcc aagaggggga catagctgtt ctctgaggaa 240
cagctactcc gtcttgcaaa 260

<210> 1002
<211> 355
<212> DNA
<213> Homo sapiens

<400> 1002
ctaacaggcc ccagttttgt ctcaatgaca gtgtttctac acctgtgggt tatgacaaag 60
acaactgcca aagaatcttc aagaaggagg actgcaagta tatcgtggtg gagaagaagg 120
acccaaaaaa gacctgttct gtcagtgaat ggataatcta atgtgcttct agtaggcaca 180
gggctcccag gccaggcctc attctcctct ggcctctaat agtcaatgat tgtgtagcca 240
tgcctatcag taaaaagatt tttgagcaaa cacttgaata tgtgtgtcct tttaatttat 300
aatttatgta tgcattgatt taatacacat tataaagaat acaaacattt aaaaa 355

<210> 1003
<211> 114
<212> PRT

<213> Homo sapiens

<400> 1003
Met Asn Val Leu Leu Gly Ser Val Val Ile Phe Ala Thr Phe Val Thr
1               5                   10                  15
Leu Cys Asn Ala Ser Cys Tyr Phe Ile Pro Asn Glu Gly Val Pro Gly
            20                  25                  30
Asp Ser Thr Arg Lys Cys Met Asp Leu Lys Gly Asn Lys His Pro Ile
        35                  40                  45
Asn Ser Glu Trp Gln Thr Asp Asn Cys Glu Thr Cys Thr Cys Tyr Glu
    50                  55                  60
Thr Glu Ile Ser Cys Cys Thr Leu Val Ser Thr Pro Val Gly Tyr Asp
65                  70                  75                  80
Lys Asp Asn Cys Gln Arg Ile Phe Lys Lys Glu Asp Cys Lys Tyr Ile
                85                  90                  95
Val Val Glu Lys Lys Asp Pro Lys Lys Thr Cys Ser Val Ser Glu Trp
            100                 105                 110
Ile Ile

<210> 1004
<211> 2385
<212> DNA
<213> Homo sapiens

<400> 1004
ttaatgcagg aattcggcac gagcgcttgg cttggcgtgg cagtgatggc tgggcttggc 60
atttcgggct tggcggtcct cctttcgcag attggaaacc gcgggctatc ctgctgggag 120
gttgtggccg aggcagtagc tcgctactga tggcctcctg gggtggagaa aagcggggag 180
gggctgaggg gtctccgaag ctggcagtct acgccacgag gaagacccgt agcgtcagga 240
gccaggagga ccagtggtac ttgggctacc cgggggaccg agtggtcttc gggcttctcc 300
tacagctggt ggaaaaagag cgtcggcagc gagagcaagc acggtgaggg cgccttagac 360
cagccccagc acgacgtccg cctggaagat cttggcgagc tccacagagc tgcccggtcg 420
ggcgacgtcc ctggggtgga gcacgtcttg gctcctggag acactggcgt ggacaagagg 480
gataggaaga agagcattca gcaactagtt cctgaatata aggaaaaaca gacacctgaa 540
agtcttcctc aaaataacaa tccagattgg catcctacta atttgaccct tagtgatgag 600
acttgtcaga gatccaagaa tctgaaagtt ggtgataaaa gtccatctgt atcaccatca 660
atgcctgaaa atcagtcagc aaccaaagaa ctgggacaga tgaacttaac agaacgagaa 720
aaaatggaca ctggagttgt acttctctca gggaatgata ctctccatga cctgtgccaa 780
tcacagctac cagaaaacaa agagagcaaa gaagcagaac aagacttgga gctgacatca 840
gaggaagagc aagaaagact taaaggatgc gaaaataagc agccacagaa aacgtctcaa 900
gaaccagaaa tggctaagga ttgtgataga gaggatatac ctatatatcc agtacttcct 960
catgtgcaaa aatctgagga aatgtggatt gaacaaggca aattagagtg gaaaaaccaa 1020
ttaaaactcg tcataaatga gttaaagcag aggtttggtg aaatttatga aaaatacaaa 1080
attccggctt gtcctgagga agagccacta cttgataact ctacaagagg aacagatgtg 1140
aaggatattc cctttaattt gacaaataac atacctggtt gtgaggaaga agatgcatct 1200
gaaatatctg tctcagtggt attcgagaca tttcctgaac aaaaagaacc cagtctcaaa 1260
aatatcatcc atccatacta tcatccgtac tctgggtccc aggaacatgt ttgccagtca 1320
tcttctaagc ttcatttaca tgaaaataaa ttagactgcg acaatgataa caaactaggc 1380
attggacata tttttagtac agataacaac tttcataatg atgcaagcac taagaaagca 1440
aggaacccag aagtggttac ggttgaaatg aaagaagacc aagagtttga tttgcaaatg 1500
acaaaaaata tgaaccaaaa tagtgacagt ggcagtacaa ataactataa aagcctgaaa 1560
cctaaattag aaaatctgag ttctttacca ccagattctg acagaacatc agaagtatat 1620
ctacatgaag aattacagca agacatgcaa aagtttaaga atgaggtcaa cacattagaa 1680
gaagagttcc tggctttgaa gaaagaaaat gttcaacttc ataaagaggt tgaagaagaa 1740
atggagaagc acagaagtaa tagcacagaa ttatcaggaa ccctaactga tggtactact 1800
gttggcaatg atgatgatgg actaaatcag cagattccta ggaaggaaaa tgaagagcat 1860
gacaggcctg cagataaaac agctaatgaa aagaacaagg tcaaaaacca aatatatcct 1920
gaggctgact ttgctgactc aatggagcca tctgaaatag cctcagagga ttgtgaattg 1980
tctcactctg tttatgagaa ttttatgttg ctgattgaac aacttagaat ggagtataaa 2040
gattctgcta gcctaccaag aatccaagac acattttgtt tgtgtgaaca cttactgaaa 2100
cttaagaata atcactgtga ccaacttaca gtaaaactta aacaaatgga aaatatggtc 2160
agtgtactac aaaatgagct atctgaaaca aaaaagacaa aattacagtt agaacttcaa 2220
aaaattgaat gggagaaaga gctgtacgat ttgagatttc tcataggcat cagaaagaaa 2280
agcatctctt tcatgaaaat tgcatgttgc aggaagaaat tgccttgctg agactggaaa 2340
tagatacaat aaaaaatcag aacaagcaaa aaaaaaaaaa aaaaa 2385

<210> 1005
<211> 556
<212> PRT
<213> Homo sapiens

<400> 1005
Met Pro Glu Asn Gln Ser Ala Thr Lys Glu Leu Gly Gln Met Asn Leu
                5                   10                  15

Thr Glu Arg Glu Lys Met Asp Thr Gly Val Val Leu Leu Ser Gly Asn
            20                  25                  30

Asp Thr Leu His Asp Leu Cys Gln Ser Gln Leu Pro Glu Asn Lys Glu
        35                  40                  45

Ser Lys Glu Ala Glu Gln Asp Leu Glu Leu Thr Ser Glu Glu Glu Gln
    50                  55                  60

Glu Arg Leu Lys Gly Cys Glu Asn Lys Gln Pro Gln Lys Thr Ser Gln
65                  70                  75                  80

Glu Pro Glu Met Ala Lys Asp Cys Asp Arg Glu Asp Ile Pro Ile Tyr
                85                  90                  95

Pro Val Leu Pro His Val Gln Lys Ser Glu Glu Met Trp Ile Glu Gln
            100                 105                 110

Gly Lys Leu Glu Trp Lys Asn Gln Leu Lys Leu Val Ile Asn Glu Leu
        115                 120                 125

Lys Gln Arg Phe Gly Glu Ile Tyr Glu Lys Tyr Lys Ile Pro Ala Cys
    130                 135                 140

Pro Glu Glu Glu Pro Leu Leu Asp Asn Ser Thr Arg Gly Thr Asp Val
145                 150                 155                 160

Lys Asp Ile Pro Phe Asn Leu Thr Asn Asn Ile Pro Gly Cys Glu Glu
                165                 170                 175

Glu Asp Ala Ser Glu Ile Ser Val Ser Val Val Phe Glu Thr Phe Pro
            180                 185                 190

Glu Gln Lys Glu Pro Ser Leu Lys Asn Ile Ile His Pro Tyr Tyr His
        195                 200                 205

Pro Tyr Ser Gly Ser Gln Glu His Val Cys Gln Ser Ser Ser Lys Leu
    210                 215                 220

His Leu His Glu Asn Lys Leu Asp Cys Asp Asn Asp Asn Lys Leu Gly
225                 230                 235                 240

Ile Gly His Ile Phe Ser Thr Asp Asn Asn Phe His Asn Asp Ala Ser
                245                 250                 255

Thr Lys Lys Ala Arg Asn Pro Glu Val Val Thr Val Glu Met Lys Glu
            260                 265                 270

Asp Gln Glu Phe Asp Leu Gln Met Thr Lys Asn Met Asn Gln Asn Ser
        275                 280                 285

Asp Ser Gly Ser Thr Asn Asn Tyr Lys Ser Leu Lys Pro Lys Leu Glu
    290                 295                 300

Asn Leu Ser Ser Leu Pro Pro Asp Ser Asp Arg Thr Ser Glu Val Tyr
305                 310                 315                 320

Leu His Glu Glu Leu Gln Gln Asp Met Gln Lys Phe Lys Asn Glu Val
                325                 330                 335

Asn Thr Leu Glu Glu Glu Phe Leu Ala Leu Lys Lys Glu Asn Val Gln
            340                 345                 350

Leu His Lys Glu Val Glu Glu Glu Met Glu Lys His Arg Ser Asn Ser
        355                 360                 365

Thr Glu Leu Ser Gly Thr Leu Thr Asp Gly Thr Thr Val Gly Asn Asp
    370                 375                 380

Asp Asp Gly Leu Asn Gln Gln Ile Pro Arg Lys Glu Asn Glu Glu His
385                 390                 395                 400

Asp Arg Pro Ala Asp Lys Thr Ala Asn Glu Lys Asn Lys Val Lys Asn
                405                 410                 415

Gln Ile Tyr Pro Glu Ala Asp Phe Ala Asp Ser Met Glu Pro Ser Glu
            420                 425                 430

Ile Ala Ser Glu Asp Cys Glu Leu Ser His Ser Val Tyr Glu Asn Phe
        435                 440                 445

Met Leu Leu Ile Glu Gln Leu Arg Met Glu Tyr Lys Asp Ser Ala Ser
    450                 455                 460

Leu Pro Arg Ile Gln Asp Thr Phe Cys Leu Cys Glu His Leu Leu Lys
465                 470                 475                 480

Leu Lys Asn Asn His Cys Asp Gln Leu Thr Val Lys Leu Lys Gln Met
                485                 490                 495

Glu Asn Met Val Ser Val Leu Gln Asn Glu Leu Ser Glu Thr Lys Lys
            500                 505                 510

Thr Lys Leu Gln Leu Glu Leu Gln Lys Ile Glu Trp Glu Lys Glu Leu
        515                 520                 525

Tyr Asp Leu Arg Phe Leu Ile Gly Ile Arg Lys Lys Ser Ile Ser Phe
    530                 535                 540

Met Lys Ile Ala Cys Cys Arg Lys Lys Leu Pro Cys
545                 550                 555

<210> 1006
<211> 3786
<212> DNA
<213> Homo sapiens

<400> 1006
atgtattcag tgctgccaga agaccgctca cagcaccttg gagaggagtt gcaagggttc 60
tgggataaag aagttttaag agctgagaat gacgcacaga agccttcttt aacaagagca 120
atcataaagt gttactggaa atcttattta gttttgggaa tttttacgtt aattgaggaa 180
agtgccaaag taatccagcc catatttttg ggaaaaatta ttaattattt tgaaaattat 240
gatcccatgg attctgtggc tttgaacaca gcgtacgcct atgccacggt gctgactttt 300
tgcacgctca ttttggctat actgcatcac ttatattttt atcacgttca gtgtgctggg 360
atgaggttac gagtagccat gtgccatatg atttatcgga aggcacttcg tcttagtaac 420
atggccatgg ggaagacaac cacaggccag atagtcaatc tgctgtccaa tgatgtgaac 480
aagtttgatc aggtgacagt gttcttacac ttcctgtggg caggaccact gcaggcgatc 540
gcagtgactg ccctactctg gatggagata ggaatatcgt gccttgctgg gatggcagtt 600
ctaatcattc tcctgccctt gcaaagctgt tttgggaagt tgttctcatc actgaggagt 660
aaaactgcaa ctttcacgga tgccaggatc aggaccatga atgaagttat aactggtata 720
aggataataa aaatgtacgc ctgggaaaag tcattttcaa atcttattac caatttgaga 780
aagaaggaga tttccaagat tctgagaagt tcctgcctca gggggatgaa tttggcttcg 840

tttttcagtg caagcaaaat catcgtgttt gtgaccttca ccacctacgt gctcctcggc 900
agtgtgatca cagccagccg cgtgttcgtg gcagtgacgc tgtatggggc tgtgcggctg 960
acggttaccc tcttcttccc ctcagccatt gagagggtgt cagaggcaat cgtcagcatc 1020
cgaagaatcc agaccttttt gctacttgat gagatatcac agcgcaaccg tcagctgccg 1080
tcagatggta aaaagatggt gcatgtgcag gattttactg ctttttggga taaggcatca 1140
gagaccccaa ctctacaagg cctttccttt actgtcagac ctggcgaatt gttagctgtg 1200
gtcggccccg tgggagcagg gaagtcatca ctgttaagtg ccgtgctcgg ggaattggcc 1260
ccaagtcacg ggctggtcag cgtgcatgga agaattgcct atgtgtctca gcagccctgg 1320
gtgttctcgg gaactctgag gagtaatatt ttatttggga agaaatacga aaaggaacga 1380
tatgaaaaag tcataaaggc ttgtgctctg aaaaaggatt tacagctgtt ggaggatggt 1440
gatctgactg tgataggaga tcggggaacc acgctgagtg gagggcagaa agcacgggta 1500
aaccttgcaa gagcagtgta tcaagatgct gacatctatc tcctggacga tcctctcagt 1560
gcagtagatg cggaagttag cagacacttg ttcgaactgt gtatttgtca aattttgcat 1620
gagaagatca caattttagt gactcatcag ttgcagtacc tcaaagctgc aagtcagatt 1680
ctgatattga aagatggtaa aatggtgcag aaggggactt acactgagtt cctaaaatct 1740
ggtatagatt ttggctccct tttaaagaag gataatgagg aaagtgaaca acctccagtt 1800
ccaggaactc ccacactaag gaatcgtacc ttctcagagt cttcggtttg gtctcaacaa 1860
tcttctagac cctccttgaa agatggtgct ctggagagcc aagatacaga gaatgtccca 1920
gttacactat cagaggagaa ccgttctgaa ggaaaagttg gttttcaggc ctataagaat 1980
tacttcagag ctggtgctca ctggattgtc ttcattttcc ttattctcct aaacactgca 2040
gctcaggttg cctatgtgct tcaagattgg tggctttcat actgggcaaa caaacaaagt 2100
atgctaaatg tcactgtaaa tggaggagga aatgtaaccg agaagctaga tcttaactgg 2160
tacttaggaa tttattcagg tttaactgta gctaccgttc tttttggcat agcaagatct 2220
ctattggtat tctacgtcct tgttaactct tcacaaactt tgcacaacaa aatgtttgag 2280
tcaattctga aagctccggt attattcttt gatagaaatc caataggaag aattttaaat 2340
cgtttctcca aagacattgg acacttggat gatttgctgc cgctgacgtt tttagatttc 2400
atccagacat tgctacaagt ggttggtgtg gtctctgtgg ctgtggccgt gattccttgg 2460
atcgcaatac ccttggttcc ccttggaatc attttcattt ttcttcggcg atattttttg 2520
gaaacgtcaa gagatgtgaa gcgcctggaa tctacaactc ggagtccagt gttttcccac 2580
ttgtcatctt ctctccaggg gctctggacc atccgggcat acaaagcaga agagaggtgt 2640
caggaactgt ttgatgcaca ccaggattta cattcagagg cttggttctt gtttttgaca 2700
acgtcccgct ggttcgccgt ccgtctggat gccatctgtg ccatgtttgt catcatcgtt 2760
gcctttgggt ccctgattct ggcaaaaact ctggatgccg ggcaggttgg tttggcactg 2820
tcctatgccc tcacgctcat ggggatgttt cagtggtgtg ttcgacaaag tgctgaagtt 2880
gagaatatga tgatctcagt agaaagggtc attgaataca cagaccttga aaaagaagca 2940
ccttgggaat atcagaaacg cccaccacca gcctggcccc atgaaggagt gataatcttt 3000
gacaatgtga acttcatgta cagtccaggt gggcctctgg tactgaagca tctgacagca 3060
ctcattaaat cacaagaaaa ggttggcatt gtgggaagaa ccggagctgg aaaaagttcc 3120
ctcatctcag ccctttttag attgtcagaa cccgaaggta aaatttggat tgataagatc 3180
ttgacaactg aaattggact tcacgattta aggaagaaaa tgtcaatcat acctcaggaa 3240
cctgttttgt tcactggaac aatgaggaaa aacctggatc cctttaatga gcacacggat 3300
gaggaactgt ggaatgcctt acaagaggta caacttaaag aaaccattga agatcttcct 3360
ggtaaaatgg atactgaatt agcagaatca ggatccaatt ttagtgttgg acaaagacaa 3420
ctggtgtgcc ttgccagggc aattctcagg aaaaatcaga tattgattat tgatgaagcg 3480
acggcaaatg tggatccaag aactgatgag ttaatacaaa aaaaaatccg ggagaaattt 3540
gcccactgca ccgtgctaac cattgcacac agattgaaca ccattattga cagcgacaag 3600
ataatggttt tagattcagg aagactgaaa gaatatgatg agccgtatgt tttgctgcaa 3660
aataaagaga gcctatttta caagatggtg caacaactgg gcaaggcaga agccgctgcc 3720
ctcactgaaa cagcaaaaca gagatggggt ttcaccatgt tggccaggct ggtctcaaac 3780
tcctga 3786

<210> 1007
<211> 4395
<212> DNA
<213> Homo sapiens

<400> 1007
atgtcctgcg gtaacgccaa gatcaactct cccgcgccgt ccttcgagga ggtggcgctc 60
atgcccaacg gcagcttcaa gaagatcagc ctctcctcct acaagggcaa gtgggtcgtg 120
ctcttcttct acccgctcga cttcaccttc gtgtgcccga cagaggtcat cgcgttctcc 180
gacagcgtga gtcgcttcaa cgagctcaac tgcgaggtcc tcgcgtgctc gatagacagc 240
gagtacgcgc acctgcagtg gacgctgcag gaccgcaaga agggcggcct cgggaccatg 300
gcgatcccaa tgctagccga caagaccaag agcatcgctc gttcctacgg cgtgctggag 360
gagagccagg gcgtggccta ccgcggtctc ttcatcatcg acccccatgg catgctgcgt 420
cagatcaccg tcaatgacat gccggtgggc cgcagcgtgg aggaggttct acgcctgctg 480
gaggcttttc agttcgtgga gaagcacggc gaggtgtgcc ccgcgaactg gaagaagggc 540
gcccccacga tgaagccgga accgaatgcg tctgtcgagg gatacttcag caagcagtct 600
agagaattca tgtattcagt gctgccagaa gaccgctcac agcaccttgg agaggagttg 660

caagggttct gggataaaga agttttaaga gctgagaatg acgcacagaa gccttcttta 720
acaagagcaa tcataaagtg ttactggaaa tcttatttag ttttgggaat ttttacgtta 780
attgaggaaa gtgccaaagt aatccagccc atatttttgg gaaaaattat taattatttt 840
gaaaattatg atcccatgga ttctgtggct ttgaacacag cgtacgccta tgccacggtg 900
ctgacttttt gcacgctcat tttggctata ctgcatcact tatattttta tcacgttcag 960
tgtgctggga tgaggttacg agtagccatg tgccatatga tttatcggaa ggcacttcgt 1020
cttagtaaca tggccatggg gaagacaacc acaggccaga tagtcaatct gctgtccaat 1080
gatgtgaaca agtttgatca ggtgacagtg ttcttacact tcctgtgggc aggaccactg 1140
caggcgatcg cagtgactgc cctactctgg atggagatag gaatatcgtg ccttgctggg 1200
atggcagttc taatcattct cctgcccttg caaagctgtt ttgggaagtt gttctcatca 1260
ctgaggagta aaactgcaac tttcacggat gccaggatca ggaccatgaa tgaagttata 1320
actggtataa ggataataaa aatgtacgcc tgggaaaagt cattttcaaa tcttattacc 1380
aatttgagaa agaaggagat ttccaagatt ctgagaagtt cctgcctcag ggggatgaat 1440
ttggcttcgt ttttcagtgc aagcaaaatc atcgtgtttg tgaccttcac cacctacgtg 1500
ctcctcggca gtgtgatcac agccagccgc gtgttcgtgg cagtgacgct gtatggggct 1560
gtgcggctga cggttaccct cttcttcccc tcagccattg agagggtgtc agaggcaatc 1620
gtcagcatcc gaagaatcca gacctttttg ctacttgatg agatatcaca gcgcaaccgt 1680
cagctgccgt cagatggtaa aaagatggtg catgtgcagg attttactgc tttttgggat 1740
aaggcatcag agaccccaac tctacaaggc ctttccttta ctgtcagacc tggcgaattg 1800
ttagctgtgg tcggccccgt gggagcaggg aagtcatcac tgttaagtgc cgtgctcggg 1860
gaattggccc caagtcacgg gctggtcagc gtgcatggaa gaattgccta tgtgtctcag 1920
cagccctggg tgttctcggg aactctgagg agtaatattt tatttgggaa gaaatacgaa 1980
aaggaacgat atgaaaaagt cataaaggct tgtgctctga aaaaggattt acagctgttg 2040
gaggatggtg atctgactgt gataggagat cggggaacca cgctgagtgg agggcagaaa 2100
gcacgggtaa accttgcaag agcagtgtat caagatgctg acatctatct cctggacgat 2160
cctctcagtg cagtagatgc ggaagttagc agacacttgt tcgaactgtg tatttgtcaa 2220
attttgcatg agaagatcac aattttagtg actcatcagt tgcagtacct caaagctgca 2280
agtcagattc tgatattgaa agatggtaaa atggtgcaga aggggactta cactgagttc 2340
ctaaaatctg gtatagattt tggctccctt ttaaagaagg ataatgagga aagtgaacaa 2400
cctccagttc caggaactcc cacactaagg aatcgtacct tctcagagtc ttcggtttgg 2460
tctcaacaat cttctagacc ctccttgaaa gatggtgctc tggagagcca agatacagag 2520
aatgtcccag ttacactatc agaggagaac cgttctgaag gaaaagttgg ttttcaggcc 2580
tataagaatt acttcagagc tggtgctcac tggattgtct tcattttcct tattctccta 2640
aacactgcag ctcaggttgc ctatgtgctt caagattggt ggctttcata ctgggcaaac 2700
aaacaaagta tgctaaatgt cactgtaaat ggaggaggaa atgtaaccga gaagctagat 2760
cttaactggt acttaggaat ttattcaggt ttaactgtag ctaccgttct ttttggcata 2820
gcaagatctc tattggtatt ctacgtcctt gttaactctt cacaaacttt gcacaacaaa 2880
atgtttgagt caattctgaa agctccggta ttattctttg atagaaatcc aataggaaga 2940
attttaaatc gtttctccaa agacattgga cacttggatg atttgctgcc gctgacgttt 3000
ttagatttca tccagacatt gctacaagtg gttggtgtgg tctctgtggc tgtggccgtg 3060
attccttgga tcgcaatacc cttggttccc cttggaatca ttttcatttt tcttcggcga 3120
tattttttgg aaacgtcaag agatgtgaag cgcctggaat ctacaactcg gagtccagtg 3180
ttttcccact tgtcatcttc tctccagggg ctctggacca tccgggcata caaagcagaa 3240
gagaggtgtc aggaactgtt tgatgcacac caggatttac attcagaggc ttggttcttg 3300
tttttgacaa cgtcccgctg gttcgccgtc cgtctggatg ccatctgtgc catgtttgtc 3360
atcatcgttg cctttgggtc cctgattctg gcaaaaactc tggatgccgg gcaggttggt 3420
ttggcactgt cctatgccct cacgctcatg gggatgtttc agtggtgtgt tcgacaaagt 3480
gctgaagttg agaatatgat gatctcagta gaaagggtca ttgaatacac agaccttgaa 3540
aaagaagcac cttgggaata tcagaaacgc ccaccaccag cctggcccca tgaaggagtg 3600
ataatctttg acaatgtgaa cttcatgtac agtccaggtg ggcctctggt actgaagcat 3660
ctgacagcac tcattaaatc acaagaaaag gttggcattg tgggaagaac cggagctgga 3720
aaaagttccc tcatctcagc cctttttaga ttgtcagaac ccgaaggtaa aatttggatt 3780
gataagatct tgacaactga aattggactt cacgatttaa ggaagaaaat gtcaatcata 3840
cctcaggaac ctgttttgtt cactggaaca atgaggaaaa acctggatcc ctttaatgag 3900
cacacggatg aggaactgtg gaatgcctta caagaggtac aacttaaaga aaccattgaa 3960
gatcttcctg gtaaaatgga tactgaatta gcagaatcag gatccaattt tagtgttgga 4020
caaagacaac tggtgtgcct tgccagggca attctcagga aaaatcagat attgattatt 4080
gatgaagcga cggcaaatgt ggatccaaga actgatgagt taatacaaaa aaaaatccgg 4140
gagaaatttg cccactgcac cgtgctaacc attgcacaca gattgaacac cattattgac 4200
agcgacaaga taatggtttt agattcagga agactgaaag aatatgatga gccgtatgtt 4260
ttgctgcaaa ataaagagag cctattttac aagatggtgc aacaactggg caaggcagaa 4320
gccgctgccc tcactgaaac agcaaaacag agatggggtt tcaccatgtt ggccaggctg 4380
gtctcaaact cctga 4395

<210> 1008
<211> 1464
<212> PRT
<213> Homo sapiens

<400> 1008
Met Ser Cys Gly Asn Ala Lys Ile Asn Ser Pro Ala Pro Ser Phe Glu
                5                   10                  15

Glu Val Ala Leu Met Pro Asn Gly Ser Phe Lys Lys Ile Ser Leu Ser
            20                  25                  30

Ser Tyr Lys Gly Lys Trp Val Val Leu Phe Phe Tyr Pro Leu Asp Phe
        35                  40                  45

Thr Phe Val Cys Pro Thr Glu Val Ile Ala Phe Ser Asp Ser Val Ser
    50                  55                  60

Arg Phe Asn Glu Leu Asn Cys Glu Val Leu Ala Cys Ser Ile Asp Ser
65                  70                  75                  80

Glu Tyr Ala His Leu Gln Trp Thr Leu Gln Asp Arg Lys Lys Gly Gly
                85                  90                  95

Leu Gly Thr Met Ala Ile Pro Met Leu Ala Asp Lys Thr Lys Ser Ile
            100                 105                 110

Ala Arg Ser Tyr Gly Val Leu Glu Glu Ser Gln Gly Val Ala Tyr Arg
        115                 120                 125

Gly Leu Phe Ile Ile Asp Pro His Gly Met Leu Arg Gln Ile Thr Val
    130                 135                 140

Asn Asp Met Pro Val Gly Arg Ser Val Glu Glu Val Leu Arg Leu Leu
145                 150                 155                 160

Glu Ala Phe Gln Phe Val Glu Lys His Gly Glu Val Cys Pro Ala Asn
                165                 170                 175

Trp Lys Lys Gly Ala Pro Thr Met Lys Pro Glu Pro Asn Ala Ser Val
            180                 185                 190

Glu Gly Tyr Phe Ser Lys Gln Ser Arg Glu Phe Met Tyr Ser Val Leu
        195                 200                 205

Pro Glu Asp Arg Ser Gln His Leu Gly Glu Glu Leu Gln Gly Phe Trp
    210                 215                 220

Asp Lys Glu Val Leu Arg Ala Glu Asn Asp Ala Gln Lys Pro Ser Leu
225                 230                 235                 240

Thr Arg Ala Ile Ile Lys Cys Tyr Trp Lys Ser Tyr Leu Val Leu Gly
                245                 250                 255

Ile Phe Thr Leu Ile Glu Glu Ser Ala Lys Val Ile Gln Pro Ile Phe
            260                 265                 270

Leu Gly Lys Ile Ile Asn Tyr Phe Glu Asn Tyr Asp Pro Met Asp Ser
        275                 280                 285

Val Ala Leu Asn Thr Ala Tyr Ala Tyr Ala Thr Val Leu Thr Phe Cys
    290                 295                 300

Thr Leu Ile Leu Ala Ile Leu His His Leu Tyr Phe Tyr His Val Gln
305                 310                 315                 320

Cys Ala Gly Met Arg Leu Arg Val Ala Met Cys His Met Ile Tyr Arg
                325                 330                 335

Lys Ala Leu Arg Leu Ser Asn Met Ala Met Gly Lys Thr Thr Thr Gly
            340                 345                 350

Gln Ile Val Asn Leu Leu Ser Asn Asp Val Asn Lys Phe Asp Gln Val
        355                 360                 365

Thr Val Phe Leu His Phe Leu Trp Ala Gly Pro Leu Gln Ala Ile Ala
    370                 375                 380

Val Thr Ala Leu Leu Trp Met Glu Ile Gly Ile Ser Cys Leu Ala Gly
385                 390                 395                 400

Met Ala Val Leu Ile Ile Leu Leu Pro Leu Gln Ser Cys Phe Gly Lys
                405                 410                 415

Leu Phe Ser Ser Leu Arg Ser Lys Thr Ala Thr Phe Thr Asp Ala Arg
            420                 425                 430

Ile Arg Thr Met Asn Glu Val Ile Thr Gly Ile Arg Ile Ile Lys Met
        435                 440                 445

Tyr Ala Trp Glu Lys Ser Phe Ser Asn Leu Ile Thr Asn Leu Arg Lys
    450                 455                 460

Lys Glu Ile Ser Lys Ile Leu Arg Ser Ser Cys Leu Arg Gly Met Asn
465                 470                 475                 480

Leu Ala Ser Phe Phe Ser Ala Ser Lys Ile Ile Val Phe Val Thr Phe
                485                 490                 495

Thr Thr Tyr Val Leu Leu Gly Ser Val Ile Thr Ala Ser Arg Val Phe
            500                 505                 510

Val Ala Val Thr Leu Tyr Gly Ala Val Arg Leu Thr Val Thr Leu Phe
        515                 520                 525

Phe Pro Ser Ala Ile Glu Arg Val Ser Glu Ala Ile Val Ser Ile Arg
    530                 535                 540

Arg Ile Gln Thr Phe Leu Leu Leu Asp Glu Ile Ser Gln Arg Asn Arg
545                 550                 555                 560

Gln Leu Pro Ser Asp Gly Lys Lys Met Val His Val Gln Asp Phe Thr
                565                 570                 575

Ala Phe Trp Asp Lys Ala Ser Glu Thr Pro Thr Leu Gln Gly Leu Ser
            580                 585                 590

Phe Thr Val Arg Pro Gly Glu Leu Leu Ala Val Val Gly Pro Val Gly
        595                 600                 605

Ala Gly Lys Ser Ser Leu Leu Ser Ala Val Leu Gly Glu Leu Ala Pro
    610                 615                 620

Ser His Gly Leu Val Ser Val His Gly Arg Ile Ala Tyr Val Ser Gln
625                 630                 635                 640

Gln Pro Trp Val Phe Ser Gly Thr Leu Arg Ser Asn Ile Leu Phe Gly
                645                 650                 655

Lys Lys Tyr Glu Lys Glu Arg Tyr Glu Lys Val Ile Lys Ala Cys Ala
            660                 665                 670

Leu Lys Lys Asp Leu Gln Leu Leu Glu Asp Gly Asp Leu Thr Val Ile
        675                 680                 685

Gly Asp Arg Gly Thr Thr Leu Ser Gly Gly Gln Lys Ala Arg Val Asn
    690                 695                 700

Leu Ala Arg Ala Val Tyr Gln Asp Ala Asp Ile Tyr Leu Leu Asp Asp
705                 710                 715                 720

Pro Leu Ser Ala Val Asp Ala Glu Val Ser Arg His Leu Phe Glu Leu
                725                 730                 735

Cys Ile Cys Gln Ile Leu His Glu Lys Ile Thr Ile Leu Val Thr His
            740                 745                 750

Gln Leu Gln Tyr Leu Lys Ala Ala Ser Gln Ile Leu Ile Leu Lys Asp
        755                 760                 765

Gly Lys Met Val Gln Lys Gly Thr Tyr Thr Glu Phe Leu Lys Ser Gly
    770                 775                 780

Ile Asp Phe Gly Ser Leu Leu Lys Lys Asp Asn Glu Glu Ser Glu Gln
785                 790                 795                 800

Pro Pro Val Pro Gly Thr Pro Thr Leu Arg Asn Arg Thr Phe Ser Glu
                805                 810                 815

Ser Ser Val Trp Ser Gln Gln Ser Ser Arg Pro Ser Leu Lys Asp Gly
            820                 825                 830

Ala Leu Glu Ser Gln Asp Thr Glu Asn Val Pro Val Thr Leu Ser Glu
        835                 840                 845

Glu Asn Arg Ser Glu Gly Lys Val Gly Phe Gln Ala Tyr Lys Asn Tyr
    850                 855                 860

Phe Arg Ala Gly Ala His Trp Ile Val Phe Ile Phe Leu Ile Leu Leu
865                 870                 875                 880

Asn Thr Ala Ala Gln Val Ala Tyr Val Leu Gln Asp Trp Trp Leu Ser
                885                 890                 895

Tyr Trp Ala Asn Lys Gln Ser Met Leu Asn Val Thr Val Asn Gly Gly
            900                 905                 910

Gly Asn Val Thr Glu Lys Leu Asp Leu Asn Trp Tyr Leu Gly Ile Tyr
        915                 920                 925

Ser Gly Leu Thr Val Ala Thr Val Leu Phe Gly Ile Ala Arg Ser Leu
    930                 935                 940

Leu Val Phe Tyr Val Leu Val Asn Ser Ser Gln Thr Leu His Asn Lys
945                 950                 955                 960

Met Phe Glu Ser Ile Leu Lys Ala Pro Val Leu Phe Phe Asp Arg Asn
                965                 970                 975

Pro Ile Gly Arg Ile Leu Asn Arg Phe Ser Lys Asp Ile Gly His Leu
            980                 985                 990

Asp Asp Leu Leu Pro Leu Thr Phe Leu Asp Phe Ile Gln Thr Leu Leu
        995                 1000                1005

Gln Val Val Gly Val Val Ser Val Ala Val Ala Val Ile Pro Trp Ile
    1010                1015                1020

Ala Ile Pro Leu Val Pro Leu Gly Ile Ile Phe Ile Phe Leu Arg Arg
1025                1030                1035                1040

Tyr Phe Leu Glu Thr Ser Arg Asp Val Lys Arg Leu Glu Ser Thr Thr
                1045                1050                1055

Arg Ser Pro Val Phe Ser His Leu Ser Ser Ser Leu Gln Gly Leu Trp
            1060                1065                1070

Thr Ile Arg Ala Tyr Lys Ala Glu Glu Arg Cys Gln Glu Leu Phe Asp

1075                    1080                    1085

Ala His Gln Asp Leu His Ser Glu Ala Trp Phe Leu Phe Leu Thr Thr
    1090                    1095                    1100

Ser Arg Trp Phe Ala Val Arg Leu Asp Ala Ile Cys Ala Met Phe Val
1105                    1110                    1115                    1120

Ile Ile Val Ala Phe Gly Ser Leu Ile Leu Ala Lys Thr Leu Asp Ala
                1125                    1130                    1135

Gly Gln Val Gly Leu Ala Leu Ser Tyr Ala Leu Thr Leu Met Gly Met
            1140                    1145                    1150

Phe Gln Trp Cys Val Arg Gln Ser Ala Glu Val Glu Asn Met Met Ile
        1155                    1160                    1165

Ser Val Glu Arg Val Ile Glu Tyr Thr Asp Leu Glu Lys Glu Ala Pro
    1170                    1175                    1180

Trp Glu Tyr Gln Lys Arg Pro Pro Pro Ala Trp Pro His Glu Gly Val
1185                    1190                    1195                    1200

Ile Ile Phe Asp Asn Val Asn Phe Met Tyr Ser Pro Gly Gly Pro Leu
                1205                    1210                    1215

Val Leu Lys His Leu Thr Ala Leu Ile Lys Ser Gln Glu Lys Val Gly
            1220                    1225                    1230

Ile Val Gly Arg Thr Gly Ala Gly Lys Ser Ser Leu Ile Ser Ala Leu
        1235                    1240                    1245

Phe Arg Leu Ser Glu Pro Glu Gly Lys Ile Trp Ile Asp Lys Ile Leu
    1250                    1255                    1260

Thr Thr Glu Ile Gly Leu His Asp Leu Arg Lys Lys Met Ser Ile Ile
1265                    1270                    1275                    1280

Pro Gln Glu Pro Val Leu Phe Thr Gly Thr Met Arg Lys Asn Leu Asp
                1285                    1290                    1295

Pro Phe Asn Glu His Thr Asp Glu Glu Leu Trp Asn Ala Leu Gln Glu
            1300                    1305                    1310

Val Gln Leu Lys Glu Thr Ile Glu Asp Leu Pro Gly Lys Met Asp Thr
        1315                    1320                    1325

Glu Leu Ala Glu Ser Gly Ser Asn Phe Ser Val Gly Gln Arg Gln Leu
    1330                    1335                    1340

Val Cys Leu Ala Arg Ala Ile Leu Arg Lys Asn Gln Ile Leu Ile Ile
1345                    1350                    1355                    1360

Asp Glu Ala Thr Ala Asn Val Asp Pro Arg Thr Asp Glu Leu Ile Gln
                1365                    1370                    1375

Lys Lys Ile Arg Glu Lys Phe Ala His Cys Thr Val Leu Thr Ile Ala
            1380                    1385                    1390

His Arg Leu Asn Thr Ile Ile Asp Ser Asp Lys Ile Met Val Leu Asp
        1395                    1400                    1405

Ser Gly Arg Leu Lys Glu Tyr Asp Glu Pro Tyr Val Leu Leu Gln Asn
    1410                    1415                    1420

Lys Glu Ser Leu Phe Tyr Lys Met Val Gln Gln Leu Gly Lys Ala Glu
1425                    1430                    1435                    1440

Ala Ala Ala Leu Thr Glu Thr Ala Lys Gln Arg Trp Gly Phe Thr Met
                1445                1450                1455

Leu Ala Arg Leu Val Ser Asn Ser
            1460


<210> 1009
<211> 1261
<212> PRT
<213> Homo sapiens

<400> 1009
Met Tyr Ser Val Leu Pro Glu Asp Arg Ser Gln His Leu Gly Glu Glu
                5                   10                  15

Leu Gln Gly Phe Trp Asp Lys Glu Val Leu Arg Ala Glu Asn Asp Ala
            20                  25                  30

Gln Lys Pro Ser Leu Thr Arg Ala Ile Ile Lys Cys Tyr Trp Lys Ser
        35                  40                  45

Tyr Leu Val Leu Gly Ile Phe Thr Leu Ile Glu Glu Ser Ala Lys Val
    50                  55                  60

Ile Gln Pro Ile Phe Leu Gly Lys Ile Ile Asn Tyr Phe Glu Asn Tyr
65                  70                  75                  80

Asp Pro Met Asp Ser Val Ala Leu Asn Thr Ala Tyr Ala Tyr Ala Thr
                85                  90                  95

Val Leu Thr Phe Cys Thr Leu Ile Leu Ala Ile Leu His His Leu Tyr
            100                 105                 110

Phe Tyr His Val Gln Cys Ala Gly Met Arg Leu Arg Val Ala Met Cys
        115                 120                 125

His Met Ile Tyr Arg Lys Ala Leu Arg Leu Ser Asn Met Ala Met Gly
    130                 135                 140

Lys Thr Thr Thr Gly Gln Ile Val Asn Leu Leu Ser Asn Asp Val Asn
145                 150                 155                 160

Lys Phe Asp Gln Val Thr Val Phe Leu His Phe Leu Trp Ala Gly Pro
                165                 170                 175

Leu Gln Ala Ile Ala Val Thr Ala Leu Leu Trp Met Glu Ile Gly Ile
            180                 185                 190

Ser Cys Leu Ala Gly Met Ala Val Leu Ile Ile Leu Leu Pro Leu Gln
        195                 200                 205

Ser Cys Phe Gly Lys Leu Phe Ser Ser Leu Arg Ser Lys Thr Ala Thr
    210                 215                 220

Phe Thr Asp Ala Arg Ile Arg Thr Met Asn Glu Val Ile Thr Gly Ile
225                 230                 235                 240

Arg Ile Ile Lys Met Tyr Ala Trp Glu Lys Ser Phe Ser Asn Leu Ile
                245                 250                 255

Thr Asn Leu Arg Lys Lys Glu Ile Ser Lys Ile Leu Arg Ser Ser Cys
            260                 265                 270

Leu Arg Gly Met Asn Leu Ala Ser Phe Phe Ser Ala Ser Lys Ile Ile
        275                 280                 285

Val Phe Val Thr Phe Thr Thr Tyr Val Leu Leu Gly Ser Val Ile Thr

290                 295                 300

Ala Ser Arg Val Phe Val Ala Val Thr Leu Tyr Gly Ala Val Arg Leu
305                 310                 315                 320

Thr Val Thr Leu Phe Phe Pro Ser Ala Ile Glu Arg Val Ser Glu Ala
                325                 330                 335

Ile Val Ser Ile Arg Arg Ile Gln Thr Phe Leu Leu Leu Asp Glu Ile
            340                 345                 350

Ser Gln Arg Asn Arg Gln Leu Pro Ser Asp Gly Lys Lys Met Val His
        355                 360                 365

Val Gln Asp Phe Thr Ala Phe Trp Asp Lys Ala Ser Glu Thr Pro Thr
    370                 375                 380

Leu Gln Gly Leu Ser Phe Thr Val Arg Pro Gly Glu Leu Leu Ala Val
385                 390                 395                 400

Val Gly Pro Val Gly Ala Gly Lys Ser Ser Leu Leu Ser Ala Val Leu
                405                 410                 415

Gly Glu Leu Ala Pro Ser His Gly Leu Val Ser Val His Gly Arg Ile
            420                 425                 430

Ala Tyr Val Ser Gln Gln Pro Trp Val Phe Ser Gly Thr Leu Arg Ser
        435                 440                 445

Asn Ile Leu Phe Gly Lys Lys Tyr Glu Lys Glu Arg Tyr Glu Lys Val
    450                 455                 460

Ile Lys Ala Cys Ala Leu Lys Lys Asp Leu Gln Leu Leu Glu Asp Gly
465                 470                 475                 480

Asp Leu Thr Val Ile Gly Asp Arg Gly Thr Thr Leu Ser Gly Gly Gln
                485                 490                 495

Lys Ala Arg Val Asn Leu Ala Arg Ala Val Tyr Gln Asp Ala Asp Ile
            500                 505                 510

Tyr Leu Leu Asp Asp Pro Leu Ser Ala Val Asp Ala Glu Val Ser Arg
        515                 520                 525

His Leu Phe Glu Leu Cys Ile Cys Gln Ile Leu His Glu Lys Ile Thr
    530                 535                 540

Ile Leu Val Thr His Gln Leu Gln Tyr Leu Lys Ala Ala Ser Gln Ile
545                 550                 555                 560

Leu Ile Leu Lys Asp Gly Lys Met Val Gln Lys Gly Thr Tyr Thr Glu
                565                 570                 575

Phe Leu Lys Ser Gly Ile Asp Phe Gly Ser Leu Leu Lys Lys Asp Asn
            580                 585                 590

Glu Glu Ser Glu Gln Pro Pro Val Pro Gly Thr Pro Thr Leu Arg Asn
        595                 600                 605

Arg Thr Phe Ser Glu Ser Ser Val Trp Ser Gln Gln Ser Ser Arg Pro
    610                 615                 620

Ser Leu Lys Asp Gly Ala Leu Glu Ser Gln Asp Thr Glu Asn Val Pro
625                 630                 635                 640

Val Thr Leu Ser Glu Glu Asn Arg Ser Glu Gly Lys Val Gly Phe Gln
                645                 650                 655

Ala Tyr Lys Asn Tyr Phe Arg Ala Gly Ala His Trp Ile Val Phe Ile
            660                 665                 670

Phe Leu Ile Leu Leu Asn Thr Ala Ala Gln Val Ala Tyr Val Leu Gln
        675                 680                 685

Asp Trp Trp Leu Ser Tyr Trp Ala Asn Lys Gln Ser Met Leu Asn Val
    690                 695                 700

Thr Val Asn Gly Gly Gly Asn Val Thr Glu Lys Leu Asp Leu Asn Trp
705                 710                 715                 720

Tyr Leu Gly Ile Tyr Ser Gly Leu Thr Val Ala Thr Val Leu Phe Gly
                725                 730                 735

Ile Ala Arg Ser Leu Leu Val Phe Tyr Val Leu Val Asn Ser Ser Gln
            740                 745                 750

Thr Leu His Asn Lys Met Phe Glu Ser Ile Leu Lys Ala Pro Val Leu
        755                 760                 765

Phe Phe Asp Arg Asn Pro Ile Gly Arg Ile Leu Asn Arg Phe Ser Lys
    770                 775                 780

Asp Ile Gly His Leu Asp Asp Leu Leu Pro Leu Thr Phe Leu Asp Phe
785                 790                 795                 800

Ile Gln Thr Leu Leu Gln Val Val Gly Val Val Ser Val Ala Val Ala
                805                 810                 815

Val Ile Pro Trp Ile Ala Ile Pro Leu Val Pro Leu Gly Ile Ile Phe
            820                 825                 830

Ile Phe Leu Arg Arg Tyr Phe Leu Glu Thr Ser Arg Asp Val Lys Arg
        835                 840                 845

Leu Glu Ser Thr Thr Arg Ser Pro Val Phe Ser His Leu Ser Ser Ser
    850                 855                 860

Leu Gln Gly Leu Trp Thr Ile Arg Ala Tyr Lys Ala Glu Glu Arg Cys
865                 870                 875                 880

Gln Glu Leu Phe Asp Ala His Gln Asp Leu His Ser Glu Ala Trp Phe
                885                 890                 895

Leu Phe Leu Thr Thr Ser Arg Trp Phe Ala Val Arg Leu Asp Ala Ile
            900                 905                 910

Cys Ala Met Phe Val Ile Ile Val Ala Phe Gly Ser Leu Ile Leu Ala
        915                 920                 925

Lys Thr Leu Asp Ala Gly Gln Val Gly Leu Ala Leu Ser Tyr Ala Leu
    930                 935                 940

Thr Leu Met Gly Met Phe Gln Trp Cys Val Arg Gln Ser Ala Glu Val
945                 950                 955                 960

Glu Asn Met Met Ile Ser Val Glu Arg Val Ile Glu Tyr Thr Asp Leu
                965                 970                 975

Glu Lys Glu Ala Pro Trp Glu Tyr Gln Lys Arg Pro Pro Pro Ala Trp
            980                 985                 990

Pro His Glu Gly Val Ile Ile Phe Asp Asn Val Asn Phe Met Tyr Ser
        995                 1000                1005

Pro Gly Gly Pro Leu Val Leu Lys His Leu Thr Ala Leu Ile Lys Ser
    1010                1015                1020

Gln Glu Lys Val Gly Ile Val Gly Arg Thr Gly Ala Gly Lys Ser Ser
1025                1030                1035                1040

Leu Ile Ser Ala Leu Phe Arg Leu Ser Glu Pro Glu Gly Lys Ile Trp
                1045                1050                1055

Ile Asp Lys Ile Leu Thr Thr Glu Ile Gly Leu His Asp Leu Arg Lys
            1060                1065                1070

Lys Met Ser Ile Ile Pro Gln Glu Pro Val Leu Phe Thr Gly Thr Met
        1075                1080                1085

Arg Lys Asn Leu Asp Pro Phe Asn Glu His Thr Asp Glu Glu Leu Trp
    1090                1095                1100

Asn Ala Leu Gln Glu Val Gln Leu Lys Glu Thr Ile Glu Asp Leu Pro
1105                1110                1115                1120

Gly Lys Met Asp Thr Glu Leu Ala Glu Ser Gly Ser Asn Phe Ser Val
                1125                1130                1135

Gly Gln Arg Gln Leu Val Cys Leu Ala Arg Ala Ile Leu Arg Lys Asn
            1140                1145                1150

Gln Ile Leu Ile Ile Asp Glu Ala Thr Ala Asn Val Asp Pro Arg Thr
        1155                1160                1165

Asp Glu Leu Ile Gln Lys Lys Ile Arg Glu Lys Phe Ala His Cys Thr
    1170                1175                1180

Val Leu Thr Ile Ala His Arg Leu Asn Thr Ile Ile Asp Ser Asp Lys
1185                1190                1195                1200

Ile Met Val Leu Asp Ser Gly Arg Leu Lys Glu Tyr Asp Glu Pro Tyr
                1205                1210                1215

Val Leu Leu Gln Asn Lys Glu Ser Leu Phe Tyr Lys Met Val Gln Gln
            1220                1225                1230

Leu Gly Lys Ala Glu Ala Ala Ala Leu Thr Glu Thr Ala Lys Gln Arg
        1235                1240                1245

Trp Gly Phe Thr Met Leu Ala Arg Leu Val Ser Asn Ser
    1250                1255                1260

<210> 1010
<211> 1065
<212> DNA
<213> Homo sapiens

<400> 1010
atgagcgata aaattattca cctgactgac gacagttttg acacggatgt actcaaagcg 60
gacggggcga tcctcgtcga tttctgggca gagtggtgcg gtccgtgcaa aatgatcgcc 120
ccgattctgg atgaaatcgc tgacgaatat cagggcaaac tgaccgttgc aaaactgaac 180
atcgatcaaa accctggcac tgcgccgaaa tatggcatcc gtggtatccc gactctgctg 240
ctgttcaaaa acggtgaagt ggcggcaacc aaagtgggtg cactgtctaa aggtcagttg 300
aaagagttcc tcgacgctaa cctggccggt tctggttctg gccatatgca ccatcatcat 360
catcattctt ctggtctggt gccacgcggt tctggtatga aagaaaccgc tgctgctaaa 420
ttcgaacgcc agcacatgga cagcccagat ctgggtaccg acgacgacga caaggccatg 480
gaccggctgg tgcagcgatt cggcactcga gcagtctatt tggccagtgt ggcagctttc 540
cctgtggctg ccggtgccac atgcctgtcc cacagtgtgg ccgtggtgac agcttcagcc 600
gccctcaccg ggttcacctt ctcagccctg cagatcctgc cctacacact ggcctccctc 660
taccaccggg agaagcaggt gttcctgccc aaataccgag gggacactgg aggtgctagc 720
agtgaggaca gcctgatgac cagcttcctg ccaggcccta agcctggagc tcccttccct 780
aatggacacg tgggtgctgg aggcagtggc ctgctcccac ctccacccgc gctctgcggg 840

gcctctgcct gtgatgtctc cgtacgtgtg gtggtgggtg agcccaccga ggccagggtg 900
gttccgggcc ggggcatctg cctggacctc gccatcctgg atagtgcctt cctgctgtcc 960
caggtggccc catccctgtt tatgggctcc attgtccagc tcagccagtc tgtcactgcc 1020
tatatggtgt ctgcggccgc actcgagcac caccaccacc accac 1065

<210> 1011
<211> 355
<212> PRT
<213> Homo sapiens

<400> 1011
Met Ser Asp Lys Ile Ile His Leu Thr Asp Asp Ser Phe Asp Thr Asp
                5                   10                  15

Val Leu Lys Ala Asp Gly Ala Ile Leu Val Asp Phe Trp Ala Glu Trp
            20                  25                  30

Cys Gly Pro Cys Lys Met Ile Ala Pro Ile Leu Asp Glu Ile Ala Asp
        35                  40                  45

Glu Tyr Gln Gly Lys Leu Thr Val Ala Lys Leu Asn Ile Asp Gln Asn
    50                  55                  60

Pro Gly Thr Ala Pro Lys Tyr Gly Ile Arg Gly Ile Pro Thr Leu Leu
65                  70                  75                  80

Leu Phe Lys Asn Gly Glu Val Ala Ala Thr Lys Val Gly Ala Leu Ser
                85                  90                  95

Lys Gly Gln Leu Lys Glu Phe Leu Asp Ala Asn Leu Ala Gly Ser Gly
            100                 105                 110

Ser Gly His Met His His His His His His Ser Ser Gly Leu Val Pro
        115                 120                 125

Arg Gly Ser Gly Met Lys Glu Thr Ala Ala Ala Lys Phe Glu Arg Gln
    130                 135                 140

His Met Asp Ser Pro Asp Leu Gly Thr Asp Asp Asp Asp Lys Ala Met
145                 150                 155                 160

Asp Arg Leu Val Gln Arg Phe Gly Thr Arg Ala Val Tyr Leu Ala Ser
                165                 170                 175

Val Ala Ala Phe Pro Val Ala Ala Gly Ala Thr Cys Leu Ser His Ser
            180                 185                 190

Val Ala Val Val Thr Ala Ser Ala Ala Leu Thr Gly Phe Thr Phe Ser
        195                 200                 205

Ala Leu Gln Ile Leu Pro Tyr Thr Leu Ala Ser Leu Tyr His Arg Glu
    210                 215                 220

Lys Gln Val Phe Leu Pro Lys Tyr Arg Gly Asp Thr Gly Gly Ala Ser
225                 230                 235                 240

Ser Glu Asp Ser Leu Met Thr Ser Phe Leu Pro Gly Pro Lys Pro Gly
                245                 250                 255

Ala Pro Phe Pro Asn Gly His Val Gly Ala Gly Gly Ser Gly Leu Leu
            260                 265                 270

Pro Pro Pro Pro Ala Leu Cys Gly Ala Ser Ala Cys Asp Val Ser Val
        275                 280                 285

Arg Val Val Val Gly Glu Pro Thr Glu Ala Arg Val Val Pro Gly Arg
    290                 295                 300

Gly Ile Cys Leu Asp Leu Ala Ile Leu Asp Ser Ala Phe Leu Leu Ser
305                 310                 315                 320

Gln Val Ala Pro Ser Leu Phe Met Gly Ser Ile Val Gln Leu Ser Gln
                325                 330                 335

Ser Val Thr Ala Tyr Met Val Ser Ala Ala Ala Leu Glu His His His
            340                 345                 350

His His His
        355

<210> 1012
<211> 9
<212> PRT
<213> Homo sapiens

<400> 1012
Ala Cys Asp Val Ser Val Arg Val Val
                5

<210> 1013
<211> 27
<212> DNA
<213> Homo sapiens

<400> 1013
gcctgtgatg tctccgtacg tgtggtg                                          27

<210> 1014
<211> 42
<212> DNA
<213> Artificial Sequence

<220>
<223> Primer sequence for the forward primer hPAPF1.

<400> 1014
cggcggatcc gccgccacca tgagagctgc acccctcctc ct                         42

<210> 1015
<211> 44
<212> DNA
<213> Artificial Sequence

<220>
<223> Primer sequence for the reverse primer hPAPRV1.

<400> 1015
cggcctcgag ctaatctgta ctgtcttcag taccttgatg gctg                       44

<210> 1016
<211> 48
<212> DNA
<213> Artificial Sequence

<220>
<223> Primer sequence for the forward primer FOPP2F1.

<400> 1016
gagaaaaccg tccaggccag taaggagttg aagtttgtga ctttggtg                   48

<210> 1017
<211> 54
<212> DNA
<213> Artificial Sequence

<220>
<223> Primer sequence for the reverse primer FOPPF1.

<400> 1017
cggcgggcat atgcatcacc atcaccatca catcataaac ggcgaggact gcag 54

<210> 1018
<211> 48
<212> DNA
<213> Artificial Sequence

<220>
<223> Primer sequence for the forward primer FOPP2RV1.

<400> 1018
caccaaagtc acaaacttca actccttact ggcctggacg gttttctc 48

<210> 1019
<211> 1758
<212> DNA
<213> Homo sapiens

<400> 1019
atgcatcacc atcaccatca catcataaac ggcgaggact gcagcccgca ctcgcagccc 60
tggcaggcgg cactggtcat ggaaaacgaa ttgttctgct cgggcgtcct ggtgcatccg 120
cagtgggtgc tgtcagccgc acactgtttc cagaactcct acaccatcgg gctgggcctg 180
cacagtcttg aggccgacca agagccaggg agccagatgg tggaggccag cctctccgta 240
cggcacccag agtacaacag acccttgctc gctaacgacc tcatgctcat caagttggac 300
gaatccgtgt ccgagtctga caccatccgg agcatcagca ttgcttcgca gtgccctacc 360
gcggggaact cttgcctcgt ttctggctgg ggtctgctgg cgaacggcag aatgcctacc 420
gtgctgcagt gcgtgaacgt gtcggtggtg tctgaggagg tctgcagtaa gctctatgac 480
ccgctgtacc accccagcat gttctgcgcc ggcggagggc aagaccagaa ggactcctgc 540
aacggtgact ctgggggccc cctgatctgc aacgggtact tgcagggcct tgtgtctttc 600
ggaaaagccc cgtgtggcca agttggcgtg ccaggtgtct acaccaacct ctgcaaattc 660
actgagtgga tagagaaaac cgtccaggcc agtaaggagt tgaagtttgt gactttggtg 720
tttcggcatg gagaccgaag tcccattgac acctttccca ctgaccccat aaaggaatcc 780
tcatggccac aaggatttgg ccaactcacc cagctgggca tggagcagca ttatgaactt 840
ggagagtata taagaaagag atatagaaaa ttcttgaatg agtcctataa acatgaacag 900
gtttatattc gaagcacaga cgttgaccgg actttgatga gtgctatgac aaacctggca 960
gccctgtttc ccccagaagg tgtcagcatc tggaatccta tcctactctg gcagcccatc 1020
ccggtgcaca cagttcctct ttctgaagat cagttgctat acctgccttt caggaactgc 1080
cctcgttttc aagaacttga gagtgagact ttgaaatcag aggaattcca gaagaggctg 1140
cacccttata aggattttat agctaccttg ggaaaacttt caggattaca tggccaggac 1200
ctttttggaa tttggagtaa agtctacgac cctttatatt gtgagagtgt tcacaatttc 1260
actttaccct cctgggccac tgaggacacc atgactaagt tgagagaatt gtcagaattg 1320
tccctcctgt ccctctatgg aattcacaag cagaaagaga aatctaggct ccaagggggt 1380
gtcctggtca atgaaatcct caatcacatg aagagagcaa ctcagatacc aagctacaaa 1440
aaactcatca tgtattctgc gcatgacact actgtgagtg gcctacagat ggcgctagat 1500
gtttacaacg gactccttcc tccctatgct tcttgccact tgacggaatt gtactttgag 1560
aagggggagt actttgtgga gatgtactat cggaatgaga cgcagcacga gccgtatccc 1620
ctcatgctac ctggctgcag ccccagctgt cctctggaga ggtttgctga gctggttggc 1680
cctgtgatcc ctcaagactg gtccacggag tgtatgacca caaacagcca tcaaggtact 1740
gaagacagta cagattag 1758

<210> 1020
<211> 585
<212> PRT
<213> Homo sapiens

<400> 1020

<210> 1021
<211> 27
<212> DNA
<213> Homo sapiens

<400> 1021
tacacggatt tcgtgggcga ggggctg                                          27

<210> 1022
<211> 9
<212> PRT
<213> Homo sapiens

<400> 1022
Tyr Thr Asp Phe Val Gly Glu Gly Leu
                5

<210> 1023
<211> 24
<212> DNA
<213> Artificial Sequence

<220>
<223> Primer PDM-930.

<400> 1023
cttccacggc tgcaccagct gtgc                                             24

<210> 1024

<211> 33
<212> DNA
<213> Artificial Sequence

<220>
<223> Primer PDM-165

<400> 1024
cacggacgtg aattctacgc tgagtatttg gcc 33

<210> 1025
<211> 29
<212> DNA
<213> Artificial Sequence

<220>
<223> Primer PDM-929

<400> 1025
cactatgatg aaggcgttcg gatgggcag 29

<210> 1026
<211> 741
<212> DNA
<213> Homo sapiens

<400> 1026
atgcagcatc atcatcatca tcaccactat gatgaaggcg ttcggatggg cagcctgggg 60
ctgttcctgc agtgcgccat ctccctggtc ttctctctgg tcatggaccg gctggtgcag 120
cgattcggca ctcgagcagt ctatttggcc agtgtggcag ctttccctgt ggctgccggt 180
gccacatgcc tgtcccacag tgtggccgtg gtgacagctt cagccgccct caccgggttc 240
accttctcag ccctgcagat cctgccctac acactggcct ccctctacca ccgggagaag 300
caggtgttcc tgcccaaata ccgaggggac actggaggtg ctagcagtga ggacagcctg 360
atgaccagct tcctgccagg ccctaagcct ggagctccct tccctaatgg acacgtgggt 420
gctggaggca gtggcctgct cccacctcca cccgcgctct gcggggcctc tgcctgtgat 480
gtctccgtac gtgtggtggt gggtgagccc accgaggcca gggtggttcc gggccggggc 540
atctgcctgg acctcgccat cctggatagt gccttcctgc tgtcccaggt ggccccatcc 600
ctgtttatgg gctccattgt ccagctcagc cagtctgtca ctgcctatat ggtgtctgcc 660
gcaggcctgg gtctggtcgc catttacttt gctacacagg tagtatttga caagagcgac 720
ttggccaaat actcagcgta g 741

<210> 1027
<211> 918
<212> DNA
<213> Homo sapiens

<400> 1027
atgcagcatc atcatcatca tcaccttcca cggctgcacc agctgtgctg ccgcatgccc 60
cgcaccctgc gccggctctt cgtggctgag ctgtgcagct ggatggcact catgaccttc 120
acgctgtttt acacggattt cgtgggcgag gggctgtacc agggcgtgcc cagagctgag 180
ccgggcaccg aggcccggag acactatgat gaaggcgttc ggatgggcag cctggggctg 240
ttcctgcagt gcgccatctc cctggtcttc tctctggtca tggaccggct ggtgcagcga 300
ttcggcactc gagcagtcta tttggccagt gtggcagctt tccctgtggc tgccggtgcc 360
acatgcctgt cccacagtgt ggccgtggtg acagcttcag ccgccctcac cgggttcacc 420
ttctcagccc tgcagatcct gccctacaca ctggcctccc tctaccaccg ggagaagcag 480
gtgttcctgc ccaaataccg aggggacact ggaggtgcta gcagtgagga cagcctgatg 540
accagcttcc tgccaggccc taagcctgga gctcccttcc ctaatggaca cgtgggtgct 600
ggaggcagtg gcctgctccc acctccaccc gcgctctgcg gggcctctgc ctgtgatgtc 660
tccgtacgtg tggtggtggg tgagcccacc gaggccaggg tggttccggg ccggggcatc 720
tgcctggacc tcgccatcct ggatagtgcc ttcctgctgt cccaggtggc cccatccctg 780
tttatgggct ccattgtcca gctcagccag tctgtcactg cctatatggt gtctgccgca 840
ggcctgggtc tggtcgccat ttactttgct acacaggtag tatttgacaa gagcgacttg 900
gccaaatact cagcgtag 918

<210> 1028
<211> 246

<212> PRT
<213> Homo sapiens

<400> 1028
Met Gln His His His His His His His Tyr Asp Glu Gly Val Arg Met
1               5                   10                  15

Gly Ser Leu Gly Leu Phe Leu Gln Cys Ala Ile Ser Leu Val Phe Ser
            20                  25                  30

Leu Val Met Asp Arg Leu Val Gln Arg Phe Gly Thr Arg Ala Val Tyr
        35                  40                  45

Leu Ala Ser Val Ala Ala Phe Pro Val Ala Ala Gly Ala Thr Cys Leu
    50                  55                  60

Ser His Ser Val Ala Val Val Thr Ala Ser Ala Ala Leu Thr Gly Phe
65                  70                  75                  80

Thr Phe Ser Ala Leu Gln Ile Leu Pro Tyr Thr Leu Ala Ser Leu Tyr
                85                  90                  95

His Arg Glu Lys Gln Val Phe Leu Pro Lys Tyr Arg Gly Asp Thr Gly
            100                 105                 110

Gly Ala Ser Ser Glu Asp Ser Leu Met Thr Ser Phe Leu Pro Gly Pro
        115                 120                 125

Lys Pro Gly Ala Pro Phe Pro Asn Gly His Val Gly Ala Gly Gly Ser
    130                 135                 140

Gly Leu Leu Pro Pro Pro Pro Ala Leu Cys Gly Ala Ser Ala Cys Asp
145                 150                 155                 160

Val Ser Val Arg Val Val Val Gly Glu Pro Thr Glu Ala Arg Val Val
                165                 170                 175

Pro Gly Arg Gly Ile Cys Leu Asp Leu Ala Ile Leu Asp Ser Ala Phe
            180                 185                 190

Leu Leu Ser Gln Val Ala Pro Ser Leu Phe Met Gly Ser Ile Val Gln
        195                 200                 205

Leu Ser Gln Ser Val Thr Ala Tyr Met Val Ser Ala Ala Gly Leu Gly
    210                 215                 220

Leu Val Ala Ile Tyr Phe Ala Thr Gln Val Val Phe Asp Lys Ser Asp
225                 230                 235                 240

Leu Ala Lys Tyr Ser Ala
                245


<210> 1029
<211> 305
<212> PRT
<213> Homo sapiens

<400> 1029
Met Gln His His His His His His Leu Pro Arg Leu His Gln Leu Cys
1               5                   10                  15

Cys Arg Met Pro Arg Thr Leu Arg Arg Leu Phe Val Ala Glu Leu Cys
            20                  25                  30

Ser Trp Met Ala Leu Met Thr Phe Thr Leu Phe Tyr Thr Asp Phe Val
        35                  40                  45

Gly Glu Gly Leu Tyr Gln Gly Val Pro Arg Ala Glu Pro Gly Thr Glu
    50                  55                  60

Ala Arg Arg His Tyr Asp Glu Gly Val Arg Met Gly Ser Leu Gly Leu
65                  70                  75                  80

Phe Leu Gln Cys Ala Ile Ser Leu Val Phe Ser Leu Val Met Asp Arg
                85                  90                  95

Leu Val Gln Arg Phe Gly Thr Arg Ala Val Tyr Leu Ala Ser Val Ala
            100                 105                 110

Ala Phe Pro Val Ala Ala Gly Ala Thr Cys Leu Ser His Ser Val Ala
        115                 120                 125

Val Val Thr Ala Ser Ala Ala Leu Thr Gly Phe Thr Phe Ser Ala Leu
    130                 135                 140

Gln Ile Leu Pro Tyr Thr Leu Ala Ser Leu Tyr His Arg Glu Lys Gln
145                 150                 155                 160

Val Phe Leu Pro Lys Tyr Arg Gly Asp Thr Gly Gly Ala Ser Ser Glu
                165                 170                 175

Asp Ser Leu Met Thr Ser Phe Leu Pro Gly Pro Lys Pro Gly Ala Pro
            180                 185                 190

Phe Pro Asn Gly His Val Gly Ala Gly Gly Ser Gly Leu Leu Pro Pro
        195                 200                 205

Pro Pro Ala Leu Cys Gly Ala Ser Ala Cys Asp Val Ser Val Arg Val
    210                 215                 220

Val Val Gly Glu Pro Thr Glu Ala Arg Val Val Pro Gly Arg Gly Ile
225                 230                 235                 240

Cys Leu Asp Leu Ala Ile Leu Asp Ser Ala Phe Leu Leu Ser Gln Val
                245                 250                 255

Ala Pro Ser Leu Phe Met Gly Ser Ile Val Gln Leu Ser Gln Ser Val
            260                 265                 270

Thr Ala Tyr Met Val Ser Ala Ala Gly Leu Gly Leu Val Ala Ile Tyr
        275                 280                 285

Phe Ala Thr Gln Val Val Phe Asp Lys Ser Asp Leu Ala Lys Tyr Ser
    290                 295                 300

Ala
305


<210> 1030
<211> 15
<212> PRT
<213> Homo sapiens

<400> 1030
His Pro Gln Trp Val Leu Ser Ala Ala His Cys Phe Gln Asn Ser
1               5                   10                  15


<210> 1031
<211> 15
<212> PRT
<213> Homo sapiens

<400> 1031

Glu Gln Gly Ser Gln Met Val Glu Ala Ser Leu Ser Val Arg His
1               5                   10                  15

<210> 1032
<211> 15
<212> PRT
<213> Homo sapiens

<400> 1032
Lys Asp Ser Cys Asn Gly Asp Ser Gly Gly Pro Leu Ile Cys Asn
1               5                   10                  15

<210> 1033
<211> 16
<212> PRT
<213> Homo sapiens

<400> 1033
Gly Gln Val Gly Pro Gly Val Tyr Thr Asn Leu Cys Lys Phe Thr Glu
1               5                   10                  15

2

1

## Description

### BACKGROUND OF THE INVENTION

### Field of the Invention

The present invention relates generally to therapy and diagnosis of cancer, such as prostate cancer. The invention is more specifically related to polypeptides, comprising at least a portion of a prostate-specific protein, and to polynucleotides encoding such polypeptides. Such polypeptides and polynucleotides are useful in pharmaceutical compositions, e.g., vaccines, and other compositions for the diagnosis and treatment of prostate cancer.

### Description of the Related Art

Cancer is a significant health problem throughout the world. Although advances have been made in detection and therapy of cancer, no vaccine or other universally successful method for prevention or treatment is currently available. Current therapies, which are generally based on a combination of chemotherapy or surgery and radiation, continue to prove inadequate in many patients.

Prostate cancer is the most common form of cancer among males, with an estimated incidence of 30% in men over the age of 50. Overwhelming clinical evidence shows that human prostate cancer has the propensity to metastasize to bone, and the disease appears to progress inevitably from androgen dependent to androgen refractory status, leading to increased patient mortality. This prevalent disease is currently the second leading cause of cancer death among men in the U.S.

In spite of considerable research into therapies for the disease, prostate cancer remains difficult to treat. Commonly, treatment is based on surgery and/or radiation therapy, but these methods are ineffective in a significant percentage of cases. Two previously identified prostate specific proteins - prostate specific antigen (PSA) and prostatic acid phosphatase (PAP)
- have limited therapeutic and diagnostic potential. For example, PSA levels do not always correlate well with the presence of prostate cancer, being positive in a percentage of non-prostate cancer cases, including benign prostatic hyperplasia (BPH). Furthermore, PSA measurements correlate with prostate volume, and do not indicate the level of metastasis.
   In spite of considerable research into therapies for these and other cancers, prostate cancer remains difficult to diagnose and treat effectively. Accordingly, there is a need in the art for improved methods for detecting and treating such cancers. The present invention fulfills these needs and further provides other related advantages.

### BRIEF SUMMARY OF THE INVENTION

In one aspect, the present invention provides polynucleotide compositions comprising a sequence selected from the group consisting of:
(a) sequences provided in SEQ ID NO: 1-111, 115-171, 173-175, 177, 179-305, 307-315, 326, 328, 330, 332-335, 340-375, 381, 382 and 384-476, 524, 526, 530, 531, 533, 535, 536, 552, 569-572, 587, 591, 593-606, 618-705, 709-774, 777, 789, 817, 823, 824, 878, 880-882, 894, 896, 907, 908, 916-919, 929-931, 938, 939, 942, 944, 945, 948, 967, 969-976, 991, 993-1002, 1004, 1006, 1007, 1010, 1013-1019, 1021, and 1023-1027;
(b) complements of the sequences provided in SEQ ID NO: 1-111, 115-171, 173-175, 177, 179-305, 307-315, 326, 328, 330, 332-335, 340-375, 381, 382 and 384-476, 524, 526, 530, 531, 533, 535, 536, 552, 569-572, 587, 591, 593-606, 618-705, 709-774, 777, 789, 817, 823, 824, 878, 880-882, 894, 896, 907, 908, 916-919, 929-931, 938, 939, 942, 944, 945, 948, 967, 969-976, 991, 993-1002, 1004, 1006, 1007, 1010, 1013-1019, 1021, and 1023-1027;
(c) sequences consisting of at least 20 contiguous residues of a sequence provided in SEQ ID NO: 1-111, 115-171, 173-175, 177, 179-305, 307-315, 326, 328, 330, 332-335, 340-375, 381, 382 and 384-476, 524, 526, 530, 531, 533, 535, 536, 552, 569-572, 587, 591, 593-606, 618-705, 709-774, 777, 789, 817, 823, 824, 878, 880-882, 894, 896, 907, 908, 916-919, 929-931, 938, 939, 942, 944, 945, 948, 967, 969-976, 991, 993-1002, 1004, 1006, 1007, 1010, 1013-1019, 1021, and 1023-1027;
(d) sequences that hybridize to a sequence provided in SEQ ID NO: 1-111, 115-171, 173-175, 177, 179-305, 307-315, 326, 328, 330, 332-335, 340-375, 381, 382 and 384-476, 524, 526, 530, 531, 533, 535, 536, 552, 569-572, 587, 591, 593-606, 618-705, 709-774, 777, 789, 817, 823, 824, 878, 880-882, 894, 896, 907, 908, 916-919, 929-931, 938, 939, 942, 944, 945, 948, 967, 969-976, 991, 993-1002, 1004, 1006, 1007, 1010, 1013-1019, 1021, and 1023-1027, under moderately stringent conditions;
(e) sequences having at least 75% identity to a sequence of SEQ ID NO: 1-111, 115-171, 173-175, 177, 179-305, 307-315, 326, 328, 330, 332-335, 340-375, 381, 382 and 384-476, 524, 526, 530, 531, 533, 535, 536, 552, 569-572, 587, 591, 593-606, 618-705, 709-774, 777, 789, 817, 823, 824, 878, 880-882, 894, 896, 907, 908, 916-919, 929-931, 938, 939, 942, 944, 945, 948, 967, 969-976, 991, 993-1002, 1004, 1006, 1007, 1010, 1013-1019, 1021, and 1023-1027;
(f) sequences having at least 90% identity to a sequence of SEQ ID NO: 1-111, 115-171, 173-175, 177, 179-305, 307-315, 326, 328, 330, 332-335, 340-375, 381, 382 and 384-476, 524, 526, 530, 531, 533, 535, 536, 552, 569-572, 587, 591, 593-606, 618-705, 709-774, 777, 789, 817, 823, 824, 878, 880-882, 894, 896, 907, 908, 916-919, 929-931, 938, 939, 942, 944, 945, 948, 967, 969-976, 991, 993-1002, 1004, 1006, 1007, 1010, 1013-1019, 1021, and 1023-1027; and
(g) degenerate variants of a sequence provided in SEQ ID NO: 1-111, 115-171, 173-175, 177, 179-305, 307-315, 326, 328, 330, 332-335, 340-375, 381, 382 and 384-476, 524, 526, 530, 531, 533, 535, 536, 552, 569-572, 587, 591, 593-606, 618-705, 709-774, 777, 789, 817, 823, 824, 878, 880-882, 894, 896, 907, 908, 916-919, 929-931, 938, 939, 942, 944, 945, 948, 967, 969-976, 991, 993-1002, 1004, 1006, 1007, 1010, 1013-1019, 1021, and 1023-1027.

In one preferred embodiment, the polynucleotide compositions of the invention are expressed in at least about 20%, more preferably in at least about 30%, and most preferably in at least about 50% of prostate tissue samples tested, at a level that is at least about 2-fold, preferably at least about 5-fold, and most preferably at least about 10-fold higher than that for other normal tissues.

The present invention, in another aspect, provides polypeptide compositions comprising an amino acid sequence that is encoded by a polynucleotide sequence described above.

The present invention further provides polypeptide compositions comprising an amino acid sequence selected from the group consisting of sequences recited in SEQ ID NO: 112-114, 172, 176, 178, 327, 329, 331, 336, 339, 376-380, 383, 477-483, 496, 504, 505, 519, 520, 522, 525, 527, 532, 534, 537-551, 553-568, 573-586, 588-590, 592, 706-708, 775, 776, 778, 780, 781, 811, 814, 818, 826, 827, 853, 855, 858, 860-862, 866-877, 879, 883-893, 895, 897, 898, 909-915, 920-928, 932-934, 940, 941, 943, 946, 947, 949-966, 968, 977-990, 992, 1003, 1005, 1008, 1009, 1011, 1012, 1020, 1022, 1028-1029, and 1030-1033.

In certain preferred embodiments, the polypeptides and/or polynucleotides of the present invention are immunogenic, *i.e.,* they are capable of eliciting an immune response, particularly a humoral and/or cellular immune response, as further described herein.

The present invention further provides fragments, variants and/or derivatives of the disclosed polypeptide and/or polynucleotide sequences, wherein the fragments, variants and/or derivatives preferably have a level of immunogenic activity of at least about 50%, preferably at least about 70% and more preferably at least about 90% of the level of immunogenic activity of a polypeptide sequence set forth in SEQ ID NO: 112-114, 172, 176, 178, 327, 329, 331, 336, 339, 376-380, 383, 477-483, 496, 504, 505, 519, 520, 522, 525, 527, 532, 534, 537-551, 553-568, 573-586, 588-590, 592, 706-708, 775, 776, 778, 780, 781, 811, 814, 818, 826, 827, 853, 855, 858 or 860-862, 866-877, 879, 883-893, 895, 897, 898, 909-915, 920-928, 932-934, 940, 941, 943, 946, 947, 949-966, 968, 977-990, 992, 1003, 1005, 1008, 1009, 1011, 1012, 1020, 1022, 1028-1029, and 1030-1033 or a polypeptide sequence encoded by a polynucleotide sequence set forth in SEQ ID NO: 1-111, 115-171, 173-175, 177, 179-305, 307-315, 326, 328, 330, 332-335, 340-375, 381, 382 and 384-476, 524, 526, 530, 531, 533, 535, 536, 552, 569-572, 587, 591, 593-606, 618-705, 709-774, 777, 789, 817, 823, 824, 878, 880-882, 894, 896, 907, 908, 916-919, 929-931, 938, 939, 942, 944, 945, 948, 967, 969-976, 991, 993-1002, 1004, 1006, 1007, 1010, 1012, 1020, 1022, 1028-1029, and 1030-1033.

The present invention further provides polynucleotides that encode a polypeptide described above, expression vectors comprising such polynucleotides and host cells transformed or transfected with such expression vectors.

Within other aspects, the present invention provides pharmaceutical compositions comprising a polypeptide or polynucleotide as described above and a physiologically acceptable carrier.

Within a related aspect of the present invention, pharmaceutical compositions, e.g., vaccine compositions, are provided for prophylactic or therapeutic applications. Such compositions generally comprise an immunogenic polypeptide or polynucleotide of the invention and an immunostimulant, such as an adjuvant, together with a physiologically acceptable carrier.

The present invention further provides pharmaceutical compositions that comprise: (a) an antibody or antigen-binding fragment thereof that specifically binds to a polypeptide of the present invention, or a fragment thereof; and (b) a physiologically acceptable carrier.

Within further aspects, the present invention provides pharmaceutical compositions comprising: (a) an antigen presenting cell that expresses a polypeptide as described above and (b) a pharmaceutically acceptable carrier or excipient. Illustrative antigen presenting cells include dendritic cells, macrophages, monocytes, fibroblasts and B cells.

Within related aspects, pharmaceutical compositions are provided that comprise: (a) an antigen presenting cell that expresses a polypeptide as described above and (b) an immunostimulant.

The present invention further provides, in other aspects, fusion proteins that comprise at least one polypeptide as described above, as well as polynucleotides encoding such fusion proteins, typically in the form of pharmaceutical compositions, e.g., vaccine compositions, comprising a physiologically acceptable carrier and/or an immunostimulant. The fusions proteins may comprise multiple immunogenic polypeptides or portions/variants thereof, as described herein, and may further comprise one or more polypeptide segments for facilitating and/or enhancing the expression, purification and/or immunogenicity of the polypeptide(s).

Within further aspects, the present invention provides methods for stimulating an immune response in a patient, preferably a T cell response in a human patient, comprising administering a pharmaceutical composition described herein. The patient may be afflicted with prostate cancer, in which case the methods provide treatment for the disease, or a patient considered to be at risk for such a disease may be treated prophylactically.

Within further aspects, the present invention provides methods for inhibiting the development of a cancer in a patient, comprising administering to a patient a pharmaceutical composition as recited above. The patient may be afflicted with prostate cancer, in which case the methods provide treatment for the disease, or a patient considered to be at risk for such a disease may be treated prophylactically.

The present invention further provides, within other aspects, methods for removing tumor cells from a biological sample, comprising contacting a biological sample with T cells that specifically react with a polypeptide of the present invention, wherein the step of contacting is performed under conditions and for a time sufficient to permit the removal of cells expressing the polypeptide from the sample.

Within related aspects, methods are provided for inhibiting the development of a cancer in a patient, comprising administering to a patient a biological sample treated as described above.

Methods are further provided, within other aspects, for stimulating and/or expanding T cells specific for a polypeptide of the present invention, comprising contacting T cells with one or more of: (i) a polypeptide as described above; (ii) a polynucleotide encoding such a polypeptide; and (iii) an antigen presenting cell that expresses such a polypeptide; under conditions and for a time sufficient to permit the stimulation and/or expansion of T cells. Isolated T cell populations comprising T cells prepared as described above are also provided.

Within further aspects, the present invention provides methods for inhibiting the development of a cancer in a patient, comprising administering to a patient an effective amount of a T cell population as described above.

The present invention further provides methods for inhibiting the development of a cancer in a patient, comprising the steps of: (a) incubating CD4⁺ and/or CD8⁺ T cells isolated from a patient with one or more of: (i) a polypeptide comprising at least an immunogenic portion of polypeptide disclosed herein; (ii) a polynucleotide encoding such a polypeptide; and (iii) an antigen-presenting cell that expressed such a polypeptide; and (b) administering to the patient an effective amount of the proliferated T cells, thereby inhibiting the development of a cancer in the patient. Proliferated cells may, but need not, be cloned prior to administration to the patient.

Within further aspects, the present invention provides methods for determining the presence or absence of a cancer, preferably a prostate cancer, in a patient comprising: (a) contacting a biological sample obtained from a patient with a binding agent that binds to a polypeptide as recited above; (b) detecting in the sample an amount of polypeptide that binds to the binding agent; and (c) comparing the amount of polypeptide with a predetermined cut-off value, and therefrom determining the presence or absence of a cancer in the patient. Within preferred embodiments, the binding agent is an antibody, more preferably a monoclonal antibody.

The present invention also provides, within other aspects, methods for monitoring the progression of a cancer in a patient. Such methods comprise the steps of: (a) contacting a biological sample obtained from a patient at a first point in time with a binding agent that binds to a polypeptide as recited above; (b) detecting in the sample an amount of polypeptide that binds to the binding agent; (c) repeating steps (a) and (b) using a biological sample obtained from the patient at a subsequent point in time; and (d) comparing the amount of polypeptide detected in step (c) with the amount detected in step (b), and therefrom monitoring the progression of the cancer in the patient.

The present invention further provides, within other aspects, methods for determining the presence or absence of a cancer in a patient, comprising the steps of: (a) contacting a biological sample obtained from a patient with an oligonucleotide that hybridizes to a polynucleotide of the present invention; (b) detecting in the sample a level of a polynucleotide, preferably mRNA, that hybridizes to the oligonucleotide; and (c) comparing the level of polynucleotide that hybridizes to the oligonucleotide with a predetermined cut-off value, and therefrom determining the presence or absence of a cancer in the patient. Within certain embodiments, the amount of mRNA is detected via polymerase chain reaction using, for example, at least one oligonucleotide primer that hybridizes to a polynucleotide of the present invention, or a complement of such a polynucleotide. Within other embodiments, the amount of mRNA is detected using a hybridization technique, employing an oligonucleotide probe that hybridizes to an inventive polynucleotide, or a complement of such a polynucleotide.

In related aspects, methods are provided for monitoring the progression of a cancer in a patient, comprising the steps of: (a) contacting a biological sample obtained from a patient with an oligonucleotide that hybridizes to a polynucleotide of the present invention; (b) detecting in the sample an amount of a polynucleotide that hybridizes to the oligonucleotide; (c) repeating steps (a) and (b) using a biological sample obtained from the patient at a subsequent point in time; and (d) comparing the amount of polynucleotide detected in step (c) with the amount detected in step (b), and therefrom monitoring the progression of the cancer in the patient.

Within further aspects, the present invention provides antibodies, such as monoclonal antibodies, that bind to a polypeptide as described above, as well as diagnostic kits comprising such antibodies. Diagnostic kits comprising one or more oligonucleotide probes or primers as described above are also provided.

These and other aspects of the present invention will become apparent upon reference to the following detailed description and attached drawings. All references disclosed herein are hereby incorporated by reference in their entirety as if each was incorporated individually.

### BRIEF DESCRIPTION OF THE DRAWINGS AND SEQUENCE IDENTIFIERS

Figure 1 illustrates the ability of T cells to kill fibroblasts expressing the representative prostate-specific polypeptide P502S, as compared to control fibroblasts. The percentage lysis is shown as a series of effector:target ratios, as indicated.

Figures 2A and 2B illustrate the ability of T cells to recognize cells expressing the representative prostate-specific polypeptide P502S. In each case, the number of γ-interferon spots is shown for different numbers of responders. In Figure 2A, data is presented for fibroblasts pulsed with the P2S-12 peptide, as compared to fibroblasts pulsed with a control E75 peptide. In Figure 2B, data is presented for fibroblasts expressing P502S, as compared to fibroblasts expressing HER-2/*neu*.

Figure 3 represents a peptide competition binding assay showing that the P1S#10 peptide, derived from P501S, binds HLA-A2. Peptide P1S#10 inhibits HLA-A2 restricted presentation of fluM58 peptide to CTL clone D150M58 in TNF release bioassay. D150M58 CTL is specific for the HLA-A2 binding influenza matrix peptide fluM58.

Figure 4 illustrates the ability of T cell lines generated from P1S#10 immunized mice to specifically lyse P1S#10-pulsed Jurkat A2Kb targets and P501 S-transduced Jurkat A2Kb targets, as compared to EGFP-transduced Jurkat A2Kb. The percent lysis is shown as a series of effector to target ratios, as indicated.

Figure 5 illustrates the ability of a T cell clone to recognize and specifically lyse Jurkat A2Kb cells expressing the representative prostate-specific polypeptide P501 S, thereby demonstrating that the P 1S#10 peptide may be a naturally processed epitope of the P501 S polypeptide.

Figures 6A and 6B are graphs illustrating the specificity of a CD8⁺ cell line (3A-1) for a representative prostate-specific antigen (P501 S). Figure 6A shows the results of a ⁵¹Cr release assay. The percent specific lysis is shown as a series of effector:target ratios, as indicated. Figure 6B shows the production of interferon-gamma by 3A-1 cells stimulated with autologous B-LCL transduced with P501S, at varying effector:target rations as indicated.

Figure 7 is a Western blot showing the expression of P501 S in baculovirus.

Figure 8 illustrates the results of epitope mapping studies on P501 S.

Figure 9 is a schematic representation of the P501 S protein showing the location of transmembrane domains and predicted intracellular and extracellular domains.

Figure 10 is a genomic map showing the location of the prostate genes P775P, P704P, B305D, P712P and P774P within the Cat Eye Syndrome region of chromosome 22q11.2

Figure 11 shows the results of an ELISA assay to determine the specificity of rabbit polyclonal antisera raised against P501 S.
SEQ ID NO: 1 is the determined cDNA sequence for F1-13
SEQ ID NO: 2 is the determined 3' cDNA sequence for F1-12
SEQ ID NO: 3 is the determined 5' cDNA sequence for F1-12
SEQ ID NO: 4 is the determined 3' cDNA sequence for F1-16
SEQ ID NO: 5 is the determined 3' cDNA sequence for H1-1
SEQ ID NO: 6 is the determined 3' cDNA sequence for H1-9
SEQ ID NO: 7 is the determined 3' cDNA sequence for H1-4
SEQ ID NO: 8 is the determined 3' cDNA sequence for J1-17
SEQ ID NO: 9 is the determined 5' cDNA sequence for J1-17
SEQ ID NO: 10 is the determined 3' cDNA sequence for L1-12
SEQ ID NO: 11 is the determined 5' cDNA sequence for L1-12
SEQ ID NO: 12 is the determined 3' cDNA sequence for N1-1862
SEQ ID NO: 13 is the determined 5' cDNA sequence for N1-1862
SEQ ID NO: 14 is the determined 3' cDNA sequence for J1-13
SEQ ID NO: 15 is the determined 5' cDNA sequence for J1-13
SEQ ID NO: 16 is the determined 3' cDNA sequence for J1-19
SEQ ID NO: 17 is the determined 5' cDNA sequence for J1-19
SEQ ID NO: 18 is the determined 3' cDNA sequence for J1-25
SEQ ID NO: 19 is the determined 5' cDNA sequence for J1-25
SEQ ID NO: 20 is the determined 5' cDNA sequence for J1-24
SEQ ID NO: 21 is the determined 3' cDNA sequence for J1-24
SEQ ID NO: 22 is the determined 5' cDNA sequence for K1-58
SEQ ID NO: 23 is the determined 3' cDNA sequence for K1-58
SEQ ID NO: 24 is the determined 5' cDNA sequence for K1-63
SEQ ID NO: 25 is the determined 3' cDNA sequence for K1-63
SEQ ID NO: 26 is the determined 5' cDNA sequence for L1-4
SEQ ID NO: 27 is the determined 3' cDNA sequence for L1-4
SEQ ID NO: 28 is the determined 5' cDNA sequence for L1-14
SEQ ID NO: 29 is the determined 3' cDNA sequence for L1-14
SEQ ID NO: 30 is the determined 3' cDNA sequence for J1-12
SEQ ID NO: 31 is the determined 3' cDNA sequence for J1-16
SEQ ID NO: 32 is the determined 3' cDNA sequence for J1-21
SEQ ID NO: 33 is the determined 3' cDNA sequence for K1-48
SEQ ID NO: 34 is the determined 3' cDNA sequence for K1-55
SEQ ID NO: 35 is the determined 3' cDNA sequence for L1-2
SEQ ID NO: 36 is the determined 3' cDNA sequence for L1-6
SEQ ID NO: 37 is the determined 3' cDNA sequence for N1-1858
SEQ ID NO: 38 is the determined 3' cDNA sequence for N1-1860
SEQ ID NO: 39 is the determined 3' cDNA sequence for N1-1861
SEQ ID NO: 40 is the determined 3' cDNA sequence for N1-1864
SEQ ID NO: 41 is the determined cDNA sequence for P5
SEQ ID NO: 42 is the determined cDNA sequence for P8
SEQ ID NO: 43 is the determined cDNA sequence for P9
SEQ ID NO: 44 is the determined cDNA sequence for P18
SEQ ID NO: 45 is the determined cDNA sequence for P20
SEQ ID NO: 46 is the determined cDNA sequence for P29
SEQ ID NO: 47 is the determined cDNA sequence for P30
SEQ ID NO: 48 is the determined cDNA sequence for P34
SEQ ID NO: 49 is the determined cDNA sequence for P36
SEQ ID NO: 50 is the determined cDNA sequence for P38
SEQ ID NO: 51 is the determined cDNA sequence for P39
SEQ ID NO: 52 is the determined cDNA sequence for P42
SEQ ID NO: 53 is the determined cDNA sequence for P47
SEQ ID NO: 54 is the determined cDNA sequence for P49
SEQ ID NO: 55 is the determined cDNA sequence for P50
SEQ ID NO: 56 is the determined cDNA sequence for P53
SEQ ID NO: 57 is the determined cDNA sequence for P55
SEQ ID NO: 58 is the determined cDNA sequence for P60
SEQ ID NO: 59 is the determined cDNA sequence for P64
SEQ ID NO: 60 is the determined cDNA sequence for P65
SEQ ID NO: 61 is the determined cDNA sequence for P73
SEQ ID NO: 62 is the determined cDNA sequence for P75
SEQ ID NO: 63 is the determined cDNA sequence for P76
SEQ ID NO: 64 is the determined cDNA sequence for P79
SEQ ID NO: 65 is the determined cDNA sequence for P84
SEQ ID NO: 66 is the determined cDNA sequence for P68
SEQ ID NO: 67 is the determined cDNA sequence for P80 (also referred to as P704P)
SEQ ID NO: 68 is the determined cDNA sequence for P82
SEQ ID NO: 69 is the determined cDNA sequence for U1-3064
SEQ ID NO: 70 is the determined cDNA sequence for U1-3065
SEQ ID NO: 71 is the determined cDNA sequence for V1-3692
SEQ ID NO: 72 is the determined cDNA sequence for 1A-3905
SEQ ID NO: 73 is the determined cDNA sequence for V1-3686
SEQ ID NO: 74 is the determined cDNA sequence for R1-2330
SEQ ID NO: 75 is the determined cDNA sequence for 1 B-3976
SEQ ID NO: 76 is the determined cDNA sequence for V1-3679
SEQ ID NO: 77 is the determined cDNA sequence for 1G-4736
SEQ ID NO: 78 is the determined cDNA sequence for 1G-4738
SEQ ID NO: 79 is the determined cDNA sequence for 1G-4741
SEQ ID NO: 80 is the determined cDNA sequence for 1G-4744
SEQ ID NO: 81 is the determined cDNA sequence for 1 G-4734
SEQ ID NO: 82 is the determined cDNA sequence for 1 H-4774
SEQ ID NO: 83 is the determined cDNA sequence for 1 H-4781
SEQ ID NO: 84 is the determined cDNA sequence for 1 H-4785
SEQ ID NO: 85 is the determined cDNA sequence for 1 H-4787
SEQ ID NO: 86 is the determined cDNA sequence for 1 H-4796
SEQ ID NO: 87 is the determined cDNA sequence for 1I-4807
SEQ ID NO: 88 is the determined cDNA sequence for 1I-4810
SEQ ID NO: 89 is the determined cDNA sequence for 1I-4811
SEQ ID NO: 90 is the determined cDNA sequence for 1J-4876
SEQ ID NO: 91 is the determined cDNA sequence for 1 K-4884
SEQ ID NO: 92 is the determined cDNA sequence for 1 K-4896
SEQ ID NO: 93 is the determined cDNA sequence for 1G-4761
SEQ ID NO: 94 is the determined cDNA sequence for 1G-4762
SEQ ID NO: 95 is the determined cDNA sequence for 1 H-4766
SEQ ID NO: 96 is the determined cDNA sequence for 1 H-4770
SEQ ID NO: 97 is the determined cDNA sequence for 1 H-4771
SEQ ID NO: 98 is the determined cDNA sequence for 1 H-4772
SEQ ID NO: 99 is the determined cDNA sequence for 1D-4297
SEQ ID NO: 100 is the determined cDNA sequence for 1D-4309
SEQ ID NO: 101 is the determined cDNA sequence for 1 D.1-4278
SEQ ID NO: 102 is the determined cDNA sequence for 1D-4288
SEQ ID NO: 103 is the determined cDNA sequence for 1D-4283
SEQ ID NO: 104 is the determined cDNA sequence for 1D-4304
SEQ ID NO: 105 is the determined cDNA sequence for 1D-4296
SEQ ID NO: 106 is the determined cDNA sequence for 1D-4280
SEQ ID NO: 107 is the determined full length cDNA sequence for F1-12 (also referred to as P504S)
SEQ ID NO: 108 is the amino acid sequence for F1-12
SEQ ID NO: 109 is the determined full length cDNA sequence for J1-17
SEQ ID NO: 110 is the determined full length cDNA sequence for L1-12 (also referred to as P501S)
SEQ ID NO: 111 is the determined full length cDNA sequence for N1-1862 (also referred to as P503S)
SEQ ID NO: 112 is the amino acid sequence for J1-17
SEQ ID NO: 113 is the amino acid sequence for L1-12 (also referred to as P501 S)
SEQ ID NO: 114 is the amino acid sequence for N1-1862 (also referred to as P503S)
SEQ ID NO: 115 is the determined cDNA sequence for P89
SEQ ID NO: 116 is the determined cDNA sequence for P90
SEQ ID NO: 117 is the determined cDNA sequence for P92
SEQ ID NO: 118 is the determined cDNA sequence for P95
SEQ ID NO: 119 is the determined cDNA sequence for P98
SEQ ID NO: 120 is the determined cDNA sequence for P102
SEQ ID NO: 121 is the determined cDNA sequence for P110
SEQ ID NO: 122 is the determined cDNA sequence for P111
SEQ ID NO: 123 is the determined cDNA sequence for P114
SEQ ID NO: 124 is the determined cDNA sequence for P115
SEQ ID NO: 125 is the determined cDNA sequence for P116
SEQ ID NO: 126 is the determined cDNA sequence for P124
SEQ ID NO: 127 is the determined cDNA sequence for P126
SEQ ID NO: 128 is the determined cDNA sequence for P130
SEQ ID NO: 129 is the determined cDNA sequence for P133
SEQ ID NO: 130 is the determined cDNA sequence for P138
SEQ ID NO: 131 is the determined cDNA sequence for P143
SEQ ID NO: 132 is the determined cDNA sequence for P151
SEQ ID NO: 133 is the determined cDNA sequence for P156
SEQ ID NO: 134 is the determined cDNA sequence for P157
SEQ ID NO: 135 is the determined cDNA sequence for P166
SEQ ID NO: 136 is the determined cDNA sequence for P176
SEQ ID NO: 137 is the determined cDNA sequence for P178
SEQ ID NO: 138 is the determined cDNA sequence for P179
SEQ ID NO: 139 is the determined cDNA sequence for P185
SEQ ID NO: 140 is the determined cDNA sequence for P192
SEQ ID NO: 141 is the determined cDNA sequence for P201
SEQ ID NO: 142 is the determined cDNA sequence for P204
SEQ ID NO: 143 is the determined cDNA sequence for P208
SEQ ID NO: 144 is the determined cDNA sequence for P211
SEQ ID NO: 145 is the determined cDNA sequence for P213
SEQ ID NO: 146 is the determined cDNA sequence for P219
SEQ ID NO: 147 is the determined cDNA sequence for P237
SEQ ID NO: 148 is the determined cDNA sequence for P239
SEQ ID NO: 149 is the determined cDNA sequence for P248
SEQ ID NO: 150 is the determined cDNA sequence for P251
SEQ ID NO: 151 is the determined cDNA sequence for P255
SEQ ID NO: 152 is the determined cDNA sequence for P256
SEQ ID NO: 153 is the determined cDNA sequence for P259
SEQ ID NO: 154 is the determined cDNA sequence for P260
SEQ ID NO: 155 is the determined cDNA sequence for P263
SEQ ID NO: 156 is the determined cDNA sequence for P264
SEQ ID NO: 157 is the determined cDNA sequence for P266
SEQ ID NO: 158 is the determined cDNA sequence for P270
SEQ ID NO: 159 is the determined cDNA sequence for P272
SEQ ID NO: 160 is the determined cDNA sequence for P278
SEQ ID NO: 161 is the determined cDNA sequence for P105
SEQ ID NO: 162 is the determined cDNA sequence for P107
SEQ ID NO: 163 is the determined cDNA sequence for P137
SEQ ID NO: 164 is the determined cDNA sequence for P194
SEQ ID NO: 165 is the determined cDNA sequence for P195
SEQ ID NO: 166 is the determined cDNA sequence for P196
SEQ ID NO: 167 is the determined cDNA sequence for P220
SEQ ID NO: 168 is the determined cDNA sequence for P234
SEQ ID NO: 169 is the determined cDNA sequence for P235
SEQ ID NO: 170 is the determined cDNA sequence for P243
SEQ ID NO: 171 is the determined cDNA sequence for P703P-DE1
SEQ ID NO: 172 is the amino acid sequence for P703P-DE1
SEQ ID NO: 173 is the determined cDNA sequence for P703P-DE2
SEQ ID NO: 174 is the determined cDNA sequence for P703P-DE6
SEQ ID NO: 175 is the determined cDNA sequence for P703P-DE13
SEQ ID NO: 176 is the amino acid sequence for P703P-DE13
SEQ ID NO: 177 is the determined cDNA sequence for P703P-DE14
SEQ ID NO: 178 is the amino acid sequence for P703P-DE14
SEQ ID NO: 179 is the determined extended cDNA sequence for 1 G-4736
SEQ ID NO: 180 is the determined extended cDNA sequence for 1 G-4738
SEQ ID NO: 181 is the determined extended cDNA sequence for 1 G-4741
SEQ ID NO: 182 is the determined extended cDNA sequence for 1 G-4744
SEQ ID NO: 183 is the determined extended cDNA sequence for 1 H-4774
SEQ ID NO: 184 is the determined extended cDNA sequence for 1 H-4781
SEQ ID NO: 185 is the determined extended cDNA sequence for 1 H-4785
SEQ ID NO: 186 is the determined extended cDNA sequence for 1 H-4787
SEQ ID NO: 187 is the determined extended cDNA sequence for 1 H-4796
SEQ ID NO: 188 is the determined extended cDNA sequence for 1I-4807
SEQ ID NO: 189 is the determined 3' cDNA sequence for 1I-4810
SEQ ID NO: 190 is the determined 3' cDNA sequence for 1I-4811
SEQ ID NO: 191 is the determined extended cDNA sequence for 1 J-4876
SEQ ID NO: 192 is the determined extended cDNA sequence for 1 K-4884
SEQ ID NO: 193 is the determined extended cDNA sequence for 1 K-4896
SEQ ID NO: 194 is the determined extended cDNA sequence for 1 G-4761
SEQ ID NO: 195 is the determined extended cDNA sequence for 1 G-4762
SEQ ID NO: 196 is the determined extended cDNA sequence for 1 H-4766
SEQ ID NO: 197 is the determined 3' cDNA sequence for 1 H-4770
SEQ ID NO: 198 is the determined 3' cDNA sequence for 1 H-4771
SEQ ID NO: 199 is the determined extended cDNA sequence for 1 H-4772
SEQ ID NO: 200 is the determined extended cDNA sequence for 1D-4309

SEQ ID NO: 201 is the determined extended cDNA sequence for 1 D.1-4278
SEQ ID NO: 202 is the determined extended cDNA sequence for 1D-4288
SEQ ID NO: 203 is the determined extended cDNA sequence for 1D-4283
SEQ ID NO: 204 is the determined extended cDNA sequence for 1D-4304
SEQ ID NO: 205 is the determined extended cDNA sequence for 1D-4296
SEQ ID NO: 206 is the determined extended cDNA sequence for 1D-4280
SEQ ID NO: 207 is the determined cDNA sequence for 10-d8fwd
SEQ ID NO: 208 is the determined cDNA sequence for 10-H10con
SEQ ID NO: 209 is the determined cDNA sequence for 11-C8rev
SEQ ID NO: 210 is the determined cDNA sequence for 7.g6fwd
SEQ ID NO: 211 is the determined cDNA sequence for 7.g6rev
SEQ ID NO: 212 is the determined cDNA sequence for 8-b5fwd
SEQ ID NO: 213 is the determined cDNA sequence for 8-b5rev
SEQ ID NO: 214 is the determined cDNA sequence for 8-b6fwd
SEQ ID NO: 215 is the determined cDNA sequence for 8-b6 rev
SEQ ID NO: 216 is the determined cDNA sequence for 8-d4fwd
SEQ ID NO: 217 is the determined cDNA sequence for 8-d9rev
SEQ ID NO: 218 is the determined cDNA sequence for 8-g3fwd
SEQ ID NO: 219 is the determined cDNA sequence for 8-g3rev
SEQ ID NO: 220 is the determined cDNA sequence for 8-h11rev
SEQ ID NO: 221 is the determined cDNA sequence for g-f12fwd
SEQ ID NO: 222 is the determined cDNA sequence for g-f3rev
SEQ ID NO: 223 is the determined cDNA sequence for P509S
SEQ ID NO: 224 is the determined cDNA sequence for P510S
SEQ ID NO: 225 is the determined cDNA sequence for P703DE5
SEQ ID NO: 226 is the determined cDNA sequence for 9-A11
SEQ ID NO: 227 is the determined cDNA sequence for 8-C6
SEQ ID NO: 228 is the determined cDNA sequence for 8-H7
SEQ ID NO: 229 is the determined cDNA sequence for JPTPN13
SEQ ID NO: 230 is the determined cDNA sequence for JPTPN14
SEQ ID NO: 231 is the determined cDNA sequence for JPTPN23
SEQ ID NO: 232 is the determined cDNA sequence for JPTPN24
SEQ ID NO: 233 is the determined cDNA sequence for JPTPN25
SEQ ID NO: 234 is the determined cDNA sequence for JPTPN30
SEQ ID NO: 235 is the determined cDNA sequence for JPTPN34
SEQ ID NO: 236 is the determined cDNA sequence for PTPN35
SEQ ID NO: 237 is the determined cDNA sequence for JPTPN36
SEQ ID NO: 238 is the determined cDNA sequence for JPTPN38
SEQ ID NO: 239 is the determined cDNA sequence for JPTPN39
SEQ ID NO: 240 is the determined cDNA sequence for JPTPN40
SEQ ID NO: 241 is the determined cDNA sequence for JPTPN41
SEQ ID NO: 242 is the determined cDNA sequence for JPTPN42
SEQ ID NO: 243 is the determined cDNA sequence for JPTPN45
SEQ ID NO: 244 is the determined cDNA sequence for JPTPN46
SEQ ID NO: 245 is the determined cDNA sequence for JPTPN51
SEQ ID NO: 246 is the determined cDNA sequence for JPTPN56
SEQ ID NO: 247 is the determined cDNA sequence for PTPN64
SEQ ID NO: 248 is the determined cDNA sequence for JPTPN65
SEQ ID NO: 249 is the determined cDNA sequence for JPTPN67
SEQ ID NO: 250 is the determined cDNA sequence for JPTPN76
SEQ ID NO: 251 is the determined cDNA sequence for JPTPN84
SEQ ID NO: 252 is the determined cDNA sequence for JPTPN85
SEQ ID NO: 253 is the determined cDNA sequence for JPTPN86
SEQ ID NO: 254 is the determined cDNA sequence for JPTPN87
SEQ ID NO: 255 is the determined cDNA sequence for JPTPN88
SEQ ID NO: 256 is the determined cDNA sequence for JP1F1
SEQ ID NO: 257 is the determined cDNA sequence for JP1 F2
SEQ ID NO: 258 is the determined cDNA sequence for JP1C2
SEQ ID NO: 259 is the determined cDNA sequence for JP1B1
SEQ ID NO: 260 is the determined cDNA sequence for JP1 B2
SEQ ID NO: 261 is the determined cDNA sequence for JP1 D3
SEQ ID NO: 262 is the determined cDNA sequence for JP1A4
SEQ ID NO: 263 is the determined cDNA sequence for JP1 F5
SEQ ID NO: 264 is the determined cDNA sequence for JP1 E6
SEQ ID NO: 265 is the determined cDNA sequence for JP1 D6
SEQ ID NO: 266 is the determined cDNA sequence for JP1 B5
SEQ ID NO: 267 is the determined cDNA sequence for JP1A6
SEQ ID NO: 268 is the determined cDNA sequence for JP1 E8
SEQ ID NO: 269 is the determined cDNA sequence for JP1 D7
SEQ ID NO: 270 is the determined cDNA sequence for JP1 D9
SEQ ID NO: 271 is the determined cDNA sequence for JP1C10
SEQ ID NO: 272 is the determined cDNA sequence for JP1A9
SEQ ID NO: 273 is the determined cDNA sequence for JP1 F12
SEQ ID NO: 274 is the determined cDNA sequence for JP1 E12
SEQ ID NO: 275 is the determined cDNA sequence for JP1 D11
SEQ ID NO: 276 is the determined cDNA sequence for JP1C11
SEQ ID NO: 277 is the determined cDNA sequence for JP1C12
SEQ ID NO: 278 is the determined cDNA sequence for JP1B12
SEQ ID NO: 279 is the determined cDNA sequence for JP1A12
SEQ ID NO: 280 is the determined cDNA sequence for JP8G2
SEQ ID NO: 281 is the determined cDNA sequence for JP8H1
SEQ ID NO: 282 is the determined cDNA sequence for JP8H2
SEQ ID NO: 283 is the determined cDNA sequence for JP8A3
SEQ ID NO: 284 is the determined cDNA sequence for JP8A4
SEQ ID NO: 285 is the determined cDNA sequence for JP8C3
SEQ ID NO: 286 is the determined cDNA sequence for JP8G4
SEQ ID NO: 287 is the determined cDNA sequence for JP8B6
SEQ ID NO: 288 is the determined cDNA sequence for JP8D6
SEQ ID NO: 289 is the determined cDNA sequence for JP8F5
SEQ ID NO: 290 is the determined cDNA sequence for JP8A8
SEQ ID NO: 291 is the determined cDNA sequence for JP8C7
SEQ ID NO: 292 is the determined cDNA sequence for JP8D7
SEQ ID NO: 293 is the determined cDNA sequence for P8D8
SEQ ID NO: 294 is the determined cDNA sequence for JP8E7
SEQ ID NO: 295 is the determined cDNA sequence for JP8F8
SEQ ID NO: 296 is the determined cDNA sequence for JP8G8
SEQ ID NO: 297 is the determined cDNA sequence for JP8B10
SEQ ID NO: 298 is the determined cDNA sequence for JP8C10
SEQ ID NO: 299 is the determined cDNA sequence for JP8E9
SEQ ID NO: 300 is the determined cDNA sequence for JP8E10
SEQ ID NO: 301 is the determined cDNA sequence for JP8F9
SEQ ID NO: 302 is the determined cDNA sequence for JP8H9
SEQ ID NO: 303 is the determined cDNA sequence for JP8C12
SEQ ID NO: 304 is the determined cDNA sequence for JP8E11
SEQ ID NO: 305 is the determined cDNA sequence for JP8E12
SEQ ID NO: 306 is the amino acid sequence for the peptide PS2#12
SEQ ID NO: 307 is the determined cDNA sequence for P711 P
SEQ ID NO: 308 is the determined cDNA sequence for P712P
SEQ ID NO: 309 is the determined cDNA sequence for CLONE23
SEQ ID NO: 310 is the determined cDNA sequence for P774P
SEQ ID NO: 311 is the determined cDNA sequence for P775P
SEQ ID NO: 312 is the determined cDNA sequence for P715P
SEQ ID NO: 313 is the determined cDNA sequence for P710P
SEQ ID NO: 314 is the determined cDNA sequence for P767P
SEQ ID NO: 315 is the determined cDNA sequence for P768P
SEQ ID NO: 316-325 are the determined cDNA sequences of previously isolated genes
SEQ ID NO: 326 is the determined cDNA sequence for P703PDE5
SEQ ID NO: 327 is the amino acid sequence for P703PDE5
SEQ ID NO: 328 is the determined cDNA sequence for P703P6.26
SEQ ID NO: 329 is the amino acid sequence for P703P6.26
SEQ ID NO: 330 is the determined cDNA sequence for P703PX-23
SEQ ID NO: 331 is the amino acid sequence for P703PX-23
SEQ ID NO: 332 is the determined full length cDNA sequence for P509S
SEQ ID NO: 333 is the determined extended cDNA sequence for P707P (also referred to as 11-C9)
SEQ ID NO: 334 is the determined cDNA sequence for P714P
SEQ ID NO: 335 is the determined cDNA sequence for P705P (also referred to as 9-F3)
SEQ ID NO: 336 is the amino acid sequence for P705P
SEQ ID NO: 337 is the amino acid sequence of the peptide P1S#10
SEQ ID NO: 338 is the amino acid sequence of the peptide p5
SEQ ID NO: 339 is the amino acid sequence of P509S
SEQ ID NO: 340 is the determined cDNA sequence for P778P
SEQ ID NO: 341 is the determined cDNA sequence for P786P
SEQ ID NO: 342 is the determined cDNA sequence for P789P
SEQ ID NO: 343 is the determined cDNA sequence for a clone showing homology to Homo sapiens MM46 mRNA
SEQ ID NO: 344 is the determined cDNA sequence for a clone showing homology to Homo sapiens TNF-alpha stimulated ABC protein (ABC50) mRNA
SEQ ID NO: 345 is the determined cDNA sequence for a clone showing homology to Homo sapiens mRNA for E-cadherin
SEQ ID NO: 346 is the determined cDNA sequence for a clone showing homology to Human nuclear-encoded mitochondrial serine hydroxymethyltransferase (SHMT)
SEQ ID NO: 347 is the determined cDNA sequence for a clone showing homology to Homo sapiens natural resistance-associated macrophage protein2 (NRAMP2)
SEQ ID NO: 348 is the determined cDNA sequence for a clone showing homology to Homo sapiens phosphoglucomutase-related protein (PGMRP)
SEQ ID NO: 349 is the determined cDNA sequence for a clone showing homology to Human mRNA for proteosome subunit p40
SEQ ID NO: 350 is the determined cDNA sequence for P777P
SEQ ID NO: 351 is the determined cDNA sequence for P779P
SEQ ID NO: 352 is the determined cDNA sequence for P790P
SEQ ID NO: 353 is the determined cDNA sequence for P784P
SEQ ID NO: 354 is the determined cDNA sequence for P776P
SEQ ID NO: 355 is the determined cDNA sequence for P780P
SEQ ID NO: 356 is the determined cDNA sequence for P544S
SEQ ID NO: 357 is the determined cDNA sequence for P745S
SEQ ID NO: 358 is the determined cDNA sequence for P782P
SEQ ID NO: 359 is the determined cDNA sequence for P783P
SEQ ID NO: 360 is the determined cDNA sequence for unknown 17984
SEQ ID NO: 361 is the determined cDNA sequence for P787P
SEQ ID NO: 362 is the determined cDNA sequence for P788P
SEQ ID NO: 363 is the determined cDNA sequence for unknown 17994
SEQ ID NO: 364 is the determined cDNA sequence for P781 P
SEQ ID NO: 365 is the determined cDNA sequence for P785P
SEQ ID NO: 366-375 are the determined cDNA sequences for splice variants of B305D.
SEQ ID NO: 376 is the amino acid sequence encoded by the sequence of SEQ ID NO: 366.
SEQ ID NO: 377 is the amino acid sequence encoded by the sequence of SEQ ID NO: 372.
SEQ ID NO: 378 is the amino acid sequence encoded by the sequence of SEQ ID NO: 373.
SEQ ID NO: 379 is the amino acid sequence encoded by the sequence of SEQ ID NO: 374.
SEQ ID NO: 380 is the amino acid sequence encoded by the sequence of SEQ ID NO: 375.
SEQ ID NO: 381 is the determined cDNA sequence for B716P.
SEQ ID NO: 382 is the determined full-length cDNA sequence for P711P.
SEQ ID NO: 383 is the amino acid sequence for P711 P.
SEQ ID NO: 384 is the cDNA sequence for P1000C.
SEQ ID NO: 385 is the cDNA sequence for CGI-82.
SEQ ID NO:386 is the cDNA sequence for 23320.
SEQ ID NO:387 is the cDNA sequence for CGI-69.
SEQ ID NO:388 is the cDNA sequence for L-iditol-2-dehydrogenase.
SEQ ID NO:389 is the cDNA sequence for 23379.
SEQ ID NO:390 is the cDNA sequence for 23381.
SEQ ID NO:391 is the cDNA sequence for KIAA0122.
SEQ ID NO:392 is the cDNA sequence for clone 23399 (also known as P554S).
SEQ ID NO:393 is the cDNA sequence for a previously identified gene.
SEQ ID NO:394 is the cDNA sequence for HCLBP.
SEQ ID NO:395 is the cDNA sequence for transglutaminase.
SEQ ID NO:396 is the cDNA sequence for a previously identified gene.
SEQ ID NO:397 is the cDNA sequence for PAP.
SEQ ID NO:398 is the cDNA sequence for Ets transcription factor PDEF.
SEQ ID NO:399 is the cDNA sequence for hTGR.
SEQ ID NO:400 is the cDNA sequence for KIAA0295.
SEQ ID NO:401 is the cDNA sequence for 22545.
SEQ ID NO:402 is the cDNA sequence for 22547.
SEQ ID NO:403 is the cDNA sequence for 22548.
SEQ ID NO:404 is the cDNA sequence for 22550.
SEQ ID NO:405 is the cDNA sequence for 22551.
SEQ ID NO:406 is the cDNA sequence for 22552.
SEQ ID NO:407 is the cDNA sequence for 22553 (also known as P1020C).
SEQ ID NO:408 is the cDNA sequence for 22558.
SEQ ID NO:409 is the cDNA sequence for 22562.
SEQ ID NO:410 is the cDNA sequence for 22565.
SEQ ID NO:411 is the cDNA sequence for 22567.
SEQ ID NO:412 is the cDNA sequence for 22568.
SEQ ID NO:413 is the cDNA sequence for 22570.
SEQ ID NO:414 is the cDNA sequence for 22571.
SEQ ID NO:415 is the cDNA sequence for 22572.
SEQ ID NO:416 is the cDNA sequence for 22573.
SEQ ID NO:417 is the cDNA sequence for 22573.
SEQ ID NO:418 is the cDNA sequence for 22575.
SEQ ID NO:419 is the cDNA sequence for 22580.
SEQ ID NO:420 is the cDNA sequence for 22581.
SEQ ID NO:421 is the cDNA sequence for 22582.
SEQ ID NO:422 is the cDNA sequence for 22583.
SEQ ID NO:423 is the cDNA sequence for 22584.
SEQ ID NO:424 is the cDNA sequence for 22585.
SEQ ID NO:425 is the cDNA sequence for 22586.
SEQ ID NO:426 is the cDNA sequence for 22587.
SEQ ID NO:427 is the cDNA sequence for 22588.
SEQ ID NO:428 is the cDNA sequence for 22589.
SEQ ID NO:429 is the cDNA sequence for 22590.
SEQ ID NO:430 is the cDNA sequence for 22591.
SEQ ID NO:431 is the cDNA sequence for 22592.
SEQ ID NO:432 is the cDNA sequence for 22593.
SEQ ID NO:433 is the cDNA sequence for 22594.
SEQ ID NO:434 is the cDNA sequence for 22595.
SEQ ID NO:435 is the cDNA sequence for 22596.
SEQ ID NO:436 is the cDNA sequence for 22847.
SEQ ID NO:437 is the cDNA sequence for 22848.
SEQ ID NO:438 is the cDNA sequence for 22849.
SEQ ID NO:439 is the cDNA sequence for 22851.
SEQ ID NO:440 is the cDNA sequence for 22852.
SEQ ID NO:441 is the cDNA sequence for 22853.
SEQ ID NO:442 is the cDNA sequence for 22854.
SEQ ID NO:443 is the cDNA sequence for 22855.
SEQ ID NO:444 is the cDNA sequence for 22856.
SEQ ID NO:445 is the cDNA sequence for 22857.
SEQ ID NO:446 is the cDNA sequence for 23601.
SEQ ID NO:447 is the cDNA sequence for 23602.
SEQ ID NO:448 is the cDNA sequence for 23605.
SEQ ID NO:449 is the cDNA sequence for 23606.
SEQ ID NO:450 is the cDNA sequence for 23612.
SEQ ID NO:451 is the cDNA sequence for 23614.
SEQ ID NO:452 is the cDNA sequence for 23618.
SEQ ID NO:453 is the cDNA sequence for 23622.
SEQ ID NO:454 is the cDNA sequence for folate hydrolase.
SEQ ID NO:455 is the cDNA sequence for LIM protein.
SEQ ID NO:456 is the cDNA sequence for a known gene.
SEQ ID NO:457 is the cDNA sequence for a known gene.
SEQ ID NO:458 is the cDNA sequence for a previously identified gene.
SEQ ID NO:459 is the cDNA sequence for 23045.
SEQ ID NO:460 is the cDNA sequence for 23032.
SEQ ID NO:461 is the cDNA sequence for clone 23054.
SEQ ID NO:462-467 are cDNA sequences for known genes.
SEQ ID NO:468-471 are cDNA sequences for P710P.
SEQ ID NO:472 is a cDNA sequence for P1001C.
SEQ ID NO: 473 is the determined cDNA sequence for a first splice variant of P775P (referred to as 27505).
SEQ ID NO: 474 is the determined cDNA sequence for a second splice variant of P775P (referred to as 19947).
SEQ ID NO: 475 is the determined cDNA sequence for a third splice variant of P775P (referred to as 19941).
SEQ ID NO: 476 is the determined cDNA sequence for a fourth splice variant of P775P (referred to as 19937).
SEQ ID NO: 477 is a first amino acid sequence encoded by the sequence of SEQ ID NO: 474.
SEQ ID NO: 478 is a second amino acid sequence encoded by the sequence of SEQ ID NO: 474.
SEQ ID NO: 479 is the amino acid sequence encoded by the sequence of SEQ ID NO: 475.
SEQ ID NO: 480 is a first amino acid sequence encoded by the sequence of SEQ ID NO: 473.
SEQ ID NO: 481 is a second amino acid sequence encoded by the sequence of SEQ ID NO: 473.
SEQ ID NO: 482 is a third amino acid sequence encoded by the sequence of SEQ ID NO: 473.
SEQ ID NO: 483 is a fourth amino acid sequence encoded by the sequence of SEQ ID NO: 473.
SEQ ID NO: 484 is the first 30 amino acids of the M. *tuberculosis* antigen Ra12.
SEQ ID NO: 485 is the PCR primer AW025.
SEQ ID NO: 486 is the PCR primer AW003.
SEQ ID NO: 487 is the PCR primer AW027.
SEQ ID NO: 488 is the PCR primer AW026.
SEQ ID NO: 489-501 are peptides employed in epitope mapping studies.
SEQ ID NO: 502 is the determined cDNA sequence of the complementarity determining region for the anti-P503S monoclonal antibody 20D4.
SEQ ID NO: 503 is the determined cDNA sequence of the complementarity determining region for the anti-P503S monoclonal antibody JA1.
SEQ ID NO: 504 & 505 are peptides employed in epitope mapping studies.
SEQ ID NO: 506 is the determined cDNA sequence of the complementarity determining region for the anti-P703P monoclonal antibody 8H2.
SEQ ID NO: 507 is the determined cDNA sequence of the complementarity determining region for the anti-P703P monoclonal antibody 7H8.
SEQ ID NO: 508 is the determined cDNA sequence of the complementarity determining region for the anti-P703P monoclonal antibody 2D4.
SEQ ID NO: 509-522 are peptides employed in epitope mapping studies.
SEQ ID NO: 523 is a mature form of P703P used to raise antibodies against P703P.
SEQ ID NO: 524 is the putative full-length cDNA sequence of P703P.
SEQ ID NO: 525 is the amino acid sequence encoded by SEQ ID NO: 524.
SEQ ID NO: 526 is the full-length cDNA sequence for P790P.
SEQ ID NO: 527 is the amino acid sequence for P790P.
SEQ ID NO: 528 & 529 are PCR primers.
SEQ ID NO: 530 is the cDNA sequence of a splice variant of SEQ ID NO: 366.
SEQ ID NO: 531 is the cDNA sequence of the open reading frame of SEQ ID NO: 530.
SEQ ID NO: 532 is the amino acid encoded by the sequence of SEQ ID NO: 531.
SEQ ID NO: 533 is the DNA sequence of a putative ORF of P775P.
SEQ ID NO: 534 is the amino acid sequence encoded by SEQ ID NO: 533.
SEQ ID NO: 535 is a first full-length cDNA sequence for P510S.
SEQ ID NO: 536 is a second full-length cDNA sequence for P510S.
SEQ ID NO: 537 is the amino acid sequence encoded by SEQ ID NO: 535.
SEQ ID NO: 538 is the amino acid sequence encoded by SEQ ID NO: 536.
SEQ ID NO: 539 is the peptide P501 S-370.
SEQ ID NO: 540 is the peptide P501 S-376.
SEQ ID NO: 541-551 are epitopes of P501S.
SEQ ID NO: 552 is an extended cDNA sequence for P712P.
SEQ ID NO: 553-568 are the amino acid sequences encoded by open reading frames within SEQ ID NO: 552.
SEQ ID NO: 569 is an extended cDNA sequence for P776P.
SEQ ID NO: 570 is the determined cDNA sequence for a splice variant of P776P referred to as contig 6.
SEQ ID NO: 571 is the determined cDNA sequence for a splice variant of P776P referred to as contig 7.
SEQ ID NO: 572 is the determined cDNA sequence for a splice variant of P776P referred to as contig 14.
SEQ ID NO: 573 is the amino acid sequence encoded by a first ORF of SEQ ID NO: 570.
SEQ ID NO: 574 is the amino acid sequence encoded by a second ORF of SEQ ID NO: 570.
SEQ ID NO: 575 is the amino acid sequence encoded by a ORF of SEQ ID NO: 571.
SEQ ID NO: 576-586 are amino acid sequences encoded by ORFs of SEQ ID NO: 569.
SEQ ID NO: 587 is a DNA consensus sequence of the sequences of P767P and P777P.
SEQ ID NO: 588-590 are amino acid sequences encoded by ORFs of SEQ ID NO: 587.
SEQ ID NO: 591 is an extended cDNA sequence for P1020C.
SEQ ID NO: 592 is the amino acid sequence encoded by the sequence of SEQ ID NO: P1020C.
SEQ ID NO: 593 is a splice variant of P775P referred to as 50748.
SEQ ID NO: 594 is a splice variant of P775P referred to as 50717.SEQ ID NO: 595 is a splice variant of P775P referred to as 45985.
SEQ ID NO: 596 is a splice variant of P775P referred to as 38769.
SEQ ID NO: 597 is a splice variant of P775P referred to as 37922.
SEQ ID NO: 598 is a splice variant of P510S referred to as 49274.
SEQ ID NO: 599 is a splice variant of P510S referred to as 39487.

SEQ ID NO: 600 is a splice variant of P504S referred to as 5167.16.
SEQ ID NO: 601 is a splice variant of P504S referred to as 5167.1.
SEQ ID NO: 602 is a splice variant of P504S referred to as 5163.46.
SEQ ID NO: 603 is a splice variant of P504S referred to as 5163.42.
SEQ ID NO: 604 is a splice variant of P504S referred to as 5163.34.
SEQ ID NO: 605 is a splice variant of P504S referred to as 5163.17.
SEQ ID NO: 606 is a splice variant of P501 S referred to as 10640.
SEQ ID NO: 607-615 are the sequences of PCR primers.
SEQ ID NO: 616 is the determined cDNA sequence of a fusion of P703P and PSA.
SEQ ID NO: 617 is the amino acid sequence of the fusion of P703P and PSA.
SEQ ID NO: 618-689 are determined cDNA sequences of prostate-specific clones.
SEQ ID NO: 690 is the cDNA sequence of the gene DD3.
SEQ ID NO: 691-697 are determined cDNA sequences of prostate-specific clones.
SEQ ID NO: 698 is an extended cDNA sequence for P714P.
SEQ ID NO: 699-701 are the cDNA sequences for splice variants of P704P.
SEQ ID NO: 702 is the cDNA sequence of a spliced variant of P553S referred to as P553S-14.
SEQ ID NO: 703 is the cDNA sequence of a spliced variant of P553S referred to as P553S-12.
SEQ ID NO: 704 is the cDNA sequence of a spliced variant of P553S referred to as P553S-10.
SEQ ID NO: 705 is the cDNA sequence of a spliced variant of P553S referred to as P553S-6.
SEQ ID NO: 706 is the amino acid sequence encoded by SEQ ID NO: 705.
SEQ ID NO: 707 is the amino acid sequence encoded by SEQ ID NO: 702 SEQ ID NO: 708 is a second amino acid sequence encoded by SEQ ID NO: 702.
SEQ ID NO: 709-772 are determined cDNA sequences of prostate-specific clones.
SEQ ID NO: 773 is a first full-length cDNA sequence for prostate-specific transglutaminase gene (also referred to herein as P558S).
SEQ ID NO: 774 is a second full-length cDNA sequence for prostate-specific transglutaminase gene.
SEQ ID NO: 775 is the amino acid sequence encoded by the sequence of SEQ ID NO: 773.
SEQ ID NO: 776 is the amino acid sequence encoded by the sequence of SEQ ID NO: 774.
SEQ ID NO: 777 is the full-length cDNA sequence for P788P.
SEQ ID NO: 778 is the amino acid sequence encoded by SEQ ID NO: 777.
SEQ ID NO: 779 is the determined cDNA sequence for a polymorphic variant of P788P.
SEQ ID NO: 780 is the amino acid sequence encoded by SEQ ID NO: 779.
SEQ ID NO: 781 is the amino acid sequence of peptide 4 from P703P.
SEQ ID NO: 782 is the cDNA sequence that encodes peptide 4 from P703P.
SEQ ID NO: 783-798 are the cDNA sequence encoding epitopes of P703P.
SEQ ID NO: 799-814 are the amino acid sequences of epitopes of P703P.
SEQ ID NO: 815 and 816 are PCR primers.
SEQ ID NO: 817 is the cDNA sequence encoding an N-terminal portion of P788P expressed in *E. coli.*
SEQ ID NO: 818 is the amino acid sequence of the N-terminal portion of P788P expressed in *E. coli.*
SEQ ID NO: 819 is the amino acid sequence of the M. *tuberculosis* antigen Ra12.
SEQ ID NO: 820 and 821 are PCR primers.
SEQ ID NO: 822 is the cDNA sequence for the Ra12-P510S-C construct.
SEQ ID NO: 823 is the cDNA sequence for the P510S-C construct.
SEQ ID NO: 824 is the cDNA sequence for the P510S-E3 construct.
SEQ ID NO: 825 is the amino acid sequence for the Ra12-P510S-C construct.
SEQ ID NO: 826 is the amino acid sequence for the P510S-C construct.
SEQ ID NO: 827 is the amino acid sequence for the P510S-E3 construct.
SEQ ID NO: 828-833 are PCR primers.
SEQ ID NO: 834 is the cDNA sequence of the construct Ra12-P775P-ORF3.
SEQ ID NO: 835 is the amino acid sequence of the construct Ra12-P775P-ORF3.
SEQ ID NO: 836 and 837 are PCR primers.
SEQ ID NO: 838 is the determined amino acid sequence for a P703P His tag fusion protein.
SEQ ID NO: 839 is the determined cDNA sequence for a P703P His tag fusion protein.
SEQ ID NO: 840 and 841 are PCR primers.
SEQ ID NO: 842 is the determined amino acid sequence for a
P705P His tag fusion protein.
SEQ ID NO: 843 is the determined cDNA sequence for a P705P His tag fusion protein.
SEQ ID NO: 844 and 845 are PCR primers.
SEQ ID NO: 846 is the determined amino acid sequence for a P711 P His tag fusion protein.
SEQ ID NO: 847 is the determined cDNA sequence for a P711 P His tag fusion protein.
SEQ ID NO: 848 is the amino acid sequence of the M. *tuberculosis* antigen Ra12.
SEQ ID NO: 849 and 850 are PCR primers.
SEQ ID NO: 851 is the determined cDNA sequence for the construct Ra12-P501S-E2.
SEQ ID NO: 852 is the determined amino acid sequence for the construct Ra12-P501S-E2.
SEQ ID NO: 853 is the amino acid sequence for an epitope of P501 S.
SEQ ID NO: 854 is the DNA sequence encoding SEQ ID NO: 853.
SEQ ID NO: 855 is the amino acid sequence for an epitope of P501 S.
SEQ ID NO: 856 is the DNA sequence encoding SEQ ID NO: 855.
SEQ ID NO: 857 is a peptide employed in epitope mapping studies.
SEQ ID NO: 858 is the amino acid sequence for an epitope of P501 S.
SEQ ID NO: 859 is the DNA sequence encoding SEQ ID NO: 858.
SEQ ID NO: 860-862 are the amino acid sequences for CD4 epitopes of P501S.
SEQ ID NO: 863-865 are the DNA sequences encoding the sequences of SEQ ID NO: 860-862.
SEQ ID NO: 866-877 are the amino acid sequences for putative CTL epitopes of P703P.
SEQ ID NO: 878 is the full-length cDNA sequence for P789P.
SEQ ID NO: 879 is the amino acid sequence encoded by SEQ ID NO: 878.
SEQ ID NO: 880 is the determined full-length cDNA sequence for the splice variant of P776P referred to as contig 6.
SEQ ID NO: 881-882 are determined full-length cDNA sequences for the splice variant of P776P referred to as contig 7.
SEQ ID NO: 883-887 are amino acid sequences encoded by SEQ ID NO: 880.
SEQ ID NO: 888-893 are amino acid sequences encoded by the splice variant of P776P referred to as contig 7.
SEQ ID NO: 894 is the full-length cDNA sequence for human transmembrane protease serine 2.
SEQ ID NO: 895 is the amino acid sequence encoded by SEQ ID NO: 894.
SEQ ID NO: 896 is the cDNA sequence encoding the first 209 amino acids of human transmembrane protease serine 2.
SEQ ID NO: 897 is the first 209 amino acids of human transmembrane protease serine 2.
SEQ ID NO: 898 is the amino acid sequence of peptide 296-322 of P501 S.
SEQ ID NO: 899-902 are PCR primers.
SEQ ID NO: 903 is the determined cDNA sequence of the Vb chain of a T cell receptor for the P501 S-specific T cell clone 4E5.
SEQ ID NO: 904 is the determined cDNA sequence of the Va chain of a T cell receptor for the P501 S-specific T cell clone 4E5.
SEQ ID NO: 905 is the amino acid sequence encoded by SEQ ID NO 903.
SEQ ID NO: 906 is the amino acid sequence encoded by SEQ ID NO 904.
SEQ ID NO: 907 is the full-length open reading frame for P768P including stop codon.
SEQ ID NO: 908 is the full-length open reading frame for P768P without stop codon.
SEQ ID NO: 909 is the amino acid sequence encoded by SEQ ID NO: 908.
SEQ ID NO: 910-915 are the amino acid sequences for predicted domains of P768P.
SEQ ID NO: 916 is the full-length cDNA sequence of P835P.
SEQ ID NO: 917 is the cDNA sequence of the previously identified clone FLJ13581.
SEQ ID NO: 918 is the cDNA sequence of the open reading frame for P835P with stop codon.
SEQ ID NO: 919 is the cDNA sequence of the open reading frame for P835P without stop codon.
SEQ ID NO: 920 is the full-length amino acid sequence for P835P.
SEQ ID NO: 921-928 are the amino acid sequences of extracellular and intracellular domains of P835P.
SEQ ID NO: 929 is the full-length cDNA sequence for P1000C.
SEQ ID NO: 930 is the cDNA sequence of the open reading frame for P1000C, including stop codon.
SEQ ID NO: 931 is the cDNA sequence of the open reading frame for P1000C, without stop codon.
SEQ ID NO: 932 is the full-length amino acid sequence for P1000C.
SEQ ID NO: 933 is amino acids 1-100 of SEQ ID NO: 932.
SEQ ID NO: 934 is amino acids 100-492 of SEQ ID NO: 932.
SEQ ID NO: 935-937 are PCR primers.
SEQ ID NO: 938 is the cDNA sequence of the expressed full-length P767P coding region.
SEQ ID NO: 939 is the cDNA sequence of an expressed truncated P767P coding region.
SEQ ID NO: 940 is the amino acid sequence encoded by SEQ ID NO: 939.
SEQ ID NO: 941 is the amino acid sequence encoded by SEQ ID NO: 938.
SEQ ID NO: 942 is the DNA sequence of a CD4 epitope of P703P.
SEQ ID NO: 943 is the amino acid sequence of a CD4 epitope of P703P.
SEQ ID NO: 944 and 945 are the cDNA sequences of two alternative splice forms of P780P.
SEQ ID NO: 946 and 947 are the amino acid sequences encoded by SEQ ID NO: 944 and 945, respectively.
SEQ ID NO: 948 is a corrected cDNA sequence for P705P.
SEQ ID NO: 949-956 are the amino acid sequences of P790P peptides.
SEQ ID NO: 957-966 are the amino acid sequences of P775P peptides.
SEQ ID NO: 967 is an extended cDNA sequence for P554S.
SEQ ID NO: 968 is the amino acid sequence for P554S.
SEQ ID NO: 969-976 are the cDNA sequences encoding epitopes of P501 S.
SEQ ID NO: 977-984 are the amino acid sequences of epitopes of P501 S.
SEQ ID NO: 985-987 are amino acid sequences of peptides of P501 S.
SEQ ID NO: 988-990 are amino acid sequences of peptides of P703P.
SEQ ID NO:991 is the cDNA encoding a CD4+ T cell epitope of P703P.
SEQ ID NO:992 is the amino acid sequence of a CD4+ T cell epitope of P703P (encoded by SEQ ID NO:991).
SEQ ID NO: 993 represents the cDNA sequence for P706P clone 8-B6 that includes P3.
SEQ ID NO: 994 represents the cDNA sequence for P706P clone 8-B6 that includes P4.
SEQ ID NO: 995 represents the cDNA sequence for P706Pclone 8B6.P4 contig.
SEQ ID NO: 996 represents the cDNA sequence for P706P ESTs 1386-1902.
SEQ ID NO: 997 represents the cDNA sequence for P706P ESTs 4927-6088.
SEQ ID NO: 998 represents the cDNA sequence for P713P clone PT4410A4 2.

SEQ ID NO: 999-1002 represent the four exons encoding the prostatic secretory protein (PSP).
SEQ ID NO: 1003 represents the full length amino acid sequence for PSP.
SEQ ID NO: 1004 represents an extended cDNA sequence for clone P1 E.
SEQ ID NO: 1005 represents the amino acid sequence of an open reading frame encoded by SEQ ID NO: 1004.
SEQ ID NO: 1006 represents the DNA sequence of the coding region of the expression construct P510.seq.
SEQ ID NO: 1007 represents the DNA sequence of coding region of the expression construct MAPS-P510S.seq.
SEQ ID NO: 1008 represents the protein sequence encoded by the DNA sequence of SEQ ID NO: 1007.
SEQ ID NO: 1009 represents the protein sequence encoded by the DNA sequence of SEQ ID NO: 1006.
SEQ ID NO: 1010 represents the DNA sequence of the coding region for a Trx-P501 S fusion construct.
SEQ ID NO: 1011 represents the amino acid sequence for a Trx-P501S fusion protein that is encoded by the DNA sequence of SEQ ID NO: 1010.
SEQ ID NO:1012 represents the amino acid sequence for a minimal P501S epitope recognized by clone 2H2-1A12.
SEQ ID NO:1013 represents the DNA sequence for a minimal P501S epitope recognized by clone 2H2-1A12.SEQ ID NO:1014 corresponds to the primer sequence for the forward primer hPAPF1.
SEQ ID NO:1015 corresponds to the primer sequence for the reverse primer hPAPRV1.
SEQ ID NO:1016 corresponds to the primer sequence for the forward primer FOPP2F1.
SEQ ID NO:1017 corresponds to the primer sequence for the reverse primer FOPPF1.
SEQ ID NO:1018 corresponds to the primer sequence for the forward primer FOPP2RV1.
SEQ ID NO:1019 sets forth a DNA sequence for a fusion of FOPP and hPAP, referred to as FOPP2.
SEQ ID NO:1020 sets forth an amino acid sequence for a fusion of FOPP and hPAP, referred to as FOPP2.
SEQ ID NO:1021 sets forth a DNA sequence for a P501S minimal CD8+ T cell epitope recognized by clone 1H1-1A6.
SEQ ID NO:1022 sets forth an amino acid sequence for a P501S minimal CD8+ T cell epitope recognized by clone 1 H1-1A6.
SEQ ID NO:1023 sets forth the sequence for the primer PDM-930.
SEQ ID NO:1024 sets forth the sequence for the primer PDM-165.
SEQ ID NO:1025 sets forth the sequence for the primer PDM-929.
SEQ ID N01026: sets forth a DNA sequence encoding the P501S D protein (amino acids 316-553).
SEQ ID NO:1027 sets forth a DNA sequence encoding the P501S C protein (amino acids 257-553).
SEQ ID NO:1028 sets forth an amino acid sequence corresponding to the P501 S D protein (amino acids 316-553).
SEQ ID NO:1029 sets forth an amino acid sequence corresponding to the P501 S C protein (amino acids 257-553).
SEQ ID NO:1030 sets forth an amino acid for a P703P epitope recognized by patient sera.
SEQ ID NO:1031 sets forth an amino acid for a P703P epitope recognized by patient sera.
SEQ ID NO:1032 sets forth an amino acid for a P703P epitope recognized by patient sera.
SEQ ID NO:1033 sets forth an amino acid for a P703P epitope recognized by patient sera.

### DETAILED DESCRIPTION OF THE INVENTION

U.S. patents, U.S. patent application publications, U.S. patent applications, foreign patents, foreign patent applications and non-patent publications referred to in this specification and/or listed in the Application Data Sheet, are incorporated herein by reference, in their entirety.

The present invention is directed generally to compositions and their use in the therapy and diagnosis of cancer, particularly prostate cancer. As described further below, illustrative compositions of the present invention include, but are not restricted to, polypeptides, particularly immunogenic polypeptides, polynucleotides encoding such polypeptides, antibodies and other binding agents, antigen presenting cells (APCs) and immune system cells (e.g., T cells).

The practice of the present invention will employ, unless indicated specifically to the contrary, conventional methods of virology, immunology, microbiology, molecular biology and recombinant DNA techniques within the skill of the art, many of which are described below for the purpose of illustration. Such techniques are explained fully in the literature. See, e.g., Sambrook, et al. Molecular Cloning: A Laboratory Manual (2nd Edition, 1989); Maniatis et al. Molecular Cloning: A Laboratory Manual (1982); DNA Cloning: A Practical Approach, vol. I & II (D. Glover, ed.); Oligonucleotide Synthesis (N. Gait, ed., 1984); Nucleic Acid Hybridization (B. Hames & S. Higgins, eds., 1985); Transcription and Translation (B. Hames & S. Higgins, eds., 1984); Animal Cell Culture (R. Freshney, ed., 1986); Perbal, A Practical Guide to Molecular Cloning (1984).

All publications, patents and patent applications cited herein, whether supra or infra, are hereby incorporated by reference in their entirety.

As used in this specification and the appended claims, the singular forms "a," "an" and "the" include plural references unless the content clearly dictates otherwise.

### Polypeptide Compositions

As used herein, the term "polypeptide" " is used in its conventional meaning, *i.e.,* as a sequence of amino acids. The polypeptides are not limited to a specific length of the product; thus, peptides, oligopeptides, and proteins are included within the definition of polypeptide, and such terms may be used interchangeably herein unless specifically indicated otherwise. This term also does not refer to or exclude post-expression modifications of the polypeptide, for example, glycosylations, acetylations, phosphorylations and the like, as well as other modifications known in the art, both naturally occurring and non-naturally occurring. A polypeptide may be an entire protein, or a subsequence thereof. Particular polypeptides of interest in the context of this invention are amino acid subsequences comprising epitopes, *i.e.*, antigenic determinants substantially responsible for the immunogenic properties of a polypeptide and being capable of evoking an immune response.

Particularly illustrative polypeptides of the present invention comprise those encoded by a polynucleotide sequence set forth in any one of SEQ ID NO: 1-111, 115-171, 173-175, 177, 179-305, 307-315, 326, 328, 330, 332-335, 340-375, 381, 382 and 384-476, 524, 526, 530, 531, 533, 535, 536, 552, 569-572, 587, 591, 593-606, 618-705, 709-774, 777, 789, 817, 823, 824, 878, 880-882, 894, 896, 907, 908, 916-919, 929-931, 938, 939, 942, 944, 945, 948, 967, 969-976, 991, 993-1002, 1004, 1006, 1007, 1010, 1013-1019, 1021, and 1023-1027 or a sequence that hybridizes under moderately stringent conditions, or, alternatively, under highly stringent conditions, to a polynucleotide sequence set forth in any one of SEQ ID NO: 1-111, 115-171, 173-175, 177, 179-305, 307-315, 326, 328, 330, 332-335, 340-375, 381, 382 and 384-476, 524, 526, 530, 531, 533, 535, 536, 552, 569-572, 587, 591, 593-606, 618-705, 709-774, 777, 789, 817, 823, 824, 878, 880-882, 894, 896, 907, 908, 916-919, 929-931, 938, 939, 942, 944, 945, 948, 967, 969-976, 991, 993-1002, 1004, 1006, 1007, 1010, 1013-1019, 1021, and 1023-1027. In specific embodiments, the polypeptides of the invention comprise amino acid sequences as set forth in any one of SEQ ID NO: 112-114, 172, 176, 178, 327, 329, 331, 336, 339, 376-380, 383, 477-483, 496, 504, 505, 519, 520, 522, 525, 527, 532, 534, 537-551, 553-568, 573-586, 588-590, 592, 706-708, 775, 776, 778, 780, 781, 811, 814, 818, 826, 827, 853, 855, 858, 860-862, 866-877, 879, 883-893, 895, 897, 898, 909-915, 920-928, 932-934, 940, 941, 943, 946, 947, 949-966, 968, 977-990, 992, 1003, 1005, 1008, 1009, 1011, and 1012, 1020, 1022, 1028-1029, and 1030-1033.

The polypeptides of the present invention are sometimes herein referred to as prostate-specific proteins or prostate-specific polypeptides, as an indication that their identification has been based at least in part upon their increased levels of expression in prostate tissue samples. Thus, a "prostate-specific polypeptide" or "prostate-specific protein," refers generally to a polypeptide sequence of the present invention, or a polynucleotide sequence encoding such a polypeptide, that is expressed in a substantial proportion of prostate tissue samples, for example preferably greater than about 20%, more preferably greater than about 30%, and most preferably greater than about 50% or more of prostate tissue samples tested, at a level that is at least two fold, and preferably at least five fold, greater than the level of expression in other normal tissues, as determined using a representative assay provided herein. A prostate-specific polypeptide sequence of the invention, based upon its increased level of expression in tumor cells, has particular utility both as a diagnostic marker as well as a therapeutic target, as further described below.

In certain preferred embodiments, the polypeptides of the invention are immunogenic, *i.e.,* they react detectably within an immunoassay (such as an ELISA or T-cell stimulation assay) with antisera and/or T-cells from a patient with prostate cancer. Screening for immunogenic activity can be performed using techniques well known to the skilled artisan. For example, such screens can be performed using methods such as those described in Harlow and Lane, Antibodies: A Laboratory Manual, Cold Spring Harbor Laboratory, 1988. In one illustrative example, a polypeptide may be immobilized on a solid support and contacted with patient sera to allow binding of antibodies within the sera to the immobilized polypeptide. Unbound sera may then be removed and bound antibodies detected using, for example, ¹²⁵I-labeled Protein A.

As would be recognized by the skilled artisan, immunogenic portions of the polypeptides disclosed herein are also encompassed by the present invention. An "immunogenic portion," as used herein, is a fragment of an immunogenic polypeptide of the invention that itself is immunologically reactive (*i.e.,* specifically binds) with the B-cells and/or T-cell surface antigen receptors that recognize the polypeptide. Immunogenic portions may generally be identified using well known techniques, such as those summarized in Paul, Fundamental Immunology, 3rd ed., 243-247 (Raven Press, 1993) and references cited therein. Such techniques include screening polypeptides for the ability to react with antigen-specific antibodies, antisera and/or T-cell lines or clones. As used herein, antisera and antibodies are "antigen-specific" if they specifically bind to an antigen (*i.e.,* they react with the protein in an ELISA or other immunoassay, and do not react detectably with unrelated proteins). Such antisera and antibodies may be prepared as described herein, and using well-known techniques.

In one preferred embodiment, an immunogenic portion of a polypeptide of the present invention is a portion that reacts with antisera and/or T-cells at a level that is not substantially less than the reactivity of the full-length polypeptide (e.g., in an ELISA and/or T-cell reactivity assay). Preferably, the level of immunogenic activity of the immunogenic portion is at least about 50%, preferably at least about 70% and most preferably greater than about 90% of the immunogenicity for the full-length polypeptide. In some instances, preferred immunogenic portions will be identified that have a level of immunogenic activity greater than that of the corresponding full-length polypeptide, e.g., having greater than about 100% or 150% or more immunogenic activity.

In certain other embodiments, illustrative immunogenic portions may include peptides in which an N-terminal leader sequence and/or transmembrane domain has been deleted. Other illustrative immunogenic portions will contain a small N- and/or C-terminal deletion (e.g., 1-30 amino acids, preferably 5-15 amino acids), relative to the mature protein.

In another embodiment, a polypeptide composition of the invention may also comprise one or more polypeptides that are immunologically reactive with T cells and/or antibodies generated against a polypeptide of the invention, particularly a polypeptide having an amino acid sequence disclosed herein, or to an immunogenic fragment or variant thereof.

In another embodiment of the invention, polypeptides are provided that comprise one or more polypeptides that are capable of eliciting T cells and/or antibodies that are immunologically reactive with one or more polypeptides described herein, or one or more polypeptides encoded by contiguous nucleic acid sequences contained in the polynucleotide sequences disclosed herein, or immunogenic fragments or variants thereof, or to one or more nucleic acid sequences which hybridize to one or more of these sequences under conditions of moderate to high stringency.

The present invention, in another aspect, provides polypeptide fragments comprising at least about 5, 10, 15, 20, 25, 50, or 100 contiguous amino acids, or more, including all intermediate lengths, of a polypeptide composition set forth herein, such as those set forth in SEQ ID NO: 112-114, 172, 176, 178, 327, 329, 331, 336, 339, 376-380, 383, 477-483, 496, 504, 505, 519, 520, 522, 525, 527, 532, 534, 537-551, 553-568, 573-586, 588-590, 592, 706-708, 775, 776, 778, 780, 781, 811, 814, 818, 826, 827, 853, 855, 858, 860-862, 866-877, 879, 883-893, 895, 897, 898, 909-915, 920-928, 932-934, 940, 941, 943, 946, 947, 949-966, 968, 977-990, 992, 1003, 1005, 1008, 1009, 1011, 1012, 1020, 1022, 1028-1029, and 1030-1033 or those encoded by a polynucleotide sequence set forth in a sequence of SEQ ID NO: 1-111, 115-171, 173-175, 177, 179-305, 307-315, 326, 328, 330, 332-335, 340-375, 381, 382 and 384-476, 524, 526, 530, 531, 533, 535, 536, 552, 569-572, 587, 591, 593-606, 618-705, 709-774, 777, 789, 817, 823, 824, 878, 880-882, 894, 896, 907, 908, 916-919, 929-931, 938, 939, 942, 944, 945, 948, 967, 969-976, 991, 993-1002, 1004, 1006, 1007, 1010, 1013-1019, 1021, and 1023-1027.

In another aspect, the present invention provides variants of the polypeptide compositions described herein. Polypeptide variants generally encompassed by the present invention will typically exhibit at least about 70%, 75%, 80%, 85%, 90%, 91%, 92%, 93%, 94%, 95%, 96%, 97%, 98%, or 99% or more identity (determined as described below), along its length, to a polypeptide sequence set forth herein.

In one preferred embodiment, the polypeptide fragments and variants provided by the present invention are immunologically reactive with an antibody and/or T-cell that reacts with a full-length polypeptide specifically set forth herein.

In another preferred embodiment, the polypeptide fragments and variants provided by the present invention exhibit a level of immunogenic activity of at least about 50%, preferably at least about 70%, and most preferably at least about 90% or more of that exhibited by a full-length polypeptide sequence specifically set forth herein.

A polypeptide "variant," as the term is used herein, is a polypeptide that typically differs from a polypeptide specifically disclosed herein in one or more substitutions, deletions, additions and/or insertions. Such variants may be naturally occurring or may be synthetically generated, for example, by modifying one or more of the above polypeptide sequences of the invention and evaluating their immunogenic activity as described herein using any of a number of techniques well known in the art.

For example, certain illustrative variants of the polypeptides of the invention include those in which one or more portions, such as an N-terminal leader sequence or transmembrane domain, have been removed. Other illustrative variants include variants in which a small portion (e.g., 1-30 amino acids, preferably 5-15 amino acids) has been removed from the N- and/or C-terminal of the mature protein.

In many instances, a variant will contain conservative substitutions. A "conservative substitution" is one in which an amino acid is substituted for another amino acid that has similar properties, such that one skilled in the art of peptide chemistry would expect the secondary structure and hydropathic nature of the polypeptide to be substantially unchanged. As described above, modifications may be made in the structure of the polynucleotides and polypeptides of the present invention and still obtain a functional molecule that encodes a variant or derivative polypeptide with desirable characteristics, e.g., with immunogenic characteristics. When it is desired to alter the amino acid sequence of a polypeptide to create an equivalent, or even an improved, immunogenic variant or portion of a polypeptide of the invention, one skilled in the art will typically change one or more of the codons of the encoding DNA sequence according to Table 1.

For example, certain amino acids may be substituted for other amino acids in a protein structure without appreciable loss of interactive binding capacity with structures such as, for example, antigen-binding regions of antibodies or binding sites on substrate molecules. Since it is the interactive capacity and nature of a protein that defines that protein's biological functional activity, certain amino acid sequence substitutions can be made in a protein sequence, and, of course, its underlying DNA coding sequence, and nevertheless obtain a protein with like properties. It is thus contemplated that various changes may be made in the peptide sequences of the disclosed compositions, or corresponding DNA sequences which encode said peptides without appreciable loss of their biological utility or activity.

**TABLE 1**

| **Amino Acids** | | **Codons** | | | | | | |
|---|---|---|---|---|---|---|---|---|
| Alanine | Ala | A | GCA | GCC | GCG | GCU | | |
| Cysteine | Cys | C | UGC | UGU | | | | |
| Aspartic acid | Asp | D | GAC | GAU | | | | |
| Glutamic acid | Glu | E | GAA | GAG | | | | |
| Phenylalanine | Phe | F | UUC | UUU | | | | |
| Glycine | Gly | G | GGA | GGC | GGG | GGU | | |
| Histidine | His | H | CAC | CAU | | | | |
| Isoleucine | IIe | I | AUA | AUC | AUU | | | |
| Lysine | Lys | K | AAA | AAG | | | | |
| Leucine | Leu | L | UUA | UUG | CUA | CUC | CUG | CUU |
| Methionine | Met | M | AUG | | | | | |
| Asparagine | Asn | N | AAC | AAU | | | | |
| Proline | Pro | P | CCA | CCC | CCG | CCU | | |
| Glutamine | Gln | Q | CAA | CAG | | | | |
| Arginine | Arg | R | AGA | AGG | CGA | CGC | CGG | CGU |
| Serine | Ser | S | AGC | AGU | UCA | UCC | UCG | UCU |
| Threonine | Thr | T | ACA | ACC | ACG | ACU | | |
| Valine | Val | V | GUA | GUC | GUG | GUU | | |
| Tryptophan | Trp | W | UGG | | | | | |
| Tyrosine | Tyr | Y | UAC | UAU | | | | |

In making such changes, the hydropathic index of amino acids may be considered. The importance of the hydropathic amino acid index in conferring interactive biologic function on a protein is generally understood in the art (Kyte and Doolittle, 1982, incorporated herein by reference). It is accepted that the relative hydropathic character of the amino acid contributes to the secondary structure of the resultant protein, which in turn defines the interaction of the protein with other molecules, for example, enzymes, substrates, receptors, DNA, antibodies, antigens, and the like. Each amino acid has been assigned a hydropathic index on the basis of its hydrophobicity and charge characteristics (Kyte and Doolittle, 1982). These values are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

It is known in the art that certain amino acids may be substituted by other amino acids having a similar hydropathic index or score and still result in a protein with similar biological activity, *i.e.* still obtain a biological functionally equivalent protein. In making such changes, the substitution of amino acids whose hydropathic indices are within ±2 is preferred, those within ±1 are particularly preferred, and those within ±0.5 are even more particularly preferred. It is also understood in the art that the substitution of like amino acids can be made effectively on the basis of hydrophilicity. U. S. Patent 4,554,101 (specifically incorporated herein by reference in its entirety), states that the greatest local average hydrophilicity of a protein, as governed by the hydrophilicity of its adjacent amino acids, correlates with a biological property of the protein.

As detailed in U. S. Patent 4,554,101, the following hydrophilicity values have been assigned to amino acid residues: arginine (+3.0); lysine (+3.0); aspartate (+3.0 ± 1); glutamate (+3.0 ± 1); serine (+0.3); asparagine (+0.2); glutamine (+0.2); glycine (0); threonine (-0.4); proline (-0.5 ± 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). It is understood that an amino acid can be substituted for another having a similar hydrophilicity value and still obtain a biologically equivalent, and in particular, an immunologically equivalent protein. In such changes, the substitution of amino acids whose hydrophilicity values are within ±2 is preferred, those within ±1 are particularly preferred, and those within ±0.5 are even more particularly preferred.

As outlined above, amino acid substitutions are generally therefore based on the relative similarity of the amino acid side-chain substituents, for example, their hydrophobicity, hydrophilicity, charge, size, and the like. Exemplary substitutions that take various of the foregoing characteristics into consideration are well known to those of skill in the art and include: arginine and lysine; glutamate and aspartate; serine and threonine; glutamine and asparagine; and valine, leucine and isoleucine.

In addition, any polynucleotide may be further modified to increase stability *in vivo.* Possible modifications include, but are not limited to, the addition of flanking sequences at the 5' and/or 3' ends; the use of phosphorothioate or 2' O-methyl rather than phosphodiesterase linkages in the backbone; and/or the inclusion of nontraditional bases such as inosine, queosine and wybutosine, as well as acetyl- methyl-, thio- and other modified forms of adenine, cytidine, guanine, thymine and uridine.

Amino acid substitutions may further be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine and valine; glycine and alanine; asparagine and glutamine; and serine, threonine, phenylalanine and tyrosine. Other groups of amino acids that may represent conservative changes include: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his. A variant may also, or alternatively, contain nonconservative changes. In a preferred embodiment, variant polypeptides differ from a native sequence by substitution, deletion or addition of five amino acids or fewer. Variants may also (or alternatively) be modified by, for example, the deletion or addition of amino acids that have minimal influence on the immunogenicity, secondary structure and hydropathic nature of the polypeptide.

As noted above, polypeptides may comprise a signal (or leader) sequence at the N-terminal end of the protein, which co-translationally or posttranslationally directs transfer of the protein. The polypeptide may also be conjugated to a linker or other sequence for ease of synthesis, purification or identification of the polypeptide (e.g., poly-His), or to enhance binding of the polypeptide to a solid support. For example, a polypeptide may be conjugated to an immunoglobulin Fc region.

When comparing polypeptide sequences, two sequences are said to be "identical" if the sequence of amino acids in the two sequences is the same when aligned for maximum correspondence, as described below. Comparisons between two sequences are typically performed by comparing the sequences over a comparison window to identify and compare local regions of sequence similarity. A "comparison window" as used herein, refers to a segment of at least about 20 contiguous positions, usually 30 to about 75, 40 to about 50, in which a sequence may be compared to a reference sequence of the same number of contiguous positions after the two sequences are optimally aligned.

Optimal alignment of sequences for comparison may be conducted using the Megalign program in the Lasergene suite of bioinformatics software (DNASTAR, Inc., Madison, WI), using default parameters. This program embodies several alignment schemes described in the following references: Dayhoff, M.O. (1978) A model of evolutionary change in proteins - Matrices for detecting distant relationships. In Dayhoff, M.O. (ed.) Atlas of Protein Sequence and Structure, National Biomedical Research Foundation, Washington DC Vol. 5, Suppl. 3, pp. 345-358; Hein J. (1990) Unified Approach to Alignment and Phylogenes pp. 626-645 Methods in Enzymology vol. 183, Academic Press, Inc., San Diego, CA; Higgins, D.G. and Sharp, P.M. (1989) CABIOS 5:151-153; Myers, E.W. and Muller W. (1988) CABIOS 4:11-17; Robinson, E.D. (1971) Comb. Theor 11:105; Santou, N. Nes, M. (1987) Mol. Biol. Evol. 4:406-425; Sneath, P.H.A. and Sokal, R.R. (1973) Numerical Taxonomy- the Principles and Practice of Numerical Taxonomy, Freeman Press, San Francisco, CA; Wilbur, W.J. and Lipman, D.J. (1983) Proc. Nat'l Acad., Sci. USA 80:726-730.

Alternatively, optimal alignment of sequences for comparison may be conducted by the local identity algorithm of Smith and Waterman (1981) Add. APL. Math 2:482, by the identity alignment algorithm of Needleman and Wunsch (1970) J. Mol. Biol. 48:443, by the search for similarity methods of Pearson and Lipman (1988) Proc. Nat'l Acad. Sci. USA 85: 2444, by computerized implementations of these algorithms (GAP, BESTFIT, BLAST, FASTA, and TFASTA in the Wisconsin Genetics Software Package, Genetics Computer Group (GCG), 575 Science Dr., Madison, WI), or by inspection.

One preferred example of algorithms that are suitable for determining percent sequence identity and sequence similarity are the BLAST and BLAST 2.0 algorithms, which are described in Altschul et al. (1977) Nucl. Acids Res. 25:3389-3402 and Altschul et al. (1990) J. Mol. Biol. 215:403-410, respectively. BLAST and BLAST 2.0 can be used, for example with the parameters described herein, to determine percent sequence identity for the polynucleotides and polypeptides of the invention. Software for performing BLAST analyses is publicly available through the National Center for Biotechnology Information. For amino acid sequences, a scoring matrix can be used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment.

In one preferred approach, the "percentage of sequence identity" is determined by comparing two optimally aligned sequences over a window of comparison of at least 20 positions, wherein the portion of the polypeptide sequence in the comparison window may comprise additions or deletions (*i.e.,* gaps) of 20 percent or less, usually 5 to 15 percent, or 10 to 12 percent, as compared to the reference sequences (which does not comprise additions or deletions) for optimal alignment of the two sequences. The percentage is calculated by determining the number of positions at which the identical amino acid residue occurs in both sequences to yield the number of matched positions, dividing the number of matched positions by the total number of positions in the reference sequence (*i.e.,* the window size) and multiplying the results by 100 to yield the percentage of sequence identity.

Within other illustrative embodiments, a polypeptide may be a fusion polypeptide that comprises multiple polypeptides as described herein, or that comprises at least one polypeptide as described herein and an unrelated sequence, such as a known tumor protein. A fusion partner may, for example, assist in providing T helper epitopes (an immunological fusion partner), preferably T helper epitopes recognized by humans, or may assist in expressing the protein (an expression enhancer) at higher yields than the native recombinant protein. Certain preferred fusion partners are both immunological and expression enhancing fusion partners. Other fusion partners may be selected so as to increase the solubility of the polypeptide or to enable the polypeptide to be targeted to desired intracellular compartments. Still further fusion partners include affinity tags, which facilitate purification of the polypeptide.

Fusion polypeptides may generally be prepared using standard techniques, including chemical conjugation. Preferably, a fusion polypeptide is expressed as a recombinant polypeptide, allowing the production of increased levels, relative to a non-fused polypeptide, in an expression system. Briefly, DNA sequences encoding the polypeptide components may be assembled separately, and ligated into an appropriate expression vector. The 3' end of the DNA sequence encoding one polypeptide component is ligated, with or without a peptide linker, to the 5' end of a DNA sequence encoding the second polypeptide component so that the reading frames of the sequences are in phase. This permits translation into a single fusion polypeptide that retains the biological activity of both component polypeptides.

A peptide linker sequence may be employed to separate the first and second polypeptide components by a distance sufficient to ensure that each polypeptide folds into its secondary and tertiary structures. Such a peptide linker sequence is incorporated into the fusion polypeptide using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46, 1985; Murphy et al., Proc. Nat'l Acad. Sci. USA 83:8258-8262, 1986; U.S. Patent No. 4,935,233 and U.S. Patent No. 4,751,180. The linker sequence may generally be from 1 to about 50 amino acids in length. Linker sequences are not required when the first and second polypeptides have non-essential N-terminal amino acid regions that can be used to separate the functional domains and prevent steric interference.

The ligated DNA sequences are operably linked to suitable transcriptional or translational regulatory elements. The regulatory elements responsible for expression of DNA are located only 5' to the DNA sequence encoding the first polypeptides. Similarly, stop codons required to end translation and transcription termination signals are only present 3' to the DNA sequence encoding the second polypeptide.

The fusion polypeptide can comprise a polypeptide as described herein together with an unrelated immunogenic protein, such as an immunogenic protein capable of eliciting a recall response. Examples of such proteins include tetanus, tuberculosis and hepatitis proteins (see, for example, Stoute et al. New Engl. J. Med., 336:86-91, 1997).

In one preferred embodiment, the immunological fusion partner is derived from a Mycobacterium sp., such as a *Mycobacterium tuberculosis-*derived Ra12 fragment. Ra12 compositions and methods for their use in enhancing the expression and/or immunogenicity of heterologous polynucleotide/polypeptide sequences is described in U.S. Patent Application 60/158,585, the disclosure of which is incorporated herein by reference in its entirety. Briefly, Ra12 refers to a polynucleotide region that is a subsequence of a *Mycobacterium tuberculosis* MTB32A nucleic acid. MTB32A is a serine protease of 32 KD molecular weight encoded by a gene in virulent and avirulent strains of M. *tuberculosis.* The nucleotide sequence and amino acid sequence of MTB32A have been described (for example, U.S. Patent Application 60/158,585; see also, Skeiky et al., Infection and Immun. (1999) 67:3998-4007, incorporated herein by reference). C-terminal fragments of the MTB32A coding sequence express at high levels and remain as a soluble polypeptides throughout the purification process. Moreover, Ra12 may enhance the immunogenicity of heterologous immunogenic polypeptides with which it is fused. One preferred Ra12 fusion polypeptide comprises a 14 KD C-terminal fragment corresponding to amino acid residues 192 to 323 of MTB32A. Other preferred Ra12 polynucleotides generally comprise at least about 15 consecutive nucleotides, at least about 30 nucleotides, at least about 60 nucleotides, at least about 100 nucleotides, at least about 200 nucleotides, or at least about 300 nucleotides that encode a portion of a Ra12 polypeptide. Ra12 polynucleotides may comprise a native sequence (*i.e.,* an endogenous sequence that encodes a Ra12 polypeptide or a portion thereof) or may comprise a variant of such a sequence. Ra12 polynucleotide variants may contain one or more substitutions, additions, deletions and/or insertions such that the biological activity of the encoded fusion polypeptide is not substantially diminished, relative to a fusion polypeptide comprising a native Ra12 polypeptide. Variants preferably exhibit at least about 70% identity, more preferably at least about 80% identity and most preferably at least about 90% identity to a polynucleotide sequence that encodes a native Ra12 polypeptide or a portion thereof.

Within other preferred embodiments, an immunological fusion partner is derived from protein D, a surface protein of the gram-negative bacterium Haemophilus influenza B (WO 91/18926). Preferably, a protein D derivative comprises approximately the first third of the protein (e.g., the first N-terminal 100-110 amino acids), and a protein D derivative may be lipidated. Within certain preferred embodiments, the first 109 residues of a Lipoprotein D fusion partner is included on the N-terminus to provide the polypeptide with additional exogenous T-cell epitopes and to increase the expression level in *E. coli* (thus functioning as an expression enhancer). The lipid tail ensures optimal presentation of the antigen to antigen presenting cells. Other fusion partners include the non-structural protein from influenzae virus, NS1 (hemaglutinin). Typically, the N-terminal 81 amino acids are used, although different fragments that include T-helper epitopes may be used.

In another embodiment, the immunological fusion partner is the protein known as LYTA, or a portion thereof (preferably a C-terminal portion). LYTA is derived from *Streptococcus pneumoniae,* which synthesizes an N-acetyl-L-alanine amidase known as amidase LYTA (encoded by the LytA gene; Gene 43:265-292, 1986). LYTA is an autolysin that specifically degrades certain bonds in the peptidoglycan backbone. The C-terminal domain of the LYTA protein is responsible for the affinity to the choline or to some choline analogues such as DEAE. This property has been exploited for the development of *E. coli* C-LYTA expressing plasmids useful for expression of fusion proteins. Purification of hybrid proteins containing the C-LYTA fragment at the amino terminus has been described *(see* Biotechnology 10:795-798, 1992). Within a preferred embodiment, a repeat portion of LYTA may be incorporated into a fusion polypeptide. A repeat portion is found in the C-terminal region starting at residue 178. A particularly preferred repeat portion incorporates residues 188-305.

Yet another illustrative embodiment involves fusion polypeptides, and the polynucleotides encoding them, wherein the fusion partner comprises a targeting signal capable of directing a polypeptide to the endosomal/lysosomal compartment, as described in U.S. Patent No. 5,633,234. An immunogenic polypeptide of the invention, when fused with this targeting signal, will associate more efficiently with MHC class II molecules and thereby provide enhanced in vivo stimulation of CD4⁺ T-cells specific for the polypeptide.

Polypeptides of the invention are prepared using any of a variety of well known synthetic and/or recombinant techniques, the latter of which are further described below. Polypeptides, portions and other variants generally less than about 150 amino acids can be generated by synthetic means, using techniques well known to those of ordinary skill in the art. In one illustrative example, such polypeptides are synthesized using any of the commercially available solid-phase techniques, such as the Merrifield solid-phase synthesis method, where amino acids are sequentially added to a growing amino acid chain. See Merrifield, J. Am. Chem. Soc. 85:2149-2146, 1963. Equipment for automated synthesis of polypeptides is commercially available from suppliers such as Perkin Elmer/Applied BioSystems Division (Foster City, CA), and may be operated according to the manufacturer's instructions.

In general, polypeptide compositions (including fusion polypeptides) of the invention are isolated. An "isolated" polypeptide is one that is removed from its original environment. For example, a naturally-occurring protein or polypeptide is isolated if it is separated from some or all of the coexisting materials in the natural system. Preferably, such polypeptides are also purified, e.g., are at least about 90% pure, more preferably at least about 95% pure and most preferably at least about 99% pure.

### Polynucleotide Compositions

The present invention, in other aspects, provides polynucleotide compositions. The terms "DNA" and "polynucleotide" are used essentially interchangeably herein to refer to a DNA molecule that has been isolated free of total genomic DNA of a particular species. "Isolated," as used herein, means that a polynucleotide is substantially away from other coding sequences, and that the DNA molecule does not contain large portions of unrelated coding DNA, such as large chromosomal fragments or other functional genes or polypeptide coding regions. Of course, this refers to the DNA molecule as originally isolated, and does not exclude genes or coding regions later added to the segment by the hand of man.

As will be understood by those skilled in the art, the polynucleotide compositions of this invention can include genomic sequences, extra-genomic and plasmid-encoded sequences and smaller engineered gene segments that express, or may be adapted to express, proteins, polypeptides, peptides and the like. Such segments may be naturally isolated, or modified synthetically by the hand of man.

As will be also recognized by the skilled artisan, polynucleotides of the invention may be single-stranded (coding or antisense) or double-stranded, and may be DNA (genomic, cDNA or synthetic) or RNA molecules. RNA molecules may include HnRNA molecules, which contain introns and correspond to a DNA molecule in a one-to-one manner, and mRNA molecules, which do not contain introns. Additional coding or non-coding sequences may, but need not, be present within a polynucleotide of the present invention, and a polynucleotide may, but need not, be linked to other molecules and/or support materials.

Polynucleotides may comprise a native sequence (*i.e*., an endogenous sequence that encodes a polypeptide/protein of the invention or a portion thereof) or may comprise a sequence that encodes a variant or derivative, preferably an immunogenic variant or derivative, of such a sequence.

Therefore, according to another aspect of the present invention, polynucleotide compositions are provided that comprise some or all of a polynucleotide sequence set forth in any one of SEQ ID NO: 1-111, 115-171, 173-175, 177, 179-305, 307-315, 326, 328, 330, 332-335, 340-375, 381, 382 and 384-476, 524, 526, 530, 531, 533, 535, 536, 552, 569-572, 587, 591, 593-606, 618-705, 709-774, 777, 789, 817, 823, 824, 878, 880-882, 894, 896, 907, 908, 916-919, 929-931, 938, 939, 942, 944, 945, 948, 967, 969-976, 991, 993-1002, 1004, 1006, 1007, 1010, 1013-1019, 1021, and 1023-1027, complements of a polynucleotide sequence set forth in any one of SEQ ID NO: 1-111, 115-171, 173-175, 177, 179-305, 307-315, 326, 328, 330, 332-335, 340-375, 381, 382 and 384-476, 524, 526, 530, 531, 533, 535, 536, 552, 569-572, 587, 591, 593-606, 618-705, 709-774, 777, 789, 817, 823, 824, 878, 880-882, 894, 896, 907, 908, 916-919, 929-931, 938, 939, 942, 944, 945, 948, 967, 969-976, 991, 993-1002, 1004, 1006, 1007, 1010, 1013-1019, 1021, and 1023-1027, and degenerate variants of a polynucleotide sequence set forth in any one of SEQ ID NO: 1-111, 115-171, 173-175, 177, 179-305, 307-315, 326, 328, 330, 332-335, 340-375, 381, 382 and 384-476, 524, 526, 530, 531, 533, 535, 536, 552, 569-572, 587, 591, 593-606, 618-705, 709-774, 777, 789, 817, 823, 824, 878, 880-882, 894, 896, 907, 908, 916-919, 929-931, 938, 939, 942, 944, 945, 948, 967, 969-976, 991, 993-1002, 1004, 1006, 1007, 1010, 1013-1019, 1021, and 1023-1027. In certain preferred embodiments, the polynucleotide sequences set forth herein encode immunogenic polypeptides, as described above.

In other related embodiments, the present invention provides polynucleotide variants having substantial identity to the sequences disclosed herein in SEQ ID NO: 1-111, 115-171, 173-175, 177, 179-305, 307-315, 326, 328, 330, 332-335, 340-375, 381, 382 and 384-476, 524, 526, 530, 531, 533, 535, 536, 552, 569-572, 587, 591, 593-606, 618-705, 709-774, 777, 789, 817, 823, 824, 878, 880-882, 894, 896, 907, 908, 916-919, 929-931, 938, 939, 942, 944, 945, 948, 967, 969-976, 991, 993-1002, 1004, 1006, 1007, 1010, 1013-1019, 1021, and 1023-1027 for example those comprising at least 70% sequence identity, preferably at least 75%, 80%, 85%, 90%, 95%, 96%, 97%, 98%, or 99% or higher, sequence identity compared to a polynucleotide sequence of this invention using the methods described herein, (e.g., BLAST analysis using standard parameters, as described below). One skilled in this art will recognize that these values can be appropriately adjusted to determine corresponding identity of proteins encoded by two nucleotide sequences by taking into account codon degeneracy, amino acid similarity, reading frame positioning and the like.

Typically, polynucleotide variants will contain one or more substitutions, additions, deletions and/or insertions, preferably such that the immunogenicity of the polypeptide encoded by the variant polynucleotide is not substantially diminished relative to a polypeptide encoded by a polynucleotide sequence specifically set forth herein). The term "variants" should also be understood to encompasses homologous genes of xenogenic origin.

In additional embodiments, the present invention provides polynucleotide fragments comprising various lengths of contiguous stretches of sequence identical to, or complementary to, one or more of the sequences disclosed herein. For example, polynucleotides are provided by this invention that comprise at least about 10, 15, 20, 30, 40, 50, 75, 100, 150, 200, 300, 400, 500 or 1000 or more contiguous nucleotides of one or more of the sequences disclosed herein as well as all intermediate lengths there between. It will be readily understood that "intermediate lengths", in this context, means any length between the quoted values, such as 16, 17, 18, 19, *etc.;* 21, 22, 23, etc.; 30, 31, 32, etc.; 50, 51, 52, 53, etc.; 100, 101, 102, 103, etc.; 150, 151, 152, 153, *etc*.; including all integers through 200-500; 500-1,000, and the like.

In another embodiment of the invention, polynucleotide compositions are provided that are capable of hybridizing under moderate to high stringency conditions to a polynucleotide sequence provided herein, or a fragment thereof, or a complementary sequence thereof. Hybridization techniques are well known in the art of molecular biology. For purposes of illustration, suitable moderately stringent conditions for testing the hybridization of a polynucleotide of this invention with other polynucleotides include prewashing in a solution of 5 X SSC, 0.5% SDS, 1.0 mM EDTA (pH 8.0); hybridizing at 50°C-60°C, 5 X SSC, overnight; followed by washing twice at 65°C for 20 minutes with each of 2X, 0.5X and 0.2X SSC containing 0.1% SDS. One skilled in the art will understand that the stringency of hybridization can be readily manipulated, such as by altering the salt content of the hybridization solution and/or the temperature at which the hybridization is performed. For example, in another embodiment, suitable highly stringent hybridization conditions include those described above, with the exception that the temperature of hybridization is increased, e.g., to 60-65°C or 65-70°C.

In certain preferred embodiments, the polynucleotides described above, e.g., polynucleotide variants, fragments and hybridizing sequences, encode polypeptides that are immunologically cross-reactive with a polypeptide sequence specifically set forth herein. In other preferred embodiments, such polynucleotides encode polypeptides that have a level of immunogenic activity of at least about 50%, preferably at least about 70%, and more preferably at least about 90% of that for a polypeptide sequence specifically set forth herein.

The polynucleotides of the present invention, or fragments thereof, regardless of the length of the coding sequence itself, may be combined with other DNA sequences, such as promoters, polyadenylation signals, additional restriction enzyme sites, multiple cloning sites, other coding segments, and the like, such that their overall length may vary considerably. It is therefore contemplated that a nucleic acid fragment of almost any length may be employed, with the total length preferably being limited by the ease of preparation and use in the intended recombinant DNA protocol. For example, illustrative polynucleotide segments with total lengths of about 10,000, about 5000, about 3000, about 2,000, about 1,000, about 500, about 200, about 100, about 50 base pairs in length, and the like, (including all intermediate lengths) are contemplated to be useful in many implementations of this invention.

When comparing polynucleotide sequences, two sequences are said to be "identical" if the sequence of nucleotides in the two sequences is the same when aligned for maximum correspondence, as described below. Comparisons between two sequences are typically performed by comparing the sequences over a comparison window to identify and compare local regions of sequence similarity. A "comparison window" as used herein, refers to a segment of at least about 20 contiguous positions, usually 30 to about 75, preferably 40 to about 50, in which a sequence may be compared to a reference sequence of the same number of contiguous positions after the two sequences are optimally aligned.

Optimal alignment of sequences for comparison may be conducted using the Megalign program in the Lasergene suite of bioinformatics software (DNASTAR, Inc., Madison, WI), using default parameters. This program embodies several alignment schemes described in the following references: Dayhoff, M.O. (1978) A model of evolutionary change in proteins - Matrices for detecting distant relationships. In Dayhoff, M.O. (ed.) Atlas of Protein Sequence and Structure, National Biomedical Research Foundation, Washington DC Vol. 5, Suppl. 3, pp. 345-358; Hein J. (1990) Unified Approach to Alignment and Phylogenes pp. 626-645 Methods in Enzymology vol. 183, Academic Press, Inc., San Diego, CA; Higgins, D.G. and Sharp, P.M. (1989) CABIOS 5:151-153; Myers, E.W. and Muller W. (1988) CABIOS 4:11-17*;* Robinson, E.D. (1971) Comb. Theor 11:105; Santou, N. Nes, M. (1987) Mol. Biol. Evol. 4:406-425; Sneath, P.H.A. and Sokal, R.R. (1973) Numerical Taxonomy- the Principles and Practice of Numerical Taxonomy, Freeman Press, San Francisco, CA; Wilbur, W.J. and Lipman, D.J. (1983) Proc. Nat'l Acad., Sci. USA 80:726-730.

Alternatively, optimal alignment of sequences for comparison may be conducted by the local identity algorithm of Smith and Waterman (1981) Add. APL. Math 2:482, by the identity alignment algorithm of Needleman and Wunsch (1970) J. Mol. Biol. 48:443, by the search for similarity methods of Pearson and Lipman (1988) Proc. Nat'l Acad. Sci. USA 85: 2444, by computerized implementations of these algorithms (GAP, BESTFIT, BLAST, FASTA, and TFASTA in the Wisconsin Genetics Software Package, Genetics Computer Group (GCG), 575 Science Dr., Madison, WI), or by inspection.

One preferred example of algorithms that are suitable for determining percent sequence identity and sequence similarity are the BLAST and BLAST 2.0 algorithms, which are described in Altschul et al. (1977) Nucl. Acids Res. 25:3389-3402 and Altschul et al. (1990) J. Mol. Biol. 215:403-410, respectively. BLAST and BLAST 2.0 can be used, for example with the parameters described herein, to determine percent sequence identity for the polynucleotides of the invention. Software for performing BLAST analyses is publicly available through the National Center for Biotechnology Information. In one illustrative example, cumulative scores can be calculated using, for nucleotide sequences, the parameters M (reward score for a pair of matching residues; always >0) and N (penalty score for mismatching residues; always <0). Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLASTN program (for nucleotide sequences) uses as defaults a wordlength (W) of 11, and expectation (E) of 10, and the BLOSUM62 scoring matrix (see Henikoff and Henikoff (1989) Proc. Nat'l Acad. Sci. USA 89:10915) alignments, (B) of 50, expectation (E) of 10, M=5, N=-4 and a comparison of both strands.

Preferably, the "percentage of sequence identity" is determined by comparing two optimally aligned sequences over a window of comparison of at least 20 positions, wherein the portion of the polynucleotide sequence in the comparison window may comprise additions or deletions (*i.e*., gaps) of 20 percent or less, usually 5 to 15 percent, or 10 to 12 percent, as compared to the reference sequences (which does not comprise additions or deletions) for optimal alignment of the two sequences. The percentage is calculated by determining the number of positions at which the identical nucleic acid bases occurs in both sequences to yield the number of matched positions, dividing the number of matched positions by the total number of positions in the reference sequence (*i.e*., the window size) and multiplying the results by 100 to yield the percentage of sequence identity.

It will be appreciated by those of ordinary skill in the art that, as a result of the degeneracy of the genetic code, there are many nucleotide sequences that encode a polypeptide as described herein. Some of these polynucleotides bear minimal homology to the nucleotide sequence of any native gene. Nonetheless, polynucleotides that vary due to differences in codon usage are specifically contemplated by the present invention. Further, alleles of the genes comprising the polynucleotide sequences provided herein are within the scope of the present invention. Alleles are endogenous genes that are altered as a result of one or more mutations, such as deletions, additions and/or substitutions of nucleotides. The resulting mRNA and protein may, but need not, have an altered structure or function. Alleles may be identified using standard techniques (such as hybridization, amplification and/or database sequence comparison).

Therefore, in another embodiment of the invention, a mutagenesis approach, such as site-specific mutagenesis, is employed for the preparation of immunogenic variants and/or derivatives of the polypeptides described herein. By this approach, specific modifications in a polypeptide sequence can be made through mutagenesis of the underlying polynucleotides that encode them. These techniques provides a straightforward approach to prepare and test sequence variants, for example, incorporating one or more of the foregoing considerations, by introducing one or more nucleotide sequence changes into the polynucleotide.

Site-specific mutagenesis allows the production of mutants through the use of specific oligonucleotide sequences which encode the DNA sequence of the desired mutation, as well as a sufficient number of adjacent nucleotides, to provide a primer sequence of sufficient size and sequence complexity to form a stable duplex on both sides of the deletion junction being traversed. Mutations may be employed in a selected polynucleotide sequence to improve, alter, decrease, modify, or otherwise change the properties of the polynucleotide itself, and/or alter the properties, activity, composition, stability, or primary sequence of the encoded polypeptide.

In certain embodiments of the present invention, the inventors contemplate the mutagenesis of the disclosed polynucleotide sequences to alter one or more properties of the encoded polypeptide, such as the immunogenicity of a polypeptide vaccine. The techniques of site-specific mutagenesis are well-known in the art, and are widely used to create variants of both polypeptides and polynucleotides. For example, site-specific mutagenesis is often used to alter a specific portion of a DNA molecule. In such embodiments, a primer comprising typically about 14 to about 25 nucleotides or so in length is employed, with about 5 to about 10 residues on both sides of the junction of the sequence being altered.

As will be appreciated by those of skill in the art, site-specific mutagenesis techniques have often employed a phage vector that exists in both a single stranded and double stranded form. Typical vectors useful in site-directed mutagenesis include vectors such as the M13 phage. These phage are readily commercially-available and their use is generally well-known to those skilled in the art. Double-stranded plasmids are also routinely employed in site directed mutagenesis that eliminates the step of transferring the gene of interest from a plasmid to a phage.

In general, site-directed mutagenesis in accordance herewith is performed by first obtaining a single-stranded vector or melting apart of two strands of a double-stranded vector that includes within its sequence a DNA sequence that encodes the desired peptide. An oligonucleotide primer bearing the desired mutated sequence is prepared, generally synthetically. This primer is then annealed with the single-stranded vector, and subjected to DNA polymerizing enzymes such as *E*. *coli* polymerase I Klenow fragment, in order to complete the synthesis of the mutation-bearing strand. Thus, a heteroduplex is formed wherein one strand encodes the original non-mutated sequence and the second strand bears the desired mutation. This heteroduplex vector is then used to transform appropriate cells, such as *E. coli* cells, and clones are selected which include recombinant vectors bearing the mutated sequence arrangement.

The preparation of sequence variants of the selected peptide-encoding DNA segments using site-directed mutagenesis provides a means of producing potentially useful species and is not meant to be limiting as there are other ways in which sequence variants of peptides and the DNA sequences encoding them may be obtained. For example, recombinant vectors encoding the desired peptide sequence may be treated with mutagenic agents, such as hydroxylamine, to obtain sequence variants. Specific details regarding these methods and protocols are found in the teachings of Maloy *et al.,* 1994; Segal, 1976; Prokop and Bajpai, 1991; Kuby, 1994; and Maniatis *et al.,* 1982, each incorporated herein by reference, for that purpose.

As used herein, the term "oligonucleotide directed mutagenesis procedure" refers to template-dependent processes and vector-mediated propagation which result in an increase in the concentration of a specific nucleic acid molecule relative to its initial concentration, or in an increase in the concentration of a detectable signal, such as amplification. As used herein, the term "oligonucleotide directed mutagenesis procedure" is intended to refer to a process that involves the template-dependent extension of a primer molecule. The term template dependent process refers to nucleic acid synthesis of an RNA or a DNA molecule wherein the sequence of the newly synthesized strand of nucleic acid is dictated by the well-known rules of complementary base pairing (see, for example, Watson, 1987). Typically, vector mediated methodologies involve the introduction of the nucleic acid fragment into a DNA or RNA vector, the clonal amplification of the vector, and the recovery of the amplified nucleic acid fragment. Examples of such methodologies are provided. by U. S. Patent No. 4,237,224, specifically incorporated herein by reference in its entirety.

In another approach for the production of polypeptide variants of the present invention, recursive sequence recombination, as described in U.S. Patent No. 5,837,458, may be employed. In this approach, iterative cycles of recombination and screening or selection are performed to "evolve" individual polynucleotide variants of the invention having, for example, enhanced immunogenic activity.

In other embodiments of the present invention, the polynucleotide sequences provided herein can be advantageously used as probes or primers for nucleic acid hybridization. As such, it is contemplated that nucleic acid segments that comprise a sequence region of at least about 15 contiguous nucleotides that has the same sequence as, or is complementary to, a 15 nucleotide long contiguous sequence disclosed herein will find particular utility. Longer contiguous identical or complementary sequences, e.g., those of about 20, 30, 40, 50, 100, 200, 500, 1000 (including all intermediate lengths) and even up to full length sequences will also be of use in certain embodiments.

The ability of such nucleic acid probes to specifically hybridize to a sequence of interest will enable them to be of use in detecting the presence of complementary sequences in a given sample. However, other uses are also envisioned, such as the use of the sequence information for the preparation of mutant species primers, or primers for use in preparing other genetic constructions.

Polynucleotide molecules having sequence regions consisting of contiguous nucleotide stretches of 10-14, 15-20, 30, 50, or even of 100-200 nucleotides or so (including intermediate lengths as well), identical or complementary to a polynucleotide sequence disclosed herein, are particularly contemplated as hybridization probes for use in, e.g., Southern and Northern blotting. This would allow a gene product, or fragment thereof, to be analyzed, both in diverse cell types and also in various bacterial cells. The total size of fragment, as well as the size of the complementary stretch(es), will ultimately depend on the intended use or application of the particular nucleic acid segment. Smaller fragments will generally find use in hybridization embodiments, wherein the length of the contiguous complementary region may be varied, such as between about 15 and about 100 nucleotides, but larger contiguous complementarity stretches may be used, according to the length complementary sequences one wishes to detect.

The use of a hybridization probe of about 15-25 nucleotides in length allows the formation of a duplex molecule that is both stable and selective. Molecules having contiguous complementary sequences over stretches greater than 15 bases in length are generally preferred, though, in order to increase stability and selectivity of the hybrid, and thereby improve the quality and degree of specific hybrid molecules obtained. One will generally prefer to design nucleic acid molecules having gene-complementary stretches of 15 to 25 contiguous nucleotides, or even longer where desired.

Hybridization probes may be selected from any portion of any of the sequences disclosed herein. All that is required is to review the sequences set forth herein, or to any continuous portion of the sequences, from about 15-25 nucleotides in length up to and including the full length sequence, that one wishes to utilize as a probe or primer. The choice of probe and primer sequences may be governed by various factors. For example, one may wish to employ primers from towards the termini of the total sequence.

Small polynucleotide segments or fragments may be readily prepared by, for example, directly synthesizing the fragment by chemical means, as is commonly practiced using an automated oligonucleotide synthesizer. Also, fragments may be obtained by application of nucleic acid reproduction technology, such as the PCR^{™} technology of U. S. Patent 4,683,202 (incorporated herein by reference), by introducing selected sequences into recombinant vectors for recombinant production, and by other recombinant DNA techniques generally known to those of skill in the art of molecular biology.

The nucleotide sequences of the invention may be used for their ability to selectively form duplex molecules with complementary stretches of the entire gene or gene fragments of interest. Depending on the application envisioned, one will typically desire to employ varying conditions of hybridization to achieve varying degrees of selectivity of probe towards target sequence. For applications requiring high selectivity, one will typically desire to employ relatively stringent conditions to form the hybrids, e.g., one will select relatively low salt and/or high temperature conditions, such as provided by a salt concentration of from about 0.02 M to about 0.15 M salt at temperatures of from about 50°C to about 70°C. Such selective conditions tolerate little, if any, mismatch between the probe and the template or target strand, and would be particularly suitable for isolating related sequences.

Of course, for some applications, for example, where one desires to prepare mutants employing a mutant primer strand hybridized to an underlying template, less stringent (reduced stringency) hybridization conditions will typically be needed in order to allow formation of the heteroduplex. In these circumstances, one may desire to employ salt conditions such as those of from about 0.15 M to about 0.9 M salt, at temperatures ranging from about 20°C to about 55°C. Cross-hybridizing species can thereby be readily identified as positively hybridizing signals with respect to control hybridizations. In any case, it is generally appreciated that conditions can be rendered more stringent by the addition of increasing amounts of formamide, which serves to destabilize the hybrid duplex in the same manner as increased temperature. Thus, hybridization conditions can be readily manipulated, and thus will generally be a method of choice depending on the desired results.

According to another embodiment of the present invention, polynucleotide compositions comprising antisense oligonucleotides are provided. Antisense oligonucleotides have been demonstrated to be effective and targeted inhibitors of protein synthesis, and, consequently, provide a therapeutic approach by which a disease can be treated by inhibiting the synthesis of proteins that contribute to the disease. The efficacy of antisense oligonucleotides for inhibiting protein synthesis is well established. For example, the synthesis of polygalactauronase and the muscarine type 2 acetylcholine receptor are inhibited by antisense oligonucleotides directed to their respective mRNA sequences (U. S. Patent 5,739,119 and U. S. Patent 5,759,829). Further, examples of antisense inhibition have been demonstrated with the nuclear protein cyclin, the multiple drug resistance gene (MDG1), ICAM-1, E-selectin, STK-1, striatal GABA_{A} receptor and human EGF (Jaskulski et al., Science. 1988 Jun 10;240(4858):1544-6; Vasanthakumar and Ahmed, Cancer Commun. 1989;1(4):225-32; Peris et al., Brain Res Mol Brain Res. 1998 Jun 15;57(2):310-20; U. S. Patent 5,801,154; U.S. Patent 5,789,573; U. S. Patent 5,718,709 and U.S. Patent 5,610,288). Antisense constructs have also been described that inhibit and can be used to treat a variety of abnormal cellular proliferations, e.g. cancer (U. S. Patent 5,747,470; U. S. Patent 5,591,317 and U. S. Patent 5,783,683).

Therefore, in certain embodiments, the present invention provides oligonucleotide sequences that comprise all, or a portion of, any sequence that is capable of specifically binding to polynucleotide sequence described herein, or a complement thereof. In one embodiment, the antisense oligonucleotides comprise DNA or derivatives thereof. In another embodiment, the oligonucleotides comprise RNA or derivatives thereof. In a third embodiment, the oligonucleotides are modified DNAs comprising a phosphorothioated modified backbone. In a fourth embodiment, the oligonucleotide sequences comprise peptide nucleic acids or derivatives thereof. In each case, preferred compositions comprise a sequence region that is complementary, and more preferably substantially-complementary, and even more preferably, completely complementary to one or more portions of polynucleotides disclosed herein. Selection of antisense compositions specific for a given gene sequence is based upon analysis of the chosen target sequence and determination of secondary structure, Tₘ, binding energy, and relative stability. Antisense compositions may be selected based upon their relative inability to form dimers, hairpins, or other secondary structures that would reduce or prohibit specific binding to the target mRNA in a host cell. Highly preferred target regions of the mRNA, are those which are at or near the AUG translation initiation codon, and those sequences which are substantially complementary to 5' regions of the mRNA. These secondary structure analyses and target site selection considerations can be performed, for example, using v.4 of the OLIGO primer analysis software and/or the BLASTN 2.0.5 algorithm software (Altschul et al., Nucleic Acids Res. 1997 Sep 1;25(17):3389-402).

The use of an antisense delivery method employing a short peptide vector, termed MPG (27 residues), is also contemplated. The MPG peptide contains a hydrophobic domain derived from the fusion sequence of HIV gp41 and a hydrophilic domain from the nuclear localization sequence of SV40 T-antigen (Morris et al., Nucleic Acids Res. 1997 Jul 15;25(14):2730-6). It has been demonstrated that several molecules of the MPG peptide coat the antisense oligonucleotides and can be delivered into cultured mammalian cells in less than 1 hour with relatively high efficiency (90%). Further, the interaction with MPG strongly increases both the stability of the oligonucleotide to nuclease and the ability to cross the plasma membrane.

According to another embodiment of the invention, the polynucleotide compositions described herein are used in the design and preparation of ribozyme molecules for inhibiting expression of the tumor polypeptides and proteins of the present invention in tumor cells. Ribozymes are RNA-protein complexes that cleave nucleic acids in a site-specific fashion. Ribozymes have specific catalytic domains that possess endonuclease activity (Kim and Cech, Proc Nat'l Acad Sci U S A. 1987 Dec;84(24):8788-92; Forster and Symons, Cell. 1987 Apr 24;49(2):211-20). For example, a large number of ribozymes accelerate phosphoester transfer reactions with a high degree of specificity, often cleaving only one of several phosphoesters in an oligonucleotide substrate (Cech et al., Cell. 1981 Dec;27(3 Pt 2):487-96; Michel and Westhof, J Mol Biol. 1990 Dec 5;216(3):585-610; Reinhold-Hurek and Shub, Nature. 1992 May 14;357(6374):173-6). This specificity has been attributed to the requirement that the substrate bind via specific base-pairing interactions to the internal guide sequence ("IGS") of the ribozyme prior to chemical reaction.

Six basic varieties of naturally-occurring enzymatic RNAs are known presently. Each can catalyze the hydrolysis of RNA phosphodiester bonds *in trans* (and thus can cleave other RNA molecules) under physiological conditions. In general, enzymatic nucleic acids act by first binding to a target RNA. Such binding occurs through the target binding portion of a enzymatic nucleic acid which is held in close proximity to an enzymatic portion of the molecule that acts to cleave the target RNA. Thus, the enzymatic nucleic acid first recognizes and then binds a target RNA through complementary base-pairing, and once bound to the correct site, acts enzymatically to cut the target RNA. Strategic cleavage of such a target RNA will destroy its ability to direct synthesis of an encoded protein. After an enzymatic nucleic acid has bound and cleaved its RNA target, it is released from that RNA to search for another target and can repeatedly bind and cleave new targets.

The enzymatic nature of a ribozyme is advantageous over many technologies, such as antisense technology (where a nucleic acid molecule simply binds to a nucleic acid target to block its translation) since the concentration of ribozyme necessary to affect a therapeutic treatment is lower than that of an antisense oligonucleotide. This advantage reflects the ability of the ribozyme to act enzymatically. Thus, a single ribozyme molecule is able to cleave many molecules of target RNA. In addition, the ribozyme is a highly specific inhibitor, with the specificity of inhibition depending not only on the base pairing mechanism of binding to the target RNA, but also on the mechanism of target RNA cleavage. Single mismatches, or base-substitutions, near the site of cleavage can completely eliminate catalytic activity of a ribozyme. Similar mismatches in antisense molecules do not prevent their action (Woolf et al., Proc Nat'l Acad Sci U S A. 1992 Aug 15;89(16):7305-9). Thus, the specificity of action of a ribozyme is greater than that of an antisense oligonucleotide binding the same RNA site.

The enzymatic nucleic acid molecule may be formed in a hammerhead, hairpin, a hepatitis δ virus, group I intron or RNaseP RNA (in association with an RNA guide sequence) or Neurospora VS RNA motif. Examples of hammerhead motifs are described by Rossi et al. Nucleic Acids Res. 1992 Sep 11;20(17):4559-65. Examples of hairpin motifs are described by Hampel *et al.* (Eur. Pat. Appl. Publ. No. EP 0360257), Hampel and Tritz, Biochemistry 1989 Jun 13;28(12):4929-33; Hampel et al., Nucleic Acids Res. 1990 Jan 25;18(2):299-304 and U. S. Patent 5,631,359. An example of the hepatitis δ virus motif is described by Perrotta and Been, Biochemistry. 1992 Dec 1;31(47):11843-52; an example of the RNaseP motif is described by Guerrier-Takada et al., Cell. 1983 Dec;35(3 Pt 2):849-57; Neurospora VS RNA ribozyme motif is described by Collins (Saville and Collins, Cell. 1990 May 18;61(4):685-96; Saville and Collins, Proc Nat'l Acad Sci U S A. 1991 Oct 1;88(19):8826-30; Collins and Olive, Biochemistry. 1993 Mar 23;32(11):2795-9); and an example of the Group I intron is described in (U. S. Patent 4,987,071). All that is important in an enzymatic nucleic acid molecule of this invention is that it has a specific substrate binding site which is complementary to one or more of the target gene RNA regions, and that it have nucleotide sequences within or surrounding that substrate binding site which impart an RNA cleaving activity to the molecule. Thus the ribozyme constructs need not be limited to specific motifs mentioned herein.

Ribozymes may be designed as described in Int. Pat. Appl. Publ. No. WO 93/23569 and Int. Pat. Appl. Publ. No. WO 94/02595, each specifically incorporated herein by reference) and synthesized to be tested *in vitro* and *in vivo,* as described. Such ribozymes can also be optimized for delivery. While specific examples are provided, those in the art will recognize that equivalent RNA targets in other species can be utilized when necessary.

Ribozyme activity can be optimized by altering the length of the ribozyme binding arms, or chemically synthesizing ribozymes with modifications that prevent their degradation by serum ribonucleases (see e.g., Int. Pat. Appl. Publ. No. WO 92/07065; Int. Pat. Appl. Publ. No. WO 93/15187; Int. Pat. Appl. Publ. No. WO 91/03162; Eur. Pat. Appl. Publ. No. 92110298.4; U. S. Patent 5,334,711; and Int. Pat. Appl. Publ. No. WO 94/13688, which describe various chemical modifications that can be made to the sugar moieties of enzymatic RNA molecules), modifications which enhance their efficacy in cells, and removal of stem II bases to shorten RNA synthesis times and reduce chemical requirements.

Sullivan *et al.* (Int. Pat. Appl. Publ. No. WO 94/02595) describes the general methods for delivery of enzymatic RNA molecules. Ribozymes may be administered to cells by a variety of methods known to those familiar to the art, including, but not restricted to, encapsulation in liposomes, by iontophoresis, or by incorporation into other vehicles, such as hydrogels, cyclodextrins, biodegradable nanocapsules, and bioadhesive microspheres. For some indications, ribozymes may be directly delivered ex *vivo* to cells or tissues with or without the aforementioned vehicles. Alternatively, the RNA/vehicle combination may be locally delivered by direct inhalation, by direct injection or by use of a catheter, infusion pump or stent. Other routes of delivery include, but are not limited to, intravascular, intramuscular, subcutaneous or joint injection, aerosol inhalation, oral (tablet or pill form), topical, systemic, ocular, intraperitoneal and/or intrathecal delivery. More detailed descriptions of ribozyme delivery and administration are provided in Int. Pat. Appl. Publ. No. WO 94/02595 and Int. Pat. Appl. Publ. No. WO 93/23569, each specifically incorporated herein by reference.

Another means of accumulating high concentrations of a ribozyme(s) within cells is to incorporate the ribozyme-encoding sequences into a DNA expression vector. Transcription of the ribozyme sequences are driven from a promoter for eukaryotic RNA polymerase I (pol I), RNA polymerase II (pol II), or RNA polymerase III (pol III). Transcripts from pol II or pol III promoters will be expressed at high levels in all cells; the levels of a given pol II promoter in a given cell type will depend on the nature of the gene regulatory sequences (enhancers, silencers, *etc.)* present nearby. Prokaryotic RNA polymerase promoters may also be used, providing that the prokaryotic RNA polymerase enzyme is expressed in the appropriate cells Ribozymes expressed from such promoters have been shown to function in mammalian cells. Such transcription units can be incorporated into a variety of vectors for introduction into mammalian cells, including but not restricted to, plasmid DNA vectors, viral DNA vectors (such as adenovirus or adeno-associated vectors), or viral RNA vectors (such as retroviral, semliki forest virus, sindbis virus vectors).

In another embodiment of the invention, peptide nucleic acids (PNAs) compositions are provided. PNA is a DNA mimic in which the nucleobases are attached to a pseudopeptide backbone (Good and Nielsen, Antisense Nucleic Acid Drug Dev. 1997 7(4) 431-37). PNA is able to be utilized in a number methods that traditionally have used RNA or DNA. Often PNA sequences perform better in techniques than the corresponding RNA or DNA sequences and have utilities that are not inherent to RNA or DNA. A review of PNA including methods of making, characteristics of, and methods of using, is provided by Corey (Trends Biotechnol 1997 Jun;15(6):224-9). As such, in certain embodiments, one may prepare PNA sequences that are complementary to one or more portions of the ACE mRNA sequence, and such PNA compositions may be used to regulate, alter, decrease, or reduce the translation of ACE-specific mRNA, and thereby alter the level of ACE activity in a host cell to which such PNA compositions have been administered.

PNAs have 2-aminoethyl-glycine linkages replacing the normal phosphodiester backbone of DNA (Nielsen et al., Science 1991 Dec 6;254(5037):1497-500; Hanvey et al., Science. 1992 Nov 27;258(5087):1481-5; Hyrup and Nielsen, Bioorg Med Chem. 1996 Jan;4(1):5-23). This chemistry has three important consequences: firstly, in contrast to DNA or phosphorothioate oligonucleotides, PNAs are neutral molecules; secondly, PNAs are achiral, which avoids the need to develop a stereoselective synthesis; and thirdly, PNA synthesis uses standard Boc or Fmoc protocols for solid-phase peptide synthesis, although other methods, including a modified Merrifield method, have been used.

PNA monomers or ready-made oligomers are commercially available from PerSeptive Biosystems (Framingham, MA). PNA syntheses by either Boc or Fmoc protocols are straightforward using manual or automated protocols (Norton et al., Bioorg Med Chem. 1995 Apr;3(4):437-45). The manual protocol lends itself to the production of chemically modified PNAs or the simultaneous synthesis of families of closely related PNAs.

As with peptide synthesis, the success of a particular PNA synthesis will depend on the properties of the chosen sequence. For example, while in theory PNAs can incorporate any combination of nucleotide bases, the presence of adjacent purines can lead to deletions of one or more residues in the product. In expectation of this difficulty, it is suggested that, in producing PNAs with adjacent purines, one should repeat the coupling of residues likely to be added inefficiently. This should be followed by the purification of PNAs by reverse-phase high-pressure liquid chromatography, providing yields and purity of product similar to those observed during the synthesis of peptides.

Modifications of PNAs for a given application may be accomplished by coupling amino acids during solid-phase synthesis or by attaching compounds that contain a carboxylic acid group to the exposed N-terminal amine. Alternatively, PNAs can be modified after synthesis by coupling to an introduced lysine or cysteine. The ease with which PNAs can be modified facilitates optimization for better solubility or for specific functional requirements. Once synthesized, the identity of PNAs and their derivatives can be confirmed by mass spectrometry. Several studies have made and utilized modifications of PNAs (for example, Norton et al., Bioorg Med Chem. 1995 Apr;3(4):437-45; Petersen et al., J Pept Sci. 1995 May-Jun;1(3):175-83; Orum et al., Biotechniques. 1995 Sep;19(3):472-80; Footer et al., Biochemistry. 1996 Aug 20;35(33):10673-9; Griffith et al., Nucleic Acids Res. 1995 Aug 11;23(15):3003-8; Pardridge et al., Proc Nat'l Acad Sci U S A. 1995 Jun 6;92(12):5592-6; Boffa et al., Proc Nat'l Acad Sci U S A. 1995 Mar 14;92(6):1901-5; Gambacorti-Passerini et al., Blood. 1996 Aug 15;88(4):1411-7; Armitage et al., Proc Nat'l Acad Sci U S A. 1997 Nov 11;94(23):12320-5; Seeger et al., Biotechniques. 1997 Sep;23(3):512-7). U.S. Patent No. 5,700,922 discusses PNA-DNA-PNA chimeric molecules and their uses in diagnostics, modulating protein in organisms, and treatment of conditions susceptible to therapeutics.

Methods of characterizing the antisense binding properties of PNAs are discussed in Rose (Anal Chem. 1993 Dec 15;65(24):3545-9) and Jensen et al. (Biochemistry. 1997 Apr 22;36(16):5072-7). Rose uses capillary gel electrophoresis to determine binding of PNAs to their complementary oligonucleotide, measuring the relative binding kinetics and stoichiometry. Similar types of measurements were made by Jensen *et al.* using BIAcore^{™} technology.

Other applications of PNAs that have been described and will be apparent to the skilled artisan include use in DNA strand invasion, antisense inhibition, mutational analysis, enhancers of transcription, nucleic acid purification, isolation of transcriptionally active genes, blocking of transcription factor binding, genome cleavage, biosensors, *in situ* hybridization, and the like.

### Polynucleotide Identification, Characterization and Expression

Polynucleotide compositions of the present invention may be identified, prepared and/or manipulated using any of a variety of well established techniques (see generally, Sambrook et al., Molecular Cloning: A Laboratory Manual, Cold Spring Harbor Laboratories, Cold Spring Harbor, NY, 1989, and other like references). For example, a polynucleotide may be identified, as described in more detail below, by screening a microarray of cDNAs for tumor-associated expression (*i.e.,* expression that is at least two fold greater in a tumor than in normal tissue, as determined using a representative assay provided herein). Such screens may be performed, for example, using the microarray technology of Affymetrix, Inc. (Santa Clara, CA) according to the manufacturer's instructions (and essentially as described by Schena et al., Proc. Nat'l Acad. Sci. USA 93:10614-10619, 1996 and Heller et al., Proc. Nat'l Acad. Sci. USA 94:2150-2155, 1997). Alternatively, polynucleotides may be amplified from cDNA prepared from cells expressing the proteins described herein, such as tumor cells.

Many template dependent processes are available to amplify a target sequences of interest present in a sample. One of the best known amplification methods is the polymerase chain reaction (PCR^{™}) which is described in detail in U.S. Patent Nos. 4,683,195, 4,683,202 and 4,800,159, each of which is incorporated herein by reference in its entirety. Briefly, in PCR^{™}, two primer sequences are prepared which are complementary to regions on opposite complementary strands of the target sequence. An excess of deoxynucleoside triphosphates is added to a reaction mixture along with a DNA polymerase *(e.g., Taq* polymerase). If the target sequence is present in a sample, the primers will bind to the target and the polymerase will cause the primers to be extended along the target sequence by adding on nucleotides. By raising and lowering the temperature of the reaction mixture, the extended primers will dissociate from the target to form reaction products, excess primers will bind to the target and to the reaction product and the process is repeated. Preferably reverse transcription and PCR^{™} amplification procedure may be performed in order to quantify the amount of mRNA amplified. Polymerase chain reaction methodologies are well known in the art.

Any of a number of other template dependent processes, many of which are variations of the PCR^{™} amplification technique, are readily known and available in the art. Illustratively, some such methods include the ligase chain reaction (referred to as LCR), described, for example, in Eur. Pat. Appl. Publ. No. 320,308 and U.S. Patent No. 4,883,750; Qbeta Replicase, described in PCT Intl. Pat. Appl. Publ. No. PCT/US87/00880; Strand Displacement Amplification (SDA) and Repair Chain Reaction (RCR). Still other amplification methods are described in Great Britain Pat. Appl. No. 2 202 328, and in PCT Intl. Pat. Appl. Publ. No. PCT/US89/01025. Other nucleic acid amplification procedures include transcription-based amplification systems (TAS) (PCT Intl. Pat. Appl. Publ. No. WO 88/10315), including nucleic acid sequence based amplification (NASBA) and 3SR. Eur. Pat. Appl. Publ. No. 329,822 describes a nucleic acid amplification process involving cyclically synthesizing single-stranded RNA ("ssRNA"), ssDNA, and double-stranded DNA (dsDNA). PCT Intl. Pat. Appl. Publ. No. WO 89/06700 describes a nucleic acid sequence amplification scheme based on the hybridization of a promoter/primer sequence to a target single-stranded DNA ("ssDNA") followed by transcription of many RNA copies of the sequence. Other amplification methods such as "RACE" (Frohman, 1990), and "one-sided PCR" (Ohara, 1989) are also well-known to those of skill in the art.

An amplified portion of a polynucleotide of the present invention may be used to isolate a full length gene from a suitable library (e.g., a tumor cDNA library) using well known techniques. Within such techniques, a library (cDNA or genomic) is screened using one or more polynucleotide probes or primers suitable for amplification. Preferably, a library is size-selected to include larger molecules. Random primed libraries may also be preferred for identifying 5' and upstream regions of genes. Genomic libraries are preferred for obtaining introns and extending 5' sequences.

For hybridization techniques, a partial sequence may be labeled (e.g., by nick-translation or end-labeling with ³²P) using well known techniques. A bacterial or bacteriophage library is then generally screened by hybridizing filters containing denatured bacterial colonies (or lawns containing phage plaques) with the labeled probe (see Sambrook et al., Molecular Cloning: A Laboratory Manual, Cold Spring Harbor Laboratories, Cold Spring Harbor, NY, 1989). Hybridizing colonies or plaques are selected and expanded, and the DNA is isolated for further analysis. cDNA clones may be analyzed to determine the amount of additional sequence by, for example, PCR using a primer from the partial sequence and a primer from the vector. Restriction maps and partial sequences may be generated to identify one or more overlapping clones. The complete sequence may then be determined using standard techniques, which may involve generating a series of deletion clones. The resulting overlapping sequences can then assembled into a single contiguous sequence. A full length cDNA molecule can be generated by ligating suitable fragments, using well known techniques.

Alternatively, amplification techniques, such as those described above, can be useful for obtaining a full length coding sequence from a partial cDNA sequence. One such amplification technique is inverse PCR (see Triglia et al., Nucl. Acids Res. 16:8186, 1988), which uses restriction enzymes to generate a fragment in the known region of the gene. The fragment is then circularized by intramolecular ligation and used as a template for PCR with divergent primers derived from the known region. Within an alternative approach, sequences adjacent to a partial sequence may be retrieved by amplification with a primer to a linker sequence and a primer specific to a known region. The amplified sequences are typically subjected to a second round of amplification with the same linker primer and a second primer specific to the known region. A variation on this procedure, which employs two primers that initiate extension in opposite directions from the known sequence, is described in WO 96/38591. Another such technique is known as "rapid amplification of cDNA ends" or RACE. This technique involves the use of an internal primer and an external primer, which hybridizes to a polyA region or vector sequence, to identify sequences that are 5' and 3' of a known sequence. Additional techniques include capture PCR (Lagerstrom et al., PCR Methods Applic. 1:111-19, 1991) and walking PCR (Parker et al., Nucl. Acids. Res. 19:3055-60, 1991). Other methods employing amplification may also be employed to obtain a full length cDNA sequence.

In certain instances, it is possible to obtain a full length cDNA sequence by analysis of sequences provided in an expressed sequence tag (EST) database, such as that available from GenBank. Searches for overlapping ESTs may generally be performed using well known programs (e.g., NCBI BLAST searches), and such ESTs may be used to generate a contiguous full length sequence. Full length DNA sequences may also be obtained by analysis of genomic fragments.

In other embodiments of the invention, polynucleotide sequences or fragments thereof which encode polypeptides of the invention, or fusion proteins or functional equivalents thereof, may be used in recombinant DNA molecules to direct expression of a polypeptide in appropriate host cells. Due to the inherent degeneracy of the genetic code, other DNA sequences that encode substantially the same or a functionally equivalent amino acid sequence may be produced and these sequences may be used to clone and express a given polypeptide.

As will be understood by those of skill in the art, it may be advantageous in some instances to produce polypeptide-encoding nucleotide sequences possessing non-naturally occurring codons. For example, codons preferred by a particular prokaryotic or eukaryotic host can be selected to increase the rate of protein expression or to produce a recombinant RNA transcript having desirable properties, such as a half-life which is longer than that of a transcript generated from the naturally occurring sequence.

Moreover, the polynucleotide sequences of the present invention can be engineered using methods generally known in the art in order to alter polypeptide encoding sequences for a variety of reasons, including but not limited to, alterations which modify the cloning, processing, and/or expression of the gene product. For example, DNA shuffling by random fragmentation and PCR reassembly of gene fragments and synthetic oligonucleotides may be used to engineer the nucleotide sequences. In addition, site-directed mutagenesis may be used to insert new restriction sites, alter glycosylation patterns, change codon preference, produce splice variants, or introduce mutations, and so forth.

In another embodiment of the invention, natural, modified, or recombinant nucleic acid sequences may be ligated to a heterologous sequence to encode a fusion protein. For example, to screen peptide libraries for inhibitors of polypeptide activity, it may be useful to encode a chimeric protein that can be recognized by a commercially available antibody. A fusion protein may also be engineered to contain a cleavage site located between the polypeptide-encoding sequence and the heterologous protein sequence, so that the polypeptide may be cleaved and purified away from the heterologous moiety.

Sequences encoding a desired polypeptide may be synthesized, in whole or in part, using chemical methods well known in the art (see Caruthers, M. H. et al. (1980) Nucl. Acids Res. Symp. Ser. 215-223, Horn, T. et al. (1980) Nucl. Acids Res. Symp. Ser. 225-232). Alternatively, the protein itself may be produced using chemical methods to synthesize the amino acid sequence of a polypeptide, or a portion thereof. For example, peptide synthesis can be performed using various solid-phase techniques (Roberge, J. Y. et al. (1995) Science 269:202-204) and automated synthesis may be achieved, for example, using the ABI 431A Peptide Synthesizer (Perkin Elmer, Palo Alto, CA).

A newly synthesized peptide may be substantially purified by preparative high performance liquid chromatography (e.g., Creighton, T. (1983) Proteins, Structures and Molecular Principles, WH Freeman and Co., New York, N.Y.) or other comparable techniques available in the art. The composition of the synthetic peptides may be confirmed by amino acid analysis or sequencing (e.g., the Edman degradation procedure). Additionally, the amino acid sequence of a polypeptide, or any part thereof, may be altered during direct synthesis and/or combined using chemical methods with sequences from other proteins, or any part thereof, to produce a variant polypeptide.

In order to express a desired polypeptide, the nucleotide sequences encoding the polypeptide, or functional equivalents, may be inserted into appropriate expression vector, *i.e.,* a vector which contains the necessary elements for the transcription and translation of the inserted coding sequence. Methods which are well known to those skilled in the art may be used to construct expression vectors containing sequences encoding a polypeptide of interest and appropriate transcriptional and translational control elements. These methods include *in vitro* recombinant DNA techniques, synthetic techniques, and *in vivo* genetic recombination. Such techniques are described, for example, in Sambrook, J. et al. (1989) Molecular Cloning, A Laboratory Manual, Cold Spring Harbor Press, Plainview, N.Y., and Ausubel, F. M. et al. (1989) Current Protocols in Molecular Biology, John Wiley & Sons, New York. N.Y.

A variety of expression vector/host systems may be utilized to contain and express polynucleotide sequences. These include, but are not limited to, microorganisms such as bacteria transformed with recombinant bacteriophage, plasmid, or cosmid DNA expression vectors; yeast transformed with yeast expression vectors; insect cell systems infected with virus expression vectors (e.g., baculovirus); plant cell systems transformed with virus expression vectors (*e.g.,* cauliflower mosaic virus, CaMV; tobacco mosaic virus, TMV) or with bacterial expression vectors (*e.g.,* Ti or pBR322 plasmids); or animal cell systems.

The "control elements" or "regulatory sequences" present in an expression vector are those non-translated regions of the vector--enhancers, promoters, 5' and 3' untranslated regions--which interact with host cellular proteins to carry out transcription and translation. Such elements may vary in their strength and specificity. Depending on the vector system and host utilized, any number of suitable transcription and translation elements, including constitutive and inducible promoters, may be used. For example, when cloning in bacterial systems, inducible promoters such as the hybrid lacZ promoter of the PBLUESCRIPT phagemid (Stratagene, La Jolla, Calif.) or PSPORT1 plasmid (Gibco BRL, Gaithersburg, MD) and the like may be used. In mammalian cell systems, promoters from mammalian genes or from mammalian viruses are generally preferred. If it is necessary to generate a cell line that contains multiple copies of the sequence encoding a polypeptide, vectors based on SV40 or EBV may be advantageously used with an appropriate selectable marker.

In bacterial systems, any of a number of expression vectors may be selected depending upon the use intended for the expressed polypeptide. For example, when large quantities are needed, for example for the induction of antibodies, vectors which direct high level expression of fusion proteins that are readily purified may be used. Such vectors include, but are not limited to, the multifunctional *E*. *coli* cloning and expression vectors such as BLUESCRIPT (Stratagene), in which the sequence encoding the polypeptide of interest may be ligated into the vector in frame with sequences for the amino-terminal Met and the subsequent 7 residues of .beta.-galactosidase so that a hybrid protein is produced; pIN vectors (Van Heeke, G. and S. M. Schuster (1989) J. Biol. Chem. 264:5503-5509); and the like. pGEX Vectors (Promega, Madison, Wis.) may also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. Proteins made in such systems may be designed to include heparin, thrombin, or factor XA protease cleavage sites so that the cloned polypeptide of interest can be released from the GST moiety at will.

In the yeast, Saccharomyces cerevisiae, a number of vectors containing constitutive or inducible promoters such as alpha factor, alcohol oxidase, and PGH may be used. For reviews, see Ausubel et al. (supra) and Grant et al. (1987) Methods Enzymol. 153:516-544.

In cases where plant expression vectors are used, the expression of sequences encoding polypeptides may be driven by any of a number of promoters. For example, viral promoters such as the 35S and 19S promoters of CaMV may be used alone or in combination with the omega leader sequence from TMV (Takamatsu, N. (1987) EMBO J. 6:307-311. Alternatively, plant promoters such as the small subunit of RUBISCO or heat shock promoters may be used (Coruzzi, G. et al. (1984) EMBO J. 3:1671-1680; Broglie, R. et al. (1984) Science 224:838-843; and Winter, J. et al. (1991) Results Probl. Cell Differ. 17:85-105). These constructs can be introduced into plant cells by direct DNA transformation or pathogen-mediated transfection. Such techniques are described in a number of generally available reviews (see, for example, Hobbs, S. or Murry, L. E. in McGraw Hill Yearbook of Science and Technology (1992) McGraw Hill, New York, N.Y.; pp. 191-196).

An insect system may also be used to express a polypeptide of interest. For example, in one such system, Autographa californica nuclear polyhedrosis virus (AcNPV) is used as a vector to express foreign genes in Spodoptera frugiperda cells or in Trichoplusia larvae. The sequences encoding the polypeptide may be cloned into a non-essential region of the virus, such as the polyhedrin gene, and placed under control of the polyhedrin promoter. Successful insertion of the polypeptide-encoding sequence will render the polyhedrin gene inactive and produce recombinant virus lacking coat protein. The recombinant viruses may then be used to infect, for example, S. frugiperda cells or Trichoplusia larvae in which the polypeptide of interest may be expressed (Engelhard, E. K. et al. (1994) Proc. Nat'l Acad. Sci. 91 :3224-3227).

In mammalian host cells, a number of viral-based expression systems are generally available. For example, in cases where an adenovirus is used as an expression vector, sequences encoding a polypeptide of interest may be ligated into an adenovirus transcription/translation complex consisting of the late promoter and tripartite leader sequence. Insertion in a non-essential E1 or E3 region of the viral genome may be used to obtain a viable virus which is capable of expressing the polypeptide in infected host cells (Logan, J. and Shenk, T. (1984) Proc. Nat'l Acad. Sci. 81:3655-3659). In addition, transcription enhancers, such as the Rous sarcoma virus (RSV) enhancer, may be used to increase expression in mammalian host cells.

Specific initiation signals may also be used to achieve more efficient translation of sequences encoding a polypeptide of interest. Such signals include the ATG initiation codon and adjacent sequences. In cases where sequences encoding the polypeptide, its initiation codon, and upstream sequences are inserted into the appropriate expression vector, no additional transcriptional or translational control signals may be needed. However, in cases where only coding sequence, or a portion thereof, is inserted, exogenous translational control signals including the ATG initiation codon should be provided. Furthermore, the initiation codon should be in the correct reading frame to ensure translation of the entire insert. Exogenous translational elements and initiation codons may be of various origins, both natural and synthetic. The efficiency of expression may be enhanced by the inclusion of enhancers which are appropriate for the particular cell system which is used, such as those described in the literature (Scharf, D. et al. (1994) Results Probl. Cell Differ. 20:125-162).

In addition, a host cell strain may be chosen for its ability to modulate the expression of the inserted sequences or to process the expressed protein in the desired fashion. Such modifications of the polypeptide include, but are not limited to, acetylation, carboxylation. glycosylation, phosphorylation, lipidation, and acylation. Post-translational processing which cleaves a "prepro" form of the protein may also be used to facilitate correct insertion, folding and/or function. Different host cells such as CHO, COS, HeLa, MDCK, HEK293, and W138, which have specific cellular machinery and characteristic mechanisms for such post-translational activities, may be chosen to ensure the correct modification and processing of the foreign protein.

For long-term, high-yield production of recombinant proteins, stable expression is generally preferred. For example, cell lines which stably express a polynucleotide of interest may be transformed using expression vectors which may contain viral origins of replication and/or endogenous expression elements and a selectable marker gene on the same or on a separate vector. Following the introduction of the vector, cells may be allowed to grow for 1-2 days in an enriched media before they are switched to selective media. The purpose of the selectable marker is to confer resistance to selection, and its presence allows growth and recovery of cells which successfully express the introduced sequences. Resistant clones of stably transformed cells may be proliferated using tissue culture techniques appropriate to the cell type.

Any number of selection systems may be used to recover transformed cell lines. These include, but are not limited to, the herpes simplex virus thymidine kinase (Wigler, M. et al. (1977) Cell 11:223-32) and adenine phosphoribosyltransferase (Lowy, I. et al. (1990) Cell 22:817-23) genes which can be employed in tk.sup.- or aprt.sup.- cells, respectively. Also, antimetabolite, antibiotic or herbicide resistance can be used as the basis for selection; for example, dhfr which confers resistance to methotrexate (Wigler, M. et al. (1980) Proc. Nat'l Acad. Sci. 77:3567-70); npt, which confers resistance to the aminoglycosides, neomycin and G-418 (Colbere-Garapin, F. et al (1981) J. Mol. Biol. 150:1-14*);* and als or pat, which confer resistance to chlorsulfuron and phosphinotricin acetyltransferase, respectively (Murry, *supra).* Additional selectable genes have been described, for example, trpB, which allows cells to utilize indole in place of tryptophan, or hisD, which allows cells to utilize histinol in place of histidine (Hartman, S. C. and R. C. Mulligan (1988) Proc. Nat'l Acad. Sci. 85:8047-51). The use of visible markers has gained popularity with such markers as anthocyanins, beta-glucuronidase and its substrate GUS, and luciferase and its substrate luciferin, being widely used not only to identify transformants, but also to quantify the amount of transient or stable protein expression attributable to a specific vector system (Rhodes, C. A. et al. (1995) Methods Mol. Biol. 55:121-131).

Although the presence/absence of marker gene expression suggests that the gene of interest is also present, its presence and expression may need to be confirmed. For example, if the sequence encoding a polypeptide is inserted within a marker gene sequence, recombinant cells containing sequences can be identified by the absence of marker gene function. Alternatively, a marker gene can be placed in tandem with a polypeptide-encoding sequence under the control of a single promoter. Expression of the marker gene in response to induction or selection usually indicates expression of the tandem gene as well.

Alternatively, host cells that contain and express a desired polynucleotide sequence may be identified by a variety of procedures known to those of skill in the art. These procedures include, but are not limited to, DNA-DNA or DNA-RNA hybridizations and protein bioassay or immunoassay techniques which include, for example, membrane, solution, or chip based technologies for the detection and/or quantification of nucleic acid or protein.

A variety of protocols for detecting and measuring the expression of polynucleotide-encoded products, using either polyclonal or monoclonal antibodies specific for the product are known in the art. Examples include enzyme-linked immunosorbent assay (ELISA), radioimmunoassay (RIA), and fluorescence activated cell sorting (FACS). A two-site, monoclonal-based immunoassay utilizing monoclonal antibodies reactive to two non-interfering epitopes on a given polypeptide may be preferred for some applications, but a competitive binding assay may also be employed. These and other assays are described, among other places, in Hampton, R. et al. (1990; Serological Methods, a Laboratory Manual, APS Press, St Paul. Minn.) and Maddox, D. E. et al. (1983; J. Exp. Med. 158:1211-1216).

A wide variety of labels and conjugation techniques are known by those skilled in the art and may be used in various nucleic acid and amino acid assays. Means for producing labeled hybridization or PCR probes for detecting sequences related to polynucleotides include oligolabeling, nick translation, end-labeling or PCR amplification using a labeled nucleotide. Alternatively, the sequences, or any portions thereof may be cloned into a vector for the production of an mRNA probe. Such vectors are known in the art, are commercially available, and may be used to synthesize RNA probes in vitro by addition of an appropriate RNA polymerase such as T7, T3, or SP6 and labeled nucleotides. These procedures may be conducted using a variety of commercially available kits. Suitable reporter molecules or labels, which may be used include radionuclides, enzymes, fluorescent, chemiluminescent, or chromogenic agents as well as substrates, cofactors, inhibitors, magnetic particles, and the like.

Host cells transformed with a polynucleotide sequence of interest may be cultured under conditions suitable for the expression and recovery of the protein from cell culture. The protein produced by a recombinant cell may be secreted or contained intracellularly depending on the sequence and/or the vector used. As will be understood by those of skill in the art, expression vectors containing polynucleotides of the invention may be designed to contain signal sequences which direct secretion of the encoded polypeptide through a prokaryotic or eukaryotic cell membrane. Other recombinant constructions may be used to join sequences encoding a polypeptide of interest to nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). The inclusion of cleavable linker sequences such as those specific for Factor XA or enterokinase (Invitrogen. San Diego, Calif.) between the purification domain and the encoded polypeptide may be used to facilitate purification. One such expression vector provides for expression of a fusion protein containing a polypeptide of interest and a nucleic acid encoding 6 histidine residues preceding a thioredoxin or an enterokinase cleavage site. The histidine residues facilitate purification on IMIAC (immobilized metal ion affinity chromatography) as described in Porath, J. et al. (1992, Prot. Exp. Purif. 3:263-281) while the enterokinase cleavage site provides a means for purifying the desired polypeptide from the fusion protein. A discussion of vectors which contain fusion proteins is provided in Kroll, D. J. et al. (1993; DNA Cell Biol. 12:441-453).

In addition to recombinant production methods, polypeptides of the invention, and fragments thereof, may be produced by direct peptide synthesis using solid-phase techniques (Merrifield J. (1963) J. Am. Chem. Soc. 85:2149-2154). Protein synthesis may be performed using manual techniques or by automation. Automated synthesis may be achieved, for example, using Applied Biosystems 431A Peptide Synthesizer (Perkin Elmer). Alternatively, various fragments may be chemically synthesized separately and combined using chemical methods to produce the full length molecule.

### Antibody Compositions, Fragments Thereof and Other Binding Agents

According to another aspect, the present invention further provides binding agents, such as antibodies and antigen-binding fragments thereof, that exhibit immunological binding to a tumor polypeptide disclosed herein, or to a portion, variant or derivative thereof. An antibody, or antigen-binding fragment thereof, is said to "specifically bind," "immunogically bind," and/or is "immunologically reactive" to a polypeptide of the invention if it reacts at a detectable level (within, for example, an ELISA assay) with the polypeptide, and does not react detectably with unrelated polypeptides under similar conditions.

Immunological binding, as used in this context, generally refers to the non-covalent interactions of the type which occur between an immunoglobulin molecule and an antigen for which the immunoglobulin is specific. The strength, or affinity of immunological binding interactions can be expressed in terms of the dissociation constant (K_{d}) of the interaction, wherein a smaller K_{d} represents a greater affinity. Immunological binding properties of selected polypeptides can be quantified using methods well known in the art. One such method entails measuring the rates of antigen-binding site/antigen complex formation and dissociation, wherein those rates depend on the concentrations of the complex partners, the affinity of the interaction, and on geometric parameters that equally influence the rate in both directions. Thus, both the "on rate constant" (Kₒₙ) and the "off rate constant" (K_{off}) can be determined by calculation of the concentrations and the actual rates of association and dissociation. The ratio of K_{off} /Kₒₙ enables cancellation of all parameters not related to affinity, and is thus equal to the dissociation constant K_{d}. See, generally, Davies et al. (1990) Annual Rev. Biochem. 59:439-473.

An "antigen-binding site," or "binding portion" of an antibody refers to the part of the immunoglobulin molecule that participates in antigen binding. The antigen binding site is formed by amino acid residues of the N-terminal variable ("V") regions of the heavy ("H") and light ("L") chains. Three highly divergent stretches within the V regions of the heavy and light chains are referred to as "hypervariable regions" which are interposed between more conserved flanking stretches known as "framework regions," or "FRs". Thus the term "FR" refers to amino acid sequences which are naturally found between and adjacent to hypervariable regions in immunoglobulins. In an antibody molecule, the three hypervariable regions of a light chain and the three hypervariable regions of a heavy chain are disposed relative to each other in three dimensional space to form an antigen-binding surface. The antigen-binding surface is complementary to the three-dimensional surface of a bound antigen, and the three hypervariable regions of each of the heavy and light chains are referred to as "complementarity-determining regions," or "CDRs."

Binding agents may be further capable of differentiating between patients with and without a cancer, such as prostate cancer, using the representative assays provided herein. For example, antibodies or other binding agents that bind to a tumor protein will preferably generate a signal indicating the presence of a cancer in at least about 20% of patients with the disease, more preferably at least about 30% of patients. Alternatively, or in addition, the antibody will generate a negative signal indicating the absence of the disease in at least about 90% of individuals without the cancer. To determine whether a binding agent satisfies this requirement, biological samples (e.g., blood, sera, sputum, urine and/or tumor biopsies) from patients with and without a cancer (as determined using standard clinical tests) may be assayed as described herein for the presence of polypeptides that bind to the binding agent. Preferably, a statistically significant number of samples with and without the disease will be assayed. Each binding agent should satisfy the above criteria; however, those of ordinary skill in the art will recognize that binding agents may be used in combination to improve sensitivity.

Any agent that satisfies the above requirements may be a binding agent. For example, a binding agent may be a ribosome, with or without a peptide component, an RNA molecule or a polypeptide. In a preferred embodiment, a binding agent is an antibody or an antigen-binding fragment thereof. Antibodies may be prepared by any of a variety of techniques known to those of ordinary skill in the art. *See, e.g.,* Harlow and Lane, Antibodies: A Laboratory Manual, Cold Spring Harbor Laboratory, 1988. In general, antibodies can be produced by cell culture techniques, including the generation of monoclonal antibodies as described herein, or via transfection of antibody genes into suitable bacterial or mammalian cell hosts, in order to allow for the production of recombinant antibodies. In one technique, an immunogen comprising the polypeptide is initially injected into any of a wide variety of mammals (e.g., mice, rats, rabbits, sheep or goats). In this step, the polypeptides of this invention may serve as the immunogen without modification. Alternatively, particularly for relatively short polypeptides, a superior immune response may be elicited if the polypeptide is joined to a carrier protein, such as bovine serum albumin or keyhole limpet hemocyanin. The immunogen is injected into the animal host, preferably according to a predetermined schedule incorporating one or more booster immunizations, and the animals are bled periodically. Polyclonal antibodies specific for the polypeptide may then be purified from such antisera by, for example, affinity chromatography using the polypeptide coupled to a suitable solid support.

Monoclonal antibodies specific for an antigenic polypeptide of interest may be prepared, for example, using the technique of Kohler and Milstein, Eur. J. Immunol. 6:511-519, 1976, and improvements thereto. Briefly, these methods involve the preparation of immortal cell lines capable of producing antibodies having the desired specificity (*i*.*e*., reactivity with the polypeptide of interest). Such cell lines may be produced, for example, from spleen cells obtained from an animal immunized as described above. The spleen cells are then immortalized by, for example, fusion with a myeloma cell fusion partner, preferably one that is syngeneic with the immunized animal. A variety of fusion techniques may be employed. For example, the spleen cells and myeloma cells may be combined with a nonionic detergent for a few minutes and then plated at low density on a selective medium that supports the growth of hybrid cells, but not myeloma cells. A preferred selection technique uses HAT (hypoxanthine, aminopterin, thymidine) selection. After a sufficient time, usually about 1 to 2 weeks, colonies of hybrids are observed. Single colonies are selected and their culture supernatants tested for binding activity against the polypeptide. Hybridomas having high reactivity and specificity are preferred.

Monoclonal antibodies may be isolated from the supernatants of growing hybridoma colonies. In addition, various techniques may be employed to enhance the yield, such as injection of the hybridoma cell line into the peritoneal cavity of a suitable vertebrate host, such as a mouse. Monoclonal antibodies may then be harvested from the ascites fluid or the blood. Contaminants may be removed from the antibodies by conventional techniques, such as chromatography, gel filtration, precipitation, and extraction. The polypeptides of this invention may be used in the purification process in, for example, an affinity chromatography step.

A number of therapeutically useful molecules are known in the art which comprise antigen-binding sites that are capable of exhibiting immunological binding properties of an antibody molecule. The proteolytic enzyme papain preferentially cleaves IgG molecules to yield several fragments, two of which (the "F(ab)" fragments) each comprise a covalent heterodimer that includes an intact antigen-binding site. The enzyme pepsin is able to cleave IgG molecules to provide several fragments, including the "F(ab')₂ " fragment which comprises both antigen-binding sites. An "Fv" fragment can be produced by preferential proteolytic cleavage of an IgM, and on rare occasions IgG or IgA immunoglobulin molecule. Fv fragments are, however, more commonly derived using recombinant techniques known in the art. The Fv fragment includes a non-covalent V_{H}::V_{L} heterodimer including an antigen-binding site which retains much of the antigen recognition and binding capabilities of the native antibody molecule. Inbar et al. (1972) Proc. Nat. Acad. Sci. USA 69:2659-2662; Hochman et al. (1976) Biochem 15:2706-2710; and Ehrlich et al. (1980) Biochem 19:4091-4096.

A single chain Fv ("sFv") polypeptide is a covalently linked V_{H}::V_{L} heterodimer which is expressed from a gene fusion including V_{H}- and V_{L}-encoding genes linked by a peptide-encoding linker. Huston et al. (1988) Proc. Nat. Acad. Sci. USA 85(16):5879-5883. A number of methods have been described to discern chemical structures for converting the naturally aggregated--but chemically separated--light and heavy polypeptide chains from an antibody V region into an sFv molecule which will fold into a three dimensional structure substantially similar to the structure of an antigen-binding site. See, e.g., U.S. Pat. Nos. 5,091,513 and 5,132,405, to Huston et al.; and U.S. Pat. No. 4,946,778, to Ladner et al.

Each of the above-described molecules includes a heavy chain and a light chain CDR set, respectively interposed between a heavy chain and a light chain FR set which provide support to the CDRS and define the spatial relationship of the CDRs relative to each other. As used herein, the term "CDR set" refers to the three hypervariable regions of a heavy or light chain V region. Proceeding from the N-terminus of a heavy or light chain, these regions are denoted as "CDR1," "CDR2," and "CDR3" respectively. An antigen-binding site, therefore, includes six CDRs, comprising the CDR set from each of a heavy and a light chain V region. A polypeptide comprising a single CDR, (e.g., a CDR1, CDR2 or CDR3) is referred to herein as a "molecular recognition unit." Crystallographic analysis of a number of antigen-antibody complexes has demonstrated that the amino acid residues of CDRs form extensive contact with bound antigen, wherein the most extensive antigen contact is with the heavy chain CDR3. Thus, the molecular recognition units are primarily responsible for the specificity of an antigen-binding site.

As used herein, the term "FR set" refers to the four flanking amino acid sequences which frame the CDRs of a CDR set of a heavy or light chain V region. Some FR residues may contact bound antigen; however, FRs are primarily responsible for folding the V region into the antigen-binding site, particularly the FR residues directly adjacent to the CDRS. Within FRs, certain amino residues and certain structural features are very highly conserved. In this regard, all V region sequences contain an internal disulfide loop of around 90 amino acid residues. When the V regions fold into a binding-site, the CDRs are displayed as projecting loop motifs which form an antigen-binding surface. It is generally recognized that there are conserved structural regions of FRs which influence the folded shape of the CDR loops into certain "canonical" structuresregardless of the precise CDR amino acid sequence. Further, certain FR residues are known to participate in non-covalent interdomain contacts which stabilize the interaction of the antibody heavy and light chains.

A number of "humanized" antibody molecules comprising an antigen-binding site derived from a non-human immunoglobulin have been described, including chimeric antibodies having rodent V regions and their associated CDRs fused to human constant domains (Winter et al. (1991) Nature 349:293-299; Lobuglio et al. (1989) Proc. Nat. Acad. Sci. USA 86:4220-4224; Shaw et al. (1987) J Immunol. 138:4534-4538; and Brown et al. (1987) Cancer Res. 47:3577-3583), rodent CDRs grafted into a human supporting FR prior to fusion with an appropriate human antibody constant domain (Riechmann et al. (1988) Nature 332:323-327; Verhoeyen et al. (1988) Science 239:1534-1536; and Jones et al. (1986) Nature 321:522-525), and rodent CDRs supported by recombinantly veneered rodent FRs (European Patent Publication No. 519,596, published Dec. 23, 1992). These "humanized" molecules are designed to minimize unwanted immunological response toward rodent antihuman antibody molecules which limits the duration and effectiveness of therapeutic applications of those moieties in human recipients.

As used herein, the terms "veneered FRs" and "recombinantly veneered FRs" refer to the selective replacement of FR residues from, e.g., a rodent heavy or light chain V region, with human FR residues in order to provide a xenogeneic molecule comprising an antigen-binding site which retains substantially all of the native FR polypeptide folding structure. Veneering techniques are based on the understanding that the ligand binding characteristics of an antigen-binding site are determined primarily by the structure and relative disposition of the heavy and light chain CDR sets within the antigen-binding surface. Davies et al. (1990) Ann. Rev. Biochem. 59:439-473. Thus, antigen binding specificity can be preserved in a humanized antibody only wherein the CDR structures, their interaction with each other, and their interaction with the rest of the V region domains are carefully maintained. By using veneering techniques, exterior (e.g., solvent-accessible) FR residues which are readily encountered by the immune system are selectively replaced with human residues to provide a hybrid molecule that comprises either a weakly immunogenic, or substantially non-immunogenic veneered surface.

The process of veneering makes use of the available sequence data for human antibody variable domains compiled by Kabat et al., in Sequences of Proteins of Immunological Interest, 4th ed., (U.S. Dept. of Health and Human Services, U.S. Government Printing Office, 1987), updates to the Kabat database, and other accessible U.S. and foreign databases (both nucleic acid and protein). Solvent accessibilities of V region amino acids can be deduced from the known three-dimensional structure for human and murine antibody fragments. There are two general steps in veneering a murine antigen-binding site. Initially, the FRs of the variable domains of an antibody molecule of interest are compared with corresponding FR sequences of human variable domains obtained from the above-identified sources. The most homologous human V regions are then compared residue by residue to corresponding murine amino acids. The residues in the murine FR which differ from the human counterpart are replaced by the residues present in the human moiety using recombinant techniques well known in the art. Residue switching is only carried out with moieties which are at least partially exposed (solvent accessible), and care is exercised in the replacement of amino acid residues which may have a significant effect on the tertiary structure of V region domains, such as proline, glycine and charged amino acids.

In this manner, the resultant "veneered" murine antigen-binding sites are thus designed to retain the murine CDR residues, the residues substantially adjacent to the CDRs, the residues identified as buried or mostly buried (solvent inaccessible), the residues believed to participate in non-covalent (e.g., electrostatic and hydrophobic) contacts between heavy and light chain domains, and the residues from conserved structural regions of the FRs which are believed to influence the "canonical" tertiary structures of the CDR loops. These design criteria are then used to prepare recombinant nucleotide sequences which combine the CDRs of both the heavy and light chain of a murine antigen-binding site into human-appearing FRs that can be used to transfect mammalian cells for the expression of recombinant human antibodies which exhibit the antigen specificity of the murine antibody molecule.

In another embodiment of the invention, monoclonal antibodies of the present invention may be coupled to one or more therapeutic agents. Suitable agents in this regard include radionuclides, differentiation inducers, drugs, toxins, and derivatives thereof. Preferred radionuclides include ⁹⁰Y, ¹²³I, ¹²⁵I, ¹³¹I, ¹⁸⁶Re, ¹⁸⁸Re, ²¹¹At, and ²¹²Bi. Preferred drugs include methotrexate, and pyrimidine and purine analogs. Preferred differentiation inducers include phorbol esters and butyric acid. Preferred toxins include ricin, abrin, diptheria toxin, cholera toxin, gelonin, Pseudomonas exotoxin, Shigella toxin, and pokeweed antiviral protein.

A therapeutic agent may be coupled (e.g., covalently bonded) to a suitable monoclonal antibody either directly or indirectly (e.g., via a linker group). A direct reaction between an agent and an antibody is possible when each possesses a substituent capable of reacting with the other. For example, a nucleophilic group, such as an amino or sulfhydryl group, on one may be capable of reacting with a carbonyl-containing group, such as an anhydride or an acid halide, or with an alkyl group containing a good leaving group (e.g., a halide) on the other.

Alternatively, it may be desirable to couple a therapeutic agent and an antibody via a linker group. A linker group can function as a spacer to distance an antibody from an agent in order to avoid interference with binding capabilities. A linker group can also serve to increase the chemical reactivity of a substituent on an agent or an antibody, and thus increase the coupling efficiency. An increase in chemical reactivity may also facilitate the use of agents, or functional groups on agents, which otherwise would not be possible.

It will be evident to those skilled in the art that a variety of bifunctional or polyfunctional reagents, both homo- and hetero-functional (such as those described in the catalog of the Pierce Chemical Co., Rockford, IL), may be employed as the linker group. Coupling may be effected, for example, through amino groups, carboxyl groups, sulfhydryl groups or oxidized carbohydrate residues. There are numerous references describing such methodology, e.g., U.S. Patent No. 4,671,958, to Rodwell et al.

Where a therapeutic agent is more potent when free from the antibody portion of the immunoconjugates of the present invention, it may be desirable to use a linker group which is cleavable during or upon internalization into a cell. A number of different cleavable linker groups have been described. The mechanisms for the intracellular release of an agent from these linker groups include cleavage by reduction of a disulfide bond (e.g., U.S. Patent No. 4,489,710, to Spitler), by irradiation of a photolabile bond (e.g., U.S. Patent No. 4,625,014, to Senter et al.), by hydrolysis of derivatized amino acid side chains (e.g., U.S. Patent No. 4,638,045, to Kohn et al.), by serum complement-mediated hydrolysis (e.g., U.S. Patent No. 4,671,958, to Rodwell et al.), and acid-catalyzed hydrolysis (e.g., U.S. Patent No. 4,569,789, to Blattler et al.).

It may be desirable to couple more than one agent to an antibody. In one embodiment, multiple molecules of an agent are coupled to one antibody molecule. In another embodiment, more than one type of agent may be coupled to one antibody. Regardless of the particular embodiment, immunoconjugates with more than one agent may be prepared in a variety of ways. For example, more than one agent may be coupled directly to an antibody molecule, or linkers that provide multiple sites for attachment can be used. Alternatively, a carrier can be used.

A carrier may bear the agents in a variety of ways, including covalent bonding either directly or via a linker group. Suitable carriers include proteins such as albumins (*e.g.,* U.S. Patent No. 4,507,234, to Kato et al.), peptides and polysaccharides such as aminodextran (e.g., U.S. Patent No. 4,699,784, to Shih et al.). A carrier may also bear an agent by noncovalent bonding or by encapsulation, such as within a liposome vesicle (e.g., U.S. Patent Nos. 4,429,008 and 4,873,088). Carriers specific for radionuclide agents include radiohalogenated small molecules and chelating compounds. For example, U.S. Patent No. 4,735,792 discloses representative radiohalogenated small molecules and their synthesis. A radionuclide chelate may be formed from chelating compounds that include those containing nitrogen and sulfur atoms as the donor atoms for binding the metal, or metal oxide, radionuclide. For example, U.S. Patent No. 4,673,562, to Davison et al. discloses representative chelating compounds and their synthesis.

### T Cell Compositions

The present invention, in another aspect, provides T cells specific for a tumor polypeptide disclosed herein, or for a variant or derivative thereof. Such cells may generally be prepared *in vitro* or *ex vivo,* using standard procedures. For example, T cells may be isolated from bone marrow, peripheral blood, or a fraction of bone marrow or peripheral blood of a patient, using a commercially available cell separation system, such as the Isolex^{™} System, available from Nexell Therapeutics, Inc. (Irvine, CA; see also U.S. Patent No. 5,240,856; U.S. Patent No. 5,215,926; WO 89/06280; WO 91/16116 and WO 92/07243). Alternatively, T cells may be derived from related or unrelated humans, non-human mammals, cell lines or cultures.

T cells may be stimulated with a polypeptide, polynucleotide encoding a polypeptide and/or an antigen presenting cell (APC) that expresses such a polypeptide. Such stimulation is performed under conditions and for a time sufficient to permit the generation of T cells that are specific for the polypeptide of interest. Preferably, a tumor polypeptide or polynucleotide of the invention is present within a delivery vehicle, such as a microsphere, to facilitate the generation of specific T cells.

T cells are considered to be specific for a polypeptide of the present invention if the T cells specifically proliferate, secrete cytokines or kill target cells coated with the polypeptide or expressing a gene encoding the polypeptide. T cell specificity may be evaluated using any of a variety of standard techniques. For example, within a chromium release assay or proliferation assay, a stimulation index of more than two fold increase in lysis and/or proliferation, compared to negative controls, indicates T cell specificity. Such assays may be performed, for example, as described in Chen et al., Cancer Res. 54:1065-1070, 1994. Alternatively, detection of the proliferation of T cells may be accomplished by a variety of known techniques. For example, T cell proliferation can be detected by measuring an increased rate of DNA synthesis *(e.g.,* by pulse-labeling cultures of T cells with tritiated thymidine and measuring the amount of tritiated thymidine incorporated into DNA). Contact with a tumor polypeptide (100 ng/ml - 100 µg/ml, preferably 200 ng/ml - 25 µg/ml) for 3 - 7 days will typically result in at least a two fold increase in proliferation of the T cells. Contact as described above for 2-3 hours should result in activation of the T cells, as measured using standard cytokine assays in which a two fold increase in the level of cytokine release (e.g., TNF or IFN-γ) is indicative of T cell activation (see Coligan et al., Current Protocols in Immunology, vol. 1, Wiley Interscience (Greene 1998)). T cells that have been activated in response to a tumor polypeptide, polynucleotide or polypeptide-expressing APC may be CD4⁺ and/or CD8⁺. Tumor polypeptide-specific T cells may be expanded using standard techniques. Within preferred embodiments, the T cells are derived from a patient, a related donor or an unrelated donor, and are administered to the patient following stimulation and expansion.

For therapeutic purposes, CD4⁺ or CD8⁺ T cells that proliferate in response to a tumor polypeptide, polynucleotide or APC can be expanded in number either *in vitro* or *in vivo.* Proliferation of such T cells *in vitro* may be accomplished in a variety of ways. For example, the T cells can be re-exposed to a tumor polypeptide, or a short peptide corresponding to an immunogenic portion of such a polypeptide, with or without the addition of T cell growth factors, such as interleukin-2, and/or stimulator cells that synthesize a tumor polypeptide. Alternatively, one or more T cells that proliferate in the presence of the tumor polypeptide can be expanded in number by cloning. Methods for cloning cells are well known in the art, and include limiting dilution.

### Pharmaceutical Compositions

In additional embodiments, the present invention concerns formulation of one or more of the polynucleotide, polypeptide, T-cell and/or antibody compositions disclosed herein in pharmaceutically-acceptable carriers for administration to a cell or an animal, either alone, or in combination with one or more other modalities of therapy.

It will be understood that, if desired, a composition as disclosed herein may be administered in combination with other agents as well, such as, e.g., other proteins or polypeptides or various pharmaceutically-active agents. In fact, there is virtually no limit to other components that may also be included, given that the additional agents do not cause a significant adverse effect upon contact with the target cells or host tissues. The compositions may thus be delivered along with various other agents as required in the particular instance. Such compositions may be purified from host cells or other biological sources, or alternatively may be chemically synthesized as described herein. Likewise, such compositions may further comprise substituted or derivatized RNA or DNA compositions.

Therefore, in another aspect of the present invention, pharmaceutical compositions are provided comprising one or more of the polynucleotide, polypeptide, antibody, and/or T-cell compositions described herein in combination with a physiologically acceptable carrier. In certain preferred embodiments, the pharmaceutical compositions of the invention comprise immunogenic polynucleotide and/or polypeptide compositions of the invention for use in prophylactic and therapeutic vaccine applications. Vaccine preparation is generally described in, for example, M.F. Powell and M.J. Newman, eds., "Vaccine Design (the subunit and adjuvant approach)," Plenum Press (NY, 1995). Generally, such compositions will comprise one or more polynucleotide and/or polypeptide compositions of the present invention in combination with one or more immunostimulants.

It will be apparent that any of the pharmaceutical compositions described herein can contain pharmaceutically acceptable salts of the polynucleotides and polypeptides of the invention. Such salts can be prepared, for example, from pharmaceutically acceptable non-toxic bases, including organic bases (e.g., salts of primary, secondary and tertiary amines and basic amino acids) and inorganic bases (e.g., sodium, potassium, lithium, ammonium, calcium and magnesium salts).

In another embodiment, illustrative immunogenic compositions, e.g., vaccine compositions, of the present invention comprise DNA encoding one or more of the polypeptides as described above, such that the polypeptide is generated *in situ.* As noted above, the polynucleotide may be administered within any of a variety of delivery systems known to those of ordinary skill in the art. Indeed, numerous gene delivery techniques are well known in the art, such as those described by Rolland, Crit. Rev. Therap. Drug Carrier Systems 15:143-198, 1998, and references cited therein. Appropriate polynucleotide expression systems will, of course, contain the necessary regulatory DNA regulatory sequences for expression in a patient (such as a suitable promoter and terminating signal). Alternatively, bacterial delivery systems may involve the administration of a bacterium (such as *Bacillus-Calmette-Guerrin)* that expresses an immunogenic portion of the polypeptide on its cell surface or secretes such an epitope.

Therefore, in certain embodiments, polynucleotides encoding immunogenic polypeptides described herein are introduced into suitable mammalian host cells for expression using any of a number of known viral-based systems. In one illustrative embodiment, retroviruses provide a convenient and effective platform for gene delivery systems. A selected nucleotide sequence encoding a polypeptide of the present invention can be inserted into a vector and packaged in retroviral particles using techniques known in the art. The recombinant virus can then be isolated and delivered to a subject. A number of illustrative retroviral systems have been described (*e.g.*, U.S. Pat. No. 5,219,740; Miller and Rosman (1989) BioTechniques 7:980-990; Miller, A. D. (1990) Human Gene Therapy 1:5-14; Scarpa et al. (1991) Virology 180:849-852; Burns et al. (1993) Proc. Nat'l Acad. Sci. USA 90:8033-8037; and Boris-Lawrie and Temin (1993) Cur. Opin. Genet. Develop. 3:102-109.

In addition, a number of illustrative adenovirus-based systems have also been described. Unlike retroviruses which integrate into the host genome, adenoviruses persist extrachromosomally thus minimizing the risks associated with insertional mutagenesis (Haj-Ahmad and Graham (1986) J. Virol. 57:267-274; Bett et al. (1993) J. Virol. 67:5911-5921; Mittereder et al. (1994) Human Gene Therapy 5:717-729; Seth et al. (1994) J. Virol. 68:933-940; Barr et al. (1994) Gene Therapy 1:51-58; Berkner, K. L. (1988) BioTechniques 6:616-629; and Rich et al. (1993) Human Gene Therapy 4:461-476).

Various adeno-associated virus (AAV) vector systems have also been developed for polynucleotide delivery. AAV vectors can be readily constructed using techniques well known in the art. See, e.g., U.S. Pat. Nos. 5,173,414 and 5,139,941; International Publication Nos. WO 92/01070 and WO 93/03769; Lebkowski et al. (1988) Molec. Cell. Biol. 8:3988-3996; Vincent et al. (1990) Vaccines 90 (Cold Spring Harbor Laboratory Press); Carter, B. J. (1992) Current Opinion in Biotechnology 3:533-539; Muzyczka, N. (1992) Current Topics in Microbiol. and Immunol. 158:97-129; Kotin, R. M. (1994) Human Gene Therapy 5:793-801; Shelling and Smith (1994) Gene Therapy 1:165-169; and Zhou et al. (1994) J. Exp. Med. 179:1867-1875.

Additional viral vectors useful for delivering the polynucleotides encoding polypeptides of the present invention by gene transfer include those derived from the pox family of viruses, such as vaccinia virus and avian poxvirus. By way of example, vaccinia virus recombinants expressing the novel molecules can be constructed as follows. The DNA encoding a polypeptide is first inserted into an appropriate vector so that it is adjacent to a vaccinia promoter and flanking vaccinia DNA sequences, such as the sequence encoding thymidine kinase (TK). This vector is then used to transfect cells which are simultaneously infected with vaccinia. Homologous recombination serves to insert the vaccinia promoter plus the gene encoding the polypeptide of interest into the viral genome. The resulting TK.sup.(-) recombinant can be selected by culturing the cells in the presence of 5-bromodeoxyuridine and picking viral plaques resistant thereto.

A vaccinia-based infection/transfection system can be conveniently used to provide for inducible, transient expression or coexpression of one or more polypeptides described herein in host cells of an organism. In this particular system, cells are first infected in vitro with a vaccinia virus recombinant that encodes the bacteriophage T7 RNA polymerase. This polymerase displays exquisite specificity in that it only transcribes templates bearing T7 promoters. Following infection, cells are transfected with the polynucleotide or polynucleotides of interest, driven by a T7 promoter. The polymerase expressed in the cytoplasm from the vaccinia virus recombinant transcribes the transfected DNA into RNA which is then translated into polypeptide by the host translational machinery. The method provides for high level, transient, cytoplasmic production of large quantities of RNA and its translation products. See, e.g., Elroy-Stein and Moss, Proc. Nat'l Acad. Sci. USA (1990) 87:6743-6747; Fuerst et al. Proc. Nat'l Acad. Sci. USA (1986) 83:8122-8126.

Alternatively, avipoxviruses, such as the fowlpox and canarypox viruses, can also be used to deliver the coding sequences of interest. Recombinant avipox viruses, expressing immunogens from mammalian pathogens, are known to confer protective immunity when administered to non-avian species. The use of an Avipox vector is particularly desirable in human and other mammalian species since members of the Avipox genus can only productively replicate in susceptible avian species and therefore are not infective in mammalian cells. Methods for producing recombinant Avipoxviruses are known in the art and employ genetic recombination, as described above with respect to the production of vaccinia viruses. See, e.g., WO 91/12882; WO 89/03429; and WO 92/03545.

Any of a number of alphavirus vectors can also be used for delivery of polynucleotide compositions of the present invention, such as those vectors described in U.S. Patent Nos. 5,843,723; 6,015,686; 6,008,035 and 6,015,694. Certain vectors based on Venezuelan Equine Encephalitis (VEE) can also be used, illustrative examples of which can be found in U.S. Patent Nos. 5,505,947 and 5,643,576.

Moreover, molecular conjugate vectors, such as the adenovirus chimeric vectors described in Michael et al. J. Biol. Chem. (1993) 268:6866-6869 and Wagner et al. Proc. Nat'l Acad. Sci. USA (1992) 89:6099-6103, can also be used for gene delivery under the invention.

Additional illustrative information on these and other known viral-based delivery systems can be found, for example, in Fisher-Hoch et al., Proc. Nat'l Acad. Sci. USA 86:317-321, 1989; Flexner et al., Ann. N. Y. Acad. Sci. 569:86-103, 1989; Flexner et al., Vaccine 8:17-21, 1990; U.S. Patent Nos. 4,603,112, 4,769,330, and 5,017,487; WO 89/01973; U.S. Patent No. 4,777,127; GB 2,200,651; EP 0,345,242; WO 91/02805; Berkner, Biotechniques 6:616-627, 1988; Rosenfeld et al., Science 252:431-434, 1991; Kolls et al., Proc. Nat'l Acad. Sci. USA 91:215-219, 1994; Kass-Eisler et al., Proc. Nat'l Acad. Sci. USA 90:11498-11502, 1993; Guzman et al., Circulation 88:2838-2848, 1993; and Guzman et al., Cir. Res. 73:1202-1207, 1993.

In certain embodiments, a polynucleotide may be integrated into the genome of a target cell. This integration may be in a specific location and orientation *via* homologous recombination (gene replacement) or it may be integrated in a random, non-specific location (gene augmentation). In yet further embodiments, the polynucleotide may be stably maintained in the cell as a separate, episomal segment of DNA. Such polynucleotide segments or "episomes" encode sequences sufficient to permit maintenance and replication independent of or in synchronization with the host cell cycle. The manner in which the expression construct is delivered to a cell and where in the cell the polynucleotide remains is dependent on the type of expression construct employed.

In another embodiment of the invention, a polynucleotide is administered/delivered as "naked" DNA, for example as described in Ulmer et al., Science 259:1745-1749, 1993 and reviewed by Cohen, Science 259:1691-1692, 1993. The uptake of naked DNA may be increased by coating the DNA onto biodegradable beads, which are efficiently transported into the cells.

In still another embodiment, a composition of the present invention can be delivered via a particle bombardment approach, many of which have been described. In one illustrative example, gas-driven particle acceleration can be achieved with devices such as those manufactured by Powderject Pharmaceuticals PLC (Oxford, UK) and Powderject Vaccines Inc. (Madison, Wl), some examples of which are described in U.S. Patent Nos. 5,846,796; 6,010,478; 5,865,796; 5,584,807; and EP Patent No. 0500 799. This approach offers a needle-free delivery approach wherein a dry powder formulation of microscopic particles, such as polynucleotide or polypeptide particles, are accelerated to high speed within a helium gas jet generated by a hand held device, propelling the particles into a target tissue of interest.

In a related embodiment, other devices and methods that may be useful for gas-driven needle-less injection of compositions of the present invention include those provided by Bioject, Inc. (Portland, OR), some examples of which are described in U.S. Patent Nos. 4,790,824; 5,064,413; 5,312,335; 5,383,851; 5,399,163; 5,520,639 and 5,993,412.

According to another embodiment, the pharmaceutical compositions described herein will comprise one or more immunostimulants in addition to the immunogenic polynucleotide, polypeptide, antibody, T-cell and/or APC compositions of this invention. An immunostimulant refers to essentially any substance that enhances or potentiates an immune response (antibody and/or cell-mediated) to an exogenous antigen. One preferred type of immunostimulant comprises an adjuvant. Many adjuvants contain a substance designed to protect the antigen from rapid catabolism, such as aluminum hydroxide or mineral oil, and a stimulator of immune responses, such as lipid A, *Bortadella pertussis* or *Mycobacterium tuberculosis* derived proteins. Certain adjuvants are commercially available as, for example, Freund's Incomplete Adjuvant and Complete Adjuvant (Difco Laboratories, Detroit, MI); Merck Adjuvant 65 (Merck and Company, Inc., Rahway, NJ); AS-2 (SmithKline Beecham, Philadelphia, PA); aluminum salts such as aluminum hydroxide gel (alum) or aluminum phosphate; salts of calcium, iron or zinc; an insoluble suspension of acylated tyrosine; acylated sugars; cationically or anionically derivatized polysaccharides; polyphosphazenes; biodegradable microspheres; monophosphoryl lipid A and quil A. Cytokines, such as GM-CSF, interleukin-2, -7, -12, and other like growth factors, may also be used as adjuvants.

Within certain embodiments of the invention, the adjuvant composition is preferably one that induces an immune response predominantly of the Th1 type. High levels of Th1-type cytokines (e.g., IFN-γ, TNFα, IL-2 and IL-12) tend to favor the induction of cell mediated immune responses to an administered antigen. In contrast, high levels of Th2-type cytokines (e.g., IL-4, IL-5, IL-6 and IL-10) tend to favor the induction of humoral immune responses. Following application of a vaccine as provided herein, a patient will support an immune response that includes Th1- and Th2-type responses. Within a preferred embodiment, in which a response is predominantly Th1-type, the level of Th1-type cytokines will increase to a greater extent than the level of Th2-type cytokines. The levels of these cytokines may be readily assessed using standard assays. For a review of the families of cytokines, see Mosmann and Coffman, Ann. Rev. Immunol. 7:145-173, 1989.

Certain preferred adjuvants for eliciting a predominantly Th1-type response include, for example, a combination of monophosphoryl lipid A, preferably 3-de-O-acylated monophosphoryl lipid A, together with an aluminum salt. MPL® adjuvants are available from Corixa Corporation (Seattle, WA; see, for example, US Patent Nos. 4,436,727; 4,877,611; 4,866,034 and 4,912,094). CpG-containing oligonucleotides (in which the CpG dinucleotide is unmethylated) also induce a predominantly Th1 response. Such oligonucleotides are well known and are described, for example, in WO 96/02555, WO 99/33488 and U.S. Patent Nos. 6,008,200 and 5,856,462. Immunostimulatory DNA sequences are also described, for example, by Sato et al., Science 273:352, 1996. Another preferred adjuvant comprises a saponin, such as Quil A, or derivatives thereof, including QS21 and QS7 (Aquila Biopharmaceuticals Inc., Framingham, MA); Escin; Digitonin; or *Gypsophila* or *Chenopodium quinoa* saponins . Other preferred formulations include more than one saponin in the adjuvant combinations of the present invention, for example combinations of at least two of the following group comprising QS21, QS7, Quil A, β-escin, or digitonin.

Alternatively the saponin formulations may be combined with vaccine vehicles composed of chitosan or other polycationic polymers, polylactide and polylactide-co-glycolide particles, poly-N-acetyl glucosamine-based polymer matrix, particles composed of polysaccharides or chemically modified polysaccharides, liposomes and lipid-based particles, particles composed of glycerol monoesters, etc. The saponins may also be formulated in the presence of cholesterol to form particulate structures such as liposomes or ISCOMs. Furthermore, the saponins may be formulated together with a polyoxyethylene ether or ester, in either a non-particulate solution or suspension, or in a particulate structure such as a paucilamelar liposome or ISCOM. The saponins may also be formulated with excipients such as Carbopol^{R} to increase viscosity, or may be formulated in a dry powder form with a powder excipient such as lactose.

In one preferred embodiment, the adjuvant system includes the combination of a monophosphoryl lipid A and a saponin derivative, such as the combination of QS21 and 3D-MPL® adjuvant, as described in WO 94/00153, or a less reactogenic composition where the QS21 is quenched with cholesterol, as described in WO 96/33739. Other preferred formulations comprise an oil-inwater emulsion and tocopherol. Another particularly preferred adjuvant formulation employing QS21, 3D-MPL® adjuvant and tocopherol in an oil-inwater emulsion is described in WO 95/17210.

Another enhanced adjuvant system involves the combination of a CpG-containing oligonucleotide and a saponin derivative particularly the combination of CpG and QS21 is disclosed in WO 00/09159. Preferably the formulation additionally comprises an oil in water emulsion and tocopherol.

Additional illustrative adjuvants for use in the pharmaceutical compositions of the invention include Montanide ISA 720 (Seppic, France), SAF (Chiron, California, United States), ISCOMS (CSL), MF-59 (Chiron), the SBAS series of adjuvants (e.g., SBAS-2 or SBAS-4, available from SmithKline Beecham, Rixensart, Belgium), Detox (Enhanzyn®; Corixa, Hamilton, MT), RC-529 (Corixa, Hamilton, MT) and other aminoalkyl glucosaminide 4-phosphates (AGPs), such as those described in pending U.S. Patent Application Serial Nos. 08/853,826 and 09/074,720, the disclosures of which are incorporated herein by reference in their entireties, and polyoxyethylene ether adjuvants such as those described in WO 99/52549A1.

Other preferred adjuvants include adjuvant molecules of the general formula

(I): HO(CH₂CH₂O)ₙ-A-R,

wherein, n is 1-50, A is a bond or -C(O)-, R is C₁₋₅₀ alkyl or Phenyl C₁₋₅₀ alkyl.

One embodiment of the present invention consists of a vaccine formulation comprising a polyoxyethylene ether of general formula (I), wherein n is between 1 and 50, preferably 4-24, most preferably 9; the R component is C₁₋₅₀, preferably C₄-C₂₀ alkyl and most preferably C₁₂ alkyl, and A is a bond. The concentration of the polyoxyethylene ethers should be in the range 0.1-20%, preferably from 0.1-10%, and most preferably in the range 0.1-1%. Preferred polyoxyethylene ethers are selected from the following group: polyoxyethylene-9-lauryl ether, polyoxyethylene-9-steoryl ether, polyoxyethylene-8-steoryl ether, polyoxyethylene-4-lauryl ether, polyoxyethylene-35-lauryl ether, and polyoxyethylene-23-lauryl ether. Polyoxyethylene ethers such as polyoxyethylene lauryl ether are described in the Merck index (12^{th} edition: entry 7717). These adjuvant molecules are described in WO 99/52549. The polyoxyethylene ether according to the general formula (I) above may, if desired, be combined with another adjuvant. For example, a preferred adjuvant combination is preferably with CpG as described in the pending UK patent application GB 9820956.2.

According to another embodiment of this invention, an immunogenic composition described herein is delivered to a host via antigen presenting cells (APCs), such as dendritic cells, macrophages, B cells, monocytes and other cells that may be engineered to be efficient APCs. Such cells may, but need not, be genetically modified to increase the capacity for presenting the antigen, to improve activation and/or maintenance of the T cell response, to have anti-tumor effects *per se* and/or to be immunologically compatible with the receiver (*i*.*e*., matched HLA haplotype). APCs may generally be isolated from any of a variety of biological fluids and organs, including tumor and peritumoral tissues, and may be autologous, allogeneic, syngeneic or xenogeneic cells.

Certain preferred embodiments of the present invention use dendritic cells or progenitors thereof as antigen-presenting cells. Dendritic cells are highly potent APCs (Banchereau and Steinman, Nature 392:245-251, 1998) and have been shown to be effective as a physiological adjuvant for eliciting prophylactic or therapeutic antitumor immunity (see Timmerman and Levy, Ann. Rev. Med. 50:507-529, 1999). In general, dendritic cells may be identified based on their typical shape (stellate *in situ,* with marked cytoplasmic processes (dendrites) visible *in vitro),* their ability to take up, process and present antigens with high efficiency and their ability to activate naïve T cell responses. Dendritic cells may, of course, be engineered to express specific cell-surface receptors or ligands that are not commonly found on dendritic cells *in vivo* or *ex vivo,* and such modified dendritic cells are contemplated by the present invention. As an alternative to dendritic cells, secreted vesicles antigen-loaded dendritic cells (called exosomes) may be used within a vaccine (see Zitvogel et al., Nature Med. 4:594-600, 1998).

Dendritic cells and progenitors may be obtained from peripheral blood, bone marrow, tumor-infiltrating cells, peritumoral tissues-infiltrating cells, lymph nodes, spleen, skin, umbilical cord blood or any other suitable tissue or fluid. For example, dendritic cells may be differentiated *ex vivo* by adding a combination of cytokines such as GM-CSF, IL-4, IL-13 and/or TNFα to cultures of monocytes harvested from peripheral blood. Alternatively, CD34 positive cells harvested from peripheral blood, umbilical cord blood or bone marrow may be differentiated into dendritic cells by adding to the culture medium combinations of GM-CSF, IL-3, TNFα, CD40 ligand, LPS, flt3 ligand and/or other compound(s) that induce differentiation, maturation and proliferation of dendritic cells.

Dendritic cells are conveniently categorized as "immature" and "mature" cells, which allows a simple way to discriminate between two well characterized phenotypes. However, this nomenclature should not be construed to exclude all possible intermediate stages of differentiation. Immature dendritic cells are characterized as APC with a high capacity for antigen uptake and processing, which correlates with the high expression of Fcγ receptor and mannose receptor. The mature phenotype is typically characterized by a lower expression of these markers, but a high expression of cell surface molecules responsible for T cell activation such as class I and class II MHC, adhesion molecules (*e.g.,* CD54 and CD11) and costimulatory molecules (e.g., CD40, CD80, CD86 and 4-1 BB).

APCs may generally be transfected with a polynucleotide of the invention (or portion or other variant thereof) such that the encoded polypeptide, or an immunogenic portion thereof, is expressed on the cell surface. Such transfection may take place ex *vivo,* and a pharmaceutical composition comprising such transfected cells may then be used for therapeutic purposes, as described herein. Alternatively, a gene delivery vehicle that targets a dendritic or other antigen presenting cell may be administered to a patient, resulting in transfection that occurs *in vivo. In vivo* and *ex vivo* transfection of dendritic cells, for example, may generally be performed using any methods known in the art, such as those described in WO 97/24447, or the gene gun approach described by Mahvi et al., Immunology and cell Biology 75:456-460, 1997. Antigen loading of dendritic cells may be achieved by incubating dendritic cells or progenitor cells with the tumor polypeptide, DNA (naked or within a plasmid vector) or RNA; or with antigen-expressing recombinant bacterium or viruses (e.g., vaccinia, fowlpox, adenovirus or lentivirus vectors). Prior to loading, the polypeptide may be covalently conjugated to an immunological partner that provides T cell help (e.g., a carrier molecule). Alternatively, a dendritic cell may be pulsed with a non-conjugated immunological partner, separately or in the presence of the polypeptide.

While any suitable carrier known to those of ordinary skill in the art may be employed in the pharmaceutical compositions of this invention, the type of carrier will typically vary depending on the mode of administration. Compositions of the present invention may be formulated for any appropriate manner of administration, including for example, topical, oral, nasal, mucosal, intravenous, intracranial, intraperitoneal, subcutaneous and intramuscular administration.

Carriers for use within such pharmaceutical compositions are biocompatible, and may also be biodegradable. In certain embodiments, the formulation preferably provides a relatively constant level of active component release. In other embodiments, however, a more rapid rate of release immediately upon administration may be desired. The formulation of such compositions is well within the level of ordinary skill in the art using known techniques. Illustrative carriers useful in this regard include microparticles of poly(lactide-co-glycolide), polyacrylate, latex, starch, cellulose, dextran and the like. Other illustrative delayed-release carriers include supramolecular biovectors, which comprise a non-liquid hydrophilic core (e.g., a cross-linked polysaccharide or oligosaccharide) and, optionally, an external layer comprising an amphiphilic compound, such as a phospholipid (see e.g., U.S. Patent No. 5,151,254 and PCT applications WO 94/20078, WO/94/23701 and WO 96/06638). The amount of active compound contained within a sustained release formulation depends upon the site of implantation, the rate and expected duration of release and the nature of the condition to be treated or prevented.

In another illustrative embodiment, biodegradable microspheres (e.g., polylactate polyglycolate) are employed as carriers for the compositions of this invention. Suitable biodegradable microspheres are disclosed, for example, in U.S. Patent Nos. 4,897,268; 5,075,109; 5,928,647; 5,811,128; 5,820,883; 5,853,763; 5,814,344, 5,407,609 and 5,942,252. Modified hepatitis B core protein carrier systems. such as described in WO/99 40934, and references cited therein, will also be useful for many applications. Another illustrative carrier/delivery system employs a carrier comprising particulate-protein complexes, such as those described in U.S. Patent No. 5,928,647, which are capable of inducing a class I-restricted cytotoxic T lymphocyte responses in a host.

The pharmaceutical compositions of the invention will often further comprise one or more buffers (e.g., neutral buffered saline or phosphate buffered saline), carbohydrates (e.g., glucose, mannose, sucrose or dextrans), mannitol, proteins, polypeptides or amino acids such as glycine, antioxidants, bacteriostats, chelating agents such as EDTA or glutathione, adjuvants (e.g., aluminum hydroxide), solutes that render the formulation isotonic, hypotonic or weakly hypertonic with the blood of a recipient, suspending agents, thickening agents and/or preservatives. Alternatively, compositions of the present invention may be formulated as a lyophilizate.

The pharmaceutical compositions described herein may be presented in unit-dose or multi-dose containers, such as sealed ampoules or vials. Such containers are typically sealed in such a way to preserve the sterility and stability of the formulation until use. In general, formulations may be stored as suspensions, solutions or emulsions in oily or aqueous vehicles. Alternatively, a pharmaceutical composition may be stored in a freeze-dried condition requiring only the addition of a sterile liquid carrier immediately prior to use.

The development of suitable dosing and treatment regimens for using the particular compositions described herein in a variety of treatment regimens, including e.g., oral, parenteral, intravenous, intranasal, and intramuscular administration and formulation, is well known in the art, some of which are briefly discussed below for general purposes of illustration.

In certain applications, the pharmaceutical compositions disclosed herein may be delivered via oral administration to an animal. As such, these compositions may be formulated with an inert diluent or with an assimilable edible carrier, or they may be enclosed in hard- or soft-shell gelatin capsule, or they may be compressed into tablets, or they may be incorporated directly with the food of the diet.

The active compounds may even be incorporated with excipients and used in the form of ingestible tablets, buccal tables, troches, capsules, elixirs, suspensions, syrups, wafers, and the like (see, for example, Mathiowitz et al., Nature 1997 Mar 27;386(6623):410-4; Hwang et al., Crit Rev Ther Drug Carrier Syst 1998;15(3):243-84; U. S. Patent 5,641,515; U. S. Patent 5,580,579 and U. S. Patent 5,792,451). Tablets, troches, pills, capsules and the like may also contain any of a variety of additional components, for example, a binder, such as gum tragacanth, acacia, cornstarch, or gelatin; excipients, such as dicalcium phosphate; a disintegrating agent, such as corn starch, potato starch, alginic acid and the like; a lubricant, such as magnesium stearate; and a sweetening agent, such as sucrose, lactose or saccharin may be added or a flavoring agent, such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar, or both. Of course, any material used in preparing any dosage unit form should be pharmaceutically pure and substantially non-toxic in the amounts employed. In addition, the active compounds may be incorporated into sustained-release preparation and formulations.

Typically, these formulations will contain at least about 0.1% of the active compound or more, although the percentage of the active ingredient(s) may, of course, be varied and may conveniently be between about 1 or 2% and about 60% or 70% or more of the weight or volume of the total formulation. Naturally, the amount of active compound(s) in each therapeutically useful composition may be prepared is such a way that a suitable dosage will be obtained in any given unit dose of the compound. Factors such as solubility, bioavailability, biological half-life, route of administration, product shelf life, as well as other pharmacological considerations will be contemplated by one skilled in the art of preparing such pharmaceutical formulations, and as such, a variety of dosages and treatment regimens may be desirable.

For oral administration, the compositions of the present invention may alternatively be incorporated with one or more excipients in the form of a mouthwash, dentifrice, buccal tablet, oral spray, or sublingual orally-administered formulation. Alternatively, the active ingredient may be incorporated into an oral solution such as one containing sodium borate, glycerin and potassium bicarbonate, or dispersed in a dentifrice, or added in a therapeutically-effective amount to a composition that may include water, binders, abrasives, flavoring agents, foaming agents, and humectants. Alternatively the compositions may be fashioned into a tablet or solution form that may be placed under the tongue or otherwise dissolved in the mouth.

In certain circumstances it will be desirable to deliver the pharmaceutical compositions disclosed herein parenterally, intravenously, intramuscularly, or even intraperitoneally. Such approaches are well known to the skilled artisan, some of which are further described, for example, in U. S. Patent 5,543,158; U. S. Patent 5,641,515 and U. S. Patent 5,399,363. In certain embodiments, solutions of the active compounds as free base or pharmacologically acceptable salts may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations generally will contain a preservative to prevent the growth of microorganisms.

Illustrative pharmaceutical forms suitable for injectable use include sterile aqueous solutions or dispersions and sterile powders for the extemporaneous preparation of sterile injectable solutions or dispersions (for example, see U. S. Patent 5,466,468). In all cases the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (e.g., glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and/or by the use of surfactants. The prevention of the action of microorganisms can be facilitated by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

In one embodiment, for parenteral administration in an aqueous solution, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, a sterile aqueous medium that can be employed will be known to those of skill in the art in light of the present disclosure. For example, one dosage may be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. Moreover, for human administration, preparations will of course preferably meet sterility, pyrogenicity, and the general safety and purity standards as required by FDA Office of Biologics standards.

In another embodiment of the invention, the compositions disclosed herein may be formulated in a neutral or salt form. Illustrative pharmaceutically-acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. Upon formulation, solutions will be administered in a manner compatible with the dosage formulation and in such amount as is therapeutically effective.

The carriers can further comprise any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions. The phrase "pharmaceutically-acceptable" refers to molecular entities and compositions that do not produce an allergic or similar untoward reaction when administered to a human.

In certain embodiments, the pharmaceutical compositions may be delivered by intranasal sprays, inhalation, and/or other aerosol delivery vehicles. Methods for delivering genes, nucleic acids, and peptide compositions directly to the lungs *via* nasal aerosol sprays has been described, e.g., in U. S. Patent 5,756,353 and U. S. Patent 5,804,212. Likewise, the delivery of drugs using intranasal microparticle resins (Takenaga et al., J Controlled Release 1998 Mar 2;52(1-2):81-7) and lysophosphatidyl-glycerol compounds (U. S. Patent 5,725,871) are also well-known in the pharmaceutical arts. Likewise, illustrative transmucosal drug delivery in the form of a polytetrafluoroetheylene support matrix is described in U. S. Patent 5,780,045.

In certain embodiments, liposomes, nanocapsules, microparticles, lipid particles, vesicles, and the like, are used for the introduction of the compositions of the present invention into suitable host cells/organisms. In particular, the compositions of the present invention may be formulated for delivery either encapsulated in a lipid particle, a liposome, a vesicle, a nanosphere, or a nanoparticle or the like. Alternatively, compositions of the present invention can be bound, either covalently or non-covalently, to the surface of such carrier vehicles.

The formation and use of liposome and liposome-like preparations as potential drug carriers is generally known to those of skill in the art (see for example, Lasic, Trends Biotechnol 1998 Jul;16(7):307-21; Takakura, Nippon Rinsho 1998 Mar;56(3):691-5; Chandran et al., Indian J Exp Biol. 1997 Aug;35(8):801-9; Margalit, Crit Rev Ther Drug Carrier Syst. 1995;12(2-3):233-61; U.S. Patent 5,567,434; U.S. Patent 5,552,157; U.S. Patent 5,565,213; U.S. Patent 5,738,868 and U.S. Patent 5,795,587, each specifically incorporated herein by reference in its entirety).

Liposomes have been used successfully with a number of cell types that are normally difficult to transfect by other procedures, including T cell suspensions, primary hepatocyte cultures and PC 12 cells (Renneisen et al., J Biol Chem. 1990 Sep 25;265(27):16337-42; Muller et al., DNA Cell Biol. 1990 Apr;9(3):221-9). In addition, liposomes are free of the DNA length constraints that are typical of viral-based delivery systems. Liposomes have been used effectively to introduce genes, various drugs, radiotherapeutic agents, enzymes, viruses, transcription factors, allosteric effectors and the like, into a variety of cultured cell lines and animals. Furthermore, he use of liposomes does not appear to be associated with autoimmune responses or unacceptable toxicity after systemic delivery.

In certain embodiments, liposomes are formed from phospholipids that are dispersed in an aqueous medium and spontaneously form multilamellar concentric bilayer vesicles (also termed multilamellar vesicles (MLVs).

Alternatively, in other embodiments, the invention provides for pharmaceutically-acceptable nanocapsule formulations of the compositions of the present invention. Nanocapsules can generally entrap compounds in a stable and reproducible way (see, for example, Quintanar-Guerrero et al., Drug Dev Ind Pharm. 1998 Dec;24(12): 1113-28). To avoid side effects due to intracellular polymeric overloading, such ultrafine particles (sized around 0.1 µm) may be designed using polymers able to be degraded *in vivo.* Such particles can be made as described, for example, by Couvreur et al., Crit Rev Ther Drug Carrier Syst. 1988;5(1):1-20; zur Muhlen et al., Eur J Pharm Biopharm. 1998 Mar;45(2):149-55; Zambaux et al. J Controlled Release. 1998 Jan 2;50(1-3):31-40; and U. S. Patent 5,145,684.

### Cancer Therapeutic Methods

In further aspects of the present invention, the pharmaceutical compositions described herein may be used for the treatment of cancer, particularly for the immunotherapy of prostate cancer. Within such methods, the pharmaceutical compositions described herein are administered to a patient, typically a warm-blooded animal, preferably a human. A patient may or may not be afflicted with cancer. Accordingly, the above pharmaceutical compositions may be used to prevent the development of a cancer or to treat a patient afflicted with a cancer. Pharmaceutical compositions and vaccines may be administered either prior to or following surgical removal of primary tumors and/or treatment such as administration of radiotherapy or conventional chemotherapeutic drugs. As discussed above, administration of the pharmaceutical compositions may be by any suitable method, including administration by intravenous, intraperitoneal, intramuscular, subcutaneous, intranasal, intradermal, anal, vaginal, topical and oral routes.

Within certain embodiments, immunotherapy may be active immunotherapy, in which treatment relies on the *in vivo* stimulation of the endogenous host immune system to react against tumors with the administration of immune response-modifying agents (such as polypeptides and polynucleotides as provided herein).

Within other embodiments, immunotherapy may be passive immunotherapy, in which treatment involves the delivery of agents with established tumor-immune reactivity (such as effector cells or antibodies) that can directly or indirectly mediate antitumor effects and does not necessarily depend on an intact host immune system. Examples of effector cells include T cells as discussed above, T lymphocytes (such as CD8⁺ cytotoxic T lymphocytes and CD4⁺ T-helper tumor-infiltrating lymphocytes), killer cells (such as Natural Killer cells and lymphokine-activated killer cells), B cells and antigen-presenting cells (such as dendritic cells and macrophages) expressing a polypeptide provided herein. T cell receptors and antibody receptors specific for the polypeptides recited herein may be cloned, expressed and transferred into other vectors or effector cells for adoptive immunotherapy. The polypeptides provided herein may also be used to generate antibodies or anti-idiotypic antibodies (as described above and in U.S. Patent No. 4,918,164) for passive immunotherapy.

Effector cells may generally be obtained in sufficient quantities for adoptive immunotherapy by growth *in vitro,* as described herein. Culture conditions for expanding single antigen-specific effector cells to several billion in number with retention of antigen recognition *in vivo* are well known in the art. Such *in vitro* culture conditions typically use intermittent stimulation with antigen, often in the presence of cytokines (such as IL-2) and non-dividing feeder cells. As noted above, immunoreactive polypeptides as provided herein may be used to rapidly expand antigen-specific T cell cultures in order to generate a sufficient number of cells for immunotherapy. In particular, antigen-presenting cells, such as dendritic, macrophage, monocyte, fibroblast and/or B cells, may be pulsed with immunoreactive polypeptides or transfected with one or more polynucleotides using standard techniques well known in the art. For example, antigen-presenting cells can be transfected with a polynucleotide having a promoter appropriate for increasing expression in a recombinant virus or other expression system. Cultured effector cells for use in therapy must be able to grow and distribute widely, and to survive long term *in vivo.* Studies have shown that cultured effector cells can be induced to grow in vivo and to survive long term in substantial numbers by repeated stimulation with antigen supplemented with IL-2 (see, for example, Cheever et al., Immunological Reviews 157:177,1997).

Alternatively, a vector expressing a polypeptide recited herein may be introduced into antigen presenting cells taken from a patient and clonally propagated *ex vivo* for transplant back into the same patient. Transfected cells may be reintroduced into the patient using any means known in the art, preferably in sterile form by intravenous, intracavitary, intraperitoneal or intratumor administration.

Routes and frequency of administration of the therapeutic compositions described herein, as well as dosage, will vary from individual to individual, and may be readily established using standard techniques. In general, the pharmaceutical compositions and vaccines may be administered by injection (e.g., intracutaneous, intramuscular, intravenous or subcutaneous), intranasally (e.g., by aspiration) or orally. Preferably, between 1 and 10 doses may be administered over a 52 week period. Preferably, 6 doses are administered, at intervals of 1 month, and booster vaccinations may be given periodically thereafter. Alternate protocols may be appropriate for individual patients. A suitable dose is an amount of a compound that, when administered as described above, is capable of promoting an anti-tumor immune response, and is at least 10-50% above the basal (*i.e.,* untreated) level. Such response can be monitored by measuring the anti-tumor antibodies in a patient or by vaccine-dependent generation of cytolytic effector cells capable of killing the patient's tumor cells *in vitro.* Such vaccines should also be capable of causing an immune response that leads to an improved clinical outcome (e.g., more frequent remissions, complete or partial or longer disease-free survival) in vaccinated patients as compared to non-vaccinated patients. In general, for pharmaceutical compositions and vaccines comprising one or more polypeptides, the amount of each polypeptide present in a dose ranges from about 25 µg to 5 mg per kg of host. Suitable dose sizes will vary with the size of the patient, but will typically range from about 0.1 mL to about 5 mL.

In general, an appropriate dosage and treatment regimen provides the active compound(s) in an amount sufficient to provide therapeutic and/or prophylactic benefit. Such a response can be monitored by establishing an improved clinical outcome (e.g., more frequent remissions, complete or partial, or longer disease-free survival) in treated patients as compared to non-treated patients. Increases in preexisting immune responses to a tumor protein generally correlate with an improved clinical outcome. Such immune responses may generally be evaluated using standard proliferation, cytotoxicity or cytokine assays, which may be performed using samples obtained from a patient before and after treatment.

### Cancer Detection and Diagnostic Compositions, Methods and Kits

In general, a cancer may be detected in a patient based on the presence of one or more prostate tumor proteins and/or polynucleotides encoding such proteins in a biological sample (for example, blood, sera, sputum urine and/or tumor biopsies) obtained from the patient. In other words, such proteins may be used as markers to indicate the presence or absence of a cancer such as prostate cancer. In addition, such proteins may be useful for the detection of other cancers. The binding agents provided herein generally permit detection of the level of antigen that binds to the agent in the biological sample. Polynucleotide primers and probes may be used to detect the level of mRNA encoding a tumor protein, which is also indicative of the presence or absence of a cancer. In general, a prostate tumor sequence should be present at a level that is at least three fold higher in tumor tissue than in normal tissue

There are a variety of assay formats known to those of ordinary skill in the art for using a binding agent to detect polypeptide markers in a sample. *See, e.g.,* Harlow and Lane, Antibodies: A Laboratory Manual, Cold Spring Harbor Laboratory, 1988. In general, the presence or absence of a cancer in a patient may be determined by (a) contacting a biological sample obtained from a patient with a binding agent; (b) detecting in the sample a level of polypeptide that binds to the binding agent; and (c) comparing the level of polypeptide with a predetermined cut-off value.

In a preferred embodiment, the assay involves the use of binding agent immobilized on a solid support to bind to and remove the polypeptide from the remainder of the sample. The bound polypeptide may then be detected using a detection reagent that contains a reporter group and specifically binds to the binding agent/polypeptide complex. Such detection reagents may comprise, for example, a binding agent that specifically binds to the polypeptide or an antibody or other agent that specifically binds to the binding agent, such as an anti-immunoglobulin, protein G, protein A or a lectin. Alternatively, a competitive assay may be utilized, in which a polypeptide is labeled with a reporter group and allowed to bind to the immobilized binding agent after incubation of the binding agent with the sample. The extent to which components of the sample inhibit the binding of the labeled polypeptide to the binding agent is indicative of the reactivity of the sample with the immobilized binding agent. Suitable polypeptides for use within such assays include full length prostate tumor proteins and polypeptide portions thereof to which the binding agent binds, as described above.

The solid support may be any material known to those of ordinary skill in the art to which the tumor protein may be attached. For example, the solid support may be a test well in a microtiter plate or a nitrocellulose or other suitable membrane. Alternatively, the support may be a bead or disc, such as glass, fiberglass, latex or a plastic material such as polystyrene or polyvinylchloride. The support may also be a magnetic particle or a fiber optic sensor, such as those disclosed, for example, in U.S. Patent No. 5,359,681. The binding agent may be immobilized on the solid support using a variety of techniques known to those of skill in the art, which are amply described in the patent and scientific literature. In the context of the present invention, the term "immobilization" refers to both noncovalent association, such as adsorption, and covalent attachment (which may be a direct linkage between the agent and functional groups on the support or may be a linkage by way of a cross-linking agent). Immobilization by adsorption to a well in a microtiter plate or to a membrane is preferred. In such cases, adsorption may be achieved by contacting the binding agent, in a suitable buffer, with the solid support for a suitable amount of time. The contact time varies with temperature, but is typically between about 1 hour and about 1 day. In general, contacting a well of a plastic microtiter plate (such as polystyrene or polyvinylchloride) with an amount of binding agent ranging from about 10 ng to about 10 µg, and preferably about 100 ng to about 1 µg, is sufficient to immobilize an adequate amount of binding agent.

Covalent attachment of binding agent to a solid support may generally be achieved by first reacting the support with a bifunctional reagent that will react with both the support and a functional group, such as a hydroxyl or amino group, on the binding agent. For example, the binding agent may be covalently attached to supports having an appropriate polymer coating using benzoquinone or by condensation of an aldehyde group on the support with an amine and an active hydrogen on the binding partner (see, e.g., Pierce Immunotechnology Catalog and Handbook, 1991, atA12-A13).

In certain embodiments, the assay is a two-antibody sandwich assay. This assay may be performed by first contacting an antibody that has been immobilized on a solid support, commonly the well of a microtiter plate, with the sample, such that polypeptides within the sample are allowed to bind to the immobilized antibody. Unbound sample is then removed from the immobilized polypeptide-antibody complexes and a detection reagent (preferably a second antibody capable of binding to a different site on the polypeptide) containing a reporter group is added. The amount of detection reagent that remains bound to the solid support is then determined using a method appropriate for the specific reporter group.

More specifically, once the antibody is immobilized on the support as described above, the remaining protein binding sites on the support are typically blocked. Any suitable blocking agent known to those of ordinary skill in the art, such as bovine serum albumin or Tween 20^{™} (Sigma Chemical Co., St. Louis, MO). The immobilized antibody is then incubated with the sample, and polypeptide is allowed to bind to the antibody. The sample may be diluted with a suitable diluent, such as phosphate-buffered saline (PBS) prior to incubation. In general, an appropriate contact time (*i.e.,* incubation time) is a period of time that is sufficient to detect the presence of polypeptide within a sample obtained from an individual with prostate cancer. Preferably, the contact time is sufficient to achieve a level of binding that is at least about 95% of that achieved at equilibrium between bound and unbound polypeptide. Those of ordinary skill in the art will recognize that the time necessary to achieve equilibrium may be readily determined by assaying the level of binding that occurs over a period of time. At room temperature, an incubation time of about 30 minutes is generally sufficient.

Unbound sample may then be removed by washing the solid support with an appropriate buffer, such as PBS containing 0.1 % Tween 20^{™}. The second antibody, which contains a reporter group, may then be added to the solid support. Preferred reporter groups include those groups recited above.

The detection reagent is then incubated with the immobilized antibody-polypeptide complex for an amount of time sufficient to detect the bound polypeptide. An appropriate amount of time may generally be determined by assaying the level of binding that occurs over a period of time. Unbound detection reagent is then removed and bound detection reagent is detected using the reporter group. The method employed for detecting the reporter group depends upon the nature of the reporter group. For radioactive groups, scintillation counting or autoradiographic methods are generally appropriate. Spectroscopic methods may be used to detect dyes, luminescent groups and fluorescent groups. Biotin may be detected using avidin, coupled to a different reporter group (commonly a radioactive or fluorescent group or an enzyme). Enzyme reporter groups may generally be detected by the addition of substrate (generally for a specific period of time), followed by spectroscopic or other analysis of the reaction products.

To determine the presence or absence of a cancer, such as prostate cancer, the signal detected from the reporter group that remains bound to the solid support is generally compared to a signal that corresponds to a predetermined cut-off value. In one preferred embodiment, the cut-off value for the detection of a cancer is the average mean signal obtained when the immobilized antibody is incubated with samples from patients without the cancer. In general, a sample generating a signal that is three standard deviations above the predetermined cut-off value is considered positive for the cancer. In an alternate preferred embodiment, the cut-off value is determined using a Receiver Operator Curve, according to the method of Sackett et al., Clinical Epidemiology: A Basic Science for Clinical Medicine, Little Brown and Co., 1985, p. 106-7. Briefly, in this embodiment, the cut-off value may be determined from a plot of pairs of true positive rates (*i.e.,* sensitivity) and false positive rates (100%-specificity) that correspond to each possible cut-off value for the diagnostic test result. The cut-off value on the plot that is the closest to the upper left-hand corner (*i.e*., the value that encloses the largest area) is the most accurate cut-off value, and a sample generating a signal that is higher than the cut-off value determined by this method may be considered positive. Alternatively, the cut-off value may be shifted to the left along the plot, to minimize the false positive rate, or to the right, to minimize the false negative rate. In general, a sample generating a signal that is higher than the cut-off value determined by this method is considered positive for a cancer.

In a related embodiment, the assay is performed in a flow-through or strip test format, wherein the binding agent is immobilized on a membrane, such as nitrocellulose. In the flow-through test, polypeptides within the sample bind to the immobilized binding agent as the sample passes through the membrane. A second, labeled binding agent then binds to the binding agentpolypeptide complex as a solution containing the second binding agent flows through the membrane. The detection of bound second binding agent may then be performed as described above. In the strip test format, one end of the membrane to which binding agent is bound is immersed in a solution containing the sample. The sample migrates along the membrane through a region containing second binding agent and to the area of immobilized binding agent. Concentration of second binding agent at the area of immobilized antibody indicates the presence of a cancer. Typically, the concentration of second binding agent at that site generates a pattern, such as a line, that can be read visually. The absence of such a pattern indicates a negative result. In general, the amount of binding agent immobilized on the membrane is selected to generate a visually discernible pattern when the biological sample contains a level of polypeptide that would be sufficient to generate a positive signal in the two-antibody sandwich assay, in the format discussed above. Preferred binding agents for use in such assays are antibodies and antigen-binding fragments thereof. Preferably, the amount of antibody immobilized on the membrane ranges from about 25 ng to about 1µg, and more preferably from about 50 ng to about 500 ng. Such tests can typically be performed with a very small amount of biological sample.

Of course, numerous other assay protocols exist that are suitable for use with the tumor proteins or binding agents of the present invention. The above descriptions are intended to be exemplary only. For example, it will be apparent to those of ordinary skill in the art that the above protocols may be readily modified to use tumor polypeptides to detect antibodies that bind to such polypeptides in a biological sample. The detection of such tumor protein specific antibodies may correlate with the presence of a cancer.

A cancer may also, or alternatively, be detected based on the presence of T cells that specifically react with a tumor protein in a biological sample. Within certain methods, a biological sample comprising CD4⁺ and/or CD8⁺ T cells isolated from a patient is incubated with a tumor polypeptide, a polynucleotide encoding such a polypeptide and/or an APC that expresses at least an immunogenic portion of such a polypeptide, and the presence or absence of specific activation of the T cells is detected. Suitable biological samples include, but are not limited to, isolated T cells. For example, T cells may be isolated from a patient by routine techniques (such as by Ficoll/Hypaque density gradient centrifugation of peripheral blood lymphocytes). T cells may be incubated *in vitro* for 2-9 days (typically 4 days) at 37°C with polypeptide (e.g., 5 - 25 µg/ml). It may be desirable to incubate another aliquot of a T cell sample in the absence of tumor polypeptide to serve as a control. For CD4⁺ T cells, activation is preferably detected by evaluating proliferation of the T cells. For CD8⁺ T cells, activation is preferably detected by evaluating cytolytic activity. A level of proliferation that is at least two fold greater and/or a level of cytolytic activity that is at least 20% greater than in disease-free patients indicates the presence of a cancer in the patient.

As noted above, a cancer may also, or alternatively, be detected based on the level of mRNA encoding a tumor protein in a biological sample. For example, at least two oligonucleotide primers may be employed in a polymerase chain reaction (PCR) based assay to amplify a portion of a tumor cDNA derived from a biological sample, wherein at least one of the oligonucleotide primers is specific for (*i.e.*, hybridizes to) a polynucleotide encoding the tumor protein. The amplified cDNA is then separated and detected using techniques well known in the art, such as gel electrophoresis. Similarly, oligonucleotide probes that specifically hybridize to a polynucleotide encoding a tumor protein may be used in a hybridization assay to detect the presence of polynucleotide encoding the tumor protein in a biological sample.

To permit hybridization under assay conditions, oligonucleotide primers and probes should comprise an oligonucleotide sequence that has at least about 60%, preferably at least about 75% and more preferably at least about 90%, identity to a portion of a polynucleotide encoding a tumor protein of the invention that is at least 10 nucleotides, and preferably at least 20 nucleotides, in length. Preferably, oligonucleotide primers and/or probes hybridize to a polynucleotide encoding a polypeptide described herein under moderately stringent conditions, as defined above. Oligonucleotide primers and/or probes which may be usefully employed in the diagnostic methods described herein preferably are at least 10-40 nucleotides in length. In a preferred embodiment, the oligonucleotide primers comprise at least 10 contiguous nucleotides, more preferably at least 15 contiguous nucleotides, of a DNA molecule having a sequence as disclosed herein. Techniques for both PCR based assays and hybridization assays are well known in the art (see, for example, Mullis et al., Cold Spring Harbor Symp. Quant. Biol., 51:263, 1987; Erlich ed., PCR Technology, Stockton Press, NY, 1989).

One preferred assay employs RT-PCR, in which PCR is applied in conjunction with reverse transcription. Typically, RNA is extracted from a biological sample, such as biopsy tissue, and is reverse transcribed to produce cDNA molecules. PCR amplification using at least one specific primer generates a cDNA molecule, which may be separated and visualized using, for example, gel electrophoresis. Amplification may be performed on biological samples taken from a test patient and from an individual who is not afflicted with a cancer. The amplification reaction may be performed on several dilutions of cDNA spanning two orders of magnitude. A two-fold or greater increase in expression in several dilutions of the test patient sample as compared to the same dilutions of the non-cancerous sample is typically considered positive.

In another embodiment, the compositions described herein may be used as markers for the progression of cancer. In this embodiment, assays as described above for the diagnosis of a cancer may be performed over time, and the change in the level of reactive polypeptide(s) or polynucleotide(s) evaluated. For example, the assays may be performed every 24-72 hours for a period of 6 months to 1 year, and thereafter performed as needed. In general, a cancer is progressing in those patients in whom the level of polypeptide or polynucleotide detected increases over time. In contrast, the cancer is not progressing when the level of reactive polypeptide or polynucleotide either remains constant or decreases with time.

Certain *in vivo* diagnostic assays may be performed directly on a tumor. One such assay involves contacting tumor cells with a binding agent. The bound binding agent may then be detected directly or indirectly via a reporter group. Such binding agents may also be used in histological applications. Alternatively, polynucleotide probes may be used within such applications.

As noted above, to improve sensitivity, multiple tumor protein markers may be assayed within a given sample. It will be apparent that binding agents specific for different proteins provided herein may be combined within a single assay. Further, multiple primers or probes may be used concurrently. The selection of tumor protein markers may be based on routine experiments to determine combinations that results in optimal sensitivity. In addition, or alternatively, assays for tumor proteins provided herein may be combined with assays for other known tumor antigens.

In other aspects of the present invention, cell capture technologies may be used prior to detection to improve the sensitivity of the various detection methodologies disclosed herein.

Exemplary cell enrichment methodologies employ immunomagnetic beads that are coated with specific monoclonal antibodies to surface cell markers, or tetrameric antibody complexes, may be used to first enrich or positively select cancer cells in a sample. Various commercially available kits may be used, including Dynabeads® Epithelial Enrich (Dynal Biotech, Oslo, Norway), StemSep^{™} (StemCell Technologies, Inc., Vancouver, BC), and RosetteSep (StemCell Technologies). The skilled artisan will recognize that other readily available methodologies and kits may also be suitably employed to enrich or positively select desired cell populations.

Dynabeads® Epithelial Enrich contains magnetic beads coated with mAbs specific for two glycoprotein membrane antigens expressed on normal and neoplastic epithelial tissues. The coated beads may be added to a sample and the sample then applied to a magnet, thereby capturing the cells bound to the beads. The unwanted cells are washed away and the magnetically isolated cells eluted from the beads and used in further analyses.

RosetteSep can be used to enrich cells directly from a blood sample and consists of a cocktail of tetrameric antibodies that target a variety of unwanted cells and crosslinks them to glycophorin A on red blood cells (RBC) present in the sample, forming rosettes. When centrifuged over Ficoll, targeted cells pellet along with the free RBC.

The combination of antibodies in the depletion cocktail determines which cells will be removed and consequently which cells will be recovered. Antibodies that are available include, but are not limited to: CD2, CD3, CD4, CD5, CD8, CD10, CD11b, CD14, CD15, CD16, CD19, CD20, CD24, CD25, CD29, CD33, CD34, CD36, CD38, CD41, CD45, CD45RA, CD45RO, CD56, CD66B, CD66e, HLA-DR, IgE, and TCRαβ. Additionally, it is contemplated in the present invention that mAbs specific for prostate tumor antigens, can be developed and used in a similar manner. For example, mAbs that bind to tumor-specific cell surface antigens may be conjugated to magnetic beads, or formulated in a tetrameric antibody complex, and used to enrich or positively select metastatic prostate tumor cells from a sample.

Once a sample is enriched or positively selected, cells may be further analyzed. For example, the cells may be lysed and RNA isolated. RNA may then be subjected to RT-PCR analysis using prostate tumor-specific primers in a Real-time PCR assay as described herein.

In another aspect of the present invention, cell capture technologies may be used in conjunction with real-time PCR to provide a more sensitive tool for detection of metastatic cells expressing prostate tumor antigens. Detection of prostate cancer cells in bone marrow samples, peripheral blood, biopsies, and other samples is desirable for diagnosis and prognosis in prostate cancer patients.

The present invention further provides kits for use within any of the above diagnostic methods. Such kits typically comprise two or more components necessary for performing a diagnostic assay. Components may be compounds, reagents, containers and/or equipment. For example, one container within a kit may contain a monoclonal antibody or fragment thereof that specifically binds to a tumor protein. Such antibodies or fragments may be provided attached to a support material, as described above. One or more additional containers may enclose elements, such as reagents or buffers, to be used in the assay. Such kits may also, or alternatively, contain a detection reagent as described above that contains a reporter group suitable for direct or indirect detection of antibody binding.

Alternatively, a kit may be designed to detect the level of mRNA encoding a tumor protein in a biological sample. Such kits generally comprise at least one oligonucleotide probe or primer, as described above, that hybridizes to a polynucleotide encoding a tumor protein. Such an oligonucleotide may be used, for example, within a PCR or hybridization assay. Additional components that may be present within such kits include a second oligonucleotide and/or a diagnostic reagent or container to facilitate the detection of a polynucleotide encoding a tumor protein.

The following Examples are offered by way of illustration and not by way of limitation.

Aspects of the invention are defined in the numbered paragraphs below:
1. An isolated polynucleotide comprising a sequence selected from the group consisting of:
   (a) sequences provided in SEQ ID NOs: 944-945, 948, 967, 969-976, 991, 993-1002, 1004, 1006-1007, 1010, 1013-1019, 1021, 1023-1027;
   (b) complements of the sequences provided in SEQ ID NOs: 944-945, 948, 967, 969-976, 991, 993-1002, 1004, 1006-1007, 1010, 1013-1019, 1021, 1023-1027;
   (c) sequences consisting of at least 20 contiguous residues of a sequence provided in SEQ ID NOs: 944-945, 948, 967, 969-976, 991, 993-1002, 1004, 1006-1007, 1010, 1013-1019, 1021, 1023-1027;
   (d) sequences that hybridize to a sequence provided in SEQ ID NOs: 944-945, 948, 967, 969-976, 991, 993-1002, 1004, 1006-1007, 1010, 1013-1019, 1021, 1023-1027, under highly stringent conditions;
   (e) sequences having at least 75% identity to a sequence of SEQ ID NOs: 944-945, 948, 967, 969-976, 991, 993-1002, 1004, 1006-1007, 1010, 1013-1019, 1021, 1023-1027;
   (f) sequences having at least 90% identity to a sequence of SEQ ID NOs: 944-945, 948, 967, 969-976, 991, 993-1002, 1004, 1006-1007, 1010, 1013-1019, 1021, 1023-1027; and
   (g) degenerate variants of a sequence provided in SEQ ID NOs: 944-945, 948, 967, 969-976, 991, 993-1002, 1004, 1006-1007, 1010, 1013-1019, 1021, 1023-1027.
2. An isolated polypeptide comprising an amino acid sequence selected from the group consisting of:
   (a) sequences encoded by a polynucleotide of paragraph 1; and
   (b) sequences having at least 70% identity to a sequence encoded by a polynucleotide of paragraph 1;
   (c) sequences having at least 90% identity to a sequence encoded by a polynucleotide of paragraph 1;
   (d) sequences provided in SEQ ID NOs:946-947, 949-966, 968, 977-990, 992, 1003, 1005, 1008-1009, 1011-1012, 1020, 1022, 1028-1029, and 1030-1033;
   (e) sequences having at least 70% identity to a sequence of SEQ ID NOs:946-947, 949-966, 968, 977-990, 992, 1003, 1005, 1008-1009, 1011-1012, 1020, 1022, 1028-1029, and 1030-1033;
   (f) sequences having at least 90% identity to a sequence of SEQ ID NOs:946-947, 949-966, 968, 977-990, 992, 1003, 1005, 1008-1009, 1011-1012, 1020, 1022, 1028-1029, and 1030-1033;
   (g) sequences consisting of at least 5 contiguous amino acids of a sequence provided in SEQ ID NOs:946-947, 949-966, 968, 977-990, 992, 1003, 1005, 1008-1009, 1011-1012, 1020, 1022, 1028-1029, and 1030-1033;
   (h) sequences consisting of at least 10 contiguous amino acids of a sequence provided in SEQ ID NOs:946-947, 949-966, 968, 977-990, 992, 1003, 1005, 1008-1009, 1011-1012, 1020, 1022, 1028-1029, and 1030-1033; and
   (i) sequences consisting of at least 20 contiguous amino acids of a sequence provided in SEQ ID NOs:946-947, 949-966, 968, 977-990, 992, 1003, 1005, 1008-1009, 1011-1012, 1020, 1022, 1028-1029, and 1030-1033.
3. An expression vector comprising a polynucleotide of paragraph 1 operably linked to an expression control sequence.
4. A host cell transformed or transfected with an expression vector according to paragraph 3.
5. An isolated antibody, or antigen-binding fragment thereof, that specifically binds to a polypeptide of paragraph 2.
6. A method for detecting the presence of a cancer in a patient, comprising the steps of:
   (a) obtaining a biological sample from the patient;
   (b) contacting the biological sample with a binding agent that binds to a polypeptide of paragraph 2;
   (c) detecting in the sample an amount of polypeptide that binds to the binding agent; and
   (d) comparing the amount of polypeptide to a predetermined cut-off value and therefrom determining the presence of a cancer in the patient.
7. A fusion protein comprising at least one polypeptide according to paragraph 2.
8. An oligonucleotide that hybridizes to a sequence recited in SEQ ID NOs: 944-945, 948, 967, 969-976, 991, 993-1002, 1004, 1006-1007, 1010, 1013-1019, 1021, 1023-1027 under highly stringent conditions.
9. A method for stimulating and/or expanding T cells specific for a tumor protein, comprising contacting T cells with at least one component selected from the group consisting of:
   (a) polypeptides according to paragraph 2;
   (b) polynucleotides according to paragraph 1; and
   (c) antigen-presenting cells that express a polynucleotide according to paragraph 1,
      under conditions and for a time sufficient to permit the stimulation and/or expansion of T cells.
10. An isolated T cell population, comprising T cells prepared according to the method of paragraph 9.
11. A composition comprising a first component selected from the group consisting of physiologically acceptable carriers and immunostimulants, and a second component selected from the group consisting of:
   (a) polypeptides according to paragraph 2;
   (b) polynucleotides according to paragraph 1;
   (c) antibodies according to paragraph 5;
   (d) fusion proteins according to paragraph 7;
   (e) T cell populations according to paragraph 10; and
   (f) antigen presenting cells that express a polypeptide according to paragraph 2.
12. A method for stimulating an immune response in a patient, comprising administering to the patient a composition of paragraph 11.
13. A method for the treatment of a prostate cancer in a patient, comprising administering to the patient a composition of paragraph 11.
14. A method for determining the presence of a cancer in a patient, comprising the steps of:
   (a) obtaining a biological sample from the patient;
   (b) contacting the biological sample with an oligonucleotide according to paragraph 8;
   (c) detecting in the sample an amount of a polynucleotide that hybridizes to the oligonucleotide; and
   (d) comparing the amount of polynucleotide that hybridizes to the oligonucleotide to a predetermined cut-off value, and therefrom determining the presence of the cancer in the patient.
15. A diagnostic kit comprising at least one oligonucleotide according to paragraph 8.
16. A diagnostic kit comprising at least one antibody according to paragraph 5 and a detection reagent, wherein the detection reagent comprises a reporter group.
17. A method for the treatment of prostate cancer in a patient, comprising the steps of:
   (a) incubating CD4+ and/or CD8+ T cells isolated from a patient with at least one component selected from the group consisting of: (i) polypeptides according to paragraph 2; (ii) polynucleotides according to paragraph 1; and (iii) antigen presenting cells that express a polypeptide of paragraph 2, such that T cell proliferate;
   (b) administering to the patient an effective amount of the proliferated T cells,
      and thereby inhibiting the development of a cancer in the patient.

### EXAMPLES

### EXAMPLE 1

### ISOLATION AND CHARACTERIZATION OF PROSTATE-SPECIFIC POLYPEPTIDES

This Example describes the isolation of certain prostate-specific polypeptides from a prostate tumor cDNA library.

A human prostate tumor cDNA expression library was constructed from prostate tumor poly A⁺ RNA using a Superscript Plasmid System for cDNA Synthesis and Plasmid Cloning kit (BRL Life Technologies, Gaithersburg, MD 20897) following the manufacturer's protocol. Specifically, prostate tumor tissues were homogenized with polytron (Kinematica, Switzerland) and total RNA was extracted using Trizol reagent (BRL Life Technologies) as directed by the manufacturer. The poly A⁺ RNA was then purified using a Qiagen oligotex spin column mRNA purification kit (Qiagen, Santa Clarita, CA 91355) according to the manufacturer's protocol. First-strand cDNA was synthesized using the Notl/Oligo-dT18 primer. Double-stranded cDNA was synthesized, ligated with EcoRI/BAXI adaptors (Invitrogen, San Diego, CA) and digested with Notl. Following size fractionation with Chroma Spin-1000 columns (Clontech, Palo Alto, CA), the cDNA was ligated into the EcoRI/NotI site of pCDNA3.1 (Invitrogen) and transformed into ElectroMax *E. coli* DH10B cells (BRL Life Technologies) by electroporation.

Using the same procedure, a normal human pancreas cDNA expression library was prepared from a pool of six tissue specimens (Clontech). The cDNA libraries were characterized by determining the number of independent colonies, the percentage of clones that carried insert, the average insert size and by sequence analysis. The prostate tumor library contained 1.64 x 10⁷ independent colonies, with 70% of clones having an insert and the average insert size being 1745 base pairs. The normal pancreas cDNA library contained 3.3 x 10⁶ independent colonies, with 69% of clones having inserts and the average insert size being 1120 base pairs. For both libraries, sequence analysis showed that the majority of clones had a full length cDNA sequence and were synthesized from mRNA, with minimal rRNA and mitochondrial DNA contamination.

cDNA library subtraction was performed using the above prostate tumor and normal pancreas cDNA libraries, as described by Hara et al. (Blood, 84:189-199, 1994) with some modifications. Specifically, a prostate tumor-specific subtracted cDNA library was generated as follows. Normal pancreas cDNA library (70 µg) was digested with EcoRI, Notl, and Sful, followed by a filling-in reaction with DNA polymerase Klenow fragment. After phenolchloroform extraction and ethanol precipitation, the DNA was dissolved in 100 µl of H₂O, heat-denatured and mixed with 100 µl (100 µg) of Photoprobe biotin (Vector Laboratories, Burlingame, CA). As recommended by the manufacturer, the resulting mixture was irradiated with a 270 W sunlamp on ice for 20 minutes. Additional Photoprobe biotin (50 µl) was added and the biotinylation reaction was repeated. After extraction with butanol five times, the DNA was ethanol-precipitated and dissolved in 23 µl H₂O to form the driver DNA.

To form the tracer DNA, 10 µg prostate tumor cDNA library was digested with BamHI and Xhol, phenol chloroform extracted and passed through Chroma spin-400 columns (Clontech). Following ethanol precipitation, the tracer DNA was dissolved in 5 µl H₂O. Tracer DNA was mixed with 15 µl driver DNA and 20 µl of 2 x hybridization buffer (1.5 M NaCl/10 mM EDTA/50 mM HEPES pH 7.5/0.2% sodium dodecyl sulfate), overlaid with mineral oil, and heat-denatured completely. The sample was immediately transferred into a 68 °C water bath and incubated for 20 hours (long hybridization [LH]). The reaction mixture was then subjected to a streptavidin treatment followed by phenol/chloroform extraction. This process was repeated three more times. Subtracted DNA was precipitated, dissolved in 12 µl H₂O, mixed with 8 µl driver DNA and 20 µl of 2 x hybridization buffer, and subjected to a hybridization at 68 °C for 2 hours (short hybridization [SH]). After removal of biotinylated double-stranded DNA, subtracted cDNA was ligated into BamHl/Xhol site of chloramphenicol resistant _{P}BCSK⁺ (Stratagene, La Jolla, CA 92037) and transformed into ElectroMax *E*. *coli* DH10B cells by electroporation to generate a prostate tumor specific subtracted cDNA library (referred to as "prostate subtraction 1 ").

To analyze the subtracted cDNA library, plasmid DNA was prepared from 100 independent clones, randomly picked from the subtracted prostate tumor specific library and grouped based on insert size. Representative cDNA clones were further characterized by DNA sequencing with a Perkin Elmer/Applied Biosystems Division Automated Sequencer Model 373A (Foster City, CA). Six cDNA clones, hereinafter referred to as F1-13, F1-12, F1-16, H1-1, H1-9 and H1-4, were shown to be abundant in the subtracted prostate-specific cDNA library. The determined 3' and 5' cDNA sequences for F1-12 are provided in SEQ ID NO: 2 and 3, respectively, with determined 3' cDNA sequences for F1-13, F1-16, H1-1, H1-9 and H1-4 being provided in SEQ ID NO: 1 and 4-7, respectively.

The cDNA sequences for the isolated clones were compared to known sequences in the gene bank using the EMBL and GenBank databases (release 96). Four of the prostate tumor cDNA clones, F1-13, F1-16, H1-1, and H1-4, were determined to encode the following previously identified proteins: prostate specific antigen (PSA), human glandular kallikrein, human tumor expression enhanced gene, and mitochondria cytochrome C oxidase subunit II. H1-9 was found to be identical to a previously identified human autonomously replicating sequence. No significant homologies to the cDNA sequence for F1-12 were found.

Subsequent studies led to the isolation of a full-length cDNA sequence for F1-12 (also referred to as P504S). This sequence is provided in SEQ ID NO: 107, with the corresponding amino acid sequence being provided in SEQ ID NO: 108. cDNA splice variants of P504S are provided in SEQ ID NO: 600-605.

To clone less abundant prostate tumor specific genes, cDNA library subtraction was performed by subtracting the prostate tumor cDNA library described above with the normal pancreas cDNA library and with the three most abundant genes in the previously subtracted prostate tumor specific cDNA library: human glandular kallikrein, prostate specific antigen (PSA), and mitochondria cytochrome C oxidase subunit II. Specifically, 1 µg each of human glandular kallikrein, PSA and mitochondria cytochrome C oxidase subunit II cDNAs in pCDNA3.1 were added to the driver DNA and subtraction was performed as described above to provide a second subtracted cDNA library hereinafter referred to as the "subtracted prostate tumor specific cDNA library with spike".

Twenty-two cDNA clones were isolated from the subtracted prostate tumor specific cDNA library with spike. The determined 3' and 5' cDNA sequences for the clones referred to as J1-17, L1-12, N1-1862, J1-13, J1-19, J1-25, J1-24, K1-58, K1-63, L1-4 and L1-14 are provided in SEQ ID NO: 8-9, 10-11, 12-13, 14-15, 16-17, 18-19, 20-21, 22-23, 24-25, 26-27 and 28-29, respectively. The determined 3' cDNA sequences for the clones referred to as J1-12, J1-16, J1-21, K1-48, K1-55, L1-2, L1-6, N1-1858, N1-1860, N1-1861, N1-1864 are provided in SEQ ID NO: 30-40, respectively. Comparison of these sequences with those in the gene bank as described above, revealed no significant homologies to three of the five most abundant DNA species, (J1-17, L1-12 and N1-1862; SEQ ID NO: 8-9, 10-11 and 12-13, respectively). Of the remaining two most abundant species, one (J1-12; SEQ ID NO:30) was found to be identical to the previously identified human pulmonary surfactant-associated protein, and the other (K1-48; SEQ ID NO:33) was determined to have some homology to R. *norvegicus* mRNA for 2-arylpropionyl-CoA epimerase. Of the 17 less abundant cDNA clones isolated from the subtracted prostate tumor specific cDNA library with spike, four (J1-16, K1-55, L1-6 and N1-1864; SEQ ID NO:31, 34, 36 and 40, respectively) were found to be identical to previously identified sequences, two (J1-21 and N1-1860; SEQ ID NO: 32 and 38, respectively) were found to show some homology to non-human sequences, and two (L1-2 and N1-1861; SEQ ID NO: 35 and 39, respectively) were found to show some homology to known human sequences. No significant homologies were found to the polypeptides J1-13, J1-19, J1-24, J1-25, K1-58, K1-63, L1-4, L1-14 (SEQ ID NO: 14-15, 16-17, 20-21, 18-19, 22-23, 24-25, 26-27, 28-29, respectively).

Subsequent studies led to the isolation of full length cDNA sequences for J1-17, L1-12 and N1-1862 (SEQ ID NO: 109-111, respectively). The corresponding amino acid sequences are provided in SEQ ID NO: 112-114. L1-12 is also referred to as P501S. A cDNA splice variant of P501 S is provided in SEQ ID NO: 606.

As described below, N1-1862 (also referred to as P503S; SEQ ID NO: 111) was found to show prostate-specific expression. Immunohistochemistry and flow cytometry analysis indicated that P503S is a plasma membrane-associated molecule. P503S contains four transmembrane domains predicted to be encoded by amino acids 59-75, 90-106, 107-123 and 218-234. P503S contains a cleavable N-terminal signal sequence at amino acids 1-29. The N-terminus of P503S encoding amino acids 1-58 is internal, amino acids 76-89 are extracellular, amino acids 124-217 are extracellular and amino acids 235-241 are intracellular. Potential N-linked glycosylation sites are at amino acids N-141, N154, N-178 and N-184. Glycosylation of P503S has been confirmed experimentally using enzymes that selectively strip sugar residues from protein and Western blot analysis. P503S is a member of a family of molecules collectively known as the tetraspanin family or TM4SF (transmembrane 4 superfamily). This family of proteins has been shown to interact, and homo and heterodimerize with, other family members. Furthermore, the TM4SF family members associate with a family of adhesion molecules known as integrins. TM4SF family members have been shown to regulate cell adhesion as well as cell proliferation by controlling signal transduction pathways associated with these physiological responses.

In a further experiment, four additional clones were identified by subtracting a prostate tumor cDNA library with normal prostate cDNA prepared from a pool of three normal prostate poly A+ RNA (referred to as "prostate subtraction 2"). The determined cDNA sequences for these clones, hereinafter referred to as U1-3064, U1-3065, V1-3692 and 1A-3905, are provided in SEQ ID NO: 69-72, respectively. Comparison of the determined sequences with those in the gene bank revealed no significant homologies to U1-3065.

A second subtraction with spike (referred to as "prostate subtraction spike 2") was performed by subtracting a prostate tumor specific cDNA library with spike with normal pancreas cDNA library and further spiked with PSA, J1-17, pulmonary surfactant-associated protein, mitochondrial DNA, cytochrome c oxidase subunit II, N1-1862, autonomously replicating sequence, L1-12 and tumor expression enhanced gene. Four additional clones, hereinafter referred to as V1-3686, R1-2330, 1 B-3976 and V1-3679, were isolated. The determined cDNA sequences for these clones are provided in SEQ ID NO:73-76, respectively. Comparison of these sequences with those in the gene bank revealed no significant homologies to V1-3686 and R1-2330.

Further analysis of the three prostate subtractions described above (prostate subtraction 2, subtracted prostate tumor specific cDNA library with spike, and prostate subtraction spike 2) resulted in the identification of sixteen additional clones, referred to as 1 G-4736, 1 G-4738, 1 G-4741, 1 G-4744, 1G-4734, 1 H-4774, 1 H-4781, 1 H-4785, 1 H-4787, 1 H-4796, 1I-4810, 1I-4811, 1 J-4876, 1 K-4884 and 1 K-4896. The determined cDNA sequences for these clones are provided in SEQ ID NO: 77-92, respectively. Comparison of these sequences with those in the gene bank as described above, revealed no significant homologies to 1 G-4741, 1 G-4734, 11-4807, 1 J-4876 and 1 K-4896 (SEQ ID NO: 79, 81, 87, 90 and 92, respectively). Further analysis of the isolated clones led to the determination of extended cDNA sequences for 1 G-4736, 1G-4738, 1G-4741, 1G-4744, 1H-4774, 1H-4781, 1H-4785, 1H-4787, 1H-4796, 1I-4807, 1 J-4876, 1 K-4884 and 1 K-4896, provided in SEQ ID NO: 179-188 and 191-193, respectively, and to the determination of additional partial cDNA sequences for 11-4810 and 11-4811, provided in SEQ ID NO: 189 and 190, respectively.

Additional studies with prostate subtraction spike 2 resulted in the isolation of three more clones. Their sequences were determined as described above and compared to the most recent GenBank. All three clones were found to have homology to known genes, which are Cysteine-rich protein, KIAA0242, and KIAA0280 (SEQ ID NO: 317, 319, and 320, respectively). Further analysis of these clones by Synteni microarray (Synteni, Palo Alto, CA) demonstrated that all three clones were over-expressed in most prostate tumors and prostate BPH, as well as in the majority of normal prostate tissues tested, but low expression in all other normal tissues.

An additional subtraction was performed by subtracting a normal prostate cDNA library with normal pancreas cDNA (referred to as "prostate subtraction 3"). This led to the identification of six additional clones referred to as 1 G-4761, 1 G-4762, 1 H-4766, 1 H-4770, 1 H-4771 and 1 H-4772 (SEQ ID NO: 93-98). Comparison of these sequences with those in the gene bank revealed no significant homologies to 1G-4761 and 1H-4771 (SEQ ID NO: 93 and 97, respectively). Further analysis of the isolated clones led to the determination of extended cDNA sequences for 1 G-4761, 1 G-4762, 1 H-4766 and 1 H-4772 provided in SEQ ID NO: 194-196 and 199, respectively, and to the determination of additional partial cDNA sequences for 1 H-4770 and 1 H-4771, provided in SEQ ID NO: 197 and 198, respectively.

Subtraction of a prostate tumor cDNA library, prepared from a pool of polyA+ RNA from three prostate cancer patients, with a normal pancreas cDNA library (prostate subtraction 4) led to the identification of eight clones, referred to as 1 D-4297, 1 D-4309, 1 D.1-4278, 1 D-4288, 1 D-4283, 1 D-4304, 1D-4296 and 1D-4280 (SEQ ID NO: 99-107). These sequences were compared to those in the gene bank as described above. No significant homologies were found to 1 D-4283 and 1 D-4304 (SEQ ID NO: 103 and 104, respectively). Further analysis of the isolated clones led to the determination of extended cDNA sequences for 1 D-4309, 1 D.1-4278, 1 D-4288, 1 D-4283, 1 D-4304, 1 D-4296 and 1 D-4280, provided in SEQ ID NO: 200-206, respectively.

cDNA clones isolated in prostate subtraction 1 and prostate subtraction 2, described above, were colony PCR amplified and their mRNA expression levels in prostate tumor, normal prostate and in various other normal tissues were determined using microarray technology (Synteni, Palo Alto, CA). Briefly, the PCR amplification products were dotted onto slides in an array format, with each product occupying a unique location in the array. mRNA was extracted from the tissue sample to be tested, reverse transcribed, and fluorescent-labeled cDNA probes were generated. The microarrays were probed with the labeled cDNA probes, the slides scanned and fluorescence intensity was measured. This intensity correlates with the hybridization intensity. Two clones (referred to as P509S and P510S) were found to be over-expressed in prostate tumor and normal prostate and expressed at low levels in all other normal tissues tested (liver, pancreas, skin, bone marrow, brain, breast, adrenal gland, bladder, testes, salivary gland, large intestine, kidney, ovary, lung, spinal cord, skeletal muscle and colon). The determined cDNA sequences for P509S and P510S are provided in SEQ ID NO: 223 and 224, respectively. Comparison of these sequences with those in the gene bank as described above, revealed some homology to previously identified ESTs.

Additional, studies led to the isolation of the full-length cDNA sequence for P509S. This sequence is provided in SEQ ID NO: 332, with the corresponding amino acid sequence being provided in SEQ ID NO: 339. Two variant full-length cDNA sequences for P510S are provided in SEQ ID NO: 535 and 536, with the corresponding amino acid sequences being provided in SEQ ID NO: 537 and 538, respectively. Additional splice variants of P510S are provided in SEQ ID NO: 598 and 599.

P510S was found to be expressed in a prostate-specific manner. Immunohistochemistry and flow cytometry analysis indicated that P510S is a plasma membrane-associated molecule. Based on sequence similarity to other multidrug resistance-associated transporter proteins, P510S is predicted to be a cell surface expressed drug transport protein that contains an ATP binding cassette. P510S contains nine transmembrane domains predicted to be encoded by amino acids 135-151, 233-249, 330-346, 351-367, 711-727, 775-791, 850-866, 867-883 and 954-970. The C-terminus of P510S is predicted to be intracellular. Potential N-linked glycosylation sites are at amino acids N-651, N-690, N-746 and N754, N792, N-1176 and N-1312.

The determined cDNA sequences for additional prostate-specific clones isolated during characterization of prostate specific cDNA libraries are provided in SEQ ID NO: 618-689, 691-697 and 709-772. Comparison of these sequences with those in the public databases revealed no significant homologies to any of these sequences.

### EXAMPLE 2

### DETERMINATION OF TISSUE SPECIFICITY OF PROSTATE-SPECIFIC POLYPEPTIDES

Using gene specific primers, mRNA expression levels for the representative prostate-specific polypeptides F1-16, H1-1, J1-17 (also referred to as P502S), L1-12 (also referred to as P501S), F1-12 (also referred to as P504S) and N1-1862 (also referred to as P503S) were examined in a variety of normal and tumor tissues using RT-PCR.

Briefly, total RNA was extracted from a variety of normal and tumor tissues using Trizol reagent as described above. First strand synthesis was carried out using 1-2 µg of total RNA with SuperScript II reverse transcriptase (BRL Life Technologies) at 42 °C for one hour. The cDNA was then amplified by PCR with gene-specific primers. To ensure the semi-quantitative nature of the RT-PCR, β-actin was used as an internal control for each of the tissues examined. First, serial dilutions of the first strand cDNAs were prepared and RT-PCR assays were performed using β-actin specific primers. A dilution was then chosen that enabled the linear range amplification of the β-actin template and which was sensitive enough to reflect the differences in the initial copy numbers. Using these conditions, the β-actin levels were determined for each reverse transcription reaction from each tissue. DNA contamination was minimized by DNase treatment and by assuring a negative PCR result when using first strand cDNA that was prepared without adding reverse transcriptase.

mRNA Expression levels were examined in four different types of tumor tissue (prostate tumor from 2 patients, breast tumor from 3 patients, colon tumor, lung tumor), and sixteen different normal tissues, including prostate, colon, kidney, liver, lung, ovary, pancreas, skeletal muscle, skin, stomach, testes, bone marrow and brain. F1-16 was found to be expressed at high levels in prostate tumor tissue, colon tumor and normal prostate, and at lower levels in normal liver, skin and testes, with expression being undetectable in the other tissues examined. H1-1 was found to be expressed at high levels in prostate tumor, lung tumor, breast tumor, normal prostate, normal colon and normal brain, at much lower levels in normal lung, pancreas, skeletal muscle, skin, small intestine, bone marrow, and was not detected in the other tissues tested. J1-17 (P502S) and L1-12 (P501S) appear to be specifically over-expressed in prostate, with both genes being expressed at high levels in prostate tumor and normal prostate but at low to undetectable levels in all the other tissues examined. N1-1862 (P503S) was found to be over-expressed in 60% of prostate tumors and detectable in normal colon and kidney. The RT-PCR results thus indicate that F1-16, H1-1, J1-17 (P502S), N1-1862 (P503S) and L1-12 (P501 S) are either prostate specific or are expressed at significantly elevated levels in prostate.

Further RT-PCR studies showed that F1-12 (P504S) is over-expressed in 60% of prostate tumors, detectable in normal kidney but not detectable in all other tissues tested. Similarly, R1-2330 was shown to be over-expressed in 40% of prostate tumors, detectable in normal kidney and liver, but not detectable in all other tissues tested. U1-3064 was found to be over-expressed in 60% of prostate tumors, and also expressed in breast and colon tumors, but was not detectable in normal tissues.

RT-PCR characterization of R1-2330, U1-3064 and 1D-4279 showed that these three antigens are over-expressed in prostate and/or prostate tumors.

Northern analysis with four prostate tumors, two normal prostate samples, two BPH prostates, and normal colon, kidney, liver, lung, pancrease, skeletal muscle, brain, stomach, testes, small intestine and bone marrow, showed that L1-12 (P501S) is over-expressed in prostate tumors and normal prostate, while being undetectable in other normal tissues tested. J1-17 (P502S) was detected in two prostate tumors and not in the other tissues tested. N1-1862 (P503S) was found to be over-expressed in three prostate tumors and to be expressed in normal prostate, colon and kidney, but not in other tissues tested. F1-12 (P504S) was found to be highly expressed in two prostate tumors and to be undetectable in all other tissues tested.

The microarray technology described above was used to determine the expression levels of representative antigens described herein in prostate tumor, breast tumor and the following normal tissues: prostate, liver, pancreas, skin, bone marrow, brain, breast, adrenal gland, bladder, testes, salivary gland, large intestine, kidney, ovary, lung, spinal cord, skeletal muscle and colon. L1-12 (P501S) was found to be over-expressed in normal prostate and prostate tumor, with some expression being detected in normal skeletal muscle. Both J1-12 and F1-12 (P504S) were found to be over-expressed in prostate tumor, with expression being lower or undetectable in all other tissues tested. N1-1862 (P503S) was found to be expressed at high levels in prostate tumor and normal prostate, and at low levels in normal large intestine and normal colon, with expression being undetectable in all other tissues tested. R1-2330 was found to be over-expressed in prostate tumor and normal prostate, and to be expressed at lower levels in all other tissues tested. 1 D-4279 was found to be over-expressed in prostate tumor and normal prostate, expressed at lower levels in normal spinal cord, and to be undetectable in all other tissues tested.

Further microarray analysis to specifically address the extent to which P501S (SEQ ID NO: 110) was expressed in breast tumor revealed moderate over-expression not only in breast tumor, but also in metastatic breast tumor (2/31), with negligible to low expression in normal tissues. This data suggests that P501 S may be over-expressed in various breast tumors as well as in prostate tumors.

The expression levels of 32 ESTs (expressed sequence tags) described by Vasmatzis et al. (Proc. Nat'l Acad. Sci. USA 95:300-304, 1998) in a variety of tumor and normal tissues were examined by microarray technology as described above. Two of these clones (referred to as P1000C and P1001C) were found to be over-expressed in prostate tumor and normal prostate, and expressed at low to undetectable levels in all other tissues tested (normal aorta, thymus, resting and activated PBMC, epithelial cells, spinal cord, adrenal gland, fetal tissues, skin, salivary gland, large intestine, bone marrow, liver, lung, dendritic cells, stomach, lymph nodes, brain, heart, small intestine, skeletal muscle, colon and kidney. The determined cDNA sequences for P1000C and P1001C are provided in SEQ ID NO: 384 and 472, respectively. The sequence of P1001C was found to show some homology to the previously isolated Human mRNA for JM27 protein. Subsequent comparison of the sequence of SEQ ID NO: 384 with sequences in the public databases, led to the identification of a full-length cDNA sequence of P1000C (SEQ ID NO: 929), which encodes a 492 amino acid sequence. Analysis of the amino acid sequence using the PSORT II program led to the identification of a putative transmembrane domain from amino acids 84-100. The cDNA sequence of the open reading frame of P1000C, including the stop codon, is provided in SEQ ID NO: 930, with the open reading frame without the stop codon being provided in SEQ ID NO: 931. The full-length amino acid sequence of P1000C is provided in SEQ ID NO: 932. SEQ ID NO: 933 and 934 represent amino acids 1-100 and 100-492 of P1000C, respectively.

The expression of the polypeptide encoded by the full length cDNA sequence for F1-12 (also referred to as P504S; SEQ ID NO: 108) was investigated by immunohistochemical analysis. Rabbit-anti-P504S polyclonal antibodies were generated against the full length P504S protein by standard techniques. Subsequent isolation and characterization of the polyclonal antibodies were also performed by techniques well known in the art. Immunohistochemical analysis showed that the P504S polypeptide was expressed in 100% of prostate carcinoma samples tested (n=5).

The rabbit-anti-P504S polyclonal antibody did not appear to label benign prostate cells with the same cytoplasmic granular staining, but rather with light nuclear staining. Analysis of normal tissues revealed that the encoded polypeptide was found to be expressed in some, but not all normal human tissues. Positive cytoplasmic staining with rabbit-anti-P504S polyclonal antibody was found in normal human kidney, liver, brain, colon and lung-associated macrophages, whereas heart and bone marrow were negative.

This data indicates that the P504S polypeptide is present in prostate cancer tissues, and that there are qualitative and quantitative differences in the staining between benign prostatic hyperplasia tissues and prostate cancer tissues, suggesting that this polypeptide may be detected selectively in prostate tumors and therefore be useful in the diagnosis of prostate cancer.

### EXAMPLE 3

### ISOLATION AND CHARACTERIZATION OF PROSTATE-SPECIFIC POLYPEPTIDES BY PCR-BASED SUBTRACTION

A cDNA subtraction library, containing cDNA from normal prostate subtracted with ten other normal tissue cDNAs (brain, heart, kidney, liver, lung, ovary, placenta, skeletal muscle, spleen and thymus) and then submitted to a first round of PCR amplification, was purchased from Clontech. This library was subjected to a second round of PCR amplification, following the manufacturer's protocol. The resulting cDNA fragments were subcloned into the vector pT7 Blue T-vector (Novagen, Madison, WI) and transformed into XL-1 Blue MRF' *E*. coli (Stratagene). DNA was isolated from independent clones and sequenced using a Perkin Elmer/Applied Biosystems Division Automated Sequencer Model 373A.

Fifty-nine positive clones were sequenced. Comparison of the DNA sequences of these clones with those in the gene bank, as described above, revealed no significant homologies to 25 of these clones, hereinafter referred to as P5, P8, P9, P18, P20, P30, P34, P36, P38, P39, P42, P49, P50, P53, P55, P60, P64, P65, P73, P75, P76, P79 and P84. The determined cDNA sequences for these clones are provided in SEQ ID NO: 41-45, 47-52 and 54-65, respectively. P29, P47, P68, P80 and P82 (SEQ ID NO: 46, 53 and 66-68, respectively) were found to show some degree of homology to previously identified DNA sequences. To the best of the inventors' knowledge, none of these sequences have been previously shown to be present in prostate.

Further studies employing the sequence of SEQ ID NO: 67 as a probe in standard full-length cloning methods, resulted in the isolation of three cDNA sequences which appear to be splice variants of P80 (also known as P704P). These sequences are provided in SEQ ID NO: 699-701.

Further studies using the PCR-based methodology described above resulted in the isolation of more than 180 additional clones, of which 23 clones were found to show no significant homologies to known sequences. The determined cDNA sequences for these clones are provided in SEQ ID NO: 115-123, 127, 131, 137, 145, 147-151, 153, 156-158 and 160. Twenty-three clones (SEQ ID NO: 124-126, 128-130, 132-136, 138-144, 146, 152, 154, 155 and 159) were found to show some homology to previously identified ESTs. An additional ten clones (SEQ ID NO: 161-170) were found to have some degree of homology to known genes. Larger cDNA clones containing the P20 sequence represent splice variants of a gene referred to as P703P. The determined DNA sequence for the variants referred to as DE1, DE13 and DE14 are provided in SEQ ID NO: 171, 175 and 177, respectively, with the corresponding amino acid sequences being provided in SEQ ID NO: 172, 176 and 178, respectively. The determined cDNA sequence for an extended spliced form of P703 is provided in SEQ ID NO: 225. The DNA sequences for the splice variants referred to as DE2 and DE6 are provided in SEQ ID NO: 173 and 174, respectively.

mRNA Expression levels for representative clones in tumor tissues (prostate (n=5), breast (n=2), colon and lung) normal tissues (prostate (n=5), colon, kidney, liver, lung (n=2), ovary (n=2), skeletal muscle, skin, stomach, small intestine and brain), and activated and non-activated PBMC was determined by RT-PCR as described above. Expression was examined in one sample of each tissue type unless otherwise indicated.

P9 was found to be highly expressed in normal prostate and prostate tumor compared to all normal tissues tested except for normal colon which showed comparable expression. P20, a portion of the P703P gene, was found to be highly expressed in normal prostate and prostate tumor, compared to all twelve normal tissues tested. A modest increase in expression of P20 in breast tumor (n=2), colon tumor and lung tumor was seen compared to all normal tissues except lung (1 of 2). Increased expression of P18 was found in normal prostate, prostate tumor and breast tumor compared to other normal tissues except lung and stomach. A modest increase in expression of P5 was observed in normal prostate compared to most other normal tissues. However, some elevated expression was seen in normal lung and PBMC. Elevated expression of P5 was also observed in prostate tumors (2 of 5), breast tumor and one lung tumor sample. For P30, similar expression levels were seen in normal prostate and prostate tumor, compared to six of twelve other normal tissues tested. Increased expression was seen in breast tumors, one lung tumor sample and one colon tumor sample, and also in normal PBMC. P29 was found to be over-expressed in prostate tumor (5 of 5) and normal prostate (5 of 5) compared to the majority of normal tissues. However, substantial expression of P29 was observed in normal colon and normal lung (2 of 2). P80 was found to be over-expressed in prostate tumor (5 of 5) and normal prostate (5 of 5) compared to all other normal tissues tested, with increased expression also being seen in colon tumor.

Further studies resulted in the isolation of twelve additional clones, hereinafter referred to as 10-d8, 10-h10, 11-c8, 7-g6, 8-b5, 8-b6, 8-d4, 8-d9, 8-g3, 8-h11, 9-f12 and 9-f3. The determined DNA sequences for 10-d8, 10-h10, 11-c8, 8-d4, 8-d9, 8-h11, 9-f12 and 9-f3 are provided in SEQ ID NO: 207, 208, 209, 216, 217, 220, 221 and 222, respectively. The determined forward and reverse DNA sequences for 7-g6, 8-b5, 8-b6 and 8-g3 are provided in SEQ ID NO: 210 and 211; 212 and 213; 214 and 215; and 218 and 219, respectively. Comparison of these sequences with those in the gene bank revealed no significant homologies to the sequence of 9-f3. The clones 10-d8, 11-c8 and 8-h11 were found to show some homology to previously isolated ESTs, while 10-h10, 8-b5, 8-b6, 8-d4, 8-d9, 8-g3 and 9-f12 were found to show some homology to previously identified genes. Further characterization of 7-G6 and 8-G3 showed identity to the known genes PAP and PSA, respectively.

mRNA expression levels for these clones were determined using the micro-array technology described above. The clones 7-G6, 8-G3, 8-B5, 8-B6, 8-D4, 8-D9, 9-F3, 9-F12, 9-H3, 10-A2, 10-A4, 11-C9 and 11-F2 were found to be over-expressed in prostate tumor and normal prostate, with expression in other tissues tested being low or undetectable. Increased expression of 8-F11 was seen in prostate tumor and normal prostate, bladder, skeletal muscle and colon. Increased expression of 10-H10 was seen in prostate tumor and normal prostate, bladder, lung, colon, brain and large intestine. Increased expression of 9-B1 was seen in prostate tumor, breast tumor, and normal prostate, salivary gland, large intestine and skin, with increased expression of 11-C8 being seen in prostate tumor, and normal prostate and large intestine.

An additional cDNA fragment derived from the PCR-based normal prostate subtraction, described above, was found to be prostate specific by both micro-array technology and RT-PCR. The determined cDNA sequence of this clone (referred to as 9-A11) is provided in SEQ ID NO: 226. Comparison of this sequence with those in the public databases revealed 99% identity to the known gene HOXB13.

Further studies led to the isolation of the clones 8-C6 and 8-H7. The determined cDNA sequences for these clones are provided in SEQ ID NO: 227 and 228, respectively. These sequences were found to show some homology to previously isolated ESTs.

PCR and hybridization-based methodologies were employed to obtain longer cDNA sequences for clone P20 (also referred to as P703P), yielding three additional cDNA fragments that progressively extend the 5' end of the gene. These fragments, referred to as P703PDE5, P703P6.26, and P703PX-23 (SEQ ID NO: 326, 328 and 330, with the corresponding amino acid sequences being provided in SEQ ID NO: 327, 329 and 331, respectively) contain additional 5' sequence. P703PDE5 was recovered by screening of a cDNA library (#141-26) with a portion of P703P as a probe. P703P6.26 was recovered from a mixture of three prostate tumor cDNAs and P703PX_23 was recovered from cDNA library (#438-48). Together, the additional sequences include all of the putative mature serine protease along with part of the putative signal sequence. The full-length cDNA sequence for P703P is provided in SEQ ID NO: 524, with the corresponding amino acid sequence being provided in SEQ ID NO: 525.

Using computer algorithms, the following regions of P703P were predicted to represent potential HLA A2-binding CTL epitopes: amino acids 164-172 of SEQ ID NO: 525 (SEQ ID NO: 866); amino acids 160-168 of SEQ ID NO: 525 (SEQ ID NO: 867); amino acids 239-247 of SEQ ID NO: 525 (SEQ ID NO: 868); amino acids 118-126 of SEQ ID NO: 525 (SEQ ID NO: 869); amino acids 112-120 of SEQ ID NO: 525 (SEQ ID NO: 870); amino acids 155-164 of SEQ ID NO: 525 (SEQ ID NO: 871); amino acids 117-126 of SEQ ID NO: 525 (SEQ ID NO: 872); amino acids 164-173 of SEQ ID NO: 525 (SEQ ID NO: 873); amino acids 154-163 of SEQ ID NO: 525 (SEQ ID NO: 874); amino acids 163-172 of SEQ ID NO: 525 (SEQ ID NO: 875); amino acids 58-66 of SEQ ID NO: 525 (SEQ ID NO: 876); and amino acids 59-67 of SEQ ID NO: 525 (SEQ ID NO: 877).

P703P was found to show some homology to previously identified proteases, such as thrombin. The thrombin receptor has been shown to be preferentially expressed in highly metastatic breast carcinoma cells and breast carcinoma biopsy samples. Introduction of thrombin receptor antisense cDNA has been shown to inhibit the invasion of metastatic breast carcinoma cells in culture. Antibodies against thrombin receptor inhibit thrombin receptor activation and thrombin-induced platelet activation. Furthermore, peptides that resemble the receptor's tethered ligand domain inhibit platelet aggregation by thrombin. P703P may play a role in prostate cancer through a protease-activated receptor on the cancer cell or on stromal cells. The potential trypsin-like protease activity of P703P may either activate a protease-activated receptor on the cancer cell membrane to promote tumorgenesis or activate a protease-activated receptor on the adjacent cells (such as stromal cells) to secrete growth factors and/or proteases (such as matrix metalloproteinases) that could promote tumor angiogenesis, invasion and metastasis. P703P may thus promote tumor progression and/or metastasis through the activation of protease-activated receptor. Polypeptides and antibodies that block the P703P-receptor interaction may therefore be usefully employed in the treatment of prostate cancer.

To determine whether P703P expression increases with increased severity of Gleason grade, an indicator of tumor stage, quantitative PCR analysis was performed on prostate tumor samples with a range of Gleason scores from 5 to > 8. The mean level of P703P expression increased with increasing Gleason score, indicating that P703P expression may correlate with increased disease severity.

Further studies using a PCR-based subtraction library of a prostate tumor pool subtracted against a pool of normal tissues (referred to as JP: PCR subtraction) resulted in the isolation of thirteen additional clones, seven of which did not share any significant homology to known GenBank sequences. The determined cDNA sequences for these seven clones (P711 P, P712P, novel 23, P774P, P775P, P710P and P768P) are provided in SEQ ID NO: 307-311, 313 and 315, respectively. The remaining six clones (SEQ ID NO: 316 and 321-325) were shown to share some homology to known genes. By microarray analysis, all thirteen clones showed three or more fold over-expression in prostate tissues, including prostate tumors, BPH and normal prostate as compared to normal non-prostate tissues. Clones P711 P, P712P, novel 23 and P768P showed over-expression in most prostate tumors and BPH tissues tested (n=29), and in the majority of normal prostate tissues (n=4), but background to low expression levels in all normal tissues. Clones P774P, P775P and P710P showed comparatively lower expression and expression in fewer prostate tumors and BPH samples, with negative to low expression in normal prostate.

Further studies led to the isolation of an extended cDNA sequence for P712P (SEQ ID NO: 552). The amino acid sequences encoded by 16 predicted open reading frames present within the sequence of SEQ ID NO: 552 are provided in SEQ ID NO: 553-568.

The full-length cDNA for P711 P was obtained by employing the partial sequence of SEQ ID NO: 307 to screen a prostate cDNA library. Specifically, a directionally cloned prostate cDNA library was prepared using standard techniques. One million colonies of this library were plated onto LB/Amp plates. Nylon membrane filters were used to lift these colonies, and the cDNAs which were picked up by these filters were denatured and cross-linked to the filters by UV light. The P711 P cDNA fragment of SEQ ID NO: 307 was radio-labeled and used to hybridize with these filters. Positive clones were selected, and cDNAs were prepared and sequenced using an automatic Perkin Elmer/Applied Biosystems sequencer. The determined full-length sequence of P711 P is provided in SEQ ID NO: 382, with the corresponding amino acid sequence being provided in SEQ ID NO: 383.

Using PCR and hybridization-based methodologies, additional cDNA sequence information was derived for two clones described above, 11-C9 and 9-F3, herein after referred to as P707P and P714P, respectively (SEQ ID NO: 333 and 334). After comparison with the most recent GenBank, P707P was found to be a splice variant of the known gene HoxB13. In contrast, no significant homologies to P714P were found. Further studies employing the sequence of SEQ ID NO: 334 as a probe in standard full-length cloning methods, resulted in an extended cDNA sequence for P714P. This sequence is provided in SEQ ID NO: 698. This sequence was found to show some homology to the gene that encodes human ribosomal L23A protein.

Clones 8-B3, P89, P98, P130 and P201 (as disclosed in U.S. Patent Application No. 09/020,956, filed February 9, 1998) were found to be contained within one contiguous sequence, referred to as P705P (cDNA sequence provided in SEQ ID NO: 335, with the amino acid sequence being provided in SEQ ID NO: 336), which was determined to be a splice variant of the known gene NKX 3.1. Subsequent studies led to the isolation of a corrected cDNA sequence for P705P. The coding region of this sequence is provided in SEQ ID NO: 948.

Further studies on P775P resulted in the isolation of four additional sequences (SEQ ID NO: 473-476) which are all splice variants of the P775P gene. The sequence of SEQ ID NO: 474 was found to contain two open reading frames (ORFs). The amino acid sequences encoded by these ORFs are provided in SEQ ID NO: 477 and 478. The cDNA sequence of SEQ ID NO: 475 was found to contain an ORF which encodes the amino acid sequence of SEQ ID NO: 479. The cDNA sequence of SEQ ID NO: 473 was found to contain four ORFs. The amino acid sequences encoded by these ORFs are provided in SEQ ID NO: 480-483. Additional splice variants of P775P are provided in SEQ ID NO: 593-597.

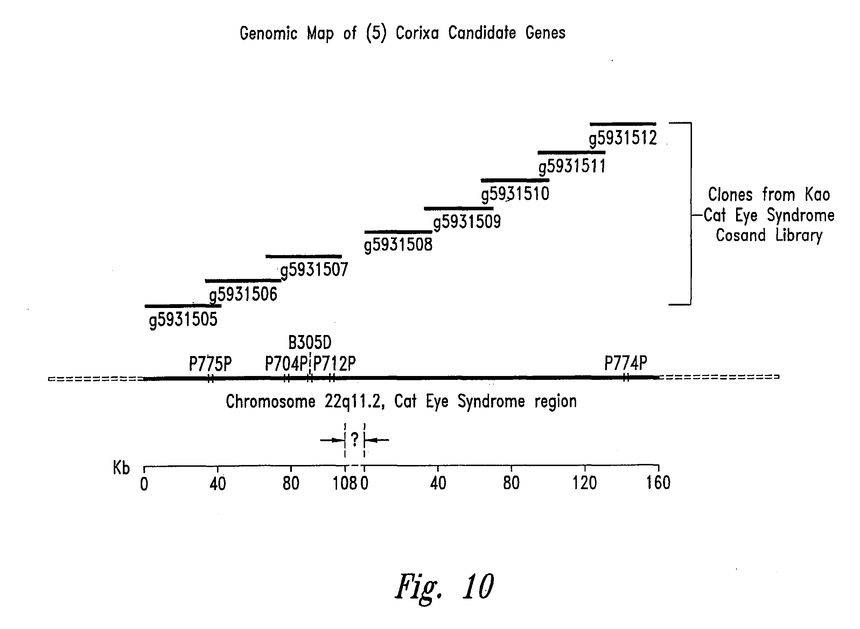

Subsequent studies led to the identification of a genomic region on chromosome 22q11.2, known as the Cat Eye Syndrome region, that contains the five prostate genes P704P, P712P, P774P, P775P and B305D. The relative location of each of these five genes within the genomic region is shown in Fig. 10. This region may therefore be associated with malignant tumors, and other potential tumor genes may be contained within this region. These studies also led to the identification of a potential open reading frame (ORF) for P775P (provided in SEQ ID NO: 533), which encodes the amino acid sequence of SEQ ID NO: 534.

Comparison of the clone of SEQ ID NO: 325 (referred to as P558S) with sequences in the GenBank and GeneSeq DNA databases showed that P558S is identical to the prostate-specific transglutaminase gene, which is known to have two forms. The full-length sequences for the two forms are provided in SEQ ID NO: 773 and 774, with the corresponding amino acid sequences being provided in SEQ ID NO: 775 and 776, respectively. The cDNA sequence of SEQ ID NO: 774 has a 15 pair base insert, resulting in a 5 amino acid insert in the corresponding amino acid sequence (SEQ ID NO: 776). This insert is not present in the sequence of SEQ ID NO: 773.

Further studies on P768P (SEQ ID NO: 315) led to the identification of the putative full-length open reading frame (ORF). The cDNA sequence of the ORF with stop codon is provided in SEQ ID NO: 907. The cDNA sequence of the ORF without stop codon is provided in SEQ ID NO: 908, with the corresponding amino acid sequence being provided in SEQ ID NO: 909. This sequence was found to show 86% identity to a rat calcium transporter protein, indicating that P768P may represent a human calcium transporter protein. The locations of transmembrane domains within P768P were predicted using the PSORT II computer algorithm. Six transmembrane domains were predicted at amino acid positions 118-134, 172-188, 211-227, 230-246, 282-298 and 348-364. The amino acid sequences of SEQ ID NO: 910-915 represent amino acids 1-134, 135-188, 189-227, 228-246, 247-298 and 299-511 of P768P, respectively.

### EXAMPLE 4

### SYNTHESIS OF POLYPEPTIDES

Polypeptides may be synthesized on a Perkin Elmer/Applied Biosystems 430A peptide synthesizer using FMOC chemistry with HPTU (O-Benzotriazole-N,N,N',N'-tetramethyluronium hexafluorophosphate) activation. A Gly-Cys-Gly sequence may be attached to the amino terminus of the peptide to provide a method of conjugation, binding to an immobilized surface, or labeling of the peptide. Cleavage of the peptides from the solid support may be carried out using the following cleavage mixture: trifluoroacetic acid:ethanedithiol:thioanisole:water:phenol (40:1:2:2:3). After cleaving for 2 hours, the peptides may be precipitated in cold methyl-t-butyl-ether. The peptide pellets may then be dissolved in water containing 0.1% trifluoroacetic acid (TFA) and lyophilized prior to purification by C18 reverse phase HPLC. A gradient of 0%-60% acetonitrile (containing 0.1% TFA) in water (containing 0.1% TFA) may be used to elute the peptides. Following lyophilization of the pure fractions, the peptides may be characterized using electrospray or other types of mass spectrometry and by amino acid analysis.

### EXAMPLE 5

### FURTHER ISOLATION AND CHARACTERIZATION OF PROSTATE-SPECIFIC POLYPEPTIDES BY PCR-BASED SUBTRACTION

A cDNA library generated from prostate primary tumor mRNA as described above was subtracted with cDNA from normal prostate. The subtraction was performed using a PCR-based protocol (Clontech), which was modified to generate larger fragments. Within this protocol, tester and driver double stranded cDNA were separately digested with five restriction enzymes that recognize six-nucleotide restriction sites (Mlul, Mscl, Pvull, Sall and Stul). This digestion resulted in an average cDNA size of 600 bp, rather than the average size of 300 bp that results from digestion with Rsal according to the Clontech protocol. This modification did not affect the subtraction efficiency. Two tester populations were then created with different adapters, and the driver library remained without adapters.

The tester and driver libraries were then hybridized using excess driver cDNA. In the first hybridization step, driver was separately hybridized with each of the two tester cDNA populations. This resulted in populations of (a) unhybridized tester cDNAs, (b) tester cDNAs hybridized to other tester cDNAs, (c) tester cDNAs hybridized to driver cDNAs and (d) unhybridized driver cDNAs. The two separate hybridization reactions were then combined, and rehybridized in the presence of additional denatured driver cDNA. Following this second hybridization, in addition to populations (a) through (d), a fifth population (e) was generated in which tester cDNA with one adapter hybridized to tester cDNA with the second adapter. Accordingly, the second hybridization step resulted in enrichment of differentially expressed sequences which could be used as templates for PCR amplification with adaptor-specific primers.

The ends were then filled in, and PCR amplification was performed using adaptor-specific primers. Only population (e), which contained tester cDNA that did not hybridize to driver cDNA, was amplified exponentially. A second PCR amplification step was then performed, to reduce background and further enrich differentially expressed sequences.

This PCR-based subtraction technique normalizes differentially expressed cDNAs so that rare transcripts that are overexpressed in prostate tumor tissue may be recoverable. Such transcripts would be difficult to recover by traditional subtraction methods.

In addition to genes known to be overexpressed in prostate tumor, seventy-seven further clones were identified. Sequences of these partial cDNAs are provided in SEQ ID NO: 29 to 305. Most of these clones had no significant homology to database sequences. Exceptions were JPTPN23 (SEQ ID NO: 231; similarity to pig valosin-containing protein), JPTPN30 (SEQ ID NO: 234; similarity to rat mRNA for proteasome subunit), JPTPN45 (SEQ ID NO: 243; similarity to rat *norvegicus* cytosolic NADP-dependent isocitrate dehydrogenase), JPTPN46 (SEQ ID NO: 244; similarity to human subclone H8 4 d4 DNA sequence), JP1 D6 (SEQ ID NO: 265; similarity to G. *gallus* dynein light chain-A), JP8D6 (SEQ ID NO: 288; similarity to human BAC clone RG016J04), JP8F5 (SEQ ID NO: 289; similarity to human subclone H8 3 b5 DNA sequence), and JP8E9 (SEQ ID NO: 299; similarity to human Alu sequence).

Additional studies using the PCR-based subtraction library consisting of a prostate tumor pool subtracted against a normal prostate pool (referred to as PT-PN PCR subtraction) yielded three additional clones. Comparison of the cDNA sequences of these clones with the most recent release of GenBank revealed no significant homologies to the two clones referred to as P715P and P767P (SEQ ID NO: 312 and 314). The remaining clone was found to show some homology to the known gene KIAA0056 (SEQ ID NO: 318). Using microarray analysis to measure mRNA expression levels in various tissues, all three clones were found to be over-expressed in prostate tumors and BPH tissues. Specifically, clone P715P was over-expressed in most prostate tumors and BPH tissues by a factor of three or greater, with elevated expression seen in the majority of normal prostate samples and in fetal tissue, but negative to low expression in all other normal tissues. Clone P767P was over-expressed in several prostate tumors and BPH tissues, with moderate expression levels in half of the normal prostate samples, and background to low expression in all other normal tissues tested.

Further analysis, by microarray as described above, of the PT-PN PCR subtraction library and of a DNA subtraction library containing cDNA from prostate tumor subtracted with a pool of normal tissue cDNAs, led to the isolation of 27 additional clones (SEQ ID NO: 340-365 and 381) which were determined to be over-expressed in prostate tumor. The clones of SEQ ID NO: 341, 342, 345, 347, 348, 349, 351, 355-359, 361, 362 and 364 were also found to be expressed in normal prostate. Expression of all 26 clones in a variety of normal tissues was found to be low or undetectable, with the exception of P544S (SEQ ID NO: 356) which was found to be expressed in small intestine. Of the 26 clones, 11 (SEQ ID NO: 340-349 and 362) were found to show some homology to previously identified sequences. No significant homologies were found to the clones of SEQ ID NO: 350, 351, 353-361, and 363-365.

Comparison of the sequence of SEQ ID NO: 362 with sequences in the GenBank and GeneSeq DNA databases showed that this clone (referred to as P788P) is identical to GeneSeq Accession No. X27262, which encodes a protein found in the GeneSeq protein Accession No. Y00931. The full length cDNA sequence of P788P is shown in SEQ ID NO: 777, with the corresponding amino acid being shown in SEQ ID NO: 778. Subsequently, a full-length cDNA sequence for P788P that contains polymorphisms not found in the sequence of SEQ ID NO: 779, was cloned multiple times by PCR amplification from cDNA prepared from several RNA templates from three individuals. This determined cDNA sequence of this polymorphic variant of P788P is provided in SEQ ID NO: 779, with the corresponding amino acid sequence being provided in SEQ ID NO: 780. The sequence of SEQ ID NO: 780 differs from that of SEQ ID NO: 778 by six amino acid residues. The P788P protein has 7 potential transmembrane domains at the C-terminal portion and is predicted to be a plasma membrane protein with an extracellular N-terminal region.

Further studies on the clone of SEQ ID NO: 352 (referred to as P790P) led to the isolation of the full-length cDNA sequence of SEQ ID NO: 526. The corresponding amino acid is provided in SEQ ID NO: 527. Data from two quantitative PCR experiments indicated that P790P is over-expressed in 11/15 tested prostate tumor samples and is expressed at low levels in spinal cord, with no expression being seen in all other normal samples tested. Data from further PCR experiments and microarray experiments showed over-expression in normal prostate and prostate tumor with little or no expression in other tissues tested. P790P was subsequently found to show significant homology to a previously identified G-protein coupled prostate tissue receptor.

Additional studies on the clone of SEQ ID NO: 354 (referred to as P776P) led to the isolation of an extended cDNA sequence, provided in SEQ ID NO: 569. The determined cDNA sequences of three additional splice variants of P776P are provided in SEQ ID NO: 570-572. The amino acid sequences encoded by two predicted open reading frames (ORFs) contained within SEQ ID NO: 570, one predicted ORF contained within SEQ ID NO: 571, and 11 predicted ORFs contained within SEQ ID NO: 569, are provided in SEQ ID NO: 573-586, respectively. Further studies led to the isolation of the full-length sequence for the clone of SEQ ID NO: 570 (provided in SEQ ID NO: 880). Full-length cloning efforts on the clone of SEQ ID NO: 571 led to the isolation of two sequences (provided in SEQ ID NO: 881 and 882), representing a single clone, that are identical with the exception of a polymorphic insertion/deletion at position 1293. Specifically, the clone of SEQ ID NO: 882 (referred to as clone F1) has a C at position 1293. The clone of SEQ ID NO: 881 (referred to as clone F2) has a single base pair deletion at position 1293. The predicted amino acid sequences encoded by 5 open reading frames located within SEQ ID NO: 880 are provided in SEQ ID NO: 883-887, with the predicted amino acid sequences encoded by the clone of SEQ ID NO: 881 and 882 being provided in SEQ ID NO: 888-893.

Comparison of the cDNA sequences for the clones P767P (SEQ ID NO: 314) and P777P (SEQ ID NO: 350) with sequences in the GenBank human EST database showed that the two clones matched many EST sequences in common, suggesting that P767P and P777P may represent the same gene. A DNA consensus sequence derived from a DNA sequence alignment of P767P, P777P and multiple EST clones is provided in SEQ ID NO: 587. The amino acid sequences encoded by three putative ORFs located within SEQ ID NO: 587 are provided in SEQ ID NO: 588-590.

The clone of SEQ ID NO: 342 (referred to as P789P) was found to show homology to a previously identified gene. The full length cDNA sequence for P789P and the corresponding amino acid sequence are provided in SEQ ID NO: 878 and 879, respectively.

Comparison of the sequence of SEQ ID NO: 355 (referred to as P780P) with sequences in the Geneseq DNA database, revealed that P780P shares 100% sequence identity with GeneSEQ accessions Z29049 and Z29050 (SEQ ID NO: 944 and 945, respectively). Z29049 and Z29050 differ from each other in that Z29049 contains a 714 bp deletion relative to Z29050. Thus, Z29049 and Z29050 appear to represent 2 alternative splice forms of the gene P780P. Z29049 encodes a protein that is 379 amino acids in length (GeneSeq Accession number Y15155; SEQ ID NO: 946). Z29050 encodes a protein that is 617 amino acids in length (GeneSeq Accession number Y15156; SEQ ID NO: 947). These two proteins are identical with the exception that Y15155 contains a 238 amino acid deletion relative to Y15156. This amino acid deletion corresponds to the 714 bp deletion in Z29049.

### EXAMPLE 6

### PEPTIDE PRIMING OF MICE AND PROPAGATION OF CTL LINES

6.1. This Example illustrates the preparation of a cytotoxic T lymphocyte (CTL) cell line specific for cells expressing the P502S gene.

Mice expressing the transgene for human HLA A2Kb (provided by Dr L. Sherman, The Scripps Research Institute, La Jolla, CA) were immunized with P2S#12 peptide (VLGWVAEL; SEQ ID NO: 306), which is derived from the P502S gene (also referred to herein as J1-17, SEQ ID NO: 8), as described by Theobald et al., Proc. Nat'l Acad. Sci. USA 92:11993-11997, 1995 with the following modifications. Mice were immunized with 100µg of P2S#12 and 120µg of an I-A^{b} binding peptide derived from hepatitis B Virus protein emulsified in incomplete Freund's adjuvant. Three weeks later these mice were sacrificed and single cell suspensions prepared. Cells were then resuspended at 6 x 10⁶ cells/ml in complete media (RPMI-1640; Gibco BRL, Gaithersburg, MD) containing 10% FCS, 2mM Glutamine (Gibco BRL), sodium pyruvate (Gibco BRL), non-essential amino acids (Gibco BRL), 2 x 10⁻⁵ M 2-mercaptoethanol, 50U/ml penicillin and streptomycin, and cultured in the presence of irradiated (3000 rads) P2S#12-pulsed (5mg/ml P2S#12 and 10mg/ml β2-microglobulin) LPS blasts (A2 transgenic spleens cells cultured in the presence of 7µg/ml dextran sulfate and 25µg/ml LPS for 3 days). Six days later, cells (5 x 10⁵/ml) were restimulated with 2.5 x 10⁶/ml peptide pulsed irradiated (20,000 rads) EL4A2Kb cells (Sherman et al, Science 258:815-818, 1992) and 3 x 10⁶/ml A2 transgenic spleen feeder cells. Cells were cultured in the presence of 20U/ml IL-2. Cells continued to be restimulated on a weekly basis as described, in preparation for cloning the line.

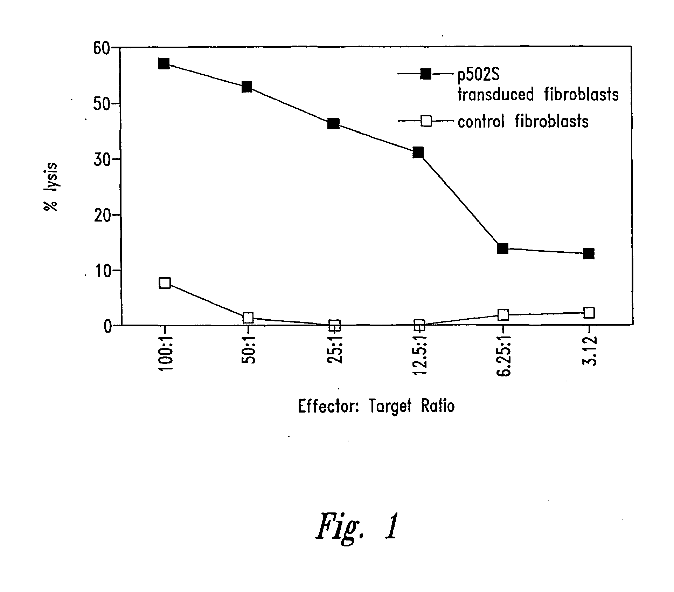

P2S#12 line was cloned by limiting dilution analysis with peptide pulsed EL4 A2Kb tumor cells (1 x 10⁴ cells/ well) as stimulators and A2 transgenic spleen cells as feeders (5 x 10⁵ cells/ well) grown in the presence of 30U/ml IL-2. On day 14, cells were restimulated as before. On day 21, clones that were growing were isolated and maintained in culture. Several of these clones demonstrated significantly higher reactivity (lysis) against human fibroblasts (HLA A2Kb expressing) transduced with P502S than against control fibroblasts. An example is presented in Figure 1.

This data indicates that P2S #12 represents a naturally processed epitope of the P502S protein that is expressed in the context of the human HLA A2Kb molecule.

6.2. This Example illustrates the preparation of murine CTL lines and CTL clones specific for cells expressing the P501S gene.

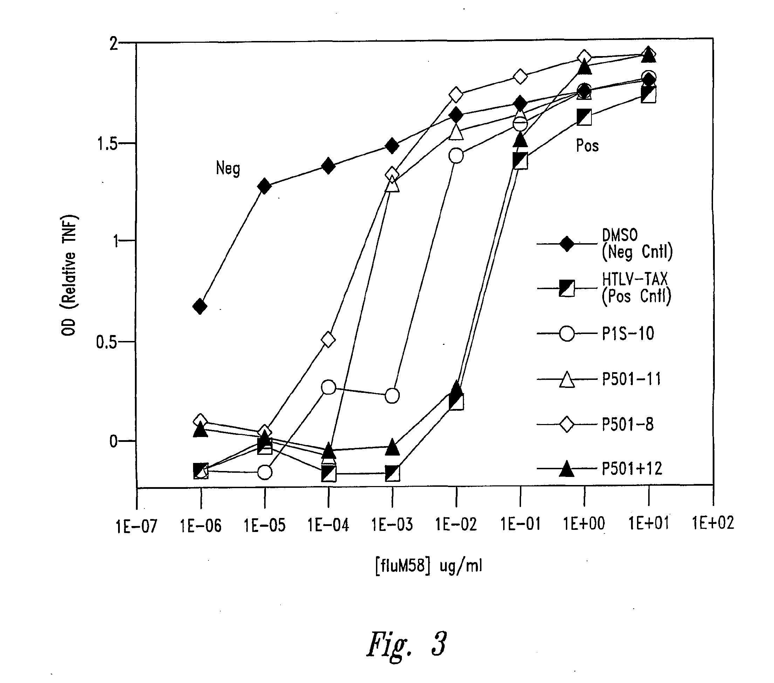

This series of experiments were performed similarly to that described above. Mice were immunized with the P1S#10 peptide (SEQ ID NO: 337), which is derived from the P501S gene (also referred to herein as L1-12, SEQ ID NO: 110). The P1S#10 peptide was derived by analysis of the predicted polypeptide sequence for P501S for potential HLA-A2 binding sequences as defined by published HLA-A2 binding motifs (Parker, KC, et al, J. Immunol., 152:163, 1994). P1S#10 peptide was synthesized as described in Example 4, and empirically tested for HLA-A2 binding using a T cell based competition assay. Predicted A2 binding peptides were tested for their ability to compete HLA-A2 specific peptide presentation to an HLA-A2 restricted CTL clone (D150M58), which is specific for the HLA-A2 binding influenza matrix peptide fluM58. D150M58 CTL secretes TNF in response to self-presentation of peptide fluM58. In the competition assay, test peptides at 100-200 µg/ml were added to cultures of D150M58 CTL in order to bind HLA-A2 on the CTL. After thirty minutes, CTL cultured with test peptides, or control peptides, were tested for their antigen dose response to the fluM58 peptide in a standard TNF bioassay. As shown in Figure 3, peptide P1S#10 competes with HLA-A2 restricted presentation of fluM58, demonstrating that peptide P1S#10 binds HLA-A2.

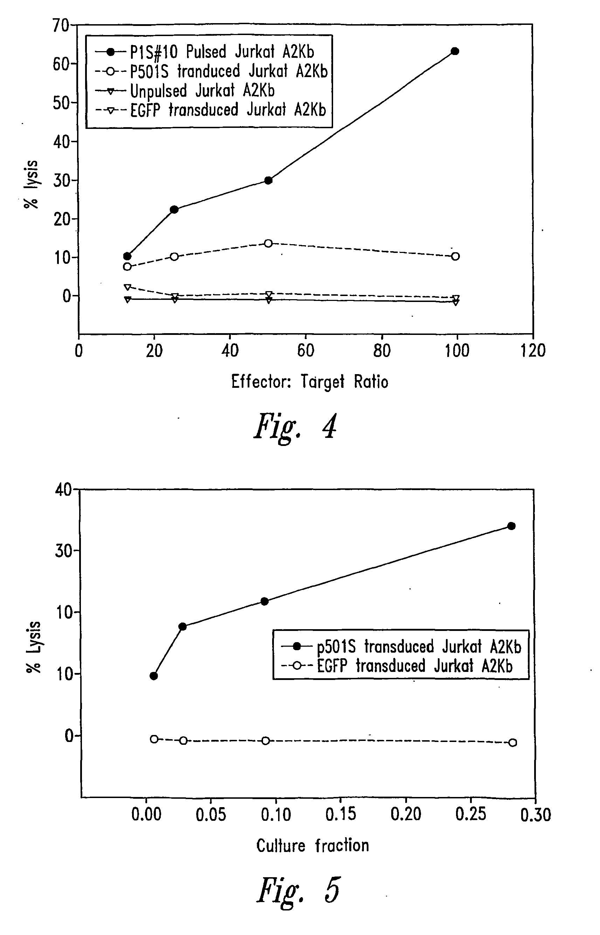

Mice expressing the transgene for human HLA A2Kb were immunized as described by Theobald et al. (Proc. Nat'l Acad. Sci. USA 92:11993-11997, 1995) with the following modifications. Mice were immunized with 62.5µg of P1S #10 and 120µg of an I-A^{b} binding peptide derived from Hepatitis B Virus protein emulsified in incomplete Freund's adjuvant. Three weeks later these mice were sacrificed and single cell suspensions prepared using a nylon mesh. Cells were then resuspended at 6 x 10⁶ cells/ml in complete media (as described above) and cultured in the presence of irradiated (3000 rads) P1 S#10-pulsed (2µg/ml P1 S#10 and 10mg/ml β2-microglobulin) LPS blasts (A2 transgenic spleens cells cultured in the presence of 7µg/ml dextran sulfate and 25µg/ml LPS for 3 days). Six days later cells (5 x 10⁵/ml) were restimulated with 2.5 x 10⁶/ml peptide-pulsed irradiated (20,000 rads) EL4A2Kb cells, as described above, and 3 x 10⁶/ml A2 transgenic spleen feeder cells. Cells were cultured in the presence of 20 U/ml IL-2. Cells were restimulated on a weekly basis in preparation for cloning. After three rounds of *in vitro* stimulations, one line was generated that recognized P1S#10-pulsed Jurkat A2Kb targets and P501 S-transduced Jurkat targets as shown in Figure 4.

A P1S#10-specific CTL line was cloned by limiting dilution analysis with peptide pulsed EL4 A2Kb tumor cells (1 x 10⁴ cells/ well) as stimulators and A2 transgenic spleen cells as feeders (5 x 10⁵ cells/ well) grown in the presence of 30U/ml IL-2. On day 14, cells were restimulated as before. On day 21, viable clones were isolated and maintained in culture. As shown in Figure 5, five of these clones demonstrated specific cytolytic reactivity against P501 S-transduced Jurkat A2Kb targets. This data indicates that P1S#10 represents a naturally processed epitope of the P501 S protein that is expressed in the context of the human HLA-A2.1 molecule.

### EXAMPLE 7

### PRIMING OF CTL IN VIVO USING NAKED DNA IMMUNIZATION WITH A PROSTATE ANTIGEN

The prostate-specific antigen L1-12, as described above, is also referred to as P501 S. HLA A2Kb Tg mice (provided by Dr L. Sherman, The Scripps Research Institute, La Jolla, CA) were immunized with 100 µg P501 S in the vector VR1012 either intramuscularly or intradermally. The mice were immunized three times, with a two week interval between immunizations. Two weeks after the last immunization, immune spleen cells were cultured with Jurkat A2Kb-P501S transduced stimulator cells. CTL lines were stimulated weekly. After two weeks of *in vitro* stimulation, CTL activity was assessed against P501 S transduced targets. Two out of 8 mice developed strong anti-P501 S CTL responses. These results demonstrate that P501 S contains at least one naturally processed HLA-A2-restricted CTL epitope.

In subsequent experiments, mice were immunized with either P501 S in the vector VR1012 as described above or with a P501 S-adenovirus construct at 1 x 10⁸ pfu/animal. Three weeks later, animals received a boost immunization, with animals that were primed with P501 S alone being boosted with either P501 S alone or P501 S-adenovirus. Spleen cells were harvested at 3 and 6 weeks post-immunization and cultured with irradiated Jurkat A2Kb cells (day 0). CTL activity was assayed on day 6, spleen cells restimulated on day 7, and a secondary CTL assay was performed on day 13. IgG1 and IgG2a responses against the N-terminal region of P501 S (amino acids 36-325 of SEQ ID NO: 113) and against a truncated form of P501S (amino acids 342-538 of SEQ ID NO: 113) were assayed using ELISA. The P501S-adenovirus construct effectively elicited both T cell and antibody responses, with P501 S alone eliciting CTL responses but not antibody responses. Immunization with P501 S alone was found to be most effective in combination with a P501 S-adenovirus boost. Immunizations with P501 S alone generated T cells that respond to peptide 370 (CLSHSVAW). In contrast, immunizations with P501S-adenovirus generated weak or no reactivity to peptide 370. These studies indicate that a combination of a DNA priming immunization followed by an adenovirus boost is a potent approach for eliciting both CTL and antibody response, and that distinct cytotoxic T cells, recognizing different epitopes, may be elicited when immunizing with P501 S or P501 S plus adenovirus versus immunizing with adenovirus alone.

### EXAMPLE 8

### ABILITY OF HUMAN T CELLS TO RECOGNIZE PROSTATE-SPECIFIC POLYPEPTIDES

This Example illustrates the ability of T cells specific for a prostate tumor polypeptide to recognize human tumor.

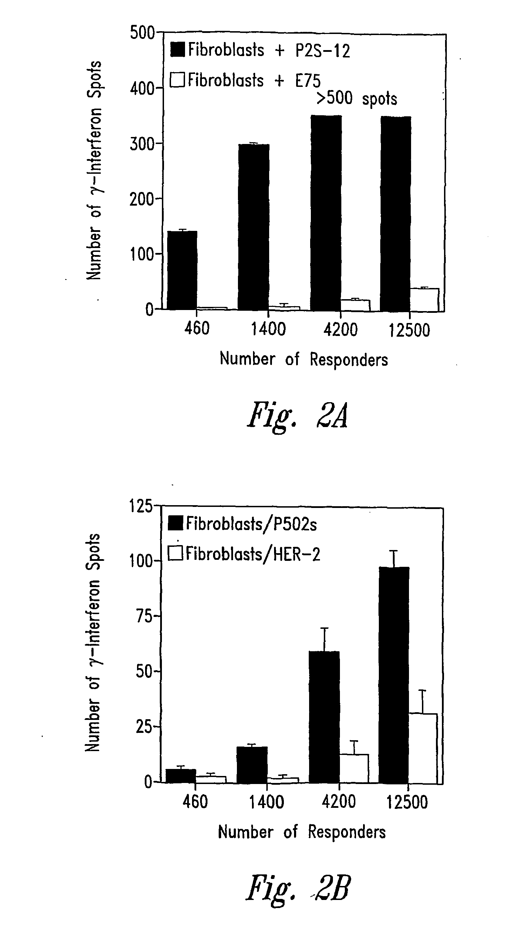

Human CD8⁺ T cells were primed *in vitro* to the P2S-12 peptide (SEQ ID NO: 306) derived from P502S (also referred to as J1-17) using dendritic cells according to the protocol of Van Tsai et al. (Critical Reviews in Immunology 18:65-75, 1998). The resulting CD8⁺ T cell microcultures were tested for their ability to recognize the P2S-12 peptide presented by autologous fibroblasts or fibroblasts which were transduced to express the P502S gene in a γ-interferon ELISPOT assay (see Lalvani et al., J. Exp. Med. 186:859-865, 1997). Briefly, titrating numbers of T cells were assayed in duplicate on 10⁴ fibroblasts in the presence of 3 µg/ml human β₂-microglobulin and 1 µg/ml P2S-12 peptide or control E75 peptide. In addition, T cells were simultaneously assayed on autologous fibroblasts transduced with the P502S gene or as a control, fibroblasts transduced with HER-2/*neu*. Prior to the assay, the fibroblasts were treated with 10 ng/ml γ-interferon for 48 hours to upregulate class I MHC expression. One of the microcultures (#5) demonstrated strong recognition of both peptide pulsed fibroblasts as well as transduced fibroblasts in a γ-interferon ELISPOT assay. Figure 2A demonstrates that there was a strong increase in the number of γ-interferon spots with increasing numbers of T cells on fibroblasts pulsed with the P2S-12 peptide (solid bars) but not with the control E75 peptide (open bars). This shows the ability of these T cells to specifically recognize the P2S-12 peptide. As shown in Figure 2B, this microculture also demonstrated an increase in the number of γ-interferon spots with increasing numbers of T cells on fibroblasts transduced to express the P502S gene but not the HER-2/neu gene. These results provide additional confirmatory evidence that the P2S-12 peptide is a naturally processed epitope of the P502S protein. Furthermore, this also demonstrates that there exists in the human T cell repertoire, high affinity T cells which are capable of recognizing this epitope. These T cells should also be capable of recognizing human tumors which express the P502S gene.

### EXAMPLE 9

### ELICITATION OF PROSTATE ANTIGEN-SPECIFIC CTL RESPONSES IN HUMAN BLOOD AND IDENTIFICATION OF CTL EPITOPES

This Example illustrates the ability of the prostate-specific antigen P501 S to elicit a cytotoxic T lymphocyte (CTL) response in blood of normal humans and defines important epitopes responsible for T-cell recognition. Such information further confirms the immunogenicity of P501S and validates its use in any of a number of illustrative immunodiagnostic and immunotherapeutic embodiments described herein.

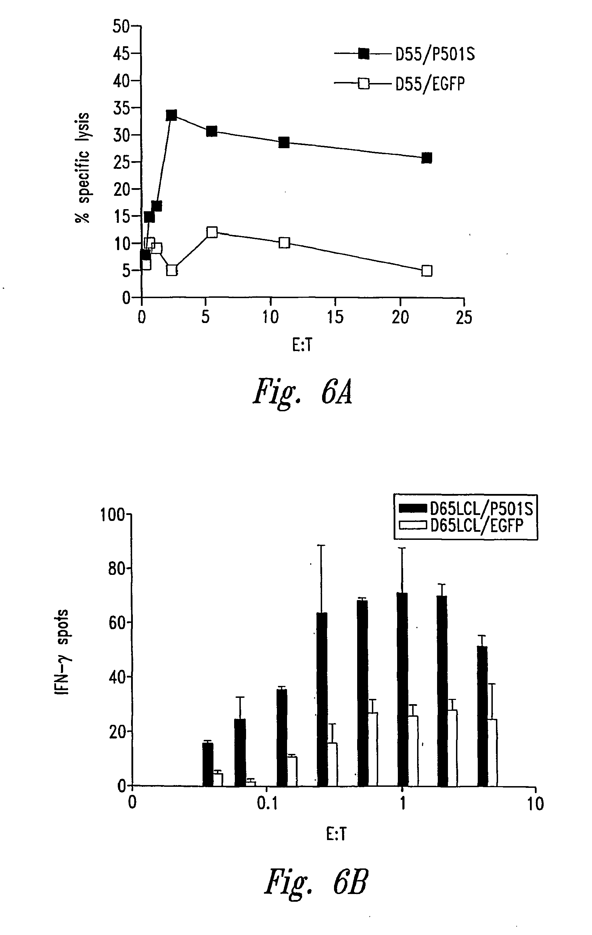

Autologous dendritic cells (DC) were differentiated from monocyte cultures derived from PBMC of normal donors by growth for five days in RPMI medium containing 10% human serum, 50 ng/ml GMCSF and 30 ng/ml IL-4. Following culture, DC were infected overnight with recombinant P501 S-expressing vaccinia virus at an M.O.I. of 5 and matured for 8 hours by the addition of 2 micrograms/ml CD40 ligand. Virus was inactivated by UV irradiation, CD8⁺ cells were isolated by positive selection using magnetic beads, and priming cultures were initiated in 24-well plates. Following five stimulation cycles using autologous fibroblasts retrovirally transduced to express P501 S and CD80, CD8+ lines were identified that specifically produced interferon-gamma when stimulated with autologous P501 S-transduced fibroblasts. The P501 S-specific activity of cell line 3A-1 could be maintained following additional stimulation cycles on autologous B-LCL transduced with P501S. Line 3A-1 was shown to specifically recognize autologous B-LCL transduced to express P501 S, but not EGFP-transduced autologous B-LCL, as measured by cytotoxicity assays (⁵¹Cr release) and interferon-gamma production (Interferon-gamma Elispot; see above and Lalvani et al., J. Exp. Med. 186:859-865, 1997). The results of these assays are presented in Figures 6A and 6B.

The ability of the prostate antigen P501 S to elicit an immune response in human males was examined as follows.

Dendritic Cells (DC) were isolated by Percol gradient followed by differential adherence, and cultured for 5 days in the presence of RPMI medium containing 1% human serum, 50 ng/ml GM-CSF and 30 ng/ml IL-4. Following culture, DC were infected for 24 hours with P501S-expressing Adenovirus at an MOI of 10 and matured for an additional 24 hours by the addition of 2 ug/ml CD40 ligand. CD8 cells were enriched from PBMC of a normal male donor by the subtraction of CD4+, CD14+ and CD16+ populations from PBMC with magnetic beads. Priming cultures containing 10,000 P501 S-expressing DC and 100,000 CD8+ T cells per well were set up in 96-well V-bottom plates in RPMI containing 10% human serum, 5 ng/ml IL-12 and 10 ng/ml IL-6. Cultures were stimulated every 7 days using autologous fibroblasts retrovirally transduced to express P501S and CD80, and were treated with IFN-gamma for 48-72 hours to upregulate MHC Class I expression. Ten u/ml IL-2 was added at the time of stimulation and on days 2 and 5 following stimulation. Following 4 stimulation cycles, multiple P501 S-specific CD8+ T cell lines were identified that produced IFN-gamma in response to IFN-gamma treated P501 S/CD80-expressing autologous fibroblasts, but not in response to IFN-gamma treated P703P/CD80-expressing autologous fibroblasts in a γ-IFN Elispot assay. The three most promising lines, referred to as 1 H1, 2H2 and 6F1, were cloned in 96-well plates with 0.5 cell/well or 2 cells/well in the presence of 75,000 PBMC/well, 10,000 B-LCL/well, 30ng/ml OKT3 and 50 u/ml IL-2 to isolate P501 S-specific clones.

To identify the class I restriction element for the P501 S-specific clones, HLA blocking and mismatch analyses were performed as follows. In γ-IFN Elispot assays, the specific response of clones to P501 S transduced autologous fibroblasts was blocked by pre-incubation with 25 ug/ml W6/32 (pan-Class I blocking antibody) and for 6F1 with BB7.2 (HLA-A2 blocking antibody), and for 1H1 and 2H2 with B1.23.2 (HLA-B/C blocking antibody). The results demonstrated that the P501S specific response of the clones is restricted to an HLA-A2 and/or HLA-B or-C allele. For the HLA mismatch analysis, autologous fibroblasts (D310, HLA-A2,A3,B44,B51,Cw5,Cw14) and heterologous fibroblasts (D326, HLA-A1, A2,B8,B51,Cw1,Cw7) that share the HLA-A-2 and HLAB51 allele were transduced with P501 S and used to stimulate the clones. In γ-IFN Elispot assays, 1H1 and 2H2 only recognized the autologous fibroblasts, suggesting (together with the antibody blocking assays above) that the relevant allele was B44, Cw5, or Cw14, and 6F1 recognized both D310 and D326, suggesting the response was restricted to HLA-A0201.

To confirm and extend the above restriction data, each of the alleles from D310 was cloned into the pBIB expression vector and cotransfected into Cos-7 cells together with full-length P501 S. 1H1 and 2H2 clones specifically recognized Cos-7 cells cotransfected with P501S and the Cw0501 alelle, while 6F1 clones recognized Cos-7 cells cotransfected with P501S and the HLA-A0201 allele. These results demonstrate that the above alleles are responsible for presenting P501 S epitopes to the above CTL.

To identify a sub-fragment of P501S that contained the epitope recognized by the CTL, 3' truncations of P501 S were cotransfected with the above alleles into Cos-7 cells. A 6F1 clone was found to recognize an epitope derived from the initial 354 nucleotides of the P501S open reading frame (ORF), a 2H2 clone (referred to as 2H2-1A12) recognized an epitope derived from the sequence of P501 S found between amino acids 471-553 of P501 S (SEQ ID NO: 113), and a 1H1 clone (referred to as 1 H1-1A6) recognized an epitope derived from the sequence of P501S found between amino acids 278-353 of P501 S (SEQ ID NO: 113).

To identify minimal epitopes recognized by these CTL, 15 or 19-mer peptides overlapping by 11 or 15 amino acids, respectively, were synthesized, pulsed onto autologous B-LCL and used as antigen presenting cells for the CTL in γ-IFN ELISPOT assays. For 1H1-1A6, a pool of four 15-21 mers that spanned amino acids 246-325 of SEQ ID NO: 113 was specifically recognized by the CTL. In the pool breakdown, peptide 291 (amino acids 291-310; SEQ ID NO: 983, cDNA sequence provided in SEQ ID NO: 972) was specifically recognized by the 1H1-1A6 CTL. Ten-mer peptides from amino acids 291-310 of P501S were then synthesized and tested as above. Peptides 291, 292, and 293 (SEQ ID NO: 984, 982 and 980, respectively; cDNA sequences provided in SEQ ID NO: 975, 976 and 973, respectively) were all specifically recognized by the CTL. These results demonstrate that each of the three 10-mer peptides contain the minimal epitope recognized by the CTL. The 8-mer peptide common to the three 10-mers with the sequence TDFVGEGL (SEQ ID NO: 981) was then synthesized and tested. In a peptide titration assay, the CD8+ T cells specific for this epitope recognized APC pulsed with 1-10µg/ml. The 8-mer was also recognized efficiently by the CTL, demonstrating that a minimal naturally processed peptide epitope recognized by the CTL spans SEQ ID NO: 981. Further analysis revealed that the minimal epitope for the clone 1H1-1A6 was YTDFVGEGL (amino acids 292-300 of P501), the amino acid and DNA sequences for which are disclosed in SEQ ID NOs:1021 and 1022, respectively. Peptide titration assays revealed that CD8+ T cells specific for this epitope recognized APC pulsed with 0.0016µg/ml of the 9-mer. Therefore a minimal CD8+ T cell epitope specific for P501 is YTDFVGEGL (SEQ ID NO:1022).

For 2H2-1A12, a pool of twenty 15-mers that spanned amino acids 462-553 was pulsed onto autologous B-LCL and tested as above. Peptides 462 and 465 (SEQ ID NO: 979 and 977, respectively; cDNA sequences provided in SEQ ID NO: 971 and 969, respectively) were both specifically recognized by the CTL. These results demonstrate that each of the two 15-mer peptides contain the minimal epitope recognized by the CTL. 10-mer peptides overlapping by a single amino acid were then synthesized from the sequence in common to the two above peptides and tested as above. Primarily peptide 464 (amino acids 464-474; SEQ ID NO: 978; cDNA sequence provided in SEQ ID NO: 970) and also peptides 463 (amino acids 463-472) and 465 (amino acids 465-475) were specifically recognized by the CTL. These results indicate that the sequence of SEQ ID NO: 978 (amino acids 464-474 of P501S) contains the minimal epitope recognized by 2H2-1A12 CTL. Further analysis revealed that the minimal epitope for the clone 2H2-1A12 was ACDVSVRVV (amino acids 465-473 of P501 S) the amino acid and DNA sequences of which are disclosed in SEQ ID NOs:1012 and 1013, respectively. The recognition of peptides 463 and 465 either reflects contamination of these peptides with peptide 464, or additional secondary and inefficient recognition of these peptides by the CTL.

This example thus confirms that P501 S is capable of eliciting a human CTL response and identifies multiple CTL epitopes responsible for T-cell recognition of this prostate-specific antigen. Such information further validates P501 S as an important target for use in immunodiagnostic and immunotherapeutic applications.

### EXAMPLE 10

### IDENTIFICATION OF A NATURALLY PROCESSED CTL EPITOPE CONTAINED WITHIN THE PROSTATE-SPECIFIC ANTIGEN P703P

The 9-mer peptide p5 (SEQ ID NO: 338) was derived from the P703P antigen (also referred to as P20). The p5 peptide is immunogenic in human HLA-A2 donors and is a naturally processed epitope. Antigen specific human CD8+ T cells can be primed following repeated *in vitro* stimulations with monocytes pulsed with p5 peptide. These CTL specifically recognize p5-pulsed and P703P-transduced target cells in both ELISPOT (as described above) and chromium release assays. Additionally, immunization of HLA-A2Kb transgenic mice with p5 leads to the generation of CTL lines which recognize a variety of HLA-A2Kb or HLA-A2 transduced target cells expressing P703P.

Initial studies demonstrating that p5 is a naturally processed epitope were done using HLA-A2Kb transgenic mice. HLA-A2Kb transgenic mice were immunized subcutaneously in the footpad with 100 µg of p5 peptide together with 140 µg of hepatitis B virus core peptide (a Th peptide) in Freund's incomplete adjuvant. Three weeks post immunization, spleen cells from immunized mice were stimulated *in vitro* with peptide-pulsed LPS blasts. CTL activity was assessed by chromium release assay five days after primary in *vitro* stimulation. Retrovirally transduced cells expressing the control antigen P703P and HLA-A2Kb were used as targets. CTL lines that specifically recognized both p5-pulsed targets as well as P703P-expressing targets were identified.

Human *in vitro* priming experiments demonstrated that the p5 peptide is immunogenic in humans. Dendritic cells (DC) were differentiated from monocyte cultures derived from PBMC of normal human donors by culturing for five days in RPMI medium containing 10% human serum, 50 ng/ml human GM-CSF and 30 ng/ml human IL-4. Following culture, the DC were pulsed with 1 ug/ml p5 peptide and cultured with CD8+ T cell enriched PBMC. CTL lines were restimulated on a weekly basis with p5-pulsed monocytes. Five to six weeks after initiation of the CTL cultures, CTL recognition of p5-pulsed target cells was demonstrated. CTL were additionally shown to recognize human cells transduced to express P703P, demonstrating that p5 is a naturally processed epitope.

Studies identifying a further peptide epitope (referred to as peptide 4) derived from the prostate tumor-specific antigen P703P that is capable of being recognized by CD4 T cells on the surface of cells in the context of HLA class II molecules were carried out as follows. The amino acid sequence for peptide 4 is provided in SEQ ID NO: 781, with the corresponding cDNA sequence being provided in SEQ ID NO: 782.

Twenty 15-mer peptides overlapping by 10 amino acids and derived from the carboxy-terminal fragment of P703P were generated using standard procedures. Dendritic cells (DC) were derived from PBMC of a normal female donor using GM-CSF and IL-4 by standard protocols. CD4 T cells were generated from the same donor as the DC using MACS beads and negative selection. DC were pulsed overnight with pools of the 15-mer peptides, with each peptide at a final concentration of 0.25 microgram/ml. Pulsed DC were washed and plated at 1 x 10⁴ cells/well of 96-well V-bottom plates and purified CD4 T cells were added at 1 x 10⁵/well. Cultures were supplemented with 60 ng/ml IL-6 and 10 ng/ml IL-12 and incubated at 37°C. Cultures were restimulated as above on a weekly basis using DC generated and pulsed as above as antigen presenting cells, supplemented with 5 ng/ml IL-7 and 10 u/ml IL-2. Following 4 *in vitro* stimulation cycles, 96 lines (each line corresponding to one well) were tested for specific proliferation and cytokine production in response to the stimulating pools with an irrelevant pool of peptides derived from mammaglobin being used as a control.

One line (referred to as 1-F9) was identified from pool #1 that demonstrated specific proliferation (measured by 3H proliferation assays) and cytokine production (measured by interferon-gamma ELISA assays) in response to pool #1 of P703P peptides. This line was further tested for specific recognition of the peptide pool, specific recognition of individual peptides in the pool, and in HLA mismatch analyses to identify the relevant restricting allele. Line 1-F9 was found to specifically proliferate and produce interferon-gamma in response to peptide pool #1, and also to peptide 4 (SEQ ID NO: 781). Peptide 4 corresponds to amino acids 126-140 of SEQ ID NO: 327. Peptide titration experiments were conducted to assess the sensitivity of line 1-F9 for the specific peptide. The line was found to specifically respond to peptide 4 at concentrations as low as 0.25 ng/ml, indicating that the T cells are very sensitive and therefore likely to have high affinity for the epitope.

To determine the HLA restriction of the P703P response, a panel of antigen presenting cells (APC) was generated that was partially matched with the donor used to generate the T cells. The APC were pulsed with the peptide and used in proliferation and cytokine assays together with line 1-F9. APC matched with the donor at HLA-DRB0701 and HLA-DQB02 alleles were able to present the peptide to the T cells, indicating that the P703P-specific response is restricted to one of these alleles.

Antibody blocking assays were utilized to determine if the restricting allele was HLA-DR0701 or HLA-DQ02. The anti-HLA-DR blocking antibody L243 or an irrelevant isotype matched IgG2a were added to T cells and APC cultures pulsed with the peptide RMPTVLQCVNVSVVS (SEQ ID NO: 781) at 250 ng/ml. Standard interferon-gamma and proliferation assays were performed. Whereas the control antibody had no effect on the ability of the T cells to recognize peptide-pulsed APC, in both assays the anti-HLA-DR antibody completely blocked the ability of the T cells to specifically recognize peptide-pulsed APC.

To determine if the peptide epitope RMPTVLQCVNVSVVS (SEQ ID NO: 781) was naturally processed, the ability of line 1-F9 to recognize APC pulsed with recombinant P703P protein was examined. For these experiments a number of recombinant P703P sources were utilized; *E. coli*-derived P703P, Pichia-derived P703P and baculovirus-derived P703P. Irrelevant protein controls used were *E. coli*-derived L3E a lung-specific antigen) and baculovirus-derived mammaglobin. In interferon-gamma ELISA assays, line 1-F9 was able to efficiently recognize both *E*. *coli* forms of P703P as well as Pichia-derived recombinant P703P, while baculovirus-derived P703P was recognized less efficiently. Subsequent Western blot analysis revealed that the *E coli* and Pichia P703P protein preparations were intact while the baculovirus P703P preparation was approximately 75% degraded. Thus, peptide RMPTVLQCVNVSVVS (SEQ ID NO: 781) from P703P is a naturally processed peptide epitope derived from P703P and presented to T cells in the context of HLA-DRB-0701

In further studies, twenty-four 15-mer peptides overlapping by 10 amino acids and derived from the N-terminal fragment of P703P (corresponding to amino acids 27-154 of SEQ ID NO: 525) were generated by standard procedures and their ability to be recognized by CD4 cells was determined essentially as described above. DC were pulsed overnight with pools of the peptides with each peptide at a final concentration of 10 microgram/ml. A large number of individual CD4 T cell lines (65/480) demonstrated significant proliferation and cytokine release (IFN-gamma) in response to the P703P peptide pools but not to a control peptide pool. The CD4 T cell lines which demonstrated specific activity were restimulated on the appropriate pool of P703P peptides and reassayed on the individual peptides of each pool as well as a peptide dose titration of the pool of peptides in a IFN-gamma release assay and in a proliferation assay.

Sixteen immunogenic peptides were recognized by the T cells from the entire set of peptide antigens tested. The amino acid sequences of these peptides are provided in SEQ ID NO: 799-814, with the corresponding cDNA sequences being provided in SEQ ID NO: 783-798, respectively. In some cases the peptide reactivity of the T cell line could be mapped to a single peptide, however some could be mapped to more than one peptide in each pool. Those CD4 T cell lines that displayed a representative pattern of recognition from each peptide pool with a reasonable affinity for peptide were chosen for further analysis (I-1A, -6A; II-4C, -5E; III-6E, IV-4B, -3F, -9B, -10F, V-5B, -4D, and -10F). These CD4 T cells lines were restimulated on the appropriate individual peptide and reassayed on autologous DC pulsed with a truncated form of recombinant P703P protein made in *E. coli* (a.a. 96 - 254 of SEQ ID NO: 525), full-length P703P made in the baculovirus expression system, and a fusion between influenza virus NS1 and P703P made in *E*. *coli.* Of the T cell lines tested, line I-1A recognized specifically the truncated form of P703P (*E*. *coli*) but no other recombinant form of P703P. This line also recognized the peptide used to elicit the T cells. Line 2-4C recognized the truncated form of P703P (*E. coli*) and the full length form of P703P made in baculovirus, as well as peptide. The remaining T cell lines tested were either peptide-specific only (II-5E, II-6F, IV-4B, IV-3F, IV-9B, IV-10F, V-5B and V-4D) or were non-responsive to any antigen tested (V-10F). These results demonstrate that the peptide sequence RPLLANDLMLIKLDE (SEQ ID NO: 814; corresponding to a.a. 110-124 of SEQ ID NO: 525) recognized by the T cell line I-1A, and the peptide sequences SVSESDTIRSISIAS (SEQ ID NO: 811; corresponding to a.a. 125-139 of SEQ ID NO: 525) and ISIASQCPTAGNSCL (SEQ ID NO: 810; corresponding to a.a. 135-149 of SEQ ID NO: 525) recognized by the T cell line II-4C may be naturally processed epitopes of the P703P protein.

In further studies, forty 15-mer peptides overlapping by 10 amino acids and derived spanning amino acids 47 to 254 of P703P (SEQ ID NO: 525) were generated by standard procedures and their ability to be recognized by CD4 cells was determined essentially as described above. DC were prepared from PBMC of a donor having distinct HLA DR and DQ alleles from that used in previous experiments. DC were pulsed overnight with pools of the peptides with each peptide at a final concentration of 0.25 microgram/ml, and each pool containing 10 peptides. Twelve lines were identified that demonstrated specific proliferation and cytokine production (measured in gamma-interferon ELISA assays) in response to the stimulating peptide pool. These lines were further tested for specific recognition of the peptide pool, specific recognition of individual peptides in the pool, and specific recognition of recombinant P703P protein. Lines 3A5H and 3A9H specifically proliferated and produced gamma-interferon in response to recombinant protein and one individual peptide as well as the peptide pool. Following re-stimulation on targets loaded with the specific peptide, only 3A9H responded specifically to targets exposed to lysates of fibroblasts infected adenovirus expressing full-length P703P. These results indicates that the line 3A9H can respond to antigenic peptide derived from protein synthesized in mammalian cells. The peptide to which the specific CD4 line responded correspond to amino acids 155-170 of P703P (SEQ ID NO: 943). The DNA sequence for this peptide is provided in SEQ ID NO: 942.

### EXAMPLE 11

### EXPRESSION OF A BREAST TUMOR-DERIVED ANTIGEN IN PROSTATE

Isolation of the antigen B305D from breast tumor by differential display is described in US Patent Application No. 08/700,014, filed August 20, 1996. Several different splice forms of this antigen were isolated. The determined cDNA sequences for these splice forms are provided in SEQ ID NO: 366-375, with the amino acid sequences corresponding to the sequences of SEQ ID NO: 292, 298 and 301-303 being provided in SEQ ID NO: 299-306, respectively. In further studies, a splice variant of the cDNA sequence of SEQ ID NO: 366 was isolated which was found to contain an additional guanine residue at position 884 (SEQ ID NO: 530), leading to a frameshift in the open reading frame. The determined DNA sequence of this ORF is provided in SEQ ID NO: 531. This frameshift generates a protein sequence (provided in SEQ ID NO: 532) of 293 amino acids that contains the C-terminal domain common to the other isoforms of B305D but that differs in the N-terminal region.

The expression levels of B305D in a variety of tumor and normal tissues were examined by real time PCR and by Northern analysis. The results indicated that B305D is highly expressed in breast tumor, prostate tumor, normal prostate and normal testes, with expression being low or undetectable in all other tissues examined (colon tumor, lung tumor, ovary tumor, and normal bone marrow, colon, kidney, liver, lung, ovary, skin, small intestine, stomach). Using real-time PCR on a panel of prostate tumors, expression of B305D in prostate tumors was shown to increase with increasing Gleason grade, demonstrating that expression of B305D increases as prostate cancer progresses.

### EXAMPLE 12

### GENERATION OF HUMAN CTL IN VITRO USING WHOLE GENE PRIMING AND STIMULATION TECHNIQUES WITH THE PROSTATE-SPECIFIC ANTIGEN P501 S

Using *in vitro* whole-gene priming with P501 S-vaccinia infected DC (see, for example, Yee et al, The Journal of Immunology, 157(9):4079-86, 1996), human CTL lines were derived that specifically recognize autologous fibroblasts transduced with P501S (also known as L1-12), as determined by interferon-γ ELISPOT analysis as described above. Using a panel of HLA-mismatched B-LCL lines transduced with P501 S, these CTL lines were shown to be likely restricted to HLAB class I allele. Specifically, dendritic cells (DC) were differentiated from monocyte cultures derived from PBMC of normal human donors by growing for five days in RPMI medium containing 10% human serum, 50 ng/ml human GM-CSF and 30 ng/ml human IL-4. Following culture, DC were infected overnight with recombinant P501 S vaccinia virus at a multiplicity of infection (M.O.I) of five, and matured overnight by the addition of 3 µg/ml CD40 ligand. Virus was inactivated by UV irradiation. CD8+ T cells were isolated using a magnetic bead system, and priming cultures were initiated using standard culture techniques. Cultures were restimulated every 7-10 days using autologous primary fibroblasts retrovirally transduced with P501 S and CD80. Following four stimulation cycles, CD8+ T cell lines were identified that specifically produced interferon-γ when stimulated with P501S and CD80-transduced autologous fibroblasts. A panel of HLA-mismatched B-LCL lines transduced with P501S were generated to define the restriction allele of the response. By measuring interferon-γ in an ELISPOT assay, the P501S specific response was shown to be likely restricted by HLA B alleles. These results demonstrate that a CD8+ CTL response to P501 S can be elicited.

To identify the epitope(s) recognized, cDNA encoding P501 S was fragmented by various restriction digests, and sub-cloned into the retroviral expression vector pBIB-KS. Retroviral supernatants were generated by transfection of the helper packaging line Phoenix-Ampho. Supernatants were then used to transduce Jurkat/A2Kb cells for CTL screening. CTL were screened in IFN-gamma ELISPOT assays against these A2Kb targets transduced with the "library" of P501S fragments. Initial positive fragments P501 S/H3 and P501 S/F2 were sequenced and found to encode amino acids 106-553 and amino acids 136-547, respectively, of SEQ ID NO: 113. A truncation of H3 was made to encode amino acid residues 106-351 of SEQ ID NO: 113, which was unable to stimulate the CTL, thus localizing the epitope to amino acid residues 351-547. Additional fragments encoding amino acids 1-472 (Fragment A) and amino acids 1-351 (Fragment B) were also constructed. Fragment A but not Fragment B stimulated the CTL thus localizing the epitope to amino acid residues 351-472. Overlapping 20-mer and 18-mer peptides representing this region were tested by pulsing Jurkat/A2Kb cells versus CTL in an IFN-gamma assay. Only peptides P501S-369(20) and P501S-369(18) stimulated the CTL. Nine-mer and 10-mer peptides representing this region were synthesized and similarly tested. Peptide P501 S-370 (SEQ ID NO: 539) was the minimal 9-mer giving a strong response. Peptide P501 S-376 (SEQ ID NO: 540) also gave a weak response, suggesting that it might represent a cross-reactive epitope.

In subsequent studies, the ability of primary human B cells transduced with P501S to prime MHC class I-restricted, P501S-specific, autologous CD8 T cells was examined. Primary B cells were derived from PBMC of a homozygous HLA-A2 donor by culture in CD40 ligand and IL-4, transduced at high frequency with recombinant P501 S in the vector pBIB, and selected with blastocidin-S. For *in vitro* priming, purified CD8+ T cells were cultured with autologous CD40 ligand + IL-4 derived, P501 S-transduced B cells in a 96-well microculture format. These CTL microcultures were re-stimulated with P501 S-transduced B cells and then assayed for specificity. Following this initial screen, microcultures with significant signal above background were cloned on autologous EBV-transformed B cells (BLCL), also transduced with P501 S. Using IFN-gamma ELISPOT for detection, several of these CD8 T cell clones were found to be specific for P501 S, as demonstrated by reactivity to BLCL/P501S but not BLCL transduced with control antigen. It was further demonstrated that the anti-P501 S CD8 T cell specificity is HLA-A2-restricted. First, antibody blocking experiments with anti-HLA-A,B,C monoclonal antibody (W6.32), anti-HLA-B,C monoclonal antibody (B1.23.2) and a control monoclonal antibody showed that only the anti-HLA-A,B,C antibody blocked recognition of P501 S-expressing autologous BLCL. Secondly, the anti-P501 S CTL also recognized an HLA-A2 matched, heterologous BLCL transduced with P501 S, but not the corresponding EGFP transduced control BLCL.

A naturally processed, CD8, class I-restricted peptide epitope of P501 S was identified as follows. Dendritic cells (DC) were isolated by Percol gradient followed by differential adherence, and cultured for 5 days in the presence of RPMI medium containing 1% human serum, 50ng/ml GM-CSF and 30ng/ml IL-4. Following culture, DC were infected for 24 hours with P501 S-expressing adenovirus at an MOl of 10 and matured for an additional 24 hours by the addition of 2ug/ml CD40 ligand. CD8 cells were enriched for by the subtraction of CD4+, CD14+ and CD16+ populations from PBMC with magnetic beads. Priming cultures containing 10,000 P501 S-expressing DC and 100,000 CD8+ T cells per well were set up in 96-well V-bottom plates with RPMI containing 10% human serum, 5ng/ml IL-12 and 10ng/ml IL-6. Cultures were stimulated every 7 days using autologous fibroblasts retrovirally transduced to express P501 S and CD80, and were treated with IFN-gamma for 48-72 hours to upregulate MHC Class I expression. 10u/ml IL-2 was added at the time of stimulation and on days 2 and 5 following stimulation. Following 4 stimulation cycles, one P501S-specific CD8+ T cell line (referred to as 2A2) was identified that produced IFN-gamma in response to IFN-gamma-treated P501S/CD80 expressing autologous fibroblasts, but not in response to IFN-gamma-treated P703P/CD80 expressing autologous fibroblasts in a γ-IFN Elispot assay. Line 2A2 was cloned in 96-well plates with 0.5 cell/well or 2 cells/well in the presence of 75,000 PBMC/well, 10,000 B-LCL/well, 30ng/ml OKT3 and 50u/ml IL-2. Twelve clones were isolated that showed strong P501S specificity in response to transduced fibroblasts.

Fluorescence activated cell sorting (FACS) analysis was performed on P501 S-specific clones using CD3-, CD4- and CD8-specific antibodies conjugated to PercP, FITC and PE respectively. Consistent with the use of CD8 enriched T cells in the priming cultures, P5401 S-specific clones were determined to be CD3+, CD8+ and CD4-.

To identify the relevant P501 S epitope recognized by P501 S specific CTL, pools of 18-20 mer or 30-mer peptides that spanned the majority of the amino acid sequence of P501 S were loaded onto autologous B-LCL and tested in γ-IFN Elispot assays for the ability to stimulate two P501S-specific CTL clones, referred to as 4E5 and 4E7. One pool, composed of five 18-20 mer peptides that spanned amino acids 411-486 of P501 S (SEQ ID NO: 113), was found to be recognized by both P501S-specific clones. To identify the specific 18-20 mer peptide recognized by the clones, each of the 18-20 mer peptides that comprised the positive pool were tested individually in γ-IFN Elispot assays for the ability to stimulate the two P501 S-specific CTL clones, 4E5 and 4E7. Both 4E5 and 4E7 specifically recognized one 20-mer peptide (SEQ ID NO: 853; cDNA sequence provided in SEQ ID NO: 854) that spanned amino acids 453-472 of P501 S. Since the minimal epitope recognized by CD8+ T cells is almost always either a 9 or 10-mer peptide sequence, 10-mer peptides that spanned the entire sequence of SEQ ID NO: 853 were synthesized that differed by 1 amino acid. Each of these 10-mer peptides was tested for the ability to stimulate two P501S-specific clones, (referred to as 1D5 and 1E12). One 10-mer peptide (SEQ ID NO: 855; cDNA sequence provided in SEQ ID NO: 856) was identified that specifically stimulated the P501 S-specific clones. This epitope spans amino acids 463-472 of P501 S. This sequence defines a minimal 10-mer epitope from P501 S that can be naturally processed and to which CTL responses can be identified in normal PBMC. Thus, this epitope is a candidate for use as a vaccine moiety, and as a therapeutic and/or diagnostic reagent for prostate cancer.

To identify the class I restriction element for the P501 S-derived sequence of SEQ ID NO: 855, HLA blocking and mismatch analyses were performed. In γ-IFN Elispot assays, the specific response of clones 4A7 and 4E5 to P501S-transduced autologous fibroblasts was blocked by pre-incubation with 25ug/ml W6/32 (pan-Class I blocking antibody) and B1.23.2 (HLA-B/C blocking antibody). These results demonstrate that the SEQ ID NO: 855-specific response is restricted to an HLA-B or HLA-C allele.

For the HLA mismatch analysis, autologous B-LCL (HLA-A1,A2,B8,B51, Cw1, Cw7) and heterologous B-LCL (HLA-A2,A3,B18,B51,Cw5,Cw14) that share the HLAB51 allele were pulsed for one hour with 20ug/ml of peptide of SEQ ID NO: 855, washed, and tested in γ-IFN Elispot assays for the ability to stimulate clones 4A7 and 4E5. Antibody blocking assays with the B1.23.2 (HLA-B/C blocking antibody) were also performed. SEQ ID NO: 855-specific response was detected using both the autologous (D326) and heterologous (D107) B-LCL, and furthermore the responses were blocked by pre-incubation with 25ug/ml of B1.23.2 HLA-B/C blocking antibody. Together these results demonstrate that the P501 S-specific response to the peptide of SEQ ID NO: 855 is restricted to the HLA-B51 class I allele. Molecular cloning and sequence analysis of the HLA-B51 allele from D3326 revealed that the HLA-B51 subtype of D326 is HLA-B51011.

Based on the 10-mer P501S-derived epitope of SEQ ID NO: 855, two 9-mers with the sequences of SEQ ID NO: 857 and 858 were synthesized and tested in Elispot assays for the ability to stimulate two P501 S-specific CTL clones derived from line 2A2. The 10-mer peptide of SEQ ID NO: 855, as well as the 9-mer peptide of SEQ ID NO: 858, but not the 9-mer peptide of SEQ ID NO: 857, were capable of stimulating the P501S-specific CTL to produce IFN-gamma. These results demonstrate that the peptide of SEQ ID NO: 858 is a 9-mer P501S-derived epitope recognized by P501S-specific CTL. The DNA sequence encoding the epitope of SEQ ID NO: 858 is provided in SEQ ID NO: 859.

To identify the class I restricting allele for the P501 S-derived peptide of SEQ ID NO: 855 and 858 specific response, each of the HLA B and C alleles were cloned from the donor used in the *in vitro* priming experiment. Sequence analysis indicated that the relevant alleles were HLA-B8, HLA-B51, HLA-Cw01 and HLA-Cw07. Each of these alleles were subcloned into an expression vector and co-transfected together with the P501 S gene into VA-13 cells. Transfected VA-13 cells were then tested for the ability to specifically stimulate the P501 S-specific CTL in ELISPOT assays. VA-13 cells transfected with P501 S and HLA-B51 were capable of stimulating the P501 S-specific CTL to secrete gamma-IFN. VA-13 cells transfected with HLA-B51 alone or P501 S + the other HLA-alleles were not capable of stimulating the P501 S-specific CTL. These results demonstrate that the restricting allele for the P501 S-specific response is the HLAB51 allele. Sequence analysis revealed that the subtype of the relevant restricting allele is HLA-B51011.

To determine if the P501S-specific CTL could recognize prostate tumor cells that express P501 S, the P501 S-positive lines LnCAP and CRL2422 (both expressing "moderate" amounts of P501 S mRNA and protein), and PC-3 (expressing low amounts of P501 S mRNA and protein), plus the P501 S-negative cell line DU-145 were retrovirally transduced with the HLA-B51011 allele that was cloned from the donor used to generate the P501 S-specific CTL. HLA-B51011- or EGFP-transduced and selected tumor cells were treated with gamma-interferon and androgen (to upregulate stimulatory functions and P501 S, respectively) and used in gamma-interferon Elispot assays with the P501 S-specific CTL clones 4E5 and 4E7. Untreated cells were used as a control.

Both 4E5 and 4E7 efficiently and specifically recognized LnCAP and CRL2422 cells that were transduced with the HLA-B51011 allele, but not the same cell lines transduced with EGFP. Additionally, both CTL clones specifically recognized PC-3 cells transduced with HLA-B51011, but not the P501S-negative tumor cell line DU-145. Treatment with gamma-interferon or androgen did not enhance the ability of CTL to recognize tumor cells. These results demonstrate that P501 S-specific CTL, generated by *in vitro* whole gene priming, specifically and efficiently recognize prostate tumor cell lines that express P501 S.

A naturally processed CD4 epitope of P501 S was identified as follows.

CD4 cells specific for P501S were prepared as described above. A series of 16 overlapping peptides were synthesized that spanned approximately 50% of the amino terminal portion of the P501S gene (amino acids 1- 325 of SEQ ID NO: 113). For priming, peptides were combined into pools of 4 peptides, pulsed at 4 µg/ml onto dendritic cells (DC) for 24 hours, with TNF-alpha. DC were then washed and mixed with negatively selected CD4+ T cells in 96 well U-bottom plates. Cultures were re-stimulated weekly on fresh DC loaded with peptide pools. Following a total of 4 stimulation cycles, cells were rested for an additional week and tested for specificity to APC pulsed with peptide pools using γ-IFN ELISA and proliferation assays. For these assays, adherent monocytes loaded with either the relevant peptide pool at 4ug/ml or an irrelevant peptide at µg/ml were used as APC. T cell lines that demonstrated either specific cytokine secretion or proliferation were then tested for recognition of individual peptides that were present in the pool. T cell lines could be identified from pools A and B that recognized individual peptides from these pools.

From pool A, lines AD9 and AE10 specifically recognized peptide 1 (SEQ ID NO: 862), and line AF5 recognized peptide 39 (SEQ ID NO: 861). From pool B, line BC6 could be identified that recognized peptide 58 (SEQ ID NO: 860). Each of these lines were stimulated on the specific peptide and tested for specific recognition of the peptide in a titration assay as well as cell lysates generated by infection of HEK 293 cells with adenovirus expressing either P501 S or an irrelevant antigen. For these assays, APC-adherent monocytes were pulsed with either 10, 1, or 0.1 µg/ml individual P501S peptides, and DC were pulsed overnight with a 1:5 dilution of adenovirally infected cell lysates. Lines AD9, AE10 and AF5 retained significant recognition of the relevant P501 S-derived peptides even at 0.1 mg/ml. Furthermore, line AD9 demonstrated significant (8.1 fold stimulation index) specific activity for lysates from adenovirus-P501S infected cells. These results demonstrate that high affinity CD4 T cell lines can be generated toward P501 S-derived epitopes, and that at least a subset of these T cells specific for the P501 S derived sequence of SEQ ID NO: 862 are specific for an epitope that is naturally processed by human cells. The DNA sequences encoding the amino acid sequences of SEQ ID NO: 860-862 are provided in SEQ ID NO: 863-865, respectively.

To further characterize the P501 S-specific activity of AD9, the line was cloned using anti-CD3. Three clones, referred to as 1A1, 1A9 and 1 F5, were identified that were specific for the P501S-1 peptide (SEQ ID NO: 862). To determine the HLA restriction allele for the P501 S-specific response, each of these clones was tested in class II antibody blocking and HLA mismatch assays using proliferation and gamma-interferon assays. In antibody blocking assays and measuring gamma-interferon production using ELISA assays, the ability of all three clones to recognize peptide pulsed APC was specifically blocked by co-incubation with either a pan-class II blocking antibody or a HLA-DR blocking antibody, but not with a HLA-DQ or an irrelevant antibody. Proliferation assays performed simultaneously with the same cells confirmed these results. These data indicate that the P501 S-specific response of the clones is restricted by an HLA-DR allele. Further studies demonstrated that the restricting allele for the P501S-specific response is HLA-DRB1501.

### EXAMPLE 13

### IDENTIFICATION OF PROSTATE-SPECIFIC ANTIGENS BY MICROARRAY ANALYSIS

This Example describes the isolation of certain prostate-specific polypeptides from a prostate tumor cDNA library.

A human prostate tumor cDNA expression library as described above was screened using microarray analysis to identify clones that display at least a three fold over-expression in prostate tumor and/or normal prostate tissue, as compared to non-prostate normal tissues (not including testis). 372 clones were identified, and 319 were successfully sequenced. Table II presents a summary of these clones, which are shown in SEQ ID NO:385-400. Of these sequences SEQ ID NO:386, 389, 390 and 392 correspond to novel genes, and SEQ ID NO: 393 and 396 correspond to previously identified sequences. The others (SEQ ID NO:385, 387, 388, 391, 394, 395 and 397-400) correspond to known sequences, as shown in Table II.

**TABLE II**

| SUMMARY OF PROSTATE TUMOR ANTIGENS | | |
|---|---|---|
| Known Genes | Previously Identified Genes | Novel Genes |
| T-cell gamma chain T-cell gamma chain | P504S | 23379 (SEQ ID NO:389) |
| Kallikrein | P1000C | 23399 (SEQ ID NO:392) |
| Vector | P501S | 23320 (SEQ ID NO:386) |
| CGI-82 protein mRNA (23319; SEQ ID NO:385) | P503S | 23381 (SEQ ID NO:390) |
| PSA | P510S | |
| Ald. 6 Dehyd. | P784P | |
| L-iditol-2 dehydrogenase (23376; SEQ ID NO:388) | p502S | |
| Ets transcription factor PDEF (22672; SEQ ID NO:398) | P706P | |
| hTGR (22678; SEQ ID NO:399) | 19142.2, bangur.seq (22621; SEQ ID NO:396) | |
| KIAA0295(22685; SEQ ID NO:400) | 5566.1 Wang (23404; SEQ ID NO:393) | |
| Prostatic Acid Phosphatase(22655; SEQ ID NO:397) | P712P | |
| transglutaminase (22611; SEQ ID NO:395) | P778P | |
| HDLBP (23508; SEQ ID NO:394) | | |
| CGI-69 Protein(23367; SEQ ID NO:387) | | |
| KIAA0122(23383; SEQ ID NO:391) | | |
| TEEG | | |

Subsequent studies led to the isolation of an extended cDNA sequence for the clone of SEQ ID NO: 329 (also referred to as P554S). This extended sequence is provided in SEQ ID NO: 967, with the corresponding amino acid sequence being provided in SEQ ID NO: 968.

CGI-82 showed 4.06 fold over-expression in prostate tissues as compared to other normal tissues tested. It was over-expressed in 43% of prostate tumors, 25% normal prostate, not detected in other normal tissues tested. L-iditol-2 dehydrogenase showed 4.94 fold over-expression in prostate tissues as compared to other normal tissues tested. It was over-expressed in 90% of prostate tumors, 100% of normal prostate, and not detected in other normal tissues tested. Ets transcription factor PDEF showed 5.55 fold over-expression in prostate tissues as compared to other normal tissues tested. It was over-expressed in 47% prostate tumors, 25% normal prostate and not detected in other normal tissues tested. hTGR1 showed 9.11 fold over-expression in prostate tissues as compared to other normal tissues tested. It was over-expressed in 63% of prostate tumors and is not detected in normal tissues tested including normal prostate. KIAA0295 showed 5.59 fold over-expression in prostate tissues as compared to other normal tissues tested. It was over-expressed in 47% of prostate tumors, low to undetectable in normal tissues tested including normal prostate tissues. Prostatic acid phosphatase showed 9.14 fold over-expression in prostate tissues as compared to other normal tissues tested. It was over-expressed in 67% of prostate tumors, 50% of normal prostate, and not detected in other normal tissues tested. Transglutaminase showed 14.84 fold over-expression in prostate tissues as compared to other normal tissues tested. It was over-expressed in 30% of prostate tumors, 50% of normal prostate, and is not detected in other normal tissues tested. High density lipoprotein binding protein (HDLBP) showed 28.06 fold over-expression in prostate tissues as compared to other normal tissues tested. It was over-expressed in 97% of prostate tumors, 75% of normal prostate, and is undetectable in all other normal tissues tested. CGI-69 showed 3.56 fold over-expression in prostate tissues as compared to other normal tissues tested. It is a low abundant gene, detected in more than 90% of prostate tumors, and in 75% normal prostate tissues. The expression of this gene in normal tissues was very low. KIAA0122 showed 4.24 fold over-expression in prostate tissues as compared to other normal tissues tested. It was over-expressed in 57% of prostate tumors, it was undetectable in all normal tissues tested including normal prostate tissues. 19142.2 bangur showed 23.25 fold over-expression in prostate tissues as compared to other normal tissues tested. It was over-expressed in 97% of prostate tumors and 100% of normal prostate. It was undetectable in other normal tissues tested. 5566.1 Wang showed 3.31 fold over-expression in prostate tissues as compared to other normal tissues tested. It was over-expressed in 97% of prostate tumors, 75% normal prostate and was also over-expressed in normal bone marrow, pancreas, and activated PBMC. Novel clone 23379 (also referred to as P553S) showed 4.86 fold over-expression in prostate tissues as compared to other normal tissues tested. It was detectable in 97% of prostate tumors and 75% normal prostate and is undetectable in all other normal tissues tested. Novel clone 23399 showed 4.09 fold over-expression in prostate tissues as compared to other normal tissues tested. It was over-expressed in 27% of prostate tumors and was undetectable in all normal tissues tested including normal prostate tissues. Novel clone 23320 showed 3.15 fold over-expression in prostate tissues as compared to other normal tissues tested. It was detectable in all prostate tumors and 50% of normal prostate tissues. It was also expressed in normal colon and trachea. Other normal tissues do not express this gene at high level.

Subsequent full-length cloning studies on P553S, using standard techniques, revealed that this clone is an incomplete spliced form of P501 S. The determined cDNA sequences for four splice variants of P553S are provided in SEQ ID NO: 702-705. An amino acid sequence encoded by SEQ ID NO: 705 is provided in SEQ ID NO: 706. The cDNA sequence of SEQ ID NO: 702 was found to contain two open reading frames (ORFs). The amino acid sequences encoded by these two ORFs are provided in SEQ ID NO: 707 and 708.

### EXAMPLE 14

### IDENTIFICATION OF PROSTATE-SPECIFIC ANTIGENS BY ELECTRONIC SUBTRACTION

This Example describes the use of an electronic subtraction technique to identify prostate-specific antigens.

Potential prostate-specific genes present in the GenBank human EST database were identified by electronic subtraction (similar to that described by Vasmatizis et al., Proc. Nat'l Acad. Sci. USA 95:300-304, 1998). The sequences of EST clones (43,482) derived from various prostate libraries were obtained from the GenBank public human EST database. Each prostate EST sequence was used as a query sequence in a BLASTN (National Center for Biotechnology Information) search against the human EST database. All matches considered identical (length of matching sequence >100 base pairs, density of identical matches over this region > 70%) were grouped (aligned) together in a cluster. Clusters containing more than 200 ESTs were discarded since they probably represented repetitive elements or highly expressed genes such as those for ribosomal proteins. If two or more clusters shared common ESTs, those clusters were grouped together into a "supercluster," resulting in 4,345 prostate superclusters.

Records for the 479 human cDNA libraries represented in the GenBank release were downloaded to create a database of these cDNA library records. These 479 cDNA libraries were grouped into three groups: Plus (normal prostate and prostate tumor libraries, and breast cell line libraries, in which expression was desired), Minus (libraries from other normal adult tissues, in which expression was not desirable), and Other (libraries from fetal tissue, infant tissue, tissues found only in women, non-prostate tumors and cell lines other than prostate cell lines, in which expression was considered to be irrelevant). A summary of these library groups is presented in Table III.

**TABLE III**

| PROSTATE CDNA LIBRARIES AND ESTS | | |
|---|---|---|
| Library | # of Libraries | # of ESTs |
| Plus | 25 | 43,482 |
| Normal | 11 | 18,875 |
| Tumor | 11 | 21,769 |
| Cell lines | 3 | 2,838 |
| Minus | 166 | |
| Other | 287 | |

Each supercluster was analyzed in terms of the ESTs within the supercluster. The tissue source of each EST clone was noted and used to classify the superclusters into four groups: Type 1- EST clones found in the Plus group libraries only; no expression detected in Minus or Other group libraries; Type 2- EST clones derived from the Plus and Other group libraries only; no expression detected in the Minus group; Type 3- EST clones derived from the Plus, Minus and Other group libraries, but the number of ESTs derived from the Plus group is higher than in either the Minus or Other groups; and Type 4- EST clones derived from Plus, Minus and Other group libraries, but the number derived from the Plus group is higher than the number derived from the Minus group. This analysis identified 4,345 breast clusters (see Table IV). From these clusters, 3,172 EST clones were ordered from Research Genetics, Inc., and were received as frozen glycerol stocks in 96-well plates.

**TABLE IV**

| PROSTATE CLUSTER SUMMARY | | |
|---|---|---|
| Type | # of Superclusters | # of ESTs Ordered |
| 1 | 688 | 677 |
| 2 | 2899 | 2484 |
| 3 | 85 | 11 |
| 4 | 673 | 0 |
| Total | 4345 | 3172 |

The EST clone inserts were PCR-amplified using amino-linked PCR primers for Synteni microarray analysis. When more than one PCR product was obtained for a particular clone, that PCR product was not used for expression analysis. In total, 2,528 clones from the electronic subtraction method were analyzed by microarray analysis to identify electronic subtraction breast clones that had high levels of tumor vs. normal tissue mRNA. Such screens were performed using a Synteni (Palo Alto, CA) microarray, according to the manufacturer's instructions (and essentially as described by Schena et al., Proc. Nat'l Acad. Sci. USA 93:10614-10619, 1996 and Heller et al., Proc. Nat'l Acad. Sci. USA 94:2150-2155, 1997). Within these analyses, the clones were arrayed on the chip, which was then probed with fluorescent probes generated from normal and tumor prostate cDNA, as well as various other normal tissues. The slides were scanned and the fluorescence intensity was measured.

Clones with an expression ratio greater than 3 (*i.e.,* the level in prostate tumor and normal prostate mRNA was at least three times the level in other normal tissue mRNA) were identified as prostate tumor-specific sequences (Table V). The sequences of these clones are provided in SEQ ID NO: 401-453, with certain novel sequences shown in SEQ ID NO: 407, 413, 416-419, 422, 426, 427 and 450.

**TABLE V**

| PROSTATE-TUMOR SPECIFIC CLONES | | |
|---|---|---|
| SEQ ID NO. | Sequence Designation | Comments |
| 401 | 22545 | previously identified P1000C |
| 402 | 22547 | previously identified P704P |
| 403 | 22548 | known |
| 404 | 22550 | known |
| 405 | 22551 | PSA |
| 406 | 22552 | prostate secretory protein 94 |
| 407 | 22553 | novel |
| 408 | 22558 | previously identified P509S |
| 409 | 22562 | glandular kallikrein |
| 410 | 22565 | previously identified P1000C |
| 411 | 22567 | PAP |
| 412 | 22568 | B1006C (breast tumor antigen) |
| 413 | 22570 | novel |
| 414 | 22571 | PSA |
| 415 | 22572 | previously identified P706P |
| 416 | 22573 | novel |
| 417 | 22574 | novel |
| 418 | 22575 | novel |
| 419 | 22580 | novel |
| 420 | 22581 | PAP |
| 421 | 22582 | prostatic secretory protein 94 |
| 422 | 22583 | novel |
| 423 | 22584 | prostatic secretory protein 94 |
| 424 | 22585 | prostatic secretory protein 94 |
| 425 | 22586 | known |
| 426 | 22587 | novel |
| 427 | 22588 | novel |
| 428 | 22589 | PAP |
| 429 | 22590 | known |
| 430 | 22591 | PSA |
| 431 | 22592 | known |
| 432 | 22593 | Previously identified P777P |
| 433 | 22594 | T cell receptor gamma chain |
| 434 | 22595 | Previously identified P705P |
| 435 | 22596 | Previously identified P707P |
| 436 | 22847 | PAP |
| 437 | 22848 | known |
| 438 | 22849 | prostatic secretory protein 57 |
| 439 | 22851 | PAP |
| 440 | 22852 | PAP |
| 441 | 22853 | PAP |
| 442 | 22854 | previously identified P509S |
| 443 | 22855 | previously identified P705P |
| 444 | 22856 | previously identified P774P |
| 445 | 22857 | PSA |
| 446 | 23601 | previously identified P777P |
| 447 | 23602 | PSA |
| 448 | 23605 | PSA |
| 449 | 23606 | PSA |
| 450 | 23612 | novel |
| 451 | 23614 | PSA |
| 452 | 23618 | previously identified P1000C |
| 453 | 23622 | previously identified P705P |

Further studies on the clone of SEQ ID NO: 407 (also referred to as P1020C) led to the isolation of an extended cDNA sequence provided in SEQ ID NO: 591. This extended cDNA sequence was found to contain an open reading frame that encodes the amino acid sequence of SEQ ID NO: 592. The P1020C cDNA and amino acid sequences were found to show some similarity to the human endogenous retroviral HERV-K pol gene and protein.

### EXAMPLE 15

### FURTHER IDENTIFICATION OF PROSTATE-SPECIFIC ANTIGENS BY MICROARRAY ANALYSIS

This Example describes the isolation of additional prostate-specific polypeptides from a prostate tumor cDNA library.

A human prostate tumor cDNA expression library as described above was screened using microarray analysis to identify clones that display at least a three fold over-expression in prostate tumor and/or normal prostate tissue, as compared to non-prostate normal tissues (not including testis). 142 clones were identified and sequenced. Certain of these clones are shown in SEQ ID NO: 454-467. Of these sequences, SEQ ID NO: 459-460 represent novel genes. The others (SEQ ID NO: 454-458 and 461-467) correspond to known sequences. Comparison of the determined cDNA sequence of SEQ ID NO: 461 with sequences in the Genbank database using the BLAST program revealed homology to the previously identified transmembrane protease serine 2 (TMPRSS2). The full-length cDNA sequence for this clone is provided in SEQ ID NO: 894, with the corresponding amino acid sequence being provided in SEQ ID NO: 895. The cDNA sequence encoding the first 209 amino acids of TMPRSS2 is provided in SEQ ID NO: 896, with the first 209 amino acids being provided in SEQ ID NO: 897.

The sequence of SEQ ID NO: 462 (referred to as P835P) was found to correspond to the previously identified clone FLJ13518 (Accession AK023643; SEQ ID NO: 917), which had no associated open reading frame (ORF). This clone was used to search the Geneseq DNA database and matched a clone previously identified as a G protein-coupled receptor protein (DNA Geneseq Accession A09351; amino acid Geneseq Accession Y92365), that is characterized by the presence of seven transmembrane domains. The sequences of fragments between these domains are provided in SEQ ID NO: 921-928, with SEQ ID NO: 921, 923, 925 and 927 representing extracellular domains and SEQ ID NO: 922, 924, 926 and 928 representing intracellular domains. SEQ ID NO: 921-928 represent amino acids 1-28, 53-61, 83-103, 124-143, 165-201, 226-238, 263-272 and 297-381, respectively, of P835P. The full-length cDNA sequence for P835P is provided in SEQ ID NO: 916. The cDNA sequence of the open reading frame for P835P, including stop codon, is provided in SEQ ID NO: 918, with the open reading frame without stop codon being provided in SEQ ID NO: 919 and the corresponding amino acid sequence being provided in SEQ ID NO: 920.

### EXAMPLE 16

### FURTHER CHARACTERIZATION OF PROSTATE-SPECIFIC ANTIGEN P71 0P

This Example describes the full length cloning of P710P.

The prostate cDNA library described above was screened with the P710P fragment described above. One million colonies were plated on LB/Ampicillin plates. Nylon membrane filters were used to lift these colonies, and the cDNAs picked up by these filters were then denatured and cross-linked to the filters by UV light. The P710P fragment was radiolabeled and used to hybridize with the filters. Positive cDNA clones were selected and their cDNAs recovered and sequenced by an automatic Perkin Elmer/Applied Biosystems Division Sequencer. Four sequences were obtained, and are presented in SEQ ID NO: 468-471. These sequences appear to represent different splice variants of the P710P gene. Subsequent comparison of the cDNA sequences of P710P with those in Genbank releaved homology to the DD3 gene (Genbank accession numbers AF103907 & AF103908). The cDNA sequence of DD3 is provided in SEQ ID NO: 690.

### EXAMPLE 17

### PROTEIN EXPRESSION OF PROSTATE-SPECIFIC ANTIGENS

This example describes the expression and purification of prostate-specific antigens in *E. coli,* baculovirus and mammalian cells.

### a) Expression of P501S in E. coli

Expression of the full-length form of P501 S was attempted by first cloning P501 S without the leader sequence (amino acids 36-553 of SEQ ID NO: 113) downstream of the first 30 amino acids of the M. *tuberculosis* antigen Ra12 (SEQ ID NO: 484) in pET17b. Specifically, P501S DNA was used to perform PCR using the primers AW025 (SEQ ID NO: 485) and AW003 (SEQ ID NO: 486). AW025 is a sense cloning primer that contains a HindIII site. AW003 is an antisense cloning primer that contains an EcoRI site. DNA amplification was performed using 5 µl 10X Pfu buffer, 1 µl 20 mM dNTPs, 1 µl each of the PCR primers at 10 µM concentration, 40 µl water, 1 µl Pfu DNA polymerase (Stratagene, La Jolla, CA) and 1 µl DNA at 100 ng/µl. Denaturation at 95°C was performed for 30 sec, followed by 10 cycles of 95°C for 30 sec, 60°C for 1 min and by 72°C for 3 min. 20 cycles of 95°C for 30 sec, 65°C for 1 min and by 72°C for 3 min, and lastly by 1 cycle of 72°C for 10 min. The PCR product was cloned to Ra12m/pET17b using HindIII and EcoRI. The sequence of the resulting fusion construct (referred to as Ra12-P501S-F) was confirmed by DNA sequencing.

The fusion construct was transformed into BL21 (DE3)pLysE, pLysS and CodonPlus *E. coli* (Stratagene) and grown overnight in LB broth with kanamycin. The resulting culture was induced with IPTG. Protein was transferred to PVDF membrane and blocked with 5% non-fat milk (in PBS-Tween buffer), washed three times and incubated with mouse anti-His tag antibody (Clontech) for 1 hour. The membrane was washed 3 times and probed with HRP-Protein A (Zymed) for 30 min. Finally, the membrane was washed 3 times and developed with ECL (Amersham). No expression was detected by Western blot. Similarly, no expression was detected by Western blot when the Ra12-P501S-F fusion was used for expression in BL21 CodonPlus by CE6 phage (Invitrogen).

An N-terminal fragment of P501 S (amino acids 36-325 of SEQ ID NO: 113) was cloned down-stream of the first 30 amino acids of the M. *tuberculosis* antigen Ra12 in pET17b as follows. P501 S DNA was used to perform PCR using the primers AW025 (SEQ ID NO: 485) and AW027 (SEQ ID NO: 487). AW027 is an antisense cloning primer that contains an EcoRI site and a stop codon. DNA amplification was performed essentially as described above. The resulting PCR product was cloned to Ra12 in pET17b at the HindIII and EcoRI sites. The fusion construct (referred to as Ra12-P501S-N) was confirmed by DNA sequencing.

The Ra12-P501S-N fusion construct was used for expression in BL21(DE3)pLysE, pLysS and CodonPlus, essentially as described above. Using Western blot analysis, protein bands were observed at the expected molecular weight of 36 kDa. Some high molecular weight bands were also observed, probably due to aggregation of the recombinant protein. No expression was detected by Western blot when the Ra12-P501S-F fusion was used for expression in BL21 CodonPlus by CE6 phage.

A fusion construct comprising a C-terminal portion of P501 S (amino acids 257-553 of SEQ ID NO: 113) located down-stream of the first 30 amino acids of the M. *tuberculosis* antigen Ra12 (SEQ ID NO: 484) was prepared as follows. P501 S DNA was used to perform PCR using the primers AW026 (SEQ ID NO: 488) and AW003 (SEQ ID NO: 486). AW026 is a sense cloning primer that contains a HindIII site. DNA amplification was performed essentially as described above. The resulting PCR product was cloned to Ra12 in pET17b at the HindIII and EcoRI sites. The sequence for the fusion construct (referred to as Ra12-P501S-C) was confirmed.

The Ra12-P501S-C fusion construct was used for expression in BL21 (DE3)pLysE, pLysS and CodonPlus, as described above. A small amount of protein was detected by Western blot, with some molecular weight aggregates also being observed. Expression was also detected by Western blot when the Ra12-P501S-C fusion was used for expression in BL21CodonPlus induced by CE6 phage.

A fusion construct comprising a fragment of P501 S (amino acids 36-298 of SEQ ID NO: 113) located down-stream of the M. *tuberculosis* antigen Ra12 (SEQ ID NO: 848) was prepared as follows. P501S DNA was used to perform PCR using the primers AW042 (SEQ ID NO: 849) and AW053 (SEQ ID NO: 850). AW042 is a sense cloning primer that contains a EcoRl site. AW053 is an antisense primer with stop and Xho I sites. DNA amplification was performed essentially as described above. The resulting PCR product was cloned to Ra12 in pET17b at the EcoRI and Xho I sites. The resulting fusion construct (referred to as Ra12-P501S-E2) was expressed in B834 (DE3) pLys S *E. coli* host cells in TB media for 2 h at room temperature. Expressed protein was purified by washing the inclusion bodies and running on a Ni-NTA column. The purified protein stayed soluble in buffer containing 20 mM Tris-HCl (pH 8), 100 mM NaCl, 10 mM β-Me and 5% glycerol. The determined cDNA and amino acid sequences for the expressed fusion protein are provided in SEQ ID NO: 851 and 852, respectfully.

### b) Expression of P501S in Baculovirus

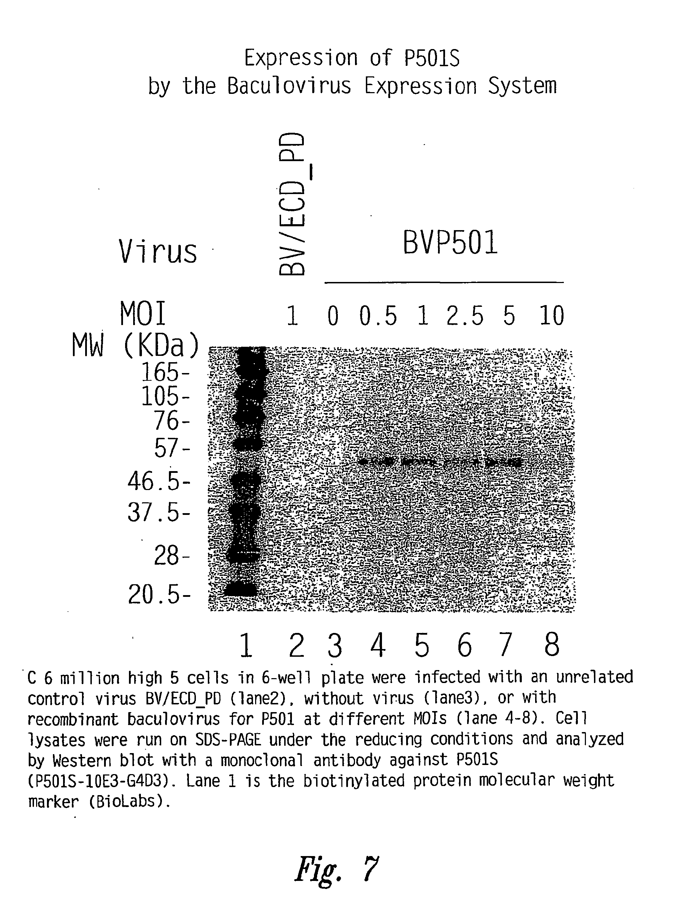

The Bac-to-Bac baculovirus expression system (BRL Life Technologies, Inc.) was used to express P501 S protein in insect cells. Full-length P501S (SEQ ID NO: 113) was amplified by PCR and cloned into the Xbal site of the donor plasmid pFastBacl. The recombinant bacmid and baculovirus were prepared according to the manufacturer's instructions. The recombinant baculovirus was amplified in Sf9 cells and the high titer viral stocks were utilized to infect High Five cells (Invitrogen) to make the recombinant protein. The identity of the full-length protein was confirmed by N-terminal sequencing of the recombinant protein and by Western blot analysis (Figure 7). Specifically, 0.6 million High Five cells in 6-well plates were infected with either the unrelated control virus BV/ECD_PD (lane 2), with recombinant baculovirus for P501 S at different amounts or MOIs (lanes 4-8), or were uninfected (lane 3). Cell lysates were run on SDS-PAGE under reducing conditions and analyzed by Western blot with the anti-P501 S monoclonal antibody P501S-10E3-G4D3 (prepared as described below). Lane 1 is the biotinylated protein molecular weight marker (BioLabs).

The localization of recombinant P501 S in the insect cells was investigated as follows. The insect cells overexpressing P501 S were fractionated into fractions of nucleus, mitochondria, membrane and cytosol. Equal amounts of protein from each fraction were analyzed by Western blot with a monoclonal antibody against P501 S. Due to the scheme of fractionation, both nucleus and mitochondria fractions contain some plasma membrane components. However, the membrane fraction is basically free from mitochondria and nucleus. P501 S was found to be present in all fractions that contain the membrane component, suggesting that P501 S may be associated with plasma membrane of the insect cells expressing the recombinant protein.

### c) Expression of P501 S in mammalian cells

Full-length P501 S (553 amino acids; SEQ ID NO: 113) was cloned into various mammalian expression vectors, including pCEP4 (Invitrogen), pVR1012 (Vical, San Diego, CA) and a modified form of the retroviral vector pBMN, referred to as pBIB. Transfection of P501S/pCEP4 and P501S/pVR1012 into HEK293 fibroblasts was carried out using the Fugene transfection reagent (Boehringer Mannheim). Briefly, 2 ul of Fugene reagent was diluted into 100 ul of serum-free media and incubated at room temperature for 5-10 min. This mixture was added to 1 ug of P501S plasmid DNA, mixed briefly and incubated for 30 minutes at room temperature. The Fugene/DNA mixture was added to cells and incubated for 24-48 hours. Expression of recombinant P501 S in transfected HEK293 fibroblasts was detected by means of Western blot employing a monoclonal antibody to P501 S.

Transfection of p501 S/pCEP4 into CHO-K cells (American Type Culture Collection, Rockville, MD) was carried out using GenePorter transfection reagent (Gene Therapy Systems, San Diego, CA). Briefly, 15 µl of GenePorter was diluted in 500 µl of serum-free media and incubated at room temperature for 10 min. The GenePorter/media mixture was added to 2 µg of plasmid DNA that was diluted in 500 µl of serum-free media, mixed briefly and incubated for 30 min at room temperature. CHO-K cells were rinsed in PBS to remove serum proteins, and the GenePorter/DNA mix was added and incubated for 5 hours. The transfected cells were then fed an equal volume of 2x media and incubated for 24-48 hours.

FACS analysis of P501S transiently infected CHO-K cells, demonstrated surface expression of P501 S. Expression was detected using rabbit polyclonal antisera raised against a P501 S peptide, as described below. Flow cytometric analysis was performed using a FaCScan (Becton Dickinson), and the data were analyzed using the Cell Quest program.

### d) Expression of P703P in Baculovirus

The cDNA for full-length P703P-DE5 (SEQ ID NO: 326), together with several flanking restriction sites, was obtained by digesting the plasmid pCDNA703 with restriction endonucleases Xba I and Hind III. The resulting restriction fragment (approx. 800 base pairs) was ligated into the transfer plasmid pFastBacl which was digested with the same restriction enzymes. The sequence of the insert was confirmed by DNA sequencing. The recombinant transfer plasmid pFBP703 was used to make recombinant bacmid DNA and baculovirus using the Bac-To-Bac Baculovirus expression system (BRL Life Technologies). High Five cells were infected with the recombinant virus BVP703, as described above, to obtain recombinant P703P protein.

### e) Expression of P788P in E. Coli

A truncated, N-terminal portion, of P788P (residues 1-644 of SEQ ID NO: 777; referred to as P788P-N) fused with a C-terminal 6xHis Tag was expressed in *E. coli* as follows. P788P cDNA was amplified using the primers AW080 and AW081 (SEQ ID NO: 815 and 816). AW080 is a sense cloning primer with an Ndel site. AW081 is an antisense cloning primer with a Xhol site. The PCR-amplified P788P, as well as the vector pCRX1, were digested with Ndel and Xhol. Vector and insert were ligated and transformed into NovaBlue cells. Colonies were randomly screened for insert and then sequenced. P788P-N clone #6 was confirmed to be identical to the designed construct. The expression construct P788P-N #6/pCRX1 was transformed into *E. coli* BL21 CodonPlus-RIL competent cells. After induction, most of the cells grew well, achieving OD600 of greater than 2.0 after 3 hr. Coomassie stained SDS-PAGE showed an over-expressed band at about 75 kD. Western blot analysis using a 6xHisTag antibody confirmed the band was P788P-N. The determined cDNA sequence for P788P-N is provided in SEQ ID NO: 817, with the corresponding amino acid sequence being provided in SEQ ID NO: 818.

### f) Expression of P510S in E. coli

The P510S protein has 9 potential transmembrane domains and is predicted to be located at the plasma membrane. The C-terminal protein of this protein, as well as the predicted third extracellular domain of P510S were expressed in *E. coli* as follows.

The expression construct referred to as Ra12-P501S-C was designed to have a 6 HisTag at the N-terminal enc, followed by the M. *tuberculosis* antigen Ra12 (SEQ ID NO: 819) and then the C-terminal portion of P510S (amino residues 1176-1261 of SEQ ID NO: 538). Full-length P510S was used to amplify the P510S-C fragment by PCR using the primers AW056 and AW057 (SEQ ID NO: 820 and 821, respectively). AW056 is a sense cloning primer with an EcoRl site. AW057 is an antisense primer with stop and Xhol sites. The amplified P501S fragment and Ra12/pCRX1 were digested with EcoRI and Xhol and then purified. The insert and vector were ligated together and transformed into NovaBlue. Colonies were randomly screened for insert and sequences. For protein expression, the expression construct was transformed into *E*. *coli* BL21 (DE3) CodonPlus-RIL competent cells. A mini-induction screen was performed to optimize the expression conditions. After induction the cells grew well, achieving OD 600 nm greater than 2.0 after 3 hours. Coomassie stain SDS-PAGE showed a highly over-expressed band at approx. 30 kD. Though this is higher than the expected molecular weight, western blot analysis was positive, showing this band to be the His tagcontaining protein. The optimized culture conditions are as follows. Dilute overnight culture/daytime culture (LB + kanamycin + chloramphenicol) into 2xYT (with kanamycin and chloramphenicol) at a ratio of 25 ml culture to 1 liter 2xYT. Allow to grow at 37°C until OD600 = 0.6. Take an aliquot out as T0 sample. Add 1 mM IPTG and allow to grow at 30°C for 3 hours. Take out a T3 sample, spin down cells and store at -80°C. The determined cDNA and amino acid sequences for the Ra12-P510S-C construct are provided in SEQ ID NO: 822 and 825, respectively.

The expression construct P510S-C was designed to have a 5' added start codon and a glycine (GGA) codon and then the P510S C terminal fragment followed by the in frame 6x histidine tag and stop codon from the pET28b vector. The cloning strategy is similar to that used for Ra12-P510S-C, except that the PCR primers employed were those shown in SEQ ID NO: 828 and 829, respectively and the Ncol/Xhol cut in pET28b was used. The primer of SEQ ID NO: 828 created a 5' Ncol site and added a start codon. The antisense primer of SEQ ID NO: 829 creates a Xhol site on P510S C terminal fragment. Clones were confirmed by sequencing. For protein expression, the expression construct was transformed into *E*. *coli* BL21 (DE3) CodonPlus-RIL competent cells. An OD600 of greater than 2.0 was obtained 30 hours after induction. Coomassie stained SDS-PAGE showed an over-expressed band at about 11 kD. Western blot analysis confirmed that the band was P510S-C, as did N-terminal protein sequencing. The optimized culture conditions are as follows: dilute overnight culture/daytime culture (LB + kanamycin + chloramphenicol) into 2x YT (+ kanamycin and chloramphenicol) at a ratio of 25 mL culture to 1 liter 2x YT, and allow to grow at 37°C until an OD 600 of about 0.5 is reached. Take out an aliquot as T0 sample. Add 1 mM IPTG and allow to grow at 30°C for 3 hours. Spin down the cells and store at -80°C until purification. The determined cDNA and amino acid sequences for the P510S-C construct are shown in SEQ ID NO: 823 and 826, respectively.

The predicted third extracellular domain of P510S (P510S-E3; residues 328-676 of SEQ ID NO: 538) was expressed in *E*. *coli* as follows. The P510S fragment was amplified by PCR using the primers shown in SEQ ID NO: 830 and 831. The primer of SEQ ID NO: 830 is a sense primer with an Ndel site for use in ligating into pPDM. The primer of SEQ ID NO: 831 is an antisense primer with an added Xhol site for use in ligating into pPDM. The resulting fragment was cloned to pPDM at the Ndel and Xhol sites. Clones were confirmed by sequencing. For protein expression, the clone ws transformed into E. coli BL21 (DE3) CodonPlus-RIL competent cells. After induction, an OD600 of greater than 2.0 was achieved after 3 hours. Coomassie stained SDS-PAGE showed an over-expressed band at about 39 kD, and N-terminal sequencing confirmed the N-terminal to be that of P510S-E3. Optimized culture conditions are as follows: dilute overnight culture/daytime culture (LB + kanamycin + chloramphenicol) into 2x YT (kanamycin and chloramphenicol) at a ratio of 25 ml culture to 1 liter 2x YT. Allow to grow at 37°C until OD 600 equals 0.6. Take out an aliquot as T0 sample. Add 1 mM IPTG and allow to grow at 30°C for 3 hours. Take out a T3 sample, spin down the cells and store at -80°C until purification. The determined cDNA and amino acid sequences for the P501 S-E3 construct are provided in SEQ ID NO: 824 and 827, respectively.

### g) Expression of P775S in E. Coli

The antigen P775P contains multiple open reading frames (ORF). The third ORF, encoding the protein of SEQ ID NO: 483, has the best emotif score. An expression fusion construct containing the M. *tuberculosis* antigen Ra12 (SEQ ID NO: 819) and P775P-ORF3 with an N-terminal 6x HisTag was prepared as follows. P775P-ORF3 was amplified using the sense PCR primers of SEQ ID NO: 832 and the anti-sense PCR primer of SEQ ID NO: 833. The PCR amplified fragment of P775P and Ra12/pCRX1 were digested with the restriction enzymes EcoRI and XhoI. Vector and insert were ligated and then transformed into NovaBlue cells. Colonies were randomly screened for insert and then sequenced. A clone having the desired sequence was transformed into E. coli BL21 (DE3) CodonPlus-RIL competent cells. Two hours after induction, the cell density peaked at OD600 of approximately 1.8. Coomassie stained SDS-PAGE showed an over-expressed band at about 31 kD. Western blot using 6x HisTag antibody confirmed that the band was Ra12-P775P-ORF3. The determined cDNA and amino acid sequences for the fusion construct are provided in SEQ ID NO: 834 and 835, respectively.

### h) Expression of a P703P His tag fusion protein in E. coli

The cDNA for the coding region of P703P was prepared by PCR using the primers of SEQ ID NO: 836 and 837. The PCR product was digested with EcoRI restriction enzyme, gel purified and cloned into a modified pET28 vector with a His tag in frame, which had been digested with Eco72I and EcoRI restriction enzymes. The correct construct was confirmed by DNA sequence analysis and then transformed into *E*. *coli* BL21 (DE3) pLys S expression host cells. The determined amino acid and cDNA sequences for the expressed recombinant P703P are provided in SEQ ID NO: 838 and 839, respectively.

### i) Expression of a P705P His tag fusion protein in E. coli

The cDNA for the coding region of P705P was prepared by PCR using the primers of SEQ ID NO: 840 and 841. The PCR product was digested with EcoRl restriction enzyme, gel purified and cloned into a modified pET28 vector with a His tag in frame, which had been digested with Eco721 and EcoRl restriction enzymes. The correct construct was confirmed by DNA sequence analysis and then transformed into *E. coli* BL21 (DE3) pLys S and BL21 (DE3) CodonPlus expression host cells. The determined amino acid and cDNA sequences for the expressed recombinant P705P are provided in SEQ ID NO: 842 and 843, respectively.

### j) Expression of a P711P His tag fusion protein in E. coli

The cDNA for the coding region of P711 P was prepared by PCR using the primers of SEQ ID NO: 844 and 845. The PCR product was digested with EcoRl restriction enzyme, gel purified and cloned into a modified pET28 vector with a His tag in frame, which had been digested with Eco721 and EcoRl restriction enzymes. The correct construct was confirmed by DNA sequence analysis and then transformed into *E*. *coli* BL21 (DE3) pLys S and BL21 (DE3) CodonPlus expression host cells. The determined amino acid and cDNA sequences for the expressed recombinant P711 P are provided in SEQ ID NO: 846 and 847, respectively.

### k) Expression of P767P in E. coli

The full-length coding region of P767P (amino acids 2-374 of SEQ ID NO: 590) was amplified by PCR using the primers PDM-468 and PDM-469 (SEQ ID NO: 935 and 936, respectively). DNA amplification was performed using 10 µl 10X Pfu buffer, 1 µl 10 mM dNTPs, 2 µl each of the PCR primers at 10 µM concentration, 83 µl water, 1.5 µl Pfu DNA polymerase (Stratagene, La Jolla, CA) and 1 µl DNA at 100 ng/µl. Denaturation at 96°C was performed for 2 min, followed by 40 cycles of 96°C for 20 sec, 66°C for 15 sec and by 72°C for 2 min., and lastly by 1 cycle of 72°C for 4 min. The PCR product was digested with Xhol and cloned into a modified pET28 vector with a histidine tag in frame on the 5' end that was digested with Eco721 and Xhol. The construct was confirmed to be correct through sequence analysis and transformed into *E*. *coli* BL21 pLysS and BL21 CodonPlus RP cells. The cDNA coding region for the recombinant B767P protein is provided in SEQ ID NO: 938, with the corresponding amino acid sequence being provided in SEQ ID NO: 941. The full-length P767P did not express at high enough levels for detection or purification.

A truncated coding region of P767P (referred to as B767P-B; amino acids 47-374 of SEQ ID NO: 590) was amplified by PCR using the primers PDM-573 and PDM-469 (SEQ ID NO: 937 and 936, respectively) and the PCR conditions described above for full-length P767P. The PCR product was digested with Xhol and cloned into the modified pET28 vector that was digested with Eco72I and Xhol. The construct was confirmed to be correct through sequence analysis and transformed into *E*. *coli* BL21 pLysS and BL21 CodonPlus RP cells. The protein was found to be expressed in the inclusion body pellet. The coding region for the expressed B767P-B protein is provided in SEQ ID NO: 939, with the corresponding amino acid sequence being provided in SEQ ID NO: 940.

### I) Expression of P767P in Mammalian Cells

For recombinant expression in mammalian cells, the full length P767P cDNA (SEQ ID NO: 587) was subcloned into the mammalian expression vector pCEP4 (Invitrogen) that was modified to contain a FLAG epitope tag. This construct was transfected into HEK293 cells (American Type Culture Collection) using Fugene 6 reagent (Roche). Briefly, the HEK cells were plated at a density of 100,000 cells/ml in DMEM (Gibco) containing 10% FBS (Hyclone) and grown overnight. The following day, 2 ul of Fugene 6 was added to 100 ul of DMEM containing no FBS and incubated for 15 minutes at room temperature. The Fugene 6/DMEM mixture was then added to 1 ug of P767P/pCEP4 plasmid DNA and incubated for 15 minutes at room temperature. The Fugene/DNA mix was then added to the HEK293 cells and incubated for 48-72 hrs at 37° C with 7% CO₂. Cells were rinsed with PBS then collected and pelleted by centrifugation.

P767P expression was detect in transfected HEK293 whole cell lysates by Western blot analysis using an anti-epitope tag antibody. Specifically, whole cell lysates were generated by incubating the cells in Triton-X100 containing lysis buffer for 30 minutes on ice. Lysates were then cleared by centrifugation at 5,000 rpm for 5 minutes at 4° C. Samples were diluted with SDS-PAGE loading buffer containing beta-mercaptoethanol, then boiled for 10 minutes prior to loading the SDS-PAGE gel. Protein was transferred to nitrocellulose and probed using an anti-FLAG mouse monoclonal antibody (Sigma) at a dilution of 1 ug/ml. The blot was revealed with a donkey anti-mouse lg coupled to HRP followed by incubation in ECL substrate.

P767P was found to be expressed on the surface of transfected HEK293 cells by flow cytometry using rabbit anti-P767P sera as follows. Cells were collected and washed with ice cold staining buffer (PBS+1 %BSA+Azide). Next, the cells were incubated for 30 minutes on ice with undiluted anti-P767P rabbit polyclonal sera. The cells were washed 3 times with staining buffer and then incubated with a 1:100 dilution of a goat anti-rabbit lg(H+L)-FITC reagent (Southern Biotechnology) for 30 minutes on ice. Following 3 washes, the cells were resuspended in staining buffer containing Propidium lodide (PI), a vital stain that allows for identification of permeable cells, and analyzed by FACS.

### m) Expression of P788P in Mammalian Cells

For recombinant expression in mammalian cells, the full length P788P cDNA was subcloned into the mammalian expression vector pcDNA3.1 (Invitrogen). This construct was transfected into HEK293 cells (American Type Culture Collection) using Fugene 6 reagent (Roche). Briefly, the HEK cells were plated at a density of 100,000 cells/ml in DMEM (Gibco) containing 10% FBS (Hyclone) and grown overnight. The following day, 2 ul of Fugene6 was added to 100 ul of DMEM containing no FBS and incubated for 15 minutes at room temperature. The Fugene6/DMEM mixture was then added to 1ug of P788P/pcDNA3.1 plasmid DNA and incubated for 15 minutes at room temperature. The Fugene/DNA mix was then added to the HEK293 cells and incubated for 48-72 hrs at 37° C with 7% CO₂. Cells were rinsed with PBS then collected and pelleted by centrifugation.

P788P expression was detected in transfected HEK293 whole cell lysates by Western blot analysis using rabbit anti-P788P sera as probe. Specifically, whole cell lysates were generated by incubating the cells in Triton-X100 containing lysis buffer for 30 minutes on ice. Lysates were then cleared by centrifugation at 5,000 rpm for 5 minutes at 4° C. Samples were diluted with SDS-PAGE loading buffer containing beta-mercaptoethanol, then boiled for 10 minutes prior to loading the SDS-PAGE gel. Protein was transferred to nitrocellulose and probed using purified anti-P788P rabbit polyclonal sera at a concentration of 1 ug/ml. The blot was revealed with a donkey anti-rabbit lg coupled to HRP (Jackson ImmunoResearch) followed by incubation in ECL substrate.

P788P expression was detected on the surface of transfected HEK293 cells by flow cytometry using rabbit anti-P788P sera. For FACS analysis, cells were collected and washed with ice cold staining buffer (PBS+1%BSA+Azide). Next, the cells were incubated for 30 minutes on ice with anti-P788P polyclonal sera (10ug/ml final concentration). The cells were washed 3 times with staining buffer and then incubated with a 1:100 dilution of a goat anti-rabbit lg(H+L)-FITC reagent (Southern Biotechnology) for 30 minutes on ice. Following 3 washes, the cells were resuspended in staining buffer containing Propidium iodide (PI), a vital stain that allows for identification of permeable cells, and analyzed by flow cytometry.

### EXAMPLE 18

### PREPARATION AND CHARACTERIZATION OF ANTIBODIES AGAINST PROSTATE-SPECIFIC POLYPEPTIDES

### a) Preparation and Characterization of Polyclonal Antibodies against P703P, P504S and P509S

Polyclonal antibodies against P703P, P504S, P509S, P501 S, P510S-E3, P510S-C, P705P, full-length P703P (referred to as P703PFL), P711 P, P767P, P788P-N, and peptides of P790P and P775P were prepared as follows. The amino acid sequences of the P790P peptides (referred to as P790P89, P790P120, P790P163, P790P180, P790P222, P790P261, P790P287 and P790P307, respectively) are provided in SEQ ID NO: 949-956, with the amino acid sequences of the P775P peptides being provided in SEQ ID NO: 957-966.

Each prostate tumor antigen expressed in an *E*. *coli* recombinant expression system was grown overnight in LB broth with the appropriate antibiotics at 37°C in a shaking incubator. The next morning, 10 ml of the overnight culture was added to 500 ml to 2x YT plus appropriate antibiotics in a 2L-baffled Erlenmeyer flask. When the Optical Density (at 560 nm) of the culture reached 0.4-0.6, the cells were induced with IPTG (1 mM). Four hours after induction with IPTG, the cells were harvested by centrifugation. The cells were then washed with phosphate buffered saline and centrifuged again. The supernatant was discarded and the cells were either frozen for future use or immediately processed. Twenty ml of lysis buffer was added to the cell pellets and vortexed. To break open the *E. coli* cells, this mixture was then run through the French Press at a pressure of 16,000 psi. The cells were then centrifuged again and the supernatant and pellet were checked by SDS-PAGE for the partitioning of the recombinant protein. For proteins that localized to the cell pellet, the pellet was resuspended in 10 mM Tris pH 8.0, 1 % CHAPS and the inclusion body pellet was washed and centrifuged again. This procedure was repeated twice more. The washed inclusion body pellet was solubilized with either 8 M urea or 6 M guanidine HCl containing 10 mM Tris pH 8.0 plus 10 mM imidazole. The solubilized protein was added to 5 ml of nickel-chelate resin (Qiagen) and incubated for 45 min to 1 hour at room temperature with continuous agitation. After incubation, the resin and protein mixture were poured through a disposable column and the flow through was collected. The column was then washed with 10-20 column volumes of the solubilization buffer. The antigen was then eluted from the column using 8M urea, 10 mM Tris pH 8.0 and 300 mM imidazole and collected in 3 ml fractions. A SDS-PAGE gel was run to determine which fractions to pool for further purification.

As a final purification step, a strong anion exchange resin such as HiPrepQ (Biorad) was equilibrated with the appropriate buffer and the pooled fractions from above were loaded onto the column. Each antigen was eluted off the column with a increasing salt gradient. Fractions were collected as the column was run and another SDS-PAGE gel was run to determine which fractions from the column to pool. The pooled fractions were dialyzed against 10 mM Tris pH 8.0. The proteins were then vialed after filtration through a 0.22 micron filter and the antigens were frozen until needed for immunization.

The P790P and P775P peptides were synthesized, conjugated to KLH and frozen until needed for immunization.

Four hundred micrograms of each prostate antigen or peptide was combined with 100 micrograms of muramyldipeptide (MDP) and injected into a rabbit. Every four weeks rabbits were boosted with 100 micrograms of antigen/peptide mixed with an equal volume of Incomplete Freund's Adjuvant (IFA). Seven days following each boost, the animal was bled. Sera were generated by incubating the blood at 4°C for 12-24 hours followed by centrifugation.

Ninety-six well plates were coated with antigen by incubating with 50 microliters (typically 1 microgram/microliter) of recombinant protein at 4°C for 20 hours. 250 microliters of BSA blocking buffer was added to the wells and incubated at room temperature for 2 hours. Plates were washed 6 times with PBS/0.1 % Tween. Rabbit sera was diluted in PBS/0.1 % Tween/0.1% BSA. Fifty microliters of diluted sera was added to each well and incubated at room temperature for 30 min. Plates were washed as described above before 50 microliters of goat anti-rabbit horse radish peroxidase (HRP) at a 1:10000 dilution was added and incubated at room temperature for 30 min. Plates were again washed as described above and 100 microliters of TMB microwell peroxidase substrate was added to each well. Following a 15 min incubation in the dark at room temperature, the colorimetric reaction was stopped with 100 microliters of 1 N H₂SO₄ and read immediately at 450 nm. All polyclonal antibodies showed immunoreactivity to the appropriate antigen or peptide.

In order to determine which tissues express P509S, immunohistochemistry (IHC) analysis was performed on a diverse range of tissue sections. Tissue samples were fixed in formalin solution for 12-24 hrs and embedded in paraffin before being sliced into 8 micron sections. Steam heat induced epitope retrieval (SHIER) in 0.1 M sodium citrate buffer (pH 6.0) was used for optimal staining conditions. Sections were incubated with 10% serum/PBS for 5 minutes. Primary antibody was added to each section for 25 minutes at indicated concentrations followed by 25 minute incubation with either anti-rabbit or anti-mouse biotinylated antibody. Endogenous peroxidase activity was blocked by three 1.5 minute incubations with hydrogen peroxidase. The avidin biotin complex/horse radish peroxidase (ABC/HRP) system was used along with DAB chromogen to visualize antigen expression. Slides were counterstained with hematoxylin to visualize cell nuclei. P509S expression was detected in prostate cancer, BPH, colon, and kidney but not in heart, lung and liver.

### b) Preparation and Characterization of Antibodies against P501S

A murine monoclonal antibody directed against the carboxy-terminus of the prostate-specific antigen P501 S was prepared as follows.

A truncated fragment of P501 S (amino acids 355-526 of SEQ ID NO: 113) was generated and cloned into the pET28b vector (Novagen) and expressed in *E. coli* as a thioredoxin fusion protein with a histidine tag. The trx-P501S fusion protein was purified by nickel chromatography, digested with thrombin to remove the trx fragment and further purified by an acid precipitation procedure followed by reverse phase HPLC.

Mice were immunized with truncated P501 S protein. Serum bleeds from mice that potentially contained anti-P501 S polyclonal sera were tested for P501 S-specific reactivity using ELISA assays with purified P501 S and trx-P501 S proteins. Serum bleeds that appeared to react specifically with P501 S were then screened for P501 S reactivity by Western analysis. Mice that contained a P501 S-specific antibody component were sacrificed and spleen cells were used to generate anti-P501 S antibody producing hybridomas using standard techniques. Hybridoma supernatants were tested for P501S-specific reactivity initially by ELISA, and subsequently by FACS analysis of reactivity with P501 S transduced cells. Based on these results, a monoclonal hybridoma referred to as 10E3 was chosen for further subcloning. A number of subclones were generated, tested for specific reactivity to P501S using ELISA and typed for IgG isotype. The results of this analysis are shown below in Table V. Of the 16 subclones tested, the monoclonal antibody 10E3-G4-D3 was selected for further study.

**TABLE VI**

| ISOTYPE ANALYSIS OF MURINE ANTI-P501S MONOCLONAL ANTIBODIES | | |
|---|---|---|
| Hybridoma clone | Isotype | Estimated [lg] in supernatant (µg/ml) |
| 4D11 | IgG1 | 14.6 |
| 1G1 | IgG1 | 0.6 |
| 4F6 | IgG1 | 72 |
| 4H5 | IgG1 | 13.8 |
| 4H5-E12 | IgG1 | 10.7 |
| 4H5-EH2 | IgG1 | 9.2 |
| 4H5-H2-A10 | IgG1 | 10 |
| 4H5-H2-A3 | IgG1 | 12.8 |
| 4H5-H2-A10-G6 | IgG1 | 13.6 |
| 4H5-H2-B11 | IgG1 | 12.3 |
| 10E3 | IgG2a | 3.4 |
| 10E3-D4 | IgG2a | 3.8 |
| 10E3-D4-G3 | IgG2a | 9.5 |
| 10E3-D4-G6 | IgG2a | 10.4 |
| 10E3-E7 | IgG2a | 6.5 |
| 8H12 | IgG2a | 0.6 |

The specificity of 10E3-G4-D3 for P501 S was examined by FACS analysis. Specifically, cells were fixed (2% formaldehyde, 10 minutes), permeabilized (0.1% saponin, 10 minutes) and stained with 10E3-G4-D3 at 0.5 - 1 µg/ml, followed by incubation with a secondary, FITC-conjugated goat anti-mouse lg antibody (Pharmingen, San Diego, CA). Cells were then analyzed for FITC fluorescence using an Excalibur fluorescence activated cell sorter. For FACS analysis of transduced cells, B-LCL were retrovirally transduced with P501 S. For analysis of infected cells, B-LCL were infected with a vaccinia vector that expresses P501 S. To demonstrate specificity in these assays, B-LCL transduced with a different antigen (P703P) and uninfected B-LCL vectors were utilized. 10E3-G4-D3 was shown to bind with P501 S-transduced B-LCL and also with P501 S-infected B-LCL, but not with either uninfected cells or P703P-transduced cells.

To determine whether the epitope recognized by 10E3-G4-D3 was found on the surface or in an intracellular compartment of cells, B-LCL were transduced with P501 S or HLA-B8 as a control antigen and either fixed and permeabilized as described above or directly stained with 10E3-G4-D3 and analyzed as above. Specific recognition of P501 S by 1 0E3-G4-D3 was found to require permeabilization, suggesting that the epitope recognized by this antibody is intracellular.

The reactivity of 10E3-G4-D3 with the three prostate tumor cell lines Lncap, PC-3 and DU-145, which are known to express high, medium and very low levels of P501 S, respectively, was examined by permeabilizing the cells and treating them as described above. Higher reactivity of 10E3-G4-D3 was seen with Lncap than with PC-3, which in turn showed higher reactivity that DU-145. These results are in agreement with the real time PCR and demonstrate that the antibody specifically recognizes P501 S in these tumor cell lines and that the epitope recognized in prostate tumor cell lines is also intracellular.

Specificity of 10E3-G4-D3 for P501 S was also demonstrated by Western blot analysis. Lysates from the prostate tumor cell lines Lncap, DU-145 and PC-3, from P501 S-transiently transfected HEK293 cells, and from non-transfected HEK293 cells were generated. Western blot analysis of these lysates with 10E3-G4-D3 revealed a 46 kDa immunoreactive band in Lncap, PC-3 and P501 S-transfected HEK cells, but not in DU-145 cells or non-transfected HEK293 cells. P501 S mRNA expression is consistent with these results since semi-quantitative PCR analysis revealed that P501 S mRNA is expressed in Lncap, to a lesser but detectable level in PC-3 and not at all in DU-145 cells. Bacterially expressed and purified recombinant P501 S (referred to as P501 SStr2) was recognized by 10E3-G4-D3 (24 kDa), as was full-length P501 S that was transiently expressed in HEK293 cells using either the expression vector VR1012 or pCEP4. Although the predicted molecular weight of P501 S is 60.5 kDa, both transfected and "native" P501 S run at a slightly lower mobility due to its hydrophobic nature.

Immunohistochemical analysis was performed on prostate tumor and a panel of normal tissue sections (prostate, adrenal, breast, cervix, colon, duodenum, gall bladder, ileum, kidney, ovary, pancreas, parotid gland, skeletal muscle, spleen and testis). Tissue samples were fixed in formalin solution for 24 hours and embedded in paraffin before being sliced into 10 micron sections. Tissue sections were permeabilized and incubated with 10E3-G4-D3 antibody for 1 hr. HRP-labeled anti-mouse followed by incubation with DAB chromogen was used to visualize P501 S immunoreactivity. P501 S was found to be highly expressed in both normal prostate and prostate tumor tissue but was not detected in any of the other tissues tested.

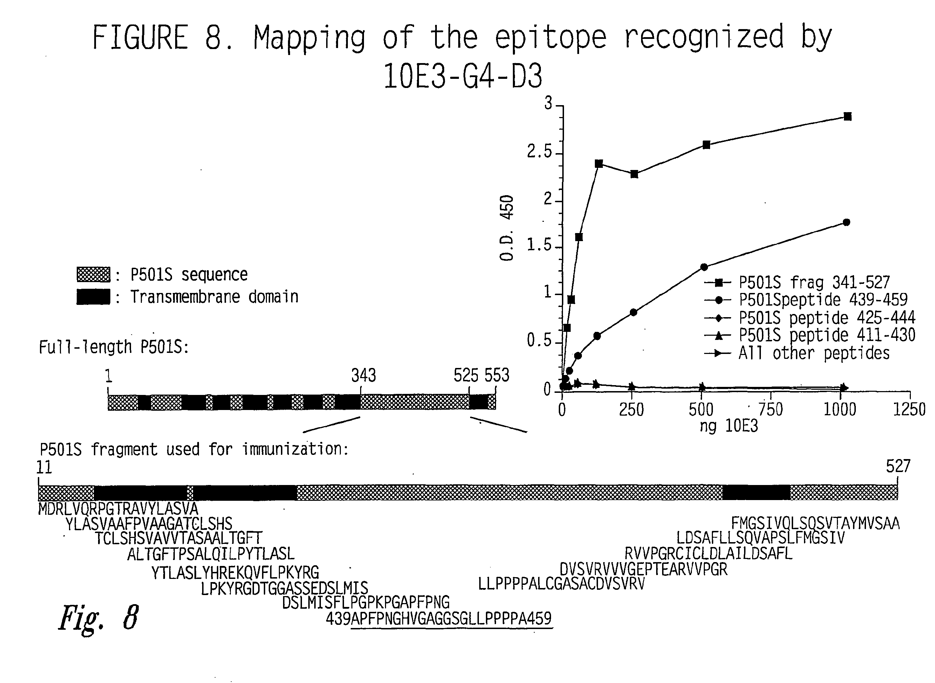

To identify the epitope recognized by 10E3-G4-D3, an epitope mapping approach was pursued. A series of 13 overlapping 20-21 mers (5 amino acid overlap; SEQ ID NO: 489-501) was synthesized that spanned the fragment of P501 S used to generate 10E3-G4-D3. Flat bottom 96 well microtiter plates were coated with either the peptides or the P501 S fragment used to immunize mice, at 1 microgram/ml for 2 hours at 37°C. Wells were then aspirated and blocked with phosphate buffered saline containing 1% (w/v) BSA for 2 hours at room temperature, and subsequently washed in PBS containing 0.1 % Tween 20 (PBST). Purified antibody 10E3-G4-D3 was added at 2 fold dilutions (1000 ng - 16 ng) in PBST and incubated for 30 minutes at room temperature. This was followed by washing 6 times with PBST and subsequently incubating with HRP-conjugated donkey anti-mouse IgG (H+L)Affinipure F(ab') fragment (Jackson Immunoresearch, West Grove, PA) at 1:20000 for 30 minutes. Plates were then washed and incubated for 15 minutes in tetramethyl benzidine. Reactions were stopped by the addition of 1 N sulfuric acid and plates were read at 450 nm using an ELISA plate reader. As shown in Fig. 8, reactivity was seen with the peptide of SEQ ID NO: 496 (corresponding to amino acids 439-459 of P501 S) and with the P501 S fragment but not with the remaining peptides, demonstrating that the epitope recognized by 10E3-G4-D3 is localized to amino acids 439-459 of SEQ ID NO: 113.

In order to further evaluate the tissue specificity of P501 S, multi-array immunohistochemical analysis was performed on approximately 4700 different human tissues encompassing all the major normal organs as well as neoplasias derived from these tissues. Sixty-five of these human tissue samples were of prostate origin. Tissue sections 0.6 mm in diameter were formalin-fixed and paraffin embedded. Samples were pretreated with HIER using 10 mM citrate buffer pH 6.0 and boiling for 10 min. Sections were stained with 10E3-G4-D3 and P501S immunoreactivity was visualized with HRP. All the 65 prostate tissues samples (5 normal, 55 untreated prostate tumors, 5 hormone refractory prostate tumors) were positive, showing distinct perinuclear staining. All other tissues examined were negative for P501 S expression.

### c) Preparation and Characterization of Antibodies against P503S

A fragment of P503S (amino acids 113-241 of SEQ ID NO: 114) was expressed and purified from bacteria essentially as described above for P501S and used to immunize both rabbits and mice. Mouse monoclonal antibodies were isolated using standard hybridoma technology as described above. Rabbit monoclonal antibodies were isolated using Selected Lymphocyte Antibody Method (SLAM) technology at Immgenics Pharmaceuticals (Vancouver, BC, Canada). Table VI, below, lists the monoclonal antibodies that were developed against P503S.

**TABLE VII**

| Antibody | Species |
|---|---|
| 20D4 | Rabbit |
| JA1 | Rabbit |
| 1A4 | Mouse |
| 1 C3 | Mouse |
| 1C9 | Mouse |
| 1D12 | Mouse |
| 2A11 | Mouse |
| 2H9 | Mouse |
| 4H7 | Mouse |
| 8A8 | Mouse |
| 8D10 | Mouse |
| 9C12 | Mouse |
| 6D12 | Mouse |

The DNA sequences encoding the complementarity determining regions (CDRs) for the rabbit monoclonal antibodies 20D4 and JA1 were determined and are provided in SEQ ID NO: 502 and 503, respectively.

In order to better define the epitope binding region of each of the antibodies, a series of overlapping peptides were generated that span amino acids 109-213 of SEQ ID NO: 114. These peptides were used to epitope map the anti-P503S monoclonal antibodies by ELISA as follows. The recombinant fragment of P503S that was employed as the immunogen was used as a positive control. Ninety-six well microtiter plates were coated with either peptide or recombinant antigen at 20 ng/well overnight at 4°C. Plates were aspirated and blocked with phosphate buffered saline containing 1 % (w/v) BSA for 2 hours at room temperature then washed in PBS containing 0.1 % Tween 20 (PBST). Purified rabbit monoclonal antibodies diluted in PBST were added to the wells and incubated for 30 min at room temperature. This was followed by washing 6 times with PBST and incubation with Protein-A HRP conjugate at a 1:2000 dilution for a further 30 min. Plates were washed six times in PBST and incubated with tetramethylbenzidine (TMB) substrate for a further 15 min. The reaction was stopped by the addition of 1 N sulfuric acid and plates were read at 450 nm using at ELISA plate reader. ELISA with the mouse monoclonal antibodies was performed with supernatants from tissue culture run neat in the assay.

All of the antibodies bound to the recombinant P503S fragment, with the exception of the negative control SP2 supernatant. 20D4, JA1 and 1 D12 bound strictly to peptide #2101 (SEQ ID NO: 504), which corresponds to amino acids 151-169 of SEQ ID NO: 114. 1C3 bound to peptide #2102 (SEQ ID NO: 505), which corresponds to amino acids 165-184 of SEQ ID NO: 114. 9C12 bound to peptide #2099 (SEQ ID NO: 522), which corresponds to amino acids 120-139 of SEQ ID NO: 114. The other antibodies bind to regions that were not examined in these studies.

Subsequent to epitope mapping, the antibodies were tested by FACS analysis on a cell line that stably expressed P503S to confirm that the antibodies bind to cell surface epitopes. Cells stably transfected with a control plasmid were employed as a negative control. Cells were stained live with no fixative. 0.5 ug of anti-P503S monoclonal antibody was added and cells were incubated on ice for 30 min before being washed twice and incubated with a FITC-labelled goat anti-rabbit or mouse secondary antibody for 20 min. After being washed twice, cells were analyzed with an Excalibur fluorescent activated cell sorter. The monoclonal antibodies 1C3, 1D12, 9C12, 20D4 and JA1, but not 8D3, were found to bind to a cell surface epitope of P503S.

In order to determine which tissues express P503S, immunohistochemical analysis was performed, essentially as described above, on a panel of normal tissues (prostate, adrenal, breast, cervix, colon, duodenum, gall bladder, ileum, kidney, ovary, pancreas, parotid gland, skeletal muscle, spleen and testis). HRP-labeled anti-mouse or anti-rabbit antibody followed by incubation with TMB was used to visualize P503S immunoreactivity. P503S was found to be highly expressed in prostate tissue, with lower levels of expression being observed in cervix, colon, ileum and kidney, and no expression being observed in adrenal, breast, duodenum, gall bladder, ovary, pancreas, parotid gland, skeletal muscle, spleen and testis.

Western blot analysis was used to characterize anti-P503S monoclonal antibody specificity. SDS-PAGE was performed on recombinant (rec) P503S expressed in and purified from bacteria and on lysates from HEK293 cells transfected with full length P503S. Protein was transferred to nitrocellulose and then Western blotted with each of the anti-P503S monoclonal antibodies (20D4, JA1, 1D12, 6D12 and 9C12) at an antibody concentration of 1 ug/ml. Protein was detected using horse radish peroxidase (HRP) conjugated to either a goat anti-mouse monoclonal antibody or to protein A-sepharose. The monoclonal antibody 20D4 detected the appropriate molecular weight 14 kDa recombinant P503S (amino acids 113-241) and the 23.5 kDa species in the HEK293 cell lysates transfected with full length P503S. Other anti-P503S monoclonal antibodies displayed similar specificity by Western blot.

### d) Preparation and Characterization of Antibodies against P703P

Rabbits were immunized with either a truncated (P703Ptr1; SEQ ID NO: 172) or full-length mature form (P703Pfl; SEQ ID NO: 523) of recombinant P703P protein was expressed in and purified from bacteria as described above. Affinity purified polyclonal antibody was generated using immunogen P703Pfl or P703Ptr1 attached to a solid support. Rabbit monoclonal antibodies were isolated using SLAM technology at Immgenics Pharmaceuticals. Table VII below lists both the polyclonal and monoclonal antibodies that were generated against P703P.

**TABLE VIII**

| Antibody | Immunogen | Species/type |
|---|---|---|
| Aff. Purif. P703P (truncated); #2594 | P703Ptrl | Rabbit polyclonal |
| Aff. Purif. P703P (full length); #9245 | P703Pfl | Rabbit polyclonal |
| 2D4 | P703Ptrl | Rabbit monoclonal |
| 8H2 | P703Ptrl | Rabbit monoclonal |
| 7H8 | P703Ptrl | Rabbit monoclonal |

The DNA sequences encoding the complementarity determining regions (CDRs) for the rabbit monoclonal antibodies 8H2, 7H8 and 2D4 were determined and are provided in SEQ ID NO: 506-508, respectively.

Epitope mapping studies were performed as described above. Monoclonal antibodies 2D4 and 7H8 were found to specifically bind to the peptides of SEQ ID NO: 509 (corresponding to amino acids 145-159 of SEQ ID NO: 172) and SEQ ID NO: 510 (corresponding to amino acids 11-25 of SEQ ID NO: 172), respectively. The polyclonal antibody 2594 was found to bind to the peptides of SEQ ID NO: 511-514, with the polyclonal antibody 9427 binding to the peptides of SEQ ID NO: 515-517.

The specificity of the anti-P703P antibodies was determined by Western blot analysis as follows. SDS-PAGE was performed on (1) bacterially expressed recombinant antigen; (2) lysates of HEK293 cells and Ltk-/- cells either untransfected or transfected with a plasmid expressing full length P703P; and (3) supernatant isolated from these cell cultures. Protein was transferred to nitrocellulose and then Western blotted using the anti-P703P polyclonal antibody #2594 at an antibody concentration of 1 ug/ml. Protein was detected using horse radish peroxidase (HRP) conjugated to an anti-rabbit antibody. A 35 kDa immunoreactive band could be observed with recombinant P703P. Recombinant P703P runs at a slightly higher molecular weight since it is epitope tagged. In lysates and supernatants from cells transfected with full length P703P, a 30 kDa band corresponding to P703P was observed. To assure specificity, lysates from HEK293 cells stably transfected with a control plasmid were also tested and were negative for P703P expression. Other anti-P703P antibodies showed similar results.

Immunohistochemical studies were performed as described above, using anti-P703P monoclonal antibody. P703P was found to be expressed at high levels in normal prostate and prostate tumor tissue but was not detectable in all other tissues tested (breast tumor, lung tumor and normal kidney).

### e) Preparation and Characterization of Antibodies against P504S

Full-length P504S (SEQ ID NO: 108) was expressed and purified from bacteria essentially as described above for P501 S and employed to raise rabbit monoclonal antibodies using Selected Lymphocyte Antibody Method (SLAM) technology at Immgenics Pharmaceuticals (Vancouver, BC, Canada). The anti-P504S monoclonal antibody 13H4 was shown by Western blot to bind to both expressed recombinant P504S and to native P504S in tumor cells.

Immunohistochemical studies using 13H4 to assess P504S expression in various prostate tissues were performed as described above. A total of 104 cases, including 65 cases of radical prostatectomies with prostate cancer (PC), 26 cases of prostate biopsies and 13 cases of benign prostate hyperplasia (BPH), were stained with the anti-P504S monoclonal antibody 13H4. P504S showed strongly cytoplasmic granular staining in 64/65 (98.5%) of PCs in prostatectomies and 26/26 (100%) of PCs in prostatic biopsies. P504S was stained strongly and diffusely in carcinomas (4+ in 91.2% of cases of PC; 3+ in 5.5%; 2+ in 2.2% and 1+ in 1.1%) and high grade prostatic intraepithelial neoplasia (4+ in all cases). The expression of P504S did not vary with Gleason score. Only 17/91 (18.7%) of cases of NP/BPH around PC and 2/13 (15.4%) of BPH cases were focally (1+, no 2+ to 4+ in all cases) and weakly positive for P504S in large glands. Expression of P504S was not found in small atrophic glands, postatrophic hyperplasia, basal cell hyperplasia and transitional cell metaplasia in either biopsies or prostatectomies. P504S was thus found to be over-expressed in all Gleason scores of prostate cancer (98.5 to 100% of sensitivity) and exhibited only focal positivities in large normal glands in 19/104 of cases (82.3% of specificity). These findings indicate that P504S may be usefully employed for the diagnosis of prostate cancer.

### EXAMPLE 19

### CHARACTERIZATION OF CELL SURFACE EXPRESSION AND CHROMOSOME LOCALIZATION OF THE PROSTATE-SPECIFIC ANTIGEN P501 S

This example describes studies demonstrating that the prostate-specific antigen P501 S is expressed on the surface of cells, together with studies to determine the probable chromosomal location of P501S.

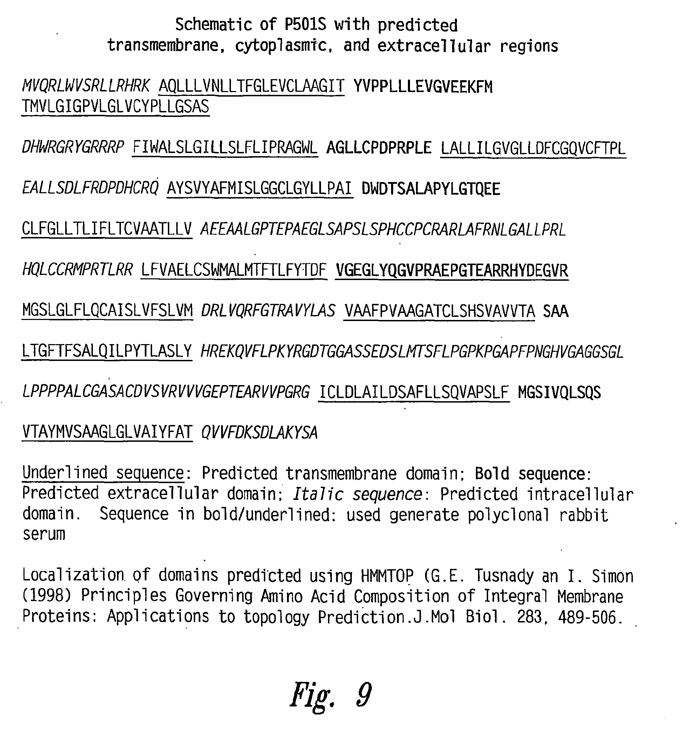

The protein P501S (SEQ ID NO: 113) is predicted to have 11 transmembrane domains. Based on the discovery that the epitope recognized by the anti-P501 S monoclonal antibody 10E3-G4-D3 (described above in Example 17) is intracellular, it was predicted that following transmembrane determinants would allow the prediction of extracellular domains of P501 S. Fig. 9 is a schematic representation of the P501 S protein showing the predicted location of the transmembrane domains and the intracellular epitope described in Example 17. Underlined sequence represents the predicted transmembrane domains, bold sequence represents the predicted extracellular domains, and italicized sequence represents the predicted intracellular domains. Sequence that is both bold and underlined represents sequence employed to generate polyclonal rabbit serum. The location of the transmembrane domains was predicted using HHMTOP as described by Tusnady and Simon (Principles Governing Amino Acid Composition of Integral Membrane Proteins: Applications to Topology Prediction, J. Mol. Biol. 283:489-506, 1998).

Based on Fig. 9, the P501 S domain flanked by the transmembrane domains corresponding to amino acids 274-295 and 323-342 is predicted to be extracellular. The peptide of SEQ ID NO: 518 corresponds to amino acids 306-320 of P501 S and lies in the predicted extracellular domain. The peptide of SEQ ID NO: 519, which is identical to the peptide of SEQ ID NO: 518 with the exception of the substitution of the histidine with an asparginine, was synthesized as described above. A Cys-Gly was added to the C-terminus of the peptide to facilitate conjugation to the carrier protein. Cleavage of the peptide from the solid support was carried out using the following cleavage mixture: trifluoroacetic acid:ethanediol:thioanisol:water:phenol (40:1:2:2:3). After cleaving for two hours, the peptide was precipitated in cold ether. The peptide pellet was then dissolved in 10% v/v acetic acid and lyophilized prior to purification by C18 reverse phase hplc. A gradient of 5-60% acetonitrile (containing 0.05% TFA) in water (containing 0.05% TFA) was used to elute the peptide. The purity of the peptide was verified by hplc and mass spectrometry, and was determined to be >95%. The purified peptide was used to generate rabbit polyclonal antisera as described above.

Surface expression of P501S was examined by FACS analysis. Cells were stained with the polyclonal anti-P501 S peptide serum at 10 µg/ml, washed, incubated with a secondary FITC-conjugated goat anti-rabbit Ig antibody (ICN), washed and analyzed for FITC fluorescence using an Excalibur fluorescence activated cell sorter. For FACS analysis of transduced cells, B-LCL were retrovirally transduced with P501S. To demonstrate specificity in these assays, B-LCL transduced with an irrelevant antigen (P703P) or nontransduced were stained in parallel. For FACS analysis of prostate tumor cell lines, Lncap, PC-3 and DU-145 were utilized. Prostate tumor cell lines were dissociated from tissue culture plates using cell dissociation medium and stained as above. All samples were treated with propidium iodide (PI) prior to FACS analysis, and data was obtained from PI-excluding (*i.e.,* intact and non-permeabilized) cells. The rabbit polyclonal serum generated against the peptide of SEQ ID NO: 519 was shown to specifically recognize the surface of cells transduced to express P501 S, demonstrating that the epitope recognized by the polyclonal serum is extracellular.

To determine biochemically if P501 S is expressed on the cell surface, peripheral membranes from Lncap cells were isolated and subjected to Western blot analysis. Specifically, Lncap cells were lysed using a dounce homogenizer in 5 ml of homogenization buffer (250 mM sucrose, 10 mM HEPES, 1mM EDTA, pH 8.0, 1 complete protease inhibitor tablet (Boehringer Mannheim)). Lysate samples were spun at 1000 g for 5 min at 4°C. The supernatant was then spun at 8000g for 10 min at 4°C. Supernatant from the 8000g spin was recovered and subjected to a 100,000g spin for 30 min at 4°C to recover peripheral membrane. Samples were then separated by SDS-PAGE and Western blotted with the mouse monoclonal antibody 10E3-G4-D3 (described above in Example 17) using conditions described above. Recombinant purified P501S, as well as HEK293 cells transfected with and over-expressing P501 S were included as positive controls for P501 S detection. LCL cell lysate was included as a negative control. P501 S could be detected in Lncap total cell lysate, the 8000g (internal membrane) fraction and also in the 100,000g (plasma membrane) fraction. These results indicate that P501 S is expressed at, and localizes to, the peripheral membrane.

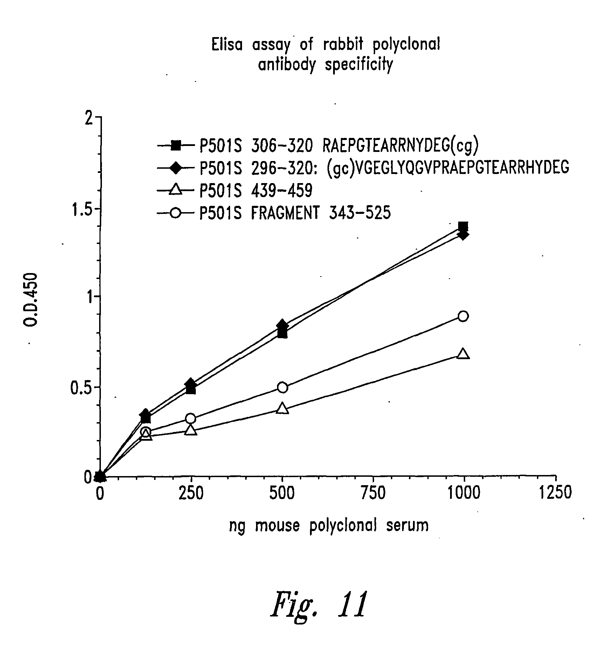

To demonstrate that the rabbit polyclonal antiserum generated to the peptide of SEQ ID NO: 519 specifically recognizes this peptide as well as the corresponding native peptide of SEQ ID NO: 518, ELISA analyses were performed. For these analyses, flat-bottomed 96 well microtiter plates were coated with either the peptide of SEQ ID NO: 519, the longer peptide of SEQ ID NO: 520 that spans the entire predicted extracellular domain, the peptide of SEQ ID NO: 521 which represents the epitope recognized by the P501S-specific antibody 10E3-G4-D3, or a P501 S fragment (corresponding to amino acids 355-526 of SEQ ID NO: 113) that does not include the immunizing peptide sequence, at 1 µg/ml for 2 hours at 37°C. Wells were aspirated, blocked with phosphate buffered saline containing 1% (w/v) BSA for 2 hours at room temperature and subsequently washed in PBS containing 0.1% Tween 20 (PBST). Purified anti-P501 S polyclonal rabbit serum was added at 2 fold dilutions (1000 ng - 125 ng) in PBST and incubated for 30 min at room temperature. This was followed by washing 6 times with PBST and incubating with HRP-conjugated goat anti-rabbit IgG (H+L) Affinipure F(ab') fragment at 1:20000 for 30 min. Plates were then washed and incubated for 15 min in tetramethyl benzidine. Reactions were stopped by the addition of 1 N sulfuric acid and plates were read at 450 nm using an ELISA plate reader. As shown in Fig. 11, the anti-P501 S polyclonal rabbit serum specifically recognized the peptide of SEQ ID NO: 519 used in the immunization as well as the longer peptide of SEQ ID NO: 520, but did not recognize the irrelevant P501S-derived peptides and fragments.

In further studies, rabbits were immunized with peptides derived from the P501 S sequence and predicted to be either extracellular or intracellular, as shown in Fig. 9. Polyclonal rabbit sera were isolated and polyclonal antibodies in the serum were purified, as described above. To determine specific reactivity with P501 S, FACS analysis was employed, utilizing either B-LCL transduced with P501 S or the irrelevant antigen P703P, of B-LCL infected with vaccinia virus-expressing P501 S. For surface expression, dead and non-intact cells were excluded from the analysis as described above. For intracellular staining, cells were fixed and permeabilized as described above. Rabbit polyclonal serum generated against the peptide of SEQ ID NO: 548, which corresponds to amino acids 181-198 of P501 S, was found to recognize a surface epitope of P501 S. Rabbit polyclonal serum generated against the peptide SEQ ID NO: 551, which corresponds to amino acids 543-553 of P501S, was found to recognize an epitope that was either potentially extracellular or intracellular since in different experiments intact or permeabilized cells were recognized by the polyclonal sera. Based on similar deductive reasoning, the sequences of SEQ ID NO: 541-547, 549 and 550, which correspond to amino acids 109-122, 539-553, 509-520, 37-54, 342-359, 295-323, 217-274, 143-160 and 75-88, respectively, of P501 S, can be considered to be potential surface epitopes of P501S recognized by antibodies.

In further studies, mouse monoclonal antibodies were raised against amino acids 296 to 322 to P501 S, which are predicted to be in an extracellular domain. A/J mice were immunized with P501 S/adenovirus, followed by subsequent boosts with an *E. coli* recombinant protein, referred to as P501 N, that contains amino acids 296 to 322 of P501 S, and with peptide 296-322 (SEQ ID NO: 898) coupled with KLH. The mice were subsequently used for splenic B cell fusions to generate anti-peptide hybridomas. The resulting 3 clones, referred to as 4F4 (IgG1,kappa), 4G5 (IgG2a,kappa) and 9B9 (IgG1 ,kappa), were grown for antibody production. The 4G5 mAb was purified by passing the supernatant over a Protein A-sepharose column, followed by antibody elution using 0.2M glycine, pH 2.3. Purified antibody was neutralized by the addition of 1 M Tris, pH 8, and buffer exchanged into PBS.

For ELISA analysis, 96 well plates were coated with P501 S peptide 296-322 (referred to as P501-long), an irrelevant P775 peptide, P501S-N, P501 TR2, P501 S-long-KLH, P501 S peptide 306-319 (referred to as P501-short)-KLH, or the irrelevant peptide 2073-KLH, all at a concentration of 2 ug/ml and allowed to incubate for 60 minutes at 37°C. After coating, plates were washed 5X with PBS + 0.1% Tween and then blocked with PBS, 0.5% BSA, 0.4% Tween20 for 2 hours at room temperature. Following the addition of supernatants or purified mAb, the plates were incubated for 60 minutes at room temperature. Plates were washed as above and donkey anti-mouse IgHRP-linked secondary antibody was added and incubated for 30 minutes at room temperature, followed by a final washing as above. TMB peroxidase substrate was added and incubated 15 minutes at room temperature in the dark. The reaction was stopped by the addition of 1 N H₂SO₄ and the OD was read at 450 nM. All three hybrid clones secreted mAb that recognized peptide 296-322 and the recombinant protein P501 N.

For FACS analysis, HEK293 cells were transiently transfected with a P501S/VR1012 expression constructs using Fugene 6 reagent. After 2 days of culture, cells were harvested and washed, then incubated with purified 4G5 mAb for 30 minutes on ice. After several washes in PBS, 0.5% BSA, 0.01% azide, goat anti-mouse Ig-FITC was added to the cells and incubated for 30 minutes on ice. Cells were washed and resuspended in wash buffer including 1% propidium iodide and subjected to FACS analysis. The FACS analysis confirmed that amino acids 296-322 of P501S are in an extracellular domain and are cell surface expressed.

The chromosomal location of P501 S was determined using the GeneBridge 4 Radiation Hybrid panel (Research Genetics). The PCR primers of SEQ ID NO: 528 and 529 were employed in PCR with DNA pools from the hybrid panel according to the manufacturer's directions. After 38 cycles of amplification, the reaction products were separated on a 1.2% agarose gel, and the results were analyzed through the Whitehead Institute/MIT Center for Genome Research web server (http://www-genome.wi.mit.edu/cgi-bin/contig/rhmapper.pl) to determine the probable chromosomal location. Using this approach, P501 S was mapped to the long arm of chromosome 1 at WI-9641 between q32 and q42. This region of chromosome 1 has been linked to prostate cancer susceptibility in hereditary prostate cancer (Smith et al. Science 274:1371-1374, 1996 and Berthon et al. Am. J. Hum. Genet. 62:1416-1424, 1998). These results suggest that P501 S may play a role in prostate cancer malignancy.

### EXAMPLE 20

### REGULATION OF EXPRESSION OF THE PROSTATE-SPECIFIC ANTIGEN P501 S

Steroid (androgen) hormone modulation is a common treatment modality in prostate cancer. The expression of a number of prostate tissue-specific antigens have previously been demonstrated to respond to androgen. The responsiveness of the prostate-specific antigen P501 S to androgen treatment was examined in a tissue culture system as follows.

Cells from the prostate tumor cell line LNCaP were plated at 1.5 x 10⁶ cells/T75 flask (for RNA isolation) or 3 x 10⁵ cells/well of a 6-well plate (for FACS analysis) and grown overnight in RPMI 1640 media containing 10% charcoal-stripped fetal calf serum (BRL Life Technologies, Gaithersburg, MD). Cell culture was continued for an additional 72 hours in RPMI 1640 media containing 10% charcoal-stripped fetal calf serum, with 1 nM of the synthetic androgen Methyltrienolone (R1881; New England Nuclear) added at various time points. Cells were then harvested for RNA isolation and FACS analysis at 0, 1, 2, 4, 8, 16, 24, 28 and 72-hours post androgen addition. FACS analysis was performed using the anti-P501 S antibody 10E3-G4-D3 and permeabilized cells.

For Northern analysis, 5-10 micrograms of total RNA was run on a formaldehyde denaturing gel, transferred to Hybond-N nylon membrane (Amersham Pharmacia Biotech, Piscataway, NJ), cross-linked and stained with methylene blue. The filter was then prehybridized with Church's Buffer (250 mM Na₂HPO₄, 70 mM H₃PO₄, 1 mM EDTA, 1% SDS, 1% BSA in pH 7.2) at 65°C for 1 hour. P501S DNA was labeled with 32P using High Prime random-primed DNA labeling kit (Boehringer Mannheim). Unincorporated label was removed using MicroSpin S300-HR columns (Amersham Pharmacia Biotech). The RNA filter was then hybridized with fresh Church's Buffer containing labeled cDNA overnight, washed with 1X SCP (0.1 M NaCl, 0.03 M Na₂HPO₄.7H₂O, 0.001 M Na₂EDTA), 1 % sarkosyl (n-lauroylsarcosine) and exposed to X-ray film.

Using both FACS and Northern analysis, P501 S message and protein levels were found in increase in response to androgen treatment.

### EXAMPLE 21

### PREPARATION OF FUSION PROTEINS OF PROSTATE-SPECIFIC ANTIGENS

The example describes the preparation of a fusion protein of the prostate-specific antigen P703P and a truncated form of the known prostate antigen PSA. The truncated form of PSA has a 21 amino acid deletion around the active serine site. The expression construct for the fusion protein also has a restriction site at 3' end, immediately prior to the termination codon, to aid in adding cDNA for additional antigens.

The full-length cDNA for PSA was obtained by RT-PCR from a pool of RNA from human prostate tumor tissues using the primers of SEQ ID NO: 607 and 608, and cloned in the vector pCR-Blunt II-TOPO. The resulting cDNA was employed as a template to make two different fragments of PSA by PCR with two sets of primers (SEQ ID NO: 609 and 610; and SEQ ID NO: 611 and 612). The PCR products having the expected size were used as templates to make truncated forms of PSA by PCR with the primers of SEQ ID NO: 611 and 613, which generated PSA (delta 208-218 in amino acids). The cDNA for the mature form of P703P with a 6X histidine tag at the 5' end, was prepared by PCR with P703P and the primers of SEQ ID NO: 614 and 615. The cDNA for the fusion of P703P with the truncated form of PSA (referred to as FOPP) was then obtained by PCR using the modified P703P cDNA and the truncated form of PSA cDNA as templates and the primers of SEQ ID NO: 614 and 615. The FOPP cDNA was cloned into the Ndel site and Xhol site of the expression vector pCRX1, and confirmed by DNA sequencing. The determined cDNA sequence for the fusion construct FOPP is provided in SEQ ID NO: 616, with the amino acid sequence being provided in SEQ ID NO: 617.

The fusion FOPP was expressed as a single recombinant protein in *E. coli* as follows. The expression plasmid pCRX1 FOPP was transformed into the *E*. *coli* strain BL21-CodonPlus RIL. The transformant was shown to express FOPP protein upon induction with 1 mM IPTG. The culture of the corresponding expression clone was inoculated into 25 ml LB broth containing 50 ug/ml kanamycin and 34 ug/ml chloramphenicol, grown at 37°C to OD600 of about 1, and stored at 4°C overnight. The culture was diluted into 1 liter of TB LB containing 50 ug/ml kanamycin and 34 ug/ml chloramphenicol, and grown at 37°C to OD600 of 0.4. IPTG was added to a final concentration of 1 mM, and the culture was incubated at 30°C for 3 hours. The cells were pelleted by centrifugation at 5,000 RPM for 8 min. To purify the protein, the cell pellet was suspended in 25 ml of 10 mM Tris-Cl pH 8.0, 2mM PMSF, complete protease inhibitor and 15 ug lysozyme. The cells were lysed at 4°C for 30 minutes, sonicated several times and the lysate centrifuged for 30 minutes at 10,000 x g. The precipitate, which contained the inclusion body, was washed twice with 10 mM Tris-Cl pH 8.0 and 1% CHAPS. The inclusion body was dissolved in 40 ml of 10 mM Tris-Cl pH 8.0, 100 mM sodium phosphate and 8 M urea. The solution was bound to 8 ml Ni-NTA (Qiagen) for one hour at room temperature. The mixture was poured into a 25 ml column and washed with 50 ml of 10 mM Tris-Cl pH 6.3, 100 mM sodium phosphate, 0.5% DOC and 8M urea. The bound protein was eluted with 350 mM imidazole, 10 mM Tris-Cl pH 8.0, 100 mM sodium phosphate and 8 M urea. The fractions containing FOPP proteins were combined and dialyzed extensively against 10 mM Tris-CI pH 4.6, aliquoted and stored at - 70°C.

### EXAMPLE 22

### REAL-TIME PCR CHARACTERIZATION OF THE PROSTATE-SPECIFIC ANTIGEN P501 S IN PERIPHERAL BLOOD OF PROSTATE CANCER PATIENTS

Circulating epithelial cells were isolated from fresh blood of normal individuals and metastatic prostate cancer patients, mRNA isolated and cDNA prepared using real-time PCR procedures. Real-time PCR was performed with the Taqman^{™} procedure using both gene specific primers and probes to determine the levels of gene expression.

Epithelial cells were enriched from blood samples using an immunomagnetic bead separation method (Dynal A.S., Oslo, Norway). Isolated cells were lysed and the magnetic beads removed. The lysate was then processed for poly A+ mRNA isolation using magnetic beads coated with Oligo(dT)25. After washing the beads in buffer, bead/poly A+ RNA samples were suspended in 10 mM Tris HCl pH 8.0 and subjected to reversed transcription. The resulting cDNA was subjected to real-time PCR using gene specific primers. Beta-actin content was also determined and used for normalization. Samples with P501 S copies greater than the mean of the normal samples + 3 standard deviations were considered positive. Real time PCR on blood samples was performed using the Taqman^{™} procedure but extending to 50 cycles using forward and reverse primers and probes specific for P501 S. Of the eight samples tested, 6 were positive for P501 S and β-actin signal. The remaining 2 samples had no detectable β-actin or P501 S. No P501 S signal was observed in the four normal blood samples tested.

### EXAMPLE 23

### EXPRESSION OF THE PROSTATE-SPECIFIC ANTIGENS P703P AND P501 S IN SCID MOUSE-PASSAGED PROSTATE TUMORS

When considering the effectiveness of antigens in the treatment of prostate cancer, the continued presence of the antigens in tumors during androgen ablation therapy is important. The presence of the prostate-specific antigens P703P and P501S in prostate tumor samples grown in SCID mice in the presence of testosterone was evaluated as follows.

Two prostate tumors that had metastasized to the bone were removed from patients, implanted into SCID mice and grown in the presence of testosterone. Tumors were evaluated for mRNA expression of P703P, P501S and PSA using quantitative real time PCR with the SYBR green assay method. Expression of P703P and P501 S in a prostate tumor was used as a positive control and the absence in normal intestine and normal heart as negative controls. In both cases, the specific mRNA was present in late passage tumors. Since the bone metastases were grown in the presence of testosterone, this implies that the presence of these genes would not be lost during androgen ablation therapy.

### EXAMPLE 24

### IDENTIFICATION OF P501 S AND P703P-SPECIFIC ANTIBODIES IN PROSTATE CANCER PATIENTS

Antibody responses specific for the prostate antigens P501 S or P703P in sera of prostate cancer patients and normal healthy donors were examined as follows.

Both Western blot analysis and ELISA analysis of sera were performed using P501S peptides (SEQ ID NO: 985-987) and P703P peptides (SEQ ID NO: 988-990). SEQ ID NO: 985, 986 and 987 represent amino acids 231-250, 246-266 and 492-521, respectively, of P501S. SEQ ID NO: 988, 989, 990, 1030, 1031, 1032 and 1033 represent amino acids 110-124, 140-154, 190-204, 62-76, 91-106, 200-214, and 229-245 respectively, of P703P. The results demonstrated that some of the prostate cancer patients had antibodies that recognize the same peptides (i.e. common epitopes) of either P501S or P703P and that these common epitopes were either not recognized or recognized at significantly lower levels by sera from normal donors. The data showed that at least 14 out of 48 prostate cancer patients possessed P501 S-specific antibodies, and that 3 out of 48 prostate cancer patients possessed P703P-specific antibodies with none of the patients having both antibodies specific for P501 S and antibodies specific for P703P.

### EXAMPLE 25

### ANTI-P503S MONOCLONAL ANTIBODY INHIBITS TUMOR GROWTH IN VIVO

The ability of the anti-P503S monoclonal antibody 20D4 to suppress tumor formation in mice was examined as follows.

Ten SCID mice were injected subcutaneously with HEK293 cells that expressed P503S. Five mice received 150 micrograms of 20D4 intravenously at day 0 (time of tumor cell injection), day 5 and day 9. Tumor size was measured for 50 days. Of the five animals that received no 20D4, three formed detectable tumors after about 2 weeks which continued to enlarge throughout the study. In contrast, none of the five mice that received 20D4 formed tumors. These results demonstrate that the anti-P503S Mab 20D4 displays potent anti-tumor activity *in vivo.*

### EXAMPLE 26

### CHARACTERIZATION OF A T CELL RECEPTOR CLONE FROM A P501 S-SPECIFIC T CELL CLONE

T cells have a limited lifespan. However, cloning of T cell receptor (TCR) chains and subsequent transfer essentially enables infinite propagation of the T cell specificity. Cloning of tumor-antigen TCR chains allows the transfer of the specificity into T cells isolated from patients that share the TCR MHC-restricting allele. Such T cells could then be expanded and used in adoptive transfer settings to introduce the tumor antigen specificity into patients carrying tumors that express the antigen. T cell receptor alpha and beta chains from a CD8 T cell clone specific for the prostate-specific antigen P501 S were isolated and sequenced as follows.

Total mRNA from 2 x 10⁶ cells from CTL clone 4E5 (described above in Example 12) was isolated using Trizol reagent and cDNA was synthesized. To determine Va and Vb sequences in this clone, a panel of Va and Vb subtype-specific primers was synthesized and used in RT-PCR reactions with cDNA generated from each of the clones. The RT-PCR reactions demonstrated that each of the clones expressed a common Vb sequence that corresponded to the Vb7 subfamily. Furthermore, using cDNA generated from the clone, the Va sequence expressed was determined to be Va6. To clone the full TCR alpha and beta chains from clone 4E5, primers were designed that spanned the initiator and terminator-coding TCR nucleotides. The primers were as follows: TCR Valpha-6 5'(sense): GGATCC---GCCGCCACC-ATGTCACTTTCTAGCCTGCT (SEQ ID NO: 899) BamHI site Kozak TCR alpha sequence TCR alpha 3' (antisense): GTCGAC-TCAGCTGGACCACAGCCGCAG (SEQ ID NO: 900) Sall site TCR alpha constant sequence TCR Vbeta-7. 5'(sense): GGATCC---GCCGCCACC-ATGGGCTGCAGGCTGCTCT (SEQ ID NO: 901) BamHI site Kozak TCR alpha sequence TCR beta 3' (antisense): GTCGAC---TCAGAAATCCTTTCTCTTGAC (SEQ ID NO: 902) Sall site TCR beta constant sequence. Standard 35 cycle RT-PCR reactions were established using cDNA synthesized from the CTL clone and the above primers, employing the proofreading thermostable polymerase PWO (Roche, Nutley, NJ).

The resultant specific bands (approx. 850 bp for alpha and approx. 950 for beta) were ligated into the PCR blunt vector (Invitrogen) and transformed into *E. coli. E .coli* transformed with plasmids containing full-length alpha and beta chains were identified, and large scale preparations of the corresponding plasmids were generated. Plasmids containing full-length TCR alpha and beta chains were submitted for sequencing. The sequencing reactions demonstrated the cloning of full-length TCR alpha and beta chains with the determined cDNA sequences for the Vb and Va chains being shown in SEQ ID NO: 903 and 904, respectively. The corresponding amino acid sequences are shown in SEQ ID NO: 905 and 906, respectively. The Va sequence was shown by nucleotide sequence alignment to be 99% identical (347/348) to Va6.2, and the Vb to be 99% identical to Vb7 (336/338).

### EXAMPLE 27

### CAPTURE OF PROSTATE SPECIFIC CELLS USING THE PROSTATE ANTIGEN P503S

As described above, P503S is found on the surface of prostate cells. Secondary coated microsphere beads specific for mouse IgG were coupled with the purified P503S-specific monoclonal antibody 1 D12. The bound P503S antibody was then used to capture HEK cells expressing recombinant P503S. This provides a model system for prostate-specific cell capture which may be usefully employed in the detection of prostate cells in blood, and therefore in the detection of prostate cancer.

P503S-transfected HEK cells were harvested and redissolved in wash buffer (PBS, 0.1% BSA, 0.6% sodium citrate) at an appropriate volume to give at least 5⁴ cells per sample. Round bottom Eppendorf tubes were used for all procedures involving beads. The stock concentrations were as shown below in Table IX.

**TABLE IX**

| **Stock concentrations** | **Sample concentration** | **Amount needed** |
|---|---|---|
| Epithelial enrich beads 4 beads/ml (Dynal Biotech Inc. Lake Success, NY) | 1⁷ beads/ml | 125 ul stock per 5 ml volume |
| 1D12 ascites antibody 2 mg/ml | 0.1 ug/ml (0.1X) to 5 ug/ml (5X) titrations | 0.05 ul to 2.5 ul stock per sample |
| α- Mamma Mu 0.9 mg/ml | 1 ug/ml (1X) | 1.1 ul stock per sample |
| Pan-mouse IgG beads 4⁸ beads/ml (Dynal Biotech) | 1⁷ beads/ml | 125 ul stock per 5 ml volume |

Blocked immunomagnetic beads were pre-washed as follows: all beads needed were pooled and washed once with 1 ml wash buffer. The beads were resuspended in a 3X volume of 1% BSA (v/v) in wash buffer and incubated for 15 min rotating at 4°C. The beads were then washed three times with 2X volume of wash buffer and resuspended to original volume. Non-blocked beads were pooled, washed three times with 2X volume of wash buffer and resuspended to original volume.

Primary antibody was incubated with secondary beads in a fresh Eppendorf for 30 minutes, rotating at 4°C. Approximately 200 ul wash buffer was added to increase the total volume for even mixing of the sample. The antibody-bead solution was transferred to a fresh Eppendorf, washed twice with an equal volume of wash buffer and resuspended to original volume. Target cells were added to each sample and incubated for 45 minutes, rotating at 4°C. The tubes were transferred to a magnet, the supernatant removed, taking care not the agitate the beads, and the samples were washed twice with 1 ml wash buffer. The samples were then ready for RT-PCR using a Dynabeads mRNA direct microkit (Dynal Biotech).

Epithelial cell enrichment was placed in a magnet and supernant was removed. The epithelial enrichment beads were then resuspended in 100 ul lysis/binding buffer fortified with Rnasin (2 U/ul per sample), and stored at - 70°C until use. Oligo (dT₂₅) Dynabeads were pre-washed as follows: all beads needed were pooled (23 ul/sample), washed three times with an excess volume of lysis/binding buffer, and resuspended to original volume. The lysis supernant was separated with a magnet and transferred to a fresh Eppendorf. 20 ul oligo(dT25) Dynabeads were added per sample and rolled for 5 min at room temperature. Supernant was separated using a magnet and discarded, leaving the mRNA annealed to the beads. The bead/mRNA complex was washed with buffer and resuspended in cold Tris-HCl.

For RT-PCR, the Tris-HCl supernatant was separated and discarded using MPS. For each sample containing 1⁵ cells or less, the following was added to give a total volume of 30 ul: 14.25 ul H₂O; 1.5 ul BSA; 6 ul first strand buffer; 0.75 mL 10 mM dNTP mix; 3 ul Rnasin; 3 ul 0.1M dTT; and 1.5 ul Superscript II. The resulting solution was incubated for 1 hour at 42°C, diluted 1:5 in H2O, heated at 80°C for 2 min to detach cDNA from the beads, and immediately placed on MPS. The supernatant containing cDNA was transferred to a new tube and stored at -20°C.

Table X shows the percentage of capture of P503S-transfected HEK cells as determined by RT-PCR.

**TABLE X**

| | % capture P503S-transfected HEK cells | % capture LnCAP cells |
|---|---|---|
| 0.1 ug/ml P503S Mab | 36.90 | 0.00 |
| 0.5 ug/ml P503S Mab | 67.40 | 2.93 |
| 1 ug/ml P503S Mab | 40.22 | 0.00 |
| 5 ug/ml P503S Mab | 13.11 | 0.00 |
| Anti-Mu beads only, non-blocked | 1.42 | 0.00 |
| Anti-Mu beads only, blocked | 15.65 | 20.21 |
| Absolute control, non-capture cells | 100.00 | 100.00 |

### EXAMPLE 28

### IMMUNIZATION OF MICE WITH RECOMBINANT P703P

*In vivo* immunogenicity studies were performed using a variety of P703P recombinant protein formulations. Specifically, groups of mice were immunized with the P703P formulations shown below in Table XI, wherein "C'amidated P703P" represents P703P amidated at the C terminal; "truncated-P703P" represents a truncated form of P703P, and "FOPP" represents a fusion of P703P and PSA.

**TABLE XI**

| GROUP | ANTIGEN | DOSE | SOURCE | ADJUVANT | ROUTE |
|---|---|---|---|---|---|
| 1 | C'amidated P703P | 20 ug | *E. coli* | AS1 | lm, sq (fp) |
| 2 | Truncated P703P | 20 ug | *E. coli* | AS1 | lm, sq (fp) |
| 3 | C'amidated P703P | 20 ug | Pichia | AS1 | lm, sq (fp) |
| 4 | P703P | 20 ug | Pichia | AS1 | Im, sq (fp) |
| 5 | FOPP | 20 ug | *E. coli* | AS1 | Im, sq (fp) |
| 6 | P703P | 10⁷ pfu | | none | Sq base of tail |
| 7 | C'amidated P703P | 20 ug | *E. coli* | MPL-SE | Im, sq (fp) |
| 8 | Truncated P703P | 20 ug | *E. coli* | MPL-SE | Im, sq (fp) |
| 9 | C'amidated P703P | 20 ug | Pichia | MPL-SE | Im, sq (fp) |
| 10 | P703P | 20 ug | Pichia | MPL-SE | Im, sq (fp) |
| 11 | FOPP | 20 ug | *E. coli* | MPL-SE | Im, sq (fp) |
| 12 | Naïve (control) | | | | |

Each protein immunization was done in four sites: subcutaneously (sq) in both footpads and intramuscularly (im) in the leg. Each immunization was done 3 weeks apart, with sera plus spleen and lymph node (LN) cells being harvested 10 days following the last immunization.

T cell proliferation and interferon-gamma assays were performed as follows. 250,000 spleen or 100,000 LN cells were plated in 96 well plates and stimulated with 1-10 ug/ml of antigen. Antigens tested include the five proteins listed above, P703P expressed in baculovirus, NS1 control protein, PSA (for FOPP groups only), and P703P peptide pools. Peptide pools consisted of 20-mer peptides overlapping by 15 amino acids with each pool containing 6-8 peptides. Con A was used as a positive control. Cultures were pulsed with H3-thymidine on day 4 after initiation of culture for assaying proliferation. For assaying IFNγ levels by ELISA, supernatants were also pulled on day 4. In addition, sera were pooled and assayed by ELISA for IgG antibodies against the recombinant proteins listed above.

All immunogens elicited strong antibody responses to the immunogen. In all cases these responses reacted with other sources of P703P protein, including strong reactivity with both Pichia forms and baculovirus forms of P703P. AS1 adjuvant elicited stronger antibody responses than MPL-SE. The best immunogen in terms of eliciting a response against Pichia and baculovirus forms of P703P was FOPP, but all of the immunogens elicited strong reactive P703P antibody responses against the *E*. *coli* derived P703P. In both the proliferation and interferon-gamma assays, all immunogens elicited fairly good T cell response to the immunogen, with most animals with detectable responses to their immunogen also responding to other sources of P703P protein. Again, AS1 adjuvant elicited better T cell responses than MPL-SE.

### EXAMPLE 29

### ANALYSIS OF P703P EXPRESSION IN PROSTATE CANCER AND NORMAL TISSUE BY IMMUNOHISTOCHEMISTRY (IHC)

Protein expression of the prostate cancer antigen P703P was analyzed in prostate cancer and normal tissue by IHC analysis using an array of anti-P703P polyclonal and monoclonal antibodies (see Table XII). P703P protein expression was also analyzed in HEK293/P703P transfectants by flow cytometry and by Western blot analysis using the same anti-P703P antibodies. The P703-R087 and P703P-2059 antibodies stained HEK293/P703P transfectants by flow cytometric analysis. P703P-2594 was used for Western blot analysis.

**TABLE XII**

| ANTI-P703 ANTIBODIES | | | | |
|---|---|---|---|---|
| Antibody | Lot# | Species/type | Immunogen | Purification |
| P703P-2594 | 370-84 | Rabbit polyclonal | bacteria derived truncated P703P (c-term 159 aa) | Immunogen |
| P703P-9245 | 690.46 | Rabbit polyclonal | bacteria derived full length P703/NS1 fusion | Immunogen |
| P703-2D4 | | Rabbit polyclonal | bacteria derived truncated P703P (c-term 159 aa) | Protein A |
| P703-8H2 | | Rabbit monoclonal | bacteria derived truncated P703P (c-term 159 aa) | Protein A |
| P703-7H8 | | Rabbit monoclonal | bacteria derived truncated P703P (c-term 159 aa) | Protein A |
| P703P-2059 | 690.88 | Rabbit polyclonal | Baulovirus derived full length form | Immunogen |
| P703P-R087 | | Rabbit monoclonal | Baulovirus derived full length form | Protein A |
| P703P-R080 | | Rabbit monoclonal | Baulovirus derived full length form | Protein A |
| PA-P703P-9245 | 858-130 | Rabbit polyclonal | bacteria derived full length P703/NS1 fusion | Protein A |
| AP-P703P-9245 | 858-109 | Rabbit polyclonal | bacteria derived full length P703/NS1 fusion | Baulovirus full length |

In order to further evaluate tissues expression of the prostate cancer antigen P703P protein, immunohistochemistry (IHC) analysis was performed on a diverse range of tissue sections. Tissue samples were fixed in formalin solution for 12-24 hrs and embedded in paraffin before being sliced into 8 micron sections. Steam heat induced epitope retrieval (SHIER) in 0.1 M sodium citrate buffer (pH 6.0) was used for optimal staining conditions. Sections were incubated with 10% serum/PBS for 5 minutes. Primary antibody was added to each section for 25 minutes at indicated concentrations followed by 25 minute incubation with anti-rabbit biotinylated antibody. Endogenous peroxidase activity was blocked by three 1.5 minute incubations with hydrogen peroxidase. The avidin biotin complex/horse radish peroxidase (ABC/HRP) system was used along with DAB chromogen to visualize antigen expression. Slides were counterstained with hematoxylin to visualize cell nuclei. For Western blot analysis, whole cell lysates were generated by incubating the cells in Triton-X 100 containing lysis buffer for 30 minutes on ice. Lysates were then cleared by centrifugation at 10,000 rpm for 5 minutes at 4° C. Samples were diluted with SDS-PAGE loading buffer containing beta-mercaptoethanol, then boiled for 10 minutes prior to loading the SDS-PAGE gel. Protein was transferred to nitrocellulose and probed using 1 ug/ml purified anti-P703P rabbit polyclonal sera 2594 (lot # 370-84) or rabbit monoclonal P703P-R087. Blots were revealed using goat anti-rabbit lg coupled to HRP followed by incubation in ECL substrate. For FACS analysis, single cell suspensions of P703P/HEK293 transfectants were generated and cells were incubated in permeabilization buffer (PBS+ saponin + BSA) at room temp. The cells were then incubated for 30 minutes with 10ug/ml of purified anti-P703P polyclonal sera or monoclonal antibodies. Following 3 washes in permeabilization buffer, cells were then incubated with a 1:100 dilution of goat anti-rabbit lg(H+L)-FITC(Southern Biotechnology) for 30 minutes at RT. After 3 washes, the cells were resuspended in buffer and analyzed by FACS.

Optimal IHC results were obtained using the heat retrieval and affinity purified AP-P703P-9245 antibody against full-length P703. IHC analysis using the above procedures with this antibody showed weak staining in 6/23 metastatic prostate cancer sections. 24/33 primary prostate cancer sections stained positive with this antibody. Additionally, staining was observed in malignant ducts, benign (normal ducts) in various prostate tumors, basal, myoepithelial cells in various prostate tumors, mast cells, and in association with lipofuchsin granules.

A panel of normal tissue including brain, breast, uterus, parotid, spleen, liver, ovary, adrenal, cervix, tonsil, esophagus, bronchus, lung, kidney, testis, pancreas, skeletal muscle, colon, small intestine, skin, and thyroid sections, were all negative.

### EXAMPLE 30

### DETECTION OF CIRCULATING PROSTATE TUMOR CELLS IN PERIPHERAL BLOOD USING CELL CAPTURE TECHNOLOGY

The peripheral bloods of 55 urology patients, diagnosed with prostate cancer, benign prostatic hyperplasia (BPH), bladder and other prostate disorders, were collected and processed using the Dynal Epithelial Enrich kit (Dynal, Lake Success, NY), which uses magnetic beads coated with the epithelial-specific antibody Ber-EP4 to specifically capture metastatic tumor cells. Total RNA was then isolated using the QIAGEN RNeasy Mini Kit (QIAGEN, Valencia, CA), DNase treated, and first strand cDNA was synthesized from the cell capture fractions using Life Technologies Superscript II Rnase H reverse transcriptase (Invitrogen Life Technologies, Carlsbad, CA). Real-time PCR was performed to evaluate the level of expression of prostate tumor genes P501S (also referred to herein as L1-12, e.g., SEQ ID NO: 110), P703P (e.g., SEQ ID NO:524), P503S (also referred to herein as N1-1862, e.g., SEQ ID NO:111), P504S (also referred to herein as FI-12, e.g., SEQ ID NO:107), P510S (e.g., SEQ ID NO:535 and 536), and P1020C (also referred to as 22553, e.g., SEQ ID NO:591) using the housekeeping genes, Actin, Beta-2-Microglobulin, and Cytokeratin 19, as controls.

By combining the expression patterns of P501 S and P504S, we were able to detect at least two-fold overexpression of the two genes combined for 10/24 prostate cancer patients and 4/25 BPH patients as compared to the mean signal for the bladder and other prostate disorders group. At least two-fold mean overexpression was also detected in the cancer group as compared to the BPH and Bladder cancer/other prostate disorders group for genes P501S, P503S, P703P, P504S, and P510S. In addition, the same samples were identified with P501 S, P504S, and P510S. Thus, expression analysis of prostate tumor sequences identified herein in blood samples following cell capture can be utilized as a diagnostic tool, for example in the detection of circulating tumor cells, e.g., metastatic tumor cells, in patients.

### EXAMPLE 31

### IDENTIFICATION OF A P703P-DERIVED CD4+ T CELL EPITOPE

A total of 40 15-mer peptides overlapping by 10 amino acids spanning amino acids 47 to 254 of the P703P protein sequence were generated by standard procedure. Dendritic cells (DC) were derived from PBMC of a normal female donor using GMCSF and IL-4 by standard procedures. CD4 T cells were generated from the same donor as the DC using MACS beads and negative selection. DC were pulsed overnight with pools containing 10 of the 15 mer peptides, with each peptide at 0.25 microgram/ml final concentration. Pulsed DC were washed and plated at 1x10e4 cells /well of 96-well round bottom plates, and purified CD4 T cells were added at 1x10e5/well. Cultures were supplemented with 60 ng/ml IL-6 and 10 ng/ml IL-12 and incubated at 37°C. Cultures were re-stimulated as above on a weekly basis and were tested for specific proliferation using as APC, DC generated and pulsed as above, supplemented with 5 ng/ml IL-7 and 10 u/ml IL-2. Following 5 in-vitro stimulation cycles, lines (each line corresponds to one well) were assayed for proliferation and cytokine production in response to the stimulating pools vs an irrelevant pool of peptides derived from the P703P sequence. 12 lines were identified that demonstrated specific proliferation (measured in standard tritiated thymidine proliferation assays) and cytokine production (measured in γ-interferon ELISA assays) in response to the stimulating peptide pool. These lines were further tested for specific recognition of the peptide pool, specific recognition of individual peptides in the pool and specific recognition of recombinant P703P protein. Using this approach, a peptide epitope derived from the prostate tumor specific protein, P703P, recognized by CD4+ T cells in the context of MHC class II, was identified. The peptide corresponds to amino acids 145-159 of the P703P protein sequence of SEQ ID NO: 525, and has the sequence GNSCLVSGWGLLANG (SEQ ID NO:992). The cDNA sequence encoding this epitope is set forth in SEQ ID NO:991.

### EXAMPLE 33

### IDENTIFICATION OF P706P EXTENDED SEQUENCES

Related clones were identified above in Example 1 (e.g., SEQ ID NO: 46, 212, 213, 214, 215, 288 and 415) as having prostate-specific expression patterns. Upon further analysis, an extended sequence for these clones was identified as follows. Sequence specific primers were used to amplify products from a prostate tumor library, resulting in the identification of clones 8B6-P3 (SEQ ID NO: 993) and 8B6-P4 (SEQ ID NO: 994). Both clones were transformed into appropriate cells, isolated, sequenced, and the resulting sequences were used as queries to search against the Genbank sequence database. This search revealed that 8B6-P3 and 8B6-P4 and other related EST sequences were components of the same genomic 7q21 BAC clone. Based on the close proximity along the genomic backbone and on the similar expression profiles, these components were concluded to be part of the same gene, referred to herein as P706P. Northern analysis demonstrated high over-expression in prostate tumors and prostate tissues, and also showed expression in brain and ovary. The four sequences disclosed herein (SEQ ID NO: 993-997) are representatives of the 4 longest contiguous pieces of cDNA-derived sequence along the genomic backbone. Contigation of clones 8B6.P3 (SEQ ID NO: 993) and 8-B6.P4 (SEQ ID NO: 994), using also available EST sequences, allowed the identification of the extended P706P sequence set forth in SEQ ID NO: 995.

### EXAMPLE 34

### IDENTIFICATION OF P713P EXTENDED SEQUENCE

The identification of SEQ ID NO: 146, also referred to as P219, was described above. This example describes the identification of an extended sequence for this clone, as well as the bioinformatic identification of the full length cDNA and protein sequences containing the same. Full length laboratory cloning efforts were initiated with an EST search and alignment. The sequence identified by this approach was confirmed with a 5' RACE assay (Clonetech; see also Chenchik et al., 1995, 1996) in which a library of adaptor ligated prostate tumor cDNAs are used as a template to amplify the desired 5' end of a gene using the 5' adaptor primer and a gene specific 3' primer. Additional sequence was identified using a nucleic acid-based screen in which a phagemid prostate tumor library was screened by growing plaques, transferring and immobilizing DNA onto nylon filters, and hybridizing with a radioactive gene-specific probe. The filters were then exposed to film and plaques corresponding to radioactively exposed positive hits were picked and prepped for DNA isolation. The DNA sequence identified using the above approach both confirmed the original clone sequence and further yielded additional 5' sequence. This extended cDNA sequence, also referred to as P713P, is set forth in SEQ ID NO: 998. In a separate analysis, it was determined that P713P corresponds to a protein referred to as Prostatic Secretory Protein (PSP), the full length cDNA sequence of which is set forth in SEQ ID NO: 999, encoding the amino acid sequence set forth in SEQ ID NO: 1000.

### EXAMPLE 35

### IDENTIFICATION OF P1 E EXTENDED SEQUENCE

The identification of the cDNA sequences set forth in SEQ ID NO: 620, 621 and 622, also referred to as P1 E, were described above in Example 1. These sequences resulted from a serological expression cloning effort and subsequent nucleic acid screening of the same cDNA expression library. An expression cDNA library was constructed from prostate tumor extracted mRNA using routine methodologies. This library was screened with autologous patient sera. (prostate tumor and patient sera were from the same individual) 10,000 pfu were plated on 6 large LB agar plates, allowed to grow up for 6 hrs, and IPTG-infused filters were applied to induce expression and immobilize proteins as they were produced. The filters were oriented to the plate and were removed 12 hours later. The filters were blocked in PBS 1% tween, and then incubated with the patient sera for 12 hours at 4 degrees Celsius. The washed filters were incubated in I-125 conjugated protein A for 1 hour at room temperature, washed again and exposed to film overnight. Exposed spots on the film indicating expressed protein recognized by the autologous sera were picked, purified, the plasmid excised, and prepped for DNA isolation and subsequent sequencing. This procedure resulted in the identification of clone PT44A22.

Primers were designed from the PT44A22 sequence in order to further screen the same expression library. For probe labeling, PCR amplification of the original PT4A22 DNA fragment was performed in the presence of radioactive nucleotides. The probe was incubated with nylon filters onto which the DNA of 10000-15000 clones of the expression library were immobilized. After a few hours of hybridization at 65 degrees in Church's hybridization buffer, the filters were washed in a solution of 1XSCP, 1% sarkosyl. Film was exposed to the washed filters overnight and positive signals were used to pick the appropriate colonies. These picks, which contained about 50 colonies each, were further purified by the method described previously, resulting in a pure colony pick which was then grown up and prepped for plasmid DNA. The plasmid containing the clone of interest was digested for fragment size and the insert was sequenced using flanking promoter primers. This procedure resulted in the clone PT44.P2. Together, PT44A22 and PT44.P2 make up the extended P1 E sequence disclosed herein as SEQ ID NO: 1004, encoding the amino acid sequence set forth in SEQ ID NO: 1005.

### EXAMPLE 36

### IMMUNOHISTOCHEMICAL ANALYSIS OF P51 0S

Identification of the prostate specific gene P510S is described above (e.g., Example 1). In order to evaluate P510S protein expression, immunohistochemistry (IHC) analysis was performed on a diverse range of tissue sections. Tissue samples were fixed in formalin solution for 12-24 hours and embedded in paraffin before being sliced into 8 micron sections. Steam heat induced epitope retrieval (SHIER) in 0.1 M sodium citrate buffer (pH 6.0) was used for optimal staining conditions. Sections were incubated with 10% serum/PBS for 5 minutes. Primary antibody was added to each section for 25 minutes at indicated concentrations followed by a 25 minute incubation with anti-rabbit biotinylated antibody. Endogenous peroxidase activity was blocked by three 1.5 minute incubations with hydrogen peroxidase. The avidin biotin complex/horse radish peroxidase (ABC/HRP) system was used along with DAB chromogen to visualize antigen expression. Slides were counterstained with hematoxylin to visualize cell nuclei.

Using this approach, P510S protein expression was detected in 31/32 primary prostate tumor samples and in 8/17 bone metastasis samples. Some expression was also detected in 4/5 normal prostate samples and 3/5 testis samples. No expression was detected in thyroid, spleen, uterus, ovary, pancreas, lung, liver, kidney, heart, stomach, large intestine, small intestine, brain and adrenal gland.

### EXAMPLE 37

### DIAGNOSTIC EVALUATION OF PROSTATE ANTIGEN P504S

Identification of the prostate specific sequence P504S has been described above (e.g., Example 1). In order to evaluate P504S protein expression, immunohistochemistry (IHC) analysis was performed on a diverse range of tissue sections. Tissue samples were fixed in formalin solution for 12-24 hours and embedded in paraffin before being sliced into 8 micron sections. Steam heat induced epitope retrieval (SHIER) in 0.1 M sodium citrate buffer (pH 6.0) was used for optimal staining conditions. Sections were incubated with 10% serum/PBS for 5 minutes. Primary antibody was added to each section for 25 minutes at indicated concentrations followed by a 25 minute incubation with anti-rabbit biotinylated antibody. Endogenous peroxidase activity was blocked by three 1.5 minute incubations with hydrogen peroxidase. The avidin biotin complex/horse radish peroxidase (ABC/HRP) system was used along with DAB chromogen to visualize antigen expression. Slides were counterstained with hematoxylin to visualize cell nuclei.

### A. P504S EXPRESSION IN COLON CARCINOMAS: CORRELATION WITH TUMOR GRADE AND PATIENT SURVIVAL

This example evaluates the immunoreactivity of P504 is in normal colon and invasive colon carcinomas, and also evaluates the correlation between P504 expression and tumor grade, stage, and patient survival. Routinely processed resected colon carcinomas (n=44), villous adenomas (V, n=4) and tubular or tubulovillous adenomas (TA/TVA, n=24) were cut at 5 µm and immunostained with P504 on a TechMate 1000 (Ventana Medical Systems, Tucson, AZ) automated immunostainer using an avidin/biotin complex (ABC) staining procedure. Positive immunostaining in cytoplasm was graded: 0 = negative staining; 1+ = up to 33% cells positive; 2+ = 34-66%; 3+ = >66%. Tumors were graded on review of H&E stained slides, and stage, location, and follow up were obtained from pathology reports and patient charts.

Invasive carcinomas were from 18 males and 26 females, ages 45-87, from right colon (23), transverse (6), left (14), and unknown (1). Tumor stage was I(4), II (17), III (18), and IV (5). Tumor grade was Grade 1 (23), 2 (9), and 3 (12). Remnant polyp was present in 7 carcinomas. Immunostaining for p504S was weakly positive in surface epithelium of normal colon. In carcinomas, P504 was graded 0 (16), 1+ (6), 2+ (8), and 3+ (14). There was no difference between superficial and deep regions of the tumors. Remnant polyp was 2+ or 3+ in 4 cases, 0 in 3 cases. The percentage of carcinomas positive for each grade of P504S correlated inversely with tumor grade and patient survival. There was no correlation between P504S expression and TNM stage or overall stage. Most adenomas (25/35) were 2+ or 3+, regardless of histologic type.

Thus, P504S was highly expressed in adenomas and well-differentiated colon carcinomas, and weak or absent in normal colon mucosa and poorly differentiated carcinomas. P504S expression correlated inversely with patient survival. In view of these results, immunostaining for P504S represents a useful adjunct with prognostic significance in the diagnostic evaluation of colon carcinomas.

### B. EXPRESSION OF P504S IN ATYPICAL ADENOMATOUS HYPERPLASIA OF THE PROSTATE

Atypical Adenomatous Hyperplasia (AAH), also known as adenosis, is characterized by the abnormal architectural patterns but without significant cytologic atypia in prostatic glandular epithelium. AAH may represent a precursor lesion of prostatic adenocarcinoma. P504S has been found to be highly expressed in prostatic adenocarcinoma, but not in benign prostatic epithelium. In this example, the expression of P504S was evaluated in AAH.

A total of 46 cases including 26 of AAH (prostatectomy: N=18, biopsy: N=6, and transurethral resection: N=2), 10 cases of adenocarcinomas with Gleason score 2+2=4 and 10 cases of benign prostatic hyperplasia (BPH) were examined by immunohistochemistry for P504S and high molecular weight cytokeratin 34βE12. 34βE12 staining confirmed the presence of patchy basal cells in all 26 cases of AAH. P504S showed strong cytoplasmic granular staining in all (10 of 10) low-grade carcinomas and in 4 of 26 cases of AAH (15 %). No BPHs or benign glands adjacent to carcinomas and AAH were positive for P504S. Diffuse P504S staining pattern was found in low-grade carcinomas, while focal P504s positivity was seen in AAH.

Thus, expression of P504S was found in all low-grade carcinomas and a small percentage of AAH, but not in BPH or other benign glands. These findings suggest that AAH is a lesion different from low-grade cancer or BPH, at least with respect to P504S expression. Moreover, AAH may be considered as a premalignant or potentially premalignant lesion.

### C. P504S AS A DIAGNOSTIC MARKER FOR MINIMAL PROSTATIC ADENOCARCINOMA ON NEEDLE BIOPSY

Establishing diagnosis of minimal prostatic cancer (PC) on needle biopsy is a major diagnostic challenge. A marker specific for prostate cancer will enhance our ability to detect minimal disease. P504S, identified by cDNA subtraction and microarray technology, has been demonstrated to be highly expressed in PC but not in benign prostatic glands. The aim of this study was to evaluate P504S expression as a means for detecting minimal PC.

A total of 111 prostate needle biopsies, including 52 of minimal prostatic adenocarcinoma with Gleason Score 6 (N=51) or 8 (N=1) and 59 benign prostates, were examined by using immunohistochemistry specific for P504S and high molecular weight cytokeratin (34βE12). Minimal PC, defined as a tumor focus <1mm in diameter on H&E staining, was present as only one single focus in the entire biopsy (N=35), or as one small focus in one needle core in addition to carcinoma present elsewhere (N=18).

P504S immunopositivity was found in 51/52 cases (98%) of minimal PC but not in any benign prostates (0/58) or benign glands adjacent to malignant glands. Atrophy, basal cell hyperplasia and transitional cell metaplasia were all negative for P504S. The only case of PC with negative staining of P504S showed features of foamy carcinoma in a biopsy obtained from the transition zone. High molecular weight cytokeratin (34βE12) immunostaining confirmed the absence of basal cells in the focus of carcinoma in all 52 cases.

The high specificity and sensitivity of P504S for detecting limited prostatic adenocarcinoma illustrates its diagnostic value in a clinical setting. Using P504S as a immunohistochemical marker can thus increase the accuracy in pathology diagnosis of minimal prostatic adenocarcinoma on needle biopsy.

### EXAMPLE 38

### IMMUNOHISTOCCHEMICAL ANALYSIS OF P767P

In order to evaluate protein expression of the prostate antigen P767P, immunohistochemistry (IHC) analysis was performed on a diverse range of tissue sections. Tissue samples were fixed in formalin solution for 12-24 hours and embedded in paraffin before being sliced into 8 micron sections. Steam heat induced epitope retrieval (SHIER) in 0.1 M sodium citrate buffer (pH 6.0) was used for optimal staining conditions. Sections were incubated with 10% serum/PBS for 5 minutes. Primary antibody was added to each section for 25 minutes at indicated concentrations followed by a 25 minute incubation with anti-rabbit biotinylated antibody. Endogenous peroxidase activity was blocked by three 1.5 minute incubations with hydrogen peroxidase. The avidin biotin complex/horse radish peroxidase (ABC/HRP) system was used along with DAB chromogen to visualize antigen expression. Slides were counterstained with hematoxylin to visualize cell nuclei. Using this approach, P767S protein expression was detected in 7/7 prostate cancers, 5/5 normal prostates, 1/1 normal kidney, 1/1 cerebellum, 0/1 normal liver, 0/1 normal heart, 1/1 normal lung, 1/1 normal colon, 1/1 normal tonsil, and 1/1 normal cortex.

### EXAMPLE 39

### P510S AND MAPS-P510S CONSTRUCTS FOR DNA IMMUNIZATION AND ANTIBODY GENERATION

The identification of prostate sequence P510S has been identified above (e.g., Example 1). Plasmid DNA containing P510S was used as template in a PCR reaction to amplify the open reading frame of P510S using P510S-AW186 sense primers with Hind III sites and a Kozak sequence, and P510S-AW187 antisense primers with Nhe I sites and a stop codon. PCR product was purified from agarose gel, digested with Hind III and Nhe I, and ligated to JA4304 vector that had been linearized with the same two restriction enzymes. The ligation mixture was transformed into E. coli and several clones were randomly selected for restriction enzyme analysis and DNA sequencing. P510S/JA4304#2 and # 3 clones were confirmed to have the desired P510S sequence. The determined DNA sequence for this construct is set forth in SEQ ID NO: 1006, encoding the polypeptide of SEQ ID NO: 1009.

For construction of MAPS-P510S, the pCRX4 vector was used. pCRX4 is a MAPS fusion vector based on JA4304 that was made to facilitate the cloning of MAPS fusion constructs. In pCRX4, MAPS was cloned down stream of Kozak sequence and was followed by a multiple cloning site. To generate the MAPS-P510S construct, a PCR reaction was used to amplify the P510S coding region using MAPS-P510S-AW184 sense primers with EcoR I sites, and P510S-AW185 antisense primers for MAPS-P510S with Kpn I sites and a stop codon. PCR product was purified from agarose gel, digested with EcoR I and Kpn I, and ligated to pCRX4 vector that had been linearized with the same two restriction enzymes. The ligation mixture was transformed into E. coli and several clones were randomly selected for restriction enzyme analysis and DNA sequencing. MAPS-P510S/pCRX4 #1 and #3 clones were confirmed. The determined DNA sequence for this construct is set forth in SEQ ID NO: 1007, encoding the polypeptide of SEQ ID NO: 1008.

### EXAMPLE 40

### DETECTION OF P501 S AND P703P-SPECIFIC ANTIBODIES IN SERA OF PROSTATE CANCER PATIENTS AND NORMAL DONORS

The identification and characterization of prostate specific antigens P501 S and P703P are described above, illustrative forms of which are set forth in SEQ ID NO: 113 (encoded by SEQ ID NO: 110) and SEQ ID NO: 525 (encoded by SEQ ID NO: 524), respectively. This example describes the detection of antibodies specific for these proteins in the sera of prostate cancer patients. Effective protein-based screening for prostate antigen-specific antibodies in patient sera has been problematic due to protein instability and/or contamination of small amounts of E. coli-derived proteins. However, the carboxyamidation of recombinant P501 S and P703P has provided improved stability for these proteins, as confirmed by SDS-PAGE. Thus, carboxyamidated P501 S and P703P proteins were used to screen for P501 Sand P703P-specific antibodies in the sera of 55 prostate cancer patients and 13 normal male donors, as follows.

Flat-bottomed immuno-plates were incubated and coated for at least 3 hours at room temperature or overnight at 4 degrees C with 50ul coating buffer (0.1 N carbonate buffer; pH 9.5) supplemented with 600 ng of carboxyamidated P501S or 300 ng of carboxyamidated P703P protein. The solution was removed and at least 150 ul of blocking buffer (1:1 mixture of 10% NFDM (Non Fat Dry Milk)/PBS and Superblock (Pierce) was added in each well and incubated for 3 hours at room temp or overnight at 4 degrees C. After washing at least 3 times with 0.05% Tween 20-containing PBS and once with PBS, 50 ul of sera from prostate cancer patients or normal donors was diluted with 10% NGS (normal goat sera; Gibco BRL, #16210-072) containing PBS, added and incubated for 3 hours at room temperature or for overnight at 4 degrees C. After washing at least 3 times with 0.05% Tween-containing PBS and once with PBS, 100 ul of HRP-conjugated anti-human IgG (Jackson ImmunoResearch Laboratories Inc.; #709-036-149) (x 8000 dilution by 1:1 mixture of 10% NFDM/PBS+Superblock) were added and incubated for 30-60 minutes at room temperature. After washing at least 6 times with 0.05% Tween-containing PBS and once with PBS, 80-100 ul of the TMB-substrate solution was added and incubated at room temperature. After obtaining suitable colors on the standard of IgG between 10-30 min, 40-50 ul (a half volume of substrate) of 0.1 N sulfuric acid solution was added to stop the enzyme reaction. Absorbances were then determined with a microplate reader (Biotec Instruments).

Using this approach, P501 S-specific antibodies were detected in 32/55 prostate cancer patients and 5/13 normal donors. P703-specific antibodies were detected in 24/55 prostate cancer patients and 0/13 normal donors. These results confirm the in vivo immunogenicity of P501 S and P703P. Moreover, the results demonstrate that the carboxyamidated forms of P501 S and P703P retain the epitopes that are recognized by rabbit/mouse antibodies, as well as by antibodies from prostate cancer patients.

### EXAMPLE 41

### GENERATION OF MONOCLONAL ANTIBODIES TO P71 1P

The identification and characterization of P711 P (SEQ ID NO: 382, encoding the protein of SEQ ID NO: 383) is described above (e.g., Example 3). For production and purification of P711 P protein used for antibody generation, E. coli expressing recombinant P711 P protein were grown overnight in LB Broth with the appropriate antibiotics at 37°C in a shaking incubator. The next morning, 10 ml of the overnight culture was added to 500 ml of 2x YT plus appropriate antibiotics in a 2L-baffled Erlenmeyer flask. When the optical density (at 560 nanometers) of the culture reached 0.4-0.6, the cells were induced with IPTG (1 mM). Four hours after induction with IPTG the cells were harvested by centrifugation. The cells were then washed with phosphate buffered saline and centrifuged again. The supernatant was discarded and the cells were either frozen for future use or immediately processed. Twenty milliliters of lysis buffer was added to the cell pellets and the suspension was vortexed. To break open the E. coli cells, this mixture was then run through a French Press at a pressure of 16,000 psi. The cells were then centrifuged again and the supernatant and pellet were checked by SDS-PAGE for the partitioning of the recombinant protein. For proteins that localized to the cell pellet, the pellet was resuspended in 10 mM Tris pH 8.0, 1% CHAPS and the inclusion body pellet was washed and centrifuged again. This procedure was repeated twice more. The washed inclusion body pellet was solubilized with either 8 M urea or 6 M guanidine HCI containing 10 mM Tris pH 8.0 plus 10 mM imidazole. The solubilized protein was added to 5 ml of nickel-chelate resin (Qiagen) and incubated for 45 minutes to 1 hour at room temperature with continuous agitation. After incubation, the resin and protein mixture were poured through a disposable column and the flow through was collected. The column was then washed with 10-20 column volumes of the solubilization buffer. The antigen was then eluted from the column using 8M urea, 10 mM Tris pH 8.0 and 300 mM imidazole and collected in 3 ml fractions. A SDS-PAGE gel was run to determine which fractions to pool for further purification.

As a final purification step, a strong anion exchange resin, e.g., Hi-Prep Q (Biorad) was equilibrated with the appropriate buffer and the pooled fractions from above were loaded onto the column. Each antigen was eluted off of the column with an increasing salt gradient. Fractions were collected as the column was run and another SDS-PAGE gel was run to determine which fractions from the column to pool. The pooled fractions were dialyzed against 10 mM Tris pH 8.0. This material was then evaluated for purity as determined by SDS-PAGE or HPLC, concentration as determined by Lowry assay or amino acid analysis, identity as determined by amino terminal protein sequence, and endotoxin level was determined by the Limulus (LAL) assay. The proteins were then vialed after filtration through a 0.22-micron filter and the antigens were frozen until needed for immunization.

To generate anti-P711P mouse monoclonal antibodies, mice were immunized intraperitoneally (IP) with 50 micrograms of recombinant P711P protein that had been mixed to form an emulsion with an equal volume of Complete Freund's Adjuvant (CFA). Every three weeks animals were injected i.p. with 50 micrograms of recombinant P711P protein that had been mixed with an equal volume of IFA to form an emulsion. After the fourth injection, spleens were isolated and standard hybridoma fusion procedures were used to generate anti-P711P mouse monoclonal antibodies.

Anti-P711 P monoclonal antibodies were screened by ELISA analysis using the bacterially expressed recombinant P711 P protein. For characterization of anti-P711 P monoclonal antibodies by ELISA, 96 well plates were coated with P711 P antigen by incubating with 50 microliters (typically about 1 microgram) at 4 degrees C for 20 hours. 250 microliters of BSA blocking buffer was added to the wells and incubated at room temperature for 2 hours. Plates were washed 6 times with PBS/0.01 % TWEEN. Fifty microliters of each undiluted monoclonal supernatant were added per well and incubated at room temperature for 30 minutes. Plates were washed as described above before 50 microliters of goat anti-mouse horseradish peroxidase (HRP) at a 1:10000 dilution was added and incubated at room temperature for 30 minutes. Plates were washed as described above and 100µl of TMB Microwell Peroxidase Substrate was added to each well. Following a 15 minute incubation in the dark at room temperature the colorimetric reaction was stopped with 100 µl 1 N H2SO4 and read immediately at 450 nm. For Western blot analysis, recombinant P711 P protein was diluted with SDS-PAGE loading buffer containing beta-mercaptoethanol, then boiled for 10 minutes prior to loading the SDS-PAGE gel. Protein was transferred to nitrocellulose and probed with each of the anti-P711 P hybridoma supernatants. Anti-mouse-HRP was used to visualize the anti-P711P reactive bands by incubation in ECL substrate. By this approach, at least seven anti-P711 P monoclonal antibodies were generated and demonstrated to be reactive with recombinant P711 protein by ELISA and/or Western analysis.

### EXAMPLE 42

### RECOGNITION OF CARBAMIDOMETHYLATED CTL EPITOPE BY P501 S-SPECIFIC T-CELLS

The identification and characterization of prostate specific antigen P501S has been described above, a representative full length DNA sequence for which is set forth in SEQ ID NO: 110, encoding the amino acid sequence set forth in SEQ ID NO: 113. CTL specific for a peptide of P501 S (peptide 370-378, amino acid sequence CLSHSVAVV) were previously demonstrated to recognize naturally processed P501 S as demonstrated by recognition of P501 S transduced target cells. In this example, mice were immunized with P501S DNA followed by a second immunization using a P501 S-containing adenovirus delivery vector 14 days later. On day 35, spleen cells were harvested and stimulated in vitro weekly using irradiated Jurkat A2kb-P501 cells. CTL lines were assayed for lytic activity against parental Jurkat A2Kb cells, Jurkat A2Kb cells transduced with P501 S, Jurkat A2Kb cells pulsed with p370-378, or Jurkat A2KbT cells pulsed with the carboxy amidated p370-378 peptide. Using this approach, it was found that p370-378 specific T cells not only recognize the synthetic unmodified 370-378 peptide, but also recognize a carbamidomethylated form of 370-378. These results are important for demonstrating cross-recognition of naturally processed P501, an ummodified synthetic peptide corresponding to amino acids 370-378, P501 S, and the modified synthetic peptide 370-378. Thus, carbamidomethylation of recombinant P501 S does not impair T cell recognition of this CTL epitope.

### EXAMPLE 43

### PREPARATION OF P501 S FUSION PROTEINS

The identification and characterization of prostate specific antigen P501S has been described above, a representative full length DNA sequence for which is set forth in SEQ ID NO: 110, encoding the amino acid sequence set forth in SEQ ID NO: 113. In this example, illustrative P501S fusion constructs were made and the encoded fusion proteins produced. Using sequence specific primers, the C-terminal truncated region of P501S (amino acids 341-527) was PCR amplified using the following conditions:
10µl 10X Pfu buffer
1µl 10mM dNTPs
2µl 10µM each oligo
83µl sterile water
1.5µl Pfu DNA polymerase (Stratagene, La Jolla, CA)
50 ηg DNA
96°C 2 minutes
96°C 30 seconds 72°C 1 minute 30 seconds X 40 cycles
72°C 4 minutes

The resulting PCR product was digested with Ncol and NotI and cloned into the commercially available vector pET32 (Novagen, Madison Wl) that was also digested with Ncol and Notl. The pET32 vector incorporates an N - terminal fusion with the Trx antigen, which is Thioredoxin, an *E. coli* protein that can enhance expression for difficult to express antigens, and can further enhance solubility of the resulting fusion protein. The resulting cDNA sequence for the coding region of this Trx-P501 S fusion construct is set forth in SEQ ID NO: 1010, encoding the fusion amino acid sequence set forth in SEQ ID NO: 1011.

### EXAMPLE 44

### EVALUATION OF P501 S IMMUNOGENICITY

The identification and characterization of prostate specific antigen P501S has been described above, a representative full length DNA sequence for which is set forth in SEQ ID NO: 110, encoding the amino acid sequence set forth in SEQ ID NO: 113. This example describes the results of a preclinical in vivo immunogenicity study evaluating vaccination with P501S protein (either unmodified or carboxy amidated), in combination with various adjuvants, compared with DNA/adenovirus P501 S immunization. The following experimental design was employed:

| **Group** | **Antigen** | **System** |
|---|---|---|
| 1 | 10 ug P501S unmodified | AS1 |
| 2 | 10 ug P501S unmodified | AS2 |
| 3 | 10 ug P501 S unmodified | AS7 |
| 4 | 10 ug P501S carboxyamidated | AS1 |
| 5 | 10 ug P501 S carboxyamidated | AS2 |
| 6 | 10 ug P501 S carboxyamidated | AS7 |
| 7 | 100 ug P501 S DNA/10⁸ pfu P501 S adenovirus | None |
| 8 | Buffer | AS2. |
| 9 | Naïve | - |
| 3 immunizations, 2 weeks apart; Harvest 8 days after last immunization; Route = i.m. + f.p.; Mice = A2Tg females (5/group); | | |

Assays were subsequently performed for evaluating CTL responses, antibody responses and CD4 responses (interferon-gamma & proliferation). The results of this evaluation demonstrated that both unmodified and carboxy amidated P501S protein delivered in adjuvant are immunogenic, as assessed by antibody and CD4 T cell responses. The CD4 and antibody responses observed with protein administration were generally stronger than the response observed with DNA/Adenovirus immunization. CTL responses were not detected following protein immunization in the adjuvant systems tested. However, CTL were consistently detectable following P501 S adenovirus immunization. The antibody and CD4 responses recognized both unmodified P501 S and P501 S modified by carboxy amidation. Comparative evaluation of responses obtained with various adjuvant systems demonstrated little effect on immunogenicity, with the exception that AS7 generated a stronger Th1 response.

### EXAMPLE 45

### IDENTIFICATION OF A NATURALLY PROCESSED CTL EPITOPE FOR P501 S

The identification and characterization of prostate specific antigen P501 S has been described above, a representative full length DNA sequence for which is set forth in SEQ ID NO: 110, encoding the amino acid sequence set forth in SEQ ID NO: 113. In this example, a naturally processed, murine A2Kb CTL epitope has been identified for P501 S. This epitope was mapped using a series of C-terminal deletion constructs, C-terminal subclones containing the last 92 amino acids of P501 S, and sets of peptides spanning P501 S regions of interest. The T cell clones used to map the epitope were derived from limiting dilutions of splenocytes from P501 S DNA immunized and P501 S-adenovirus immunized C57BL/6 A2Kb transgenic mice. Thirty-seven T cell clones were identified as reactive to P501 S-transduced Jurkat A2Kb targets in CTL assays. The lines, however, were not reactive to Jurkat A2Kb cells pulsed with a previously identified naturally processed P501 S epitope corresponding to amino acid residues 370-378 of SEQ ID NO: 113. This observation pointed to the existence of a second epitope other than the 9-mer epitope of amino acid residues 370-378. The T cell clones were thus tested with Jurkat A2Kb cells transduced with C-terminal deletion constructs denoted FragA-FragE. None of the T cell clones generated CTL responses to these deletion constructs, indicating that the epitope likely mapped within the C-terminal 92 amino acids of P501 S (461-553). Six T cell clones were demonstrated to be responsive to Jurkat A2Kb clones expressing P501 S amino acid residues 462-553 or 462-517. Using a murine ELISPOT assay, these transduced targets, and Jurkat A2Kb targets pulsed with various 20-mer, 15-mer, 10-mer and 9-mer peptides, allowed for the identification of a naturally processed epitope for P501S, having the sequence SACDVSVRV, and corresponding to amino acid residues 464-472 of SEQ ID NO: 113.

### EXAMPLE 46

### CHARACTERIZATION OF P790P EXPRESSION

The identification of prostate antigen P790P has been described above, the full length cDNA sequence for which is set forth in SEQ ID NO: 526, encoding the amino acid sequence set forth in SEQ ID NO: 527. For recombinant expression in mammalian cells, P790P was subcloned into pCEP4 and VR1013 (a polylinker modified VR1012 vector) mammalian expression vectors. The constructs were transfected into HEK293 cells (ATCC) using Lipofectamine 2000 (Gibco). The HEK293 cells were plated in a 6 well dish at a density of 300,000 cells/ml in DMEM (Gibco) containing 10% FBS (Hyclone) and allowed to incubate for 6-8 hours. One ug of DNA was then added to 100 ul of DMEM containing no FBS and 3 ul of Lipofectamine 2000 was added to 100 ul of DMEM containing no FBS. Both were incubated for 5 minutes at room temperature. The lipofectamine/DMEM mixture was added to the DNA/DMEM mixture and incubated for 15 minutes at room temperature. The lipofectamine 2000/DNA mix was then added to the HEK293 cells and incubated for 48-72 hrs at 37 degrees C with 7% CO2. Cells were collected and pelleted by centrifugation and washed twice with PBS.

For Western blot analysis, whole cell lysates were generated by diluting samples directly into SDS-PAGE loading buffer containing beta-mercaptoethanol, then incubated for 10 minutes at 70 degrees C prior to loading the SDS-PAGE gel. Protein was transferred onto nitrocellulose and probed using the mouse anti-FLAG antibody (Kodak Scientific Imaging, Cat# IB13026) at a concentration of 10ug/ml. The blot was detected with goat anti-mouse-HRP followed by incubation in ECL substrate. An identical blot was probed with anti-P790 peptide sera (3-13-2001, 3875L) at 1/1000. This blot was detected with goat anti-rabbit-HRP followed by incubation in ECL substrate. Using this approach, expression of recombinant P790P protein was detected in transiently transfected HEK293 cells using an anti-FLAG polyclonal antibody.

### EXAMPLE 47

### EXPRESSION OF P504S IN ADENOMATOUS HYPERPLASIA OF THE PROSTATE

This example describes how the prostate antigen P504S, and antibodies specific for P504S, represent important diagnostic reagants useful in the detection of prostate carcinoma, and, additionally, in the differentiation of atypical adenomatous hyperplasia (AAH) from prostate carcinoma. AAH of the prostate, also known as adenosis, is characterized by abnormal architectural patterns, but without significant cytologic atypia, in prostatic glandular epithelium. It is often difficult to distinguish AAH from prostatic carcinoma. In addition, it has been suggested that AAH is a precursor lesion of theprotatic adenocarcinoma. P504S (the DNA and amino acid sequences for which were disclosed in SEQ ID NOs: 107 and 108), is a protein that we have previously shown to be highly over-expressed in prostatice adenocarcinoma, and therefore represents an excellent marker for prostate cancer.

Here we use immunohistochemistry to determine the expression profile of P504S in a number of different prostate samples. A total of 80 samples were examined, including 40 of AAH (prostatectomy: n=30, biopsy: n=6, and transurethral resection: n=4), 20 cases of prostatic adenocarcinomas and 20 cases of benign prostatic hyperplasia (BPH). Immunohistochemistry for P504S and a basal cell specific marker, 34βE12, was performed on all samples.

The 34βE12 stain confirmed the presence of patchy basal cells in all 40 cases of AAH. P504S staining showed strong cytoplasmic granular staining in 20/20 prostatic carcinomas and in 6 of 40 cases of AAH (15%). No BPH or benign prostate glands adjacent to carcinomas or adjacent to AAH were positive for P504S. A diffuse staining pattern of P504S was found in prostate carcinoma samples, while focal positivity was seen in the positive cases of AAH.

These findings demonstrate that combination staining using 34βE12 and P504S has a strong diagnostic value in distinguishing AAH from prostatic adenocarcinoma, and thus further validates the use of P504S in any of a variety of illustrative diagnostic and therapeutic embodiments described herein, for example.

### EXAMPLE 48

### GENERATION OF AN ILLUSTRATIVE P703P FUSION PROTEIN

The identification and characterization of related forms of the prostate specific antigen P703P has been described above, one representative DNA sequence for which is set forth in SEQ ID NO: 171, encoding an amino acid sequence set forth in SEQ ID NO: 172. In this example, an illustrative P703P fusion construct was made and the encoded fusion protein produced. This recombinant fusion protein can be used, for example, in diagnostic methods for the detection of prostate cancer and/or to elicit an immune response in a subject for the prevention and/or treatment of prostate cancer.

Full length human PAP DNA was obtained by PCR from a prostate cancer tissue cDNA library (PT library) with primers hPAPF1 and hPAPRV1. The sequences for these primers are: hPAPF1
5'CGGCGGATCCGCCGCCACCATGAGAGCTGCACCCCTCCTCCT-3' (SEQ ID NO: 1014) and hPAPRV1
5'CGGCCTCGAGCTAATCTGTACTGTCTTCAGTACCTTGATGGCTG-3' (SEQ ID NO: 1015).

The PCR product was then cloned into the PCR BluntII TOPO vector. To modify the hPAP a second PCR was performed with primers FOPP2F1
5'GAGAAAACCGTCCAGGCCAGTAAGGAGTTGAAGTTTGTGACTTTGGTG-3' (SEQ ID NO: 1016) and HPAPRV1 (SEQ ID NO: 1015), using hPAP/TOPO BluntII as a template.

FOPP/PCRX1 (P703P containing DNA) was also modified using primers FOPPF1
5'CGGCGGGCATATGCATCACCATCACCATCACATCATAAACGGCGAGGAC TGCAG-3' (SEQ ID NO: 1017) and FOPP2RV1
5'CACCAAAGTCACAAACTTCAACTCCTTACTGGCCTGGACGGTTTTCTC-3' (SEQ ID NO: 1018).

Finally, using primers FOPPF1 and hPAPRV1, and the modified hPAP and P703P PCR products as templates, a PCR was performed to generate FOPP2. This PCR product was then cloned in to the PCRX1 vector at the restriction enzyme cutting sites Ndel and Xho1. The FOPP2/PCRX1 plasmid was then used to transform the E. coli strain Tuner (DE3) pLys S and plated out on LB plates containing kanamycin and chloamphenicol. Using SDS-PAGE analysis and Western blot analysis with antibodies against His-Tag, the transformant was shown to express the FOPP2 protein when expression was induced using IPTG.

To generate large quantity of the FOPP2 protein, the expression clone culture was inoculated into 20 ml of STB containing 50 µg/ml kanamycin and 34 µg/ml chloamphenicol, and grown at 37°C until the OD600 read approximately 1. The culture was then diluted into 1 liter of STB containing 50 µg/ml kanamycin and 34 µg/ml chloamphenicol, and grown at 37°C until the OD600 read approximately 0.4. IPTG was then added to a final concentration of 1mM, and the culture was incubated at 37°C for 2 hours. The cells were centrifuged at 5000 RPM for 8 minutes, washed in PBS, re-centrifuged, and stored at -70°C.

To purify the protein, the cell pellet was re-suspended in 25 ml of 10mM Tris-Cl (pH 8.0) containing 2 mM PMSF and protease inhibitors. The cells were sonicated several times and the lysate was centrifuged for 60 minutes at 4000 RPM. The precipitate, containing the inclusion bodies, was washed twice with 10 mM Tris-HCl (pH 8.0) containing 1% CHAPS. The inclusion body was dissolved in 40 ml of binding buffer (10 mM Tris-HCl, pH 8.0, 100 mM sodium phosphate, and 8M urea). The solution was mixed with 10 ml of Ni-NTA resin (Qiagen) for 30 minutes at room temperature. The mixture was then poured into a 25 ml column, and washed with 50 ml of wash buffer (10mM Tris-Cl pH 6.3, 100 mM sodium phosphate, 0.5% DOC and 8M urea). The bound protein was then eluted using elution buffer (10 mM Tris-Cl, pH 8.0, 350 mM Imidazole, 100 mM sodiumphosphate, and 8M urea). The fractions containing FOPP2 were combined and dialyzed extensively using a large volume of Tris-Cl, pH 8.0, aliquoted, and stored at -70°C. The FOPP2 sequence was confirmed, and the DNA and amino acid sequence disclosed in SEQ ID NOs: 1019 and 1020, respectively.

### EXAMPLE 49

### EXPRESSION OF THE PROSTATE ANTIGEN P501 S IN E. COLI

This example describes the recombinant production of P501S polypeptides that are useful, for example, in immunodiagnostic and immunotherapeutic applications. The identification and characterization of prostate specific antigen P501 S has been described above, a representative full length DNA sequence for which is set forth in SEQ ID NO: 110, encoding the amino acid sequence set forth in SEQ ID NO: 113. This example describes the expression of recombinant P501S C (corresponding to amino acids 257-553) and P501S D (corresponding to amino acids 316-553) antigens using an E. coli expression system combined with an N-terminal histadine tag and a TAT (twin arginine translocator) signal peptide fusion.

### Expression of P501 S C in E. coli:

The P501S C coding region was PCR amplified with the following primers:
PDM-930 5'cttccacggctgcaccagctgtgc 3' (SEQ ID NO:1023)
PDM-165 5'cacggacgtgaattctacgctgagtatttggcc 3' (SEQ ID NO:1024)

### Expression of P501S D in E. coli:

The P501 S D coding region was PCR amplified with the following primers:
PDM-929 5'cactatgatgaaggcgttcggatgggcag 3' (SEQ ID NO:1025)
PDM-165 5'cacggacgtgaattctacgctgagtatttggcc 3' (SEQ ID NO:1024)

### The PCR was performed with the following reaction components:

10µl 10X Pfu buffer
1µl 10mM dNTPs
2µl 10µM of each primer
83µl of sterile water
1.5µl Pfu DNA polymerase (Stratagene, La Jolla, Ca)
50ng DNA

### PCR amplification was performed using the following reaction conditions:

96°C for 2 minutes, followed by 40 cycles of:
96°C for 20 seconds;
67°C for 15 seconds; and
72°C for 2 minutes, followed by a final extension step of:
72°C for 4 minutes.

The PCR products were digested with EcoRI and cloned into pPDM and pTAT (pPDM using a TAT signal peptide leader) at the Eco721 and EcoRI. Constructs were confirmed to be correct through sequence analysis and transformed into BLR (DE3) pLys S, HMS 174 (DE3) pLys S, Rosetta (DE3), and Rosetta (DE3) pLys S cells to confirm protein expression. The amino acid sequence for the P501 S C-His construct was confirmed, and is disclosed in SEQ ID NO:1029, with the corresponding DNA sequence disclosed in SEQ ID NO:1027. The amino acid sequence for the P501S D-His construct was confirmed, and is disclosed in SEQ ID NO:1028, with the corresponding DNA sequence disclosed in SEQ ID NO:1026. These recombinant P501S polypeptides can be used, for example, in immunodiagnostic and/or immunotherapeutic embodiments, such as those described herein.

From the foregoing it will be appreciated that, although specific embodiments of the invention have been described herein for purposes of illustration, various modifications may be made without deviating from the spirit and scope of the invention. Accordingly, the invention is not limited except as by the appended claims.

## Claims

1. An isolated antibody, or antigen-binding fragment thereof, that specifically binds to a polypeptide comprising an amino acid sequence of SEQ ID NO: 113, wherein the antibody or fragment binds to any of the following amino acid sequences: SEQ ID NOs: 985-987, 978, 980-984, 1012, 1022; amino acid residues 246-325, 278-353, 370-378, 462-553, 462-517, 464-472, 471-553 of SEQ ID NO: 113.

2. A method for detecting the presence of a cancer in a patient, comprising the steps of:
(a) contacting a biological sample obtained from the patient with an antibody or antibody fragment according to claim 1;
(b) detecting in the sample an amount of polypeptide that binds to the antibody or antigen-binding fragment; and
(c) comparing the amount of polypeptide to a predetermined cut-off value and therefrom determining the presence of a cancer in the patient.

3. A composition comprising an antibody or antibody fragment according to claim 1, and a physiologically acceptable carrier or immunostimulant.

4. A composition according to claim 3 for use in stimulating an immune response in a patient.

5. A composition according to claim 3 for use in the treatment of a prostate cancer in a patient.

6. A diagnostic kit comprising at least one antibody or antibody fragment according to claim 1 and a detection reagent, wherein the detection reagent comprises a reporter group.
